# EUROPEAN PATENT APPLICATION

(11) **EP 1 612 281 A2**
(43) Date of publication of application: **04.01.2006**
(21) Application number: 05252797.5
(22) Date of filing: 06.05.2005
(51) Int. Cl.: C12Q 1/68

(54) **Methods for assessing patients with acute myeloid leukemia**

(30) Priority: 06.05.2004 US 568635 P
(71) Applicant: Veridex, LLC, Warren, NJ 07059 (US)
(72) Inventor: Raponi, Mitch, San Diego, CA 92101 (US)
(74) Representative: Mercer, Christopher Paul

(57) **Abstract**

Methods for treating cancer, and preferably hematological malignancy, patients include analyzing gene expression profiles and/or molecular markers of a patient to determine status and/or prognosis of the patient. The invention also provides methods of analyzing whether a non-relapsed or non-refractory patient is likely to respond to treatment with farnesyl transferase inhibitors (FTIs) and, optionally, other therapeutics. The methods are also useful for monitoring patient therapy and for selecting a course of therapy. Genes modulated in response to FTI treatment are provided and are used in formulating the profiles.

## Description

### BACKGROUND OF THE INVENTION

This application relates to US Patent Application Serial No. 60/637,265 filed December 17,2004.
This invention relates to diagnostics, prognostics, and treatments for acute myeloid leukemia (AML) based on the detection of molecular markers and/or gene expression analysis.

Karyotyping is currently effective in providing prognostic value while it also serves to identify biologically distinct subtypes of AML. In addition, mutations in genes such as FLT3, c-KIT, AML1, GATA1, CEBPA and N-RAS are implicated in the pathogenesis of the disease. It is clear that screening for FLT3 and CEBPA mutations can stratify groups that have different risks of relapse. Effective risk stratification can allow for the appropriate use of allogeneic stem cell transplantation or other adjuvant therapies. Two papers published recently describe gene expression profiling of newly diagnosed adult AML patients and its use in predicting clinical outcome. Bullinger et al. (2004); and Valk et al. (2004). These studies show how gene-expression profiling can further refine clinical outcome prediction.

Valk et al. (2004) evaluated 285 patients (bone marrow or peripheral blood) on the Affymetrix U133A chip. The patient samples encompassed a wide range of cytogenetic and molecular abnormalities. Only 16 clusters were identified indicating AML may not be as heterogeneous as previously thought. Several of the clusters corresponded well with the cytogenetically and molecularly defined sub-types of AML thus supporting their use in the WHO classification system. These clusters were also seen by Bullinger et al. (2004) and other previously published smaller studies. Schoch et al. (2002); Debernardi et al. (2003); and Kohlmann et al. (2003). These clusters, not surprisingly, correlated with prognosis since they were associated with well known prognostic karyotypes.

Bullinger et al. (2004) investigated expression profiles from 116 adult patients (65 peripheral blood and 54 bone marrow) using cDNA arrays. In addition to the work done by Valk et al. (2004) they also developed a133 gene classifier for predicting clinical outcome across all cytogenetic risk groups. Using a training set of 59 samples and a testing set of 57 samples they showed that the 133 genes clustered patients into poor and good outcome groups (p = 0.006 log rank; odds ratio, 10, 95% CI, 2.6-29.3).

Notably, the genes identified in both these studies overlap, only in part, to predictor genes previously identified in childhood leukemia. Yagi et al. (2003). Also, there is no overlap between the prognostic gene set identified by Bullinger et al. (2004) and the 3 genes recently identified that predict response to tipifarnib. US patent application serial no. 10/883,436.

The farnesyl transferase (FTase) enzyme mediates the covalent attachment of a carbon farnesyl moiety to the C-terminal CAAX (C, cysteine; A, aliphatic residue; X, any amino acid) recognition motif. Reiss et al. (1990). This farnesylation is further processed by cleavage of the 3 terminal amino acids (AAX) and methylation of the C-terminal isoprenyl-cysteine. The inhibition of protein farnesylation abrogates the correct subcellular localization required for protein function. Originally, the oncogenic Ras protein was thought to be the target for the antiproliferative effects of FTIs in cancer biology. Reuter et al. (2000). However, it has since been shown that inhibition of Ras farnesylation does not account for all of actions of tipifarnib. For example, FTIs do not always require the presence of mutant Ras protein to produce antitumor effects. Karp et al. (2001). Indeed, while early clinical studies were designed around populations with a high frequency of ras mutations, such as advanced colorectal and pancreatic cancer, no significant difference in response rates were seen when compared to placebo. Van Cutsem et al. (2004); and Rao et al. (2004).

Several other farnesylated proteins have been implicated as candidate targets that may mediate the antitumorigenic effects of FTIs including the small GTPase proteins Rho B, the centromere proteins CENP-E and CENP-F, the protein tyrosine phosphatase PTP-CAAX, and the nuclear membrane structural lamins A and B. The inhibition of farnesylation of these proteins may lead to the antiproliferative effect of FTIs and also indirectly modulate several important signaling molecules including TGFβRII,

MAPK/ERK, PI3K/AKT2, Fas (CD95), NF-κB, and VEGF. Adnane et al. (2000); Morgan et al. (2001); Jiang et al. (2000); Na et al. (2004); Takada et al. (2004); and Zhang et al. (2002). Regulation of these signaling pathways leads to the modulation of cell growth, proliferation, and apoptosis. Thus, FTIs may have complex inhibitory effects on several cellular events and pathways.

There are currently no methods for determining status or predicting overall survival of these patients.

### BRIEF SUMMARY OF THE INVENTION

The invention is a method of using one or more gene signatures for predicting prognosis in patients with acute myeloid leukemia. These signatures can be used alone or in combination depending upon the type of drug treatment.

The present invention encompasses a method of assessing prognosis (survival/outcome) in an AML patient by obtaining a biological sample from the patient; and measuring the expression levels in the sample of genes selected from those encoding mRNA: i) corresponding to SEQ ID NOs: 1 or 5-22; or ii) recognized by the probe sets selected from psid numbers corresponding to SEQ ID NOs: 1 or 5-22 as depicted in Table 7 where the gene expression levels above or below pre-determined cut-off levels are indicative of AML prognosis.

The present invention encompasses a method of determining the status of an AML patient by obtaining a biological sample from the patient; and measuring the expression levels in the sample of genes selected from those encoding mRNA: i) corresponding to SEQ ID NOs: 1 or 5-22; or ii) recognized by the probe sets selected from psid numbers corresponding to SEQ ID NOs: 1 or 5-22 as depicted in Table 7 where the gene expression levels above or below pre-determined cut-off levels are indicative of the AML status.

The present invention encompasses a method of determining treatment protocol for an acute myeloid leukemia (AML) patient by obtaining a biological sample from the patient; and measuring the expression levels in the sample of genes selected from those encoding mRNA: i) corresponding to SEQ ID NOs: 1 or 5-22; or ii) recognized by the probe sets selected from psid numbers corresponding to SEQ ID NOs: 1 or 5-22 as depicted in Table 7 where the gene expression levels above or below pre-determined cut-off levels are sufficiently indicative of likelihood that the patient will respond to treatment to enable a physician to determine the degree and type of therapy recommend.

The present invention encompasses a method of treating an acute myeloid leukemia (AML) patient by obtaining a biological sample from the patient; and measuring the expression levels in the sample of genes selected from those encoding mRNA: i) corresponding to SEQ ID NOs: 1 or 5-22; or ii) recognized by the probe sets selected from psid numbers corresponding to SEQ ID NOs: 1 or 5-22 as depicted in Table 7 where the gene expression levels above or below pre-determined cut-off levels are indicate a likelihood that the patient will respond to treatment and; treating the patient with adjuvant therapy if they are likely to respond.

The present invention encompasses a method of generating an acute myeloid leukemia (AML) patient prognostic patient report by obtaining a biological sample from the patient; measuring gene expression of the sample; applying a Relapse Hazard Score to the results of step b.; and using the results obtained in step c. to generate the report.

The present invention encompasses a composition comprising at least one probe set selected from SEQ ID NOs: 1-22; or the psid numbers corresponding to SEQ ID NOs: 1 and 5-22 as depicted in Table 7.

The present invention encompasses a kit for conducting an assay to determine acute myeloid leukemia prognosis in a biological sample comprising: materials for detecting isolated nucleic acid sequences, their complements, or portions thereof of a combination of genes selected from those encoding mRNA: i) corresponding to SEQ ID NOs: 1 or 5-22; or ii) recognized by the probe sets selected from psid numbers corresponding to SEQ ID NOs: 1 or 5-22 as depicted in Table 7.

The present invention encompasses articles for assessing acute myeloid leukemia prognosis in a biological sample containing: materials for detecting isolated nucleic acid sequences, their complements, or portions thereof of a combination of genes selected from those encoding mRNA: i) corresponding to SEQ ID NOs: 1 or 5-22; or ii) recognized by the probe sets selected from the group consisting of psid numbers corresponding to SEQ ID NOs: 1 or 5-22 as depicted in Table 7.

The present invention encompasses a microarray or gene chip for performing the method of claim 1, 2, 3, or 4.

The microarray of claim 35 comprising isolated nucleic acid sequences, their complements, or portions thereof of a combination of genes selected from those encoding mRNA: i) corresponding to SEQ ID NOs: 1 or 5-22; or ii) recognized by the probe sets selected from the group consisting of psid numbers corresponding to SEQ ID NOs: 1 or 5-22 as depicted in Table 7 where the combination is sufficient to characterize acute myeloid leukemia status or prognosis in a biological sample.

The present invention encompasses a diagnostic/prognostic portfolio comprising isolated nucleic acid sequences, their complements, or portions thereof of a combination of genes selected from those encoding mRNA: i) corresponding to SEQ ID NOs: 1 or 5-22; or ii) recognized by the probe sets selected from the group consisting of psid numbers corresponding to SEQ ID NOs: 1 or 5-22 as depicted in Table 7 where the combination is sufficient to characterize acute myeloid leukemia status or prognosis in a biological sample.

### BRIEF DESCRIPTON OF DRAWINGS

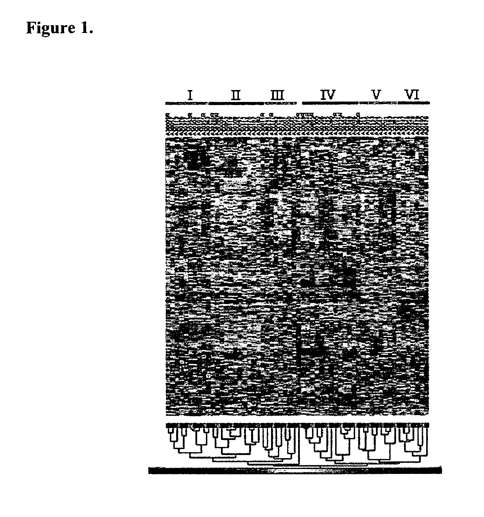

Figure 1. Unsupervised clustering of relapsed and refractory AML patients. The dendogram shows the unsupervised k-means clustering of 58 relapsed or refractory AML patients, where each column represents a patient and each row represents a gene. The expression ratio for each gene was calculated by dividing the expression level of that gene in a patient by the mean of all other patients. The color bar indicates the fold-change (Iog₂). Red is upregulated, blue is down-regulated. White indicates no change. The presence of 6 main clusters is shown.

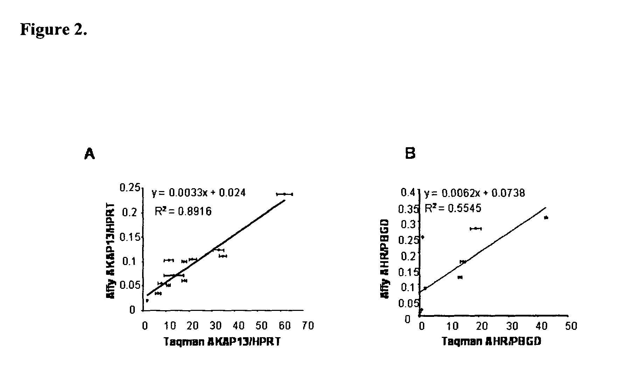

Figure 2. Real-time RT-PCR of 2 genes. AHR and AKAP13 were measured by real-time RT-PCR. The HPRT or PBGD control genes were used to normalize gene expression values. Error bars are standard deviations. The resulting values were plotted against the corresponding microarray data and linear regression analysis was performed.

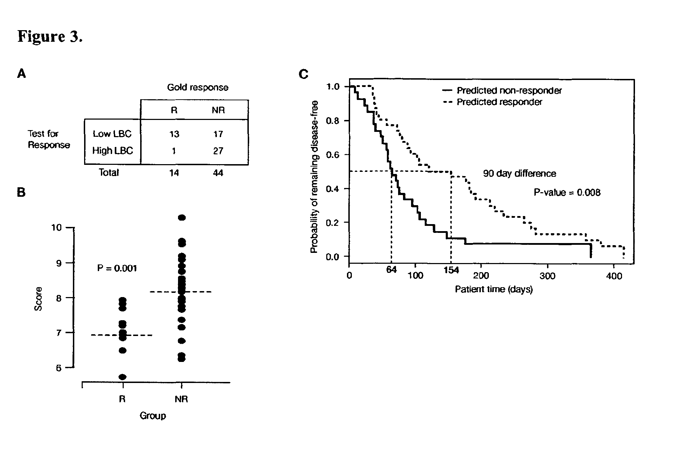

Figure 3 depicts the predictive value of the AKAP13 gene. Panel A shows a 2x2 table generated from a LOOCV performed using AKAP13 expression as a classifier on the responders (R) and non-responders (NR). Panel B shows the AKAP 13 expression values for the same 58 patients. The P value indicates a significant difference in the gene expression between the mean values of each response group. Panel C shows the Kaplan-Meier curves generated from patients classified by the AKAP13 gene as being responders and non-responders.

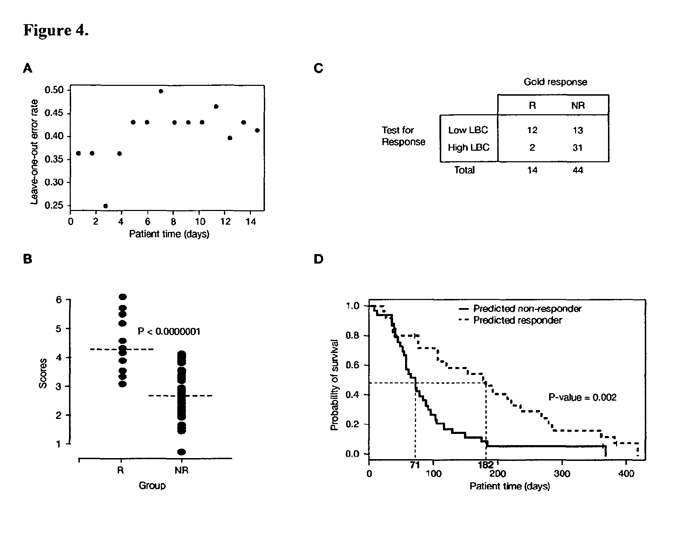

Figure 4 provides identification of a minimal set of predictive markers. In Panel A, a LOOCV was performed using a sensitivity of 100%. Independent classifiers were tested that contained from 1 to 19 genes. The resulting error rate is plotted. Panel B shows a 2x2 table generated from a LOOCV performed using the 3-gene signature as a classifier on the responders (R) and non-responders (NR). Panel C shows the scores generated from the 3-gene classifier. The P value indicates a significant difference in the gene expression between the response groups. Panel D is the Kaplan-Meier curves generated from patients classified by the 3-gene signature as being responders and non-responders. Median survival times are also indicated.

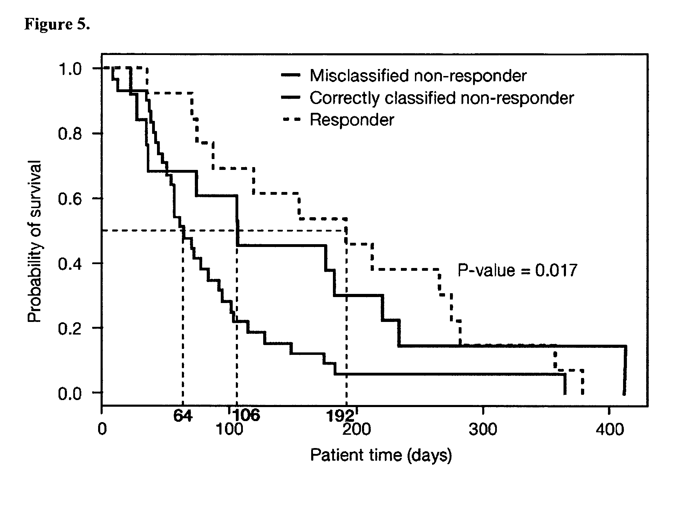

Figure 5. A Kaplan-Meier analysis was performed on patients classified by the 3-gene signature as being predicted responders and non-responders. The survival curve of patients who were clinically defined as non-responders but classified as responders using the 3-gene signature is shown. Median survival times are also indicated.

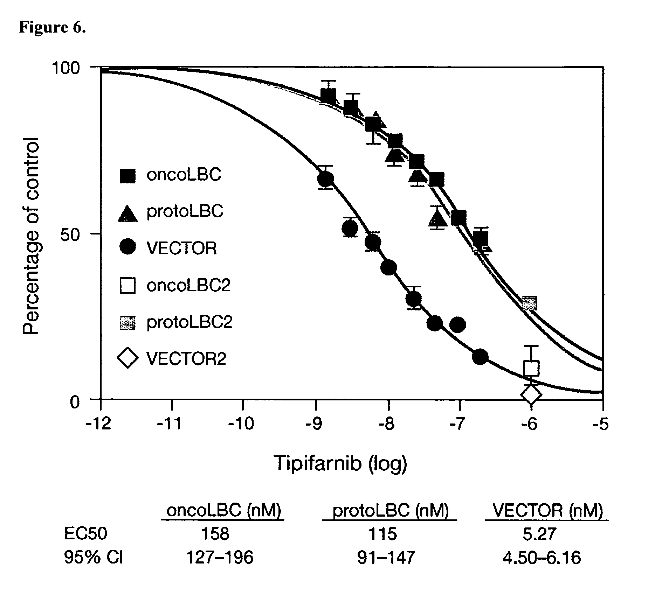

Figure 6 depicts over-expression of AKAP13 in an AML cell line. Cell counts were normalized to cultures with no drug (indicated at -12 log units) to give a percentage of control. Error bars indicate standard errors of the mean. Open data points indicate results from a second experiment exploring higher concentrations of drug.

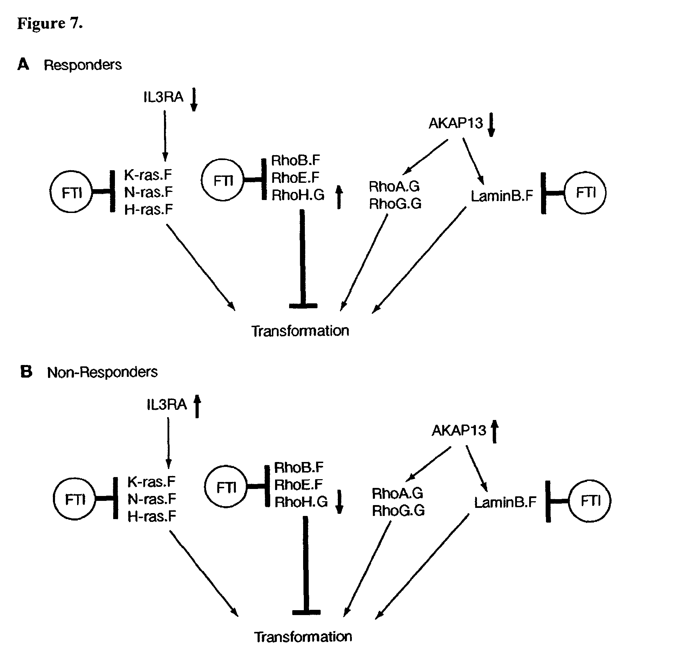

Figure 7 provides a model of FTI action in relapsed or refractory AML. A. In responders the IL3RA and AKAP13 genes are lowly expressed allowing for down-regulation of the ras, and RhoA, and lamin B pathways, respectively. Up-regulation of RhoH leads to increased inhibition of cellular transformation pathways. Together this allows for greater efficacy in FTI antitumorigenicity. B. The opposite expression profile is seen in non-responders allowing for the expression of compensatory pathways.

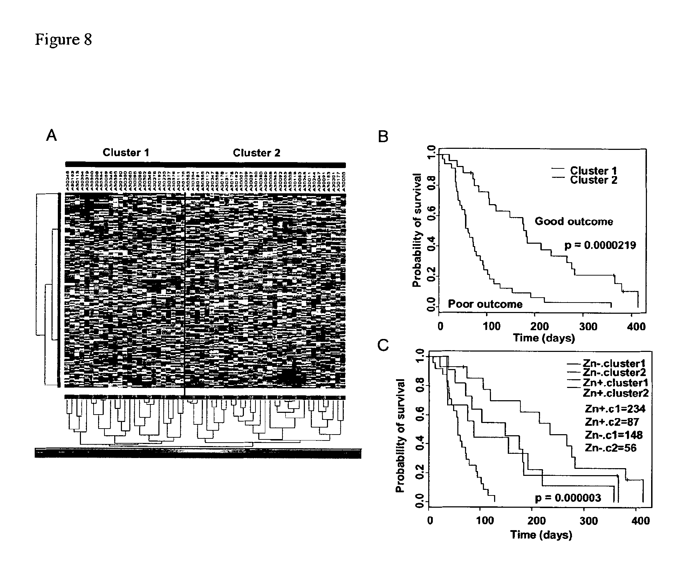

Figure 8. The Zarnestra predictive gene signature has superior utility to an independent prognostic gene signature. In panel A columns represent AML samples from relapsed or refractory patients and rows represent 167 probe sets that correspond to 103 of the 133 prognostic genes identified by Bullinger et al. ¹, ordered according to hierarchical clustering. Panel B shows Kaplan-Meier survival estimates of the cluster-defined groups of patients. In panel C the 3-gene classifier has been used to identify responders of tipifarnib in the good and poor prognostic groups defined by the Bullinger signature. Kaplan-Meier survival curves are shown for patients identified as being responders to tipifarnib in the good (Zn+.cluster1) and poor (Zn+.cluster2) prognostic groups. The median survival times for each group are indicated.

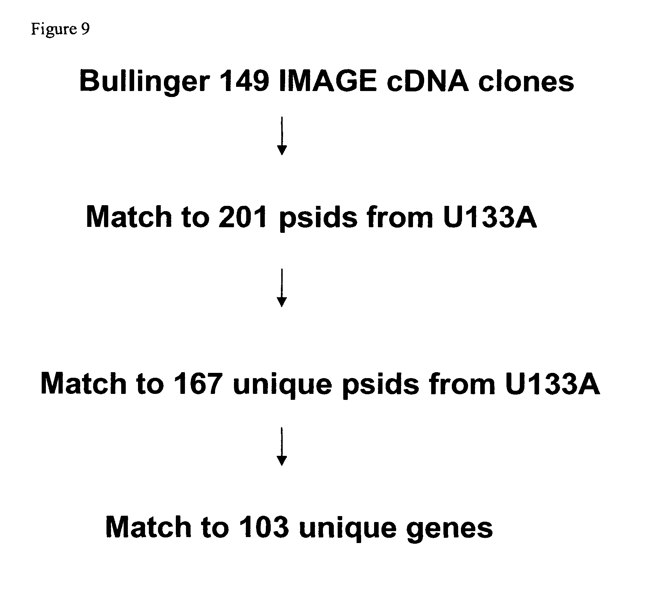

Figure 9 is a flow chart depicting how the genes from Bullinger et al. (2004) were matched to 167 probe sets (103 unique genes) on the Affymetrix U133A chip.

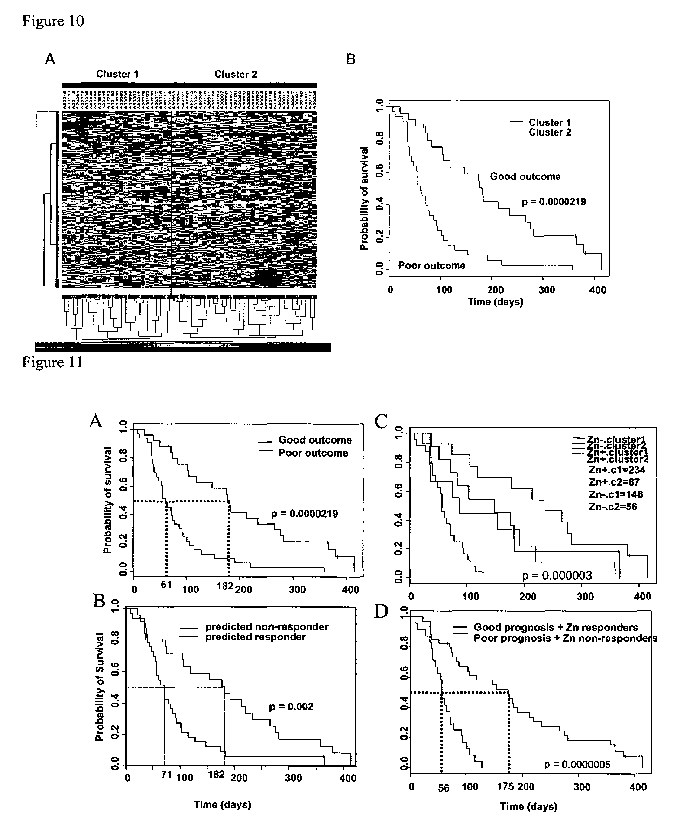

Figure 10 shows the utility of the 167 probe set signature in relapsed or refractory AML patients. In panel A columns represent AML samples from relapsed or refractory patients and rows represent 167 probe sets that correspond to 103 of the 133 prognostic genes identified by Bullinger et al. (2004), ordered according to hierarchical clustering. Panel B shows Kaplan-Meier survival estimates of the cluster-defined groups of patients.

Figure 11 provides comparisons of prognostic and Zarnestra predictive gene signatures. Panel A shows the Kaplan-Meier survival curves for the good and poor prognostic clusters as defined by the subset of 103 Bullinger et al. (2004) genes. Panel B shows the Kaplan-Meier survival curves for the good and poor prognostic clusters as defined by the 3-gene signature that predicts response to Zarnestra. Panel C shows the Kaplan-Meier survival curves for the good and poor prognostic clusters from Panel A further stratified by the 3-gene Zarnestra signature. Panel D shows the Kaplan-Meier survival curves for patients who are predicted to have a poor prognosis and not respond to Zarnestra versus the remainder of patients.

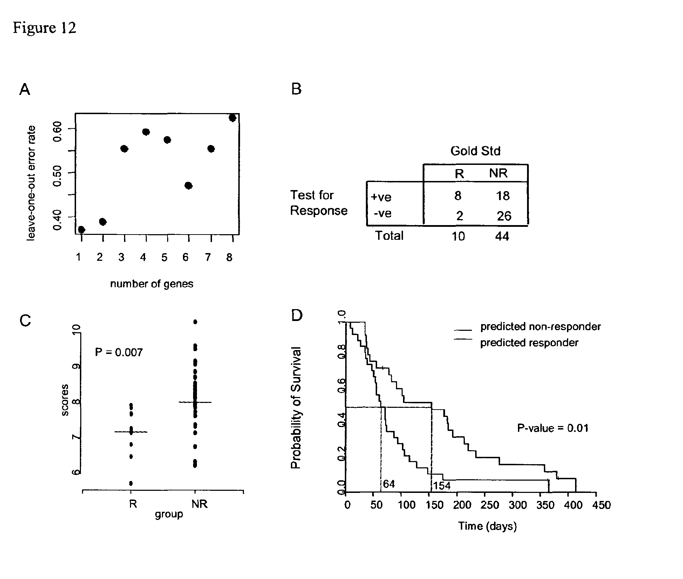

Figure 12 Identification of a minimal set of predictive markers. a) A LOOCV was performed selecting for genes with a sensitivity of 100%, specificity of 40% and fold-change > 2. Independent classifiers were tested that contained from 1 to 8 genes ranked by the AUC . The resulting error rate is plotted. b) A 2x2 table generated from a LOOCV performed using AKAP13 as a classifier on the responders (R) and non-responders (NR). c) The gene-expression values of AKAP13. The P value indicates a significant difference in the gene expression between the response groups. d) The Kaplan-Meier curves generated from patients classified by AKAP13 as being responders and non-responders. Median survival times are also indicated.

### DETAILED DESCRIPTION OF THE INVENTION

A subset of genes previously described to have prognostic value in newly diagnosed AML is shown here to have utility in relapsed and refractory AML patients treated with a molecularly targeted therapy (Zarnestra). Currently there is no method for predicting response to farnesyl transferase inhibitors (such as Zarnestra). Also, current methods for understanding the prognosis of patients with AML is limited to histological subtype and karyotyping, both of which are not ideal markers for determining clinical outcome. The current signatures expand upon these traditional technologies by providing better stratification of prognostic high risk and low risk patients.

US Patent Application Serial No. 10/883,436 demonstrates that a 3-gene classifier (including AHR, AKAP13 and MINA53) predicts relapsed, refractory AML patient response to tipifarnib (Zarnestra®, R115777) with the lowest error rate. This was also seen when a leave-five-out cross validation was performed. When more genes were added the error rate increased indicating that additional genes introduced noise to the classifier. For the 3-gene classifier the LOOCV demonstrated a sensitivity of 86% and specificity of 70% with an overall diagnostic accuracy of 74%. Kaplan-Meier analysis again showed a significant difference in survival between the predicted responder group and the non-responder group. Moreover, comparing the incorrectly classified non-responders to the correctly classified non-responders, the misclassified non-responders showed a better overall survival.

Zarnestra® is an orally available non-peptidomimetic competitive farnesyl transferase inhibitor (FTI) that has been shown to inhibit the proliferation of a variety of human tumor cell lines both in vitro and in vivo. End et al. (2001); and Cox et al. (2002). A phase I clinical trial of tipifarnib demonstrated a 32% response rate in patients with refractory or relapsed acute myeloid leukemia. Karp et al. (2001). Activity has also been seen in early clinical trials for myelodysplastic syndrome (MDS) (Kurzrock et al. (2004)), multiple myeloma (MM) (Alsina et al. (2003)) and chronic myeloid leukemia (CML). Cortes et al. (2003). Complete remission was defined as less than 5% bone marrow blasts with a neutrophil count greater than 1000/µL, a platelet count less than 100,000/µL, and no extramedullary disease. While it is clear that FTIs function by inhibiting protein farnesylation, it is still not known what genes are implicated in the antitumor effects of tipifarnib in hematopoietic malignancies. Microarray technology allows for the measurement of the steady-state mRNA level of thousands of genes simultaneously, thereby representing a powerful tool for identifying genes and gene pathways that correlate with FTI action. Global gene expression monitoring was therefore employed in a phase 2 clinical study of tipifarnib in relapsed and refractory AML to identify genes that predict response to this FTI in hematologic malignancies.

The present invention encompasses a method of assessing prognosis (survival/outcome) in an AML patient by obtaining a biological sample from the patient; and measuring the expression levels in the sample of genes selected from those encoding mRNA: i) corresponding to SEQ ID NOs: 1 or 5-22; or ii) recognized by the probe sets selected from psid numbers corresponding to SEQ ID NOs: 1 or 5-22 as depicted in Table 7 where the gene expression levels above or below pre-determined cut-off levels are indicative of AML prognosis.

The present invention encompasses a method of determining the status of an AML patient by obtaining a biological sample from the patient; and measuring the expression levels in the sample of genes selected from those encoding mRNA: i) corresponding to SEQ ID NOs: 1 or 5-22; or ii) recognized by the probe sets selected from psid numbers corresponding to SEQ ID NOs: 1 or 5-22 as depicted in Table 7 where the gene expression levels above or below pre-determined cut-off levels are indicative of the AML status.

The present invention encompasses a method of determining treatment protocol for an acute myeloid leukemia (AML) patient by obtaining a biological sample from the patient; and measuring the expression levels in the sample of genes selected from those encoding mRNA: i) corresponding to SEQ ID NOs: 1 or 5-22; or ii) recognized by the probe sets selected from psid numbers corresponding to SEQ ID NOs: 1 or 5-22 as depicted in Table 7 where the gene expression levels above or below pre-determined cut-off levels are sufficiently indicative of likelihood that the patient will respond to treatment to enable a physician to determine the degree and type of therapy recommend.

The present invention encompasses a method of treating an acute myeloid leukemia (AML) patient by obtaining a biological sample from the patient; and measuring the expression levels in the sample of genes selected from those encoding mRNA: i) corresponding to SEQ ID NOs: 1 or 5-22; or ii) recognized by the probe sets selected from psid numbers corresponding to SEQ ID NOs: 1 or 5-22 as depicted in Table 7 where the gene expression levels above or below pre-determined cut-off levels are indicate a likelihood that the patient will respond to treatment and; treating the patient with adjuvant therapy if they are likely to respond.

In the above methods, the patient can have relapsed or refractory AML, non-relapsed or non-refractory AML, treated or untreated AML. In one embodiment, the patient has non-relapsed or non-refractory AML and the method allows a determination of whether the patient will respond to treatment with an FTI.

In the above methods, the SEQ ID NOs. are preferably 1, 5 and 6 or, 1. The methods may further include measuring the expression level of at least one gene constitutively expressed in the sample.

In the above methods, the comparison of expression patterns can be conducted with pattern recognition methods, preferably using a Cox proportional hazards analysis. The pre-determined cut-off levels are preferably at least 1.5-fold over- or under- expression in the sample relative to benign cells or normal tissue. The pre-determined cut-off levels are preferably at least a statistically significant p-value over-expression in the sample having metastatic cells relative to benign cells or normal tissue. Preferably, the p-value is less than 0.05.

In the above methods, gene expression can be measured on a microarray or gene chip including, without limitation, a cDNA array or an oligonucleotide array. The microarray or gene chip can further contain one or more internal control reagents. Gene expression can also be determined by nucleic acid amplification conducted by polymerase chain reaction (PCR) of RNA extracted from the sample. In one embodiment, PCR is reverse transcription polymerase chain reaction (RT-PCR) and can further contain one or more internal control reagents. Gene expression can also be detected by measuring or detecting a protein encoded by the gene such as by an antibody specific to the protein. Gene expression can also be detected by measuring a characteristic of the gene such as DNA amplification, methylation, mutation and allelic variation.

The present invention encompasses a method of generating an acute myeloid leukemia (AML) patient prognostic patient report by obtaining a biological sample from the patient; measuring gene expression of the sample; applying a Relapse Hazard Score to the results of step b.; and using the results obtained in step c. to generate the report. The report can also contain an assessment of patient outcome and/or probability of risk relative to the patient population. The present invention further encompasses reports generated by this method.

The patient for whom the report is generated, can have relapsed or refractory AML, non-relapsed or non-refractory AML, treated or untreated AML. In one embodiment, the patient has non-relapsed or non-refractory AML and the method allows a determination of whether the patient will respond to treatment with an FTI.

The present invention encompasses a composition comprising at least one probe set selected from SEQ ID NOs: 1-22; or the psid numbers corresponding to SEQ ID NOs: 1 and 5-22 as depicted in Table 7.

The present invention encompasses a kit for conducting an assay to determine acute myeloid leukemia prognosis in a biological sample comprising: materials for detecting isolated nucleic acid sequences, their complements, or portions thereof of a combination of genes selected from those encoding mRNA: i) corresponding to SEQ ID NOs: 1 or 5-22; or ii) recognized by the probe sets selected from psid numbers corresponding to SEQ ID NOs: 1 or 5-22 as depicted in Table 7. Preferably, the SEQ ID NOs. are 1, 5 and 6, or 1. The kit can further contain any addition including, without limitation, instructions, reagents for conducting a microarray analysis and a medium through which said nucleic acid sequences, their complements, or portions thereof are assayed.

The present invention encompasses articles for assessing acute myeloid leukemia prognosis in a biological sample containing: materials for detecting isolated nucleic acid sequences, their complements, or portions thereof of a combination of genes selected from those encoding mRNA: i) corresponding to SEQ ID NOs: 1 or 5-22; or ii) recognized by the probe sets selected from the group consisting of psid numbers corresponding to SEQ ID NOs: 1 or 5-22 as depicted in Table 7. Preferably, the SEQ ID NOs. are 1, 5 and 6, or 1. The articles can further contain any addition including, without limitation, instructions, reagents for conducting a microarray analysis and a medium through which said nucleic acid sequences, their complements, or portions thereof are assayed.

The present invention encompasses a microarray or gene chip for performing the methods encompassed by the invention. The microarrays contain isolated nucleic acid sequences, their complements, or portions thereof of a combination of genes selected from those encoding mRNA: i) corresponding to SEQ ID NOs: 1 or 5-22; or ii) recognized by the probe sets selected from the group consisting of psid numbers corresponding to SEQ ID NOs: 1 or 5-22 as depicted in Table 7 where the combination is sufficient to characterize acute myeloid leukemia status or prognosis in a biological sample. Preferably, the microarrays provide a measurement or characterization is at least 1.5-fold over- or under-expression. Preferably, the microarrays provide a measurement that is statistically significant p-value over- or under-expression. Preferably, the p-value is less than 0.05. The microarray can be any known in the art, including, without limitation, a cDNA array or an oligonucleotide array and can further contain other nucleotide sequences and one or more internal control reagents.

The present invention encompasses a diagnostic/prognostic portfolio comprising isolated nucleic acid sequences, their complements, or portions thereof of a combination of genes selected from those encoding mRNA: i) corresponding to SEQ ID NOs: 1 or 5-22; or ii) recognized by the probe sets selected from the group consisting of psid numbers corresponding to SEQ ID NOs: 1 or 5-22 as depicted in Table 7 where the combination is sufficient to characterize acute myeloid leukemia status or prognosis in a biological sample. The portfolio preferably provides a measurement or characterization of at least 1.5-fold over- or under-expression. The portfolio preferably provides a measurement that is statistically significant p-value over- or under-expression. Preferably, the p-value is less than 0.05.

The mere presence of nucleic acid sequences having the potential to express proteins or peptides ("genes") within the genome is not determinative of whether a protein or peptide is expressed in a given cell. Whether or not a given gene capable of expressing proteins or peptides or transcribing RNA does so and to what extent such expression or transcription occurs, if at all, is determined by a variety of complex factors. Nevertheless, assaying gene expression can provide useful information about the cellular response to a given stimulus such as the introduction of a drug or other therapeutic agent. Relative indications of the degree to which genes are active or inactive can be found in such gene expression profiles. In some instances, the presence of a molecular marker can, by itself or with the use of gene expression information, provide useful information about treatment efficacy too. The gene expression profiles and molecular markers of this invention are used to identify and treat AML patients.

Cancers, including hematological malignancies, typically arise from mutations in a variety of genes. The same type of cancer may arise as a result of, or coincident with, one or more mutations that differ from those of another patient having the same type of cancer. The fact that there are often multiple molecular bases underlying the same cancers is consistent with the observation that some therapies that affect one patient do not necessarily equally affect another patient with the same type of cancer. Further, from a diagnostic point of view, the presence of particular mutations such as translocations, deletions, or SNPs can have powerful implications. In some instances, such molecular markers are themselves useful indicators for diagnosis, prognosis, or treatment response determinations. This is particularly true where the molecular mutations can be associated with response to particular treatments.

The LBC oncogene or AKAP13 is a chimera derived from the fusion of an LBC proto-oncogene (SEQ ID NO: 1) on chromosome 15q with an unrelated sequence originating in chromosome 7q. The truncation of the proto-oncogene at the C-terminus of the sequence results in the gene gaining transforming ability. This truncation could also arise from mechanisms other than a translocation. For example, aberrant splicing could result in RNA transcripts with C-terminus truncations. The gene has a number of expression products including mRNA and a protein. While the precise manner in which the LBC oncogene functions is not completely understood, it is clear that it can be present in a range of tissues including skeletal muscle, heart, lung, prostate, ovary, small intestine, and hematopoietic cells. Treatment of cancers originating in tissues where the oncogene could be manifested (but is not) is within the scope of this invention.

There is great flexibility available in formatting the assays of this invention because the gene is the product of a truncation and because it produces recognizable expression products. Not only can the absence of the gene or its products be used, but so too can detection of the modulated expression of this gene. Thus, a gene expression profile can include this gene. Preferably, the absence or modulation of the gene is used as an indicator of prognosis and status of previously treated patients and untreated patients.

Any suitable method of detection may be used to identify the LBC oncogene as a molecular marker. Methods useful for detecting the presence of the LBC oncogene include any method for detecting known mutant genes or sequences including, without limitation, the single strand conformation polymorphism technique, chemical and thermal denaturing gradient analysis, chemical and enzymatic cleavage methods, mass spectrometry, RFLP and allele specific amplification, ratiometric PCR, bead-based and microarray-based systems as well as in situ hybridization, heteroduplex analysis, and microarray analysis, ELISA, Western, fluorescence activated cell sorting (FACS), antibody-based techniques, methylation-based PCR, and SNP detection.

The most preferred method for detecting the presence or absence of the LBC oncogene is via PCR. In this method, cells are first obtained from the patient according to routine sample preparation methods. A peripheral blood sample or a bone marrow sample is preferable. RNA is then extracted according to well-accepted procedures and amplified as follows. Target sequences are amplified using, e.g., 250 nM of primers and 250 nM of TaqMan® probe in ABI TaqMan® buffer. Thermal cycling is conducted at 50°C for 2 minutes, 95°C for 10 minutes, followed by 50 cycles of 95°C for 15 minutes and 62°C for 1 minute. Examples of the primers and probe are shown in Table 1.

This technique measures the amount of LBC transcript present in the sample. Measured quantities can be normalized by running similar RT-PCR experiments in which the same samples are used to amplify endogenous control genes such as HPRT.

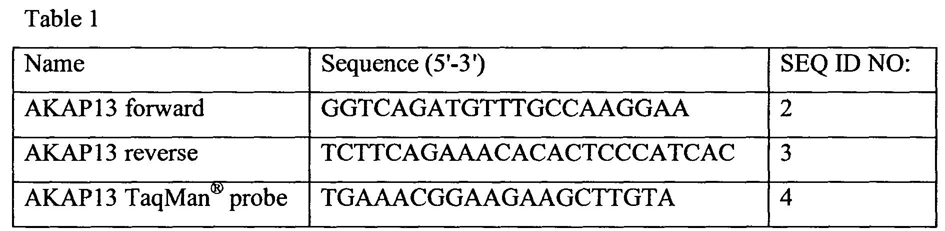

Another method for determining the presence or absence of the LBC oncogene is to assay the length of the RNA transcript. Since the LBC oncogene has a 3' translocation, the transcript length will be shorter than the LBC proto-oncogene transcript. To determine the transcript size a forward primer homologous to both the proto- and onco LBC transcripts is used (e.g. AKAP13 forward primer from Table 1) in conjunction with a reverse primer homologous to the universal polyA tail of RNA transcripts. Preferably initial cDNA synthesis will incorporate an additional unique sequence tag 3' of the polyA sequence to confer additional specificity for the PCR reaction.

If the genomic translocation is being measured then any method known in the art can be used including isolating genomic DNA using standard techniques and PCR primers used that are specific for the LBC oncogene. For example, the forward primer homologous for both the onco- and proto-LBC genes could be used in conjunction with the polyA sequence as described above. Alternatively, the reverse primer could be homologous to the 3' translocated sequence. The readout would be the size of the amplicon where presence of the oncogene is consistent with a shorter product than the proto-oncogene, or presence or absence of an oncoLBC-specific amplicon.

Assays for the LBC oncogene status of the cell also can determine normal/abnormal tissue distribution for diagnostic purposes using techniques such as immunohistochemical analysis (IHC). Any method known in the art can be used, for example, the antibodies to LBC protein may be used in conjunction with both fresh-frozen and formalin-fixed, paraffin-embedded tissue blocks prepared for study by IHC. Each tissue block may consist of 50 mg of residual "pulverized" tumor.

Briefly, frozen-sections may be prepared by rehydrating 50 ng of frozen pulverized tumor at room temperature in phosphate buffered saline (PBS) in small plastic capsules; pelleting the particles by centrifugation; resuspending them in a viscous embedding medium (OCT); inverting the capsule and pelleting again by centrifugation; snap-freezing in -70°C isopentane; cutting the plastic capsule and removing the frozen cylinder of tissue; securing the tissue cylinder on a cryostat microtome chuck; and cutting 25-50 serial sections containing intact tumor cells.

Permanent-sections may be prepared by a similar method involving rehydration of the 50 mg sample in a plastic microfuge tube; pelleting; resuspending in 10% formalin for 4 hr fixation; washing/pelleting; resuspending in warm 2.5% agar; pelleting; cooling in ice water to harden the agar; removing the tissue/agar block from the tube; infiltrating and embedding the block in paraffin; and cutting up to 50 serial permanent sections.

For the IHC assay, the sections are overlaid with a blocking solution containing: 3% bovine serum albumin (BSA) in PBS or other blocking reagents. The blocking reagents include non-specific serum or dry milk. Blocking is allowed to proceed for 1 hr at room temperature. Anti-LBC protein antibody is diluted with PBS buffer containing 3% BSA, 0.1 % TritonX™-100 and t-octylphenoxypolyethoxyethanol, at a ratio of 1:100. The sample sections are generally overlaid with the antibody solution for 16 hr at 4°C. The duration and temperature conditions may be varied according to the antibody selected and the material tested. The optimal conditions are determined empirically. The antibody treated sections are then washed three times in PBS for 15 min. each to remove unbound antibody and then overlaid with PBS containing 3% BSA and a secondary antibody at a dilution of 1:2000. The secondary antibodies may be coupled to a chromogenic enzyme such as: horseradish peroxidase, alkaline phosphatase, fluorescein iso-thiocyanate, or other suitable enzymes. Alternatively, the secondary antibody may be conjugated to biotin and used in conjunction with chromophore-labeled avidin.

Another exemplary method for detecting the presence of a gene is via in situ hybridization. Generally, in situ hybridization comprises the following major steps: (1) fixation of tissue or biological structure to be analyzed; (2) prehybridization treatment of the biological structure to increase accessibility of target DNA, and to reduce nonspecific binding; (3) hybridization of the mixture of nucleic acids to the nucleic acid in the biological structure or tissue; (4) post-hybridization washes to remove nucleic acid fragments not bound in the hybridization and (5) detection of the hybridized nucleic acid fragments. The reagent used in each of these steps and the conditions for use vary depending on the particular application.

In this case, a hybridization solution comprising at least one detectable nucleic acid probe capable of hybridizing to a gene (at its chromosomal locus) is contacted with the cell under hybridization conditions. Any hybridization is then detected and compared to a predetermined hybridization pattern from normal or control cells. Preferably, the probes are alpha-centromeric probes. Such probes can be made commercially available from a number of sources (e.g., from Visys Inc., Downers Grove, IL). In a preferred embodiment, the hybridization solution contains a multiplicity of probes, specific for an area on the chromosome that corresponds to the translocation of the sequences that make up the chimera (e.g., 15q24-25).

Hybridization protocols suitable for use with the methods of the invention are described, e.g., in Albertson (1984); Pinkel (1988); EP No. 430,402; and Methods in Molecular Biology, Vol. 33: In Situ Hybridization Protocols, Choo, ed., Humana Press, Totowa, NJ (1994), etc. In one particularly preferred embodiment, the hybridization protocol of Pinkel et al. (1998) or of Kallioniemi (1992) is used. Methods of optimizing hybridization conditions are well known (see, e.g., Tijssen (1993) Laboratory Techniques in Biochemistry and Molecular Biology, Vol. 24: Hybridization With Nucleic Acid Probes, Elsevier, NY).

In a preferred embodiment, background signal is reduced by the use of a detergent (e.g., C-TAB) or a blocking reagent (e.g., sperm DNA, cot-1 DNA, etc.) during the hybridization to reduce non-specific binding. Preferably, the hybridization is performed in the presence of about 0.1 to about 0.5 mg/ml DNA (e.g., cot-1 DNA).

The probes may be prepared by any method known in the art, including synthetically or grown in a biological host. Synthetic methods include but are not limited to oligonucleotide synthesis, riboprobes, and PCR.

The probe may be labeled with a detectable marker by any method known in the art. Methods for labeling probes include random priming, end labeling, PCR and nick translation. Enzymatic labeling is conducted in the presence of nucleic acid polymerase, three unlabeled nucleotides, and a fourth nucleotide which is either directly labeled, contains a linker arm for attaching a label, or is attached to a hapten or other molecule to which a labeled binding molecule may bind. Suitable direct labels include radioactive labels such as ³²P, ³H, and ³⁵S and non-radioactive labels such as fluorescent markers, such as fluorescein, Texas Red, AMCA blue, lucifer yellow, rhodamine, and the like; cyanin dyes which are detectable with visible light; enzymes and the like. Labels may also be incorporated chemically into DNA probes by bisulfite-mediated transamination or directly during oligonucleotide synthesis.

Fluorescent markers can readily be attached to nucleotides with activated linker arms incorporated into the probe. Probes may be indirectly labeled by the methods disclosed above, by incorporating a nucleotide covalently linked to a hapten or other molecule such as biotin or digoxygenin, and performing a sandwich hybridization with a labeled antibody directed to that hapten or other molecule, or in the case of biotin, with avidin conjugated to a detectable label. Antibodies and avidin may be conjugated with a fluorescent marker, or with an enzymatic marker such as alkaline phosphatase or horseradish peroxidase to render them detectable. Conjugated avidin and antibodies are commercially available from companies such as Vector Laboratories (Burlingame, CA) and Boehringer Mannheim (Indianapolis, IN).

The enzyme can be detected through a colorimetric reaction by providing a substrate for the enzyme. In the presence of various substrates, different colors are produced by the reaction, and these colors can be visualized to separately detect multiple probes. Any substrate known in the art may be used. Preferred substrates for alkaline phosphatase include 5-bromo-4-chloro-3-indolylphosphate (BCIP) and nitro blue tetrazolium (NBT). The preferred substrate for horseradish peroxidase is diaminobenzoate (DAB).

Fluorescently labeled probes suitable for use in the in situ hybridization methods of the invention are preferably in the range of 150-500 nucleotides long. Probes may be DNA or RNA, preferably DNA.

Hybridization of the detectable probes to the cells is conducted with a probe concentration of 0.1-500 ng/µl, preferably 5-250 ng/µl. The hybridization mixture will preferably contain a denaturing agent such as formamide. In general, hybridization is carried out at 25°C-45°C, more preferably at 32°C-40°C, and most preferably at 37°C-38°C. The time required for hybridization is about 0.25-96 hours, more preferably 1-72 hours, and most preferably for 4-24 hours. Hybridization time will vary based on probe concentration and hybridization solution content which may contain accelerators such as hnRNP binding protein, trialkyl ammonium salts, lactams, and the like. Slides are then washed with solutions containing a denaturing agent, such as formamide, and decreasing concentrations of sodium chloride or in any solution that removes unbound and mismatched probe.

The temperature and concentration of salt will vary depending on the stringency of hybridization desired. For example, high stringency washes may be carried out at 42°C-68°C, while intermediate stringency may be in the range of 37°C-55°C, and low stringency may be in the range of 30°C-37°C. Salt concentration for a high stringency wash may be 0.5-1 times SSC (0.15M NaCl, 0.015M Na citrate), while medium stringency may be 1-4 times, and low stringency may be 2-6 times SSC.

The detection incubation steps, if required, should preferably be carried out in a moist chamber at 23°C-42°C, more preferably at 25°C-38°C and most preferably at 37-38°C. Labeled reagents should preferably be diluted in a solution containing a blocking reagent, such as BSA, non-fat dry milk, or the like. Dilutions may range from 1:10-1:10,000, more preferably 1:50-1:5,000, and most preferably at 1:100-1:1,000. The slides or other solid support should be washed between each incubation step to remove excess reagent.

Slides may then be mounted and analyzed by microscopy in the case of a visible detectable marker, or by exposure to autoradiographic film in the case of a radioactive marker. In the case of a fluorescent marker, slides are preferably mounted in a solution that contains an antifade reagent, and analyzed using a fluorescence microscope. Multiple nuclei may be examined for increased accuracy of detection.

Additionally, assays for the expression product of the LBC oncogene can also be used to determine whether the LBC oncogene mutation has occurred. Most preferably, such assays are immunoassays. Immunoassays, in their most simple and direct sense, are binding assays. Certain preferred immunoassays are the various types of enzyme linked immunosorbent assays (ELISAs) and radioimmunoassays (RIA) known in the art. IHC detection using tissue sections is also particularly useful as are in situ hybridization and enzyme immunoassay.

In one exemplary ELISA, protein-specific antibodies are immobilized onto a selected surface exhibiting protein affinity, such as a well in a polystyrene microtiter plate. Then, a test composition containing the desired antigen, such as a clinical sample, is added to the wells. After binding and washing to remove non-specifically bound immune complexes, the bound antigen may be detected. Detection is generally achieved by the addition of another antibody, specific for the desired antigen, that is linked to a detectable label. This type of ELISA is a simple "sandwich ELISA." Detection may also be achieved by the addition of a second antibody specific for the desired antigen, followed by the addition of a third antibody that has binding affinity for the second antibody, with the third antibody being linked to a detectable label.

Variations of ELISA techniques are well known. In one such variation, the samples containing the desired antigen are immobilized onto the well surface and then contacted with the antibodies of the invention. After binding and appropriate washing, the bound immune complexes are detected. Where the initial antigen specific antibodies are linked to a detectable label, the immune complexes may be detected directly. Again, the immune complexes may be detected using a second antibody that has binding affinity for the first antigen specific antibody, with the second antibody being linked to a detectable label.

In embodiments of the invention in which gene expression is detected for determining AML prognosis or status, the use of gene expression portfolios is most preferred. A portfolio of genes is a set of genes grouped so that expression information obtained about them provides the basis for making a clinically relevant judgment such as a diagnosis, prognosis, or treatment choice. In this case, gene expression portfolios can be fashioned to help make therapeutic decisions regarding AML patients.

Preferred methods for establishing gene expression profiles (including those used to arrive at the explication of the relevant biological pathways) include determining the amount of RNA that is produced by a gene that can code for a protein or peptide or transcribe RNA.

This is best accomplished by reverse transcription PCR (RT-PCR), competitive RT-PCR, real time RT-PCR, differential display RT-PCR, Northern Blot analysis and other related tests. While it is possible to conduct these techniques using individual PCR reactions, it is often desirable to amplify copy DNA (cDNA) or copy RNA (cRNA) produced from mRNA and analyze it via microarray. A number of different array configurations and production methods are known to those of skill in the art and are described in US Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637.

Microarray technology measures steady-state mRNA levels of thousands of genes simultaneously thereby presenting a powerful tool for identifying AML patient gene expression profiles. Two microarray technologies are currently in wide use. The first are cDNA arrays and the second are oligonucleotide arrays. Although differences exist in the construction of these chips, essentially all downstream data analysis and output are the same. The products of these analyses are typically measurements of the intensity of the signal received from a labeled probe used to detect a cDNA sequence from the sample that hybridizes to a nucleic acid sequence at a known location on the microarray. Typically, the signal intensity is proportional to the cDNA quantity, and thus mRNA, expressed in the sample cells. A large number of such techniques are available and useful. Preferred methods can be found in US Patents 6,271,002; 6,218,122; 6,218,114; and 6,004,755.

Analysis of the expression levels is conducted by comparing such intensities. This is best done by generating a ratio matrix of the expression intensities of genes in a test sample versus those in a control sample. For instance, the gene expression intensities from a tissue that has been treated with a drug can be compared with the expression intensities generated from the same tissue that has not been treated with the drug. A ratio of these expression intensities indicates the fold-change in gene expression between the test and control samples.

Gene expression profiles can be displayed in a number of ways. A common method is to arrange a ratio matrix into a graphical dendogram where columns indicate test samples and rows indicate genes. The data are arranged so genes that have similar expression profiles are proximal to each other. The expression ratio for each gene is visualized as a color. For example, a ratio less than one (indicating down-regulation) may appear in the blue portion of the spectrum while a ratio greater than one (indicating up-regulation) may appear as a color in the red portion of the spectrum. Commercially available computer software programs are available to display such data including "GENESPRINT" from Silicon Genetics, Inc. and "DISCOVERY" and "INFER" software from Partek, Inc.

The differentially expressed genes are either up regulated or down regulated in diseased cells, as deduced by an assessment of gene expression as described above. Up regulation and down regulation are relative terms meaning that a detectable difference (beyond the contribution of noise in the system used to measure it) is found in the amount of expression of the genes relative to some baseline. In this case, the baseline is the measured gene expression of a normal cell. The genes of interest in the diseased cells are then either up regulated or down regulated relative to the baseline level using the same measurement method. Preferably, levels of up and down regulation are distinguished based on fold changes of the intensity measurements of hybridized microarray probes. A 1.5 fold difference is preferred for making such distinctions. That is, before a gene is said to be differentially expressed in treated versus untreated diseased cells, the treated cell is found to yield at least 1.5 times more, or 1.5 times less intensity than the untreated cells. A 1.7 fold difference is more preferred and a 2 or more fold difference in gene expression measurement is most preferred.

One method of the invention involves comparing gene expression profiles for various genes to determine whether a person is likely to respond to the use of a therapeutic agent. Having established the gene expression profiles that distinguish responder from non-responder, the gene expression profiles of each are fixed in a medium such as a computer readable medium as described below. A patient sample is obtained that contains diseased cells (such as hematopoietic blast cells in the case of AML) is then obtained. Most preferably, the samples are of bone marrow and are extracted from the patient's sternum or iliac crest according to routine methods. Preferably the bone marrow aspirate is processed to enrich for leukemic blast cells using routine methods. Sample RNA is then obtained and amplified from the diseased patient cells and a gene expression profile is obtained, preferably (in the case of a large gene portfolio) via micro-array, for genes in the appropriate portfolios. The expression profiles of the samples are then compared to those previously analyzed for prognostic outcome. When a small number of genes are used in the portfolio such as when the three gene profile is used, a simple nucleic acid amplification and detection scheme is the most preferred method of measuring gene modulation. In such a case, PCR, NASBA, rolling circle, LCR, and other amplification schemes known to skilled artisans can be used with PCR being most preferred. Where the portfolios include a large number of genes or it is desirable to measure the expression of numerous other genes then it is preferred to assess the expression patterns based on intensity measurements of microarrays as described above.

In similar fashion, gene expression profile analysis can be conducted to monitor treatment response. In one aspect of this method, gene expression analysis as described above is conducted on a patient treated with any suitable treatment at various periods throughout the course of treatment. If the gene expression patterns are consistent with a positive outcome the patient's therapy is continued. If it is not, the patient's therapy is altered as with additional therapeutics, changes to the dosage, or elimination of the current treatment. Such analysis permits intervention and therapy adjustment prior to detectable clinical indicia or in the face of otherwise ambiguous clinical indicia.

With respect to the molecular markers of the invention, a number of other formats and approaches are available for diagnostic use. Methylation of genomic regions can affect gene expression levels. For example, hypermethylation of gene promoter regions can constitutively down-regulate gene expression whereas hypomethylation can lead to an increase in steady-state mRNA levels. As such, detection of methylated regions associated with genes predictive of drug response, prognosis or status can be used as an alternative method for diagnosing gene expression levels. Such methods are known to those skilled in the art. Alternatively, single nucleotide polymorphisms (SNPs) that are present in promoter regions can also affect transcriptional activity of a gene. Therefore, detection of these SNPs by methods known to those skilled in the art can also be used as a diagnostic for detecting genes that are differentially expressed in different prognostic outcomes.

Articles of this invention are representations of the gene expression profiles useful for treating, diagnosing, prognosticating, staging, and otherwise assessing diseases. Preferably they are reduced to a medium that can be automatically read such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. Clustering algorithms such as those incorporated in "DISCOVERY" and "INFER" software from Partek, Inc. mentioned above can best assist in the visualization of such data.

Additional articles according to the invention are kits for conducting the assays described above. Each such kit would preferably include instructions in human or machine readable form as well as the reagents typical for the type of assay described. These can include, for example, nucleic acid arrays (e.g. cDNA or oligonucleotide arrays), as described above, configured to discern the gene expression profiles of the invention. They can also contain reagents used to conduct nucleic acid amplification and detection including, for example, reverse transcriptase, reverse transcriptase primer, a corresponding PCR primer set, a thermostable DNA polymerase, such as Taq polymerase, and a suitable detection reagent(s), such as, without limitation, a scorpion probe, a probe for a fluorescent probe assay, a molecular beacon probe, a single dye primer or a fluorescent dye specific to double-stranded DNA, such as ethidium bromide. Kits for detecting surface antigens contain staining materials or are antibody based including components such as buffer, anti-antigenic antibody, detection enzyme and substrate such as Horse Radish Peroxidase or biotin-avidin based reagents. Kit components for detecting blast cells generally include reagents for conducting flow cytometry, blast cell adhesion assays, and other common blast cell assays.

Conventional anti-cancer agents include, without limitation, tyrosine kinase inhibitors, MEK kinase inhibitors, P13K kinase inhibitors, MAP kinase inhibitors, apoptosis modulators and combinations thereof. Exemplary drugs that are most preferred among these are the "GLEEVEC" tyrosine kinase inhibitor of Novartis, U-0126 MAP kinase inhibitor, PD-098059 MAP kinase inhibitor, SB-203580 MAP kinase inhibitor, and antisense, ribozyme, and DNAzyme, Bcl-XL, and anti-apoptotics. Examples of other useful drugs include, without limitation, the calanolides of US Patent 6,306,897; the substituted bicyclics of US Patent 6,284,764; the indolines of US Patent 6,133,305; and the antisense oligonucleotides of US Patent 6,271,210; platinum coordination compounds for example cisplatin or carboplatin, taxane compounds for example paclitaxel or docetaxel, camptothecin compounds for example irinotecan or topotecan, anti-tumor vinca alkaloids for example vinblastine, vincristine or vinorelbine, anti-tumor nucleoside derivatives for example 5-fluorouracil, gemcitabine or capecitabine, nitrogen mustard or nitrosourea alkylating agents for example cyclophosphamide, chlorambucil, carmustine or lomustine, anti-tumor anthracycline derivatives for example daunorubicin, doxorubicin or idarubicin; HER2 antibodies for example trastzumab; and anti-tumor podophyllotoxin derivatives for example etoposide or teniposide; and antiestrogen agents including estrogen receptor antagonists or selective estrogen receptor modulators preferably tamoxifen, or alternatively toremifene, droloxifene, faslodex and raloxifene, or aromatase inhibitors such as exemestane, anastrozole, letrazole and vorozole.

Anti-cancer agents can also include therapeutics directed to gene therapy or antisense therapy or RNA interference. These include, without limitation, oligonucleotides with sequences complementary to an mRNA sequence can be introduced into cells to block the translation of the mRNA, thus blocking the function of the gene encoding the mRNA. The use of oligonucleotides to block gene expression is described, for example, in, Strachan and Read, Human Molecular Genetics, 1996. These antisense molecules may be DNA, stable derivatives of DNA such as phosphorothioates or methylphosphonates, RNA, stable derivatives of RNA such as 2'-*O*-alkylRNA, or other antisense oligonucleotide mimetics. Antisense molecules may be introduced into cells by microinjection, liposome encapsulation or by expression from vectors harboring the antisense sequence.

In gene therapy, the gene of interest can be ligated into viral vectors that mediate transfer of the therapeutic DNA by infection of recipient host cells. Suitable viral vectors include retrovirus, adenovirus, adeno-associated virus, herpes virus, vaccinia virus, polio virus and the like. Alternatively, therapeutic DNA can be transferred into cells for gene therapy by non-viral techniques including receptor-mediated targeted DNA transfer using ligand-DNA conjugates or adenovirus-ligand-DNA conjugates, lipofection membrane fusion or direct microinjection. These procedures and variations thereof are suitable for *ex vivo* as well as *in vivo* gene therapy. Protocols for molecular methodology of gene therapy suitable for use with the gene is described in Gene Therapy Protocols, edited by Paul D. Robbins, Human press, Totowa NJ, 1996.

Compounds identified according to the methods disclosed herein may be used alone at appropriate dosages defined by routine testing in order to obtain optimal inhibition or activity while minimizing any potential toxicity. In addition, co-administration or sequential administration of other agents may be desirable.

The invention is further illustrated by the following nonlimiting examples. All references cited herein are hereby incorporated herein by reference.

### Example 1

### Clinical Evaluation and Response Definitions

The current study was part of an open label, multicenter, non-comparative phase 2 clinical study in which patients with relapsed or refractory AML (Harousseau et al. (2003)) were treated with tipifarnib at a starting oral dose of 600 mg bid for the first 21 consecutive days of each 28-day cycle. Patients were enrolled into 2 cohorts, those with relapsed AML and those with refractory AML. A total of 252 patients (135 relapsed and 117 refractory) were treated. Eighty patients chose to provide bone marrow samples for RNA microarray analysis, for which a separate informed consent was required. The overall response rate was relatively low in this study. Therefore, for the purposes of the gene expression profiling, response to tipifarnib was defined as patients who had an objective response (complete remission [CR], complete remission with incomplete platelet recovery [CRp] or partial remission [PR]), a hematological response (decrease of >50% of leukemic blast cells in bone marrow) as determined by either central review or by the clinical site, or stable disease (no hematological response but no progression of the disease) as determined by both central review and the clinical site. Complete remission with incomplete platelet recovery was defined similarly, except for a platelet count less than 100,000/µL sufficient to ensure transfusion independence. Partial remission was defined as at least a 50% decrease in bone marrow blasts with partial neutrophil (>500/µL) and platelet count (>50,000/µL) recovery. Response had to be confirmed at least 4 weeks after first documentation.

### Sample Collection and Microarray Processing

Bone marrow samples were collected from patients before treatment with tipifarnib, diluted with phosphate buffered saline (PBS) and centrifuged with Ficoll-diatrizoate (1.077 g/mL). White blood cells were washed twice with PBS, resuspended in fetal bovine serum (FBS) with 10% dimethyl sulfoxide (DMSO) and immediately stored at -80°C. Cells were thawed and total RNA was extracted from cell samples using the RNeasy Kit (Qiagen, Valencia, CA). RNA quality was checked using the Agilent Bioanalyzer. Synthesis of cDNA and cRNA was performed according to Affymetrix (Santa Clara, CA) protocols.

### Microarray Processing

Two rounds of linear amplification were performed because the RNA yield for several samples was too low to obtain enough labeled cRNA for chip hybridization using one round of amplification. For hybridization, 11 µg of cRNA were fragmented randomly by incubation at 94°C for 35 minutes in 40 mM Tris-acetate, pH 8.1, 100 mM potassium acetate, and 30 mM magnesium acetate. Fragmented cRNA was hybridized to U133A arrays at 45°C for 16 hours in a rotisserie oven set at 60 rpm. Following hybridization, arrays were washed (with 6x SSPE and 0.5x SSPE containing Triton X-100 [0.005%]), and stained with streptavidin-phycoerythrin (SAPE; Molecular Probes, Eugene, OR). Quantification of bound labeled probe was conducted using the Agilent G2500A GeneArray scanner (Agilent Technologies, Palo Alto, CA).

The total fluorescence intensity for each array was scaled to the uniform value of 600. Chip performance was quantitated by calculating a signal to noise ratio (raw average signal/noise). Chips were removed from further analysis if their signal-to-noise ratio was less than 5. Genes were only included in further analysis if they were called "present" in at least 10% of the chips. Eleven thousand seven hundred twenty three Affymetrix probe sets remained following this cut-off. The quality of the gene expression data were further controlled by identifying outliers based on principal components analysis and by analyzing the normal distributions of the gene intensities (Partek Pro V5.1).

### Statistical Analysis

To identify genes that predict response with high sensitivity, a percentile analysis was employed. Genes that were up- or down-regulated in 100% of responders compared to at least 40% of non-responders were identified. The chi-squared test and Student's t-test were then used to test the significance of the correlations between patient response and patient co-variates, including ras mutation status, and gene expression. Unsupervised k-means and hierarchical clustering were performed in Omniviz. The predictive value of the selected genes was then analyzed by leave-one-out and leave-five-out cross validation methods. Here, one (or five) sample(s) was (were) removed from the data set and the marker was reselected from 11,723 genes. The predictive value of this gene was then tested on the left-out sample(s) using a linear discriminant analysis. Sensitivity was calculated as the number of true positives detected by the test divided by the sum of true positives plus false negatives. Specificity was calculated as the number of true negatives detected by the test divided by the sum of true negatives and false positives. Positive predictive value was calculated as the number of true positives divided by the number of true positives and false positives. Negative predictive value was calculated as the number of true negatives divided by the number of true negatives and false negatives. The positive likelihood ratio of a patient responding to treatment is sensitivity divided by 1 minus specificity. Receiver operator curves (ROC) were used to choose appropriate thresholds for each classifier, requiring a sensitivity of 100%. The ROC diagnostic calculates the sensitivity and specificity for each parameter.

### Real-Time RT-PCR Validation

TaqMan® real-time RT-PCR was employed to verify the microarray results of the AHR and AKAP13 genes. For each 1 µg sample of amplified RNA, cDNA was produced using T7 oligo(dT) primer and Superscript II reverse transcriptase according to the manufacturer's instructions (Invitrogen). Primers and MGB-probes for AKAP13 gene and control gene PBGD were designed using Primer Express (Applied Biosystems), while those for AHR gene and control gene HPRT were available as Assays-on-Demand from ABI. Primer/probe sequences for AKAP13 were as follows: AKAP13 forward, 5'GGTCAGATGTTTGCCAAGGAA3'; AKAP13 reverse, 5'TCTTCAGAAACACACTCCCATC-3'; AKAP13 probe, 6FAM-TGAAACGGAAGAAGCTTGTA-3'. All primers and probes were tested for optimal amplification efficiency above 90%. The relative standard curve was composed of 5 dilutions (10-fold each) of HeLa cDNA (in most cases, ranging from 25 ng to 2.5 pg). RT-PCR amplification mixtures (25 µL) contained 100 ng template cDNA, 2x TaqMan® universal PCR master mix (12.5 µL; Applied Biosystems), 500 nM forward and reverse primers, and 250 nM probe. Reactions were run on an ABI PRISM 7900HT Sequence Detector (Applied Biosystems). The cycling conditions were: 2 min of AmpErase UNG activation at 50°C, 10 min of polymerase activation at 95°C and 50 cycles at 95°C for 15 sec and annealing temperature (59°C or 60°C) for 60 sec. In each assay, a standard curve and a no-template control along with template cDNA were included in triplicates for the gene of interest and control gene. The relative quantity of each gene was calculated based on the standard curve, and was normalized with the quantity of the control gene. The median coefficient of variation (based on calculated quantities) of triplicate samples was 8%. The correlation between repeated runs using independently diluted templates from the stock was above 0.95. Samples were only compared with microarray data if duplicate TaqMan® experiments showed reproducible results.

### Cell Line Culture and AKAP 13 Over-expression Assay

The AKAP 13 vectors, oncoLBC and protoLBC, and vector control (pSRalpha-neo) were obtained from Dr. Deniz Toksoz. Zheng et al. (1995). The HL60 cell line was obtained from the American Tissue Culture Collection and grown in RPMI 1640 with 10% FBS. Cells were transiently transfected with each vector using the Effectene reagent (Qiagen) according to the manufacturer's instructions and kept under G418 (600 µg/mL) for 7 days. tipifarnib was then added in various concentrations (0, 1.5, 3.1, 6.3, 13, 25, 50, 100, 200, 1000, and 10,000 nM) to duplicate cultures (1.5x10⁵ cells/mL). Cells were counted at Day 6. Cell counts were normalized to cultures with no drug to give a percent of viable control cells.

### Results

### Expression Profiling of Relapsed and Refractory AML

FTIs were originally designed to specifically inhibit FTase activity, thereby blocking the oncogenic ras pathways. Therefore, we initially analyzed DNA from the bone marrow of 80 patients with relapsed or refractory AML for activating ras mutations and investigated the possible correlation between ras mutation and the response to tipifarnib. While 26% of the AML samples harbored N-ras mutations, mutation status did not correlate with objective response or overall survival. Harousseau et al. (2003). We therefore performed gene expression profiling to identify novel signatures that could be used to predict response to the FTI tipifarnib. Bone marrow samples were obtained for gene expression analysis from 80 patients prior to treatment with tipifarnib. Table 2 shows the patient information.

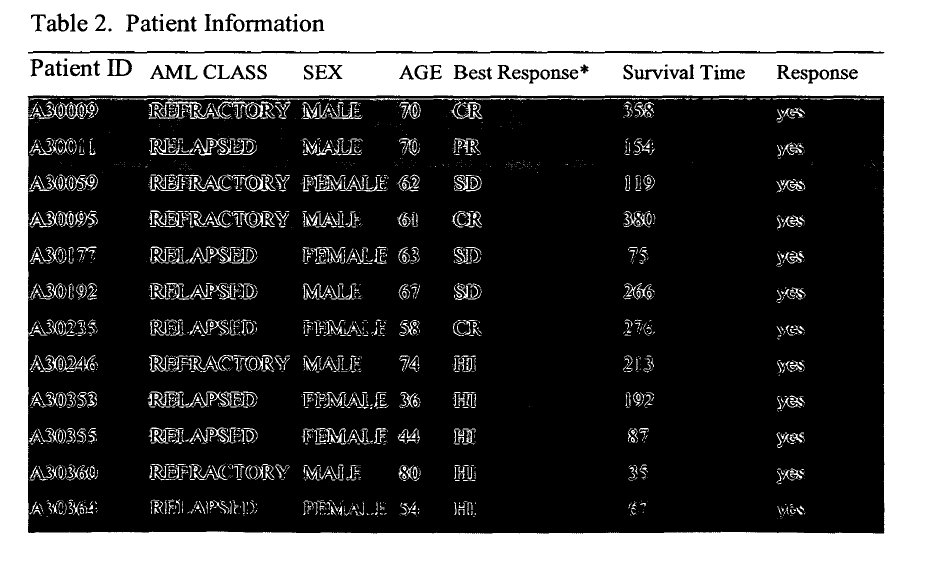

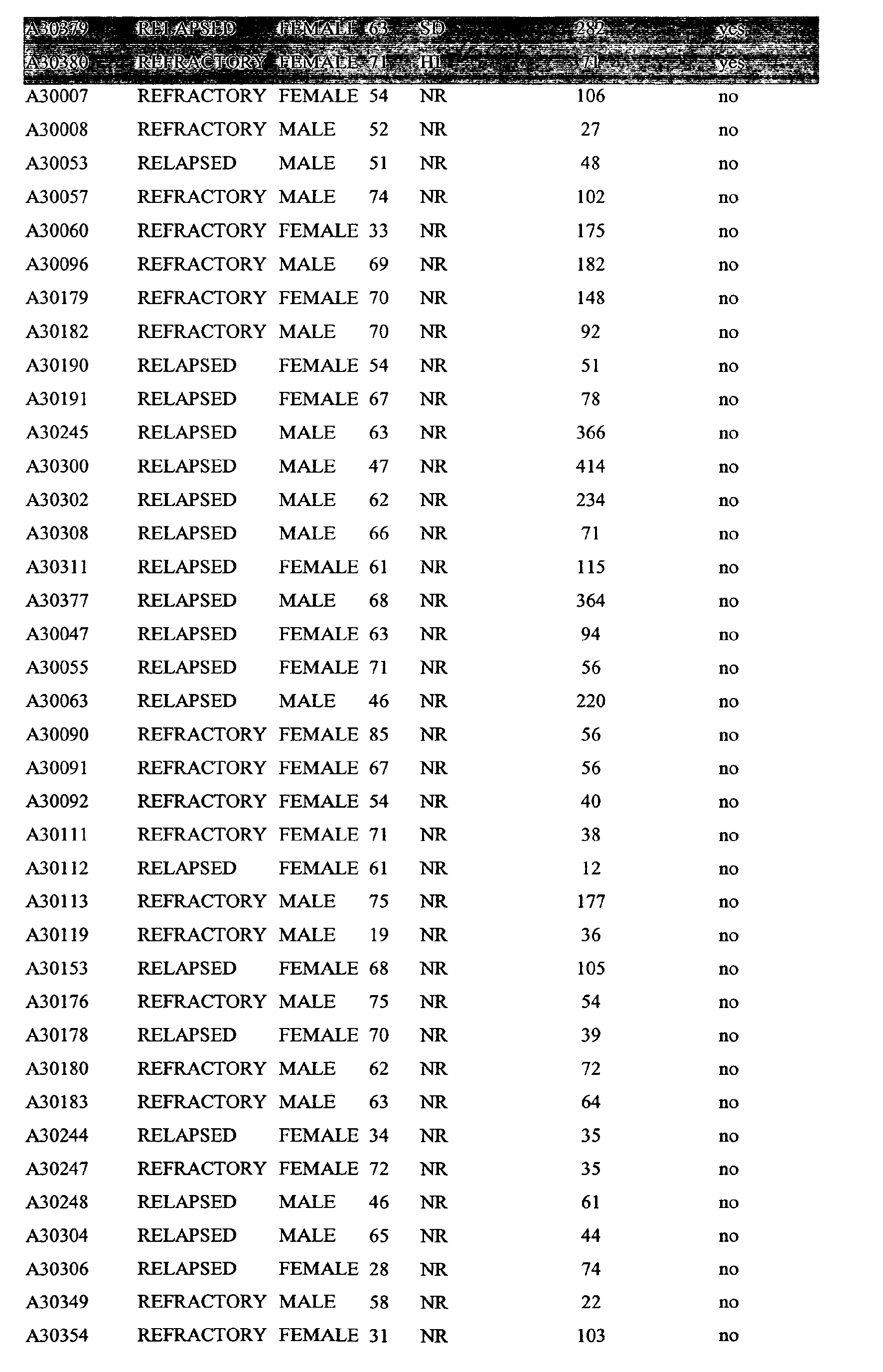

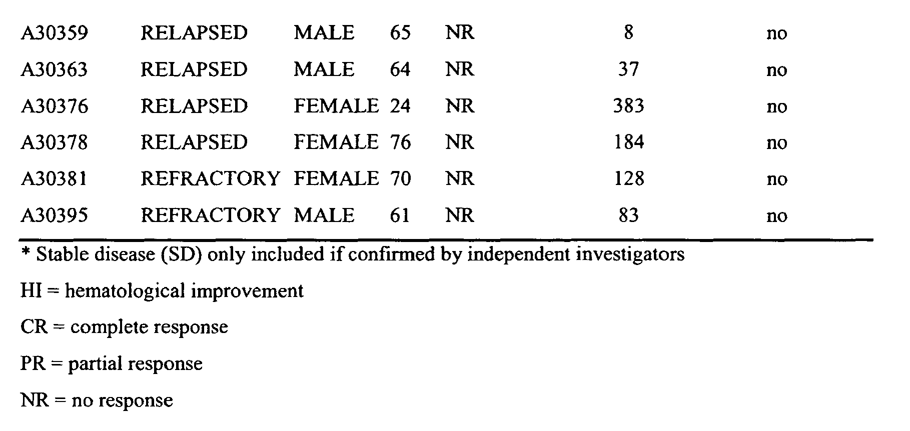

Fifty-eight of the 80 samples passed quality control measures including RNA quality and chip performance. There were no significant differences in age, sex, AML class (relapsed or refractory), cytogenic risk factors, baseline blast counts, response, and overall survival between these 58 patients and the remainder of the clinical study population (N = 194; Table 3).

**Table 3**

| co-variate | 58 subset | 194 remainder | p-value |
|---|---|---|---|
| response | 14 | 28 | 0.1237 |
| male | 28 | 119 | 0.1055 |
| average age | 60 | 56 | 0.1046 |
| relapsed | 31 | 104 | 0.8977 |
| cytogenetic risk | 34 | 5 | 0.1503 |
| average blasts | 55% | 50% | 0.1629 |

The gene expression data were integrated with the clinical information and retrospective analyses were performed to identify genes that could separate responders from non-responders with a high level of sensitivity. The data went through several filtering steps before identification of differentially expressed genes. First, genes that were not expressed in at least 10% of the samples were removed. This reduced the number of genes from approximately 22,000 to 11,723 genes. For unsupervised analyses genes that showed little variation in expression across the dataset (coefficient of variance of <45% across all the samples) were also excluded and quantile normalization was applied to the remaining 5,728 genes. At this stage an unsupervised k-means clustering analysis was performed to identify any differences between patients based on their global gene expression profiles.

Six main clusters of patients were identified using this technique. No separation between responders and non-responders was observed (Figure 1). Only a handful of genes may be associated with the anti-tumor effect of FTIs, for example, it is possible that the differential expression of a single gene that is involved in FTI biology impacts clinical response and this would be masked by the noise introduced from the other 11,722 genes.

### Example 2

### Identification of Genes that Are Differentially Expressed Between Responders and Non-responders

We next performed supervised analysis using the gene expression data to identify genes that were differentially expressed between all responders and at least 40% of non-responders. These criteria were chosen to identify genes that could predict response to tipifarnib with the highest level of sensitivity possible. From 11,723 genes, a total of 19 genes (Table 4) were identified that could stratify responders and non-responders (Table 5) and that gave significant P values in a t-test (P <0.05). The genes included those involved in signal transduction, apoptosis, cell proliferation, oncogenesis, and potentially, FTI biology (ARHH, AKAP13, IL3RA).

**Table 4**

| List of Top 19 Genes that Predict Response to tipifarnib | | | |
|---|---|---|---|
| Seq ID NO | Gene symbol | Gene ID | Functional description |
| 5 | AHR | NM_001621 | Apoptosis, cell cycle, signal transduction |
| 1 | AKAP13 | NM_006738 | Small GTPase mediated signal transduction, oncogenesis |
| 6 | MINA53 | NM_032778 | Cell proliferation |
| 7 | IDS | NM_000202 | Glycosaminoglycan degradation |
| 8 | OPN3 | NM_014322 | G-protein coupled receptor protein signaling |
| 9 | GPR105 | NM_014879 | G-protein coupled receptor protein signaling |
| 10 | TENC1 | NM_22748 | Signal transduction |
| 11 | TNFSF13 | NM_003808 | Cell proliferation |
| 12 | SVIL | NM_003174 | Cytoskeletal anchoring |
| 13 | IL3RA | NM_002183 | Receptor signaling |
| 14 | C6orf56 | NM_014721 | - |
| 15 | FRAG1 | NM_014489 | Tumor suppressor |
| 16 | GOSR1 | NM_004871 | Intra-Golgi transport |
| 17 | KIAA1036 | NM_014909 | - |
| 18 | BTG3 | NM_006806 | Regulation of cell cycle |
| 29 | MAPK8IP3 | NM_015133 | Regulation of JNK cascade |
| 20 | LILRB3 | NM_006864 | Immune response |
| 21 | ARHH | NM_004310 | Small GTPase mediated signal transduction |
| 22 | NPTX2 | NM_002523 | Heterophilic cell adhesion |

**Table 5**

| Results of Top 19 Genes that Predict Response to tipifarnib | | | |
|---|---|---|---|
| SEQ ID NO: | Gene symbol | Specificity | P value |
| 5 | AHR | 0.52 | 0.00000255 |
| 1 | AKAP13 | 0.63 | 0.00006133 |
| 6 | MINA53 | 0.50 | 0.00006934 |
| 7 | IDS | 0.50 | 0.00023964 |
| 8 | OPN3 | 0.40 | 0.00064297 |
| 9 | GPR105 | 0.43 | 0.00087608 |
| 10 | TENC1 | 0.43 | 0.0010309 |
| 11 | TNFSF13 | 0.40 | 0.00104219 |
| 12 | SVIL | 0.45 | 0.00145723 |
| 13 | IL3RA | 0.40 | 0.00198392 |
| 14 | C6orf56 | 0.40 | 0.00261553 |
| 15 | FRAG1 | 0.45 | 0.00298989 |
| 16 | GOSR1 | 0.45 | 0.01201057 |
| 17 | KIAA1036 | 0.43 | 0.01262079 |
| 18 | BTG3 | 0.47 | 0.01659402 |
| 19 | MAPK8IP3 | 0.40 | 0.01817428 |
| 20 | LILRB3 | 0.41 | 0.02374898 |
| 21 | ARHH | 0.40 | 0.02721922 |
| 22 | NPTX2 | 0.45 | 0.03346833 |

### Real Time RT-PCR Validation of Gene Markers

To verify the microarray gene expression data, TaqMan® real time RT-PCR was performed on cDNA that was used for generating the labeled target cRNA for microarray hybridization. Two genes were selected to verify the gene expression data. The AHR and AKAP13 genes were chosen because the use of these genes resulted in the highest level of specificity for responders. The correlation coefficient was 0.74 for AHR and 0.94 for AKAP13 indicating that the microarray gene expression data could be validated by PCR (Figure 2).

### Identification of the AKAP13 Gene as a Marker of Resistance

AKAP13 was over-expressed in patients who were resistant to tipifarnib. The predictive value of this gene was calculated for the 58 samples using a leave-one-out cross validation (LOOCV; Figure 3A). AKAP 13 gene expression predicted non-response with a negative predictive value (NPV) of 96%, and low expression levels mediated response with a positive predictive value (PPV) of 43% (χ² = 13.7; P = 0.0022). The overall diagnostic accuracy was 69% and positive likelihood ratio of responding was 2.4. Therefore, stratification of this patient population based on AKAP13 gene expression increased the response rate from 24% (14/58) in the entire group to 43% (13/30) among those patients with low expression of the gene. Expression values for the AKAP13 gene in each patient are shown in Figure 3B. When survival was analyzed by Kaplan-Meier analysis, the median survival of patients with low expression of this gene was 90 days longer than those patients who had high expression levels (P = 0.008; Figure 3C).

### Identification of a Minimal Set of 3 Gene Markers

LOOCV was used to identify a candidate set of gene markers that could predict response to tipifarnib with an improved accuracy compared to AKAP13 alone. Classifiers were built with an increasing number of genes based on t-test P values, and the error rate of these classifiers was calculated using LOOCV while keeping the sensitivity of predicting response at 100% (Figure 4A). The 3-gene classifier could predict response with the lowest error rate (Figure 4A). This was also observed when a leave-five-out cross validation was performed. When more genes were added the error rate increased, indicating that additional genes were introducing noise to the classifier. For the 3-gene classifier, the LOOCV demonstrated a NPV of 94% and a PPV of 48%, with an overall diagnostic accuracy of 74% and positive likelihood ratio of 2.9 (Figure 4B). The combined expression values for the 3 genes in each patient are shown in Figure 4C. Therefore, for the group of patients with this gene signature, the response rate to tipifarnib was 48% (12/25) compared to 24% (14/58) in this patient population.

Using the 3-gene signature (AHR, AKAP13 and MINA53), Kaplan-Meier analysis again showed a significant difference in survival between the predicted responder group and the non-responder group (Figure 4D). The 13 patients who were incorrectly classified as responders had a better overall survival compared to the 31 patients correctly classified as non-responders (Figure 5). Interestingly, the 2 patients that were misclassified as non-responders only demonstrated hematological improvements with relatively short overall survival times (71, 87 days).

### Over-expression of AKAP13 Increases Resistance to tipifarnib in AML

The AKAP13 gene was the most robust marker of resistance to tipifarnib. We therefore investigated its involvement in FTI biology by over-expressing the oncoLBC and protoLBC variants of this gene in the HL60 cell line. Transient transfectants were then tested for sensitivity to tipifarnib. Over-expression of both AKAP13 variants in this AML cell line model led to an approximate 20-fold increase in resistance to tipifarnib compared to control cells (Figure 3). Both the LBC oncogene and proto-oncogene increased the resistance to tipifarnib to the same extent, as seen by a parallel rightward shift of the kill curves by more than one log-unit compared to control.

### Discussion

Two groups recently identified gene expression profiles of newly diagnosed adult AML patients that are useful for predicting clinical outcome. Bullinger et al. (2004); and Valk et al. (2004). These profiles seem to be more powerful than currently used prognostic markers such as karyotyping. Moreover, expression profiles have been found that predict response to anticancer compounds including standard chemotherapeutics (Chang et al. (2003); Okutsu et al. (2002); and Cheok et al. (2003)) and novel selective anticancer agents. Hofmann et al. (2002); and McLean et al. (2004). Similarly, pharmacogenetic profiles have recently been found that correlate with patient response to the tyrosine kinase inhibitor gefitinib. Paez et al. (2004) and Lynch et al. (2004). In that study, a subgroup of non-small cell lung cancer patients had activating mutations within the target epidermal growth factor receptor that correlated with clinical response to the targeted therapy.

In a phase 2 study of relapsed and refractory AML patients, we have identified gene expression profiles that predict response to tipifarnib, a novel farnesyl transferase inhibitor. This class of compounds is showing promise in the treatment of hematological malignancies (Karp et al. (2001); Kurzrock et al. (2004); Alsina et al. (2003); Cortes et al. (2003); and Thomas et al. (2001)) and solid tumors such as breast cancer (Johnston et al. (2003)) and recurrent glioma. Brunner et al. (2003). However, while clinical responses are being demonstrated, there is a growing need to tailor therapy by identifying patients who are most likely to respond to the drug and are, therefore, the best candidates for treatment. Furthermore, while ras was considered to be a primary target of this class of drugs, several clinical studies have shown that they are not necessarily effective in populations with a high frequency of ras mutations. Van Cutsem et al. (2004); and Rao et al. (2004).

Several gene markers were identified that have the potential to predict response to tipifarnib. A subset of these markers was both predictive of drug response and also thought to have the potential to be involved in FTI biology. One of the top candidates discovered from the microarray studies was the lymphoid blast crisis oncogene (oncoLBC or AKAP13). This gene functions as a guanine nucleotide exchange factor for the Rho proteins (Zheng et al. (1995)) and as a protein kinase A anchoring protein. Carr et al. (1991). AKAP13 contains a region that is homologous to an α-helical domain that is known to interact with lamin B. Foisner et al. (1991). This association could lead to lamin B activation via protein kinase A, consequently increasing mitotic activity. Both RhoB and lamin B are farnesylated and are candidate targets of FTIs. AKAP13 is also a proto-oncogene, because loss of its 3-prime end causes cellular transformation. Sterpetti et al. (1999). While it was originally identified from a patient with chronic myeloid leukemia, its expression has not been documented in AML.

The identification of several genes that are potentially involved in FTI biology (ARHH, AKAP 13, IL3RA) support the idea that the interaction of multiple pathways can affect how FTIs function in this population of AML patients (Figure 7). These genes interact with several farnesylated proteins including ras, rho, and potentially lamin B. Rho proteins are potentially important antitumorigenic targets for FTIs. Sahai et al. (2002); and Lancet et al. (2003). RhoB, RhoA, and RhoC have been found to be over-expressed in multiple cancer types. Sahai et al. (2002). In addition, RhoH (ARHH) is frequently re-arranged in tumors of myeloid origin, and this may lead to its over-expression. Pasqualucci et al. (2001). While most of these Rho proteins are geranygeranylated, they interact closely with each other and the farnesylated ras, RhoE, and RhoB small GTPases. Sahai et al. (2002); and Li et al. (2002). Furthermore, it has been shown that RhoH, RhoB, and RhoE can act in an antagonistic fashion to the transforming abilities of RhoA and RhoG. Li et al. (2002). The activity of RhoA, and possibly other related small GTPases, is increased by the guanine nucleotide exchange factor lymphoid blast crisis oncogene (AKAP13). Sterpetti et al. (1999); and Toksoz et al. (1994). In addition, AKAP13 may increase mitotic activity by activating lamin B via protein kinase A. Foisner et al. (1991). It is also well known that the IL3 receptor activates ras pathways. Testa et al. (2004). Therefore, as indicated in Figure 7, the increased activity of IL3RA and AKAP13, and the decrease in RhoH expression could lead to an increased cellular profile of transformation. This might allow for the leukemic blast cell to overcome the anti-tumorigenic effects of FTIs through compensatory pathways. In contrast, when IL3RA and AKAP13 are under-expressed and there is an increase in RhoH activity, FTIs may be more effective in blocking these pathways.

Finally, we demonstrated that over-expression of AKAP13 (both oncoLBC and protoLBC variants) increased the IC₅₀ of the HL60 AML cell line by approximately 20-fold. This indicates that over-expression of AKAP13 is a relevant marker of resistance and that it may also be a useful alternative drug target for patients who are resistant to tipifarnib.

Overall, our findings allow development of a gene expression based diagnostic assay to identify patients likely respond to tipifarnib. This information could be used to better direct treatment to the appropriate patient population. Using survival as the gold standard, the gene signature predicts a level of response to therapy that cannot be predicted by using conventional clinical response criteria. Alternatively, this raises the question of whether the gene signature for predicting response to FTI treatment also has prognostic value irrespective of FTI therapy. We thus evaluated a prognostic signature previously identified in newly diagnosed AML patients who were treated with conventional chemotherapy. Bullinger et al. (2004). While this signature showed utility in the current patient population our 3-gene signature further stratified these poor and good prognostic groups showing that it is a predictor of response to FTIs.

### Example 3

### Analysis of an AML prognostic gene signature

The 3-gene signature can predict prognosis irrespective of the type of drug treatment. To determine this, we first evaluated a gene-expression signature recently identified in newly diagnosed AML patients who were treated with conventional chemotherapy. Bullinger et al. (2004). This signature was defined using a cDNA array and therefore we first matched these genes with the probes present on the Affymetrix gene chip. Of the 133 predictive genes identified by Bullinger et al., 167 probe sets (corresponding to 103 unique genes) were matched to the Affymetrix U133A chip. The 3 genes identified in our present analysis are not present in the Bullinger et al. 133 gene list. SEQ ID NOs:23-189. Two main groups of patients were defined by hierarchical clustering using these 167 probe sets (Fig 8A). Kaplan-Meier analysis showed a clear stratification of these clusters into patients with good and poor prognosis (Fig 8B, p = 0.000003). Our data therefore show that a subset of the 133-gene prognostic signature identified by Bullinger et al. (2004) can also be used in a relapsed and refractory cohort of patients. Consequently, this indicates that the prognostic gene profile is surprisingly robust across different microarray platforms, and different classes of AML.

Neither of the clusters defined by the prognostic gene signature had significantly more responders. However, when the tipifarnib 3-gene signature was applied to the good and poor prognostic groups, patients who responded to tipifarnib were further stratified from both prognostic groups (Fig 8C). Therefore, the 3-gene signature has independent utility to the prognostic signature and that it is specific for FTI treatment in this population of patients.

### Example 4

### Clinical Evaluation and Response Definitions

The current study was an open label, multicenter, non-comparative Phase 2 study investigating the efficacy and safety of farnesyl transferase inhibition with tipifarnib administered as a single agent, at a starting oral dose of 600 mg b.i.d. for the first 21 days of each 28 day cycle in AML. Subjects were enrolled into two cohorts, those with relapsed AML and those with refractory AML. A total of 252 patients (135 relapsed and 117 refractory) were treated. For the purposes of the gene expression profiling response to tipifarnib was defined as patients who had an objective response (CR, CRp, or PR) (as described above), or patients who demonstrated confirmed stable disease, or a hematological response (decrease of >50% of leukemic blast cells) as determined by either central review or by the clinical site at any time during follow up.

### Sample Collection and Microarray Processing

All samples were obtained from patients who had consented to the described processing and analyses. Bone marrow samples were collected from patients before treatment with tipifarnib, diluted with PBS and centrifuged with Ficoll-diatrizoate (1.077g/ml). White blood cells were washed twice with PBS, resuspended in FBS with 10% DMSO and immediately stored at -80°C. Cells were thawed and total RNA was extracted from cell samples using the RNeasy Kit (Qiagen, Valencia, CA). RNA quality was checked using the Agilent Bioanalyzer. Synthesis of cDNA and cRNA were performed according to Affymetrix (Santa Clara, CA) protocols. Two rounds of linear amplification were performed because the RNA yield for several samples was too low to obtain enough labeled cRNA for chip hybridization using one round of amplification.

For hybridization, 11 µg of cRNA were fragmented randomly by incubation at 94°C for 35 min in 40 mM Tris-acetate, pH 8.1, 100 mM potassium acetate, and 30 mM magnesium acetate. Fragmented cRNA was hybridized to U133A arrays at 45°C for 16 h in a rotisserie oven set at 60 rpm. Following hybridization, arrays were washed (with 6x SSPE and 0.5x SSPE containing Triton X-100 (0.005%)), and stained with streptavidin-phycoerythrin (SAPE; Molecular Probes, Eugene, OR). Quantification of bound labeled probe was conducted using the Agilent G2500A GeneArray scanner (Agilent Technologies, Palo Alto, CA).

The total fluorescence intensity for each array was scaled to the uniform value of 600. Chip performance was quantitated by calculating a signal to noise ratio (raw average signal/noise). Chips were removed from further analysis if their signal-to-noise ratio was less than 5. Genes were only included in further analysis if they were called "present" in at least 10% of the chips. Approximately 12,000 Affymetrix probe sets remained following this cut-off. Gene expression data quality was further controlled by identifying outliers based on principal components analysis and by analyzing the normal distributions of the gene intensities (Partek Pro V5.1).

### Statistical Analysis

Unsupervised hierarchical clustering and clustering was performed in Omniviz. Kaplan-Meier analysis was performed using S-Plus.

### Example 5

### A prognostic signature identified in de novo AML has utility in relapsed and refractory patients

Two papers were recently published describing gene-expression profiling of newly diagnosed adult AML patients and its use in predicting clinical outcome. Bullinger et al. (2004); and Valk et al. (2004). We have profiled 58 patients with relapsed and refractory AML using the Affymetrix U133A gene chip. Of the 133 predictive genes identified by Bullinger et al. 167 probe sets (corresponding to 103 unique genes) were identified on the U133A chip (Fig 9). Bullinger et al. (2004). The 167 probe sets are listed in the Sequence Listing Table and designated SEQ ID NOs: 23-189.

Two main groups of patients were defined by hierarchical clustering using these 167 probe sets (Fig 10A). Kaplan-Meier analysis showed a clear stratification of these clusters into patients with good and poor prognosis (Fig. 10B, p = 0.0000219). Our data therefore shows that a 103 gene subset of the 133-gene prognostic signature identified by Bullinger et al. (2004) can also be used in a relapsed and refractory cohort of patients. Table 6. Consequently, this indicates that the prognostic gene profile is surprisingly robust across different microarray platforms, different classes of AML, and for different treatment algorithms.

**Table 6**

| SEQ ID NO: | cluster1 mean | cluster2 mean | ratio | good prognostic group |
|---|---|---|---|---|
| 23 | 2.101613636 | 0.980102944 | 2.144278466 | up |
| 24 | 1.574545244 | 0.899302214 | 1.750852182 | up |
| 25 | 2.459568465 | 1.161727897 | 2.117163987 | up |
| 26 | 1.752902097 | 0.968494897 | 1.809923937 | up |
| 27 | 2.395730325 | 0.72630844 | 3.298502667 | up |
| 28 | 1.319861385 | 1.36567127 | 0.96645614 | down |
| 29 | 1.269874887 | 1.430330507 | 0.8878192 | down |
| 30 | 0.986377684 | 1.432950683 | 0.688354244 | down |
| 31 | 1.177005842 | 1.428309412 | 0.824055231 | down |
| 32 | 1.095239589 | 0.99028255 | 1.105986962 | up |
| 33 | 1.798024918 | 0.806182842 | 2.230294202 | up |
| 34 | 5.041411016 | 0.60238936 | 8.369024013 | up |
| 35 | 1.159807609 | 1.639962342 | 0.707216001 | down |
| 36 | 1.300310388 | 1.324114868 | 0.982022345 | down |
| 37 | 1.342190037 | 2.306688065 | 0.581868896 | down |
| 38 | 1.320735142 | 1.367135334 | 0.966060279 | down |
| 39 | 3.891659096 | 1.14773151 | 3.390739962 | up |
| 40 | 1.519690498 | 1.219697115 | 1.245957278 | up |
| 41 | 1.328167684 | 1.250925424 | 1.061748094 | up |
| 42 | 4.052024058 | 0.844109372 | 4.800354307 | up |
| 43 | 2.091653255 | 0.964077201 | 2.169591038 | up |
| 44 | 1.991638259 | 1.045332875 | 1.905267027 | up |
| 45 | 2.231280889 | 0.889145566 | 2.509466362 | up |
| 46 | 1.815199475 | 0.992602441 | 1.828727596 | up |
| 47 | 1.3198052 | 1.283492456 | 1.028292136 | up |
| 48 | 2.222738653 | 0.801693589 | 2.772553857 | up |
| 49 | 1.591280353 | 1.037860182 | 1.533231913 | up |
| 50 | 1.050088807 | 1.267434876 | 0.828515 | down |
| 51 | 1.07046517 | 1.621321185 | 0.660242511 | down |
| 52 | 1.768390061 | 0.83169323 | 2.126252803 | up |
| 53 | 1.253048826 | 1.17309932 | 1.068152376 | up |
| 54 | 1.24240959 | 1.057597528 | 1.174747063 | up |
| 55 | 1.039468561 | 1.565715718 | 0.663893546 | down |
| 56 | 0.897290104 | 2.462204436 | 0.364425509 | down |
| 57 | 2.042794407 | 1.039120916 | 1.965887103 | up |
| 58 | 2.935364557 | 1.109613717 | 2.645393178 | up |
| 59 | 0.771931017 | 1.585630652 | 0.486829021 | down |
| 60 | 1.206470925 | 1.279664724 | 0.942802363 | down |
| 61 | 1.099482264 | 1.491611294 | 0.737110445 | down |
| 62 | 4.901329448 | 0.871582063 | 5.623485909 | up |
| 63 | 2.346190152 | 0.930016604 | 2.522740069 | up |
| 64 | 0.774893647 | 2.117870914 | 0.365883323 | down |
| 65 | 8.626480573 | 0.518118436 | 16.64963061 | up |
| 66 | 2.002466652 | 2.360710711 | 0.84824737 | down |
| 67 | 1.031573096 | 1.410790966 | 0.731201943 | down |
| 68 | 1.236611762 | 1.579356606 | 0.782984512 | down |
| 69 | 4.252841813 | 1.038446727 | 4.095387566 | up |
| 70 | 2.330630017 | 0.740455045 | 3.147564506 | up |
| 71 | 1.271065192 | 1.275443314 | 0.996567372 | down |
| 72 | 4.079291876 | 0.770236845 | 5.296152606 | up |
| 73 | 1.613398862 | 1.053542946 | 1.531403032 | up |
| 74 | 2.462533188 | 0.694618451 | 3.54515948 | up |
| 75 | 1.736866874 | 1.312450625 | 1.323376926 | up |
| 76 | 0.973964566 | 1.442647881 | 0.675122862 | down |
| 77 | 2.146894817 | 1.117648799 | 1.920902898 | up |
| 78 | 1.121767102 | 1.496868758 | 0.749409122 | down |
| 79 | 1.09122088 | 1.120796083 | 0.973612325 | down |
| 80 | 1.563910635 | 1.102945516 | 1.417940063 | up |
| 81 | 1.473673649 | 1.474149259 | 0.999677367 | down |
| 82 | 1.148794849 | 1.399237842 | 0.821014709 | down |
| 83 | 1.350310245 | 1.327639719 | 1.017075812 | up |
| 84 | 1.44906906 | 1.424150806 | 1.017496921 | up |
| 85 | 1.790625889 | 1.255091645 | 1.426689354 | up |
| 86 | 1.423005429 | 1.45961841 | 0.97491606 | down |
| 87 | 1.09603256 | 1.181485007 | 0.927673693 | down |
| 88 | 1.125580943 | 1.178287994 | 0.955268108 | down |
| 89 | 0.891946685 | 1.753005455 | 0.508809988 | down |
| 90 | 1.2942793 | 2.776461422 | 0.466161456 | down |
| 91 | 1.102290438 | 1.391074208 | 0.792402326 | down |
| 92 | 1.186998217 | 2.302906551 | 0.515434817 | down |
| 93 | 0.79821098 | 1.919103099 | 0.415929181 | down |
| 94 | 1.236023874 | 1.187331666 | 1.041009778 | up |
| 95 | 1.087313678 | 1.212772631 | 0.896551959 | down |
| 96 | 1.820970134 | 1.219911387 | 1.492706891 | up |
| 97 | 0.893432913 | 1.529980396 | 0.583950563 | down |
| 98 | 1.076214323 | 1.702252048 | 0.632229713 | down |
| 99 | 1.407137316 | 1.229815501 | 1.144185705 | up |
| 100 | 2.943883289 | 0.906317573 | 3.248180746 | up |
| 101 | 1.666394606 | 0.87032571 | 1.914679281 | up |
| 102 | 1.499937462 | 0.921725287 | 1.627315083 | up |
| 103 | 1.035229803 | 1.508152041 | 0.686422705 | down |
| 104 | 1.317050838 | 1.350558887 | 0.975189495 | down |
| 105 | 1.481608791 | 1.161107338 | 1.276030856 | up |
| 106 | 1.177285398 | 1.293627618 | 0.910065139 | down |
| 107 | 1.328716012 | 1.354505535 | 0.980960194 | down |
| 108 | 1.245636653 | 1.388354047 | 0.897203891 | down |
| 109 | 1.057630522 | 1.407147783 | 0.751612968 | down |
| 110 | 1.394737541 | 1.22633541 | 1.137321429 | up |
| 111 | 1.194410314 | 1.007530697 | 1.185482803 | up |
| 112 | 1.653993358 | 1.201194991 | 1.37695659 | up |
| 113 | 1.318561503 | 1.233384342 | 1.069059707 | up |
| 114 | 1.045812959 | 1.463795973 | 0.714452682 | down |
| 115 | 1.721871636 | 1.213128563 | 1.419364517 | up |
| 116 | 1.437978693 | 1.211203408 | 1.18723138 | up |
| 117 | 1.480402866 | 1.289306967 | 1.148215983 | up |
| 118 | 1.750186104 | 1.199777316 | 1.458759122 | up |
| 119 | 1.987020453 | 2.331667877 | 0.852188458 | down |
| 120 | 1.419274906 | 1.587359923 | 0.894110331 | down |
| 121 | 1.412275401 | 1.410313065 | 1.001391419 | up |
| 122 | 1.339345508 | 1.331753924 | 1.005700441 | up |
| 123 | 1.747595879 | 1.473302004 | 1.186176272 | up |
| 124 | 1.237925661 | 1.240617377 | 0.997830341 | down |
| 125 | 1.176676928 | 1.14697739 | 1.025893743 | up |
| 126 | 1.256304659 | 1.357899094 | 0.925182633 | down |
| 127 | 2.013680769 | 0.801461983 | 2.512509405 | up |
| 128 | 1.405740421 | 1.158990553 | 1.212900673 | up |
| 129 | 1.346444485 | 1.136343796 | 1.184891835 | up |
| 130 | 9.592759013 | 1.560596128 | 6.146855576 | up |
| 131 | 1.768393033 | 1.23257422 | 1.434715252 | up |
| 132 | 1.40453464 | 1.860231401 | 0.755032218 | down |
| 133 | 1.673447728 | 3.930927905 | 0.425713157 | down |
| 134 | 1.283792446 | 1.501667999 | 0.85491097 | down |
| 135 | 1.214492279 | 1.091382628 | 1.11280155 | up |
| 136 | 1.561740275 | 1.228333636 | 1.271430033 | up |
| 137 | 1.734251715 | 1.844252606 | 0.940354759 | down |
| 138 | 1.408580649 | 1.582858413 | 0.889896807 | down |
| 139 | 1.1383915 | 1.741970797 | 0.653507798 | down |
| 140 | 1.079716972 | 1.450127515 | 0.744566917 | down |
| 141 | 1.783529874 | 1.314108612 | 1.357216487 | up |
| 142 | 1.136773942 | 1.003606776 | 1.132688588 | up |
| 143 | 1.220645614 | 1.113413039 | 1.096309789 | up |
| 144 | 1.183017496 | 1.211183566 | 0.976745003 | down |
| 145 | 1.113306085 | 1.067417158 | 1.042990621 | up |
| 146 | 1.211346488 | 1.1871952 | 1.020343148 | up |
| 147 | 0.998901987 | 1.654269584 | 0.60383265 | down |
| 148 | 1.133736575 | 1.914231031 | 0.592267368 | down |
| 149 | 1.412382921 | 1.384849519 | 1.019881873 | up |
| 150 | 3.988440713 | 0.965026485 | 4.132985753 | up |
| 151 | 1.187128423 | 1.462785203 | 0.81155348 | down |
| 152 | 3.691405839 | 0.765303503 | 4.823453473 | up |
| 153 | 2.216130138 | 0.848821568 | 2.610831558 | up |
| 154 | 2.199861346 | 0.773086467 | 2.845556663 | up |
| 155 | 1.67633492 | 1.194585675 | 1.403277265 | up |
| 156 | 7.743976211 | 0.841225224 | 9.205592025 | up |
| 157 | 2.591731743 | 1.08342825 | 2.392158173 | up |
| 158 | 1.273363236 | 1.097292728 | 1.160459013 | up |
| 159 | 1.095879149 | 1.302490014 | 0.8413724 | down |
| 160 | 0.987046127 | 2.124625445 | 0.464574181 | down |
| 161 | 2.103990301 | 0.929475745 | 2.263631205 | up |
| 162 | 1.439273076 | 1.211738853 | 1.187774967 | up |
| 163 | 1.121983318 | 1.251788385 | 0.896304305 | down |
| 164 | 1.197910138 | 1.34693432 | 0.889360468 | down |
| 165 | 1.144102989 | 1.261720239 | 0.906780246 | down |
| 166 | 1.078544278 | 1.162115183 | 0.928087244 | down |
| 167 | 1.46401688 | 0.960152377 | 1.524775561 | up |
| 168 | 1.262437604 | 1.105379077 | 1.142085669 | up |
| 169 | 1.349621283 | 1.10729713 | 1.218842935 | up |
| 170 | 1.203037349 | 1.068047506 | 1.126389361 | up |
| 171 | 1.395352279 | 1.172354292 | 1.190213819 | up |
| 172 | 1.131210862 | 1.399636303 | 0.80821772 | down |
| 173 | 0.859929427 | 1.37477642 | 0.625504929 | down |
| 174 | 0.72293765 | 2.343345902 | 0.308506588 | down |
| 175 | 1.340847351 | 1.601484153 | 0.837252962 | down |
| 176 | 1.259818484 | 1.213892186 | 1.037833918 | up |
| 177 | 0.60640428 | 2.092078796 | 0.289857285 | down |
| 178 | 1.099549227 | 1.166865002 | 0.942310572 | down |
| 179 | 1.615399946 | 1.733324276 | 0.931966378 | down |
| 180 | 0.734401434 | 2.496195783 | 0.294208267 | down |
| 181 | 2.158835355 | 0.765517273 | 2.82010012 | up |
| 182 | 0.957971846 | 1.386779118 | 0.690789061 | down |
| 183 | 0.982131268 | 1.162818279 | 0.844612856 | down |
| 184 | 2.421692274 | 0.791473723 | 3.059725425 | up |
| 185 | 1.268020946 | 0.973474927 | 1.302571757 | up |
| 186 | 1.054213523 | 1.147045848 | 0.91906834 | down |
| 187 | 1.251020003 | 1.271391137 | 0.983977288 | down |
| 188 | 1.122443987 | 1.153661375 | 0.972940597 | down |
| 189 | 1.301034813 | 1.029185966 | 1.264139675 | up |

The prognostic signature is independent of a 3-gene signature that predicts response to tipifarnib.

We have previously identified a 3-gene signature (AHR, AKAP 13, MINA53) that predicts response to tipifarnib in relapsed and refractory AML patients. These genes can stratify patients into good and poor prognostic outcome groups (Fig 11B, p = 0.002). The question arises as to whether this gene signature is predicting response to FTI treatment or merely identifying patients who have a generally good prognosis. When the 3-gene signature was applied to the good and poor prognostic groups, responders were further stratified from the prognostic groups (Fig 11C, p = 0.000003). Following the application of both gene signatures there is clear stratification of a group of patients that do not respond to tipifarnib and have a poor prognosis irrespective of treatment type (Fig 11D, p = 0.0000005). Therefore, the 3-gene signature seems to be independent of the prognostic signature that has been identified and it is specific for FTI treatment in this population of patients. As a result we suggest that the prognostic signature maybe used in conjunction with drug-specific signatures (such as the tipifarnib predictive profile) to better manage patient therapy.

### Example 6

### Identification of genes that are differentially expressed between responders and non-responders (not including stable disease patients)

Four patients were removed from the analysis since they were classified as having stable disease and these patients cannot be clearly defined as either responders or non-responders. Inclusion of stable disease patients may bias the analysis for selecting genes associated with prognosis irrespective of drug treatment. This resulted in comparing 10 responders with 44 non-responders. Selected genes were required to show a specificity of 40% and a minimum mean fold-change of 2.0. These criteria were chosen to identify genes that could predict response to tipifarnib with the highest level of sensitivity possible. From 11,723 genes, a total of 8 genes were identified that could stratify responders and non-responders (Table 6a) and that gave significant P values in a t-test (P <0.05). The genes included those involved in signal transduction, apoptosis, cell proliferation, oncogenesis, and potentially, FTI biology.

### AKAP13 is the most robust marker

We next aimed at identifying a minimal set of genes that would provide the best diagnostic accuracy from the 8 selected genes. Classifiers were built with an increasing number of genes based on the AUC values from receiver operator characteristic analysis, and the error rate of these classifiers was calculated using LOOCV while keeping the sensitivity of predicting response at 100% (Fig. 12a). The AKAP13 gene could predict response with the lowest error rate of less than 40% (Fig. 12a). The error rate increased to more than 50% when more than 2 genes were used in the classifier. For the AKAP13 the LOOCV demonstrated a NPV of 93% and a PPV of 31%, with an overall diagnostic accuracy of 63% and positive likelihood ratio of 2.0 (Fig. 12b). The expression value for AKAP 13 in each patient is shown in Fig. 12c. Therefore, for the group of patients with low expression of AKAP 13, the response rate to tipifarnib was 31% (8/26) compared to 18% (10/54) in the current patient population. Using the AKAP13 gene, Kaplan-Meier analysis showed a significant difference in survival between the predicted responder group and the non-responder group (Fig. 12d).

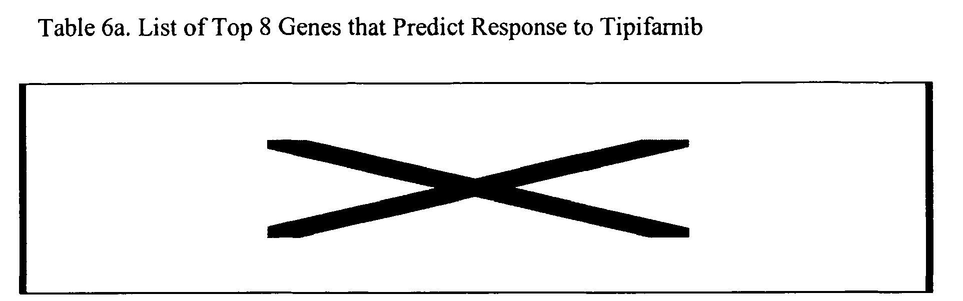

AUC = area under the curve from receiver operator characteristic analysis. This is an indication of the overall diagnostic accuracy.

### Example 7

### Antibodies (Prophetic)

An LBC oncogene-derived peptide is synthesized, coupled to keyhole limpet hemocyanin, and used to immunize rabbits for production of polyclonal antibodies. The sera are tested for reactivity against the corresponding peptide with ELISA, and the positive batches are affinity-purified. The purified antibody specifically detects the peptide that has the epitope in tissue sections. This is verified by complete abolishment of the signal if the corresponding peptide is added simultaneously with the antibody. In addition to this polyclonal antibody, which works well in IHC, monoclonal antibodies able to detect the protein in its natural fold are produced. To produce monoclonal antibodies, a purified antigen, produced in mammalian cells to ensure natural fold and posttranslational modifications, is generated. The antigen, LBC onco protein-IgG constant part fusion protein, is expressed in mouse myeloma cells, and the protein is purified using the Fc part as bait. This purified antigen is recognized in Western blot by the C-terminal polyclonal antibody. The antigen is used to generate mouse monoclonal antibodies against LBC peptides by selecting out of the positive clones those that produce antibodies that react against LBC peptide instead of the IgG constant part. Kits for the clinical identification of LBC oncogene can be readily fashioned employing these and similar antibodies. Such kits would include antibodies directed to LBC peptide identification (and hence, LBC oncogene), appropriate indicator reagents (e.g., enzymes, labels, and the like), and (optionally) other reagents useful in the clinical application of such a kit such as dilution buffers, stabilizers, and other materials typically used in such assays.

### Example 8

### Immunohistochemistry (Prophetic)

An affinity-purified polyclonal antibody against the C-terminal peptide of LBC oncogene is used for the IHC detection and localization of LBC oncogene. Four µm sections from formalin-fixed and paraffin embedded normal and tumor tissue is mounted on 3-aminopropyl-triethoxy-silane (APES, Sigma, St. Louis, MO) coated slides. The sections are deparaffinized and rehydrated in graded concentrations of ethanol and treated with methanolic peroxide (0.5% hydrogen peroxide in absolute methanol) for 30 minutes at room temperature to block the endogenous peroxidase activity. Antigen retrieval is done in a microwave oven twice for 5 minutes (650W). An Elite ABC Kit (Vectastain, Vector Laboratories, Burlingame, CA) is used for immunoperoxidase staining. The LBC peptide antibody is used at an optimal dilution of 1:2000. Both the biotinylated second antibody and the peroxidase-labeled avidin-biotin complex are incubated on the sections for 30 minutes. The dilutions are made in PBS (pH 7.2), and all incubations are carried out in a moist chamber at room temperature. Between the different staining steps the slides are rinsed three times with PBS. The peroxidase staining is visualized with a 3-amino-9-ethylcarbazole (Sigma) solution (0.2 mg/ml in 0.05 M acetate buffer containing 0.03% hydrogen peroxide, pH 5.0) at room temperature for 15 minutes. Finally, the sections are lightly counterstained with Mayer's haematoxylin and mounted with aqueous mounting media (Aquamount, BDH). In control experiments the primary antibodies are replaced with the IgG fraction of normal rabbit serum or the primary antibody was preabsorbed with the LBC peptide. These stainings indicate the presence of the LBC oncogene in a subset of cells.

Although the foregoing invention has been described in some detail by way of illustration and example for purposes of clarity of understanding, the descriptions and examples should not be construed as limiting the scope of the invention.

**Table 7**

| Sequence Listing Description | | |
|---|---|---|
| SEQ ID NO: | psid | description |
| 1 | 208325_s_at | AKAP 13 A kinase (PRKA) anchor protein 13 |
| 2 | | AKAP 13 forward primer |
| 3 | | AKAP 13 reverse primer |
| 4 | | AKAP 13 probe |
| 5 | 202820_at | Aryl hydrocarbon receptor |
| 6 | 213188_s_at | Myc-induced nuclear antigen, 53 kDa |
| 7 | 210666_at | Iduronate 2-sulfatase (Hunter syndrome) |
| 8 | 219032_x_at | Opsin 3 (encephalopsin, panopsin) |
| 9 | 206637_at | G protein-coupled receptor 105 |
| 10 | 217853_at | Tensin-like SH2 domain-containing 1 |
| 11 | 209500_x_at | Tumor necrosis factor (ligand) superfamily, member 13 |
| 12 | 202565_s_at | Supervillin |
| 13 | 206148_at | Interleukin 3 receptor, alpha (low affinity) |
| 14 | 204049_s_at | Chromosome 6 open reading frame 56 |
| 15 | 41858_at | FGF receptor activating protein 1 |
| 16 | 213020_at | Golgi SNAP receptor complex member 1 |
| 17 | 203940_s_at | KIAA1036 |
| 18 | 213134_x_at | BTG family, member 3 |
| 19 | 213178_s_at | Mitogen-activated protein kinase 8 interacting protein 3 |
| 20 | 211133_x_at | Leukocyte immunoglobulin-like receptor, subfamily B. member 3 |
| 21 | 204951_at | Ras homolog gene family, member H |
| 22 | 213479_at | Neuronal pentraxin II |
| 23 | 201042_at | AL031651.33 from clone RPS-1054A22 on chromosome 20q11.22-12 Contains the TGM2 gene for transglutaminase 2 (C polypeptide, protein-glutamine-gamma-glutamyltransferase), a novel gene, a putative novel gene, ESTs, STSs, GSSs and a CpG island, complete sequence |
| 24 | 201195_s_at | AB018009.1\| L-type amino acid transporter 1, complete cds |
| 25 | 201212_at | D55696.1\| cysteine protease, complete cds |
| 26 | 201249_at | NM_006516.1 solute carrier family 2 (facilitated glucose transporter), member 1 (SLC2A1) |
| 27 | 201250_s_at | NM_006516.1\| solute carrier family 2 (facilitated glucose transporter), member 1 (SLC2A1), |
| 28 | 201445_at | NM_001839.1\| calponin 3, acidic (CNN3), |
| 29 | 201621_at | NM_005380.1\| neuroblastoma, suppression of tumorigenicity 1 (NBL1) |
| 30 | 201910_at | BF213279.1\| cDNA clone IMAGE:4070203 5', mRNA sequence |
| 31 | 201911_s_at | NM_005766.1\| FERM, RhoGEF (ARHGEF) and pleckstrin domain protein 1 (chondrocyte-derived) (FARP1), |
| 32 | 201934_at | N92524.1\|N92524 zb28d02.s1 Soares_parathyroid_tumor_NbHPA Homo sapiens cDNA clone IMAGE:304899 3', |
| 33 | 202154_x_at | NM_006086.1\| tubulin, beta, 4 (TUBB4), |
| 34 | 202219_at | NM_005629.1\| solute carrier family 6 (neurotransmitter transporter, creatine), member 8 (SLC6A8), |
| 35 | 202242_at | NM_004615.1\| transmembrane 4 superfamily member 2 (TM4SF2), |
| 36 | 202285_s_at | J04152.1\|HUMGA733A Human gastrointestinal tumor-associated antigen GA733-1 protein gene, complete cds, clone 05516 |
| 37 | 202286_s_at | J04152.1\|HUMGA733A Human gastrointestinal tumor-associated antigen GA733-1 protein gene, complete cds, clone 05516 |
| 38 | 202287_s_at | NM_002353.1\| tumor-associated calcium signal transducer 2 (TACSTD2), |
| 39 | 202310_s_at | NM_000088.1\| collagen, type I, alpha 1 (COL1A1), |
| 40 | 202311_s_at | NM_000088.1\| collagen, type I, alpha 1 (COL1A1), |
| 41 | 202312_s_at | NM_000088.1\| collagen, type I, alpha 1 (COL1A1), |
| 42 | 202411_at | NM_005532.1\| interferon, alpha-inducible protein 27 (IFI27), |
| 43 | 202458_at | NM_007173.1\| protease, serine, 23 (SPUVE), |
| 44 | 202468_s_at | NM_003798.1\| catenin (cadherin-associated protein), α-like 1 (CTNNAL1), |
| 45 | 202478_at | NM_021643.1\| tribbles homolog 2 (Drosophila) (TRIB2), |
| 46 | 202481_at | NM_004753.1\| short-chain dehydrogenase/reductase 1 (SDR1), |
| 47 | 202712_s_at | NM_020990.2\| creatine kinase, mitochondrial 1 (ubiquitous) (CKMT1), |
| 48 | 202723_s_at | AW117498.1\| Soares_NFL_T_GBC_S1cDNA clone IMAGE:2605098 3' similar to gb:U02368 PAIRED BOX PROTEIN PAX-3 |
| 49 | 202724_s_at | NM_002015.2\| forkhead box O1A (rhabdomyosarcoma) (FOXOIA), |
| 50 | 202759_s_at | BE879367.1\| cDNA clone IMAGE:3887262 5', |
| 51 | 202760_s_at | NM_007203.1\| A kinase (PRKA) anchor protein 2 (AKAP2), |
| 52 | 202789_at | AL022394.3\| clone RP3-511B24 on chromosome 20q11.2-12 Contains the 3' end of the TOP1 gene for topoisomerase (DNA) I, the PLCG1 gene for phospholipase C gamma 1, gene KIAA0395 for a possible homeobox protein, a 60S Ribosomal Protein L23A (RPL23A) pseudogene, ESTs, STSs, GSSs and a CpG island, |
| 53 | 202834_at | NM_000029.1\| angiotensinogen (serine (or cysteine) proteinase inhibitor, clade A (alpha-1 antiproteinase, antitrypsin), member 8) (AGT), |
| 54 | 202860_at | NM_014856.1\| KIAA0476 gene product (KIAA0476), |
| 55 | 202889_x_at | T62571.1\| Stratagene lung (#937210) cDNA clone IMAGE:79729 3', |
| 56 | 202890_at | T62571.1\| Stratagene lung (#937210) cDNA clone IMAGE:79729 3', |
| 57 | 202947_s_at | NM_002101.2\| glycophorin C (Gerbich blood group) (GYPC), transcript variant 1, |
| 58 | 202949_s_at | NM_001450.1\| four and a half LIM domains 2 (FHL2), |
| 59 | 203139_at | NM_004938.1\| death-associated protein kinase 1 (DAPK1), |
| 60 | 203287_at | NM_005558.1\|ladinin 1 (LAD1), |
| 61 | 203886_s_at | NM_001998.1\| fibulin 2 (FBLN2), |
| 62 | 204014_at | NM_001394.2\| dual specificity phosphatase 4 (DUSP4), |
| 63 | 204015_s_at | BC002671.1\| dual specificity phosphatase 4, clone MGC:3713 IMAGE:3605895 |
| 64 | 204082_at | NM_006195.1\| pre-B-cell leukemia transcription factor 3 (PBX3), |
| 65 | 204141_at | NM_001069.1\| tubulin, beta polypeptide (TUBB), |
| 66 | 204351_at | NM_005980.1\| S 100 calcium binding protein P (S100P), |
| 67 | 204361_s_at | AB014486.1\| mRNA for RA70, |
| 68 | 204362_at | NM_003930.1\| src family associated phosphoprotein 2 (SCAP2), |
| 69 | 204416_x_at | NM_001645.2\| apolipoprotein C-I (APOC1), |
| 70 | 204642_at | NM_001400.2\| endothelial differentiation, sphingolipid G-protein-coupled receptor, 1 (EDG1), |
| 71 | 204694_at | NM_001134.1\| alpha-fetoprotein (AFP), |
| 72 | 204698_at | NM_002201.2\| interferon stimulated gene (20kD) (ISG20), |
| 73 | 204729_s_at | NM_004603.1\| syntaxin 1A (brain) (STX1A), |
| 74 | 204777_s_at | NM_002371.2\| mal, T-cell differentiation protein (MAL), transcript variant a, |
| 75 | 204863_s_at | BE856546.1\| Soares_NSF_F8_9W_OT_PA_P_S1 cDNA clone Image:3299420 3' similar to SW:IL6B_human P40189 interleukin 6 receptor β chain precursor |
| 76 | 204864_s_at | NM_002184.1\| Homo sapiens interleukin 6 signal transducer (gp130, oncostatin M receptor) (IL6ST), mRNA |
| 77 | 204881_s_at | NM_003358.1\| UDP-glucose ceramide glucosyltransferase (UGCG), |
| 78 | 204885_s_at | NM_005823.2\| mesothelin (MSLN), transcript variant 1, |
| 79 | 204950_at | NM_014959.1\| caspase recruitment domain family, member 8 (CARD8), |
| 80 | 204955_at | NM_006307.1\| sushi-repeat-containing protein, X chromosome (SRPX), |
| 81 | 204966_at | NM_001703.1\| brain-specific angiogenesis inhibitor 2 (BAI2), |
| 82 | 204989_s_at | BF305661.1\| NIH_MGC_17 cDNA clone IMAGE:4139065 5', |
| 83 | 204990_s_at | NM_000213.1\| integrin, beta 4 (ITGB4), |
| 84 | 205108_s_at | NM_000384.1\| apolipoprotein B (including Ag(x) antigen) (APOB), |
| 85 | 205336_at | NM_002854.1\| parvalbumin (PVALB), |
| 86 | 205453_at | NM_002145.1\| homeo box B2 (HOXB2), |
| 87 | 205600_x_at | AI052747.1\| Soares_total_fetus_Nb2HF8_9w Homo sapiens cDNA clone IMAGE:1676553 3' similar to gb:M92299 homeobox protein HOX-B5 |
| 88 | 205601_s_at | NM_002147.1\| homeo box B5 (HOXB5), |
| 89 | 205608_s_at | U83508.1\| angiopoietin-1 mRNA, complete cds |
| 90 | 205609_at | NM_001146.1\| angiopoietin 1 (ANGPT1), |
| 91 | 206289_at | NM_002141.1\| homeo box A4 (HOXA4), |
| 92 | 206298_at | NM_021226.1\| Rho GTPase activating protein 2 (RhoGAP2), |
| 93 | 206674_at | NM_004119.1\| fms-related tyrosine kinase 3 (FLT3), |
| 94 | 207111_at | NM_001974.1\| egf-like module containing, mucin-like, hormone receptor-like sequence 1 (EMR1), |
| 95 | 207826_s_at | NM_002167.1\| inhibitor of DNA binding 3, dominant negative helix-loop-helix protein (ID3) |
| 96 | 207935_s_at | NM_002274.1\| keratin 13 (KRT13), |
| 97 | 208130_s_at | NM_030984.1\| thromboxane A synthase 1 (platelet, cytochrome P450, family 5, subfamily A) (TBXAS1), transcript variant TXS-II, |
| 98 | 208414_s_at | NM_002146.1\| homeo box B3 (HOXB3), |
| 99 | 208621_s_at | NM_003379.2\| villin 2 (ezrin) (VIL2), |
| 100 | 208622_s_at | NM_003379.2\| villin 2 (ezrin) (VIL2), |
| 101 | 208623_s_at | J05021.1\|HUMVIL2 Human cytovillin 2 (VIL2), complete cds |
| 102 | 208977_x_at | BC004188.1\| tubulin, beta, 2, clone MGC:2826 IMAGE:2964559, |
| 103 | 209119_x_at | AV703465.1\| cDNA clone ADBCHG08 5', |
| 104 | 209120_at | AV703465.1\| cDNA clone ADBCHG08 5', |
| 105 | 209122_at | BC005127.1\| adipose differentiation-related protein, clone MGC:10598 IMAGE:3844174, |
| 106 | 209191_at | BC002654.1\| tubulin beta MGC4083, mRNA (cDNA clone MGC:4083 IMAGE:3605559), |
| 107 | 209209_s_at | AW469573.1\| hd29e09.x1 Soares_NFL_T_GBC_S1 cDNA IMAGE:2910952 3' similar to TR:Q14840 Q14840 mitogen inducible gene MIG-2 |
| 108 | 209210_s_at | Z24725.1\| mitogen inducible gene mig-2, |
| 109 | 209227_at | AU158251.1\| PLACE1 cDNA clone PLACE1011740 3', |
| 110 | 209228_x_at | U42349.1\| N33 mRNA, complete cds |
| 111 | 209239_at | M55643.1 \|HUMNFKB34 Human factor KBF1 mRNA, complete |
| 112 | 209270_at | L25541.1 \|HUMLAMB1K laminin S B3 chain (LAMB3) mRNA, complete cds |
| 113 | 209289_at | AI700518.1\| we37d09.x1 NCI_CGAP_Lu24 cDNA clone IMAGE:2343281 3', |
| 114 | 209309_at | D90427.1\|HUMZA2G zinc-alpha2-glycoprotein precursor, complete cds |
| 115 | 209324_s_at | BF304996.1\|601888511F1 NIH_MGC_17 cDNA clone IMAGE:4122242 5', |
| 116 | 209325_s_at | U94829.1\| retinally abundant regulator of G-protein signaling hRGS-r (hRGS-r) |
| 117 | 209372_x_at | BF971587.1\|602239834F1 NIH_MGC_46 cDNA clone IMAGE:4328385 5', |
| 118 | 209443_at | J02639.1\|HUMCINHP plasma serine protease (protein C) inhibitor |
| 119 | 209560_s_at | U15979.1\| Human (dlk) mRNA, |
| 120 | 209679_s_at | BC003379.1\| hypothetical protein from clone 643, mRNA (cDNA clone MGC:5115 IMAGE:2984805), |
| 121 | 209730_at | U38276.1\| semaphorin III family homolog |
| 122 | 209810_at | J02761.1 \|HUMPSPBA pulmonary surfactant-associated protein B (SP-B) |
| 123 | 210139_s_at | L03203.1\|HUMGAS3X peripheral myelin protein 22 (GAS3) |
| 124 | 210347_s_at | AF080216.1\| C2H2-type zinc-finger protein |
| 125 | 210510_s_at | AF145712.1\| soluble neuropilin-1 |
| 126 | 210615_at | AF280547.1\| neuropilin-1 soluble isoform 11 (NRP1) alternatively spliced |
| 127 | 210854_x_at | U 17986.1\| GABA/noradrenaline transporter |
| 128 | 211000_s_at | AB015706.1\| for gp130 of the rheumatoid arthritis antigenic peptide-bearing soluble form (gp130-RAPS |
| 129 | 211535_s_at | M60485.1 \|HUMFGFAA fibroblast growth factor receptor |
| 130 | 211743_s_at | BC005929.1 proteoglycan 2, bone marrow (natural killer cell activator, eosinophil granule major basic protein), (cDNA clone MGC:14537 IMAGE:4043815), |
| 131 | 211915_s_at | U83110.1\|HUMTUB4Q02 beta-tubulin (TUB4q) |
| 132 | 212012_at | AF200348.1\| melanoma-associated antigen MG50 |
| 133 | 212013_at | AF200348.1\| melanoma-associated antigen MG50 |
| 134 | 212283_at | AF016903.1\| agrin precursor |
| 135 | 212285_s_at | AF016903.1\| agrin precursor |
| 136 | 212298_at | BE620457.1\| 601483690F1 NIH_MGC_69 cDNA clone IMAGE:3886055 5', |
| 137 | 212382_at | AK021980.1\| cDNA FLJ11918 fis, clone HEMBB1000272 |
| 138 | 212385_at | AK021980.1\| cDNA FLJ11918 fis, clone HEMBB1000272 |
| 139 | 212386_at | AK021980.1\| cDNA FLJ11918 fis, clone HEMBB1000272 |
| 140 | 212387_at | AK021980.1\| cDNA FLJ11918 fis, clone HEMBB1000272 |
| 141 | 212531_at | NM_005564.1\| lipocalin 2 (oncogene 24p3) (LCN2), |
| 142 | 212570_at | AL573201.2\| Placenta COT 25-Normalized cDNA clone CSODI043YM01 3' |
| 143 | 212573_at | AL573201.2\| Placenta COT 25-Normalized cDNA clone CSODI043YM01 3' |
| 144 | 212664_at | AL567012.3\| Fetal Brain clone CS0DF028YP03 3-PRIME, |
| 145 | 212740_at | BF740111.1\| 7n12e08.x1 NCI_CGAP_Brn23 Homo sapiens cDNA clone IMAGE:3564398 3' similar to TR:Q99570 Q99570 ADAPTOR PROTEIN |
| 146 | 212771_at | AU150943.1\| NT2RP2 cDNA clone NT2RP2003984 3', |
| 147 | 213147_at | NM_018951.1\| homeo box A10 (HOXA10), |
| 148 | 213150_at | NM_018951.1\|homeo box A10 (HOXA10), |
| 149 | 213231_at | L19267.1\|HUMDNA59A Homo sapiens 59 protein mRNA, 3' end |
| 150 | 213338_at | BF062629.1\| 7h62h07.x1 NCI_CGAP_Co16 clone IMAGE:3320605 3', |
| 151 | 213423_x_at | AI884858.1\| w185f06.x1 NCI_CGAP_Brn25 cDNA clone IMAGE:2431715 3' similar to TR:Q14911 Q14911 N33 PROTEIN FORM 1. [2] TR:Q14912 ;, |
| 152 | 213539_at | NM_000732.1\| CD3D antigen, delta polypeptide (TiT3 complex) (CD3D) |
| 153 | 213553_x_at | W79394.1\| Soares_fetal_heart_NbHH19W clone IMAGE:346956 3' similar to gb:X00570 APOLIPOPROTEIN C-I PRECURSOR (HUMAN); |
| 154 | 213843_x_at | AW276522.1\|AW276522 xr15a02.x1 NCI_CGAP_Lu28 cDNA clone IMAGE:2760170 3' similar to SW:NTCS_HUMAN P53796 SODIUM- AND CHLORIDE-DEPENDENT CREATINE TRANSPORTER 2 ;, |
| 155 | 214292_at | AA808063.1\|AA808063 of50f09.s1 NCI_CGAP_CNS1 cDNA clone IMAGE:1427657 3' similar to gb:X53587 Integrin β 4 subunit precursor |
| 156 | 214433_s_at | NM_003944.1\| selenium binding protein 1 (SELENBP1), |
| 157 | 214696_at | AF070569.1 \|AF070569 clone 24659 |
| 158 | 214721_x_at | AL162074.1\| DKFZp762L106 (from clone DKFZp762L106) |
| 159 | 215073_s_at | AL554245.3\| PLACENTA COT 25-Normalized cDNA clone CSODI082YA02 5' |
| 160 | 215446_s_at | L16895.1\|HUMX7LOX lysyl oxidase (LOX) gene, exon 7 |
| 161 | 215812_s_at | U41163.1\| creatine transporter (SLC6A10) gene, partial cds |
| 162 | 216641_s_at | U58994.1\|HSU58994 ladinin (LAD) gene, complete cds |
| 163 | 216899_s_at | AC003999.2\| PAC clone RP5-1139P1 from 7, complete sequence |
| 164 | 217013_at | AC004522.3\| PAC clone RP4-604G5 from 7, complete sequence |
| 165 | 217014_s_at | AC004522.3\| PAC clone RP4-604G5 from 7, complete sequence |
| 166 | 217293_at | AF209975.1\| tissue-type aorta |
| 167 | 217410_at | AK021586.1\| cDNA FLJ11524 fis, clone HEMBA1002547, highly similar to agrin precursor mRNA |
| 168 | 217419_x_at | AK021586.1\| cDNA FLJ11524 fis, clone HEMBA1002547, highly similar to agrin precursor mRNA |
| 169 | 217875_s_at | NM_020182.1\| transmembrane, prostate androgen induced RNA (TMEPAI), |
| 170 | 218062_x_at | NM_012121.2\| Cdc42 effector protein 4; binder of Rho GTPases 4 (CEP4), |
| 171 | 218368_s_at | NM_016639.1\| tumor necrosis factor receptor superfamily, member 12A (TNFRSF12A), |
| 172 | 218801_at | NM_020121.2\| UDP-glucose ceramide glucosyltransferase-like 2 (UGCGL2), |
| 173 | 219008_at | NM_021925.1\| hypothetical protein FLJ21820 (FLJ21820), |
| 174 | 219218_at | NM_024696.1\| hypothetical protein FLJ23058 (FLJ23058), |
| 175 | 219452_at | NM_022355.1\| dipeptidase 2 (DPEP2), |
| 176 | 220386_s_at | NM_019063.1\| echinoderm microtubule associated protein like 4 (EML4), |
| 177 | 220416_at | NM_024837.1\| ATPase, Class I, type 8B, member 4 (ATP8B4), |
| 178 | 220558_x_at | NM_005705.1\| pan-hematopoietic expression (PHEMX), |
| 179 | 221009_s_at | NM_016109.1\| angiopoietin-like 4 (ANGPTL4), |
| 180 | 221942_s_at | AI719730.1\| as92b12.x1 Barstead aorta HPLRB6 cDNA clone IMAGE:2353055 3' similar to gb:X66534_cds1 Guanylate cyclase soluble, α 3 chain |
| 181 | 222108_at | AC004010.1\| BAC clone GS1-99H8 from 12, complete sequence |
| 182 | 222192_s_at | AK021672.1 sapiens cDNA FLJ11610 fis, clone HEMBA1003985 |
| 183 | 222193_at | AK021672.1\| cDNA FLJ11610 fis, clone HEMBA1003985 |
| 184 | 33304_at | U88964.1\|HSU88964 HEM45 mRNA, complete cds |
| 185 | 33768_at | L19267.1\|HUMDNA59A 59 protein mRNA, 3' end |
| 186 | 35666_at | U38276.1\|HSU38276 semaphorin III family homolog mRNA, complete cds |
| 187 | 37004_at | J02761.1 \|HUMPSPBA pulmonary surfactant-associated protein B (SP-B) |
| 188 | 37005_at | D28124.1 \|HUMZAPII mRNA for unknown product, complete cds |
| 189 | 37996_s_at | L08835.1\|HUMDMKIN DMR-N9, partial cds; and myotonic dystrophy kinase (DM kinase) gene, |

### Reference list

5,242,974
5,561,071
5,384,261
5,571,639
5,405,783
5,593,839
5,412,087
5,599,695
5,424,186
5,624,711
5,429,807
5,658,734
5,436,327
5,700,637
5,445,934
6,004,755
5,472,672
6,133,305
5,527,681
6,218,114
5,529,756
6,218,122
5,532,128
6,271,002
5,545,531
6,271,210
5,554,501
6,284,764
5,556,752
6,306,897
Adnane et al. (2000) "Inhibition of farnesyltransferase increases TGFbeta type II receptor expression and enhances the responsiveness of human cancer cells to TGFbeta" Oncogene 19:5525-5533
Albertson (1984) EMBO J. 3:1227-1234
Alsina et al. (2003) "Farnesyltransferase inhibitor FTI-R115777 is well tolerated, induces stabilization of disease, and inhibits farnesylation and oncogenic/tumor survival pathways in patients with advanced multiple myeloma" Eur. J. Haematol. 70:269
Brunner et al. (2003) "Farnesyltransferase inhibitors: an overview of the results of preclinical and clinical investigations" Cancer Res. 63:5656-5668
Bullinger et al. (2004) "Use of gene-expression profiling to identify prognostic subclasses in adult acute myeloid leukemia" N. Engl. J. Med. 350:1605-1616
Carr et al. (1991) "Interaction of the regulatory subunit (RII) of cAMP-dependent protein kinase with RII-anchoring proteins occurs through an amphipathic helix binding motif' J. Biol. Chem. 266:14188-14192
Chang et al. (2003) "Gene expression profiling for the prediction of therapeutic response to docetaxel in patients with breast cancer" Lancet 362:362-369
Cheok et al. (2003) "Treatment-specific changes in gene expression discriminate in vivo drug response in human leukemia cells" Nat. Genet. 34:85-90
Cortes et al. (2003) "Efficacy of the farnesyl transferase inhibitor R115777 in chronic myeloid leukemia and other hematologic malignancies" Blood 101:1692-1697
Cox et al. (2002) "Farnesyltransferase inhibitors: promises and realities" Curr. Opin. Pharmacol. 2:388-393
Debernardi et al. (2003) "Genome-wide analysis of acute myeloid leukemia with normal karyotype reveals a unique pattern of homeobox gene expression distinct from those with translocation-mediated fusion events" Genes Chrom. Cancer 37:149-158
End et al. (2001) "Characterization of the antitumor effects of the selective farnesyl protein transferase inhibitor R115777 in vivo and in vitro" Cancer Res. 61:131-137
EP 430,402
Esteva et al. (2003) "Phase III study of weekly docetaxel and trastuzumab for patients with HER-2-overexpressing metastatic breast cancer" J Clin Oncol. 20:1800-1808.
Foisner et al. (1991) "Protein kinase A- and protein kinase C-regulated interaction of plectin with lamin B and vimentin" Proc. Natl. Acad. Sci. USA 88:3812-3816
Gene Therapy Protocols, edited by Paul D. Robbins, Human press, Totowa NJ, 1996
Harousseau et al. (2003) "Zarnestra□ (R115777) in patients with relapsed or refractory acute myelogenous leukemia (AML): results of a multicenter phase 2 study" Blood 102:176a. Abstract 614
Hofmann et al. (2002) "Relation between resistance of Philadelphia-chromosome-positive acute lymphoblastic leukaemia to the tyrosine kinase inhibitor STI571 and gene-expression profiles: a gene-expression study" Lancet 359:481-486
Jensen et al. (2001) "Estrogen receptors and proliferation markers in primary and recurrent breast cancer" Proc Natl Acad Sci U S A. 98:15197-15202.
Jiang et al. (2000) "The phosphoinositide 3-OH kinase/AKT2 pathway as a critical target for farnesyltransferase inhibitor-induced apoptosis" Mol. Cell. Biol. 20:139-148
Johnston et al. (2003) "Phase II study of the efficacy and tolerability of two dosing regimens of the farnesyl transferase inhibitor, R115777, in advanced breast cancer" J. Clin. Oncol. 21:2492-2499
Kallioniemi (1992) Proc. Natl. Acad. Sci. USA 89:5321-5325
Karp et al. (2001) "Clinical and biologic activity of the farnesyltransferase inhibitor R115777 in adults with refractory and relapsed acute leukemias: a phase 1 clinical-laboratory correlative trial" Blood 97:3361-3369
Kohlmann et al. (2003) " Molecular characterization of acute leukemias by use of microarray technology" Genes Chrom. Cancer Genes Chrom. Cancer 37:396-405
Kurzrock et al. (2004) "Phase II study of R115777, a farnesyl transferase inhibitor, in myelodysplastic syndrome" J. Clin. Oncol. 22:1287-1292
Lancet et al. (2003) "Farnesyltransferase inhibitors in hematologic malignancies: new horizons in therapy" Blood 102:3880-3889
Li et al. (2002) "The hematopoiesis-specific GTP-binding protein RhoH is GTPase deficient and modulates activities of other Rho GTPases by an inhibitory function" Mol. Cell. Biol. 22:1158-1171
Lynch et al. (2004) "Activating mutations in the epidermal growth factor receptor underlying responsiveness of non-small-cell lung cancer to Gefitinib" N. Engl. J. Med. 350:2129-2139
McLean et al. (2004) "Pharmacogenomic analysis of cytogenetic response in chronic myeloid leukemia patients treated with imatinib" Clin. Cancer Res. 10:155-165
Methods in Molecular Biology, Vol. 33: In Situ Hybridization Protocols, Choo, ed., Humana Press, Totowa, NJ (1994)
Morgan et al. (2001) "Cell-cycle-dependent activation of mitogen-activated protein kinase kinase (MEK-1/2) in myeloid leukemia cell lines and induction of growth inhibition and apoptosis by inhibitors of RAS signaling" Blood 97:1823-1834
Na et al. (2004) "Inhibition of farnesyltransferase prevents collagen-induced arthritis by down-regulation of inflammatory gene expression through suppression of p21(ras)-dependent NF-kappaB activation" J Immunol. 173:1276-1283.
Okutsu et al. (2002) "Prediction of chemosensitivity for patients with acute myeloid leukemia, according to expression levels of 28 genes selected by genome-wide complementary DNA microarray analysis" Mol. Cancer Ther. 1:1035-1042
Paez et al. (2004) "EGFR mutations in lung cancer: correlation with clinical response to gefitinib therapy activating mutations in the epidermal growth factor receptor underlying responsiveness of non-small-cell lung cancer to Gefitinib" Science 304:1497-1500
Pasqualucci et al. (2001) "Hypermutation of multiple proto-oncogenes in B-cell diffuse large-cell lymphomas" Nature 412:341-346
Pinkel (1988) " Fluorescence in situ Hybridization with Human Chromosome-Specific
Libraries: Detection of Trisomy 21 and Translocations of Chromosome 4" Proc Natl Acad Sci USA 85:9138-9142
Pinkel et al. (1998) "High resolution analysis of DNA copy number variation using comparative genomic hybridization to microarrays" Nature Genetics 20:207-211
Rao et al. (2004) "Phase III Double-Blind Placebo-Controlled Study of Farnesyl Transferase Inhibitor R115777 in Patients With Refractory Advanced Colorectal Cancer" J Clin Oncol 22:3950-3957
Reiss et al. (1990) "Inhibition of purified p21ras farnesyl:protein transferase by Cys'AAX tetrapeptides" Cell 62:81-88
Reuter et al. (2000) "Targeting the Ras signaling pathway: a rational, mechanism-based treatment for hematologic malignancies?" Blood 96:1655-1669
Sahai et al. (2002) "RHO-GTPases and cancer" Nat. Rev. Cancer 2:133-142
Schoch et al. (2002) "Acute myeloid leukemias with reciprocal rearrangements can be distinguished by specific gene expression profiles" Proc. Natl. Acad. Sci. USA 99:10008-10013
Sterpetti et al. (1999) "Activation of the Lbc Rho exchange factor proto-oncogene by truncation of an extended C terminus that regulates transformation and targeting" Mol. Cell. Biol. 19:1334-1345
Strachan and Read, Human Molecular Genetics, 1996
Takada et al. (2004) "Protein farnesyltransferase inhibitor (SCH 66336) abolishes NF-kappaB activation induced by various carcinogens and inflammatory stimuli leading to suppression of NF-kappaB-regulated gene expression and up-regulation of apoptosis" J Biol Chem. 279:26287-26299.
Testa et al. (2004) "Interleukin-3 receptor in acute leukemia" Leukemia 18:219-226
Thomas et al. (2001) "R115777, a farnesyl transferase inhibitor (FTI), has significant anti-leukemia activity in patients with chronic myeloid leukemia (CML)" Blood 98
Tijssen (1993) Laboratory Techniques in Biochemistry and Molecular Biology, Vol. 24: Hybridization With Nucleic Acid Probes, Elsevier, NY
Toksoz et al. (1994) "Novel human oncogene lbc detected by transfection with distinct homology regions to signal transduction products" Oncogene 9:621-628
Valk et al. (2004) "Prognostically useful gene-expression profiles in acute myeloid leukemia" N. Engl. J. Med. 350:1617-1628
Van Cutsem et al. (2004) "Phase III trial of gemcitabine plus tipifarnib compared with gemcitabine plus placebo in advanced pancreatic cancer" J. Clin. Oncol. 22:1430-1438
Yagi et al. (2003) "Identification of a gene expression signature associated with pediatric AML prognosis" Blood 102:1849-1856
Zhang et al. (2002) "Farnesyltransferase inhibitors reverse Ras-mediated inhibition of Fas gene expression" Cancer Res. 62:450-458
Zheng et al. (1995) "Direct involvement of the small GTP-binding protein Rho in lbc oncogene function" J. Biol. Chem. 270:9031-9034

## Claims

1. A method of assessing prognosis (survival/outcome) in an acute myeloid leukemia (AML) patient comprising the steps of
(a) obtaining a biological sample from the patient; and
(b) measuring the expression levels in the sample of genes selected from the group consisting of those encoding mRNA:
i) corresponding to SEQ ID NOs: 1 or 5-22; or
ii) recognized by the probe sets selected from the group consisting of psid numbers corresponding to SEQ ID NOs: 1 or 5-22 as depicted in Table 7
wherein the gene expression levels above or below pre-determined cut-off levels are indicative of AML prognosis.

2. A method of determining the status of an acute myeloid leukemia (AML) patient comprising the steps of
(a) obtaining a biological sample from the patient; and
(b) measuring the expression levels in the sample of genes selected from the group consisting of those encoding mRNA:
i) corresponding to SEQ ID NOs: 1 or 5-22; or
ii) recognized by the probe sets selected from the group consisting of psid numbers corresponding to SEQ ID NOs: 1 or 5-22 as depicted in Table 7
wherein the gene expression levels above or below pre-determined cut-off levels are indicative of the AML status.

3. A method of determining treatment protocol for an acute myeloid leukemia (AML) patient comprising the steps of
(a) obtaining a biological sample from the patient; and
(b) measuring the expression levels in the sample of genes selected from the group consisting of those encoding mRNA:
i) corresponding to SEQ ID NOs: 1 or 5-22; or
ii) recognized by the probe sets selected from the group consisting of psid numbers corresponding to SEQ ID NOs: 1 or 5-22 as depicted in Table 7
wherein the gene expression levels above or below pre-determined cut-off levels are sufficiently indicative of likelihood that the patient will respond to treatment to enable a physician to determine the degree and type of therapy recommend.

4. The use of adjuvant therapy on a patient who has been identified as likely to respond to said therapy by the method comprising the steps of:
(a) obtaining a biological sample from the patient; and
(b) measuring the expression levels in the sample of genes selected from the group consisting of those encoding mRNA:
i) corresponding to SEQ ID NOs: 1 or 5-22; or
ii) recognized by the probe sets selected from the group consisting of psid numbers corresponding to SEQ ID NOs: 1 or 5-22 as depicted in Table 7
wherein the gene expression levels above or below pre-determined cut-off levels are indicate a likelihood that the patient will respond to treatment.

5. The method of claim 1, 2 or 3, or the use of claim 4 wherein the patient has relapsed or refractory AML.

6. The method of claim 1, 2 or 3, or the use of claim 4 wherein the SEQ ID NOs. are 1, 5 and 6.

7. The method of claim 1, 2 or 3, or the use of claim 4 further comprising measuring the expression level of at least one gene constitutively expressed in the sample.

8. The method of claim 1, 2 or 3, or the use of claim 4 wherein the comparison of expression patterns is conducted with pattern recognition methods.

9. The method or use of claim 8 wherein the pattern recognition methods include the use of a Cox proportional hazards analysis.

10. The method of claim 1, 2 or 3, or the use of claim 4 wherein the pre-determined cut-off levels are at least 1.5-fold over- or under- expression in the sample relative to benign cells or normal tissue.

11. The method of claim 1, 2 or 3, or the use of claim 4 wherein the pre-determined cut-off levels have at least a statistically significant p-value over-expression in the sample having metastatic cells relative to benign cells or normal tissue.

12. The method or use of claim 11 wherein the p-value is less than 0.05.

13. The method of claim 1, 2 or 3, or the use of claim 4 wherein gene expression is measured on a microarray or gene chip.

14. The method or use of claim 13 wherein the microarray is a cDNA array or an oligonucleotide array.

15. The method or use of claim 13 wherein the microarray or gene chip further comprises one or more internal control reagents.

16. The method of claim 1, 2 or 3, or the use of claim 4 wherein gene expression is determined by nucleic acid amplification conducted by polymerase chain reaction (PCR) of RNA extracted from the sample.

17. The method or use of claim 16 wherein said PCR is reverse transcription polymerase chain reaction (RT-PCR).

18. The method or use of claim 17, wherein the RT-PCR further comprises one or more internal control reagents.

19. The method of claim 1, 2 or 3, or the use of claim 4 wherein gene expression is detected by measuring or detecting a protein encoded by the gene.

20. The method or use of claim 19 wherein the protein is detected by an antibody specific to the protein.

21. The method of claim 1, 2 or 3, or the use of claim 4 wherein gene expression is detected by measuring a characteristic of the gene.

22. The method or use of claim 21 wherein the characteristic measured is selected from the group consisting of DNA amplification, methylation, mutation and allelic variation.

23. A method of generating an acute myeloid leukemia (AML) patient prognostic patient report comprising the steps of:
(a) obtaining a biological sample from the patient;
(b) measuring gene expression of the sample;
(c) applying a Relapse Hazard Score to the results of step b.; and
(d) using the results obtained in step c. to generate the report.

24. The method of claim 23 wherein the report contains an assessment of patient outcome and/or probability of risk relative to the patient population.

25. The method of claim 23 wherein the patient is relapsed or refractory.

26. A patient report generated by the method according to claim 23.

27. A composition comprising at least one probe set selected from the group consisting of: SEQ ID NOs: 1-22; or the psid numbers corresponding to SEQ ID NOs: 1 and 5-22 as depicted in Table 7.

28. A kit for conducting an assay to determine acute myeloid leukemia prognosis in a biological sample comprising: materials for detecting isolated nucleic acid sequences, their complements, or portions thereof of a combination of genes selected from the group consisting of those encoding mRNA:
(a) corresponding to SEQ ID NOs: 1 and 5-22; or
(b) recognized by the probe sets selected from the group consisting of psid numbers corresponding to SEQ ID NOs: 1 and 5-22 as depicted in Table 7.

29. The kit of claim 28 wherein the SEQ ID NOs. are 1, 5 and 6.

30. The kit of claim 29 further comprising reagents for conducting a microarray analysis.

31. The kit of claim 28 further comprising a medium through which said nucleic acid sequences, their complements, or portions thereof are assayed.

32. Articles for assessing acute myeloid leukemia prognosis in a biological sample comprising: materials for detecting isolated nucleic acid sequences, their complements, or portions thereof of a combination of genes selected from the group consisting of those encoding mRNA:
(a) corresponding to SEQ ID NOs: 1 and 5-22; or
(b) recognized by the probe sets selected from the group consisting of psid numbers corresponding to SEQ ID NOs: 1 and 5-22 as depicted in Table 7.

33. The articles of claim 32 wherein the SEQ ID NOs. are 1, 5 and 6.

34. The articles of claim 32 further comprising reagents for conducting a microarray analysis.

35. The articles of claim 32 further comprising a medium through which said nucleic acid sequences, their complements, or portions thereof are assayed.

36. A microarray or gene chip for performing the method of claim 1, 2, 3, or 4.

37. The microarray of claim 36 comprising isolated nucleic acid sequences, their complements, or portions thereof of a combination of genes selected from the group consisting of those encoding mRNA:
(a) corresponding to SEQ ID NOs: 1 and 5-22; or
(b) recognized by the probe sets selected from the group consisting of psid numbers corresponding to SEQ ID NOs: 1 and 5-22 as depicted in Table 7
where the combination is sufficient to characterize acute myeloid leukemia status or prognosis in a biological sample.

38. The microarray of claim 36 wherein the measurement or characterization is at least 1.5-fold over- or under-expression.

39. The microarray of claim 36 wherein the measurement provides a statistically significant p-value over- or under-expression.

40. The microarray of claim 36 wherein the p-value is less than 0.05.

41. The microarray of claim 36 comprising a cDNA array or an oligonucleotide array.

42. The microarray of claim 36 further comprising one or more internal control reagents.

43. A diagnostic/prognostic portfolio comprising isolated nucleic acid sequences, their complements, or portions thereof of a combination of genes selected from the group consisting of those encoding mRNA:
(a) corresponding to SEQ ID NOs: 1 and 5-22; or
(b) recognized by the probe sets selected from the group consisting of psid numbers corresponding to SEQ ID NOs: 1 and 5-22 as depicted in Table 7
where the combination is sufficient to characterize acute myeloid leukemia status or prognosis in a biological sample.

44. The portfolio of claim 43 wherein the measurement or characterization is at least 1.5-fold over- or under-expression.

45. The portfolio of claim 43 wherein the measurement provides a statistically significant p-value over- or under-expression.

46. The portfolio of claim 43 wherein the p-value is less than 0.05.


SEQUENCE LISTING

<110> VERIDEX, LLC
Raponi, Mitch

<120> METHODS FOR ASSESSING PATIENTS WITH ACUTE MYELOID LEUKEMIA

<130> P040673EP

<160> 189

<170> PatentIn version 3.3

<210> 1
<211> 1737
<212> DNA
<213> Homo sapiens

<400> 1
gtggtcacct tccccagcta gaaagccttc tttttatatc aacttccttt taattatgaa
60

gtattacaat caagcataaa gacataataa atgttaacat ctttcctttg tgtctgttta
120

aaaaatgttt cttattacag aaaatttcaa atagatttaa agtagagaaa tgcacgtgat
180

gaaaccccat gcacttgcca ttccatttca atagttatta cttcacggaa gagttggcaa
240

tgatgagaac atgtcaaaca cctggaaatt cctgtctcat tcaacagact cactaaataa
300

aatcagcaag gtcaatgagt caacagaatc acttactgat gagggtacag acatgaatga
360

aggacaacta ctgggagact ttgagattga gtccaaacag ctggaagcag agtcttggag
420

tcggataata gacagcaagt ttctaaaaca gcaaaagaaa gatgtggtca aacggcaaga
480

agtaatatat gagttgatgc agacagagtt tcatcatgtc cgcactctca agatcatgag
540

tggtgtgtac agccagggga tgatggcaga tctgcttttt gagcagcaga tggtagaaaa
600

gctgttcccc tgtttggatg agctgatcag tatccatagc caattcttcc agaggattct
660

ggagcggaag aaggagtctc tggtggataa aagtgaaaag aactttctca tcaagaggat
720

aggggatgtg cttgtaaatc agttttcagg tgagaatgca gaacgtttaa agaagacata
780

tggcaagttt tgtgggcaac ataaccagtc tgtaaactac ttcaaagacc tttatgccaa
840

ggataagcgt tttcaagcct ttgtaaagaa gaagatgagc agttcagttg ttagaaggct
900

tggaattcca gagtgcatat tgcttgtaac tcagcggatt accaagtacc cagttttatt
960

ccaaagaata ttgcagtgta ccaaagacaa tgaagtggag caggaagatc tagcacagtc
1020

cttgagcctg gtgaaggatg tgattggagc tgtagacagc aaagtggcaa gttatgaaaa
1080

gaaagtgcgt ctcaatgaga tttatacaaa gacagatagc aagtcaatca tgaggatgaa
1140

gagtggtcag atgtttgcca aggaagattt gaaacggaag aagcttgtac gtgatgggag
1200

tgtgtttctg aagaatgcag caggaaggtt gaaagaggtt caagcagttc ttctcactga
1260

cattttagtt ttccttcaag aaaaagacca gaagtacatc tttgcatcat tggaccagaa
1320

gtcaacagtg atctctttaa agaagctgat tgtgagagaa gtggcacatg aggagaaagg
1380

tttattcctg atcagcatgg ggatgacaga tccagagatg gtagaagtcc atgccagctc
1440

caaagaggaa cgaaacagct ggattcagat cattcaggac acaatcaaca ccctttccgg
1500

gaatggatgg aggtgcttta actgatgcga gtctcacaga gtcctatttc agcaccagct
1560

ttattggagt caatggattt ggaagccctg tagaaacaaa gtatcccttg atgcagtaca
1620

ctctggttgt cttccaacgt cttcgggtct gcacctctgg attcagcaat accaaaaaca
1680

ccggccccca aacttcccac ctacttgaac caataaactt cctcttgtgt ttaaact
1737


<210>  2
<211>  21
<212>  DNA
<213>  Homo sapiens

<400>  2
ggtcagatgt ttgccaagga a
21


<210>  3
<211>  24
<212>  DNA
<213>  Homo sapiens

<400>  3
tcttcagaaa cacactccca tcac
24


<210>  4
<211>  20
<212>  DNA
<213>  Homo sapiens

<400>  4

tgaaacggaa gaagcttgta
20

<210> 5
<211> 5496
<212> DNA
<213> Homo sapiens

<400> 5
attcagccgg tgcgcgcggc ggcgggaggc agtggctggg gagtcccgtc gacgctctgt
60

tccgagagcg tgccccggac cgccagctca gaacaggggc agccgtgtag ccgaacggaa
120

gctgggagca gccgggactg gtggcccgcg cccgagctcc gcaggcggga agcaccctgg
180

atttgggaag tcccgggagc agcgcggcgg cacctccctc acccaagggg ccgcggcgac
240

ggtcacgggg cgcggcgcca ccgtgagcga cccaggccag gattctaaat agacggccca
300

ggctcctcct ccgcccgggc cgcctcacct gcgggcattg ccgcgccgcc tccgccggtg
360

tagacggcac ctgcgccgcc ttgctcgcgg gtctccgccc ctcgcccacc ctcactgcgc
420

caggcccagg cagctcacct gtactggcgc gggctgcgga agcctgcgtg agccgaggcg
480

ttgaggcgcg gcgcccacgc cactgtcccg agaggacgca ggtggagcgg gcgcggcttc
540

gcggaacccg gcgccggccg ccgcagtggt cccagcctac accgggttcc ggggacccgg
600

ccgccagtgc ccggggagta gccgccgccg tcggctgggc accatgaaca gcagcagcgc
660

caacatcacc tacgccagtc gcaagcggcg gaagccggtg cagaaaacag taaagccaat
720

cccagctgaa ggaatcaagt caaatccttc caagcggcat agagaccgac ttaatacaga
780

gttggaccgt ttggctagcc tgctgccttt cccacaagat gttattaata agttggacaa
840

actttcagtt cttaggctca gcgtcagtta cctgagagcc aagagcttct ttgatgttgc
900

attaaaatcc tcccctactg aaagaaacgg aggccaggat aactgtagag cagcaaattt
960

cagagaaggc ctgaacttac aagaaggaga attcttatta caggctctga atggctttgt
1020

attagttgtc actacagatg ctttggtctt ttatgcttct tctactatac aagattatct
1080

agggtttcag cagtctgatg tcatacatca gagtgtatat gaacttatcc ataccgaaga
1140

ccgagctgaa tttcagcgtc agctacactg ggcattaaat ccttctcagt gtacagagtc
1200

tggacaagga attgaagaag ccactggtct cccccagaca gtagtctgtt ataacccaga
1260

ccagattcct ccagaaaact ctcctttaat ggagaggtgc ttcatatgtc gtctaaggtg
1320

tctgctggat aattcatctg gttttctggc aatgaatttc caagggaagt taaagtatct
1380

tcatggacag aaaaagaaag ggaaagatgg atcaatactt ccacctcagt tggctttgtt
1440

tgcgatagct actccacttc agccaccatc catacttgaa atccggacca aaaattttat
1500

ctttagaacc aaacacaaac tagacttcac acctattggt tgtgatgcca aaggaagaat
1560

tgttttagga tatactgaag cagagctgtg cacgagaggc tcaggttatc agtttattca
1620

tgcagctgat atgctttatt gtgccgagtc ccatatccga atgattaaga ctggagaaag
1680

tggcatgata gttttccggc ttcttacaaa aaacaaccga tggacttggg tccagtctaa
1740

tgcacgcctg ctttataaaa atggaagacc agattatatc attgtaactc agagaccact
1800

aacagatgag gaaggaacag agcatttacg aaaacgaaat acgaagttgc cttttatgtt
1860

taccactgga gaagctgtgt tgtatgaggc aaccaaccct tttcctgcca taatggatcc
1920

cttaccacta aggactaaaa atggcactag tggaaaagac tctgctacca catccactct
1980

aagcaaggac tctctcaatc ctagttccct cctggctgcc atgatgcaac aagatgagtc
2040

tatttatctc tatcctgctt caagtacttc aagtactgca ccttttgaaa acaacttttt
2100

caacgaatct atgaatgaat gcagaaattg gcaagataat actgcaccga tgggaaatga
2160

tactatcctg aaacatgagc aaattgacca gcctcaggat gtgaactcat ttgctggagg
2220

tcacccaggg ctctttcaag atagtaaaaa cagtgacttg tacagcataa tgaaaaacct
2280

aggcattgat tttgaagaca tcagacacat gcagaatgaa aaatttttca gaaatgattt
2340

ttctggtgag gttgacttca gagacattga cttaacggat gaaatcctga cgtatgtcca
2400

agattcttta agtaagtctc ccttcatacc ttcagattat caacagcaac agtccttggc
2460

tctgaactca agctgtatgg tacaggaaca cctacatcta gaacagcaac agcaacatca
2520

ccaaaagcaa gtagtagtgg agccacagca acagctgtgt cagaagatga agcacatgca
2580

agttaatggc atgtttgaaa attggaactc taaccaattc gtgcctttca attgtccaca
2640

gcaagaccca caacaatata atgtctttac agacttacat gggatcagtc aagagttccc
2700

ctacaaatct gaaatggatt ctatgcctta tacacagaac tttatttcct gtaatcagcc
2760

tgtattacca caacattcca aatgtacaga gctggactac cctatgggga gttttgaacc
2820

atccccatac cccactactt ctagtttaga agattttgtc acttgtttac aacttcctga
2880

aaaccaaaag catggattaa atccacagtc agccataata actcctcaga catgttatgc
2940

tggggccgtg tcgatgtatc agtgccagcc agaacctcag cacacccacg tgggtcagat
3000

gcagtacaat ccagtactgc caggccaaca ggcattttta aacaagtttc agaatggagt
3060

tttaaatgaa acatatccag ctgaattaaa taacataaat aacactcaga ctaccacaca
3120

tcttcagcca cttcatcatc cgtcagaagc cagacctttt cctgatttga catccagtgg
3180

attcctgtaa ttccaagccc aattttgacc ctggtttttg gattaaatta gtttgtgaag
3240

gattatggaa aaataaaact gtcactgttg gacgtcagca agttcacatg gaggcattga
3300

tgcatgctat tcacaattat tccaaaccaa attttaattt ttgcttttag aaaagggagt
3360

ttaaaaatgg tatcaaaatt acatatacta cagtcaagat agaaagggtg ctgccacgga
3420

gtggtgaggt accgtctaca tttcacatta ttctgggcac cacaaaatat acaaaacttt
3480

atcagggaaa ctaagattct tttaaattag aaaatattct ctatttgaat tatttctgtc
3540

acagtaaaaa taaaatactt tgagttttga gctactggat tcttattagt tccccaaata
3600

caaagttaga gaactaaact agtttttcct atcatgttaa cctctgcttt tatctcagat
3660

gttaaaataa atggtttggt gctttttata aaaagataat ctcagtgctt tcctccttca
3720

ctgtttcatc taagtgcctc acattttttt ctacctataa cactctagga tgtatatttt
3780

atataaagta ttctttttct tttttaaatt aatatctttc tgcacacaaa tattatttgt
3840

gtttcctaaa tccaaccaat tttcattaat tcaggcatat tttaactcca ctgcttacct
3900

actttcttca ggtaaaaggg caaataatga tcgaaaaaat aattatttat tacataattt
3960

agttgtttct agactataaa tgttgctatg tgccttatgt tgaaaaaatt taaaagtaaa
4020

atgtctttcc aaattatttc ttaattatta taaaaatatt aagacaatag cacttaaatt
4080

cctcaacagt gttttcagaa gaaataaata taccactctt tacctttatt gatatctcca
4140

tgatgatagt tgaatgttgc aatgtgaaaa atctgctgtt aactgcaacc ttgtttatta
4200

aattgcaaga agctttattt ctagcttttt aattaagcaa agcacccatt tcaatgtgta
4260

taaattgtct ttaaaaactg ttttagacct ataatccttg ataatatatt gtgttgactt
4320

tataaatttc gcttcttaga acagtggaaa ctatgtgttt ttctcatatt tgaggagtgt
4380

taagattgca gatagcaagg tttggtgcaa agtattgtaa tgagtgaatt gaatggtgca
4440

ttgtatagat ataatgaaca aaattatttg taagatattt gcagtttttc attttaaaaa
4500

gtccatacct tatatatgca cttaatttgt tggggcttta catactttat caatgtgtct
4560

ttctaagaaa tcaagtaatg aatccaactg cttaaagttg gtattaataa aaagacaacc
4620

acatagttcg tttaccttca aactttaggt ttttttaatg atatactgat cttcattacc
4680

aataggcaaa ttaatcaccc taccaacttt actgtcctaa catggacttt caaaaagaaa
4740

aaatgacacc atcttttatt cttttttttt tttttttttt gagagagagt cttactctgc
4800

cgcccaaact ggagtgcagt ggcacaatct tggctcactg caacctctac ctcctgggtt
4860

caagtgattc tcttgcctca gcctcccgag ttgctgggat tgcgggcatg gtggcgtgag
4920

cctgtagtcc tagctactcg ggaggctgag gcaggagaat agcctgaacc tgggaatcgg
4980

aggttgcagg gccaagatcg ccccactgca ctccagcctg gcaatagacc gagctccgtc
5040

tccaaaaaaa aaaatacaat ttttatttct tttacttttt ttagtaagtt aatgtatata
5100

aaaatggctt cggacaaaat atctctgagt tctgtgtatt ttcagtcaaa actttaaacc
5160

tgtagaatca atttaagtgt tgaaaaaaat ttgtctgaaa catttcataa tttgtttcca
5220

gcatgaggta tctaaggatt tagaccagag gtctagatta atactctatt tttacattta
5280

aaccttttat tataagtctt acataaacca tttttgttac tctcttccac atgttactgg
5340

ataaattgtt tagtggaaaa taggcttttt aatcatgaat atgatgacaa tcagttatac
5400

agttataaaa ttaaaagttt gaaaagcaat attgtatatt tttatctata taaaataact
5460

aaaatgtatc taagaataat aaaatcacgt taaacc
5496

<210> 6
<211> 1051
<212> DNA
<213> Homo sapiens

<220>
<221> misc_feature
<222> (23)..(26)

<220>
<221> misc_feature
<222> (23)..(26)

<220>
<221> misc_feature
<222> (23)..(23)
<223> n is a, c, g, or t

<220>
<221> misc_feature
<222> (26)..(26)
<223> n is a, c, g, or t

<400> 6
agtaaatcag gtaggaaatc acnttngwyt twkcttccag tagtcattac caccttttac
60

tgcacaattc acaagcatgt tttcctggtg aattggactg aaaattacat tttgacaact
120

ttttttcctt ttatccccaa cttttgccag aaagcagaaa aatgctctat ttttataaag
180

aaagattaaa ttctccaatg atattttaaa aaatatcaac ctacatgccc tttagaatgt
240

taaataacaa tgactatttt aagactcgaa gacccactat tttgagtatt ttttatagac
300

tttaaatact gggttttttt cctccttcaa tctcaggctt ttctccatct tttaagcggc
360

ctctgtkact cccttttgtc cataggtgtt gcgtgcctct catctgtggg gaagtattat
420

ttaataaaat ttatgttaca ggataacttt attttaatgg gcatgggctt gtctatacac
480

aaaggtatgt atattttcat tataaaaaac cagtttaaaa ttttttctta ttttaattgt
540

ttgaaatttt tctagagcca aagaagctct ttaaagaagt tgtttcttcc aaagaacaaa
600

gccaggttaa tgacattcaa ttctaaatga tacattggaa ttgtgccttt tctaccacac
660

tggattaaat aaacttgtca aaatatggtt ttgtcatttt ctgagacact tggtaatttg
720

ctgttctttc tttcatgtgc ccgttagtac atttggccaa ctgamcdkct gtaacaggga
780

ggtctcatgt ttgttagtag atacgcaggt aaggraactt gaattcatcc tctcctcggc
840

tcaggtcttt gcttttcttt taatatatgc atataaaata gtaggcattt gattctgcsa
900

aggcactaga ctacttgaaw ttaacattct gtccagaggg ataataccag gctttccttt
960

tccttcatct gttgtaagtt tcaggtcctt gacagaaata gctggactat tccaaatttg
1020

ctcagtgcat caaatgtgma aagggagcgg a
1051


<210> 7
<211> 1098
<212> DNA
<213> Homo sapiens

<400> 7
ggccgcgtcg aagccgaaat gccgccaccc cggaccggcc gaggccttct ctggctgggt
60

ctggttctga gctccgtctg cgtcgccctc ggatccgaaa cgcaggccaa ctcgaccaca
120

gatgctctga acgttcttct catcatcgtg gatgacctgc gcccctccct gggctgttat
180

ggggataagc tggtgaggtc cccaaatatt gaccaactgg catcccacag cctcctcttc
240

cagaatgcct ttgcgcagca agcagtgtgc gccccgagcc gcgtttcttt cctcactggc
300

aggagacctg acaccacccg cctgtacgac ttcaactcct actggagggt gcacgctgga
360

aacttctcca ccatccccca gtacttcaag gagaatggct atgtgaccat gtcggtggga
420

aaagtctttc accctggtac tgctccatgt ccagagtctg ggttctcttg gtttgtggtg
480

tctgaatcca gcattcccat cctggggatg gggctgtctt tgcagagccc tcttctggct
540

gggcgagtcc ctcgctagtc agtgcttcct ttctaaaaaa ctgacttgtc aacccagcca
600

cgttttcacc caaagtgaaa aagggtagaa agagctttgc ttcctttcag aaaccactga
660

gggtgtgctg ttgggtcttt cagctcctcg ggtggtaggg aggacacagg ctggggaggt
720

ggcagtgttg ggtggagtcc agctcagggc cccaccctct cccctgcagg gtacctgtca
780

gtaaacctag gggtggggtg aggacacctg agggcctccc gtgtggccag cattgctgtt
840

gctgacttat tgcccaatga gggggctgtg cttaggtagg ggctgctatc cactctggaa
900

attaagtcag aaagatgagt gtaatctggt acccaggatc tatagggtcc cagagacgga
960

cattccttac ctcaaatgct gcctgataaa ctggctctct ttaatgccaa tatggggata
1020

gtgaaaagca aacaaacttt aaaacttttt atttatagaa gtaatgcatg agtatttgag
1080

aaaaaaaaaa aaaaaaaa
1098

<210> 8
<211> 2110
<212> DNA
<213> Homo sapiens

<400> 8
cgagccccgc cgcaagctga gcgcctccgc ccgccaggcg cgccggcgcc gggccatgta
60

ctcggggaac cgcagcggcg gccacggcta ctgggacggc ggcggggccg cgggcgctga
120

ggggccggcg ccggcgggga cactgagccc cgcgcccctc ttcagccccg gcacctacga
180

gcgcctggcg ctgctgctgg gctccattgg gctgctgggc gtcggcaaca acctgctggt
240

gctcgtcctc tactacaagt tccagcggct ccgcactccc actcacctcc tcctggtcaa
300

catcagcctc agcgacctgc tggtgtccct cttcggggtc acctttacct tcgtgtcctg
360

cctgaggaac ggctgggtgt gggacaccgt gggctgcgtg tgggacgggt ttagcggcag
420

cctcttcggg attgtttcca ttgccaccct aaccgtgctg gcctatgaac gttacattcg
480

cgtggtccat gccagagtga tcaatttttc ctgggcctgg agggccatta cctacatctg

540

gctctactca ctggcgtggg caggagcacc tctcctggga tggaacaggt acatcctgga
600

cgtacacgga ctaggctgca ctgtggactg gaaatccaag gatgccaacg attcctcctt
660

tgtgcttttc ttatttcttg gctgcctggt ggtgcccctg ggtgtcatag cccattgcta
720

tggccatatt ctatattcca ttcgaatgct tcgttgtgtg gaagatcttc agacaattca
780

agtgatcaag attttaaaat atgaaaagaa actggccaaa atgtgctttt taatgatatt
840

caccttcctg gtctgttgga tgccttatat cgtgatctgc ttcttggtgg ttaatggtca
900

tggtcacctg gtcactccaa caatatctat tgtttcgtac ctctttgcta aatcgaacac
960

tgtatacaat ccagtgattt atgtcttcat gatcagaaag tttcgaagat cccttttgca
1020

gcttctgtgc ctccgactgc tgaggtgcca gaggcctgct aaagacctac cagcagctgg
1080

aagtgaaatg cagatcagac ccattgtgat gtcacagaaa gatggggaca ggccaaagaa
1140

aaaagtgact ttcaactctt cttccatcat ttttatcatc accagtgatg aatcactgtc
1200

agttgacgac agcgacaaaa ccattggggt ccaaagtttg atgttaatcc aagttcgtcc
1260

tttgtaggaa tgaaggatgg caacgaaagg tggggcctta aattggatgc cacttttgga
1320

ctttcatcat cctcctgaag aagaagtgtc tggaataccc gttctatgta atatcaacag
1380

aaccttgtgg tccagcagga aatccgaatt gcccatatgc tcttgggcct caggaagagg
1440

ttgaacaaaa acaaattctt ttaattcaac gggtgcttta cataatgaaa aaaccacttg
1500

tgcacacgat gggcatctaa catcatcatc ttctaatgtg ttggagattt tcatttcaaa
1560

tatatttttt aaattactct attttccaaa acacgtaatg catttttctc gaaaatacct
1620

tactgtaaaa ataactgtcg cgtacacatg tgtgaagtag ctagaacata ctgaattttt
1680

tttgtactgt tggactctat tcagtgtcat gtcctatatc tgatcaagtt atcaaggaga
1740

taattctaga atgaaaaaga aaatcctctt gttggaaaca aaagacgttt tatatgtgca
1800

gtatgacaaa gaggagtttc agagacaact ttgaatcctt gtcagcctgg agaccagcac

1860

cagaggaatc tacaaggcaa actcccatat atttgcttcc cccaaattgc tgcccctaca
1920

gactcaaagc tctttttctt tgttttgttg tttctctaaa aatttactgt tctttgtcga
1980

tgctatataa gccagggagt tctaagacgc cagctctttg agatttgctc attcccctgt
2040

atttcccaca tatatattac atatacccgc taataaattt atgtttgttt taaaaaaaaa
2100

aaaaaaaaaa
2110

<210> 9
<211> 2416
<212> DNA
<213> Homo sapiens

<400> 9
gaacagtgtt accttggagc ctacaatgag aggtatttca aaatgagtga agcatgactc
60

tcacagatga aggcctagac gcaggatctt taatggaaaa acacttgggc cacttcaaga
120

cgacaaacgc tcactgggca aaacaccttc actgaaaaga gacctcatat tatgcaaaaa
180

aaatcttaag aggcctctgc cttcagaagt tacaagatga tcaattcaac ctccacacag
240

cctccagatg aatcctgctc tcagaacctc ctgatcactc agcagatcat tcctgtgctg
300

tactgtatgg tcttcattgc gggaatccta ctcaatggag tgtcaggatg gatattcttt
360

tacgtgccca gctctaagag tttcatcatc tatctcaaga acattgttat tgctgacttt
420

gtgatgagcc tgacttttcc tttcaagatc cttggtgact caggccttgg tccctggcag
480

ctgaacgtgt ttgtgtgcag ggtctctgcc gtgctcttct acgtcaacat gtacgtcagc
540

attgtgttct ttgggctcat cagctttgac aggtattata aaattgtaaa gcctctttgg
600

acttctttca tccagtcagt gagttacagc aaacttctgt cagtgatagt atggatgctc
660

atgctcctcc ttgctgttcc aaatattatt ctcaccaacc agagtgttag ggaggttaca
720

caaataaaat gtatagaact gaaaagtgaa ctgggacgga agtggcacaa agcatcaaac
780

tacatcttcg tggccatctt ctggattgtg tttcttttgt taatcgtttt ctatactgct
840

atcacaaaga aaatctttaa gtcccacctt aagtcaagtc ggaattccac ttcggtcaaa
900

aagaaatcta gccgcaacat attcagcatc gtgtttgtgt tttttgtctg ttttgtacct
960

taccatattg ccagaatccc ctacacaaag agtcagaccg aagctcatta cagctgccag
1020

tcaaaagaaa tcttgcggta tatgaaagaa ttcactctgc tactatctgc tgcaaatgta
1080

tgcttggacc ctattattta tttctttcta tgccagccgt ttagggaaat cttatgtaag
1140

aaattgcaca ttccattaaa agctcagaat gacctagaca tttccagaat caaaagagga
1200

aatacaacac ttgaaagcac agatactttg tgagttccta ccctcttcca aagaaagacc
1260

acgtgtgcat gttgtcatct tcaattacat aacagaaatc aataagatat gtgccctcat
1320

cataaatatc atctctagca ctgccatcca atttagttca ataaaattca aatataagtt
1380

tccatgcttt tttgtaacat caaagaaaac atacccatca gtaatttctc taatactgac
1440

ctttctattc tctattaata aaaaattaat acatacaatt attcaattct attatattaa
1500

aataagttaa agtttataac cactagtctg gtcagttaat gtagaaattt aaatagtaaa
1560

taaaacacaa cataatcaaa gacaactcac tcaggcatct tctttctcta aataccagaa
1620

tctagtatgt aattgttttc aacactgtcc ttaaagacta acttgaaagc aggcacagtt
1680

tgatgaaggg ctagagagct gtttgcaata aaaagtcagg tttttttcct gatttgaaga
1740

agcaggaaaa gctgacaccc agacaatcac ttaagaaacc ccttattgat gtatttcatg
1800

gcactgcaaa ggaagaggaa tattaattgt atacttagca agaaaatttt tttttctga
1860

tagcactttg aggatattag atacatgcta aatatgtttt ctacaaagac ttacgtcatt
1920

taatgagcct ggggttctgg tgttagaata tttttaagta ggctttactg agagaaacta
1980

aatattggca tacgttatca gcaacttccc ctgttcaata gtatgggaaa aataagatga
2040

ctgggaaaaa gacacaccca caccgtagaa catatattaa tctactggcg aatgggaaag
2100

gagaccattt tcttagaaag caaataaact tgattttttt aaatctaaaa tttacattaa
2160

tgagtgcaaa ataacacata aaatgaaaat tcacacatca catttttctg gaaaacagac
2220

ggattttact tctggagaca tggcatacgg ttactgactt atgagctacc aaaactaaat
2280

tctttctctg ctattaactg gctagaagac attcatctat ttttcaaatg ttctttcaaa
2340

acatttttat aagtaatgtt tgtatctatt tcatgcttta ctgtctatat actaataaag
2400

aaatgtttta atactg
2416

<210> 10
<211> 3763
<212> DNA
<213> Homo sapiens

<400> 10
acttccaagt tctggtacaa ggcggatatt tcaagagaac aagccatcgc catgttgaag
60

gacaaggagc cgggctcatt cattgttcga gacagccatt ccttccgagg ggcttatggc
120

ctggccatga aggtggccac gcccccacct tcagtcctgc agctgaacaa gaaagctgga
180

gatttggcca atgaactcgt ccggcacttt ttgatcgagt gtaccccgaa gggagtgcgg
240

ttgaaagggt gctcgaatga accatatttc gggagcctga cggccttggt gtgccagcat
300

tccatcacgc ccttggcctt gccgtgcaag ctgcttatcc cagagagaga tccattggag
360

gaaatagcag aaagttctcc ccagacggca gccaattcag cagctgagct gttgaagcag
420

ggggcagcct gcaacgtgtg gtacttgaac tctgtggaga tggagtccct caccggccac
480

caggcgatcc agaaggccct gagcatcacc ctggtccagg agcctccacc tgtgtccaca
540

gttgtgcact tcaaggtgtc agcccagggc atcaccctga cagacaatca gaggaagctc
600

ttcttccgga ggcattaccc cgtgaacagt gtgattttct gtgccttgga cccacaagac
660

aggaagtgga tcaaagatgg cccttcctca aaagtctttg gatttgtggc ccggaagcag
720

ggcagtgcca cggataatgt gtgccacctg tttgcagagc atgaccctgt gcagcctgcc
780

agtgccattg tcaacttcgt atcaaaggtc atgattggtt ccccaaagaa ggtctgagaa
840

ctcccctccc tccctggacc caccgatgcc tctcgaagcc ctggagacag ccgttgggtg
900

agggtggggc ccccactttt taccaaacta gtaaacctga cattccaggc ccatgagggg
960

aaagaggatc ttccagctct gcaaaaacaa gaacaaacaa catcaccgtg aattggcctt
1020

tcctgaaagt gacttatctg acacatctct gtagccacat gctttttggg tagaagaagc
1080

tgggcatggg tgcaccccac cccctagggt ccccatggga aagggacatg caaggaaaca
1140

gcacagaaca cgaggtggtc cccatgtccc tggcacacta gcattctggg ggatgaggaa
1200

tccccagccc ttgaggcaga ggtgccgagt gactgccatg cttcgcccgt ccgcatgggc
1260

gcttctgtcc agctgcaccc gaggccgggg gtttccctca cctcggtctt cccaagatgg
1320

agatgctaac gaaactgaga agggggcgta tgtttgacga aggtttgtgc aagtcaggcc
1380

cttctggaac acagcagggc ctacaacgag gggcctttgc gatgggctgt gaggatgggg
1440

gtggtgggaa gaattggcca cgttagagac cccatgccac cccaccatgg tgagtgctct
1500

gtgcctcctg ctcacctgtg gtgagctggg cgagctgggc gagctgggcg agctgggctg
1560

gggagagcct gtgaggaccg agaggagaaa tgagaagaag gaacaaaaat attatttcta
1620

tgtaatttat attttactta tgccaaatta tttatgataa tttgccattg ctatactgta
1680

ccagtgtcaa atgctgcagc ctgccaagct gtgattttgt gaggcttgtc cctatgtagg
1740

atgcaccgca ggcccctggc cactgaaaga gtgtgcagtg gactgtgggt ctcccatatg
1800

cggtgccgcc caaaggtggc tttgcctcaa gcaacctacc ctgatgtttt actcattgga
1860

atgtttttcc ccgattgtgg atgacttctt ttctgatgga gagagtccag gagggatgga
1920

aaactcctgg atttaagctc agcatccccc acatgggctt ttcgatcatc ttcaggcctg
1980

aagctgcacg acctgaagtt cgcctgcatt tatcagccct tttgtgctgc tccttgccac
2040

cttggggttc ctgctgggga ccatgtgtgg ttgtggcatg tgtgagcaga agggaggatg
2100

aggaaaaaga gaagaaaccc cggtactgac aagctgtttt tgagtgccac tgtttgccat
2160

catctaagcc actgaatcaa gtgtatttca ggcttatttc aacattccaa tgccctggtt
2220

ttcctgcttg aatctgttcg tggtcaaagg tttgggggaa tttgtgaccc tggaccatcc
2280

ccagagtgaa agatggagct gggccacatc agaataaggc cttggcccca tcctctcaca
2340

gcctaggtgc tctgcaggca tactgactgt cctgattgcg atccagcccg aaattccctc
2400

ctctgctttc aaaagtcaaa tcccccattc ttaggccaca ctggtgtcac aagctcctgt
2460

cagggagctg gggtttggga atgtgctttg tgaactctgc tttaaagtga ggggccgagg
2520

aaaacttaga aacaggcaga gttggaagca gccaaatcac agtgggtgtt gtgtgtgtgt
2580

gcgtgtgtgc atgcgtgcgt gtatgcgtgt gtgaaagcag gtggaccatt ccacttttta
2640

gctcctattg atgcaccaaa ccaagtgcct catttctgtg ccaaatgttt gccttggtcg
2700

tcgtggacct ccttctctaa cttgaggtgg catgactgtc aggaggtgct ggcattttca
2760

gcagatcctc atgtgttgac cctgatgtct ttagcagagg cctctagcat ctcggttttt
2820

catccactgc aggaatgtgg ccacagggag cagaggtttg tactttcccc aagaggtcct
2880

catcctgaga cggtctctac ccatgtttaa cccaaagagt gcaggccagg ttccttatcc
2940

ttctgatgaa ggatgagaga gctcatttag aagtcagagc aaactagggt ctcagtattg
3000

agaaacgcag cctgccaggg aatcacagag acatcggggt gcccgcgatg gccctcatga
3060

agccatgcct cgacggcatt caggaagccc tgcaaacgtg ctttttgaac tcattggcca
3120

ggtgtgattt ttacacaagg taaacgtggt caagggcatc ggggaatttg ctccaagcag
3180

atagctccct ctgaggaacc aaaggaagca agtttccatg atttctgaag agctggtata
3240

ggaagtttct ttcttccttt tgtgttacat gtgcattaaa cagaacaagc tgtgtgtcat
3300

cacagattgt actgtgggct cagaaaccgt gagagagccc ccaccgtgga caccggctct
3360

atggccacag gaaaaggaac gtttccaggc attttgtctc cagggctccc gctggacagg
3420

cacgtactgc cccggggagt aaatgcggag agttcacgaa ctgtgcccaa cgcatgttat
3480

agccagggtc ctactaacta ctcagtaaaa gaacgtattg ttgtattcct ccagtgttaa
3540

gctatagcca tgttaaaagt cactgtgcat ttattctcag catcaaatac cttgtaacgt
3600

cttctctgcc ttgttagtgc atatttttac ttttctgata ctgtaaagaa tatatccagt
3660

atgtaaatga atgttctata aatcttttgt atagtcattt tctctgctcc ttaaatatca
3720

tctctattca gagtataata aaattatgaa cttggtaagc ctc
3763

<210> 11
<211> 1684
<212> DNA
<213> Homo sapiens

<400> 11
cgacggagtg ccaggagcac taacagtacc cttagcttgc tttcctcctc cctccttttt
60

attttcaagt tcctttttat ttctccttgc gtaacaacct tcttcccttc tgcaccactg
120

cccgtaccct tacccgcccc gccacctcct tgctacccca ctcttgaaac cacagctgtt
180

ggcagggtcc ccagctcatg ccagcctcat ctcctttctt gctagccccc aaagggcctc
240

caggcaacat gggggcccca gtcagagagc cggcactctc agttgccctc tggttgagtt
300

ggggggcagc tctgggggcc gtggcttgtg ccatggctct gctgacccaa caaacagagc
360

tgcagagcct caggagagag gtgagccggc tgcaggggac aggaggcccc tcccagaatg
420

gggaagggta tccctggcag agtctcccgg agcagagttc cgatgccctg gaagcctggg
480

agaatgggga gagatcccgg aaaaggagag cagtgctcac ccaaaaacag aagaatgact
540

ccgatgtgac agaggtgatg tggcaaccag ctcttaggcg tgggagaggc ctacaggccc
600

aaggatatgg tgtccgaatc caggatgctg gagtttatct gctgtatagc caggtcctgt
660

ttcaagacgt gactttcacc atgggtcagg tggtgtctcg agaaggccaa ggaaggcagg
720

agactctatt ccgatgtata agaagtatgc cctcccaccc ggaccgggcc tacaacagct
780

gctatagcgc aggtgtcttc catttacacc aaggggatat tctgagtgtc ataattcccc
840

gggcaagggc gaaacttaac ctctctccac atggaacctt cctggggttt gtgaaactgt
900

gattgtgtta taaaaagtgg ctcccagctt ggaagaccag ggtgggtaca tactggagac

960

agccaagagc tgagtatata aaggagaggg aatgtgcagg aacagaggcg tcttcctggg
1020

tttggctccc cgttcctcac ttttcccttt tcattcccac cccctagact ttgattttac
1080

ggatatcttg cttctgttcc ccatggagct ccgaattctt gcgtgtgtgt agatgagggg
1140

cgggggacgg gcgccaggca ttgttcagac ctggtcgggg cccactggaa gcatccagaa
1200

cagcaccacc atctagcggc cgctcgaggg aagcacccgc cggttggccg aagtccacga
1260

agccgccctc tgctagggaa aacccctggt tctccatgcc acacctctct ccaggtgccc
1320

tctgcctctt caccccacaa gaagccttat cctacgtcct tctctccatc tatcggaccc
1380

cagtttccat cactatctcc agagatgtag ctattatgcg cccgtctaca gggggtgccc
1440

gacgatgacg gtgccttcgc agtcaaatta ctcttcgggt cccaaggttt ggctttcacg
1500

cgctccattg ccccggcgtg gcaggccatt ccaagccctt ccgggctgga actggtgtcg
1560

gaggagcctc gggtgtatcg tacgccctgg tgttggtgtt gcctcactcc tctgagctct
1620

tctttctgat caagccctgc ttaaagttaa ataaaataga atgaatgata aaaaaaaaaa
1680

aaaa
1684


<210>  12
<211>  6719
<212>  DNA
<213>  Homo sapiens

<400>  12
tcggcgggaa gcggcgatcc tgccaccggg aggtgtggaa gagccgggta gattctggct
60

acattggaga ttggttgctt tctaaaactg aaggagaagc ccatgaagag atggtggatt
120

ctcactgagt tttgactagc ggaagaaaag agagagttca agtggatggc cttgaggact
180

tgaaaagctg agatatgatg attttgaagt catttcacat cgaagccatg atttaaatat
240

cggcgttaag atttcaacaa gaaaaactta agcttccttg gattcccacg tcaaaggaaa
300

gtttcaagct ttcagaagga gttctcactc gaagataaag aacagctcgc taaccacgaa
360

agaggaatcg atgctcagct tttagttgca cttcctaaag ttgcagaatt aagacaaatc
420

tttgaaccaa agaagaaaga attcttagaa atgaaaagaa aagaaagaat tgccaggcgc
480

ctggaaggga ttgaaaatga cactcagccc atcctcttgc agagctgcac aggattggtg
540

actcaccgcc tgctggagga agacacccct cgatacatga gagccagcga ccctgccagc
600

ccccacatcg gccgatcaaa tgaagaggag gaaacttctg attcttctct agaaaagcaa
660

actcgatcca aatactgcac agaaacctcc ggtgtccacg gtgactcacc ctatggttcg
720

ggtaccatgg acacccacag tctggagtcc aaagccgaaa gaattgcaag gtacaaagca
780

gaaagaaggc gacagctggc agagaagtat gggctgactc tggatcccga ggccgactcc
840

gagtatttat cccgctatac caagtccagg aaggagcctg atgctgtcga gaagcgggga
900

ggaaaaagtg acaaacagga agagtcaagc agagatgcga gttctctgta ccccgggacc
960

gagacgatgg ggctcaggac ctgtgccggt gaatccaagg actatgccct ccatgtgggt
1020

gacggctctt ccgacccgga ggtgctgctg aacatagaaa accaaagacg aggtcaagag
1080

ctgagtgcca cccggcaggc ccatgacctg tccccagcag ccgagagttc ctcgaccttc
1140

tctttctctg ggcgagactc ctccttcact gaagtgccac ggtcccccaa gcacgcccac
1200

agctcctccc tgcagcaggc agcctcccgg agcccctcct ttggtgaccc acagctatcc
1260

cctgaggccc gacccaggtg cacttcacat tcagaaacgc caactgtcga tgatgaagaa
1320

aaggtggatg aacgagccaa gctgagcgtc gccgccaaga ggttgctttt cagggagatg
1380

gaaaaatctt ttgatgaaca aaatgttcca aagcgacgct caagaaacac agctgtggag
1440

cagaggctac gccgtctgca ggacaggtcc ctcacccagc ccatcaccac tgaagaggtg
1500

gtcatcgcag ccacattgca ggcctctgct caccaaaagg ccttagccaa ggaccagaca
1560

aatgagggca aagagcttgc tgagcaagga gaacctgatt cctccactct aagcttggcc
1620

gaaaagttgg ccttgtttaa caaattgtcc cagccagtct caaaagcgat ttctacccgg
1680

aacagaatag acacgagaca gaggagaatg aacgctcgct atcaaactca gccagtcaca
1740

ctgggagagg tggagcaggt gcagagtgga aagctcattc ctttctcacc tgccgtgaac
1800

acatcagtgt ctaccgtagc atccacggtt gctccaatgt atgccggaga tcttcgcaca
1860

aagccacctc ttgaccacaa tgcaagtgcc actgactata agttttcttc ttcaatagaa
1920

aattcggact ctccagttag aagcattctg aaatcgcaag cttggcagcc tttggtagag
1980

ggtagcgaga acaagggaat gttgagagaa tatggagaga cagaaagcaa gagagctttg
2040

acaggtcgag acagtgggat ggagaagtat gggtcctttg aggaagcaga agcatcctac
2100

cccatcctga accgagccag ggaaggagac agccataagg aatctaaata tgctgttccc
2160

agaagaggaa gcctggaacg ggcgaaccct cccatcaccc acctcgggga tgaaccgaag
2220

gaattttcca tggctaaaat gaatgcacaa ggaaacttgg acttgaggga caggctgccc
2280

tttgaagaga aggtggaggt ggagaatgtt atgaaaagga agttttcact aagagcggca
2340

gagttcgggg agcccacttc cgagcagacg gggacagctg ctgggaaaac tattgctcaa
2400

accacagccc ccgtgtcctg gaagccccag gattcttcgg aacagccaca ggagaagctc
2460

tgcaagaatc catgtgcgat gtttgctgct ggagagatca aaacgccgac aggggagggc
2520

cttcttgact cacccagcaa aaccatgtct attaaagaaa gattggcact gttgaagaaa
2580

agcggggagg aagattggag aaacagactc agcaggaggc aggagggcgg caaggcgccg
2640

gccagcagcc tgcacaccca ggaagcaggg cggtccctca tcaagaagcg ggtcacagaa
2700

agtcgagaga gccaaatgac gattgaggag aggaagcagc tcatcactgt gagagaggag
2760

gcctggaaga cgagaggcag aggagcggcc aacgactcga cccagttcac tgtggctggc
2820

aggatggtga agaaaggttt ggcgtcacct actgccataa ccccagtagc ctcagccatt
2880

tgcggtaaaa caagaggcac cacacccgtt tccaaacccc tggaagatat cgaagccaga
2940

ccagatatgc agttagaatc ggacctgaag ttggacaggc tggaaacctt tctaagaagg
3000

ctgaataaca aagttggcgg gatgcacgaa acggtgctca ctgtcaccgg caaatctgtg
3060

aaggaggtga tgaagccaga tgatgatgaa acctttgcca aattttaccg cagcgtggat
3120

tataatatgc caagaagtcc tgtggagatg gatgaggact tcgatgtcat tttcgatcct
3180

tatgcaccca aattgacgtc ttccgtggcc gagcacaagc gggcagttag gcccaagcgc
3240

cgggttcagg cctccaaaaa ccccctgaaa atgctggcgg caagagaaga tctccttcag
3300

gaatacactg agcagagatt aaacgttgcc ttcatggagt caaagcggat gaaagtagaa
3360

aagatgtctt ccaactccaa cttctcagaa gtcaccctgg cgggtttagc cagtaaagaa
3420

aacttcagca acgtcagcct gcggagcgtc aacctgacgg aacagaactc taacaacagc
3480

gccgtgccct acaagaggct gatgctgttg cagattaaag gaagaagaca tgtgcagacc
3540

aggctggtgg aacctcgagc ttcggcgctc aacagtgggg actgcttcct cctgctctct
3600

ccccactgct gcttcctgtg ggtaggagag tttgcaaacg tcatagaaaa ggcgaaggcc
3660

tcagaacttg caactttaat tcagacaaag agggaacttg gttgtagagc tacttatatc
3720

caaaccattg aagaaggaat taatacacac actcatgcag ccaaagactt ctggaagctt
3780

ctgggtggcc aaaccagtta ccaatctgct ggagacccaa aagaagatga actctatgaa
3840

gcagccataa tagaaactaa ctgcatttac cgtctcatgg atgacaaact tgttcctgat
3900

gacgactact gggggaaaat tccgaagtgc tcccttctgc aacccaaaga ggtactggtg
3960

tttgattttg gtagtgaagt ttacgtatgg catgggaaag aagtcacatt agcacaacga
4020

aaaatagcat ttcagctggc aaagcactta tggaatggaa cctttgacta tgagaactgt
4080

gacatcaatc ccctggatcc tggagaatgc aatccgctta tccccagaaa aggacagggg
4140

cggcccgact gggcgatatt tgggagactt actgaacaca atgagacgat tttgttcaaa
4200

gagaagtttc tggattggac ggaactgaag agatcgaatg agaagaaccc cggggaactt
4260

gcccagcaca aggaagaccc caggactgat gtcaaggcat acgatgtgac acggatggtg
4320

tccatgcccc agacgacagc aggcaccatc ctggacggag tgaacgtcgg ccgtggctat
4380

ggcctggtgg aaggacacga caggaggcag tttgagatca ccagcgtttc cgtggatgtc
4440

tggcacatcc tggaattcga ctatagcagg ctccccaaac aaagcatcgg gcagttccat
4500

gagggggatg cctatgtggt caagtggaag ttcatggtga gcacggcagt gggaagtcgc
4560

cagaagggag agcactcggt gagggcagcc ggcaaagaga agtgcgtcta cttcttctgg
4620

caaggccggc actccaccgt gagtgagaag ggcacgtcgg cgctgatgac ggtggagctg
4680

gacgaggaaa gggggcccca ggtccaggtt ctccagggaa aggagccccc ctgtttcctg
4740

cagtgtttcc agggggggat ggtggtgcac tcggggaggc gggaagagga agaagaaaat
4800

gtgcaaagtg agtggcggct gtactgcgtg cgtggagagg tgcccgtgga agggaatttg
4860

ctggaagtgg cctgtcactg tagcagcctg aggtccagaa cttccatggt ggtgcttaac
4920

gtcaacaagg ccctcatcta cctgtggcac ggatgcaaag cccaggccca cacgaaggag
4980

gtcggaagga ccgctgcgaa caagatcaag gaacaatgtc ccctggaagc aggactgcat
5040

agtagcagca aagtcacaat acacgagtgt gatgaaggct ccgagccact cggattctgg
5100

gatgccttag gaaggagaga caggaaagcc tacgattgca tgcttcaaga tcctggaagt
5160

tttaacttcg cgccccgcct gttcatcctc agcagctcct ctggggattt tgcagccaca
5220

gagtttgtgt accctgcccg agccccctct gtggtcagtt ccatgccctt cctgcaggaa
5280

gatctgtaca gcgcgcccca gccagcactt ttccttgttg acaatcacca cgaggtgtac
5340

ctctggcaag gctggtggcc catcgagaac aagatcactg gttccgcccg catccgctgg
5400

gcctccgacc ggaagagtgc gatggagact gtgctccagt actgcaaagg aaaaaatctc
5460

aagaaaccag cccccaagtc ttaccttatc cacgctggtc tggagcccct gacattcacc
5520

aatatgtttc ccagctggga gcacagagag gacatcgctg agatcacaga gatggacacg
5580

gaagtttcca atcagatcac cctcgtggaa gacgtcttag ccaagctctg taaaaccatt
5640

tacccgctgg ccgacctcct ggccaggcca ctcccggagg gggtcgatcc tctgaagctt
5700

gagatctatc tcaccgacga agacttcgag tttgcactag acatgacgag ggatgaatac
5760

aacgccctgc ccgcctggaa gcaggtgaac ctgaagaaag caaaaggcct gttctgagtg
5820

gggagacgcc agaggagcct cacggtcacg tccaacaaca ccactgcacc agggaaatgg
5880

atatatattt ttggactggt gtttttcaca aagtattttt caatcagagt tttcagaacc
5940

tgacattgtt aaagatactg cttgtcccgg agttgtgtat tttgtaaatg ttcaagggaa
6000

ctgtttggaa acttctttcc accattcagg aggttatcag aattaataaa agtatctgtt
6060

atgtgcactt aagccgcagc tgctatagat agcactgcct tcttgttcca gctaggcaat
6120

gccttttttt tttttttgga agcagttctc tttataaagt gttattttga tagtttgtgg
6180

attctaaaat atatatatat ttatataaac accatataag tcaaatatgt atttaacaaa
6240

gcaatatgta ttcattcact ttcaagattt gttttggtgt caaaataaca tgaaaaggta
6300

gatggagttg cttctgttga attagctctg ccaccaatat gtatcttcat acacgtttgg
6360

aaatgtttcc tgcagcatta ggtatgactt gttctgagta ctgcttccgg tgctaaaatg
6420

aacaaagaat ttgtacttaa tggcatggac tctggagaat ctatgcgaat caacctttct
6480

accttaatat ctccccaaaa atgtatagtg ccttgttttt atgtacagtt tatatacaga
6540

aaagtttgct ctgcattttt gatgatggtt tggaacatta tctacaattt tactctcaaa
6600

tagtcaaaat aaaaacatct caatttctaa taccggttgt aaacaaacag tacacatgtc
6660

attttgtgat ataggactcc caaataaaag tatcagaata aacacaacaa ttaactggt
6719

<210> 13
<211> 1460
<212> DNA
<213> Homo sapiens

<400> 13
   gcacacggga agatatcaga aacatcctag gatcaggaca ccccagatct tctcaactgg
   60
   aaccacgaag gctgtttctt ccacacagca ctttgatctc catttaagca ggcacctctg
   120
   tcctgcgttc cggagctgcg ttcccgatgg tcctcctttg gctcacgctg ctcctgatcg
   180
   ccctgccctg tctcctgcaa acgaaggaag atccaaaccc accaatcacg aacctaagga
   240
   tgaaagcaaa ggctcagcag ttgacctggg accttaacag aaatgtgacc gatatcgagt
   300
   gtgttaaaga tgccgactat tctatgccgg cagtgaacaa tagctattgc cagtttggag
   360
   caatttcctt atgtgaagtg accaactaca ccgtccgagt ggccaaccca ccattctcca
   420
   cgtggatcct cttccctgag aacagtggga agccttgggc aggtgcggag aatctgacct
   480
   gctggattca tgacgtggat ttcttgagct gcagctgggc ggtaggcccg ggggcccccg
   540
   cggacgtcca gtacgacctg tacttgaacg ttgccaacag gcgtcaacag tacgagtgtc
   600
   ttcactacaa aacggatgct cagggaacac gtatcgggtg tcgtttcgat gacatctctc
   660
   gactctccag cggttctcaa agttcccaca tcctggtgcg gggcaggagc gcagccttcg
   720
   gtatcccctg cacagataag tttgtcgtct tttcacagat tgagatatta actccaccca
   780
   acatgactgc aaagtgtaat aagacacatt cctttatgca ctggaaaatg agaagtcatt
   840
   tcaatcgcaa atttcgctat gagcttcaga tacaaaagag aatgcagcct gtaatcacag
   900
   aacaggtcag agacagaacc tccttccagc tactcaatcc tggaacgtac acagtacaaa
   960
   taagagcccg ggaaagagtg tatgaattct tgagcgcctg gagcaccccc cagcgcttcg
   1020
   agtgcgacca ggaggagggc gcaaacacac gtgcctggcg gacgtcgctg ctgatcgcgc
   1080
   tggggacgct gctggccctg gtctgtgtct tcgtgatctg cagaaggtat ctggtgatgc
   1140
   agagactctt tccccgcatc cctcacatga aagaccccat cggtgacagc ttccaaaacg
   1200
   acaagctggt ggtctgggag gcgggcaaag ccggcctgga ggagtgtctg gtgactgaag
   1260
   tacaggtcgt gcagaaaact tgagactggg gttcagggct tgtgggggtc tgcctcaatc
   1320
   tccctggccg ggccaggcgc ctgcacagac tggctgctgg acctgcgcac gcagcccagg
   1380
   aatggacatt cctaacgggt ggtgggcatg ggagatgcct gtgtaatttc gtccgaagct
   1440
   gccaggaaga agaacagaac
   1460

<210> 14
<211> 4318
<212> DNA
<213> Homo sapiens

<400> 14
gcttcccggc tgccaggcta cagaactcgc ctcgccactc ctgagacatg gacaacgccg
60

ttgacggact ggacaaagct tctatagcaa actcagatgg ccccacagca ggttcccaaa
120

cacctccctt caaaagaaag gggaaactat ccaccattgg taaaatcttt aagccttgga
180

aatggaggaa aaagaagacc agcgacaaat ttagagaaac ctcggcagta ttagaaagga
240

agatatccac acgacaaagt agagaggagc tgataagaag gggagtgctt aaggaattgc
300

ctgatcaaga tggagatgta acagttaact ttgaaaattc aaacgggcac atgataccca
360

tcggagagga atctacccga gaggaaaatg tagtaaagtc tgaagaaggt aatggctctg
420

tatctgaaaa aacaccacct ctggaggaac aggcagaaga taagaaagag aacactgaaa
480

accactctga aacaccggca gctcctgctc tacctccttc tgctcctcct aagcctagat
540

ccaaacctaa acccaaaaaa tcacctgtgc ctccgaaagg ggccactgct ggggccagcc
600

acaaaggtga tgaagtgcct cccattaaaa aaaataccaa ggctcctggt aagcaggccc
660

ccgtccctcc acccaagcca gcaagccgaa acacgacccg agaggctgct ggctcctctc
720

attcaaaaaa aacaactggc tctaaagcat cagcttcgcc atccacttca tccacctcat
780

ctcgtcccaa agcttcaaag gagacagttt ctagcaaagc agggacagtg gggaccacca
840

agggcaagag aaaaactgac aagcagccaa taacttctca cctgtcctca gacacaacaa
900

cttctggcac atccgacctg aaaggagagc ctgcagagac cagagtggag agtttcaaac
960

tcgaacagac tgtccctgga gctgaggagc agaacacagg caaattcaag tccatggtcc
1020

ctccaccccc tgtggctcca gcaccttctc ctctggcccc ccctctccct cttgaggatc
1080

agtgcattac tgcctcagac actccagttg tcctcgtcag cgttggagct gacctgcccg

1140

tctctgcctt agacccaagt cagcttcttt gggctgaaga gccgacgaac agaaccactc
1200

tctactcagg cactggctta agtgttaaca gagaaaatgc aaaatgtttc acaaccaaag
1260

aggagctggg gaagacagtg cctcagctac tgactcctgg gctgatgggc gaatcttcag
1320

aatcctttag tgcctcagaa gatgaaggcc acagggaata ccaagccaat gactctgact
1380

cggacgggcc tatcttgtac accgatgatg aggacgaaga cgaagatgag gatggcagtg
1440

gagaaagtgc tttggcaagt aaaatacgcc ggagggatac tcttgctatc aaacttggca
1500

acagaccatc taagaaagaa ctagaggaca aaaacatctt gcagcgtaca tctgaagaag
1560

agaggcagga aatccgacaa caaattggaa ccaagttagt caggaggctg agccagaggc
1620

ccacaactga agaattagag caaagaaaca tcctaaaaca aaagaatgaa gaagaggaac
1680

aagaagcaaa aatggaactt aaacgcagac tcagcagaaa gctcagcctg agacccacag
1740

tggcagagct acaagctcga aggatcctgc gatttaacga gtatgtagaa gtcacggatt
1800

ctcctgacta tgaccgccga gcagacaagc cctgggccag gttaacacct gcagacaagg
1860

cagcaataag gaaagaacta aatgaattta aaagcacaga aatggaagtt catgaagaga
1920

gtcgacagtt tacaaggttt catcgtccat aacgaagagt gagactattt ggaaacagag
1980

actgatcatc tttgggggaa gccctgcttc ctgaaaacct gatattgcac tgggatttga
2040

atgtgtgtgt ttccgtttaa cttgtggtga aggaagtgtg tgactcagct tggctgggaa
2100

gtgctcctca cctgtgtggc ccagatggct ccaacaagca aaaggaagga tgcagtgact
2160

cttgctgccc agaatgcaca gcgatggtaa gagacaccgt ggatattctt catcagaagc
2220

tttaatcaaa gaagatgagc agaagacaat cactggctcc ctgttaccag aaagccaaat
2280

tttataatgc gggtgaaaga aaaatataga ttaaaattgc tgggcactga aaaggggtgg
2340

gagttggaaa tcagaaagaa agagaaatat ttcattatgt taatgaagaa aagagaaaat
2400

tgaagcataa atgtattttt gaagtagtta ttcggtagct gagaacttat taagggattt

2460

aggaatttaa cctcctgatg tggattcctc aaagacttga tcggagaata ctcttgtttc
2520

accattatgt tggtacagta tagtaccatt attttatgtt gtgccagtga atccagttgc
2580

cagaaataag tctgaatgtt ttcatgcatc tttttcttaa atgcaagagc ttctactgac
2640

tcttaacaag catgcttacc caaattttgt tgcatgcttt taagtagcat tagctatgca
2700

cacttgacat ttttaaatat ccctttccta gtgtaccagc cttcacagga taagaggact
2760

gggaataaag tcagatgtaa tgtgacccta gaaaactgta gcaagcttca gaaataaatc
2820

ttttgatctt tccctatctt gattagatcc aaagtcaaag caaccatact tcacctagag
2880

aagacagtat tggcaatcat gacacctgta ataaaaactt gaatccaaag tcaaaactta
2940

agcaagatac aaatgtgctg cctgcagtta gtcctgcatg ggaataagga ctagttattt
3000

ttttagtgct gcattttttt aaagttggat tgctgctatt taaaaaaaaa aaaaggacac
3060

agatcaaaaa aacacccaaa ggcttaacag aacatggaaa gttcccacta agttgtgtgt
3120

gaggaaatcc tcatcttgta ttttaagaat ctgcagatgg catccataga tacagtacag
3180

tatttactgt caatgcaaaa gaatcagtga ggttaaagta gtggaagttt tccctatgag
3240

cctacagtct gtattgttta cattaagaaa ctctctatta ttgtgttgct attttccatg
3300

gagtcacagg cacactatga cttctgctcc caaatacata tgaccatatg taaataccac
3360

cttgcatttc attgttcttc acaagtgctt tgtgcccctt taattaaaaa cacatgaaat
3420

gaataacaaa acaagaccca aatagagttt attttattct cagagtttgg actacaattt
3480

tctaagatgt ttctggttca agactgtcat tttctatttc aaccgaaaag taagcattta
3540

acccggtgaa taaatgtaga tcctgccatt cattggtatt ttaaagaacc acttgaaatc
3600

ggatcatttt atttaaaaat aaaaaatgtt aatgccagtt ggcctactat aaaaagccag
3660

gaccatgtta gaattaggaa aagtaaccag cattactgca cctcttgtta gactctgtgc
3720

attaataaaa cacacctaga gtctgttgtc atttcactaa taggataaga caaatttttt

3780

gctttgaaaa aatttttctg catgcccagg tgtctgtctc tggctgaggt tttgtctatt
3840

ttacagtgtt tcaatccagc cataaaaatt aacttgtgat tttttttttc ccaaagtcat
3900

gcttttcctt aattatattt ttattttatt attttagtgt cttgagaaaa ataccaagag
3960

atataatgtt tcttttaatt gtctatgctt aatcatcttt aaacactttt taaatttcta
4020

accacaagac ctctctataa tggtaaatgt aagacatcac cattttatca ctcaaagtat
4080

gttattgaaa gtttctattt ggttgataaa aggaacaatt ttttcccact tttgatgcct
4140

gtgatgcaat tttttattgc ctacaatgag atacacttag tacaaaaaat gaaaatctgg
4200

tatttcaaaa ttgcatttct tgtataatag gtcagattta ttaactactc atactttttc
4260

tttacactaa tcgatacatt tagaaaaaat tttctaagtt agagacaagt ttaaaagt
4318

<210> 15
<211> 3015
<212> DNA
<213> Homo sapiens

<400> 15
gggagcaaat tcattccctt gctgggttgc tgggctctgg cactgcgctg gccctcgttg
60

gaggtctgca gaagtgcaca gaaagtgtgg gcaatggact ggccatttcc acagttttgg
120

aaacttttaa aaatccatat tcttgctggg attacagatg tgagccatcg tgcccagccc
180

acccactttg ttaatcatga tcagagcagg ggtgccattc taccaccttg gcacacctgc
240

ctagaggagt gagtggcgct gggagcagac tgaggaacat cttagctgag cagtgaatcc
300

agcactgttc taaggaagct cagatggtct gtgactcatc cctgtcctct ggactgaatc
360

tgaggccttg gaactcaccc agggtcttag tggaggcaca gctctgagct atgggtgaag
420

agcatctgaa cccatctaca cattgaccaa acacaggcag gttccagtct gcgggggaga
480

tggagaaggt gccaccatgc tgctgcacaa atcccaggat agctggggaa tgagaatcca
540

gcactttggg cctaggacct ggaaggagag gtcttttggg agaggtatgg ggacatctga
600

aggaggactt cttggaaggg gaggggctgg agcagaggca agagtgattt tgtcagatgg
660

agagaaggtt ctccacatgg gcggggctgg cagaggcaga tgcccagaca ggtcacaact
720

ctctaggtgc ccaattacct gccctcggtg agctcagcca tcggcgggga ggtgccccag
780

cgctacgtgt ggcgtttctg catcggcctg cactcggcgc ctcgcttctt ggtggccttc
840

gcctactgga accactacct cagctgcacc tccccgtgtt cctgctatcg cccgctctgc
900

cgcctcaact tcggcctcaa tgtcgtggag aacctcgcgt tgctagtgct cacttatgtc
960

tcctcctccg aggacttcag tgggtgcctg gatgagggag gagtggggaa gtgttccctg
1020

aggggacggg cctgccccta ccacattcgg accttcctac ttcgtggtgt gggcactccc
1080

tgcaatgtgg ctcccaatcc tctttccctc cagccatcca cgaaaatgct ttcattgtgt
1140

tcattgcctc atccctcggg cacatgctcc tcacctgcat tctctggcgg ttgaccaaga
1200

agcacacagt aagtcaggag gtacggtcta tccctagcgg gggctccaag gcagcccaga
1260

agaaaatcaa ggacatctgt cctcaggatt cgggtaatgg tgaggtggga actgagttct
1320

aatgggaacc ctggcagagg ggtgctgggg ttggggctgg ggcttgggaa caaactgagg
1380

gtggttggtc agaatctagg actatagcac tgtgtttggg tagtgtggga gactggagaa
1440

tagaagagat ggaggctgca aatggggata gaatgaggag tcgcatctag gcggcagtga
1500

gttaggaaca gtgtcagagg actggtctgt cataattcca gcgccccacc catgtacatg
1560

ggtttctttt cccccacagt atttggaggt ggggttggtg ggtgactcca tgtaactttc
1620

atactccatc cccctgaagt ggaagcggca ggaataccac tgctcctcgg cccatcactc
1680

ccccagaggc agggacgtga acacatagtc gtcattcttg ctgcccctgc ctgtgctccc
1740

ttccatgacc gctagtggga gccaagggag ataaggccca gcccttagga gttagggctg
1800

agaggggagc ccacgctctc atacccagca agctgcagag tgatcagaca gcccattccc
1860

taggatcgca agtcctacag ctggaaacag cggctcttca tcatcaactt catctccttc
1920

ttctcggcgc tggctgtcta ctttcggcac aacatgtatt gtgaggctgg agtgtacacc
1980

atctttgcca tcctggagta cactgttgtc ttaaccaaca tggcgttcca catgacggcc
2040

tggtgggact tcgggaacaa ggagctgctc ataacctctc agcctgagga aaagcgattc
2100

tgaacccttc agtcctgctt gggaggacgc agcccactgc ccagaaacaa gaaacacgat
2160

accattctgg ccttccccac cccacatcct ctcttggcct tactgaagat gggggaaggg
2220

taagaaggaa gggtgtaggc caaggctcac cccagtgctg ctggcttctc ctctccaccc
2280

ctcatatggg cgtggggtcc tcaaacatca cctttacctg agaggcccca agaagctgag
2340

ctggcagaga gctctaccat ttggtgctaa aaaaaaaaaa acgtcctgag gttcatgacc
2400

accatccagt ttctggcctt tacacagtca cctttcactg aggtcaggag cccctgagca
2460

gtggctgctc cctgacaacc acagccattt ctctgcgcgg gggtcattca taggactaat
2520

gtatttcatg atctactgtg cacatccagg cctgtggcca cagtcccctg ctaaagttgc
2580

tcaggtgttc tagtcctgac ttcacctttt tgatttggtg tgtgccctag ggtatgtacc
2640

cttccccatc tgagcctcgg tgtgtccatg tgtctggtgg gggatgggtg gactgtatga
2700

tttccaagga ctctaccagt cagtggttct gatgtcatcg ggtggaggtg gtgttctata
2760

cctaaaggat gacctgctcc agaaacagca ccagcacagc atgtattttc ttctcttctg
2820

aaagttctgg cttgtagacc cctcccctcc tttgcaaagg tatgggatag aggggtcaga
2880

tgcagatctc tactgtaaaa tgggctccct ggtatctcct gtcttcccta ctgctccaaa
2940

ccctaaattt tggttgtaca ttttatttga aaggaaaata aatttttttt ttgggccaaa
3000

aaaaaaaaaa aaaaa
3015


<210>  16
<211>  558
<212>  DNA
<213>  Homo sapiens


<220>
<221>  misc_feature

<222>  (250)..(260)

<220>
<221>  misc_feature
<222>  (252)..(252)
<223>  n is a, c, g, or t

<400>  16
ttttttataa ttaaataaat aactttattt atgatacttc agattttata aatgccaact
60

acatagcaaa gtcccataaa ttttaaccga gacacaacca ccaatcaagg ccctttgaaa
120

aatcaaagat aataaagtgt caagggagaa aagttttgtt ctcccaacag agaatttaat
180

caatgaactt acttctctga tcatttaagg ttcacaatgg gaaaacttct tttcttttaa
240

gggtaccctg anatcaatta acagtcaatc aacccacatt tagaaaaata aaccccatag
300

ttaagaaaaa tatatcaaat gcccttcaca cccataatgt aacaaaactt tgacaccaga
360

atgaaaaact tctcagtctc tgtaccaaac caggtgttag tccacccatg agcaagttcg
420

cctgaataac agcatctata caccatgatg tagctgagaa ctacagctga tatcctttta
480

ttgcctgtgg ctcggactga agccaatact ataaatacca cagtgaattc caatggtgaa
540

gagcagcagt gtagatgt
558


<210>  17
<211>  5481
<212>  DNA
<213>  Homo sapiens

<400>  17
ccgctggtcc gagctgtctg gcctcagttt ccctccgact tttctccgct ctgccagccc
60

tcactgctgc ccgtcattgt tctcgcagtt agatgggggt gctttgtgac ggctgccaag
120

ttggggtgtg ttctctttat tccgtttttc aaacagaaca aggcctccaa ggctgacccc
180

agacaaccca ccccctcgga ccctaattca ccttattgca ctgatttttt ttatcaagtc
240

gtattttatt gtacaggagc cacgccctga tttcttaaag gcgccttgca ctctggccat
300

gtgttatctc tgcagccggt gtgtgggagg cctcttgtga gccagttgtt ttcccgcctc
360

caccaccccc ctcgaagatt tagggatgcc aggggggaag aaggtggctg ggggtggcag
420

cagcggtgcc actccaacgt ccgctgcggc caccgccccc tctggggtca ggcgtttgga
480

gaccagcgaa ggaacctcag cccagagaga tgaggagcca gaagaggaag gggaagagga
540

cctgcgagac ggaggcgtcc ccttctttgt caaccggggt gggctacctg tggatgaggc
600

cacctgggaa aggatgtgga aacacgtggc caagatccac cccgatggag agaaggtggc
660

gcaacggatc cgtggggcca cagacctgcc caagatcccc ataccgagtg tgcctacgtt
720

ccagccgtct acacctgtcc ctgagcgcct ggaagctgtg cagcgctata tcagagagct
780

gcagtacaat cacacaggga cacagttctt tgaaattaag aagagcagac ctctgacagg
840

gctgatggac ctggccaagg aaatgaccaa agaggccctg ccaatcaaat gcctggaagc
900

cgtgatcctg ggaatttacc tcaccaacag catgcccacc ctggagcgct tccccatcag
960

cttcaagacc tacttctcag ggaactactt ccgccacatc gtgctggggg tgaacttcgc
1020

gggccgctac ggtgcgctgg gcatgagtcg gcgcgaggac ctgatgtaca agccgcccgc
1080

cttccgcacg ctcagcgagc tcgtgctgga cttcgaggcc gcctacggcc gctgctggca
1140

cgtgctcaag aaggtgaagc tgggccagag cgtgtcacac gacccgcaca gcgtggagca
1200

gatcgagtgg aagcactcgg tgctggacgt ggagcgcctg ggccgcgatg acttccgcaa
1260

ggagctggag cgccacgccc gcgacatgcg gctcaagatt ggcaaaggga cgggccctcc
1320

ctctcccacc aaggaccgga agaaggatgt ttcttccccg cagcgggccc agtccagccc
1380

ccaccgcagg aacagccgca gtgaaagacg gccctcgggt gacaagaaga cttccgagcc
1440

caaagccatg ccagacctta acgggtacca gatccgggtc tgaggcggat gccagcaccc
1500

caggccccac ccactcttgg gggccaggat ccacctgctg gaaccagcct tatgcatggg
1560

gaaggcgggg ctggtgacaa ggcagggcaa gaggctgcag gaagagtgtg ttccagctca
1620

gccccccaag ctgctctcgc tcccactgag ccaagccccc taactttggg cctagaggcc
1680

gttagtattt tatttggagt ttttaactct acaactgaag tttaaggtat ttggggaaaa
1740

cttagtccaa atggatctgc tgatggtggg aaggccagtg cttaacaaat ccatgtgtca
1800

tggggccagg tgagggaaac tgctggttct gctggtgcct ctgcccctgg cttctctctg
1860

ggagttgggt gcatcttatc agtgggaaat ctcccagcct taccaggcct gtgatggggg
1920

gtgggggtgg ggtggagatg tttctccagt tctgcctgcc ctggcagaat cttgacccag
1980

ggaaagggaa gcagggtagg agtccttctg agaaaggtct gtgtagccca ttaaccagga
2040

gcttggcaca ggccacatct gccccaagag catgagctcg tggctcagga gagccttcag
2100

gcccttgagg cccccatggg cagtgctgtg tgggcagagg aggggtgata tgagagcgag
2160

cccagggaag gacctctggg caaaaagttc ccaggcccta actgcgtcta cttgctcagt
2220

cccagctctg cctgttgctc tagcccacag gctccctgcg gagggtctgg gcttggcgga
2280

ggacccagaa tggcactgag gccagcatgg ctgtgggagt tgagcaaacc ctgggtgcca
2340

gtccaggagc tgtggctgga catggggtga tggggcggga tgcttgggcc tgggtctctc
2400

cgccagcagt gccaggagcc cttgctgggg aaatcaagac cagactagga tgcttctgcc
2460

ccaggcctgc cttccattct ttaacagccc atcttggctt ggggttgcaa tgatggctgg
2520

gccagtcact tgtggcaggg catcagggcc ctggcaggga agaacctagg cacctggggt
2580

tgtccccagc ctgcccgtca gcatgagata cccagtggga aagtgagagg atgcggagag
2640

gttggcagag ccaggggtag gttctggagg ctcaagcaac aaggaggtgc aggtaaaggg
2700

tgcagtgcag ccactgaggg acagctggga actgggggat gcaagtgaga aagggatgtg
2760

ggggagagtt caggatcagg ctgcttgagg agtaatgggt tacctacagc agagacgaga
2820

tggctgtttg tcaggaggcg gtagaaaggt agaaaggatt cagattgtgg aagggtaaat
2880

ggatgagatg accattaagg ttttgtttta ctgagaggct gtaagtctgc cagggacagc
2940

tgtcagtaag gctgcagaag ggctgggggg ctacacaagg aagagcagaa ctagggttgt
3000

aagctcaaat gacgagcaaa cggtgagaag gaggcctgaa gggctgtgtg gggactatgg
3060

ctacctgaag agggcacacc ctgttaaagg ggccacagct gctcagctgt tcttacagtg
3120

ccggccctgt gttgccagat tgtctgcttt gtcagaggcc agaattctga ctttttatgt
3180

gaaataggat ttttaaatgc tggtgatcac ttaaaaaaaa cttaaaaacc caatactggc
3240

aaaagaggat gccagtttgc aatccctgaa gtagagagag ctcgtgctgg ggagaagtcc
3300

accaagatgc tttgaggcgg gctgtagcaa agtcctgttt ctcagagctg ggctgggctg
3360

gggtaggatc cttgcagctg aggaggagga aaagccactg agtctcctcc cagagcggga
3420

caaaccagag gccctttgca gttgctgggt caccagcggg ggtggcgcat ggagtacaga
3480

cagtgtagct cttggcctgc cagggagacg gatggtgcct ttgaagcaaa gaggaaggga
3540

aggcagaacc agggatgcct ttgctgatga gagtgcctgt cagggaaggc gccacaggct
3600

ggcagctctt caaaccagca gcgcttgacc cagagccaga tccagcatgg cctggccaga
3660

ggcaccctgg gaggccagct ggtcagtccc ttgcctcccc aagttccccc tggggtcaat
3720

gagccctggg aggatgccta acctaactcc agccagacta ataggggcat ggtgaccctt
3780

gactcaccat cccatcccag ctttcaggga gtgggggtag tgtggtctcc atgttcctac
3840

tatgcctaag aagagatggc tcaccttggg aggtgccagg ctgaaactag gtcctttccg
3900

ggtctggatg ctgccgctca ggagcagggg cgtggcctca gctgcctctg ggagcttccc
3960

gggaatgaca gggtttgagg ggagtagata tgagagggag ccgctcctgg ttctggagtc
4020

ttaggaggtt ccaacttgca ggatcctttc ccagagccct ccatggagaa aacagcaaaa
4080

tgaagccctt acctgcttgc tgtctgcaag ggagggagcc gagccccagc tgataatccc
4140

ccagcactca cccttcctga gctgagactt cggggctgtg gagaccagca caggacatag
4200

tggtgctttt taaatttatt tttaactgtt tctcatatgt agcaacccct cctcccctcc
4260

tgggcatgtt tacacaggct ctgctctggg ggctggcctg gctgtgaggt ttctggggag
4320

gcagagaggc agggactttg gggccttagt caccatccat ggtatcacct catctcactt
4380

cctgtgaggg acagggcctg gctgatgtga tcccagctcc ccccagttca ggactgtctt
4440

tcagctcctt tgcccctgga ggtgggggct gctggctgag gaggggtcaa ggtgagttca
4500

agaaagctac ctgtggaaaa tggaccaggt tgggggggtg attgcaaagt ctccccaaag
4560

cctggctcct catgctcagt gccaggggca gaacactggg gagccaggta tagagagcct
4620

tcctgtcata actgccagtc ctcttcctcc aaggcctctg catattctca tgttcccctc
4680

acccatcatg ccagccaccc ctatccctct tctagcaggg ccaagatggg gacagcagca
4740

gccctctggc cttgggatgt gatgataaag caagcctagg gccagggttt ggggagcaga
4800

gagagccaag aagttgacca cgtgtgattt ccagcccttc ccactgggac ttgacttccc
4860

aggtcaagga gtccgtctca ttctggctgg tcgagtgacc agaggcctgt gtgaatgtgt
4920

gcacctgctt ttcctgcctg gaatgttttc tggctcagct gcagcaacat ctgtgagccc
4980

agtgtctgcc ctgtgtccct gggctcgctc caagtgcagg aacatacatg cagggcccaa
5040

catgatgatg gtgtgaaggg caggaaacag tcctctgaag gagtggggag gtgggcagtc
5100

tgcccccgcc aggtaccatc gcctcctgcc agcttcctta gaccaggcag ggctgccatg
5160

gtgctagctg caagtccatc agtattgacc gtctcgctcc atcttggtcc tccggagtcc
5220

caagtttcct tttcatcaaa tctgacaaga gagaagaaac atgggtgtgc ttggcccaca
5280

gggcctggtg gtgatggacc tccccgctcc ctcaagctct ggatggctgc agtgttgtac
5340

tagactttgt tcaggctgtt ctcatctcag tattgcccct tcctttcact ttcacacttc
5400

atctcattcc tgttgtcact ttccccgaaa cgaataaagt ctccccagct cctgctgtgt
5460

aggctgggca gaaaccacaa c
5481


<210> 18
<211> 743
<212> DNA
<213> Homo sapiens


<220>
<221> misc_feature

<222>  (531)..(736)

<220>
<221>  misc_feature
<222>  (531)..(531)
<223>  n is a, c, g, or t

<220>
<221>  misc_feature
<222>  (655)..(655)
<223>  n is a, c, g, or t

<220>
<221>  misc_feature
<222>  (657)..(658)
<223>  n is a, c, g, or t

<220>
<221>  misc_feature
<222>  (665)..(665)
<223>  n is a, c, g, or t

<220>
<221>  misc_feature
<222>  (667)..(667)
<223>  n is a, c, g, or t

<220>
<221>  misc_feature
<222>  (672)..(673)
<223>  n is a, c, g, or t

<220>
<221>  misc_feature
<222>  (702)..(702)
<223>  n is a, c, g, or t

<220>
<221>  misc_feature
<222>  (736)..(736)
<223>  n is a, c, g, or t

<400>  18
tttggcatgc cactaactat tttttatttg caactacatg atggcacaca attctcttaa
60

acaacgacat aaaatagatt tccttgtata taaataactt acatacgctc cataaagtaa
120

attctcaaag gtgctagaac aaatcgtcca cttctacagt gttctcgtat ccaacagagt
180

tgatgcacaa tatataaata ctcaagtcca atattaaaaa cttaggcact tgactaactt
240

taataaaatt tctcaaacta tatcaatatc taaagggcat atatttttta agaaagatta
300

ttctcaataa cttctataaa aataagtttg atggtttggc ccatctaact tcactactat
360

tagtaagaac ttttaacttt taatgtgtag taaggtttat tctacctttt tctcaacatg
420

acaccaacac aatcaaaaac gaagttagtg aggtgctaac atgtgaggat taatccagtg
480

attccggtca caatgcattc caggaggagg tacccatgtc actggaattg ngcgatatgg
540

tttatttttt cttccctgat ttggataacc aaatggaaca ggaggaggat agtgattctg
600

atggccattc cctcgataca ttcctggctt ttttctgggc aagggtgccc acatngnnag
660

agtgnanata tnnagtctga aatctggtac acaggacttg cngctgcagt caccgaactg
720

ggttcacttt catttncttt act
743

<210> 19
<211> 4701
<212> DNA
<213> Homo sapiens

<400> 19
gcggccgcca cacccagcac cacaggcacc aagtccaaca cgcccacatc ctccgtgccc
60

tcggccgccg tcacacccct caacgagagc ctgcagcccc tgggggacta tggcgtgggc
120

tccaagaaca gcaagcgtgc ccgggagaag cgcgacagcc gcaacatgca agtacaggtc
180

acccaggaga tgcgcaacgt cagtataggc atgggcagca gtgacgagtg gtctgatgtt
240

caagacatta ttgactccac gccagagctg gacatgtgtc cagagacccg cctggaccgc
300

acaggaagca gcccaaccca gggcatcgtg aacaaagctt tcggcatcaa caccgactcc
360

ctgtaccatg agctgtcgac ggcagggtct gaggtcatcg gggatgtgga cgaaggggcc
420

gacctcctag gggagttctc aggaatgggc aaagaagtgg ggaatctgct actggaaaac
480

tcacagcttc tggaaaccaa aaacgccttg aatgtggtga agaatgacct gattgccaag
540

gtcgaccagc tgtccgggga gcaggaggtg ctgaggggcg agttggaggc tgctaagcag
600

gccaaagtca agctggaaaa ccgtatcaag gagctggaag aggaactgaa aagagtgaag
660

tccgaggcca tcatcgcccg ccgtgaaccc aaagaagagg cggaggatgt aagcagctat
720

ctctgtacag aatcggacaa aatccccatg gcccagcgcc gccgcttcac gcgggtggag
780

atggcccgtg tgctcatgga gcggaaccag tacaaggagc ggctgatgga gctgcaggag
840

gctgtgcggt ggactgagat gatcagagcg tcccgagagc acccatccgt ccaggagaag

900

aagaagtcga ccatctggca gttcttcagc cgcctcttca gctcttcctc cagcccccct
960

ccggccaagc gcccctatcc ctcggtgaac atccactaca agtcacccac cactgccggc
1020

ttcagccagc gccgcaacca tgccatgtgc ccgatctcgg caggcagccg gcccctggaa
1080

ttcttccctg acgacgactg cacgtcctcc gcccgtcgag agcagaagcg cgagcagtac
1140

cgccaggtgc gtgagcacgt gcgtaacgac gacggccgtc tgcaggcctg cggctggagc
1200

ctgcccgcca agtacaagca gctgagtccc aacgggggcc aggaggacac gcggatgaag
1260

aacgtgccgg tgccggtgta ctgccgccct ctggtggaga aggaccccac catgaagctg
1320

tggtgtgccg cgggcgtcaa cctgagcggg tggaggccca atgaggacga cgctgggaat
1380

ggagtcaagc cagcgccagg ccgcgatccc ctgacctgcg accgcgaagg agacggcgag
1440

cccaagagcg cccacgcgtc tcccgagaag aagaaggcca aggagctccc tgaaatggac
1500

gccacctcca gccgggtgtg gatcctgacc agcaccctga ccaccagcaa ggtggtgatc
1560

atcgacgcca accagccggg cacggtggtg gaccagttca ccgtctgcaa cgcgcacgtg
1620

ctgtgcatct ccagcatccc cgcggccagc gacagcgact accctcccgg ggagatgttc
1680

ctggacagcg acgtgaaccc agaggacccg ggcgcagatg gcgtgctggc cggtatcacc
1740

ctggtgggct gtgccacccg ctgcaacgtg ccgcggagca actgctcctc ccgaggggac
1800

accccagtgc tagacaaggg gcagggggag gtggccacca tcgccaacgg gaaggtcaac
1860

ccgtcccagt ccacagagga ggccacagag gccacggagg tgccagaccc tgggcccagc
1920

gagccagaga cagccacatt gcggcccggg cctctcacag agcacgtctt cactgaccca
1980

gccccgaccc cgtcctctgg cccccagcct ggcagcgaga acgggccaga gcctgacagc
2040

agcagcacac ggccagagcc agagcccagc ggggacccca cgggagcagg cagcagtgct
2100

gcacccacca tgtggctggg agcccagaac ggctggctct atgtgcactc ggctgtggcc
2160

aactggaaga agtgcctgca ctccatcaag ctgaaggatt ctgtgctgag cctggtgcat

2220

gtcaaaggcc gtgtgctggt ggctctggcg gacgggaccc tggccatctt ccaccgtggt
2280

gaagatggcc agtgggatct gagcaactat cacctaatgg acctgggcca cccgcaccac
2340

tccatccgct gcatggctgt tgtgtacgac cgcgtgtggt gtggctacaa gaacaaggtg
2400

cacgtcatcc agcccaagac catgcagata gagaagtcat ttgacgccca cccgcggcgg
2460

gagagccagg tgcggcagct ggcgtggatc ggcgatggcg tatgggtgtc catccgcctg
2520

gactccaccc tgaggctcta ccatgcacac acgcaccagc atctacagga cgtggacatt
2580

gagccctacg tcagcaagat gctaggcact ggcaagctgg gtttctcctt cgtacgcatc
2640

acggccctgc ttgtcgcggg cagccggctc tgggtgggca ccggcaacgg agtggtcatc
2700

tccatccccc tgacagagac tgtggtcctg caccgaggcc agctcctggg gctccgagcc
2760

aataagacat cccccacctc tggggagggc gcccgtcccg ggggcatcat ccacgtgtat
2820

ggcgatgaca gcagtgacag ggcggccagc agcttcatcc cctactgctc catggcccag
2880

gcccagctat gcttccatgg gcaccgcgat gccgtgaagt tctttgtctc ggtgccaggg
2940

aacgtgctgg ccaccctgaa tggcagtgtg ctggacagcc cagccgaggg ccctgggcca
3000

gctgcccctg cctcggaggt cgagggccag aagctgcgga acgtgctggt gctgagcggc
3060

ggggagggct acatcgactt ccgcattgga gacggagagg acgacgagac ggaggagggc
3120

gcaggggaca tgagccaggt gaagcccgtg ctgtccaagg cagagcgcag tcacatcatc
3180

gtgtggcagg tgtcctacac ccccgagtga agctgctgcc ctgcctggcc cgacctgtac
3240

ataggacccc cgaccacctg acccccgccc ggcccgcggg gtagccagcc aggcgccgcc
3300

gcccctcttc taacctctca acctgcagct ttcacctgag tctggcccct ccagcgggca
3360

gggagtgcgg ggatgcggat cagctgggag gaggagggga ggggtgcttc cacccgaggg
3420

gaagatgctc tcgggacagt ttcccgggca gctcctggcc agcttccagc ccagagtcct
3480

caagtccagg gcaccttggg cccagcgcag gcagaatccg aggtggtcct ggctctaccc

3540

tgggcctcct actccccagc acccctggag gaggcagggg ctccccgccg ccgaggctgc
3600

ctgccctggg cccacctctg catgctgctc atggggccac cctgcctcct gggccctcac
3660

tctgcctagg ggagctgggc caggcactag cctttgccca gggaggtggg cctcaggctg
3720

cccaggtgcc tgcaccccag ccggccttct ctggggcctc cccgtcgtca agcctctatc
3780

ctgtctgtcc ccaccccagc tgtcccctgc ccagggagct ggcataaaag cacgaggccc
3840

ggctccctgg ggcagctgct tgagaacaga gactgctacc ccatcctgcc catgcaggca
3900

ggctcttgcc agccccgttc tgacccgtgt ccccccaggc tctgcctggg cagaagactc
3960

accttggagg agtgggccct ggagtcctgt ccctcccaga agcccccagg gtgggatttc
4020

tcaggctgcc agggcaggcc caggcctcag gaagaagggg aggcccctgg cctctccggg
4080

atcagtccta ggacacaggc tcagcctcag gttgatgggg gatgatgtgc tcccggggcc
4140

tgcctcctgc acggggctcc acggagccca gctcccagac acgctactaa gtgcctaggg
4200

ttgcccgctg tggcctgctc ccagggagca acagagaggc caccaagcag aggcccgtgg
4260

ggctgaggat ggagccgccc ccagccgact ccaagcccgc agagggcaga cgccaccctg
4320

gactgctctc cctgcccagc tgggcctctc tggcctattc ctaccttcca ggcccactgc
4380

actcctgtct gggaggccct tatgagggca gcccagcccc cgcacccacc cccaaccaga
4440

gaagcacaga tcttggggag ctgccccaca agccccgctg gccaccgagg gctgcagccg
4500

ctgcgctgcc ggcttctccc caccaccctg ccacctccac tgtgatgtat gtccgctccc
4560

tcgtctgttc ccccaggatc tcgaagtgac tccgggctga gcagtggggc ggctggggga
4620

ggggtgacga ttctcctcag gctttggccc tgcaagcaaa cccacatatc tgctctgtat
4680

gtaataaatg tcttaacgtc g
4701

<210> 20
<211> 2170
<212> DNA

<213>  Homo sapiens

<400>  20
ctgagggctc atccctctgc agagcgcggg gtcaccggga ggagacgcca tgacgcccgc
60

cctcacagcc ctgctctgcc ttgggctgag tctgggcccc aggacccgcg tgcaggcagg
120

gcccttcccc aaacccaccc tctgggctga gccaggctct gtgatcagct gggggagccc
180

cgtgaccatc tggtgtcagg ggagcctgga ggcccaggag taccgactgg ataaagaggg
240

aagcccagag cccttggaca gaaataaccc actggaaccc aagaacaagg ccagattctc
300

catcccatcc atgacagagc accatgcggg gagataccgc tgccactatt acagctctgc
360

aggctggtca gagcccagcg accccctgga gctggtgatg acaggattct acaacaaacc
420

caccctctca gccctgccca gccctgtggt ggcctcaggg gggaatatga ccctccgatg
480

tggctcacag aagggatatc accattttgt tctgatgaag gaaggagaac accagctccc
540

ccggaccctg gactcacagc agctccacag tggggggttc caggccctgt tccctgtggg
600

ccccgtgaac cccagccaca ggtggaggtt cacatgctat tactattata tgaacacccc
660

ccaggtgtgg tcccacccca gtgaccccct ggagattctg ccctcaggcg tgtctaggaa
720

gccctccctc ctgaccctgc agggccctgt cctggcccct gggcagagcc tgaccctcca
780

gtgtggctct gatgtcggct acgacagatt tgttctgtat aaggaggggg aacgtgactt
840

cctccagcgc cctggccagc agccccaggc tgggctctcc caggccaact tcaccctggg
900

ccctgtgagc cctcccacgg ggggccagta caggtgctat ggtgcacaca acctctcctc
960

cgagtggtcg gcccccagcg accccctgaa catcctgatg gcaggacaga tctatgacac
1020

cgtctccctg tcagcacagc cgggccccac agtggcctca ggagagaacg tgaccctgct
1080

gtgtcagtca tggtggcagt ttgacacttt ccttctgacc aaagaagggg cagcccatcc
1140

cccactgcgt ctgagatcaa tgtacggagc tcataagtac caggctgaat tccccatgag
1200

tcctgtgacc tcagcccacg cggggaccta caggtgctac ggctcataca gctccaaccc
1260

ccacctgctg tctttcccca gtgagcccct ggaactcatg gtctcaggac actctggagg
1320

ctccagcctc ccacccacag ggccgccctc cacacctggt ctgggaagat acctggaggt
1380

tttgattggg gtctcggtgg ccttcgtcct gctgctcttc ctcctcctct tcctcctcct
1440

ccgacgtcag cgtcacagca aacacaggac atctgaccag agaaagactg atttccagcg
1500

tcctgcaggg gctgcggaga cagagcccaa ggacaggggc ctgctgagga ggtccagccc
1560

agctgctgac gtccaggaag aaaacctcta tgctgccgtg aaggacacac agtctgagga
1620

cagggtggag ctggacagtc agagcccaca cgatgaagac ccccaggcag tgacgtatgc
1680

cccggtgaaa cactccagtc ctaggagaga aatggcctct cctccctcct cactgtctgg
1740

ggaattcctg gacacaaagg acagacaggt ggaagaggac aggcagatgg acactgaggc
1800

tgctgcatct gaagcctccc aggatgtgac ctacgcccag ctgcacagct tgacccttag
1860

acggaaggca actgagcctc ctccatccca ggaaggggaa cctccagctg agcccagcat
1920

ctacgccact ctggccatcc actagcccgg ggggtacgca gaccccacac tcagcagaag
1980

gagactcagg actgctgaag gcacgggagc tgcccccagt ggacaccagt gaaccccagt
2040

cagcctggac ccctaacaca gaccatgagg agacgctggg aacttgtggg actcacctga
2100

ctcaaagatg actaatatcg tcccattttg gaaataaagc aacagacttc tcaacaatca
2160

atgagttaat
2170


<210> 21
<211> 1445
<212> DNA
<213> Homo sapiens

<400> 21
ctgccccaca cacactaacc caaccatctt ggggtggact ccctgccagc ccaactgttg
60

tattttcagt tcttccagtg tgaatcagtt aatattctcg ggaacgaggg agaggttgat
120

cctatgagga aatcaaccac agtgaaaagg cttgggccgc ttttgttttc gcctcctttt
180

gttgaacaaa tttgatttcc ggagtcagtc attttactgt caagacattt cttcggcatt
240

ctgcaacagt ttccaacatg gctagatcca tcagaaactg aagccgtgga gaacgctctc
300

ggggcctttg ccacttcttg gagtagaagc cgacagagag ctgtttggaa acttctcctt
360

cacacaccag ttgaagacta ggctttggag gttttcaaag cagacggtgc ttggatgggc
420

agggagaagt aacattctgc aaatcgccgt cagaggtcct gaggacacag acctacctgg
480

cttgcattcc ccttgctgaa tggcgtgtgc tgcagctgcc cactgagggc tcttttccct
540

gggattctgg acttcagagt aggacagcag gctgggaaga tgctgagttc catcaagtgc
600

gtgttggtgg gcgactctgc tgtggggaaa acctctctgt tggtgcgctt cacctccgag
660

accttcccgg aggcctacaa gcccacagtg tacgagaaca caggggtgga cgtcttcatg
720

gatggcatcc agatcagcct gggcctctgg gacacagccg gcaatgacgc cttcagaagc
780

atccggcccc tgtcctacca gcaggcagac gtggtgctga tgtgctactc tgtggccaac
840

cataactcat tcctgaactt gaagaacaag tggattggtg aaattaggag caacttgccc
900

tgtacccctg tgctggtggt ggccacccag actgaccagc gggagatggg gccccacagg
960

gcctcctgcg tcaatgccat ggaagggaag aaactggccc aggatgtcag agccaagggc
1020

tacctggagt gctcagccct tagcaatcgg ggagtacagc aggtgtttga gtgcgccgtc
1080

cgaactgccg tcaaccaggc caggagacga aacagaagga ggctcttctc catcaatgag
1140

tgcaagatct tctaaacccc aagagacttc acacaacact tatgtatgca ccccaaagac
1200

taatggggag agggagggcc gggaagccag gaaagcttgg tgttttctct gggtacaccc
1260

caagcagcgt ctccgttttg gatacagtta ttgatgaggc ttggccactg gatgttttca
1320

ctaactacac tctacaagtg aactccttgc ccaggccagt tagaaaatcc cttggggaac
1380

tgtgatgaat attccatctt tgattaaaaa agtgaaatag tctccataaa aaaaaaaaaa
1440

aaaaa
1445

<210> 22

<211>  2429
<212>  DNA
<213>  Homo sapiens

<400>  22
   ggcggtgcac gccggctgcc cgctgcccgc gatgcccatg cagggcggcg cgcagagtcc
   60
   cgaggaggag ctgagggccg cggtgctgca gctgcgcgag accgtcgtgc agcagaagga
   120
   gacgctggcc agcgcgaggg ccatccgcga gctcacgggc aagctagcgc gctgcgaggg
   180
   gctggcgggc ggcaaggcgc gcggcgcggg ggccacgggc aaggacacta tgggcgacct
   240
   gccgcgggac cccggccacg tcgtggagca gctcagccgc tcgctgcaga ccctcaagga
   300
   ccgcctggag agcctcgagc accagctcag agcaaacgtg tccaatgctg ggctgcccgg
   360
   cgacttccgc gaggtgctcc agcagcggct gggggagctg gagaggcagc ttctgcgcaa
   420
   ggtggcagag ctggaggacg agaagtccct gctgcacaat gagacctcgg ctcaccggca
   480
   gaagaccgag agcaccctga acgcgctgct gcagagggtc accgagctgg agcgaggcaa
   540
   tagcgccttt aagtcaccag atgcgttcaa ggtgtccctc ccactccgca caaactacct
   600
   atacggcaag atcaagaaga cgctgcctga gctgtacgcc ttcaccatct gcctgtggct
   660
   gcggtccagc gcctcaccag gcattggcac ccccttctcc tatgcggtgc cagggcaggc
   720
   caacgagatc ttgctgatcg agtggggcaa caaccccatc gagctgctca tcaacgacaa
   780
   ggttgcgcag ctgcccctgt ttgtcagtga cggcaagtgg caccacatct gtgtcacctg
   840
   gacgacacgg gatggcatgt gggaggcatt ccaggacgga gagaagctgg gcactgggga
   900
   gaacctggcc ccctggcacc ccatcaagcc cgggggcgtg ctgatccttg gacaagagca
   960
   ggacaccgtg gggggtaggt ttgatgccac tcaggcattt gtcggggagc tcagccagtt
   1020
   caacatatgg gaccgcgtcc ttcgcgcaca agaaattgtc aacatcgcca actgctccac
   1080
   aaacatgccg ggcaacatca tcccgtgggt ggacaataac gtcgatgtgt tcggaggggc
   1140
   ctccaagtgg cccgtggaga cgtgtgagga ggctctcctt gacttgtagc cgccttctcc
   1200
   tctgtccagg aggccgggat caggctgttg ccatggaagt tcagggccat agactgcccc
   1260
   acttaaactc ttgtcagtct gggctcaggg ttcccagagc tcattcccca ggaatctcta
   1320
   agaccagggc tggggcagtg tctgtcactg gcttgtttgt tccctaccaa tattctgttg
   1380
   ctgtttgaag tagtgccagg gtcccctggg aagatgcccc caagacacct gccccaagtg
   1440
   ggtggatatc tgccttcctg ctgcaagtgg aggcaggtcc agcagcccct cttcagagcc
   1500
   cctgtaaatg ctatcgcagc ctgagtcctg ccgccttcca gttccttggt gtcccgtgca
   1560
   ccccttctgt ctgtcccctt tcatgctgtg cagccgtccc gctggagtgg ccatgtccct
   1620
   tgtgcattga gtgcatcccc gctggtgact aagctcgcag caagcgctac ccccgatctg
   1680
   caaaagggcc tctccctttg tgttctatac attgtgaatc ttcccgtctg aagaacgccc
   1740
   agcctgccca gacaaagccc cgccttcccc aaagcagagg ggctgtctgt gtctccagaa
   1800
   aggggacatc ggggggggag gggggctcag aaaggagaag ggctgtgatc tccggtccct
   1860
   tcccccatca tccttcctta gactgatgct ttgactgaat catcactagc tatggcatta
   1920
   aaaggcctct cttctcatct ggtgccaaag gttccgttgc agctttttac aaccatccgg
   1980
   tgtggtttgg aggatttgtt tttttttttt cccaacagaa aagaacagcc attagaagaa
   2040
   ggctcccatt ttctgatgtt ccgccccact gtgaagagtg tgctcgtttt aaattcatgt
   2100
   tgattcttgt aagcactgga ctgtcttcat caagtatttc ccctacagaa ctcctcaaga
   2160
   aaaacagaga tcatttggct agagattgtc tgagtgactc caagctactc actgtattgg
   2220
   acgggagtag taatttattt taaagataaa gtgactaagt ggggaaattt ataaagctaa
   2280
   atattatata ttttattttt catacatgtt tgaagtgcaa atctgtggat attccatttg
   2340
   taggaccaag tcgacatgcc catcctgaca ttgtatgcta cgagaactct tctgatgatg
   2400
   gaatttcgat taaagtgcac tgaaagatg
   2429

<210> 23
<211> 166942
<212> DNA

<213>  Homo sapiens

<400>  23
gatcttgtat acagcaaatc ctaaataatc cactaaaaaa tttctagaac taataagcaa
60

gttcagcaag gtggcaggat aaaagaccaa tatacaaaaa aaattaatta tgtttctaaa
120

cactagcaat gaacaatcca aaaacaaaga ggaaaaaata gaaaaaatag aattaagaaa
180

atgatttcat tttcaacagc atcataaaca ataaataatt atgaacaaat tcaacaatag
240

atgtgcaaaa gtcatacttg aaaataacaa atattgttga aagaaaagaa ggcctaaata
300

aagagaaaga tatcccatgt ttgtggattg gaaaacagta ttattaagat gccaatacag
360

ccaggcgcag tggttcatgc ctgtaatccc agcactttgg gaggctgagg tgggcagatc
420

acctgaggtc aggggttcga aaccagcctg gccaacatgg cgaaacctca tctctactaa
480

aaatacaaaa attagctggg catggtggtg ggcacctgta atcctagcta cttgggagcc
540

tgaggcagga gaatcgcttg aacctgagag gtggaggttg cggtgagatg agatcgtgtc
600

attgcactcc agcctgggtg acagagtgag actccatctc aaaaaaaaaa aaaaaaaaat
660

accaatactt cccaaaatga tctacctatt caatgcaatt cctatccaga ttccagcagg
720

cctttttttt ttgtaaacat tgacaagctg atgttaaaat tcatatggaa attgtcaatc
780

ttgagaaaga agagcaaagt tggaggactc acactgtgag tcccaatttc agaacttact
840

acagagctac aacaatcaag attgtgtagt actgtcataa ggacagacat aaatcaactg
900

aatggaattt agtgtctaga cactaatcct cacattatgg ccaattgatt ttcaacagtg
960

ccaggactct tcaatgggga aagaatagcc tcttcaacaa acagtgctgg aacgactgaa
1020

tattcacatg taaaagaatg aagtttgacc tcttcttcac accacatgta aaaatcaatt
1080

cagaatggat catagaccta gaagtaagaa gtacaactat aaacctcttt gaagaaaata
1140

tgagtaaatc ttcatgacct tggtttcggc aaagccttct taaatataac accaaaagca
1200

caagcaacaa aaaagtacat aaattgggct ttctcagaat taaaaactag aaattcttca
1260

tttggaccct ataaaaaagg aaagacagcc cccgccccac ccagaatatt tgcaaatcat
1320

gctgcaagtt tccgaagtgt aatgaacatt tgactacctg tgaagtgatg agccagttat
1380

tgggctataa ctgtaaactc ctcccccctc cccaccattt attaaccaca ataatcctgt
1440

gacttcacaa tatcccctct ttacagatga ggaaacagag gctcagagat gtgactcgcc
1500

caatagagtg tgatgctggt atagatggaa cggggattca gccccaagtc cctcttgtct
1560

ccagctctct ttccatcacc ctgtattgct tgttggagaa tgccagacat agaagatctg
1620

cagccttgtt ttacgccagg ggaaactgag gtccaagaaa ggaaatactt ctccaagttc
1680

ccgctgccgc ctcctgcctc ctgatttgtt gctgtttgtt acagcaaact acagggaacc
1740

tatacgataa aatctcactc atctgggctt atctaatgca aagttacatg aacaatcatc
1800

tattttaaaa gaaataattt gattactctt taatttgtga gggaaggaat gttgggcagt
1860

agaaaggaca ttcaagttta taacctgaaa gatgtgaatt tgagaactga cttgtacttt
1920

attaattctg gaatctagga tatatgacta cgctgtgccc cagtgtcctc atctctcaca
1980

tgagagggtt gtgctttacc cacctctccc agtgtggtgg gggctcaggc aagatgagca
2040

atgccactgg gcttcaaaga cgatggagtc ggcacaagtg tagtaattgt ttcatgtcaa
2100

aaggacgagg gctaaagttg ctgagaagta gagctctttt tgcttctctc tctctctctt
2160

tctctctata gaaatattac tgttttaatt taatgagacg gctggacgtg gtggttcacg
2220

cctgtaatcc cagcactttg tggggctgag gcggatggat cacctgagat caggagtttg
2280

agaccagcct ggcgaacagg atgaaacccc atctctacta aaaatacaaa agttagccag
2340

gcatggtggc aggcacctgt aatctcagct actctggagg ctgaggcagg agaatcgctt
2400

gaatccggga ggcagaggtt gcggtgagcc aagatcgcac cactgcactt cagcccgggc
2460

aacaagagtg aaactccgtc tcaagacatc ataaattaat taattaattt aatgagtcac
2520

taaagattgc tgatccattg tgggcaaaca ggtactgctt aagtgtttga aaagttcttt
2580

tattttttac ttctttgaga caggatctca ctctgccagc caggctggag tgcagtggca
2640

caatcatagc tcacagcagt cttgaactca taggctcaag ccaccacacc tggctaattt
2700

ttaaaatttt ttgtagagat gggatttcac tatgttgccc aggctggtct tgaactcctg
2760

gcctcaagca accctcccac ctctgccccc caaagtgctg ggattacaag tgtgagccaa
2820

cgcacctggc ctgataagtt cttataagtg gatttggcag tctgtgagag cctggcttgt
2880

ttatggtgat gtgcctttcc catagctagt gcccaagtca gcaaacttcc cttggcctgt
2940

tcttgtaaat aaagtttgac tagaacacaa ccccacctat ttgcttatgt gttgtttgtg
3000

gcggcttttg ccatacaatg gcagagttga gtggttgaga tgaaggctgt atggtggccc
3060

acaacgtcta aaatattttg agcacatgac acctgttacc acctccaggg gaagcagtcc
3120

cactgcacag aggatagcaa agcgtactaa tgcctgccac taggagcggc agtccgagaa
3180

tctcaaccta ctgcatggtt tgctggtgct ggggttgcca agaaagccac ttgcacaggc
3240

cttggatggc acaatgcttt attcacctag agaagagaca gagcaagatc agcctcaaca
3300

gtcagcatcc aaaagccaaa attgacaaat gggatctaat taaactaaag agcttctgca
3360

cagcaaaaga aactaccatc agagtaaaca ggcaacctac agaatgggag aaaatttttg
3420

ccatctactc atctgacaaa gggctaatat ccagaatcta caatgaactc aaacaaattt
3480

acaagaaaaa aacaacccca tcaaaaagtg ggcgaaggat atgaacagac acttctcaaa
3540

agaagacatt tgtgcagcca aaagacacat gaaaaaatgc tcatcatcac tggccatcag
3600

agaaatgcaa atcaaaacca caatgagata ccatctcaca ccagttagaa tggcaatctt
3660

taaaaagtca ggaaacaaca ggtgctggag aggatgtgga gaaataggaa cacttttact
3720

ctgttggtgg gactgtaaac tagttcaacc attgtggaag tcagtgtggt gattcctcag
3780

ggatctagaa ctagaaatac catttgaccc tgccatccca ttactgggta tatacccaaa
3840

ggattataaa tcatgctgct ataaagacac atgcacacat atgtttactg tggcactatt
3900

cacaatagca aagacttgga accaacccaa atgtccaaca atgatagact ggattaagaa
3960

aatgtggcac atatacacca tggaatacta tgcagccata aaaaatgatg agttcatgtc
4020

ctttgtgggg acatggatga agctggaaac catcattctc agcaaactat cacaaggaca
4080

ataaaccaaa caccacatgt tctcactcat aggtgggaac tgaacaatga gaacacatgg
4140

acacaggaag gggaacatca cacaccaggg cctgttgtgg ggttggggga gggggagggg
4200

atagcattcg gagatatacc taatgttaaa tgatgagtta atgggtgcag cacaccaaca
4260

tggcatgtgt atacatatgt aacaaacctg cacattgtgc acatggaccc taaaacttaa
4320

aatataaaaa aaaaatagca tcagtccccc atggccagcg ggtctcactt gggaaccagt
4380

gctcactatt gcactcttgc agcccctctt ttgtgctgca ggtgagggac tctgttcctg
4440

ttccctctct gccaggaaca catacagcag tgggctggcc aggtgtcata tgacacccgt
4500

gtctaagcag actaaagaag tacacatcag ccaggcgtgg tggctcatgc ctgtaatccc
4560

agcactgtgg gaggccaagg tgggggtgga tcacctgagg tcaggagttc gagatcagcc
4620

tggctgacat ggtgaaaccc catctctact aaaaaaatta aaaatacaaa aattagccgg
4680

gcgtggtggc aggtgcctgt aatcccagca acttgggagg ctgaggcagg agaatcgctg
4740

gaacccggga ggcggaggtt gcagtgagcc gatattgcgc cattgcactc cagcctgggt
4800

gacagatcaa gactctgtct caaaaaaaaa aaaaaaagta tacatcaagg ccagaacagg
4860

aaaagacatt tccaagagag gtgatatacc cagcacaggc tttgagggtt ctttactttc
4920

ctgtatggaa atattccagg ccagaggcag gctcttatgc aaccacacaa gggccggcag
4980

cctgtgtctg cctttcccaa catctttact atctgtcccc ttacaagaaa gagtttgccg
5040

atctccagat taatgtgata tgcacttcag cccgcagaga tcatgatggc ttctgagtta
5100

ttgggttctg aattaatgag gtttctgtgt atttcctaac atgtgctatt tcaaactctc
5160

agatacttag attgggcagt tctcctctcc ttggcccagg ggcccactgt gtcattgtgg
5220

cctgtcagat ctgcatccgg gctcaaagcc aggttacatg cattctgctt ccctggatag
5280

gatctctgtg acattgctac cctggaggat ttgtttctac ttcaattaaa tcagattagt
5340

ccactcacat gctcttgtct tgggcaaaga gccctgaatt tggaacccag ttgaagtccc
5400

aactccatca ctgcagaact gggtgacttg ggacaaatca ctgcttggca tggggctgac
5460

gtatcaagaa ggcctaagta tttccgcatc tccctggaag ccttctcccc atgtttaaca
5520

tgaacagctg caatgagaga cagcactggg atgcttacag gcagcagctc cagaccccca
5580

tgatctgagt ttaagtctgg atctcattac ctaacctgct ctttggccaa gtgacttcac
5640

ttccctgtgt cttggtttca tcatctgtac actgggaagg ataataatag gctctatctc
5700

ataaggttat ggggacttca tgaattgcta ttgctcaagt aacaagctat atatctgcca
5760

aatcaataca agttgtttgc tgatacagaa caatctgtgt aaactcagag agtgacagcc
5820

tgagatgggg cacagctgga cccaatcagg aggtcctgga gtatgaactg tgtccgctta
5880

aggtcacagc tgttgtaaga agatcctgga gaaggaccct ggccaattgc aggagggtgg
5940

ccaggcctca aaatcaagtt ctcccaggaa ggaatggatg atggggaaca gtttaacctg
6000

taagtgaaga aaaagcaagg acagtgggac tcaatggctc tggatgaaga tggagcataa
6060

aaatggttct tgccttgagg ttaaaactgg ggcaagagac ggatgttcca gtggggaggg
6120

cttctgcccc atgaccagga agacatgttg gccccaccga ggctggctga cccttctcgg
6180

ctggggtgct gtaaggagct tcttggttaa ggtgcagaat gggatgagat gaagcatgta
6240

aggcccctcc cagatccaat ggtccagact aatatttctg ttggctttga gattggactg
6300

ctgataagtg tggtaggaac aaagccattg ctattttaac attccaaact ttgaagcttg
6360

ccaagaactt tataaagagt gtctgctcaa atttcccagg ccgcctcctg atgaagtgct
6420

cagtggcctg acattgctat gagaccctcc ccgcttcctc ccatggatta tgacacaggg
6480

atggtgatga cagctcctgg caaggtcagg agtcagctca gcctggcctg agtcatggga
6540

aagtaattta tatgagagct gtcattgctt aagaacccaa agtaggtagg acctccaaac
6600

tctcttgata tttgccatag ttgcctttga aacctcaaaa aggccatcca gacctggccc
6660

ctagtttacc cggaatgcag tggtgtctgg actttatttg aatggcctgc aagtctgtaa
6720

agtcattcct agtggtagat ttcttatctc ctcagccaaa acaagcacag agagttctgg
6780

ttatgaggca ataaagttta ttaggctctg ggattacatt atagcatgac ctgttataca
6840

cggtttcaga gatgctgtgg ttccagtcaa gctcccccca ccatcatcta cccggccaac
6900

aatggaacag actggtagtg aactcctggt ccctggaggc agcaaaagaa gccacttacc
6960

tgagaagtat gttatagagg gtattctgca aacttaagta gaagaggggc tgcaaccttc
7020

aatgcctaca aaggctgaga aggcagtgag gaggagggaa tttggcccac ttggacagga
7080

gagacagatg gtgtgcctgc ctctgcagac ggccactgct caggtcaacc aggtgttggc
7140

atgccggatt gcatcgccag attgtccgat ttttctaaaa ggaagctgaa aacctagatt
7200

tgtatgtaag aatctctcga ttttaaatgt tggaaactaa tttggatctt gttggaacac
7260

cagtttgcca cttttgcaag tagagttact ctctgctgag tatctagggc aggagtctgc
7320

gcagctgacc gcggtctcag taagaacccc ttgttataag aagaatctgt tagccttggg
7380

tcacagtctg tagctttctt caagttcatc ctggcctgcc acctgcagcc ttgtgtggag
7440

ccattggcct gagggtgaag gggccttggt tctggtccca gccctgacac tgatgacctg
7500

tgtgacctca ggaaagtctc tgtccctctc tggcctgtta ccccttctgg aattagccta
7560

gtgtgtctct ggcccctgcc agctcaacca gtttaggcct ccacctggga gaaacccaca
7620

gtggagaatt ttccaaagca gagctggttc ttgatttggg gtaaatgatt gttttctttc
7680

tttccttctt tcttttcttt tttttttttt taatttaaga gacagggtct cactctgtta
7740

cctaggctag agtgtagtgt gtagtggtat gatcataggc cactgtaacc acaaactcct
7800

gggctcaagc aatcctctgg ccttggtctc ctgagtagct gggagtacag gcaagcacca
7860

ccatgcccag ctaattttca cattttttgt agagataggg tctttgtatg ttgcccagac
7920

tggtctcaaa ctcttgccct caagcaattc tcctgcctcg gcctctcaaa gtgttgagat
7980

tataggcttg agccacggca tccggcctat tttctagact gattgtgcat ctttaggtat
8040

ttccaaaact gagaaaataa tttaccagaa aggaggcctc ccagtgactt ccagcatttg
8100

tgccgatggc tgatggagtg acctgttagc atttgtcaaa tgccagtggt gaaggttagg
8160

tggtgacaga aggtagatgg ggcttgtgac tgacggaaaa cagcaggtga ggggtcaccc
8220

cagaggcctc tcatctctgt ccttacttac tgcagcctgg gtagtccttc ctgtctgtgg
8280

gcctcagttt cctcctctga aaatgagaaa atcagtttgg ctggatggtt tctgagtccc
8340

ctttgagccc cggagttctg tgattccaac gaatttatgt ttcaaagttg tagttttatc
8400

agctgcacaa ggacttaaat ctttttttca attcaacaga tgtttcccct tccttcctcc
8460

ctccctccct ccttccttcc ttctttcctt ctttccttcc ttccctccct tctttccttc
8520

cttccttcct tccatccttc cttccttcct ctgcctttct ttctctcttc tcttctttct
8580

tcttactctg tcatccaggc tgcagtggca cggtcatagc tcactgtaac cttgaactca
8640

tacagatgat cttggagata gctcttctga ggcctgccaa caggtagtaa atgagtttgg
8700

aattggaccc tccaccagcc gagccttaag atgaccacaa ccaaggccaa cacctcgtga
8760

aaacccttga gtccgaacca cccagcgaag ccacagaaac agagaaacca ctattagttc
8820

cagctgctaa gtttttgggt cattagttca gcagcaatgg gtaatcagtg cagcccttct
8880

gccacgagct gagctcttgg ggctggggct gggaagggaa gggttaaccc cagagcccag
8940

actttcaggt gggcagcaag gttagtgcct ccagcttggg ggaccgttca ttacagagtt
9000

acccagtggt tcaaccaacg tttgggtctg ctgccagctg ggcctcagcc ctcctgtggc
9060

acctgtcagg gcaggagcat tctgttgtgg ggggcggggg gtgggggagg agggacattc
9120

tggaaactga ctgattcatt agcagctttg cctccctgca tctgcccgcg atgcaccagc
9180

tgcaagtaga gccggcacag ctgatcggcc tgtgttcctg gcaggggtca gggctgcctt
9240

gactcctcag ctcccgaccc tgggcctatg gttttgagtc cagagggagg tgttgaggag
9300

ctcccctaaa ccccagagac tgctgataaa ccaggcccac aggcaaggcg cacctcctgg
9360

gtgccatccc catcttgttt aatcctcatg gtaaccctcc caaagtaggc atgcccatca
9420

gcgccatgct gcaggaggaa accgaggccc aggtttgtgc agctggaacg cacagagctc
9480

tctgactcca gagtccacct gatcatccgc tctgcttcct tggccccacc atggggagca
9540

tgatggatgt ggtgcctcag tggtttgggg gagctgcttc gaggaagaag gtgggagagt
9600

tgaggaggca gggctcctga cccccctact ccccgctcca ccgaaagcag ctctactact
9660

ctgtgctatg ccattagggg atcgggtaca atgtttcaca gtgtttcaca aatgtgtggt
9720

ctgaacagag aatgttccag tcccaagtcc ccataccgtg tgacctgagg agccaataat
9780

aacccctgtc tcctgaggaa ttatggagaa ttcagtgagg tcaaacaggc aaagctcaga
9840

acacagtgcc aggcacacag tgagagctca gtgtggtggt ggtggtggtt gtggtggttc
9900

aaggaaggat tcgctttaat caaagggcct gtggtatgga gaaaacattc ctctaggcaa
9960

tggggcgcct ttggaggctt ctaacagagt gacctccctc aagtttgtgt gtggagaggc
10020

ctccagccca atgcccacca catgggggag ccgtgttctc tgccagaaaa cttgaaatag
10080

gaagataagc ctggtggggc ggggttaggg gaaagggagg agaaataacc gttgtgggtt
10140

ttctgcgaaa gcagagccta ggggaggttg aatggacagt gacaatatta gggattgaag
10200

tggagccata aatcctggct cattatcccc agaaaccaga gggatcaggt tgacttgaag
10260

aggagccaaa ggtaagaaga gaggctggct cccaaggtga gaagagctga ggtccagcat
10320

ggggaaggac cagcctctcc ctctgagtcc aggttctttc tctggcactc ctggaggcag
10380

cccatcctgt ccctgcatga accctgccag taatagggaa ctcaatgaca tgtgcagtag
10440

cctgttctca cgaggttcat ccctggatgt tcaaaagagc ttcctgcctg ccgagtgcaa
10500

accttccccc agaagcttca ttcaataatt cagtaagtct ctgggcacct accatacgcc
10560

ctgccctcat gaggaaggag caacatagca ctgagcaaga caaagtccca actctcaagg
10620

agcttaggtt cagctgtagg aaggcaaata ttatacaatg atatcacata atgccagatg
10680

ataatacggg ctatgacaaa aattaaaata gagtgacatg acatagtgac tacccttgat
10740

agggtggaca gggaaagtca tgaaacattt aagggcaggc ataaatgaca agaagagact
10800

agctatggca aactgcattt tccaaagatg gcttgagcag tatctcccat cacattcact
10860

cttatgcagt gtgactcacc actccttcag caagagatgg agtctatttc tccagccttg
10920

attctgggtg ggacctgtga ttgctttgac caatggaata aggcaggagt gatgctgtgt
10980

tatttctgga tgtaaccttt aattactcca gtagcttttg cttctgggtt cttcggactc
11040

tgtttcttag gccacttcct cttggaatcc agctgccatg ctgggagaat tctaagccac
11100

atggagtggc caaccatgta actgggctct cagccaacag caagcgtcaa ctgcccacca
11160

tatgaatgcg ctgtcttcca tccagccctc gagccttcag atgactgcag ccctagcccc
11220

catctgactg cagctgcaga aagaaacact caactgggca cagtcaaccc cggaaacata
11280

ataaattact gttttgtttt tgtttttgag atggggtctc actctatcac ccaggcggga
11340

gtgcagtggt gtgatatcgg ctcactgcaa cctccacctc ccaggttcaa gcaattctcc
11400

tgcctcagcc tcccgagtag ctgggactac aggcgcccgc caccatgcct ggctaatttt
11460

tgaattttta ttagagacgg ggtttttcca tgttagtcag gttggtctcg aactcccaac
11520

ctcaagtgat ctgcctgcct cggcctccca aagtgctggg attacaggcg tgagccacca
11580

tccccagcaa taaattacta tgttatgtct ctaactttta gagtataaga aataagtaga
11640

acaccacaga atagctttgt gcaaaagccc tgaagaggga aagagtttgg gatattttga
11700

gaaatcaaaa aagtccagtt tggttacagc agaatgggtg actggggaac tgacagagag
11760

aggcaggagt gcgccagacc atgcagctca tagtaaggac aacagggaga cattgaatgg
11820

ctaggcaagg gcatgcatga ttggatttat gtttgagaaa ctccctctgg ctgcagggag
11880

aagaatgaat tctagggggt tgtggtattt tttaataatg ttgaattttg ggaggctaat
11940

attcattttt tatttattca ttttttaaga gacaggatct cattctctgt cactcaagct
12000

ggagtgcagt ggcttgatca tagctcactg cagccttgaa ctcttgggct caaggatac
12060

cgccacctca gcctccgagg tagttcagcc tcatacaatg aattggagtt tattcactct
12120

ttttctattc tctggaagag attatcaaag atgaatgtta gatagcactt accaatgaag
12180

ccatctgagg ttgaagtttt cttactggaa gggttttaac aactgattca atttatttaa
12240

ttgttaaagg actatctagg gttttttatt ttgtcttaaa tcaaaattgg aatgttatac
12300

cttctagtat gttgcttatt tcacttaaaa tttcaaattt attggatcaa aatttttcaa
12360

aatagcctct tttttcttta atgttggcag catctgtagt gatgttcatt ttttatttct
12420

aatatttttg tgccatttct ctttaattaa tcttcatgaa tattcatctt ttaactggag
12480

catttggtct tttgatattt aatataattt atcaatgtat atgggtgcaa acccactagc
12540

ttattttgtg atttctcccc tcccctccct ctgtccctcc ctccctccct tcctccttcc
12600

ttcctttctt cttaccgcct cctccctttc tcccttcctt ccttccttcc cattttgaac
12660

tgattacttt tgcgttctat ttcccctctc cactagtttg gggattatgc actgttttta
12720

ttcttttaat gttatcattg aaattacaac atgaatattt aacttactga agtctaaata
12780

taatcagtac cattgctctt cttccaggcc ttagaacact gctactctaa tttgacatat
12840

actccatggt tttggtgtat tctaattatt tttttaaaaa agataaactc tacaagacat
12900

taatgttatt gttttatgta gtcagtgctc agctagactg gcagatggat tgatcatttc
12960

tttgttcttc acttctcctt tcatctcaag ccttccatca gtgttcgtat ttcttctaca
13020

taaagtgtac actgtaacaa gtttcaacaa gcagaatgtg ctctattctt ttttgtctga
13080

gcatgctttt atttaaccca tattcttgaa gtacatattg atgagcatgg aattttagtt
13140

gggtagttaa tttctttcag cacattgaat gtatcatgtt actatcacct ggctctgttg
13200

ttgctgttga gaagtcttca tcactctgtc atttctttga aggttatttt tcttattttc
13260

tggttgctcc ttagattttt ctctttgtct gtgatgtttt tctccttcac tgtgatgtct
13320

agacgtggat ttattcttat cactttcctt gagatttgtt gggatttttg aatttgtgga
13380

ttagtacctc tcatcagttt tggaaagttc tctgtcatta tttcttcagt tagtgtgtct
13440

gccccatttt ctctcttgta tttttctgaa actgctatta aatagatgtc agatattttc
13500

agcctctctt ccatgtctcc caacttctct tttatgttta acacctatct acttctctgt
13560

actatattct ggatcattta tcttctgcct cactcgtgtt tttctttagc tctgtttaat
13620

cttctatcaa acccaaccat cgacttctat gtttcagtta ttgtattttt catttctaga
13680

agttctattt gggtttctct attttttcag ttattaaaat tgttgcaaaa atatgtaaca
13740

taaagtttac catcttaacc atttttaagt ttacagttca gtggtactaa atacattcat
13800

aatgttgtgc aaccatcagt atttggttct cttttaattc tgctggtact tttcatggtt
13860

tctgttccca gtagatattt gtaaggtaac atgccttcct tcctgcggtg ccccatctag
13920

attgctaatc ctcacttatc tgtacccatc ctcccaaatc tggaggtaag tctcatctcc
13980

ttttaaaacc tttgactgga cagtcatggt agaaactaaa gccatcatca tttaatcatt
14040

ttgtgtttac tcaaaaaaca aagaaacaaa caaacttcta ttaagttcta ggctgttctc
14100

tagggcatgc aataatgaat caggcctgga accttccatg gaggacctgt ttcccggggg
14160

gaggctgata tgagaaaaag gatatttaga atgaattcca cataccttct ttgaatgcca
14220

gctgtgtgct ggtcacattg ctgggttctg agtaagcagg aggtaagata caatcttctt
14280

ctcaaagttt gtagtagagt agcagagatg aatgggccct taagtaatca tgtaaaatgt
14340

caaaaatgcc aggcacatga ttggtgcctt ttaaataatc tctgagtgaa tgaacggtga
14400

attggcgtgg gagttcagag gagagaaaaa ttttttctga ttggaacata ttaaggaaat
14460

ctgtaatggc atttgagtca ggccttagaa atctgttact tttggacatc caaagacata
14520

ggagaaggta ggggcaattc aagtaggaaa acagcatgaa taaaggcatg gactgtcttt
14580

ttattttaaa ctgctctatt gagatataat tcatatatcg tgaagtccgc ctgtttgaag
14640

tgtacaattc aatggtttta agtatactta agagtcgtgt gaccatcacc ataatttaat
14700

tttagaacat tttcatcatc ctaaaagaaa actactcatt aagtcattcc ttatcctcct
14760

gcctctccca ccaccacagc cacaagcaac cactaatcta tcttctatct gtttatttat
14820

tctgaacatt tcatataaat gaaatcgtac aatatgcagt cttttgtgac tggcattttt
14880

catttacaat aaagttttca tgtttcatcc atgttgtagc atatatcagt actttattcc
14940

tttttattgc agaataatat tccattgcat gaatatactg tattttgttt actcctttat
15000

tcactgcatt caaagaatgg atgtttacta tcatccattg atagacactt gggttgtttc
15060

tactttttgg ctcctgtgaa tatttgtgta caacttttat gtgggcatat gttttttatt
15120

ctgtaattcc accaggaatg ggattgatag gtaaaatggt agctctatgt ttaacctttt
15180

gaagaactgc cgaactgttg ccaaaacagc tgcaccattc gatgtttcca gaagcagtgt
15240

atgaaggttt taatttttcc acctccttgc tcacacttgt tattatctgt cttatggatc
15300

atagcgttct gctgggggtg aagtggcatc tctttgtggt tttgattttc atctcttagt
15360

ggctaatgat gttgaatgtg agcacctttt catgtgctta ttagccattt gtatagcttt
15420

tatggagaaa tgcctattca aatcccttgc ccatcttttt ggggggaggg ggacaaggtc
15480

tcactctgtc acccaggctg gagtgcagtg gtgccatcat agctcactgc agcctcaaac
15540

tcctgggctc aagggatcct cctgcctcag tctccaaagt agctgggact attatgtgcc
15600

acaggtccag ttaattgttt ttttgttgtt gttttttaga aatggggtct tgctatgttg
15660

ctcaggctgg tcttgaactc ctggcctcaa gacatcctcc tgcctcagcc tcctaaagca
15720

ctaggattac aggcatgagc cactgcaccc agactattta ccttcctatt gttgaattgt
15780

aagggttctt tatatattct ggatgctaga ctcagatata taatttgcaa atgtttcccc
15840

catcccacgg cttgtgtttt cactttatgg cattctatga gatggaaaag tgttaaattt
15900

ttatgaagtc cagtttatca gtcttctcct gtattaccaa tgcttttgct attatatctt
15960

atgttctgtt ttgtaatgtg gggtgagtcc cacgggcggg ttcagtttgt ggaaactcat
16020

caggctgtta taatttgtac ttttctgtgc ctgtaagact tcaaaaccca gttgtaaaaa
16080

acaggactga taataattgc agcttccttt actggctcat cgtgaggact ggatgggcaa
16140

acatactcat agtgcttagc acagagccag ccactgctta gtgctgacac gtgggggtga
16200

agttgttact gtagttattg tttttttgtt tttgtgcttg tttttgtttt tgagatggag
16260

tctcgctctg tcgcccgctg gagtttagtg gtgcgatctt ggctcactgc aacctccgta
16320

tgccgggttc aagcaattct cctgcctcag cctcccgagt agctgggact acagctgtgt
16380

gccaccatgc ccagctaatt ttttgtattt ttagtagaga cagggtttca ccatgctggc
16440

caggctggtc tccggctcct gaccttgtga ttcacctgcc tcggcctccc taagtgttag
16500

gattacaggc gtgagccact gcgcccggcc tgtagttatt gttaaaatcc agctctgtcc
16560

cttgtcgggt ggtgatcttg gccctaagtc actttacctt gttggccctc actcttctca
16620

tctgtaaaat aggttagcac catccacttc accagggcca ccagggcccc caatgaaaca
16680

agatgcgaca tcatcaggct tgaagtctgc ctccaagagg gcctcacaaa agcttgggtt
16740

catgaaacag ggaagaggaa agagaaaaat cagactcatg attgaagagg gaaaattgtc
16800

agtgcggtga tgagtgtttg ggtgcctgcc acgaactggg tgtggggcgg cccctcagag
16860

tttccacaag cagggttggc caggcctggg aaggaatgaa gcgggagttg gccaaaagag
16920

agttgtcttc caggggagtg gggtctggtg gtgccagggc gctcagcttg ggcagggctg
16980

gtgtacaaat cttggcttta ggcctggctg ggcaaaagtt gattgactct cagtgcagaa
17040

caatcagaaa ccaaacctcc ctctagcccc tggcccccca cccccattgg ttgcccctat
17100

gtgattccag tcttcctgtc ttcacccaaa caatcccacc cacacctccc ctattccttt
17160

cttgttcttg aggtctcgct ccctgccccc atcaccaggt caggttttaa gaggcctaaa
17220

acttatacca tcggaggaga gagtttcatt aaagtagaac atgtatagag agaaggcaca
17280

aatcatgtct acagcttgat gaattttccc aaactcaaca cgctcatgta aactgacatt
17340

caagtcaaga atcaggccag gcatggtggc tcacgcccca gcactttggg aggccaaggc
17400

aggtggatca cctgaggtca ggagtttgag actagccagg ccaacatggt gaaaccctgt
17460

ctctaatgaa aatacaaaag ttagccaagc atggtggtac acacctgtag tcccagctac
17520

tcataaggcc gaggcaggag gatcacttga acccgggaag cggaggttgc agtgagccaa
17580

gattgcccca ctgcactcca gcatgggcaa caaagggaga ttctgtctca aaaaaaaaaa
17640

aaaaaaaaaa aaaaaagaat cggaccatta ttcccagcat cccagaagcc tctcggtacc
17700

cctttcttgt tatccacccc ttaaacataa ccactctcct gacttctgac gtcatagatg
17760

agttttgcct ggttttgaac tttatatgtt tgaaatcatc cagtgcgtgc ccgggtgtct
17820

agcatctttt tttttggttt gagatggagt ctcactctgt cacccaggct ggagtgcagt
17880

cgcgcgatct tggctcactg taagctccgc ctcccgggtt caagcgattc tccttactca
17940

gcctcctgag tagctgggat tacagttgtg caccaccaca cccagctaat ttttgttttt
18000

tagtagagat ggggtttcac catgttggct aggctggtct caaactcctg acctcaactg
18060

atccgcctgc cttggcctcc ccaaagtgct gggattacag gcgtgagcgc cgcgcctggc
18120

aagagtctgg catcttttac ttaacatgtt agcaagattc aaccatgttt gctgcatgca
18180

gctgtacttt gtttcctctc attcctatag cggcccttgg aatgacaata ccacagcttc
18240

tatgtccact cagcaagcgt tgcattttgg ggttgttttc agctttggga tgtctggggg
18300

aaaagctgct atgaatattc ttgcatgtgt cttttggggg acatacgggc tcatttctgt
18360

tgagtcatcc atgaggggag ttgctggctc acagggagag atgaatgttc cgctgtagaa
18420

gattcaagga ctgcaaattg tgttccagag tggccacacc agcttcctct ctcctcccag
18480

ctgtggagga aagtcctgcc attccacatc ttcactgaca ccttgtgtgt gtgtgtgtgg
18540

gtatacacag tatgtgtgtg tggtaggacc tagttcatcc atgcaatgca cacagtgtat
18600

gtgtgcatat atactgtgtg tgtagatatg taaacacaca tatggtgtgt gtgtgtatat
18660

atatatatgt atccccatta ttctgagccc tagtccttcc ctcctggcac ctctcacttc
18720

acactggtgg tctgtctgaa tgttctgtgg gacaagcctg tcctcaggac ctaaatcagc
18780

acctggccct ggcctgtatt aggagctata tagatgtgtg taagtatata tatatatata
18840

tatatatata tatatatata tatatataca cacacacaca cacacacaaa taggtagata
18900

cacacacaca tatatgtgtg tatctattta tctatttttt gctttttcat ttttagtcat
18960

tctcataagg aggctgtgat accacatcat ggttttaagt ttgagggaac gtctttaaga
19020

aaaagaagcc aggcatggtg gctcacgcct gtaatcccag cactttggga ggccaaggca
19080

ggtggattac ctgaggtcaa gagttcaaga ccagcctggc caatgtggtg aaaccttgtc
19140

tccactgaaa gtacaaaagg tagccaggtg tggtggcatg tgcctgtaat cccagctact
19200

tagaagtctg aggcaggaga atcgcttgaa cccgggaggc ggaggttgca gtgagccaag
19260

attgtgccat tgcactccag cctgggtgac agagcaagac tctatctcaa aaaaaaaaag
19320

aagaagaaga agataaaaga aaatgataaa ttgagtacac gaaaaagata aaagtaaaag
19380

aaaatgataa attgagtaca aaagtaaata tttataaaga atgtgaaaag aaatcacaac
19440

aaactgctgg agactttcaa gtttcgattc ctttcttctg agtgtttctg agcatttctg
19500

agcctgtttc ccagaaatgt gtgcattcag atgcttcctg atagtgacct gggtccctgc
19560

cctgtgtgaa cactgccggg cttcctggga ttcctggtac ggggcccctg cagatgagtc
19620

tccctgaggc ttaaatgtca tcagcttccc aggaagtcgc ctctgcctgg tgctcacctc
19680

tggcccctgc ctaacacccc agccccagct cacaaacttc cccacctacc cctcctcctc
19740

ttctgggctc aaaagctccc ctgctcacaa acactccagc tcaaaaactc cccagctcac
19800

aaactgcccc acctacccct caccctttct tggactgact cttcctcccc aggctctcag
19860

cttccatagt agcttccaag aaaggccttc tctgacttcc ttgtctagga tattctccat
19920

ttttaagtcc acagcctgtg accaatggca acagagcaga aggttagagc aattactgtg
19980

gatggagctt gcccagtagg cctcctagct ccattttcta gctttgtgat ctcaggcctg
20040

tcctgtaacc tctctgggcc tcagaaacct ctctctctct ctatatatat atatatataa
20100

taatatataa taataatata taatatgtat aataataata tataatattt ataataataa
20160

tatatataat atgtatatat aatatacata tacctattat atataatata cctattatat
20220

ataatataca taaacctatt atatataata tacctattat atataagata cctattatat
20280

ataatatacc tattatatat aagataccta ttatatataa tatacatata cctattatat
20340

atataatata cctattatat atatataata tacctattat atatataata tacctattat
20400

atatataata tacctattat atatataata tacctattat atataataca cattatatat
20460

aatatataac acacattata tataatatat aacacacatt atatattata tataatacac
20520

attatatatt atatataata cacatatatt atatataata cacattatat attatatata
20580

atacacatat attatatata atacacatta tatattatat ataatacaca ttatatataa
20640

tatataatac acattatata taatacttat tatatattat acataatata catattatat
20700

ataatacata atatacgtat attatataat atacatatta tatataatac ataatatacg
20760

tatattatat aatatacata ttatatatat tatactatat ataatactca tatatataca
20820

tattatatat acatattata tataatactc atattatatg tacatattat acatacatat
20880

tatatataat acacatatat acatattata tataatacac acacacatta tatatgtctt
20940

atatataata tacatattat atatgtatta tattattata gatataatat acatattata
21000

tctatatata aaatatatat gtagagagag agagacaggg tctcattttg tctcccaggc
21060

tggagtgcag tggtgcaatc ttggctcact acaacctcca cctcccaggc tcaaggatc
21120

ctccagcttc agccccccga gtacctggga ctacaggaac gcgtcaccat gcctggctaa
21180

ttttttttta ttttttgtag agatggggtt tcgccatgtt gccgaggctg gtgtcaaact
21240

cctggactca agtgatcctc ccacctcagc ctcccaaagt gctgagatta caggtgtgaa
21300

ccactgcacc tggacttaca gtcttatttt cacatataaa gcatcttgtt taaggctcac
21360

aatatcccta ggaggtgtac cctactggaa cccctatctc ccagaaaagg gtatggaggg
21420

gactcaggac ctgagttgcc cgaggcacct gacctcaagc ctgccctgaa aaacgccctg
21480

tgctccccga gaaggccccg catagtgcct ggcgcagagt gtgactcaaa aacattgatt
21540

cctttttctc catccctttg aactttcaga acccttgagt attgtatttt ttttaaaatt
21600

cacattgaag tatatcatgc atacaaacag agtgcgatca tgcatatgcg gttgacacat
21660

cttcccatgt tgaacgtgct tgtttaccca gcacccggat gaagaaaatc acaaccatcc
21720

caggcccctg cagcccctca ggctgccttc ggcctctcta ccaccagggg aagcatgatc
21780

acggctgttg gtgaacttca catgggtaga accgtgtagt cctttgtgtc tgcctatgac
21840

cctcatggtg gggttgtcat cgtagagcct gcgttctcaa ggctgtatgc accatcattt
21900

ttccatcact tttgcttttg atgggtctct ttggcctttc ccctttgaaa tggaacacag
21960

ggcttctcgg actcccagac gtgtctctgt gcctctgccc aggcctgttg gggagggagg
22020

tggggaggct gggggagtgt atgttctgac ccagtgccag ccccaagtaa acgtgcttct
22080

aggaggccct ggcttggcct tccaccctct gcccctcctt ctccgactcc atcaacattc
22140

tgttgttctg agcttggctt gaggccaatg ggtgagctgg cgggcatcct aggtaaacaa
22200

aggcagctgg acagaagaaa gtctttaggg gtgggagggt ggacccaatc agaagccctc
22260

tccacgaggg ctgtgagagc ttatatgggg ttcctgccct gagagccctg gaacatggta
22320

ggacgggagg gtgccggtag aggttgggcc ttctccaggc taggccctgc cccggggagg
22380

gaagctgcct gggttcaagt cccagctctg aaacttacca gctgtgtgac tttgggcaaa
22440

tttcttaacc tctctatgcc tcagtttttt gatctataaa atgaaggcag taaataactc
22500

attgcatgta aaggatttct gacctgcagt caatgttggc tgttaggagg atgatgtgcc
22560

gagagacagc taggtacttg atgtgcacta ttttattgaa cctgcgcatt agtcatggaa
22620

gtagacacca tgtcatcccc atttggcaga taaggaagtg gaggcacaga gagggtgaca
22680

acctgcctga agccacactg tgagtctgtg gcagagccag ctctgatccg gagtccaggc
22740

ccctggcacc aagctatgag cagagcttgg gtggggattg gcagccctga cctgccaaag
22800

agcagtgccc agcactgtcc ttgtggggcc cagactcagc cctcacatgg ggtttgaggg
22860

gcaaggcctc aggctcattt tctctgtctg taaaatggac atatgtgccc caaagagtca
22920

gtatccttcc ccttcctggg ttcctggcta gtgggctccc cggagtgggg caggcttggg
22980

ggctgatcag ggaggcctgt gcacctgccc agtgtcagca gccctggctg gagcagccct
23040

gcccctgctg tatagccggg aggctgttca ggcctggaag ccagagttgg cagcccttgt
23100

tggggctgtg ccctgaccct gccctcaggg tgctccggaa gaagggacag gcacagaaac
23160

tgctattcct aactaaggct gccgagtatc cagtatcgta aaggtctggg gaggtcaagg
23220

cagttggctg cagtccagga aggcttcaca aaggaggcct ccttggggca gggttttgca
23280

ggttgagtaa gagttcatta ggtagagagg atagtaaaag tgtttttagc acagggaatg
23340

gcaataggtc ccaactactc catccttgcc cttgcttacc ctctctgtat ttcagggggc
23400

tggaagttgg aaaatgacat tttccagagc tgcttcccag ccggtttcag gttgggatct
23460

gccagtggga aagaggcctg aggaggggag atgccagcct gcctctggct gcagcctcag
23520

gtggcttccg caggctctgt atttcccagc agctatgttg gttctagggc tgtgggcgcc
23580

agctcgtggg ctttgcagct gcttcccaat ttctaggtca cactccttct cccatctgct
23640

cctctgcccc tttggatact tctggaatga acttcctcta tgaactccct ctctgcctga
23700

aatagtagtg cggtttctgt tttccagctg gtgactcact gatacaatta tcaagccacc
23760

cagccagcac caggttgtag cgtccaagga acagaaggat tcggcaccca gaacacgtga
23820

tgtcatgttt ttattttgat ttatttttta tgtgcatgtc atgtctttct acatgacttc
23880

ccaagggtgg ggactgtccc catcatctat tcattcaaca gatactcatg agtgcttcga
23940

tggtggggaa tgagatgaat gaccaagtct gcacatccac ggaatcgcag tctagacctg
24000

cttcatccag tactagccag actggctacc gagcacttga aatgcagcta gttcaaattg
24060

agacctgctg tcagtataaa acacacacca catttcaaag attgagtaca aaaataagaa
24120

ggtcaaatac cttactaatt cttatattga ttacatgttg aaataatgtt ttggatatat
24180

tggattaaat aaaatacatt attaaagcta attccaccta tggctttttt actgttttca
24240

atgtggtcac tagaaaattc taaattacat atgtggcttg catttgtggc tcatgtatta
24300

tttctatggg acagcacagg tcttgtggga gttgagttag gaaacattca tttaccaatt
24360

cagcatttat tagtgtattt aagagatacg tggtccctgc ctcgagggct tcagatagga
24420

tggagaaggg gacagatagg ccaacgtgga agtaaaattt actgtgataa atgtttgaat
24480

tggtccatat tccaggactc tggggacccg aaaagatatc aaggaaggtt tcacagaaga
24540

ggtgactttg gtgttaagca ttataggatg agtaggagtt tgccaagcag ggaaagaaaa
24600

ggaggggcac tccaggctca gggaactaca tacacaaaag ccaagaggta gacaactacg
24660

cagggtgttc tggggataca ggaagttaat tgtggcccaa aaacagactg ttggtggaag
24720

gggccggaga tggagccagc ctgtgtagga ccttgaatgc cagtaaagtt taaacgcagg
24780

gacagggctg aggctcagga aagactccta ggcaggaagt gggggtccag gtgtgagcga
24840

gctcacctac ccatctccct ccaaactcat aaaggaacag accagaccta gtttgagtgt
24900

gaaaaataaa ctattttatt tcagtgtttg ctccttgcgg ttcagaagca catctactgc
24960

ctggttggaa cccaaggctt ttataaaacc gtagagaaat agagctctat gtatagagaa
25020

aatatacatg ttgattaatt gtgtgactct ttcctgtgca aagcagaaag ttctaaatgc
25080

aacagcatga ttctctccaa gtccttccct gggatttggg gggccctgga ggctgtgatc
25140

tcacctccaa tagagaatcc ccaattcttc cagcccaagg gaggcccagt catgtagaaa
25200

gagcaggaga taaagtcaaa gctgacaact catgggttcc ccaagcttcc tccggggcag
25260

gggctatgtt tgggggcctt agccctgcaa agaaggggta gctggggtgc caaccttgtt
25320

ggttagtgac cagcactggc agctaaggct gttgggaagt ctaggcattg cagcgggcag
25380

gctcatgggt agagcagggg tgtccagggc tcttctcccc tagaactggg gtctgaaggg
25440

tggtacctgg gcataggaag gaaccaggac agggctgggg acagaaggtg gtcacagtca
25500

tggtttcact ctcagaaata tcctgggcct atggcttaag gcttcgtgga gcagggagtg
25560

gaccttgtgg ttatttacaa ggctgggcca tataaaagca ttgcaaacat ggagtggaga
25620

ggatccttgg agatgagctg gttcaatcac tcctctgacc aacaaggaaa caaaggccca
25680

gagaggagaa ggcagtgcct ggccagacgt gggacctgaa cccagccagg gctctgactc
25740

ccagtccccc agtcccctct ctacctcctt gtcttggctg agtctttttt tgataaaggc
25800

cccagacagc ctctccgaca gtctcaggtc aggctggggt tataaatgga gcagtggact
25860

cagagtcaga ggcccagact ctgctcttgg gccttcacat tacccagcct tgctaataac
25920

cacgaggcca ctggtgtgga gggggctgcc tcctctctct aagcctcagt ctccttatcc
25980

tggaagcagc gcccacaaca gtttcccatg gtgattaaat gggttggagg ccccgtgagc
26040

cccagcaagt gtgggaggca cccagcacat agtaggtgct tcacaatggt gaggttgagg
26100

gggagggcta ttaagcttgg gctgctggga tgtggagggg accttgggcc tatcactgtc
26160

cccacccagc tctggctgtc ccaagggccc ctggagctgg gtgcagtcat gggaagaggt
26220

acgggatgca gtctagggag ctggattccc tgatccagcc aaggggcatg ctgtcccttt
26280

tttgcctgct ccaaggagct atgaagtaga tcaaacaata tggtgggtgg tcaatggctt
26340

tccaaagggc atctgggcag gagagggaat gtaggtcttt cctctctcac cccagcccca
26400

gtcagccaag gtcagggctc ccactgtttc tggcacagag cattcctcac agcaaagggg
26460

gtgagtgggg acccacaggc tcaggaggct gagatgggcc aggggcacat tccatttccg
26520

agagccccca taggctgccc accctgccct ggggtctggg gcccaagaag gggcatattt
26580

tgctcactag cttgggataa ggattgggat caaggtgggg gctctcagca ggctgggagc
26640

aggggtccct taggcggggc caatgatgac attccggaag cccttcacag ccttcagctt
26700

gtcgctctcg aagttcacca ccagcttgtg gaggcccatg tggagcggca gcaggtccat
26760

tctcacctta acttcctccc ctgcctccac ggggtctggg ctgcagggag agagggggtg
26820

gtgaggaaag gggcccaagg cctggctccc gcatgctggg gtccagccag gaagtcacgt
26880

gacctccgag gatcagcaca gcgtgtccat gaatgggcct cattctcggc cctctgcggg
26940

gcctggacaa tctgccctcc tagagtgaac agtgcctgtg cacgcatgtg tgtccgtgtg
27000

cataaaagca gatggtgcat ttaggggcac aaggggatag gctcaggtag ccgtgtgtgt
27060

gaaaatgtat tgagctgtac ataggtgatc tgtgcttttt actgtatata agttacatta
27120

cagtaaaaaa gtaaaaaaga agtgtgtgct cgtatgtatg tgtgcagcta cgtatctatg
27180

tggaaacacc cacgcatgca tatgtgtgtg catgctcatg tgtggacatt cggagggcag
27240

atacctgtct atgtgaggat gcacgtgtct gcaggtgagc gcctattgac ggatgatgtc
27300

actgtggagg tctgtggcag atctgtgcat gggtggtgtc agcattgaca gagaatgtga
27360

cggtgggaac tctagcccca gggtgtctgg gagtgggcac agctgtgtgg gagatgagag
27420

gaggggattt tcagccaggg cttggtacct aaggacagtc tctaccccca ccggcatctg
27480

cccgcttccc ccaggcccca aagctagagc agccagcact tacatctcca ccgtcttctg
27540

ctcctcagtc aggccggccc cctccacagt gaaggtgcag ccttccaggg ccacagggag
27600

cgggttctgc agggacacct cagccaccag cttgcgtttc tgcttgggct ccccaaggat
27660

ctggaagagg gcatggggca gatgtcaagg gcaggtggga tccaggctgg gctgtctgca
27720

ttcactgcaa tttggcccaa tatttgctga acaaagctgt acccaggtct gccacgatca
27780

ccccccctcc ccccacctct gtgcctcagt ttcttcatcg ggaaaacacc gattgtgcta
27840

ccggcccagg tctcctgcat ggagctgcaa tgaggctcca cgtgacccag gggatgtcca
27900

aagtgcctcc cttgtcccag ggtctggagg ggaaagatca caggcttttg agccaggctg
27960

atctctgcct cttcttcact gtgtgacctg gggaagttac tccccactct gtacaccttg
28020

gattcttcat gggagagaaa tgcctgactg agggttcttg tgaggattaa ctgagctaat
28080

atgtgcaagc ttccagcaga atgtctgaca cactgctcac tgccgctccc tctgctgttt
28140

accatttaag tgtaattgtt gcccttaggg gttgtgggct gcggctcctc taagtgaagg
28200

gctataatga tgatgatggc gatgataagg atagttagct ttcagtgagc ccttatcatg
28260

tgctgaggat ggctctatgt gtttcctgtg gatatgcttt taattcttac aacacccctc
28320

agtgctatta ttaaccccat gttacagatg aataaactga ggcacagagg ggcaaactca
28380

catgcccagg gctgccctgg tacaaattca ggcagtgaga ctctagtctg tgcccttcac
28440

tcccccaccc caccctgccc caccagtgcc cagtgcctgg gggttccagc cttagtagta
28500

tagcatttga aggattcatg actctaagat ccccaaattc catgacccta acactctggg
28560

attttcagat tcattcctag aggctacgag tccccaattc tctaggcttc atgatgcttt
28620

tcttcgatga ggctaggatt tgaggatcgc taaggcacca aaggtctgga gcttgcaggg
28680

atgcaggtgt gtggggtggg ggtagggatc tgccctctcc ccagcgctga gctgccctgg
28740

gaccctgccc cttgcccagc ctgcccttac ccggatcttg atttctggat tctccaggta
28800

gaggtccctc tcagccagca ggtagctgtt gataactggc tccacgagga gggcccgcac
28860

cttgatgagg ttggactccg taaggcagtc acggtatttc tcatagagga tgcaaagagg
28920

aacgctcttc tctgcagaag gggagaaagg agggtgctca tgatgcagaa tcacccctcc
28980

caactctctt gcaactccaa gaggcacaga gaggggaagg aatgtgcttg gggtcacaca
29040

gcgggggaat ggctgggcgg atccctgaac tccccactgt ggctccaggg ttcccagcga
29100

ggagctggag agtggacact gagcagcggg tagaggacgc acttggactc ccacgggcct
29160

tggttctaat cctgcctttg cctcttctaa tgggtgaccc cgcgtgagcc actccacctc
29220

ccaggtcctc gatgtttgga tctgcgtggc acaggcactc ctaaggctgt gggaggatga
29280

gctgagcgag tgtcccggaa gagcctggtg cgctcgggac tcagggtgtg tgagccgcgg
29340

gcccttcatc ctggggagga tcagagccga catctgcttg ctgcaccagg cactgcactt
29400

cccacggcgg gctcctactt cacctacatg acaaccctaa aaggcttgta cgtgtattac
29460

cacccaactt acagatgaga aaactgagtc tccagcaggt gaaataacgt tgtcctaggt
29520

cacacagctg atatgtactg tgcaggcatc tggactaata atccagatct tagagtccaa
29580

caatatggtc cacactcact gaagaatgcc tctcatggac ataaggtcat ttccctccac
29640

gaggctcgtg atgctgtacc cgttgcacag atgaagaatc acctctgcca gtaagtgctc
29700

agattgggag acttgcctca ggccctgtct ggccactcac tggctgtgtg agcccgggga
29760

gatgacctcc cctctctgtt tcccctctgt ttcctctctg ttcccagatc ttatcacctg
29820

ctgggcagag gtaagaccaa gggtcgttgc agagcaaaag ccgcacagtg atatggggat
29880

gagaaagaag atggcaggaa caaagctacc cactgaagtg tggttcttag ccccatgttt
29940

ctgaaaagaa cactgaggct ggaggacctt agcagtttag cattttaagg attcatgtag
30000

tttagcattt aagaaaacag acctggggtc tgtttgtagg aggcagggag gagcagtaaa
30060

taaatacaag tagttgggtg tagtggctcg cgcctgtaat cccagcactt tgggaggctg
30120

aggcaggagg attgcttgag cccaggagtt tgagaccagc ctgagcaaca tagtgagacc
30180

ccgtctctat aaaaaataca aaaattcaag ccgggagtgg tggggtgagc ctatagttcc
30240

agctacttgg gaggctgagg tgggaggatc gcttgggcct ggaaggtcga ggctgcagtg
30300

agccgtgatt gcaccactgc actccagcct gggcaacaga gtgagaccct gtctcaaggg
30360

ggaaaaaaag agagaaaata caaacaaagt catgcatatt caattctggc cagatacagt
30420

gatgtgctgt ggactctgag tttcagacct gagtctactg ctaaagaggg agattggcaa
30480

ggcttaggag gccagaggca aatcagcacc ctcagagact ttccccagga ggtcaccaga
30540

aggacggatt gagacccgag gagacagtca ctttctcact gtgggattca aggttcaggc
30600

tttgaccttg aacctttgac cgtgaacact ggtggtctgc gtcctcctct ttggaaaatg
30660

ctgccccgac ctgcccacct ctttggcctt tcattccctg ggtggatctg ctccaacctc
30720

tcatcagatg atgtgatctg gggaagaaaa ccaaactttt gtgccaaaaa ggacaaaaga
30780

gggtgaaaat tactaggtca agacctggtg gtgcagagtg gaaattctag ctagctccac
30840

aggaaagaag aaaaacatta tcctccgggg gcttggagga tgtggcccaa ctgtcgacta
30900

ttctaggagg tggaggccag ggcagggctg gacctttgaa agtctccgtc cacaagccag
30960

tggccacggc cttggtcttg ggccagatgc tggctgggaa gaggaaacct gtatggtcag
31020

tctcaaccat tctttagaca atttgccctg ttctagaagg ttactctggg gacagtcacc
31080

gaggccacat tggctgcatt cctgtgaccc acctggaggg atgagggagg agagagcatg
31140

aaagggtgga ggcaggaagg agaagagaaa gaagcagcag agaaaggagg caggggtggg
31200

ggcaactgga ttcctttcag ccatccaggg cctggcttcc tgtccaccag cacacggaaa
31260

ttccccagat cttatcatca aagaataaca agtaactcaa ccacagtatg ggggcttaga
31320

gggaacgcat ctcagcttga gcttggcatt ggaggttcca acaggggact tggaccagcc
31380

tcactttcca gccagaggcc aatgcccagg accttctgtc ctccccttcc tctccctgaa
31440

ggctatgtct ccagtggatc cccaacagga gggacctgct gctcaccctc tgtggtgagc
31500

atgttcctgg gaggatgaga aaagtcagca tgcttggctg atgccagtgc ggaacaatgc
31560

tctcagggtc ccgatggtga cttagaggtg agggctgact ccacagaggc ctgggaggaa
31620

gtgattttgg tgtgtctggc ccaggcctgg agttcacctc atgccactca ttctaggaca
31680

gacactttct ccccttggag cagctctgag cacgagaagg agctggtggg tgtgcactga
31740

aacgatggga ctgggtctcc aatgtcaagc ccaagacttc accactgctc aatatatccg
31800

tgtaacaaaa ccgcacttgg acctcctaaa tgtatacaca cacacacaca cacacacaca
31860

cacatacaca cacacaaaac agggctggaa gggctcttaa aagaggaaag caccttcccc
31920

tttgtgtgaa tggggaaccc atagccttgc tggaggagag gaatacccag gagcccacag
31980

ccaggcagca ggcagcaggc agcaggaact agaacttggg tgtccttgtg tcctgaaccc
32040

ccacacgcct gtagagatgg atcctgtctc ggactctcgg ggcctggatg agcagcgagg
32100

gccgggtcct ttctacacca ccgaggtgtc agggacacag gaacccccga gcccagggct
32160

gacaccactg cctgggacag aaggtgttca ggaaaggtcc cgggcttctc gcctccccac
32220

gcctcttagc actcgcactc agggaggagt gagggcccca ccgtgtgctg gtgctgctgt
32280

gccctttcca gatacctcct gagcacgcat cctctccctc agtcctcacg gctgcgggcg
32340

catcaggcag gcatgtcatc accccatgta acaggcgagc atgacctaag actcagagag
32400

catgttcacc gcgcccaagg tggtgcattc agagggcagc agagctgaga ttcaaagcca
32460

ggtcttcctg gcctcgggaa atgtctttca acagcttcag tgttgggccc tgtcctggta
32520

ccaagaggat ctgccctctg agggcctggc ccctgctgag gagtctgccc cgggccggac
32580

agtgggtgtg cccagggcgt gcagctccag gagtgcccac ggtgggccca agatagaaaa
32640

accaggaggc agcaactgtc aacactgcag acgccccgcc cgcaacacag acctgctttc
32700

ttaggagggt gaaagctgtg ccccagacgc cagcccatgg gcaccaaggg tccaggcact
32760

gtcatagagg agagggacga ccactggttt gacaggcagg agtccgggga aaacagacgg
32820

ggaaacaaac tctgtaaaca caaaccatgc cccacccgag ggctctccag catctccagg
32880

tgtggccaca ctgaatccca ggcatgagtg cctactgtgt gcacactctc ttaccccga
32940

tggtcctgca ctgttggatc atgaccccca ggagcctgat ggggacgcta aggtgcaggg
33000

gagtggccat ttgctctggt gacacagcga gatgctgccc aaagacgatg tcctacttga
33060

ttccactgcc cctcagagga tggccaatgt ctcattctgc agctcccagg tcctctccta
33120

gcctggaaac ccctctgccc tcctcacacc tgcagggctc caaatggcag aaagtgggat
33180

ggggctgctg ggtggtggtg gggctgctgg gcggagccct gggcttgatt cctggtccct
33240

cactgcctcg ctatgtgacc tcagtggcca cggctcctct gagggcctca agcttatatt
33300

taggaagata accaggcaca gtgacaaagc cagacagtgg aagggaccca gaggacaacc
33360

actgtgcagg aagcctggtg acaaagtctt ggctcggggc tgggttgggt ggtctaatga
33420

caccatgtca ctgcgaaggg gatctgccta aaaaggcaac taggctggtg ctggggacac
33480

tggagaggga caatcaagtc caggaagaaa gcacttctcc ctgcagaggc ctggacgctc
33540

ccacctttgg ctcgccgatc tgctctgccc aacatagcag cttctggcca catgtggctg
33600

tctaactttc aacacaatta aaactttagt tttggccagg tgcggtggct cacacctata
33660

atcccagcac tttgggaggc cgaggcaggt agatcacttg aggccaggag tccgagacca
33720

gcctggccaa catggtgaaa tcccatctct actaaaaaca caaaaattag ctgggcatgg
33780

tggtgcacgc ctgtaatccc agctacttgg gaggctgaga ttgcagtgag ctgagatcgt
33840

gccactgcac tgcagactgg gcaacagagt aagactccat ctcaaaaaaa ataaaaaaac
33900

aagcaaacaa acaaacaaac tttagttcta cggttgcacc agccacattt caagcgctta
33960

gcagccacgt gcagctggtg gcagccatgc tggataggcg gtgcagactc agcacgtctc
34020

catcactgtg gaaagtgcaa caggcacttt ccaaggcgct gggttagaga cttttctttg
34080

aggccgagag caaccagagg cacaggagag gttctcgaag ggcccatgga gagaggctgc
34140

tgaggcatga ggggctggag acagtggaat tcactgggag agggcagcag agggtctgtg
34200

aaagcagtca ggggtcacac gaagatgagg tgaaaccaaa cagtatagct gctgtgagga
34260

caggttaact ccccaactgt gcaaccttaa gaaaattact taacctctct gtgcctcagt
34320

ctactcatct ggaaaacgga gccatgttag aatccacccc ctcactcttg caataagaat
34380

taagtgggtt gatatttata aagtgcttag gacagggcct gccccagagt cagcgccatg
34440

taagtgtctg tggggtgaaa tcacacatgc taagtggtta ggtcactacc tagcatgttg
34500

tcagttggcg gtcaacaaat gctccaggaa cacagggctt taccagagaa aggctccagg
34560

ttgaggttga gcaggtactt ggtgccacac tcgggcccca agatcccatt gtagctgacg
34620

gtgcgggcac agagcaggag gcggcagacg tactcctcag cggtgttgtt ggtgatgtgg
34680

gcaaagacgt caaagtcact gcccatgttc atgctctggc ccacacggat ccgcatggcc
34740

atccctgtct cctccttctc ggccagtttg ttcaggtggt tcgcccttgt gaaggcctcc
34800

ctctcctctg aggaccctgt aggggttgag aagagagcct caatcacagc tggaatagat
34860

cacaggaagg gggctgcaac agggcgagct gtcttcgcag aggcctgatg actcagggca
34920

gccatgaagg agccccttct ctacatttct gggccagaag gacacagcat ttcattaatt
34980

ttccactagt gaactcctat acatccctca agactcaacc ccaaatgacc cctcctccag
35040

gaagccctct ctgaccactc cttcccctgt gttgttttct gtttcaaact taccttcttg
35100

agctgctcga gggctaaggc tgtgttttac ttatccagtg cctacaacag tgcttggtgc
35160

atactaggca cgaatacagt gcaatctttc ttaccttcaa gcaattacac agggattact
35220

tctacctaaa atcctttccc tccctgtctc tgattgctcc cttccttata ttccatttgc
35280

ctctctcctg tgctcctttg atttttttct cccagtgttg ttgggtgcat atctatttat
35340

tctatgaaac tatcagctct agacctttca ggggatgggg agagcatcat ttcattctgt
35400

gttatccctt tcagctagag tatggccagg caaccctgga aagtcagtga gggtcaggcc
35460

aaggcaggaa ctatggtttc ttagtcccct ctgtatgctt tgctatgctt agtatttgct
35520

tagaaattag ataccatatt gaattggctg ttcgatgtgc tcaaatccaa tttttaaaaa
35580

taaatcaagt cctttcccct gcaactgatt taacatcagt agcagagaac tctttcccta
35640

aatgaataat cctctaccac tgtccctcaa catgtttgct cctgtttcta aggcattgct
35700

gaagatggag tatagcctac ggggccattg ctgtacgtag gctaccaatt aaaacacacg
35760

cacacacata cacgagcctg ccgggctggg aaaactggat gcttatcttc aaggctgcat
35820

taaagactct gagggcacat accctctggg tatttgtagg tgtgggtgat atcctcccgc
35880

tcgtctcggc ccacgctctt agtgctgatc ttcagcccaa cgatcaggga acggttgatg
35940

gatttgtgca cagacccatc gtcctgctgg atccagtcta ccacgtcggc attgacctcc
36000

gcaaagacaa agggcgcatc gtacttggtg ctcaggtcgc cctccttgat ggcacgaact
36060

ggaactgggc cacagcagta cgtccctggc agaggtagaa aggggaaggg atgggcctgg
36120

gtgaaggttg ggtggtgtcc tctaaaacgc tccacaattc aatcacatgt agccaaaaac
36180

ctcaagacca cgcggccctc tgacggaagg actccacttc caggagggca ccacagggca
36240

agggttcaga ataggggctc tggagcacaa cttttaaggt tcaaatccca gctcagcctg
36300

ttattagctg gtgactttgg gccagtcact aacttctcga agcggcagtt tcatgtggag
36360

ataagatctg gaccacctaa tgagcattcc tgaattatag atgtaaagct catagcctat
36420

actaaatgct atgcaagtat ttgataaata aaatggaaat gtatcccaga catcctgagg
36480

aaattatgga gccaaggaca ggcaacatgt tagcaaggag gacatctgtc tcgtttcctg
36540

ctcatactga gagtggagct cagtgccccg catggggcca gccccagggc gtgtgtgttc
36600

tccaagtgaa tacgggtgcc tgatgtttac gtaagtgtgg ttgataaccg ccccacactg
36660

tccgcagact tccaatgctg aggactagag gctggataga tacagtctag cacatccatg
36720

tgatggacca cggtcacatt aaataaaaat tcaaatcaca gaaaactatc tgctggagaa
36780

atgccaatgc tttatccaga agtggacaga gctgggaaaa aatgttccat gccgctggag
36840

gtcttaaacc agggcccacg gatcccccca aggcactacc aacagaattc aggggtctat
36900

gaaccctatg atggggaaaa aagggtatct ttgttttcac catagcactt tcttcaattg
36960

caaaagcagg aaatgaccat agcagcctga ggagcactta ggacttgtca ctcataagaa
37020

ccacaggcgt ggtcattaca accttacagc tgtcccagat ggcaggaaat taatttatgc
37080

tcataaatta tctctacaac tatattatgg aacttactga ctctaccgct agatcttgtt
37140

ctataaagaa gcatatgtta ttatatcata aattcatttt ttaaaacttg tgacaactgt
37200

attttaatgt aattggtttt ctttgtaacg ctatgtatta tattttatac atttaaaaat
37260

attcctagaa agtgtccata tgcatcacaa aattgtgcct tagtgtactc tctctttcaa
37320

acacacaggt actacatata tatatgtgtg tttatataca tacatatata aacacacaca
37380

tatacacccg catacaaata cacgatttga aatcagtgga agctcgtgtg aatgttaatg
37440

ctgcttattt tagagggagg agacaaagat gctatttgat ttttttcttt gagcttctct
37500

gtatttcctg ctttccgcaa tgcacgtgaa ttatttttga gatttatcaa gaagtgtgtt
37560

attaagatgt aagtgaggtc ccccaccctt ctgtggaccc atcagtggtc tccgggtgag
37620

ctcctagttc tgccgccctc cagctgagtc actttaactc tccaagcctc gtcttcccca
37680

tctgtttgac gcgacagtgc ccgcccacgc accttcgctc ttctcctggg gcgttgggtc
37740

cagggcctgc cagccctcgt accccggctg caggtccggc ctggtcatcc acgactccac
37800

ccagcagtgg aagttcctga gggggatagg ggggcgggaa tgaagcagaa catgagcaac
37860

attcatcgcc acctcccacg ggcagaccat gcattcatgt ccccagatgc aagctcgcct
37920

cgtcccaaac tgagcccagg gtgtctcccc actgccttgt tcttcccccc acgggggccc
37980

atctcacagc accctctcta gccaaggcgc ccactcctac acctcagagc ccttctcaac
38040

tcctctcccc tccttgcccc tctcagtggc ccctttgacc tcctaatttc ttggctccat
38100

cccttctttt ctgtccgttc agggcttatc actccccacc tggaccacgc cccagccccc
38160

cgcccatctc ttcccctcta gccctcctgg cagttgtcag ccccgccctc tcccactctg
38220

aatccttgag tgacaaccta ttgcttttgg aatcaagcct gggcccccta atgtggcatt
38280

ccagactccc gacaacattt gcagcatgat tcttcctgag gccccatcat gtatgtggtt
38340

gactttccct ctaggccttc cccaagcctg ctcaaatact gctgaacttt taaggcccaa
38400

ttcaaatgtg acctcctcca agaagccctc caaggtttcc tctccatggg gccctcccta
38460

ctgggctccg atcccaccct ggcccccacc tcaccagatc atctcgctct tgtcaccctg
38520

gatctcccca aactcattgc ggaagtactc gatgagaagg ttgctgttct ggtcatgggc
38580

cgagttgtag ttggtcacga cgcgggtagg gatgcccagg cacctcagca ctgttggaga
38640

ggagtggaaa gcggggtgag gtcctggaga catccgccct gctctgggcc tccacctgcc
38700

catctgcatt ccagggaaca ctgtacctgc tgtccaagtg ctgagaacat gagccagccc
38760

ttcctgccgg gacaatggcg cccacctctc tggtcacccc atgtccattc tatgcccctc
38820

acatcacagt tgccagaagg agactctaaa atacaaaccc cggccaggcg tggtggctca
38880

cgcctataat cccagcactt tgagaggccg aggggggcct gaggtcaaga gtttgagacc
38940

agcctggcca atatggtgaa accccgtctc tactaaaaat acaaaaatta gccaggcatg
39000

gtggtgcacg cctgtaatcc cagctactca ggaggaggct gaggcaggag aattgcttga
39060

acctgggagg cggaggttgt ggtgagccaa gattgcacca ctgcactcca gcctgggcga
39120

cagagtgaga ctccatctca aaatgaaata aagtacaaat ctgatgtcac cactcctgtg
39180

atcaattgtc cttggttccc catggccctt gaggtcaagg ccagtgccct ggccatggcc
39240

cttggccctg cagggtctgg cccctgaacc cctcctcgca cgctgtgctc ctccacactg
39300

gcctccagca tgctctgcct ctgccagctt caaggcatcg gtgtctgcaa ttccctctgc
39360

ctggaaggct gcctgccagg ttcaggttgc ccatcatttt gcattcatag cacggctgga
39420

gatccttcat ccttccagtg tacaggcatg tgagggattc tttaattagt cctccaaggc
39480

cagtgaggga tctgtcttat gttagatcct ggagcccagc acaggcctgt cattgactat
39540

tttgtcaaat gggatgaagg agtggaggaa ggagagagga gaggaaagag ggagggaggg
39600

aaggagtgat tgaatgactg actctcccca agatagggac ttaaagactc aaacagcaga
39660

acaggcagag gagggcacag aggtcgggag acccagctgc tttcagctgt gtgacctggg
39720

accagtccct tcatgtctct gggccttggt tccatctctt tgtgcattag tgatgccact
39780

gaggactcat catcattccc aatgtacaag aacacggatg cacgtggccc tataaatagg
39840

tcgcagcaca gatcaggtcc ctcagacttg tccggaggga gggccctgca tacctgccaa
39900

cgccctgttt gctgggaaag ggctgatgtt tgcaatgaca ggttaaacag caacaggaca
39960

ttgactggca tctgcaagct gagctcagct cagagaggcg aggagcacag gggagggagg
40020

aaaatcccgg tgtggatctc acccacacgg cccactgggt gctcctggca gacggaatta
40080

tgggtgcttt tactctctca ttttaaaaat gtgcttttta tgtgtattgt aaaagtaaca
40140

gattcccaga aaaaaatatc taaaaaatga agagaaacaa gattaggcgt aataaggtaa
40200

tctgtgggac ttttgtttta cttttagttt tgattgaagt tgagtttggg agtgggggag
40260

aagggatgcg acctttgccc tggggccacc atcgataggc cagggtaaga gcttactggt
40320

tggccctgga ttcctgccag gagatttgta ggagcttcga ggaaggagga aggaacagct
40380

atgacctccc cctcttgcct ggtttctgtc cccctgaggc ctgggcaggg ctcagcccta
40440

aggctgccct cagtgaaggc tggtgtccta accacaggtc ttatcccacc atgcagtggg
40500

cctgaggaca gaccggagga cgggggaggt ctgtctctgc tttccaggga gccaagtgcc
40560

actcttgccc caggggtggt gtcccgggag gcctcctcat cagtctaaca ggaggctgcc
40620

ccagcaccgt gcacacctgg atcagagact gaccctgctg ctcaccagct gtatgacctt
40680

gggcaagcca ctcccctctc tgagcctcca tttcctcctc agtaagacaa gctaataata
40740

gcagcttgtg gggatttgtg gtgattcaga gagaggccgc gggtgtgggc acagccttgt
40800

ctgcccattc ccagagcaag cattgagggg gtgctcggga acagcacagt gtgtcatggt
40860

gcccgttacg gcaaacgtcg tccatcagtg ctggctgatt catggacatt gaggaagtac
40920

agtgagggat ccggctaaga aagctggaat gccaggctcc cctggaaatc cccaaaccca
40980

agcctaaccg gagccagaga ctgtgctaag tgatctctcc ctggtattaa gagcttccta
41040

tgaatgagat cagtcaccat tgtctccatt ttatagatga ggaaattgag gcttggaggc
41100

atcaattctc ttgcccaagt ccacccaggg gttagtggca gagctggatt caaacccagg
41160

cagtccagct ccagagttca tgggccaatt caccattggc tccccacgtg aactccacgg
41220

ccacagtggt cagacagccc ttttcttggc caagttttcc ctggctccat cctacacagc
41280

ctgggaagag gctgcgggga ccagaacaca gccctgcagt gggcagctga ggggcaggat
41340

ctgctgctca atcttcaggc ggccacagcc cagggtgacc aggatgacca gggtttctgc
41400

cggtgagcct ggggatccag gtcttggatt tcggggtttt tccaggaaaa cccatgggct
41460

tctttgaaaa gaggtgtttt agggctctca gagggaagtg tctggcatga aggagtttcc
41520

acaagaaaaa ggaattcaga ggtcacgggg aacaggcagt cccaggcaga gaggcaggag
41580

tcagaaggca aggacagccg agctgagaac ctaggttagg ctgccctgac ccagcctgct
41640

ggtgtggagg atgtgctggg ggccctgcca ggcacccttt cctgggcctt agcactggtt
41700

ttggaatcag aaaagctgga agccccagac cagcctttta gattgagcct ggtaataata
41760

atagtcgtga caataaatag tatcatcaca taagtatttt ctattatgca cccaaaatca
41820

ttttaagcac ttcccacatt catttattta tttagagatg aggtctcgct ctgtcaccca
41880

ggctggagta cagtggtgca atcatgactc accgcagcct cgacctcctg ggctcaagtg
41940

atccccctgc ctcagcctcc caaacagcta agactacagg tgcaccacca ccacgctcag
42000

ctacctcaca catattaact catttaatcc tcacaaccat cccagtataa aggtactacc
42060

gttgtgccca ttttacatac aatggaactg tggttcagag gagttaagtg atttacctaa
42120

ggacacacag ctggtaagaa gcagagctag gatttgaacc caggccttga cttcagaatt
42180

tgtgctctct ctcttttttt tttttttttt ttttttttga gagaggatct tgctccgttg
42240

cccaggctgg agtgcaatgg tgcaatcata gctcattgca gccaacctcc aggggtcaag
42300

cgatcttcct gcctcagcct tctgagtagc caagactcca gacacacaac catgcctgct
42360

aatttttatt ttattttatt tttttgtaga gacaaggtct cactatattg cccaggctgg
42420

tctcaaactc ccggcctcaa gcaatcctca tgtcttgagt ctacgttctt agccagcatg
42480

tgatgctaac ccattctcat aagcaccatc atcagcctgg caacaatcat cgacattttc
42540

tggccttaaa ttttgaagat ttttgtttta gatttatttt acttttttgg ttttaaattg
42600

ctcgatattc cccctctaca ttttagaaca tgctttcttt cttgacactg atattactgt
42660

taggatccag ttattactgg ctaatatttg ccgagagtga cactgggcta ggttctgtgc
42720

tgagtagctt catgtcacac ccactctagg aggaaggtct tgatgttgtc cccattttcc
42780

agacgaggaa actgaggttc agaaagaagt catttgctca aggtcacaga gcaagtggtg
42840

ggagggaggg ggtttttaac tcaagttaaa aatcctgagt ttttgagaac gtgggctcca
42900

gtgcccaagt tctcagccct gagcccatgc agtctctcca atgatagccc ttgatcctaa
42960

caaccactgg agggatggct attggataaa ggaccggaga gggtggtcac aggctctagg
43020

ccacatagcg cattgagagt gttggtggca gggggcagga ccagggcagg gatgtcctga
43080

ttcctactcc agtgctcttc gtgccccctc ccagggctca tgacccacat cccagcgtgc
43140

agctcacctg tgcaggccac ggcggcgaag acccagcact ggccatactt gacgcgctgg
43200

cagccgtggt tcttccagcg ccgcaggatg tccacgctgc cgatccagga catggggctg
43260

acgccgtccc cgtagttgtt gtcccagcgt cccagcagca caccctggtc atcgttgcag
43320

ttgacctgca accagtgggg cagcacgggg actgagcctg ggatggggtg tcggctgtgc
43380

cgcctgggtg agcctgagta cagcaggggg tgaaagctgg ggaggggagc tccaggtga
43440

ggaaagagca ggtgcaaagg ggccatcgtg tgacagactg gtgtgcggca gatggtgggg
43500

gctggagccg ggggacagga gggagatggg gaagaaggag gtggggcagg gtcaaggact
43560

tgaacgcgat actggggatg tgactctgtc ctatgaacac aggattgctc gggagtgaca
43620

tggtgtgatc taagttttac aaagacccgc ttgggccccc caccccagat tgtgtcgttc
43680

cccctaaact cccattacct cgagaataaa atcagcctcc tgaccatgat ccaagaggcc
43740

cttctggtct cacccctgcc cacctctgca gcttcctctg cacccacggg ccctgctcac
43800

tccactccag gcacactggc ctctcttcct tcctcaaacc tgccaaagtc gctcccttct
43860

cagggccttt gcacagctgt gccctctgcc tggaatgccc ttctcctggg taccacctta
43920

gggagcccct cagcccctcc ctgagcctgg ccctgcagtc caacgctctc acacctcggg
43980

ttccttcaga gcgtgcacca tcatttatga accatgtgaa ctgcctgctc gttcatatgg
44040

tagtttggtt agtgtctgtc tcccctcccc tcccctcccc tctataacac agtctccata
44100

aagctagaga tcacacagga cttgcacaca gctgtctccc cagtgcttag gacataagtg
44160

gtacttgata aatatttact gaagaaatga ataaattaat tgctctgggg gaccccatag
44220

catcaccctc atctgtgcaa atcttatctt tccaatataa ggtcttttcc ccaagactta
44280

cccatctccc gggtccccca ccgcgcctag cctgtgtctc cactgcgagg gagaggcctg
44340

cgggagcccc ctgtaggccc cgcccctgga tgggccatgc cactcactca ccatgccact
44400

caccacccgg cccacgtaga cggggctgct gcggcgggag cagtcacggc cggcgttctt
44460

caggaacttg gggttgacat ctagaaggat caggcagatg tctaggatcc catcttcaaa
44520

ctgtgtcaga ggaaacaaga ggagaaagag ggagctggga aggcacctcc tccaggaagc
44580

ctgcgttgaa gcctccagca gctgtttccc actctgtccc acacagcaga ctgggacact
44640

ccggccgagt ctaccaaatc tctctggtgg cagcttctct tggcacagga gggaatggag
44700

tgggacaatg actgggtgag ccatgagtgt gtgacgaaga gggtaacaat tttcatccag
44760

ggcctggttg ggggaacctg gaaatctgga gcaggagccc atggagtggg cagcccggtg
44820

tccctggggc tccccctcag agtcaccttg ggccctgtca ctgtcccttt ctcctcttgc
44880

tcagccaaat ctcccacttc cccaaggcgt gacccaccct gcagagctga tgtcagcccc
44940

ctcgtccccc tccaacccag cccacacacg ctgctctgat gggaactgag acagattcta
45000

aatacagaaa tccagtccca tcactcttct gtttcaaccg tatccacagt aaaacctaga
45060

cttcaccgtg caaacttcaa ggcccttcat gatctgctcc catccccttt ttgacctcat
45120

ctctcaccac tcacacccac actcactctg cccaggctca ctgccccagg gcccttgcat
45180

gtgctatttc tctgctcaga atgctcttcc cgctgctctc tgcagggctc acacctcctc
45240

atccttgcca ttcaaatgtc atctttctca aaagcaggac cccttcttgc caccttccac
45300

tggaccaccc acctccaggg gcccttctgt ctgctctttt ccccccactt ctgtggtcct
45360

ttcctgggcc agatctccct ccaagctgca caacagcctt ccacggtgtg aaccctctgt
45420

ggctccctat tgccatcggc ctaatgttca gggctgttcc acaaactgga cactgctggc
45480

ttctcccacc tcatctcttg gggctccttc acacacaagg tatgccccaa gctacaatat
45540

acctctcagg cacgacataa actttccaac ctctgagctt tcacttgcat agttcccttg
45600

gcctagtatg tcgtgcacac acctccttgc atggcaaact cctatttgtt cttcaaagcc
45660

cagctcaaat attccctact tcaagaagta ccccctgatc cccataggct gttattcctt
45720

ccttcctcta ggtctcatct tcctggactg tgatcacctc cagaagcttt gcaaaggcca
45780

tgtcccacag cccagcacaa gaccggatac aaaacaagac cctgtgggcc gggcgcggtg
45840

gctcacgcct gtaatcccag cactttggga ggctgaggca ggcggatcac gaggtcagga
45900

gatcgagacc atcctggtta acacggtgaa accccgtctc tactaaaaaa atacaaaaaa
45960

ttagccgggt gtggtggtgg gcgtctgtag tcccagctac tcgggaggct gaagcaggag
46020

aatggcgtga acccaggagg cagatcttgt agtgagccga gattgcacca ctgcactcca
46080

gcctgggcaa cagagcgaga ctccgcctca aaaaaaaaaa aaaaaaaaaa aacaggaccc
46140

tgggaaataa aggaagaact tgactttgtt ctccttctca ggagagaata aaaagccatg
46200

aaaaatcaga gccaattcat tttctgatgt attttatgca tcacggtcat accaccattt
46260

catttatatc ccacagcagg ctacggggtt gctgagcagt tagttttgca attatcatca
46320

tgatattcag atgagaaaac tttggtcaaa gcctgagatc atacagggaa gatttcccta
46380

tggtttcagg gtaggaccct ccctagaggg tcattgaaca ttgaaacctg acccagaggc
46440

tgctggggga agcctcaggg tgaaccccac cccaggaggc caatacctcc taacacacca
46500

ggaggttgag gggccagggg tgcaatggtt cttgctttgt gagttttccc agcagagtct
46560

caagaatttg tacaggtggc tggatgataa atgaaattta tcatcaccag tttagagaac
46620

cagcccaggc tggcccactg gggagtgaga aaccacgtga aacagatgag ccattggctg
46680

agccagatgg taggagccta aaggtcaggc tggctttact gactccagcc aagcccagcc
46740

tagacagcca tgcatggctg acccacagac ttgtgagaaa taataagtga ttgttgtttt
46800

tagcccctaa atttcaggct tgttatgcag cagcagctag cagatacaac cagatcagca
46860

gttcccaaag tatggttatt agacaagcag catcagcatc acctgggaac ttgtttgaag
46920

agcaaattct caacccctag tgttttaaga ctctcagtga tcctgagaac cactgatctg
46980

ggtgagggtt taaagcctct tcttagcaca tgcctagcac acagggctca ccccagcaca
47040

actccaagta ctgcctattg tttcaataca tgagctttac tgttccctct tccccctccc
47100

aggataccaa ccagcttcac agttcccaaa ggctctccta gttctttctc caccttgaat
47160

ccaaggctgc cgcctttgct gtgtgacctc agggcagcct tggccctctc tgaaccttca
47220

gctgtttcag ggcctttgca tccctgacag catgaagtgg gtgactctgt gactcactcc
47280

cacctgtgca gctttggctg cccccagaca cagggccggg cacacagaag ggcctgagat
47340

ggttgggaga gacagggtgt ggcccttacc tgcccaaaat tccaaggtat gttcttgatg
47400

aacttggccg agccctggta gataaagccc tgctgggtga ggacatactc ctgccgctcc
47460

tcttccgagt ccaggtacac agcatccgct gcaggcagga agaaagggac ctcagctctg
47520

ggtctgcgga ggcccagggt ccctgcaaat ctggagtcaa gggtgccaat tccctaggcc
47580

aagccagcgt ctcagggatc catccttctt ttaattagaa aaagtaacca ttaatgaata
47640

atcccacgtc aatgagataa ggtgttatcg gttagtgaca acaaaggctg ccatttcttc
47700

aacactcact gtgtgccagg acctgtgata aatgtatgac ctgtattaat aaatttaatc
47760

tccagaagag ccttatgagg aagggatcct tgaaagcccc attttaaaga ccagaaaact
47820

aaggctcagg aatattttat agctaggaaa actgagacta aggacacagg agcactggac
47880

cgggagtcag gaaactcctg attctcactt gctgtgtgac cctgggtagg ctactttgcc
47940

ttctctgccc accttaacac ctcactgtgt cagcccaaaa tgcagatctg aaaggtgctt
48000

tggaaacgaa agccctgagg tagcgtgagg gaggcgcatg attcaaggca gagagaactc
48060

agtagcagag gggaaggcca agaaagggtt cctgcagctc aggagaggtg gggtcctgtc
48120

cagcttgaga gagttacaga gggctccctg gaggaggtgg catctgaatg ttgtcttaaa
48180

aaatgattaa gttttagaga gttggaacct ggagggtagc attctaagca gaggaccaga
48240

aagcacaaag ctttggctgc tggaaaatgc agggcattta gttgtctgtg gccagtgagg
48300

ggagaagaag gagctgagga agttggggca agatggtagg agcccaaagg tcaggctggc
48360

tttactgact ccacttaacc ggtgaagaaa ctgaggccaa gaaggggcaa tctggagccc
48420

cattatgact ttggtgagcc ccaggcactc ttgacttcat gcattccttc ctccattaga
48480

aaaaaggtaa ttgaatgtta tgactgcctt ggttaattac agctatctca atattacata
48540

ttaaaaccta ttgttcaact taaatgttca tttttttctc ctgattttaa attacattaa
48600

aacatgttca cgggccccta agagtttctc aggccccagg cacagtgcct gccgggtcta
48660

atggagaagc tggcctggga aggaagcagc tgccggcccc tcagacccct gggctttgtc
48720

atgcccgtct gcagcaacca gcagccctga ctttcatcgt agtgaggaaa aatgggaaag
48780

aggccaagtc tcttgtgtat tctgatcttt ggctctggaa acttctataa aaattaacct
48840

tgaagtcaat gaactataat ggctctgcag cagccggggg gtgtgggata agaatgaaag
48900

agaaagaaga acccacagtg tttcaacatt agaatgagtg tcagcacttg ttgaattgtc
48960

cccaccccag ctcaggatgc caagactgtg acatccccag ctcaggtccc catctggtaa
49020

aggtgggggc actggggacg tgaagccttg catataactg gcactcgatc atattggtct
49080

tggaactgac atacaggagg gtcccatttt ccaggcagac ccggcccttt cccacccatc
49140

agactgattt cctcccgcag cgcccctgcg ctcaccccag cccaacttcc tgcctctgca
49200

tcaccagctc cacagttccc gccccgggat gtgggtcact ttagtcactg tgccgcttgg
49260

ctgaccgcca ctccaacccg ccgccgggtg aggccaagca ggaggaaggg ggccatcccc
49320

cagaaccacg cttcccattc cctgaactct gaaaccttgt ggtgtggacg agggtgggga
49380

caccggcgtg ttcctgtcct gttggcagag ctgccaacta gttgctaccc tctgcagatt
49440

gactaaaatt caaaacaaac ctatggggcg ggggggggga tccagtaagg ccacttctgt
49500

cccgagaaat cattccattg gccacagggt atgaagcaag ggtgctcgtt gacgcatggt
49560

ccctgcgtgc catgagcctc caggcacaac ctcatgtcca tcagtggggg ctggaccatc
49620

cacatgggca agaaacagga cactcaacgc ccttagaaag aatgagcaga tgtggccggg
49680

cgtggtggct cacgcctgta atcctagcac tttgggaggc cgaggtgggc ggatcacctg
49740

agatcaggag tttgagacca gcctggccaa catggtgaaa ctctgtctct actaaaaata
49800

caaaaattag cccggcgtgg tggtgcatgc ctgtaatccc aggtactcgg gaggctgagg
49860

catgagaatc atttcaaccc gggaggcaga ggttggagtg aggcgagatt gcaccactgc
49920

actccagcct gggtgacaga gagagccttg gtctcaaaaa aaagaaaaaa aaaaaaagaa
49980

tgaatgagca gatgtatgca gaccaatgtg gacagagcct cacggtgtat tgtgagggga
50040

aaaaaccaag ttgtaggtta atgtatacag tgggatcctt ttatgtttgt gatagagaga
50100

cagagaccta taaaatacag caatttctcc aggcacatac agactgacct aaggaggtga
50160

gcccccgcaa aatatctaac aatggtgtcc cccaaggagg aggggtcaaa gggggaggcc
50220

atttctctgt agtcttgaag ccctcccctt ctctcccctt ttttggagac agagtcttgc
50280

tctgtcaccc aggctggagt gcagtggcac aatcccagct aactgcagcc tcaacctcct
50340

ggactcaagc gatcctccca ccttaacctc ctgagtagtt gggactacag atgtgtgtca
50400

tcacatcggt ctaatttttg tattttttat agagacggtg ggtttcgcca tgttggccag
50460

gctggtcttg aactcctgcc tcaagtgatc cccctgcctc agcctctcaa agtgccggga
50520

ttacaggtgt gggattacag gtgtgggatt acactgcacc tgggcttgaa gcttttctaa
50580

agagaatgct gtcatggttt actgttatac tgggaaataa aaatcaaaat atggaaaggt
50640

gaaaaatttt aaaagctttc tgctcaaata taaaatgcct caaaatagag aagaaaaaag
50700

ataaagcagt gggccagcaa gaaggtggtt gtctaacagc caggaggcca gaatttctgc
50760

ccttttgaag agggacaccc gattcccagg aggggcctgg cccatgatgc ttctttccct
50820

gctttgtttc attagaatag gaaggaaaca gatcattaga tctgactgcc ttgctagtta
50880

gatgggaaaa tcgaggcctg gggaggtgac tggcaacccc tgcctcctcc cccttgctca
50940

agtcagtttc ctttgttcca agctctcccc tctgactttc tagttttgtc caaggttgtc
51000

cacttattta ttcaaagaga ttccaaaggc cacttcacca ctctgagcct cagtttcctg
51060

tgtgtgaagt ggggaagtgt catctccccc ttgggattgt tgagaggccc gactgagata
51120

gtgtataagg aggcagctgg acggtctaac tgctcctact cacccccctc ccccagccct
51180

ccccccaacc tcacctccgg gtttcccttc cccaaaccag tctgggtgag atttaaaaca
51240

gggcctccca cctcagccgg gcacggtggc tcacgcctgt aatcccagca ctttggcagg
51300

ccgaggcggg tggatcacct gaggacggta gctcgggacc agcctggcca atgtggtgaa
51360

actccgtttc tactaaaaat acaaaaatta gccaggcgtg gtggcacatg cctgtaatcc
51420

cagctactgg ggaggctgag gcaggaggat tgcttgaacc tggtgggcag aggttgcagt
51480

gagctgagat tgtaccattg cactccagcc caggcaacaa gagcaaaact ccgtctcaaa
51540

aacaaaacaa aacaaaacaa aaaacacaca aaaaacaggg ccccatgaaa cagaaccagg
51600

acacagtgcc tgggcctagg catcagctgg gcaccctatt cctgatgtgg ggcttttgaa
51660

accaagtcac cgctaagccc cagttcatag tggtctggga atatacaggc ctcaaacagc
51720

gagtgaagtc ccttggtaaa tatgtggaat tgggcatatc attctgcttc tttttatttt
51780

tattttttta gagacagggt ctcaccctga taaccagcct ggagtgcagt ggtataatca
51840

tagctcactg cagcctcgac ttcctggaca caagtgatcc tcccacctca acctcccaat
51900

tagctgggac cacaggcgtg cactaccaca cccagctact gttaaaattt ttttttgtag
51960

agacagggtc ttgctctgtt gcccaggctg gtctttaact cctggctcgc tcaagtgatt
52020

ctccctcctc agcctcccaa agaaccgaga ttacaggcat ggatcactga gcccggcctc
52080

attttgtttc tataaacagg gataacagga gtactcatgt cttgagggtt ttgtgaggct
52140

ggagcacagt gggcctcact cagtccactt tgatgtgtgt ctcttatccc ctgttcttct
52200

cacctccagt agcctcctcc atgggcggat ccagccctgc ccaccccaac gctgtgagtg
52260

gatggcgtgt ggctcacctg ggcaccaggc gttgaagagc aaaatgaagt ggcccagcac
52320

aaagctggat ccctggtagc cagtggaggc ctccaggctg aggcgataca ggccgatggg
52380

ggcgttggcc ggggtggtga gctgcagcga gagggtgcag tcttgctggt ccaccacggt
52440

ggctgtccag tcaccctcct ccacagcatc tcttagtgga aaacgggcct tggtcccggc
52500

ctcctggcta ggggctgggc ctgtggaggg agaagcagta gccgtgagct aggggtagca
52560

gggctggcag ggcagggcac ctacgtgggg tcccccagct tgggctccag gcctagttct
52620

gccactaacc actgaaataa gtttggcaag aagtttgtct ccctgagcct cagtttcctc
52680

atctgtaaag gggggctcag aaccccagcc ttttagggtg gttttgtggg aatgcataaa
52740

aagccacagg catacagcat gtcctcaact aaagagtcct cctgagagag gggccatgtg
52800

gcccagagcc aaggctcagt tccactgctc tgcaatgatg tgactcggga caagcccatc
52860

cctctctctg ggatcacctg gccccatttg tgtagcatag acaaggatta ggcaacatgc
52920

cctagggccc tcctttcctg cctacatcct ctacctgcta gcgtcccgct ttttctctca
52980

gagaaggaag cccagacgct gagcaggaag ggacctcggt cctctgagag ccccttgctg
53040

taggccagcg gaggatgctg gctggcccac acagcctcaa ggtgtctgtc tccccaggcc
53100

ccccatctgc tgtcccccac atctccatag gcagttccga gatgctggtc ataacgaccc
53160

ctcaccttcc tacagccttt tacacttcat gaagcacctg cttcctttga gaccccttgc
53220

ccaggtgaga aaactgaggc tcagagcaaa gcggtctccc aaggatatcc taggagtttg
53280

tggcaaaggt cgcatctgaa gtgggtcttt agctctcagt tgtcttctac ggtgcctctt
53340

cttcccaaag aggacactcc ccagccgggt tctcaaaggc acagtgatgg gcacactctc
53400

aaagcagctg cttttccagg tggaggggag ggcccgcccc cacaggcagg tctgagtcat
53460

gggcagggcg aggaggggct ggtcactttc tggacatggg tggtggagag aagacagagg
53520

acgttctctg cctggctggg cgggtgccca ggaaagttta tttttattct tccatctccc
53580

tccttgcagc tctgcccacc caccgtggac attattgggg cctgctcacc caggggctcc
53640

ctcccagccc ccagccccct cctccctgcc agccagcccc acccacaccc agctcaaatg
53700

taaccatgaa ctaacaggca tgacacaggc ctctcaggcc ctgtccaagt gccaggggcc
53760

gactggaagc ctttgaatgc tccctgactt ccagagctgg catttgggga ggagccatgc
53820

aggcctgggg tcccagaccc accctacgtc tggctccctg actctgcccc tgagcactgt
53880

gtgacctggg ccacatcacc aggcctctct gatctgcttt cttatccaag aagaaaacag
53940

gcttaggcca catgcctggg agagtccaat ctcaggttcg aaactggctc tctgaacttc
54000

gacaagttac ttagcttctc tatgccacag ttttctcatc tgtaaaatga ggccagtagc
54060

agtacctccc tataaggttg ttgtgaagct taaagaggac aggcatacaa agcatcccac
54120

aaggcacctg gtgtatagta agtgctcagg aaacaatagt tagtcttgtt cagttttcat
54180

gaacgcctcc tggctctgtt tagagggttc cgtgagagag ttcatagaaa gcattttgaa
54240

cagtgaccag ctcacagtaa tcatgcagga aatgtgagct atcgtcagtg ttattaaaag
54300

gggccattaa aagtctgaat tctcaggcca aatagcagat cgtgaggact ggagtggaat
54360

gtagaaattg tctagttcga ttgaagaact tcacagattt ggaaactgag gttgagaggg
54420

gtggtgctgg ggtgaccaag catcacagtt tgctcaggac agcctcagtt tacgcctgtg
54480

gtcccatcat aattcttaag ggtgctcttt ccaatctcag aaatgtccct gctcggagga
54540

tcaatgatct ggtctctgtg gcaacaggcc tgggccagat cccatcatca ctgtcgctgt
54600

tgacatgtca cccttgggga caagtcagga aacgcatttc cctttctctc tatcttgggg
54660

cagccatgca cacacactca cacacccctt tcttcccaga tcctggggga acaggagaag
54720

gacaggctgt cccacggtcc acccgctgct cacgtaagct gacttcacaa attccttggc
54780

tgcggttcta caccatcctc tttgtgagac cagaggcacc atgagggcca ctctgggccc
54840

aaatgaccac agggagcctg ggggtcctgg ccactatccc agaccatggt ggccgggctg
54900

ggatggggtg ggtgggcagg accttccaga gtaaagcctt ctatcagcct cataagggtt
54960

cccttcactc ctccaggctg acttcactcc cattgtgact gccacacccc ctcaaacccc
55020

gaccccacct tggagggaac tttatccctc tgacaaggat ggcatttgtc tagtgtggcc
55080

tggaccccat aacggagtct gtcgggtctc aaactgtctg ggtgaaaaca cacaacatct
55140

aagaatgtgt ggggtcaggc cacatgctcc attcgctgag tgaccttagc cagctctcct
55200

gcttctctgg gcctcagttt cccatcacaa gctacaatgt tcccacacag gagcagagtc
55260

agctggccag gccttgagcc gggcgctgac ccaggccagg cctggagttg ggggagggcc
55320

tggtttcctg gtattgtccc tcaggcttct ccgggccccg ggaatttctt ggttgcttgg
55380

acagggaagc tgtgtgcact ctgtactcag agatctgggc tcttccgtgg ctgcctacag
55440

gggctgcgga agtgaccggg gcaggttggt tatggtttga gataagttgg tggagactgg
55500

aggacagaga ttagcttgct gaggtgggaa ggtcagcagc agcccttcac ccaaccccaa
55560

actcagggcc ctaattacct aacactacgc tgcccagaca attggagaag gagaaataag
55620

aaaggggaca tctggagtga aagaagagga aagaaggcag ccttatcctc agcgaacgaa
55680

cgcaggagca gaaaagcaaa tcccccatgt tctcacttat aagtgggaga gaaatgacga
55740

gaacatatgg acgcatagag ggaaacaaca cacactgggg cctccttgag ggtggagggt
55800

gagaggaggg agaggagcag aaaaaataac gcttgggtac tagacttagc acctgggtga
55860

caaaataatc ggtaccacaa acccccgtga cacgagttta cctatataac aaacctgcac
55920

gtgtacccat gaacctaaaa taaaagtttt aaaaaagaaa gaaagaaaga aagaaaaata
55980

aataaaaaag aaagtagaaa tcacaggctg cccaagccag cagctcatct accattccat
56040

ttcacagagg aacacactga gccctggcag gcacttgtga taaatgcgcc tgctgcaggg
56100

acatagggat gaaaagcctc actctgcagt cggggtttga agccttctgt gtccctgact
56160

cactgtgtga ctgaccttcc tgagccagtg acttgacctt tctgagcctc agctttccca
56220

tctgcaaaat ggagtccttt ataacttcta agctgcgagg attcaagaca agcatgcgtt
56280

cagtgggtgc cccacagatg ttgtgatttt cactatcaca agtctgactc ttctcaggcc
56340

accagaccca gcccttaact gccatgtacc ctaacttttc acttcgtaaa gggggaaact
56400

gagactggag ggtcagagac ttgctggggg ctccttggct ctgttagcaa gagaactttg
56460

ggggccagtg tgcttgtggg ggagcccctg ggcagtgggt gtgtctccac caggagggca
56520

ggggcaggga ggagaaggat ggaggagcta tccgaggcta ggctgcagtc cccatgataa
56580

gccttatcaa cagccttcta gttcaatgca ggcctcagcc aaggaatgct tgctgccatc
56640

tctgccctca gagacttcag aaagagctgg cctttcccaa cccgagtcct agccaggtgg
56700

cccctcactg acctcaaggg aatggctgcc accagaaacc agacccactg atttggcatt
56760

tgaggccatt ttcccaggaa tggccaacaa agaaaattgc gcatcacgca tctggggatg
56820

agacgcctgg ggacttggtg tccctttacc tatccctggg gccccagtac aaagataagt
56880

aacagctgca aatatctgca ggccctcccc catagcagac acttaacaac cttccaatgt
56940

gagggctttc agatgatgaa acctcagagg ctcagagagg ttaagtaatt tgcctaaggt
57000

cacacagcag gtaggcagcc cagcctgcgc tagaatccta gtctttctgt ccagccccgg
57060

agattgaggc agcctcctaa tgcttggaga atgaactgct gggacccaga actgcccaca
57120

tggaaggctg aagctagctg ggctttttct ctgccaccca ggcctgattt gggcctgatc
57180

ccagtgcctg gctgggttgc gtggatttca ggcagcagag agattagaaa gttgttttga
57240

tatgggaaag ggcagacagg atctttttct tttttctttt aaggtggtgt cttgctatgt
57300

tgtccaggct ggattcaaag tcctaggctc aaatgatcct ctcacctcag cctcccaaat
57360

agctgggatt acagacatgt accaccatgc ctggcttgtg gataggaatt tttttactac
57420

agacagggtt tctccacgtt ggtcaggctg gtcttgaact cccaacctca ggtgatccgc
57480

cctcctcagc ctcccaaagt gctgggatta caggtgtgag ccaccacgcc cagcctgtgg
57540

atgggatcct gatcaacttt cctggttgaa ggctgacctg caaccctggt gataataggc
57600

tttcagaagc tcagggttcc agaggcacag acttgctcac aaagctggtg gtctgccatg
57660

acatctcctt cctttccggg cctcagttcc ctcatctcta aaatggggct gatgacaggt
57720

ggcaatctcc cagggaacaa agaaggtaaa gttatttgtc ctgttctctg tcagggttct
57780

ggggcatgtc ggggtggaga ggaagtggtg gtggtggtgg tggtggtgga gtggggttgc
57840

aggtactcac cggtcacgac actgaaggtg agactgtcta cactggcctc gtagttgcgg
57900

ccctcaaagt gcagggtcag ccagaagggc tggccccgtc gcaccaccag cttctcccgg
57960

cacaggtcgg ccgtgtggtg gtctcggcca ttggtctcca gctccagatc acacctctct
58020

aagaccagct ctatggaaac agaaggagac gcatgagcct cgggggcatc cttcagacct
58080

ccagtgatgc acctgccctc cctagacatg ggggccttgt gccctcttac tccccacaaa
58140

gcacagccat tgtttcgact gacctgtctc cccagccctg gtcacacggg ggctaggtgg
58200

ggcccaatct ccctaactct tttttttctt tttaatttaa ttgagaaaag gtcttgcttt
58260

gttgcccagg ctggagtgca gtggtgtgat catagctcac tgtagccttg aattcctggg
58320

ctcaagggat cgtcccaccc cagcctcctg agtagctgag aggacaggcg tgcaccacta
58380

attttttcat ttatcagtga gctgggacta taggtatgtg cccagctaat ttcttcattt
58440

ttggttttgc catgttgccc aggctgctct cgaactcctg ggccaagtga tggctcctgc
58500

cttgacctac caaagtgcag gtgcgagcca cagtgcccag ctttgcctaa atcttttaat
58560

cacaaggtcg tgaccccctg tggcacttcc tagctgtgtg acctccagca gccatgggac
58620

cagcagtatc tacctcccag ggctggtgtg agcaagaaat gaattaatgc atgtaaaaca
58680

cttagtacaa tgtctgttgt gtaataaata ctcaataaac acgagctatt ctattgctat
58740

cagagggttc ttttggttta agcaggggca gtatttggta caggtcaatt acatggcctt
58800

tgcctttaga tccaatgttc aatttacagg aaatacccag ggctgaggaa caagttaaac
58860

acagaaccag gatgcagttg acaaacttca tatactggga aattattgac aaagggtccg
58920

gtttctttaa taaatagatt gcaagaaaga aaatgaaaaa aacattgaag agagaaccta
58980

taaagagaaa cttaaacccg ttgtgatgta ttgatctatt tggatcttga tttcaaaaaa
59040

gtattttaaa acatttatta catgtatgag acaactggaa atttaaatac tgatggcata
59100

atggtgatat caaagaattt ttgttaattt gtttgtgtta aaaaaaagag agtccttatc
59160

ctttaaaggc atatactgaa acattgacag atgacatgat gtgatgtctg agatgaaggt
59220

gcgcaggggc aggagacacc acggtggtca gtgctgggtg atgaggacat aggaatttat
59280

taaacaattc tgtctatttt tgtgtatgtt caatattctc caaaacaaaa cattttaac
59340

atgataggtg ctttggagac ttccagaggt aggttttagt tctgctgtgt tgctgtgtga
59400

gcctggataa gtgtctctcc ctctctgtga ctcctcctct gaagtctcaa ggggctgctg
59460

tgagtctaga agatgtgttg ggaaccccct tccccacggc ccagcccagg acagaagctt
59520

gatacgtgaa agttccattt acagtttcag atggagcaga ggaccagggc acggtcaggg
59580

tgacagatgg gtgggggtct tgggggaaaa caaatctcat aaaccttggc aagctcaagg
59640

tcaggatggg gaagccctta aggtgggagg agtgggcaag acagactctg agtgtggagc
59700

cactggacag catgtggtcc aagtgaggcc caggggcagg aatttgttca aagtcacgcc
59760

gcaagttggt ccttgcagtg ggagagccca ggtctcctgc tcaccatcga ctattccaaa
59820

aatgagatgc aaagtgcctt gtatgtgaat tatctcgact taaatcccaa aacaactccg
59880

cttacataga tgctatcatg attcccattt tgtagacaaa gacaccaggt ggctccaggt
59940

cccagagcaa gtggctgctt tgggattgga atccagagct gtaggattct gtaggattct
60000

gccctgggaa ccacctcact acagctcagt tttttctcca gcccctctta tgaccacccc
60060

ttcgccacca tgtcctgtgt tgttctcaaa cctgggggca gatagttctg cgccactgaa
60120

gtatcaggga tgtcggtccc cgtgtaatca caacagaaga gacctcagtg gccagaagga
60180

aaaacttcct gctggcctat tgcggcgagg aaatgttttt ggaaacttca gacccaggga
60240

gcttgagcag gaaagaggcc attgttgttc ccatggaaga ccctggagat acggccagtc
60300

cctgggctgt gggagctggg gcattttctc catctgttgt ccgaagaagg gtttccagaa
60360

atcacgccca gctccaaaga tgcaggtcta gaattgtagg atttatagga tttctgcact
60420

tagaaagggc ctaggccttc taggccagtg agacctctgt cagaagtggc cactgtgaca
60480

tggggctgga ggctcatcat aactggcatc tccaggatct cccagcccca ctgagccctg
60540

cttttgagca tgggggtgag aagatgcatt gaggacaagc ggccaccgag gcgacacatc
60600

acagtgtgtt gggagctggg cccgagggag gcaggtggct gagatcacac agcaggtca
60660

ggtgggtcca cagttcctgc cacccattcc tgctccacct gccccatgtc cctgtcccac
60720

accccttcct gggggttgca atgggtgcag aagccatccc cagatgaact tgacccaaga
60780

gccagctggg attctgggtc cagcagtgac atcctccatt gtagaaatcc ctccagctgg
60840

gacctacgag agtgctgctt gtgtgtgggg agggagtcag gggcaccagt atgaaattag
60900

ttaagcctct gttccactcc tctcgttcct cacagatgca ccaaaggctg gagcagagga
60960

ccagggcacg gtcagggtga cagatgggtg ggggtcttgg gggaaaacaa atctcataaa
61020

ccttgtccta taaaataagg acactgttgt ccttatttta taggtgagac tcaacaattg
61080

gccaaggtct agggttgcag aacgtgaggg cgggaagaac aagcctgatt tccgaaggct
61140

cctggatagg atgccgaaag ctcctctctg gccgctggag ccaaggactc agccaggctt
61200

ggcttgcaca ccaccggcgt ggcatgcaca ccacctggtc ctggctggca agaaccaccc
61260

aaacctaagt gttctgaggc caggccagca ccccctgcac agctccaggc acacatcaga
61320

gagccagccc ccactgactg actgaggaca cgtgccagtg gtccgccagg acagtacttt
61380

acagtttaca acaccccgtc gcgttccatt gcctcacctg atccagggag gcaagggtta
61440

atggttatgc agtgtggatg gggaaactga ggctccaagc agcattgaga cgcctcctca
61500

cccagcccgg tctgcctgtt gctctccaaa tcaggactta gggattcagc tcccaccggg
61560

tcctgacccc caatgccccg gggcccctga gtggcggctg cggtgactct gatactcacc
61620

ctcggccatg gtcgggcggg ggcggtggct ccttccactg gcggcgagac cctccaagtg
61680

cgaccactgg cggctggcac tgccgaggcg gagagcggcg ctaacttata gcccgctttg
61740

gggcgggccg ggggcggggc cccgcgggaa ggcggcgacc tgggaggcca cccattgccc
61800

agtcccgggc ccacgccgcc agcgctgggg ctcacccagg ggaccggagc ccgagggagg
61860

gacggcggcc ggacgagggc gccccctggg ggagcggaca gggacacaca actagcccag
61920

gatacagaca cacctggacc cacagactca gaccttggag aacagacacc tggacacaca
61980

cactcagata cagacacacg cagcaccctc atagaaacac acaacggaac cggggtgcac
62040

ctggacacac agatgtggac tcttaggtca gatctgggat caagaacacc tccccacccc
62100

caacacacac acacacacac acacacacac acgcacacac tcacacacat actcagatat
62160

acatacacag agagcagacg cagacacacc aagacagggc tgtggagata cacacacaaa
62220

cacagataca ctcccacaga tacaccaaac acggctggcc acgcagacac agtgacatgg
62280

atacaaattc agattcgaat ggcagacaca caccaggaca tagatacagc ctgacacagc
62340

caggtagcct tgaccgcgag ccacacacag ctgtggacac agatgagcag gtgtccagag
62400

gcacctggca cacacacaga cctcctagac accaatgcca ctgtcagatg tggacgcaaa
62460

atcagagagg gcacccacag acacacaaag atgtggctac tcagaggtag acacacatgc
62520

aggcatggac tcaggtacag acatgcagac acacctgcaa aaacagacac acccatgtcc
62580

cttcctgtag caaaacccag ataatcctcc gatctcaccc atccttgtat ctgtctaatc
62640

agccatccat ctaatcagcc atccatctac ccatccatcc actcacccat gcatcaccta
62700

cccccccacc tatccatcca ctcatccctt catccatccc tccctcggcc ttctcttccc
62760

tccctccctc aatccatcca tccactgagc tttcttgagg ccccactgtg tgccagaaag
62820

gagcaagact aactttccag ggactcagcc taggacttct cgaacctggc aaccaggggt
62880

cctatctctc atcctggtat ctctggtctc ttggtctcct gtgcttccgg atccactgtg
62940

agccctgagc gggaagggtt cccggagaga cagaggccca cggggcttcc ctggcacttt
63000

ttccaaaaca agctctacat tccggagcaa gctctacatt ccagaacaag ctgtacattc
63060

cggaacaagc tctacatcta ttgatgaatt tggaagatag aagtagcccc cgtctccatt
63120

cctaggtgac atatggctca gggaaaacaa cccgtgttgc ttcagtggat atttctccct
63180

gggtcaaacg ggctctggct ggagtctcag tgaacaaacc cacaaagagt gttgggccca
63240

gctgacccgt aattgcccca ttttatagag attgttgcag aaactgctcc ctgcagctgg
63300

tgaaagcttg gaggaaagag cagcgtgagg agaatcaccc gaccgagagg gaagtcggtc
63360

aagaggccgg ggttcaagtt cccactctgc ccctgaccag ctgtgtggcc atgggcagga
63420

ttttgcctca gtttcctcct ctgcaaaatg ggggaaagaa tccctccctc acaggcctga
63480

gtaagactca atctaaatga ccaagtttgg gtgccttata cgtggtgagc atgcaataac
63540

tggggccctg agatttggca tggaaatatc aagtatccca gcacctggct atttcgggac
63600

ctcaaggaga gacgggaggc atggggacag tcagttctgc agaacaaagg ctgtgtttcc
63660

acaggcgtta gccctgcccc agatgaaagg atgggtttgg ccttttctac caggtatttt
63720

ttcctttaaa accctcagcc tgggagctcc ctagaagaca gggagccacc taagtatttt
63780

atttttttga gacagggtcc cactctgtca cccagatcgt gtagtggtgt gatctttgct
63840

cactgcagcc tccatccctc gggctcaagc gatcctcctg cctcagcttc cctgagtagc
63900

tgggactaca ggagtgcgcc accatgccca gttaatattt ctaatttttg tagagatgga
63960

ggtctcacta tgttgcccag gctggtctca aactcctgag ctctagtgat ctgcacgcct
64020

cgacctccca aagtgctggg attatagtca tgaaccactg tgcccagccc acgagagatt
64080

tttaaactct tcttgaccac agtgtccagc atggtgcctg gctcaccacc agcccccaaa
64140

agtgtttgat ttgagaaatc atcaattcta tcagcagcag caccagctcc attctgaaca
64200

ttgttattcc ctcttcctgc aggcctggac ttagttcatt acacagagtg ggtatgtcac
64260

gcagctctgg gtttaaattt tggcctcctc actgtgtagc catgtccact acacagcaag
64320

tcacttaacc tctgtgcctc agtttcctca tttgtaaaat gggggcagta ttggtaccca
64380

ctccccagag tgtcatgagg actgagtgag ctaattcatg gagagtttgt acagcagcag
64440

ccaaacagtc agccctcaat ggatgctggc tgtgagaaga atcccattag attattcctg
64500

gatcccagca cagggcttag cacagaattc caggcctgtc ccgcacccag ccagctgccc
64560

ccacttcctc tcccacattc cacagaacta tggccacctg agtgacgcat ggcgttgagc
64620

aaccccagga tgtggccagc cggggtcagg actggcttct aggaagttgg cagacagagg
64680

ccagaaccca ggcttggccc agagacctgg attccagtcc cagtgaccag gcacccttgc
64740

ccctctctgg gtttcagttt tcccatttgt tcaaagggac tgggaaactg tgccctgtct
64800

tacctcaccg gggctgtaaa ggtttcaaaa ctcccctgag tcccagagag ggtgatcatg
64860

gcctcaagat ccccagacac tgcaatcagg gccctttgtc atctattcag gggaagtgtt
64920

gtgcaatgct tagagcagga gggctgtgag gtcactcttg gctgtgtgac tctggataag
64980

ttactaggcc tctctgagct tcagtttcct cctctataaa attggtatcg taagagtccc
65040

tgccactggg gattagagat catgtttcta cagtgcttac aggtcacata gtaagtactc
65100

agtaaatgtc acctaagctt tatgatgatg tcatcaagag tcaggaccct ctccagaaaa
65160

gggagagtcc ccgtgtgtgg gtgaagcagg ttgcagtcaa tgtagcatat ttatttggtg
65220

ggtgttgctc agaaagactc tctgtctcac aaataaatct tgttctcaca gccactgatg
65280

cctagaatcc cagcgttaga gagattcagt cccttccaca aggattctca agcctcaagg
65340

tggagacaaa ggtgaatatt tagaaacttt ggaataaccc actaccagcc agatattcca
65400

tttaaacctc acagtatgat tcttatcatt tgttaaatga ggaaccctgg gttcagagag
65460

gggaagtgcc tcacccaagg ccacacagtc aggaagtggg aaagcagggc ctgtgctggc
65520

tccgccaagg ttcagaggtc agtcctgtct ccccgtgccc tgcagggcag tcctcagggg
65580

cctgggccat gtccagttct acagctttta gcaagtgagg cctccgagga ctgcagcttg
65640

tgctcctaac acatggcatg tggtatacac tggatgctta ataaatgttt atgacctgag
65700

ggaggaatga gttacccaca gctggagaaa gaatagaagg aaggaaggaa acacagaaac
65760

aagattaaag ttcagcaagg ttggagaaac tgcctaaggt cacacagcat gaccaggctc
65820

tcgggccctg gctcagctca gccactcctc aaggacaagg tgcctccagg ccagagacag
65880

acgtagacag ctgatgtgag agggagggag ggcggggtgg gcaggagtgt gccagggcat
65940

ctggaggctt ttctaccaac tactgactaa ccgggatgag cagtgtctgg tgtgagggag
66000

catagggagg gtggggcagg ctctcctgag gtgggatggg gtggggcagg ggtgttaggt
66060

tgcggactta aggaaaatgt caggaggcag gtgtgacctt tcaagccctt tgagagaatg
66120

gcctgtctgg ggcaggatct gggtctccca ggctgtggag gacagagaac agggcatctg
66180

tggagggagg gaggattctt ccagcgtgtc cactttgcag atggagaaac tgacctggtt
66240

gtcttgtcct cagctctgac tccacaatgt accaccctcc aggtgtagtg catccccctt
66300

ccaccgtccc tgcagccggc acggggtccc agccccttga gcttgatggg ccatgggtag
66360

gcacttttat tcacttctct gaagcatcag tttctgtttc tgtgaaagtg agaatcacat
66420

cccagacctc atgaggcact ggaggccctg cagggctgac ccagcttacc cttcaatcgc
66480

atcttccaca ttcatccctt ccagcctcac caggcatggt acttgccctc aaatgagccc
66540

tgcaaacacc cacctctagg gctttgctcc agctgtgtcc tctacccagg atggccttcc
66600

ctcttcacct atcgaagttt aacccaggct ttacccatcc tccggaaacc cacccagaga
66660

cttccttctt gctctattgg ccccacaccc acaggacata gtcacagcgt aacttgtata
66720

agttctagtg tttgtgtatc tctgtctccc tccacccacc tggctctcag tagctgctca
66780

ataaaggctt gttgaatgaa ttaatgaccg taccaggggt tctaaggagg agagagagac
66840

aacttgttca agcccaagcc aggttcttgg ggttggcccg ggagctctga taggacttgt
66900

ggtgccagtt gcccctggta ggaccaggaa tcggggtgag gcgctagggc ctctcgcagc
66960

ctttgctgtc tgcaccagag atgaaagatc tgggtctgac ttaccctcca atcatatctt
67020

ctacaatccc aggacaacca ctgggagaaa aacaaaggac agacctgggg atgtgtgggt
67080

agcagagtag gaatacccac cgctgggcct cagagggggc aacagccttt ccccaggcca
67140

cacagcaagt cagggcagag tcagggtggg gttccaaaca ctatagaagt aagcagtggc
67200

cctgtaccag ccaggacgtt gcatgaggta tgattccaca agactgttca atttctatgg
67260

gaacagataa gtaaatatac atacagtaca tacatataaa catacagaag aaggtttgga
67320

aaactccagt ccaaattgct ggcagtggtg ggaggagata tgggggtcta tgactggggg
67380

actggcagtg gtaagttttg aaatttgaga aagctgtttt tatgtaactt ggaattgggt
67440

gttgcattta acaaggagtg ggaaggaaga tgaggctcag atgctggaac cggcagtgag
67500

tccctgtggc aggggccaca gctccaggct ccaacttgcc ctcctcttat tggctgtggc
67560

ctcctcttct acaaaaagga ctcaacacga cacgccatcc tgctgggctc aaaggatgag
67620

atcagaagaa acgggagata aggaaaataa gtcaattcta ttattgttat tatgatgtgg
67680

ctccagttcc tagcaatcca gacacccgag ggaacgggga gttttaggat ttttttggag
67740

aacagagttc tggccatctc tggcagcaag atttgctttt ctcataggac tcaaactgac
67800

aatccgatta tcccttccct agcacacgac cctaacaaag atggtcatct gatatgacaa
67860

ctccaaaaga tgaggagaca ttagggctca ggaaacacta gaggtgggag gtgctgagag
67920

ggtcagaaaa accacacaag caggaccgga gctctgacat tgacaacaga tggcgtcatt
67980

tggtgttggg ataaacagtc ctgctcattc tgtgcagata gagcagggca attgtcaatg
68040

attgccgtgg tgacccagag ccatcctatg agtagagaga ctttgtgact tgcacattaa
68100

gcccacaaga cacttttcac tcccttctgg gttcttctag cagtagcaag gttcaactac
68160

aaggacacgg gcctgggttc aaatccagct ctgccatcga ctgtcctgtg tgatcttgaa
68220

tgaatcacag cacatctcca agcctcagtt gtcccctctg tgaaatgggg gtgataaaaa
68280

cagtgcctac attacaggat ttttgtgaag gttatcacca ttcttggcag acagtaagca
68340

ttcaaataaa ggtggttgat tactcagcaa taaaaaggaa agatggattg acacatgcta
68400

caacaaggat gaacttccaa accatcatgc taagtgaaca cagccagtca cgaaagacca
68460

catatcgtct gattccattt atacaaaaca cccacaatag gcaaatgcat agatatggag
68520

ggtacatcag gggttgtcag gagccaaggg aagggagaaa ggaagaggga ggaatgagga
68580

gtaattgcta atgggtacag ggtttctttt cgggagtaaa gaaaacattc taaaattgga
68640

tggtggtgat gtttgcacaa atccgcaaat agactaaaag ccattgaatt gtacacttaa
68700

atgcatgatt tttatggtat gtgaaatata tctcaaaacg tggttttcaa aaatgtttga
68760

aagatttaaa ataaaaaagc caggaataaa gctctgaaag tctttgcaaa tattgtctca
68820

ttctcaaata aaaaaaaaat ctaagagaaa ctgcacatgt atgtgcccaa ggagatgtct
68880

ctaggagggg tctttacagc attgtctgtt atggcaaaaa cccatcacaa cctaagtgcc
68940

cagtagaggg ggttggataa agcagccatg cctggaaata ttactcagca gttgaaagaa
69000

agaagagaca gtctcctgtg tgtggacacg aaaagaattc caagtcataa tgtagagcga
69060

agaaagcaca ctgcaggaca ttgcatgaag tacgattcca ccagactgtt caatttctat
69120

gggaacagat acagtacata aatgtaaaca tacagaagaa ggtttggaaa aatccactcc
69180

aaattgctgg cagggtggga ggggatatgg ggggccataa ccaggggaca gacggtagta
69240

atgaaaggtg atttcagtat tccctggaag gtgagaaaag tcttctgtga agaaataatg
69300

ttatctatca cttaggtaat taaagattaa ttttacaaaa aaaagaataa cctgttttac
69360

atttagaatt aagcttttat taaaaaagga gatactccaa aataaataaa ttctggcaaa
69420

gtagatagaa aaaagtaaag tcgaagataa aggagaaagt gaatgtgccc cccacaaagc
69480

ccacaatcaa cccctaaaat acttttgtta atattggcac agtcttgcag aaccaaaccc
69540

aaaaacaagc acatgaataa ataagaaatt caagatgaga attgaatcac atctaagaaa
69600

cgtaattttg aaggctacac caagtttctg tttacttcac aaactgttta tgtctcaaag
69660

tgctcctgca gcaccaccct ggaaagtgct tgaaaatctt ctattttgtg attttgtgtc
69720

aatgaaggtt cagagaggtg cagtgtattg cccaaggtca cacagctcag aaagagtgaa
69780

gcaggggtgc cgagaagtgc ttgctggaat atcatgcaag gggaattaag cctgcgtcgt
69840

gggtgcctgg ccaagctccc atggccatgt ggggctgctg gagacacgga gggatgaagc
69900

accagcatac tacagcattc ccctacatcc ctcccacaag gctgagtcat tggctgtgtc
69960

tgcctggcac acccagttca gtcaataaga gtcaaacaca ctttcattca tggaaataaa
70020

tgaccctttg ggggcagtgg cggagctcgt ttgactaaat tgaacacaag tttggctaaa
70080

ctgaccactt gtttctcccg cctgtctgat gagctgcttg agtgacgtct ggctgaactg
70140

agtgcttgta ggtctgaagg aacattcagc ttgacccagt tagtttaaat ccctgtcttc
70200

gcacctgcag aggggctgct gtcacctcgt gccagcatca caggagacgg gaggtttggc
70260

ctcagtcctg gaagggcagg gctcagtctc cagaataata aaccccacac acatgtacag
70320

gcatctctag ttggtggtgt cctctttgta ggcacagcga gtcctttgtt ttgcttctgg
70380

ctgactgctc ttttctatag gtttgttgat actgtcaaac tgtcaatcaa gtggccccac
70440

ccaactctag ttaaggacag gtctgggacc caaactcagc ctatcagaag ctcttctggg
70500

actttgactc ttacaactct gacacaagtt ctaaataatg gttgaagttt gctggttcat
70560

gtctagaact acgtcagctc tcgtcttttc taaagttggg ctttttagct gtttatttga
70620

ttttttaaaa gtcatattta tggagttaaa atttacatac aatacaattc acccgtgttg
70680

tattacatac aatacaattc acccatgttg tattacatac agtgcaattc tcccatatca
70740

tgcaatacag ttctgtgact cgacaaacat atatagtcgt gttactacac cacaatcatg
70800

atatagaaca tttccatcac cccccaaatt cccctcatgc ccttttgcag gcaattctct
70860

ccccctaccc tccgcccctg gcaactactg atctggttac tgcgccgatg gttttgtctt
70920

tccagaatgt cataggcatg aaatcacgca gtatacagcc ttttgtgtct ggcttccttc
70980

gcttaccgta atgtatttga ttcatccctg ttgttgcagg tatcagcggt tcattccttt
71040

ttattactga gtactattct gtcgcatgaa tataccacaa tttatttgta cattaaccag
71100

ttgaaggacc ctggggttgt tctaggtgat tatgaatgga gctgccatgc acatagaggt
71160

accgctcatt gtgtggacag atgtcttcac ttctcttggg caaatacctg tgagccatcc
71220

ccatatcctt ccagctaatt ttttggttgt ggttaagctg cactgagcct cttttcattc
71280

atgcaaccca aaccaggttt gggtcccaga cctgccctta actagagttg ggtgggacca
71340

cttgattgac agtttgacag tatcaacaaa cctatggaaa agaatgggac agtgacatct
71400

ccctcgtcta attctcccag tgactctatg gggtttatgg attattgacc tcattttacc
71460

aaggaaacgg aggcttggag agcctaaata acctgcccca gaccacacat caggtactca
71520

aatctaaatc aaagtcacaa aggaagccac agccaccttt ctaatttcct agtcaccttg
71580

gtgggagggg aactttcaca aggaaggttc taacaagttt ttattgactg acgggtgatc
71640

gaataaatgc ctcactcaaa ggagtttatt ctctgggcct cagtttccca tttgtgaaat
71700

gacatgtttg gaccaagtaa agaatttctg atgcccctag cgtccgtgat gctcacatgc
71760

ggtggctgtg aaagtggagt catcagggcc ttcttttcgg atgaagaatt ccagcaaaaa
71820

ggagctggtg ggggaggggg agggggagcc cagcacttac tccccaatca gttcccacag
71880

gcggcctcct gagcttgtgc ccagggctgg ttgaccccta gaagagcaat ttgcacttcc
71940

tgcctttctg gcctaaaaaa cgaaaagaaa agaaaaagcc cctgtcacct tggattaaca
72000

tcaccccctc tgctgagaat cttctaaggg gtgcagcccc ctctcttcac caatattttc
72060

agaatgtcca gttttctaaa caagacttga cagggtggtt tcttttttaa gctttttaaa
72120

ataaaataaa tcaccctgtg catgcacgat cctggaacct ccaattagat taaccctatg
72180

gtttcctaag cggcagccag ctgtgggcac agagaggtag gaggaaggaa gcgacggggg
72240

cctcacctat gagccggact cacctgcttc ccaggtcacc tcccttccca cggctcacag
72300

gacagtaccc aggcccatca ggaagctcag aggcagaatg actgagggtt tcaaggcccg
72360

acagctccta gctgtacaac ctactgcaaa tttcttcatg tctctgacct cagtttccac
72420

accctgataa tgggggtaac gagaggacca agctcacaga gtagttaaga ataaatgagt
72480

tattgcaggg tttagtgttt ggcgcaggga aaagctgcag atgttgactg tttttagtac
72540

tcttataatt acttctatgg gctttgagga cctcgattcc aactgccccc ctgcaagtca
72600

gctctgaggg taggtgagag agcatggtca ttgttcacct cctgtgtgcc aggctctgtg
72660

tgaagaagct ttatgcatgt gattgccctc cacctccttt tttttctttt tcttttcttt
72720

tttttttttt tttgagatgg agacttttgc tctatcgccc aggctggagt gcagtggctt
72780

gatctcagct cactgcaacc tccacctcct ggattcaagc aattctcctg cctcagcctc
72840

ctgagtagct gagattacag gcatgagcca ccatgcccgg ctaatttttg tatttttcat
72900

agagatgggg tttcaccatg ttggccaggc tgggttcgaa ctcctgacct caggtgatct
72960

gcccgccttg gcctcccaaa gtgctaggat tacagacgtg agccacagcg cctggctgtg
73020

attgcctttt gattgtttgt ttgttctcag ccgcatctgg ccacacaagg accgtaagtt
73080

aatccttctc tcaggtagtt ctatgtctag atgatgaatg gctccatcac ccaacttcca
73140

gatgtaaaga gtaggggtca gaggccgggt gcggtggctc acacctgtaa tcccagcact
73200

ttgggaggcc gaggcgggca gatcacgagc tcaggagatc aagaccatcc tggctaacac
73260

ggtgaaattc cacctctacc aaaaatacaa aaaattagcc gagtatggtg gcggatgcct
73320

gtggtctcag ctactcggga ggctgaggca ggagaatggc atgaacccgg gaggtggagc
73380

ttgcagtgag ccgagactgc actactgcac tccagtctgg gcaacagaac gagactccat
73440

ctcaaaaaaa aaaaaaaaag agtaggggtc agggagagca aggagcatgc ctctggtcac
73500

tgggggaact tcttgagctg gggtgggccc tgtgatctgt ctgggtggat tcatactgga
73560

tctgatccct tccctgtggg tcctggtccc atgctctggc acagactaca gatggggaaa
73620

gtgaggcact tgcccaggac cagatctgcc caggtgccaa gcccgcactt tttgcaaaat
73680

accagcatta gctagggagg gagtaaggat gagaagggca gcaggaggag gtgggtggta
73740

ttgttgtcca ggtgggagag gacggaggct gggctgggag gtgggaggac cctgctcaca
73800

ccccctttgt gcacactgtt tcttgtgctt ttttatcctt cacagcttaa ccccaggatc
73860

acgtctcgac gacagccgcc tcatcaacgg ccgcttggcc atcatttgcc aagggcttac
73920

tgagtgcagg ggccctgaaa tgtacatgca tcgtcttcag tctactcacc agccctgact
73980

ttaaatgcca tctatgtgct gaaggctccc acatttgcat cttcagtcct gtcctcactc
74040

ttaaatccca ggtccaggtt tccaatcgcc tcctcaaaat tcctgctgtg ccccacccgc
74100

tattcactcc cccgcagtat ccccatctcg gtggaagcaa caccaccatt ccctatgctc
74160

agtccccagg gtcagccttg actcctctct ctcagatccc acctcaattt gtcaggaaat
74220

cctggttgcc ctgcctttaa aatgtctcta gaatctggcc acttctcaca acctctgcct
74280

ctgccaccat cattctggtg caaaccacca gtttctttca cctggactag ggctccctgt
74340

tttccccctt gctccccact tccaatctgt attccacaca acagccaaag gaatctcttt
74400

aaaacttaag tcagttcctg ccactcctct gctcaagagc ctgcagtaac cccctgcttc
74460

actctgatta aagccaaagt tctcatcagg tccccaagac ctccaccatc tgatcccgtt
74520

gttctcggac tttctctctg accactccct gctcacttgg ccccggcacg ctggcctccc
74580

tgccattcct tcagcttgcc aggcgtgtcc tctgtttaga acttttgcac tggtcgttcc
74640

ctcggcctgg agtgctgttc tttgacttgg tggcctgggt atagtgtggc gcctgagaag
74700

tctgtgctgc atccacatgg caggctctgg agagtgtgga gggaacttgg tgatgaaggg
74760

acagagattt ggcccacaca cattagatgc tgtgtgaccc tggagattgc ttaactgtgt
74820

ctcaatttcc tctttagcca aatgggctct aagtgagctg atgatgttac tggggtccct
74880

gactcattgt agcttgtggg gagaaagtgg tcatgccctc agaaactgtc tggtcaaccc
74940

tgccattgta gagctgggaa cattgaggcc agagaggagt cccattgtcc aaagacccat
75000

aaacagttag agagagagct atggggtttt tgaaagatta attgacttat tgaataattt
75060

ctttattgaa catttatcga gttcctactg taggcttaac tgtgagtaag acagtttctg
75120

ctggcaagga actctcagct tagtggacaa aacagatggc aaggtgctca ctatgcaaat
75180

agtaattcct ggcctggtgt gatggctaac ccgggtaatc ccaacacttt gggaggccaa
75240

gttgggtaga tcacttgagc ccgagagttc aaaaccagct tgggcaacac agtgagactt
75300

cgtctttaca aaaataaaaa taaaatatta gccaggcatg gtggcacatg cctgtaaacc
75360

tagctactta agggaggctg aagcaggagg attgcttgag cctaggaagc tgagactgca
75420

gtgtgccatg atcatgccat tgcataccag tctgggcaac agagtgagac cctgtctcaa
75480

aacaataaca acaacaacaa caacaacaaa acaagaacaa caacaattaa ttttcacaac
75540

ctgtgttcag ggtcgtagcg gaagaaggct tgggacctga gagaccccca gataaaaaag
75600

ggattatctc tgtgtggcag atcaggacag cctccatgga gaaggtgaca caatgtgggt
75660

tttgcagtat gaatatgaat tatgcagata aggcaaagag gagcatttca cactgataga
75720

ttttggatgt tgtctgctct aagtctcatg tcgaattgta atccccagtg ttgaaggtgg
75780

ggcctggtgg gaggtgattg ggtcatgaaa agtggatttt tctgcagcca taaaaaagag
75840

tgagttcatg tcctttgtag ggatgtggat gaaactggaa accatcatcc tcagcaaact
75900

aacacaggaa cagaaaacca aacaccgcat gttctcactc ataagtggga gttgaacgat
75960

gagaacacat ggacacgggg aggggaacat cacaccctgg ggcctgttga ggggtgggga
76020

gaaaggggag gaagagcatt aggacaaata cctaaggcat gcggggctca aaacctagat
76080

gatgggttga taggtgcagc aaaccaccat gacacacgta tacctatgta acaaacctgc
76140

acgttcagca catgtatcct ggaacttcga gtaaaattgt ttaaaacagt ggatttctca
76200

tgagtggttt agcctcatag ccccggcact gtcctcatga tagtgagtga gttctaacgg
76260

gatccggctg tttaaaggtg tgcggcatct tccccctctc tctcttgcac ctgctcccac
76320

tatgtgatat acctgctccc acttcacctt caccttctac catgagtaaa agctccctga
76380

ggcctcccta gaagccaagt ggatgtcagc accacgcttg tatggcctgc agaaccctga
76440

gccaattaaa cctcttttct ttataaatta cccagcttca gacattcctt tatagcaacg
76500

taagaacagc ctaacagaca tgcaaacaga cttgtctgtg cacacagggg taaagttaca
76560

gaagtgagga atggtgtaga gaattcacag gaagaaggag tcgaacggta tggctggggt
76620

tttgggggta cttggaagga atgtcaaggc tggagtggtt agtggggctc ggacaacaaa
76680

cgtctctgtc taccaggctc agtttaggac acgggaagcc atgggaaagt ttaaagcagg
76740

gaagtggcat agtcaaggct gcattctggg aagatagtgc tggaattgtt tatcatggaa
76800

ttttagattt tttttttttt tttttttttt gaggcagagt cttgctctat tgcccaggct
76860

ggagtgcaat ggtgcaatct cggctcactg caacctctgc ctcctaggtt cgagccattc
76920

tccttcctca gcctcctgag tagctgggac tacaggtctg cgccaccacg cccagccaat
76980

tattttgtat ttttagtaca tttcaccata ttggccaggc tggtcttgaa ctcctgacct
77040

catgtgatcc acccaccttg acctcccaaa gtgctgggat tacaggtgtg agccaccatg
77100

cctggccaga attttagatc ttgaatttag tgattatcat catccctggg ccctttttaa
77160

aagaagagca aattgaggcc agaaagggaa atggcaccca ccgttcatca gggacagaag
77220

catttgctcc caccttggtc tgtgtaggtg gggctgagcc tgaggctgga gtatgtttgc
77280

agatcctgtg gggtgagctg ttcccagtgg gaccagttcc cagttctctg tgacatccct
77340

ctctctgctc ctccccttcg gacctgccct gcaggctcca aatatcgtgg ttgatatgga
77400

aactctcctg ttacacattc cttttccagt tgtgtgaccc tggcagtgtc cttcctctct
77460

ggagcttcgg tttccccatc tgtctaatgg gagtgataaa tggagtgatg gctggcactg
77520

attatcaagc actcgggcat ctcctccaat tctcccaaag gccccaagga agaagagggt
77580

ctgccattat cagtgccctg tccaccggca gaaagtgtga ggcagagctc gttgtggaaa
77640

gcaaggctca taatttacct cactaacctg tgtggttctg gatggcacag agccaactgg
77700

gactctcgtt tttctcattc tggtccctac tcccttcttc gtgtgaccta agaccttaac
77760

agctgtgtaa gtgaaagtgg aacaaacaaa gcaccccctg agagacttct catgatacca
77820

aggggcagcc ccagaggaca gaccaacgct cctctcaatc cctaaaaagc ctctgcctga
77880

ctgagcacag ctgggagcag tgatgtcagg cctggcttgg tagatcaaag cagcaatggc
77940

cccagggcct gatgggtgca cgggaggggc agggctgagt agttgatcta gctcttaaag
78000

atccttgagc ttgaagtcac cctgggacag cctcagcaag cgctcaaggc tggcacttgg
78060

gaaaggtcac atggctttga tgcttggcca gttttagatg gggagaagca ctactatgtt
78120

agagtctaga ttatcttaga aacccaactc tacccaggag ttcctgaaga aagaccctga
78180

aatctctcag gcagcaaacg gaggtcaact tggtcaccaa gcgtggcttg gtgcccactc
78240

tggacccagc tctgtgccag gccctggggg agatgaagaa atgaggcagg cctgttttct
78300

ttctgtggct tcaacagaac atctgtggtt tccccctaat taatcgtaat acatgctcac
78360

tgtttaaaat ttataaaata cagaacagcc aaaagatgaa agttaaagtc aatccaagta
78420

tcaccaacaa agagaacaac tgttaacatt ttgttttatc ttgttcagtg ttgctatatc
78480

atatttaggt gtatgtgtag atacaaactc acataattat gtatttagtt tctaaaaatg
78540

gaatcactca attgtccagg aaatgtttgt tgaacattca ccctgtgcca agcatgtggc
78600

aggcagcgga ccagaggatg cgattcctac tgtcatgaaa gttgcctcta agcactcttt
78660

tgtcccttgc cttttttcac tgtgtgccta acattttcaa atcacatgta tttctacaac
78720

atatttattg gctatcaaga atttcatcac atggctgaat ttgtcaagcc ccacttttct
78780

tgcgcattta ggttgcttcc catttttttca ctactgcaaa cagtgccatc cctgtagcct
78840

tgggctcata aggtggattg gcttattcca tatcaatttc aaccccctct tatatgcctt
78900

cctcaactgc aggggctgga agggtgaaga ttgcatttcc caactccctt gcagctaggg
78960

tttagagacc aattaagtag ttcccattaa atgcatttgt gtgaggtttt gaaggcaaaa
79020

gtaagagaga ggtcatcttc ctccttttgc tggttgtgaa ggcattgaga cctcccacag
79080

gtgtgtctac agtgcctggt caccaactct tgggtgttga gaagtggtga tggggcatca
79140

gccgcatctg atggctggag tgaagctaag gtgacaaact cctgaagaca atagttccca
79200

agcttctggg tggtggcaga ggcagcagct ttcctgggca ggccagttta gcggtgacat
79260

tctaagaatc gctcctagaa gcccaggtcc cttgcttcag aattttccat gattttgcaa
79320

gaacacaact tctcgcatta aatctcccta tgttgaaagt agtgaatgtg gtttctgtgt
79380

cctgcacctg cactctgggt gatataagct acatctttga gcctgttggc aactatttcc
79440

ttagactaaa tctctaccat ggaacttata agtcaagaat tcattttttt ttgtttattt
79500

aaatagtttt actgagacat aatgaacata ccatgtttaa agtgtacaat ttgacgttta
79560

gtgtattcac aaagttgtag atctatcatc atcatctaat tttggaacat ttcttcaccc
79620

caaaaagaaa tttcatgccc attaggagtc aattccttac ctctctcccc actcctaggc
79680

aaccgtgaac ctatttccta tctttatagg tttacttatt ctgggcattt catataaatc
79740

aaattataca atatgttatc ttttgtggct gccttctttc acttagcata atgttttcaa
79800

ggttcatcca tgtcgtggca catatcagaa ttgtatttct ttttattact ggataatgtt
79860

ctgttatatg aatataccgc attttattta tccattcatc agctgataga cattcgggta
79920

ctctccactt ttattttatt tttagagaca gggtcttgtt ctgtagccca ggctgcagtg
79980

cagtggcaca atcacagctc actgcagtct caacctcctg ggctcaagcg atcctccccc
80040

cttagcctcc tgagtagctg ggactacaga tgcgtgctac cacacttggc taattttatc
80100

ttattttatt ttattgtaga gacaaggtct cgctatgttg cctaacctga tctcaaactc
80160

ctgggctgaa gcgatcctcc tgcctcagcc tcccaaagtg ttaggattac agtcgtgggc
80220

tactgagcct ggcttgtttc cacttttaaa ctcttgtgaa taatgatact gtgaacattt
80280

gtgtacgtgt ttttgtgcgg atgtatattt taatttctct tggggaggct tgtgtcagtg
80340

aaagttgggg tgttggtcgg gggccgagca ggctgtagtg gtacttggga aggagaagga
80400

aggggaatgg aggcttactg taacatccaa aaagcatgat gataaaattg caattggatt
80460

gtgcctgcat ttcttctgga aaacaagact tgaatgcaaa gacagcacca aacaaatcta
80520

gaatgacgag cctcaacatg tagtctaaca agatggtttc aggcccaagt gatgtaactg
80580

gtggaatttg gaggaagaat ccttcctttg aagtttttct atattgttgt gctgagaacg
80640

cttcatggta ttcagtcaac tggagcgtgt acgtggagtt ttgatattgc cgcctgcctt
80700

tggccagcag aacagtttac tcctccaaga gctcactcag gtgaaaagtc aggaatttac
80760

tcagacattc tgacacatgt gtcatcaatc ttggcgtttc ctccccctga cattgattta
80820

ttacagtctc tggctccgtc tatgtgtgga tggaagatgt ctgggaaagt cagagaagcc
80880

tgttacgagt ttggtctcca aatccaagga ttgatatttt tgccggattg gactacttct
80940

gtttgaaaaa gaggccgggc gtggtggctc atgcctgtaa tcccagcact ttgggaggcc
81000

gaggcgggtg gatcacctga ggtcaggagt tcgagaccag cctggccaac atggtgaaac
81060

cccgtcttta ttaaaaatac aaaaaaatta gctggacctg gtggcacatg cctgtagtca
81120

atcccagaca ctcaggaggc tgaagtatga gaattgcttg aatccaggag gcggaggatg
81180

cagtgagcca aggtcacacc actgcactac agcctgggtg acagagtgag actccatatc
81240

aatacaaaaa aaaaaaaaaa cgaaagaaaa aaaaaaagaa aagggtggtg gtgggagtta
81300

ttgttggtta tctcctggta cttgtcttcc tcttcttctc taataaagga atttttagca
81360

gagaaaaaaa catttctctt ggatctatat acctagaagt ggaattgctg gatcacatgg
81420

taactcaatg tttaactttt tcagggacta ccgaaccgcc ttccaaagtt gttgcacgat
81480

ttcacattcc cactagcaat gtatgaattt ccaattgttc catatcctcg ccaaaactta
81540

atgttgtctc tcattttcgt tagagccagt ctagtgggta tgaagtcata tttcatggtt
81600

tagatagaca tttccctaat gactgaagat gctgaacatc ttttcatgtg cttactggcc
81660

atttgcatgt cttctttgga gaaatatctc ttcaaatcct ttgcccattt ttaggttatt
81720

tgacttttat ttatttattt agagacagag tctcactctg ttgcccaggc tggagtgcag
81780

tggcacaccc tcggctcact gcaacttcca cctcccgagt tcaggtgatt ctcatgcctc
81840

agcctaccca gtagctggga ctacaggtgc cagccaccat gcccagctaa tttttgtatt
81900

tttggtagag atggggtttc accatgttgg ccaggttggt ctcaaactcc tgacctcaag
81960

tgatccacct gccttggcct cccaaagtgc tgggattaca ggtgtaagcc actgcacctg
82020

gccccttaca ttttattatg tatatgagtt gcaaagcttc cttatatact ctggatacaa
82080

atcccttaat aagatatgtg attttccttt ttttttagtt tatgtgggtt gtctttccac
82140

tttcttgatg atattgtttg cagcagaaaa ctttttaatt gtaatgaagt tcagtctttt
82200

tttctttagc catttgtgcc tttaatgtta tatataagaa gacttagatt aacccaaggt
82260

catgaaagtt taatcctgtg tttttttcta agagttttat acttttaact tacatttagg
82320

tctatgatcc attttgagtt gatttttgtg tatggtgtgg atgtgaatat ctagttgtcc
82380

tagcaccatt tgttgaaaga ctcttctttc ccctgttgaa tgaatggtct tggtaccatt
82440

gttgaaaggc aattggccat aagcataaag gtttatctct ggatgctcaa ttttattccc
82500

ttaatctata tgtctatcct taggctggta ccaaactgtc ttgattacct gagcattgta
82560

gttagtttat ttggggtctc ttgcaattcc atatgaattt taggatcatc ttgtcaattt
82620

ctgcaaaaaa aaaaaaaaaa aaaaaaaaaa aaccagctgg aattttgaca gagattgcat
82680

tgaaccttta gacgaattcc aagagtattg ccatcttaat aatattaagt cctttgatcc
82740

atgaacatgg agtctgcctc catttattta gatctttcat ttctttcaac aatatttggt
82800

agttttatgt atacaagtct tgtatacctt ttgttaaatt tatgcttaag aatttattct
82860

tgttgatgct attgtaaatt aaattgtttt cttaatttga ttttagattg ttcactgctg
82920

gtgtatagaa atataattgc tttttatgta ttgattttac atacaggttg cacaaccttg
82980

ctgaatttgt ttattagttt tagtaatttt ctagttgatt ccttaccatt ttcttgtata
83040

taaaagatta tatcatctgc aaatatggtt ttatttcttt cccatctgga tgtcttttat
83100

ttcatattct taactaattg ctctggttag aacctccagt acgatgttga atagaagtgg
83160

caagaatggg catcgctgtc ttgttcctga tctttgggag ggaaacatct aatcttttgc
83220

caggaagtat gatgttagcc gtaggttttt catcgatgcc ctttatcagg ttaagggcac
83280

ctattttttt gaaggctttt gttttgttgt gtgaatcatg cttctttgag ataccagtca
83340

ttctttcttg gctactccct tggccacata attcccatga caagctgggt cactacaaag
83400

gaatagcctt cagaaatgac caaaattctc caggtaatga aaaatcagca gaaaggggga
83460

ggaggggtgg aggagggagg ggggaggacg ggggaacaca catcctcttc cttgatccaa
83520

ttacaatact cctgcttgtg caaccaaata cagaactggc cctttggtga ctgtaattaa
83580

aatgtgggtc cctggctggg cacagtggct cacgcctgta atccccagca ctttgggagg
83640

ctgaggcgca cagatcacct gagatcagga gttcaagacc agcctggcca acatggcaaa
83700

accctgtctc tactaaaaac acaaaaaaat tagctgggtg tggcaggatg cgcctgtagt
83760

cccagctact caggaggctg agataggaga attgcttgaa gccgggaggc agatcgcgcc
83820

actgcactcc agactgggag acagaacgag actccatctc aaaaaaaaaa atgtgggtcc
83880

ctttatgagc tttcaactaa attagtggtt ctcaaccctc agtccaaagc accccccact
83940

ttaatcactt tttaattgag gtatagcatt gtgacagttg attctatgtg tcaacttggc
84000

taggccacca tctttttaaa gatgaaatta gtatttaaat cagccgaatt gagtaaagca
84060

gatcacgaag gtaaagcagc agacagagtg aagcaatctg attaccgtgt gggtgggcct
84120

tggtcaatcc attaaaagcc ttcataggaa aggactgagt tctgccagca ggcggccttc
84180

agacttgaac tgcaatgtca gctcttttct gggtctccgg cctgccagcc tagcctgcag
84240

atttgggact tgccagcctc cagtcatgta agccaattcc ttaaaataaa ataaatctct
84300

cccctcttct ctgctctctc tgtctctctc tctgtctctc tctacacaca tacacacaca
84360

cacacacaca cacacacaca cacccctatt ggttctgttt tctgtttctc tggagaaccc
84420

taatacgagc ctatatacag aagagtgcac atcactgtac aacttgatac atttttacaa
84480

actgaaccca tcagatcacg aaacaggaac atgaccatca ccccggaagt tccccttgtg
84540

cttccatcca ccctccatcc cccacaggta atcactgtcc tgccttttaa tagcatggat
84600

gagttgtgct gattctagaa ctttctataa gtggtgtcac tcagcacgca ctcttttgtg
84660

gctggcttct ctcactcaac cttatgctcg tgtgagaccc atctgtactg ccaagtgtag
84720

ttgtgagtga ctgattcttc cttctgtggg tgctccattg tgtggctcac ctcagtttat
84780

gcatccattc agcttcgaga tattcaagat attcatgttg tttccagttt ggaactatta
84840

caaatggtgc tgctgtaaac attcttgcac atatcttttt tttttttttt ttttcagaca
84900

gggtctcact ctgtcaccca ggctggagtg gtgtgccacc atcagggctc actgcagcct
84960

ctacctccct aggctccagc gatcctccca cctcagcctc ccaggtagct gggactacag
85020

gtgcgtgcca ctatacctgg ctaatttttg tattttttgg tagagacgag gttttttcac
85080

gttgcccagg ctggtctcaa actcctgggc tcaagcaatc taccacctcg gcctccaaaa
85140

gtgttggggt tacagccatg agcccctgca cctggccaca tatcttttga tgaacacgtg
85200

aacacattgt agctgggttt aaacccacaa gtgtgattac ccaagccccc tttctctatc
85260

aaacatttta agctttcttt catttaaatg ttcgaaatga aacttatgga tgataatgca
85320

cacacacatc aatgcaatgt ctctaatata aaggagacat aaaatggaag tgacttatga
85380

taaaataatg tgtatttcaa tatattcttg ggcacaacga ccccagaagg cataatgaag
85440

atgccagaac cctgtatgcc cataattagt aagtctggat tccagagatg taagtagacc
85500

agacgccatg gcacaaaaga agtcagaaaa atgaaatggc aggaggtgag ggtgaagatt
85560

aaaatctgct gtttgggcaa gacataacag acttgactta tttgtatgtg ataacagaaa
85620

gaaagacttt aattggagta aggagagtac cccaagacac gctgccgcct gtcagtgggg
85680

gaaataagga aggtgagcct cagtgtgcag tgacaaagaa ttcccatttt atgacacagg
85740

ccacggtagc tgtgtggtca cccagaaaat gcatcttcct tgaaaatccc atctgcaatg
85800

gaggggtgca ttctgccttc agatccctca tgaggcagag gcgacatgca gatagatcat
85860

gaaagcagct ttaatccaaa atgctgtgaa ggagccatgt ggggcaccta tggaaacaat
85920

ttctgagcaa gattggaaaa caaatggtga ccccatcatc tcaatgtatc cttttcacaa
85980

catagattca aaacgatcat ccttatggtc attttgtgag attcagtgag atttcttgca
86040

tctctactaa tttactaata cttgtctcta agattgtaaa aaagagtcac acatctcctc
86100

tattataagt atctgcagtt ggtttcactg tttttgctgt aactgcattc atgccctgga
86160

acccttttcc tctagatatg tagctggcaa tggtttaaca cattgactag acaggctgtg
86220

aggaatgaga atgttattca ataattgact cactgtcatt gcatttatgt gtccttgtca
86280

ataacaacct ttttctaagt tattgagttt ttcacgtaac ttcttgtaga aacctttcag
86340

tactgcaatg tactaaacgc tgacctatat gtagttgtca taggtttgca tctggcagac
86400

gttcgaaagc tatgaaggat gttgggcaaa tttctgttgt gtggggctgt cccatccatt
86460

gtggggtgcc cagcattccc gattcttgcc ctgtaaatgt cactagagcc cccagtgcaa
86520

aaacgacccc ccatgaatta tgtaaaatgt cccctagaga actactgatt taaacccact
86580

ccatctcaat cttaaccttt tcaccccaca ctaagttgtt ggcatttgag tccaaggtcg
86640

ggggttggta gtgactctta ttatattgtt tttcatttct ttttagagac aggatcttgc
86700

tttgtcacct gggttggagt gcagtggcac attcagagct cactgcagcc tcgatcactg
86760

gggctcaagc gatcctccca cctcagcctc ctgagtagct aggtctacgg gtgcgtgcca
86820

acacgcccag ctaaattttt tggtgtgttt tttgtagaga cagggtgtct ctatgttgcc
86880

cagggttgtc ttaagcttct ggccttaaga caaggccaga agtggtgatc ctccctcctc
86940

agcctcccaa attgctggga ttacaggcac aagccactgt gcccagccat tcttattata
87000

ttttattttc ttatattcag cccatcggtc ctgctggtca gggtctttgg acatcctggt
87060

tctgatatcc aatgccttat tgcccctcct agctgtgctg tactgggcca tcttcccacc
87120

ttaaaatgat ttatagacat gttgaacagg atggggctgg ggacagagcc ccgtgacatt
87180

ccactgtgga cctttgtcaa cggcaatagc aagctgatcg ttgatatctt ctggcttctg
87240

gttatcatag ggatacagcc cagcttccat caatttgttc ttccagtgcc taaatgttag
87300

aactccatgc aggtggaaag agtgtatctc agaagacaga agtgacttgc ccaggctcac
87360

tcagcaagtc agagtcagag caggggcaaa gtaagctgcc tgaggctgac ccactccagg
87420

gcttagggga tttaaattcc tctgatggct actcagcagc tcagagtgga ccatgcactg
87480

ctctgatcta gcctaaaaag gggtggaatc cgagggcctg ccccagccaa gcacacggtg
87540

tccattagcc tattttggca tctggtctga ctctggagaa tgggcagggg gccagtgctc
87600

tgcagagagg gtgggttgag cagacccatc tgattagagg caccagcagc tgcctactca
87660

ggcttctggg acagagtgac ggacagagcc cccacatgct ctgtgcttca gacctaagaa
87720

cataatctct accctctggg cctcagttct gctacctgta aagtgggagc aattcctcct
87780

cccttgctta cttcccagag gtattgtaag gaaccagcaa agaggactag actgtttagg
87840

ccttctagaa cattccaaac acattgtcct ttgctctgag agaaatggga gccactgaag
87900

gagtttgaac agaggagaga agcaatgtgc cttagatttt taaaaggatg ttccggatac
87960

tgtgtgagga acagatcaca ttacggggaa gcttggagct gctgaaccag taaggagact
88020

attgaaataa tccaggccag agacaatggt ggaggtaggg gagctggtga gaaagggtca
88080

cgttctggat acatcttgaa agtagagcca acagaattta ctaacagact gagtgtgcgt
88140

gtgagaggcg tgtcaaaggt gattccaagg gttttgacct cagtaggagg gagttgtcat
88200

tgcccgggaa gggaaggttg caggagttgc tggtttagtg gggcgaaagc agaagtttga
88260

gtttgagaca tgtgaagttt gagttgcctc agacatccaa atggaaatgt caggtatgtc
88320

atcattaaat aaataagccc tgctgcctcc tgaggagcac atgacttcaa cagcatggac
88380

tcagctttct aagaaatggc agcttggtca gacatgagtg tcagcattcg agcacccacc
88440

tcctggccac tgttttctct gtctgctcag cttcttaata tactcgcctc atctcccatg
88500

tctcccctgg tgcctcagtt ccagccctac tcaatgggta gctgcttttt ctctaagaca
88560

ctctgggtcg acctggaaac cccaaaagca aattctgaat acttgaggca gcttgtattt
88620

aaaatcagat catcctgtct ctttagccca ggagtttgag gctgcagtga gttatgattg
88680

caccactgca ctccagcatg ggcaacagaa caagatctca tctcttaaaa aaaaaaaaaa
88740

agaaaaaatc agatcatccc aaacaaatgc cccccggtgt gtaaaaatct acccaggtgt
88800

ttttgtgcac tgtagagaca gatatgttta tttcctataa gtggattatt atgcacgcag
88860

gagagctctg aacccatccc tgccatgaac aggtggggaa ctattagtga gtcagtcttt
88920

gagcctgcgt gctcatctgt aaaatgggca caagggaagt gacaatcaga gcggagttca
88980

ggaccacatc ttctggacac tgactcagcc gggctcctga gctggtcttc tgggttcggc
89040

cagaatcact gattgttcag gaagcaactc ctggagacca gattgttcca agaaggagag
89100

gaaactcaaa tcttagaaat gggtggtggc tttcctatgt cacacagcaa cgcaggtctg
89160

aactggggct gggaccaggc tctcctgagg tcagttcagg ggatcaccct caccctgcct
89220

cctcactgca tacacacact cccagggtct ctgaacttaa agcagcccac ttcctagcca
89280

aagaaacatt ccctcgaccc cacctcgtgt tccactttgc cagggaccct ccctcccttc
89340

tctctcctcc ctgcctggac attctctctg tactgctgcc acctgctcag ctgcttggtt
89400

ttacgcagcc ccccatgaat tcaaccttca gagaaaagtc tagatttctg gcctctgtgg
89460

gtccatggac ccaagtataa agtttataga gcatttactt tgtgccaaat actgttatga
89520

gtgatcaatc acttaccaac cctcttagca accttaagac attgaaacag gtggggcgca
89580

gtggctcacg cctgtaatcc taacactttg ggaggccaag acgggtgaat cacgaggtca
89640

ggtgttcgag accatcctga ccaacatggt gaaactctgt ctctactaaa aataccaaaa
89700

ttagccaggc gtggtggtgc acacctgtag tcccagctac tcgggaggct gaggcaggag
89760

aatagcttga acccagtagg cagaggttgc agtgagctga gatcgcacca ctgtgctcca
89820

gcctgggtga cagagtgaga tgccatctca aaacaaaagc aaaaacaaaa aacactgata
89880

ctatcattag tccattttac taagtaaact aagtcccaga aaggtcaaaa ggcagagctg
89940

ggattttaaa aagtggccat caggctctta acccccatgc tctggtggcc agaaatcttg
90000

ccctgatcaa tttctatggt cactgcttca ggcagccccc tcagctctca cccctgcctg
90060

tccctggtcc aagagagcca ggcatcgggg ggtagcctcc agggaagata tgtccaggct
90120

aaaaagcagg gaaggctaca gtgggtctgg gtcagtatca gagagggaca tgggctagga
90180

caggctcact ctcagtaccg tccacccata gctgagtttg gctctgccag gggctgggcc
90240

aagccctgca ggccaagcag gagacacccc ccagggaagg ccaggccctg aaaaacaagc
90300

ttcctccctc tgccagctca gcctgcccag ccccgctgga ggcctcagtg gcaaagccgc
90360

catcctccag ggccaaggct gggctttcag gacattcagg ccccagagcc tgaggccccc
90420

agccctgccc tgagctctag ccttttcttc tgaaaagaga cccctgttct ctccctgagc
90480

acatattttg accctttatt ttctgcctag gcaagaagca gggagaggag ccccagaatg
90540

cctgtaagga catctaagct gtccatttaa caactgggga aactgaggca cacagcagag
90600

aagtggctta ctaagattag tctgctggta aagtggtggg cctgggacta gaactcagat
90660

ctgagtcact ctcaagtcta gccatccacc gcattgcagg gcctctaaga ggagaggaga
90720

ggcttgggag aatgcagggt atgcttggaa cacccccaaa gaggagtgtg tcaaacagat
90780

gctcctgtga tgccagggtc cacactttcc tggtaacaag agagttagga cagagccctc
90840

aggactaaaa ccatttcctt agaaaaaggg aaaaccaaaa caaagcaaaa aataaaaaca
90900

caaacagtca aacccacaat gagcagtcat actcaccact ccctcacatg gaggaacaaa
90960

cttagatgga agatgtggag gtggcttggt gttgccatcc atccatcttt ccatccatcc
91020

atccatccat ccttccatcc atccatccat caatccatcc attcatcaaa catttgtttg
91080

cccacataat gtattccagg tgctatgctg ggtgattggg atttagcttt tcacaaacca
91140

gtctcaattc tcctggaaac agacaagaca agaggcaatt atcaatatgc acaaatgtag
91200

ccagctccat gttgttgggt caagaacagg agacatagat cccgaaagag ggcaccaaac
91260

ctaacctagg aaagagcatg gtcagggaag gcatctagga ggaggtgatg ttggagctat
91320

cttttaaacg atatgtagga gttagccagg taggcaagtg ggaacagcag gtgccaaggc
91380

ctggtggggc aagacagtgc agcctcttta aggaactgct gggaagccag tcaccctgaa
91440

ggaggcttgg ggaaagacga agtgagtcca acccatgtgg atcttctggg gcttccccaa
91500

gccctgatgt ttaggtttct gctctgacaa aggtcaggag gaggttatgg agatgctggg
91560

ggctgggcca agaacaggga gcagaggcgg ggaagctgga gaccaggcag attctttgga
91620

acttcctgga ataagacctc caagttcatg gcatgtccac ggctcagggc cagggtgctt
91680

gcttttctga acccacgttt cccatttgag gctgaacagg tggtggaatt ctaggtgctt
91740

agagttagaa aatgatgatg atgacgatga ccacttatcc ctggataagg gcctggcctg
91800

gatcaggttt cccacaaata ctggttctaa ttcttgccac actgtttgct gtggaggtca
91860

gggaggtcaa tgaagtttca cccaggatcc tacagctgct cagaagtaga gttgggattg
91920

gatccagggg tatctgatcc gagcttcagc caggtgtgaa aggggtcctc agtgggacgg
91980

gctgttgcag gtgggacccc aaagcaggct ctgagacagg atttgtgtga aagtggctta
92040

ttagggagtg accccaggaa acaccagagc caagcgggat gctgggtagc gaagggaagg
92100

aggccaagta caggaaacac cagagccagg cgggatgcta gataggaaag ggaaggaggc
92160

caagtacagg aatgccagca aggtgagtcc tcaggcaggg cagaggcgga agagagctgg
92220

agtctctatt gcccctgccc tggcagtcgt tgggtagtgg ctgtaccccc aagggaatga
92280

gaggaggcat cagttttcgg gcacttccag ctccctctat gtccaggcaa aaggacttct
92340

ggcagctggg gcagcccacg acagagacac aggtgcaggc tgctggggat aagtgcatat
92400

caggagctgc tgtgctggag atcagaaagg gattcctagg atctacaggt gcacagagga
92460

gctgagcaac catcccagga cctggccagt ttctgtggtc acctcgctgt ctgcaaaatg
92520

cttccgtact gttccgcctc cacacttttg cacatgctgt gctcactcat tcaacaaata
92580

gtcctcaaga ccctactatg cacccactat gagctaggct caggagcagg gcggtgaaca
92640

gaacagcctg gcttggtctt tccccttatg gagcttagag gctggctggg agacagacac
92700

caggtcatca ctgcaaagag atgtgagctc tgaaaggaaa gagggaggtt ctctgagaac
92760

agaaaaactt gatgtaaact gagggcatca aggagagctt ccctgaggaa gtgacgtctc
92820

agctgagatc tagtggggga agagacacaa cacagacagt gggaacagcc agccacccag
92880

agcttccctg atgagggtct ctcagcccca ggagctcctg ggggaggcca gtcctgttca
92940

ggagacttgg acaagctgaa tcccttcacc gagcctcagt ttcctcatct atgcaagcac
93000

gcagaagcag cttgggcttc cagctctgac cttggctcaa aatatccact gagaagtggg
93060

cacagcagct gttggactct cagtgacctc tggccctgtg accccagagc cttcctccct
93120

gtgccctctg tggtcagact gtgacctctg gggcttatca gcctggcccc tagggtaccc
93180

cacgtatgat gcctgagttg caaaacaact gcccagttcc ccagaaggct gcttgagaaa
93240

tgtcagccct caggggaagc tgacgtggcg tcagtgcttt cagagctatt gcagaaatgg
93300

ctccatttct ggagaaagcc cagcctgcgt gatagcaaga ctggctgcta gactcagagc
93360

tggcagcggc ttggtgtgca tccctgggca gcctgtaacc tcacagtatc acagcttatc
93420

ctttgggggc ctgcaaacct tccatgcgtg ttcattcatt caacaaatat ttactgagta
93480

cctgctatgt gccaggcaat gttctaaaca ctaggggaga cagcagtaaa caaaaaacaa
93540

gcaaaagaac aaaacccaaa aattaaaggc ctgcccttgt ggagcttaca ttctgctgag
93600

ggagtccaac aacagacaaa taaacaaaca tgtgtataca taatattggc cggtgataag
93660

tactttgcag gaaaatacag agggacagaa ctgctaaata agacagtgag gtcaaggaag
93720

tcctctttca agaggtaacg tttgagtaaa gacctaaagg aagtgagaga ggaaactatg
93780

tggatatctg gggaatgagt acaccaaggg tatacaaagc caagggaaga gtaagtgcaa
93840

agatcctggg gcaggaatgc acttgatgtg taaggaatgg caagaaggcc ggtgtggctg
93900

gagtccagtg atggagatgg aggatgcatt ggacactggg gttacaaacc tgagtgcatt
93960

ttgtcaatgt gtttgcatat gtgcgtgtgt gtgtgagaga gagagaggaa gagagacgga
94020

cagagagaca gagacagaga gaaaggagac acagagagac ctggagacac atagcttcct
94080

ttctttcttc caccaccccc tccccatctt ggcatcatca tgattaagag aggggaaaac
94140

acagatgatt aaatgattta aatgtgcaag taaaataaca cctcagtgag cacattatgc
94200

aaatctattt aattaacatc ctaaatgagg tgaagccacc aaggcggatg agctaatttc
94260

cctgggggct ccagctccat tcttgccctc cctgaatgcc agaaacttct ctgggtggaa
94320

ggaaaggggg tgggcagggg ctcacaagag ggcctgctgc atgctggcac ttggcacaca
94380

ggggaggctt taccctaatt tactctcaat tagcagcaac agttactgcc ctgtttcata
94440

gaggagaaaa ctgaggcttg gagagggcgc agagattaga acccagacct ccttagagct
94500

ctttgctaaa aaaattttca acttgtctct tggagaggtt tgagccttta gaaaggactg
94560

ccgcttcctt acatctttaa ggcaggaatt atctgcaaac cttttctgta aagggacaga
94620

taataggctt tgtgggccag aaccaactgt gtctttgtag ttcaaaagca gccacagata
94680

atctatcaat gaataggcat ggctgtgttg cagtaaaact ttatttacaa aaacaggtcc
94740

cgaggccaat ctacaggctg cagtttgctg attgctctaa gggatctgac tgaatcagag
94800

cttcccctgt gcaaagcagt taaccacgga aggccgtggg actctaggca agtccctcat
94860

ccctctgatc ctgtttcttc acctggacaa tgaaggaatg ttctgggtga ggtccagtga
94920

tgagaggggg gtgaagccgc ccataggaag acagaagctg ccctgaactt tgggtggggc
94980

agaccctggc aagacactgt ttggctacta tcactgcatc acaacttcgt ggtgtaaaac
95040

tcgcagggtc ctgtgggtca ggatagcagg catagcataa ggagagacag tttgtcctga
95100

tacgtgatgt cagctgggaa cacttgaagg gtgactcaat ggctggaggg tggaatcatc
95160

tggaagcatc tgcacttgca ggtctgagga ttgacactgg ctgtcagctg ggagttcagc
95220

tgggctgttg ccagagcacc tctacgtggc ccctctgagg ggtctctctg catagactgt
95280

ttgggcttcc ccacagcatg gtggctgggc tccaagagag ttaggtggaa atgcattttt
95340

atgacctagc tatggaagcc acatgttgtc acttccatca cactctattg tcaagacagt
95400

cacaaagggg aaggaacata gacccccact gctcaataga aggagagtca aggtcacagc
95460

gtaagaaaac catatgggat gggtgatgtt gtggccaact atggccaata caatctaccc
95520

catgctcatg gtgagaggcc cccatgtgga gtctgggcct agaaccttgc ttcactgatt
95580

caaggcagca tcagccctgt tactgggagc ccaagctgtt cagacccctc cctccagacc
95640

catccaagcc ttttctggtc aaaaccagcc catgcttaat cctgggtctc cttccaaagt
95700

aaccctgagc cattgctgca gtaggactgt attctcagtc agagttaagc cttttttctt
95760

acaaacaatg aaggagggcc gggtgcacgg tggttcatgc ctgtaatccc agcactttgg
95820

gaggccaagg tgggcggatc acgaggtcag gagttcgaga ccagcctggc caacatggtg
95880

aaaccccctc tctactaaaa ataaaaagat tagccaggca tggtggcagg tgcccgtaat
95940

cccagctatt cgggaggctg aggcaggaga attgcttgaa cttgggaggc ggaggttgca
96000

gtaagccaag actgtgccac tacactccag cctgagcaac acagtgagac tttgtctcaa
96060

aaaaaaaaaa aatgaaggaa acataagcaa acagctccca cccacacgtc agccctggtg
96120

accccagcat ttacagtaga gttggccaag cagatggaga aatgttgaga caaaactagc
96180

tgggaaatgc ctcccaagcc acaggatgtg attatgtgtg gtgggctgga gggtggagct
96240

ggggtcctgg catggacccc cagagcagag gccttcacag agcagctggg tggaatgggg
96300

tccccgccag aggctggggt gcaccagggt cacgcggctg ccgcagatga ttgcagagtg
96360

gaaggaatca ctgaggtcag agttgccgag gctctgcggg gagatctgaa agcctgagca
96420

gcccccaatc aaattaacaa tggctccctg acctggctca ttcctgaatt ccacctcctc
96480

caccatctga tcctctcccc agcaatgaga gtgagttgga ggaggagccc tggactccag
96540

acagggaggg actcgctggg gtctcacaat aagccagagg ctgagctgag actagaaccc
96600

agggccctgc ctcttccact gaggcagtcc agcagaggcc tctggggctt cagaatccct
96660

tgtatgtaca tgggtctagg ccggacacag tggctcatgc ctgtaatcct agcactttgg
96720

gaggctaagg cgggtggatc acctgaggtc agcagttcga gaccagcctg gccaacatgg
96780

tgaaacacag tctctactga aaaaaaagaa aattagccag gtgtggtggc acattcctat
96840

aatcccagct acttgggagg ctgaggcagg agaattactt gaacccagga ggcggaggtt
96900

gcagtgagcc gagatagcgc cattgcactc cagcctggac aacagagtga gactctgtct
96960

caaacaaaac aaaacaaagc aaaacaaaac aaaacaaaaa gggtctaata ttaataatca
97020

tgccacctcc cactcatggc actcacatgc cagtgcttca cctcacagaa ccccccaaca
97080

gtgctagagg gtaggactcc tctgtcaccc ccatttccag aggaggatat gtgacttaga
97140

gaatgaatgg tggagctggg acaagacccc aagcctggca cttgcactca ggggcacgtt
97200

ttgtgggtgg gaagcggccc tccctgaaag tgctggcatg ctagcatacc cccagccctg
97260

ttcaagtgct tggaagcttc ctgacccagg ccactggcaa ccagaactcc cagggcccct
97320

ggctgaatcc ggctatgttg tcaggagttc ctggcaagtg tggaagcagg tgggccccac
97380

agacttccca accccagcta tcctagcagc tgcagccagg ataattgtcc ccattttttt
97440

tggggggggg gggtggggaa acagagtctc cctctgtttg cccaggctgg aatgcagtgg
97500

tgtgatcttg gctcactgca acctccacct gtgcatcaac ctccagggta gctgggatta
97560

caggcgtgcg ccaccacgcc tggctacttt tttgtatttt tagtagaggc gaggtttcac
97620

catgttggcc aggctggtct cgaactcctg gcctcaagtg attcacccgc cttggcctcc
97680

caaagtgctg agattacagg catgagccac tgtgcctggt caattgtccc cattttaaca
97740

gataggcaaa tcgaagccca gagaggaaac caggccaccc tttccagagg ggttgaggcg
97800

aggagcagaa gctgcagtta gaccctagct ccttccccag ggtgagctgg atgcgtggga
97860

accatggagg agcaggcacg agtgagcggc ttcctccctg cagatctcag cagatcagac
97920

agtcgccatg actcatcccc acatcccatc ggccctgagg ctgccctggc ctgggacttc
97980

ctagctccag tcttgttgct caggggtgtg gaggacacca gatctaccct taccctccac
98040

ccatgcctgg ggagacattg ttcttaatga aacacaccag atgggtgggg tgaccatgtc
98100

atttattggc caaaccagaa caatttggag agctattaat aatgatgtgg tgattgttag
98160

cattaaccag gactggccaa gacaagtagg gatgtctggt ctctctattg acaactgcaa
98220

tagtcattgc ctaactccaa gtactagacg caccaatgtc agtcactcag gcatcagcca
98280

caggttaggg ccaaagtgtt ccggggcagg tatatttttg agaattcctc aagtcagggg
98340

gcactcctag gggaattcaa gtctacgtgt aggacatact ttgaaatgag actgctgagt
98400

ttgcatcttg gtcctaccat gtaagggctg tatgagctca gacccgttac ctattccctc
98460

tgaccctgag tttcctgatc tatagaacca attgaccccc gtagaactgt tgcgggaaca
98520

aaaagagata actcagggta aatgacccac aatgctggca cagcatatat gctcagtaaa
98580

taagaggtcg ttattacttt gtattaatgt gattattaac tatgctgaaa aaggcagacc
98640

tcacactttc agatatggcc ttgccctggg gttactcaca gtgttgcggg gaatgaagtc
98700

caggctaagg gactgagtgc tgggcatgaa ggacacaccg agggttgtgg ggttcggaga
98760

tgtaggagag gcttgggtag gggcatcagg gtgggcttcc tggaggtggc ccctgggcag
98820

ggtcataatc atgtcaagat taaggtccta agaaacacct gtttctaaag agcttatcgt
98880

gctccctttt ctcagtataa ctacataaga gctcactgaa gttcagcttt ttatcttcat
98940

gacttcaatg gtctctttgg gatgtcaaat atcaagttct ttcccccaaa aagttcattg
99000

agctcacatt aaatctgctt attgctccac ccaggccagg aacagaagcc ctagagcttg
99060

ctcaagcatg aatggaggat ggaagtggag gcttaggggc aggggggtga ccaggggaac
99120

cctagcgagc agggcagcct gacctggggg tggagggaag ggtctgagcc cgagccaggg
99180

ccgttgctgc ctctgggcct cttgagtctg gctctgagcc tgtcagcttt ggtttttcct
99240

cagtggggac cacctttctg ttttctacgg acatagctgg acacagtggt cctccgcaca
99300

tgctccctcc agaacccacc tagcatctct gattcccaaa gccaaatcct gggaaaaaag
99360

aatcttattg gttaccttga gtcacgtgtc tatcgcctca accagtcagc ctacaaccag
99420

tggccggtgg cacggtagaa gcagaactga tgggtggagg ttgttggaca tttactatgg
99480

ataggtaata ctttgacatg tagaaaaggg agaagccttt tccaggagga gggtacagca
99540

tgggcaaagg ctttggcggc agagaatcgg gtgcttagtg tggcgaacac cgagtggcta
99600

agttcctatt agccttcctg tggctgctag aagggcagct cccttagcca caaccctgcc
99660

cctgtgcctg gagccacaga cacggcccag agagggcagt ccggtgaatc agcagcagaa
99720

cccaagctca aactcctacc tcctttttcc cagtccatgc ttgagtgtct aaggcatagg
99780

agggagtggg gggagaggaa cagggagaat gaaatcaaga aataacaggc ttcacaggag
99840

cggtaatgac attttaactt aaaaattgca aggagatggg ggctgcggag gtgtatgcaa
99900

atttcatgta gatgagatgc aaatgagcct gcctgctcct gcctggcgca cctggatgtg
99960

gctcagtgac aaatcctaat gggctctgcg tggctgtgtg attgattagc aaggccggct
100020

gggatgacaa ctgcactcct ctcctgggag ctgggggaga ggtgagactc tgtttctagg
100080

cagacctgaa ccccctctga taggggaccc tgaggggtgg ctctactagt tcaaggatag
100140

caaatttagg gcacatctac tacttatatc cctgccagca cccatggcag acatcattaa
100200

tcaaacatag aattctttct tactgagcca accaggtgct gtaggcagcc cctactaatc
100260

gaaaagattt ggcattcatc ctttcattca ttcatctgac aaactttgtt aagtttcttc
100320

cctgtgccaa acatacattg aacaggtaga ctcaatttct gctctcatga aactcacagt
100380

ttagtagggg atataagcat taaataaaat attgcaaaat ggcaatcgag atacatgcta
100440

tgaaggagaa gtacctggtt tttaacaaaa gaggattttg accacttcag agtggtcagg
100500

aaaggctgaa cctcttgctg gtatctgaag gataaggagg gcttagctag ttgacaaggc
100560

tgaagcctta agatgagaaa gagcttgtag cagacactgt cagtgctaca cccagatacc
100620

ctatgatcac ttcttgttgc tgtgcttccc tcctctggct tctctgtgtg tgtatgtctg
100680

tgtattttaa acaacttatt ttgagatata gccaatccac catgcaattc actcatttaa
100740

agtgtacgat tcaatggttt ttagtatatt cacagatatg tataaccatc accacagtca
100800

attttggaac atttttgtca tctcaaaaag aaatcctgta tcctcgaggg atcactgcca
100860

tatcctccca cctcccctat ccccagtcct aagcaaccac tgacctactt tctccatata
100920

tagatttacc tattctggac atttctataa atggagacat ataatatgta gtctcttatg
100980

actaacttct ttcatttagc ataatgttta caaagtcatc tatattatta caggtgtcag
101040

tacttctttc ctttaaacag tttttattgt ggtaaaatat atataacact taccattttg
101100

tttttaagtg tgcaattcag tgacattaat tacatttgca atgtacaccc attaccactg
101160

tctcttttca aaactttttc atcactccaa acagaaactc tctacccatt aggcaataac
101220

tccccattcc cacctcctct cagccactgg caacctctta actactttaa tgtctctatg
101280

aatttgccta ttctagttat ttcatataat ggactcatac atattggtca ttttgtgtct
101340

ggcatatttc acttagcata atcttttcaa ggtgcatcca tgtcgtgtgt atcagaactc
101400

attccttttt atgtggcaag ataatgttcc attgtatgtg tgtgcgtgtg tgtatatata
101460

tatataacaa aatgtgatta tataaaacaa atggaattat atgtgtatat tttgtttatt
101520

cattcatctg ttaatggaca cttgggttat ttccatcttt gagctattgt aaataatgcc
101580

gcaataaaca ttggcataca agaatctctt tgagtttctg ttttcaaatc ttttgggttt
101640

gtaactagga atggaactgc taggtcattc tatgtttaac tttttgagga acttccaaac
101700

tgttttacac agcagctgca cggttttaca ttcccactaa ccatgtataa gggttctaat
101760

ttcttcatgt ccttgccaac atttgttatt tgctgcattt aaaacatcat agccatccta
101820

gtagctatga gatggtatct cattgtgatt ttgatttgca tttccctatt gactggtgat
101880

gtatcttttc atgtgcttgt tggccatctg tacatcttct ttggagaaat aactattcaa
101940

gttctttacc tagttggtaa ttgggttgta tttttttgtt gttgtagtat aaattcttta
102000

catattctgt atattaaact catcaaatat acaatttgca aatattttct cccattctat
102060

atgttgcctt ttcactttct tgataatttc ctttgatgca ctaaagattt taattttgat
102120

gaagtctaat ttatccattt ttatttacgt tgattgtatt tttggtgtca tatctgagaa
102180

gtatctattg gcaaatccaa gctcattaag atttacccct actttttttc tatgattttt
102240

gttgttgttg ttgttgtttt gagatggagt ctcgctctgt tgcccaggct ggagtgcagt
102300

ggcgggatct cggctcactg caagctccgc ctcccaggtt cattccattc tcttgcctca
102360

gcctcccgag tagctgggac tacaggcacc cgccaccacg gccatctatt ttttttgttt
102420

tgttttgttt tgtattttta gtagagattg ggtttcgccg tgttagccag gatgatctcg
102480

atctcctgac ctcgtgatcc acctgccttg gcctcccaaa gtgctgggat tacaggcgtg
102540

agccaatgca cctggctttt tctgtgagtt ttatagtttt agctcttata tttaggttgt
102600

tgatctattt ttagtaaatt ttttaatatg gtgtgaagca ggggtccaac tttattcttt
102660

tatgtttgga aatccaattg tctcagcata atttgttgaa gagactattc tttctccatt
102720

gaatggactt gggacccttg tcaaaaatca ttggccatag atgcatggga ttattttggg
102780

attcaaaatt ctattccttt gtccatacat ttatctttat ggaagtacca cactgttttg
102840

attactatac ctttgcagta agttttgaaa ttgaaaactc tgggtcctcc aactttggcc
102900

ttctttccaa gatgttttgg ccatttgggg cctattgcaa tctgttttaa atttgagaag
102960

tggcttttta tttctggaca aaaaagcagt tggaattttg ataggattac attgaaccta
103020

tatattgctt tgggtggtat tgacgtctta acaatcctat ccatgaatat agtgtgtctt
103080

tccatttatt taggtcttct ttaatttctt tcagtaatgt tttgtaggtt tccgtgtata
103140

aatctttcat ctccttggtt aaatttagta ctgggtattt tattcttcta gtgctgttgt
103200

aaatgaaatt gctttcttaa tttccttttt gaattgtttg ttgctggtat ataaaaacac
103260

agctgatttt tgtgtgttga tcttctaact tgaaatttcg ctgaatttat tagctgtagt
103320

agctttcgtg tggattcttt gggatttttg agatataaaa taatgagatc tgatctttta
103380

aatatataaa tagttttact tctgcttttc cagttggatg ccttttcccc ctttttcttg
103440

tctaactgcg ctggctagaa cttacagtaa aatactgaat agcagtgctg caagtgggca
103500

ttcttgtctt tctcctgacc tcagagagaa agcttttagt ctttcaccat tgaatttgat
103560

gttggttgtg ggtttttcat aaatgccttt tatcatgttg agtatgttca cttttattcc
103620

tatttttctg agtgttttta tcatgaaaag ctattgggtt ttgtcaaatg ccttttctgc
103680

attgattaag atggtcgtgt ggtttaattt cctttattcc attaatttgg catattacac
103740

tgattgattt tcttatgttt agctatcttt gcacttcgag aataaatccc acttggtcat
103800

gctgtataat ccttttaaga tgctgtcatg tttggtttgc tagcattttg ttgaggattt
103860

ttgcatttat attcatgagg tatattcata agtttagtaa ttttctttgt gatgtccaca
103920

tttggctttg atatcaggat aagagtggct tcgtaaaatg ttaggaagtg acttcattcc
103980

tttttaatgg ctaaataata tttcattatg tgtatatttt taaaatccgt ttttaaaaaa
104040

tccattcaac agctaatcaa catttgggtt gcttctactt tttgactatc agaaataatt
104100

ctcaataagc attcatgtat aaatttttgt gtggatatgt tttcatttct cttgggtatt
104160

gacctagtaa tggaattgtt tggtcatatg ataacttgat gtttcacagt ttgaggaacc
104220

ggcaaactgc ttgccaaagt agttgcacca ttttacattg ccaccagcag tgtatgagag
104280

ttctgatttc tccacatctt caccaacact tgttatacgt ctttttatta tagccatctt
104340

tgtggatgtg aaacaatatc tccttgtggt tttgatttgc atttccctga tggccagtaa
104400

tgttgaatat cttgtcatgt gcttattggc catctgtaca tcttctttgg agaaaagcct
104460

attcaggtcc tttgtccagt ttaaaattta ctattattaa tagtagtatt tagctgtgag
104520

agctttttac atattattga tacagaatat tatcttttat gggcccacac ttgcaactct
104580

cttccaaggt ctgccctgga gctaccagac tctgctttgt gccacatgat tctgcagaaa
104640

gcctgaagtg ccttggaggt gtaaaatgtc ctccagagtt cccccacgca gtcaggcaga
104700

ggcagggact tggtctgaaa ttgcaccttg gcttccctcc cttccctgtt ctgcttgcct
104760

ttctggtttc tcttgtgatc acctcttcaa tgaatcacct gtacctgaat cctcacccct
104820

aggtccactt ctggggaacc tgaccccaga aaaagcttgg aatggctgag gaaatgaaaa
104880

caagttagtg cttctggaac tcagaagaca tggagattga gatgaggctg aagtggaggg
104940

cagaggctga caacacaggc tttgtagatc acagaaaggt tggattttct atctggagag
105000

ctatgaggtg ctactgttca gggttaagcg gggctgacat tatattacat tttgaaatac
105060

ccctctagtt tctgggtgga gaacagatga aggggatcca agtgactgca ggaagaccac
105120

tgcggaccct gttatagttg tctggggcta gaggatggag gctgggatga gggtgaggac
105180

gatggaggga gagagaagta gaagataaca gagagatcag gagagtggat ttaggggtta
105240

aggggaggtt tgaggacaac ttgcaagttt ccagcctggg tacctggatg gatggtcagt
105300

gcacagagag ggaagcagga ctggaagggc aggtcagaga ttccacatgc aaaacaccac
105360

cagttttcct tcctggtctg acctaatccc tccatttcac agatggggaa aggaggtcct
105420

aaggaagtac ctgactaccc agagttcccc agcatgcccc aggcagaggg caatgggacc
105480

cccagagtcc tgccgcctat ccagagcact taccctacag gggactctct ggctctggcc
105540

tcagagagct attttcagct ggggtttgag acgaggcaaa tttgtcctgg gctggaggcc
105600

aattcacagc ccctggagaa caggacatct ctgctttatg aggtcagact gtattctgac
105660

cccagccagc caccctgtct gggtctaaag tgggtgtgac agccaccctg tctgtgtcac
105720

aagggtgtga ggggcctacc tcaaagtggc tgacaaaaca cacgctcatc cacaatcctg
105780

ggtttggcat tgatccagaa ccaggcccag accctgtgct ttccccttct tggccccttt
105840

cctggctgtg tgaccttggg caagtcattg cccctctcca ggctttagtc ttcccatctg
105900

taaaatgggg ccatctcaga gtatgacttc tgtgacccaa agggttgtag taaatatcag
105960

atgggaaata gatggacaaa aagtgctttg cgaactgtaa agtgcctaga cttctattgg
106020

ggctcagtcc tatgtctgcc ccacatcact gcccagcagt gaaaagtctt ttcaaagctt
106080

tgttccttca caagatggct actcctctgg aaaggaagaa gtcttggtgt ggaacacaag
106140

gatccagaga tcttattttc ctgacccagc aggcttggca aatccagtgg acagatggaa
106200

aaactgagga gcacctatct actcagaggc actaggctgt taagcctgga ttcaaaagca
106260

gggctctcca agatgagaaa gatcctcatc catccaacgc ctccattgga aagatggggt
106320

aaactgagca ccagagagag gaagcaacct gcctcgggtg accaggccat cagcaacagg
106380

gcagagtggc acctgaagtg cctgacttcc aggctgtgct tctctttctg tctcttgcct
106440

accagcccct tgagcttgaa aaaagtgttc ccatcacagt gggatgttga cagtcacatc
106500

actggctgac ttggggttcc ttgaaagccc atcttcttcc accacagcag gcttcaaact
106560

ttgtttaaaa atcccatgga ggggcactgt ggcctctgcc tcctcagagc agtctctcag
106620

gggctgaggc tttgctgatt caaagctatg tcatcactcg cagggaatct ctgggggagg
106680

ccatgccatg ccatgcccct actgtgggca tatttggcca aagtcaataa actaggccca
106740

gatcaagtcc agctctgccc ctcactgtgg cactgggcaa ggtacctcca aatctctctg
106800

agtttcagct ttgtcctcag taaaataggg tggaatcagt cctcataatg ttgtaaaaaa
106860

aattcaacaa gccctgtaaa gcttttagag gatttagctc aaagctaggc acaaagaaaa
106920

tgcaaaagaa atgttagctg ttattattcg atatttttat acattgttac tcattatgac
106980

atttcatttc tgcattccct tcttccccag gggatgcagt gaggggagag attgttgggg
107040

tacagttctt tctggggaca gagggatgca caaggtaacc ttggggactc ctcccgccca
107100

agtattctta gattacctgt gcccgctttg ccccccttcc ttctagcagt agctgtggtc
107160

tctcacaagt cagtttccat cccagcaccc cagtttctgt ggctccctgc ctgtggtacc
107220

cactggctgg atgctgttgc tatggcaaca gcattttaaa agccgtcggc cctctctgag
107280

gcaacaggca tggatggaga tgggctgatc aacgcctctt gcatctttgg gcctgtctgg
107340

atggaaggca gggaggggaa tgcaagaatc aaacctggct ttgggtggag gactcaggcc
107400

ccagccccgg gtgttcccca aatgcacccc cactttgctg agtcacacag gaagagcact
107460

tggctacaaa cgcagattcc agccacctgc cagcaccaag agtctatttt aatgtctggg
107520

gtaggattcc ctaggaagcc ttttgaccgg ctctgctcat ggctctgagg caggcaggcc
107580

ctagaccaca cctggggaag tcttcagtct tggaagattc ctgaagccat tttaggtatc
107640

aagtgtgtga ctcttggctg agacagctcc tgagaagccc tagttgtcct cctgtgcctg
107700

gcccaggcct gcctctctcc ccttcacagc ctcattgtgg gggacctgtg ccccacagcc
107760

tctttctgga gccaggaaaa ttcagagcta ggtgaccaag aggccacttc agtaatgcat
107820

cctactgcag aggtaaggca aacactttca gcttaatgac actccagaga ggcgctggca
107880

ggggccactc tggccagtat gaagggtggg gcttctggat gctccatcta agatccgaca
107940

gataggcccc ttcccacggt ccccaaccac caggacccag ctgcccacag aaccccaatg
108000

gtactgggcc acagaggctg ggcatagaga agtggaagtt gggcagggaa ggggacagaa
108060

gacattgagt tcagggaggc ttggtgggga ggtgagagag aggagggacc tctaaggcgg
108120

agaggattct ctctggagga caaggacaag gttccacagg gttccttgaa cttgagaatg
108180

aaggtcccat gcctgctgaa gaggccttgt gagacaggga gcacagaaga catgtccttc
108240

ctttctaagc acaggaaccg gcctctggct ccaggaggca attctctttg ccaagaccaa
108300

tggagctggc tgtgcgtgct gtctacgagt gccagctcct gagggttggc agataaatgc
108360

tctggatgcc tcacgcctca gggggtgttc aaggattcca tgctgtcttt cagagatctc
108420

cagaaagatc cagctacagt tgccaacatg gaaatttgta tgagggtggg gcacagtggc
108480

tcatacgtat aatcccagca ctttgggagg ctgagtccag aggatcactt gagcttgaga
108540

tcagcctgcc tgggcaacat agcaagaccc catgtctaca aaaaataaat aaataataag
108600

ctgagcatgg tggtgcataa ctgtagtccc agctacttag gaggctgagg tgggaggatt
108660

ggttgaaccc aggaatttaa ggctatgatt gcaccactgc actccagcct ggacaccaga
108720

gtgagaccct gtctctggtg gggtcggggg gggtgggcgg aaaaggcaat cccagcactt
108780

tggcaggcca aggcaagagg atcacttgag cccaggagtt tgagaccagc ctgggcaatg
108840

tagtgaggcc ccatctctaa acataaaata caatgaagtt ttaaaaatta aaaaaaagaa
108900

aatttgcttg acaacatact ctttattccc tttgcagatt cgcctcctac tcccccagca
108960

gaggttcctg gatcagcacc aaacacactg atccttaaat cctcatctca gggtccattt
109020

tcctggaatc caagacaagg aagcactccc gaatctcccc gaggattctg cctaagggca
109080

acactggccc tcgtacccga ggggagagct gtgtatgttc ctgagctgca ggtgggtgtc
109140

tgcctgcctg cgagcgagac ctctggaggg gtctgtgccg acaggtcttt ccacaatgag
109200

agcctggagg gatggagcca atcacaccat ttattgtgcc ctggttctga cgcccactct
109260

tgctattctg catgggtgaa gatcgggtct tccaggagtt ttctggagcc aggggcaggt
109320

cgacagttct catcctccca gcagaggcag aatgtaaaac cagtgctcca tggtgacgtc
109380

tcactgggac tgaccagagc tcccagctac ccctccagct gggccctggc ccagtgctcc
109440

tggaggctgg tgcgactgaa ggggcctctc agctagaggg aggcaagggg acttgtctgg
109500

ggtgagtcct caagggatgt ctgtgagtct gtcccctctg aggagaagct gcccccagtg
109560

aggcgaggga cctggctctg cctggggggc tgctgccgga agaaggtggt ggcaagcaaa
109620

tgctcgcggg cagggtcagg cagcttgtgg gagcctttgc cgtcttcagc ctctgcagcc
109680

tggccagctt cctggggtgg agagcctgcc tggaaagttc tgttctgttc ccctctgggg
109740

ggcccaggag gccaatagga ctttggcaaa tggtccatgc ccagtgagtc tagtggaggc
109800

tgatcccacc tccccttgga agtgacctct acactcacac cctggccctt ggctgccacc
109860

cctcttcgtt ctgggcgcgc tgagtgagca gctggagcct ctgggcaaga gctgtgggtc
109920

agtgccttct ccaggagcat ccggatctcc tcatgcctga tccgggcctc ctcccatagc
109980

tcctggccca ggcctgcccg gctcagggag gccacctgca gccccacggc tgcgagcttc
110040

tcagcaggga actcagatgc cagtcgctgc aggtagcggt ggaggcggcg ctggtcccca
110100

ggactgaccc ttgaggtctt gtccagcctg agctgggcca tcaggtcctg acagtccatg
110160

tggaaccagg tcatctgggg gacaagaaga gagggaggtg ggggctcagg ctggaagtgg
110220

gaccatggag gactgaatta ataagtgccc aatgccaggg ccccgctcag cttggccatg
110280

atcttctcca ctgaccatga tgaccctggt ttccctctct tccctggggt gcacacacct
110340

gccatctgga aacagccccc aagcaccctt agaatcatca gacacctttt tttttttttt
110400

gagatggagt ctcgctccat cacccaggct agagtgcagt ggtgtgatct cggctcactg
110460

caagctctgc ttcccaggtt caaacaattc tcctgtctca gcctcccgag tagctgaggc
110520

tataggcacc tgctgccatg cccagctaat ttttgtattt ttagtaggac ggggtttcac
110580

cttgttggtc aggctggtct cgaactcctg acctcaggtg atccacccgc cgcagcctcc
110640

caaagtgctg ggattatagg catgacccac tgcgcccagc ccatcagaca ccttctaata
110700

gacctacggg ccagcaggga cttagccctc tctggggctc agtttcttct tctgtaaaat
110760

ggaagttgca ctagatgatc atgaaaagcc ttttgagagg tgaaattcct ctccatggtt
110820

gtattaaccc ttgagagtgc tgtgagggag gagtctgcct aatgcttatt gagcactgac
110880

cgtttgccag gttctgtgtg tctcaggcag gattgcactg aactgcacca gcagcccatt
110940

tcacagagaa gaaaactgag gctcagagaa gtgaactcac ttgccctagg tcacatagca
111000

agtcagggag gagctgggat tccacctctg atctgtcctc caagcacctt catgggcaga
111060

aggagaaacc ggccaggcag gggtgtgccc tcaccatcac accttgagac tgtcagccac
111120

accaggcccg caggccccct ccctccagcc gagcctctgc ctcattttgt gattctgtct
111180

gtccccgctt cccccacaaa acgtctgcag actgcaaaga agccaacacg ttcccagccg
111240

ctcgtgaaaa acataatctt tctctccatt aatttagaac ccgatagcga gctgttaggc
111300

tgtagggatt ggagaaccat tttctccgaa gagaccatct ttccacggat taagggaact
111360

tgactttaga taaatggctt catattcgtg caagcacacg gagctcatgg ccgggaaaga
111420

gctccataaa tctccgttag tggcaccatt gtgttattat tatttaggac caaagctgct
111480

gcacacacgc tccccaccag gggctgagcc tggtgctggg ctgtcccatc atctgtctgt
111540

gtgtccagga cttgaccttc caggctaagc tggacagaga tggatagcac ctcagaaatc
111600

atcttgccca accccttatt gaacggattg aaattcaggg ccagtgagga aacagggcca
111660

gcccagttcc aacagcgaga gttggactca gagtcagggt tgctacctcc cccgtatgac
111720

ttctaggcct ggcacagacg ccacctccac acaggccatc cccaggcttc cagcctatcc
111780

tgactccccg cacagcctcc ctgagacctt gtgggccgag cccgtgccag gcaccatgga
111840

tagagccagg accaactcga acccagtccc tgtctcaggg agctcgcctc gcaggcacca
111900

tgcaagagac aagcgagtga caaggaccag gacaacttca ggtcaaagtg agcacccaga
111960

agcgacaaaa cagggtgacg agagagaacc agagtgggca gtcagaaagg gtgtctgcaa
112020

agaggggatg tgtgagctga gagcggaaga aagacaagaa gccagccggt caacgggaag
112080

agcttcccag gctgagggaa cagcacgtgc aaagacctca gagttggagc acgtttgatc
112140

gatccgggaa ggccagtggg gctgaaattg gctgaaatca gtgggcaagg caaagaagct
112200

accagatgtc tttaaccctg gaggcactac tcctcttctt cctcctctta atgataataa
112260

taatattgat gttaataaca attctgaatt tgaacaaagc cctgtgccag gggttacttc
112320

cattgaaatc tgtcattagc tggtaaggct ggcagtagag ccagatggtc tgggtttgaa
112380

tctctgccca ctcagttagc tgtgtgaccc tgggtgagtt atctgacctc tctgtttcct
112440

taggtggaaa tgggcataat agtagtacct gtctcatagg atcggggcga caataaaatg
112500

atacgtgaaa tgactagaac agggcctggc ttgtcataag aactacacgg ccattaacta
112560

tggtcttagc aaagagctga ggcctagagc agtgaagact gtggttgagg tcacagagct
112620

cacgcaagga catgaagccc cagactccaa ggctcggatt ccgcctgtat cgtgctctta
112680

actgtacaca gctggaactg ggggagccct ttgctgccac tgccaacatc tgcctccctt
112740

gcccactgtc ccaaggagac agagagtgcc ctgtcctccc agggccccag gcgtgagttg
112800

accagagtcc ctcctgctgc cctctgaagc agcctgtgat cgctgtggcc tgcacgtggg
112860

gctggcctga cagctgggag gctggggtca ggtctttctc atgctttgtc cttggtgaca
112920

agtgcaaggg gcagccaagg gcactgggtc tgggccagcc atgggggtcc ctgtcccagc
112980

tggaccacac tctcccaggc tgctcctttg tccccacaga cctcagtttt cccataattt
113040

ttttttaaag agagatttgg ctacatgaat ttaggacagg tggcagctcc taaatcaaat
113100

catccttcac cactgccctc tttgggcagt tttctcatct gtaaaatggg aacaacattg
113160

gttccctgtc atcaaagagg tatggataag aatgaagtca agtaaggtgt gtgaagtgct
113220

cacaggaggt ctggcatggt gtacacactc agaaatgtgc actcccctcc caacaacccc
113280

tcatcctcac catggtcaag cctgcttggt gaagggatct gtcgtcattt ctcttctctt
113340

tggggaagca tataaggcat gtcacggggt accacagaca catctatgtt caaatctcag
113400

ctcttcccct tcctctgttt ttttcctgtt acaagacttg gggagtgaat cacgatgagc
113460

ctccgactgt gtctgggtat ccctgtccct gctgtgcccc cagccagggg atgggggtgg
113520

tgtctgtgca agtgggtggg ggggggctgt gtctaggtat ccaagggatg ggggcggtgt
113580

ctatgcagtc aggccatggg gctgtgtcta ggtatccctg tccccaccat agccccagcc
113640

aggggatcgg ggggtatctg tgcaggtggg ccgcggggct caccctcttg cagaagcggt
113700

gcaggtggag cagcgtctcg aggtccgtgc gttgccgctc ggccgccatg taaaagtggg
113760

tcagctcagc ctggaaggcc cgctgggtag aggcaaaggc tgccagctct gggaactctg
113820

cacgttctgc ctccccagcc cctctggctg tagctcccag ctgagcggcc tgcttggaca
113880

gctccaggcc tcggcggtac tgggcctgag aggggagagg agggggcgcc cactcagcac
113940

ccaccttggc tggggcctcc ctcggaggtc agcaacctcc agctgccttg ctggcgagcc
114000

aggagacttc ctgagcttca gtttcctcat ctgtagaatg gggaccctaa tagcacccac
114060

ctcacagggc ttagctcctg gtgagggttg aacaattaag agcagttatc acgcttctgg
114120

aagtctccag gccctcgcca tggcagttca ctttgcctag ccgggctctg cttagatgtt
114180

ccttctggaa aacttctctg actccaaagg ctcctgctct gagtccccac attctccctt
114240

actgtgcgct tccctgtttg tccccgcccc cgcccccgct ccccactggc ctgagaattc
114300

ctgagcgctg atacggggct gaatcaccct gtggtccaga gccttgtcac atcgcatcac
114360

gttgatgcag cttccctgcc ctccaacccc cggctctgtg aggctgagtt attaggtcta
114420

ggatggggcc ctggaaactg cattttaaga aaccctctgg gggattcaga atattctttg
114480

ggcttggcca tctggtaaga atctctgggg gagataacat ctcagctgaa ctctgcaatc
114540

caagctggtt ccctcactgt ctctggaact tttcttgctc tttcccacct ccacagcttt
114600

gccctggcag tgccaccccc tctaacgccc tctccatctg ctccagtcct gctcctctgt
114660

tgtctggaac gcactgcagc ctggaggaag aagttctcaa attctgcgtg ggctttctcc
114720

actgtctcca aacttccatc cttgggggtc agtgattgca ggcaccgcct tccttcctgc
114780

tccatccagt cgctgacctg gggcaggaga ggcagatttg gacatgaggg gctgctagga
114840

gacctccctt gtccagctcc cccacttcct tgcaggctga ggtcttctgt cttcactcat
114900

tcagtggcta ggcctgtgac cactggcttg gactgaaatt ctcccaattc tgggcgtggg
114960

aagaaatgac ttggggaggg ggagtgtgtg gtcatgttct tcaaagcggg accctggagg
115020

gcatctggcc tggccaacgc cctcagtgca cagttatctg aatgaaggca cagggtggtg
115080

aacttcaggc tactctaacc actcagagac gccggatcat aaaaggggtg gcctcagggg
115140

tgatgagctc cctgttcggg tgtgtgtgtg tgtttgtgtg cgcgtgcaca tgcaaacagt
115200

gctggagaag aagctgcagg ctgcagagga aagtccatgg gcctgctggg aactcaccat
115260

gtgaacatgg taagccactc cccctttctg ggcctcagtt tactcagtgg taaaatgggg
115320

tagacatgaa gattcaatga gggatagagg gtatataatg gctctcaacc ccgagtggaa
115380

gttcctttct tattctgggg catcttaaag agcctattca ctcactttat cttcacagat
115440

ccaatgtagc acgtactgat attacccccc attcaacaaa tgaagatact gaagcactga
115500

agagttaaac atcttgtcca gggccacacc acgagtaagt gctggagcca ggaatgaacc
115560

agggctgtct gatgcaggag tgtgccgtgc cccacagctc caggctgaca ggaatgtgtt
115620

ggttctcggc accggcagtc agatgaagcc acaccaggtg caggttccag aatcagaccc
115680

cgagatatgc aaccacaagc aggtaacata atgtctccca gcctcagttt cctcatctgt
115740

gcagtggatg aaatcctagc accttcccca tgagttcaga aagcaatgat gttcacaacg
115800

gctctggccc agggcttggc acagagttgg cgctcaataa gtgcagctct tggggctatg
115860

ttttcaccca ctgagcactt attgagcacc aggtcatgtg ctacgcccca aggatgcctg
115920

cctggccttt gaaggatcgc cagcttggtg tcctttctcc agaacaaagc acctgactag
115980

ggacgggccc agagtcgggg ctttcctgtg gaatgtggcc aggcagaacc cccacacccc
116040

aagccagaca cccctttcac ctggtgaatg gcagcttcca ggcggcccag gcggacacgg
116100

agctccagct tccccaggag gctgttggaa gcggtgacca gaacatgaag ctgctcgtcc
116160

acaaggctgt acaaggcagt ggctgcagcc agatggctcc tgggaggtgg gcggaggtga
116220

gaaaaggcct ctgttgccct cttcaccctg ctgtacttct gtcccgcata ggcctgcccc
116280

tccatctctg tggccccagc ctgggtttcc agctaacttg ctgtgtgact gtgggcaggc
116340

ccttcaccaa gggcctcagc cttggtcttc catgggactg ggaggacaag agaacacagt
116400

aggaagtgaa ctgaccctcc cccttcccta gaagggaatg aagctggccg gacagatctg
116460

ggttcaaagc ccagctctgc tgccttctag taggaggctc tgtgcaaatt tctcaacctt
116520

tctatgtctc tgctttttat tctgtgaggt ggagataatc acagaaccca cttcatagca
116580

tggttgtaag gattaaatga tctaacgccc tggccctcaa ctctagctat acattataac
116640

ctgccctggg agctttaaca aatcttagtg ccctgcccca ggccaacaaa gtcagactct
116700

gagtgcaacc aacacactga tgtttccctt tttttttttt tttttttttt gagacggagt
116760

ctcactctgt cacccaggct ggtcacccag gctagagtgc agtggcgcga tctccattca
116820

ctgcaacctc tgcctcctgg gttccaacga ttctcctgcc tcagcctcct gggtagctgg
116880

gattacaggc acgcaccacc atgccgggct aatttttgta tttttagtag agacagggtt
116940

tcacatattg gccaggctgg tcttgaactc ctgacttcag gtgatctgtc tgcctcagcc
117000

tccaaaagtg ctggcattat aggcataagc caccacgccc ggctgagaca ctgatgcttt
117060

ctaaagcacc tcaggcggtt ccagggacag ccagggcaga gaaccagcgc tcatgcgtgc
117120

aaaacactta acacagtgcc cagcgcacga tgtttgtcag gtccgtgcta tttctattgc
117180

aatggtggga agtagctgca acatgcctgc cggccctgcc tccatttcac agatgggaaa
117240

actgaggtca agggagggga atcgacttgc agatgacaac ctgagttgga accccaccca
117300

gggctgtttc tgcacataat tttaaaaaat gctcaaagcc acaagtcact aaggaagaag
117360

tgtggatggg atttcaggca tggtgatatt tgtgtgaagg gaggaatctc caaccctgct
117420

ctgccctcct ctgccttcag agggaaaatg gaagagagaa gatggaagaa aagcttttga
117480

cttcctggag ggtgctttgc tctacctcag cctccccatc tgactgcagc tccccacggg
117540

ttgccaccct tgcagggaag aggtgtggcc cagggtgcgt gaataggata gaaaatctct
117600

gcctgcctgg agagggaggg acggagggac ggggacagcc taggggctgc agggacccgc
117660

tgcagaacaa acgcccacag cagctcatgt gggaccacag tgacccctgc tggagatgct
117720

ggtagctgcg ggtctgcggg gcctcggatg gacaggaggg gcctgggctg gtggggcagg
117780

taagggtgga agaggctgag aaaggcagtg ccaaccccca ccaggcactg aggggcaggg
117840

agatgacccc ataccagtgc tgcccgtgcg tccatctgtg taagcctgct ctcaagtcta
117900

gtgcagctct gggtggtgtg ttcaagattc tgggaccaga ctgcctggat tcaagtccgg
117960

gccccaccac atcccagcta tgtgaccctg gactaatcac ttaccttctc tgagcctcag
118020

gttcctccac tgtaaaatag gcataatatc tgcagctacc tcggagtgaa ttaagtgagc
118080

tggtatttgt aaatccctga gaaaaatgct ggacacatac aaagtgctat ttaggtcttg
118140

gttctgtaaa caaaatgttc ttaaaagctc atgcatacca gcccttgact ttgaatataa
118200

gtagctgtgt tctaagaggg tgaaaatact tgcaattact gaccaattta atgactgact
118260

tttccccacc ccctaaattt aagaaagctg ggactttcca ggaatgtcta taatttattc
118320

tggctctgag acccctgaga caccatgagg ttctcacaag tgtgtctgag cagcctaccg
118380

ggaaaggcag ggccgggtag gggttaaatg cccagaaggc ctggcttcaa atcccttacc
118440

agccgctgtc tagctgctat ttcccttcgc tgagcctcag ttacccatca gccaaatgga
118500

caatgtggac ctcacagggt tcttgccaga aataaatgag gcaggctctt gcaggggttc
118560

agcgtggtgt gtgggtcaga gaaagtgctt ttccaaaagg gtgaccagaa atgggtaggc
118620

gacatcctct gatgcccatc ccaggctcaa gatggctggg gctgaaggag ggaaagcaga
118680

gaaggggcga ggaaattgcc agggctcagg gcaggtttct gtgaccaggc gtcagcctgg
118740

gatacaggcc ctgcaactac tgcaaagctg ggcgccctcg gggctcagct atgtgtgccc
118800

taggtggtgt ggtcccagct ccaagctcct gggccagaaa caggatgggg tggaagagga
118860

aaggcctgga cgtctgtacc tgacatcagg gctgaagtcc agcctgctgg catcatgctg
118920

cagcctggcc agggtggctc caccttcccg ctggaggccc agtaggccgg ggtccctcag
118980

cacagcctcc atcagctcct tggacttgct caggcacctg gtagcctcct gccagcgtag
119040

ggaggtgccc tgaggcccca tccccactct ccctctgcaa ccttccaagg ctccccacac
119100

ccagatcaag tccaactccc agtttggcat tcaaggtcct ccagaaagtc tgtcatttct
119160

gtctctaaaa catcccctct agccatgtcc cctctcgtca ccacggcaac ctcattggtc
119220

caccctccag catctcttgc tgggacaccc attccagcct cttcgctgat ctccctgctg
119280

ccctctggct ccccctacag tctgttctcc acatggcagt tgaacggatc tcaaagtgca
119340

aatctgataa cccctggttt aaaactctcc agtggctttc cctgcaggct tcgaactgaa
119400

tcccagccca actcttcctt ggtccagctc ctgctgcatc tagaagctcc actcatgctt
119460

ctttcccact cattctgctc cacactggcc tccttgctac tcctgaacac cccaggtata
119520

atcccacctc agggccttca catgtgtttg ccccctgcct ggagcacagc cccccacaaa
119580

ggcaggctcc ttctcaaact tcaagcctca acttaaactc cagctcttca gagaggccct
119640

cctccctccc tcattattcc caatcttact tccacaggac ttgacacaat ttggaatgac
119700

atacccattt ataatagttt gctttttaaa ctaccacctc tgccacagca ccacgagccc
119760

caagaggcca gggataagtc agtttgctcc aggctggtct ccagtgcagg gtacaaagtg
119820

ggtgcccctc aaatatgagt caaatgaatg atgaaaaaca ggaataatgt cctccccctt
119880

ttccccaggc cgtccccttc tcgaagccct tggtccatgg ggtctggggt gtgatgtagc
119940

ttccccagtc accatgctcc aggctcacct gcatcccccc aggggggtcg gccttctcga
120000

attcctcgat ggaagcttgt agcagggaag aggcctggtg gaggtcagca aggaaagggt
120060

ccagcttctg caggacagag gaccaaaggg caggtgtcag cccaggccaa ccaggaggat
120120

gcctcttgca tctcaggagc acagagggga ggtgggaggc agtggctctc caactccagg
120180

tgcatcagaa gcatctggag agctggttaa aacacaggct ctgggctctg ctcctgaggt
120240

tctgcttcag ttcctctggg gcgaggcatg gtagaaagtg catttctaac aagttcccag
120300

gtgttgctga tgctgctggc ctggggaacc acactttgag aaccactggt gttgagtatg
120360

agtgagggag aagaaaggag acaggcctga gcgactccca ggccagtggg gataccatga
120420

ttggtaagga aaggaagagg gcagagttag gggaacattt tcagctgagt cccacttcgg
120480

cccagctcca tcacttacaa gctgtgagac cttgggcaag ttacttaacc tctctgtgtc
120540

tctgcttcct cctctttaaa atggagatca tagtagttcc tgccttatag ggttgtgtga
120600

ggattaaacc cattaatata tgcaaggcac ttagaacaga gcccagccca tactgtgttt
120660

atcatcattc tcacttccat gtagtgggtc tacaggtaag atttcatgta aaggaaaggg
120720

tttaaagcta aaatacagtt caaaaaccag tggattcatt ggaggcctct ggaaagggaa
120780

gctaggaccc tgggggacaa gcggaaaagg agattgttca gtaggtatct tttggacca
120840

tttgaaatgt gaatatactt actgcctatt caaaaacaca caattttaaa aagatcactt
120900

gggcaaaaag taaataagcc accatgttac actgttagta tagaccggcg gttctcaatg
120960

aggggtgact ttgcccccgg ggacattcag caatgtcagg agactttttt aattgtcata
121020

atttgtgggg tgcaaacagt gggtagagcc agggctggta ccaagcatgc cgcaatacac
121080

aagacagctc ccaacaaaac agaattgctc aaccccaaat gtgaataggt taaggttgag
121140

caaccctggt ctagagagga cccggggcgg gcattgatgg gcatcggttt cggtgtggtt
121200

taagcccacc gaaaggcacc ctcgccctat gtgtcctcgt gtactaaatt gggtatgaaa
121260

tgccccctct tggggttgtg gggaggaggg actgtgatta cacaggaaag gagcccaaca
121320

ctggcggctg ccttatttcc tccctcttat aggagggaac cagaccctga gcttcctgag
121380

ttgtgtgggt ccatagagca agtgcctgta aatttatttt attttatttt aagattcttt
121440

ttgagacaga gtctcactgt gccacctagg ctggagtgca gtggtgtgat cttggctcac
121500

tgcaatctct gcctcctggg ttcaagcaat tctcatgcct cagcctcccg agtagctggg
121560

actacaggtg cataccacca tgcctggcta actttttctt tcttgtattt ttagtagata
121620

tgaggtttta ccatgttggc caggctggtc tcaaactcct cacctcatgt gatccaccca
121680

tctcggcctc ccaaagtgct gggattacag gtgtgaatca cagcgcccgg ccctgtaaat
121740

ttattttcag ttaacataga cctagctctt tacccgtatt aactaaatta atcctcacaa
121800

aaaccccatg aggaggtact attattatcc cctgtttagc tgatgaggaa accaaggctc
121860

agagaaggta agtaacttat ccaaaatcac acagcagagc tgggattgca atgtaggcag
121920

cctggcccca gagcctgagc tcttaatcat ttgactctgt accttcactg taaacatttg
121980

ttgagtgacc gtttattttt tagcatagca gggttgatgg atccatggga cagaagagtg
122040

aagagaagaa gagaaggagt gggggtcagg ggctaaaggc tgtgacggta aacacctgga
122100

agaaatgaac ccactcggtg tggcagtagg ggaaggggcc ttccaggact gcgggcagct
122160

ggctggggtc cacatggtgg ctgagggcct tcaatgaggt cagcacctcc acctgcagac
122220

caaagaatca ggagaaccag ggactcaaag tgaggacagt cattttagaa ggtcctgccc
122280

agatccctgc cccagcacca gttcatttcc cacaagtaag gttgactcca gctttaaaag
122340

ttgctttgcc agcaaccaat cttctctgga aactctgaca aatcaaaatt cagaaccaga
122400

ccctggttag ggaatggcaa ataaacagca ctcatactgc cactctccta gcttgtgccc
122460

aaggcagaca tcattaatca atcatagcac tcgttccctc tgagcttggc tctttcccag
122520

tacagctctc cgggcaatca tacaaactga tggaagctgg cccttgtgat gaaacttctt
122580

tgtaatcttt gcacagttcc ctgaggagcc aagagctgtt tccttcttgg ggctggtgag
122640

aaaagtcact aaatcccaac agtgtcctct aagacatgga agaactaggt gactgcttct
122700

gccaggcaga gaaatttgag gtttatgctg ggaatagctc cagataccta ggcttgggat
122760

ttggtggatt ctgtggtttt tttggaccca cctgatattc ctgctactca aagtgtggtc
122820

catgggccag catcgccggc atcacctagg acatgttgga aattcagagt cccaggaccc
122880

acccagaact actcaatcag aacctgcaca ttaatgtatt tccccaagag atcagtaagc
122940

acattaaagt tggaacagca cacctccagc ccacaatctg cctcttcact aaccaggagg
123000

aaggagagtt ttaaaatcac atcccgaagc aggacagagg actgactgcc cttactcatt
123060

actgtggtgt atggcactaa ttaaagggag ctgcagggct ctttcctgca gagtcccgct
123120

tcagctccac atctgtatta attagggcga gaaatacagg aagagcaggt gtgaccgcag
123180

tgtgaaaaag gcactggagt caggtgactc cagctctgcg tctagcttgc tgtgtggcct
123240

taggcttcag tttccccctc tgaaaacaaa gggaggaggg atggaccaga tgaagctttt
123300

caaataggtt ctatgccatg tgcaattctc gatcatgtgg cagcacccac caggagctgc
123360

taggattgcc agagagatat cactctgtga ctgatttgac aatgcctgcc acggatctat
123420

actggggatt ggtagcgctt aggcctcgta tttattgcct tacactagat tacctccagg
123480

cctcttctag ctcatgaatt ctgcagttct ccctctcctt aaccccagct cagctcgagc
123540

ccaacaactc ccttcctcaa ccgccgaccc tgagtccccc tcacctggac gtcaggtaat
123600

gtctgcagct ggagagccgc ctccttctcc cccaggaaga gaatagcccg gatagaggct
123660

gggacctgag cctgtcaaag acaaccactg cagagttgct ctgcccaagg cttcatgaag
123720

cctcacacac tttgatttcc tccttcatcc cctgtaagat gcaggcaaat gggtcacaag
123780

gacagagtgg ggtccctcca tgactggaat ctttgtgtga tacgtttgga ccgtgaatca
123840

ttggttagaa gtcatgagag cagcaggttc aagttgtgtt gcaaatcttt ccagatgacc
123900

tgcccctctg ggccccagtt tgcccatcta caaagcaaag gaggtcagac cataaggtaa
123960

gcagatgcag aacctccatt gactctgctg cccataattg ctggatagga atcttctctc
124020

ctcccttctc agctcatgag ctttatgtga tctaagtctg agccaatcag tgtgttgcat
124080

tcacccccag gccacagggt tgggtgtgga gggactcctg aatccactgt aactgagaca
124140

caaagaaatt tttgctgaga cctctgggaa aataatctct tactctttcc taatggattt
124200

aaattagaga ccatgtgaag tcagagctgc tgggagccat ccggccacca aggggcgcgg
124260

ggtgccaagc ggggtggctt ggcatcctcc aaagagaacg gggccagaaa tggagagaga
124320

ggaaccaggt tctaatgata atatctgagc ccttggatca ggccatactt gaagctggac
124380

ttggctcctg gactgtccat tgacatgaac caatgagctt caagcttgtt agggctgagt
124440

tttctgttgg ctgcagtaaa agtccctgcc tgagagtcct ggtcccagcc ttaagcaccc
124500

ccggactcta tgaatgtcag gaggcccctg gtggctccca ggacttacta ggctaaggct
124560

ctccacctcc cactcacctg ggtggcctgc agggcgctga ccagaccggg ctgtgggggc
124620

tgtctcctgg cgtcaatcag gaccgccagc cccttggctt tatcttcagg cctgtgggtg
124680

gtaaacagaa ttgacagtct tgctggatgg cctcggacag ggggcaggac ctagtggaag
124740

gtgagggaac ctggctgcta gaaaccttga tttactggtg aactcttcat cctgttaaga
124800

ccaagttcca atgccccttc taggaagccc tccgtggttc ttctccaggc agagcctttt
124860

gcttcctctt tggtgctcct gcaagacctc atacagccct cgtttatagc cctactattc
124920

ctaactctag caactaaccc aggtacagta ggcaagtcac ttgggactca tccacttttt
124980

tgtgtaggca gaatccttat tttgctcaag aatccaactc cctccaaaaa gccaagttcc
125040

cccagagcct ggcttctgat tggttgaggc caacagtagt gacgtaattc ctctggccag
125100

tgattggttt agatggggag ggtcatgtga catttctggc caatgatatg tgagggaaag
125160

ttttccaggg aaagaggctt ctgggaaaga cattccctcc tttaaaaagt tcttaacaaa
125220

gagatatgag gcagagacat acaatgatac atttccttaa cagataaaac attttaaatg
125280

ggaatttctg ttgattgcag ccaaaacacc ctccctggtt cagccacatt gcactgagct
125340

tacagtgggc cacttttcag tgactatctt aggattttcc aaactggcac aattttcaag
125400

acttgttaaa atacagattc ctcaccctac tcctctccca tccccacccc tctagccatc
125460

tggccagtgg gtacagggtg gccagggaat ctgtgtggtt ccaagcaccc tcggccagtc
125520

taatgatcag gcaggattga aaaatagccc ccaactgccc tcaagcctta caaccactca
125580

gtgcacagac aaaacctgag gtggcccagt gggagggtag agctgtggca ctgccattag
125640

taccttgtgg tctgggcaag ttatttcatc tccctgagtc aatttaataa tgatagtacc
125700

ggccttatgg gctgtagtga ggactgatga attcattctt gtaaagcact cagtgactgc
125760

acagagtaaa tgggatgtaa atatttgcta tgattatcct agaaggggaa actgaggcac
125820

agaaaggccc agtggcagat agtgacagga ggctctggga ggtgatttca agtctctgac
125880

tttatgtcct tgcctccctc tggcgatgcc catgctggac agtgatttgt ttccagacct
125940

gtctctgcta ccaacctgga ggcccttaaa agtcagggcc cggtgtgagg tgcctctgtc
126000

cctagcacca tgtagcatga agccaagccc agagcagggg cccagccaat gttcattcag
126060

agcacaagtg gagaaaacca gcccagcaca caagcacctg gtgtgcactg cactccacag
126120

tttacatcct ctacaccccc tcatccctgc cacttgtgcc cagcttggtc cttagagccc
126180

tcttggtcat gccctttatt caggcgagcc ttggggaggg gagattattt ccctatggtt
126240

gcatagccag tcggtgggat cccagagctc tgtctaccac actagtcact ttccaccaca
126300

gaaaaagggg gtcccatctc ccactcagtc cagaaaagcc actccacctt ggacaagaag
126360

aaaaaaaatg ttagtgacca gcaggtggca gtgatggctc cagtggggtg ggatgcatga
126420

agatagttcc agagggttcc atcaccttcc accccttacc cccaacagca gacctctagg
126480

ttgagttggg gcttattagc cagtcctgag cagcatcccc tctaagactg ccctggctag
126540

gggctgggtc ccgaggaaaa gagggcgcct cttccccagc cagtccatta agtctgaatg
126600

gtggcagggg taggagtgtt aggaggagca ttagaaatca gcctgagtcc actcatcatt
126660

cattcactca ctcactcact gactcaccca ctcattcttt cactctctca ttcattcatt
126720

tactcattca cccctttcct ttgtttattc aaatatttat aattcagatg acagctcaat
126780

tatcacctcc tcagggaagc cttccctgat ttcccaattg aaattaacat cccctattct
126840

tcttactcta caacagtggt tcttaaagtg tagtcccagg cccagcacct gttatcaccg
126900

ggaaatttgt tacaaatgca gattttcagg tcccacccac cttcatcaac tgaatcagaa
126960

aatctggggg atgggcccct gcaatctgtt ttaacagccc accaggtgat tcgaatgcac
127020

cttcaaattt gaaaaccggc caggcacggt ggctcacgcc tgtaatctca gcactttggg
127080

aggccgaggt gggtggatca cctgaggttg ggagctcaag accagcctga ccaacatgga
127140

aaaatgcggt ctctactaaa aatacaaaat tagccaggtg tggtggtgca tgcctgtaat
127200

cccagctact cgggaggctg aggcaggaga atcgcttgaa cccgggaggc ggagattgca
127260

gtgagctgag attgcattgt tgcactccag tctgggcaac aagagtgaaa ctccatttca
127320

aaaaaaaaaa aaaaatgaaa accactgatc tacaaacatc actgttttct tcatcacact
127380

gacactcctg aaagtttctt ggccatggtt tgtttcttgc actctgtcca tctctcccac
127440

cagcctgaca accccccgat ggcagggact gaggctgttt tgctttcccc cggcatctgt
127500

ctggggactg gctctgtgaa cagacgactg gatggaagga tatggtgcct ctccactcag
127560

tgtctggtcc agggctctgg gcccagaggg ttgaggtggg gatggagcag tcagggtggc
127620

ccagatcctt acctggggat ggtacacagg taggacagta gcttggtgac ctctgaaact
127680

gtgcaccatg gtgcctccca ggccccctct gtagttgaca ccagaagcag ggccgcccg
127740

gccctgtccc ggccacctgg aggacagagg gcacacgtga gcagtgagaa cttgtggggg
127800

gccctctagg gacctctcct gtggcctgca gacctttggc cgtaacggtt cctttgcctg
127860

gaacaccctt tctctccttc tccttctgtg aactctgact tctacttatc cttcaagacc
127920

cagctcaaac accacttcgt gagaaaagtc ttccctgaat ctcctctgtg cagagcactc
127980

cctctgtata agatacgtgg gcaggcaaag ctcatggcat gagctctggg gtcacacaga
128040

aaggggtcaa gtctctgctc tgcagtgact cactgtgtgt ctgtgggcaa gtcagtgcac
128100

ctctgagctt cagtttcctc atctgttaaa cagggagaat aatatgctga gcttattagg
128160

ttatttcaag gatgaaatca gacagctcct gagaagcact taatacacag caagagctca
128220

gtgaatgatt gagttgctca atcgttggtc gttacaatga tcactgtctc tgttctcgtc
128280

tccagtccaa ggggtggtga gcgtgcagcc ctgtggccac cagcactatt tcttcttgca
128340

gactttcaac cctgggtttc cgacaggcta tttcctgttc caggaatctg ggcggtgtgc
128400

ctcactctcc ccacaggagt catgaggctg aggctccggg agccagtaca gcccatcctc
128460

atggggagga aaggggaggg caaatacagc agcagggaaa gaggctcggg ccttgggaaa
128520

ccacacctgt ctcgcagcct ggactcagag ccctgcactt cagtcacctc actccaggat
128580

gccctgcaat gaccaccagc cactccacgt gggcaccacc ctctgcagcg tgcaaaaccc
128640

ttcacagctc acgaagcctt ggacaaacct aaccaccttc acaagtgccg cctcacaggg
128700

gtccaaccac ctggaacatt ctttcacata tgtcttaaaa tctgatataa tttttaaaat
128760

aaaaaaaaaa tttgtggctg ggcacggtgg ctcacgcctg taatcccagc actttgggag
128820

gctgaggcgg gtggatcacg aggtcaggag atcgacacca tcctggctaa catggtgaaa
128880

ccccgtctct actaaaaaat acaaaaaaat tagccaggca tggtggcggg cgcctgtagt
128940

cccagctact tgggaggctg aggcatgaga atggcgtgaa cctgggaggt ggagcttgca
129000

gtgagccgag atctcgccac tgcactccag cctgggcgac agagtgagac tctgtctcag
129060

aaaaaaaaaa aaaaaaattt gtgtgtatat aaatgtgtat acatatatat caatgttatc
129120

ctcttgatat tatggatttg atctttttcc tactattcac ttacaaattt cgaaagtaaa
129180

gacattttgt tggggataag aggggagact tgaacagaaa ctggaaattg attagtatga
129240

ggacattctt tttcattttg ttaggggccc gggtgcagtg gctcatgcct gtaatcccag
129300

cattttggga gaccaaggta agagaatggc ttgagctcgg gagttcgaga ccagcctggg
129360

caacatagca agaccttgtc tttactaaaa attaaaaaaa taattagcca ggagtggtgg
129420

tgtgtgcctg cagtcccagc tgctcaggag gctgaggtgg gagaatcgct tgagccgggg
129480

aggttgaggc tgcaagaacc ataattgttt cactgcactc cagcctgggt gacagagtga
129540

gaccttgtct caaaagaaat ttttttttat ctgttaagtg tactaaatgg tattgtgatt
129600

attagggaac acactgaagt atttaggtga tagaatatga agatttgtaa tatttttata
129660

aatacttcag aaacaaatag atgaagcatg acaaaatgtt aagatttatt aaatctgggt
129720

gataggtatg tgggtattca tgatactatt ctgtctactt ttctataggt ttgaaatttt
129780

tcataatata aaagtccaaa agaaggtgaa gtcattaaac catgaaataa atgcacaact
129840

tgtgaaggtg gaggtgcggg tacaggtccc tccaaaatcc tatcgcagct gtttgttggg
129900

tcccttgggt caggaaatgc tacatcacat acctgggtcc attttgctga tgaggaaact
129960

ggggtgggga agcagcttcc cagtccccca tgggctcagg tacccaaact ctctgcaagg
130020

gtagagacca tgcctgattc accccttgaa ttaggggtgc ctttgtctgc tacacacctg
130080

aggcctggag ctcaattgca gctctgatgg tgctgcactc ccctgctcta taaccttcta
130140

tggctccttg tggccttgaa gagaaactgc aagcttctgg acctggcatt gaagtgtctc
130200

aggctctgca cactcttcca tcctcatttc atacactagg tgttaggggc gaagctgtgt
130260

cccctgcccc tccaaattta tatgtggaag tcttaactcc cagtacatca gcatgtgact
130320

gtatttggag atagggtgtt taaagaggta attcagttaa aatgaggtta ctgtgggccc
130380

taatagaata tggccagtgt ccttacaaga agaaatttgg acacagaggg aagaccccat
130440

gaagatacag ggagaagatg ggcatttgca agccaaggag agaggccaca gaaggaaaca
130500

accctgccaa caccttgatc ttggacttcc agcctccaga cctatgagaa aatgaatctc
130560

tgttgcttaa gccacccacg ttatggcagt cctgggacac taatatacta agtctcccct
130620

tgccaccaat gacagatgat accacccagc tttgttcacc ctattccttc tgccaggtat
130680

gcccttctgt cctatctcta tctggtgaaa tccccctcat cgctcaaaga ctagccaaga
130740

tgccacctct tcctggaaga cctccttact gccttccctc tttagaagct cttccccagt
130800

cccaggttcc caaagcaaat gtaggcctct gtgagatcct gcaagactga gagcccttac
130860

gggtgtgcac tgggtcaaac tcattgttgc atccctgtgc tcagcacaag gctggataat
130920

caatgtctgt gattgattca tgaaggaatg gagggttaga gcaacacctc tcaaactttg
130980

atgggcctgg ggatcttgtt agaatgcaga tgccagttca gcaggcctga gattctgcct
131040

ctccccctag ctccaaggtc acgctgatgt aggtagtccc cagaccacac tttgagcagc
131100

aaagggttca cacactcagg acattccaca ctgggacaca gtagtcatac gattagttca
131160

catgtctggt tccccacact gcctggaaca gggccctgca cacacccagt gccagctgag
131220

ttgattacag caaggggcag ttgtccacct gatttgtctt cagctgggcc aaggagaccc
131280

ttttgcccca aatgttatcc tcacctacca gctggcgcca gaaataccat cattagcatc
131340

tcagaaccgt ggcttgaagc tgtgctgttc acagatattg tgagcctcag gtgaaaatga
131400

ggatggcaaa atgagggtgg tgggtaagtc ggggttgccc aaagttgctg aaaaatgtgt
131460

tgcatctgcc agcagcatga agagtcactc caagtttggg gagggaggaa tggtaagtaa
131520

ttgaatttgc aagcaattaa aagcattttt tcatatcaaa acgagtgtga atgtattcta
131580

tttatggata tttaaaatgt atagaaattt taaaggcaaa aaggatttca aagatctata
131640

tggagcaact gagagctcag ggtgaggtct caatctcagg ggctctgttc attctcttct
131700

gcattaggga ttttccagtg gaaagacagg gaagccctct cctaaggctt cgtgcctgtg
131760

gccccaaaga tgtccatgcc ctaatcccca gaaccttgaa tatgttacct tacatggcaa
131820

aagggacact gcagatgtga tgaagggcag gaccttgagg tggggagatt atcctggatc
131880

atccactcta atcataggaa tccttaaaag cagagaactt ttcctggctt tggtaggagc
131940

aaggtgccaa gatggatggg gggtgaggga gatgcaacat tgctggcttt gaggatggag
132000

gaaggggcca caagccaagg aaagtagtgg tctctagaag ctggaaaagg caaggaaatg
132060

gagtctccct tagagcctcc aggaaagaat gcagctctgc agacagctgg atgtagccta
132120

gcgagatcca tgttgagctt ctaacataca gagcgatcag ataatacatt tgtgttgttt
132180

tacgtcacta atttgtaata acttgttatg gccacaatgg gaaacacagt gcctattaaa
132240

caaaacccca tccccagcat gggagcagac attcacacct gcatgttgat cagtttcttt
132300

cctgagaaca gatctttatg aaaactttac ggggagacct gcttcacagg gctggcttgg
132360

agcagatcta gatcttgaag cacaggctct ggggactgtt tgccctataa ggaaactcca
132420

acagaggccg gtgtggacgc agagacacca gcgaggagac cactgcagca ggcaagagac
132480

cacgctggtt tggacttggg tggggtgaag tcaacagatt ctgaatacaa tttaatggga
132540

gagttgacag ttaacaaatt gaatgtggga tgtaagagaa agagcaatca aggatgatgt
132600

ggtcatcttt tactgccatg aggaaagact ggaaggttgg gaagtgtggg gtgaggggat
132660

gtgaagtcag gagtccatat ttgggcatgc taagttagag atgtctgtga gacctccacg
132720

cagagacatc aagtagtaaa cagctggatg gataagtctg gagttcatgg ccagggccca
132780

ggctggaaaa atcagtgtcg gggttgtcag cctggagaga gcatttacag aaaatattcc
132840

acctccagga tgagatcgcc taggaagtga gtgtggatgg agaggagaaa gggaccaagg
132900

actgggcttt gggggccctc caggatttac aggttgtgga gatgaggaga caggaaagga
132960

ggctgcaaaa ttgtggccag tgaggtgaga ggaggactgg gtgactgaag atctaatggc
133020

aagtgaagaa agcctttcaa caggaggaag tggccatcca tgccaaatgc tgcggaaggg
133080

cctagggtga tgaggactga gaatcgaccc ctggatctag aaacatggaa cgcaatgacg
133140

accttgacaa gacgtgtctc agtggcacag tggggaaggg attctgatta gagtgagtgg
133200

aagagagaag gtgtggaaac agggaacaca gacaactgct ttgtggagtt ttaatgtaaa
133260

agggtgaggg tgggaacacg ggggcctaga atagctcatt tcaagctggg caatattgca
133320

gcgtgttctc atcctgatgg gaatggccca gagccaaaga ggaaagatga tcctgtagga
133380

aggagaagag aatcttgctg gagggatgtc tttgaggagg taaagaggaa cagagcacgg
133440

gttcagggtc agcctcaggc acatggacaa ttcatccata ctagaatgct gagcctgtgg
133500

gtacagatgc cagcaggtag gtgaatgacc ttccttctgg tctgactgag taagcagaag
133560

caattcatag acttccttcc aattgcttct gcttactcac tcagaccaga aggaaggtca
133620

tcagctgaga tttaagttgg ggaaggtggt gttgaaggtc aaaggagaga gaaggtgtga
133680

agtcatcaca caggactgtg ggggagtcat tcaggactct ggaggtgaag taggatccac
133740

tagcaccatt gggaccccca gaggtcagtg gtcaggaatt caacacaatt agcctgcaca
133800

attgtgtgga tttttccagc ttcgttcagc ttgagggtgc aggtggagag ttggatctca
133860

cggaggttgg gatttatctg ggtaagtgtg aaggtggaag aggaaagcaa gcgagtgagt
133920

gtttgaaagg aagtgattac aatgtgagcc tggaatctaa gctaggaaga agggatgtga
133980

ggatgtgaca gaaggtgggg gagacataat gatggggaag ggatgtggtg gagagtgagg
134040

aagatatgat gatggggaag ggatgtggtg gagggtgggg agacatgctg gaaaagggga
134100

gtgaaaaggt ggttgaacca ataggttcaa ggttttggga ggcctgaaga tgatgaaact
134160

tggggcagtg ggtgacctgg aaagagaagc agtggtaata aaggagaggg agccttaaca
134220

tgaaaacccg gcggggagaa gttggttacg ggggatgcga tggggggagt catgtgggga
134280

atgaggacaa gatcactaga gaagagcagg acaaaaaacg gacagaagga gcacgcatgt
134340

ggacgttgct cattcacaca gcaaatgctt tttgcacaca tgctacacat caggcatgtg
134400

cgagctaaca gtgcatgttg caacccccaa gaattacaac aaaagtaact gagggaagag
134460

tgacagtgag ccaggtccta tctgtctatt ctcctcctct caaggaggcc cagagagggc
134520

agtgacttct ccaaggacac acagggagct aatgatagag ttagaactaa aacaaaggtc
134580

acctteetcc catagcagag ccttccatgg acaaaatctg agcccttggc ccatgatggc
134640

cctaagagga tcatgagatc ctttgtgtac aattaatatg gaacaaacag caggagcttc
134700

agcaaagatg accaggaaaa tgacagataa ggtctcctgg gagctgcaaa aagacagggc
134760

agtgctcaag gtcctgaaga gatatggaag tctgtgaagt aaaatagtat ttcaggacaa
134820

tcagtctcca gaaagcactg tggttctctt gtctctggac ctttgcacat gctgttccct
134880

ttgtctggaa cgccgttccc aactcttcac ctggataatt cctactcata ccataggact
134940

gggctttacg ttacttcctc cgggaagtca tccttgacct ctactaccct aagcctgaac
135000

caggggactc atctgtgctc ccacagcccc ctgtccttcc caactgtcac actttgttta
135060

cactgggttt caataatctg ttgtgataac ctgttggctt ttccccacca ctagaatata
135120

ggtttcctga tagcagagaa cacatcagac ttgttctctt atgaatcgcc agtgctaagc
135180

ccaggaacca gtaaatgtct gttacaacaa tggggaaata ttaaatacga gtgataagac
135240

cttctgtatt ggatgctgct agtaccccaa acccagatcc cctcagcagg cagagaaccc
135300

atccccagag tctgaaagta tcagttgtca gtggctcaca gatgccccct ctctgaagaa
135360

gcactctggg ctgaactgga tcactggctc cccctgcccc accccattcc aggatcagcc
135420

cgaagttcct gcagttcatt aatgacggac agactgggga tcagcccaca gttaatgact
135480

gacagacaag gcttataaaa ggccagtccc ctacaccttc agctggagcc agctctgtgg
135540

ggcgggtcat gccccagtgc tctccttggg gtcagcccac agccttgctt ggctccttcc
135600

cctgccttga ccctctgtcc ttctcctcag agccctcctc caataggtca ctttcccaaa
135660

aatctatgtc tcaggctctg cttctgggga acccagtatc caccttggct gcttcactga
135720

tggatggaca agtagacaga tggacagaca gatagatggg tggatggata ccaacaaatt
135780

gctttcatct attccaattc tcccatggga aagatgagga aactgaggcc caaggaggct
135840

tggtctagaa cctaggcctc cagggcattc cctgcctccc ctccacccaa cagcagctga
135900

agatgctctg gggcccccag ctccctccct accacctcct gcttgaatcc cctggaacct
135960

ggcaggcatg ctatcctgga gcggaaaacc ttgacaccta gagccccaat cctgggctca
136020

ggccctgggg gctcctcctc cagggtgggg cctctttctg gggagcctgc agaagcttct
136080

ggcaaggcag ccttttcctc agtcagtggg ctgggggcag tggctgggcc agatgttttg
136140

gtctgagttg ttccagctgg gaaaaagcaa caacaaagaa aaggacgtta agcagaagat
136200

gggctttcta aggggaaaac gtctcctctt tctctttctg actaataata gcttacaact
136260

attgatctct cccatgtgcc aggcattgcg ctaagcccct tacttctagt gttacccctc
136320

cccacacctc actttttttt ctttagtgac agagtctcac tctgttgccc aggctggaat
136380

gcagtggcat gaacatagct cactgcagct ttgaactcct gggcttgaaa gatcctccca
136440

cctcagcctc ccgagtagct ggggctaaag gcatgcacca cgaagcccag cttttttatt
136500

tattccttcc ttctttcctt ccttccttcc ttccttccgt cttccttcct tccttttttc
136560

cttcttttct ttctttcttg taaattcctg gcctcaagcc acccaaattc tcccgcttca
136620

gccaccaaag cactgggatt acaggataag cctctacacc tagcttctgg tgtcgctttg
136680

aaccctcaaa ataacactgt gaaataggtg acattcccat tgcacagata gggagaacgt
136740

ggactcagag aaactcagta cttggccaac gtcacacagc cagtgtatgg caaaggtgga
136800

gtgtgaactc aggcagttgt aacacagtca cagtgccttg ctcaattcca gacacatgag
136860

acttcaagcc ccttaacatg ccacactgta tactaaaccc ctgaagctga gccagcccag
136920

gtggtctcta tcacttacaa ccaaaaccac ctaaaatgtg atctcaacca gacaataacc
136980

tccttgtaca gctgagcatg tgggcatctt acctttcccc aaagatttgg ctcggttggg
137040

tttaggagag tagagggaac acaggacttt ccagggccca ttgtgctgga gtgaggcttt
137100

ctccggggtc acagagggtt gcctctgccc tctcaagaat gagaatttga gcccaggagt
137160

ggggggctca ggggaggagg tgtttctgct agggcagggc atgggcttgg gaagtctccc
137220

atttctctct ttggtcttca cctctatctt tgtttcagag gctgcagctg caggagaagc
137280

tggggacagg cggggagagg aggcttgtct tggtccaggc ctgagctgca ccatattctc
137340

tgggtttcct agaggtccct gcatcttgga ggcagctggc tcttgtgaga cacctgccct
137400

gagaccagag gcacagtcca gtgcgtcctc aaggacattc atgtaggagc cttgggcgg
137460

cctttctgag ttgtgggtgg gtgctttaag attgaccttt ctcctgaagc ccaaattata
137520

gggcctctcc tcagagcttt ctggctttgt gcaagcccga tttcccaagg aaggtccttc
137580

attggcctct gagagaggca gtatcttttg aaataaggga gtttccttag ttccagctat
137640

ctcctctagt gccccagaag tacacccact ggatgtgcct gcctccctac tgggagactc
137700

tcctctgctc tcttgggaaa agcctaggag agccacatag tctccctcga agtcctggct
137760

gaccacctcg tccatcctga aagccacctc cccacaccgg gcttgctcca ccttgatgag
137820

tcctggatac ttgctcccat atgtcctgcc cttacccttg gccaaactct gggcaggaag
137880

cacctggttg tctggggagc tggcctggtg caactcttga gggctgctca aatccagtgc
137940

ctctaatgga aggggccccc aggcttggca gagggcattc actacttggg gtctgtcatc
138000

cacaaactct gggctggtta tcttggtcca aggcacaggg gcaatcccat tttctgatgc
138060

taccaagcag ttgtgtaggg gatttccctc aaagtcactg ttgatggcct ccagccattc
138120

ttgggtgaaa agtatagggt aggctggctc tggaacaggc ttcttgtcca ctgtgcagag
138180

gtccagggag aggcatttga tcatgatgca gacagactgc tcctcctggg gctccacttg
138240

gaggtagaag tcaccctggc gcaataagag agggttgagg ggtgccaagt ggaccacaac
138300

ttcatccctc agacagagtg gccagccctc gtgtaagaag aggcagtgtg agtagggagc
138360

ctgtggagag aaggggatgg catcagagaa cctggccctg aaaggctcca cccttggatt
138420

tgctcctttc cctctcccac gtggaatctg gaacaggtca gggactagga tgaggcagca
138480

tttaggaggc actcactgtc aaggttgtgc aaaggttgac tctcctgccc caccctcgtc
138540

ctggccctga ccttggaaat cctagcctac tgacccaagt gagaaagctc agagcaccat
138600

gcactagtta gcaatcggct ttcccaagtg gtccccttgc ttggctcatg ctcccttcct
138660

ctctgcatct atccagaaca tttcccaacc caattcaagt cttagccctc cattttaaag
138720

ccagggagac gtggtttaga caaacgtcta tgttgctaat ttgtgcaaat tgcttaactt
138780

ctttgagccc caatttccct atctgcaaat aggataataa tacatgtcag tattactggg
138840

ttggtatgag gattaagtga gattatacag atggcctcaa tttatggtgg ggttatatcc
138900

tggtaaatcc atcataaatt gaaactatca taagtcaaaa atgcatttaa tgtacctaat
138960

ctacagaaca tcatagctta tttagactag cctatcttaa aagtgcacag agcacttaca
139020

tcagcctaca gttggccaaa atcatctaac acaatgccta ttttataata aagtgttgaa
139080

catcttattg aatttggata gtgtatgagg gtgaagtaga aacagtattg aatgagaagt
139140

caggaaatct gaccctgcca ctaacttact agatggctgt ccagtttttc ctgacccaaa
139200

ggttattctg ggcctgaatg tttatttttt atttgtttgt ttatttattt ctcattaaat
139260

aaatgaaggc ttatttgttt attttttatt tatttcccat taatggataa taacacgaag
139320

gtgaagcaaa actttaaaaa gaaatttttt tttaacagga gttgccagaa attccctgta
139380

taggtgggta cagagggtca ttctggaaca ggctgtgacc caagtgatcc ctctcagttg
139440

agcctgtcca ctgggctgtg cgggaccagt gttcaacagt gacaccatgt ggcaacaacg
139500

gaaacaacag ctccactggg ctgacaacag gtctttaact ggcttttgga aaaagagtct
139560

gagttctgtg gattgagtaa gtcccagaca atcttatata atttggatgt gtgtagggga
139620

gatgtacaga gagtgagact gaatttcctg ctaattaaac aggtgtttcc tggaattatt
139680

gattgcggaa atagtgagat ctgaccatag ttatacctta tgttgataaa cccatttata
139740

tcagtcatat tgtagattgg gctgcctcag agaagcttaa tatccataaa ccttcatgag
139800

tctgtaggtt ttctcgaccc ttttcactta gccctcttgg gtttgcaaca tgctttctct
139860

ctcaccttct cattgaatga gaaaaaacag cccagccatt ttttgcaaac agcaaagcac
139920

cagagtgatg atggctttgc tcatctcact tgactttcac agtaactcag tttgatgtag
139980

gcagtccagg cattattatt ttcattttac agatgatgca actgaggctc agtgtggtga
140040

aacatttggc tcatagccac acagctgata agcatcaggg acttggacct aggccttcac
140100

atttcaagtc agctgtatct gtccccaagc cccaccagac ttcatgtgaa ggtggctgct
140160

tctgggtgat ggtggctgga gaggcagact ttgaggctgc catgctctta tttcagatgg
140220

agaaacaagg aagtgggata gcgatagcca atagctactg ggcacttgct gcatgccaga
140280

gtctttgcta agccatgtgt tgtcatctcc atgtgttgtc atctccatgt gttaggtgct
140340

gtgttattaa cgtttctgct tgacacataa ggaaactgaa actcagagag gtggcatgga
140400

tctccaaagt tcacagtgat gagcttggat ttgaacctgg ggcatctgaa cccagaagcc
140460

atcctgttaa tcacacatga cttcccattg ctctgagatt gacagatatt tgcctatcat
140520

tacaattaga ttaaactcac ctacaaggaa aaataaagac gcggaaatgt tgttaccttg
140580

aggaagcggc cagtgcaaat gaagtttaaa tggccaaata agggaagtgg gcaattgtgt
140640

agcttgtcag ggagaagaaa taaggatggg ggagcatctg catgaaatag tgtcatgtgg
140700

aagtggctgc aaatgcttcc atgagaagct cccttcttct ccaatactag ttcccatgta
140760

ttgagctatg atctatgagc caggctctgt gctaagcatg taaaatagat tatctcactt
140820

tccttctggt tcctaaccct ggacttttag ctgccacgtt caatttattt ctaccactgg
140880

aatgacgcac acagtcccca cccttccttg ttgcttctga tgagctgtgt ccctgacagg
140940

cagtgggcaa ctcaggacag gctgatggga ccttagaacc tctttgtgat tcttgaggct
141000

gaggtagcag gagggtggat gttaataaca acagagcagg gaacagtagg gaaacccatg
141060

aagggaatgg caagttcctg tggtgggcat ttgacggctt ggtctcctca ggcttctctc
141120

atccttttct gttttttgtt ttttgttttt tgtttttgag acagggtctc actgtgccac
141180

ccaggctgga gtgcaatggc gtgatcttgg ctcactgcaa cctctgcctc ctgggttcaa
141240

gcgattctcc tgcctccaag cgattctctt gcctcagcct cctgagtagc tgggactaca
141300

ggcatgtgcc accacgcccg gctaattttt gtatttttag tagagacggg gtttcaccat
141360

gttggccagg ctggtctcaa acccctgacg tcatgatcca cctgccttgg cctcccaaag
141420

tactgggatt acaggcatga gccaccactg ccggccttct catccttttc ttaaaacagc
141480

tccaccctct gaccatagag gtagacacgt gactcaagct tggccaatcc tggtacccca
141540

ctcctctggc cacagtgatt ggtccaggaa tgggcatatg acccaagcct ggccaatcag
141600

gatccttcct tgagattttt ctatgtgcag tggatgggaa agagtttatt ttcctctcta
141660

ggatagaata taaatccttg agcttccctg ggttccagcc ttgtgggaac acttagcctg
141720

gaagaataat gccaacaagc agcgcaaagc agagactagg gacagaaagg atcttcaagg
141780

gatttgatca gtggatcaac ttgggcctga ggcctgatcc acccttccct ggtttgagtg
141840

aggattctgt catttgcagc caatgagtct ggctaaaaca cttttctctc cactttgcct
141900

tggcagaggc ctccctgaga tctgagtttg aagaggtcac aggggaagaa agcaagagtc
141960

ctgatgcctc aggggctcca gggaagctct aggaccttct ctgctatttc cactgaagct
142020

gcctcacttc accaaaggca cttggcagtg cccacaatgt caggcgcagg gcaggctccc
142080

caagaccagg gagcccccaa ggatgccttt actcaggcct tcttctctgc tggtgacagt
142140

acaatgtaat gaactcatag atgtcacaag gtaaatcccc ttcgatctgg ggggctgatg
142200

tctccccctg ggcaagcaat gatacttgga aagtcccccc acaccctcag ctccccgtga
142260

aacaagacac aggccagccc acctccttct ctgcccgcct ctagccccat gactttctgg
142320

aacagcttgt tccctgtccc tgtttccctc ttgacttaca caggctgctt ctcgcacttg
142380

ctcacacagg cgcttggcag ggatcaggaa atccagaagg aagctcagcc catccccctg
142440

gaagccagag tccaggagac ggaacacctg acccaggacg gtgggtgctg tggcctcgaa
142500

aggaggatag aggcccgcca gggcatgctg gatggctgtg tcgagggatg gagggtcctg
142560

tgggggacag gaggaggggg tgataatagc aatgataata agggcagaga ccacttcccg
142620

tgaccttcca ggccccatat gcctaaggcc cctgctaatt ctctgacctc ctctcctacc
142680

ccacccccct cactcatcac tgccagccac actggccttc tttcctgtcc ctcaaacaca
142740

ctaggcacaa tctcacctcg gggcctttgc actggctgtt ccctctgcct agaatgctct
142800

tcctcaagac agcctcattc ccacacctct tgcaggtctt tgctcaaatg tcatctgctg
142860

atccacggag acgtggagga ataggggtgg ggggtgggca ttcaggtcag ggtcaaaggg
142920

aaagaggatt ttggcattgc cccagtctga ccgcctggct ggcctgcagg cctgcagcct
142980

gaagctgagc cacgagggaa gcccagccca gaccagacag ccccactcca catgcagatg
143040

ggagagtgag aataagtgat tgctgttttg ccactgagct ttggggtcat tttcacacaa
143100

gcaataactg atacaagtcc acggctgcag actgtacagc cagctgcctc ccatggacct
143160

cctagatgcc tctcagccac caaccgccaa catatcagac ccaacctggc aacttcccaa
143220

acctgctcct cccccagtcc tacctcggtg atgaccccac cgcccagtca ggctcctagg
143280

ccagaaatct gctcacagca ctgcttctcc tcctgcctcc caacccacaa tcagccctca
143340

gtgtgtccca caccacttcc cctccaccaa cagctctcca gccaggtgct ccgcgctgtc
143400

ccctgccccc gccctgatcc aggcttcacc gcctcttcca ggaagggatc cagaaggcag
143460

tcagcccgtc cccctggcag tcagagtcca gaagagggaa aactgggccc aggacccagg
143520

atcacctgac tcctctcttt ggctgccaaa ttctcgtctg cctcacccca gccattttcc
143580

accaggaccc cagagcgacc tttctcaaat gcaaaccaga cctcatcagt ctctgctcca
143640

aactcctcaa aggttcccac cgtcttcagg aagcatcagc ctccaggcat ccagctcccc
143700

tgagatctgg cagctgccct ccgcccccgc ctcccctcca gcctcgccac ccctctccat
143760

gcttcggcca cagtggcaca tgccttccct tgctcacctc gaggccaccg cacatgcagt
143820

ggcttcctgc tggaatgccc ttcacttcca cccctcctca cctggccaac tcctcattca
143880

agactcaggt cagctgtcaa ctcctccagg agattttctt gggtgccccc aactacccca
143940

gggagatcag gtgtcccctt ttctgggatc ctatagtacc cctattacag cggttcccat
144000

ctaaaggcag gtgtaatggg tagaatgacc tccaatgacg ccctcatcct aatctctgga
144060

acctgtgaac gtgctgttac aagacaaagg ggagttaagg ctaccaatgg aattaagttt
144120

gctaatcagc tgactttaaa acaaggagac catcctggat tagacaaatg ggcccagtgt
144180

aatcaccagg tggaagagga tgcagaagag ggaagggtaa gaggaaatgt gactatgggc
144240

caggggtggt ggctcacacc tgtaatccca gcactctggg aagctgaggt gggaggatcg
144300

cctgaggcta ggaatttgag accagcctgg gcaacatagt gagatcccat ctcttattac
144360

attaaaaatc gtaaataggc tgggtgtggt ggctcatgcc tgtatcccag cactttggga
144420

ggctgaggtg ggtggatccc ttgaggtcag gagttcaagg ccagcctggc caacatagca
144480

aaaccccatc tttactaaaa atacaaaaaa gtagcccggt gtggtgatgc atgcctgtaa
144540

tcccagctac tcgggaggcc aaggcatgag aattgcttga acctgggaga tggaggtttc
144600

agtgagccaa gatggcgcca ctgcactcca gcctaggcga cagagcaata ataaataaaa
144660

taaaataata aataaatagc ggaaatatga caacggaagc aggatcagag agatgtgatg
144720

ttgctggctt ggaacataga gcgggagcca aggaatgcgg gcgtctccta gaagctggag
144780

aaggcaagca aatggattct cccctagagc ctccagaaga gaaatagccc tgccaagact
144840

ttgtttttgg cccagtgaaa tgcatgtcag gcctctgatc ttttattacc tgcaaggtac
144900

tatacttgtg tagcttgaaa ctaaagtgca tgatcatttg ctacggtggc aacaggaggc
144960

aggtacagta agcactggct tcttttcctc tttggggcct cggtgtctgc acaggaccag
145020

gcacaaacag gtgcttcata aattttatgg cagaagaaac aaaatgtccc tctctgaatc
145080

acaatttttc ttttcctttt cttgtcttct ctttattatt attattatta ttattattat
145140

tatttttgag acagagtctt gctctgttgc ccaggctgga gtgcagtgtt ccgatctctg
145200

cttgctgcag cctccgcctt ctgggttgaa gcgattctcc tgcctcagcc cgagtagctg
145260

gaattacagg gatgagccac cgtgcttggc ctttttgttt gtttgtttgt ttgtttgttt
145320

ttaaagacaa gatcttgctc tgttgcccag actggagtgc agtggtgcta tctcggctca
145380

ctgcagtctt gacttcctgg gctccagcaa tccttccacc tcagcctccc gagtagctgt
145440

ggaccacagg cacccaccac catgcctagc taatttttgt atttttggta gagatggggt
145500

ttcaccatgt tgcccaggct ggtcttgaac tcctgagctc aagctagcca cccgtctcgc
145560

cttcccaaaa tgctgggatt agaggcatga accaccacgc ctggtcagaa ccaccatttt
145620

tctgtcaaat agacaagttg gactaggcca gaggtttcca cccaggtatg gtcttaggac
145680

ccataggtca tgagcctcac gggggccctt ctggttgccc cagcctggtt gcaagctcca
145740

ggtccacctg ttgcccaggg gggcaaaggg agatgagaat aaataaggac cccactcaaa
145800

gggagcttac ttaaggaggg aaaaatcatt ttagtaaatg aggcccctga agaaagagaa
145860

atacaatagg aactctgagg aggaggaggg tggcatttct caatgccagc aagattccaa
145920

tgccaggagg cttttacaga ggactgaggc tccctcccac ccgtagccca cacagtgctg
145980

ggcgcagggg accgccatca ctatttgtag aatgaatgag tgaatgaatg agtgtccaca
146040

gcccattgtg atggaaacag ggtccgggca ctgccgtggc agtgagctca gagaggatga
146100

tggaccgaca ggcatctggt gctgaaatca catgtaccca ggaggtttta cactgaggtg
146160

gagggagggg ccttcaactt ggcagaacag ggaaggtctc atctggcccg gtgtcctctt
146220

ctaatccccc cccaaccccc accaatcatg gtcaaactct gacatttccc accccatgca
146280

atcctgggag cagtcccttc ccctgccagg tgccctcccc actccctccc tcctctctcc
146340

tgagacttgg gctctggcct ctctgttgag acgttggctc cagcctctct gtgcagcctt
146400

gagtaggtcc tttccctctc tgggcctctt tccttgtgta aagaaagcat attgagctgg
146460

gctaagcttg atttgttttg gttttggaaa tttaatcatt catgatgact tttatgatgt
146520

ttagcatctc ctcataccat gcatactatt agcacttact atttttcttc aaatccactc
146580

actttttcaa aacttacata attgtgtttt tcaatgagac tctgtatcat cacagtggtt
146640

atgcaagtgc agccagactg cccgggttca aactctggtt ctgtcactta ctggttgtgt
146700

gtacctggca agtcagattt aactcgttgt gcctcagttt cctcatctgt aaaatggaga
146760

ctgtaattac acccacctta aggagttgat caagattggg tgtattgagg taatacatgc
146820

gaattctctg cacaggacta gaatttagta ggtgctcatt aagtaagagc catcactaca
146880

atttctgtgg tcatattatt attattatta ttattatggt gtgtgtgtgt gtgtgtgtgt
146940

gtctgtgtgt gtgtgtgtgt gtgtgtggtt ttaaggagca gaaagtttaa taggcaagaa
147000

agaaagaagg aagaagaaaa cagttcccct gtatggagac agagggagga ggattctaac
147060

aaagagaaaa ccccgtgtgc tgcagaaaag tggctgcttc tacgaggagg ttggaggaga
147120

tggtgtctga tttgcatagg gctcaaggga ttggtttgac caggcatgtc actcatgtag
147180

cccacgaaaa aactggccct actaccctag ctttttaata tgcaaatgca gggcgccatg
147240

atgttctaca caggtgggga tatgtggggg cggccatgtt accaggcaca tgtgggggca
147300

agggcaagaa gaagaaggta ggaatcacca tgtttgggtg gatctgtggc aatattataa
147360

ctgttattac atcatcacta taaatggaga aataactgta aactggcata aatcgaatgt
147420

aactccaaaa ataaactgat gatgcattgg atggtttttg tattatttca tttattaaaa
147480

tatatttaaa tatattactt atataaagta tattttttgt ttactgtttg tatttctgtg
147540

aaaataaact actttaaaaa ataaaaataa acaatgaaaa cgaatagtac cattaaattc
147600

tagggacatc tggtcacctc ttggagccta agggggtctt ctccctctct ttctcccaaa
147660

tgcatcagag agatgttaaa gacatcttat ccccagagga agtctttctc cttaggtctg
147720

gagaagctca gggaatttga aagggactga ttgtccatca catggttcca tgtcatttag
147780

ttccagtctg tgacatacct tgtgtctaac caggcccgca ttcctggccc ctatactatg
147840

ctcatgacct tctggactga cccagggaac cttgtagtgc agagggacat agaaaactac
147900

ctgctcacag tggttatacc tgggcaatgg agtgggggtg gggggatgtg tatacttcta
147960

tttcaatgtc tttgcaatga gccactgcta ttatagtctt tttttttttt ttttgagaca
148020

gagtctcact ttgtcaccca ggctggagta cagtgatgca atcttggctc actgcaacct
148080

ccgcctcccg ggctcaagtg attcttctgc ttcagcctcc catgtagctg ggattatagg
148140

cacccaccac cgcgcccagc taatttttgt agttttagta gagatgcagt ttcaccatgt
148200

tggccaagct ggtcttgaac tcctgacctc aggtgatccg ccctcctcgg cctcctgaag
148260

tgctgagatt acaggcatga gacactgtgc ctggccccat tataatctta agtatcattt
148320

tcgtcaagta aaataaacaa atttaaaggg cacaggttga tgccttttta acctatgtat
148380

gagcccctgt aaccacttac ccagatcaag agacaaaaga gctccatact ccagagagag
148440

gccccttgcc agttgtctat gtcatcactg tggtctgcat cctaacctca gggaaacagt
148500

gagttaaact atctttccct gatgtgcctc agtttcccct tctgcacata gatgggattc
148560

tgattcctat cctacctcac tcagatgatt gttctagaca cacaaggagc taattaatgg
148620

atgtacaggg tcttggggaa agtcaaaggc atggccgctg ctgttattgc tattcattct
148680

tacttatttt cactcatcct gtcagcccaa gcttaagtcc cagcctctgt gtgaagcatc
148740

ccccatctcc tggtccctcc ctgtgtccac tactgtcacc ccaatgataa caacagtctc
148800

caggcggcta ctgtgcccag gtcccctact tgcattctct tgcttagccc acacaacgat
148860

gccaggagcc aagtaggaat gacccgtttt gcaagtcaag aaaccgaggc tgatcttgcc
148920

caagaccgca cagaagagaa agacagagcc aggatccaat ccagattggg gcctgccaag
148980

tctgcccaga tcttcccctg tttcctgccg ccccctctgc ctggggacca ccagctgtgg
149040

ggactgacga tcccagctgc ctgtccttcc cagtttctag tagagcctga agaaactgtt
149100

tacctctcac agagcaaggc tcagagaccg ggacaccctt agctttcaca aattggcttg
149160

gtttgccaca cactgttacc atttaagggg cagccagtaa aaaggaagta tgttccgagt
149220

taacagagct ccgagctctt ggccagcaca ttacccacta caggcctgaa ccccaggggc
149280

tcataagcca tccaagtatt ggaggaagca cactgtggtg ggtgtggggg tgcccaggcg
149340

gctgcaggaa ggaacgctag ccactctcat tcccaatcca cccggcagcc cagcctggga
149400

ccacaggaac ccaaacacca gacgtgggcc cctcagcatg atttccatga aacttaatag
149460

atgtgtttcc attccttccc agtgaacaca gcccgcagcc cggcaggagc caggctgttc
149520

ccaggcagga gccagcgggt gtgaggctga aaaggaagaa ggtgagacga gccacagccc
149580

cctcccctgc agcccaaggt cagagaagga gctcatcaag gggacaggca gaaccaggcc
149640

cgcactgctg gctcctatac catgctcacg accttctgga ctgacccagg gaagcttgca
149700

ggttaggagg gactacttgc tcacagcggt tataacctgg gcaggggagt aggggcgtgc
149760

gtatacttcc atttcaatgt ctttgcaatg agctgctatt attataatct taaataccat
149820

ttttatcaag taaaataaac aaatataaaa ggcataggat gatgcctttt tacctatgta
149880

tgaaccctgt aaccacttac ccagatcaag agacaaaaga cctccaccac tccagaaaga
149940

ggccccttgc cagccaactc tcccacccct gactattgga atttttttta aagaaaacag
150000

tgtcataccg tatgatctat agtatgaata aaaccatgta agcctagaaa aaactccaag
150060

tggacattaa gtaaagtgtt aactgcagtt acccctgagg gctgtgcggg gcaggggcct
150120

cgtaaggcag ccttttaatt ttcttcatag tttcccggat tttccaaaca ttccgtaacc
150180

agcttgtata gcttttataa ttaggagggg aaaaaataaa agtgtgagag tatgtgtttt
150240

taaggctggt tccctttgta aatggtctca ccagatgttg ttagctggac tttctgagat
150300

gtggattaag cttttctttt cttttctttt caagaaagag ttttgctctt gttgcccagg
150360

ctggagtgca atggtgccat ctcgactcac tgcaacctct gcctcctggg ttcaagcgat
150420

tctcctgcct cagcctccca ggtagctggg attacgggca cctgccaaca cgcccagcta
150480

attttttgta tttttagtag agacagggtt tcatcatgtt ggccaggctg gtctaaaact
150540

cctgacctca ggtgatccac ccaccttggc ctcccaaagt gctgggatta cagtcatgag
150600

ccactgcacc cagctggact gagcttttct taaaggtcca gatttatcca tttactgcaa
150660

cgggcaaaga ttttagtatc acttagcatc tgccgcctcc ttacttggga agagaggact
150720

gagaaaagcc aaaatggaca gagtcctagg gctaccttca agctctgccc tgttccttgg
150780

ggaaaaaaaa aatgcctagg gagggtgaga ttatgcccct gactctctgg gtgatcctgg
150840

cctcagtttc cccacctata aaagggaggc actaagcata tctatctcca agggcccttc
150900

cagtaggggc attctagaat tcaaagtata atcaggggcc gggtgcagtg gctcatgcct
150960

ataatcccag cactttggga ggctgagaca ggaggatctc ttggggccag aagttcgaga
151020

ctagcctggc caacatagtg acagcctgtc tctataaaaa ataggaaaac ttagccaggc
151080

atggtagctc atggtagtcc cagttacttt aggcactgag acagaaggat cgcttaagcc
151140

caggaggtgg agactgcagt aaactatgat cacaccactg tactccagcc tgggcagagc
151200

gagtccccaa ctccaaaaaa aaaaaaaagt ataatctatt tcaggaaggt ttggagctgg
151260

gatgtggttt ctgaatggga tcagttaatc ctcagttggt gagaaggttt aaaaagatct
151320

gtcttggctg ggcacggtgg ctcatgcctg taatcccagt actttgggag gctgaggcag
151380

gcagatcacc tgatgtcagg agttcgagac cagcctggcc aacatagtgc aaccctgtct
151440

ctactaaaaa tacaaaaact agccgggtgt ggtggcgggt gctgaaaatc acagctactc
151500

agaaggccga ggcaggagaa ttgcttgaaa ctgggaggca gaagctgcag tgagcagaga
151560

ttgggccact gcaccccagc ctgggtgaca gagtgagatc tgtctcaaaa aaaaaaaaaa
151620

tctgtctctt ccatcttcct gcctcaggcc tagggagagt cagtctaaac caacagccac
151680

aggtataaat aagtgcttcc aggggcctga caggtaacat gaagacagac actggctgag
151740

tgtgagaaag cactcacttc caccctttgg agatagtcag cctcagcacc agggaagcaa
151800

tggggaatag aggggcttgg ggcacacacc ccctctcatc agcatcagct gagtgttgct
151860

ttggcccagc accacctgat ctactgattt ttcagtaaaa cctcccaatt ttgaaaaagt
151920

tggccaagcc aaacacatct gtgagccaga tacggcccag cgggtgtcag gttgtaattt
151980

ccagtctcaa tgacctggga cactcgtcct gaacttctcc aggctggaga ttctagaacc
152040

agcattctgt ggctctggct gccaggaagt tcttgctcac ttccaactaa aacttctgct
152100

ttaagtcctt tttatttttg tgtgggtgga ggtatgagtg agtattggct cgtataaaaa
152160

aactttcaga aactgggaaa acagccctga tgcagtgagc cgagaagcaa gtggctgtca
152220

gaagacccac gttatagtcc agctctgtct gtatcatctg catgacctcg accccctgcc
152280

cctcactgag ccttagtttt gtatttgttc ctttctctcc ctctcccctt ttcctctgcc
152340

ttgggctcac catctcccac ccaaaccaca tcatgatcac aattgtaacc agagctgctt
152400

cctactgtct tgtgtgttag gcccctgcta agcatttttc attaactatt ttatctcaat
152460

aattctatgg gctgggtatt tttattccca ttttacaagt taagaaactg agctcaaggc
152520

tctactgcta ctaaatggta gcactggggt atgaccctgg gcacataacc accagctgtc
152580

ctgtctcttt ccccgggctc cctgctacca cgccaggctc ctcttagtca tcatagtggg
152640

cttcctaaca cagagatcca actgtgaccc tccactgctc aacaacctcg ggtggctccc
152700

tattgcctta gcaactcact ggtctggtgc tgtggcccgt cagtcagctc ccagcttacc
152760

ttccctactt cttctaaaca tactttatgc tgtagcccaa ggctttccaa tcatcatggg
152820

cccctttcat gaggttcttt ctgccatgtt cacatattct aatttgttat tggcttactc
152880

ttttttatta attattattt acttaacgtt tgactttttg tcaattcttt ttcaacttaa
152940

atctattttt tggctgggtg tggcgactca tgcctataat cccagcactt ttgggaggcc
153000

aaggcaggag aatcgcttga gcccaggagt tcaagaccag cctaggcaac agagggaggc
153060

cctatctcta caaaaaataa aaagttagct gagcatggta gcacgcacct gtagtcctag
153120

ctgcttggga ggctgaggtg ggaggattgc ttgagcctgg gagatagagg ctgcagtgag
153180

ctatgatcat accactgtac tccatccagc ccaggtgatg gagcgagacc ctgtctcaaa
153240

acaaaaaatt atttatattt attattatgt gtttgtgctg tttaaattcc ttcctaatac
153300

tcattaagat gaaaagctgt aaactaaaaa ttgttcatct aagtgccatc taaaatctcc
153360

cctcctggaa cctttggtgg atgccacatc ttggaaagtg ctgcctggac acacatctgt
153420

gtctctgctt ccactgcccc actccctgga ctgagcaacc tcctgcccac ccttcagagc
153480

ccttccagac ttgcttctct tctctttgct tttcaggaca catgtgcttc caggcagaag
153540

gatggacccc caccacccat cagacacagc ctagaattat ctgtggatga acctgccttc
153600

tccatggact gccagccccc aaagcaggaa ccacgtcctt atagagggcc tggcacacag
153660

tagggaactg gggtggggta ctgccatgca gcaggtatgt gggcaatgtt gaatggatta
153720

aactgaactt ggctagggtg gtggctcatg cctgtaaacc gagcactttg ggaggttgag
153780

gtgggaggat cacttgaggt caggagtttg agaccagact ggccaacatg gtaaaacccc
153840

atctctacta aaaatgcaaa aattagctgg gcctagtgtc acacacctat agtcccagct
153900

acgcaggagg ctgaggcatg agaattgctt aaacctggga ggcagagatt gcagtgagcc
153960

aagacagtcc cactgcacta cagcctgggc aatagagcga gactccatct aaaaaaaaaa
154020

aaaaaaaaaa aacctgaacg tgaccagttt ttctcacagg tggcccacaa aacaattttg
154080

ggagaacata gattgatgtg aaatcacagc gagaaagtta ctccagttct cttccattcc
154140

tcacgatgac accaaggaca atacctcagt ctggagccag tctatctcga agacctctca
154200

ggcacttact catcactctg tttaacaaaa gaaaacagtc ctcgggcttg aaattgttca
154260

ccaccaacct catccagcca gaatttacta acacagattt gccttcattg agtacatatt
154320

tttatagtta cttttttttt tttttttgag acaaagtctc gctcttgtcc tccaggctgg
154380

agtgcaatgg tgcgatctcg gctcactgca acctccgcct ccggggttca agtgattctc
154440

ctgcctcagc ctcccgagta gctgggatta caggcacctg ccaccatgcc cggctagttt
154500

tttctgtttt cagtagagac ggggtttcac catgttggcc aggcaggtct tgaactcctg
154560

acctctggtg atccgccagc ctcggcctcc caaagtgctg agattacagg cgtgagccac
154620

tgcgcccggc ttagttactt tctatttatg acaatggata ctgatttttt catggttgtg
154680

atataaaatt ttctatttaa atatattttt cagtgtacaa agtaagttga tttaaagaaa
154740

aatattgagc aaataataag tagtagattt tatcattttc ttatatattt tatccagtaa
154800

gtggatgtgt aaaagttgtt tcagtgatac taacatttgg gaaccatggg acaggatcaa
154860

ccctaagccc ctttgccata agattctctg acatgggggt gcccccaaac actagacagc
154920

tgttaatcaa atgccccaca aaaattacag ctatgatggg ttatcataaa ggaaagatac
154980

atgacatgct agagtaagag tcagggaaat cagggaaggc tttcctgaag aggtgacatt
155040

tgggtgaaga tttaccgggt aaataagaat tcattaggtg aaggtattcc tccaagcagg
155100

aacagcatgt gcaaaggccc tggggcagca gggagcgtga accataagag gctcgcaagc
155160

aaggctggag tggctggagc acagaagtga ggccagggag gtaggctggc aacaaccttt
155220

attctttaaa aaacaggcaa gggaaggaaa tctgcacatt gttcccaatc tggaaagagt
155280

tgctcaccct ttgacagggg gcagaccagt gcctgaccct tgatctgagg atctggggtt
155340

caatgagggg tttctaatga ctaggttagg tgtgaaagca ggagaggctt ggcaaacacc
155400

aggtgcacgc tgtggccctg gagtgaccat gaccgccgag tgcaggctca gaattcagac
155460

tgtcctaact tgcactttct gggtggggtg accttgggca gctacttaag ttcccagagc
155520

ctgaggtccc catctgttgg agatagccgc aggtctaatc ttagagttgg tatgagttac
155580

tgactcctgg atgtaaatca cctagcacag ggccttgcct gtagggagag gtgaataacc
155640

actggcttat gagagtattt tcaaacagct ccaagagcca tgtgtgtgta cacctgtccc
155700

cgcagtgcat ggatgtaggt gcccggtttt gcaatatgtc tgtggtgtgt atgccaatgt
155760

atgcccatag tttagcagta tgggggagca ggtgcagaag gcatgggtgt atgagagggt
155820

gtgaacatgt gttcagctgt gtgagtcagt gggagcattc aggtgtgctc tgtgcgtgca
155880

tgtgtatgtg tgtgtgcatg tgtacgtgtg tgtgcatgtg tacgtgtgtg tgagtgcctg
155940

cgtgtgtgtg tgtgtgtgtg tgagagagag agagagagag tatgaacacc tctatatgga
156000

attcttctgc ttgtggagat gcatttgctg tgagtgaaaa cccacacaaa ttcaacatgg
156060

ttggtcacag tcacagggtt aaagccttgg cctgtcttca cagctttttt caaaatcctt
156120

acagcccctg tgaatctgcc tggggctctt gtatctgtct ggagctgggc tgaaatcctc
156180

caaaaacaga cttttctttc attccctgtt gccagatact ggcacaaacg catccctgcc
156240

accaccacca ccacacacac acacacacac acacacacac acacattctc ccagaatgtc
156300

tcaacttttt tcaaggttcc agctccaaaa gtcccttcct cttccctctc cccagccaat
156360

tcccatggaa gcaccctggg tttcaatgtg tatatctgac tttccatagc tgagtctaaa
156420

acagcaaaag catcccaagt tttggtaagc agttctctcc ttttcaccct ggacccttaa
156480

ccctcctcgc cctgcactgc ctggctggcc tgggagggga ctttaacccc caccaccact
156540

cttccccatc caaactccct gccgggacaa tgtgcatatc acagtcagtt ccaaaacaaa
156600

catgcaaaac caccccgttt cagccctcag cctcagggct gagattcagg ctcaggtcta
156660

ggggttgggg aatattaaag tttacaagta caatcttact ccatccatac acacacatgg
156720

agaagccagg agaggacgcg gccagagatg ggctcctgcc cttgaacccc tgctggggtg
156780

gaagcaggta agcacaaccc tgccaaggca ctgagggtcg tctcagtgta aaaggccagg
156840

gccacccggg gctgttctgc agggaatggg gagggctgtg cccactgaaa gggggcagag
156900

cgaggtggtc caggccttgg cgcgttacca tggtgacggg ctgccagcgg cgggcggcgc
156960

ggctcctctc ctgggcgcgg ggtctgtggg tccgcgggtc cgtctgtctg gggcagccct
157020

cggtcccgcc tagccctgga gccaggggat agggcaggcc cggggcaccg accccggcgt
157080

catctcggag gagcggccgg ggaggacagg gagagagact gagagagaga cagagaggga
157140

ctgaggctgg agacggcgag agacataagg gagccgcggg cggcgcgggt cgggccacca
157200

gacggggggc ggcagcagag gcggggcggg gcggggcgcg gcgggacagc ggcccctttg
157260

tgcctgagcc ctttgtgccc agcccgcgcg ccccgccccc accgcccccc cttccctccg
157320

tccaccccct cagcagccag ctggaacccg aggaacccga gtccgccccg ccaagttttc
157380

ctccccgctc ctcgctcttc ccacggcgca cccggcccgg acagccccat tgtacagacc
157440

cgcagagcga ggcctcgagc cgcagcagcc gggttaagcg cccgcggacc tcgctggcag
157500

gcccaagttc gagtctcctc ctcctacact tcccagggag tcacagactc tcttaagctt
157560

cgatttcttc atctgtaaaa tggggtggga ggaggatgat gatgatgatg atgatgatga
157620

tgatgatgat gatagtgcct gctgcttaga tttggtgatg aggagtaagc atctgtccca
157680

gcgcccttga attctcccac attatgcccc atcatgacgg cctaattcaa ggctttgagg
157740

tgatgatggc tgtgtgcgag ttctgttctt gactctcaga taatgacctt ggccaagtcg
157800

gtatttccta ctgagcctca gctttggagt gagactaaga aactagatga ccgctagggt
157860

ctatatgtgt gtgtcccctc ccaaattcgt atgtccagac ctaatcctca gtatgatggc
157920

attaggaggt ggggcctttg gaaagtgatt gggtcatgag aggacagagc cctcacgaat
157980

gggattagtg cctctctcta tatatatata tacacctaca tatatatacc tatatatata
158040

catatataca cctatatata tacatatata cacctatata tatacatata taggcactaa
158100

tatgtatagg catatataca cacacatata tatacacaca catatatata cacacatata
158160

tacatatata tacacacaca cgcacacaca tatatatttg agatggagtc tcactctgtt
158220

gcccacccaa gctggagtgc agtggcatga tctcagctca ctgcaacctc tacctcccag
158280

gttcaagcaa ttttcctgcc tcagcctccc gagcagctgg gattttaggc acccgccacc
158340

acacctggcc aatttttgta tttttagtag agactgcatt tcgccatgtt ggccaggctg
158400

gtctcgaact cctgacctca ggtgatctgc ccacctcacc ctcctaaagt gctgggatta
158460

caggcgtgag ccaccatgcc cagcttagtg cccaagaggc cccagagagc tagctgcctt
158520

gccccttcca ccatggaagg acacagcgag aaggggctgt ctatgaacca gaaaggccca
158580

gaaacataag gtccagaaag aaggctctta ccaaacaccc aacttgcttg tgccttgatc
158640

tagggcttcc cagcctccag aactgtgaga agagaatttc tgttgtgtat aagcccctca
158700

gtctaaggta tgttgttata gcagcccaga ctaaaacagt gaccaaagtt attcctggtt
158760

agatggctca gggttttgtc catttattta tcaaacactg tgcactgagt cagcctgtct
158820

cccccactag actatacatt acttgagagt cccggccctc aagtaatgta cagtctagtg
158880

ggggagacag gcaaggagca gataaacagg aaaaataaaa aaataataac tgtggtaact
158940

gctaagaagg aaacaaacaa atgttgacat tgggaacagt agagggatca acttatagct
159000

ggtggtctag gaagcctggg tctgaaagcc tagatctgaa aggggaggaa ttagaagagt
159060

ggagatgggg agacgcagag gaaccaagtt ctaagcagag agaaccgcat atgcaaaggc
159120

tctgaggcaa tattaagctt aaaggagctg gggagtaagt gaagaactga aaaagacgag
159180

tgtagttgta agggttagag ttcagaccca agccttattc acagcccatc tcccacccca
159240

cataggttaa tcagtctttt agcagagaaa acctgaatta ggagccatcc tatcccaact
159300

tttgataagt tccttcacat ccccgacctt cacttgctta tcactaattt gggactaata
159360

atcccctgca atatttccac agcccagcaa tcaagtaaac tctctctaca ggagatggtc
159420

tggatggttt tagtatcatg actccagatg tgaatttagg agagaacaga cttgatgttc
159480

agtacagaat tccactgaga gttgtggggt cacagcttga aggttttaga gtcccaggtt
159540

gccatgagac agaattgaca tgacagccag cgttctgggg ttgcaggcaa cagaagcgga
159600

ttctgagtta attaagcaga aaagaatgga ttggcagtca gagaatcaat gcgaaggctg
159660

gggcctatcc tctggaaagc actggagtaa gtaggatgct ctggggggtc tcgttatcgg
159720

aaagagttgg aaacgtttga acacatggaa ttacattaag acaaacaaac acaccaaagt
159780

agaactttat atgtgtcagg tagttttcta agtatataac agttatgaag tagattctat
159840

tattgtcctc actttacaaa tgaaaaacta aggcacagag agggtatgta gcctgcccaa
159900

tgtcagatag ctagtaaact gtggatttca gaattggaca cagctatcca attccgtaag
159960

ttatgttatt aaccactttg ctatgctacc tgcataaatc ctaaccattc tctctgccct
160020

tgtgttcctc actgatggac tcagaacctt aaaagagcca ggcatggtgg catgctcctg
160080

tagtcccagc tacttaggag gccaaggtgg gaggatcact tgagcccagg agttcaaggc
160140

tgcagtgagc tatgattgca ccattgcact ccaacctggg agacagagtg agaccccatc
160200

tctaaaaaac aaacaaacaa aataaaacct ttatagagaa attctaattg cgtcatgtgc
160260

ccagggcctc actaagactg cacctacaca tatcaggaat agttggtcac ccaaaaggaa
160320

attaaattgc ataagaagga gaagaaatga atatgaaatt gtccataaac aagtgcaatt
160380

acagcctggg cacagtggct catgcctgta atcccagcac tttgggaggc cgaggtgggc
160440

agattgcctg agctcaggag tttgagacca gccggggcaa catggcaaaa ccttgtctct
160500

actaaaaatg caaaaaaata accaggcatg gtggcgcaca cctgtaatcc cggctactgg
160560

gaggttgagg caggagaatc acttgaacct gggaggtgga ggttgcagtg agcagagatc
160620

acaccactac actccagcct gggtgacaga gtgagactct gtctcaaaac aacaacaacc
160680

accaccacca ccacaacaac aaaaaggtgc aattacggtc tatccattgg gcctctcaac
160740

atactttata tccttgttca caggcatgtt cttccaaaac tcctctctac tgatgtggta
160800

tagcagttcc atatgcaacc aacaatgtgc acacacacat acacacacac acctctatgg
160860

tttgtagtca tactgtgtgt gacctcaggg aggttcactt ttctgagctt cagaatcgtc
160920

ttttgtaaaa gatgggtaag attcgtcatg gggaagaata aataagttaa tgaatgtaaa
160980

gtgctttgca ctgtgtctgg tacatagaat taacatgcgt catataaaat agtgctgtta
161040

ttccaaaaaa tagggaggag ggaatacttc caaactcatt ctatgaggcc agtattactc
161100

caataccaaa accagacaaa gacacatcta aaaaagaaaa ctacaggcca atatctctga
161160

tgaatatgga tgcaaaactt cttaacaaaa tactagcaaa ctgaattcaa caatacatta
161220

aaaagatcat tcatcatgac taggtgggat ttatctctgg gatgcaagga tagttcaaca
161280

tatgcaaatc aatccattct gttgtgatac atcacatcac atacatcaca tcaacagaat
161340

gaaggacaaa aaccatatgg tcatttcaat cgatattgaa aatgcatttg ataaaagtca
161400

acttccactc acgattaaaa accctcaaaa aaccaggtat agaaggaaca tacctcaaca
161460

taataaaagc catatataac agacccacag ctagtatcat atggaatggg aaaaaactga
161520

aagcctttcc tctaagatct ggaacacaac aaggatgccc actttcgcca ctgttattca
161580

acgtagtact ggaagtccta gctagagcaa tcaggcaaca gaaagaaata cagcgcatcc
161640

aaattggaaa ggaagaagtc aaattatcct tgtttgtcga tgatataatc ttatatttgg
161700

gaaaacctaa agactccacc acaaaactat tagaactgat aaacaaattc aataagattg
161760

caggatacaa aatcaacata tgaaaatcag tagcatttct atatgccaac agtgaacaat
161820

ctgaaaaaga aatttaaaaa taatcccatt taaaatagct aaaaataaaa ttaaatatct
161880

aagaactaat ttaaccaaag aaatacaaga tctctataat gaaaactata aaatataaag
161940

tgaaagaaat tgaagaggac accaaataat ggaaagatac tcccaaagca atatacagat
162000

tcagtgcaat ccctatcaaa atatcattga cattcttcac ggaaatagaa aaaacaatcc
162060

taaaatttat gtggaaccac aaaagaccca gaatagtcaa aactatccta aacaaaaaga
162120

accaaactgg agtaatcaca ttacctgact tcaaattata ctacagagct atagtaacca
162180

aaacagcatg ttactgacat aaaaacaaat acatagaaca atggaacaga atagagaacc
162240

cagaaacaaa tctgcacacc tacagtgaac tcatttttga caaaggtgcc aagaacatac
162300

acaggggaaa agatagtcca ataaatggtg atgggaaaac tggatatcca tatgcagaag
162360

aatgaaacta gacccctatc ttttgccata tacaaaaatc aaatttaaat gaattaaaga
162420

cttgaatcta agactttaaa ctatgaaacc acagcaataa aacattgggg gaactctcca
162480

ggacttcggt ctgggcaaaa tttcttgagt aatacccaac aagcacaggc aaccaaagca
162540

aaaatggaca aatgagatca catcaagtta aaaagctctt gcacagcaaa gcaaacaatc
162600

aacaaagtga aaagacaacc cacagaatgg gagaaaatat ttgcaaacta tccatctgat
162660

gagggattaa caaccagaat atataagaag ctaagacaac tatatagaac aaaatctaat
162720

aatttgattt taaaacatgc aaaaagattt gaatagacgt ttctcaaaag aagatggcat
162780

acaaatggca aacaggtata tgaaaattgc tcaacatcat tgatcatcag agaaatgcaa
162840

atcaaaacta caatgaggta tcatcccacc ccagtgagca tggcttttat ccaaaagaca
162900

ggcaataaca aatgccagcg aggatgtgga gaaaaaggaa cccttttacg ctgttggtgg
162960

gaatgtgaat tagtacaacc actatggaga acagttggga ggttcctcaa aaaactaaat
163020

atagatctac catatgaccc agcaatccca ctactagatc tatacccaaa agaaaggaag
163080

tcagtatatg gaagagatca tctgcactcc catgtttgtt gcaacactct tcacaatagc
163140

caaagtttgg aagcagccta agtgtccatc aacagatgag tggataaaga aaatgtggta
163200

cttacacaca atggaatact gctcagccat aaaaaagaat gagatgttgt tatctgcaac
163260

aacatagatg gaactggagg tcagtatgca aagggaaata agccagacac agaaagacaa
163320

acatcacatg ctacacttat ttgtgagatc taaaaatcaa aacaattcaa cgaatggaga
163380

cagagagtgg aaggatgttt atcagaggct cagaagggta gtggtggggg gtgggggtgg
163440

ggggggttgg ttaataggta caaaaacata gtaagaatga ataagaccta gtatttggta
163500

gcacaacagg ataactatag caaataataa tgtaactgtg cacttcaaaa taactaacgg
163560

tataattgga ttgtttgtaa cacaaaagac aaatgcttga ggggatggat actccattct
163620

ccattatgtg attattatac attgcatgcc tgtgtcaaaa catctgaagt gccccataaa
163680

tatatacacc tactatgtac ccacaaaaat taaaatgaaa aataaaaata gaataaaata
163740

gtgttatgtt aggggccggg agttgtggat tcaccctcca aatacacagg cttaactgtt
163800

ggactggact tggttcatct ctctgggact ttccagtgga tcttccaagg acttcaagca
163860

ttcacttcct tcctctctct ctctggcaaa tgaataactg aatcatgtgc caattgctac
163920

actaaatttt gagaaataac caaatccatc actagtctta catatatggt ggagtgagaa
163980

aatggggaag ggctgtggaa atgggctctg ttactggttc gctgtgcagc tctctgcatg
164040

gggccacagt ttcctcagca taaaatggtg attatataat ctctcctcat gaggctcttg
164100

taagaatcca gtgaaataat gcattgaaag ggtttaacac agtgcttggc atataacagg
164160

ttctcagcaa atggaagctc tgcctgtgat tttgagattt ccaggcctaa gtgccaggac
164220

acctcaccag gggacatgct tacttggagg aagggagttg cctaatttaa gcaggatgtc
164280

atgggattca gaatgcattc attcaatcat ttaatacata tttatttgct cctagtatga
164340

ggatgctaaa tacttggcag gtgtgcatat actcatccct cccataccca tgacagatgt
164400

cactaatcca tcatggaact ttctgctgaa cctagaaact gccccagagt cctcctcaat
164460

agaaagtgca ggcagctgct accaatcaat acatgttgac atgaagacga tgcttattca
164520

ccatctgaag ttcacaatgt atgaggaaat gggctccaga ggcagaagcc tccagagaca
164580

gaaggcttgg gtttgcattt tgccataggt ccttatgata atgtgtaggc actaaaggag
164640

cgtatcagcc ttggagtgga ccactcagat ctcccttcaa gacagagtct gtgagctgca
164700

gatgtcttca ggatctgctt cagcttttga gctgaggcca cctgcttcct gggcagcctg
164760

cagctaaaca cactgtgaat gaggtttcgt tatttctgcc taatatagga tttctctatg
164820

aataatcttt gtgccagagt cccctgttgg gctggccaag accttctcag agctgcgcca
164880

cagtctgaag ctcttgctac ccaatccttc ttcattcccc ctctcctttc ctagtcagac
164940

ctgcgtcaac atccaaaggc tccccttttt tctcctgctc cctctcccca ttatcctttg
165000

caggcattac ccccatgcat ctcttggcat tcttaactct gtctttggcc tctgtttctc
165060

agaggacaca ggtgacctca gcactgagct gcagaagact gggctggaaa ggagtggtcc
165120

tcatcttctc tgatggggag aataggggtg gcacagaggt tattgtgagt gctaggggca
165180

aagagtgacc cagaacaggg aggctgaagg gcacctccct cagcacgaaa ttggccttcc
165240

ctatcaagta ctcttttttt atttttgaga cagaattttt gctcttgttg cccaggctag
165300

agtgcaatgg tgcagtctcg gctcactgca acctccacct cccaggttca agtgattctc
165360

ctgcctcagc ctcccaagta gctgtgatta taggcgccca cgaccacgcc cagctaattt
165420

ttgtattttt agtagagacg gggtttcacc atgttggcca ggctggtctc aaactcctga
165480

cctcaggtga tccactcacc ttggtctctc aaagtgctgg gatgacaggc atgagccacc
165540

gtgcctggcc aaggactctt gattgcaaat gacagaaact caactcaatc tcaaagtaaa
165600

aagggacttt aagcgcctta taatggagaa gtggtgccgg agctctgtca tcattctaat
165660

agcttcttcc ctatgactag agaagttggc ctccaggcct ataaggtctc aagctggcaa
165720

taatagtata aagacatcat caaaatagta atctcaggga agaccctgat tggctttgct
165780

tgggtcacat gtcaattctt gaaccaatca ctgtgaccaa ggtaatgaag atattctgat
165840

tggctggaat agattctatg cccacccctg ggtgggagca catgatgatg gctctaccag
165900

ggctgcatag agagaggaga agctctccaa aacaaacagg gggatgggaa cacaaggtaa
165960

acaacaaaac ctttggtgat cattatccac ttcagaaaca cggagaagga aaaagacaaa
166020

aaagacagct tcttctctca atgacctcct cttttcacag gggaaaatct gatgtttaaa
166080

tgtttcctga tgaggtccta ggatgggctt tggggagtga cctgtgggta ggttagggac
166140

ctcaaacact gtctcaccct ttcctaggat gcccacaatc tcggtgggaa ggatgagatg
166200

gaaataacct atggaaataa aacctcttag agctcctaaa aaagactgaa aacttacctt
166260

gtttaactgt cctgatttat gccagggagc ttcacccagg gaggagtgac tcacgttgtc
166320

acactgtcca gccaggacaa caatctgtta tacttcatct gtccttaaat cggcctcttg
166380

atggagcagc ctgtgctgta taagcggaga ccttgagttc aggcccctct ctacccacta
166440

actccgtgag gcatcttggg ggtttgcctg aattctcaga acctcagttt tctacctgta
166500

aaatggaacc cataacctct gctttgcctg tttcccgtct gctgtgaggc aacaacacac
166560

gcatttgtca cgtctcacta gggatggagg tcataagatg catttattga gtgtccactc
166620

tgtgccagag acagcgtgct ggatgctggc catatcaggg tgaacatgac agacagtccc
166680

tgccttcatg tagctcacag tctagaagag agtcagatct taataatgag tgccttatac
166740

aaatggtggt aaggtcttaa aggggaaaaa agagcacaaa tcaatggaac ccactggagg
166800

tccaggaggg tggccctgag ggcttaagct gggattcaaa gagaaaacat aagaagtcag
166860

ggaaggctgg gctcggtggg tcacacctgt aatcccagct ctttgggagg ctgaggcagg
166920

caaatcacga ggtcaggaga tc
166942

<210> 24
<211> 4539
<212> DNA
<213> Homo sapiens

<400> 24
cggcgcgcac actgctcgct gggccgcggc tcccgggtgt cccaggcccg gccggtgcgc
60

agagcatggc gggtgcgggc ccgaagcggc gcgcgctagc ggcgccggcg gccgaggaga
120

aggaagaggc gcgggagaag atgctggccg ccaagagcgc ggacggctcg gcgccggcag
180

gcgagggcga gggcgtgacc ctgcagcgga acatcacgct gctcaacggc gtggccatca
240

tcgtggggac cattatcggc tcgggcatct tcgtgacgcc cacgggcgtg ctcaaggagg
300

caggctcgcc ggggctggcg ctggtggtgt gggccgcgtg cggcgtcttc tccatcgtgg
360

gcgcgctctg ctacgcggag ctcggcacca ccatctccaa atcgggcggc gactacgcct
420

acatgctgga ggtctacggc tcgctgcccg ccttcctcaa gctctggatc gagctgctca
480

tcatccggcc ttcatcgcag tacatcgtgg ccctggtctt cgccacctac ctgctcaagc
540

cgctcttccc cacctgcccg gtgcccgagg aggcagccaa gctcgtggcc tgcctctgcg
600

tgctgctgct cacggccgtg aactgctaca gcgtgaaggc cgccacccgg gtccaggatg
660

cctttgccgc cgccaagctc ctggccctgg ccctgatcat cctgctgggc ttcgtccaga
720

tcgggaaggg tgatgtgtcc aatctagatc ccaacttctc atttgaaggc accaaactgg
780

atgtggggaa cattgtgctg gcattataca gcggcctctt tgcctatgga ggatggaatt
840

acttgaattt cgtcacagag gaaatgatca acccctacag aaacctgccc ctggccatca
900

tcatctccct gcccatcgtg acgctggtgt acgtgctgac caacctggcc tacttcacca
960

ccctgtccac cgagcagatg ctgtcgtccg aggccgtggc cgtggacttc gggaactatc
1020

acctgggcgt catgtcctgg atcatccccg tcttcgtggg cctgtcctgc ttcggctccg
1080

tcaatgggtc cctgttcaca tcctccaggc tcttcttcgt ggggtcccgg gaaggccacc
1140

tgccctccat cctctccatg atccacccac agctcctcac ccccgtgccg tccctcgtgt
1200

tcacgtgtgt gatgacgctg ctctacgcct tctccaagga catcttctcc gtcatcaact
1260

tcttcagctt cttcaactgg ctctgcgtgg ccctggccat catcggcatg atctggctgc
1320

gccacagaaa gcctgagctt gagcggccca tcaaggtgaa cctggccctg cctgtgttct
1380

tcatcctggc ctgcctcttc ctgatcgccg tctccttctg gaagacaccc gtggagtgtg
1440

gcatcggctt caccatcatc ctcagcgggc tgcccgtcta cttcttcggg gtctggtgga
1500

aaaacaagcc caagtggctc ctccagggca tcttctccac gaccgtcctg tgtcagaagc
1560

tcatgcaggt ggtcccccag gagacatagc caggaggccg agtggctgcc ggaggagcat
1620

gcgcagaggc cagttaaagt agatcacctc ctcgaaccca ctccggttcc ccgcaaccca
1680

cagctcagct gcccatccca gtccctcgcc gtccctccca ggtcgggcag tggaggctgc
1740

tgtgaaaact ctggtacgaa tctcatccct caactgaggg ccagggaccc aggtgtgcct
1800

gtgctcctgc ccaggagcag cttttggtct ccttgggccc tttttccctt ccctcctttg
1860

tttacttata tatatatttt ttttaaactt aaattttggg tcaacttgac accactaaga
1920

tgatttttta aggagctggg ggaaggcagg agccttcctt tctcctgccc caagggccca
1980

gaccctgggc aaacagagct actgagactt ggaacctcat tgctacgaca gacttgcact
2040

gaagccggac agctgcccag acacatgggc ttgtgacatt cgtgaaaacc aaccctgtgg
2100

gcttatgtct ctgccttagg gtttgcagag tggaaactca gccgtagggt ggcactggga
2160

gggggtgggg gatctgggca aggtgggtga ttcctcccag gaggtgcttg aggccccgat
2220

ggactcctga ccataatcct agccccgaga caccatcctg agccagggaa cagccccagg
2280

gttggggggt gccggcatct cccctagctc accaggcctg gcctctgggc agtgtggcct
2340

cttggctatt tctgttccag ttttggaggc tgagttctgg ttcatgcaga caaagccctg
2400

tccttcagtc ttctagaaac agagacaaga aaggcagaca caccgcggcc aggcacccat
2460

gtgggcgccc accctgggct ccacacagca gtgtcccctg ccccagaggt cgcagctacc
2520

ctcagcctcc aatgcattgg cctctgtacc gcccggcagc cccttctggc cggtgctggg
2580

ttcccactcc cggcctaggc acctccccgc tctccctgtc acgctcatgt cctgtcctgg
2640

tcctgatgcc cgttgtctag gagacagagc caagcactgc tcacgtctct gccgcctgcg
2700

tttggaggcc cctgggctct cacccagtcc ccacccgcct gcagagaggg aactagggca
2760

ccccttgttt ctgttgttcc cgtgaatttt tttcgctatg ggaggcagcc gaggcctggc
2820

caatgcggcc cactttcctg agctgtcgct gcctccatgg cagcagccaa ggacccccag
2880

aacaagaaga cccccccgca ggatccctcc tgagctcggg gggctctgcc ttctcaggcc
2940

ccgggcttcc cttctcccca gccagaggtg gagccaagtg gtccagcgtc actccagtgc
3000

tcagctgtgg ctggaggagc tggcctgtgg cacagccctg agtgtcccaa gccgggagcc
3060

aacgaagccg gacacggctt cactgaccag cggctgctca agccgcaagc tctcagcaag
3120

tgcccagtgg agcctgccgc ccccacctgg gcaccgggac cccctcacca tccagtgggc
3180
ccggagaaac ctgatgaaca gtttggggac tcaggaccag atgtccgtct ctcttgcttg
3240
aggaatgaag acctttattc acccctgccc cgttgcttcc cgctgcacat ggacagactt
3300
cacagcgtct gctcatagga cctgcatcct tcctggggac gaattccact cgtccaaggg
3360
acagcccacg gtctggaggc cgaggaccac cagcaggcag gtggactgac tgtgttgggc
3420
aagacctctt ccctctgggc ctgttctctt ggctgcaaat aaggacagca gctggtgccc
3480
cacctgcctg gtgcattgct gtgtgaatcc aggaggcagt ggacatcgta ggcagccacg
3540
gccccgggtc caggagaagt gctccctgga ggcacgcacc actgcttccc actggggccg
3600
gcggggccca cgcacgacgt cagcctctta ccttcccgcc tcggctaggg gtcctcggga
3660
tgccgttctg ttccaacctc ctgctctggg aggtggacat gcctcaagga tacagggagc
3720
cggcggcctc tcgacggcac gcacttgcct gttggctgct gcggctgtgg gcgagcatgg
3780
gggctgccag cgtctgttgt ggaaagtagc tgctagtgaa atggctgggg ccgctggggt
3840
ccgtcttcac actgcgcagg tctcttctgg gcgtctgagc tggggtggga gctcctccgc
3900
agaaggttgg tggggggtcc agtctgtgat ccttggtgct gtgtgcccca ctccagcctg
3960
gggaccccac ttcagaaggt aggggccgtg tcccgcggtg ctgactgagg cctgcttccc
4020
cctccccctc ctgctgtgct ggaattccac agggaccagg gccaccgcag gggactgtct
4080
cagaagactt gatttttccg tccctttttc tccacactcc actgacaaac gtccccagcg
4140
gtttccactt gtgggcttca ggtgttttca agcacaaccc accacaacaa gcaagtgcat
4200
tttcagtcgt tgtgcttttt tgttttgtgc taacgtctta ctaatttaaa gatgctgtcg
4260
gcaccatgtt tatttatttc cagtggtcat gctcagcctt gctgctctgc gtggcgcagg
4320
tgccatgcct gctccctgtc tgtgtcccag ccacgcaggg ccatccactg tgacgtcggc
4380
cgaccaggct ggacaccctc tgccgagtaa tgacgtgtgt ggctgggacc ttctttattc
4440

tgtgttaatg gctaacctgt tacactgggc tgggttgggt agggtgttct ggcttttttg
4500

tggggttttt atttttaaag aaacactcaa tcatcctag
4539

<210> 25
<211> 1850
<212> DNA
<213> Homo sapiens

<400> 25
gaatcgcctg ccacaggtgt ctgcaattga actccaaggt gcagaatggt ttggaaagta
60

gctgtattcc tcagtgtggc cctgggcatt ggtgccgttc ctatagatga tcctgaagat
120

ggaggcaagc actgggcagt gatcgtggca ggttcaaatg gctggtataa ttataggcac
180

caggcagacg cgtgccatgc ctaccagatc attcaccgca atgggattcc tgacgaacag
240

atcgttgtga tgatgtacga tgacattgct tactctgaag acaatcccac tccaggaatt
300

gtgatcaaca ggcccaatgg cacagatgtc tatcagggag tcccgaagga ctacactgga
360

gaggatgtta ccccacaaaa tttccttgct gtgttgagag gcgatgcaga agcagtgaag
420

ggtataggat ccggcaaagt cctgaagagt ggcccccagg atcacgtgtt catttacttc
480

actgaccatg gatctactgg aatactggtt tttcccaatg aagatcttca tgtaaaggac
540

ctgaatgaga ccatccatta catgtacaaa cacaaaatgt accgaaagat ggtgttctac
600

attgaagcct gtgagtctgg gtccatgatg aaccacctgc cggataacat caatgtttat
660

gcaactactg ctgccaaccc cagagagtcg tcctacgcct gttactatga tgagaagagg
720

tccacgtacc tgggggactg gtacagcgtc aactggatgg aagactcgga cgtggaagat
780

ctgactaaag agaccctgca caagcagtac cacctggtaa aatcgcacac caacaccagc
840

cacgtcatgc agtatggaaa caaaacaatc tccaccatga aagtgatgca gtttcagggt
900

atgaaacgca aagccagttc tcccgtcccc ctacctccag tcacacacct tgacctcacc
960

cccagccctg atgtgcctct caccatcatg aaaaggaaac tgatgaacac caatgatctg
1020

gaggagtcca ggcagctcac ggaggagatc cagcggcatc tggatgccag gcacctcatt

1080

gagaagtcag tgcgtaagat cgtctccttg ctggcagcgt ccgaggctga ggtggagcag
1140

ctcctgtccg agagagcccc gctcacgggg cacagctgct acccagaggc cctgctgcac
1200

ttccggaccc actgcttcaa ctggcactcc cccacgtacg agtatgcgtt gagacatttg
1260

tacgtgctgg tcaacctttg tgagaagccg tatccgcttc acaggataaa attgtccatg
1320

gaccacgtgt gccttggtca ctactgaaga gctgcctcct ggaagctttt ccaagtgtga
1380

gcgccccacc gacatgtgtg ctgatcagag actggagagg tggagtgaga agtctccgct
1440

gctcgggccc tcctggggag cccccgctcc agggctcgct ccaggacctt cttcacaaga
1500

tgacttgctc gctgttacct gcttccccag tcttttctga aaaactacaa attagggtgg
1560

gaaaagctct gtattgagaa gggtcatatt tgctttctag gaggtttgtt gttttccctg
1620

ttagttttga ggagcaggaa gctcatgggg gcttctgtag cccctctcaa aaggagtctt
1680

tattctgaga atttgaagct gaaacctctt taaatcttca gaatgatttt attgaagagg
1740

ggcgcaagcc ccaaatggaa aactgttttt agaaaatatg atgatttttg attgcttttg
1800

tatttaattc tgcaggtgtt caagtcttaa aaaataaaga tttataacag
1850


<210> 26
<211> 2856
<212> DNA
<213> Homo sapiens

<400> 26
tagtcgcggg tccccgagtg agcacgccag ggagcaggag accaaacgac gggggtcgga
60

gtcagagtcg cagtgggagt ccccggaccg gagcacgagc ctgagcggga gagcgccgct
120

cgcacgcccg tcgccacccg cgtacccggc gcagccagag ccaccagcgc agcgctgcca
180

tggagcccag cagcaagaag ctgacgggtc gcctcatgct ggctgtggga ggagcagtgc
240

ttggctccct gcagtttggc tacaacactg gagtcatcaa tgccccccag aaggtgatcg
300

aggagttcta caaccagaca tgggtccacc gctatgggga gagcatcctg cccaccacgc
360

tcaccacgct ctggtccctc tcagtggcca tcttttctgt tgggggcatg attggctcct
420

tctctgtggg ccttttcgtt aaccgctttg gccggcggaa ttcaatgctg atgatgaacc
480

tgctggcctt cgtgtccgcc gtgctcatgg gcttctcgaa actgggcaag tcctttgaga
540

tgctgatcct gggccgcttc atcatcggtg tgtactgcgg cctgaccaca ggcttcgtgc
600

ccatgtatgt gggtgaagtg tcacccacag cctttcgtgg ggccctgggc accctgcacc
660

agctgggcat cgtcgtcggc atcctcatcg cccaggtgtt cggcctggac tccatcatgg
720

gcaacaagga cctgtggccc ctgctgctga gcatcatctt catcccggcc ctgctgcagt
780

gcatcgtgct gcccttctgc cccgagagtc cccgcttcct gctcatcaac cgcaacgagg
840

agaaccgggc caagagtgtg ctaaagaagc tgcgcgggac agctgacgtg acccatgacc
900

tgcaggagat gaaggaagag agtcggcaga tgatgcggga gaagaaggtc accatcctgg
960

agctgttccg ctcccccgcc taccgccagc ccatcctcat cgctgtggtg ctgcagctgt
1020

cccagcagct gtctggcatc aacgctgtct tctattactc cacgagcatc ttcgagaagg
1080

cgggggtgca gcagcctgtg tatgccacca ttggctccgg tatcgtcaac acggccttca
1140

ctgtcgtgtc gctgtttgtg gtggagcgag caggccggcg gaccctgcac ctcataggcc
1200

tcgctggcat ggcgggttgt gccatactca tgaccatcgc gctagcactg ctggagcagc
1260

taccctggat gtcctatctg agcatcgtgg ccatctttgg ctttgtggcc ttctttgaag
1320

tgggtcctgg ccccatccca tggttcatcg tggctgaact cttcagccag ggtccacgtc
1380

cagctgccat tgccgttgca ggcttctcca actggacctc aaatttcatt gtgggcatgt
1440

gcttccagta tgtggagcaa ctgtgtggtc cctacgtctt catcatcttc actgtgctcc
1500

tggttctgtt cttcatcttc acctacttca aagttcctga gactaaaggc cggaccttcg
1560

atgagatcgc ttccggcttc cggcaggggg gagccagcca aagtgataag acacccgagg
1620

agctgttcca tcccctgggg gctgattccc aagtgtgagt cgccccagat caccagcccg
1680

gcctgctccc agcagcccta aggatctctc aggagcacag gcagctggat gagacttcca
1740

aacctgacag atgtcagccg agccgggcct ggggctcctt tctccagcca gcaatgatgt
1800

ccagaagaat attcaggact taacggctcc aggattttaa caaaagcaag actgttgctc
1860

aaatctattc agacaagcaa caggttttat aattttttta ttactgattt tgttattttt
1920

atatcagcct gagtctcctg tgcccacatc ccaggcttca ccctgaatgg ttccatgcct
1980

gagggtggag actaagccct gtcgagacac ttgccttctt cacccagcta atctgtaggg
2040

ctggacctat gtcctaagga cacactaatc gaactatgaa ctacaaagct tctatcccag
2100

gaggtggcta tggccacccg ttctgctggc ctggatctcc ccactctagg ggtcaggctc
2160

cattaggatt tgccccttcc catctcttcc tacccaacca ctcaaattaa tctttcttta
2220

cctgagacca gttgggagca ctggagtgca gggaggagag gggaagggcc agtctgggct
2280

gccgggttct agtctccttt gcactgaggg ccacactatt accatgagaa gagggcctgt
2340

gggagcctgc aaactcactg ctcaagaaga catggagact cctgccctgt tgtgtataga
2400

tgcaagatat ttatatatat ttttggttgt caatattaaa tacagacact aagttatagt
2460

atatctggac aagccaactt gtaaatacac cacctcactc ctgttactta cctaaacaga
2520

tataaatggc tggtttttag aaacatggtt ttgaaatgct tgtggattga gggtaggagg
2580

tttggatggg agtgagacag aagtaagtgg ggttgcaacc actgcaacgg cttagacttc
2640

gactcaggat ccagtccctt acacgtacct ctcatcagtg tcctcttgct caaaaatctg
2700

tttgatccct gttacccaga gaatatatac attctttatc ttgacattca aggcatttct
2760

atcacatatt tgatagttgg tgttcaaaaa aacactagtt ttgtgccagc cgtgatgctc
2820

aggcttgaaa tcgcattatt ttgaatgtga agggaa
2856

<210> 27
<211> 2856
<212> DNA
<213> Homo sapiens

<400> 27
tagtcgcggg tccccgagtg agcacgccag ggagcaggag accaaacgac gggggtcgga
60

gtcagagtcg cagtgggagt ccccggaccg gagcacgagc ctgagcggga gagcgccgct
120

cgcacgcccg tcgccacccg cgtacccggc gcagccagag ccaccagcgc agcgctgcca
180

tggagcccag cagcaagaag ctgacgggtc gcctcatgct ggctgtggga ggagcagtgc
240

ttggctccct gcagtttggc tacaacactg gagtcatcaa tgccccccag aaggtgatcg
300

aggagttcta caaccagaca tgggtccacc gctatgggga gagcatcctg cccaccacgc
360

tcaccacgct ctggtccctc tcagtggcca tcttttctgt tgggggcatg attggctcct
420

tctctgtggg ccttttcgtt aaccgctttg gccggcggaa ttcaatgctg atgatgaacc
480

tgctggcctt cgtgtccgcc gtgctcatgg gcttctcgaa actgggcaag tcctttgaga
540

tgctgatcct gggccgcttc atcatcggtg tgtactgcgg cctgaccaca ggcttcgtgc
600

ccatgtatgt gggtgaagtg tcacccacag cctttcgtgg ggccctgggc accctgcacc
660

agctgggcat cgtcgtcggc atcctcatcg cccaggtgtt cggcctggac tccatcatgg
720

gcaacaagga cctgtggccc ctgctgctga gcatcatctt catcccggcc ctgctgcagt
780

gcatcgtgct gcccttctgc cccgagagtc cccgcttcct gctcatcaac cgcaacgagg
840

agaaccgggc caagagtgtg ctaaagaagc tgcgcgggac agctgacgtg acccatgacc
900

tgcaggagat gaaggaagag agtcggcaga tgatgcggga gaagaaggtc accatcctgg
960

agctgttccg ctcccccgcc taccgccagc ccatcctcat cgctgtggtg ctgcagctgt
1020

cccagcagct gtctggcatc aacgctgtct tctattactc cacgagcatc ttcgagaagg
1080

cgggggtgca gcagcctgtg tatgccacca ttggctccgg tatcgtcaac acggccttca
1140

ctgtcgtgtc gctgtttgtg gtggagcgag caggccggcg gaccctgcac ctcataggcc
1200

tcgctggcat ggcgggttgt gccatactca tgaccatcgc gctagcactg ctggagcagc
1260

taccctggat gtcctatctg agcatcgtgg ccatctttgg ctttgtggcc ttctttgaag
1320

tgggtcctgg ccccatccca tggttcatcg tggctgaact cttcagccag ggtccacgtc
1380

cagctgccat tgccgttgca ggcttctcca actggacctc aaatttcatt gtgggcatgt
1440

gcttccagta tgtggagcaa ctgtgtggtc cctacgtctt catcatcttc actgtgctcc
1500

tggttctgtt cttcatcttc acctacttca aagttcctga gactaaaggc cggaccttcg
1560

atgagatcgc ttccggcttc cggcaggggg gagccagcca aagtgataag acacccgagg
1620

agctgttcca tcccctgggg gctgattccc aagtgtgagt cgccccagat caccagcccg
1680

gcctgctccc agcagcccta aggatctctc aggagcacag gcagctggat gagacttcca
1740

aacctgacag atgtcagccg agccgggcct ggggctcctt tctccagcca gcaatgatgt
1800

ccagaagaat attcaggact taacggctcc aggattttaa caaaagcaag actgttgctc
1860

aaatctattc agacaagcaa caggttttat aattttttta ttactgattt tgttattttt
1920

atatcagcct gagtctcctg tgcccacatc ccaggcttca ccctgaatgg ttccatgcct
1980

gagggtggag actaagccct gtcgagacac ttgccttctt cacccagcta atctgtaggg
2040

ctggacctat gtcctaagga cacactaatc gaactatgaa ctacaaagct tctatcccag
2100

gaggtggcta tggccacccg ttctgctggc ctggatctcc ccactctagg ggtcaggctc
2160

cattaggatt tgccccttcc catctcttcc tacccaacca ctcaaattaa tctttcttta
2220

cctgagacca gttgggagca ctggagtgca gggaggagag gggaagggcc agtctgggct
2280

gccgggttct agtctccttt gcactgaggg ccacactatt accatgagaa gagggcctgt
2340

gggagcctgc aaactcactg ctcaagaaga catggagact cctgccctgt tgtgtataga
2400

tgcaagatat ttatatatat ttttggttgt caatattaaa tacagacact aagttatagt
2460

atatctggac aagccaactt gtaaatacac cacctcactc ctgttactta cctaaacaga
2520

tataaatggc tggtttttag aaacatggtt ttgaaatgct tgtggattga gggtaggagg
2580

tttggatggg agtgagacag aagtaagtgg ggttgcaacc actgcaacgg cttagacttc
2640

gactcaggat ccagtccctt acacgtacct ctcatcagtg tcctcttgct caaaaatctg
2700

tttgatccct gttacccaga gaatatatac attctttatc ttgacattca aggcatttct
2760

atcacatatt tgatagttgg tgttcaaaaa aacactagtt ttgtgccagc cgtgatgctc
2820

aggcttgaaa tcgcattatt ttgaatgtga agggaa
2856

<210> 28
<211> 1607
<212> DNA
<213> Homo sapiens

<400> 28
gctctgtagc acccaggagc ggggaagcga agtgcgagag accccggacc ccagcgctgt
60

ctcttcccgc cgcccgaacc accatgaccc acttcaacaa gggcccttcc tatgggctct
120

cggccgaagt caagaacaag attgcttcca agtatgatca tcaggcagaa gaagatcttc
180

gcaattggat agaagaggtg acaggcatga gcattggccc caacttccag ctgggcttaa
240

aggatggcat catcctctgc gaacttataa acaagctaca gccaggctca gtgaagaagg
300

tcaacgagtc ctcactgaac tggcctcagt tggagaatat tggcaacttt attaaagcta
360

ttcaggctta tggtatgaag ccacatgaca tattcgaagc aaatgatctt tttgagaatg
420

gaaacatgac ccaggttcag actactctgg tggctctagc aggtctggct aaaacaaaag
480

gattccatac aaccattgac attggagtta agtatgcaga aaaacaaaca agacgttttg
540

atgaaggaaa attaaaagct ggccaaagtg taattggtct gcagatggga accaacaaat
600

gtgccagcca ggcaggtatg acagcttacg ggactaggag gcatctttat gatcccaaaa
660

tgcaaactga caaacctttt gaccagacca caattagtct gcagatgggc actaataaag
720

gagccagcca ggcagggatg ttagcaccag gtaccagaag agacatctat gatcagaagc
780

taacattaca gccggtggac aactcgacaa tttccctaca gatgggtacc aacaaagttg
840

cttcccagaa aggaatgagt gtgtatgggc ttgggcggca agtatatgat cccaaatact
900

gtgctgctcc tacagaacct gtcattcaca acggaagcca aggaacagga acaaatggtt

960

cggaaatcag tgatagtgat tatcaggcag aataccctga tgagtatcat ggcgagtacc
1020

aggatgacta ccccagagat taccaatata gcgaccaagg cattgattat tagatccaca
1080

cagaaggagc tcagtattta gtcctttgtt tttattcagt gagaaccaag ctagccttga
1140

gtaattttta tcttgtcttc ctaaaacact attaagctta ttgtactttt aagaaaaatt
1200

gccttacgta cattcctttt tcctttttct gcctcttccc tcaatagttg ccttttagtg
1260

ctgtaatagg ttaaatccta cagcataatc aataactcgc atatgaagta aaaaggaata
1320

ctgtgaaagg ggagtactct tgtacagcca gttcttttat gcaaaaatct atgcattttt
1380

acaatcttat attaaactgg tattttcaaa caataggaaa cttttttttt ttttttttac
1440

agtttagtgt atctggtttc tacatggaag actaaactca tgcttattgc taaatgtggt
1500

ctttgccaac taaatttaag atgcagcatt ttagaaattt acatatcaat gtttctacag
1560

tattgtttgc taatttttaa ataaagtcat gatcagtgtg aaaaaaa
1607


<210> 29
<211> 1929
<212> DNA
<213> Homo sapiens

<400> 29
acccccgcac ccagctccgc agaccggcgg gcgcgcgcgg gctctggagg ccacgggcat
60

gatgcttcgg gtcctggtgg gggctgtcct ccctgccatg ctactggctg ccccaccacc
120

catcaacaag ctggcactgt tcccagataa gagtgcctgg tgcgaagcca agaacatcac
180

ccagatcgtg ggccacagcg gctgtgaggc caagtccatc cagaacaggg cgtgcctagg
240

acagtgcttc agctacagcg tccccaacac cttcccacag tccacagagt ccctggttca
300

ctgtgactcc tgcatgccag cccagtccat gtgggagatt gtgacgctgg agtgcccggg
360

ccacgaggag gtgcccaggg tggacaagct ggtggagaag atcctgcact gtagctgcca
420

ggcctgcggc aaggagccta gtcacgaggg gctgagcgtc tatgtgcagg gcgaggacgg
480

gccgggatcc cagcccggca cccaccctca cccccatccc cacccccatc ctggcgggca
540
gacccctgag cccgaggacc cccctggggc cccccacaca gaggaagagg gggctgagga
600
ctgaggcccc cccaactctt cctcccctct catcccccctg tggaatgttg ggtctcactc
660
tctggggaag tcaggggaga agctgaagcc cccctttggc actggatgga cttggcttca
720
gactcggact tgaatgctgc ccggttgcca tggagatctg aaggggcggg gttagagcca
780
agctgcacaa tttaatatat tcaagagtgg ggggaggaag cagaggtctt cagggctctt
840
tttttggggg gggggtggtc tcttcctgtc tggcttctag agatgtgcct gtgggagggg
900
gaggaagttg gctgagccat tgagtgctgg gggaggccat ccaagatggc atgaatcggg
960
ctaaggtccc tgggggtgca gatggtactg ctgaggtccc gggcttagtg tgagcatctt
1020
gccagcctca ggcttgaggg agggctgggc tagaaagacc actggcagaa acaggaggct
1080
ccggccccac aggtttcccc aaggcctctc accccacttc ccatctccag ggaagcgtcg
1140
ccccagtggc actgaagtgg ccctccctca gcggaggggt ttgggagtca ggcctgggca
1200
ggaccctgct gactcgtggc gcgggagctg ggagccaggc tctccgggcc tttctctggc
1260
ttccttggct tgcctggtgg gggaagggga ggaggggaag aaggaaaggg aagagtcttc
1320
caaggccaga gggaggggga caaccccccca agaccatccc tgaagacgag catcccccctc
1380
ctctccctgt tagaaatgtt agtgccccgc actgtgcccc aagttctagg ccccccagaa
1440
agctgtcaga gccggccgcc ttctcccctc tcccagggat gctctttgta aatatcggat
1500
gggtgtggga gtgaggggtt acctccctcg ccccaaggtt ccagaggccc taggcgggat
1560
gggctcgctg aacctcgagg aactccagga cgaggaggac atgggacttg cgtggacagt
1620
cagggttcac ttgggctctc tctagctccc caattctgcc tgcctcctcc ctcccagctg
1680
cactttaacc ctagaaggtg gggacctggg gggagggaca gggcaggcgg gcccatgaag
1740
aaagcccctc gttgcccagc actgtctgcg tctgctcttc tgtgcccagg gtggctgcca
1800

gcccactgcc tcctgcctgg ggtggcctgg ccctcctggc tgttgcgacg cgggcttctg
1860

gagcttgtca ccattggaca gtctccctga tggaccctca gtcttctcat gaataaattc
1920

ttcggaatt
1929

<210> 30
<211> 1319
<212> DNA
<213> Homo sapiens

<400> 30
gctgcagagc actatgaaat catggtacgt agtccccggc acctgtcgtt attcctatat
60

cctcctgcaa ctgtggtttg aaactgcgca ttctctagta gtatatatcg tgcctgtctt
120

caaaaacatt taccttttta tactcattcc ccccaggcat ggggtagtgt cagtcggact
180

gcacagggaa cacggtttcc agtggctttg gcccctactc gggaaacgtc tgcctgttct
240

cgatggtgat ggggtggctg ccattccctt ggttttccta agccctttct aacgagagtc
300

tcaaacaagc ggaggcgagg gccaattcaa ccccattctt tccagcgccc cgcaccatag
360

cacctgccca cctgagaacc aggaacgcac cctctctgtg gagctctgac tggtgtacct
420

ggaaacaaac agcaacttgc aaacggacga agagcctgcg tgtgttaatc atttgcttac
480

aagatgtacc agacggttcc agtactaaca aagggaataa aaatacctca cgccacaatc
540

cagcatatga tgttaaggca aaccaaaaaa aaaccaaaaa acaaaacaag aaaaacaaaa
600

acgggtgggg gtgggccgaa aaatttaccc ctcgttgggg cgcgccaatg tatgcaccgg
660

ccagggcagg acacgggccg aaagggggtc ccaaaaacgg gccggtgaac ctgggtgggc
720

tggcacaggc ggacacaccc ccgccgcaca gaacaagaac acaacgtaag caacgtacct
780

gttcttacgc tcttcgaacg acgacggcac aagataccaa cagcaaatac aaagagagca
840

aaataacaca accaacagac aaaacaagaa ccacagacaa cctacggaac caacacgtaa
900

cagcgaagaa gaaacgaaac aaccagagag acacaaacaa cacagacaca agaacacaaa
960

ctagaagaac cacaatgagc gaagcgagca gcgagggcac acgccagggg ccgaacacaa
1020

ggaacggccg agacagcgaa acaagaaggg caggaacaga gcaacggaag acacatagga
1080

gaacgcaaga gagacgcaac aagaacgcaa acaagaccga gtagcaggaa gacaacaaga
1140

aaagtggaaa aaggcgaaaa aaacgcgaaa agaaaaaaag agaaaaaacg ccggaaaaaa
1200

cacaaaaaac aacgcagaag acaaccaaga acaaacagtc aaagaacaaa gaaacaagag
1260

caacaaacag aaaacgaaca agaaacagaa gaagcacaag aacacggaga aaaccagca
1319

<210> 31
<211> 3442
<212> DNA
<213> Homo sapiens

<400> 31
cgccgcagcc gccggcgctg tggagatatt ctctaagccg ctttcatcat gggagaaata
60

gagcagaggc cgaccccagg atcacgactg ggggccccgg aaaattcggg gatcagtacc
120

ttggaacgtg gacagaagcc gcccccaaca ccttcaggaa aactcgtgtc catcaaaatc
180

cagatgctgg atgacaccca ggaggcattt gaagttccac aaagagctcc tgggaaggtg
240

ctgctggatg cagtttgcaa ccacctcaac ctcgtggaag gtgactattt tggcctcgag
300

tttcctgatc acaaaaagat cacggtgtgg ctggatctcc taaaacccat tgtgaaacag
360

attagaaggc caaagcacgt tgttgttaag tttgtggtga aattctttcc gcctgaccac
420

acacaactcc aagaagaact cacaaggtac ctgttcgcgc tgcaggtgaa gcaggacttg
480

gctcaaggca ggttgacgtg taatgacacc agcgcagctc tcttgatttc acacattgtg
540

caatctgaga ttggggattt tgatgaagcc ttggacagag agcacttagc aaaaaataaa
600

tacatacctc agcaagacgc actagaggac aaaatcgtgg aatttcacca taaccacatt
660

ggacaaacac cagcagaatc agatttccag ctcctagaga ttgcccgtcg gctagagatg
720

tatggaatcc ggttgcaccc ggccaaggac agggaaggca cgaagatcaa tctggccgtt
780

gccaacacgg gaattctagt gtttcagggt ttcactaaga tcaatgcctt caactgggcc
840

aaggtgcgga agctgagctt caagaggaag cgctttctca tcaagctccg gccagatgcc

900

aatagtgcgt accaggatac cttggaattc ctgatggcca gtcgggattt ctgcaagtcc
960

ttctggaaaa tctgtgttga acatcatgcc ttctttagac tttttgaaga gcccaaacca
1020

aagcccaagc ccgtcctctt tagccggggg tcatcatttc ggttcagtgg tcggactcag
1080

aagcaggttc tcgactatgt taaagaagga ggacataaga aggtgcagtt tgaaaggaag
1140

cacagcaaga ttcattctat ccggagcctt gcttcacagc ctacagaact gaattcggaa
1200

gtgctggagc agtctcagca gagcaccagc cttacatttg gagaaggtgc cgaatctcca
1260

gggggccaga gctgccggcg aggaaaggaa ccgaaggttt ccgccgggga gccggggtcg
1320

cacccgagcc ctgcgccgag gagaagcccc gcgggtaaca agcaggcgga cggagccgcc
1380

tcggcgccca cggaggaaga ggaggaggtc gttaaggata ggacccagca gagtaaacct
1440

cagcccccgc agccaagcac aggctccctg actggcagtc ctcacctttc cgagctgtct
1500

gtgaactcgc aggggggagt ggcccctgcc aacgtgacct tgtctcccaa cctgagcccc
1560

gacaccaagc aggcctctcc cttgatcagc ccgctgctga atgaccaggc ctgcccccgg
1620

acggacgatg aggatgaggg ccggaggaag agattcccaa ctgataaagc gtacttcata
1680

gctaaggaag tgtctaccac cgagcgaaca tatctgaagg atctcgaagt tatcacttcg
1740

tggtttcaga gcacagtgag caaagaggac gccatgccgg aagcactgaa aagtctcata
1800

ttcccgaatt ttgaaccttt gcacaaattt catactaatt ttctcaagga aattgagcaa
1860

cgacttgccc tgtgggaagg ccgctcaaat gcccaaatca gagattacca aagaatcggc
1920

gatgtcatgc tgaagaacat tcagggcatg aagcacctgg cggctcacct gtggaagcac
1980

agcgaggcct tggaggccct ggagaatgga atcaagagct cccggcggct ggagaacttc
2040

tgcagagact ttgagctgca gaaggtgtgt tacctaccgc tcaacacctt cctcctgcgg
2100

ccactgcacc ggctcatgca ctacaagcag gtcctggagc ggctgtgcaa acaccacccg
2160

ccgagccacg ccgacttcag ggactgccga gccgctttgg cagagatcac ggagatggtg

2220

gcacagctcc acggtacgat gatcaagatg gagaatttcc agaagctgca cgaactcaag
2280

aaagatttga ttggcattga caatcttgtg gttccgggaa gggagttcat ccgtctgggc
2340

agcctcagca agctctcggg gaaggggctc cagcagcgca tgttcttcct gttcaacgac
2400

gtcctgctat acacgagccg ggggctgacg gcctccaatc agtttaaagt ccacgggcag
2460

ctcccgctct atggcatgac gattgaggag agcgaagacg agtggggggt gccccactgc
2520

ctgaccctcc ggggccagcg gcagtccatc atcgtggccg ccagttctcg gtccgagatg
2580

gagaagtggg ttgaggacat ccagatggcc attgacctgg cggagaagag cagcagcccc
2640

gcccctgagt tcctggccag cagcccccct gacaacaagt cccctgatga agccaccgcg
2700

gctgaccagg agtcagagga tgacctgagc gcctcgcgca catcgctgga gcgccaggcc
2760

ccgcaccgcg gcaacacaat ggtgcacgtg tgctggcacc gcaacaccag cgtctccatg
2820

gtggacttca gcatcgcagt ggagaatcag ttgtctggaa acctgctgag gaaattcaaa
2880

aacagcaacg ggtggcagaa gctgtgggtg gtgttcacaa acttctgcct gttcttctac
2940

aaatcacacc aggacaatca tccccttgcc agcctgcctc tgctcggcta ctcgctcacc
3000

atcccctctg agtccgagaa catccagaaa gactacgtgt tcaagctgca cttcaagtcc
3060

cacgtctact acttcagggc ggaaagcgag tacacgttcg aaaggtggat ggaagtgatc
3120

cgcagtgcca ccagctctgc ctcgcgaccc cacgtgttga gccacaaaga gtctcttgtg
3180

tattgatggc cggacacact cgtttccgca gtggctgctt tcctggaaga cgtttccttt
3240

cttctgtatt aatgaagcct ggtaaaatta acacctgtct gaaaatcaaa aacatggctt
3300

cccagcagct ctcctgtctc cacagccgcg ttttttaacc ccgacctctc agcgtttgaa
3360

tgaacagcgc tcccacctcc agtcctggca tccgctgggg gcgctgttct ttagctagtg
3420

ccagtattaa aacattgtca tt
3442

<210>  32
<211>  578
<212>  DNA
<213>  Homo sapiens


<220>
<221>  misc_feature
<222>  (393)..(558)

<220>
<221>  misc_feature
<222>  (393)..(393)
<223>  n is a, c, g, or t

<220>
<221>  misc_feature
<222>  (551)..(551)
<223>  n is a, c, g, or t

<220>
<221>  misc_feature
<222>  (558)..(558)
<223>  n is a, c, g, or t

<400>  32
tttttttctg tttaagactg taacatggaa aaaatgttta tacgttaagt acaaaaggga
60

cataaaattg tatatacaga ataacgacta tgtaaacaca ccaaaaatct atgcacagaa
120

atgtctaggg ggaacattta acaccaaaag attaacagtg gtagcatttg ggtggcaaac
180

ttgattcttt ctaaaattcc catattttcc ttaataagca gtaattataa ttacaacggg
240

aaataatttc tttaagtacc cagtgcagtg tcactgtcaa ataaacatca gtggctttgg
300

ccccaattct taaggtggca aacgccgctg ccccactccc cacccatccc caatagggct
360

tgagcacctg tagccctgct gagcacgtct gtngagcccc agcctccagc actttccatc
420

gcttccagta taacttttcc tttctcttaa ttcaggagca gtttgcaacc acaggggcca
480

acgatagaga cgtgaggggg cattttgttt tttttttgga cggagtttgc tgttgttgcc
540

aagccggagt naatggcncg atttcaatcc ctcggggc
578


<210>  33
<211>  1648
<212>  DNA
<213>  Homo sapiens

<400>  33
atgcgggaga tcgtgcacat ccaggccggc cagtgcggca accagatcgg ggccaagttc
60

tgggaagtca tcagtgatga gcatggcatc gaccccagcg gcaactacgt gggcgactcg
120

gacttgcagc tggagcggat cagcgtctac tacaacgagg cctcttctca caagtacgtg
180

cctcgagcca ttctggtgga cctggaaccc ggaaccatgg acagtgtccg ctcaggggcc
240

tttggacatc tcttcaggcc tgacaatttc atctttggtc agagtggggc cggcaacaac
300

tgggccaagg gtcactacac ggagggggcg gagctggtgg attcggtcct ggatgtggtg
360

cggaaggagt gtgaaaactg cgactgcctg cagggcttcc agctgaccca ctcgctgggg
420

ggggggacgg gctccggcat gggcacgttg ctcatcagca aggtgcgtga ggagtatccc
480

gaccgcatca tgaacacctt cagcgtcgtg ccctcaccca aggtgtcaga cacggtggtg
540

gaaccctaca acgccacgct gtccatccac cagctggtgg aaaacacgga tgaaacctac
600

tgcatcgaca acgaggcgct ctacgacatc tgcttccgca ccctcaagct ggccacgccc
660

acctacgggg acctcaacca cctggtatcg gccaccatga gcggagtcac cacctccttg
720

cgcttcccgg gccagctcaa cgctgacctg cgcaagctgg ccgtcaacat ggtgcccttc
780

ccgcgcctgc acttcttcat gcccggcttc gcccccctca ccaggcgggg cagccagcag
840

taccgggccc tgaccgtgcc cgagctcacc cagcagatgt tcgatgccaa gaacatgatg
900

gccgcctgcg acccgcgcca cggccgctac ctgacggtgg ccaccgtgtt ccggggccgc
960

atgtccatga aggaggtgga cgagcagatg ctggccatcc agagcaagaa cagcagctac
1020

ttcgtggagt ggatccccaa caacgtgaag gtggccgtgt gtgacatccc gccccgcggc
1080

ctcaagatgt cctccacctt catcgggaac agcacggcca tccaggagct gttcaagcgc
1140

atctccgagc agttcacggc catgttccgg cgcaaggcct tcctgcactg gtacacgggc
1200

gagggcatgg acgagatgga gttcaccgag gccgagagca acatgaacga cctggtgtcc
1260

gagtaccagc agtaccagga cgccacggcc gaggaagagg gcgagatgta cgaagacgac
1320

gaggaggagt cggaggccca gggccccaag tgaaactgct cgcagctgga gtgagaggca
1380

ggtggcggcc ggggccgaag ccagcagtgt ctaaaccccc ggagccatct tgctgccgac
1440

accctgcttt ccccatcgcc ctagggctcc cttgccgccc tcctgcagta tttatggcct
1500

cgtcctcccc cacctaggcc acgtgtgagc tgctcctgtc tctgtcttat tgcagctcca
1560

ggcctgacgt tttacggttt tgttttttac tggtttgtgt ttatattttc ggggatactt
1620

aataaatcta ttgctgtcag ataccctt
1648

<210> 34
<211> 3917
<212> DNA
<213> Homo sapiens

<400> 34
tagtcggagc gaggtggcga gtcgctgagc ccgccgcggc cccgagagcg gctgcagccg
60

ccgccgccgg gaaggagagg gcgaggcgcg cccgagccgc cgccgccgcc gccaccgccg
120

ccgccgccac caccgccacc ggagtcgcgg gccagccggg cagcctccgc gggccccggc
180

cggggcgggg ggcgcgggcc acaggcccct gctccggccg tcgtttgcag accgcgggcg
240

ccgatgtcgc ccgcgccccg ttaggatgag tctcgggtcg ggcgaggagc cgccgcagcc
300

gccgccgccc gagccgcggg caggagcctc gggagccgcc gccgccgccg ccgccgcccg
360

gccgggcccc gacgccgccc gcgcgccccc gggcccccga cacacatgag attcttcagg
420

ctcactttca agtgcttcgt ggactgcttc tgactgcgcc gcccgcgccc cgcaccccgc
480

cgtccgcccg ccgccccgtc ccccggcccg gccgcccccc ggcccccggc cggcccgcgc
540

cctcggggcc ctccccggtg ccgccggtgc cccccgcctg accgccgccc cccgtgaggc
600

gccgcgaccc cggcccggcc gtgcggcccg ccggggccat ggcgaagaag agcgccgaga
660

acggcatcta tagcgtgtcc ggcgacgaga agaagggccc cctcatcgcg cccgggcccg
720

acggggcccc ggccaagggc gacggccccg tgggcctggg gacacccggc ggccgcctgg
780

ccgtgccgcc gcgcgagacc tggacgcgcc agatggactt catcatgtcg tgcgtgggct
840

tcgccgtggg cttgggcaac gtgtggcgct tcccctacct gtgctacaag aacggcggag
900

gtgtgttcct tattccctac gtcctgatcg ccctggttgg aggaatcccc attttcttct
960

tagagatctc gctgggccag ttcatgaagg ccggcagcat caatgtctgg aacatctgtc
1020

ccctgttcaa aggcctgggc tacgcctcca tggtgatcgt cttctactgc aacacctact
1080

acatcatggt gctggcctgg ggcttctatt acctggtcaa gtcctttacc accacgctgc
1140

cctgggccac atgtggccac acctggaaca ctcccgactg cgtggagatc ttccgccatg
1200

aagactgtgc caatgccagc ctggccaacc tcacctgtga ccagcttgct gaccgccggt
1260

cccctgtcat cgagttctgg gagaacaaag tcttgaggct gtctggggga ctggaggtgc
1320

caggggccct caactgggag gtgacccttt gtctgctggc ctgctgggtg ctggtctact
1380

tctgtgtctg gaagggggtc aaatccacgg gaaagatcgt gtacttcact gctacattcc
1440

cctacgtggt cctggtcgtg ctgctggtgc gtggagtgct gctgcctggc gccctggatg
1500

gcatcattta ctatctcaag cctgactggt caaagctggg gtcccctcag gtgtggatag
1560

atgcggggac ccagattttc ttttcttacg ccattggcct gggggccctc acagccctgg
1620

gcagctacaa ccgcttcaac aacaactgct acaaggacgc catcatcctg gctctcatca
1680

acagtgggac cagcttcttt gctggcttcg tggtcttctc catcctgggc ttcatggctg
1740

cagagcaggg cgtgcacatc tccaaggtgg cagagtcagg gccgggcctg gccttcatcg
1800

cctacccgcg ggctgtcacg ctgatgccag tggccccact ctgggctgcc ctgttcttct
1860

tcatgctgtt gctgcttggt ctcgacagcc agtttgtagg tgtggagggc ttcatcaccg
1920

gcctcctcga cctcctcccg gcctcctact acttccgttt ccaaagggag atctctgtgg
1980

ccctctgttg tgccctctgc tttgtcatcg atctctccat ggtgactgat ggcgggatgt
2040

acgtcttcca gctgtttgac tactactcgg ccagcggcac caccctgctc tggcaggcct
2100

tttgggagtg cgtggtggtg gcctgggtgt acggagctga ccgcttcatg gacgacattg
2160

cctgtatgat cgggtaccga ccttgcccct ggatgaaatg gtgctggtcc ttcttcaccc
2220

cgctggtctg catgggcatc ttcatcttca acgttgtgta ctacgagccg ctggtctaca
2280

acaacaccta cgtgtacccg tggtggggtg aggccatggg ctgggccttc gccctgtcct
2340

ccatgctgtg cgtgccgctg cacctcctgg gctgcctcct cagggccaag ggcaccatgg
2400

ctgagcgctg gcagcacctg acccagccca tctggggcct ccaccacttg gagtaccgag
2460

ctcaggacgc agatgtcagg ggcctgacca ccctgacccc agtgtccgag agcagcaagg
2520

tcgtcgtggt ggagagtgtc atgtgacaac tcagctcaca tcaccagctc acctctggta
2580

gccatagcag cccctgcttc agccccaccg cacccctcca gggggcctgc ctttccctga
2640

cacttttggg gtctgcctgg gggaggaggg gagaaagcac catgagtgct cactaaaaca
2700

actttttcca tttttaataa aacgccaaaa atatcacaac ccaccaaaaa tagatgcctc
2760

tccccctcca gccctagccg agctggtcct aggccccgcc tagtgcccca cccccaccca
2820

cagtgctgca ctcctcctgc ccctgccacg cccaccccct gcccacctct ccaggctctg
2880

ctctgcagca cacccgtggg tgacccctca ccccagaagc agcagtggca gcttgggaaa
2940

tgtgaggaag ggaaggaggg agagacggga gggaggagag agaggagaag ggaggcaggg
3000

gaggggcagc agaaccaagg caaatatttc agctgggcta tacccctctc cccatccctg
3060

ttatagaagc ttagagagcc agccagcaat ggaaccttct ggttcctgcg ccaatcgcca
3120

ccagtatcaa ttgtgtgagc ttgggtgcga gtgcacgcgt gcgtgagtac ggagagtata
3180

tatagatctc tatctcttag caaaggtgaa tgccagatgt aaatggcgcc tctgggcaaa
3240

ggaggcttgt attttgcaca ttttataaaa acttgagaga atgagatttc tgcttgtata
3300

tttctaaaaa gaggaaggag cccaaaccat cctctcctta ccactcccat ccctgtgagc
3360

cctaccttac ccctctgccc ctagccaagg agtgtgaatt tatagatcta actttcatag
3420

gcaaaacaaa agcttcgagc tgttgcgtgt gtgagtctgt tgtgtggatg tgcgtgtgtg
3480

gtccccagcc ccagactgga ttggaaaagt gcatggtggg ggcctcgggg ctgtccccac
3540

gctgtccctt tgccacaagt ctgtggggca agaggctgca atattccgtc ctgggtgtct
3600

gggctgctaa cctggcctgc tcaggcttcc caccctgtgc ggggcacacc cccaggaagg
3660

gaccctggac acggctccca cgtccaggct taaggtggat gcacttcccg cacctccagt
3720

cttctgtgta gcagctttaa cccacgtttg tctgtcacgt ccagtcccga gacggctgag
3780

tgaccccaag aaaggcttcc ccgacaccca gacagaggct gcagggctgg ggctgggtga
3840

gggtggcggg cctgcgggga cattctactg tgctaaaaag ccactgcaga catagcaata
3900

aaaacatgtc attttcc
3917

<210>  35
<211>  1743
<212>  DNA
<213>  Homo sapiens

<400>  35
ctaaagagta tggcatcgag gagaatggag accaaacctg tgataacctg tctcaaaacc
60

ctcctcatca tctactcctt cgtcttctgg atcactgggg tgatcctgct ggctgttgga
120

gtctggggca aacttactct gggcacctat atctccctta ttgccgagaa ctccacaaat
180

gctccctatg tgctcatcgg aactggcacc actattgttg tctttggcct gtttggatgc
240

tttgctacat gtcgtggtag cccatggatg ctgaaactgt atgccatgtt tctgtccctg
300

gtgttcctgg ctgagctcgt agctggcatt tcaggttttg tgtttcgtca tgagatcaag
360

gacaccttcc tgaggactta cacggacgct atgcagactt acaatggcaa tgatgagagg
420

agccgggcag tggaccatgt gcagcgcagc ctgagctgct gtggtgtgca gaactacacc
480

aactggagca ccagccccta cttcctggag catggcatcc cccccagctg ctgcatgaac
540

gaaactgatt gtaatcccca ggatctacac aatctgactg tggccgccac caaagttaac
600

cagaagggtt gttatgatct ggtaactagt ttcatggaga ctaacatggg aatcatcgct
660

ggagtggcgt ttggaatcgc attctcccag ttaattggca tgctgctggc ctgctgtctg
720

tcccggttca tcacggccaa tcagtatgag atggtgtaag gagaagtctt tcaagaatga

780

cggaataaga gacctgtttt aaaaaggaac tgcagcaatc tttgaaagac ttccaaagaa
840

tgttagagca cagtacataa tacacttgcc ctgctccctc taccccttac cccacaacgt
900

gcaactgaca ctcccaccca gtctctgctc cacctttcag cccacgtcac gtgtagtgtc
960

cattttgtga agccctgttg tgccacagag tgtagccagg tccccctgca gctagtccta
1020

gtgaacctca ccccgaggcc ctgcatgggc agcccctcca tctgtacttg gtccaactgc
1080

aactcatcat cggtgactgg ttatcacacc atcgctcgct ttgggccctg catgtagtgt
1140

gggaggctcc tgttagctcc tcactgtggt aaatgccaca cacctttaag tagataagca
1200

gacgatagtt atctgttctt ttgacttaat ctcatttggt ttgattttcc ctctactaag
1260

gctttcctac cttcttcagg ctgcctaaga catgtaagcg aaacacttca ataattgtcc
1320

atgaggagaa aaaaagcatt gtcatgcatg aaggaaactg aacttgaggt ggcctccttg
1380

cttgttacat acctgggtat gtgtaggcag tttagtgcat ctttgcctct cagttgaaac
1440

ctgtataacc ctgttacaaa gctgtgttgt tgcttcttgt gaaggccatg atattttgtt
1500

ttttccccaa ttaattgcta ttgtgttatt ttactaactt ctctctgtat tttttcttgc
1560

attgacatta tagacattga ggacctcatc caaacaattt aaaaatgagt gtgaaggggg
1620

aacaagtcaa aatattttta aaagatcttc aaaaataatg cctctgtcta gcatgccaac
1680

aagaatgcat tgatattgtg aacatttgtg atatatgtat taataaatag agcaattaca
1740

agc
1743


<210> 36
<211> 2259
<212> DNA
<213> Homo sapiens

<400> 36
gttctcccct tcccggcttt cggtccggag gaggcgggag cagcttccct gttctgatcc
60

tatcgcgggc ggcgcagggc cggcttggcc ttccgtggga cggggagggg ggcgggatgt
120

gtcacccaaa taccagtggg gacggtcggt ggtggaacca gccgggcagg tcgggtagag
180

tataagagcc ggagggagcg gccgggcggc agacgcctgc agaccatccc agacgccgga
240

gcccgagccc cgccgagtcc ccgcgcctca tccgcccgcg tccggtccgc gttcctccgc
300

cccaccatgg ctcggggccc cggcctcgcg ccgccaccgc tgcggctgcc gctgctgctg
360

ctggtgctgg cggcggtgac cggccacacg gccgcgcagg acaactgcac gtgtcccacc
420

aacaagatga ccgtgtgcag ccccgacggc cccggcggcc gctgccagtg ccgcgcgctg
480

ggctcgggca tggcggtcga ctgctccacg ctgacctcca agtgtctgct gctcaaggcg
540

cgcatgagcg cccccaagaa cgcccgcacg ctggtgcggc cgagtgagca cgcgctcgtg
600

gacaacgatg gcctctacga ccccgactgc gaccccgagg gccgcttcaa ggcgcgccag
660

tgcaaccaga cgtcggtgtg ctggtgcgtg aactcggtgg gcgtgcgccg cacggacaag
720

ggcgacctga gcctacgctg cgatgacctg gtgcgcaccc accacatcct cattgacctg
780

cgccaccgcc ccaccgccgg cgccttcaac cactcagacc tggacgccga gctgaggcgg
840

ctcttccgcg agcgctatcg gctgcacccc aagttcgtgg cggccgtgca ctacgagcag
900

cccaccatcc agatcgagct gcggcagaac acgtctcaga aggccgccgg tgaagtggat
960

atcggcgatg ccgcctacta cttcgagagg gacatcaagg gcgagtctct attccagggc
1020

cgcggcggcc tggacttgcg cgtgcgcgga gaacccctgc aggtggagcg cacgctcatc
1080

tattacctgg acgagattcc cccgaagttc tccatgaagc gcctcaccgc cggcctcatc
1140

gccgtcatcg tggtggtcgt ggtggccctc gtcgccggca tggccgtcct ggtgatcacc
1200

aaccggagaa agtcggggaa gtacaagaag gtggagatca aggaactggg ggagttgaga
1260

aaggaaccga gcttgtaggt acccggcggg gcaggggatg gggtggggta ccggatttcg
1320

gtatcgtccc agacccaagt gagtcacgct tcctgattcc tcggcgcaaa ggagacgttt
1380

atcctttcaa attcctgcct tccccctccc ttttgcgcac acaccaggtt taatagatcc
1440

tggcctcagg gtctcctttc tttctcactt ctgtcttgaa ggaagcattt ctaaaatgta
1500

tcccctttcg gtccaacaac aggaaacctg actggggcag tgaaggaagg gatggcacag
1560

cgttatgtgt aaaaaacaag tatctgtatg acaacccggg atcgtttgca agtaactgaa
1620

tccattgcga cattgtgaag gcttaaatga gtttagatgg gaaatagcgt tgttatcgcc
1680

ttgggtttaa attatttgat gagttccact tgtatcatgg cctacccgag gagaagagga
1740

gtttgttaac tgggcctatg tagtagcctc atttaccatc gtttgtatta ctgaccacat
1800

atgcttgtca ctgggaaaga agcctgtttc agctgcctga acgcagtttg gatgtctttg
1860

aggacagaca ttgcccggaa actcagtcta tttattcttc agcttgccct tactaccact
1920

gatattggta atgttctttt ttgtaaaatg tttgtacata tgttgtcttt gataatgttg
1980

ctgtaatttt ttaaaataaa acacgaattt aataaaatat gggaaaggca caaaccagaa
2040

gttggcattt gtgaaaagtc cctccagatt tctatcactt tggtctctaa tttcccaaga
2100

cttgtatttt ttttttattt caaattataa cacttttttt tcccccagaa gtgggtgttt
2160

catgttgcta ctctggtgtg tcccaagata tcctaactgg ccagtgtaaa tgctattctt
2220

tctaaataag attatttgga aacttccttc aaactgcag
2259

<210> 37
<211> 2259
<212> DNA
<213> Homo sapiens

<400> 37
gttctcccct tcccggcttt cggtccggag gaggcgggag cagcttccct gttctgatcc
60

tatcgcgggc ggcgcagggc cggcttggcc ttccgtggga cggggagggg ggcgggatgt
120

gtcacccaaa taccagtggg gacggtcggt ggtggaacca gccgggcagg tcgggtagag
180

tataagagcc ggagggagcg gccgggcggc agacgcctgc agaccatccc agacgccgga
240

gcccgagccc cgccgagtcc ccgcgcctca tccgcccgcg tccggtccgc gttcctccgc
300

cccaccatgg ctcggggccc cggcctcgcg ccgccaccgc tgcggctgcc gctgctgctg
360

ctggtgctgg cggcggtgac cggccacacg gccgcgcagg acaactgcac gtgtcccacc
420

aacaagatga ccgtgtgcag ccccgacggc cccggcggcc gctgccagtg ccgcgcgctg
480

ggctcgggca tggcggtcga ctgctccacg ctgacctcca agtgtctgct gctcaaggcg
540

cgcatgagcg cccccaagaa cgcccgcacg ctggtgcggc cgagtgagca cgcgctcgtg
600

gacaacgatg gcctctacga ccccgactgc gaccccgagg gccgcttcaa ggcgcgccag
660

tgcaaccaga cgtcggtgtg ctggtgcgtg aactcggtgg gcgtgcgccg cacggacaag
720

ggcgacctga gcctacgctg cgatgacctg gtgcgcaccc accacatcct cattgacctg
780

cgccaccgcc ccaccgccgg cgccttcaac cactcagacc tggacgccga gctgaggcgg
840

ctcttccgcg agcgctatcg gctgcacccc aagttcgtgg cggccgtgca ctacgagcag
900

cccaccatcc agatcgagct gcggcagaac acgtctcaga aggccgccgg tgaagtggat
960

atcggcgatg ccgcctacta cttcgagagg gacatcaagg gcgagtctct attccagggc
1020

cgcggcggcc tggacttgcg cgtgcgcgga gaacccctgc aggtggagcg cacgctcatc
1080

tattacctgg acgagattcc cccgaagttc tccatgaagc gcctcaccgc cggcctcatc
1140

gccgtcatcg tggtggtcgt ggtggccctc gtcgccggca tggccgtcct ggtgatcacc
1200

aaccggagaa agtcggggaa gtacaagaag gtggagatca aggaactggg ggagttgaga
1260

aaggaaccga gcttgtaggt acccggcggg gcaggggatg gggtggggta ccggatttcg
1320

gtatcgtccc agacccaagt gagtcacgct tcctgattcc tcggcgcaaa ggagacgttt
1380

atcctttcaa attcctgcct tccccctccc ttttgcgcac acaccaggtt taatagatcc
1440

tggcctcagg gtctcctttc tttctcactt ctgtcttgaa ggaagcattt ctaaaatgta
1500

tcccctttcg gtccaacaac aggaaacctg actggggcag tgaaggaagg gatggcacag
1560

cgttatgtgt aaaaaacaag tatctgtatg acaacccggg atcgtttgca agtaactgaa
1620

tccattgcga cattgtgaag gcttaaatga gtttagatgg gaaatagcgt tgttatcgcc
1680

ttgggtttaa attatttgat gagttccact tgtatcatgg cctacccgag gagaagagga
1740

gtttgttaac tgggcctatg tagtagcctc atttaccatc gtttgtatta ctgaccacat
1800

atgcttgtca ctgggaaaga agcctgtttc agctgcctga acgcagtttg gatgtctttg
1860

aggacagaca ttgcccggaa actcagtcta tttattcttc agcttgccct tactaccact
1920

gatattggta atgttctttt ttgtaaaatg tttgtacata tgttgtcttt gataatgttg
1980

ctgtaatttt ttaaaataaa acacgaattt aataaaatat gggaaaggca caaaccagaa
2040

gttggcattt gtgaaaagtc cctccagatt tctatcactt tggtctctaa tttcccaaga
2100

cttgtatttt ttttttattt caaattataa cacttttttt tcccccagaa gtgggtgttt
2160

catgttgcta ctctggtgtg tcccaagata tcctaactgg ccagtgtaaa tgctattctt
2220

tctaaataag attatttgga aacttccttc aaactgcag
2259

<210>  38
<211>  2805
<212>  DNA
<213>  Homo sapiens

<400>  38
cgggtctgat agtccctacc tgtcaggact ggtgttagga tgagataatg tttgtgaact
60

gtaaacatat ataaacgtgt gctactgtga gaactggaac aaagaagaga gggagtgaga
120

gaaatcaagg gagggctggg gctgggaaag aacgaaaagg gagtcgcgta tagaggagag
180

gcgacagtcg cgagccacac tttgcaatga aactctttag actttctgcc gggagagcgg
240

cccagacgcg ccaggtctgt agcaggaggc cgcgagggcg ggtccccaga agcctacagg
300

tgagtatcgg ttctcccctt cccggctttc ggtccggagg aggcgggagc agcttccctg
360

ttctgatcct atcgcgggcg gcgcagggcc ggcttggcct tccgtgggac ggggaggggg
420

gcgggatgtg tcacccaaat accagtgggg acggtcggtg gtggaaccag ccgggcaggt
480

cgggtagagt ataagagccg gagggagcgg ccggggcgca gacgcctgca gaccatccca
540

gacgccggag cccgagcccc gacgagtccc cgcgcctcat ccgcccgcgt ccggtccgcg

600

ttcctccgcc ccaccatggc tcggggcccc ggcctcgcgc cgccaccgct gcggctgccg
660

ctgctgctgc tggtgctggc ggcggtgacc ggccacacgg ccgcgcagga caactgcacg
720

tgtcccacca acaagatgac cgtgtgcagc cccgacggcc ccggcggccg ctgccagtgc
780

cgcgcgctgg gctcgggcat ggcggtcgac tgctccacgc tgacctccaa gtgtctgctg
840

ctcaaggcgc gcatgagcgc ccccaagaac gcccgcacgc tggtgcggcc gagtgagcac
900

gcgctcgtgg acaacgatgg cctctacgac cccgactgcg accccgaggg ccgcttcaag
960

gcgcgccagt gcaaccagac gtcggtgtgc tggtgcgtga actcggtggg cgtgcgccgc
1020

acggacaagg gcgacctgag cctacgctgc gatgagctgg tgcgcaccca ccacatcctc
1080

attgacctgc gccaccgccc caccgccggc gccttcaacc actcagacct ggacgccgag
1140

ctgaggcggc tcttccgcga gcgctatcgg ctgcacccca agttcgtggc ggccgtgcac
1200

tacgagcagc ccaccatcca gatcgagctg cggcagaaca cgtctcagaa ggccgccggt
1260

gaagtggata tcggcgatgc cgcctactac ttcgagaggg acatcaaggg cgagtctcta
1320

ttccagggcc gcggcggcct ggacttgcgc gtgcgcggag aacccctgca ggtggagcgc
1380

acgctcatct attacctgga cgagattccc ccgaagttct ccatgaagcg cctcaccgcc
1440

ggcctcatcg ccgtcatcgt ggtggtcgtg gtggccctcg tcgccggcat ggccgtcctg
1500

gtgatcacca accggagaaa gtcggggaag tacaagaagg tggagatcaa ggaactgggg
1560

gagttgagaa aggaaccgag cttgtaggta cccggcgggg caggggatgg ggtggggtac
1620

cggatttcgg tatcgtccca gacccaagtg agtcacgctt cctgattcct cggcgcaaag
1680

gagacgttta tcctttcaaa ttcctgcctt ccccctccct tttgcgcaca caccaggttt
1740

aatagatcct ggcctcaggg tctcctttct ttctcacttc tgtcttgagg gaagcatttc
1800

taaaatgtat cccctttcgg tccaacaaca ggaaacctga ctggggcagt gaaggaaggg
1860

atggcacagc gttatgtgta aaaaacaagt atctgtatga caacccggga tcgtttgcaa

1920

gtaactgaat ccattgcgac attgtgaagg cttaaatgag tttagatggg aaatagcgtt
1980

gttatcgcct tgggtttaaa ttatttgatg agttccactt gtatcatggc ctacccgagg
2040

agaagaggag tttgttaact gggcctatgt agtagcctca tttaccatcg tttgtattac
2100

tgaccacata tgcttgtcac tgggaaagaa gcctgtttca gctgcctgaa cgcagtttgg
2160

atgtctttga ggacagacat tgcccggaaa ctcagtctat ttattcttca gcttgccctt
2220

actgccactg atattggtaa tgttcttttt tgtaaaatgt ttgtacatat gttgtctttg
2280

ataatgttgc tgtaattttt taaaataaaa cacgaattta ataaaatatg ggaaaggcac
2340

aaaccagaag tcggcatttg tgaaaagtcc ctccagattt ctatcacttt ggtctctaat
2400

ttcccaagac ttgtattttt tttttatttc aaattataac actttttttt cccccagaag
2460

tgggtgtttc atgttgctac tctggtgtgt cccaagatat cctaactggc cagtgtaaat
2520

gctattcttt ctaaataaga ttatttggaa acttccttca aactgcagga gggcgagctc
2580

tgagggcacg agaagctaaa actagctgct tttgatgaaa aagagtgcca gtctttggtc
2640

atctctaaac aaggcttatc accaatggag acagaaaact ctagttcaag agctgtacct
2700

cctttgaatc ccagccctac tcgaaataag tggtactatt tccatttagc ctttgagcaa
2760

atcacttaac tcaaaggcgt tgtggctcta agattaaacg acttt
2805


<210>  39
<211>  6728
<212>  DNA
<213>  Homo sapiens

<400>  39
   agcagacggg agtttctcct cggggtcgga gcaggaggca cgcggagtgt gaggccacgc
   60
   atgagcggac gctaaccccc tccccagcca caaagagtct acatgtctag ggtctagaca
   120
   tgttcagctt tgtggacctc cggctcctgc tcctcttagc ggccaccgcc ctcctgacgc
   180
   acggccaaga ggaaggccaa gtcgagggcc aagacgaaga catcccacca atcacctgcg
   240
   tacagaacgg cctcaggtac catgaccgag acgtgtggaa acccgagccc tgccggatct
   300
   gcgtctgcga caacggcaag gtgttgtgcg atgacgtgat ctgtgacgag accaagaact
   360
   gccccggcgc cgaagtcccc gagggcgagt gctgtcccgt ctgccccgac ggctcagagt
   420
   cacccaccga ccaagaaacc accggcgtcg agggacccaa gggagacact ggcccccgag
   480
   gcccaagggg acccgcaggc ccccctggcc gagatggcat ccctggacag cctggacttc
   540
   ccggaccccc cggacccccc ggacctcccg gaccccctgg cctcggagga aactttgctc
   600
   cccagctgtc ttatggctat gatgagaaat caaccggagg aatttccgtg cctggcccca
   660
   tgggtccctc tggtcctcgt ggtctccctg gcccccctgg tgcacctggt ccccaaggct
   720
   tccaaggtcc ccctggtgag cctggcgagc ctggagcttc aggtcccatg ggtccccgag
   780
   gtcccccagg tccccctgga aagaatggag atgatgggga agctggaaaa cctggtcgtc
   840
   ctggtgagcg tgggcctcct gggcctcagg gtgctcgagg attgcccgga acagctggcc
   900
   tccctggaat gaagggacac agaggtttca gtggtttgga tggtgccaag ggagatgctg
   960
   gtcctgctgg tcctaagggt gagcctggca gccctggtga aaatggagct cctggtcaga
   1020
   tgggcccccg tggcctgcct ggtgagagag gtcgccctgg agcccctggc cctgctggtg
   1080
   ctcgtggaaa tgatggtgct actggtgctg ccgggccccc tggtcccacc ggccccgctg
   1140
   gtcctcctgg cttccctggt gctgttggtg ctaagggtga agctggtccc caagggcccc
   1200
   gaggctctga aggtccccag ggtgtgcgtg gtgagcctgg cccccctggc cctgctggtg
   1260
   ctgctggccc tgctggaaac cctggtgctg atggacagcc tggtgctaaa ggtgccaatg
   1320
   gtgctcctgg tattgctggt gctcctggct tccctggtgc ccgaggcccc tctggacccc
   1380
   agggccccgg cggccctcct ggtcccaagg gtaacagcgg tgaacctggt gctcctggca
   1440
   gcaaaggaga cactggtgct aagggagagc ctggccctgt tggtgttcaa ggaccccctg
   1500
   gccctgctgg agaggaagga aagcgaggag ctcgaggtga acccggaccc actggcctgc
   1560
   ccggaccccc tggcgagcgt ggtggacctg gtagccgtgg tttccctggc gcagatggtg
   1620
   ttgctggtcc caagggtccc gctggtgaac gtggttctcc tggccccgct ggccccaaag
   1680
   gatctcctgg tgaagctggt cgtcccggtg aagctggtct gcctggtgcc aagggtctga
   1740
   ctggaagccc tggcagccct ggtcctgatg gcaaaactgg cccccctggt cccgccggtc
   1800
   aagatggtcg ccccggaccc ccaggcccac ctggtgcccg tggtcaggct ggtgtgatgg
   1860
   gattccctgg acctaaaggt gctgctggag agcccggcaa ggctggagag cgaggtgttc
   1920
   ccggaccccc tggcgctgtc ggtcctgctg gcaaagatgg agaggctgga gctcagggac
   1980
   cccctggccc tgctggtccc gctggcgaga gaggtgaaca aggccctgct ggctcccccg
   2040
   gattccaggg tctccctggt cctgctggtc ctccaggtga agcaggcaaa cctggtgaac
   2100
   agggtgttcc tggagacctt ggcgcccctg gcccctctgg agcaagaggc gagagaggtt
   2160
   tccctggcga gcgtggtgtg caaggtcccc ctggtcctgc tggaccccga ggggccaacg
   2220
   gtgctcccgg caacgatggt gctaagggtg atgctggtgc ccctggagct cccggtagcc
   2280
   agggcgcccc tggccttcag ggaatgcctg gtgaacgtgg tgcagctggt cttccagggc
   2340
   ctaagggtga cagaggtgat gctggtccca aaggtgctga tggctctcct ggcaaagatg
   2400
   gcgtccgtgg tctgaccggc cccattggtc ctcctggccc tgctggtgcc cctggtgaca
   2460
   agggtgaaag tggtcccagc ggccctgctg gtcccactgg agctcgtggt gcccccggag
   2520
   accgtggtga gcctggtccc cccggccctg ctggctttgc tggcccccct ggtgctgacg
   2580
   gccaacctgg tgctaaaggc gaacctggtg atgctggtgc caaaggcgat gctggtcccc
   2640
   ctgggcctgc cggacccgct ggaccccctg gccccattgg taatgttggt gctcctggag
   2700
   ccaaaggtgc tcgcggcagc gctggtcccc ctggtgctac tggtttccct ggtgctgctg
   2760
   gccgagtcgg tcctcctggc ccctctggaa atgctggacc ccctggccct cctggtcctg
   2820
   ctggcaaaga aggcggcaaa ggtccccgtg gtgagactgg ccctgctgga cgtcctggtg
   2880
   aagttggtcc ccctggtccc cctggccctg ctggcgagaa aggatcccct ggtgctgatg
   2940
   gtcctgctgg tgctcctggt actcccgggc ctcaaggtat tgctggacag cgtggtgtgg
   3000
   tcggcctgcc tggtcagaga ggagagagag gcttccctgg tcttcctggc ccctctggtg
   3060
   aacctggcaa acaaggtccc tctggagcaa gtggtgaacg tggtcccccc ggtcccatgg
   3120
   gcccccctgg attggctgga ccccctggtg aatctggacg tgagggggct cctgctgccg
   3180
   aaggttcccc tggacgagac ggttctcctg gcgccaaggg tgaccgtggt gagaccggcc
   3240
   ccgctggacc ccctggtgct cctggtgctc ctggtgcccc tggccccgtt ggccctgctg
   3300
   gcaagagtgg tgatcgtggt gagactggtc ctgctggtcc cgccggtccc gtcggccccg
   3360
   tcggcgcccg tggccccgcc ggaccccaag gcccccgtgg tgacaagggt gagacaggcg
   3420
   aacagggcga cagaggcata aagggtcacc gtggcttctc tggcctccag ggtccccctg
   3480
   gccctcctgg ctctcctggt gaacaaggtc cctctggagc ctctggtcct gctggtcccc
   3540
   gaggtccccc tggctctgct ggtgctcctg gcaaagatgg actcaacggt ctccctggcc
   3600
   ccattgggcc ccctggtcct cgcggtcgca ctggtgatgc tggtcctgtt ggtccccccg
   3660
   gccctcctgg acctcctggt ccccctggtc ctcccagcgc tggtttcgac ttcagcttcc
   3720
   tgccccagcc acctcaagag aaggctcacg atggtggccg ctactaccgg gctgatgatg
   3780
   ccaatgtggt tcgtgaccgt gacctcgagg tggacaccac cctcaagagc ctgagccagc
   3840
   agatcgagaa catccggagc ccagagggaa gccgcaagaa ccccgcccgc acctgccgtg
   3900
   acctcaagat gtgccactct gactggaaga gtggagagta ctggattgac cccaaccaag
   3960
   gctgcaacct ggatgccatc aaagtcttct gcaacatgga gactggtgag acctgcgtgt
   4020
   accccactca gcccagtgtg gcccagaaga actggtacat cagcaagaac cccaaggaca
   4080
   agaggcatgt ctggttcggc gagagcatga ccgatggatt ccagttcgag tatggcggcc
   4140
   agggctccga ccctgccgat gtggccatcc agctgacctt cctgcgcctg atgtccaccg
   4200
   aggcctccca gaacatcacc taccactgca agaacagcgt ggcctacatg gaccagcaga
   4260
   ctggcaacct caagaaggcc ctgctcctca agggctccaa cgagatcgag atccgcgccg
   4320
   agggcaacag ccgcttcacc tacagcgtca ctgtcgatgg ctgcacgagt cacaccggag
   4380
   cctggggcaa gacagtgatt gaatacaaaa ccaccaagtc ctcccgcctg cccatcatcg
   4440
   atgtggcccc cttggacgtt ggtgccccag accaggaatt cggcttcgac gttggccctg
   4500
   tctgcttcct gtaaactccc tccatcccaa cctggctccc tcccacccaa ccaactttcc
   4560
   ccccaacccg gaaacagaca agcaacccaa actgaacccc cccaaaagcc aaaaaatggg
   4620
   agacaatttc acatggactt tggaaaatat ttttttcctt tgcattcatc tctcaaactt
   4680
   agtttttatc tttgaccaac cgaacatgac caaaaaccaa aagtgcattc aaccttacca
   4740
   aaaaaaaaaa aaaaaaaaaa agaataaata aataagtttt taaaaaagga agcttggtcc
   4800
   acttgcttga agacccatgc gggggtaagt ccctttctgc ccgttgggtt atgaaacccc
   4860
   aatgctgccc tttctgctcc tttctccaca ccccccttgg cctcccctcc actccttccc
   4920
   aaatctgtct ccccagaaga cacaggaaac aatgtattgt ctgcccagca atcaaaggca
   4980
   atgctcaaac acccaagtgg cccccaccct cagcccgctc ctgcccgccc agcaccccca
   5040
   ggccctgggg acctggggtt ctcagactgc caaagaagcc ttgccatctg gcgctcccat
   5100
   ggctcttgca acatctcccc ttcgtttttg agggggtcat gccgggggag ccaccagccc
   5160
   ctcactgggt tcggaggaga gtcaggaagg gccacgacaa agcagaaaca tcggatttgg
   5220
   ggaacgcgtg tcatcccttg tgccgcaggc tgggcgggag agactgttct gttctgttcc
   5280
   ttgtgtaact gtgttgctga aagactacct cgttcttgtc ttgatgtgtc accggggcaa
   5340
   ctgcctgggg gcggggatgg gggcagggtg gaagcggctc cccattttta taccaaaggt
   5400
   gctacatcta tgtgatgggt ggggtgggga gggaatcact ggtgctatag aaattgagat
   5460
   gcccccccag gccagcaaat gttccttttt gttcaaagtc tatttttatt ccttgatatt
   5520
   ttttctttct tttttttttt ttttgtggat ggggacttgt gaatttttct aaaggtgcta
   5580
   tttaacatgg gaggagagcg tgtgcgctcc agcccagccc gctgctcact ttccaccctc
   5640
   tctccacctg cctctggctt ctcaggcctc tgctctccga cctctctcct ctgaaaccct
   5700
   cctccacagc tgcagcccat cctcccggct ccctcctagt ctgtcctgcg tcctctgtcc
   5760
   ccgggtttca gagacaactt cccaaagcac aaagcagttt ttccctaggg gtgggaggaa
   5820
   gcaaaagact ctgtacctat tttgtatgtg tataataatt tgagatgttt ttaattattt
   5880
   tgattgctgg aataaagcat gtggaaatga cccaaacata atccgcagtg gcctcctaat
   5940
   ttccttcttt ggagttgggg gaggggtaga catggggaag gggccttggg gtgatgggct
   6000
   tgccttccat tcctgccctt tccctcccca ctattctctt ctagatccct ccataacccc
   6060
   actccccttt ctctcaccct tcttataccg caaacctttc tacttcctct ttcattttct
   6120
   attcttgcaa tttccttgca ccttttccaa atcctcttct cccctgcaat accatacagg
   6180
   caatccacgt gcacaacaca cacacacact cttcacatct ggggttgtcc aaacctcata
   6240
   cccactcccc ttcaagccca tccactctcc accccctgga tgccctgcac ttggtggcgg
   6300
   tgggatgctc atggatactg ggagggtgag gggagtggaa cccgtgagga ggacctgggg
   6360
   gcctctcctt gaactgacat gaagggtcat ctggcctctg ctcccttctc acccacgctg
   6420
   acctcctgcc gaaggagcaa cgcaacagga gaggggtctg ctgagcctgg cgagggtctg
   6480
   ggagggacca ggaggaaggc gtgctccctg ctcgctgtcc tggccctggg ggagtgaggg
   6540
   agacagacac ctgggagagc tgtggggaag gcactcgcac cgtgctcttg ggaaggaagg
   6600
   agacctggcc ctgctcacca cggactgggt gcctcgacct cctgaatccc cagaacacaa
   6660
   cccccctggg ctggggtggt ctggggaacc atcgtgcccc cgcctcccgc ctactccttt
   6720
   ttaagctt
   6728

<210> 40
<211> 6728

<212>  DNA
<213>  Homo sapiens

<400>  40
agcagacggg agtttctcct cggggtcgga gcaggaggca cgcggagtgt gaggccacgc
60

atgagcggac gctaaccccc tccccagcca caaagagtct acatgtctag ggtctagaca
120

tgttcagctt tgtggacctc cggctcctgc tcctcttagc ggccaccgcc ctcctgacgc
180

acggccaaga ggaaggccaa gtcgagggcc aagacgaaga catcccacca atcacctgcg
240

tacagaacgg cctcaggtac catgaccgag acgtgtggaa acccgagccc tgccggatct
300

gcgtctgcga caacggcaag gtgttgtgcg atgacgtgat ctgtgacgag accaagaact
360

gccccggcgc cgaagtcccc gagggcgagt gctgtcccgt ctgccccgac ggctcagagt
420

cacccaccga ccaagaaacc accggcgtcg agggacccaa gggagacact ggcccccgag
480

gcccaagggg acccgcaggc ccccctggcc gagatggcat ccctggacag cctggacttc
540

ccggaccccc cggacccccc ggacctcccg gaccccctgg cctcggagga aactttgctc
600

cccagctgtc ttatggctat gatgagaaat caaccggagg aatttccgtg cctggcccca
660

tgggtccctc tggtcctcgt ggtctccctg gcccccctgg tgcacctggt ccccaaggct
720

tccaaggtcc ccctggtgag cctggcgagc ctggagcttc aggtcccatg ggtccccgag
780

gtcccccagg tccccctgga aagaatggag atgatgggga agctggaaaa cctggtcgtc
840

ctggtgagcg tgggcctcct gggcctcagg gtgctcgagg attgcccgga acagctggcc
900

tccctggaat gaagggacac agaggtttca gtggtttgga tggtgccaag ggagatgctg
960

gtcctgctgg tcctaagggt gagcctggca gccctggtga aaatggagct cctggtcaga
1020

tgggcccccg tggcctgcct ggtgagagag gtcgccctgg agcccctggc cctgctggtg
1080

ctcgtggaaa tgatggtgct actggtgctg ccgggccccc tggtcccacc ggccccgctg
1140

gtcctcctgg cttccctggt gctgttggtg ctaagggtga agctggtccc caagggcccc
1200

gaggctctga aggtccccag ggtgtgcgtg gtgagcctgg cccccctggc cctgctggtg
1260

ctgctggccc tgctggaaac cctggtgctg atggacagcc tggtgctaaa ggtgccaatg
1320

gtgctcctgg tattgctggt gctcctggct tccctggtgc ccgaggcccc tctggacccc
1380

agggccccgg cggccctcct ggtcccaagg gtaacagcgg tgaacctggt gctcctggca
1440

gcaaaggaga cactggtgct aagggagagc ctggccctgt tggtgttcaa ggaccccctg
1500

gccctgctgg agaggaagga aagcgaggag ctcgaggtga acccggaccc actggcctgc
1560

ccggaccccc tggcgagcgt ggtggacctg gtagccgtgg tttccctggc gcagatggtg
1620

ttgctggtcc caagggtccc gctggtgaac gtggttctcc tggccccgct ggccccaaag
1680

gatctcctgg tgaagctggt cgtcccggtg aagctggtct gcctggtgcc aagggtctga
1740

ctggaagccc tggcagccct ggtcctgatg gcaaaactgg cccccctggt cccgccggtc
1800

aagatggtcg ccccggaccc ccaggcccac ctggtgcccg tggtcaggct ggtgtgatgg
1860

gattccctgg acctaaaggt gctgctggag agcccggcaa ggctggagag cgaggtgttc
1920

ccggaccccc tggcgctgtc ggtcctgctg gcaaagatgg agaggctgga gctcagggac
1980

cccctggccc tgctggtccc gctggcgaga gaggtgaaca aggccctgct ggctcccccg
2040

gattccaggg tctccctggt cctgctggtc ctccaggtga agcaggcaaa cctggtgaac
2100

agggtgttcc tggagacctt ggcgcccctg gcccctctgg agcaagaggc gagagaggtt
2160

tccctggcga gcgtggtgtg caaggtcccc ctggtcctgc tggaccccga ggggccaacg
2220

gtgctcccgg caacgatggt gctaagggtg atgctggtgc ccctggagct cccggtagcc
2280

agggcgcccc tggccttcag ggaatgcctg gtgaacgtgg tgcagctggt cttccagggc
2340

ctaagggtga cagaggtgat gctggtccca aaggtgctga tggctctcct ggcaaagatg
2400

gcgtccgtgg tctgaccggc cccattggtc ctcctggccc tgctggtgcc cctggtgaca
2460

agggtgaaag tggtcccagc ggccctgctg gtcccactgg agctcgtggt gcccccggag
2520

accgtggtga gcctggtccc cccggccctg ctggctttgc tggcccccct ggtgctgacg
2580

gccaacctgg tgctaaaggc gaacctggtg atgctggtgc caaaggcgat gctggtcccc
2640

ctgggcctgc cggacccgct ggaccccctg gccccattgg taatgttggt gctcctggag
2700

ccaaaggtgc tcgcggcagc gctggtcccc ctggtgctac tggtttccct ggtgctgctg
2760

gccgagtcgg tcctcctggc ccctctggaa atgctggacc ccctggccct cctggtcctg
2820

ctggcaaaga aggcggcaaa ggtccccgtg gtgagactgg ccctgctgga cgtcctggtg
2880

aagttggtcc ccctggtccc cctggccctg ctggcgagaa aggatcccct ggtgctgatg
2940

gtcctgctgg tgctcctggt actcccgggc ctcaaggtat tgctggacag cgtggtgtgg
3000

tcggcctgcc tggtcagaga ggagagagag gcttccctgg tcttcctggc ccctctggtg
3060

aacctggcaa acaaggtccc tctggagcaa gtggtgaacg tggtcccccc ggtcccatgg
3120

gcccccctgg attggctgga ccccctggtg aatctggacg tgagggggct cctgctgccg
3180

aaggttcccc tggacgagac ggttctcctg gcgccaaggg tgaccgtggt gagaccggcc
3240

ccgctggacc ccctggtgct cctggtgctc ctggtgcccc tggccccgtt ggccctgctg
3300

gcaagagtgg tgatcgtggt gagactggtc ctgctggtcc cgccggtccc gtcggccccg
3360

tcggcgcccg tggccccgcc ggaccccaag gcccccgtgg tgacaagggt gagacaggcg
3420

aacagggcga cagaggcata aagggtcacc gtggcttctc tggcctccag ggtccccctg
3480

gccctcctgg ctctcctggt gaacaaggtc cctctggagc ctctggtcct gctggtcccc
3540

gaggtccccc tggctctgct ggtgctcctg gcaaagatgg actcaacggt ctccctggcc
3600

ccattgggcc ccctggtcct cgcggtcgca ctggtgatgc tggtcctgtt ggtccccccg
3660

gccctcctgg acctcctggt ccccctggtc ctcccagcgc tggtttcgac ttcagcttcc
3720

tgccccagcc acctcaagag aaggctcacg atggtggccg ctactaccgg gctgatgatg
3780

ccaatgtggt tcgtgaccgt gacctcgagg tggacaccac cctcaagagc ctgagccagc
3840

agatcgagaa catccggagc ccagagggaa gccgcaagaa ccccgcccgc acctgccgtg
3900

acctcaagat gtgccactct gactggaaga gtggagagta ctggattgac cccaaccaag
3960

gctgcaacct ggatgccatc aaagtcttct gcaacatgga gactggtgag acctgcgtgt
4020

accccactca gcccagtgtg gcccagaaga actggtacat cagcaagaac cccaaggaca
4080

agaggcatgt ctggttcggc gagagcatga ccgatggatt ccagttcgag tatggcggcc
4140

agggctccga ccctgccgat gtggccatcc agctgacctt cctgcgcctg atgtccaccg
4200

aggcctccca gaacatcacc taccactgca agaacagcgt ggcctacatg gaccagcaga
4260

ctggcaacct caagaaggcc ctgctcctca agggctccaa cgagatcgag atccgcgccg
4320

agggcaacag ccgcttcacc tacagcgtca ctgtcgatgg ctgcacgagt cacaccggag
4380

cctggggcaa gacagtgatt gaatacaaaa ccaccaagtc ctcccgcctg cccatcatcg
4440

atgtggcccc cttggacgtt ggtgccccag accaggaatt cggcttcgac gttggccctg
4500

tctgcttcct gtaaactccc tccatcccaa cctggctccc tcccacccaa ccaactttcc
4560

ccccaacccg gaaacagaca agcaacccaa actgaacccc cccaaaagcc aaaaaatggg
4620

agacaatttc acatggactt tggaaaatat ttttttcctt tgcattcatc tctcaaactt
4680

agtttttatc tttgaccaac cgaacatgac caaaaaccaa aagtgcattc aaccttacca
4740

aaaaaaaaaa aaaaaaaaaa agaataaata aataagtttt taaaaaagga agcttggtcc
4800

acttgcttga agacccatgc gggggtaagt ccctttctgc ccgttgggtt atgaaacccc
4860

aatgctgccc tttctgctcc tttctccaca ccccccttgg cctcccctcc actccttccc
4920

aaatctgtct ccccagaaga cacaggaaac aatgtattgt ctgcccagca atcaaaggca
4980

atgctcaaac acccaagtgg cccccaccct cagcccgctc ctgcccgccc agcaccccca
5040

ggccctgggg acctggggtt ctcagactgc caaagaagcc ttgccatctg gcgctcccat
5100

ggctcttgca acatctcccc ttcgtttttg agggggtcat gccgggggag ccaccagccc
5160

ctcactgggt tcggaggaga gtcaggaagg gccacgacaa agcagaaaca tcggatttgg
5220

ggaacgcgtg tcatcccttg tgccgcaggc tgggcgggag agactgttct gttctgttcc
5280

ttgtgtaact gtgttgctga aagactacct cgttcttgtc ttgatgtgtc accggggcaa
5340

ctgcctgggg gcggggatgg gggcagggtg gaagcggctc cccattttta taccaaaggt
5400

gctacatcta tgtgatgggt ggggtgggga gggaatcact ggtgctatag aaattgagat
5460

gcccccccag gccagcaaat gttccttttt gttcaaagtc tatttttatt ccttgatatt
5520

ttttctttct tttttttttt ttttgtggat ggggacttgt gaatttttct aaaggtgcta
5580

tttaacatgg gaggagagcg tgtgcgctcc agcccagccc gctgctcact ttccaccctc
5640

tctccacctg cctctggctt ctcaggcctc tgctctccga cctctctcct ctgaaaccct
5700

cctccacagc tgcagcccat cctcccggct ccctcctagt ctgtcctgcg tcctctgtcc
5760

ccgggtttca gagacaactt cccaaagcac aaagcagttt ttccctaggg gtgggaggaa
5820

gcaaaagact ctgtacctat tttgtatgtg tataataatt tgagatgttt ttaattattt
5880

tgattgctgg aataaagcat gtggaaatga cccaaacata atccgcagtg gcctcctaat
5940

ttccttcttt ggagttgggg gaggggtaga catggggaag gggccttggg gtgatgggct
6000

tgccttccat tcctgccctt tccctcccca ctattctctt ctagatccct ccataacccc
6060

actccccttt ctctcaccct tcttataccg caaacctttc tacttcctct ttcattttct
6120

attcttgcaa tttccttgca ccttttccaa atcctcttct cccctgcaat accatacagg
6180

caatccacgt gcacaacaca cacacacact cttcacatct ggggttgtcc aaacctcata
6240

cccactcccc ttcaagccca tccactctcc accccctgga tgccctgcac ttggtggcgg
6300

tgggatgctc atggatactg ggagggtgag gggagtggaa cccgtgagga ggacctgggg
6360

gcctctcctt gaactgacat gaagggtcat ctggcctctg ctcccttctc acccacgctg
6420

acctcctgcc gaaggagcaa cgcaacagga gaggggtctg ctgagcctgg cgagggtctg
6480

ggagggacca ggaggaaggc gtgctccctg ctcgctgtcc tggccctggg ggagtgaggg
6540

agacagacac ctgggagagc tgtggggaag gcactcgcac cgtgctcttg ggaaggaagg
6600

agacctggcc ctgctcacca cggactgggt gcctcgacct cctgaatccc cagaacacaa
6660

cccccctggg ctggggtggt ctggggaacc atcgtgcccc cgcctcccgc ctactccttt
6720

ttaagctt
6728

<210> 41
<211> 6728
<212> DNA
<213> Homo sapiens

<400> 41
agcagacggg agtttctcct cggggtcgga gcaggaggca cgcggagtgt gaggccacgc
60

atgagcggac gctaaccccc tccccagcca caaagagtct acatgtctag ggtctagaca
120

tgttcagctt tgtggacctc cggctcctgc tcctcttagc ggccaccgcc ctcctgacgc
180

acggccaaga ggaaggccaa gtcgagggcc aagacgaaga catcccacca atcacctgcg
240

tacagaacgg cctcaggtac catgaccgag acgtgtggaa acccgagccc tgccggatct
300

gcgtctgcga caacggcaag gtgttgtgcg atgacgtgat ctgtgacgag accaagaact
360

gccccggcgc cgaagtcccc gagggcgagt gctgtcccgt ctgccccgac ggctcagagt
420

cacccaccga ccaagaaacc accggcgtcg agggacccaa gggagacact ggcccccgag
480

gcccaagggg acccgcaggc ccccctggcc gagatggcat ccctggacag cctggacttc
540

ccggaccccc cggacccccc ggacctcccg gaccccctgg cctcggagga aactttgctc
600

cccagctgtc ttatggctat gatgagaaat caaccggagg aatttccgtg cctggcccca
660

tgggtccctc tggtcctcgt ggtctccctg gcccccctgg tgcacctggt ccccaaggct
720

tccaaggtcc ccctggtgag cctggcgagc ctggagcttc aggtcccatg ggtccccgag
780

gtcccccagg tccccctgga aagaatggag atgatgggga agctggaaaa cctggtcgtc
840

ctggtgagcg tgggcctcct gggcctcagg gtgctcgagg attgcccgga acagctggcc
900

tccctggaat gaagggacac agaggtttca gtggtttgga tggtgccaag ggagatgctg

960

gtcctgctgg tcctaagggt gagcctggca gccctggtga aaatggagct cctggtcaga
1020

tgggcccccg tggcctgcct ggtgagagag gtcgccctgg agcccctggc cctgctggtg
1080

ctcgtggaaa tgatggtgct actggtgctg ccgggccccc tggtcccacc ggccccgctg
1140

gtcctcctgg cttccctggt gctgttggtg ctaagggtga agctggtccc caagggcccc
1200

gaggctctga aggtccccag ggtgtgcgtg gtgagcctgg cccccctggc cctgctggtg
1260

ctgctggccc tgctggaaac cctggtgctg atggacagcc tggtgctaaa ggtgccaatg
1320

gtgctcctgg tattgctggt gctcctggct tccctggtgc ccgaggcccc tctggacccc
1380

agggccccgg cggccctcct ggtcccaagg gtaacagcgg tgaacctggt gctcctggca
1440

gcaaaggaga cactggtgct aagggagagc ctggccctgt tggtgttcaa ggaccccctg
1500

gccctgctgg agaggaagga aagcgaggag ctcgaggtga acccggaccc actggcctgc
1560

ccggaccccc tggcgagcgt ggtggacctg gtagccgtgg tttccctggc gcagatggtg
1620

ttgctggtcc caagggtccc gctggtgaac gtggttctcc tggccccgct ggccccaaag
1680

gatctcctgg tgaagctggt cgtcccggtg aagctggtct gcctggtgcc aagggtctga
1740

ctggaagccc tggcagccct ggtcctgatg gcaaaactgg cccccctggt cccgccggtc
1800

aagatggtcg ccccggaccc ccaggcccac ctggtgcccg tggtcaggct ggtgtgatgg
1860

gattccctgg acctaaaggt gctgctggag agcccggcaa ggctggagag cgaggtgttc
1920

ccggaccccc tggcgctgtc ggtcctgctg gcaaagatgg agaggctgga gctcagggac
1980

cccctggccc tgctggtccc gctggcgaga gaggtgaaca aggccctgct ggctcccccg
2040

gattccaggg tctccctggt cctgctggtc ctccaggtga agcaggcaaa cctggtgaac
2100

agggtgttcc tggagacctt ggcgcccctg gcccctctgg agcaagaggc gagagaggtt
2160

tccctggcga gcgtggtgtg caaggtcccc ctggtcctgc tggaccccga ggggccaacg
2220

gtgctcccgg caacgatggt gctaagggtg atgctggtgc ccctggagct cccggtagcc

2280

agggcgcccc tggccttcag ggaatgcctg gtgaacgtgg tgcagctggt cttccagggc
2340

ctaagggtga cagaggtgat gctggtccca aaggtgctga tggctctcct ggcaaagatg
2400

gcgtccgtgg tctgaccggc cccattggtc ctcctggccc tgctggtgcc cctggtgaca
2460

agggtgaaag tggtcccagc ggccctgctg gtcccactgg agctcgtggt gcccccggag
2520

accgtggtga gcctggtccc cccggccctg ctggctttgc tggcccccct ggtgctgacg
2580

gccaacctgg tgctaaaggc gaacctggtg atgctggtgc caaaggcgat gctggtcccc
2640

ctgggcctgc cggacccgct ggaccccctg gccccattgg taatgttggt gctcctggag
2700

ccaaaggtgc tcgcggcagc gctggtcccc ctggtgctac tggtttccct ggtgctgctg
2760

gccgagtcgg tcctcctggc ccctctggaa atgctggacc ccctggccct cctggtcctg
2820

ctggcaaaga aggcggcaaa ggtccccgtg gtgagactgg ccctgctgga cgtcctggtg
2880

aagttggtcc ccctggtccc cctggccctg ctggcgagaa aggatcccct ggtgctgatg
2940

gtcctgctgg tgctcctggt actcccgggc ctcaaggtat tgctggacag cgtggtgtgg
3000

tcggcctgcc tggtcagaga ggagagagag gcttccctgg tcttcctggc ccctctggtg
3060

aacctggcaa acaaggtccc tctggagcaa gtggtgaacg tggtcccccc ggtcccatgg
3120

gcccccctgg attggctgga ccccctggtg aatctggacg tgagggggct cctgctgccg
3180

aaggttcccc tggacgagac ggttctcctg gcgccaaggg tgaccgtggt gagaccggcc
3240

ccgctggacc ccctggtgct cctggtgctc ctggtgcccc tggccccgtt ggccctgctg
3300

gcaagagtgg tgatcgtggt gagactggtc ctgctggtcc cgccggtccc gtcggccccg
3360

tcggcgcccg tggccccgcc ggaccccaag gcccccgtgg tgacaagggt gagacaggcg
3420

aacagggcga cagaggcata aagggtcacc gtggcttctc tggcctccag ggtccccctg
3480

gccctcctgg ctctcctggt gaacaaggtc cctctggagc ctctggtcct gctggtcccc
3540

gaggtccccc tggctctgct ggtgctcctg gcaaagatgg actcaacggt ctccctggcc

3600

ccattgggcc ccctggtcct cgcggtcgca ctggtgatgc tggtcctgtt ggtcccccg
3660

gccctcctgg acctcctggt ccccctggtc ctcccagcgc tggtttcgac ttcagcttcc
3720

tgccccagcc acctcaagag aaggctcacg atggtggccg ctactaccgg gctgatgatg
3780

ccaatgtggt tcgtgaccgt gacctcgagg tggacaccac cctcaagagc ctgagccagc
3840

agatcgagaa catccggagc ccagagggaa gccgcaagaa ccccgcccgc acctgccgtg
3900

acctcaagat gtgccactct gactggaaga gtggagagta ctggattgac cccaaccaag
3960

gctgcaacct ggatgccatc aaagtcttct gcaacatgga gactggtgag acctgcgtgt
4020

accccactca gcccagtgtg gcccagaaga actggtacat cagcaagaac cccaaggaca
4080

agaggcatgt ctggttcggc gagagcatga ccgatggatt ccagttcgag tatggcggcc
4140

agggctccga ccctgccgat gtggccatcc agctgacctt cctgcgcctg atgtccaccg
4200

aggcctccca gaacatcacc taccactgca agaacagcgt ggcctacatg gaccagcaga
4260

ctggcaacct caagaaggcc ctgctcctca agggctccaa cgagatcgag atccgcgccg
4320

agggcaacag ccgcttcacc tacagcgtca ctgtcgatgg ctgcacgagt cacaccggag
4380

cctggggcaa gacagtgatt gaatacaaaa ccaccaagtc ctcccgcctg cccatcatcg
4440

atgtggcccc cttggacgtt ggtgccccag accaggaatt cggcttcgac gttggccctg
4500

tctgcttcct gtaaactccc tccatcccaa cctggctccc tcccacccaa ccaactttcc
4560

ccccaacccg gaaacagaca agcaacccaa actgaacccc cccaaaagcc aaaaaatggg
4620

agacaatttc acatggactt tggaaaatat ttttttcctt tgcattcatc tctcaaactt
4680

agtttttatc tttgaccaac cgaacatgac caaaaaccaa aagtgcattc aaccttacca
4740

aaaaaaaaaa aaaaaaaaaa agaataaata aataagtttt taaaaaagga agcttggtcc
4800

acttgcttga agacccatgc gggggtaagt ccctttctgc ccgttgggtt atgaaacccc
4860

aatgctgccc tttctgctcc tttctccaca ccccccttgg cctcccctcc actccttccc

4920

aaatctgtct ccccagaaga cacaggaaac aatgtattgt ctgcccagca atcaaaggca
4980

atgctcaaac acccaagtgg cccccaccct cagcccgctc ctgcccgccc agcaccccca
5040

ggccctgggg acctggggtt ctcagactgc caaagaagcc ttgccatctg gcgctcccat
5100

ggctcttgca acatctcccc ttcgtttttg agggggtcat gccgggggag ccaccagccc
5160

ctcactgggt tcggaggaga gtcaggaagg gccacgacaa agcagaaaca tcggatttgg
5220

ggaacgcgtg tcatcccttg tgccgcaggc tgggcgggag agactgttct gttctgttcc
5280

ttgtgtaact gtgttgctga aagactacct cgttcttgtc ttgatgtgtc accggggcaa
5340

ctgcctgggg gcggggatgg gggcagggtg gaagcggctc cccattttta taccaaaggt
5400

gctacatcta tgtgatgggt ggggtgggga gggaatcact ggtgctatag aaattgagat
5460

gcccccccag gccagcaaat gttccttttt gttcaaagtc tatttttatt ccttgatatt
5520

ttttctttct tttttttttt ttttgtggat ggggacttgt gaatttttct aaaggtgcta
5580

tttaacatgg gaggagagcg tgtgcgctcc agcccagccc gctgctcact ttccaccctc
5640

tctccacctg cctctggctt ctcaggcctc tgctctccga cctctctcct ctgaaaccct
5700

cctccacagc tgcagcccat cctcccggct ccctcctagt ctgtcctgcg tcctctgtcc
5760

ccgggtttca gagacaactt cccaaagcac aaagcagttt ttccctaggg gtgggaggaa
5820

gcaaaagact ctgtacctat tttgtatgtg tataataatt tgagatgttt ttaattattt
5880

tgattgctgg aataaagcat gtggaaatga cccaaacata atccgcagtg gcctcctaat
5940

ttccttcttt ggagttgggg gaggggtaga catggggaag gggccttggg gtgatgggct
6000

tgccttccat tcctgccctt tccctcccca ctattctctt ctagatccct ccataacccc
6060

actccccttt ctctcaccct tcttataccg caaacctttc tacttcctct ttcattttct
6120

attcttgcaa tttccttgca ccttttccaa atcctcttct cccctgcaat accatacagg
6180

caatccacgt gcacaacaca cacacacact cttcacatct ggggttgtcc aaacctcata

6240

cccactcccc ttcaagccca tccactctcc accccctgga tgccctgcac ttggtggcgg
6300

tgggatgctc atggatactg ggagggtgag gggagtggaa cccgtgagga ggacctgggg
6360

gcctctcctt gaactgacat gaagggtcat ctggcctctg ctcccttctc acccacgctg
6420

acctcctgcc gaaggagcaa cgcaacagga gaggggtctg ctgagcctgg cgagggtctg
6480

ggagggacca ggaggaaggc gtgctccctg ctcgctgtcc tggccctggg ggagtgaggg
6540

agacagacac ctgggagagc tgtggggaag gcactcgcac cgtgctcttg ggaaggaagg
6600

agacctggcc ctgctcacca cggactgggt gcctcgacct cctgaatccc cagaacacaa
6660

cccccctggg ctggggtggt ctggggaacc atcgtgcccc cgcctcccgc ctactccttt
6720

ttaagctt
6728

<210> 42
<211> 597
<212> DNA
<213> Homo sapiens

<400> 42
agctgaagtt gaggatctct tactctctaa gccacggaat taacccgagc aggcatggag
60

gcctctgctc tcacctcatc agcagtgacc agtgtggcca aagtggtcag ggtggcctct
120

ggctctgccg tagttttgcc cctggccagg attgctacag ttgtgattgg aggagttgtg
180

gccatggcgg ctgtgcccat ggtgctcagt gccatgggct tcactgcggc gggaatcgcc
240

tcgtcctcca tagcagccaa gatgatgtcc gcggcggcca ttgccaatgg gggtggagtt
300

gcctcgggca gccttgtggg tactctgcag tcactgggag caactggact ctccggattg
360

accaagttca tcctgggctc cattgggtct gccattgcgg ctgtcattgc gaggttctac
420

tagctccctg cccctcgccc tgcagagaag agaaccatgc caggggagaa ggcacccagc
480

catcctgacc cagcgaggag ccaactatcc caaatatacc tgggtgaaat ataccaaatt
540

ctgcatctcc agaggaaaat aagaaataaa gatgaattgt tgcaactctt aaaaaaa
597

<210> 43
<211> 1647
<212> DNA
<213> Homo sapiens

<400> 43
ggcaggcatg ggagccgcgc gctctctccc ggcgcccaca cctgtctgag cggcgcagcg
60

agccgcggcc cgggcgggct gctcggcgcg gaacagtgct cggcatggca gggattccag
120

ggctcctctt ccttctcttc tttctgctct gtgctgttgg gcaagtgagc ccttacagtg
180

ccccctggaa acccacttgg cctgcatacc gcctccctgt cgtcttgccc cagtctaccc
240

tcaatttagc caagccagac tttggagccg aagccaaatt agaagtatct tcttcatgtg
300

gaccccagtg tcataaggga actccactgc ccacttacga agaggccaag caatatctgt
360

cttatgaaac gctctatgcc aatggcagcc gcacagagac gcaggtgggc atctacatcc
420

tcagcagtag tggagatggg gcccaacacc gagactcagg gtcttcagga aagtctcgaa
480

ggaagcggca gatttatggc tatgacagca ggttcagcat ttttgggaag gacttcctgc
540

tcaactaccc tttctcaaca tcagtgaagt tatccacggg ctgcaccggc accctggtgg
600

cagagaagca tgtcctcaca gctgcccact gcatacacga tggaaaaacc tatgtgaaag
660

gaacccagaa gcttcgagtg ggcttcctaa agcccaagtt taaagatggt ggtcgagggg
720

ccaacgactc cacttcagcc atgcccgagc agatgaaatt tcagtggatc cgggtgaaac
780

gcacccatgt gcccaagggt tggatcaagg gcaatgccaa tgacatcggc atggattatg
840

attatgccct cctggaactc aaaaagcccc acaagagaaa atttatgaag attggggtga
900

gccctcctgc taagcagctg ccagggggca gaattcactt ctctggttat gacaatgacc
960

gaccaggcaa tttggtgtat cgcttctgtg acgtcaaaga cgagacctat gacttgctct
1020

accagcaatg cgatgcccag ccaggggcca gcgggtctgg ggtctatgtg aggatgtgga
1080

agagacagca gcagaagtgg gagcgaaaaa ttattggcat tttttcaggg caccagtggg
1140

tggacatgaa tggttcccca caggatttca acgtggctgt cagaatcact cctctcaaat
1200

atgcccagat ttgctattgg attaaaggaa actacctgga ttgtagggag gggtgacaca
1260

gtgttccctc ctggcagcaa ttaagggtct tcatgttctt attttaggag aggccaaatt
1320

gttttttgtc attggcgtgc acacgtgtgt gtgtgtgtgt gtgtgtgtgt aaggtgtctt
1380

ataatctttt acctatttct tacaattgca agatgactgg ctttactatt tgaaaactgg
1440

tttgtgtatc atatcatata tcatttaagc agtttgaagg catacttttg catagaaata
1500

aaaaaaatac tgatttgggg caatgaggaa tatttgacaa ttaagttaat cttcacgttt
1560

ttgcaaactt tgatttttat ttcatctgaa cttgtttcaa agatttatat taaatatttg
1620

gcatacaaga gaaaaaaaaa aaaaaaa
1647


<210> 44
<211> 2446
<212> DNA
<213> Homo sapiens

<400> 44
gcgccgcggc gcggagtccc ggctgcggga tagaccgagg gccatggccg cctctcccgg
60

acccgccggc gttggcggcg ccggagcagt ctacggctcc ggctcttcgg gcttcgccct
120

cgactcggga ctggagatca aaactcgctc ggtggagcag acgctactcc cgctggtttc
180

tcagatcacc acgcttatta atcataaaga taataccaaa aagtctgata aaactctgca
240

agcaattcag cgtgtaggac aagctgtcaa cttggcagtt ggaagatttg ttaaagtagg
300

agaagctata gccaatgaaa actgggattt gaaagaagaa ataaatattg cttgtattga
360

agctaaacaa gcaggagaaa caattgcagc acttacagac ataaccaact tgaaccatct
420

ggaatctgat gggcagatca caatttttac agacaaaaca ggagtgataa aggctgcaag
480

attacttctt tcttcagtga caaaagtgtt gttgctggca gaccgagtag tcattaaaca
540

gataataaca tcaagaaata aggttctcgc aactatggaa agactagaga aagtgaatag
600

ctttcaagag tttgtccaaa tattcagtca atttggaaat gaaatggtgg agtttgcaca
660

tctgagtgga gatagacaaa atgatttgaa agatgaaaag aaaaaggcaa aaatggcagc

720

agctagggca gttcttgaaa agtgtacaat gatgcttctc acagcttcaa agacatgtct
780

gaggcatcct aactgcgaat cagcccataa aaacaaagaa ggagtatttg accgtatgaa
840

agtggcattg gataaggtca ttgaaattgt gactgactgt aaaccgaatg gagagactga
900

catttcatct atcagtattt ttactggaat taaggaattc aagatgaata ttgaagctct
960

tcgggagaat ctttattttc agtccaaaga gaacctttct gtgacattgg aagtcatctt
1020

ggagcgtatg gaggacttta ctgattctgc ctacaccagc catgagcaca gagaacgcat
1080

cttggaactg tcaactcagg cgagaatgga actgcagcag ttaatttctg tgtggattca
1140

agctcaaagc aagaaaacaa aaagcatcgc tgaagaactg gaactcagta ttttgaaaat
1200

cagtcacagt cttaatgaac ttaagaaaga acttcatagt acagcgacac agctggcagc
1260

agatctatta aaataccatg ctgatcatgt ggttctaaaa gcattaaaac ttactggagt
1320

agaaggaaat ttagaagctt tggctgaata tgcctgtaaa ctctctgaac agaaagagca
1380

gcttgttgag acctgtcgat tgttacgaca catatctggg acagaacctc tggaaataac
1440

ctgtatacat gcagaggaga catttcaggt gactggccaa cagataattt ctgctgctga
1500

aacattgaca ttgcatccat ctagtaaaat tgctaaagaa aacctagatg tattttgtga
1560

agcttgggaa tcccaaatta gtgacatgtc aacactgctg agagaaatca atgacgtgtt
1620

tgaaggaaga cgaggagaga agtatggcta cctttcactt ccaaagccaa tgaagaataa
1680

tgcaaacctg aaatcattaa agccagacaa gcctgactct gaggagcaag ccaagatagc
1740

aaagcttgga cttaagctgg gtttgctcac ctctgacgct gactgcgaaa ttgagaagtg
1800

ggaagatcag gagaatgaga ttgttcaata tggacggaac atgtccagta tggcctattc
1860

tctgtattta tttactagag gagaggggcc actgaaaact tcccaggatt taattcatca
1920

actagaggtt tttgctgcag agggtttaaa gcttacttcc agtgttcaag ctttttcaaa
1980

acagctgaaa gacgatgaca agcttatgct tctcctggaa ataaacaagc taattcctct

2040

atgccaccag ctccagacag taactaagac ttctttgcag aataaagtat ttctaaaggt
2100

tgacaagtgt attacgaaga caagatccat gatggctctc ttagtccaac ttctttcact
2160

ttgttataaa ctgctgaaga agcttcagat ggaaaataac ggatgggtct cagttacaaa
2220

taaggacact atggatagta aaacttgaga agcttttggg gtcagatctc tggaacatca
2280

tgtgatgaag ctgacatttt taaaaatcaa atgatccttt atcttttcag aaattcatca
2340

attttataaa gaaaacaata ttgaaatttt gctctatttt ctgatcatga aactgattgt
2400

aaagcttttt gacaactaat aaatgtcttg gtaattgcta gattct
2446

<210> 45
<211> 4221
<212> DNA
<213> Homo sapiens

<400> 45
gtcggccgtc ccctttaatt tttaaataca cggtcccctc ttttctctgg gggggcaag
60

caagaaatca aagaaggagg agacaagccg tcaattttct ccaaaacaaa ccccaccggg
120

caatttggtc tcggggtagg gggagacggg gtgattgcaa attattccag gacgagatcc
180

agttctccag cgggaaaggg gcaaaggaac gccgcgcgtt ggaagggcca gggtacgcag
240

ctccccttgc agcgcccgca ggacccccgc aagctcgtgc cggcgaaatc ggagaccgcc
300

gatctgtcct cgttctctcc tgcacgtctg gctgcattcg gaggaagacc tggggcgcga
360

gcgagcggcg acagcatgag cctgtgctga cctccgcgcg gcgggccgag cccagggctt
420

tgtcgcggta cctgcgccca gcccgcgccg caactctgtg cccagctttt gcaatctttt
480

gttgcagcgc tgaccgcacc aagttaaatg ctcccttgca atttttcttt tttttgtttg
540

tttgtttaat ttttggagag ctcgcgatct tggaaaagcc tcagacgcca tctacagtta
600

aaacgtaggt aactgccctc tcccgcaccc cccccttaca cgccccccac cctttccacc
660

aaaaaaaggg ggtgcagcgc ggattctggc tgccgtgcgt cgccagccgg tagacccgtg
720

cttgtttcct ttctcttttt gtttggcttc taacgcgttg ggactgagtc gccgccgtga
780

gctccccgaa gactgcacaa actaccgcgg gctcctccgc cccgtctgcg attcggaagc
840

cggcctgggg gtcgcgtcgg gagccctgcg ctgcagctcc gcaccttagc agcccgggta
900

ctcatccaga tccacgccgg ggacacacac acagagtaac taaaagtgcg gcgattctgc
960

acatcgccga ctgctttggg gtaacaaaaa gacccgagtt gcctgccgac cgaggacccc
1020

cgggagccgg gctcggagca gacgaggtat ccggcggcgc ccatttgggg gcttctaact
1080

ctttctccac gcagcccctc ttctgtcccc tcccctctcg ctccctttta aaatcagtgg
1140

caccgaggcg cctgcagccg cactcgccag cgactcatct ctccagcggg tttttttttg
1200

tttgtcgtgt gcgatcctca cactcatgaa catacacagg tctaccccca tcacaatagc
1260

gagatatggg agatcgcgga acaaaaccca ggatttcgaa gagttgtcgt ctataaggtc
1320

cgcggagccc agccagagtt tcagcccgaa cctcggctcc ccgagcccgc ccgagactcc
1380

gaacttgtcg cattgcgttt cttgtatcgg gaaatactta ttgttggaac ctctggaggg
1440

agaccacgtt tttcgtgccg tgcatctgca cagcggagag gagctggtgt gcaaggtgtt
1500

tgatatcagc tgctaccagg aatccctggc accgtgcttt tgcctgtctg ctcatagtaa
1560

catcaaccaa atcactgaaa ttatcctggg tgagaccaaa gcctatgtgt tctttgagcg
1620

aagctatggg gacatgcatt ccttcgtccg cacctgcaag aagctgagag aggaggaggc
1680

agccagactg ttctaccaga ttgcctcggc agtggcccac tgccatgacg gggggctggt
1740

gctgcgggac ctcaagctgc ggaaattcat ctttaaggac gaagagagga ctcgggtcaa
1800

gctggaaagc ctggaagacg cctacattct gcggggagat gatgattccc tctccgacaa
1860

gcatggctgc ccggcttacg taagcccaga gatcttgaac accagtggca gctactcggg
1920

caaagcagcc gacgtgtgga gcctgggggt gatgctgtac accatgttgg tggggcggta
1980

ccctttccat gacattgaac ccagctccct cttcagcaag atccggcgtg gccagttcaa
2040

cattccagag actctgtcgc ccaaggccaa gtgcctcatc cgaagcattc tgcgtcggga
2100

gccctcagag cggctgacct cgcaggaaat tctggaccat ccttggtttt ctacagattt
2160

tagcgtctcg aattcagcat atggtgctaa ggaagtgtct gaccagctgg tgccggacgt
2220

caacatggaa gagaacttgg accctttctt taactgagct catgccccac ggagacttag
2280

caggttccag gagtgagcga gggcagcgga aaggagttct tccgggggac acgaattgcc
2340

tggctgagta gcaagaaaga cacactctta agtttcttgg ttcagagcag gaaaaccttc
2400

aaggagctga ctgaccacgt agcatggggg caagaggcgt gggatgggga ttggggtgag
2460

atggatggga gcccgctgga gcttgtcttc cctaacatag cctgggagac caccccttgc
2520

cacttgggcc acttccgcct accccacttt tcattttgtt ccaaaatagt tgcagatcct
2580

gacagaatca aaactctctg cctcaaacac acatcctggc atcgcactgt tagcatttaa
2640

cttcttgtta ggattcaggg aaggaacagt tggccaagaa ttttttttct tttaaacaag
2700

ccaaccacct agctggtaat taatgaggtt cacttaaaaa aaaaattcgg tgcacacaga
2760

ctgacatgaa acctgggtgc tacagtaaaa gaaaacaaaa gtccagtttg tgtctcttaa
2820

tcgctcactt caactcattt cttctaaata aactatttaa tatcctggtc aggaaatgac
2880

atgttaatgc tttgctccct gaagggggaa aaaatctgtc ctttaacaag ctattctgtt
2940

ttgtgtcaat tgggtccgtg gcaaggaagc tattaggaag tcaaacggtc caggatgcat
3000

tacctgctaa tccttaggtt taaaggggga aagaaaaggg aagaagaaag gaaaagagaa
3060

atccaactcc tttttcatgt tttgcttttg aacaatgagg gtttgtgtga caggcattcc
3120

tctttgctga gatgatagca atggcctgag attttagcaa gctcctggag tctgatgctt
3180

ttgcagtact ctgatcgcaa ctaaacattt gtctttgttt tattagaaac tagtgaaaca
3240

aagcaggttg tcccacatgt ataaaataca gggcagctat ttagttttct ttacagagaa
3300

tgatcctttt aaggcttgta aggccctctg gtttggacaa aaaccctcag tagagacaag
3360

cgggaaggat aattagctga aagctatgat gatataaata aaaacagctc tctatcccaa
3420

tacgcacctt tgtattttca agaactcttc tatttattaa ggaaaatgtc acattgtgat
3480

gtattaagcc agtacttcaa ttacgggttg acttgggatg acatattaca tgctgtagtt
3540

aacatttata attctttttc cttgtttgag tatttctgtc tctgaaataa ccttttactt
3600

ggcttttcta gatagcttta tttgatttcg agtggcaaaa tgttttttat tacggctttt
3660

ctattgctgt atgatacaga actcttttgg cataaatatt tgtgttccca gtacctcact
3720

tgttcggatt tgactgcctg tatatgtttt gtgaaatggt cctgtttttg ggtaggtgac
3780

acgtggactc tagtatgtaa atgttacttg aatctgtgct tcataatagt gtgtggcatg
3840

tatgtgcaga ctcttggatg ctttatgcct gcgcaccagg agccctgtcc tcacgttccc
3900

aggagggcgg cttcaccctt cgtaaccagg agacaaggcg gccatggatt tgcccttgat
3960

tctattttgc taatggaaga tagaaaggag agaaggtttt tttttttttt taacattctg
4020

aagatggtgc tgtgtcaaga aggacctttt ttttcccctc tcccctattt tttaagtacc
4080

ttggaggagg agaggttggt gacatgcatg gtggggatct atggcctctg gtgctttgtc
4140

ctgtatttgg tttaatgttt ttgtcctaat ctcttcaatc aataaaattg tgcgtattta
4200

actaaaaaaa aaaaaaaaaa a
4221


<210> 46
<211> 1401
<212> DNA
<213> Homo sapiens

<400> 46
cgtattgctc ggcccgggga gtttcgcccc ctgcccggct ccgcggcgcg gaggatggcg
60

tggaaacggc tgggcgcgct ggtgatgttc cctctacaga tgatctatct ggtggtgaaa
120

gcagccgtcg gactggtgct gcccgccaag ctgcgggacc tgtcgcggga gaacgtcctc
180

atcaccggcg gcgggagagg catcgggcgt cagctcgccc gcgagttcgc ggagcgcggc
240

gccagaaaga ttgttctctg gggccggact gagaaatgcc tgaaggagac gacggaggag
300

atccggcaga tgggcactga gtgccattac ttcatctgtg atgtgggcaa ccgggaggag
360

gtgtaccaga cggccaaggc cgtccgggag aaggtgggtg acatcaccat cctggtgaac
420

aatgccgccg tggtccatgg gaagagccta atggacagtg atgatgatgc cctcctcaag
480

tcccaacaca tcaacaccct gggccagttc tggaccacca aggccttcct gccgcgtatg
540

ctggagctgc agaatggcca catcgtgtgc ctcaactccg tgctggcact gtctgccatc
600

cccggtgcca tcgactactg cacatccaaa gcgtcagcct tcgccttcat ggagagcctg
660

accctggggc tgctggactg tccgggagtc agcgccacca cagtgctgcc cttccacacc
720

agcaccgaga tgttccaggg catgagagtc aggtttccca acctctttcc cccactgaag
780

ccggagacgg tggcccggag gacagtggaa gctgtgcagc tcaaccaggc cctcctcctc
840

ctcccatgga caatgcatgc cctcgttatc ttgaaaagca tacttccaca ggctgcactc
900

gaggagatcc acaaattctc aggaacctac acctgcatga acactttcaa agggcggaca
960

tagagacagg atgaagacat gcttgaggag ccacggagtt tgggggccac agcacctggg
1020

cacacacccg agcacctgtc cattggcatg cttctgctgg gtgagcagga cagctcctgt
1080

ccccagcgaa gaatccggct gcccctgggc cagtcccagg acctttgcac aggactgatg
1140

ggtgtaactg acccccacag ggaggcagga aaacagccag aagccacctt gacacttttg
1200

aacatttcca gttctgtaga gtttattgtc aattgcttct caagtctaac cagcctcagc
1260

agtgtgcata gaccatttcc aggagggtct gtccccagat gctctgcctc ccgttccaaa
1320

acccactcat cctcagcttg cacaaactgg ttgaacggca ggaatgaaaa ataaagagag
1380

atggcttttg tgaaaaaaaa a
1401

<210> 47
<211> 1528
<212> DNA
<213> Homo sapiens

<400> 47
ccggctccca ttccggctcc agcctccaat ccgaccccca tttcggctgc agcctcggac

60

ctagctccgg ccctcggtct atccggttgc atcctccctc cctgttccgg atcttatctt
120

gcgccagcgc ctactccagg atcccgtagc cagacctcaa gccatggctg gtcccttctc
180

ccgtctgctg tccgcccgcc cgggactcag gctcctggct ttggccggag cggggtctct
240

agccgctggg tttctgctcc gaccggaacc tgtacgagct gccagtgaac gacggaggct
300

gtatcccccg agcgctgagt acccagacct ccgaaagcac aacaactgca tggccagtca
360

cctgacccca gcagtctatg cacggctctg cgacaagacc acacccactg gttggacgct
420

agatcagtgt atccagactg gcgtggacaa ccctggccac cccttcatca agactgtggg
480

catggtggct ggagatgagg agacctatga ggtatttgct gacctgtttg accctgtgat
540

ccaagagcga cacaatggat atgacccccg gacaatgaag cacaccacgg atctagatgc
600

cagtaaaatc cgttctggct actttgatga gaggtatgta ttgtcctcta gagtcagaac
660

tggccgaagc atccgaggac tcagtctgcc tccagcttgc actcgagcag agcgacgaga
720

ggtggaacgt gttgtggtgg atgcactgag tggcctgaag ggtgacctgg ctggacgtta
780

ctataggctc agtgagatga cagaggctga acagcagcag cttattgatg accactttct
840

gtttgataag cctgtgtccc cgttgctgac tgcagcagga atggctcgag actggccaga
900

tgctcgtgga atttggcaca acaatgagaa gagcttcctg atctgggtga atgaggagga
960

tcatacacgg gtgatctcca tggagaaggg tggtaacatg aagagagtgt ttgaaagatt
1020

ctgccgaggc ctcaaagagg tggagagact tatccaagaa cgtggctggg agttcatgtg
1080

gaatgagcgt ttgggataca tcttgacctg tccatctaac ctgggcactg gacttcgggc
1140

aggagtgcac atcaaactgc ccctgctaag caaagatagc cgcttcccaa agatcctgga
1200

gaacctaaga ctccaaaaac gtggtactgg aggagtggac actgctgcta caggcggtgt
1260

ctttgatatt tctaatttgg accgactagg caaatcagag gtggagctgg tgcaactggt
1320

catcgatgga gtaaactatt tgattgattg tgaacggcgt ctggagagag gccaggatat

1380

ccgcatcccc acacctgtca tccacaccaa gcattaactc cccatcgcca gctgatgact
1440

caagattccc aggagttttg ctcattctaa tgatggccca ttctacttgc tctggacctg
1500

cccccgcatc ccctgcctcc atcctagt
1528

<210> 48
<211> 765
<212> DNA
<213> Homo sapiens

<400> 48
taaccaagaa aactaaaagg gagttggtga aagacatctt tggactgctt ctctcagttc
60

ctgctgtcaa acaatctgaa gtacttttaa gtgtaacctg ctcactaacc ctcaccctga
120

cacccagcta tgtgtcgttg tcttgacact gtgtgggaag ctttggttgg gcaacacatt
180

gtcaaagtta aaatccaatg tatctccatc catgaggtca ttccgaatga tggattccat
240

gtcacagtct aagcgctcaa tgaacatgcc atccaagtca cttgggagct tctcctggtg
300

gagaaggccc attctgccat agccattgca actgctcacg gaggagtaac cccccacggc
360

actcatctgc atggggtggg gcaaaggcac ttgtacaggt gtcttcactt gggtcacgcg
420

gttcataccc gatgtgtggg gcatggtgct taccgagtgg ggcatgggac gcccgttaac
480

tgcacatgtc tgctgtacat gtccaacgtg ggtatgggag ctgggattca tcattatagt
540

atgacatgcc tcgctgccat atgttgacat gaccgaatta gggcccatca tgacgttatg
600

gcccaaatcc cagctgatgt gctcggctac gccacgaaca acatgtgtca tcatgtcatt
660

attggtgaga gagtcagatt gtatcacctt cttcaatact cccagcgcac aggcatactg
720

actcatacct acattactcg actcattgtg cttgagtgtc ttata
765

<210> 49
<211> 5723
<212> DNA
<213> Homo sapiens

<400> 49
   gcagccgcca cattcaacag gcagcagcgc agcgggcgcg ccgctgggga gagcaagcgg
   60
   cccgcggcgt ccgtccgtcc ttccgtccgc ggccctgtca gctggagcgc ggcgcaggct
   120
   ctgccccggc ccggcggctc tggccggccg tccagtccgt gcggcggacc ccgaggagcc
   180
   tcgatgtgga tggccccgcg aagttaagtt ctgggctcgc gcttccactc cgccgcgcct
   240
   tcctcccagt ttccgtccgc tcgccgcacc ggcttcgttc ccccaaatct cggaccgtcc
   300
   cttcgcgccc cctccccgtc cgcccccagt gctgcgttct ccccctcttg gctctcctgc
   360
   ggctggggga ggggcggggg tcaccatggc cgaggcgcct caggtggtgg agatcgaccc
   420
   ggacttcgag ccgctgcccc ggccgcgctc gtgcacctgg ccgctgccca ggccggagtt
   480
   tagccagtcc aactcggcca cctccagccc ggcgccgtcg ggcagcgcgg ctgccaaccc
   540
   cgacgccgcg gcgggcctgc cctcggcctc ggctgccgct gtcagcgccg acttcatgag
   600
   caacctgagc ttgctggagg agagcgagga cttcccgcag gcgcccggct ccgtggcggc
   660
   ggcggtggcg gcggcggccg ccgcggccgc caccgggggg ctgtgcgggg acttccaggg
   720
   cccggaggcg ggctgcctgc acccagcgcc accgcagccc ccgccgcccg ggccgctgtc
   780
   gcagcacccg ccggtgcccc ccgccgccgc tgggccgctc gcggggcagc cgcgcaagag
   840
   cagctcgtcc cgccgcaacg cgtggggcaa cctgtcctac gccgacctca tcaccaaggc
   900
   catcgagagc tcggcggaga agcggctcac gctgtcgcag atctacgagt ggatggtcaa
   960
   gagcgtgccc tacttcaagg ataaggtgga cagcaacagc tcggcgggct ggaagaattc
   1020
   aattcgtcat aatctgtccc tacacagcaa gttcattcgt gtgcagaatg aaggaactgg
   1080
   aaaaagttct tggtggatgc tcaatccaga gggtggcaag agcgggaaat ctcctaggag
   1140
   aagagctgca tccatggaca acaacagtaa atttgctaag agccgaagcc gagctgccaa
   1200
   gaagaaagca tctctccagt ctggccagga gggtgctggg gacagccctg gatcacagtt
   1260
   ttccaaatgg cctgcaagcc ctggctctca cagcaatgat gactttgata actggagtac
   1320
   atttcgccct cgaactagct caaatgctag tactattagt gggagactct cacccattat
   1380
   gaccgaacag gatgatcttg gagaagggga tgtgcattct atggtgtacc cgccatctgc
   1440
   cgcaaagatg gcctctactt tacccagtct gtctgagata agcaatcccg aaaacatgga
   1500
   aaatcttttg gataatctca accttctctc atcaccaaca tcattaactg tttcgaccca
   1560
   gtcctcacct ggcaccatga tgcagcagac gccgtgctac tcgtttgcgc caccaaacac
   1620
   cagtttgaat tcacccagcc caaactacca aaaatataca tatggccaat ccagcatgag
   1680
   ccctttgccc cagatgccta tacaaacact tcaggacaat aagtcgagtt atggaggtat
   1740
   gagtcagtat aactgtgcgc ctggactctt gaaggagttg ctgacttctg actctcctcc
   1800
   ccataatgac attatgacac cagttgatcc tggggtagcc cagcccaaca gccgggttct
   1860
   gggccagaac gtcatgatgg gccctaattc ggtcatgtca acctatggca gccaggcatc
   1920
   tcataacaaa atgatgaatc ccagctccca tacccaccct ggacatgctc agcagacatc
   1980
   tgcagttaac gggcgtcccc tgccccacac ggtaagcacc atgccccaca cctcgggtat
   2040
   gaaccgcctg acccaagtga agacacctgt acaagtgcct ctgccccacc ccatgcagat
   2100
   gagtgccctg gggggctact cctccgtgag cagctgcaat ggctatggca gaatgggcct
   2160
   tctccaccag gagaagctcc caagtgactt ggatggcatg ttcattgagc gcttagactg
   2220
   tgacatggaa tccatcattc ggaatgacct catggatgga gatacattgg attttaactt
   2280
   tgacaatgtg ttgcccaacc aaagcttccc acacagtgtc aagacaacga cacatagctg
   2340
   ggtgtcaggc tgagggttag tgagcaggtt acacttaaaa gtacttcaga ttgtctgaca
   2400
   gcaggaactg agagaagcag tccaaagatg tctttcacca actccctttt agttttcttg
   2460
   gttaaaaaaa aaaacaaaaa aaaaaaccct cctttttttc ctttcgtcag acttggcagc
   2520
   aaagacattt ttcctgtaca ggatgtttgc ccaatgtgtg caggttatgt gctgctgtag
   2580
   ataaggactg tgccattgga aatttcatta caatgaagtg ccaaactcac tacaccatat
   2640
   aattgcagaa aagattttca gatcctggtg tgctttcaag ttttgtatat aagcagtaga
   2700
   tacagattgt atttgtgtgt gtttttggtt tttctaaata tccaattggt ccaaggaaag
   2760
   tttatactct ttttgtaata ctgtgatggg cctcatgtct tgataagtta aacttttgtt
   2820
   tgtactacct gttttctgcg gaactgacgg atcacaaaga actgaatctc cattctgcat
   2880
   ctccattgaa cagccttgga cctgttcacg ttgccacaga attcacatga gaaccaagta
   2940
   gcctgttatc aatctgctaa attaatggac ttgttaaact tttggaaaaa aaaagattaa
   3000
   atgccagctt tgtacaggtc ttttctattt ttttttgttt attttgttat ttgcaaattt
   3060
   gtacaaacat ttaaatggtt ctaatttcca gataaatgat ttttgatgtt attgttggga
   3120
   cttaagaaca tttttggaat agatattgaa ctgtaataat gttttcttaa aactagagtc
   3180
   tactttgtta catagtcagc ttgtaaattt tgtggaacca caggtatttg gggcagcatt
   3240
   cataattttc attttgtatt ctaactggat tagtactaat tttatacatg cttaactggt
   3300
   ttgtacactt tgggatgcta cttagtgatg tttctgacta atcttaaatc attgtaatta
   3360
   gtacttgcat attcaacgtt tcaggccctg gttgggcagg aaagtgatgt atagttatgg
   3420
   acactttgcg tttcttattt aggataactt aatatgtttt tatgtatgta ttttaaagaa
   3480
   atttcatctg cttctactga actatgcgta ctgcatagca tcaagtcttc tctagagacc
   3540
   tctgtagtcc tgggaggcct cataatgttt gtagatcaga aaagggagat ctgcatctaa
   3600
   agcaatggtc ctttgtcaaa cgagggattt tgatccactt caccattttg agttgagctt
   3660
   tagcaaaagt ttcccctcat aattctttgc tcttgtttca gtccaggtgg aggttggttt
   3720
   tgtagttctg ccttgaggaa ttatgtcaac actcatactt catctcattc tcccttctgc
   3780
   cctgcagatt agattactta gcacactgtg gaagtttaag tggaaggagg gaatttaaaa
   3840
   atgggacttg agtggtttgt agaatttgtg ttcataagtt cagatgggta gcaaatggaa
   3900
   tagaacttac ttaaaaattg gggagattta tttgaaaacc agctgtaagt tgtgcattga
   3960
   gattatgtta aaagccttgg cttaagaatt tgaaaatttc tttagcctgt agcaacctaa
   4020
   actgtaattc ctatcattat gttttattac tttccaatta cctgtaactg acagaccaaa
   4080
   ttaattggct ttgtgtccta tttagtccat cagtattttc aagtcatgtg gaaagcccaa
   4140
   agtcatcaca atgaagagaa caggtgcaca gcactgttcc tcttgtgttc ttgagaagga
   4200
   tctaattttt ctgtatatag cccacatcac acttgctttg tcttgtatgt taattgcatc
   4260
   ttcattggct tggtatttcc taaatgttta acaagaacac aagtgttcct gataagattt
   4320
   cctacagtaa gccagctgta ttgtaagctt cccaccgtga tgatcatttt tttgaagatt
   4380
   cattgaacag ccaccactct atcatcctca ttttggggca gtccaagaca tagctggttt
   4440
   tagaaaccca agttcctcta agcacagcct cccgggtatg taactgaact tggtgccaaa
   4500
   gtacttgtgt actaatttct attactacgt actgtcactt tcctcccgtg ccattactgc
   4560
   atcataatac aaggaacctc agagccccca tttgttcatt aaagaggcaa ctacagccaa
   4620
   aatcactgtt aaaatcttac tacttcatgg agtagctctt aggaaaatat atcttcctcc
   4680
   tgagtctggg taattatacc tctcccaagc ccccattgtg tgttgaaatc ctgtcatgaa
   4740
   tccttggtag ctctctgaga acagtgaagt ccagggaaag gcatctggtc tgtctggaaa
   4800
   gcaaacatta tgtggcctct ggtagttttt ttcctgtaag aatactgact ttctggagta
   4860
   atgagtatat atcagttatt gtacatgatt gctttgtgaa atgtgcaaat gatatcacct
   4920
   atgcagcctt gtttgattta ttttctctgg tttgtactgt tattaaaagc atattgtatt
   4980
   atagagctat tcagatattt taaatataaa gatgtattgt ttccgtaata tagacgtatg
   5040
   gaatatattt aggtaataga tgtattactt ggaaagttct gctttgacaa actgacaaag
   5100
   tctaaatgag cacatgtatc ccagtgagca gtaaatcaat ggaacatccc aagaagagga
   5160
   taaggatgct taaaatggaa atcattctcc aacgatatac aaattggact tgttcaactg
   5220
   ctggatatat gctaccaata accccagccc caacttaaaa ttcttacatt caagctccta
   5280
   agagttctta atttataact aattttaaaa gagaagtttc ttttctggtt ttagtttggg
   5340
   aataatcatt cattaaaaaa aatgtattgt ggtttatgcg aacagaccaa cctggcatta
   5400
   cagttggcct ctccttgagg tgggcacagc ctggcagtgt ggccaggggt ggccatgtaa
   5460
   gtcccatcag gacgtagtca tgcctcctgc atttcgctac ccgagtttag taacagtgca
   5520
   gattccacgt tcttgttccg atactctgag aagtgcctga tgttgatgta cttacagaca
   5580
   caagaacaat ctttgctata attgtataaa gccataaatg tacataaatt atgtttaaat
   5640
   ggcttggtgt ctttcttttc taattatgca gaataagctc tttattagga attttttgtg
   5700
   aagctattaa atacttgagt taa
   5723

<210>  50
<211>  842
<212>  DNA
<213>  Homo sapiens

<400>  50
aacctgggcc accggcttgt acatagcacc gtgctaggct ctgggatccc aaatggaccc
60

ttttaacttt ctgaagatgg gaccgtcccc tggaggaaag tcattcctgc ctaatccatc
120

gagagaaaga ggcttacgaa aaactttgcc tctgatgctc agccccaccc ccaaatagca
180

cacaagcttg ttaaccccac ctcttacaaa atgtttagat tctgtaggtg ttaaaagcct
240

ttctggaagt attgcattct gccgtgttta taggtgttca ctttcctcca gagctgatta
300

actactgaca tgacttggct ttctcatcca gaaattatgg aaacagggtc tgtcagtggc
360

aggaggccgt gctgtgtttt acttggatga cacaatgcag tttacttgcc tcttcatacc
420

catgcatgct gctcacccta gacaatgaca tataagccgt atatagatca atgtccacat
480

atatatacac acacacatat atatatataa agtgtaacaa ggaacactaa aacagtgttg
540

attcttgtct ctgaagacaa ataattaaac ttttttttcc caataaagaa tggatttaat
600

taaactatgt attgcaaaaa aagtagctaa gtgttagaga tggtgaatct attccatttt
660

agtaagacaa attctgaatt taagcatcag tagtgcattg atttgggtgt cttaccaata
720

ttttcttgtt tctttggggg ggggaaagcg catctggttg caattccggc tgttcggttg

780

tacctaagtt tcgctggtaa gaggaggcag agtacgcttc ctaacagggg ttttaaaaaa
840

gg
842

<210> 51
<211> 5210
<212> DNA
<213> Homo sapiens

<400> 51
ccagaggctg agcccgggcg agcccgccgt gcgcacagct ctgcccgccg cccctggagc
60

ggatccccag ccgtccctgg gcgttctccc gggttctagc aaagcccccc ggggtgccca
120

tcagtttcct tgggtgacta cagcgtgtgt tttttctttc ctctttcccc tgcctgtgtg
180

cccttctcca ggatggcaga ggcggaattg cacaaggaaa ggctgcaagc catagcagaa
240

aaaagaaaga ggcagactga aatagaaggc aagcgacaac agcttgacga gcagatactt
300

ctgctgcagc attccaagtc caaagtgctt cgggagaaat ggctgctgca gggcataccc
360

gctggaactg ccgaagagga ggaagccagg aggcggcagt ctgaagagga tgagttcaga
420

gtcaagcaac ttgaagataa cattcagagg ctggagcaag aaatacaaac gctagaaagt
480

gaagagtccc agatatctgc caaagagcaa atcatcctag agaaactgaa ggaaacagaa
540

aaatccttca aggactttca gaagggtttc tccagtacgg atggagatgc agtaaattac
600

atttcctccc agcttcccga cctgccaatc ctctgttcac gaacagcaga accatcacct
660

gggcaggacg ggaccagcag agcggctgga gtcgggtggg agaatgtgct gctaaaggaa
720

ggtgagtcag cctcgaacgc cacagaaaca tccggcccag acatgactat caagaagcct
780

ccccagcttt ctgaggatga tatctggcta aaaagcgagg gagacaacta tagtgccacc
840

ctcctggagc ctgctgccag ctctctttcc ccagatcaca aaaacatgga aattgaggtg
900

tctgttgcag aatgtaaaag tgttcctgga atcacctcta ccccacatcc catggaccat
960

ccctccgctt tctattcacc cccgcataat ggcctcctta ctgatcacca cgaatccctg
1020

gataatgatg ttgccagaga gatccgctat ctagatgagg tgctagaggc caactgctgt
1080

gattctgctg tggatggaac gtacaatgga acatcctccc cagagcctgg tgcagtggtt
1140

ctggtgggcg gcctaagccc ccctgtccac gaggcgaccc agccagaacc cactgaaaga
1200

acagctagcc ggcaggcacc tcctcacatc gagctcagta atagcagccc tgaccccatg
1260

gcagaggcag aaagaacaaa tggccattcc cccagccagc ctagagatgc gctgggggac
1320

agcctgcagg tgcctgtcag ccccagctcc acaaccagct cacggtgttc ttcccgagat
1380

ggagagttca ctctcaccac actgaaaaag gaggccaagt ttgagctgcg tgccttccat
1440

gaggacaaga agccctccaa gctctttgag gatgacgagc atgagaaaga acaatactgc
1500

attagaaaag tgaggccttc agaggagatg ctggagctgg aaaaggagag gagagagctc
1560

atccgcagcc aggccgtcaa gaagaatcct ggcattgcag caaaatggtg gaatcccccg
1620

caggaaaaaa ccatcgagga gcagctggac gaggaacatc tggagtcgca caaaaagtac
1680

aaggagcgca aagagagaag ggcacagcag gaacagttgc tgctgcagaa gcagttacag
1740

cagcagcagc agcagccccc atcgcagctc tgcacagccc ctgcctcttc tcatgaacgc
1800

gcaagcatga ttgacaaagc aaaggaggac attgtcacag agcagataga tttctctgct
1860

gctcgcaaac aatttcagct gatggagaat tccaggcaag cggtggccaa gggccagagt
1920

acacccaggc tgttctccat caagcctttc tacaggcctc tggggtcagt caactcagac
1980

aagccactga ctaatccgag accaccttct gtcgggggac ctccagaaga cagtggtgcc
2040

tcagccgcca aggacagaaa atcccccggt gccctggaga ccccatcggc agcaggaagc
2100

cagggcaaca cagcctctca ggggaaggaa gggccctaca gcgagccttc taaacgtggg
2160

cccttatcta aactgtgggc tgaggatgga gaatttacga gcgcccgggc tgtcctcact
2220

gtggtcaagg atgatgacca tgggattttg gatcagttct caagatctgt caatgtctcc
2280

ttgacccaag aggagcttga ctctggtctg gacgaattgt cggtgaggtc tcaggatacc
2340

acagtcctgg agaccctatc caatgatttc agcatggaca acatcagtga cagcggggca
2400

tccaatgaga caaccaatgc cctccaggaa aattcactgg ctgatttttc tctgccccag
2460

acaccacaaa ctgacaaccc ctcagagggc cgaggagaag gcgtctccaa gtcatttagt
2520

gatcatggtt tctattcccc ttcctccacg ctgggggact ctccgttggt tgatgacccc
2580

ttggagtatc aggctggcct cctggtgcag aatgccattc aacaagccat agccgagcag
2640

gtggataaag ctgtgtccaa aaccagcagg gatggagcag agcaacaggg acctgaagcg
2700

actgtagagg aagctgaagc tgcggctttc ggctcagaaa agcctcagag catgtttgag
2760

ccacctcagg tgtcttctcc tgttcaagag aaaagggatg tattaccaaa gattctgcct
2820

gctgaagaca gggcgctcag ggaaaggggg cccccccagc cactgccagc tgtgcagccc
2880

agtggcccga ttaacatgga ggagaccagg cccgaaggaa gctatttcag caagtactcg
2940

gaggcagctg agctgagaag cacagcctcc ctcctggcca ctcaagaatc tgacgtgatg
3000

gttgggcctt tcaagctgag gtccaggaaa cagcggactt tgtccatgat tgaggaagag
3060

atccgagcag ctcaggaaag ggaagaggag ctgaagaggc agagacaagt cttgcagagt
3120

acgcagagcc ccaggacaaa gaatgcccca tcactgccct ccagaacatg ctacaaaact
3180

gctccaggga aaatagagaa agtcaaacct cctccatccc ccaccactga aggccccagc
3240

ctgcagcctg acttagcccc tgaagaggct gccggaaccc agcggcccaa gaatctgatg
3300

cagaccctca tggaagacta tgagacacac aaatctaaaa ggcgcgagag aatggatgat
3360

agtagttaca cttctaagtt actgtcttgc aaggtgactt ccgaggtcct cgaggccaca
3420

cgggttaatc gaagaaagag cgcactggct ttgcgctggg aagcagggat ctatgccaac
3480

caggaggaag aagacaacga ataaacttcc ttcaacccag gaagcgtctt tggtgcttgg
3540

gagaccaaga aaccaagaaa ttaacaactg aaagcatttt aatggactat ttattaaagt
3600

gcaaacaaac tcagcaatcc ttatgtagac cagaagctgc aatatacaat gatgaaaatg
3660

aaacgaaaag gaagtccccc catcagactc tggacaccca ggaatcaaaa gagaaaggac
3720

ttttttggtg actcaatccc agtgtctggt ttggggccaa tctacaatgg atccaaaagg
3780

acaaaacatg ttacaagcaa atcaacatag ttgctgcagg cggaactgag accagcctct
3840

ctgggcgatg aaggacaatg gtggtagggc cacttagaga gcagtagggc ccaggctggt
3900

gcagcctaca tggcacagga tatttccctg caccagtttc ccacctgtca cttgcagcat
3960

cagagggtgt tgcattaact ccattttctc cagcagccat aaaatgaatg gcgtttctct
4020

ccatatttgc ccagaagagg cagttatgat tccacttctc ctacatttaa ttatttttaa
4080

ttgagaacaa ctcatgatca caattcataa aatccatatt ttaagacaat agttcttcat
4140

gtcacaaaca caatctattc tacatgagga ggctgagaaa ggggaagaga aaaacaaacc
4200

aaatggatga tgttagcttt aggatcgtgt ctgcttataa tattcactga tctgcagttt
4260

atgagaacac attttcacag ttatgtttct tcataggaaa actatattta gtgggcaaca
4320

actattttta tgatgggatg ggggagtata tacacgtata gaatctgtac gcgttgaaca
4380

acttggttca agatggtggg ggcattttta gagcggcaat aattgaaaaa aaaggcgaac
4440

tctgccttgg agaggtagat gataagaaat aaaaaggtgt ttataactat tttgtattat
4500

aaagtgggcc ttagagatag gaagaagaat gatggattcc ttttggatca atcagaaagg
4560

aaacacgaaa gaaaagtcag gaaggtagag agagaaaaag ggagggaagg agaaagaatg
4620

ggaataaaat aaggaggtaa gagatactat ttttgctgag caaccagtgt gtttcaggat
4680

gatacaaaga aaaatataga atagaaataa gtgcaggctt ggaatcagct acaaatccta
4740

aagatggggt gtgtgtggat gtgtgtgtgt gtgtgtgtac accattgtgt gtttgtaaaa
4800

tgtgtatgtt catgagtaag ggtgtgtgtg tgtgtgtatt aaaattccag agtgaccgtg
4860

gcacttgggt gtacaggtaa ttcctccaga gctgtttgct ggcttcagga gtggagtgag
4920

aatttctttt ttatgaaaag ggatataaag gcaccgagct gatgcagtat ttgtaatatt
4980

aagttgacct aacaaggtat ttgcatgagt cacaattaca aagttttgag cggttttgta
5040

atttgacatt taggaaagtc tcctatttat tctcatactt tacattcatg cttagtatac
5100

tatagaggat gccagcttta atctttctgt catttaaagc aatatgataa gggtattcaa
5160

taattgggtg ccctaaattt ctggatgaga aaattttcaa ttctggccag
5210


<210> 52
<211> 146547
<212> DNA
<213> Homo sapiens

<400> 52
gatcttcaaa ggttggggtt tgatagtgcc ttgaataatt tttaacttta tattgccagc
60

ggaagaagca ttctcttttt agatttaaaa aatgtagata caaatattag ggttttattt
120

tttagtgaaa catttcaaac atacaggaat agataattat gtaatgaaca ctcgtatgtc
180

caccatctgg ctttgtaaaa tcttaaaatt atgtcttatg tgctcaattg ttttatttca
240

taaaagatac tgataaacat agctgaagtc acttgtatac cattcacctt cttccctgta
300

gattactatg aactcggtct ttttattctc atacatattt tttgtatttt tgcagtatat
360

ttatgtgttc ataaacaata tgtaatttta caatatgtaa cacactagta acatactaat
420

ttaaaacttg tttttagttt acaatatgtt gtaaactatt gtaagctaaa gacatattgt
480

acaacctatt gtaaaataaa aacaggtttt agttttaaat taggtatgtt actgggatca
540

ttctgcaact tgtttattcc tctccagctt tgattttgtg gttttattat cttaacctac
600

acttttaatt aatccatttt atttgttaca tggtattcta ttatatcata aaaacttatc
660

tattctgttg gtttttgttg ttggtcattt gagaccatgt cttcgctctg tcacccaggc
720

tggagtacag tggcgtgatc ttggctcact gtgacctctg cctcccggat tcaagtggtt
780

ttggtgcctc agcctcctga gtagctggga ttataggcgt gtgccaccat gcccagctaa
840

tttttgtatt tttaatagag acgggatttc accatgttgg ccaggctggt cttgaactga
900

cctcaagtga tctgctcacc tcagctgcac aaagtgctgg gattacaggt gttagccacc
960

ataccctgcc tctattctct tgttaagagg catttagcat ggttatacag tctcttgccc
1020

tcataaacag tgctggaaga aacacatgtt tcttgtgtat atgaatgaaa atttgtttta
1080

tacattagat atttccaaat tgttctctta agtacttcag tttacatcat tactctcctc
1140

ctccctcccc tcccaccccc acccacaaca gtattcctct ttttccatat ccttgctaat
1200

gtttctaaag ttttgctttt tacatttggg tcttagatcc actagaatgt atttttgcat
1260

tgggatgaag ttgaaaccta atatattttc caaatgagta aactgttgtc acagaactat
1320

ttagttgtat tacctcctct cttgtatatc agatatatct acatatatgt cagactgttt
1380

ctgggctgtc tgtcctcttt aattagttcg tgtatctgtt tctgcatcag tagcatactg
1440

tcttaactac tgtagcttta taaagtctat tgagtaggac aagtttgttt cattcttcaa
1500

aattgctttg gctattcttg gccctctgct gtttcatatt aactttcaga taaacttgtc
1560

aaattctaat gaaaactgtt gataaacttg ttgattaaca aattctaata aaaactgttg
1620

agatttttat tggaattgca atacatttat agattaacgg agaaagatat tgacaataca
1680

attgagtttc caattcacga acatgttata cctctccatt aattcatgtc ttttgaatgt
1740

atccaccaat atggttttgt aattttcttc ataaaggttt tacatttaaa aaattcttat
1800

ttttaagtga tcttatagtt tttattgcta atgtgaatga gatttttttc cattatgttt
1860

ctgttggtta ttcctgaagt ggtaatgctt ataattttgg ggtgttggtc ttgtatctgg
1920

cagcagatac aagaggtgct cagagttgtt cagggttgct gaactcttag ttctaaaagt
1980

gtctgtcatt tggggtttct atgtagataa tttaattatc tataaaaaca gttcttcatt
2040

ttcagttcat atatttcata tttctttaag ttttaatttt tatttttaaa cacaattatc
2100

cataaaaccc taaccctttc cctagtcaac agcagtcaca gccaaatgtt ttattaattg
2160

ctatactcag tgtttcttgt atctcatacc ttctggggtt tcttgtcttg ttgaaataca
2220

ccctttaatg tttctttagt gaagacccaa cagtggcact cactcacctt tgtttacctg
2280

aaaatttctt tattttcatc ttaattcata gtctgtcttt tctccagtca aggaagtgtc
2340

ttatagggaa gattctggtt tcactatgct gtatccaggg atatatatgt atttatagat
2400

agacttttaa tctgaggact aatgtatttt atcctacagt attaccaatc attatttctt
2460

ccataacttc tagaccattc cttttgtact tcttttttag agtcctatta gatgagtgtt
2520

gacacttttc aatctagaca tcttttttaa actatatttt catactcttt gtctctttag
2580

gtctgatttt ttaagttcag ggaatatttt cattttgggt gagttgtagc actcacttcc
2640

aattcactaa ttctaattat atttaatcta caagttattc catctataat ttatttcaat
2700

taccactttt tgttttcaaa atttctaatt ttatatctga ttttgtttca tttttgtttt
2760

ataatttcat gttctttcta gattttacat ctttttatgc atactaaaca tactcacttg
2820

aaagtctttg taagattgtt ctataaaatg ttacctgaag tgaattcatg tgctaattac
2880

tgttggcagt ttttctgagc cattttcctt gtgtcttttg aaatttctgt ttgtaagccc
2940

ttttaactgg gaggtgtttt ttgcttatgt actgttctct tttctttccc ttcttctcct
3000

tctctgttca ttactttttg tcttggaggg agtctcttag cccagctcag cccaagctaa
3060

taaggcccct gacttcaagt ccccccttct tgcatggagc aagaagtgat cctcctgtct
3120

cagcctccca aagtgctggg taggcgtgag ccaccacatc cagcctagag cacttacatt
3180

gctacatatt tggagcaaac ataattaaag tatgacttac tgtgacccag cagtcaataa
3240

cagatatgtt tttgtaatag aatgttgaag ggtggtaact ggtggtttta cttcatggag
3300

ggctttaggt cggctatatg ggaagctgcc atcatgatgc ttttgttcta cacatttctg
3360

ctcagcctcc agagatgtct cctattctaa gagtcgtctt tgtttactct tgtgtctata
3420

ttttacttac ttaattccct cacataatag tgtatcattg tatcccatct gtgctctatc
3480

aagaaatgta gcacaagcat gatgtgaact atgaattttc gacaatttaa aaaattaaag
3540

gaatcattca ggggtaaaaa gcacaaagtt gtagaacagc tgaagtgtgt tctttttatg
3600

tatcctgtct ttgccccctt tctggaaact tggattggcc tacttgcagt cactgtcaca
3660

gttacactac ttctagcctc aaaggtcatt gaaaggattg ggatttttga gaaccacaat
3720

aacacttgct ctaggtactg aaaaggttgt cctgaactca ttcatatctt tttatgttca
3780

tcccagtttg tttttgcttt tccccccagg attttgatta cacagacatc ttgcaagcat
3840

atttccattc tttctctccc actctgtgcc tggtgcccgt aggatctgaa atagcattgg
3900

aatgacagct agccttctgg aacctgcagt ttcaaccaaa gaaactctga ttccagataa
3960

cccaggagtt tcttatcctg cataatctag gagtttacaa aaaggctgct tatgggtact
4020

cactgctaag tacaatccac tgccaagttt ggaagcatta cctgctctca caacccaggg
4080

aaagtagccc ttagtaaaat tcaggttttt tcaaaccagc cttaacctta caaacagaat
4140

ttatcatcat gatttttctt tcaaatgcag tacagcaaac tttaggatca atgttgacat
4200

cgtattgcgt ttagtcctgt taaaatactc tcagtgccaa atacacactt ctgccttttt
4260

tctctttaga gtctagaatt ctgtggggtt ccaaaaatgt atgtgcttac aacctttat
4320

atgtaaccac ctagggatta aagggctaaa tgtgaaggaa agtatcttta caatattcat
4380

cttgggaagc tctcattttg gtgagttcta ttagagaaaa taagacacaa gctcattaaa
4440

aatatatata tattctgagc ctggcatctt ggtataaaat tcaaaacgtt ttaaatctat
4500

ttattagtaa gactaacata gacctaagtg tgaagactaa aatattaaag cttgtaggtg
4560

atatcatggt tgaatatgac cttagagtag attccttagg acagaaaaac accaacaaga
4620

gaaacaattg actaaatgga cttcattaaa attaaaaatt tatgttcaac aaaagacact
4680

gctaaaataa atacagaatt tacagactaa aagaaaatat ttgcaacatg tttatctgaa
4740

agggcttata tccagactat acaaagaatt cctgtagctc cataataaaa aaacagacat
4800

cccatccgca taataaaaag agcaaaagac ttgaacatat actatacaaa agaagataca
4860

ggaatggtca ataaataagc aaatgaaaga gtgctcgaca tcactaatta tcagtggagt
4920

gaagatttaa ccacagggag atacgaattc cagttctagg tatttttacc tcaagtgaaa
4980

taaaaacatt tatccatgaa aaaacttgta caagaatgtt catagcagtg ctctattcat
5040

aagagtgcaa aactggaaac atcccagaat agataacaca ttatggtata gtcatagaat
5100

atactatatt tatcagcagt taagaaggga actacttatg tattcaacaa catggatgaa
5160

tcttaaaaac atggttaagg gccaggtgcg gttggtgcat gcctgtaatc caagcactct
5220

gggaggccaa ggcgggtgga tcacttcagg tcaggagttc gagaccagcc tggccaacat
5280

ggcgaaacac cgtctctaca aaaaaataca aaaattagcc gagggtggcg cacacctgta
5340

atctcagcta ttcaggaggc tgagacatgg gaatcgcttg aacctgaggg gtagaggttg
5400

cactgagccg agattgcatc actgtactcc agcctggtga cagagcgaga cgctgtctca
5460

gagggaaaaa aaaaagttaa aaaggcagac agaggagtct atattgtatg attctattta
5520

tataatctag actaggcaaa atttatccat gatgatagta atcagattga aggatcagtg
5580

gttgcttggt ggaaattgac tgagaaggga aatagtcatg taagaatact ggcaaatgtc
5640

ctcttcccca ttacaaacgt ctgagaaagt tactagaaaa cagaatccaa tttgccttct
5700

cacccccaac tttgtaatct tgaatccaga tcctgattcc acccaaccac tctgctgaat
5760

ggatagcagc tatattagag agacaatata gtgggcctac tagttttaaa aagtcatcca
5820

aagaatgtca tctacaatca gttaaattag atttttaccc attactgaag tgtgattgaa
5880

ttagggaaag aggagaggtg gtcaaaggca gccttctcca gtggattcct tgtcttcttg
5940

agatggtttt gtgagacctt gcctggaaga cctaggtgga tagtttattt tctaaaagta
6000

gtcaagaaag cttgttcagg gcattgaaca ctgcagggac aagcatactt cttggaggcc
6060

agccatctcc tgctggatct aagcggggtg gcttctgcct ccacctccat ctcagcttat
6120

ttgagagaat cctagatcct tggtgcagaa gcccagccag cctgtgaaga aaagttggtc
6180

tggtagtatc ctgtgaatga cgtgcgggtt gggtttttct aaatctcgtt tacctgactc
6240

ctaccttaca ttccagtggt gtgactaagg aactgagcaa acgtaaccct aggggacttt
6300

gagacaggct ttggtagtcc atgtgcacta gagtgagggg aaagaggcca ctccagagtt
6360

gggtactcta ctgttgaagg tgagcctttt gtgtccaatt tttccgtgca tgtgtaagaa
6420

cagttgtcat ggagatttac tactgacttg tgcatgagga cagcttatat ctcacctttt
6480

cttattcacc ctgttaaatg tcaaaatgta ctaagactta ggtatttttt ctgtgtcacc
6540

aactactaaa tcacccaccc atgatagcag gtagttactg aaactcttgt agccaaacaa
6600

gtgcataaca ctggtacaca tgatgtgaga gtgcagatga ctccttagct taactgtgta
6660

agcttttaat ttctgagtac ttttaggatt gagaagtgga agacattctt gctctaggta
6720

tctcatgctt ctgcacaccc agaaggctgg ttgcgccccc accccacccc ccgccccctt
6780

tttttttttt ttgatagggt catgctctgt tgcccaggct gaaaggctgg ttatcttcac
6840

cacaggtctt atgtctcaag gaccacgggt ccacgtcata gtcaggaacc aggcatgtgt
6900

gggcagaaat cactaccact ttcctttgtt gcgggtgggg aaattaagag cagagattct
6960

tggattctta aattccttct ctatgtttat gtcatctagt ctgttttatg ctgttttatg
7020

taattccctg cttctggatt tcttgaattt ttttcccctt ctcacttgtc ataattggct
7080

cttgcttgtg agtctatttt cattcagctt ggtagcagaa gtcttcttca ctattgttta
7140

gattcagttt tcacatggtt ttagtaaccc ttcattgggt ttagaatcaa cttttaaccc
7200

ttccaccaaa agctcttatt tttttaaagg cttcccagaa acatagtttc tctcaatctc
7260

atcaagaaat gcttttaaat gcagaagtta gaaaatggtg aataattatg gctgttcctg
7320

gaagtcattg tgtccttagt aattacatgt tgtagtcttt ggttctgctt ctttgacagc
7380

tccaccacac tgtaatttaa tggaattcac ttagatttaa tggaattcag tcctggtaga
7440

aaactgctat tgggtcacag agttggcagc aaggctgtga aattgatgtg ggaatggggg
7500

tgattatgtg ctttgtgttg acagaagtta gtttttggga catcccatgt gggtttttcc
7560

caggaacata aacattttag agaattagag ctagaaactt tcttctggga aagccttaga
7620

agtcagttgt gcttggcagt gagatgggac aatgtgcatt cttaacaggt taaatgtgcc
7680

aaactctctc ttcagtacta ttctttttta cgtttgctta aggtcaagtt cctgctcttt
7740

tgttcaaagt cttgctttgt cacccatggt ggagtgcagt ggcgtgatct tggctcactg
7800

caacctctgc ctcctgcgtt caagcaattc ttgtgtctta gcctccctat tagctagctg
7860

ggattatagg cttgcgccac cttgcctggc tatattcaaa gtctgtcttt actacactgt
7920

cccaaagtaa tctacatttg ggtttgggtc tttacagggt gagaaggact ggcagaaata
7980

cgagactgct cggcggctga aaaaatgtgt ggacaagatc cggaaccagt atcgagaaga
8040

ctggaagtcc aaagagatga aagtccggca gagagctgta gccctgtact tcatcgacaa
8100

ggtgagagca tcttcccatc ggcattgtct agtgttgagc ttaacaaagg gagtttctgc
8160

tctgccccag gccctgtgcc acatactgta tatcacaact caacatacat atatttgtat
8220

gtaaaactga atcaaaagtt gcacaaaaca atgcttagcc ttactgtgag cagtagattc
8280

tgatcttttt ttaactctat ttcattcctt tagaaagttg gttataactt ataaaattgt
8340

ttaaataaac catgcaggta tcaccattat atagttttaa agaccacgct ctagaggaga
8400

tcaatttctt ggtactcacg agaagaaaaa aaagttcatg tttttccccc agtcttgtat
8460

tttaacataa gttctgaatt gtctcaaaat tcacactagc tttttgagtt tgattgccca
8520

gagtagcagc actccctcat ttgctgcctt atttaatttt ttttttcctt ttctccatcc
8580

ctatttcaga ggacagaata gaaagaaaag gagcatgaat atttaacatc cctatagatt
8640

tcatagtgag tgggggagga agtaacactg cagaatccag agttcccagg ccatgctgac
8700

tgatgctgtg gcttcaaaca gaattgggag gcttctgttt tggatgccta gaaattgcat
8760

aagggtcaaa aggctgggcg cagtggctca cgcctgtaat cccagcactt tgggaggcca
8820

aggcaggtgg aacacgaggt caagagacca tcctggccaa tgtggtgaaa ccccatctct
8880

actaaaaata caaaaaaaaa aaaaaaaata aaaattggcg tggtggcggg cgcctgtagt
8940

cccagctact tcggaggctg aggcaggaga atggcatgaa cccgggagac ggagcttgca
9000

gtgagccaaa attgcgccat tgcactctag cctggcgaca gagcgagact gtctcaaaaa
9060

aaaaaaaaaa aaaaacacag aacgaaattg tgtaaggatc atgtctcttc cattcatgct
9120

catcttttct ttctttcctg ggcagcttgc tctgagagca ggcaatgaaa aggaggaagg
9180

agaaacagcg gacactgtgg gctgctgctc acttcgtgtg gagcacatca atctacaccc
9240

agagttggat ggtcaggaat atgtggtaga gtttgacttc ctcgggaagg actccatcag
9300

atactataac aaggtccctg ttgagaaacg agtaagttaa tgtacctgta ctgtctgact
9360

tgttttccat tattcaacaa gcatgggttg actgcttttt tgtgtgcttt gcactttgct
9420

gggcaccagc aaaagtgact tgagacaggc aacatggcac atgcctgtag acccagctat
9480

tcagaaggct gagacaggag gatcactgga gaccaaaagt ttgaggctgt agtgtgctgt
9540

gatcacacct gtgaatagcc actgtactcc agcctgggca aaatagtgag accttgcctc
9600

tcatattaaa ataaataaaa ataaatgact tgaaagaggg gagaggtatt atttgatatc
9660

ttgtgtacct ccttttagtc actgttccgc cctgtgctac cttcctggta aggagtggcc
9720

ttctcctata gcagtattca atggagcatg ctgtagaata ggcccttaga gcaaactcag
9780

catatgattt aactatatct tagtatcccc tggtcatgtg gtacatttgt cacttttcat
9840

ctttgctcag cctttctcca actcctgact gagtgaaagg ttaatgttag tccccagatc
9900

tctgagccca tcactgaaga gggtactata gctctgtagt cccaacctaa tcttttccat
9960

agtattgggc ttgaaccaga gacttacagt gtgcaggatc ctaataatcc agcccaccag
10020

cagtcctctt cttataaaga cacctcttct cataaggaca ccataatcag taatcaggtt
10080

ttgctggctg tggaagtact ctggacagat tagataacag tatgtatcaa tgtaaatttt
10140

tgaagctgat actgtgtaaa tgtaaggaaa tatctatttt tagaacatac acactgaaat
10200

gtttaggacc atattataag taagtactta taaatgtact ttaggaccat attataagta
10260

agtacttata aatgtacttt aggaccatat tataagtaag tgcttataaa tggctcagaa
10320

caaacaatta tgtatatata tctgtaaata tacacatgga gagagtgagc atacatgcac
10380

actgtgcaaa tgatgaatgg ggtaaaatgt taacagtgaa tctcggtgac ggatgtatgc
10440

gtgttccttg tgcctttttc tttgcaagtt tctttaagtt tggggttatt tctaaagtta
10500

ggattttttt caagagtaat aattaggatt cacttatatc ttttaggttt ttaagaacct
10560

acaactattt atggagaaca agcagcccga ggatgatctt tttgatagac tcaatgtgag
10620

tagatgaagc acacaatgtt gaagggagtc ccagccagag cctcacagta cctaaagggg
10680

agggttgctg gcagatgact tgggctctcc ctttagcctg gcctgctctg tggcatccca
10740

tacactctct cttcctccct ctgagtccac ggtgaatctg tagtcaagag aagacacatc
10800

tgctgcagaa caactgctaa agcactcagg gtggggggta gatgagccct accttttatc
10860

cttccctgct cctgcagagc tttcccttca ctgaatgccc agccacccct gctggagacc
10920

caaaacctgt ccctgatatc ttaataggga aggaaaaatt tgctttattg gtttgttaag
10980

cccaccaacc tgggccaggc ttggtggctt acgcctgtta tcccagcact ttgagaggtc
11040

aagtagggca gatcccttga ggtcaggagt ttgagaccag cctgggcaac atggcaaaac
11100

cattatccag gtgtggtagt ccacacctgt agtcccagct gcttaggagc ctcagaagga
11160

ttgcatgagc ccgggaggtg gaggcagcag tgaaccgaga ttgtaccact gcactccagg
11220

ctgggtgtca gagtgagacc ctgtcataca cacacacaaa gattgaaata cttcatagag
11280

aggcatcatg taaccctgta ttctggaatt cttttcctct ttccctaact tcccacttgt
11340

agggccataa acttgacaag attgtgactg cactggcata aattactcct agggctgcca
11400

gaaggagcag gtagtatagc tttgacctaa atctgttgct ttgtctcctc cagactggta
11460

ttctgaataa gcatcttcag gatctcatgg agggcttgac agccaaggta ttccgtacat
11520

acaatgcctc catcacgcta cagcagcagc taaaagaact gacagcccgt aagtattgct
11580

tggccagata gggcccacac ccctactaat ggtatccggt gaccttgctt atctaaggcc
11640

tagagtcagt tctacttttt ttccctacca ttgtggtcag acactttttc cctttagacc
11700

tctagtagcg agataatgct ttgttgtata aacataggat caatgctgtt tccccttcc
11760

caccccaact tcaagattaa tttcatggaa tgctgacagc ttttcacctg ctacaaaaat
11820

gcctgcattt gtgttttata catatattac ttaaaagccc acagagtgaa gacagtgctg
11880

tgatgttctg ttaaattgga gagagtaaag ttgtcatggt taccagaaaa atacaggaac
11940

aagtcctttc cttgaggttt ctgtcatttt tttcccataa gagagtagat actttctata
12000

gctgattttt agaatattat caccaagatc cacgggcttt ttttttcctg gagcccagtc
12060

actattgaat ttccactgcc ctctgtaaat acatcagatg gccttagaat atgaaccaga
12120

aaagccagag gtaggtatgt tgagggtcag tattttaagg tggtataaag aatgccacac
12180

tatacaaata aaaacaattt ttttggatgg catttttaat ttttcttttt aaataagata
12240

ctaccaggcc atgtgcagtg gctcacacct gtaatcccaa cacttgggta gccgaggcag
12300

gagaatcact tgaacccagg agttccagac cagcctgggc aatgtagtga gaccttgtct
12360

ctactaaaat ttctaaaaat taggcaggtg tggcggtgtg cacctgtcat cccagctacc
12420

tgggaggctg agcccaggag tttagggctg taatgagcta cgaccatacc accgcactcc
12480

agcttgggtg acagggtgag acgctatctc taaaataata ataataacca taataatact
12540

accaactgtg gcttaatttt tttttttttt tttttttttt tgagacggag tcttgctgtc
12600

acccaggttg gagtgcagtg gcgcgatctc ggctcactgc aagctccgcc tcctggggtt
12660

cacgccattc tcctgcctca gcctcccgag tagctgggac tacaggtgcc cgccatgtcg
12720

cccggctaat tttttgtatt tttagtagag acagggttca ccgtgttagc caggatggtc
12780

tcgatctcct gaccttgtga tccgcccgcc tcggcctccc aaagtgctgg gattacaggt
12840

gtgagccacc gcgcccggcc aaaattttta aacttttaac gtcccttcaa ttcctcgttc
12900

tgttctggct acattgggtg gagaggtggg gaggtaggaa gtggccagta tagaacttat
12960

gaatatgggc aacaaaccat agtaatatca gcagcaccta tgatgagcat atgggtgggg
13020

aggaagcaag gagatgaagg gtccctagcc aagttccctg agtaaaaaga aaggtgtaga
13080

ttttattata ttacctagac tcagagttcc tcttgtgata ggaatttacc caggccaagc
13140

ttacatataa acttcctact atcattttct taagactgtt gactcaaatt ttgaccttag
13200

tccttggggg caatttgaat taatcatctg agtaagataa gagccagcaa aatcatgggg
13260

acgaggcact gggggaagac atactgtgtg ttcacttttg gtgtacaaac tgaccctctt
13320

gctaccatgt tcctttcttt acagcggatg agaacatccc agcgaagatc ctttcttata
13380

accgtgccaa tcgagctgtt gcaattcttt gtaaccatca gagggcacca ccaaaaactt
13440

ttgagaagtc tatgatgaac ttgcaaacta aggtatcttg gataaaatga agggaactgt
13500

gtctgctgtg ggcagattat ctgcgaatga gaggattcag ggctgagata tcagcaggcc
13560

agtgctgggt ctgttgtaga aggtctatgc taaagataaa caaatggaaa tatgatagta
13620

gttaatctct gatcaagata ctgaatatca gagtatccat tattcaagaa atctttattc
13680

tacgcataga agttctatac tggccactcc ctattttgaa aaactaactt tggtgtacat
13740

tctaattgct aattactgtc cttattgtac atgaggaaag tggatttaaa gagagtcttc
13800

ataattcctt gctacagccc agaaatacag ccatttccaa attagattgt ttcaaattga
13860

aaagatgatg aaacaattat atatttaaag tctttcaaat cctagcatag ctatttttat
13920

ttttacgcat tccacatgaa tcatattttt atatagttca tcatcaaaca tctgttgaat
13980

actggccgtg tgccagacaa cttgctgccc taaagggaaa gactggtgtg caaataggtg
14040

ctattccttg tccctgagcc tctttcctcc tcctaactcc gcctggtact gagcttttaa
14100

accataaggc acttaacatt tgatgatgaa catttttgtg ttagcagtga cttcataact
14160

aattcatcct aacatggtgg tccacagtga gaatttgcag atatttcatt atagaacaga
14220

tacacagggt taaaaaggaa acaaattata ttgaaataca gttatcaaaa tacattttaa
14280

agtccgataa tagatgtgct tccttattaa ggtcttgagg tggcctgtag taaatttcaa
14340

ggtagtaatt aattaatatc aatgatactt taagatttct gcagcaattc aaatatgaaa
14400

tgaagatgtc taacatctat tgatgataaa gtcataagtg ctgctgctat tactatggtt
14460

tgttccccat attcataact gaagaagaaa tactaaattt cagctagaag ctgggtgcag
14520

tggctcacac ctgtaatctc agcattttgg gaggttgagg tgggagaatc acttgagctg
14580

agtaattcaa gaccaacctg ggcaacatag tgagaccctg tctgtattaa aaatttaaaa
14640

ataaatataa ataagttcca aaaagaggtt attgaaatta tagatttttt ctcatccaag
14700

ttcatggaat tctatacttg tacaactgga gtttaagaac tactactgtg gaatgagact
14760

gcattttaag gcccagaata ttttccattt gttgagattt ccttaagata agtacccatg
14820

agtaatatag ctggttcaaa aggtacacac aacttataat ttatgctaca gatgccatct
14880

ggctttcttg aagagacgta catgtccaca gtgaatgagt atacatccat agacctttga
14940

ttccttggat catatgattg gcaggcaaag aactctatag acggcccatc ctagcagctc
15000

acatttgctg ccactctgtt aaatgctggt catctggaat tgtttattct cctcatgtta
15060

tcattacaag ccagaaataa aagcatgaat attcaggtgc tgtttaccta attcaggaaa
15120

acccctccca ggagcatgag gaggtctttt gttgctctcg ctctggaacc gggtttgggc
15180

agcccaggct gcctgccaga tgagccctcc aaacccagga ttcctctcct gtgtgcccac
15240

aaagcaaaca cacgcccctg tttgccaagt gaatcaatgg agtctttgat gctttgagag
15300

aatttgacat agcctgccaa tttagcattc acagaaatgg ttaaaagatc atggtttgct
15360

acatttctat cctaaaaaga atctggctag tggtgtctgt gatgcttttg tatatagaat
15420

cagtcccctc agtttggctt tggacaacat agatgttgga agtaagccat tcatagtaaa
15480

acatatttag tgatcctctc acaatggtgg cctctgtttc caatcttggg ctgtgtgtat
15540

ctaacaacaa acaattccag gttcagtgag cagcccagtg tgtgtcagac atgttattta
15600

ctccgtagag gttgtcttca cctcacatag atgaccatag gtatggggtt ctcctgccct
15660

ctgagtagcg tgagctcagt gacaggggct ctagctccat gttgttatcc actgctgtct
15720

ctaagcagga atctgagaag aacttagatt caccaggggt gcttcaaaga ttctaacaag
15780

caagaaaggc tgaaactgcc cttgtctctt cccttataga tatggattcc atgagagttc
15840

acatggggaa aaaagctcca cagctaaaag tgttttcaca tccattgagt gacaactaag
15900

gtccttccca cctcagactt ctctcctttt tttttgagac ggagtctcgc tctgttgccc
15960

aggctggagt gcaggggcat gatctcggct cactgcaagc tctgcctccc gggttcatgc
16020

cattctactg cctcagcctc ccgagtagct gggactacag gcacccacca ccacgcctgg
16080

ctaatttttt gtatttttag tagagacggg gtttcaccgt gttagccagg atggtcttga
16140

tctcctgacc ttgtgatccg ccctcctcag cctccccaaa gtgctgggat tacaggcgtg
16200

agacaccgcg cctggtgacc gctctccttt taaaaacaga atgccaaagc taagccctgc
16260

catgtcctgg tttgggacag actggctgag ctgtttggct tcctgggtgt cttttaattg
16320

ccagtttgta gccccttgag tactctcttc agtgcatcca ttttccttga gaagctggca
16380

ccgccttccc agagtactgg tgggatctga ggaacgagct ggggttgccc agtagatgca
16440

agtatcctgc ttcagtttcc taaagaggag ctcctacagt gttcttagaa tgaaagctaa
16500

gaactggcac aggatctatt tgataaacag ctgtcatttg tagccatcct cccagatgta
16560

tgacagcaga gtgttctctg atggaaagat cagcacccag attcccagcc ccagcgggtt
16620

cctttccctg agcccctagg ttactgccct gtaaaaccct tgtttctttc cagatcactg
16680

aagaacaaat agttgaattc actagtcctt gtgcatcttc tccctgcctt catgaagcca
16740

cccttgtttt tttttatctg acaaaccact gacagagaca gcctggtcca gataacatct
16800

tggtttcacc ttctcaggtg gagccatttt tcctctacag ctcataacct taccactatt
16860

atttccccta gattgatgcc aagaaggaac agctagcaga tgcccggaga gacctgaaaa
16920

gtgctaaggc tgatgccaag gtcatgaagg atgcaaagac gaagaagtat gtacctggta
16980

ttgtgaaagt tggggctggt agagaaaagt gtgcagcatc tgtcagggcc cctggggccc
17040

tggcttttcg atggtttctg agaaatgtct tttggaaatc tctatactag ggcttttatt
17100

gactcaaagt ggcaggatgg gtacagtgtg ctcttgtcta gagcccaggc ctggttcttg
17160

aggactttgc tattcttcta gggtagtaga gtcaaagaag aaggctgttc agagactgga
17220

ggaacagttg atgaagctgg aagttcaagc cacagaccga gaggaaaata aacagattgc
17280

cctgggaacc tccaaactca attatctgga ccctaggatc acagtggctt ggtaagtgtt
17340

gagccctcct tgagctcctg ctgctagctt aagaaaggtg gagggggttc cgagagcact
17400

ggtggccttc acatgccatt cctaagctac acactttagt cctctgggga aacttctggc
17460

ttcagctgtg tacaagttac tctggttgct gaaccttgtc tgtaaatgca ttgcaacatt
17520

ctggttgctc ctaaatggtc agtgctgtta cactgccttg tagtgtatat ttttaagagg
17580

caggtgccat ccctattcct aatggaactt taggacagga atggaaactc ttagcttctg
17640

gaagaatagg aaagagccca tccatctcta cacttcaaca aaacttttcg ttgaattttt
17700

ttgcagttga aggtaggaac aagagataaa gatgagaata cagatctgag caacttacac
17760

agagaggcag agggaccttt aacaaaagca gataaatact aggtttctct gtgtgtcaca
17820

cacagtgtca cataacatct gaaacaagtg gctttgttat ggaagatgtt tagtttgagc
17880

tgttaagttc tgagcatagg tggagatatc ctcccctatg gcacttgcta gtccgggcta
17940

aagtttccat ctaggtcttt tgtacctctt tctgctcgtt ttgccttgtt tggtgctaga
18000

gatttggtta gctcttaaaa ggcaatatag tatagtggtt aagaacatgg agaaaaactg
18060

cttgggttca aatttcaggt cacttaccag ctagctgtgt gagaaaatca gtaaattacc
18120

taacgtctct gcctcagttt catctgtacg tttttcactt gtacatctgc agaagcacct
18180

actttagaga tcactgccaa gattaaatga gttgatcaca taaaaccttt agaacatgct
18240

tggtgcacta ttaatttgca tcctcactag acagatgtga aagagaagat ggaacatctg
18300

accctgggcc tcagatatgg gccattgctg agtcacccta atccccccct tatttctcct
18360

ttgtttgcag gtgcaagaag tggggtgtcc caattgagaa gatttacaac aaaacccagc
18420

gggagaagtt tgcctgggcc attgacatgg ctgatgaaga ctatgagttt tagccagtct
18480

caagaggcag agttctgtga agaggaacag tgtggtttgg gaaagatgga taaactgagc
18540

ctcacttgcc ctcgtgcctg ggggagagag gcagcaagtc ttaacaaacc aacatctttg
18600

cgaaaagata aacctggaga tattataagg gagagctgag ccagttgtcc tatggacaac
18660

ttatttaaaa atatttcaga tatcaaaatt ctagctgtat gatttgtttt gaattttgtt
18720

tttattttca agagggcaag tggatgggaa tttgtcagcg ttctaccagg caaattcact
18780

gtttcactga aatgtttgga ttctcttagc tactgtatgc aaagtccgat tatattggtg
18840

cgtttttaca gttagggttt tgcaataact tctatatttt aatagaaata aattcctaaa
18900

ctcccttccc tctctcccat ttcaggaatt taaaattaag tagaacaaaa aacccagcgc
18960

acctgttaga gtcgtcactc tctattgtca tggggatcaa ttttcattaa acttgaagca
19020

gtcgtggctt tggcagtgtt ttggttcaga cacctgttca cagaaaaagc atgatgggaa
19080

aatatttcct gacttgagtg ttccttttta aatgtgaatt tttatttctt tttaattatt
19140

ttaaaatatt taaacctttt tcttgatctt aaagatcgtg tagattgggg ttggggaggg
19200

atgaagggcg agtgaatcta aggataatga aataatcagt gactgaaacc attttcccat
19260

catcctttgt tctgagcatt cgctgtaccc tttaagatat ccatcttttt ctttttaacc
19320

ctaatctttc acttgaaaga ttttattgta taaaaagttt cacaggtcaa taaacttaga
19380

ggaaaatgag tatttggtcc aaaaaaagga aaaataatca agattttagg gcttttattt
19440

tttcttttgt aattgtgtaa aaaatggaaa aaaacataaa aagcagaatt ttaatgtgaa
19500

gacatttttt gctataatca ttagttttag aggcattgtt agtttagtgt gtgtgcagag
19560

tccatttccc acatctttcc tcaagtatct tctattttta tcatgaattc ccttttaatc
19620

aactgtaggt tatttaaaat aaattcctac aacttaatgg aaacttaagt gtctgcctct
19680

ttgttacaaa gggcctcagg ccagagttgg ggctgggact tagtgtggga tgaggtctca
19740

ccactcaggt cagcaagagt agattcctcc caggagcaga tgaggcaggg cctggcctgg
19800

aaaggagtgt tgtgtgcctg tcctgctgct gtgggtgtgg gttaaagagg atccaaagtc
19860

catatcctta gataaaagac aggaaaggaa ggaagggtgc aaaaaatcca cagtaagagg
19920

tgtggtgcaa agacagcatc ggaggcctag cgtaggtaaa gtatattgag caggatgttg
19980

cagctgtatt tgagtcaaaa gttttggaga gctttccatg tgtactggcc agaagcagaa
20040

gggagctgct ttaggttttt tcacgtggct aagcttacaa tcaggatggg gagaattaag
20100

gttccagatc ccagaagccc cttcccacat gggatgcaaa cttctccctg cattgactgc
20160

ttaattgaaa aaggcagtgt ggtgtggtct ctgaccactt tctggtgctc cagttaccct
20220

agctggctat ggtctctaca aaaattgttc ctttgctcat cctaaattat aggtaagtct
20280

ggaattcaga atgaaagtga ggtctctgtc taggtcaaat ggactgcagg gagcaggaag
20340

gccagaaggg tcaaaattgg taaattaatg gaagggccct gggccctcta gcttgttagc
20400

acgagcaggt aggtgtcttc ggggaggcga acccatagtc tctgttctcc ctgaggctgg
20460

aggcagggct ggtactaggc ccacaacaga gaatcctgga ggtccttggc agcctgtatt
20520

tttattaacc agtgaaagag gcagcattca ccccagggaa taagggaggt tatgtagcca
20580

tcactgcttt ggagcagaga gactccagcc aggtgttttt gaattctgtg ttgaggtagc
20640

tgggccactg aatgtaggga atacaacaga gaggctcttt tggtacccat ttgatacact
20700

gtggctggta ggtaagtagg ttggtgtaag tggacctcag atgccacagc taggggacaa
20760

cctttggtcc aaaactagaa gtcaggtaag gaggccttgc aggcagaaga ccttgctgat
20820

gaacaggaag taatgcctac ctctctggca aggacctctc ttgcaagtct gagggcagat
20880

gctgggcaac aagatgctgc tcaacacaac taagaagggc atcccagaca gtggcagcag
20940

gcctgggaga ccaaagccag agaatgggaa gctgacctgg ccactgggga tgaaagaaga
21000

ggctgaacag gtaagtgggg gccagatgta aaggaccttg aaggatgctt aacccaggag
21060

tcaagaataa acttatccac ttgcctagtc cagtaggttt tacttccagc cacgtccaag
21120

aacaggccag atgggcaggg tctgagaaac tccctcccat caggcctttc tatgatgccc
21180

gagttcggtg tgggcaaagg gcttccacgt gctacccaag ccagatatct ctgtgctgac
21240

ttggcttttc tcctctctta cctcaccttc atctcccatc ccccaaatct ggccagcaag
21300

tcctgtctct ccattctccc tgccaaaagc tgtcaaattt cagacctctg ctacattaaa
21360

acatgaaaag taaaaaacct tcacaaccct cagccctttc cagccactac ctttcccttt
21420

tttcccaaac aagcacctgc aataagtcat caccatccag tcattccccc acccactgca
21480

actagcttct gtcccatgag cccacaattc cttctcttca ctctgaccaa tgtcgccaat
21540

gactagataa ccaaaccaat acatgggctc tccattctgc gtcttagagt ctttgtggca
21600

ctggacactg ttgaccactc cttccatcat tttgagggta cgatgatttt tagcaacttt
21660

gctgagtggt gcaactatta accatgtcag ttttagaaca ttttcatctc cctgtaagat
21720

cccttatgct catttacagt taatccacat tcccactccc ggcctccact aacctttcta
21780

aatttgcatt ctgaacattt catgtaaatg gaatcattca ataggtgacc actaccttct
21840

ctctgatttt cacaatgcca ccctctcctg gttttactcc tgtccctatc aggctttttc
21900

tgagtcacct tttctatcag gtcctaagtg ccatgattct tacttcagca tagctgattt
21960

catttgcacc taaaggtgat gacttctctt tctctctagc ctagagccct tttctaagct
22020

ccagactcac attatcaact acacctctgc agggcatgtc tacgggcacc tcaatctcag
22080

cgtctctaga actgagctca gtacccacca ccccaagcct gttgggcctg ggcttcacat
22140

ctcagggaaa ggcaccagat cctctgtccc ccaacccaga aactagggta gccaccatcc
22200

ttcactcctc tcgtttccac acccagtaaa ttactccaaa atcatctcct caataccata
22260

aaatttattt tctgaaaaaa ttttactcct tagaaattct tcaaataatg agacactatc
22320

aacttgtttt gaaccaaaaa gcacttttta gagaaaacac attaccctga aatgacctca
22380

gtcaggccag ttttggatgc ttataagtag tcccctgaca aaaatcagta aacaaggaga
22440

caaagaaatt taacacagac ttagtaattt tcacaatccc aagaattcca ttcagacaag
22500

cctcttagaa atcactaaat agcaaagact tttttagatt ttataattgt actgtgtgtg
22560

tgtgtgtgtg tgtgtgtgtg tgtgtgtgta tacacacaca attttttttt tttttttttg
22620

agacggagtc tagctctgtc acccaggctg gagtgcagtg gcgaaatcct ggctcactgc
22680

aacctctgcc gcctgggttc aaaccattct cctgtctcag cctcctgcca ccacgcccag
22740

ctaatttttg tatttttcgt agagatgggg tttcaccatg ttggccaggc tggtctcgaa
22800

ctcctgacct catgatcttc ccgcctcagc ctcccaaagt gctgggatta caggcgtgag
22860

ccaccgtgtc tggcccacac aaatgttttt aaaggagtct tttaggccag gagcagttag
22920

ctcatgcctg taatcccagc agtttgggag accaaggcag aaagatcact tgagcctcgg
22980

agcccaaggc tgcagtgagc tatgactgta actgcattcc agcttaggtg acagtgagat
23040

tctatctata cacacacaca ccacacagtc ttttagaaaa attgagatag taatggataa
23100

taagagttga ggtttgggtc aaaataatat gggaggggtt agtgggtaga atttggcatg
23160

aaacaaaagt ggccaccaat tcattattga agctagatga tgggtatgtc ggcattcact
23220

atactataca cgtagacaca tttgagattt ttcttttttt ttaattttat tattattata
23280

ctttaagttt tagggtacat gtgcacaatg tgcaggttag ttacatatgt atacatgtgc
23340

catgctggtg tgctgcaccc attaactcgt catttagcat taggtatatc tcctaaagct
23400

atccctcccc cctcccccca ccccacaaca gtccccagag tgtgatgttc cccttcctgt
23460

gtccatgtgt tctcattgtt caattcccac ctatgagtga gaatatgcgg tgtttggttt
23520

tttgttcttg cgatagttta ctgagaatga tgatttccag tttcatccat gtccctacaa
23580

aggacatgaa ctcatcattt tttatggctg catagtattc catggtgtat atgtgccaca
23640

ttttcttaat ccagtctatc attgttggac atttgggttg gttccaagtc tttgctattg
23700

tgaatagtgc cacaataaac aaacgtgtgc atgtgtcttt atagcagcat gatttatagt
23760

cctttgggta tatacccagt aatgggatgg ctgggtcaaa tggtatttct agctctagat
23820

ccctgaggaa tcaccacact gacttccaca atggttgaac tagtttacag tcccaccaac
23880

agtgtaaaag tgttcctatt tctccacatc ctctccagca cctgttgttt cctgactttt
23940

taatgattgc cattctaact ggtgtgagat ggtatctcat tgtggttttg atttacattt
24000

ctccgatggt cagtgatggt gagcattttt tcatgtgttt tttggctgca taaatgtctt
24060

cttttgagaa gtgtctgttc atgtcctttg cccacttttt gatggggttg tttgtttttt
24120

tcttgtaaat ttgtttaagt gctttgtaga ttctggatat tagccctttg tcagatgagt
24180

aggttgtgaa aattgtctcc cattttgtag gttgcctgtt cactctgatg gtagtttctt
24240

ttgctgtgca gaagctcttt agtttaatta gatcccattt gtcagttgtg gcttttgttg
24300

ccattgcttt tggtgtttta gacatgaagt cctgcccatg cctatgtcct gaatggtaat
24360

gcctaggacc tcttcaagga gaactacaaa ccactgctca atgaaataaa agaggataca
24420

aacaaatgga agaacattcc atgctcatgg gtaggaagaa tcaatatcat gaaaatggcc
24480

atactgccca aggtaattta tagattcaac gccatcccca tcaagctacc aatgactttc
24540

ttcacagaat tggaaaaaac tactttaaag ttcatatgga accaaaaaag agcccgcatc
24600

gccaagtcaa tcctaagcca aaagaacaaa gctggatgca tcacgctacc tgacttcaaa
24660

ctatactaca aggctacagt aaccaaaaca gcatggtact ggtaccaaaa cagagatgta
24720

gatcaatgga acagaacaga gccctcagaa ataacgccgc atatctacaa ctatctgatc
24780

tttgacaaac ctgagaaaaa caagcaatgg ggaaaggatt ccctatttaa taaatggtgc
24840

tgggaaaact ggctagccat atgtagaaag ctgaaactgg atcccttcct tacaccttat
24900

acaaaaatta attcaagatg gattaaagac ttaaacgtta gacctaaaac cattcttttt
24960

ctttttttga gatggagtct tgctctgttg cccaggctgg agtgcagtgg tacaacctcg
25020

gctcactgca acctctgcct cccaggttta aacaattctc ctgccttagc ctcctgagta
25080

gctgggatta caggcatgtg ccacttgtaa tgctaatttt tttgtagttt tagtagagac
25140

cgggtttcgc catgttggcc aggctggtct cgaactcctg acctcaggtg atctgcccac
25200

ctcagcttcc caaagtgctg ggattacagg tgtgagccgc tgcacccggc ccagccaccg
25260

tacctggcca agatttttct ttaaaaatgt ctttttattt tagacacagg gtctcacctc
25320

tgttgcccag gctggagtac actggtgtga tcatagctca ctatagcctt gaactcctgg
25380

gctcaaacaa tcctccgact tcaacttcct gaagagctga gactacaggt gcatgctacc
25440

atgcctggct aattttttta tttttatatt tttagagaca aggcctcact atgttgccca
25500

ggctggtctc caactcttgg ggtcaagcaa tcttcctgtc tcagcctccc cagatactga
25560

tattataggt gtgagctacc atgcccagca gagatttttc ataacatgtt taaagcaata
25620

cagtaactgg ttattttgaa gattattagt atatatccat agaggaattg aggggggaaa
25680

agcaacatct taatgttata ctaaaaggta attatgctta ggttacaatt ttcagtgata
25740

acatcacata ctaattcaga aagtattccc tttgcagcaa catggatgaa tctaaataac
25800

attatgctga acgcagacac aagagtacat actgtatgat tccatttatg tgaaattcca
25860

gaataggcaa cactaatccg tagtgacaga aagtcaatca gtggttgctg gatggggaca
25920

gaggagactg actgcgaagg cgcaccagga aacattttgg ggtggtggaa atattctcta
25980

tcttgattgg gtggaggtta catgagggta tacatttgtc aaagcacaaa ttttacactt
26040

aaaaatggat gtattttata tgtaaattat tcctcaacaa aaattatttt ttaagtaaaa
26100

aaaaaatcta agtaaaaaaa gttggccggg cacggtggct cacacctgta atcccagcac
26160

tctgggaggc caaggtgggt ggatcacctg aggtcaggag ttcaagacca ccctggccac
26220

ggtgaaaccc catctctact aaaaatataa aaaattagct gggcgtggtg gtgcgggcct
26280

gtaatcccag ctactcggga ggctgaggca ggagaatcgc ttcaacccgg gaggcggagg
26340

ctgcagtaag ccgagatcac accattgcac tccagcctgg gcaacaagag cgaaactccg
26400

tctcaaaaaa aaaaaaaagt tgtttgctct ctctctcaac ttagaaggga gggaaagtga
26460

tgatatgctt tgagagattc tattagatga ctcaaaaagc gactcatggt gaaaatacag
26520

aaatccaaga ttcatgtgaa gagttgaata tagtttactg aaaatgtgag tgtggggaaa
26580

atcaatgttc gtgaatcaat atattgtttt aaatatgtga tttaaagtat gtacctgtag
26640

ctaaataaac tattatatgt ggacatttgc atctctaaaa agtttatatt ttagttgcaa
26700

aaaaaaaaca acccacaaaa caacttggag tgaccctatg atgaaaatga gtgtttgcct
26760

tgtatctgga gtggccatct ctttcaggca tatacagtct ctcactttgc tctatctcca
26820

cagacactaa caggtttcag taaagccaag tgtcccttct cagtgctcca atggccccca
26880

tcacactgaa ttgtcatggt ctgttggcat ttctgtaccc actgctggag tgggaacttg
26940

ttctgactca gctgtgctgt cttagtgctc tagaggatgc atccgtgaag cgtgttgcaa
27000

ggaggagaga gaaaaacaga aaagtgcagg tctgccctgg cctgggcttt ccccttctct
27060

tcctttattg ccatatgttg cctcagtgat gcagtcacag tgtccctgcc acctttcaga
27120

agaagataca tctgcagccc agctggagaa cctcaacatc tgggtcccac tatgggggcc
27180

aatcagcaga gatacaccaa ggtcaatgga gacagcagtc cctgggagca gccgagccag
27240

gtgtgatttt gccttgagag agaacacgag gctcttgagg gacagataac acaacccagg
27300

tgaggaggaa agcaggtgga catgacctaa gaagtgtgag gcctgatgac taagttggag
27360

ccacagatag atttgggggc caggggtcag gagcctaaac agcaggttgt gggtggagtt
27420

caaggtgcac tgaggttagt tcttagcaag tagtactggc tgtgtgctga ggtgcaggct
27480

ggctcacaaa cccagaggag gggctgttca gatgtctaac acgagaaact gagtaaaaat
27540

taaagaatgg tttaggccag gtgcagtggc tcacgcctgt aatccctgca ctttgggagg
27600

ctgcagtggg tggatcccct gaggtcagga gttcgagacc aacctggcca tcatgatgaa
27660

acccccgtct ctactaaaaa tacaaacaat tagctgggca tggtggcacg cgcctgtaat
27720

cccagctact caggaggctg aggcaggaga cttgcttgaa cccgggaggc agaggttgca
27780

gtgaaccgag attgcgccac tgcacttcag ccaggacgac aagagtaaaa aaaaaaaaaa
27840

aaaaaaatgg tttaaagaaa aaggacaatg ttttacacat ggatcaaatt agacactgca
27900

agactataaa tctgcaccct tctaatgtga aaagggcttc actgagctat acttgtatat
27960

tatgccctcg atattcactc aaggccatta caaggaacaa atgaaaacgg catcagcatt
28020

tgctattaac cagttgagaa cattctccac tatttcaact aaataagtat ccaggtgaga
28080

aacttctttt ataatttaac cccagaaatt ttttttactc caaagaacca gttaacagaa
28140

tagaaagtgt tattgttttg gctatcagga agctcagcca tgtttaattc tcaatcacat
28200

cagctacatt aacccataaa ccatattatc gtcctcctcc ttataatctc atctcctatt
28260

tctatatttt attctctatt tttaagtact caaaacattt ttaaagggct gaaaaagaat
28320

acacttgtgg tagacatagc aacgtgccac ccagatcccc ttcaaagaag gacttgttga
28380

cccaactatt gggagagctg tcagcagaaa atcttcagct gtcagcccct cgaagatcat
28440

cttggctgca gagtgccatc tggcccaggc taggctcttc ccaggacatt acaactcctt
28500

tccaggctag tgccagacaa cagagatcct gggagttggc tgagactgtc atcaggcctg
28560

catctcagtt tagttttccc tgtgcccact cctgtttctt tccagtacct cccagagtgt
28620

tgatcccagg gccactcccc aataaacata ctgcacacta acctgtcaca gagtccacat
28680

ccaaagaacc caacctgtga cagcaccaaa tcaaaagaga ctcaggaggc tgaggcagga
28740

ctgcttgaac tcgggaggcg gagattgcag tgagccaaga ttgtgccatt gcacgccagc
28800

ctgggcgacg agagagaaac tctgtctcaa aaaaaaaaaa aaaaaaagag agaaaacata
28860

tcaggtaggg gtattttaca aaggagagag gatatacaat aaagtgttca tacatacatg
28920

ggaagcaaaa cagcactgac tctggggcca gactacctgg gtttgaatcc tgccttttag
28980

ctctgtgcct cagttttttc atctggaaaa tggggataat aagagcactt atagggctgt
29040

aatgaggaag aaatgagaca atgtggataa agcagttcca tagtgtttac tatatgtcaa
29100

ccactgttct aagtggtcat ttaataaaca gctgtgaggc caaacccaca ggtcccgagg
29160

ggcagttggg ctgggactct ccccagactc tgggcatggg cacaggtaca gtgtaggcca
29220

gggcatgctg aagcatgcat gttcacagaa ataggcctga gcagggctag catgggtgac
29280

gtcacaggcg tgtgcttgtg tgggtgctgg gctgctcctc ccagacagcg cagctgcacg
29340

tattgccatt cacacccacg cacccatata tatgctgagc tgggggaagc ccacacagac
29400

cacaccctct gcatgccctg tttttcctgc acctcctttg gtggaaggag gcacctgagg
29460

cctgggatgc tggcccagaa gtcccaggag gaaatgggtc ctgttccctg attacaggcc
29520

tggcaacggg ctgaaaactc aagctcttgt tggcagggat ttcttgagcc cctagaggtc
29580

cagacccaga ggcaaaagta aaagcaagga agccttaccc tgcatgttag gaagctccca
29640

aaaaaatgga gaaacggatg taaaaagtgc atcagagccc acaggctgcc ggagtcagca
29700

cagtaagctc aatgagggct gcagcactta agagggtgcc tcctgtacaa aacaggatca
29760

gacagccaga gaggggcccc attcagcccc cgtctgcctt cctggtctca tcactccctg
29820

tcccctcctc agtcaggaaa ctctagcttt gtaaccctaa attcaacaac ctcttcctgg
29880

cctcagggcc tttgtacatg ctgttacctc tacttggaat gtacttttct ccaacccacc
29940

tttaataatt cacctcgcca tcgactcact tcctttagac ctctgctgag gtctgaagag
30000

atctcgaccc aatgtttttc cttctagccc tcatcataat tgtctttatg taacagagtg
30060

tttatttaat gtctgaactc ccagagagct ggcgctgtgt ctctcttgtg ctcctcatgt
30120

tgtcaatgcc caacacagag aagagcaggg tccacagaac ttcaagccta agccagttca
30180

agccacactc tgtgagcact cacgttctgc tggattccct gtgcccaagg ccagcggtga
30240

gcatgcctcc cctcacccat gctgattaac cagatagcct ccatgcccac tgactactga
30300

ccaccaggac ttacctaagc cagtggatct cctgtgaggc agaaaactca tggagtcagg
30360

ccgacttggg ttctagttcc cacctagtca tgttatgtgg ctttggggca aattgtagaa
30420

cttctctgaa tctctgtttc ctcatcagta aattgtggac catcattcct gattagaaga
30480

taattatgag gatcaggtgt gaccacaggt gcacaccttc agcacagggt ctacgctagc
30540

tccgtcccgc ccacgtaaag acctgccact aggtggcagt ccatctcagc taccccaccg
30600

ctccttactc cacagaggct ggctaggaca aagggtctca atgtcatgct gaattctgag
30660

cttcttaggg gacgcacttg aggaaatcaa cgttaacaca caagtttcca ctacagcaag
30720

acaaagtcag aagcagcagc cccaggtcac tatccagctg agtgggttgc ctgagcacac
30780

gctcttacaa actctagtct agtcctacta tctcacaatt cccaggaagc ctggctcttt
30840

ctctggcatc agctggaagc tctgttgggc ttctcacctc tgaaatgcag taagttgtcc
30900

tgctgtgcct ggcagccgac ccactgcctg gctcaacatg tcagacaggc tccaccgcga
30960

ccgtgggtgt cctcaatttt ggcggtgctg acaagggtag atagattgca ggggtgagtt
31020

tcaagaatgg cttcagaaag acaaagagta tgaattatat tccataacca gaaaaaggcc
31080

ttgtggctgg agaatggaga gtgaaggatc acttgtgaga gatgaaactg gtgagatggg
31140

caggtcatat agaacctgct aggccaagca gggagtgtgg attttaggtg caatgggaat
31200

tcacggaggg ttttagcctg gatgtagggg atcatgattt aatttcattt taaagattta
31260

aagcatgata aatccaggca aaaccaaact gatagatcca ggcaaaacca aacaaggtga
31320

gtagtctggg tgacaggaga tggggccttc agcttgtggg ctgagtgaca aggtcttgat
31380

ggattggagg tgaggcaaag ggaggaatca aagttagctg caaggttttt gaccttccaa
31440

cttagagaag tttagcgccg cgttctgaga aagtgaagac tgtggaagga atcgtttggg
31500

gagggaaggc gagctgtcta aggggggggg atgtggaaca tgcagtggga tgctggggcc
31560

tggaacgtag gggagatgca ggtttggagt caccggtgcc tgtgttgttg ggcaaagcca
31620

ggggaaaggt caggtcgcct tgggatctgg cctagcactg agtgcagagg aagagaagct
31680

tctgagatcg agaggcagcg gctacaggag gaggaagaaa gcaaggcggg gaaaaccaaa
31740

gttcaggaga gcatttgcag gagggcgctg cggggtaggg tctgagtcgg agccccgaat
31800

cggactcgag tcctgtcgcg tggggacgga gcgtgcagag gcccatgcga gggacagaca
31860

caaaggccct gggagctgca ggtcagacca ctggacagcg cggccgcgga cctaacgccc
31920

ctgtaagggg tgctcacgct tggggggaac tctccgaaag agaggaccac tgaaagccac
31980

cctggcagca ggggcggaac agacagacgt gggtctggcc gtcgccagaa ctgcctgcgg
32040

accagccccg cgctgccggg ggggcgggac atgtcggctg tccatcagtg cccgcgacca
32100

atccgcaggc agccccgccc ccgcccctcc cgggagaggg cgcgcggagg acccgccgcc
32160

gccggtgtga ggcggcccgt cctggctccc ttgtccggga agcccgccca ggtaactgag
32220

tttggccggg cattcccgga ggacgcgcca tcccctcacg tcccgtcccg gtcgctgcat
32280

gggcggtgtt cgggacgcgg gggctgcgtc tgtgcgggac cgcggggtag cggccgcccg
32340

tgcgagtacg cctgactgac gcgccggccg ccgggcctgc ggcctgtggg cggggctgcc
32400

gtgcgtgaca gggccgctcg tggccgccgg gctgtgtccg gggccgcgtg agaaagctct
32460

gcgggactga gggctgggtg ggtcgaccgg gaacggcgcg cgctcccgcc gccatcgcgc
32520

ctccgtcccc gcctgcggcc tgtctggggg tcgcggggcg caggcgcggc gggccgacgg
32580

gcgggggtcc tccccacggg tgcgcgggcg cctttgttcc cggcgccgaa gcggggtggg
32640

gcctcagggc agccccgccc cgccgcgctg aggccccgcc ccgtgtccgc ctgcaggagc
32700

cgccgccggg tcccgctcgt ctgccgcctc agcctcagcc ccaacctcag ccgccgccgt
32760

tgcgcttgct cccgggcggt cctggcctgt gccgccgccg cccccagcgt cggagccatg
32820

gcgggcgccg cgtccccttg cgccaacggc tgcgggcccg gcgcgccctc ggacgccgag
32880

gtgctgcacc tctgccgcag cctcgaggtg ggcaccgtca tgactttgtt ctactccaag
32940

aagtcgcagc gacccgagcg gaagaccttc caggtcaagc tggagacgcg ccagatcacg
33000

tggagccggg gcgccgacaa gatcgagggg gccagtaagt gcgcccactt cctgcctggg
33060

cccgccccgc gcgggggtcg tgggagcccg gcccgactgc ttgcaccccg gccggccgcc
33120

ccagcgactt gggcaaactt tcgggccctc ccagactccc tccgggcccc gcccccgctt
33180

cgtctcgggt ggtcactggg ggcggggggc atccgggtcc tcggtcacct gacaggacac
33240

cccccctccc ccagctgggg ggagtgttcc aggcgctttg ccctgaggcc taaaaatcct
33300

cgcgggctgg agacctgcgg tgcaggcatc gggcccccca gaccctggga gtggtggcgg
33360

cgtcggcgag gggagctaag gcagtggccc ccaccctgca cgggaacctg ggcctgacc
33420

agacggtccc cgcccactct ttatccagaa agagcagtct gtgaaactct ccgggccccc
33480

aggctgggct cttatttgca aaggaatctt tgggttccct aagtagaact taggcagatg
33540

ttgggtaggg ctggtcttgg agcagagctg ggcctactca tctccctctg ggggagaggg
33600

aggaaggtgg tcttgtttgg tttggagaag cctgagagaa gccagtggct ggaatttttt
33660

tttttttttt tctgctactt ttgtcccaaa gctgggtttt ggaggcctat gtgggtgggc
33720

agacactcaa tgccagggca gggactcccc ttgccctggg aagcacgttc ccaggagatg
33780

ggccccataa aggcctgccc cagcgccttg gagtcaggta ttcttctcag gagtgcagct
33840

agtttgtgag tctagccctt tgtcctctgt ccggtggctt cagggagcgg gagcggcttc
33900

tcttagtttc ctgccttttc gccttcccag gcctgagctt ggctgctttt gcgctggtct
33960

ctagtgggag gagggagttg ggggattttg aaaaacccca gatctttgag agttgttcca
34020

cttagcgcgt gcctgtgctg tggtcggatg ggcactctgc tctgccctcc attcccccgc
34080

ctctcagtgt gaagagatgg tgtgtctgca actctagact gggttgcttc ttgcagggac
34140

ccccggagac tagggttaga gagacccagg ctcagaacca ggtctgtgtg aaggagggag
34200

atcaggaagt gacaccccaa gggggggtcc cacaaacctt cttgggtatg gtgtgcatgt
34260

gtgtagatag ttgaggagat tgggaggaag gaaggtcttg tggtgaggca gccaaccatc
34320

ctcagctaca aactccagat ctgacacaga tctaatgcag ctgctcaact tagaaatggc
34380

agcccccttc ctcagtgccc ccactgaccc tgtcagccat tgggagaaca ttttagtgac
34440

ttgagagcct gtggattcca aagtaaagga agaatcgaca ctgcaaagag ctgagataac
34500

cctgggaaca tcactcaggc atcaggctcc aggcatggtc cccttcctta ctccctatgc
34560

tgtgttgagt tggtggcact ggctggaagt tctcagagat ctcctgggta acagactgca
34620

ttttaaacca cgcatattag caggcaataa atgtagcaca gtggttgatg gcaagtctgg
34680

atttgaagcc catatattca ttgtgtgttt cccggccagt tacttacctt ctctgagcct
34740

tgatttcctc atgtgtaaaa tgggggtaat aatatatcca gtatatatgg ttgctccagg
34800

atcaaatgag acaattcata taaaacatct agcattgtct gacacataaa tgtttaataa
34860

atatgagctg ttattagcaa taacattaac aatagaccgt ggctgccctt caagaccctg
34920

aactgggaat ggtgaggggg tgaggtgtgg ctataggtga ggatgaaggt gcaggaaggg
34980

aggaggacgg tgaccaaatt cacatttcac ttagattgct actcagaaaa aggcaggaaa
35040

cagttttgtt ttgccccaac ccatgtgccc actcacccac ccaccatgta gaacagtgtt
35100

tctgtagaga aagtgtttgc agttgtgcac atttgactcc tgggtgtgac ctttcttgtt
35160

tgtaagttgg ggcctgaaca gaaaagaggg gtgcagagag gggatgattt atcatgcctc
35220

atgtatttgg gggatggagg caggcataca gaggacctga taacccccta cccaggggtg
35280

agcaggtggg tcagccagag gtgtacaaat tcagtgatct catcagttag ctgggattta
35340

acaggcctgg agagtaggca gaggatgtag gaggaccctg gcttttggat tttgggggct
35400

cagattgaaa agagcctgga tgaaaagtct ggtcgatgct tgtgtgttca gacttaacta
35460

atggaggctt ccactgaagc cgtgccctgc ttctacccat tcgtgggctc tccttgttgg
35520

tgggttctga tccctgcggc ctctgaggag gttggactgc cagccgggtt ttctgccacc
35580

tggactcttg cccaagcacc tcaggtatct ggcctttcct gctgacccta ccagggtacc
35640

agcagtgcct gaagcctggc ctaagagaga gacactgtgc taccttcagg gtgttggtag
35700

acctgaggga gagacagtgt ggtttaagga gcactcatac tgatatgtaa gaccttcttc
35760

tgccctgact ctgtgccctc tcttgtcagc agttgtgaaa tggagctgac acctcctgat
35820

ttctagggtg gtcatttggc tcaaaagcgg ggtggggaag tgcttaggtg ttgacaagtt
35880

tggttgtcct ggtggtatga caggaaccag gttggtgttt ggccttttat gttgcagata
35940

ctgacataaa tgaataaaat gcagttatgt agagttccag ctgagtgcta ttgagacctt
36000

tcgtcccatt ttacagatga gcaaatggaa atggagaatc gtttgtgccc aaggtgttgt
36060

gtcagtgtgt gccagggcct cagctggggc cagctcacta gtcagaggct gactagcaac
36120

cccaagccca tgttccctgt ggtccacgtg gctcctctgt gagacacctt tgtgctcaga
36180

ccatgcatta cagggcaaag accactttgg cttttgtatc ccaagacagc ctcagcctct
36240

ctaggctctg gctccacact ttttcttctg gtgttttctg agttgagatg gttcagactc
36300

gtgtttaatg aggttgtcca tttgagggtc tttgtgggcc cagtaaacac tggtgtagac
36360

actgtggagg tactctgaat actggctgag catgtccatg gggacttctg aggccccagg
36420

cttctgtctt tggctgatcc ttcactagat acttttctag atgccaggcc ctctgaggtg
36480

aaggagtatg ggtgcttgct tttagggaaa tgaaaacagg caatgacaat tgtgagcagt
36540

acctttgatg aagagggtga gggtggcgga agaaaacagc taactgcctt gatgaggcag
36600

gcgggctcag aagggaggta gtagtggaac cagctggctg gggcttgacc cctgtggctg
36660

gggagcagac cacaccagga ggcctgctcc gagggctagg cccacagcac gggagagggc
36720

aaggcccagg ccggtgtcat caagggtttt ctttgtactg gctgttactc ccagtgggag
36780

aggggcagga gcacatgtcc ggatctccag agctagccat ccctgcctgt gtatttcctg
36840

tctgatgtag ttttttccaa gggaagtcag accttttagt gtttcctcag caccccaggc
36900

ttgccccgat gtgtgggttt ccaggccccc ccgcccagct gggatcctct gtttcctgtg
36960

aacctaggaa aagggcctgc ttcacagata actggaggag ctgagcacac ccagtcctgg
37020

gacccacaac acctccagct tctctccaga ctcttgtgtg tgtgtgtaca cagatgcaca
37080

ggattcagtt ggagtttaga gatcttggct accagctttg ctctgggtag cttcttggaa
37140

tgaggcacat ttattaatat ttgcaataag tattattata cctccccatt tgcccagtgc
37200

taggcaccaa ggattcaaaa gtttattgag acatggtttg tgccattgat gaactcttgg
37260

aaaagacaga tgtagaggca tatgattata accctgtgtg ttacatacca taatataaag
37320

gcctctacca agaactgtga ggagctctgc caggaaggca ttaaaggaga ggtgaccttt
37380

gaggtaggcc ttgaattact gaggcacctg tctgtgccga ggctaaggaa ctgggaatca
37440

ctctgtgcag gggtagggaa cagatatggt gtggtagaga ggaagctggg actggttgga
37500

gacagctata gccctagtgt gccatgtcca gcagattaca ctcagtcctg gaggcgacgg
37560

gagtcagcgg aggctgttgt gggactgact tgcctctgcc tgggagacca cagtggcagt
37620

ggtttgtcag gcaggtgttg gggagggagg ccagtgagct cctacggttt gcacagtttg
37680

caggagagag ctgctgaggg cccagacaag accttcttgc tgaggcaggg tggggatgcc
37740

atggtggtag atgggagaag ggctgggtct tgaggggtga gacagagctt agtagttaag
37800

catttgggcc ctagactctg cttgaattaa aatcctgttg tgcagcttac ttgctctgtg
37860

accttaaagc atatgactac ctctctgggc tgcttatctg taaaaaggat agtattaaat
37920

ggcatttatt ttataaggtt tttgtgagga tttgatgaga acctgtataa agtgcttagc
37980

acctgtataa agtgcttagc atggagtgtg gcacagtgta agtgcttact acgtaggatc
38040

cctgacagga cagagatgag gattaagttc tcaggaggac ctggtgggga ggagttcgaa
38100

tgcacctgct gtcctaaggg gctggtgcct ggccaccaag ccctttgatt cagaggcaag
38160

gaggcccgcc catcctctct tgcagctctc agcagtccct ctattgtcag ggctctttcc
38220

cactgctggt tttgcaggga gacagggcag ttggctggaa gcagctgctt tgggtggtaa
38280

ccccttctgt agagtgaagg tatgatattg tcctatttct aagtgcctag cccaggacgc
38340

tatctggaga gatcttgtct ttgaggaagt gctctaatgc cacagcctcg atgtggtagg
38400

gcctgcctct gctgtcactg ttctgtgtca gtctccgtta actcacagca tctcttctta
38460

ctctgtgcct tttcctgtgg gatgtggctt caaggagacc acagatgata agttggagga
38520

agggagtgag gcctgagatt gcaaaggtgt gatattagga gactgatttt tccccctttc
38580

cacttgcttt gttagaacta gagaagtcac tgcagactgt gaggagagtc ctaggagaga
38640

tgggtgtggg ataggaggat gggaatgttc tggtgcccct tgtagagtgc cttgttgaga
38700

ggagcatggg aacagtgcgt ccttggaggg aatctctgag agcagcatgt ttacaggtgg
38760

caagactcaa ggtggagcca cgggtgagga gtggcttcag gctaccattg accagatagt
38820

cccaccttgc tggtaattga gctgaagtgc ctggagattt gtggctagag ctggtcagat
38880

ttgggttcag gtctcagctc ttactgtgac cttgggcaga ttacttttcc ctgtctgaaa
38940

ttgatcttta gctgtaaaag taatttctcc tcacagagct gtatattaat aataagggag
39000

attatatata agagtaagag attttatgtg aaaactaccc agcacaacat ttgtcactta
39060

gtggccactt aacagtattc atttccttta ctctcacctt tgttaaccac agagctccaa
39120

tcttttccac cactaagctc ggccacaagt ggctgctcca acaacctggg ctaggagtct
39180

gtttcctact tgctttctgt gttccttccc tgagtctgta cacagaggac tggggcatca
39240

aaaaactctg gaattggaat agcatattgg gagttcatgg aacattttct cacctgttgt
39300

attgtttgaa cttcacagca gctccatgaa tccatggagg gcaggagtta ctatccctct
39360

cttacagagg agaaaaccaa gccctaaggg taaggtcaca tagcaagttc atggcagatc
39420

ctaggatttc agattcctgc tatagtattc agtttattct cccattctgg ggaaaatgat
39480

aataggatgg accgttctat aggaacagag ggttattttg ttttgttttg tttttcttcc
39540

ctctggccac atgtgtttga ccagcctaga cttccatctg tggacaagct ggactcattg
39600

cctcaatttc aaaacccgtc ctgcactcat ccttgccaca acaccggtgt cgagatgctt
39660

ggcccaccca gacctttccc acactcctac atgggggtga atgtagccgc tgattggggg
39720

tgggacacag aagagcctca ccctcttgac tttctggttt gtggtgacac ttccgcctaa
39780

gcttctacag aacatgggaa cctggggctc catgccccaa atatcctgag aagggctttt
39840

ccaggattct ctatgacaac tcaagagcat gcattcattc atgtgctctt taatttgtca
39900

tacatttgtt gtgggccttg ctactgtgtg ttgggcacac tgagctggat gtgattactc
39960

cagcctctga cttgttcatg gtggcagtag atctctagcg tggtttgtgt tccatctcac
40020

catacctact ctccttaaaa aaaaacaaaa aactagcttt cttgggcctc agtggctcat
40080

tgaataagtg agagacgact tgtgcttccc tggctttaga ctctggcatt tgctaactgt
40140

gccaagcact gtggagacca gtagtggaca taaggttagg gtagatacag ccagaaagga
40200

gataaatagg cagacagata aaattcttat aggatttggt gagttctatg aaggaaataa
40260

ggcaagaggc tgctctgtca gggtaggtga gggtgtgctg tgataacaaa tgatgcccac
40320

atatcaatgg ctggcaacaa ggaaggttaa tttcccctgt ttgctatgtg tgctaaccta
40380

caccaccgtc cctggagagt ggcctcagtg gttctgggtg aggcctctag tacagaacct
40440

gaacaccctg ggcaaactag gctggagagg gatttggagt atggaggttt agaaggaatt
40500

aatttctatg acttaggatg catgtggtga gtgtggcttt gctgttgtgg tagagtagag
40560

tgctgcctgt gtgggtcaag agactgtctt ggctgacatc tttgaaatag gtatatttgt
40620

gtggcccttg cctaattgtc atctctgcca tagctgcccc cgccttcccc accaacccat
40680

ggccctactt gagctgcctg ctgagcctac tctgttgtgg ccacagtatc ctcctgctgc
40740

ccagagtttg agtttctcgc tacttctgcc actcatcaca cttgcttccc agagctttgc
40800

tgggtgtgct gaagtctcca gagaaagtgg ggaatatggg tgcctagaga cttctagggg
40860

gtgttttcag cccctagaga gtagagaagt gtgagaaggg gttggtcccc gaatattctg
40920

tagttttggg gaagaagcaa tggggacagt ggagttggtt gccttaagag aggctatggt
40980

cccaagagat gggacttgga gagtctctac ttgcattcct ggctaagccc ttaatattct
41040

cgcacccctc cttgcaagac tagttccttt tgaggtggtt cttggctagc ttttgagagg
41100

ctgcagtggc tgtgtgccca ggcttaagtc ctagctttct ccccactctt tacctcctag
41160

gaaccgggcc catcctgggg gaggcaggga ggagctcctt tttatagtcc acatgtaggc
41220

tttgtgtata ggctctgctg cttccttccg gccctggccc ttcctggtgt tggccggtgg
41280

ccctggcttc agcaagactc aggatgcaga gtaaacgggg cagggcgttc tcctacagaa
41340

gactggcatc cctttttact gagcccaggc ctggcacaga ggaggctgga gttaggaagc
41400

aagaaaacga ggtgacccag tccttgatcc tgagaagctt gtgatctgga ccatgcagaa
41460

aaccgctaat gcccagggct ggcaggagac agctgtgcct aaagtgctgt aggctgggcc
41520

tgagggccaa gatggaatcg cttttctccc tgtagcagcc catcctaccc cctggggtcc
41580

ctccacctgc cagctggcac acaggtgttg ggtaaagcat ctgtacaaag gctgtgaggg
41640

gcctgggcgc aaggcagcaa ggcttgggta cggaggagag tgggtggtct tactgatgtt
41700

ctgggccctg acagtgaaca gctggccaaa ctggtgagga gttgggccat ggcgagtttg
41760

gccacttgtt tgtgtgtgtt tagttcttcc tgggcacagg tggggagttc actttggggt
41820

tggcttcctg ttttcttgga cacctgggga tatggttgag ttaaaatgtt ctgagggaaa
41880

tgattgttga gggcatgggc ttgggtcata gtgtccttgg cttggccttg cccatggtag
41940

gagtcgggta ttgaccacac tgggttctgg ctgtgtaggt gtggatgtgt ggtgggagaa
42000

ttccaacccc accttgactt cagcctaagg cccagagagt gtgggcctga gttcacaggg
42060

tgactaaccc taggaaggag tgaaggagag ctccagttgg ccataggtcc cacaggtttc
42120

tcagcttact tgggggcagg aggtgagcta aggaatacaa ctccattccc tcagttggga
42180

ggttgggcag cgatgggggc aggggtgatg gtccttctct gaggcactga agacccactt
42240

tatggtcacc ctattctgcc tgatagtcca ccacctgttt ggaggcagct cccctcttcc
42300

tgccctagtc acttcctcca ccttcaacag taattgagta taatgtgcct gtgttagctt
42360

gtccatccta actgggactg tggccttttc ttgggcgttc ttagcatatc taccaaggag
42420

gttggaaaag gtaacatatg tcactttgtt aaggattttg ctgagggcct tttgaggcca
42480

gcagtgaaac acagagcatt ccctccagtg agaacagaat gtctgttgcc agactgatca
42540

caagaggatg gcctggttct ggcctgagaa ctggtctaca ccggaagcat gaagctccat
42600

acaatcatgt gtgctagtaa agtttgggca agcagctgtc ctaagcaggt aaaacgatag
42660

gttattcaac ttcaacttca gagaaagaaa tccaaggtag ccatctctct cagtagggga
42720

ggtgagttca aagaatttaa gctgggctgg gcacggtggc tcacgcctgt aatcccagca
42780

ctttgggagg ccaaggtagg gggatcacga ggtcaggagt tcaagaccag cctggctaag
42840

atggtgaaat cccatctcta ctaaaaatac aaaaattaac caggcgtggt gtcaggcgcc
42900

tgtaacccag ctactcagga agctgaggca ggataattgc ttgaacccgg gaggcagagg
42960

ttgcagtgag ccaagatcgc gccattgcac tccagcctgg gtgacagagc aagactctgt
43020

ctctttaaaa aaaaaaaaaa atttaagctg ggctttctgg gcaggcagtg agtgaatgag
43080

atccctggca ccttggagtg agatcagagt ctagatcact cactggggcc ctgtttccca
43140

ggaagcaaaa agggaacagg agagtggctt ggcttgggct ggcatggcgc aaagggagtg
43200

cagccagatg ctgtggcggc tgggtcaagt agctctaaaa caggagagcc tagtgggttc
43260

tgctgaagca ggatgtctca gcaagcaggc ctctgacctg acgacttccg gtcattattt
43320

agaacgtctc cagccccaca gatttactct gttggaggct taaggactaa accaacattt
43380

actgtccaag aggtggcagt aacacagaaa ccaaagaggc aggaaccaag gttggggtgg
43440

tgagatgtca tggggttcag ggagtggcag aggggaaggc ctctattctc cctttcccac
43500

ttgtctccct acctcagccc ctgttgctct gacgcttccc ccaccactat gagtcctagg
43560

cccctgttgt ttccttgtag gagggagacc ccagattttt atttcttctc ccaacccatt
43620

ccatttcctc tcccttgtga gagggctctg cagagacagg gcagcagaga gagacttgga
43680

gatatgctgg gctgtggtca ggagggcctc atttatctag tgagcaggaa caggttatat
43740

agcagtgatg ctttcatagt atttttcttg aataaggtcc ccaaaacctt attaagttcc
43800

tggtaggcat ggagcattga agatggcaga catggtgcag tctctcaagg agtttacgct
43860

ctagtggaga aaacaatcag acagccttaa aatacatgta tggctactat aataaatact
43920

gtgaagcagg agtgcaccac gaaacctgga gtcctctcca tcctcccctt ctgtctgtgc
43980

tggccctgct tggccacttc caacaaggat tctgaggcat tacagagcac acgtctaggg
44040

tacaggcagt cttggctttg aagtattgtt ggctttgcca ctcaccaact gccaccttag
44100

gtaagttaat taacctcttt gtgctcaatt tcctcactcg ggaattgggg accctaatac
44160

ttacttcata gtgttatttg caagtataaa atataaggca gtttttatcg ttgtaaggca
44220

gtcttatcaa gagctttatg caatgtctat gcaaagtaaa tatgcaagaa attagtgtta
44280

ggatgccaga tgctgctgtt gctcatcgtc tcttcccacc cagcagaggt agtaactact
44340

tcatcctcta cttctcagct tcttatagta tttgtctgtg gactctaacg taagctatct
44400

agcataatac tcatatctaa tttttccttc cacattcaag ctccttgagg tacttgtatt
44460

ccaagtatct agtttaatgt ctctcatgta ggtgctcaga acgtatggtt aattttgaat
44520

aataaagcca gtgggacttc tcagagggct ggattggggg ctggtgagag aaaaggagat
44580

gcgaaggatc attctgaagt ttggagtttg ggtacctgtt tgaatggtga tgctcttagc
44640

tgacccaggg ggtatggcaa gaggccttgg ttctggtgag agagagaaga cagtgtttgg
44700

ttttgaggag ttgtctttga gacgttttga gatgaagagt ggggatagca agtaggcagc
44760

tgaattgaag gatctgaagc ttaagaggag aggactaggc tagagacctg tttgtgaatt
44820

atctgtacat aggaggtaat taagggtgtg ggcatggctg acactgatga ggaagaaggc
44880

agagcaccgg aagagaggac cttgacctga gccttgacca aggccattgc taaatgcagg
44940

taggcctgta gcgcacaaag aagcatgaaa accaggcaag gttgtgcagg agaaatcaag
45000

ggcagagctt caaggagaga gggcgtgatc aaccatgttg attgctctga ggtgtcagga
45060

aggaggacca tagataagtg atttatgggt caggcacggt gattcatgcc tgtaatccca
45120

gctctttggg aggccaagac tggcgagtca tttgagccca ggagttcaag aacagcctgg
45180

gcaacatggc aaaacaccat ctctgcaaaa aatataatca gcctggcgtt gtggtttgcc
45240

tgtagtccca gctacctgaa aggccaaggt gggaggatca cctgcacctg ggaggtaga
45300

ggctacagtg agccctgttg gtgccactgc actccagcct ggacaacaga gtgacttatg
45360

ggccaggcat ggtggctcac acctgtaatc ccagcacttt gggaggccga agtgagagga
45420

tcacttgagg ccaggagttt gagacaagct tgggcaacat agtgagaccc catctctata
45480

aaatataaag aaaaatttta aaagtgactt atacccattg ctctggttct ccaagtagcc
45540

tgagtgtact atgtggggga gcattttatg agggactttt gaggtcctgg gcatccctga
45600

gacatggccc tggtgggtcc ccagtctctc tcctgtagct aggagcctgg catacacagg
45660

ccatctagaa agaacttagg cagcatcagt ctgcaaggga ggcagagttt ggagcacact
45720

agactcctga cacagaggct gacccatggc tgcctggaga atcttctggg cttgtgcaga
45780

aaagttgggg aaagccacct ttccctactg tggcagtcaa ggcttggtat aggccatctt
45840

ccctcccttc tcatccatga tactctgaat gccctgtttg tggaacttag tggactcctg
45900

cacctctgtc ttggtttcca tgaacccctc tgcccaatgt gtgcttgtca gccctccaga
45960

cctggctttt acagagcact ccaagacatt gctctacaca cactcagctc cacagacacc
46020

tccctgttgt tctactcgtt gcatgacctc atagttatct gcttccccat ctgtttcctc
46080

catcaggcag gggttttctc aaaggcagga actcaaatgc cttattgatc tctgtttccc
46140

taggacccag cccagggcct gacatggagt agatagatgc ccgataaatg tttgatcaga
46200

tgatggaatg aatgactgca taagccgaat agatcactgc tgagtgctct tccagcttta
46260

atttctctgg gtgggacctc ctttcccttc cttcctttgg attctgttct tacctctgaa
46320

ataggtgaga ttaatttaga gtcctaggga agagggctgc ctgcctggat ggtacaaagg
46380

ctggctgtgg ctgctctgtg tttctctgta tacccagtgt cagggatagc agctgctgtg
46440

gggacactgc agagtgtccc agttctgcca tgactcacca tatgaccatg acaagaccct
46500

ttggaccctc tgtttcctca ttgtttcaaa gagatttgtc tagaatctag accaagcttg
46560

tccaactcgc tgcccgcggg tctggtttga aggcagctgc tgcacagtaa tgggaaggac
46620

agtaagcccc accatcgcct tccctttgcc atataaccct gtgacctcag aagtccctga
46680

acccagatcc ttgtttgtaa aatggaaagg gatagctacc cacccctcca ctttgacctt
46740

cctttggtgg cagccagatg tgctcaggga aacctcagat ttgtgagtat gtttctactg
46800

agaggcagga ggaggaatat tatagtctca ggaagaggga ggaatgagag ggagaggaga
46860

actctcaccc tttccacagg ctttgggcat atgcagccag cttggaaatg ctgaccatcc
46920

tgtgacctgg ctactattgc atcaggcttc cttcccacct gtgtccaggt cctatctttg
46980

tctcttcttt ggctacctcc atccttttcc ttcagagctg caaaaacctt gccaaagccg
47040

tatggtcctg atgggtgcag agcccctgtg cctctggctg cctgtgaagg gggatctgca
47100

gtgcagtgcc tgccgtaccc cacagggagt tgtataggaa cctggggatg gggtgactga
47160

ggtagggggc cagaagtggc ccagatgata ctcacttctg ctggaacttt tctttgtcca
47220

tctttcagca tcctctggtg gagaaggctt agctccatta atgctctcct aggtcctggc
47280

tgtgtatttt ctttggtatg ggagtgggga actgggaggg actcatctgt ttgccacaga
47340

ctacatgtgg agagggtgat tggtgagagg tcattttgga gatctacaca cctcttctga
47400

cccctgggac cactctcctg aatctcctca tggtcttggc ttcgggccca gcagggcgtg
47460

ggtgctcggc atcctgctgc ttttactgtt tttcccaaag ctctgttagc ttcccgtggt
47520

tcccactggc tataggaatc tttatgtaag gacttcccag tgtttccaga ctgtatttgc
47580

ccccaggggt cacattcccc ccaactatgc ctgctgctgc tttttctgtt cccgttgtta
47640

cctcactgac cactgcattt tcagtatttg ttgtagcccc tcagcctctg tggcccactg
47700

tgccacctct tccaggaagc catccttgat accatctttc tggtaccatt tttgagcatc
47760

tgtggccttt gctgcctgtt agataattag ctctggaatc taactcccat gtgtttactc
47820

ctgctcttaa gtactctgaa cattttgagg cctccccagg ccctgggttt gtgttggact
47880

ggtagggaat ggagagaaga gtcaaatgtg gtttcttcct tggaggagct cataggccag
47940

tggcctaaga caccagggaa gcaccagcag gtgtgacaag tggtgacaca caagagagga
48000

gggggcacct agtgtgcccc tggcctgggc ctggtggggg cttaagtgag gaggtaggag
48060

tggcttgtgc aaaggctgag gcaggaagga gtatggggag acatgggctg gtgtctgtgt
48120

tgtgttaatt ggacttggac ccttttgagg agggctgggc tttccctgcc ttcctccagt
48180

gccctctgca gagtggggca cacaggcctt gaggggttct ggggtccaca ggccacaggt
48240

gggggtagta aaaaccccca gaagggaagg aacctgtcca ggaaatcttc gcttacctct
48300

ttcaagctct ttagatttta aagagacctt tcatatttac tatgagttgc agttttttta
48360

agttacaaaa tttctttact ccatgcatgt ttaaatggat agtttttgct accatttaca
48420

tcaattagct tacacacttt gggttttgtg tttttgtttt gtgtttttag agaaaggatc
48480

tcgctttgtt ggccaggctg gagtacagta ctgtgatcat agctcataga gcttcctact
48540

catctgcagc ctcaaaccct tgggctcaag tgatctttcc caccgcctcc caagtagctg
48600

ggactacagg catgtgctac catgtctagc taagtttttt taatttttat tttttgcaga
48660

ggtggggtct tcctctgtta cccagatggg tcttgaactc ctggcctcaa gcagtcctct
48720

tgcctcagcc ttctaaagtg agcgtatgca ctttataaag ggaaaaccct ctctgaagag
48780

aaatatttca gacagggcat ggttgctcat gcttctaatc ccagcacttt gggaggccga
48840

ggcaggcgaa tcactggagg tcaggagttt gagagcagct ggccaacatg gtgaaacccc
48900

gtctctacta aaaatacaaa aatcagctgg gcatggtggt gtgcatctgt aatcccagct
48960

acttgggaga ctgaggtatg agaatcactt gaaccccgga ggcagaggtt gcagtgaacc
49020

gagattgcac cactgcactc cagcctgggc aacagagcga gacacccaga cccagagaaa
49080

tatttcagtg aaagcatgtt tcatgttcag aaatttgggg tacttttggt tatggttttc
49140

ttggccatat cttcatcatt tggtgggttt agttttcaat atgtcctcct gtccctttct
49200

gattaaatca cttctgtatc tcttacctca ttggcatctc actgtagtcc aatgaaggag
49260

gtgccaggag tggtgattct attttgcaga tgaagaaaat gaggctcaga gagatgggtc
49320

caggcctcct gcccaccttg ccagtgcttt tgttcataat acctctctac accctttttg
49380

cctgctgagc ggttaacagg cttataaatt tgggggacct ctgtctgttg ggctatttgg
49440

aagtggcttt tttgagcaca ggggatacta ggacacaggt tccaacaaga aacttaggtc
49500

taatttaggg tcttggattt gatgattggg ctccctctcc cccttctagg gcctgccatg
49560

tgtctgcccc ctagggcagg attggctggc tctcactggc tgtgctctga ggagccctgc
49620

ctttgctctt ctctttctct tccctagggt tcctgctgtc cttgaactct gccggggcaa
49680

caggaatact gcccttatgc acatcctcgt ggtggccaga cagggtggtt atcccacttt
49740

actggacagg aagagagcta gtggcaggcc tgtgacttgg cctgatgtct cgggggagtc
49800

ggggtgggag caagagtcaa agacaggaag cagccctacc ttcagaagag ccaggcacac
49860

agatgggtcc tttgtggtta ttgtcttctc cctttcccag gttccagact ggctgcccgc
49920

catatagctt gtcccttcga cagcaccctc tctccccact cagtggattg ggtgctgtcc
49980

cagggtgggg gcttactctc ctgcagaggc aggttctcag ctggccctga gggttgtata
50040

gcttcacagc tcccagagtg cttgggcata gggtggctca gatgaggaaa caggctcctc
50100

ttttaaaggg aaagcaactg ccattactgg catgtccaaa agggggatgc tgtccaggac
50160

tgcatggccc agaagctccg ctctgagtaa ctgctggtct ctaatctctg tggtctcagg
50220

ccaggagtct gtccctgggc tgagaaggga ggggagaaga gggaagagat ggaaaaggaa
50280

cattgctctt cccagtctcc ctggcctcgt catataacct tttattttac aaattcagtc
50340

ttgtggcatt ttgctttaat gcttagagct ggccccaggt gccaggcagc aggcacattt
50400

ccttctgccc aaaaaacaaa acacacatga atctgaggtt gggcgtggta agggcaggcc
50460

aaggctgacc gtcctatgtg gatatctgtg aggtagtgtg cctacatgta agggtctgtt
50520

gtgggtctac tcccacgaaa gccttcatct tacgatgaac cagagaggac tgcagacttg
50580

ccaaggccta gctatatgct tggagccagg atttgaaact tgatctttgg ggatgcacgt
50640

gcctcagact attggtgagt gtgctggtgg ggcactgcag gctcaggaag cagcgtgtct
50700

gcatatgctt atctgtacac aagtctgtaa gagtccccct tctccccata caatgccccc
50760

atccctgcca ggcttctttt tgagctggcg aaatagctgc ttgtattctt ctcttcatgg
50820

atcatcttta tttcacccaa gtttgggttt gcacctagtg gagccttggc aagtggcctt
50880

atttgtctcc actactgcca gcttttgtag gctccatgtc aatggcacta ggcacacagg
50940

tagacaggtg ggctgtctcc agacttctct attaccagca cttcccagca tggccctcac
51000

agcaaccatg tgaggtaaga ggaatttcca gtttcctgat ggggaaactg agaccaaaag
51060

agtgagggac tgccaaggcc tcatagctag aatttaagag ctgtgtggtt ctcctcacta
51120

agctgggaag gggatggtca gggaaagctt cttgaaagag caggagaagg cagatctcca
51180

tcagggcagg gagcagagca aggccccagg attctgcctg gggcaccagg aagcctggct
51240

tatgagaagg ttctcagtcc tcagagctga catgtgatat acagtccagg cacaggtgtg
51300

tgtggtccct cattacttca gagcccctta agaaagactg aatctatttc ttcatcagag
51360

tttgtgagcg attcaatata agggactaaa tccccctcac tgccccaagc agcttatctt
51420

gtcactgtct accatagcca catttggaat ggtaatgccc aggccattcc tgaagcccag
51480

gcagcattca ctgtgggctt ggggacatgg tgccattcct gaagcctggg cagccatcac
51540

catgggcctg gggacgtggt gctgggcaga atgaatatgc tgcagccctg atgggctgtg
51600

ctggctgggc tttgccttct tgggtcaagg cgagcctggg cattaggcct ttgtcattat
51660

gctatacatc aggcttctgg gctgctactt gtgggatcct gcccttgttc tcaaagagtt
51720

cctccttctc actgccccat cttcttcata gtgacggcct cttcgtaccc ctggcttttc
51780

ttttccccag agttcccctg aatgttgtcc tctcatggtt ctgagttggg gaggagtgct
51840

aggtcatgga tttttgggta taacgtgtga ctttcctact cccctaagta gttctcccct
51900

tctcaagctc ctaagttctg ggaaggtctc ttttcctgcc ctcctgacta atggcagaga
51960

ggtctgaccc agactgtgcc agactggatc attcgctctg tcacctgcca ccagggcaga
52020

ggccattcct ctctggggag tctccttgtg aatttctggg gaccagctct ggctctctca
52080

agaaagagga tgctgccata tttgttctgg gaggcaggaa gatgaagggt gaggttggga
52140

ggtagcacag gatgtggctt tcaagaacct agtagagcat ctcactgtgc tgccagggcc
52200

aggctggtgc cttcttacgg aagacagtac aagggatcag gtgtggccaa caggaacttg
52260

ggagcattgg atgcctggcg tggagctgcg ctatgcttac ctggttccta tctcaggcac
52320

ctgttctgcc ttcaactgat ctcaatggaa atatgtgcag gagtgcaggc ctgttgactc
52380

tgtgtgtgtg tgtgtgtgtg tgtgtgtgtg tgtgtgtaca gtcacactca cctttgcaag
52440

aaggtgtggg agggggatga cagcagctcc acaccggaag agcagactgg tttctgtttc
52500

aaaaggcaga gtttgtggtt gttctgcagt ttctgatcta gccatttccc ctgatggaag
52560

ctcctccttc tgtcattgga agccctgtgt tctcggttag gtcttttatg gcccagttct
52620

ccctgcccct gtttctaccc gagtataact gttcattttt gttccccatc cctatttggt
52680

tttccctcct tctgcctgtg tacctgcctt tttcatttct tcagtcatta agcaagccct
52740

ggaggggccc tgcctggcct taggtatgtg agcatgtatg tacgtgtttg tatgcacgag
52800

gcatcaagtg tattagtacc acaggaagga gcttctcagt tgggcccttg gtggaagaga
52860

agggagccgg ggaggtggac atttccagac taggaatcaa gatgaaatag ggatgggaag
52920

caacagctat tcttgcccta ggtgtggctt agtgtggatg gagcagagag ggtgagaggg
52980

caaaagctgg ggaggtaaat tcctgagccc tgtcacaaag agccttcaag gccatagaag
53040

gtgtttgttg tttgttctcc ttagactggc agtgggcagc ctcgggaggg gcttgaatga
53100

ggtctagggg ggtcagaata atgttttatg aagagccaga agactgggga aggatggctg
53160

atgggtgaga accaggggtc agggaaaaca gcagaaaagg caggactctc agctggagga
53220

caggctggag gggtttgctg aggggacagg tggttccagg tagaaccaga aatgtgtcgg
53280

ggagctaggg ttagggccag aagcaatgcc cactgttcca ggtgggatag atgtggagtt
53340

ttgtgacagt tgctagaggg acaggacagg aagagagcat ctgggggagt tgggccgtta
53400

ggacattcca ggcaggttgg tgtgcagttc gggctggaag ggctcctggg gtgggggctg
53460

gccaggcaaa tgctggcact gcatctggct ccgaagttac caggccagtc tgggtttaga
53520

ggattttaag aggcttcaga gcttggggag tactcaaaga aagtcatagc tgttggctaa
53580

gccaagcttt tttcctttga cctaatctac cttaaatcgg tgatttctgg gctggaatgt
53640

ctgccctcca gccagcaggc agcccctccc tgtgctggag agtgtgtcag agcctgtgca
53700

cctttgctga gggagcagag gagtccattg gatgggcccc ctccatgtcc cacatatagt
53760

gctgggtgtt tctagctgtt acgacttttg atcttaccaa catctttctg atgctattgt
53820

gttactctta tgttactctc ttcacaggtg tagcagtgag gtcagagtgg ctgtcacttt
53880

attgagatca catagctaat gtgaggtaga gctaggattt tagttcagcc agtctcaccc
53940

agagctcgtg catgtttttt ctaccaccct gccttctaga gaaactcagt caagcccttt
54000

gctttacaca tggtaaaact gagactctga gaagttaaat aaattattca tcattgcctc
54060

aagttttagt ggctgagggc aggtcatctg ctattcaagc ccgtgtacct ctctgactat
54120

gccttgtgaa ggccacggga gcatgcaagg gccttggata gctaagataa ggaaatggtc
54180

ccaggcaaaa tggtggtctg ggtccaggat ggttgctgct cttggacagc cagtgccttg
54240

gtcaggggat ggggtgggga ttaggcccct tgctgagact tgtgtgggga tggctttagc
54300

agttaggcat ttcagggatg caccagtcct ctgggccaga gcctactttc tcccagggat
54360

cctgaggccc atcttgacct acccagccct gcctctggga ctttatgatt tgatttgtgt
54420

gtaccttgtg tcaggaagtg gtcctcatga acagccttgg gtccctattt cttcttctgg
54480

agtctagctc ttttgcaggt caaaatgtct cagtgaggga acaaagatag ggaagggaca
54540

ccccacagaa ccacctttcc cccatatagc cctctctgac atctcagccc ttacttagca
54600

aaggcctatc cctagactca acccagccct cttattacca gagtgactga gtggtcttgg
54660

ctctgcctct ggggttcctc cctgagagag agtgtaagaa tgaggaaacc aggctgccct
54720

cctttcggtg ttgactctgg gagaccccag cttgatcttg gcctctttgc taccttccta
54780

gttgacattc gtgaaattaa ggagatccgc ccagggaaga cctcacggga ctttgatcgc
54840

tatcaagagg acccagcttt ccggccggac cagtcacatt gctttgtcat tctctatgga
54900

atggaatttc gcctgaaaac gctgagcctg caaggtggga gttaaggggg tagaggaggt
54960

agaggatagt taggggaatg cctgctggct cctgcccagt gggaggtatg tgccctcggg
55020

gcagctattg ataccttgct cacagccaca tctgaggatg aagtgaacat gtggatcaag
55080

ggcttaactt ggctgatgga ggatacattg caggcaccca cacccctgca gattgagagg
55140

taagaaccac tcctgaaggg gttagggctg ggagcattag ggaccagggg gacaggaca
55200

gcagaccttt gtgtgcccag acatctccca ggcctgactg tagatggaga agtggccctc
55260

acctcaggct tctctctcca ggtggctccg gaagcagttt tactcagtgg atcggaatcg
55320

tgaggatcgg taagtactga gctgtggctg tagcccagca gggtggggat gggcatccag
55380

aaccttagcc aggcctctaa gtagctgccc ggagagccag aggacccagg ggaccttaag
55440

tggggccagg agggtgggca gaaggttctg ccacgtgtag ctttctgcac aaagtcctct
55500

ggtggccttg gtgtgagctg gtgagaggag ccagccacat gctgtgtgcc ctgccactat
55560

gggcagagac tggatgtgta gaactggctt tgggtgtcct ggaggtgagg gtggccagta
55620

gcgtgtgaag ggtgccctga aactctggct ctgagacctg gtatggcagc cagaggggcc
55680

aaggagagaa gggggagcaa agaccagata gtgtgggtgc tgcaccatgg tgaggtcctt
55740

tgacctgaaa gaaatgggaa gccatggaag ggttctgagg agaggagtga tgtgacctga
55800

cttgggttac tcaagatcac tctgcctgct gtgtggagag ctcagttagg agctgtttcc
55860

acactccagg tgaaaaatga gagggcttgg atcatgatgg taatggtagg aagtggctgg
55920

attttggcca tgttgtattt tgaagatagc gccagaaaga atgtccttaa cagattgatt
55980

ttggggtgtg agaggcattg aggataatag tatgctgttt ggcttgagca actggaaaac
56040

agagtcacca ttgactgaga tgagaagatc atgggactag cccatttgga gggaagcttg
56100

ggttcagttc gaggcatgtt aagttttctg atgcctatta gctgtccagg gagagatgtt
56160

gagtaggcag gtggttagag tgagtttagg tacatacaca gtaggggcta cagattttaa
56220

cttgggagta tgtagataac actgaaagcc acagggctgg gtgagatcac ctagggggca
56280

agcagaccaa gaaaagggca gggtccagtg gctgatccca gaccaccccc atattagaga
56340

ttggaggggt cagggacaac tagtgcggta agaggagaac ccccagagtg ttgagttctg
56400

gcagcccaag gaagaagaca gtggtgaaat ctgaccgttg acccttggat ttaacatggt
56460

gagaagagcg gtgtcaccct agcggtgagg ggctcaaggg aatgagaggg agcaagatga
56520

ctcttgcaag gagttttgct atggagggga gcaaagaaga gggcagtagc tggagagcta
56580

agcaggggca agacaggtgt tttggtggga agaagtaaca agctgttcgt atgctgatgg
56640

gaaagagacc acagagggca agatcggtga tgcaggcagt tgcaaccaga aggcctctgt
56700

ttcctcactg gaaaacaagg accctgatag cacctacctc ctgggtgact gaggatcaga
56760

ggagggggcg cctatataga gccagccctc cctccgcagt gcttgatcca ctcagtgtgg
56820

cgctcagccc cctcggggaa ttttggtgcc tcctcccagg ctcttggggc ccagaggagg
56880

tggtcccacc ctcccctccc ctccttgaca ccaccttcca ggagagaaag cccccccctt
56940

cccccttctt gtccatcctg ttgccaggtc cagcagtgtc cccagcctca catcctgggc
57000

aggacacccc tggagagcag gggcgcttgt tctctcagtt tctttcctgg agccaggccc
57060

tgtctagccc caggaggtgc ccagttgcca gtcattgtat ttggtggagc atgcacctcc
57120

tccttcctcc ctctcatggg ttagtgggtt ctgaggacca aactcccagc atcccagtgg
57180

gcagcaggta gggacctgct gccatgctgc ccccaacagg gaagttgtga gtgagccctg
57240

ccttctgtct tggataggct gactgccacc ttggcacagg cctcagcagt gccggtagga
57300

ggggaggcct ggagtgtggt ttttcttcct cccatgcctg acccacttta ctgaatctgt
57360

tcacatcttc cttgccagaa ttacaggttt tttttgtttg ttttgttttt gttttttttt
57420

tttttttttt tgctcagatg aggaactgag gtccaaagaa aggaagggac taacctcacc
57480

ccatgggttc tgatccagtc ttgccctcag gcctccaggt tcccagaata gtgttgttta
57540

cctgtccagc cccaggtggg ctcgacccac aggtcagagg tcatgagaag ctggatgaga
57600

ccactgggga tgtccctgtt ttctcagtat atcagccaag gacctgaaga acatgctgtc
57660

ccaggtcaac taccgggtcc ccaacatgcg cttcctccga gagcggctga cggtaagtgc
57720

cacccagggc tgtctgtaga tgggggcagg ggaagccaag agcccttcag ctgggggcct
57780

gactgcctga ctggcactcc tgctctatac catgcaggac ctggagcagc gcagcgggga
57840

catcacctac gggcagtttg ctcagctgta ccgcagcctc atgtacagcg cccagaagac
57900

ggtgcatgag ccacctgccc tccctcaacc cctgcccctg ctcttccacc ccacacccca
57960

gctctgcctg cctcagccct gctgccctgc ttagggcctt ttgggtgcca tttctccagt
58020

tcttctcctc ttgaggcctg cccccatctg ctgctctagc ctgccttctt actagcccat
58080

ttcccacatg gcctcccagg ggtcttgccc tgaccaggtt ctgtttcctg cagatggacc
58140

tccccttctt ggaagccagt actctgaggt ttggtttgga gtggggaggt ggggttttcc
58200

ctgggccccc ttcatctctc cactgggcga ttcttgatcc aggtgggagg cagtgggagt
58260

agggaaccat gctggaccat tctgggaagc tgtgctggct gggagttggg ttctgccttc
58320

cgtggggcac cttgttgtct gttgaccata ctagctagct taccttctct ccctgcaggg
58380

ctggggagcg gccggagctt tgccgagtgt cccttcctga gttccagcag ttccttcttg
58440

actaccaggg ggtatggctg ggctgacatt ggcccaggct ggtaggttgt gggggctgg
58500

gctcatccct gactggaggc ttctctcatc ccctgccctc cctaccccat caggagctgt
58560

gggctgttga tcgcctccag gtgcaggagt tcatgctcag cttcctccga gaccccttac
58620

gagagatcga ggagccatac ttcttcctgg atgaggtgag cccgatgttt cacccatttt
58680

ttgtcaagag aatgagtagg ggtgaccagg accccacccg ggctccagga gctagacgct
58740

ccttagggat gccaccttgt ttctacctac tgtgcacctt gcccaccccc agttgggaca
58800

gagcactctc tctcctaccc ccaacctacc atcttgggtt ggacagggca gggactcact
58860

gtctcttccc ttccacatgt ttctggacag tttgtcacct tcctgttctc caaagagaac
58920

agtgtgtgga actcgcagct ggatgcagta tgcccggaca ccatgaacaa ccctctttcc
58980

cactactgga tctcctcctc gcacaacacg tgagtgtggc tccttcaggc ccacccagct
59040

tcttccccag gagggcccat ctgaccatac ctacctgcct ctccttgcct atccaggtac
59100

ctgaccgggg accagttctc cagtgagtcc tccttggaag cctatgctcg ctgcctgcgg
59160

atgggctgtc gctgcattga gtgtgcgtgg ggtccagggc tgggggaggg aagatgggag
59220

gcctgcccgc ttgaccatgg tgatgttgct ccccagtgga ctgctgggac ggcccggatg
59280

ggatgccagt tatttaccat gggcacaccc ttaccaccaa gatcaagttc tcagatgtcc
59340

tgcacaccat caaggagcat gcctttgtgg cctcagagtg agtcggaggc tggatgaccc
59400

aggggttaac ttggctccag gtctctcgtt ctagagggac agagggcaga aagactcctc
59460

aaatgccctg tcccctctcc ctcagccttt catctttgtc cttcctcttg gcctctcctc
59520

gtcacctgct ccctgcttga gctgttgctt cccaagttac actttctgtt tcctacgtgt
59580

tgggcccact ctcttcttca tgggtccttt agactgtaga acacatgctc ttctcatctt
59640

cagaaaacat gccctggttt ctttaggcca gtgtcctgcc aactcctttg ccctcacagc
59700

ccagaagatt gtctctgttc cctgtgtccc attccttcaa ttctgtttac tgcttaaact
59760

gtggtcactg gtctccctta gctcaccagg gctctaacag ctaactttgg aggcttctcc
59820

tctctcctgg cctctttggt gtgaaacatt tttcttgcac tgctgagcag cctccataat
59880

tgggccagct cgggactgca tcagttgcca ccctttggtt tccactgctg ccacagctgt
59940

agaacccctc tctgccccac cagtggctat ggcctgcctc ttttctggga tagtttttac
60000

agacaagaag cccccaggcc cttggcttcc aacagctcac tgtgaggggc tacttagacc
60060

cagagaattg cagaatctgt ttcactgtgc ttgtccccca tcccgcaggt acccagtcat
60120

cctgtccatt gaggaccact gcagcattgc ccagcagaga aacatggccc aatacttcaa
60180

gaaggtgctg ggggacacac tcctcaccaa gcccgtggag atctctgccg acgggctccc
60240

ctcacccaac cagcttaaga ggaagatcct catcaaggtg gggtggcggg cttattgcgg
60300

aagccccaca cttctcagtg ccttgcccag gccatggctt cagctgttgg gcctaaacct
60360

gggtgaggag gtggggtgag gactggggtc tgcattgccc tgttctggtt gcccctacag
60420

cacaagaagc tggctgaggg cagtgcctac gaggaggtgc ctacatccat gatgtactct
60480

gagaacgaca tcagcaactc tatcaagaat ggcatcctct acctggagga ccctgtgaac
60540

cacgtgagga ctgggccagg ctgggggtgg taggccagtg ggtgtgagga ccctggctca
60600

caagtccctc tttggtctgt tccaggaatg gtatccccac tactttgttc tgaccagcag
60660

caagatctac tactctgagg agaccagcag tgaccagggc aacgaggatg aggaggagcc
60720

caaggaggtg aggaaccagc tcaggtctgg gggctgggcc aggtcaggcc tgggccaggg
60780

tcacagtatc tttgctgttg ccttcccctg acaggtcagc agcagcacag agctgcactc
60840

caatgagaag tggttccatg ggaagctagg ggcagggcgt gacgggcgtc acatcgctga
60900

gcgcctgctt actgagtact gcatcgagac cggagcccct gacggctcct tcctcgtgcg
60960

agagagtgag accttcgtgg gcgactacac gctctctttc tggtaacact tcccatgcag
61020

atgcgtatgt tcagtcagcg tgtgtacaca gacatcacat cacccagaga taatcagtta
61080

acatttgagc ctttgatcca ggacaataat taggctttac atggaacata atttcaccta
61140

catacacaca cacactctct gtctcacccc ccccccatac ccctcccttt tcggttcatt
61200

tgaagcccac acctttggtt catgtgactg cccacacctg agctcctcag gagattggcc
61260

tccctccttg aggctccctc cttgagttcc accctcattt ggggtggaac ttggtctttg
61320

gggccctggc ctgttttccc cagcctccct cactctgtgt cttccacagg cggaacggga
61380

aagtccagca ctgccgtatc cactcccggc aagatgctgg gacccccaag ttcttcttga
61440

cagacaacct cgtctttgac tccctctatg acctcatcac gcactaccag caggtgcccc
61500

tgcgctgtaa tgagtttgag atgcgacttt cagagcctgt cccacagacc aacgcccacg
61560

agagcaaaga gtgagggaag ggcctggggg cggacaaggc agggcagggc catgggtggt
61620

gctggccggg cctgactctg cctgttctca ggtggtacca cgcgagcctg accagagcac
61680

aggctgagca catgctaatg cgcgtccctc gtgatggggc cttcctggtg cggaagcgga
61740

atgaacccaa ctcatatgcc atctctttcc ggtgaggggt gtggcactgg gttgtggggc
61800

cttgcttggg tctgagctgc cctgaccctg tgtgactgtt ttgtccttgt gaagggctga
61860

gggcaagatc aagcattgcc gtgtccagca agagggccag acagtgatgc tagggaactc
61920

ggagttcgac agccttgttg acctcatcag ctactatgag aaacacccgc tataccgcaa
61980

gatgaagctg cgctatccca tcaacgagga ggcactggag aagattggca cagctgtgag
62040

ggggctgtgg tagacggggc atggcagggg aggcaggaga gacccagaat cttaccagtc
62100

tctggatgtg tgtaacagca agacctggtg tgttgtagaa gttcgtggga gggcccctga
62160

ctccagctgg gagccacagt gtgggtacca ggagggtgtc tgcaggaggg gacatctgag
62220

cagcatcttt aaggatgggg acaggcacat aagcagaggg ttcctcatga gtcaagatgt
62280

ggagtgagga ggttctgggg ctcacactgg gagaggtgca cacagtggaa actcatccac
62340

ctgggcttgg cctggactct gtcctagggc agatgagatg aggctaccca agagtggttg
62400

tggagcctcc gcctggtgga tggtatggag ggcagagcca caggaggtga gaccagtgag
62460

ggaaaaagtc aggacccatg gcagcacagc tggtgataag ggccctggac agaaggaagg
62520

ggtctaggac cagaagatca gttgagagac tgctgtcgtc agtagacagg gctggctggg
62580

gagagagagg cctgttgtcc actggcacct gggactcaag tgatggagag gagcggcagg
62640

aggaatgtgg ggagtgggaa ggcttttcct gactttgggc tcagcagtga accaacaagc
62700

cactgaacta aagcactgaa tatgggtagt ctgtgaaggg cccacctgca catagctcag
62760

aaatacttgg gagtttgggc atttaggacc tgagagattt tagaatcctg ggctgggcct
62820

tacatgtaag aatgtgaaga aaggaaaggg aacagtgagg ccgggcctga ggcctctgaa
62880

gccgtctcta ctgaggtcga aggatccctg tggatcaggt gcaagtttgc tgcactgggg
62940

gaaagggaag ctgctccaga aaccagtagc tgctttctac ctctgggctc tggggcatta
63000

acatatccca ttgtgtcctg tttccaggag cctgactacg gggccctgta tgagggacgc
63060

aaccctggct tctatgtaga ggcaaaccct atgccaactt tcaaggtaca gctcaggcct
63120

ctgggcatag gaagctgggg agggtcccca gctgcttggg gcttcatttc tgtgttctgg
63180

gccatctgtg gtctttgtgg agaagtggtg gttgtggttt tccgaggccc aagaggttgt
63240

gagagatgtg tggttggtga gcgaccgcag gtctcccagg gccgacccac tccctagccc
63300

ctacccctta agtcagcagt gaccctttaa actgctcctg tgggtgaccc ctgtttcctt
63360

cctgggcagc agcggggtag gagctgggag gcacactggc cactcattcc ccggcctcat
63420

ggagccctgt tacacaggtt cttttttgtt ttgccttttt ctctaaaaag gcctgggccc
63480

tatttctcac cccctcccct ccacagccag gggacctgga aggaagtttt tctttccact
63540

tcacagatga gtggtagggt cccatccagg ccccatatgc ttccaggtcc cggggaggca
63600

ctcaggccca ctgcagaccc tgccttcttg gcctgccagc cctgactcct gggagcggat
63660

tcaaggttct ccctgtggcc cagggtgggg cttaaggttc cacctgggtc ccgaggtatt
63720

tcactggcag aggccctgcc tctctgatca tatctgtcct ggagcttccc tgcagggcag
63780

taacaagaac tataactagg gcctgttgat ggcagtgtca tctcccactg gcctgacccc
63840

aggcagggaa gcccaagcac atgtgtgtgt gcacatgaag ccccaggttg gcagtggcag
63900

ggagagcctt tctgctctga ctggtgcttc tcacctctgc agtgtgcagt caaagccctc
63960

tttgactaca aggcccagag ggaggacgag ctgaccttca tcaagagcgc catcatccag
64020

aatgtggaga agcaagaggg aggctggtaa gccagtggtg tggtaggccc agagtccaag
64080

ggccccagtg gagctggggc cccaaagaca tgcatttgtg atgtgcttgt ctcctgtgtg
64140

caccagcagt gcctgcctca ccctggctta ggcatgggaa cccctaccaa aggataccct
64200

cctcatggga gttcggggtg gttgctggag gtcagcaccc tgtggctccc acaggtggcg
64260

aggggactac ggagggaaga agcagctgtg gttcccatca aactacgtgg aagagatggt
64320

caaccccgtg gccctggagc cggagaggga ggtaagacca gaaccacctg agtaacagca
64380

ttccctattc ccagattctc cttggcatgc ccccatcaca cagtcccaag cacgcacgtg
64440

catgcacaga cacctgtcac atgggctggt cgcacagccc atgtggccat gcacgtagtc
64500

tcccatatac ctgtgctaca tttggcagtc acaggtacac ccacaagatg tatttgtccc
64560

atgcacacgg atatcccctc acacacatat gcagtagcca tgctgaccat tggtgggctt
64620

tgcttcccac agcacttgga cgagaacagc cccctagggg acttgctgcg gggggtcttg
64680

gatgtgccgg cttgtcagat tggtgagctc ccatctgttt ctcttgccca ctgttcccta
64740

gggtgagatt cttctttgta tctctttcct gctcctaggg aggaagctga tggctgaacc
64800

ccaaagtctg ccctcactcc aagctctccc catgctctgg acatcccctt gacaccctgg
64860

gctccctttg tcactgtcag ctttgacccc tgcatgttta cctatgccag gcactgggga
64920

acccgtcaga atcagcagaa caaagttgat gctgctggtt ggctgacatc cccaggccta
64980

atgggttgta ccacagggat gtgacagagg cttggagatg gggtggagag ggagacacgg
65040

cctgaggaag aagcatgagt cagagctgag ttggaacact ggctccacca caaccagcag
65100

tgacttgttt gagttgtagc gtcgtcatct gtcaaatggg ccagcagagt agtccttccc
65160

ctcatggggt gtgggaagga ggaaatggga tgagatagaa ctcatttgag ccagtgcctg
65220

cggtgtggag tggggtggag ggggtgagat gtctattccc agctgttatc tgctctcgcc
65280

ctcccagcca tccgtcctga gggcaagaac aaccggctct tcgtcttctc catcagcatg
65340

gcgtcggtgg cccactggtc cctggatgtt gctgccgact cacaggagga gctgcaggac
65400

tgggtgaaaa agatccgtga agtggcccag acagcagacg ccagggtgag attctgctgg
65460

aaccttctgg ggggcagtgt gtgggcccgt caggcagctg aggcctccag ctcccagcac
65520

aggccccctc agagcagtgg ggcagccgat ggcctatgct agataggcca cagcccctgc
65580

cttagctagg gattgagaag aaacagacac ataagatgca atgtggtgtg tttaggttgt
65640

gttgagtttg agatactgat gggtcgttga agtgggtctg cttagtgggc aattggatac
65700

acaagaccag aggtaagagg agaagtcctg ggctggaagc aggagtggag tgggggctga
65760

ggcatagaag ggatgaggtg gtgtcgcata ggaagagaac ttgggatgga ttgagaagct
65820

ccatatgaga aggcatgggc gaggaaagga gcccaggaaa ggcaggtcag acagactgca
65880

ggccactggt gagggagagg gtctgctgtg ttgagagcta cagaggtaaa gacaggaacg
65940

ataagaactt gcaagtgccc tttggattca gcagcagcta atagcccttg tgagactttt
66000

gagctgcatc agcagcatgg ctggcattag atggcagtgg gcagcaggga gtctggaatg
66060

gcaaaagcag agcctttgta gggcctgtcc ttgagggaga gaacaggtga ctggggtttg
66120

agcaggctct catgcagatg gctgaaagga gcgagcagcg tgtgggagga gatggagccc
66180

aagcttagct ggtaggacca gccttctgga gaaggtggcc tcttcccctg agcctggggg
66240

aacagagaag tattctcaag ggttagggaa ttcctacttg gtggcccttt caccaagtgc
66300

atttggtaga tgggtctgta gctgctttgg agagggcacc cgcaagccaa atagagaagg
66360

gatagggtgc ttgccaggct gtcccctaga aggaaaaggt gctggcatac caggtggttg
66420

aaggactgga tctgggggtc taggctgaaa agggaagagt ctgaatgtaa gagccactga
66480

ggccgggcgt ggtggctcac gcctgtaatc ccagcacttt gggaggccga gatgggcaga
66540

tcacttgagg tcaggacttc gagaccagcc tggccaatat ggtgaaaccc tgtctctact
66600

gaaaatacaa aaattagccg ggcatgttgg ccggcacctg taatcccagc tactcgggag
66660

gctgaggcag gagaatcgct tgaacccgtg aggtggagat tgcagtgagc cgagatcacg
66720

ccactgaact ccagcctggg cgacagagca agactctgtc tcaaaaaaaa aaaaaaaaaa
66780

aaaaagccac tgaaagaaag tcctccagga gaggatgggt tggggtgctt gtaagtgggc
66840

ttctgtagag atttggcatg gagatttgcc atgaagagtg ggtggttata tttgagttgg
66900

gggcgctgag gcagtgagga tcatccttag gacacagatg gaaaccaagc caggggcctg
66960

tatccccagt gcagtcacag gtaacacaga agaatcattt ttaccccagc atgaggctgg
67020

gtaataaatg gtcagaaagt gtcatttggg agtgtggcca attccaggat ccctggaagg
67080

tcactgggaa catgggtggt gatgaggagt ctgtatcccc caccccccct aagaggttg
67140

gagggcaaat gtgtcctctg ggacagaaaa gaggaaagat gatttcagga tgaggctgag
67200

gacagagctc cctcatttac tgttgggtgt ggtgtctgga aggtacaggg gaaggtggga
67260

gaggggccca gagcacctgc agtgtgggca tgcccaaggt tggcaggggc ttccttctcc
67320

tgggcagggc tgtagcctgg ggctacaggg ccttgtgtgt gtcaccagct cactgaaggg
67380

aagataatgg aacggaggaa gaagattgcc ctggagctct ctgaacttgt cgtctactgc
67440

cggcctgttc cctttgatga agagagtaag ggccagggcc caggcggggt gtgcatgtgc
67500

ctggagggcc tggtgggtgc aaaaagagta tttaggtatc cccccaacac ttcctgggtg
67560

ggcgggctct gtaagtgttt tccctgtttg gcccagagat tggcacagaa cgtgcttgct
67620

accgggacat gtcatccttc ccggaaacca aggctgagaa atacgtgaac aaggccaaag
67680

gcaagaagtt ccttcagtac aatcgactgc agctctcccg catctacccc aagggccagc
67740

gactggattc ctccaactac gatcctttgc ccatgtggat ctgtggcagt cagcttgtgg
67800

ccctcaactt ccagacccct ggtgaggaag tcccctgtga ggagggtgag gaggggcact
67860

gtggggcagc tggactggaa tacaccataa tctgcctctt ccagacaagc ctatgcagat
67920

gaaccaggcc ctcttcatga cgggcaggca ctgtggctac gtgctgcagc caagcaccat
67980

gcgggatgag gccttcgacc cctttgacaa gagcagcctc cgcgggctgg agccatgtgc
68040

catctctatt gaggtgggtg ctgctcatct gggcttcagg gtaggaaagg ggctgcttgc
68100

cgttggagtc tgtttatgtt gagttctcca aaagtaggta ttttcaggca tcaaggtggt
68160

cagggtgggt ggggcctgag ctgagtcttt gaaggggtga agatagcatg cagtcactgt
68220

atgcatctag gacgtgcaga gccatggtgt gacttgttct gatgacttgt tttgttctga
68280

tgagatcttt tttttcccta aggggaggtc atttaaaaga tttctgtgtt aaaatagtaa
68340

tggatttaac atgaagtaga ttgcaaaact cacatggaaa ttttggctaa agctcagact
68400

tcagataaac tacaggcagc agctgctctg gacagcttag ggtccctgat gtcgtgaggg
68460

actccatggg cagtgtcccg ggggcccagc agagggcgcg ctgcctccac tccacagatg
68520

ctgactgagc ctccgcagtg gggaattgga gggagcagga aggacaatcc caggcccttc
68580

tttgtctgcc tacaggtgct gggggcccga catctgccaa agaatggccg aggcattgtg
68640

tgtccttttg tggagattga ggtggctgga gctgagtatg acagcaccaa gcagaagaca
68700

gagtttgtgg gtcagtctgt cttcccagtc atcctcctca tcctgctggg gcactgcaag
68760

cctctcccca ccagtcatcc catcctctcc cacggtgacc tgaagccttt tgtcgttgcc
68820

ttcacagtgg acaatggact caaccctgta tggccagcca agcccttcca cttccagatc
68880

agtaaccctg aatttgcctt tctgcgcttc gtggtgtatg aggaagacat gtttagtgac
68940

cagaatttcc tggctcaggc tactttccca gtaaaaggcc tgaagacagg tgaggaccat
69000

tcctggaggc agtgcccctg caatcttgct ggcagggtgg ggctgggccc cttgctctgt
69060

ccctcgtggg ctgaggccca ggcttttcct cctcctagga tacagagcag tgcctttgaa
69120

gaacaactac agtgaggacc tggagttggc ctccctgctg atcaagattg acattttccc
69180

tgccaaggta tctgcagcag gggtgggctg gcctggggta ggtgggagga gagccaggca
69240

gcagcctcta gaagtgcaga ggagtcattg accctcttgt gcacctggct tcgttgaagc
69300

aggagaatgg tgacctcagt cccttcagtg gtacgtccct gcgggagcgg ggctcagatg
69360

cctcaggcca gctgtttcat ggccgagccc gggaaggctc ctttgaatcc cgctaccagc
69420

agccgtttga ggacttccgc atctcccagg agcatctcgc agaccatttt gacagtcgag
69480

aacgaaggtg aggaagatgg aggggtgcta gagccaggaa ggcagtggct aggtcctcct
69540

tcttcagtgt ttctttctcc tgggtagaaa agttgtaata ttgtctggca ttgggctgca
69600

aggccctgcc tgccagtaag gacactcttc ccttctgtcc agggccccaa gaaggactcg
69660

ggtcaatgga gacaaccgcc tctagttgta ccccagcctc gttggagagc agcaggtgct
69720

gtgcgccttg tagaatgccg cgaactgggt tctttggaag cagccccctg tggcggcctt
69780

ccgggtctcg cagcctgaag cctggattcc agcagtgaat gctagacaga aaccaagcca
69840

ttaatgagat gttattactg ttttgggcct ccatgcccca gctctggatg aaggcaaaaa
69900

ctgtactgtg tttcgcatta agcacacaca tctggccctg acttctggag atggatcctt
69960

ccatcttgtg gggccaggac catggccgaa gccccttgga gagagaggct gcctcagcca
70020

gtggcacagg agactccaag gagctactga cattcctaag agtggaggag gaggaggagc
70080

cttgctgggc cagggaaaca aagtttacat tgtcctgtag ctttaaaacc acagctgggc
70140

agggtgagaa gctagatgcc cctgcagttt ggccctggag ccagggcaga ggaatgtagg
70200

gcctgcatgg agaagggttc tgccctgcct gaggaggagg acacagcaca agggcacatt
70260

gcccatggct gggaacatga cccagcctga aagatacagg ggatcatgtt aaaaatagca
70320

gtattatttt tcgtctcaat ggtattgtaa ctaagttatt tactcctcct gctcctcacc
70380

cctgtaggga aaccttggag aggagagtgg caggtgggct gcctgctgtg ttaagaggac
70440

ttagtttgtg atgtaaggca ctgtcaggaa tggggggcgg gccagggtgg gaagagaaga
70500

aatagcagag cctattttgg tgaggttttt tgtttttaag tcaaagaaga ctcagtatgc
70560

tttccctgag gaatgaaaaa gggattgagg agttgcctga ctcctgggtg ggtggggtac
70620

aggcagttag gtgctgaatg aagctgccat ccttgctgca gcttctaact ggtaaaaaga
70680

tccagggatg gagatgggaa ggttagaaag gcagccctca cctctgagga cagaggccgg
70740

ggtccaggcc cgtgggcgca aaggtgcctc atagcatagc cagcattcag cacacacaaa
70800

cctactgccc acatttgggc tcagggttgg ccatttgcta gttctgctgc cctcttaaga
70860

tctgactgcc aaataaatca tcctcatgtc cttttccctt tgacttgtat gctctttcgg
70920

gggctcagga aagcctgttg catgggacat gctcactaga aacagtcgcc agatgattat
70980

tctgcagtag aagcaggtag gaaatttctg gaaatttctc aggttaagca gcaagagctg
71040

taacccctcc tctgggctaa caggagttgt gggtccactc tctcctgccc accctctgag
71100

ggtgtgtctg agcagagtac atcctgcctg ggttctttgt ggccagccag ttttggtggt
71160

gagctggaaa caaccagagc ccctttccag ttccacagaa acctctcctt tcaaaaatgt
71220

tgcattcagt tcgttaacac tgccaggtgc cattctgatt gcctctagga cttggcagct
71280

gaaatctctg ggccctttca acacagttga aaggcccttc tcttcctgaa gctctgtttc
71340

catagttggc tgtgctggga tggaacaaaa atgacgccac acaaaaaatt taagatagat
71400

ccggttccgt ggatgacatg aactgatgat agccagtatc aaacagcgat agtgctcagg
71460

ttcttgtgtg actttctttt acagcttcac agtcccagtt catagagagg agggtggctt
71520

tttcccatac acaagaaggt ggtgggtggg aattcacctg ggccctcatg atccatgttt
71580

cctctctagg tttttatggc ctggagagaa aggtttccta tcagagaagg aagaggactg
71640

tgtaggccct tctgttaggg cccatccacg ttggttagga cgtccttggc gtgtttgtta
71700

gtgttgaccc ttttagtttt catcaatacg tatctctatt tgctgaacaa gtgtctttct
71760

ggaacacaac ctggggaaca gacagctctg cctttcttaa ggcagctttg tagacctgag
71820

gctcacctct cttgggctct gtaagacatt gcctttgcct cactgcaaat gttttgattg
71880

ttcttcctag tggaaaacgt gccaactctc aagcaaattt aaagatcatt ttcacttcaa
71940

gattcctcca gacctgacaa atgttgattg acccagaagg cagagtgttt gttggtgggg
72000

aagaccctta cttggggccg aatgctttgg catcaggagt tgtttgcccc atcccatgca
72060

tgcaggccgt gtccctacag tgcgacagct cagggttatg acctgtggag tctcaaccct
72120

aacagcacat tagcaccact tggggagctt ctgaaaaata ctggtacccg gggccttagc
72180

ataggtattg ttcaaaacct ctcaaggtga tttaaatgag taacaagggt tgaaaaaccc
72240

tgattttggc agcacaaacc aaaagagcag gcagggccgg gagagggaag tcagtggtac
72300

cagaaggtag atgggctccc ttgcaggctc cttgttctcc tgccatcacc agtagaacct
72360

tctggctgac agaccaggga caagtagact gggttcaaag tgacagacct ttcactttca
72420

acagctttgg ctcagcagac atgtacacat acaaagtaga gcctacaagg tcaggggcat
72480

tctgcccccg ccacaggact aaagactgcc ctgcgggaag atggcaggag cagtttctga
72540

cctcagttga gtatctgtgg ccatgagcag aaaaggcagg ggtctgcctc ctgaccaagc
72600

acatcttgaa catcacctga gagcttgaac atcactaagg acctagacac tcacttgtct
72660

ttcaacttga gcccatcact caccatgtga gttctgttga gggtgggtag aaagcaaata
72720

ggttcaggtt atcccaccag actaactcgg tgaatgaaag gatcatgcct tcttcacatt
72780

ttaattaaat ggatcaagca cagtctcaga ctgcaactcc tgtcttctct gggtacttta
72840

gtaattccca ggggacttgt gcttaggggt taatctaggc attctgttcc ctgcctggag
72900

gaaccccaca gcaacccccc tggctggccg ctcctgcgaa ttccacccat agtatcccaa
72960

gtgagtcact gcctgagtct acccactaca gcattcagct gattggcttt gccagttgca
73020

tgccagtttg gtctagtttc caaggacctg gtttttggga ttcaacacac acagctgctt
73080

ccccatgaca taagctggac tcctattctg gtgtccagaa tcactgcctg gctccactgt
73140

aggtgatttc atttcaaagg accctttttc cttctgtccc aggccttcag ggccacccca
73200

acaaaattca aatgctttgg gtcttagaag ctggggcttt agtctttttc ctccagaaag
73260

cccctgggtc cttatgcata ttccctaacc cccgacaccg ccccagcaaa tgacaggcta
73320

tgcttttcag cactctcaca ggaaacagaa aagaacttgc cagtgtcctc tcaccagatg
73380

ataccaggtt tgactgtttg cctttgtcta tttagcataa tttctaaaat atcatagttt
73440

tctcatgtag aagccagtgt ggtgatctca gcaatgcaga agacagtgag ttggaacttc
73500

tctggtgagc tgagggaaac tgccccactc atccactgga gtttaagatc cacagaagtg
73560

ggcaagaaca agtgaaatgt ttttggcttt tttcctgact cccccacaaa ataagtcagc
73620

agatgtgaaa tatatttatg tttttattta ggaataatca aaaggtttga agtaccattg
73680

aggcccattt caaattgtac aagcaatttg tccgtgttcc cctcccttat ccaaatccac
73740

agatacaaaa tctaggaaat gtgcaatttg catcttagaa atagcagcat ccccacaggg
73800

gacctcgtga ggcctggaga ttcagcccca agagggcaac cccactcctg cctcagcacg
73860

accacagtcc aaaccgaagt taagttccat ttttttgtta aaacgtttac ccagggtaaa
73920

gaattcccat tctacccaag gcttaagtga tagagtatgc tgttttctcg gtttggtctc
73980

tcacacctgg ccatggcagg agatgggacc ctcttgcaga gtttggctaa agtttcattg
74040

tgcctcaaaa caaacaaaaa cagggcagtt gcctcttact tgtttagcac caagatgatc
74100

accttttcag ccatctctcc tgacagcaga ctgctcaaca gtcactcccc agtcttattg
74160

cccagaagtc cggccacagg gaacaaggct ttgtgctgaa gaccaaaatg caaatgggtg
74220

ctagaaaacc aggaatcaag accttgtcag gaggcatcta caggcctttg gcaacctccc
74280

tgacttccac aaaggctgcc ctcagtaaaa ctcaatgaac aatggccagc aggggagaaa
74340

ggaaggtggg cgtgttttct ctttaataac aattatggca caatctgacc ctcctaacac
74400

ttctgaacaa agcaactgct cagaagcaca gcgggaaccc tctacacagg ggtgactgct
74460

tctcccctgg ccccgaccca gtctaaccga gaacaaccgg attgctgtgc tgggtccctg
74520

ctgaggagta gggactggcg ctcctcactc ctgctgacaa tctcatgtgg ggctctggcc
74580

ctgttccaag caaactgcaa gaacgagacc aaacagtatg ggcaggaacc agggatgagt
74640

gacaagatct ccctaaacaa attacaggat tcataggtac catgtagatt tttcttttaa
74700

atgtatttct actcaggttt gattcttggg aaagaccaag cacttgtagc tctctattca
74760

gtgactaaac aacctgacat ttcactttta actccagtca cattaagttt tttttttttt
74820

tttgagacgg agtttcgctc tgtcgcccag gctggagtgc agtggcacga tctcagctca
74880

ctgcaacctc cacctcccgg gttcaagtga ttctccttcc tcagcctcct gagtagctgg
74940

gattacaggc gcgcaccacc acgcccggct aatttttgta tttttagtag aaacggggtt
75000

tcaccatgcc gatcaggctg gtctcgaact cctgacctca ggtgatccgc ctcccttggc
75060

ctcccaaagt gctgggatta caggtgtgag ccactgcgcc cagccaagtc acgttaattt
75120

tgaatcatcc acttacggaa aggaacagaa gttaaaaagc attaaccaag aaaaactatt
75180

cattaacaca tacaggtgct catgtgtgtg tgtgcacgcg cacatgaaca cacgtttcaa
75240

gccagtctcc tgaaggaaga ggcactaatg gcaggtaaat gctcaggctc ctctcagtac
75300

cctggggctc accgcctatc caagtggaca gcagggaagg tgcctagagt ccccaccaga
75360

gcggctgcct tcctcccttc gctcagctgc cagagcagcc acgaaagcca aattctcagg
75420

gctgtggaag ctttggacca gcaacaacaa gctagtacct atatgttctt tatcatcctc
75480

aaagtcctgc gaacccaaag atagaacttt cagaaattga ggaagggggt gctttcccct
75540

gtccttgtcc atatggtaac cagggtctgc ctagtctcat ccccagcctc tgggttccct
75600

cccctgctct agacttcagg gcagttagaa tggatgactc tgctggatct agagctaaat
75660

agaaacttct ttgccataga aacttctctg ttgaaggact gcgtccccat tcaaagaggt
75720

cagatttgtg ggcaggcaag agctcaggaa tgaagaacca aaaaggaggc tctcaacccc
75780

tctccaaggc cagacgctct ctgtactccc attaacttca caaccaagtg cagacgctcg
75840

gtgagttcag agaccacttg gttagccctg ccccacccct cctggtgtga cctggcactg
75900

ggaacacaca gattgcagca gaaacctgtt ggttccattt gcactcagag caaaccaagc
75960

agcaccttga ttgccacttt tcctccagcc actctgtcaa aatcaaaact ggttgcctca
76020

ccagccctgg tgtggcatgg cccaaccatg tccctgccgc cccacttaaa tggccagaga
76080

aaactacatg gtgggtagat gaggttgaag ccttgcttgg ggaagggact tgagcacagg
76140

agaaaggctt gagctccaca actgcccatt tgctcagaca atggagcttt tctgagatca
76200

agtgctcccc aaactacatg ggagtagccc cccgtggtct gggcaggctg gctgagagag
76260

gtctgtgctc tcaggtaaaa ggagagaagg aagctgggtc aagagaccct gaagaggagg
76320

gtccgtgtct ggagtgaggg gaactgagcc ctgctggcta cctgacctgg gtgggctgct
76380

ggctttgccc tggaagactt gactatgtgg gccttcatgt gtctagagga tttttcttct
76440

ccagcatata actaccttga gaatggatgt gatatgtgaa aatttaaatt taatacaaaa
76500

gcacatctgc aaatggtgag tcaaactggt gaaatctccc aggtaaactc tgagcaagaa
76560

aaaagagaag atgactttag gaaaatccta aacgaggttt ggttttaaaa ttttcttctt
76620

ggctggctct cctctcctgc aagttgcctt tatccacttg gggtgtgata tctgaactga
76680

aaaagctgca ccaatctgag gatattggtc caaaaagaga caagagacca cgaggctcaa
76740

gcagagagca tccacacagc atccaaccag aaaggccagt gtccttcctc catgaaaggc
76800

agccagggac agatgtccca gagccaggag agagagggag agaacacagg acctgcccct
76860

aggccactga ctgagcagga taaaacccac aagggcacat ctcaggctac ggaatagaga
76920

gatttcctgg tgggacggcc tcccaaggct agctggagat gagaccacta gtacatgtgt
76980

gagcccgcaa ggcactaccc aatgttctgt ccaacttcct ccttgtgcta ctagagagca
77040

cagtgtggag tccttgtgtc aaggtgcttt ggtccacctg aacagccacc tgtcactcag
77100

ccaagagaaa ggagctttaa gttgtacaat cactaccaaa ccccaaggcc ctggcaagcc
77160

atccaactag ggcgttcttt ttcaggaacc caagattctc aaaaatgaca ctcctcccct
77220

cccaaaataa aaaagctcct ttatagtcaa aaacgaaaac aagtaatcaa aatatacaaa
77280

gcatatgtat caaatttaaa tttcctttat cagaatgatc atctggtttc acctttgaga
77340

actcgatcta caaccccatg taaaaaacaa atgggtacaa gacatcagca acaaaaaaac
77400

actaaaagat gattttacta tctctgacga gtccaactgc atacagagaa aagggataag
77460

attagccact ttttataggc tttttcttca ggagagacta acctcaccca actctcaact
77520

ctgcagccca caaatgcctt ctactgaagg caacaacaca atttaaccct taggcagtgt
77580

gcaaaaccac agtgagctgt cctgtgacag ggctacatac ctggctgctg ccatcccaca
77640

tcccccactg ccactgggct agtgtgggtg tgtctctgtg cacgactggc tggattcagg
77700

gttttgcccc caaacccttg agtacctcct acaatgtccc tttaagcacc tgcgtgatga
77760

ctgcctccct catcatcgct tttgatcatc tttcaaggat aaggtccaga gcatactcac
77820

tcactctcat ccaacagcag aggaagaagg atctgtccag actgctgggc tccttacatg
77880

ggcattcttg ccttttgtag tgtagccctg gctgttgggc agggctaaca tctataagaa
77940

aaacatacca gggcaggcag cactgtactt ttccacctga aagatgagct agtgcccatg
78000

gctgggtcac tgtaggtcct gcatgaactt gtccaccaac tgtgttttgc tgtttgggtt
78060

gtaaattaac cgtgaataca agtaaccaag aaaatcctct ccacagacca actcttcagc
78120

cagtgcatgc agggctctgg aggtgatgcc agcaggatct ggaggtgagg cactgggtgg
78180

tttctcagac acccggagat ggcttcagcc tgggccgccc cagctgccta gtgcactggt
78240

gtccctccaa cacaggctac tcccccaaca cccatgtgcc accaagtttc tcaactgcac
78300

acgccagatg cgcgttttac gagaatcaaa tgagtattta tactgtatat gtgtgtgtgt
78360

atatatatat atataaatta atctgcacgt atataaacac taaaaggaga aattccaagt
78420

gtctgttctc tgaaggaaag caatcactcg ccattcttgg caattcctca aaggttttg
78480

agaccaaaca tctttactcc ccaaattaag aatacaaaaa taggctgcaa aacttgccaa
78540

gtactcacaa gtccctgttt gagacaaata agatgtggtc cctattggga cagggagggc
78600

agaagatgac actttagaag aacatgattt tggtttcaaa agcaagacct ctcctggaag
78660

gcagaagggg ccaccgccat gcttgtcctt ggagccacat gagcacggtg ccctgccagg
78720

tccagttccc tgggtagggg agaaggacgg tccctaaaac accaccagtg cttcttgccc
78780

aaagcccttg ccctctctct ggggtcttct agcactgtca ctctgcccag cccaggaggt
78840

gtctgtgccc acgaagcctg gtacttgtgg cttggcactc tgggaagcag gtggtttcta
78900

agaaatgagc ggagctacag gtggacaggt ctttagttcg cccctcttta ccagctactc
78960

tcagagcaac cggtcttggg gaaattgagg atttaccatt tagaacagct ctgtaagcaa
79020

gttttctaat tttcaaaggc accatttcct gtaattttct caattcaaaa aaggacatga
79080

gggttaggct aaagtagagt tcttgtttcc aagtttgggg acataaatat aaaactttaa
79140

aagcatcctc acaaggacca gtgatgccca gcacccttgg gcatgcatcc agcacaccgg
79200

ccagaccctg gggtgggagc cacgggcact ctgggtgttc tgccagggat ctctgcatgc
79260

aagtctttgt gctaaagacc accctacctc tgataattcc aagaagtcaa caaatccctc
79320

cagccttcac ccttactaat gggccagtgt gttattcctt cccaaaccat aggaggctct
79380

gaaatgagtg tggagaaagg gctctgaaaa ccgcagtggt caaagcttca ggctgtggct
79440

atagatttcc acttacgtcc ctaccccatg tcttttttcc atctttctct cactccaaac
79500

atgtctagat taaaaaaaaa atgcaggcac ataggtcagc acattaggct taacaacaaa
79560

tgtttcttta gttgatctga taactgaaaa gttatggtcc atgattcaaa ctcccatgaa
79620

aaagcagaat tacggtaaca actaaagaga atggtaaagc atccaacgct aatcacattg
79680

ccagtccatt ttttgaactg agggttacag caggttctgc actgagtagc ccacaaggca
79740

aagcaagcct gacatgaaac gtagccggtc atcacttaat gcagcagaga tctcttcaaa
79800

ggtactgctt ccttgaggaa cacaaagggg gcacaaaggg gttccagacg tagctttgtg
79860

cctataactt ccctgcctga ctgtggaagg tgaggggcac ctgtgaccca gcaatcccca
79920

gagaacagac acaggtcatt tttagagatg acgtaactga caatgcactg cttggctaac
79980

caaagtttct acgtaatgac agtgttgata atctgatgtt ttatttaaca tatagaggtg
80040

gatgtatatg ttaaaagctt gattctgtgt ctatgaatat gactatgaac tctgaactat
80100

tttatccatt ggaaggtaaa ggaaaggtcc tacattgtgg gaggaagaac tgatgagaac
80160

cccatcttgc ttgctgcttg cttggtggtg ggcaggccga gggtagcggc aggctctggg
80220

ctgtctgcga ggattctgga agctctccca ggtgcccagc agccgggcat gggaacggca
80280

tgtggcagca gagagtcggg tttggctctt ccacgtggca ggcggtttcc cagactggcc
80340

agtccttcac attaatcaga tcaaattcag tctgtttctg agaagaaaac acatgcctgt
80400

cactctacgg cagctgccac cacctgcccc ccaggcagcc tggtccttgc tataccaggg
80460

tggcatcact ggctttgagg acctcttaat tccatgagca atgaatggcc ttctgccacc
80520

cactccccca ccctccagcc tgagggaatg tgggttaagg acaggatgct ggctcaacct
80580

tgtaaggcca tcagactctc tgagagaccc tgctaaaccc taggcctagc agcaggagca
80640

gtggtttggc ctctccaggc ccactaaatc cccctagctc cccaggcttc cgccaccacc
80700

gtctctggag tcctgtccta aaatgcttca tcctgccctc tccttccagg ctctccagaa
80760

catactgttc caatataata gcagctgggt ctagttctga tatgtattct actgatgtct
80820

tcacttcatt tgtaacttgc aaccctaaca tcaaagtcaa agtaaggctg atatcagaga
80880

ggttcatcat ctgtgattca taaagaaatg gtccttaatt gcagctggct gagaaagcca
80940

tgagaaacca ggtacagata aacagattgg cccttcctgt acccacagtt acctgtctca
81000

gacagaagag aacaatgcca atggcaatgg ccaccccctg ccattcctgc tctgtcccac
81060

aaaggacagc aacatgtcac tttgtgttgg atttagtagc tgaggggagc caatgcttgt
81120

cattcaaatc cactcatcac atttccatgt tgactttaag aaaaaaacca tggtaaaata
81180

taaataacat aaaattcacc attacgacaa ttttaagtgt acaattcagt agcattaagt
81240

acatttacaa tgttataaaa ccatcactct atttccagca atttttcatc atcctaaaca
81300

gaaattatat atccattaaa caacaactcc ccatgtcctc caccccttag gccctggtaa
81360

cctctatttt accttctatc gctatgcctt tgcctatttt agatacctca tgtaagttga
81420

atggtacaat atttgtcttt ttgtgtcttg acatctcact tagcaaaaat gttttcaagg
81480

tccatccatg ttgtaggata tatcagaatt ttattccttt tttggctgaa taatatctta
81540

ctgtatgtac acacacattt tctttattta tctggtggta agtacttgtt tccgcctttt
81600

ggctactgtg aataatgcca ctaagaacac tgatgtacat gcatatgtga gtccctgttt
81660

tcagtccttg gggcgtatac ctaggagtga aattgctgga tcacatggta atattctata
81720

ttcaccttct tgaagaacca caaagtcgtt tcccaaggag ctgtaccatt ttatattccc
81780

accagcaatg cacaagggat ccagtttctc cacatcctcc caacacgttg ttttctctgg
81840

gtgtgtgttt gtaacagcca tcttaagtgt gaagtggtat cacgctgtgg ttttgatttg
81900

catttcccta atgccaaagg ttcgtctttt catgtactta ttggccattt gtatattttc
81960

ctatgagaaa tgtctaaatc atttgcccgt ttttgaattg gttttgttgt tcagttttag
82020

gagttctata catattctga atataaatct cttatcagat gtatgatttt cagctctttt
82080

ctcccattct gtgggttctt tcattctttt aacagtgttc tttgaaacac aaaagtttta
82140

gattttgacg taatccaaat tattattttc ttttgttgcc tatgctttga gtgtcataca
82200

taagaaatca ttgcctggcc agatggggtg gcttgcacct gtaatctcag cactttggga
82260

ggttggggag gaatgcttga agtcaggagt tcaagaccag cctgggcaac atagcaagat
82320

cctgtctaca cacacaccca cacacaaaat agtaataata aaattagcca ggtgtgcaca
82380

cttgtaatcc tagctactca ggaggctaag gtgggatgac tgcttgagcc caggagtttg
82440

aaactgcagt aggtttgatt gtaccactgc actccagcaa cagagcaaga acctgtctca
82500

aaaaaaaaaa aaaaaaaaaa aaagtaataa ttgcctaatc aaaaatctgg aaggattcta
82560

ttttcttcta agtattttat agctttagct ttttttagtt ctttgatcta ttttaagtaa
82620

atttctgtat atagtacaag gtaagagtat aatttcatac ttttgcatgt atatatccag
82680

tttcccagca ccttttggtg aaaagactgt cttttcccat tgaatggtct ttgcacccct
82740

gtcaaaaatc aattgaccat tacgtgggga tttctgggct ttttatttta ttccattggt
82800

tttgtatgtc tctccttatg ccagtaccac actgttttga ttactgtagc tttgtagtac
82860

aatttgaaat caagtgagtg tgactccttc aactttgttt tttctcagtt tgtctatttg
82920

gggtcccctg agattctata taaactttag gatggattat tcttttataa ttgatagttt
82980

acatatttat ggggtacatg tgaatatttt ttacatgcac agaatgtgta atgatcaagt
83040

cagcatattt agggtatcca tcaccttagt atttattatt tctgtgtgtt ggtaatattt
83100

caagtcctct attctagcta ctttgaaata tacaatatgt tgttgctaac catagtcaca
83160

ctagtctgct atttaacatt agactttgtt tcttctatct actataagtc tatacccatt
83220

taccaacctc ttttcatttc ccccttctac tctcacaccc ttcccaacct gtgctatcta
83280

tcattctatt ctctatctcc atgaaatcaa gatttttagc tgccacatat gagtaagaac
83340

gtgtatttgt ctttctgtgc ctgggatatt ccaatgaaca taatgacctc cagttctatt
83400

attttttgtc tttttaataa tagccattct aactgggata aaatgacatc tcattgtggt
83460

tttgatttgt atttcccttt tgatttgtga tgttgagtgt tctttaatat acctattggc
83520

catgtgtatg tcttcttttg tgaattgtct attcatgtct tttgtccccc cacccacccc
83580

cacctttttt tttttttttt ggacacagga tctcgctctg taacccaggc tctggaatgc
83640

agtggcacaa ccacggctca ctgcagtctc aacctcctgg gctcaactga tccccccact
83700

tcagcctcct gagtagctgg gactacaggt atgtgccact acacccgact aatttttgta
83760

gagatggggt ttcaccatgt tgcccaggct ggtctcaaac tcctgggctc aagtgatcca
83820

cctacctcgg cctcccaaag tgttgggatt ataggtgtta gcctctgtgc cccacctttt
83880

gcccactttt taatatgatt ttttgttgaa ttccttatat attctagata ttagtccttt
83940

gttggatgaa tagttttgca aatattttct cccactcaac atattgtctc tgtgctgatt
84000

atttcctttg ctatgcagaa gatttttaat atagttccct ttgtctattt ttgttttagt
84060

tgtgtttttg aggtcttagc cataaaatcg ttgctcagac caatgtcctg aagtgttctg
84120

cactgttttc ttctagtagt ttcataattc aaggtcttat atttaaatct ttaatctgtc
84180

ttaacttttg tatatggtga gagatacagg tccagtttta ttcttttgct tatggatatc
84240

taattttccc agcatcattt attgaagagg gtgtcctttc ctcattgcat gttcttggca
84300

cctttgttga aaatcagctg gctgtaaata tgtggcttta tttctgggtt ctattttctt
84360

ttcctttggt ctgtgggtct atttttatac caataccatg ttgttttggt tactatagcc
84420

ttgtgatata ttttgaagtc aggtaatgtg atgcctacag ctttgttctt tttgcttagg
84480

attgctttga ctattttagc tctttcgtgg ttctgtacaa attttagggt ttttttctac
84540

tcctgtgaaa aaatcacatt ggtattttga tagcaattgc attgaatctg tagattgctt
84600

tggacagtgt ggccatttta atattaattt ttccaatcca taagcttggg atgtctttcc
84660

atttgtgtcc tcttcagttt ctttcatcag tgttttacag ttttccttgc agagatcttt
84720

cacttccttg attaaattta ttcgtaagta tcttttttta tttttggtag ctattgtaaa
84780

tcagattacc ttcttgattt ctttttcagc tattttatta ttggtgtata gaaactctga
84840

tttttgtacg ttaactttat atcctgccac tgtgctgaat ttgcttacca gctttaagag
84900

ttttggtgga gcatttttgg tttttctaag tatcagatca tgtcatctgc aaaaagggac
84960

aatttttctc ttttccaatt tggatgcctt ttctttctct tgcctgattt ctctggctag
85020

aacttccagt actatgctga ataggagcgg tgaaagtggg catccttgtc atgttccagt
85080

tttcagaaga aggattttca gcttttcccc attcagtatg atgctagctg tgggtttatt
85140

tatggccttt actatcttct ttctatgcag ttttctgagt gttttttttt tatcatgaag
85200

caatgttgaa ttttatcaaa tgctttttct gtttattgag atgaccacac gatttttgct
85260

cttcattctg ttgatgtgat gtattgcatt aactgaatgg tgtatgttaa accatccttg
85320

cattataatt ttttgagaca gagtttcgct cttgtagccc aggcggaagc acaggggtgc
85380

aatcttgtag cccaggtgga agcacagggg tgcaatctcg gctcactgca acctctgctt
85440

cctgggttca agcaattctc ctgcctcagc ctcctgagta gctaggatta caggcatgtg
85500

ccaccatgcc tggctaattt tgtattttta gagatagggt ttcaccatgt tggccaggct
85560

ggtctcaagc tcctgacctc aggtgatctg cccaccttga cctcccaaag tgctggaatt
85620

acaggcatga gccaccatgc ccagccaagg attattgata tgtgaggttt tgttattgtt
85680

atactgttgt tttctagttg ttttatatat ttttttcctt tttcttttgt ttggcattgt
85740

actctggtag ttttctgtag tggtagcatt tgagtctttt cctcatctgt gtgtttgctt
85800

tgccagtgag ttttatactt ttgtgtgttt tacatagtgt taaatgtcat gttttcactt
85860

ccatgtttag gactcttgtg agcatttctt gtagggccag tctagtggtg atcaattctc
85920

tcagcttttg cctgtcttgg aaatacttta tttctccttc atttatgaag gataattttg
85980

ctagacatag tatccttggc ttactttttt tccccttcag ccctgtcaac atattatctc
86040

attttctcct gatctgtaag gtttctgctg aaatccactg ttagtctgat ggggcttcct
86100

ttatagttgc ttacacacat ttctcttgct gtccttagaa ttatctgact ttagagagtc
86160

tgactataat gtgccataga gaataccttt ttgcactgta tccatttaga ggctatcagg
86220

gcctcctata tctagatgtc taaatctctt gtgagatttg ggaagttttc acctattaat
86280

attatttcat taaataggtt ttctcactct ttcattttct cttaaccctc tgggatccca
86340

ataatttaga tatgtagttg ctttattgtg tcccatatgt cacaaaggct ttgctcattt
86400

tttattcttt tttatttctg agttatgtca aaagacctgt gttctagctc taagactttt
86460

ttttctgctt gatctaatat attgttgaag ctttcaattg tattctgtac tttgtttcat
86520

gaagtcttct attccagcat ttgctttttt atgacatata tctctttgat aaaattatca
86580

ttcatttccc aaattgtttt tctttttcac attctcttac atctcattga gcttccttac
86640

actcaataat ttgaattctt tttctgggat tttgtgagtt tattttttat tggaatctat
86700

tgcaggaaat ttattgtgtt ccttctgagg tgtcatattt ccttgctttt tcatgtttgt
86760

gtccttatgt tgatatctgc acatctggtg taacagttgt ttcttctaat tttttgcatt
86820

tgcatttgta agggaggact ttttcctaga cgtatctatg gtgttgtttg ggtaggacac
86880

tttggctttg attcttggta tgtgcagtag tgtagtctct gtatgatttc tttggctata
86940

aatagcatca gtggtatctg tgatttcctc agtggctgag tagttactag tggaagctat
87000

ggtaaaattt tgctaaggac tggaatgcca ggtgggccat tctccaggca ccagtggtag
87060

cagcagtgga cagagcatgc ctgtctttgg acctcagagg agcatacaat ggcaccagtg
87120

ttagtgggtt caagcaggct gattcttggg ctttcgggtg gcttactcag atgtcagtag
87180

tggcagcagt tcccaggcat gtgatcaggt tctcaggccc ctgggcagct ggcgtggcat
87240

gggagatggc agtgacagtg ttgaaatgac cctctgggtc ccaagtgata tgtgcttgtg
87300

ttggcagtgg ctgcaacagg ctgggcggcc cattctgcag gcccacaagt ggtacataca
87360

ggtagtagtg gccaagtggg taagccccaa cctcaggtac ccaagaggag tattcaagtg
87420

ccaatggtgg tggactaggc tggacaatcc cccagcccct agactgtgtg ctctggccac
87480

ggaggtggtg gtgtaaagcc aggctgggca gtctcatctt caggctccct atgatgtgtg
87540

caggtgccaa ccaaggtggg caggtgcagg gcaatcccta ggccaccttc agaatgcttg
87600

gatagggagt ggcagtgctg tactactacc ttccattgga gagcacaggg ttgctggcca
87660

gtggggaatg catgtgccac tcacacctca ctcagctcca gtggcacttg tgcctcagcc
87720

cctgctatgg tagctggcac catagttgta cctcagccac aacaacagga gcacatgcct
87780

tgtagtgctt cagccccagc tgcagtaacc cagtcactta tgcctcagct ctggggaaaa
87840

cagtctgcag ttctcttaca cctcagccct ggcactcagg gactccagga cagtgtacag
87900

tcttttgggg ggtgggcttt aaatggcatc ttgctgtagc tgttttgttc tcaggaagtg
87960

tgggggaccc agtgcgagct ccgttctgga gcagttccat tgaacaatct cctggcacct
88020

cctatgttaa gtttcaggga ctgcaagggc tgaggagctc tcccgtggct agaactgcag
88080

aatcctttca tggtggaaat gtggaccact gagggtctct cacttacctt ttccccatgc
88140

tggggggtct ctctcagttc ccagccaatc ccagctgaac aggctgcctt gcttcttttt
88200

ccttccttac tttgtttcct gtctcttttc cgtgaattcc agtgttctgt cttggataat
88260

ctattcaaag tgtgattatc tactctctat ttttgttctt aatggaagag gcgagtacaa
88320

aatgcctcta gtcagccatt ttggttgggt cttcctggat tttcttattt ctgtaaaaat
88380

gcgactatgc ttttgataga ttttacattg actctgtagt ttgctttggg tcacgtctac
88440

atcttaatat caatccatga acatgggata tctttctatt tatttaggtt atctttaatg
88500

tttttcagca acattttgtt gttttcaaga tacacttctt tggcctcctt ggttaaatgt
88560

attcctaagc actttttctt tttgatgccc ttgtaaatga taccattttt aaaatttctt
88620

tttcagattg ttcattgcaa gtgtataaaa atacgaccaa tttttttaga cagatagggt
88680

ctcactatgt tgcccaggct ggcctccttg aattcctgcc tcagcctccc aagtagtggg
88740

actacagtta tgtcactgtg cccagctaaa tacaaatgat tttttttgtt tttatatcct
88800

gcagctttgc caagttcatt tattagctct taactatttt tgtgtgtgtg tggattcaag
88860

tttttttttt gttgttgttg ttgtttttgt tttttttttt tgagaaggag tctcgctctg
88920

tcaccaggct ggagtgcagt ggcatgatct cggctcactg caagctctgc ctgccaggct
88980

caagtgattc tcctgcctca gccctaaccc tgagactaaa ggcacatgcc accatgccca
89040

gctaattttg tatttttagt agagacgggg tttcatcatg ttggccaggg tggtctcgat
89100

ctcttgacct tgtgatccac ctgccttggc ctctcaaagt gctgggatta caggcgtgag
89160

ctaccacaac tggcctcaag tttctacata tatgatcatg tcatctgtga acaaagataa
89220

ttttacttct tcctttcgaa cttggatcct tttatttatt ttttttgcct aactgctctg
89280

gctagaactt ccaatgctgt tttgaataga tgtggcaaaa ctgggcatct ttattttatt
89340

cctgatctca ggggaaaagt tttcggtctt tccccattgt tgagtatgat gttaattgtg
89400

ggcttttcat atatggtctt catcatattg tcatattcct agttactgag tgtttattgt
89460

catgaaagca agttgacttg ttagatgctc tttctgcttg aattaggatg atgtgtttta
89520

ttctctttat tcttgtaatg tggtgaatta cattgatatt catgtgccag gcaatccttg
89580

cattgcaggg atagatccct cttgctcatg aggtataatc cttttagtat gctgctgaat
89640

ttggcttgct attttgttgg ggatttttgt atctatattc ataagagata ttagtctgta
89700

gatttctttt cttgtagtgc ccttgtcttg ctctggtgtc agggtaatgg tgacctcatg
89760

agttaagaag tgtttactcc tcttcaattt ttttggagga gtttgaggag aactggtgtg
89820

ttaattcttc tttcaatatt tggaagaatt caccaggggt cctagacttg tcactgttgg
89880

aatgtttctg ataacaatct tcttaccagt tattgatata ttttggtttt ttatttattt
89940

gtcagttgat tttgggagag tgcgggtttc taggaatttc atctaggtta cccaatttat
90000

tggcacataa ctgttaatag tagtctctta taatatcata ttcatgtgaa aatatcttat
90060

attcatgtga aaatgtaaca tcccctcttt cctttctgat gttagtaatt taagtcttct
90120

ctttcattct tatttaaagg tttgtcaatt ttgttttttc aagaaccaac ttttggtttc
90180

attgattctc tatatctgct ctaaccttta tttccttcct tctggtaact ttgggtttca
90240

tttgctcttt tctagttctt taaagtgtac atttagttta ctgatttgag atcttaaaaa
90300

aagttttttt aagacatggg gtcttacgct gtcacccagg ctggaatgca gtggtgcaag
90360

tatagctcac tataacctca aactcctggt aaacaatcct cccaccccag acacctgagt
90420

agctggcact acaggaacat gccaccacac ctggcttatt ttttactttt ttgtagagac
90480

aggatcccac tttgttaccc aggcaggtct caaactcctg gcctcaagtg atcctcctgc
90540

cttggcctcc caaagcactg ggattataga tgcaagccac catgctcgac tgagattttt
90600

tattttttga gaaggagttt cgctcttgtt gcccaggctg gagtgcaatg gtgcggtctc
90660

agttcactgc aacctctgcc tcctgggttc aaattattct cctgcctcag cctcccaagt
90720

agctgggatt acaggtgcct gccaccatgt ccacctaatt tttgtatttt tagtagagac
90780

agggtttcac tatgttggcc aggctggtct cgaactcctg acctcaggtg atccacccac
90840

cttagcctcc caaagtgtgg ggattacagg cataagccac tgcgcccagc gtaagttctc
90900

ttttaatgta tgcatttaca gctacagatt tacctcttag gattgctttc actatgtgtc
90960

ataagttctg gtatgctgtg ttttcatttg tctcaaggta ttttttactt tcccttatga
91020

tttctttgac ccattagttt ttaaatagtg tgttaatttc cacctatttg tgtatttcca
91080

ctttttattt ttcaatgtct agtttcattc cactgtaact ggaatgtact ctgtataatc
91140

tcagttttta aaaatgtatt aacacttgtt tcatggctta acatacggtt tatcctggag
91200

agtattccat gtgaatttga gaagactgtg tattctgctg ttgttgggtg gtattctgct
91260

tatgtctatt tggtctaatt tgcttagagt gttgttgaaa tcctctattt cctgattgat
91320

cttctgtctg gtttctatat ccattatcgg acatggaata ttaaagtctc caactatttt
91380

tgtctttctc cctttaattc tgtcatttct gtttcatatg ttttggagct ctgttagggg
91440

tatatatata tataatatat ataaataaat atataaaaat aaatatatat ttataataaa
91500

tatattttta tttcatatat aaatatatat cttataaata tatatattta tataacataa
91560

atatatattt atatatatta tatattatat ataatatata tttatataaa tatatatatt
91620

atatataata tatttatata taatatatat ttatatatta taaatatata ttatatatta
91680

taaatatata ttatatatta tatattatat aaatattata tataatatat attatatata
91740

atatatataa aaatatatat atttatatat ataaatattt tatcaatata atatccttgt
91800

ctcttgtaac ctttcttgat ttaacattta ttttgtgtgc ctttttcaaa agaggacact
91860

ggaggtgaag aaggaagctc ttgactctcc taaagctgat cccaaagtga aggctttgaa
91920

ggccaagaag gcagtgctga aaggcatcca cacagccacc aagaaaaaaa aaaaaaagat
91980

tcacacatca cccaccttcc tgtggcccaa gacattatga ctctgaagac agcctcaata
92040

tcctcagaag agcactacca ggagaaataa gcttgaccac tatgccatca tcaagttccc
92100

aataaccagt gaccactgag tctgatgtga attagataga agacaacaat acacctgtgt
92160

tcactgtgga tgttaaaagc caaaaagtac caggtcaggc tgtgaagcag ctctatgaca
92220

ctgatgtggt caaagtcaac acattgatca gggctgatgg agagaagaag gcgtatgttc
92280

aactggctcc tgattacaat gcttctgagg ttgccaacaa aactgggatc atcttggctg
92340

agtcttgctg gctaattcta aattatatat atataacatg ttatacatat attttatata
92400

taattgtata tatttaaaat acatatatat tttatatata attgtatata tttaaaatac
92460

atatatattt tatatataat tgtatatatt taaaatacat atatatttta tatataattg
92520

tatatattta aaatatatat tttatatata attgtatata ttttaaatat attttatata
92580

taattgtata tatttaaaat atatatattt tttttctcca gaaaaaacag attattttgt
92640

ctggtgataa tatagccacc tcagctgtca tatggttact attttctagg tgtatctttt
92700

tctatccttt tactttcaac ctccttgtgt ccttatatct aacatgagtc tcttaagaca
92760

gcatataaac actatttgtt taactccaat ctgccaatct ttgtttttaa tagaagagct
92820

taatcccttt acatttaatg tgattacaga tcaaggattt acttctgcca tttggctatt
92880

tgtttttaat atgtcttata tgtttttgat tcctcaattc cattactacc tccttttgta
92940

tttatttgat tgttcacaat gtactgtttt gattctgttt ttacaatgca ctaatttgtt
93000

agttttacta tttagacaga ttacatctta cattctatgc ccattaatat agatttataa
93060

ttttttaatg aatttgtctt ttaaattata aagcacaaag agaaaaagtt ataaaccaaa
93120

agtacaatag tactggctct tatatttacc tctaaagtta tcttaactag tgctcttttt
93180

tttttttttt ttttttgtga tggagtcttg ctctgcccca cccaggctgg agtacagtgg
93240

tgcgatctcg gctcactgca agctctgcct cccaggttca agcgattctc ctgcctcagc
93300

ctcccaagta gctgggatta gcaccaccag gcccagctaa gttttgtatt ttttgtagag
93360

atgggttttc accatgttgg ccaggctggt ctcaaactcc tgacctcgtg atccacccgc
93420

ctcggcctcc caaagtgctg ggattacagg tgtgagccac ggcacctggc ctattttttc
93480

ttatggcttc aagtttctgt tcagtatcct ttcatttcag catgaaggac tccctttagc
93540

atttctggta gggcatgtct actaataatg atctccttca attttatttg gaaatgtctg
93600

gatttctctt tcatttttga agaatagttt tgccatatac agaaatcttg gtttagaggt
93660

tttttttttt tctttttttc acgactttag atatgtcatc tcactgcctt tgggcttcca
93720

agatttttga tgaggaatcg gctgttaatc ttgtcaaaaa ttctgtgtga cgagttgctt
93780

ctctcttgct gctttcaaaa tcctgtcttc aggttttgac aatcctaata taacatgtct
93840

tggtgtggat ctgagttcat cctgcttgga ttttattgag ctttttggat gtgtagattc
93900

atgtctttca tcagatttgg gaaattttct atcattattt ctttaaatat tctttctgtc
93960

cctttctttt ttctgcgatt cctatatgag tatatgagta tgaatatact aatgaatata
94020

ataactatat gaatatatga gtacattagt atgcttgatg atgtctcaca gctctctcag
94080

gttttttttc ctaaatattt tttttctttc ttctcctttg gataatttca attattttct
94140

cttcaaattc actgttctct tcttttgcat gcccaaatct ttgttgaatc cctctagtgg
94200

gttttccatt tcagggatta tactttttag ctctagaatt tgtttggttc ctttttataa
94260

tttctatctc tttattgata ttctcatttt gttcatgcat ggttttcctg atttccttta
94320

gttcttcgtc catgttttcc tttagctcgc tgaacatact taagatagtt gatttaatgt
94380

ctttgggtaa taattccaat gtctggactt cctcaaggat ggtgcctgtc aatttttttt
94440

cctgtgaatg gaccatggtt ttctatttct ttttatgttt tgtaattttt tgttgagaac
94500

tggacattat gagtattaca ttgtggtaac tgagaatcta attctgtcat gcctcagaga
94560

ttgctgattt ttgcttattg agggctggag ccaactattt gtgacttttc caactatttt
94620

tgtgaagtgt gtattctttg cagtatgggg tcagtgaagt ttctgttgtg atatctctgt
94680

ggttaaccag tgacctaaga tttccttaaa tatctggctt gccaaaagga ggaataaaga
94740

aaaaaaaaaa taggtactgt ctctttaaat ccccttgtgg ctagaaagct ccctctgccc
94800

aagggcattg agacaatgat cagcctctgc actggccccc tcagtgatta aatgcagtaa
94860

tcagaaatca aaaaactctt tttttttttt ttttgcaatg gagcctcact ctgttgccca
94920

ggctggaacg cagtggtgtc atcttggctc actgcaactt ctgcctccca ggttcaagtg
94980

attctcctgc ctcagcctcc caaatagctg ggattacagg cactcaccaa catgcctggc
95040

taatttttgt atttttaata gagacagggt tttaccatgt tggccaggct ggcctcaaac
95100

tcctgacctc aagtgatcca cacctcagcc tcccaaagtg ctgggattac aggtgtgagc
95160

caccacacct ggccacaaaa acttgacttt tggaggacaa agttgttatt gcctaccctg
95220

gcaccaacag tctgcaccag gaacattttc cacagttgcc tgctgcagag cttgcggatg
95280

ctgaaacaaa aaaaaaccat caaaatttgt aagcctctcc atcaagctct tccctggatg
95340

ttgcaagtgt tcaactagac ttctgagtcc caaaacagtt gcttgagaca attactgcca
95400

gctcaactgc tttggtggag gaaaagattc ctggtgctgc tactgccatc ttccataaca
95460

tcattctcca tgtcagcttt cgtaatcaac ttgttcaaac atcctcatct ctcttgagca
95520

tgacaccctg gcctactggt atattacaag aagcacagag ctgggtataa tataaggacc
95580

aacttacaaa cttggacaaa gacttgtagg agagactacc tgctctgcat tataaaaaag
95640

ttaaactagc cataaaaacc tgttgttctg actccagggt gtgccatcag ctactttata
95700

aagcaggctg tgagctgtgg gccccaaagg aggggagtgt tttgttgttg ccgtattgaa
95760

aacatggctg gagttggagg aggctgtaag ggagccagtg gcccttgtac tcctacccta
95820

agtgctgacc agggctttgg gctccaccag gtgtttgctc tcactgtggg ctttgtggaa
95880

ctggattttg ccttagtatg ttaatatgag aagaacaggc tttctgcaaa tgctaaacag
95940

atggatgaca tggtactccc cgcccccaac ccaccaaatt atgggccaaa aaggaagtct
96000

cttttccctt aattcagtaa agaaacaata gcataactat gtttcaaact gttgtgttca
96060

aatgaattta aaatattcag agaaagaaaa agaattttgt cctttttaag acatcctaag
96120

tagaaagcaa ctggggtgga ggagctggga tttgccaggc caaaataaac aaacaacaat
96180

ggttggctat tgtatggaaa caatgcaaca ccatccagga aattctgtgg gatttctgag
96240

ggataggcca ctatggttgg ccctggggat gaggtaccaa acacaaggcc ccacactgag
96300

gcagctcatg ccaaagtcac cacggaacca tcacattgcc caacaccaca aatagtgtct
96360

cctgtacccc ctcccacatt gccaactcag ctagcaatgc tgccattttg gaacccccaa
96420

tcccttcagc ttctacaata ctccgccctc ctggctctca cctgagcttc tggctgccct
96480

ctgatggggt ctctaatgag aacacaaatg tcaaagggta aggagggaag ggagaggctg
96540

ggaactcagt gaccaggaac ctagaaaatc aacacgtaat aacatgactt gtctgtggct
96600

tcaaaactat gtcttaaata aaaataatct tctatgatac attttgcttt cgagtacaat
96660

ccagagaaac tgaggaacaa aattcaagct tttgattcca tgtatgtcaa agaaggaagg
96720

tgattttcca ttatactaaa catttcttct acatcaggca taaccagaga aacacaagac
96780

tttcatcatc agaaaaaagc acattatgta tctaaggaca attaaaggct ctctttcctt
96840

ttcatcaatt tgagctgaat ttcaactcat gtcctgttgc tgtctcctac ccagtggctg
96900

tggatctgcc cgactcagag gatgttttga cctgttctcc tattgctaag aaatggctag
96960

aaaagcttgg acccctttag ggtcaccatg ttccttcccc tgcgcaggtt tttaaaaaca
97020

catgaaacaa aaaacagctc tcaaccgttt atcatattat attcctatgg cttctgctgc
97080

tagttgtcct ctgaagcagc caggcggtta atgctgctgc caggcagcct tagagacagc
97140

agatgcccct gtgcagtaaa ttcagtgccc aaccataagt gcctcctccc cttacccaca
97200

caggatagcc atggcccctg tggactcctt accgagctga cgtccagcct ggggactcga
97260

tgtgtcaaag ggctctgagc tggcctcagg gacacggggc tcccacgact cactgttctc
97320

agacacctct gaataggtgt cacccatccc tttgtgaact gctgtgagct caccagtacc
97380

agggccctgg tcctcactgc ctgtgtctgc cacggctctg gtctcctccc ccatttctc
97440

agcaaaccac tgcttgacct gctgggagct catctgggtc ttgtcacaga ggttctgcag
97500

gtcctcttca tacagcatct tgtgtgtcat gtaatagtcc tgcaggagct cccggttgcc
97560

aggggcaatg accagtagcc ctggtgggaa gttgcctcgc ttatagtctt cgtaccattt
97620

gagttggccg ttcttcagtg cgtacctgct atctccaaac cagcgcacca cctctggccg
97680

tggcagaccc gtctgggcca tgatggagtc atagtcctgg ttgcttggcc actgtgtctg
97740

gacaaagagc tgccgcagca agtgccgctg ctgggcagtc tttttgcagc tcactttgcc
97800

tgggagctgc tctgccagtt tgtttgactc atcatcctca gggtcacagg cacccagccc
97860

tggaatctcg ttcctgccat tggcttcagt gaccctcagg ttcttcaggt tgattttaat
97920

ggggctgact ttgcgctctg ccaagatatg gctgctgggc atttccagag agccattttc
97980

accagagacc cttagctcac tggccaaatc ctcttctcca ccctcatcct cagcagcctc
98040

ctcttcctcc tgagaggcat tctcctcagc cttcttggtc tcctcagcat tcactttttt
98100

ccgtctctct gaaaaccagc tatcaatttc tcgtcgggtc attttggttt cacttctcag
98160

gcggtccagt tcctcatcaa gaggaagagg gttttgtgca aaactgctct ccagggctct
98220

gagctgctca ggggctctct ccttgtattt ggttggtgtg aagtcaggag tctggtgcca
98280

agattgtcgt ttggcagaag ggtgggttgc tagtgtggct gttgtcggaa tgcaggttac
98340

ctcagggacc ttggacgatg gggagaagga cacctctggc acagagtcaa tgatgatgga
98400

actgtgatct ccaggtatca tcgctctgga gcccttcaag ttccggcagt ggtatctacg
98460

atcactgaac catttccgca cctctctggt actgaggccc gtcacttttg tgagatgttc
98520

aacttcgctc tgccctggga actggttccg acagaagctc cctttcagag ctgacagctg
98580

ttcatgagat ttcttatttt tgtagatgct agcatcaagg aaggcttggg aggttatgct
98640

ggggcaggcc gtgaggagcg actgggccgc attgaccacc ttcacagctg acgttgtatt
98700

tgaacacaca gtgttaatgg gtgccacacc tggctgcttg gggacggatg tcaccgtgag
98760

ggggagaggg gaacttgttg cttgcaaccc attggccatc aatggctgag tgaccagaag
98820

tccccctcct gtaccctctg gctgccccac aacgtgacct ggaagagcgg cctggatgag
98880

atgctggaca ttgccagcac tggcgacgag tggggtattt agaaccgtaa ttgtgggctg
98940

aggcacagac tggatgactg tattgaacat ctttttccgg gcatcctcaa tctcctcagg
99000

ggaccagctg atcccctgct tcagcctttg ggctgtgaac cagatcttga gctgttcttc
99060

tggatacttg gtcaccacag tcaaatagca gagctcggct ttggtggggt aggggaactt
99120

gtggaaggag ttcttcagga agctgttaga gtccatggct gcattgtacg ttggaatgct
99180

gctcaggggg atcatcactt tgggaagggc cttggccgtg ggcagtggct ggtggacatg
99240

gtgttgggca tgcactgggg gctgctgctg gagggagagg aactgtgcta tgccagctgg
99300

caaaactggc actgttccta tcaggggccc gttggcggca tgggggtttt ttgcagagct
99360

ggcagatgcc tggctgactg gaactgcccc attgatgaag gaatggtccc cctctctcac
99420

ctccatttct ccagtcgaca gctttggtaa ggcctcaccc acaggctggc tagggacatt
99480

ctccttgagt gtatgaattt ttttggcttc agctttgcct ttcattatct tcatgattgg
99540

agttttggta atgatgattt ctgcctgtcc atcagcccct tcagcactgg gctcacccgc
99600

taggtcagga gtgctggtgc tctcagggat gctctgctcc acaaccacat gattgtctgg
99660

cttggccacg ttccacacaa agctggcttc cccggagtga catgtggcat tgtgcaagga
99720

aagcccctca ggggtttttg ccagaaaact gcacccactg catacaaagg ttgggtcttt
99780

attaaagtct gtgtgctctg agttcatatg tcccacaaat tgggtcatgt catgggatct
99840

gaaatcgcag tatttacagg aatataaata gccatctaaa gtgctccgat gcccattggc
99900

cagtgtagag ccatcagtac tgctggggtt ctgtgctgcc tcactgctgg cagcagatgc
99960

ttctgggggc agatcctgct ggggtccttc aggcaaggtc tcagcgggct gggcctccat
100020

gctggcatct tgcaacacca cagtcttcac tgggatcatg catggtgtgg tggatttcct
100080

cttgctggcc atggtgacaa tcactgggtg atcagcagtt gagagcttgt cccataaggg
100140

gcctaacaat acaagttcca gcttctcgat gagggccaaa gaaacagttt ctaaatgcag
100200

ctttttcagt tgtttgcaga aagcaggttt tccctattca atctaaggaa agggagaaaa
100260

aaatgattaa gttctcttaa gtgctttctg tatatttctc ggatacccag acacattcca
100320

ctgcttgcct tctaccatct aggtcttttc acagtaagtt taatcagcaa cttggatatg
100380

aagtctaagt taggaacccc ctaatgacaa aaactggacc ctgaagaatg acctggggat
100440

ggtccaatac caactgatag acttaagcaa cgtatttttg tttcttcccc accaccccct
100500

acatatccct gcattaagta attcagatta aaaaacaaaa cgccctcttt ttggacctcc
100560

aaattaattc tgaccatgtc agagtgagaa caaaagatcc tagtgtaatt cttgtttaca
100620

ttttggacca acacgcggtt ttcttttcat agccttcccg catatgagaa caagatgacg
100680

cctctgtctg tcccaagcca ggcccaggga ccaagttagc tgcagtgtgt gaacggaagg
100740

ggtggggtgg gcgggggtgg ctaggaagac cggctgctgt aagagccagg cctctgtagc
100800

ctttccttgc ctgccgcact tattccaggc cacagaaaag cgatcacctt acagttactg
100860

gctgttatgt atcccggacc actgttactg agatgaaacc aaaggtcaga gggtcaggga
100920

atccacagaa ccagtttgtg gcccagcaag ctatacattt tctgtgtacg accactcaaa
100980

gggtggtctc accttgagat ctgagaacag ttggactgcc tccccaagtg ggtccctgac
101040

ccctgagtag cctaactggg aggcacctcc cagtaggggc cgactgacac ctcatacagc
101100

tgggtgcccc tctgagatga agcttccaga gggaggatca gacagcaaca tctgccgttc
101160

tgcagtattt gctgttctgc agcctccatt ggtgataccc aggcaaacag ggtctggagt
101220

ggacctccag aaaactccaa cagacctgca tctgagggtc ctgactgtta gaaggaaaac
101280

taacaaacag aaaggacatc cacaccaaaa ccccatctgg acgccaccat catcaaagac
101340

caaaggtaga taaaaccaca aagatgggga gaaaccagag cagaaaagct gaaaattcta
101400

aaaatcagag tgcctcttct cctccaaagg aacgcagctc ctcacctgca atggaataaa
101460

gctggatgga gaatggcttt gatgagttga gagaagaggg cttcagacga tcagtaataa
101520

caaacttctc tgagctaaag gaggatgctc gaagccatca caaagaagct aaaaaccttg
101580

aaaaaagatt agatgaatag ctaactagaa tgaacagtgt agagaagacc ttaaatgacc
101640

tgatagagct gaaaaccatg gcatgagaac tacatgacgc atgcacaagc ttcagtagcc
101700

gatttgatca agtggaagaa agagtatcag tgattgaaga tcaaattaat gaaatgaagc
101760

gagaagttta gagagaaaag agaaaaaaga aacgaacaaa gcctccaaga aatatgggac
101820

tatgtgaaaa gaacaaatct acgtctgatt ggtgtacctg aaaatgacag ggagaatgga
101880

accaagttgg aaaacactct tcaggatatt atccaggaga acttccccaa cctagcaagg
101940

caggccaaca ttaaaattca gaaatacaga gaacaacata aagatactcc tcgagaagag
102000

caactccaaa acacattaat tgtcagattc accaaagttg aaatgaagga aaaaatgtta
102060

agggcagcca gagagaaagg tcgggttacc cacaaaggga agcccatcag aataacagcg
102120

gatctctcgg cagaaactct acaagccaga agagaagtgg gggccgatat tcaacattct
102180

taaagaaaag aactttcaat ccagaatttc atatccagcc aaactaagct tcataagtga
102240

aggagaaata aaatccttta cagacaaaca aatgctgaga gattttgtca ccaccaggcc
102300

tgccttacaa gagctcctga aggaagcact aaacatggaa aggaacaact ggtaccagcc
102360

actgcaaaaa acatgccaaa ttgtaaagac catcaatgct agaaagaaac tgcatcaact
102420

aacgagcaaa ataaccagct aacatcataa tgacaggatc aaattcacac ataacaatat
102480

taaccttaaa tgtaaatggg ctaaatgcac caagcagacc taactgacat ctacagaact
102540

ctccacccca aatgaacaga atatacattt gtctcagcac cacagcacac ttactccaaa
102600

attgaccaca tagttggaag taaagcactc ctcagcaaat gtaaaagaac agaaattata
102660

ataaactgtc tctcagacca cagtgcaatc aaactagaac tcaggattaa gaaactcact
102720

caaaaccgct caactacatg gaaactgaac atcctgctcc tgaatgacta ctgggtacat
102780

aacgaaatga aggcacaaag aaagatgttc tttgaaacca atgagaacaa agacacaaca
102840

taccagaatc tctgggacac atttaaagca gtgtgtagag ggaaatttat agcactaaat
102900

gcccacaaga gaaagcagga aatatctaaa attgacaccc taacatcaca attaaaagaa
102960

ctagagaagc aagaacaaac acattcaaaa gctagcagaa ggcaagaaat aactaagatc
103020

agagcagaac tgaaggagat agagacacaa aaaacccttc aaaaaatcaa tgaatccagg
103080

agctggtttt ttgaaaacat caaaattgat agacagctag caagactaat aaagaagaaa
103140

agagagaaga atcaaataga cacaataaca aacaataaag gggatatcac caccgatccc
103200

atagaaatac aaactaccat tagagaatac tataaatacc tctacgcaaa taaactagaa
103260

aatctagaag aaatggataa attcctggac acatacaccc tcccaagact aaaccaggaa
103320

gaagttgaat ccctgaatac accaataaca ggttctgaaa ttaaggcaat aatcaatagc
103380

ctaccaacca aaagaagtcc aggaccagac agatccacag ctgaatctac cacaggtaca
103440

aagaggagct ggtactattc cttctgaaac tattccaatc cacagaaaaa gagggaatcc
103500

tccctaattc attttatgag gccaacatcg tcctgatacc aaagcctggc agagacacaa
103560

caaaaaaaga gaattttaga ccaatatccc tgatgaacat cgatgcaaaa atcctcaata
103620

aaatactggc aaatcgaatc cagcagcaca tcaaaaagct tatctaccac gaccaagttg
103680

gcttcatacc tgggatgcaa ggctggttca acatatgcaa atcaataaat gtaatccatc
103740

atataaacag aaccaaagac aaaaaccaca tgattatctc aatagatgca gaaaaggcct
103800

ttgacaaaat tcaacagccc ttcatgctaa aaactctcaa taaattaggt attgatggga
103860

cgtatctcaa aataataaga gctatttatg acaaacccac agccaatatc atactgaatg
103920

ggcaaaaact ggaagcattc cctttgaaaa ctggcacaag acagggatgc cctctctcac
103980

cactcctatt aacatagtgt tggaagttct ggccagggca atcaggcagg agaaagaaat
104040

aaaggttatt cgattaggaa aagagcaagt caaattgtcc ctgtttgcag atgacatgat
104100

tgtatattta gaaaacccca tcgtctcagc ctaaaatctc cttaagctga taagcaactt
104160

cagcaaagtc tcaggataca aaatcaatgt gcaaaaatca caagcattcc aatacaccaa
104220

taacagacaa acagagagcc aaatcatgag tgaactccca ttcacgattg cttcaaagag
104280

aataaaatac ctaggaatcc aacttacaag ggatgtgaag gacctcttca aggagaacta
104340

caaaccagtg ctcaacgaaa taagaggaca caaacaaatg gaagaacatt ccatgctcat
104400

ggataggaag aatcaatatt gtgaaaatgg ccatactgcc caaggtaatt tatagattca
104460

atgccatccc catcaagcta ccaatgactt tcttcacaga attggaaaaa actactttaa
104520

aggtcatatg gaacaaaaaa agagcccaca ttgccaagac tatcctaagt caaaagaaca
104580

aacttggagg catcatgcta cttgacttca aactatacta tactacaagg ctacagtaac
104640

caaaacagca tggtactggt accaaaacag agatgtagac caatggaaca gaacagagcc
104700

ctcggaaata ataccacaca tctacaacca tgtgatcttt gacaaacttg acaaaaacaa
104760

gaaatgggga aaggattccc tatttaataa atggtgctgg gaaaactggc tagccatatg
104820

cagaaagctg aaactggatc ccttccttac accttataca aaaattaatt cgagatggat
104880

taaagactta aatgttagac ctaaaaccat aaaaacccta ggagaaaacc taggcaatac
104940

cattcaggac ataggcatgg gcaaggactt catgtctaaa acaccaaaag caatggcaac
105000

aaaagacaaa attgacaaat gggatctaat taaactaaag agcttctgca aagcgaaaga
105060

aaccaccatc agagtgaaca ggcaacctac agaatgggag aaaatttttg caatctaccc
105120

atctgacaaa gggctaatat ccagaatcta caaagcactt aaacaaattt acaagaaaaa
105180

catcaaagaa ccccatcaac aagtgcacaa aggatatgaa cagacacttc tcaaaagaag
105240

acatttatgc agccaaaaga cacatgaaaa aatgctcatc atcactggcc atcagagaaa
105300

tgcaaatcaa aatcacaatg agataccatc tcacaccagt tagaatggcg accattaaaa
105360

tgtcaggaaa caacaggtgc tggagaggat gtggagaaat aggaacactt ttacactgtt
105420

ggtgggactg taaactagtt caaccattgt ggaagacagt gtggccattc gtcaaggatc
105480

tagaactaga aataccattt gacccagcca tcccattact gggcatatac ccaaaggatt
105540

ataaatcatg ctgctataaa gacacatgca cacgtatgtt tattgcggca ctattcacaa
105600

tagcaaagac ttggaaccaa cccaaatgtc catcaatgat agactggatt aagaaaatgt
105660

ggcacatata caccatggaa tactatgcag ccataaaaaa tgatgagttc atgtcctttg
105720

tagggacatg gatgaagcta gaaaccatca ttctgagcaa actatcacaa ggacagaaaa
105780

ccaaacacca catgttctca ctcataggtg ggaactgaac aatgagaata cttggacaca
105840

gggtggggaa catcacacac cacggcctgt cgtggggtgg ggagagggac agcattagga
105900

gatataccta atgtaaatga caagttaacg agtgcagcac accaacatgg cacatgtata
105960

cctatgtaac aaacctgcat gttgtgcaca tgtaccctaa aacttaaagt ataatttaaa
106020

aaagaattat aagtaatctt cacatatgct tttaagtatt tttttaaaag aaaggaaaga
106080

agaaagaaga ggaaaccaga aacaaataat cattacacta tccagacatt acataacatc
106140

ccttacctgt gaatttagat gtattgaact tttaataaaa tagattgact tcacactttt
106200

aaaatgctaa gaaagaaaac agctaaggca gaaaactaac ctgaaaacaa aaccaatggt
106260

tttatatata aaagaccgaa attaaacagt tgataactca aaaagaaata ataataataa
106320

ttgtttcaca ggatgccatg agaaaaggga ttctgtgagc cctattacta atatagaatg
106380

ataatcatca ttattacttt tcgttttagc aaatcataat gctctaatga ctaaaacaat
106440

agtaattcta atactaaaat caagttatca gtatttggat ttgatacaaa gatgtaaaga
106500

aaattaaaat atctctattc aaattctcag taaataaaca cggctatttt atgatgaaaa
106560

taatttttaa aactttgaag gtgagggtaa acataaaccc actctaacaa cttctcaccc
106620

cttcccccac caatttgaac tggcatgcac acttttttac ttaattttta attataaaga
106680

taaaatgtaa tttaaataaa tttaatgtct ataatttggt ataccttctc atttagtcca
106740

gatattttat tcatattttt atcacaatga gcacacattt gtgctttttt caccaaacat
106800

tacgttataa acctttttgt tacacaagct tcataagttt aacatttaat agctacctaa
106860

taatgtacca caatgaattt gacttatcca cccctgctgt tgaaaagcat gccaatctca
106920

tgggtgacag tgaaacaaac atctctgggc atacaggatg tttttcttct atcaaattat
106980

ttcctgaggt taagtgcaca ggaatgggct taccatctca aagagcatca acatttctaa
107040

gctcctttgt atatatcatt ggcaaacatt ttaaatcaag tgctttatga tcccaaatca
107100

gggaaactaa aaataataca gttatggacc cagtgagcaa atcagatttt cagacaagtt
107160

atggaagaca tcaagtagaa tatctgaaag cacaaacgac aaaagaaaaa acagataaat
107220

tggatgtcat caaaattaaa aacttgcatt cttcaaaggt caccatcaag aaagtgaaaa
107280

gatagacaat ccacagaaag aaaaggtttg caaatcatct atctggtaaa gtatatccag
107340

attatataaa taacttacaa ctcaaccata gaaagataat ttttaaacgg gtaaaggatt
107400

tgaatagaca cttctccaaa gaagatatac aaatggccag taaagcacat aaaaaactgc
107460

tcaacatcat tagttattag gaaaacacaa atcaaaacca caatgagata ccacttcaca
107520

cccaccaaga tggctgtaat tacagcgaca gataacaagt atagatgagg atgcagagaa
107580

actggagccc tcacgtattg ctgatgaaaa tgtaaaatgg tatagccact ttggaaaact
107640

gtttggcaaa caaaatgtta aatatagagt tgccataaga tccagcaatt tggccgggcg
107700

cagtggctca cacctgtaat cctagcactt tgggaggcca aggcgggcgg atcaccagag
107760

gtcaggagtt caagatcagc ctgaccaaca tggagaaacc ccatctctac taaaaatgct
107820

aaattagcca ggcatgttgg tgcatgactg taatcccagc tactcaggag gctgaggcag
107880

gagaattgct tgaacctggg aggcggaggt tgcagtgagc caagatcatg ccattgtact
107940

ccagcctggg caacaagagc taaactgtgt ctcaaaaaaa aaaaaccaaa aacaaaacaa
108000

aacaaaaaaa acaaaacacc agcaatttaa ttcctaagta tatacacagt ataaataaaa
108060

acatgtccac cccaaaatat gtacacaaat gttcacagca gcattattca aataatggcc
108120

aaaaggtgga aacaacccaa acatctatca gctggtgaat gggtaagtaa aatatgatat
108180

atccatacga tgaattcttc agccatgaaa aggaatgaag ttctgatgca tgctgcaaca
108240

tggatgatcc tgatgctaag tgaaagaagt cagacacaaa tgtcacatac tgtatgattc
108300

ccatttatat gaaatatcca gaaaaggcaa atccatagag gcagatagta gattagtggt
108360

tgccagggac tgagagtagg ggagaagggg gagtggctgc taatgagtca ccaggcaatg
108420

aaaattgttc tgaaattaga tagtggggat ggctgcacaa ctctgaatat gataaaaacc
108480

tcaggaagga catcgaaacc aagaaaggta gaggacagta aattatagca ctcagttcta
108540

tagaagaggg tcagcattta catttctaag gtttcaatat aggtgacaca gttctgtgct
108600

acttcctgac tcaccttggt aatatttact gtggccctag agaagctgaa tggcagtgcc
108660

tgtgaacatc aggtttgcca gaggagaggg aagagagatg cagacctact acctggaagt
108720

agcaacaaca caaaggaaaa agagcatgag cacaccagaa ggccaggcag cagaactcag
108780

gagaagctaa ctgcagagcc ctagcaacac aacacaagca ctccaggcac agggccagaa
108840

ctgctatttg taatggggac tgggattcac tgaggaaaag gtatatagtg ttcctggtat
108900

tttacttact aaggctgaga agtggacaac atgaagatgg atttgatttt tggcaatagt
108960

cccaatgtca ttcaaaatta agtcttggcc aggcacagca gctcacgtct gtaatcccag
109020

cactttggga agccaaggta ggaagatcat ttgaggccag gagttcggga ttagcctggg
109080

caacatagaa attgtaaaaa attagtcagg aacaatggtg tgtgcctgta gtctcagcta
109140

ctcaggaggc tgaggtggga ggatcacctg agcccaagag ttcagagtta cagtaagcaa
109200

ggatcatgcc actgcactcc agccaggatg acagagcatg accttgtctc ttaaaaaaag
109260

aaaaattaag tctcaaatta gccctaaatt atcattcgaa taaatgacta agaagggtac
109320

cactgctaac catgttctgg gtagatggac cattgactat aagcaattac aaaaaagcag
109380

tagtggcagc agtggcagcc acagccagtg tttactgagt gtttcatatt gttggaagtg
109440

cttttataca catcccctca tttcactcat aataaaccca agaaaaggca ctataattat
109500

tctcatttta cagacaagaa ggacaaggct cagaaaagtg cagtgactgc ctggagcgca
109560

cacaactggg agacgacaga agctgaataa gaacccaagc agtctcgctc cagagcctgc
109620

aacttcaacc actccacaca gtgagctctc tgagaaacct ccagattgtt gcaataaggg
109680

ctacagacct cttgaagtaa tattttcctc cagcatttat taagaaaaag ttcaaacaaa
109740

tggcaaagtt gaaagagatt gacagtgaac acccatatcc ccaccacctg gatgcttact
109800

aaggtggctg tttttagcaa cattacccat ttaggtaagc ttgggtttat ttgttatgta
109860

tttttaaagg ggttaaaaat ggtgcttaaa cagcaaagtt ctaattatct agaaaatcat
109920

tcatccaaaa gacactcatg gagcatgtct gataaatgag gcactaccat gtggaaacat
109980

gctattgtct aagtcaagcc ctagtgaagt gagctgatag aaatgtaagg tgcggccaaa
110040

agagattcag aaggaaaggc caggtgatgt ttgcattttg tcatttttgt acaatccttt
110100

acatgcccca tcttgaaacc ttctgccttg taaatggatc tttaaatggg gccagagcag
110160

tggctcacac ctataatccc agcacttgga aggccgaaac gagtggatca cttgaggtca
110220

ggagtttgag accagcctgg ccaacaacat ggtgaaaccc cgtctctact aaaaacacaa
110280

taaattagcc aggtgtggtg atgtgtggct ataatcccag ctatcgggaa gctgaggcag
110340

gagaatcact tgaacccagg aggcagagat tgcagtgagc caagatcgca ccactgcact
110400

ccagcctggg acacagagta aggctccgtc tcaaaaaata aataaataca aataaaaata
110460

aaaataaata aatggaaaaa taaaaacaaa agtgcataac tttttttaaa aatctgtctt
110520

tgggaattgg taattgctca attcactaaa ttagcaaagc tatagttaat agccactata
110580

tttcagcact gcagtataca tatgtgagtt ttttagataa aaatcttaaa gtgtttatct
110640

ttaaattata gactgaatta ggaactttaa aaaattctga ggaatgaatt ggaagaacag
110700

taagaattca atgaaggaat tcaaatactt agaaagtaac tcttgacaga ctcttccaag
110760

cgttatgata gtcagcctgc agccctcttg aacatatcac ccagtattta tcattaaaaa
110820

cggaaatagg ccaggcgcgg tggctcacgc ctgtaatccc agcactttgg gaggccaagg
110880

agggtggatc acaaggtcag gagatcgaga ccatcctggc taacacggtg aaaccacgtc
110940

tctactaaaa aaaaaaaaaa aaaaaaaata caaaaaaatt agctgggcat tatggcgggc
111000

gcctgtagtc ccagccactc aggaggctga ggcaggagaa tggagtgaac ccaggaggcg
111060

gagcttgcag tgagcagaga tggcgccact gcactccagc ctgggcgaca gagcgagact
111120

gtccaaaaaa aaaaaaaaag gaaataatgg tagaagttga cagaattaca gaaacataac
111180

tatagtggga gattttaaga taatattcta aagatcaagt ggacaaagaa aaaagatgta
111240

aagaatgtga ctactatgag gttgatctga cagacatgaa tatagtcttg agtattctat
111300

agtaattgac ttcacattct acaccatgaa acacatttaa aagttactcg tgatgtataa
111360

cagaccataa ataaacctca acccattcca aaataaaaat tcataggtag cattccacta
111420

tttagtgcta gcaacaaaga cttactttga aatttatcct ttttactttg tttcaaatta
111480

tgctctaagt ggaaatttct ctctcggaaa gaaagaaaag tgaaaatcct tcctattcac
111540

ccccagagat aaaccattgt taacagtgca gtatatatga tttcaggcat gtttcctagg
111600

catagcttac tataattaat tcttttaaaa aaccaaaaat ggaattgtat ttttacatcc
111660

tattttgacc ctgagttttc gtctcacaat tatttaattt caatatcttt ccatgtcacc
111720

atctctagtt tacctcagtc ttttaaacaa cttcagcatt ctaggttatg gatgcatcat
111780

agtttattta accattctcc tgataagaga catttgcatt tctccaggtt tttaactatt
111840

acaatgactc ggtgaccatc cctgcacata tttttataca tttgagaatt tctgaaatac
111900

tgaatttctt acaagtaaaa ttgcagtagc atttaaaata ttgttagcaa aatacaaaat
111960

tgtctttcaa gtaaactgta ctgattttcg ctgttaccac caaagaactg aggatgcact
112020

cactatcact gcatattctt gctattttca gtctttgtta atttgatata taaaacacta
112080

attactagta tttggaatct acatttcttt atgagcaagg ctgaatatct tttcacatgt
112140

cattgacgat tttgtataaa attatctttt gtccattttc ctatgggatg tcaagtgttc
112200

tccatatatt aagaaaatgg gctattcttt acctgtcata gatgaaactg gggtttcatc
112260

tccatttttt ggagcccatc taccacagga ggcagtaaat gacattaaag aattactgtt
112320

aatgttgcta tgtatgataa tggtatttca gttatgttac aaacaaaagt cttaatcagt
112380

tggtgatgca cactcgaata tttatgggta aaataatttg ttatatagga tttactggga
112440

ggaaaaaccc aaccccctag aaaaaaatga gggatgggta gaaatacatg aaataaattt
112500

gaccatgaac ttataattgt tcaagttggg ttgggatata tatggctcat atatatgctc
112560

ttatgtacat ttgacatttt cataattaaa aaaaaattaa aagctcgaga catattttaa
112620

aaagaaatct tgtatcataa atgaagctta aatttatatt tttaaaaaac attctgtttt
112680

aaatgtttct ctcttccctt cctttggaaa atgctgacct tggaaacaaa aatcagatgc
112740

agccacccac cctactgctg cccaagccaa tcctaagtaa atcagttggc ttgctgggaa
112800

gtttccatga ccgaaaagaa aacagaattc acttcaattt catcatcaaa gtccacgaaa
112860

gcttgcaaag cacttccata tccttctcaa tccatcctca tagtctcctg aattggttat
112920

tattggcctt ccctcattgt agtattgagg agctatcgag caagaaaggg gaggtgatat
112980

gaccagtcac acacgacata tttttggcag agccagatat tctgattcta aattcagtgt
113040

tctcttttag actatattca ttctgaatac caactaaaca atacatccac tacctgtaac
113100

tattttgaca tttttttctt acagagggtt cgaagatttt acaacctgaa ctattctagt
113160

gttttgtttt gttctttaaa aaggcttgac aatgtagtcc tagctacttg ggaggctgag
113220

atgggaggat ctcatgagcc caggagtttg aggctgaagt gagagctatg gttccgtcat
113280

tgcattccag taatgtgggc aacagagcaa gaccctgtct cctaaaaatg aaaaaagagg
113340

ctggaccagt ggctcatacc tgcaatccca gcactttggg aggccaagga gacaggattg
113400

cctgagccca ggagtttgag aacagcctgg gcaacataag gagactccgt ctctacaaaa
113460

aatttaaaaa atagctggca tggtgacacg catgcttgta gtcacagcta attgggaagc
113520

tgaggcggat gactgcttaa gcctgggagg tcgtagctgc agtgagttgg gattatgcta
113580

ctgcacttta gcctgggcaa cagagcaaga ccctgtctct aaataaataa ataaataaat
113640

aaataaatgg taaaaaggct tgacagttat tatgatcctt agtatttata acacttttat
113700

accgtcagaa gagggtctgt attgcttact cttatgtatt aatagagaac tcttttaaca
113760

tgaggcttgg tgattagacc acggtaagtc agtaggactc tataacaaaa agaacatgtg
113820

gaaaactgtc cttcctcagg aatgtgtgtg ggcaggacct tggcagtcca gtaagtggat
113880

gtgctggttg caattgtcag atttctgctg aaaacaaaaa agttacaaag aactcaagtc
113940

taaatcctca aatgtcaaag caatgtcaag cccagagctg gcctcaattt gccctctggt
114000

tacttaaggg tcacaataat ttctgcattt agtaatcacc atcagccaag ggctcaaggc
114060

tagagctctc tacttgtttt tccccctcaa acagtgctgt tttttttttt tttttttttt
114120

tttgagacgg agtctcgctc tgtcgcctag gctggagtgc agtggcgcga tctcggctca
114180

ctgcaagctc cacctcccgg gttcacgcca ctctcctgcc tcagcctccc aagtagctgg
114240

gactacatgt gtctgccacc acgcccagct aattttttgt atttttatta gagacgggtt
114300

ttcaccgtgt tagcgagcat ggtctcgatc tcctgacctc gtgatccaca cgcctccgcc
114360

tcccaaagtg ctgggattac aagcgtcagc caccgcacct ggccttcccc ctcaactctt
114420

atttttgtct aatcaccaac agaaatagcc cactctagtc agatattatc acctctaaag
114480

taaacattaa tttgttcttt ctcaagctca gaaggtgatt gtgatataaa tacaaaggcc
114540

tcagaattaa agagacttta gagatcatat attctaacca ctcacctttg catgaatttt
114600

accttaagta ccactaccag gtggttagga tggcctatac ctgaagatct ccaaagatag
114660

ggagcttggc acttattgac actagaaaga tgtttgaaat ggttccttgt agtaagcacc
114720

aaaaacaaca aaaccttcct ataagtttta tttgctaaat ttgttttttc tctcaaatag
114780

agttgaggac ttactatgtg ctaggcactg ggtatacaag gatttttgaa aagatgctag
114840

ctagccctat aactcaagaa gctaacagtc tgttgtggtt attaagctat ccaccagggc
114900

tctgggcctt gccctccact ggatgcattg aatttccctg cctacctgaa gttaggtgaa
114960

accaatgaaa cgtgaacaaa tgtgtcacct ccaggggaaa agttttatga gaaagtatag
115020

gctttgcctc actatttatt ctctggcctg caactggtta tgttccagat ggcggctact
115080

ctatcagcct gagtcctaaa agaagaacaa tagctgacct gccatgtaca tgcagtatga
115140

acaagaaata acctttgtca ctttaagcta ctgaggtttt tggagtagct tgttactgca
115200

gcccatcctc actgatacag agtctgaaga cagacaaaca aaatcatcag caaaatgatc
115260

aacaaagtca tcaacaaaat gatcaacacc aatcagacaa taagctgagt gttgtagtgg
115320

aagcatgcac aaagttctgt ggagcatagg aaaagacttt ctacccacct ctgtcttccg
115380

gggaacttgg ggtaaggcct catagaggag gttgcttttg gattagagct cgaaggatga
115440

ataaatactg ccaagtagat aagttgagaa aagagtgttc tagacaaagg gaacacttta
115500

tacaaaaacc gtagagggag gagaaaacat ggcatgattg gagagctaga aaggattcag
115560

ttttgctatt gtacaaatct gaatgtggta gatttcctat gaagctacta cagccgaagc
115620

tttggggacc ctcacttcac ttgcacaggc accttccaaa gcctctatct aactttatat
115680

ctttataatt ttattcttag atttttttct taggagggtc ccctttacta atgttcaagt
115740

tccacaaaag ctggatctgc ttgtgatgga tcaggttgca ggaataggag gtgattctag
115800

gtatacaaac aagatatttg actttctata tgatacagtt ttttttggtt ttttttgttt
115860

tgttttgttt ttgttttgtt ttgttttgag gcagagtctt actctatcac ccaggctgga
115920

gtgcagtggt acaatcacgg ctctctgcag cctcgacctc ccagactcaa gtgatcctcc
115980

cgcttcagcc tcccaagtag ccagtattac agatgcgtgc caccatgccc agctattttt
116040

tttttgtaga gatgaggttt cgccatgttg cccaggctga tcttgaactc ctggactcaa
116100

gtgacccacc cgccttagcc tcccaaagtg ctgggattac aggcatagcc actgtgccca
116160

gccgacacat ttttcaattg gaaaaacgtg attttttgga aaaaaagaat catgttccct
116220

aaaagtcttc ttttccccta aaccaataat cctcagttcc tgtactttgt gtgactggga
116280

cagagactcc acaccactct ccactgttct tctctgaatg tgctcttatt tgtctatagc
116340

cttctcaaca gtggcatcta aatctaaatc taattctcca gatgtagtct gagcgtattt
116400

atcaccttcc ttaaacttac tcatgtcata tttaatcttt cacaaaagtt tttacccaaa
116460

cgatgccaca ttatttataa tgtgagattc ccctgaaaaa tatggatcaa tgctcttgga
116520

tttaatccac agcaccagga cttgtaccaa aatacaatac ctgcttgttt caatttaggt
116580

ttggcagaat ttgaggaaca agtttgcaga gaaaagatag tcaattcatt taacaaatgt
116640

ttactaaatg ccttttctat gccagacatg ccagaatatg gggaagacaa aaaaaaatag
116700

atagaaatct ctgccttcct ggaatttaca ttctagtttg ggggagagag ataataaata
116760

aaataagaaa atgaaattat gtgtcagagc aaaaagtgga agaaaagcat agcagtgaaa
116820

ggctgaagaa atgtggagca tggcaggggt gagactgcaa tttagatata cagtggtcag
116880

aagagaactc actgagaatg tgacactgga gccaatactc agaggtgtgg aagtgagcca
116940

tctgggtaag tgaggaagca cactccaggc acaggcatca gctggtgcaa agaccttgag
117000

gtgagaatgt gcctgacatg ttcaaagaac agcaaggagg ccagcagagc tgggcggcgg
117060

tagtagagag acagtagtat gagacaaggt caaagaggct gtggtagggg gcagggaggg
117120

gatccatgca ggtccactgc catgatctga gtctctctga aatgggcagt gccaagagag
117180

ggctttgagc agaaggttgg cgtcatccga cacgtgtcct tcagcatggc tctgctggct
117240

atgttgagaa cagactgtag gaaaacacag tggagaagta gggaaacttg tcagacatca
117300

atctaggcag gagatgacag cagcatggac caggatggta gagatggagg taagaagaag
117360

tggctggatt ctggatatgt tttgaaagta aagccgaaaa gacttcctaa aggaatggat
117420

aggaggatgt gaacattgta gccacgggaa tcaaggatga ctccaagatt tttgatctaa
117480

gcaactagaa ggatagtgtt gccatcaacc aagataggtt taaacctggg aagctttgtg
117540

aaaaaagcaa agcaaatctg tgctgctaaa actagaaggt ccttttccag ggaaaaaact
117600

ggattgaact ggtttggtca caagtgttgt aggaaattaa tgggtcagac tgctaatgag
117660

caaagaggct gtgtagtgcc aatgttccca atgaaaagac tctggtcttt gccaaaagct
117720

gctcaaatct acagagaaac tacagaaatg ggggttgggg ggaggctggg accaaatcta
117780

gggtattata agagccaagt agttagaaat tgcactctct gaggtgtgag acagcgcctt
117840

tcaaagttaa acatgttact ttaaatgtac ttgcatccta aatgttgttc caagtactaa
117900

gtaaattact ttattaaatt taaaactaaa cagtaaaaaa gaaattgttc tggatgtggt
117960

tttacttctg agattacatt tgttttgttt taattgaaat tttccagtgt gggccagata
118020

gtttacaggc tgaatagctt gcaggctggt gagctcactt gaatttctca agaacatttg
118080

ttaaaaaaaa aaaaaaaaga aagaaagaaa gaaaaattct ctctggctga ccactgtgga
118140

taagatgttc cttttacctc ttccaggctt agtctgtatt gatgattttc ttcaatggcc
118200

tcactacaaa gtccagcaag atcttacaca caagaaaaaa tgctcaattt cactcacagt
118260

aagagaaata caaattaaaa ttactctgat tatttttctt tcacttactt tggaaaggct
118320

tgaaacattg gataataata tcctgagttg gcaaggtttt gcagaaatag gcactctcat
118380

acactgcttg ctggagtgaa aattggtaag acttctatta agaagtattt aacaagaaca
118440

aaattataaa agctcattcc ctgactcacc agattctgcc tctaggaagg cagcctgctg
118500

atatactttg cacaatggta agtcatacat gtataaggta atctactatc agcatggtaa
118560

tggcaaagga ctggaaataa cctaatcact catcaacagg agataatcaa atcaattatg
118620

gcacacttat acaatggagt aacacacttg tgaaaaagaa caagggaggt gctttttatg
118680

ctgctatgga ttaaccttca agctatcttg atatatgtta agttttttaa aaagcaaagc
118740

acagaataaa aattatacgc tagaaatacc tcgagaaaaa tatgtgagaa actaataata
118800

gtgtttgccc ctggcgaggg gaagagggag atgtgtttga gggaagcttt tccctgtagc
118860

cccttttaaa ttctgaacaa tgttaatgtg ctatgtattc aaaacaaaaa gttaaaaatt
118920

gaataaatac ataagtggaa aaaatgttag catatttccc aaaatacatg ggttgttaca
118980

aataacaagt gagaaagtga gaagctaatg gcgtcagagt agaggtcctt acagagtatg
119040

taactgggtc aaataccaag tagtgctcct ttacatttca aacttctaat agctacagat
119100

gagcttttgg tgtaacttct gtaggggtgc atgtgagcac ttcacttaaa actatatatt
119160

atatattgca tttgcctctg gccataaagc catagttcaa aaataactta gagatatcca
119220

atatacaaaa caaaaaataa ataaataaaa gaacaagcaa aacaaaaaaa atacccaaaa
119280

tggaggagtg ggacaatata tgcagtccaa cttctttcct aattaaaaaa tcctgcagca
119340

atctgttatc cagtctccaa ctgagtatct cccaaatgtg ggagctaatt atcatttatg
119400

acaatttatt acaaagcccc tttcttagtt ggacctggtt cccagctttt agatttgtct
119460

tttggacata aatcaactct ttttactaca tgacgcatca actctctttt ctatatatca
119520

gcttttcaca cagttgaaga tcatttcctc ttttagtatt tacttctgca gctaagtgtc
119580

ctgaattcat tgcacattct ctctatacat tctaatttgt ctgcatttct cacaaaatgt
119640

ggcaattaga actgaacaaa gcattcaaga tgtagcccaa ccagaagaga attaaatgga
119700

agctgtgttt tcatcatcct attaccatga ctaatacaac caaaaactga gctagataca
119760

agttctattc tccccctcac agaggaggac tttgaggtga agtgacccag tgggacaagg
119820

aaacactgct agaattaagg aggattagga ttctttcatc acaaggccat ttggatacac
119880

gttttcagac actcatttat aattccaaca gaggacttct gagctatctt cccctttgcc
119940

tctttgaaat ctaccttctt aagaaaggaa tgttagactt tctctgaaca tcctatctaa
120000

atgtctttgg gggctccccc aaatccaagc tgaattattc tctatcacag catcctaatt
120060

gtttctttca taacatttat caaaatttgt ctggatcttc atctgattct tttcctgctt
120120

attgtctttc actttggcta ggctgtaagc tctgtaagta aggtcagggc ctatatccac
120180

tgtttccgta gcatatagca caatgtctgg cacataataa tcgttttcag tgagtgaatg
120240

ggtgaataaa cggtagctga taatggagtt aagactctaa catcagacag agccctaaca
120300

ctgtggactg tgtattcctg ggcaaatgac ttaagtcctc acaatccatt tcctcattgg
120360

taaaatgagg agctattaat acctacctca tagacctgac atgacattcg gtgaattgat
120420

aggaagtaca caaaatgatt tagcatgctg tctgatatac aggaaacact caataaatca
120480

tagttactgc agctactgct actgttctaa ttaccaccac atcccactca gaggctggcc
120540

actgaaggat gacaattaac tgtaacagct agaggtacat cagctgtttt acccagccta
120600

cctttctttc tttttaaaaa attgtgataa aaaacacata acataaaatt cattgtctta
120660

accatttgtt tttttctttt gagatggagt ctcactcttg tcgctcaggt tggagtacag
120720

tggcgtgatc ttgactcact gcaatctccg cctcccaggt tcaagcgatt ctcctacctc
120780

agcctcctga gtagctggga ttataggcac ctaccaccac gcagggctaa tttttgtact
120840

tttagtagag acagggtttc gccatgttgg ccaggctggt ctcgaactcc tgacctcagg
120900

tgatcagcct gccttggcct cccaaagtgc tgggattaca ggcgtgaacc accgcaccca
120960

gactgtctta accattttta agtgtacagt aaggtagtgt taagcacatt cacattgttg
121020

accaacagat gcccagaact ttttaatctt gcaaaagatg caaatactat acccatcaaa
121080

taactattcc ccttactctc ttcccccagc ccctggcaac taccatacta ctttctaaga
121140

gtttgactac tttagttacc tcatataaat gaagttatat ttcaagatgt agcccaacta
121200

gaagagaatg aaaagcaagc tgttttcatc atcctactac cactactaat actaccagaa
121260

acctagctag ctaaaaaaag tacttatctt gggctgggcg cggtggctca cacctgtaat
121320

cccagcactt tgggaggccg aggcgggtgg atcatgaggt caggagatcg aaaccatttt
121380

ggctaacatg gtgaaacccc atctctacta aaaatacaaa aaattagccg ggcgtgctgg
121440

cgaatggcat gaacgcggga ggtggaggtt gcagtaaact gagatcgcgc cactgaactc
121500

cagcctgggt gacagggcaa tactctattt caaaaacaaa agaatttcct ttcattttaa
121560

ggctaagtaa tattccatga tatgtatata tcatatttta tttatccatt catccactga
121620

tggacattta ggttgcttcc ccctcttggt tattgtgaat aatgctgcag tgaatatggg
121680

tgtgcaaata tctctttggg atcttatttt ttattctttt gggtatacac tttttcttgt
121740

ttaagctctc cacaatccta tgatgctggc agagcagcag aaatcacagg agtcatcaat
121800

tcgtctttac cttttttgat cctgtcatca aattatcttc ctctcaagct cactccctag
121860

tcctttctga taacctcctg gttcctctat tttcaagtga attcaacagg tcaaatttaa
121920

aatttatttt acttgtccaa aagggaagga agctattata cctttcctat ccctttctgt
121980

tggaagaact ctaggttcct catccagaga agggtactgc cccaggtctg agcagaatga
122040

agctgcaggt actcagggtc taagccccct ggaacaatag agggctatgg ctctacctta
122100

gtcggcccag gacagtttct aaaatgtttc ctttatgagt actgttttga atgctgttaa
122160

tatttcatca ggaagtatca gtttcccttg acaggtaaga ttaagtccta ttcactgggt
122220

tgatcagcct tatcattaca gccaggatat ttctctgcaa atccccttcc aatcttatgc
122280

tgcccaacta atgggaccaa ctggatttgc tcaataaagt tctgtatttt cccagagctc
122340

ttcattttca cattattgtc ctggggtggg gggcagcaga acagagatgt tgacagccaa
122400

atggaagatg cattgggagg cttctgaaga tacttgttct gtctttgaga aaccttagag
122460

catcaaggtg aagcaaaagg aaaggagagg aatagtgctc agtacctgag ccttctctaa
122520

tctaaaacgc aataaagtca atcataaatc ctgaaaatga gagtggctgt gatggtgcaa
122580

aatataaaac tccaacaact atttatcagg aatgaagtac atatatctcc ctattcgcat
122640

ctcagccttc ctcctgcaat atgtgagtga gtgtgcatga acccacactc accagtgata
122700

cactcaggag agtactgggg aagagttaac ttccgctgca agatagttag catatttatt
122760

tcctttccat gagacttcag gagcctaaat cagaagtttc taagagcttc tcttgatgtc
122820

atatattctg agaatggata catctaagaa ctgatgtaat gtgtctgcca ttatgaattt
122880

ggtaaaagtt ctatttatct gtttttaaac aaaaggactg ctttaatgtt acatattggt
122940

ttgttattac atattggctg gttatttgtt attggttgtt tctatgacac acttggacaa
123000

gaaaatttct agtccaagta tatcatagtg gtattacttg agacaccact taaatctcat
123060

aaaaatagaa aagatcccct aactggcctc tcccatcttc cccctccccc atgccagact
123120

gcctctgtcc cacttattca acttccttgc tcatcaatct ttggtgcatt gcaggcccca
123180

ttccttcctc tccttctgcc caaaccactt gcaccaaggt catcagtcaa gtggctcttt
123240

cctactcctt caagtctcaa accactcagg acccaaagga agttaagacc cacacctcta
123300

taacctctcc aaagggagca aactcatgcc caggactttg caatctcctc tacccaagag
123360

atttccaaat cacccttgcc agccctgacc tatctaccaa gttccaaatc cattacttcc
123420

aactgccatt gatccctgca ggttttaaaa aactacatgc ataaatttct ggctccgttg
123480

accctggcac tcacccttcc aaaaaacgaa acaagcaaat aaaaaatata gcctaggtct
123540

acttcctaaa tcttcatttc acctctcacc atttgtctta gtccgttcgg ggtgctataa
123600

caaaaacatc atagactgga tggcttataa acaacagaaa tgtatttctc acagttcttg
123660

aggccaggaa gtccaagatg aaggtgctgg catattcagt gtctggtgag ggcctacttc
123720

ctcatagaca gacaacttcc cactgtcctc acatggcaga agggaccagg gagtgtctgg
123780

ggtctctttt ataaggccac tagttccatt catgagagct ctatcccagt gacctaatta
123840

ccctccaaat gcctcacctc ctaagacaat cacattaggg attaaaattt caacacgtga
123900

attttaggag atacattcag tccactgcac catacatcca gatttccaag catacttatt
123960

tttttcctag tttcctttat tcacatctct cttttctctt ttcccacatg aaatgttaat
124020

ggtctcggaa gcacttaaca tcttttcttg gtagagggac ttttagaaat tgtggtaaaa
124080

tacacataac ataaaattta ccatcttaat catttttagg tggagagttc agtagcatta
124140

agtattaaca cagtcacatt gttgtacaac catcatcacc atctgcctcc agaactcttt
124200

gccttataaa actgaaacct tacacccatt aaacaataac tcccatttcc tgcccacccc
124260

tgactcctga caactaccat tctgctttct gtctctgtag atttggctac tctaggtatt
124320

ttacataagt ggactcatat agtatttgcc tttttataac tgatcaattt aacttagcat
124380

aatatcctca agtttcatcc atattatatg tcaggattta cttcctttta aaggctgaat
124440

aatatccccc tgtatgtata tacaacattt tatccatcta tcaatgaaca cttgggttgc
124500

ttctaccttt tgcctattat gaataatgtt gctatgaaca tggacataca atctctttga
124560

gtccctacta tcaactcttc tgggtaacta cccagaagtg gaattgctta atcacatggt
124620

gattctattt ttaatttctt gaggaattgc catactgttt tccataacag ctgcaccatt
124680

ttacattccc accaacagtg cactgagttc ccaagagttc tccacattca tggcaatatc
124740

tgttattttc tttttttttc ttcagagttg ccatcctaat gggtgtgagg ctgtatctca
124800

ctgtggtttt gatttgtatt gcctaattat attaacgttg agtattttta atgtgcttat
124860

tcatttgtgt atcttctttg aataaatgtc tattcaagtc ctttgaccat tttaatcagg
124920

ttgtctgggg ttttttgctg tttttgttgg gttgtagaaa ttctctatat gttctagata
124980

ttaccccttt attagatata tggtttgcaa gtgtcatgaa gcttttctcc tctgtcttct
125040

tctaagcatt ttatagtttt agatcttttg tttaggtctt cgattcattt tgagttaact
125100

tttatatata tggtgtaagg taaggatcca actctattat tttccattag atagccagtt
125160

ttctcagcac catttgttga aaagactgtc ctattcccat tgaaaggtct tggcatcctt
125220

gtaaaaaatc atttgacctt atgtgtgagg atttgtttct ggtctctcca ttctactcca
125280

ttggtctata tgccttcatt aacaattttt aatattaaat agatgttgaa ataatatttt
125340

gcatatattg ggttaaatat attcttagat tttttttttt tactgtttca atgtggccca
125400

taggacactt atcacgtatt ggctctcatt gtatttctgt tggacagcac tgcctagatg
125460

cttcagactg gagccaccgg gcagtgtgga gagcaacagc taacgtcatg ggattagatg
125520

atcatctgaa agctcctctc ctcttgtgtt tatcattata gtacttaggg agcaggatat
125580

cactgtggct gagaattgag gcttctgagt caggcaagtc caggtttcaa gacctgccct
125640

gtccaacaca gcagccacta gccataggta ctcatgtatg tactgagtat ctgaaatgtg
125700

gctaatgtga attgagatgt actgtaaatg taaaatacag accagatttc aaagactcag
125760

taccaaaaaa agtaaaatat ctagttaata acttttatat caattacatg ttaaaatatt
125820

agtatttctg ataaattggg ttaaataaaa tgtattatta aaattaattt gacttgtgtt
125880

tcttagtagg ctatttttta gatcaagttc acagcaaaac tgaccagaag gtatagatat
125940

ttcccatata cccactgccc cacacatgca tagcctactc cattatcaac attccccacc
126000

agaatggtac atttgttaca actgatcaat ctacactgac acatcattat cacccaaagt
126060

ccacagttta cattagagtt cactcttggt gtacattcta tgggtttgga caaatttata
126120

atgatatgta tctacaatat tataatatca tacaaaatag ttctactgct ctaaaaatct
126180

tctgtgctct gcctgttcat ccctccctcc tccctaactc ctggcaacca ctgatcattt
126240

actgtctcta tagttttgcc tttttcagaa tgtcatatag ttggaatcat acaggtatgt
126300

agcctttttg gattggcttc tttcacttag taatatgcat ttaatctccc cccattgctt
126360

tttatttttt gacttgttaa ttttgatttt ttaaaaatgt ggctcctaga acattttaaa
126420

tgacatatgc agctcacctt atgtttctgc tgaacatttc tcagcttggc atgggcagtt
126480

tttctcagca tggcatcttc ctgtctttcc agtcactcaa gtccaagcct ggttgtactt
126540

ggtttgctgc aacctaaaca caccagcctc cttgccttca cacatactag agttccttca
126600

ttagagtttt ctttaacccc ctccctcacc tgaagactaa ttcatgctca gacccaattc
126660

aaatacgaca tctttagtga agcctttcta aacttccact gtacctaaat catttttat
126720

agcacattct accttataat ttttttgtat gtggctgact ttctctccag attgtgaaat
126780

ctttgaatta ggccccatgt tttgttcatc tttgggtcct caatccttag tacagagcct
126840

gacacatggt aggcaatcag agatgttaag ttaataaatg aataaaatgc aggaaaatta
126900

acatatcctc taataagaga taacatattc acttagaatc aaatagctta gaaaagaacc
126960

tcaaatgcca taaacaaggg tgtaaaattc tatcagccaa cagctaaaat tattcacaag
127020

ctaaaatttg tattcagtca aagcaacaca ttaatcaata atatttataa agtatatcac
127080

agtgaaaaga tggtaaagca cctcctaatg gttaatgata gtgattggcc tggagatgtg
127140

gaaactagct atggaaacta gatggttaaa acaaccacat tccgtaccat tagacgctga
127200

cagcccagac cccaccaaaa tagaggcagt gaaacagcct ttgaagttgt tttcaaagga
127260

agccaccaag atgattaaag ggttggaaaa tgagatttat gggtaatggc cacagaaacg
127320

aagacaattt tgcctaggca agagaaaagg ccgaagggtg acttaataac catcttcaat
127380

cattgttatt acacagagaa tgatgaccag atggtctcca ttttcaccag agaaagaata
127440

ttgaaaatgg acttacgctg cagcataaaa aaaaaaaaaa ggtctagttt taaacaaaga
127500

actcaatggt taagaactag tatacagcaa agcagacctg gggagtgtgt ggtgtagcca
127560

tgtctgaact catctgttat gctgggaaag cagcaagtgg gtagggtgtg ttcaggtctt
127620

caacagtcct ttgttgaggg caaattctca actggctagt cctcaaatga atcacgtagc
127680

atacaaccac aggagtggaa aagaaagaga tatgaaaatt gaaacacagc accctctggt
127740

gtacatagct gtcttctaat ctaggaaaat tcttcttttt tttttgagac ggagtctcgc
127800

tgtcgcccag gctggagtgc agtggcgcaa tctcggctca ttgcaagctc cgcctcccgg
127860

gttcacgcca ttctcctgcc tcagcctccc gcgggggact acaggcgccc accacctcgc
127920

ccggctaatt ttttgtattt ttagtagaga cggggtttca ccgtgttagc caggatagtc
127980

tcgatctcct gacctcgtga tccccccgcc tcggcctccc aaagtgctgg gattacaggc
128040

atgagccacc acgcccggct ggaaaattct tgatatgtat cagtattttt caaaatgtaa
128100

gctccaaacc atgacagagt caggagaaac ttacacaagg ctggtcttag ggttcgttta
128160

atcgctacat ccatctttat caagcacctg cgatgcattt ctccctgtta ccaggaggta
128220

ccaggaagcc tgttacacaa gcaacctcaa cttctgccta tagcaaactt ggattcttcc
128280

caatcacctt ttgaatgctg aattcagact ctgatacaac tagctggcac atgaccatgc
128340

aatgctgaga catgataaca tctgccagat aatgtgaatt ttgccctgtg ttcaggagta
128400

cattacatat aaaagttaag caacacctaa atgggggtgt tcccttagac attctccctg
128460

ctatgttgtg gtgtatacac ccttctttac tgatctctgt ctgccaaatc ccagccggcc
128520

actttttaca tcctcctagg ctttatgctg caaaaccttc ctaatatcct tttaagtaca
128580

aaagacattc aatgagctca aaaagtttga gacccaatca agaatctttt gcagccctct
128640

cagtatcttt agccatcttg ttgccagatg ggaacttcat tcacatgaca cacatacttc
128700

cttcatgtga ctcaccattg ctaagcaact ggtatgtcct aggtgaataa aacatatgcc
128760

ctgtgtctga gattcacagt gcagtagggg aacatggata gatacgggtg taaatagatc
128820

agctgaagca tgagttgaag ctcaaggctc tacatgccaa gaacagagga aaaaggcaat
128880

ggagagaccg gacaagaact atcaggacat cagaacagaa cttacatcct gtgagagcag
128940

gacttatgct aaaaacaagt cttaaaggat gaaaagaagt tgtccaggca gacaagacag
129000

gaatatgtat catcctaggc ataaagtccc aagacatgtc accttttgaa gaatggagcc
129060

atgtggcaca ctgtcctcca gtcccaaagg tctccttgaa gagacatgaa aatcccacct
129120

gaagaactca cccttgcaga ttgtacatag cacagaccta aatggcaaca aacaagcaaa
129180

caaaaaacgc agtacagact aggcactgat ctgatggaag cgagacaggc tggagtagca
129240

aagggtctga ctaagaagct taggaggaat tcctctgata cccttcttgg tgccccctcc
129300

ccagcaacat ctaacagcct cactcgggcc caacttccct gttgcactgg tggcactgca
129360

ttgcaattgt gggtacaaga ttcctccccc acaaaactgt gggctgaggc aagagttcta
129420

cctttttatt actacgcctc tgacactcag cacatatgaa gcacaagcaa aatttgatga
129480

ctaagctaag taattagttc atatgtgtga ttagggtttg aaagggcaga accttggacc
129540

tatgatccca aatacctttg tctatctagt tcacctggtt ttgttcctcc tcagaagcca
129600

tctatcctcc ttaccttggt tcatgttttc ctatctctcc attttataca cataaaccaa
129660

ctcctaccct caaatcaggg aaaagggaca gatattcctg tcctcaagtt aagagtgacc
129720

tatgtcaaag tgaaaaacaa ctaagccaaa aaggtaacag ggaactggct tcttgagcac
129780

tcagaagaat ttgagattac tgcctcgcga gggaaaaagg gaaacccaac ccgattacag
129840

tgtaaaaata ggtcactgct tgaatacagc cctttggtta ctggctgcaa gccccttttt
129900

gcttttgact ccagtgtcac aagaaagtgg cagcatagtc ttagaataag ggctccacag
129960

ccagctggct gctcggccag gccttcctgc tggcattcta gaagcagcat gcagggccag
130020

gcacctactg gggagctcag taagcctttg tcactgctga catgaaatac aagagttggt
130080

aagtttcttt ggttttgttt tgaacaaact ggcagatagt tcaaggttgt ccttgtaagt
130140

ctctctcact tgcattcagc tatacattgt ttagtaggaa atgagtatag ttcagctagt
130200

tcacttatcc ccatgccaaa ccagcaggca gcctcttttc catgagagac agttataatt
130260

ttgaattatt gtaatgctgt acttgcagca aactcggtgt gtgtgtgtgt atgtgcacac
130320

atggctggga gtggggggta gtgccaaaat gttggttcta tggcaaacaa caacaacaaa
130380

acaaacaaac aaaaaaaccg gctgtgtata gctaaaaaac atggccaaga aaaggtaga
130440

atagaaaaca tgttttatta tcctgatact aaagcctggc agagacacaa taaaaaaaga
130500

gaattttaga ccaatatccc tgatgaacat cgatgcaaaa atcctcaata aaatactggc
130560

aaaccgaatc cagcagcaca tcaaaaagct tatctaccat gaccaagttg gcttcatacc
130620

taggatgcaa ggctggttca acatatgcaa atcaataaat gtaatccatc atataaacag
130680

aaccaaagac aaaaaccaca tgattatctc aatagatgca gaaaaggcct ttgacaaaat
130740

tcaacagccc ttaatgctaa aaactctcaa taaattaggt attgatggga cgtatctcaa
130800

aatagtaaga gctatctatg acaaacccac agccaatatc atactgaatg gcaaaaactg
130860

gaagcattcc ctttgaaaac tggcacaaga caggaatgcc ctctctcacc actcctattc
130920

aacatagtgt tggaagttct ggccagggca atcaggcagg agaaggaaat aaagggtatt
130980

caattaggaa aagaggaagt caaattgtcc ctgtttgcag atgacatgat tgtatatcta
131040

gaaaacccca tcatctcagc ccaaaatctc cttaagctga taagcaactt cagcaaagtc
131100

tcagcataca aaatcaatgt gcaaaaatca caagcattcc gatacaccaa taacagacaa
131160

acagagagcc aaatcatgag tgaactccca ttcacaattg cttcaaagag aataaaatac
131220

ctaggaatcc aactaacaag ggatgtgaag gacctcttca aggagaacta cagaccactg
131280

ctcaatgaaa taaaagagga tacaaacaaa tggaagaaca ttccatgctc atgggtagga
131340

agaatcaata tcgtgataat ggccatactg tccaaggtaa tttacagatt caatgccaac
131400

cccatcaagc taccaatgac tttcttcaca gaattggaaa aaactacttt aaaggtcata
131460

tggaaccaaa aaagagcccg cattgccaag tcaatctaag ccaaaagaac aaagctggag
131520

gcatcatgct acctgacttc aaactatact acaaggctac agtaaccaaa acagcatggt
131580

actggtacca aaacagagat gtagaccaat ggaacagaac agagccctca gaaataatgc
131640

cacatatcta caaccatctg atctttgaca aacctgacaa aaacaagaaa tgggggaagg
131700

attccctatt taataaacgg tgctgggaaa acgggctagc catatgtaga aagctgaaac
131760

tggatccctt ccttacacct tatacaaaaa ttaattcaag atggattaaa gacttaaatg
131820

ttagacgtaa aaccataaaa accctagaag aaaacctagg caataccatt caggacatag
131880

gcatgggcaa ggacttcatg tctaaaacac caaaagcaat ggcaacaaaa gccaaaattg
131940

acaaatggga tctaattgaa gagcttctac aaagcaaaag aaactaccat cagagtgaac
132000

aggcaaccta cagaatggga gaaaattttt gcaatctacc catctgacaa agggctaata
132060

tccagaatcc acaaagaact caaacaaatt tacaagaaaa aaacaacccc atcaacaagt
132120

gggcaaagga tatgaacaga cacttctcaa aagaagacat ttatgcagcc aaaagacaca
132180

tgaaaaaatg ctcatcatca ctggctatca gagcaatgca aatcaaaacc acaatgagat
132240

accatctcac accagttaga atggcaatca ttaaaaagtc aggaaacaac aggtgctaga
132300

gaggatctgg aaaaatagga acacttttac actgttgggg ggactgtaaa ctagttcaac
132360

cattgtggaa gtcagtgtgg cgattcctca gggatctaga actagaaatg ccatttgacc
132420

cagccatccc attactgggt atatacccaa aggattataa aacatgctgc tataaagaca
132480

catgcacatg tatgtttatt gcggcactat tcacaataac aaagacttgg aaccaaccca
132540

aatgtccaac aaagatagac tggattaaga aaatgtggca catatacacc atggaatact
132600

atgcagccat aaaaaatgat gagttcatgt cctttgtaag gacatagatg aagctggaaa
132660

ccatcattct cagcaaacta tcgcaaggac aaaaaaccaa acaccgcatg ttctcactca
132720

taggtgggaa ttgaacaatg agaacacaca gacacaggaa ggggaacatc actcaccagg
132780

gcctgttgtg gggtaggagg agtggggagg gatagcatta ggagatatac ttaatgttaa
132840

atgaagagct aatgggtgca gcacaccaac atggcacatg tatatgtaac aaacctgcac
132900

gttgtgcaca tgtaccctaa aacttaaagt ataattaaaa aaataaaata aaataaagaa
132960

aatatgtttt attaacaaac agcatatttt ataatggcgg tgatcaatat ttattaaaca
133020

caaggttcta gagggctctt ctggtaggat aattctaaga aagaggttcc agatgtcaac
133080

ttacatattc ccttataaaa cacagccctg gcttacacag tatctggtgt acatgtctct
133140

tctctccaat gaactataag atctttgagg taagggttca gtgctgcacc actcagctac
133200

actaatggca atgtgcacac agtgagcact cattaaatac atcagttaca gttcacttgc
133260

agttgcttgc ttactaccaa aaaccggggt caatcattat aagcattccc atccaacacc
133320

caatttgttg catggggaat gaacttcagt ttgggtccct tgctattaaa gcactcaata
133380

cccattattt gtttgcattt atgtcgaact catagatgca ggatctgaaa agttaaatga
133440

agcttaacat aggcatccaa ataaaattaa ttagaattta tatctcaact atttctaaca
133500

gagtttgagt aacaagactc aacagaagga aaacagggta ataacagaga ctaagaggga
133560

aaaaaaggaa ggcagcaggg agtgaaggct ggagaagaga agaaggaagg aaaagaagga
133620

gggacagagg gtggaaacat ctatctaagc aagagcttct tcctcaagtc aaaaatttct
133680

gcaaaaaaca acatcttttt ggacaaattt acatgatgct tctaacatct gctcattgtg
133740

aaaaatgcag gataaaaacc tacaaattca gtatttttag tatttttatc taaatttata
133800

tttcatcaaa acaagataaa tttgaaaaat attaatacat atataggcaa gacatttcct
133860

tactttgcgg aaatatgctc acataaataa tccattcttt gccaaaatag agcccttcca
133920

acacctgttc cccaatctcc aaggcagggt cttggtccat gggttagaat cactaaaaca
133980

cacaaaatac aaggaaatgt atctattaag caccatttgg attctaagat gcaagtaaga
134040

aacctaattt gaaagggctt acataataaa ggtggtttgc tggcacatgc aaatggaaag
134100

ttcagcggct tcaggttgag tttgagtcag ttgctctatg atgaaaacaa gggcctattt
134160

tctctctctt tttttttttt tttttttttt ttttgggaga cggagtcttg ctctgtcgcc
134220

caggctggag tgcagtggca tgatctcggc tcactgcaag ctccgcctcc tgggttcacg
134280

ccattctcct gcctcagcct cccgagtagc tggggctata ggcgcccacc accatgcccc
134340

gctaattttt tttttttgta ttttttagta gaaacggggt ttcaccatgt tagccaggat
134400

gctctcggtc tcctgacctc gtgatccgcc cgcctcggcc tcccaaagtg ctgggattac
134460

aggcgtgagc caccgtgcct ggccaataag ggcctatttt ctttctttct acttcgtcaa
134520

ccacatccta ggggtagctt ccctcgtgac agcaaggtaa ctccagcagc cccaagcttc
134580

atgtcccaaa catccagaag gacacagaga gagggccgtc ttggggaggt ctatcagaag
134640

actgaagagc cttcttccca aagtcctcag caagcatgct ggtggcctgc acaggtcaca
134700

tgcccagcca tgaacaaacc tcttggcatg actgccactg ggttaatcac tgtgcaacag
134760

gtgtggggtt tacctgactg ggcttaaaca atcaagatcc atccctggag ctgggggtgg
134820

gaccaatcct tctcctacca gaagacagaa acaaacattc cctgtttcct atggaaggga
134880

ctggggaggg agggagaaga gacattgtga gaaggggaac aattagaatg ccctccaccc
134940

ttcacctggc actgaatttg accctgtgat tcccagtatc aagggaaaat atggaaatgt
135000

attttaagtg acactatttt tatcgtttaa caaagacaaa tgtttattct tatacagaga
135060

taaattcttt tgaagggaaa cccaaggaga agaatgtctt ggaactgtgt ttaagagaaa
135120

ataggtaaag caggaggctt agtttggaaa gatgcagtca cactcaatag gccattcctt
135180

tttttttttt gagacagtct cttgctctgt tgcccaggct ggagggcagt ggcgccatct
135240

tggctcactg caacctctgc ctcccaggtt taagcgattc tcctgcctca gccttccaag
135300

tagctgggac tgcatgtgca tgccaccatg ccaggctaat ttttgtattt ttttagtaga
135360

gatggggggt tttgccatgt tggccaggct ggtcaacagg ccattcctac atggatcctc
135420

cagaaggcag agcatgggac agtaaatcat atcataatcc agtgaagtta ctttggcaga
135480

gggctgagat gatatagctc agatgctttc ccttcaaatc atcgaactta ctgcaaagag
135540

tagtaactca catatcttag aacccctttt ttaatgttta tttttgtagg tacatggtag
135600

gtatatatat ttatgggtta catgagatat tttggtacag gcatgtgacg cataataatc
135660

acatcagggt aaatgaggaa ttcatcgcct caagtattta tcctttgaat tatactgtta
135720

tttgtaaatg tacgattatt ttctacaata gtcaccctgt tgtgctggca aatactaggt
135780

cttattcaat ttttttgtac ccacgaacca tccctatttc ccccccgtca ctcaccctac
135840

tacccttccc agcctctggt aaccatcctt ctactctgta tctcgataag ttcaattctt
135900

ttaattttta gctccaacaa ataactgaga acatgcaaag tttgtctttc tgctcctggc
135960

ttatttcact taacataatg acctccagtt ctatccatgt tgttgcaaat gacaagatct
136020

cattcttttg tatcgttgaa tagtacttca ttgtgtatat gtactacagt tttctttatc
136080

cattcatctg ttgatgggca cataggttgc cttccaaatc ttggctattg tgaatagtgc
136140

tacaataaac ataagtatgc agatatctct tcgatatact aatttccttt ttgggggtat
136200

atacccagca gtgggatggc tggattgtat gatagctcta tttttagttt ttagaggaac
136260

ctccaaactg ttctccacag tgggaaatta cattcccagc aacagtgtac aaggtgtccc
136320

ttttctccat atcctcacca gcatttgtta cttattgcct gccttttgga taaaggtcat
136380

tttaactggg gtgaggtgat atgtccttgt agttttgatt tgcatttctc tgataaatga
136440

tgctgagcac tttttcctat gcccgtttgc catttgtatg tcttctgaga aatgtctatt
136500

caaatccctt gcccattttt aatgggatta ttagattttt ttcctatcga gttgtttgag
136560

ctctttatat attctgatta ttaatccttt gtcagaaagg tagtttgcaa atattttctc
136620

ccatattgtg ggttgtccct tcactttgtt aattgttccc cttgctgtgc agaagctttt
136680

taacttgatg tgattccatt tgttcatttt tgctttggtt gcctgtgctt gtccaggtat
136740

tactcaagaa atctttgccc acttctgttt catgggtcta tgtgtctgtt tttatgccaa
136800

tatcatgctg ttttggttac tataactctg tgtataattt gaagttaggt aatgtgattc
136860

ttccagtttt gttctttttg ctctggatag ctttggttat tctgggttgt ttgtggttcc
136920

aataaaaatt tttgaattgt tttctctatt tctgtgaaga atgtcattca tattttgata
136980

gagattgcat tgactctgta gattgctttg ggtagtatga acattttaac aatatttatt
137040

cttccaatcc atgaattaga ccccttatgt atcatattta taaaagtaag ctcaagcact
137100

ttaaaatcat gacctctaga gtgttttaaa ttactgtcat tatcagatta tcctctgagt
137160

gtgcatgtgt gtgtgtgtac atgtgtgcac ctgtcattat ttcaaatttt aaaatatttg
137220

agtgctacta gatgctcagc tctaagagac agggtacaag gaggaagagt tcaaaactat
137280

agttgtctct gccctcaaga agtgtaacaa ctactactac catcaactgg ataaaattat
137340

aggatgtcag gcactgtgct ttacatttat tttctcattt aatttgatcc tcacaataac
137400

cctatgagct aaatatcaac ttgattttcc aaattgggaa accaaggctt gaatggctaa
137460

gtcatttgac tcagtcacac aagcaacagg caacagcaag gattcaaagc caggtactcc
137520

caattccaaa gcctagattc ttaaccacag cacatctggc tgacaagaaa acacaaacat
137580

ccattaaggg tgaggagtag aaagagtata tttacagtga agagctaagt cctgagatat
137640

ggagtaaagt ggcttaggag aaatcaaaga atgcaaatag ggtgaggagg agccacatac
137700

acagctgtta gctttgtgaa ccctgtgaac actgatgcct aactgatcaa acacaatctg
137760

tctgctggcc tctgacccat gatctactag tgtactatgt ctttagccac tgacagaatg
137820

gaaaaccaat gacgttttga caggccttaa gacctttcaa agttatctcc aaggatggaa
137880

tcagctcttt ccctgatgtg gacagacttc tattctgttc tagacataag gccatatatg
137940

gaggaagttt gaaatcctct ggccaaatac tagatagtac ttccattgtt taataattaa
138000

gtgattttta taaccctatc cctaagagca tatggcaatt gtctttgccc acaggatcaa
138060

ctgtcactaa ccttttagct tggtggaaaa agctaaatgt cttataatgt ctaaaaaaat
138120

gtaataccac ttcctctgtg cttgtctcca tgtaccagga agtcgtgggt ctaagtggat
138180

gtggtgtccc tctcatgcct cagcagtccc tttacaaagc agcagacttc tgggtaccca
138240

ggtcggggca gacctcccaa ccttcagggt atgcttctag gttcccagtg ctccaagaga
138300

gggagaaata cttcctctat aaaataatgg ggagtcagag gcatatggga tttccctttg
138360

cttccatgaa attaggagaa agcaccaaca ctttctagta attccacagt gaataggatg
138420

tgctaccagc ccagcatcag tagggcttct cttcattctc aagaagtgta attacctgac
138480

gtggctgcct ggaattaaga ttgccgctat caaggtaata agaaacagtt ccattcttcc
138540

ccatgagcca attattatca gggtcacatc tggaaccttg gcttcatttt cccgattcaa
138600

tccatatttg aatgaccact atggaccagg ttctatgcta acatctcact gactcaaaca
138660

gtcttaggaa atggatatta tttctgtctt acctatgtgg aagctaaata aagctcagga
138720

aagttatatc ttttgtcaca gagctggaca attatagttg gataccagac tgaatctcct
138780

gacactatgt tctgggctga ctccactatg tgccactgac tcccgtagtg ctgaggtttg
138840

gggccttata atattattat tataataaca atacatggga tttcaatggt catcctcata
138900

atcaagctct tactctactc tcaaaggaga aagaacatat catgttgaag ggggctatta
138960

gaaggcaaat gtggtaagtg ctgcaatgaa tggacacaag cagtttagaa accctatgac
139020

gaggatcaac tctgactgga gtgaggatga aggaaaactt aagagatgtg aagccatcca
139080

ctggggagga gatgcttttg aggttaacag aagctcttaa acacagagtt ggaagcaggc
139140

aaacactaag ggattcgtgg accacctctt acatcagggt ttctcaatct tttattgac
139200

atttagggct agataatcct atgtgctggg gggctgtcct gtaaattgca ggatatttag
139260

taacatccct gactagatgc ccactagatc ccagtagagc acactgtccc caggtcatga
139320

caaccaaaaa gtctccagac actgccaaat atcccctagg ggacacagtt atccctagtt
139380

gagaaccagt gcctttggtg aagatgtgcc agaaactggg gattggctaa atgcggaggc
139440

tggggaggtg cagagcaaga acaggaagcc atgaaccaga taggaaagag ggacagatgt
139500

gtgactcaag tcctctcagc aactccctca ggtatggact ctgactacct taaagcaaac
139560

ggagggtcag gcacagtagc tcacgcctgt aatcctacca ctttgggagg ctgagccagc
139620

aggatcactt gagcccagga gttcaagtct agcctaggca atagagtaag gccctgtatc
139680

tgttttttaa aaaacacata tttaaaaaaa gacaaatgga ggcaaataag atcccatgat
139740

gagggccctt tgatgaatgt ccagtaggac cctgattggg agtaggacat ttataagtgg
139800

agaggatgga ttctagaccc acgcagcttg gaacagaaaa acccttactc tctttctcct
139860

ttcagtaaat caaagaaaga gaagacaacc agggggaatg ctaatagaat agtcataatg
139920

aacctggaat tcccacgccc atttgggagg attggagagg aactggataa ggagaaatac
139980

tgatgtgtgt tgtgtctgtg actgcagtgc catccttacc ttctcggcca tctcctattc
140040

cctcacctct cacccttgga agggttttgg cccaggaaaa gcaaaaggag ttgggatact
140100

tacttcaggg agggaggaag gaaagaaaag aagggagtag agatgagaga attctgaggt
140160

tcaaaagata tctattgaca gtgttcacaa aaaggaccat gtttcccagt aaaaagaaaa
140220

caaggtaaag ctgaggctgg ggatgggggt tgaggaaagg gaaggggcct acaagtaaat
140280

gtttacaaaa tgcccactga gtgtcaattc tgggcaaggc acattgcatg agtcaccacc
140340

ttcaattttc acagcatcat gtaataccta tatatgaagt ggttataaca agctcctttc
140400

tatagagaag gaaagtgagg ttcagaggca tcttcacaga ctgaggggac tcccagcgtg
140460

gccaggggct tgtttggcag actcctccat gctgtgcctc ctcacacttc agcacccaca
140520

gcctcttgcc tcatgcaagc tgtagctgcc tgcttgtctg tttgctctgc aacattagag
140580

cagaggggct ctaatctgtg tcaggtgctg tgctttgagt tctgtgactc tagtatttcc
140640

ttatgtgcca ggcacaggcc ccagtgatac aaagaaggga gcaagagtta acgctgaggt
140700

gaacaaagga cccatggaag cccagctgtg tgaatttaca gcacaactaa tgagcacacc
140760

cagttacata actgcctctc ctgctctgct cctatgctca gcagccacta gaggaaagca
140820

gggctgtctt tcttttcctg tgctatatat aattccaact gtttgggcag ggaaatgtgc
140880

tacagctcca cagctcagtt acattagatc ttaatttttt cttaaaaacc tagcaatttc
140940

taacagtgat gccacaaaga taataataca gtaatcttaa gattgagata ctttacccac
141000

atttcctgca cttttctacc ttgaaaacac tcattatttc tggttccact ttgagaagca
141060

gcaagcaagg aaagggtgaa aatgtgagat gctgaagaga cagcttgcaa cattccataa
141120

ttacagcctt caatgctgga acctcaagtc tccaatatca aatggaacag tacttcccct
141180

tggtatctaa ctaggagctg atccattaac caggaatata tttttgatat agagtaggcc
141240

agttttgttt ctggttcttt tgcatctcct ttcctaggcc ctaaacacag actccatgac
141300

ctcatgacag tagaatgaat gctgcataaa gagaatcatt cattcgtcct gggacttcac
141360

tgaagatgat ttttgcttag taagcctctt cttcagatat aatactaaaa ggtatgtttt
141420

ctgagaccta ttaaagcaca catttactaa aatttacatt aagttcatgt cagtcctaaa
141480

aggttctcta atttctgaaa gccatagtct gttattctaa tgtgaattgt tacacacaca
141540

tacagacaca ccttgaacat tttaaaatta catttattta agcatactca ctcactcata
141600

cttttgccca atttggttgg gctttcccct aaaccaattc ttctaaaatg gtgagacttt
141660

aacaaataaa atgatatagc tgatattttc atagggtaat aggttaagaa gaaaaaaatc
141720

caggcttttg aaatactaat tgtattagca ttaatactaa tacctaggcc aggcgcagtg
141780

gctcacacct gtaatcccag cactttggga ggctgaggca ggtggaacac ttgaggtcag
141840

gagtttaaga ccagcctggc caacatgagg aaaccccatc tctactaaaa atacaaaaat
141900

tagccaggcg tcgtggcaca tgcctgtaat cccagctact cggaaggcca aagcaggaga
141960

atcactcgaa cccaggaggt ggaggttgca gtgagccgag actgtgccac tgcactacag
142020

cctgagggac agagcaagac tccatctgaa aaaaaaaaac caaacaaaca aacaaacaaa
142080

caaaaaaaac taatatctaa atattagcag tactatgatt tatagacatg aactcttata
142140

aagagtatgt atatggctgg gcatggtggc tcatgcctgt aatcctggca ctatgggagg
142200

ctaaggctgg cagatcgctt gagcctagga gtttgagacc agcctggaca acatggcaaa
142260

accttgtctc tacaaaaata caaaaattag atgggcattg tggcgtgcgc ctcatgcctg
142320

cagttccagc tacttggcgg ggctgagaca ggaggattgc ctgaacccag gaggtcgagg
142380

ctgcagtgag ccaagatcat gccaccgcac tccagcctgg gtgacagagg gagaacctgt
142440

ttcaaaaaag aaagacttgc ttaatactgg gcagctcagt tatagcaagt actagtcaag
142500

ggtggcatgt cactgtgttc agagttggat gaacctgggt tctaatctgg actcagctac
142560

ttcctagtgt aggtcatttg ggtcttgggc aaagtattga acctcccaaa cctcagttct
142620

cttttgcaca atggaaatca tctgaatata gacaagatag ggcccactat aaaaattaaa
142680

caagatagtc tgtgtggaag tatctttaac aggcattaag tgcctgttaa gtgtctttaa
142740

caggagaatt aagtagcatg ttcaggcttt ttgcctcctt cctcttcccc tccactcccc
142800

agtgaccaac ttgcaccctg agtaaggttt tgcagcacca tccttctcct gtggcttgta
142860

aacctctgca gtaacttcag caatctacac tgcctgcacc ctgccctccc tgtatgctcc
142920

cctgaatcac aaccatattt gcctttctag agcacatatc attgtcttca gtgattccct
142980

gcaggacaaa gtctgcccac ttcagcctgg tatcatgatc ctgtcttagt ctggtccaac
143040

ccaccctttc tgaacagttc cttacaaacc cacatcttcc tacctcaact ctgcctaggc
143100

cattcccctg cttcaagctc ttacagagcc tcccatggcc tacagtataa attcctaact
143160

gtttcaggtg gcatttgaga ccgatggtat caggtccctg cgcctctcca gtttcactgt
143220

ctgctactcg tttccctcct tatactttac acccaagcgt aaatctactt atagtacctg
143280

aaaccccatg ctgggtcatc actcttcagc tttacatata ctcctctctt tgacgagaat
143340

actcttctca tccatttggc aaattcttca acagacttca aacctctctc aaatgcctct
143400

acttctgtgg tacagtctgt ccagaactgt gtcttgtaca tttttctact actttgcacc
143460

tgtgcctgtc atacaacgat ttctccagtt tctccggttt tttttttgta attcactgac
143520

cattattaat gtcttctctg ctaaaagtat aacaacactc agctttgcac agagcagtta
143580

cttgggaaat gtttgcattg ctgaacacag gcagcttcca atctcatggg aacatcaaat
143640

gaaatgcaga ggatacatgc tatacaacac agtcaagagg actgaaagca ggcagcagga
143700

gaattccaca atattcctgt ttaaaatgtt aaaatgttgg tggctgccag agctcaggaa
143760

aagaaaattt ttttatgtgg ttacagctca ctctaacgag tcaattacat tgacctaaag
143820

cccatggtgc tcaaaactgt atttctctct ccaaaacctc attcttttat aaagctccct
143880

cctctgtgga atgaatgtgg gcaatcgctt ttgcagtgtt tgacgatgcc agtttatggc
143940

tatgtttttc tgttcactga actggctcat ttcttcagga gaacaaactg ttctccactg
144000

aagcaggaat tggctgggtt ctaacaaaaa gatttttccc ccctgacagt gtttttgcaa
144060

aagacccaag tcaatttata gccaagtaac aaaaggtcag aaaggttagt caagcaatgg
144120

gcgtaattcc tgtgtgtcac agccaataaa ggaaatgggt ggggataaat agtatgcaat
144180

tttaacctga aattatttgt gcaaaactat tcttgtaaac tatacaaaat tacccatgac
144240

ttccactata taggatttct cactctgatt tttttaaagg tacatttact ttggaagaac
144300

tgagatgacc tcaaattcct cattcacaaa tggaaaatgt taaaaacttt cttcacacta
144360

atataagaaa aacttggtca ttgttttgca gccctgaaac agaattgttc acataaaaaa
144420

aaaaatctca tagttaagtc acaattaaat acaatttgca tgactgaatt taaaataaat
144480

tgcaaaattc ccttgccaaa agggaaacaa cattgaccaa aaggataggt caagaaaaga
144540

ccctacaaaa gtgacagttc ctctgtttta atcatgctaa aagtataaca aactactgca
144600

ttattcagtg agcataagaa caagaaaagc ctgcctttaa agcagacacg aagaactgac
144660

atcaccagcc aagcaagcgg aaggcagatg agtaatagcc tgcactgggc ggccaaccag
144720

gcccaggccc atcagtctgt ggagtaagaa agcctttcag atatcaaggt cgttgggagg
144780

cctccttccc ccagttctcc aatacaaagt ctaatctact caccatttaa aacaatctta
144840

gcatccaaaa gatgaatggt taaatagaac cctgtcaccg ttcagacaag atggtctaaa
144900

cccatttctc tccccacctc cttgtaagca caactgtaaa tcctgaaaat aacacaaaag
144960

gcaaccaaag gagaagtctg aaagaaaaag gaaggtgaat tacttaggga cccaggattg
145020

gaggaacaac atagcagcac atcttatgac cacaccccca ttcaatagaa gaaggtggcc
145080

gagggtgttt cccaaccccc aacctagcaa cagagggcag cagaggattg aatggagtt
145140

ctgccgacaa taccaagcca gctggaagca ccaacaaggg ggatagctca ggaccccact
145200

aacaataaag cagtctgggg aaacactttt ccccccaact acagggcccc caggaagcaa
145260

gctgcatccc agcaggatgg agactccctc ctctacctag atatcaggca gcatggcctg
145320

gggaaactcc ttacccccat cagacagcac caacagggac tagtgggagc cgtggcagca
145380

ccagataaac caagtggacc aaaagagtac tgcaaaggct ctgaaaagta aatcatcgtt
145440

ggaaccacaa gagtaaatcc cagctcacat gctgcatcta aacactgtga ccgcctgcta
145500

aaatggaaga tttaaatagg acccagactc tcttaataga caaaatgtcc gaagtagatt
145560

ttaaaaaatc acctgttggc caggcgtggt agctcatgtc tgtaatccca gtactttggg
145620

aggctgagat ggtttaatca tttgaggcca ggagttcgag accaacctgg gcaacatggc
145680

aaaaccatga agatggactg aaaacagaca gaacctccaa cacttatgaa acaatagcaa
145740

aagatccaat attcatatta tcagagtcac aggagagaat aaaaagaagg gctgaaagag
145800

tattcaaaaa gataatgact gaaaattccc caaatttgct gaaggacata aacctagatt
145860

cagaaggtga acatatccca aataggataa actcaaagaa atccacacta agacacatct
145920

taattatatt tctgtaaatt aaagacaaag aataactctt gaaagcagag aaaaacaaca
145980

tactacctat aagggaatat caagcgacaa caggtttctt atctaaaacc atggaggcct
146040

ggccgggcac actggctcac gcctgtaatc ctatgacttt gggaggccaa ggcaggcaga
146100

tcatttgagg ttaggagttt gagaccagct tggccaacat ggtgaaaccc cgtctctact
146160

aaaaataaaa aataataaca aataaaatta aaaaataaaa ccatggaggc cagaaggaag
146220

tggcacaaca tttttatggt taaaggaagt ttttcaaaca gaaaggaaat gattaaaaga
146280

attctgaagc atcaaaaggg aacagaaaac aacagaaaag gcagaaatat ggggagatat
146340

atatatatat agattatcct cctgtagtcc cagctatttg ggaggctaaa gcaggaatat
146400

tgcttgagtc tagaagttca cttctaccct gggcaacata gcaagactct gtgtcttaaa
146460

aaaattaaaa attaaaaggg ggaaggtaaa gggacctgaa tggaaatcag gtttccacat
146520

ttcactcaaa gtggtaaaat actgatc
146547

<210> 53
<211> 2099
<212> DNA
<213> Homo sapiens

<400> 53
   aagaagctgc cgttgttctg ggtactacag cagaagggta tgcggaagcg agcaccccag
   60
   tctgagatgg ctcctgccgg tgtgagcctg agggccacca tcctctgcct cctggcctgg
   120
   gctggcctgg ctgcaggtga ccgggtgtac atacacccct tccacctcgt catccacaat
   180
   gagagtacct gtgagcagct ggcaaaggcc aatgccggga agcccaaaga ccccaccttc
   240
   atacctgctc caattcaggc caagacatcc cctgtggatg aaaaggccct acaggaccag
   300
   ctggtgctag tcgctgcaaa acttgacacc gaagacaagt tgagggccgc aatggtcggg
   360
   atgctggcca acttcttggg cttccgtata tatggcatgc acagtgagct atggggcgtg
   420
   gtccatgggg ccaccgtcct ctccccaacg gctgtctttg gcaccctggc ctctctctat
   480
   ctgggagcct tggaccacac agctgacagg ctacaggcaa tcctgggtgt tccttggaag
   540
   gacaagaact gcacctcccg gctggatgcg cacaaggtcc tgtctgccct gcaggctgta
   600
   cagggcctgc tagtggccca gggcagggct gatagccagg cccagctgct gctgtccacg
   660
   gtggtgggcg tgttcacagc cccaggcctg cacctgaagc agccgtttgt gcagggcctg
   720
   gctctctata cccctgtggt cctcccacgc tctctggact tcacagaact ggatgttgct
   780
   gctgagaaga ttgacaggtt catgcaggct gtgacaggat ggaagactgg ctgctccctg
   840
   atgggagcca gtgtggacag caccctggct ttcaacacct acgtccactt ccaagggaag
   900
   atgaagggct tctccctgct ggccgagccc caggagttct gggtggacaa cagcacctca
   960
   gtgtctgttc ccatgctctc tggcatgggc accttccagc actggagtga catccaggac
   1020
   aacttctcgg tgactcaagt gcccttcact gagagcgcct gcctgctgct gatccagcct
   1080
   cactatgcct ctgacctgga caaggtggag ggtctcactt tccagcaaaa ctccctcaac
   1140
   tggatgaaga aactgtctcc ccggaccatc cacctgacca tgccccaact ggtgctgcaa
   1200
   ggatcttatg acctgcagga cctgctcgcc caggctgagc tgcccgccat tctgcacacc
   1260
   gagctgaacc tgcaaaaatt gagcaatgac cgcatcaggg tgggggaggt gctgaacagc
   1320
   attttttttg agcttgaagc ggatgagaga gagcccacag agtctaccca acagcttaac
   1380
   aagcctgagg tcttggaggt gaccctgaac cgcccattcc tgtttgctgt gtatgatcaa
   1440
   agcgccactg ccctgcactt cctgggccgc gtggccaacc cgctgagcac agcatgaggc
   1500
   cagggcccca gaacacagtg cctggcaagg cctctgcccc tggcctttga ggcaaaggcc
   1560
   agcagcagat aacaaccccg gacaaatcag cgatgtgtca cccccagtct cccacctttt
   1620
   cttctaatga gtcgactttg agctggaaag cagccgtttc tccttggtct aagtgtgctg
   1680
   catggagtga gcagtagaag cctgcagcgg cacaaatgca cctcccagtt tgctgggttt
   1740
   attttagaga atgggggtgg ggaggcaaga accagtgttt agcgcgggac tactgttcca
   1800
   aaaagaattc caaccgacca gcttgtttgt gaaacaaaaa agtgttccct tttcaagttg
   1860
   agaacaaaaa ttgggtttta aaattaaagt atacattttt gcattgcctt cggtttgtat
   1920
   ttagtgtctt gaatgtaaga acatgacctc cgtgtagtgt ctgtaatacc ttagtttttt
   1980
   ccacagatgc ttgtgatttt tgaacaatac gtgaaagatg caagcacctg aatttctgtt
   2040
   tgaatgcgga acaatagctg gttatttctc ccttgtgtta gtaataaacg tcttgccac
   2099

<210>  54
<211>  5525
<212>  DNA
<213>  Homo sapiens

<400>  54
cgccggccga ggaggagggg tgggggtagc ggcggcgccc gcggcccgga gcgggggttg
60

ggggagtaga gaaagcgggg cgcgcggagg aacgctgggt ccccggcgcc gcgggagctg
120

ggaggaccga gccggccgag cgagcagcgc ggcagcacag tccccgcgtg gcgcagcgcg
180

gcggggacgc ggggaccgcc cggatctcct tccactgcgc cccgcgctct gcggtcctcg
240

gccgcctctt cttccttcac tcactgcccg ggcgggagcg gcgcccaagt cgggtgcgcc
300

atgtctgggg ccgggtagcc ccgccgcccg ccggcccgcc agctcgccct ccgagccacc
360

cgccagcggg ccggccggcc gggaagcgcg ggacaggcag atgcagtgag tgaggggggg
420

gccatggcgg aggagcggcc cccccggctg gtggattact tcgtggtagc tgggcttgca
480

gggaacggag cacccatccc tgaggaaacg tgggttcctg aacccagtgg gcccctgcgc
540

cctccccggc cagctgagcc catcacagat gtggcagtca tcgctagggc actgggcgag
600

gaagtgcccc agggctacac atgcatccag gcttctgctg ggggccaccc cttggaactc
660

agtgctggac ttctgggtgg aactcaaccc gtcatctgct accgcagggg ccgtgacaag
720

ccccccctcg ttgagctggg ggtgttgtat gaggggaagg aacgtcccaa gcctggcttc
780

caagtgcttg acacgacacc ctacagccac tcagcaaacc tggcccctcc aggccccggg
840

cacccccgca cctacctcac ttaccggcgg gcagcagagg gggcagggct gcatgccctg
900

ggcatcactg acctctgcct ggtgctgccc agtaagggcg agggcactcc tcatacttac
960

tgccggctgc cccgcaacct caaccctggc atgtggggcc cagcagtgta cctgtgctat
1020

aaggtgggcc tggcgaaggc caacacgctg gtgtacgagg cagttctacg aggcgttccc
1080

aagggccagg ctatcagagc gacaggcacg ggcactgggc ctgctgagcg ccgtggagcg
1140

gggtcgggca ctggggggca gagctgtgcg cagccggcgt gccatcgctg tgctgtcccg
1200

ctggcctgcc ttccctgcct tccgcgcctt cctcaccttc ctttaccgct actccgtctc
1260

aggcccccac cgcctaccct tggaagcgca catctcccac ttcattcaca acgttccctt
1320

cccttcccca cagagacccc gcatcctagt gcagatgtct ccctatgaca acttgctcct
1380

ctgtcagcct gtatcctcac ccctgcccct cagtggtgcc agcttcctgc agctgctgca
1440

gagcctgggc cctgagctgg ctatcacact gctgctggct gtgctcacag agcacaagct
1500

gctagtccac tcgctgcggc cagacctgct caccagcgtc tgtgaggccc tcgtctcgat
1560

gatcttccca ctgcactggc agtgccccta cattcctctg tgcccgctgg tgctggcaga
1620

tgtgctgagt gccccagtgc ccttcattgt gggtatccac tccagctact ttgatctgca
1680

tgacccgcct gctgatgtca tctgtgtaga ccttgatacc aacacgctct tccagactga
1740

ggaaaagaag ctcctctccc ctcggaccct gccccgcaga ccctacaagg ttctgctggc
1800

cacactgaca aacctgtacc agcagctgga ccagacatac actggacctg aggaggaagc
1860

atccctggag ttcctactga cagactacga ggcagtgtgt ggccgcaggg cccggctgga
1920

gcgcgaagtc caaggagcct tcctccgctt catggcctgt ctgctcaagg gctaccgggt
1980

cttcctgcgc ccactcaccc aggccccctc cgagggagct cgtgatgttg acaacctttt
2040

cttcctgcag ggcttcctca aatcccggga acgctccagc cacaaacttt actctcagct
2100

gctgcacaca cagatgttct cacagttcat tgaggagtgc tcttttggct ctgctcgcca
2160

tgctgccctt gaattctttg actcttgtgt tgaaaaggtc cacccagagc aggagaagcc
2220

tgagccgaca cccttagtgg agctagagga gctgtcagga agtgagctca ctgtctttat
2280

cacacctccc gaggagcctg ccttaccaga gggcagtgaa tccactcccc agtactgcta
2340

tgatggattc ccagagctac gggctgagtt gtttgagtct cttcaagagc aacctggggc
2400

cctgcctgtg ccaggccctt cccgtagcgc ccccagcagt cctgctcctc gccgtaccaa
2460

acaggagatg aaagttgcac agcggatggc acagaagtca gcagctgtgc ctgagctgtg
2520

ggcccggtgc ctgctggggc actgctatgg gctgtggttc ctgtgtctgc ctgcctatgt
2580

gcggtcggca ccctcccgag tgcaggcact gcacacagcc taccatgtgc tgcgccagat
2640

ggagagcggc aaggtggtgc tccctgatga ggtgtgttac cgggtactga tgcagctctg
2700

ctcacactat gggcagcctg tgctgtctgt gcgggtcatg ctggagatgc gtcaggcagg
2760

cattgtgccc aacaccatca cctatggcta ctacaataag gctgtgttgg aaagcaagtg
2820

gccgtctggc acaccaggtg ggcgtctgcg ctgggccaag ctccggaatg ttgtcctggg
2880

ggctgctcag ttccgccagc ccttgagaga acggcaacag cagcagcagc agcagcagca
2940

gcagcagcag cagcagcagc agcagcagga gcaggtgtca gcacatcaag aggcaggcag
3000

ctcccaggca gagccctatt tggagcgccc ttcccctact cgccctcttc agcgccagac
3060

tacttgggct gggcgaagtc tgagagaccc agcctcaccc cctggacgcc tggtgaagag
3120

tggtagcctg ggcagtgccc gaggggcaca gcccactgtg gaggccggtg tggcccacat
3180

gatagaggcc ttgggggtcc tggaaccccg gggatcacct gtgccctggc acgatggaag
3240

tctctcagac ctgagcctga caggggagga gccgctccct ggaggcagcc cagggggctc
3300

aggctcagcc ctgagtgccc agtccactga ggccctggaa gggctaagtg ggcggggacc
3360

caaggctggt gggcgacagg atgaggcagg caccccccga cgagggctgg gtgcccgcct
3420

ccaacagctg ctcactcctt cccgccactc ccctgcctcc cgcattcccc cacctgagct
3480

gcctcctgac ctgccacccc cagcccgccg cagccccatg gacagtcttc tgcacccccg
3540

ggagcgccct ggatccactg cctccgagag ctcagcctct ctgggcagtg agtgggacct
3600

ctcagaatct tctctcagca acctgagtct tcgccgttcc tcagagcgcc tcagtgacac
3660

ccctggatcc ttccagtcac cttccctgga aattctgctg tccagctgct ccctgtgccg
3720

tgcctgtgat tcgctggtgt atgatgagga aatcatggct ggctgggcac ctgatgactc
3780

taacctcaac acaacctgcc ccttctgcgc ctgccccttt gtgcccctgc tcagtgtcca
3840

gacccttgat tcccggccca gtgtccccag ccccaaatct gctggtgcca gtggcagcaa
3900

agatgctcct gtccctggtg gtcctggccc tgtgctcagt gaccgaaggc tctgccttgc
3960

tctggatgag ccccagctct gcaacggcca catgggggga gcctcccggc gggttgagag
4020

tggggcatgg gcatacctga gccccctggt gctgcgtaag gagctggagt cgctggtaga
4080

gaacgagggc agtgaggtgc tggcgttgcc tgaactgccc tctgcccacc ccatcatctt
4140

ctggaacctt ttgtggtatt tccaacggct acgcctgccc agtattctac caggcctggt
4200

gctggcctcc tgtgatgggc cttcgcactc ccaggcccca tctccttggc taacccctga
4260

tccagcctct gttcaggtac ggctgctgtg ggatgtactg acccctgacc ccaatagctg
4320

cccacctctc tatgtgctct ggagggtcca cagccagatc ccccagcggg tggtatggcc
4380

aggccctgta cctgcatccc ttagtttggc actgttggag tcagtgctgc gccatgttgg
4440

actcaatgaa gtgcacaagg ctgtggggct cctgctggaa actctaggcc ccccacccac
4500

tggcctgcac ctgcagaggg gaatctaccg tgagatatta ttcctgacaa tggctgctct
4560

gggcaaggac cacgtggaca tagtggcctt cgataagaag tacaagtctg cctttaacaa
4620

gctggccagc agcatgggca aggaggagct gaggcaccgg cgggcgcaga tgcccactcc
4680

caaggccatt gactgccgaa aatgttttgg agcacctcca gaatgctaga gaccttaagc
4740

ttccctctcc agcctagggt ggggaagtga ggaagaaggg attctagagt taaactgctt
4800

ccctgttgcc ttcatggagt tgggaacagg ctgggaagga tgcccagtca aaggctccaa
4860

gcgaggacaa caggaagagg gatccactgt taccaaaagt cctgattccc ccatcaccaa
4920

cctacccagt ttgttcgtgc tgatgttggg ggagatctgg ggggagttgg tacagctctg
4980

ttcttccctt gtcctatacc gggaactccc ctccagggta cccacagatc tgcattgccc
5040

tggtcatttt agaagttttt gttttaaaaa acaactggaa agatgcagag ctactgagcc
5100

tttgccctga atgggaggta gggatgtcat tctccaccaa taatggtccc tcttccctga
5160

cgttgctgaa ggagcccaag gctctccatg cctttctacc taagtgtttg tattttattt
5220

taaattattt attctggagc cacagccccc ttgcttatga ggttcttatg gagagtgaga
5280

aagggaaggg aaatagggca ccatggtccg gtggtttgta gttccttcaa agtcaggcac
5340

tgggagctag aggagtctca agctcccctt aggaagaact ggtgccccct ccagtcctaa
5400

tttttcttgc ctgccccgcc ttggggaatg cctcacccac ccaggtcctg acctgtgcaa
5460

taaggattgt tccctgcgaa gttttgttgg atgtaaatat agtaaaagct gcttctgtct
5520

ttttc
5525


<210>  55
<211>  615
<212>  DNA
<213>  Homo sapiens


<220>
<221>  misc_feature
<222>  (1)..(608)

<220>
<221>  misc_feature
<222>  (1)..(1)
<223>  n is a, c, g, or t

<220>
<221>  misc_feature
<222>  (3)..(3)
<223>  n is a, c, g, or t

<220>
<221>  misc_feature
<222>  (5)..(5)
<223>  n is a, c, g, or t

<220>
<221>  misc_feature
<222>  (219)..(219)
<223>  n is a, c, g, or t

<220>
<221>  misc_feature
<222>  (258)..(258)
<223>  n is a, c, g, or t

<220>
<221>  misc_feature
<222>  (494)..(494)
<223>  n is a, c, g, or t

<220>

<221>  misc_feature
<222>  (573)..(573)
<223>  n is a, c, g, or t

<220>
<221>  misc_feature
<222>  (600)..(600)
<223>  n is a, c, g, or t

<220>
<221>  misc_feature
<222>  (608)..(608)
<223>  n is a, c, g, or t

<400>  55
ncntnttgtc tttagctagt tttaataaat cttttcaaaa tgatggtcaa gaactctaga
60

aaacgggtgg aggggatgct cctttatagc aggaaaggaa ttccattaat tgtagaaatt
120

ctcattaaat ttcagctttg gttcttcaga agaaacactc atataacttc tgcagtctgc
180

tgtgtctgaa caccatctac ctgaggcagg ggccaagtnt cccttcatca tcaaaggcca
240

aaattggatt caaaggantt tctgggctct cactgttggt gacatctaat ctggatggtt
300

tagatccaat ggggtaagtt tataatttct tcaaaatttt cattctgaac agatacacca
360

ttttcatttg ggttgttttt cccaaatcgg ggagtgctct ccactgtcac ttttgactgg
420

gtgtgagggt aaccacatgt ggggctgccc aactgggttt tcccatttcc cgggagcgtt
480

tgttgtacat gggnaggtgc agacacctct gttcctccag tgaggagctc ccttgggcta
540

tatcacccgt ttctctgatc actgggtttt ctnatctgta ggcttctgtt ctccgggggn
600

cttttcanaa actct
615


<210>  56
<211>  615
<212>  DNA
<213>  Homo sapiens


<220>
<221>  misc_feature
<222>  (1)..(608)

<220>
<221>  misc_feature
<222>  (1)..(1)
<223>  n is a, c, g, or t

<220>
<221>  misc_feature

<222>  (3)..(3)
<223>  n is a, c, g, or t

<220>
<221>  misc_feature
<222>  (5)..(5)
<223>  n is a, c, g, or t

<220>
<221>  misc_feature
<222>  (219)..(219)
<223>  n is a, c, g, or t

<220>
<221>  misc_feature
<222>  (258)..(258)
<223>  n is a, c, g, or t

<220>
<221>  misc_feature
<222>  (494)..(494)
<223>  n is a, c, g, or t

<220>
<221>  misc_feature
<222>  (573)..(573)
<223>  n is a, c, g, or t

<220>
<221>  misc_feature
<222>  (600)..(600)
<223>  n is a, c, g, or t

<220>
<221>  misc_feature
<222>  (608)..(608)
<223>  n is a, c, g, or t

<400>  56
ncntnttgtc tttagctagt tttaataaat cttttcaaaa tgatggtcaa gaactctaga
60

aaacgggtgg aggggatgct cctttatagc aggaaaggaa ttccattaat tgtagaaatt
120

ctcattaaat ttcagctttg gttcttcaga agaaacactc atataacttc tgcagtctgc
180

tgtgtctgaa caccatctac ctgaggcagg ggccaagtnt cccttcatca tcaaaggcca
240

aaattggatt caaaggantt tctgggctct cactgttggt gacatctaat ctggatggtt
300

tagatccaat ggggtaagtt tataatttct tcaaaatttt cattctgaac agatacacca
360

ttttcatttg ggttgttttt cccaaatcgg ggagtgctct ccactgtcac ttttgactgg
420

gtgtgagggt aaccacatgt ggggctgccc aactgggttt tcccatttcc cgggagcgtt
480

tgttgtacat gggnaggtgc agacacctct gttcctccag tgaggagctc ccttgggcta
540

tatcacccgt ttctctgatc actgggtttt ctnatctgta ggcttctgtt ctccgggggn
600

cttttcanaa actct
615



<210>  57
<211>  1077
<212>  DNA
<213>  Homo sapiens



<220>
<221>  misc_feature
<222>  (969)..(970)

<220>
<221>  misc_feature
<222>  (969)..(969)
<223>  n is a, c, g, or t

<400>  57
gagcccctcc cctcggcccg cgcgggagga gtgtgaccca ggtgccgctt cctctcgccg
60

ccgagggtca ggagcccggg agcgcgaccc tcccccggcc cggcctggcc cggcctggcc
120

agtccccgcg gtctctgccc gggctgacgc ccaggaatgt ggtcgacgag aagccccaac
180

agcacggcgt ggcctctcag cctcgagcct gatccgggga tggcctctgc ctccaccaca
240

atgcatacta ccaccattgc agagcctgat ccagggatgt ctggatggcc ggatggcaga
300

atggagacct ccacccccac cataatggac attgtcgtca ttgcaggtgt gattgctgct
360

gtggccatcg tcctagtctc cctcctcttc gtcatgctgc gctacatgta ccggcacaag
420

ggcacgtacc acaccaatga ggccaagggc acggagtttg ctgagagtgc agatgcagcc
480

ctgcagggag accctgccct ccaagatgct ggtgatagca gcagaaagga gtactttatt
540

tgagggacaa cagacttcac ttccctgaat gcctccccca tctccatcag gaaaaataca
600

ccccatcgcc cagcacccct gctgatacca ccagacagag agagagagca cttgattctt
660

cccgagatag ccacctggaa acactaggtg cctgcccagg gaggaacgga ggaggactcg
720

cgctacaaga ggccactccc agggacccag ggaggcgatg gccaccccag aggccacctt
780

ttgctccacc ggaggtggga gaaaatctgg gcacgatcgg gccccctggg cagtgcagga
840

caacatcagc tcactggcag gaaagtcctt gttgagggtg agggggtgct ggggtacccg

900

ggggctgggg aagcaaggaa ataagtcatc tgtatgctga ctggggataa tggcatcaaa
960

tgtcagtcnt tgacatttgg ggggaacagc aggtgccaga gctaaaaggt acctttgtct
1020

gccattgatc cagctcagaa cgattggaaa taaatttgaa atgtaaccga gcaaaaa
1077

<210> 58
<211> 1416
<212> DNA
<213> Homo sapiens

<400> 58
cagaaggttg gggccatcca accaggcagt ccgcctgcac agcagctgtc cctgctcatc
60

gggctggagg acagaagaca gaaccctaaa accacaggtt gctgaaaagc caggagtcaa
120

aatgactgag cgctttgact gccaccattg caacgaatct ctctttggca agaagtacat
180

cctgcgggag gagagcccct actgcgtggt gtgctttgag accctgttcg ccaacacctg
240

cgaggagtgt gggaagccca tcggctgtga ctgcaaggac ttgtcttaca aggaccggca
300

ctggcatgaa gcctgtttcc actgctcgca gtgcagaaac tcactggtgg acaagccctt
360

tgctgccaag gaggaccagc tgctctgtac agactgctat tccaacgagt actcatccaa
420

gtgccaggaa tgcaagaaga ccatcatgcc aggtacccgc aagatggagt acaagggcag
480

cagctggcat gagacctgct tcatctgcca ccgctgccag cagccaattg gaaccaagag
540

tttcatcccc aaagacaatc agaatttctg tgtgccctgc tatgagaaac aacatgccat
600

gcagtgcgtt cagtgcaaaa tgcccatcac cacgggaggg gtcacttacc gggagcagcc
660

ctggcacaag gagtgcttcg tgtgcaccgc ctgcaggaag cagctgtctg ggcagcgctt
720

cacagctcgc gatgactttg cctactgcct gaactgcttc tgtgacttgt atgccaagaa
780

gtgtgctggg tgcaccaacc ccatcagcgg acttggtggc acaaaataca tctcctttga
840

ggaacggcag tggcataacg actgctttaa ctgtaagaag tgctccctct cactggtggg
900

gcgtggcttc ctcacagaga gggacgacat cctgtgcccc gactgtggga aagacatctg
960

aattcaacac agagaagttg ctgcttgtga tctcacacac agatttttat gttttctttc
1020

tcacccaggc aatcttgcct tctggtttct tccagccaca ttgagacttt cttctagtgc
1080

ttttcagtga tactcacgtt tgcttaaacc ctttagtgct ttgtgatagt tcagtcccag
1140

ggaaagagaa aactcgccct aggccctagg tgggaagatg gtttgaaatt tttgtaatcg
1200

agtaaggcac acccaaatgt aaaaatcctt ttgaatgatg cctttataaa tctttctctc
1260

actgtctatt taagtgcaat taacatatgt cacgaacttg aaagttttct aaactcaata
1320

aggtaatgac cagttgttat ttacagctct gtaacctccc gttgcgtcaa gtctaaacca
1380

agattatgtg acttgcaata aagttattca gaacag
1416

<210> 59
<211> 5910
<212> DNA
<213> Homo sapiens

<400> 59
cggaggacag ccggaccgag ccaacgccgg ggactttgtt ccctccacgg aggggactcg
60

gcaactcgca gcggcagggt ctggggccgg cgcctgggag ggatctgcgc cccccactca
120

ctccctagct gtgttcccgc cgccgccccg gctagtctcc ggcgctggcg cctatggtcg
180

gcctccgaca gcgctccgga gggaccgggg gagctcccag gcgcccggga ctggagactg
240

atgcatgagg ggcctacgga ggcgcaggag cggtggtgat ggtctgggaa gcggagctga
300

agtcccctgg gctttggtga ggcgtgacag tttatcatga ccgtgttcag gcaggaaaac
360

gtggatgatt actacgacac cggcgaggaa cttggcagtg gacagtttgc ggttgtgaag
420

aaatgccgtg agaaaagtac cggcctccag tatgccgcca aattcatcaa gaaaaggagg
480

actaagtcca gccggcgggg tgtgagccgc gaggacatcg agcgggaggt cagcatcctg
540

aaggagatcc agcaccccaa tgtcatcacc ctgcacgagg tctatgagaa caagacggac
600

gtcatcctga tcttggaact cgttgcaggt ggcgagctgt ttgacttctt agctgaaaag
660

gaatctttaa ctgaagagga agcaactgaa tttctcaaac aaattcttaa tggtgtttac
720

tacctgcact cccttcaaat cgcccacttt gatcttaagc ctgagaacat aatgcttttg
780

gatagaaatg tccccaaacc tcggatcaag atcattgact ttgggttggc ccataaaatt
840

gactttggaa atgaatttaa aaacatattt gggactccag agtttgtcgc tcctgagata
900

gtcaactatg aacctcttgg tcttgaggca gatatgtgga gtatcggggt aataacctat
960

atcctcctaa gtggggcctc cccatttctt ggagacacta agcaagaaac gttagcaaat
1020

gtatccgctg tcaactacga atttgaggat gaatacttca gtaataccag tgccctagcc
1080

aaagatttca taagaagact tctggtcaag gatccaaaga agagaatgac aattcaagat
1140

agtttgcagc atccctggat caagcctaaa gatacacaac aggcacttag tagaaaagca
1200

tcagcagtaa acatggagaa attcaagaag tttgcagccc ggaaaaaatg gaaacaatcc
1260

gttcgcttga tatcactgtg ccaaagatta tccaggtcat tcctgtccag aagtaacatg
1320

agtgttgcca gaagcgatga tactctggat gaggaagact cctttgtgat gaaagccatc
1380

atccatgcca tcaacgatga caatgtccca ggcctgcagc accttctggg ctcattatcc
1440

aactatgatg ttaaccaacc caacaagcac gggacacctc cattactcat tgctgctggc
1500

tgtgggaata ttcaaatact acagttgctc attaaaagag gctcgagaat cgatgtccag
1560

gataagggcg ggtccaatgc cgtctactgg gctgctcggc atggccacgt cgataccttg
1620

aaatttctca gtgagaacaa atgccctttg gatgtgaaag acaagtctgg agagatggcc
1680

ctccacgtgg cagctcgcta tggccatgct gacgtggctc aagttacttg tgcagcttcg
1740

gctcaaatcc caatatccag gacaaaggaa gaagaaaccc ccctgcactg tgctgcttgg
1800

cacggctatt actctgtggc caaagccctt tgtgaagccg gctgtaacgt gaacatcaag
1860

aaccgagaag gagagacgcc cctcctgaca gcctctgcca ggggctacca cgacatcgtg
1920

gagtgtctgg ccgaacatgg agccgacctt aatgcttgcg acaaggacgg acacattgcc
1980

cttcatctgg ctgtaagacg gtgtcagatg gaggtaatca agactctcct cagccaaggg
2040

tgtttcgtcg attatcaaga caggcacggc aatactcccc tccatgtggc atgtaaagat
2100

ggcaacatgc ctatcgtggt ggccctctgt gaagcaaact gcaatttgga catctccaac
2160

aagtatgggc gaacgcctct gcaccttgcg gccaacaacg gaatcctaga cgtggtccgg
2220

tatctctgtc tgatgggagc cagcgttgag gcgctgacca cggacggaaa gacggcagaa
2280

gatcttgcta gatcggaaca gcacgagcac gtagcaggtc tccttgcaag acttcgaaag
2340

gatacgcacc gaggactctt catccagcag ctccgaccca cacagaacct gcagccaaga
2400

attaagctca agctgtttgg ccactcggga tccgggaaaa ccacccttgt agaatctctc
2460

aagtgtgggc tgctgaggag ctttttcaga aggcgtcggc ccagactgtc ttccaccaac
2520

tccagcaggt tcccaccttc acccctggct tctaagccca cagtctcagt gagcatcaac
2580

aacctgtacc caggctgcga gaacgtgagt gtgaggagcc gcagcatgat gttcgagccg
2640

ggtcttacca aagggatgct ggaggtgttt gtggccccga cccaccaccc gcactgctcg
2700

gccgatgacc agtccaccaa ggccatcgac atccagaacg cttatttgaa tggagttggc
2760

gatttcagcg tgtgggagtt ctctggaaat cctgtgtatt tctgctgtta tgactatttt
2820

gctgcaaatg atcccacgtc aatccatgtt gttgtcttta gtctagaaga gccctatgag
2880

atccagctga acccagtgat tttctggctc agtttcctga agtcccttgt cccagttgaa
2940

gaacccatag ccttcggtgg caagctgaag aacccactcc aagttgtcct ggtggccacc
3000

cacgctgaca tcatgaatgt tcctcgaccg gctggaggcg agtttggata tgacaaagac
3060

acatcgttgc tgaaagagat taggaacagg tttggaaatg atcttcacat ttcaaataag
3120

ctgtttgttc tggatgctgg ggcttctggg tcaaaggaca tgaaggtact tcgaaatcat
3180

ctgcaagaaa tacgaagcca gattgtttcg gtctgtcctc ccatgactca cctgtgtgag
3240

aaaatcatct ccacgctgcc ttcctggagg aagctcaatg gacccaacca gctgatgtcg
3300

ctgcagcagt ttgtgtacga cgtgcaggac cagctgaacc ccctggccag cgaggaggac
3360

ctcaggcgca ttgctcagca gctccacagc acaggcgaga tcaacatcat gcaaagtgaa
3420

acagttcagg acgtgctgct cctggacccc cgctggctct gcacaaacgt cctggggaag
3480

ttgctgtccg tggagacccc acgggcgctg caccactacc ggggccgcta caccgtggag
3540

gacatccagc gcctggtgcc cgacagcgac gtggaggagc tgctgcagat cctcgatgcc
3600

atggacatct gcgcccggga cctgagcagc gggaccatgg tggacgtccc agccctgatc
3660

aagacagaca acctgcaccg ctcctgggct gatgaggagg acgaggtgat ggtgtatggt
3720

ggcgtgcgca tcgtgcccgt ggaacacctc acccccttcc catgtggcat ctttcacaag
3780

gtccaggtga acctgtgccg gtggatccac cagcaaagca cagagggcga cgcggacatc
3840

cgcctgtggg tgaatggctg caagctggcc aaccgtgggg ccgagctgct ggtgctgctg
3900

gtcaaccacg gccagggcat tgaggtccag gtccgtggcc tggagacgga gaagatcaag
3960

tgctgcctgc tgctggactc ggtgtgcagc accattgaga acgtcatggc caccacgctg
4020

ccagggctcc tgaccgtgaa gcattacctg agcccccagc agctgcggga gcaccatgag
4080

cccgtcatga tctaccagcc acgggacttc ttccgggcac agactctgaa ggaaacctca
4140

ctgaccaaca ccatgggggg gtacaaggaa agcttcagca gcatcatgtg cttcgggtgt
4200

cacgacgtct actcacaggc cagcctcggc atggacatcc atgcatcaga cctgaacctc
4260

ctcactcgga ggaaactgag tcgcctgctg gacccgcccg accccctggg gaaggactgg
4320

tgccttctcg ccatgaactt aggcctccct gacctcgtgg caaagtacaa caccaataac
4380

ggggctccca aggatttcct ccccagcccc ctccacgccc tgctgcggga atggaccacc
4440

taccctgaga gcacagtggg caccctcatg tccaaactga gggagctggg tcgccgggat
4500

gccgcagacc ttttgctgaa ggcatcctct gtgttcaaaa tcaacctgga tggcaatggc
4560

caggaggcct atgcctcgag ctgcaacagc ggcacctctt acaattccat tagctctgtt
4620

gtatcccggt gagggcagcc tctggcttgg acagggtctg tttggactgc agaaccaagg
4680

gggtgatgta gcccatcctt ccctttggag atgctgaggg tgtttcttcc tgcacccaca
4740

gccaggggga tgccactcct ccctccggct tgacctgttt ctctgccgct acctccctcc
4800

ccgtctcatt ccgttgtctg tggatggtca ttgcagttta agagcagaac agatctttta
4860

ctttggccgc ttgaaaagct agtgtacctc ctctcagtgt tttggactcc atctctcatc
4920

ctccagtacc ttgcttctta ctgataattt tgctggaatt cctaactttt caatgacatt
4980

ttttttaact atcatattga ttgtccttta aaaaagaaaa gtgcatattt atccaaaatg
5040

tgtatttctt atacgctttt ctgtgttata ccatttcctc agcttatctc ttttatattt
5100

gtaggagaaa ctcccatgta tggaatccca ctgtatgatt tataaacaga caatatgtga
5160

gtgccttttg cagaagaggg tgtgtttgaa atcatcggag tcagccagga gctgtcacca
5220

aggaaacgct acctctctgt cccttgctgt atgctgatca tcgccagagg tgcttcaccc
5280

tgagttttgt tttgtattgt tttctgacag tttttctgtt ttgtttggca aggaaagggg
5340

agaagggaat cctcctccag ggtgatttta tgatcagtgt tgttgctcta ggaagacatt
5400

tttccgtttg cttttgttcc aatgtcaatg tgaacgtcca catgaaacct acacactgtc
5460

atgcttcatc attccctctc atctcaggta gaaggttgac acagttgtag ggttacagag
5520

acctatgtaa gaattcagaa gacccctgac tcatcatttg tggcagtccc ttataattgg
5580

tgcatagcag atggtttcca catttagatc ctggtttcat aacttcctgt acttgaagtc
5640

taaaagcaga aaataaagga agcaagtttt cttccatgat tttaaattgt gatcgagttt
5700

taaattgata ggagggaaca tgtcctaatt cttctgtcct gagaagcatg taatgttaat
5760

gttatatcat atgtatatat atatatgcac tatgtatata catatatatt aatactggta
5820

tttttactta atctataaaa tgtcgttaaa aagttgtttg tttttttctt tttttataaa
5880

taaactgttg ctcgttaaaa aaaaaaaaaa
5910

<210> 60

<211>  2442
<212>  DNA
<213>  Homo sapiens

<400>  60
gcgggattcc gggccgggcc ggcctgggct gcaatcaatg cggctttgtc tgggacgccc
60

acatcccaga ggccattccc gggtcggcaa atcggagcgc ggcggggcgc gcgggggtga
120

gataagcggc catgtgatcc cacctgggct ggaaggggag gggcgccagg tgaggcggcg
180

gccggtgggg cgcgggcggc cacgcggggc tcctgcagca tggctgtcag caggaaggac
240

tggtccgcgc tgtccagcct tgcccggcag aggactctgg aggatgagga ggaacaggag
300

cgcgagcgca ggcggcggca ccgcaacctg agctccacca cggacgatga ggctcccagg
360

ctcagccaga atggagaccg gcaggcctct gcttctgaga gactaccgag cgtggaagaa
420

gcagaggtgc ccaagccact gcccccagcc tccaaagatg aggacgagga catccagagc
480

atcctcagaa cacggcagga gcggaggcag aggcggcagg tggtggaggc tgcacaggcc
540

cccatccagg agaggctgga ggcagaggag gggaggaaca gcttgagccc tgtgcaggcc
600

acacagaaac ccctagtctc caagaaggaa ctggaaatcc cacctcgccg gagactgagt
660

cgggaacagc ggggcccctg gcccctggag gaggagagct tggtgggcag ggagccagaa
720

gagaggaaga aaggggttcc agaaaagtcc ccagtcttgg agaagtcctc catgccaaag
780

aagacggcac ctgaaaagag cctggtctcc gataaaacct ccatctctga gaaggtgctg
840

gcctcagaga agacatctct atcagagaag atagcagtgt cagagaaaag aaacagctca
900

gagaagaagt ctgttctaga aaaaaccagt gtctctgaga agtcgctggc cccagggatg
960

gcactgggct caggaaggag gctggtgtct gagaaagctt ccatctttga gaaggcactg
1020

gcctcagaga agagcccaac tgcagatgct aagccggccc caaagagggc cacagcctca
1080

gagcagcccc tggcgcagga gccgccagcc tctgggggaa gcccagccac caccaaggag
1140

cagagaggaa gggccctccc tgggaagaac ctgccctctt tggcaaagca gggggcttca
1200

gaccctccga ctgtggcctc ccgcctccca cccgtcacac tccaggtgaa aatccccagc

1260

aaggaggaag aggcagatat gtcctcaccc acacagcgaa cctacagcag ctccctcaaa
1320

cgctccagcc ccaggaccat ctcctttcgg atgaaaccca agaaagaaaa ctcggaaaca
1380

accctaactc gcagtgccag catgaagctc ccagacaaca cagtgaagtt gggagagaag
1440

ctggagagat accacacggc catacggaga tcagaatctg tcaagtctcg gggtctgcct
1500

tgcactgagt tattcgtggc tcctgtgggt gtagccagca agcgccacct ctttgagaag
1560

gaactggcgg gccagagccg agcagaacca gcctccagcc ggaaggagaa cttgaggctc
1620

tcaggggttg tgacatcaag gctcaacctg tggatcagca ggacccagga atctggagat
1680

caggaccccc aggaggcaca gaaagcatca tctgcaaccg agaggactca gtggggacag
1740

aaatctgact cctcgctgga cgctgaggtg tgacaagccc cgccaagaca gacctgcaag
1800

tcttcgtctc aagggacctc cctcatgcca ggcccctgcc tctcacagca gcaccctttc
1860

ctctcattgt ccctgttccc ttgttggctg tggatctgtt tggccagggt ccctggggtc
1920

aggaatattt gcaagactca gccagctcct tcccagccca gcctcttggg gctgggactt
1980

tctcaccctg cggcaggcac aacagatgct gggacccagt ctctgcccag gtcacagcac
2040

aagtgcacat cagcactatg gggcctatgt cctgcccaga gacctctgct ccttcctgct
2100

cacatccaca gtcagggcac ggcgcccctc aagaactcca gagtcacctg tctcatcggc
2160

tcccaacaag tgcctctttg tctatgatgt cccccttctc tgaggcctgg acccacccat
2220

ctttgtccct gggggctgct cccagccact gaggcccgct ctggccaggg gagaaggagc
2280

tgccgtgcgt cttccctgtg ccccgtctcc ctgcttggtt ctcccctccc ttccctggcc
2340

ggctgccatg gccaggagct aagtgccttt ttgtgtgcaa ccacttaccc tttctctgaa
2400

aaacctgttc tcaggaagga tctgataaac tcatttactc tc
2442

<210> 61
<211> 4139
<212> DNA

<213>  Homo sapiens

<400>  61
tcgcggccgc cgagcgcagt gccccgcggg tcttacagga gaggggaccg tcctgggctg
60

gcctggacca tggtgctgct ctgggagcct gcaggagcct ggcttgctct gggcctggcc
120

ctggccctgg gccccagcgt ggccgcagct gcccctcggc aggactgcac gggcgtggag
180

tgcccgccgc tggagaactg cattgaggag gcgctggagc cgggtgcctg ctgtgccacg
240

tgtgtgcagc agggctgcgc ctgcgagggc taccagtact atgactgcct acagggtggc
300

ttcgtgcgcg gccgcgtgcc cgccggtcag tcctattttg tggacttcgg gagcactgag
360

tgctcctgcc caccaggcgg cggcaagatc agctgccagt tcatgctgtg cccggagctg
420

ccgcccaact gcatcgaggc tgtagtggtg gctgacagct gcccacagtg cggccaggtg
480

ggctgcgtcc acgcgggcca cgagtacgcc gctggccaca ctgttcacct gccgccctgc
540

cgggcctgcc actgccctga cgccggtgga gagctcatct gctaccagct ccccggttgc
600

cacgggaact tctcagatgc cgaggagggt gaccccgagc gacactacga agacccctac
660

agctatgacc aggaggtggc cgaggtggaa gcagcaacag ccctgggggg tgaggtccag
720

gcgggtgcag tccaggcagg cgcagggggc cccccagctg ctctgggagg tgggagtcag
780

ccactgtcca ccatccaggc acccccctgg ccagctgtcc tccccaggcc cacagcggct
840

gctgccctgg gtcccccagc cccagtgcag gccaaagcta ggagagtgac cgaggacagt
900

gaggaggaag aagaggagga ggaggagaga gaggaaatgg ctgtcactga gcagctggca
960

gcaggtggcc acagggggct ggatgggctg cccactacag ccccagctgg acccagtctt
1020

cctatccagg aggagagggc agaagctggg gcaagggcag aagctggggc aaggcctgaa
1080

gagaacctca tcctggatgc ccaagccacg tcccgcagca ctgggccgga gggcgtgacg
1140

catgcaccga gcctgggcaa ggctgctctc gtcccaactc aggccgtgcc tggctctccc
1200

agggacccag tcaagcccag cccccacaac atcctgtcca catcactgcc tgatgcagcc
1260

tggatcccac ccacccgaga agtgcccagg aagccgcaag ttctgcccca ttcccacgtg
1320

gaggaggaca cagaccccaa ctctgtccat tctatcccca gaagtagccc tgaaggctcc
1380

accaaggacc tgatcgagac ttgctgcgca gccggacagc agtgggccat tgacaatgac
1440

gagtgcctgg agatccctga gagtggcact gaggacaacg tctgcaggac agcccagagg
1500

cactgctgtg tctcctactt gcaggagaag agctgcatgg ccggcgtcct gggagccaag
1560

gagggtgaga cctgtggggc tgaggacaac gacagctgcg gcatctccct gtacaagcaa
1620

tgctgtgact gctgtggcct gggcctccgc gtgcgggccg agggccagtc gtgtgagtcc
1680

aatcctaacc tgggctatcc ctgcaatcat gtcatgctct cctgctgtga gggtgaagag
1740

cctctcatag tacctgaggt tcgccgacct ccagagcccg cagctgcacc acggagagtt
1800

tcagaggcag agatggcggg ccgagaggcc ctgtcactgg gcacagaggc cgagctgccg
1860

aacagcctgc cgggcgatga ccaggatgag tgccttctcc tcccgggaga gctgtgccag
1920

cacctttgca tcaatactgt gggttcttac cactgtgcct gctttcctgg cttctcactg
1980

caggacgatg gccgcacttg ccgcccagag ggtcaccctc cacagccgga agccccacag
2040

gagcctgcac tgaagtcaga attttcccag gtggcctcta acaccatccc gctgccactg
2100

ccgcagccca atacctgcaa agacaatgga ccctgcaagc aggtgtgcag cactgttggg
2160

ggctcagcca tatgctcctg ttttcccggc tatgccatca tggcggatgg cgtgtcctgt
2220

gaagacatca acgagtgtgt gacggacctg cacacgtgca gccggggcga gcactgtgtg
2280

aacacactgg gctccttcca ctgctacaag gcactcacct gtgagccagg ctatgccctc
2340

aaggatggcg agtgcgaaga cgtggatgag tgtgcgatgg gcacgcacac ctgccagccg
2400

ggcttcttgt gccagaacac caagggctcc ttctactgcc aggccaggca gcgctgcatg
2460

gatggcttcc tgcaggatcc tgaaggcaac tgtgtggaca tcaacgagtg cacgtcactg
2520

tccgagccat gtcggccagg cttcagctgc atcaacacgg tgggctccta cacgtgccag
2580

aggaacccgc tgatctgcgc gcgcggctac cacgccagcg atgatggggc caagtgtgtg
2640

gacgtgaatg agtgtgagac aggtgtgcac cgctgcggtg agggccaagt gtgccacaac
2700

ctccctggct cctaccgctg tgactgcaaa gccggctttc agcgggatgc cttcggccgg
2760

ggctgcatcg acgtgaatga gtgctgggcc tcgccaggcc gcctgtgcca gcacacgtgt
2820

gagaacacac tcggctccta ccgctgttcc tgcgcctccg ggttcctgct agcagcggac
2880

ggcaagcgct gtgaagacgt gaatgagtgt gaggcccagc gctgcagcca ggagtgtgcc
2940

aacatctatg gctcctacca gtgctactgc cgccagggct accagctggc tgaggatggg
3000

cacacctgca cagacatcga cgagtgtgct caaggcgccg gcatcctctg caccttccgc
3060

tgtctcaacg tgccagggag ctaccagtgt gcatgccctg agcagggcta caccatgacg
3120

gccaacggga ggtcctgcaa ggacgtggat gagtgtgcac tgggcaccca caactgttcc
3180

gaggctgaga cctgccacaa catccagggt agcttccgct gcctgcgctt cgagtgtcct
3240

cccaactatg tccaagtctc caaaacgaag tgcgagcgca ccacgtgcca tgacttcctg
3300

gagtgccaga actcgccagc gcgcatcacg cactaccagc tcaacttcca gacgggcctc
3360

ctggtgcctg cgcatatctt ccgcattggc cccgcgccag ccttcacggg ggacaccatc
3420

gccctgaaca tcatcaaggg caatgaggag ggctactttg gcacgcgcag gctcaatgcc
3480

tacacgggtg tggtctacct gcagcgggcc gtgctggagc cccgggactt tgccctggac
3540

gtggagatga agctctggag gcagggctcc gtcaccacct tcctggccaa gatgcacatc
3600

ttcttcacca cctttgccct gtgaggtgcc agcacgggcc acctgcgggt gtggcgcagc
3660

cagggctcac actgcgtggg agggactggg tcactattgt ggtttttact ataactttgt
3720

aaattaactt aattttgctg acttgactcc tgtggcttct ggacccctcc tctgccccgc
3780

aggaggaagt tccacggcag gtggtgcgtt cccatgtagg caccaagtgg aagcttgcac
3840

ggtgggccac ggccgtggcg ggtgccctgt gggtgaggct gggtgatgac ctgaggacca
3900

gagacacgcg accatgttgg ggctcttgga ctcctctgga tgacccgtcc ccaaacgttg
3960

acattccatt tcatgttcca ctgtgattaa cttcttttct tttttaaaaa atcattttaa
4020

agttttttgt ttaactataa agtagtacat gtacattata taaaaaaaaa gttcaactag
4080

tatgaaaggg ttataaagta acagaggaaa acgcctcttg gtccctttaa aaaaaaaaa
4139

<210> 62
<211> 2361
<212> DNA
<213> Homo sapiens

<400> 62
   ccccctccgc tctgctgcgc cgcccggctg ggccccgagg ccgctccgac tgctatgtga
   60
   ccgcgaggct gcgggaggaa ggggacaggg aagaagaggc tctcccgcgg gagcccttga
   120
   ggaccaagtt tgcggccact tctgcaggcg tcccttctta gctctcgcct gcccctttct
   180
   gcagcctagg cggcccaggt tctcttctct tcctcgcgcg cccagccgcc tcggttcccg
   240
   gcgaccatgg tgacgatgga ggagctgcgg gagatggact gcagtgtgct caaaaggctg
   300
   atgaaccggg acgagaatgg cggcggcgcg ggcggcagcg gcagccacgg caccctgggg
   360
   ctgccgagcg gcggcaagtg cctgctgctg gactgcagac cgttcctggc gcacagcgcg
   420
   ggctacatcc taggttcggt caacgtgcgc tgtaacacca tcgtgcggcg gcgggctaag
   480
   ggctccgtga gcctggagca gatcctgccc gccgaggagg aggtacgcgc ccgcttgcgc
   540
   tccggcctct actcggcggt catcgtctac gacgagcgca gcccgcgcgc cgagagcctc
   600
   cgcgaggaca gcaccgtgtc gctggtggtg caggcgctgc gccgcaacgc cgagcgcacc
   660
   gacatctgcc tgctcaaagg cggctatgag aggttttcct ccgagtaccc agaattctgt
   720
   tctaaaacca aggccctggc agccatccca cccccggttc cccccagcgc cacagagccc
   780
   ttggacctgg gctgcagctc ctgtgggacc ccactacacg accagggggg tcctgtggag
   840
   atccttccct tcctctacct cggcagtgcc taccatgctg cccggagaga catgctggac
   900
   gccctgggca tcacggctct gttgaatgtc tcctcggact gcccaaacca ctttgaagga
   960
   cactatcagt acaagtgcat cccagtggaa gataaccaca aggccgacat cagctcctgg
   1020
   ttcatggaag ccatagagta catcgatgcc gtgaaggact gccgtgggcg cgtgctggtg
   1080
   cactgccagg cgggcatctc gcggtcggcc accatctgcc tggcctacct gatgatgaag
   1140
   aaacgggtga ggctggagga ggccttcgag ttcgttaagc agcgccgcag catcatctcg
   1200
   cccaacttca gcttcatggg gcagctgctg cagttcgagt cccaggtgct ggccacgtcc
   1260
   tgtgctgcgg aggctgctag cccctcggga cccctgcggg agcggggcaa gacccccgcc
   1320
   acccccacct cgcagttcgt cttcagcttt ccggtctccg tgggcgtgca ctcggccccc
   1380
   agcagcctgc cctacctgca cagccccatc accacctctc ccagctgtta gagccgccct
   1440
   gggggcccca gaaccagagc tggctcccag caagggtagg acgggccgca tgcggcagaa
   1500
   agttgggact gagcagctgg gagcaggcga ccgagctcct tccccatcat ttctccttgg
   1560
   ccaacgacga ggccagccag aatggcaata aggactccga atacataata aaagcaaaca
   1620
   gaacactcca acttagagca ataaccggtg ccgcagcagc cagggaagac cttggtttgg
   1680
   tttatgtgtc agtttcactt ttccgataga aatttcttac ctcatttttt taagcagtaa
   1740
   ggcttgaagt gatgaaaccc acagatccta gcaaatgtgc ccaaccagct ttactaaagg
   1800
   gggaggaagg gagggcaaag ggatgagaag acaagtttcc cagaagtgcc tggttctggg
   1860
   tacttgtccc tttgttgtcg ttgttgtagt taaaggaatt tcatttttaa aagaaatctt
   1920
   cgaaggtgtg gttttcattt ctcagtcacc aacagatgaa taattatgct taataataaa
   1980
   gtatttatta agactttctt cagagtatga aagtacaaaa agtctagtta cagtggattt
   2040
   agaatatatt tatgttgatg tcaaacagct gagcaccgta gcatgcagat gtcaaggcag
   2100
   ttaggaagta aatggtgtct tgtagatatg tgcaaggtag catgatgagc aacttgagtt
   2160
   tgttgccact gagaagcagg cgggttgggt gggaggagga agaaagggaa gaattaggtt
   2220
   tgaattgctt tttaaaaaaa aaagaaaaga aaaagacagc atctcactat gttgccaagg
   2280
   ctcatcttga gaagcaggcg ggttgggtgg gaggaggaag aaagggaaga attaggtttg
   2340
   aattgctttt ttaaaaaaaa a
   2361

<210> 63
<211> 1849
<212> DNA
<213> Homo sapiens

<400> 63
ggcacaaggg gaagaagagg ctctcccgcg ggagcccttg aggaccaagt ttgcggccac
60

ttctgcaggc gtcccttctt agctctcgcc cgcccctttc tgcagcctag gcggcccggg
120

ttctcttctc ttcctcgcgc gcccagccgc ctcggttccc ggcgaccatg gtgacgatgg
180

aggagctgcg ggagatggac tgcagtgtgc tcaaaaggct gatgaaccgg gacgagaatg
240

gcggcggcgc gggcggcagc ggcagccacg gcaccctggg gctgccgagc ggcggcaagt
300

gcctgctgct ggactgcaga ccgttcctgg cgcacagcgc gggctacatc ctaggttcgg
360

tcaacgtgcg ctgtaacacc atcgtgcggc ggcgggctaa gggctccgtg agcctggagc
420

agatcctgcc cgccgaggag gaggtacgcg cccgcttgcg ctccggcctc tactcggcgg
480

tcatcgtcta cgacgagcgc agcccgcgcg ccgagagcct ccgcgaggac agcaccgtgt
540

cgctggtggt gcaggcgctg cgccgcaacg ccgagcgcac cgacatctgc ctgctcaaag
600

gcggctatga gaggttttcc tccgagtacc cagaattctg ttctaaaacc aaggccctgg
660

cagccatccc acccccggtt ccccccagcg ccacagagcc cttggacctg ggctgcagct
720

cctgtgggac cccactacac gaccaggggg gtcctgtgga gatccttccc ttcctctacc
780

tcggcagtgc ctaccatgct gcccggagag acatgctgga cgccctgggc atcacggctc
840

tgttgaatgt ctcctcggac tgcccaaacc actttgaagg acactatcag tacaagtgca
900

tcccagtgga agataaccac aaggccgaca tcagctcctg gttcatggaa gccatagagt
960

acatcgatgc cgtgaaggac tgccgtgggc gcgtgctggt gcactgccag gcgggcatct
1020

cgcggtcggc caccatctgc ctggcctacc tgatgatgaa gaaacgggtg aggctggagg

1080

aggccttcga gttcgttaag cagcgccgca gcatcatctc gcccaacttc agcttcatgg
1140

ggcagctgct gcagttcgag tcccaggtgc tggccacgtc ctgtgctgcg gaggctgcta
1200

gcccctcggg acccctgcgg gagcggggca agacccccgc cacccccacc tcgcagttcg
1260

tcttcagctt tccggtctcc gtgggcgtgc actcggcccc cagcagcctg ccctacctgc
1320

acagccccat caccacctct cccagctgtt agagccgccc tgggggcccc agaaccagag
1380

ctggctccca gcaagggtag gacgggccgc atgcgggcag aaagttggga ctgagcagct
1440

gggagcaggc gaccgagctc cttccccatc atttctcctt ggccaacgac gaggccagcc
1500

agaatggcaa taaggactcc gaatacataa taaaagcaaa cagaacactc caacttagag
1560

caataacggc tgccgcagca gccagggaag accttggttt ggtttatgtg tcagtttcac
1620

ttttccgata gaaatttctt acctcatttt tttaagcagt aaggcttgaa gtgatgaaac
1680

ccacagatcc tagcaaatgt gcccaaccag ctttactaaa gggggaggaa gggagggcaa
1740

agggatgaga agacaagttt cccagaagtg cctggttctg tgtacttgtc cctttgttgt
1800

cgttgttgta gttaaaggaa tttcattttt taaaaaaaaa aaaaaaaaa
1849


<210> 64
<211> 2470
<212> DNA
<213> Homo sapiens

<400> 64
atggacgatc aatccaggat gctgcagact ctggccgggg tgaacctggc tggccactcg
60

gtgcaggggg gcatggccct gccgcctccc ccgcacggcc acgaaggggc ggacggcgac
120

ggcaggaagc aggacatcgg cgacatcctc caccagatca tgaccatcac cgaccagagc
180

ttggacgagg cgcaagcaaa gaaacatgcc ctgaactgtc acagaatgaa accagcgctc
240

ttcagcgtcc tgtgtgagat caaagagaaa acaggtctca gcatcagagg agcccaggag
300

gaggaccctc ccgatcccca gctaatgaga ctggacaata tgcttttggc agaaggggtt
360

tcaggtcctg agaaaggtgg gggatcggcg gcagcagctg cagccgcggc agcctctgga
420

ggttcttcag ataactctat tgaacactca gattacagag ccaaattgac ccagatcaga
480

caaatctatc acacagaact ggagaaatat gaacaggcat gtaatgaatt tactacacat
540

gtgatgaacc ttctccgaga acagagtaga acacgtccca tttctccaaa agagattgaa
600

agaatggtgg gcatcatcca tcgaaaattt agttccattc agatgcagct caaacaaagc
660

acttgtgaag cagttatgat tttaagatca aggttccttg atgccagacg gaaaaggcgt
720

aacttcagta aacaggccac agaaatcttg aatgaatatt tttactcaca cctcagcaac
780

ccctacccca gtgaagaagc caaagaggag ctggccaaga aatgcagcat cacagtgtca
840

caggtatcca attggtttgg caacaaacga atcaggtaca agaagaacat tggcaagttt
900

caggaagaag ccaacctcta tgctgcaaag acggccgtga cagctgcaca cgcagtagca
960

gcagctgtgc agaacaacca gaccaattcg cccaccacac caaattccgg ttcttctggt
1020

tcttttaacc tcccaaattc tggggacatg ttcatgaaca tgcagagtct gaatggggat
1080

tcttaccaag ggtcccaagt cggagccaat gtgcaatcac aggtggatac cctccgtcat
1140

gttatcaatc agacgggagg ctacagtgat ggccttggag gaaattcact gtacagtcca
1200

cataatttaa atgctaatgg aggctggcag gacgcaacaa ctccatcttc tgtgacttct
1260

cctacagaag gcccaggaag tgtgcactcg gatacctcta actaatctct ggccacactt
1320

ttcctgagct acatgccttg ataagtgcat tcagagcaat aggaggaaaa ggaaagcgtt
1380

tttgtagccc accatctaca gctttactgt aaaaccttgt cttattcgag aacttggtaa
1440

atctgttttt aaggaatcat aatcatttgt atttatactt aaaaacacac aatgttaaaa
1500

aaaataaagc actttatcca attaggccaa gatttaacat tgttgacagt cctgtagcta
1560

ttttatcata atttattatc aatattttac attaatggtt tcacagttgc caattacttg
1620

gccttaaggg taaaaagtac aatatacact aaacctcaac cgttaaagca gatgcaaaaa
1680

ttcacctcac ctaaattgaa cttcttgcat atttccatta ctgacttgga ttgtctttct
1740

ttcatatcac taatggagtt ggaataaaga gctgtttgcc tatccctgtt aatgatggtt
1800

gtgtttaaga atcttcctcg tcacgtttgt gttcagatct cttatgttat aattagatca
1860

gagactggta gcatcgtttc tctctctgaa agcaccagtg cccagagtct gctcggtaat
1920

aaaattatgg atccagattg ttctgagaga cgaagatact tgctgctgat agaggtgaaa
1980

acgagattga tccgtctggg gttttacggt gtgcactggg tgctgcacag acttgtcaag
2040

gtttgctacg tcctctgggc atctgcaaaa ggccctgctc tctggagtgt tgtatatagt
2100

gtagcaaaag agtatttata catcccacca atcaaaacac agctttatta cctcatgcga
2160

actcatacaa accaatagaa tttcaacatg ttctgtagct tagagtgctc acttactacc
2220

tctgaacaat actcacgctg tagtttgtct ctttcttatc tttttgcatc ttgtaattaa
2280

ctctttgttt cccttcataa aatgtaatgt acattgtaat cttttaaaag aaaaatcagg
2340

gttgcacttg caacttttaa aaaaccgagt gtggaaacat tgggtcttaa ttcaacacag
2400

gatcggtaaa actgttgtaa atactgagaa acattttgaa tgttcttcaa tcttattact
2460

aatccatgca
2470


<210>  65
<211>  1594
<212>  DNA
<213>  Homo sapiens

<400>  65
gcccgccggt ccacgccgcg caccgctccg agggccagcg ccacccgctc cgcagccggc
60

accatgcgcg agatcgtgca catccaggcg ggccagtgcg gcaaccagat cggcgccaag
120

ttttgggagg tcatcagcga tgagcatggg atcgacccca caggcagtta ccatggagac
180

agtgacttgc agctggagag aatcaacgtg tactacaatg aggctgctgg taacaaatat
240

gtacctcggg ccatcctggt ggatctggag cctggcacca tggactctgt caggtctgga
300

cccttcggcc agatcttcag accagacaac ttcgtgttcg gccagagtgg agccgggaat
360

aactgggcca agggccacta cacagaggga gccgagctgg tcgactcggt cctggatgtg
420

gtgaggaagg agtcagagag ctgtgactgt ctccagggct tccagctgac ccactctctg
480

gggggcggca cggggtccgg gatgggcacc ctgctcatca gcaagatccg ggaagagtac
540

ccagaccgca tcatgaacac cttcagcgtc atgccctcac ccaaggtgtc agacacggtg
600

gtggagccct acaacgccac cctctcggtc caccagctgg tggaaaacac agatgaaacc
660

tactccattg ataacgaggc cctgtatgac atctgcttcc gcaccctgaa gctgaccacc
720

cccacctacg gggacctcaa ccacctggtg tcggccacca tgagcggggt caccacctgc
780

ctgcgcttcc cgggccagct gaacgcagac ctgcgcaagc tggcggtgaa catggtgccc
840

ttccctcgcc tgcacttctt catgcccggc ttcgcgcccc tgaccagccg gggcagccag
900

cagtaccggg cgctcacggt gcccgagctc acccagcaga tgttcgactc caagaacatg
960

atggccgcct gcgacccgcg ccacggccgc tacctgacgg tggctgccat cttccggggc
1020

cgcatgtcca tgaaggaggt ggacgagcag atgctcaacg tgcagaacaa gaacagcagc
1080

tacttcgtgg agtggatccc caacaacgtg aagacggccg tgtgcgacat cccgccccgc
1140

ggcctgaaga tgtcggccac cttcatcggc aacagcacgg ccatccagga gctgttcaag
1200

cgcatctccg agcagttcac ggccatgttc cggcgcaagg ccttcctgca ctggtacacg
1260

ggcgagggca tggacgagat ggagttcacc gaggccgaga gcaacatgaa cgacctggtg
1320

tccgagtacc agcagtacca ggacgccacg gccgacgaac aaggggagtt cgaggaggag
1380

gagggcgagg acgaggctta aaaacttctc agatcaatcg tgcatcctta gtgaacttct
1440

gttgtcctca agcatggtct ttctacttgt aaactatggt gctcagtttt gcctctgtta
1500

gaaattcaca ctgttgatgt aatgatgtgg aactcctcta aaaattacag tattgtctgt
1560

gaaggtatct atactaataa aaaagcatgt gtag
1594

<210> 66

<211>  439
<212>  DNA
<213>  Homo sapiens

<400>  66
   ggtgggtctg aatctagcac catgacggaa ctagagacag ccatgggcat gatcatagac
   60
   gtcttttccc gatattcggg cagcgagggc agcacgcaga ccctgaccaa gggggagctc
   120
   aaggtgctga tggagaagga gctaccaggc ttcctgcaga gtggaaaaga caaggatgcc
   180
   gtggataaat tgctcaagga cctggacgcc aatggagatg cccaggtgga cttcagtgag
   240
   ttcatcgtgt tcgtggctgc aatcacgtct gcctgtcaca agtactttga gaaggcagga
   300
   ctcaaatgat gccctggaga tgtcacagat tcctgcagag ccatggtccc aggcttccca
   360
   aaagtgtttg ttggcaatta ttcccctagg ctgagcctgc tcatgtacct ctgattaata
   420
   aatgcttatg aaaaaaaaa
   439


<210>  67
<211>  1323
<212>  DNA
<213>  Homo sapiens

<400>  67
ccattgtggt ggaattcccc tgcgctccct aacatgccca accccagctg cacctcctct
60

ccctaccccc tccctgagga aattaggaac ctgttggcag atgttgaaac atttgtagca
120

gatatactga aaggagaaaa tttatccaag aaagcaaagg aaaagagaga atcccttatt
180

aagaagataa aagatgtaaa gtctatctat cttcaggaat ttcaagacaa aggtgatgca
240

gaagatgggg aagaatatga tgaccctttt gctgggcctc cagacactat ttcattagcc
300

tcagaacgat atgataaaga cgatgaagcc ccctctgatg gagcccagtt tcctccaatt
360

gcagcacaag accttccttt tgttctaaag gctggctacc ttgaaaaacg cagaaaagat
420

cacagctttc tgggatttga atggcagaaa cggtggtgtg ctctcagtaa aacggtattc
480

tattattatg gaagtgataa agacaaacaa cagaaaggtg aatttgcaat agatggctac
540

agtgtcagaa tgaataacac tctaagaaag gatggaaaga aagattgctg ttttgaaatc
600

tctgctcctg ataaacgtat atatcagttt acagcatctt ctcccaaaga tgctgaagaa
660

tgggtacagc agctgaaatt tgtattgcaa gatatggaat ctgatattat tcctgaggat
720

tatgatgaga gaggagaatt atatgatgat gttgatcatc ctctaccaat aagcaatcca
780

ctaacaagca gtcaaccaat agatgatgaa atttatgaag aacttccaga agaagaagag
840

gacagtgctc cagtgaaagt ggaagaacaa aggaagatga gtcaggatag tgtccatcac
900

acctcagggg ataagagcac tgattatgct aatttttacc agggattgtg ggattgtact
960

ggagcttttt ctgatgagtt gtcatttaag cgtggtgatg tgatttacat tcttagcaag
1020

gaatacaata gatatggctg gtgggtagga gaaatgaagg gagccattgg cttggtgcct
1080

aaagcctaca taatggagat gtatgatatt tgagagtcct ggaaaaggaa aattcttctg
1140

cttgtctgca aatgctttgg atttagaagc gtcatgaaag cacgagtgac agctcctaac
1200

ctctccttgt tttattaaac attacttatc tttgactgtt attttatgca gtcgctcatt
1260

aaaatattct ctgatgtgaa attaatgagg gtattatgta atagatgcac cagtaggtat
1320

act
1323


<210>  68
<211>  2248
<212>  DNA
<213>  Homo sapiens


<220>
<221>  misc_feature
<222>  (2084)..(2085)

<220>
<221>  misc_feature
<222>  (2084)..(2084)
<223>  n is a, c, g, or t

<400>  68
cggcacgagg tgacttcctt gttgtgagcc ccggcccggc agtgtcccga ctcgtagccc
60

cgctgttctt aatccgggcc gctagcctga gtctaggtcg cagccgcagc cccaccccgt
120

cggtcacctt ttcagcgcag gtcctttccc cgcacgccct gcgctcccta acatgcccaa
180

ccccagcagc acctcctctc cctaccccct ccctgaggaa attaggaacc tgttggcaga

240

tgttgaaaca tttgtagcag atatactgaa aggagaaaat ttatccaaga aagcaaagga
300

aaagagagaa tcccttatta agaagataaa agatgtaaag tctatctatc ttcaggaatt
360

tcaagacaaa ggtgatgcag aagatgggga agaatatgat gacccttttg ctgggcctcc
420

agacactatt tcattagcct cagaacgata tgataaagac gatgaagccc cctctgatgg
480

agcccagttt cctccaattg cagcacaaga ccttcctttt gttctaaagg ctggctacct
540

tgaaaaacgc agaaaagatc acagctttct gggatttgaa tggcagaaac ggtggtgtgc
600

tctcagtaaa acggtattct attattatgg aagtgataaa gacaaacaac agaaaggtga
660

atttgcaata gatggctaca gtgtcagaat gaataacact ctaagaaagg atggaaagaa
720

agattgctgt tttgaaatct ctgctcctga taaacgtata tatcagttta cagcatcttc
780

tcccaaagat gctgaagaat gggtacagca gctgaaattt gtattgcaag atatggaatc
840

tgatattatt cctgaggatt atgatgagag aggagaatta tatgatgatg ttgatcatcc
900

tctaccaata agcaatccac taacaagcag tcaaccaata gatgatgaaa tttatgaaga
960

acttccagaa gaagaagagg acagtgctcc agtgaaagtg gaagaacaaa ggaagatgag
1020

tcaggatagt gtccatcaca cctcagggga taagagcact gattatgcta atttttacca
1080

gggattgtgg gattgtactg gagctttttc tgatgagttg tcatttaagc gtggtgatgt
1140

gatttacatt cttagcaagg aatacaatag atatggctgg tgggtaggag aaatgaaggg
1200

agccattggc ttggtgccta aagcctacat aatggagatg tatgatattt gagagtcctg
1260

gaaaaggaaa attcttctgc ttgtctgcaa atgctttgga tttagaagcg tcatgaaagc
1320

acgagtgaca gctcctaacc tctccttgtt ttattaaaca ttacttatct ttgactgtta
1380

ttttatgcag tcgctcatta aaatattcct ctgatgtgaa attaaatgaa ggatattaat
1440

gtaaattaga tgcaaccagt taagttatac ctgttgctat tttgcaaaga aataattata
1500

gtttttattt acccatttga tttgtgtgaa gaattcatca ctattttata cgtaacatat

1560

agtctactat agcatagtat gctactattg ctacttctgg tgtgatttgt aatgtttctt
1620

aatcattgga catcaattat ttttagagag taatgtataa tttcatagca ttttaaattt
1680

acgtgtatca tgcgagtttt ttttggtaga tgctgaagaa tgtggttgct aaacaaagaa
1740

tgctaaagaa tgttcaaact ttatagataa ctttattgtt attatttatt ttcacacatt
1800

taattcctat taagtacagc cgcaagaaag aaaaaatgat gaagttgcaa atggcagtgt
1860

gctgtcacct gcaacagaag tgcgtaccag aagtatgact catgcaaagc attttaccgt
1920

acaaatatcc tggtgcgatg atgggcctgg caacattttc tactgtactt ttgtttaaat
1980

ttaatgaaac aaaaaattcc taagaaatac caccctacca ctaacaaaat ggtataaaga
2040

atctcccagc caggccaaca tggtgaaacc ctgtctctac taanaattag ccaagtgtgg
2100

taatgttcac ctgcagtccc agctacttgg gaggccaagg cacgagaatt gcttgaacaa
2160

ggtaggcaga ggttgcagtg agccaagatc gcgccactgc actccagcct gggcgacaga
2220

gtgagactcc atctcagaaa aaaaaaaa
2248


<210> 69
<211> 417
<212> DNA
<213> Homo sapiens

<400> 69
acctcccaac caagccctcc agcaaggatt caggagtgcc cctcgggcct cgccatgagg
60

ctcttcctgt cgctcccggt cctggtggtg gttctgtcga tcgtcttgga aggcccagcc
120

ccagcccagg ggaccccaga cgtctccagt gccttggata agctgaagga gtttggaaac
180

acactggagg acaaggctcg ggaactcatc agccgcatca aacagagtga actttctgcc
240

aagatgcggg agtggttttc agagacattt cagaaagtga aggagaaact caagattgac
300

tcatgaggac ctgaagggtg acatccagga ggggcctctg aaatttccca caccccagcg
360

cctgtgctga ggactcccgc catgtggccc caggtgccac caataaaaat cctaccg
417

<210> 70
<211> 2753
<212> DNA
<213> Homo sapiens

<400> 70
gtcgggggca gcagcaagat gcgaagcgag ccgtacagat cccgggctct ccgaacgcaa
60

cttcgccctg cttgagcgag gctgcggttt ccgaggccct ctccagccaa ggaaaagcta
120

cacaaaaagc ctggatcact catcgaacca cccctgaagc cagtgaaggc tctctcgcct
180

cgccctctag cgttcgtctg gagtagcgcc accccggctt cctggggaca cagggttggc
240

accatggggc ccaccagcgt cccgctggtc aaggcccacc gcagctcggt ctctgactac
300

gtcaactatg atatcatcgt ccggcattac aactacacgg gaaagctgaa tatcagcgcg
360

gacaaggaga acagcattaa actgacctcg gtggtgttca ttctcatctg ctgctttatc
420

atcctggaga acatctttgt cttgctgacc atttggaaaa ccaagaaatt ccaccgaccc
480

atgtactatt ttattggcaa tctggccctc tcagacctgt tggcaggagt agcctacaca
540

gctaacctgc tcttgtctgg ggccaccacc tacaagctca ctcccgccca gtggtttctg
600

cgggaaggga gtatgtttgt ggccctgtca gcctccgtgt tcagtctcct cgccatcgcc
660

attgagcgct atatcacaat gctgaaaatg aaactccaca acgggagcaa taacttccgc
720

ctcttcctgc taatcagcgc ctgctgggtc atctccctca tcctgggtgg cctgcctatc
780

atgggctgga actgcatcag tgcgctgtcc agctgctcca ccgtgctgcc gctctaccac
840

aagcactata tcctcttctg caccacggtc ttcactctgc ttctgctctc catcgtcatt
900

ctgtactgca gaatctactc cttggtcagg actcggagcc gccgcctgac gttccgcaag
960

aacatttcca aggccagccg cagctctgag aagtcgctgg cgctgctcaa gaccgtaatt
1020

atcgtcctga gcgtcttcat cgcctgctgg gcaccgctct tcatcctgct cctgctggat
1080

gtgggctgca aggtgaagac ctgtgacatc ctcttcagag cggagtactt cctggtgtta
1140

gctgtgctca actccggcac caaccccatc atttacactc tgaccaacaa ggagatgcgt
1200

cgggccttca tccggatcat gtcctgctgc aagtgcccga gcggagactc tgctggcaaa
1260

ttcaagcgac ccatcatcgc cggcatggaa ttcagccgca gcaaatcgga caattcctcc
1320

cacccccaga aagacgaagg ggacaaccca gagaccatta tgtcttctgg aaacgtcaac
1380

tcttcttcct agaactggaa gctgtccacc caccggaagc gctctttact tggtcgctgg
1440

ccaccccagt gtttggaaaa aaatctctgg gcttcgactg ctgccaggga ggagctgctg
1500

caagccagag ggaggaaggg ggagaatacg aacagcctgg tggtgtcggg tgttggtggg
1560

tagagttagt tcctgtgaac aatgcactgg gaagggtgga gatcaggtcc cggcctggaa
1620

tatatattct acccccctgg agctttgatt ttgcactgag ccaaaggtct agcattgtca
1680

agctcctaaa gggttcattt ggcccctcct caaagactaa tgtccccatg tgaaagcgtc
1740

tctttgtctg gagctttgag gagatgtttt ccttcacttt agtttcaaac ccaagtgagt
1800

gtgtgcactt ctgcttcttt agggatgccc tgtacatccc acaccccacc ctcccttccc
1860

ttcatacccc tcctcaacgt tcttttactt tatactttaa ctacctgaga gttatcagag
1920

ctggggttgt ggaatgatcg atcatctata gcaaataggc tatgttgagt acgtaggctg
1980

tgggaagatg aagatggttt ggaggtgtaa aacaatgtcc ttcgctgagg ccaaagtttc
2040

catgtaagcg ggatccgttt tttggaattt ggttgaagtc actttgattt ctttaaaaaa
2100

catcttttca atgaaatgtg ttaccatttc atatccattg aagccgaaat ctgcataagg
2160

aagcccactt tatctaaatg atattagcca ggatccttgg tgtcctagga gaaacagaca
2220

agcaaaacaa agtgaaaacc gaatggatta acttttgcaa accaagggag atttcttagc
2280

aaatgagtct aacaaatatg acatccgtct ttcccacttt tgttgatgtt tatttcagaa
2340

tcttgtgtga ttcatttcaa gcaacaacat gttgtatttt gttgtgttaa aagtactttt
2400

cttgattttt gaatgtattt gtttcaggaa gaagtcattt tatggatttt tctaacccgt
2460

gttaactttt ctagaatcca ccctcttgtg cccttaagca ttactttaac tggtagggaa
2520

cgccagaact tttaagtcca gctattcatt agatagtaat tgaagatatg tataaatatt
2580

acaaagaata aaaatatatt actgtctctt tagtatggtt ttcagtgcaa ttaaaccgag
2640

agatgtcttg tttttttaaa aagaatagta tttaataggt ttctgacttt tgtggatcat
2700

tttgcacata gctttatcaa cttttaaaca ttaataaact gattttttta aag
2753

<210> 71
<211> 2032
<212> DNA
<213> Homo sapiens

<400> 71
tccatattgt gcttccacca ctgccaataa caaaataact agcaaccatg aagtgggtgg
60

aatcaatttt tttaattttc ctactaaatt ttactgaatc cagaacactg catagaaatg
120

aatatggaat agcttccata ttggattctt accaatgtac tgcagagata agtttagctg
180

acctggctac catatttttt gcccagtttg ttcaagaagc cacttacaag gaagtaagca
240

aaatggtgaa agatgcattg actgcaattg agaaacccac tggagatgaa cagtcttcag
300

ggtgtttaga aaaccagcta cctgcctttc tggaagaact ttgccatgag aaagaaattt
360

tggagaagta cggacattca gactgctgca gccaaagtga agagggaaga cataactgtt
420

ttcttgcaca caaaaagccc actccagcat cgatcccact tttccaagtt ccagaacctg
480

tcacaagctg tgaagcatat gaagaagaca gggagacatt catgaacaaa ttcatttatg
540

agatagcaag aaggcatccc ttcctgtatg cacctacaat tcttctttgg gctgctcgct
600

atgacaaaat aattccatct tgctgcaaag ctgaaaatgc agttgaatgc ttccaaacaa
660

aggcagcaac agttacaaaa gaattaagag aaagcagctt gttaaatcaa catgcatgtg
720

cagtaatgaa aaattttggg acccgaactt tccaagccat aactgttact aaactgagtc
780

agaagtttac caaagttaat tttactgaaa tccagaaact agtcctggat gtggcccatg
840

tacatgagca ctgttgcaga ggagatgtgc tggattgtct gcaggatggg gaaaaaatca
900

tgtcctacat atgttctcaa caagacactc tgtcaaacaa aataacagaa tgctgcaaac

960

tgaccacgct ggaacgtggt caatgtataa ttcatgcaga aaatgatgaa aaacctgaag
1020

gtctatctcc aaatctaaac aggtttttag gagatagaga ttttaaccaa ttttcttcag
1080

gggaaaaaaa tatcttcttg gcaagttttg ttcatgaata ttcaagaaga catcctcagc
1140

ttgctgtctc agtaattcta agagttgcta aaggatacca ggagttattg gagaagtgtt
1200

tccagactga aaaccctctt gaatgccaag ataaaggaga agaagaatta cagaaataca
1260

tccaggagag ccaagcattg gcaaagcgaa gctgcggcct cttccagaaa ctaggagaat
1320

attacttaca aaatgcgttt ctcgttgctt acacaaagaa agccccccag ctgacctcgt
1380

cggagctgat ggccatcacc agaaaaatgg cagccacagc agccacttgt tgccaactca
1440

gtgaggacaa actattggcc tgtggcgagg gagcggctga cattattatc ggacacttat
1500

gtatcagaca tgaaatgact ccagtaaacc ctggtgttgg ccagtgctgc acttcttcat
1560

atgccaacag gaggccatgc ttcagcagct tggtggtgga tgaaacatat gtccctcctg
1620

cattctctga tgacaagttc attttccata aggatctgtg ccaagctcag ggtgtagcgc
1680

tgcaaacgat gaagcaagag tttctcatta accttgtgaa gcaaaagcca caaataacag
1740

aggaacaact tgaggctgtc attgcagatt tctcaggcct gttggagaaa tgctgccaag
1800

gccaggaaca ggaagtctgc tttgctgaag agggacaaaa actgatttca aaaactcgtg
1860

ctgctttggg agtttaaatt acttcagggg aagagaagac aaaacgagtc tttcattcgg
1920

tgtgaacttt tctctttaat tttaactgat ttaacacttt ttgtgaatta atgaaatgat
1980

aaagactttt atgtgagatt tccttatcac agaaataaaa tatctccaaa tg
2032


<210>  72
<211>  679
<212>  DNA
<213>  Homo sapiens

<400>  72
gggtccccaa ggaacatggc tgggagccgt gaggtggtgg ccatggactg cgagatggtg
60

gggctggggc cccaccggga gagtggcctg gctcgttgca gcctcgtgaa cgtccacggt
120

gctgtgctgt acgacaagtt catccggcct gagggagaga tcaccgatta cagaacccgg
180

gtcagcgggg tcacccctca gcacatggtg ggggccacac catttgccgt ggccaggcta
240

gagatcctgc agctcctgaa aggcaagctg gtggtgggtc atgacctgaa gcacgacttc
300

caggcactga aagaggacat gagcggctac acaatctacg acacgtccac tgacaggctg
360

ttgtggcgtg aggccaagct ggaccactgc aggcgtgtct ccctgcgggt gctgagtgag
420

cgcctcctgc acaagagcat ccagaacagc ctgcttggac acagctcggt ggaagatgcg
480

agggcaacga tggagctcta tcaaatctcc cagagaatcc gagcccgccg agggctgccc
540

cgcctggctg tgtcagactg aagccccatc cagcccgttc cgcagggact agaggctttc
600

ggctttttgg gacagcaact accttgcttt tggaaaatac atttttaata gtaaagtggc
660

tctatatttt ctctacgcc
679

<210> 73
<211> 2088
<212> DNA
<213> Homo sapiens

<400> 73
   catgaaggac cgaacccagg agctccgcac ggccaaggac agcgatgatg atgatgatgt
   60
   cgctgtcacc gtggaccgag accgcttcat ggatgagttc tttgagcagg tggaggagat
   120
   tcgaggcttc attgacaaga tcgcagagaa cgtggaggag gtgaagcgga agcacagtgc
   180
   catcctggca tcccccaacc ccgatgagaa gacgaaggag gagctggaag aactcatgtc
   240
   cgacataaag aagacagcaa acaaagttcg ttccaagtta aagagcatcg agcagtccat
   300
   cgagcaagag gaaggcctga accgctcctc cgctgacctg aggatccgga agacacagca
   360
   ctccacgctg tccagaaagt ttgtggaggt catgtcggag tacaacgcca cgcagtccga
   420
   ctaccgcgag cgctgcaaag gccgcatcca gaggcagctg gagatcaccg gcaggaccac
   480
   gaccagtgag gagctggagg acatgctgga gagtgggaac cccgccatct ttgcctctgg
   540
   gatcatcatg gactccagca tctcgaagca ggctctgagc gagattgaga cgcggcacag
   600
   tgagatcatc aagctggaga acagcatccg tgagctacac gacatgttca tggacatggc
   660
   catgctcgtg gagagccagg gagagatgat tgacaggatc gagtacaatg tggaacacgc
   720
   ggtagactat gtggagaggg ccgtgtctga caccaagaag gccgtcaagt accagagcaa
   780
   ggcgcgccgg aagaaaatca tgatcatcat ctgctgtgtg atcctgggca tcgtcatcgc
   840
   ctccactgtt gggggcatct tcgcctagaa gccacccaaa ctgccactcc actccaggtg
   900
   ggccactcca aggaggccct ggctgctgcc acctggctgg gctgccctcc caacccccgc
   960
   ctctggctca gagcaccctc cctcccggcc cccatgctcc cttctctgcc atgggccctc
   1020
   cgtccccgcc ccgtgtcgtg tgcatgatct ctgtgagtgt gcgtctgtac gggaagaggc
   1080
   agagggaggc agccagcggg gcgtgatgca gtgtgcacag cgaggagcag acccaggcag
   1140
   ggccgccagg gtgacacagg ccacgcttcc ttgccttcag taactcggtg ggcccaggtt
   1200
   ctgctcttcc ctggggaccc taacctcgcc tccagctgac ctgccctgtc ctctccagct
   1260
   gtccccacaa gcagagccct gaggggtggg gaccagctgg ccacatggtg ctgcttttca
   1320
   ggttagggga gaggtgaccc tgagggacag cccagctctg agtctcagtc gctgatcact
   1380
   gccagggagg ctcaggctgc catggctcca ggctccctcc cctgcctagg ggcaaagtcc
   1440
   atcgggtcct gggcctcagc ttcccttccc acattcctcc ggccccagga gcaacccctt
   1500
   gggctaggtc tgaccccagg tgtccctctg gaaggggctg gctggtgccc tatttccagc
   1560
   caccccagca gctagggagg caaagcaggc tgcagtcagt ccctcaagcc agcgttgcat
   1620
   gtttgggatg gtggctcctg ttgtcttgcg ctctgggaag tcagatgtca tttcaggcct
   1680
   gcagtctcat cctgcccttg ccatcctccc atcgatgtgc cacgtgggtg tcacgtgtcc
   1740
   cagatgcagt attcggcagc cagccgggga gggctacctc ctcctcctca ccaccttggg
   1800
   gcttctcatg ggaaatgtgc cccgccccca ggaccctctc ccttgtggac aggcagggag
   1860
   atgcatgcga gtgcatgcag caggggatgg ggccgtgtcc gtgtgcccca ccctccctcg
   1920
   gctttactcc tgcccagtga ctgtgaccac tgtccgtgtt gccttcttga acagcgattc
   1980
   cccccaaccc cttcaccaaa ggtcttggta caaccagctg cccattttgt gaaattttta
   2040
   tgtagaataa acatttgtat ctgtaaaaaa aaaaaaaaaa aagcgttc
   2088

<210> 74
<211> 1056
<212> DNA
<213> Homo sapiens

<400> 74
gtctgcgcgg agtctgagcg gcgctcgtcc cgtcccaagg ccgacgccag cacgccgtca
60

tggcccccgc agcggcgacg gggggcagca ccctgcccag tggcttctcg gtcttcacca
120

ccttgcccga cttgctcttc atctttgagt ttatcttcgg gggcctggtg tggatcctgg
180

tggcctcctc cctggtgccc tggcccctgg tccagggctg ggtgatgttc gtgtctgtgt
240

tctgcttcgt ggccaccacc accttgatca tcctgtacat aattggagcc cacggtggag
300

agacttcctg ggtcaccttg gacgcagcct accactgcac cgctgccctc ttttacctca
360

gcgcctcagt cctggaggcc ctggccacca tcacgatgca agacggcttc acctacaggc
420

actaccatga aaacattgct gccgtggtgt tctcctacat agccactctg ctctacgtgg
480

tccatgcggt gttctcttta atcagatgga agtcttcata aagccgcagt agaacttgag
540

ctgaaaaccc agatggtgtt aactggccgc cccactttcc ggcataactt tttagaaaac
600

agaaatgccc ttgatggtgg aaaaaagaaa acaaccaccc ccccactgcc caaaaaaaaa
660

agccctgccc tgttgctcgt gggtgctgtg tttactctcc cgtgtgcctt cgcgtccggg
720

ttgggagctt gctgtgtcta acctccaact gctgtgctgt ctgctagggt cacctcctgt
780

ttgtgaaagg ggaccttctt gttcgggggt gggaagtggc gaccgtgacc tgagaaggaa
840

agaaagatcc tctgctgacc cctggagcag ctctcgagaa ctacctgttg gtattgtcca
900

caagctctcc cgagcgcccc atcttgtgcc atgttttaag tcttcatgga tgttctgcat

960

gtcatgggga ctaaaactca cccaacagat ctttccagag gtccatggtg gaagacgata
1020

accctgtgaa atactttata aaatgtctta atgttc
1056

<210> 75
<211> 581
<212> DNA
<213> Homo sapiens

<400> 75
tctttttgga acaggtccaa tgatttcaga tcttctggaa aaggcttttt gtcatttgct
60

tctatttcca caacacttac atcagtgaaa ttgccatctg aatacatttg atcttttgaa
120

ttaaaattgt gccttggagg agtgtgaggt gaccactggg caatatgact ctttgaagga
180

tctggaacat taggccagat gtgtttttta attaggtctc gcttattaaa gcagaacagc
240

actcccagaa gagttgtcaa taggaatgct aatcaaacag gcacgactat ggcttcaatt
300

tctccttgag caaactttgg ggtagtaaaa gtgaattctg gaccatcctt cccaccttca
360

tctgtgtatg ctgccattcg taccatgtac aatgtgtcac tagtcaaaga ggacaatgta
420

tattctgtgt gggaagaatc cacattcaca gcagtttcat ttccaatgat ggttctataa
480

aatatattat aatttctgat aaactcattc tgaacatcaa catgaagttg catccactct
540

aagacagctt ctgctttccc tacatttatt gaccgaacag a
581

<210> 76
<211> 3085
<212> DNA
<213> Homo sapiens

<400> 76
gagcagccaa aaggcccgcg gagtcgcgct gggccgcccc ggcgcagctg aaccgggggc
60

cgcgcctgcc aggccgacgg gtctggccca gcctggcgcc aaggggttcg tgcgctgtgg
120

agacgcggag ggtcgaggcg gcgcggcctg agtgaaaccc aatggaaaaa gcatgacatt
180

tagaagtaga agacttagct tcaaatccct actccttcac ttactaattt tgtgatttgg
240

aaatatccgc gcaagatgtt gacgttgcag acttgggtag tgcaagcctt gtttattttc
300

ctcaccactg aatctacagg tgaacttcta gatccatgtg gttatatcag tcctgaatct
360

ccagttgtac aacttcattc taatttcact gcagtttgtg tgctaaagga aaaatgtatg
420

gattattttc atgtaaatgc taattacatt gtctggaaaa caaaccattt tactattcct
480

aaggagcaat atactatcat aaacagaaca gcatccagtg tcacctttac agatatagct
540

tcattaaata ttcagctcac ttgcaacatt cttacattcg gacagcttga acagaatgtt
600

tatggaatca caataatttc aggcttgcct ccagaaaaac ctaaaaattt gagttgcatt
660

gtgaacgagg ggaagaaaat gaggtgtgag tgggatggtg gaagggaaac acacttggag
720

acaaacttca ctttaaaatc tgaatgggca acacacaagt ttgctgattg caaagcaaaa
780

cgtgacaccc ccacctcatg cactgttgat tattctactg tgtattttgt caacattgaa
840

gtctgggtag aagcagagaa tgcccttggg aaggttacat cagatcatat caattttgat
900

cctgtatata aagtgaagcc caatccgcca cataatttat cagtgatcaa ctcagaggaa
960

ctgtctagta tcttaaaatt gacatggacc aacccaagta ttaagagtgt tataatacta
1020

aaatataaca ttcaatatag gaccaaagat gcctcaactt ggagccagat tcctcctgaa
1080

gacacagcat ccacccgatc ttcattcact gtccaagacc ttaaaccttt tacagaatat
1140

gtgtttagga ttcgctgtat gaaggaagat ggtaagggat actggagtga ctggagtgaa
1200

gaagcaagtg ggatcaccta tgaagataga ccatctaaag caccaagttt ctggtataaa
1260

atagatccat cccatactca aggctacaga actgtacaac tcgtgtggaa gacattgcct
1320

ccttttgaag ccaatggaaa aatcttggat tatgaagtga ctctcacaag atggaaatca
1380

catttacaaa attacacagt taatgccaca aaactgacag taaatctcac aaatgatcgc
1440

tatctagcaa ccctaacagt aagaaatctt gttggcaaat cagatgcagc tgttttaact
1500

atccctgcct gtgactttca agctactcac cctgtaatgg atcttaaagc attccccaaa
1560

gataacatgc tttgggtgga atggactact ccaagggaat ctgtaaagaa atatatactt
1620

gagtggtgtg tgttatcaga taaagcaccc tgtatcacag actggcaaca agaagatggt
1680

accgtgcatc gcacctattt aagagggaac ttagcagaga gcaaatgcta tttgataaca
1740

gttactccag tatatgctga tggaccagga agccctgaat ccataaaggc ataccttaaa
1800

caagctccac cttccaaagg acctactgtt cggacaaaaa aagtagggaa aaacgaagct
1860

gtcttagagt gggaccaact tcctgttgat gttcagaatg gatttatcag aaattatact
1920

atattttata gaaccatcat tggaaatgaa actgctgtga atgtggattc ttcccacaca
1980

gaatatacat tgtcctcttt gactagtgac acattgtaca tggtacgaat ggcagcatac
2040

acagatgaag gtgggaagga tggtccagaa ttcactttta ctaccccaaa gtttgctcaa
2100

ggagaaattg aagccatagt cgtgcctgtt tgcttagcat tcctattgac aactcttctg
2160

ggagtgctgt tctgctttaa taagcgagac ctaattaaaa aacacatctg gcctaatgtt
2220

ccagatcctt caaagagtca tattgcccag tggtcacctc acactcctcc aaggcacaat
2280

tttaattcaa aagatcaaat gtattcagat ggcaatttca ctgatgtaag tgttgtggaa
2340

atagaagcaa atgacaaaaa gccttttcca gaagatctga aatcattgga cctgttcaaa
2400

aaggaaaaaa ttaatactga aggacacagc agtggtattg gggggtcttc atgcatgtca
2460

tcttctaggc caagcatttc tagcagtgat gaaaatgaat cttcacaaaa cacttcgagc
2520

actgtccagt attctaccgt ggtacacagt ggctacagac accaagttcc gtcagtccaa
2580

gtcttctcaa gatccgagtc tacccagccc ttgttagatt cagaggagcg gccagaagat
2640

ctacaattag tagatcatgt agatggcggt gatggtattt tgcccaggca acagtacttc
2700

aaacagaact gcagtcagca tgaatccagt ccagatattt cacattttga aaggtcaaag
2760

caagtttcat cagtcaatga ggaagatttt gttagactta aacagcagat ttcagatcat
2820

atttcacaat cctgtggatc tgggcaaatg aaaatgtttc aggaagtttc tgcagcagat
2880

gcttttggtc caggtactga gggacaagta gaaagatttg aaacagttgg catggaggct
2940

gcgactgatg aaggcatgcc taaaagttac ttaccacaga ctgtacggca aggcggctac
3000

atgcctcagt gaaggactag tagttcctgc tacaacttca gcagtaccta taaagtaaag
3060

ctaaaatgat tttatctgtg aattc
3085

<210> 77
<211> 1637
<212> DNA
<213> Homo sapiens

<400> 77
gaggcgaacc ggagcgcggg gccgcggtcg ccccgaccag agccgggaga ccgcagcacc
60

cgcagccgcc cgcgagcgcg ccgaagacag cgcgcaggcg agagcgcgcg ggcgggggcg
120

cgcaggccct gcccgcccct tccgtcccca cccccctccg ccctttcctc tccccacctt
180

cctctcgcct cccgcgcccc cgcaccgggc gcccaccctg tcctcctcct gcgggagcgt
240

tgtccgtgtt ggcggccgca gcgggccggg ccggtccggc gggccggggg atggcgctgc
300

tggacctggc cttggaggga atggccgtct tcgggttcgt cctcttcttg gtgctgtggc
360

tgatgcattt catggctatc atctacaccc gattacacct caacaagaag gcaactgaca
420

aacagcctta tagcaagctc ccaggtgtct ctcttctgaa accactgaaa ggggtagatc
480

ctaacttaat caacaacctg gaaacattct ttgaattgga ttatcccaaa tatgaagtgc
540

tcctttgtgt acaagatcat gatgatccag ccattgatgt atgtaagaag cttcttggaa
600

aatatccaaa tgttgatgct agattgttta taggtggtaa aaaagttggc attaatccta
660

aaattaataa tttaatgcca ggatatgaag ttgcaaagta tgatcttata tggatttgtg
720

atagtggaat aagagtaatt ccagatacgc ttactgacat ggtgaatcaa atgacagaaa
780

aagtaggctt ggttcacggg ctgccttacg tagcagacag acagggcttt gctgccacct
840

tagagcaggt atattttgga acttcacatc caagatacta tatctctgcc aatgtaactg
900

gtttcaaatg tgtgacagga atgtcttgtt taatgagaaa agatgtgttg gatcaagcag
960

gaggacttat agcttttgct cagtacattg ccgaagatta ctttatggcc aaagcgatag

1020

ctgaccgagg ttggaggttt gcaatgtcca ctcaagttgc aatgcaaaac tctggctcat
1080

attcaatttc tcagtttcaa tccagaatga tcaggtggac caaactacga attaacatgc
1140

ttcctgctac aataatttgt gagccaattt cagaatgctt tgttgccagt ttaattattg
1200

gatgggcagc ccaccatgtg ttcagatggg atattatggt atttttcatg tgtcattgcc
1260

tggcatggtt tatatttgac tacattcaac tcaggggtgt ccagggtggc acactgtgtt
1320

tttcaaaact tgattatgca gtcgcctggt tcatccgcga atccatgaca atatacattt
1380

ttttgtctgc attatgggac ccaactataa gctggagaac tggtcgctac agattacgct
1440

gtgggggtac agcagaggaa atcctagatg tataactaca gctttgtgac tgtatataaa
1500

ggaaaaaaga gaagtattat aaattatgtt tatataaatg cttttaaaaa tctaccttct
1560

gtagttttat cacatgtatg ttttggtatc tgttctttaa tttatttttg catggcactt
1620

gcatctgtga aaaaaaa
1637

<210> 78
<211> 2085
<212> DNA
<213> Homo sapiens

<400> 78
tggccactcc cgtctgctgt gacgcgcgga cagagagcta ccggtggacc cacggtgcct
60

ccctccctgg gatctacaca gaccatggcc ttgccaacgg ctcgacccct gttggggtcc
120

tgtgggaccc ccgccctcgg cagcctcctg ttcctgctct tcagcctcgg atgggtgcag
180

ccctcgagga ccctggctgg agagacaggg caggaggctg cacccctgga cggagtcctg
240

gccaacccac ctaacatttc cagcctctcc cctcgccaac tccttggctt cccgtgtgcg
300

gaggtgtccg gcctgagcac ggagcgtgtc cgggagctgg ctgtggcctt ggcacagaag
360

aatgtcaagc tctcaacaga gcagctgcgc tgtctggctc accggctctc tgagcccccc
420

gaggacctgg acgccctccc attggacctg ctgctattcc tcaacccaga tgcgttctcg
480

gggccccagg cctgcacccg tttcttctcc cgcatcacga aggccaatgt ggacctgctc
540

ccgagggggg ctcccgagcg acagcggctg ctgcctgcgg ctctggcctg ctggggtgtg
600

cgggggtctc tgctgagcga ggctgatgtg cgggctctgg gaggcctggc ttgcgacctg
660

cctgggcgct ttgtggccga gtcggccgaa gtgctgctac cccggctggt gagctgcccg
720

ggacccctgg accaggacca gcaggaggca gccagggcgg ctctgcaggg cgggggaccc
780

ccctacggcc ccccgtcgac atggtctgtc tccacgatgg acgctctgcg gggcctgctg
840

cccgtgctgg gccagcccat catccgcagc atcccgcagg gcatcgtggc cgcgtggcgg
900

caacgctcct ctcgggaccc atcctggcgg cagcctgaac ggaccatcct ccggccgcgg
960

ttccggcggg aagtggagaa gacagcctgt ccttcaggca agaaggcccg cgagatagac
1020

gagagcctca tcttctacaa gaagtgggag ctggaagcct gcgtggatgc ggccctgctg
1080

gccacccaga tggaccgcgt gaacgccatc cccttcacct acgagcagct ggacgtccta
1140

aagcataaac tggatgagct ctacccacaa ggttaccccg agtctgtgat ccagcacctg
1200

ggctacctct tcctcaagat gagccctgag gacattcgca agtggaatgt gacgtccctg
1260

gagaccctga aggctttgct tgaagtcaac aaagggcacg aaatgagtcc tcaggtggcc
1320

accctgatcg accgctttgt gaagggaagg ggccagctag acaaagacac cctagacacc
1380

ctgaccgcct tctaccctgg gtacctgtgc tccctcagcc ccgaggagct gagctccgtg
1440

ccccccagca gcatctgggc ggtcaggccc caggacctgg acacgtgtga cccaaggcag
1500

ctggacgtcc tctatcccaa ggcccgcctt gctttccaga acatgaacgg gtccgaatac
1560

ttcgtgaaga tccagtcctt cctgggtggg gcccccacgg aggatttgaa ggcgctcagt
1620

cagcagaatg tgagcatgga cttggccacg ttcatgaagc tgcggacgga tgcggtgctg
1680

ccgttgactg tggctgaggt gcagaaactt ctgggacccc acgtggaggg cctgaaggcg
1740

gaggagcggc accgcccggt gcgggactgg atcctacggc agcggcagga cgacctggac
1800

acgctggggc tggggctaca gggcggcatc cccaacggct acctggtcct agacctcagc
1860

gtgcaagagg ccctctcggg gacgccctgc ctcctaggac ctggacctgt tctcaccgtc
1920

ctggcactgc tcctagcctc caccctggcc tgagggcccc actcccttgc tggccccagc
1980

cctgctgggg atccccgcct ggccaggagc aggcacgggt gatccccgtt ccaccccaag
2040

agaactcgcg ctcagtaaac gggaacatgc cccctgcaga cacgt
2085

<210> 79
<211> 5059
<212> DNA
<213> Homo sapiens

<400> 79
   ctggttctca acttcttttg aaataatgtt catagagaag gagggctgtc tgagattcga
   60
   gggaaacaag ctctcaggac ttccggtcgc catgatggct gtgggcggta aacgcggtta
   120
   gtgcaagcat ctgggccatc ttcaatggta aaaaagatac agtaaagaca taaataccac
   180
   atttgacaaa tggaaaaaaa ggagtgtcca gaaaagagta gcagcagtga ggaagagctg
   240
   ccgagacggg tatacaggga gctaccctgt gtttctgaga ccctttgtga catctcacat
   300
   tttttccaag aagatgatga gacagaggca gagccattat tgttccgtgc tgttcctgag
   360
   tgtcaactat ctggggggga cattcccagg agacatttgc tcagaagaga atcaaatagt
   420
   ttcctcttat gcttctaaag tctgttttga gatcgaagaa gattataaaa atcgtcagtt
   480
   tctggggcct gaaggaaatg tggatgttga gttgattgat aagagcacaa acagatacag
   540
   cgtttggttc cccactgctg gctggtatct gtggtcagcc acaggcctcg gcttcctggt
   600
   aagggatgag gtcacagtga cgattgcgtt tggttcctgg agtcagcacc tggccctgga
   660
   cctgcagcac catgaacagt ggctggtggg cggccccttg tttgatgtca ctgcagagcc
   720
   agaggaggct gtcgccgaaa tccacctccc ccacttcatc tccctccaag gtgaggtgga
   780
   cgtctcctgg tttctcgttg cccattttaa gaatgaaggg atggtcctgg agcatccagc
   840
   ccgggtggag cctttctatg ctgtcctgga aagccccagc ttctctctga tgggcatcct
   900
   gctgcggatc gccagtggga ctcgcctctc catccccatc acttccaaca cattgatcta
   960
   ttatcacccc caccccgaag atattaagtt ccacttgtac cttgtcccca gcgacgcctt
   1020
   gctaacaaag gcgatagatg atgaggaaga tcgcttccat ggtgtgcgcc tgcagacttc
   1080
   gcccccaatg gaacccctga actttggttc cagttatatt gtgtctaatt ctgctaacct
   1140
   gaaagtaatg cccaaggagt tgaaattgtc ctacaggagc cctggagaaa ttcagcactt
   1200
   ctcaaaattc tatgctgggc agatgaagga acccattcaa cttgagatta ctgaaaaaag
   1260
   acatgggact ttggtgtggg atactgaggt gaagccagtg gatctccagc ttgtagctgc
   1320
   atcagcccct cctcctttct caggtgcagc ctttgtgaag gagaaccacc ggcaactcca
   1380
   agccaggatg ggggacctga aaggggtgct cgatgatctc caggacaatg aggttcttac
   1440
   tgagaatgag aaggagctgg tggagcagga aaagacacgg cagagcaaga atgaggcctt
   1500
   gctgagcatg gtggagaaga aaggggacct ggccctggac gtgctcttca gaagcattag
   1560
   tgaaagggac ccttacctcg tgtcctatct tagacagcag aatttgtaaa atgagtcagt
   1620
   taggtagtct ggaagagaga atccagcgtt ctcattggaa atggataaac agaaatgtga
   1680
   tcattgattt cagtgttcaa gacagaagaa gactgggtaa catctatcac acaggctttc
   1740
   aggacagact tgtaacctgg catgtaccta ttgactgtat cctcatgcat tttcctcaag
   1800
   aatgtctgaa gaaggtagta atattccttt taaatttttt ccaaccattg cttgatatat
   1860
   cactatttta tccattgaca tgattcttga agacccagga taaaggacat ccggataggt
   1920
   gtgtttatga aggatggggc ctggaaaggc aacttttcct gattaatgtg aaaaataatt
   1980
   cctatggaca ctccgtttga agtatcacct tctcataact aaaagcagaa aagctaacaa
   2040
   aagcttctca gctgaggaca ctcaaggcat acatgatgac agtctttttt ttttttgtat
   2100
   gttaggactt taacacttta tctatggcta ctgttattag aacaatgtaa atgtatttgc
   2160
   tgaaagagag cacaaaaatg ggagaaaatg caaacatgag cagaaaatat tttcccactg
   2220
   gtgtgtagcc tgctacaagg agttgttggg ttaaatgttc atggtcaact ccaaggaata
   2280
   ctgagatgaa atgtggtaaa tcaactccac agaaccacca aaaagaaaat gagggtaatt
   2340
   cagcttattc tgagacagac attcctggca atgtaccata caaaaaataa gccaactctg
   2400
   acatttggat tctaccatag actctgtcat tttgtagcca tttcagctgt cttttgatta
   2460
   atgttttcgt ggcacacata tttccatcct tttatgttta atctgtttaa aacaagttcc
   2520
   tagtagacac catctggttg agtcagtttt ttttatggtg tattttgaac ccattctgat
   2580
   agtctctttt aactggaaga tttcaattac ttacgttaat gtaattatta atatgttagg
   2640
   atttatcctc agtcagccag tttgttatgt cttttctatt ctactgttat cacatttgta
   2700
   ccacttaaag tggaatctag gcactttatc accatttaga tcctattacc ttttctcatc
   2760
   taggatatag ttatcttcta cataatcttt ctgtatctta aaacccatca ataaattatt
   2820
   atatattttc tacttttaat cactcagaag atttaaaaaa ctcatgagaa gagtaatctg
   2880
   ttatgttttt ccagatattt accatttctg ttgctcttcc ttcattattt tccaaatttc
   2940
   gttctgcaaa tttccacttc ttctgataga cgttttttag ttcttttaga gtggttctga
   3000
   taggtacaga ttctcttatt ttttgcttcc tctgaggaca tctttttctc accttcattc
   3060
   tcagtgatgt tttttgcttg tagtattttt agttgacatt gttttctgtt cagcagtttc
   3120
   cttttagctt ccgtatttcc tgatgagaaa tctgcagtca ttcaaattgt tgtttccctg
   3180
   tatgtagtgt gtcatttttc tgtcagattt caaggtattt atctttagtt tttagccatt
   3240
   tcattatgtt ggggatgagt ttccttgttt tattcccttt ggaatttgct ccaattcata
   3300
   aatttgcagt tttatgtctt ttaccaaact tagaggtttt cagcctaatt tctaaaaata
   3360
   ctttttatta gcctgatttt catctttata ggaaatagtt taagtgatga caagttccaa
   3420
   tagcttatat gcccagaagg ccttcaaaat aagaattttg aaagaataca gaaaacaaac
   3480
   ttttatatcc ttctcatgtc ttctactgta aaattcatat gctttgctac tctaaaccta
   3540
   gtttgaaatc aacagtcttg agaatagatg aaaattttga tgaatagtgg aattctttta
   3600
   aatggaaacc tcttacatgt gattttcctt gccatctaga aataaaccat agtatttatg
   3660
   ttgaatcaat caatattata ttttgttttt ttcctcctct tctgagactc ttattgtgga
   3720
   aatgttagac ttttatgttt tcctaaatgt ccctgatatt ctacttattt agaacatctt
   3780
   ttcatttttt ccattattct gattgggtaa ttttaatttg tctattttca aatttgctgg
   3840
   agtgttcacc tgttgttgtc tgtgtcgtcc cactgagtgc attcaccacc ttttaaattt
   3900
   tggtcactgt atgtatcagt tctaaaattt ccattttgtt ctctatattt taaatttctt
   3960
   ggcttatatt ctattttcct gcaaatgtgt cagcatttgc ttgtttgagc tttttttttt
   4020
   tcaagacagg gtctcaactc tgttacccag gctggagtgc agtggtgcga tctcagctca
   4080
   ctgcaacctc tgcctcctgg ttcaagcgat tattgtgcct cagcctcctg agtagctggg
   4140
   attacaggca tgcaccacca cagcccagct aattttttgt atttttagta gagacagagt
   4200
   tttgctatgt tggccaggct ggttttgaac tcctggcctc aagtgatcca cccacctcag
   4260
   cctcccaaag tgctgggatt acaggccact acacctggca catttgagta tttttttttt
   4320
   tttttttttt ttgagatgga gtctcgctct gtcatctagg ctggagtgca gtggtgtgat
   4380
   ctcagctcac tgcagcctct gtctcccggg ctcaagcgat tctcttgcct cagcctcctg
   4440
   agtagctagg actacaggtg catgccaaca cgcccggcta atttttttaa aaaatatttt
   4500
   tagtagagac agggtttcac cattttggcc aggatggtct cgatctcctg acctcatgat
   4560
   ccacccgcct cggccttcca aagtgctggg attacaggca tgagccaccg tgcctggcct
   4620
   catttgagta tttttataat gtctctttta aagtctttgt cagataattc cactgtacat
   4680
   gttattcagt gtttggtgtc cactgagttg tcatttgcca gacaagtgga gatttttgca
   4740
   gctcatcctt gtattctcag tagttccgat atgtaccctc gacatgtgaa tgttatctta
   4800
   tgagactctg ttttatttgt atccaacaga agatgtttat tatttatttg gctttctgtg
   4860
   aactgaggtc ttaatatcag ctcattttaa aagtctttgc agtggtattc ggatctatcc
   4920
   tgtgtgtgcc tatgagattg ggtgcagtgt atcctgttag ctccattctc agggcgtttg
   4980
   aatgtgaatt aggaccagcg caatgaatgc tcaagttggg gttgggcgtt agaattcata
   5040
   aaagtcttta tatgctcag
   5059

<210> 80
<211> 1789
<212> DNA
<213> Homo sapiens

<400> 80
gcgcgctcgt cgccgaggcg agctaagccg ccgctcgcca tggggagccc cgcacatcgg
60

cccgcgctgc tgctgctgct gccgcctctg ctgctgctgc tgctgctgcg cgtcccgccc
120

agccgcagct tcccaggatc gggagactca ccactagaag acgatgaagt cgggtattca
180

caccctagat ataaagatac cccgtggtgc tcccccatca aggtgaagta tggggatgtg
240

tactgcaggg cccctcaagg aggatactac aaaacagccc tgggaaccag gtgcgacatt
300

cgctgccaga agggctacga gctgcatggc tcttccctac tgatctgcca gtcaaacaaa
360

cgatggtctg acaaggtcat ctgcaaacaa aagcgatgtc ctacccttgc catgccagca
420

aatggagggt ttaagtgtgt agatggtgcc tactttaact cccggtgtga gtattattgt
480

tcaccaggat acacgttgaa aggggagcgg accgtcacat gtatggacaa caaggcctgg
540

agcggccggc cagcctcctg tgtggatatg gaacctccta gaatcaagtg cccaagtgtg
600

aaggaacgca ttgcagaacc caacaaactg acagtccggg tgtcctggga gacacccgaa
660

ggaagagaca cagcagatgg aattcttact gatgtcattc taaaaggcct ccccccaggc
720

tccaactttc cagaaggaga ccacaagatc cagtacacag tctatgacag agctgagaat
780

aagggcactt gcaaatttcg agttaaagta agagtcaaac gctgtggcaa actcaatgcc
840

ccagagaatg gttacatgaa gtgctccagc gacggtgata attatggagc cacctgtgag
900

ttctcctgca tcggcggcta tgagctccag ggtagccctg cccgagtatg tcaatccaac

960

ctggcttggt ctggcacgga gcccacctgt gcagccatga acgtcaatgt gggtgtcaga
1020

acggcagctg cacttctgga tcagttttat gagaaaagga gactcctcat tgtgtccaca
1080

cccacagccc gaaacctcct ttaccggctc cagctaggaa tgctgcagca agcacagtgt
1140

ggccttgatc ttcgacacat caccgtggtg gagctggtgg gtgtgttccc gactctcatt
1200

ggcaggatag gagcaaagat tatgcctcca gccctagcgc tgcagctcag gctgttgctg
1260

cgaatcccac tctactcctt cagtatggtg ctagtggata agcatggcat ggacaaagag
1320

cgctatgtct ccctggtgat gcctgtggcc ctgttcaacc tgattgacac ttttcccttg
1380

agaaaagaag agatggtcct acaagccgaa atgagccaga cctgtaacac ctgacatgat
1440

ggttcctctc ttggcaattc ctcttcattg tctacatagt gacatgcaca cgggaaagcc
1500

ttaaaaatat ccttgatgta cagattttat ttgtaatttt aaaagtctat tttattatga
1560

gctttctttg cacttaaaaa ttagcatgct gctttttgta cttggaagtg tttcaaaaaa
1620

ttatatgacc atatttactc tttctaacct ttctttactc catcatggct ggttgatttg
1680

tagagaaatt agaacccata accatacaca ggctatcaac atgttattca atgtgacacc
1740

taactctttt ctattttgtt ttttaagtaa gacttttatt aataaaacg
1789

<210> 81
<211> 5399
<212> DNA
<213> Homo sapiens

<400> 81
   gccgcgcggg agagcgggag cctcggccct ccgcgcggct gcagctacct accctgcgcc
   60
   cggccaggtc cccgacttag ggatggcaaa cttgcgcccc gtggccgccc ccgccagcgc
   120
   cggcccccgc tcctgctgct gacggcgccc aggaaatcca cagcagtgat acatgtgacg
   180
   tccacactga cagtgccctc ctgtgggcat ggctcaggtt gtgcgcagtt cctggcacac
   240
   tggctgtaac tccgcccctt tctctccctc tcagtaaagc aagattacgc ggtgacatgc
   300
   ctcacagctg atcacgacac acggggatgg agagcaagag ttatggagaa tacaggttgg
   360
   atgggcaagg gacataggat gaccccagcc tgtcccctct tactgtctgt gattctgtcc
   420
   ctgcgcctgg ccaccgcctt cgaccccgcc cccagtgcct gctctgccct ggcctcgggt
   480
   gtgctctacg gggccttctc gctgcaggac ctctttccta ccatcgcctc gggctgctcc
   540
   tggaccctgg agaaccctga ccccaccaag tactccctct acctgcgctt caaccgccag
   600
   gagcaggtgt gcgcacactt tgccccccgc ctgctgcccc tggaccacta cctggtcaac
   660
   tttacctgcc tgcggcctag ccccgaggag gcggtggccc aggcggagtc agaggtgggg
   720
   cggccagaag aggaggaggc agaggcggca gcggggttgg agctgtgcag cggctcaggc
   780
   ccctttacct tcctgcactt cgacaagaac ttcgtgcagc tgtgcctgtc ggctgagccc
   840
   tccgaggccc cgcgcctgct ggcgcccgct gccctagcct tccgctttgt cgaggtcttg
   900
   ctcatcaaca acaacaactc tagccaattc acctgtggtg tgctctgccg ctggagtgag
   960
   gagtgtggcc gcgctgccgg cagggcctgc ggctttgctc agccaggctg cagctgccct
   1020
   ggagaggcgg gggccggctc caccaccacc acatctccag gccctcctgc tgcccacacc
   1080
   ctgtccaatg ccctggtgcc cgggggccca gccccacctg ctgaggccga tttgcactcg
   1140
   gggagcagca atgatctgtt cacaaccgag atgagatatg gtgaggagcc ggaagaggaa
   1200
   ccgaaagtga aaacccagtg gccgaggtct gcagatgagc ctgggctata catggcgcag
   1260
   acaggcgacc cggcggctga ggagtggtcc ccgtggagcg tgtgttccct gacgtgtggg
   1320
   cagggtctgc aggtgcggac ccgctcctgt gtgtcctccc cctatgggac cctgtgcagc
   1380
   gggcccctgc gggagaccag gccctgcaac aattcagcca cctgcccagt gcacggcgtg
   1440
   tgggaggagt gggggtcctg gagcctgtgc tcccgcagct gcgggcgggg gtcccggagc
   1500
   cggatgcgga cctgcgtgcc cccccagcac ggcggcaagg cctgcgaggg tcctgagctg
   1560
   cagactaagc tctgcagtat ggctgcctgc ccggtggaag gccagtggtt agaatggggt
   1620
   ccctggggcc catgctccac gtcctgtgcc aatgggaccc aacagcgcag ccggaagtgc
   1680
   agcgtggcgg gcccagcctg ggccacatgc acgggtgccc tcactgacac ccgggagtgc
   1740
   agcaacctcg agtgcccggc cactgatagc aagtgggggc catggaatgc gtggagcctg
   1800
   tgctctaaga cgtgtgacac aggctggcag cgccgcttcc gcatgtgcca ggccacgggc
   1860
   acgcagggct acccctgcga gggcaccgga gaggaggtga agccttgtag tgagaagagg
   1920
   tgtccagcct tccatgagat gtgcagggat gagtacgtga tgctgatgac gtggaagaag
   1980
   gcagctgctg gcgagatcat ctacaacaag tgccccccga atgcctcagg gtctgccagc
   2040
   cgccgctgtc tcctcagtgc ccaaggcgtg gcgtactggg ggctgcccag ctttgctcgc
   2100
   tgcatctccc atgagtaccg ctacctgtat ctgtcactta gggagcacct ggccaagggg
   2160
   cagcgcatgc tggcaggcga gggcatgtcg caggtggtgc gcagcctgca ggagctactg
   2220
   gcccggcgca cctactatag tggggacctg ctcttctctg tggacattct gaggaatgtc
   2280
   actgacacct ttaagagggc cacctacgtg ccctcggctg atgatgtgca gcgcttcttc
   2340
   caggtggtga gcttcatggt ggatgcggaa aacaaggaga agtgggacga tgctcagcag
   2400
   gtgtcccctg gctctgtgca cctgctccgt gtcgtggagg acttcattca cctggtgggc
   2460
   gatgctctca aggccttcca gagctctctg attgtcacag ataatctagt gatcagcatt
   2520
   cagcgagagc ccgtctcagc tgtgtccagt gacatcacgt tccccatgcg gggccgccgg
   2580
   ggcatgaagg actgggtgcg gcactcagag gaccgcctct tcctgcccaa ggaggtgctc
   2640
   agcctctcct ccccagggaa gccagccaca tctggggcag caggcagccc tggcaggggg
   2700
   aggggcccag gaacggtgcc tcctggccca ggccactccc accagcgcct cctcccagca
   2760
   gaccctgatg agtcctccta ctttgtgatc ggtgctgtac tctaccgcac ccttggcctc
   2820
   atcctgccgc ctcccaggcc cccgctggcc gtcacatccc gggtgatgac agtgactgtg
   2880
   cgccccccta cccagcctcc agctgagccc ctcatcactg tggagctctc ctacatcatc
   2940
   aatgggacca cggatcccca ttgcgccagc tgggactact ccagagcaga tgccagctca
   3000
   ggagactggg acactgaaaa ttgccagacc ctggagaccc aggcagctca cacccgctgc
   3060
   cagtgccagc acctgtccac ctttgctgta ctagcccagc cgcccaagga cctgaccctg
   3120
   gagctggcgg gctccccctc ggtccccctg gtgatcggct gtgcagtgtc gtgcatggcg
   3180
   ctgctcaccc tgctcgccat ctatgccgcc ttttggaggt tcataaaatc tgaacgctcc
   3240
   atcatcttgc tgaacttctg cctgtccatc ttggcatcca acatcctgat cctcgtgggc
   3300
   cagtcccggg tgctgagcaa gggcgtgtgc accatgacgg ctgccttcct gcacttcttc
   3360
   tttctctcct ccttttgctg ggtgcttacc gaggcctggc agtcctacct ggctgtcatt
   3420
   gggcggatgc gcacccgcct cgttcgcaag cgcttcctct gcctgggctg gggtctgcct
   3480
   gccctggtgg tggccgtgtc tgttggcttt acccgaacga aaggatacgg tacatccagc
   3540
   tactgctggc tctccctgga gggcggcctg ctctacgcct ttgtgggccc tgcagccgtc
   3600
   attgtcctgg tgaacatgct catcggaatc atcgtcttca acaagctcat ggcacgtgat
   3660
   ggcatctccg acaaatccaa gaagcagagg gccgggtcgg agcggtgccc ctgggccagc
   3720
   ctgctcctcc cctgctcagc gtgtggagcg gtccccagcc ccctgctcag ctcagcctcg
   3780
   gccaggaacg ccatggcctc actctggagc tcctgcgtgg tgctgcccct gctggcgctc
   3840
   acctggatgt ctgccgtcct ggctatgaca gaccgccgtt ccgtcctctt ccaggccctc
   3900
   tttgctgtct tcaactccgc gcagggcttt gtcatcactg ctgtgcactg cttcctgcgc
   3960
   cgagaggtcc aggatgtggt gaagtgccag atgggggtgt gccgggctga tgagagcgaa
   4020
   gactcccctg actcgtgtaa gaacgggcag ctgcagatcc tgtcagactt tgaaaaggat
   4080
   gtggatctgg cttgtcaaac agtgctgttc aaggaggtca acacttgcaa cccgtccacc
   4140
   atcacgggca cactatcccg cctgtccctg gatgaggatg aggagcccaa gtcctgcctc
   4200
   gtgggccctg agggcagcct cagcttctca ccactgcctg ggaatatcct ggtgcccatg
   4260
   gcagcctcac cagggctggg ggagcctccg cccccacagg aggccaaccc tgtttacatg
   4320
   tgtggggagg gtggcctgcg gcagctggac ctcacatggc tgcggcccac tgagccaggc
   4380
   tctgagggag actacatggt gctgccccgg cggactttga gcctgcagcc tggcggtggg
   4440
   ggtggaggtg gtgaggatgc ccccagggcc cggcccgagg ggaccccccg gcgagctgcc
   4500
   aagacagtgg cccacactga aggctacccc agcttcctgt ccgtggacca ctcgggcctg
   4560
   gggctgggcc ctgcctatgg atctctccag aatccctatg gaatgacctt ccaaccgcca
   4620
   ccgccgacac ccagcgcccg ccaagtgccc gagccagggg agcgcagccg gaccatgcct
   4680
   cgcaccgtgc ccggctctac catgaagatg ggctccctgg agcgaaagaa attacggtat
   4740
   tcagacctgg actttgaggt gatgcacacc cggaaacggc attcagaact ctaccacgag
   4800
   ctcaaccaga agttccacac tttcgaccgc taccgcagcc agtccacggc caagagggag
   4860
   aagcggtgga gtgtgtcctc gggtggggcg gccgagcgga gcgtgtgcac cgataagccc
   4920
   agccctgggg agcgccccag cttgtcccaa catcggcgcc atcagagctg gagcaccttc
   4980
   aaatctatga cactgggctc gctgcccccc aagccccgag aacggctgac tctgcaccgg
   5040
   gcagcagcct gggagcccac agaaccaccg gatggtgact tccagacaga ggtgtgagtg
   5100
   ccacgctgga ctgcccactg catataaata tatatatctc tctattttca cactccactt
   5160
   tggaactacc caggagccag cgccctctcc cctctcccga gggctgggca gggaggcgcc
   5220
   gtggactcag ccaggctggg ggagccggac atggcttggc ctggggtccc agggcccttc
   5280
   cttgtttctc agaggcccct cagccactgg aaccccatct tcagcccagc ctgtccgtcc
   5340
   ctgtcccggg ctggggaggg gggaggggaa ctttgttggg aataaacttc actctgtgg
   5399


<210> 82
<211> 696
<212> DNA
<213> Homo sapiens

<400> 82
gaacagttat ttctccagtc ctggaaggga ggcagggacc tatgggccca tgaagaaagt
60

gctggttgac aaccctaaga accggatgct gcttattgag aaccttcggg agtcccagcc
120

ctaccgctac acggtgaagg cgcgcaacgg ggccggctgg gggcctgagc gggaggccat
180

catcaacctg gccacccagc ccaagaggcc catgtccatc cccatcatcc ctgacatccc
240

tatcgtggac gcccagagcg gggaggacta cgacagcttc cttatgtaca gcgatgacgt
300

tctacgctct ccatcgggca gccagaggcc cagcgtctcc gatgacactg agcacctggt
360

gaatggccgg atggactttg ccttcccggg cagcaccaac tccctgcaca ggatgaccac
420

gaccagtgct gctgcctatg gcacccacct gagcccacac gtgccccacc gcgtgctaag
480

cacatcctcc accctcacac gggactacaa ctcactgacc cgctcagaac actcacactc
540

gaccacactg ccgagggact actccaccct cacctccgtc tcctcccacg actctcgcct
600

gactgctggt gtgcccgaca cgcccacccg ctggtgttct ctgccctggg gcccacatct
660

ctcagagtga gctggcagga gccgggtgcg agcggc
696

<210> 83
<211> 5645
<212> DNA
<213> Homo sapiens

<400> 83
cgcccgcgcg ctgcagcccc atctcctagc ggcagcccag gcgcggaggg agcgagtccg
60

ccccgaggta ggtccaggac gggcgcacag cagcagccga ggctggccgg gagagggagg
120

aagaggatgg cagggccacg ccccagccca tgggccaggc tgctcctggc agccttgatc
180

agcgtcagcc tctctgggac cttggcaaac cgctgcaaga aggccccagt gaagagctgc
240

acggagtgtg tccgtgtgga taaggactgc gcctactgca cagacgagat gttcagggac
300

cggcgctgca acacccaggc ggagctgctg gccgcgggct gccagcggga gagcatcgtg
360

gtcatggaga gcagcttcca aatcacagag gagacccaga ttgacaccac cctgcggcgc
420

agccagatgt ccccccaagg cctgcgggtc cgtctgcggc ccggtgagga gcggcatttt
480

gagctggagg tgtttgagcc actggagagc cccgtggacc tgtacatcct catggacttc

540

tccaactcca tgtccgatga tctggacaac ctcaagaaga tggggcagaa cctggctcgg
600

gtcctgagcc agctcaccag cgactacact attggatttg gcaagtttgt ggacaaagtc
660

agcgtcccgc agacggacat gaggcctgag aagctgaagg agccctggcc caacagtgac
720

ccccccttct ccttcaagaa cgtcatcagc ctgacagaag atgtggatga gttccggaat
780

aaactgcagg gagagcggat ctcaggcaac ctggatgctc ctgagggcgg cttcgatgcc
840

atcctgcaga cagctgtgtg cacgagggac attggctggc gcccggacag cacccacctg
900

ctggtcttct ccaccgagtc agccttccac tatgaggctg atggcgccaa cgtgctggct
960

ggcatcatga gccgcaacga tgaacggtgc cacctggaca ccacgggcac ctacacccag
1020

tacaggacac aggactaccc gtcggtgccc accctggtgc gcctgctcgc caagcacaac
1080

atcatcccca tctttgctgt caccaactac tcctatagct actacgagaa gcttcacacc
1140

tatttccctg tctcctcact gggggtgctg caggaggact cgtccaacat cgtggagctg
1200

ctggaggagg ccttcaatcg gatccgctcc aacctggaca tccgggccct agacagcccc
1260

cgaggccttc ggacagaggt cacctccaag atgttccaga agacgaggac tgggtccttt
1320

cacatccggc ggggggaagt gggtatatac caggtgcagc tgcgggccct tgagcacgtg
1380

gatgggacgc acgtgtgcca gctgccggag gaccagaagg gcaacatcca tctgaaacct
1440

tccttctccg acggcctcaa gatggacgcg ggcatcatct gtgatgtgtg cacctgcgag
1500

ctgcaaaaag aggtgcggtc agctcgctgc agcttcaacg gagacttcgt gtgcggacag
1560

tgtgtgtgca gcgagggctg gagtggccag acctgcaact gctccaccgg ctctctgagt
1620

gacattcagc cctgcctgcg ggagggcgag gacaagccgt gctccggccg tggggagtgc
1680

cagtgcgggc actgtgtgtg ctacggcgaa ggccgctacg agggtcagtt ctgcgagtat
1740

gacaacttcc agtgtccccg cacttccggg ttcctctgca atgaccgagg acgctgctcc
1800

atgggccagt gtgtgtgtga gcctggttgg acaggcccaa gctgtgactg tcccctcagc

1860
aatgccacct gcatcgacag caatgggggc atctgtaatg gacgtggcca ctgtgagtgt
1920
ggccgctgcc actgccacca gcagtcgctc tacacggaca ccatctgcga gatcaactac
1980
tcggcgatcc acccgggcct ctgcgaggac ctacgctcct gcgtgcagtg ccaggcgtgg
2040
ggcaccggcg agaagaaggg gcgcacgtgt gaggaatgca acttcaaggt caagatggtg
2100
gacgagctta agagagccga ggaggtggtg gtgcgctgct ccttccggga cgaggatgac
2160
gactgcacct acagctacac catggaaggt gacggcgccc ctgggcccaa cagcactgtc
2220
ctggtgcaca agaagaagga ctgccctccg ggctccttct ggtggctcat ccccctgctc
2280
ctcctcctcc tgccgctcct ggccctgcta ctgctgctat gctggaagta ctgtgcctgc
2340
tgcaaggcct gcctggcact tctcccgtgc tgcaaccgag gtcacatggt gggctttaag
2400
gaagaccact acatgctgcg ggagaacctg atggcctctg accacttgga cacgcccatg
2460
ctgcgcagcg ggaacctcaa gggccgtgac gtggtccgct ggaaggtcac caacaacatg
2520
cagcggcctg gctttgccac tcatgccgcc agcatcaacc ccacagagct ggtgccctac
2580
gggctgtcct tgcgcctggc ccgcctttgc accgagaacc tgctgaagcc tgacactcgg
2640
gagtgcgccc agctgcgcca ggaggtggag gagaacctga acgaggtcta caggcagatc
2700
tccggtgtac acaagctcca gcagaccaag ttccggcagc agcccaatgc cgggaaaaag
2760
caagaccaca ccattgtgga cacagtgctg atggcgcccc gctcggccaa gccggccctg
2820
ctgaagctta cagagaagca ggtggaacag agggccttcc acgacctcaa ggtggccccc
2880
ggctactaca ccctcactgc agaccaggac gcccggggca tggtggagtt ccaggagggc
2940
gtggagctgg tggacgtacg ggtgcccctc tttatccggc ctgaggatga cgacgagaag
3000
cagctgctgg tggaggccat cgacgtgccc gcaggcactg ccaccctcgg ccgccgcctg
3060
gtaaacatca ccatcatcaa ggagcaagcc agagacgtgg tgtcctttga gcagcctgag
3120
ttctcggtca gccgcgggga ccaggtggcc cgcatccctg tcatccggcg tgtcctggac

3180

ggcgggaagt cccaggtctc ctaccgcaca caggatggca ccgcgcaggg caaccgggac
3240

tacatccccg tggagggtga gctgctgttc cagcctgggg aggcctggaa agagctgcag
3300

gtgaagctcc tggagctgca agaagttgac tccctcctgc ggggccgcca ggtccgccgt
3360

ttccacgtcc agctcagcaa ccctaagttt ggggcccacc tgggccagcc ccactccacc
3420

accatcatca tcagggaccc agatgaactg gaccggagct tcacgagtca gatgttgtca
3480

tcacagccac cccctcacgg cgacctgggc gccccgcaga accccaatgc taaggccgct
3540

gggtccagga agatccattt caactggctg cccccttctg gcaagccaat ggggtacagg
3600

gtaaagtact ggattcaggg tgactccgaa tccgaagccc acctgctcga cagcaaggtg
3660

ccctcagtgg agctcaccaa cctgtacccg tattgcgact atgagatgaa ggtgtgcgcc
3720

tacggggctc agggcgaggg accctacagc tccctggtgt cctgccgcac ccaccaggaa
3780

gtgcccagcg agccagggcg tctggccttc aatgtcgtct cctccacggt gacccagctg
3840

agctgggctg agccggctga gaccaacggt gagatcacag cctacgaggt ctgctatggc
3900

ctggtcaacg atgacaaccg acctattggg cccatgaaga aagtgctggt tgacaaccct
3960

aagaaccgga tgctgcttat tgagaacctt cgggagtccc agccctaccg ctacacggtg
4020

aaggcgcgca acggggccgg ctgggggcct gagcgggagg ccatcatcaa cctggccacc
4080

cagcccaaga ggcccatgtc catccccatc atccctgaca tccctatcgt ggacgcccag
4140

agcggggagg actacgacag cttccttatg tacagcgatg acgttctacg ctctccatcg
4200

ggcagccaga ggcccagcgt ctccgatgac actgagcacc tggtgaatgg ccggatggac
4260

tttgccttcc cgggcagcac caactccctg cacaggatga ccacgaccag tgctgctgcc
4320

tatggcaccc acctgagccc acacgtgccc caccgcgtgc taagcacatc ctccaccctc
4380

acacgggact acaactcact gacccgctca gaacactcac actcgaccac actgccgagg
4440

gactactcca ccctcacctc cgtctcctcc cacgactctc gcctgactgc tggtgtgccc

4500

gacacgccca cccgcctggt gttctctgcc ctggggccca catctctcag agtgagctgg
4560

caggagccgc ggtgcgagcg gccgctgcag ggctacagtg tggagtacca gctgctgaac
4620

ggcggtgagc tgcatcggct caacatcccc aaccctgccc agacctcggt ggtggtggaa
4680

gacctcctgc ccaaccactc ctacgtgttc cgcgtgcggg cccagagcca ggaaggctgg
4740

ggccgagagc gtgagggtgt catcaccatt gaatcccagg tgcacccgca gagcccactg
4800

tgtcccctgc caggctccgc cttcactttg agcactccca gtgccccagg cccgctggtg
4860

ttcactgccc tgagcccaga ctcgctgcag ctgagctggg agcggccacg gaggcccaat
4920

ggggatatcg tcggctacct ggtgacctgt gagatggccc aaggaggagg gccagccacc
4980

gcattccggg tggatggaga cagccccgag agccggctga ccgtgccggg cctcagcgag
5040

aacgtgccct acaagttcaa ggtgcaggcc aggaccactg agggcttcgg gccagagcgc
5100

gagggcatca tcaccataga gtcccaggat ggaggaccct tcccgcagct gggcagccgt
5160

gccgggctct tccagcaccc gctgcaaagc gagtacagca gcatcaccac cacccacacc
5220

agcgccaccg agcccttcct agtggatggg ccgaccctgg gggcccagca cctggaggca
5280

ggcggctccc tcacccggca tgtgacccag gagtttgtga gccggacact gaccaccagc
5340

ggaaccctta gcacccacat ggaccaacag ttcttccaaa cttgaccgca ccctgcccca
5400

cccccgccat gtcccactag gcgtcctccc gactcctctc ccggagcctc ctcagctact
5460

ccatccttgc acccctgggg gcccagccca cccgcatgca cagagcaggg gctaggtgtc
5520

tcctgggagg catgaagggg gcaaggtccg tcctctgtgg gcccaaacct atttgtaacc
5580

aaagagctgg gagcagcaca aggacccagc ctttgttctg cacttaataa atggttttgc
5640

tactg
5645

<210> 84
<211> 14121
<212> DNA

<213>  Homo sapiens

<400>  84
attcccaccg ggacctgcgg ggctgagtgc ccttctcggt tgctgccgct gaggagcccg
60

cccagccagc cagggccgcg aggccgaggc caggccgcag cccaggagcc gccccaccgc
120

agctggcgat ggacccgccg aggcccgcgc tgctggcgct gctggcgctg cctgcgctgc
180

tgctgctgct gctggcgggc gccagggccg aagaggaaat gctggaaaat gtcagcctgg
240

tctgtccaaa agatgcgacc cgattcaagc acctccggaa gtacacatac aactatgagg
300

ctgagagttc cagtggagtc cctgggactg ctgattcaag aagtgccacc aggatcaact
360

gcaaggttga gctggaggtt ccccagctct gcagcttcat cctgaagacc agccagtgca
420

ccctgaaaga ggtgtatggc ttcaaccctg agggcaaagc cttgctgaag aaaaccaaga
480

actctgagga gtttgctgca gccatgtcca ggtatgagct caagctggcc attccagaag
540

ggaagcaggt tttcctttac ccggagaaag atgaacctac ttacatcctg aacatcaaga
600

ggggcatcat ttctgccctc ctggttcccc cagagacaga agaagccaag caagtgttgt
660

ttctggatac cgtgtatgga aactgctcca ctcactttac cgtcaagacg aggaagggca
720

atgtggcaac agaaatatcc actgaaagag acctggggca gtgtgatcgc ttcaagccca
780

tccgcacagg catcagccca cttgctctca tcaaaggcat gacccgcccc ttgtcaactc
840

tgatcagcag cagccagtcc tgtcagtaca cactggacgc taagaggaag catgtggcag
900

aagccatctg caaggagcaa cacctcttcc tgcctttctc ctacaacaat aagtatggga
960

tggtagcaca agtgacacag actttgaaac ttgaagacac accaaagatc aacagccgct
1020

tctttggtga aggtactaag aagatgggcc tcgcatttga gagcaccaaa tccacatcac
1080

ctccaaagca ggccgaagct gttttgaaga ctctccagga actgaaaaaa ctaaccatct
1140

ctgagcaaaa tatccagaga gctaatctct tcaataagct ggttactgag ctgagaggcc
1200

tcagtgatga agcagtcaca tctctcttgc cacagctgat tgaggtgtcc agccccatca
1260

ctttacaagc cttggttcag tgtggacagc ctcagtgctc cactcacatc ctccagtggc
1320

tgaaacgtgt gcatgccaac ccccttctga tagatgtggt cacctacctg gtggccctga
1380

tccccgagcc ctcagcacag cagctgcgag agatcttcaa catggcgagg gatcagcgca
1440

gccgagccac cttgtatgcg ctgagccacg cggtcaacaa ctatcataag acaaacccta
1500

cagggaccca ggagctgctg gacattgcta attacctgat ggaacagatt caagatgact
1560

gcactgggga tgaagattac acctatttga ttctgcgggt cattggaaat atgggccaaa
1620

ccatggagca gttaactcca gaactcaagt cttcaatcct caaatgtgtc caaagtacaa
1680

agccatcact gatgatccag aaagctgcca tccaggctct gcggaaaatg gagcctaaag
1740

acaaggacca ggaggttctt cttcagactt tccttgatga tgcttctccg ggagataagc
1800

gactggctgc ctatcttatg ttgatgagga gtccttcaca ggcagatatt aacaaaattg
1860

tccaaattct accatgggaa cagaatgagc aagtgaagaa ctttgtggct tcccatattg
1920

ccaatatctt gaactcagaa gaattggata tccaagatct gaaaaagtta gtgaaagaag
1980

ctctgaaaga atctcaactt ccaactgtca tggacttcag aaaattctct cggaactatc
2040

aactctacaa atctgtttct cttccatcac ttgacccagc ctcagccaaa atagaaggga
2100

atcttatatt tgatccaaat aactaccttc ctaaagaaag catgctgaaa actaccctca
2160

ctgcctttgg atttgcttca gctgacctca tcgagattgg cttggaagga aaaggctttg
2220

agccaacatt ggaagctctt tttgggaagc aaggattttt cccagacagt gtcaacaaag
2280

ctttgtactg ggttaatggt caagttcctg atggtgtctc taaggtctta gtggaccact
2340

ttggctatac caaagatgat aaacatgagc aggatatggt aaatggaata atgctcagtg
2400

ttgagaagct gattaaagat ttgaaatcca aagaagtccc ggaagccaga gcctacctcc
2460

gcatcttggg agaggagctt ggttttgcca gtctccatga cctccagctc ctgggaaagc
2520

tgcttctgat gggtgcccgc actctgcagg ggatccccca gatgattgga gaggtcatca
2580

ggaagggctc aaagaatgac ttttttcttc actacatctt catggagaat gcctttgaac
2640

tccccactgg agctggatta cagttgcaaa tatcttcatc tggagtcatt gctcccggag
2700

ccaaggctgg agtaaaactg gaagtagcca acatgcaggc tgaactggtg gcaaaaccct
2760

ccgtgtctgt ggagtttgtg acaaatatgg gcatcatcat tccggacttc gctaggagtg
2820

gggtccagat gaacaccaac ttcttccacg agtcgggtct ggaggctcat gttgccctaa
2880

aagctgggaa gctgaagttt atcattcctt ccccaaagag accagtcaag ctgctcagtg
2940

gaggcaacac attacatttg gtctctacca ccaaaacgga ggtgatccca cctctcattg
3000

agaacaggca gtcctggtca gtttgcaagc aagtctttcc tggcctgaat tactgcacct
3060

caggcgctta ctccaacgcc agctccacag actccgcctc ctactatccg ctgaccgggg
3120

acaccagatt agagctggaa ctgaggccta caggagagat tgagcagtat tctgtcagcg
3180

caacctatga gctccagaga gaggacagag ccttggtgga taccctgaag tttgtaactc
3240

aagcagaagg tgcgaagcag actgaggcta ccatgacatt caaatataat cggcagagta
3300

tgaccttgtc cagtgaagtc caaattccgg attttgatgt tgacctcgga acaatcctca
3360

gagttaatga tgaatctact gagggcaaaa cgtcttacag actcaccctg gacattcaga
3420

acaagaaaat tactgaggtc gccctcatgg gccacctaag ttgtgacaca aaggaagaaa
3480

gaaaaatcaa gggtgttatt tccatacccc gtttgcaagc agaagccaga agtgagatcc
3540

tcgcccactg gtcgcctgcc aaactgcttc tccaaatgga ctcatctgct acagcttatg
3600

gctccacagt ttccaagagg gtggcatggc attatgatga agagaagatt gaatttgaat
3660

ggaacacagg caccaatgta gataccaaaa aaatgacttc caatttccct gtggatctct
3720

ccgattatcc taagagcttg catatgtatg ctaatagact cctggatcac agagtccctg
3780

aaacagacat gactttccgg cacgtgggtt ccaaattaat agttgcaatg agctcatggc
3840

ttcagaaggc atctgggagt cttccttata cccagacttt gcaagaccac ctcaatagcc
3900

tgaaggagtt caacctccag aacatgggat tgccagactt ccacatccca gaaaacctct
3960

tcttaaaaag cgatggccgg gtcaaatata ccttgaacaa gaacagtttg aaaattgaga
4020

ttcctttgcc ttttggtggc aaatcctcca gagatctaaa gatgttagag actgttagga
4080

caccagccct ccacttcaag tctgtgggat tccatctgcc atctcgagag ttccaagtcc
4140

ctacttttac cattcccaag ttgtatcaac tgcaagtgcc tctcctgggt gttctagacc
4200

tctccacgaa tgtctacagc aacttgtaca actggtccgc ctcctacagt ggtggcaaca
4260

ccagcacaga ccatttcagc cttcgggctc gttaccacat gaaggctgac tctgtggttg
4320

acctgctttc ctacaatgtg caaggatctg gagaaacaac atatgaccac aagaatacgt
4380

tcacactatc atgtgatggg tctctacgcc acaaatttct agattcgaat atcaaattca
4440

gtcatgtaga aaaacttgga aacaacccag tctcaaaagg tttactaata ttcgatgcat
4500

ctagttcctg gggaccacag atgtctgctt cagttcattt ggactccaaa aagaaacagc
4560

atttgtttgt caaagaagtc aagattgatg ggcagttcag agtctcttcg ttctatgcta
4620

aaggcacata tggcctgtct tgtcagaggg atcctaacac tggccggctc aatggagagt
4680

ccaacctgag gtttaactcc tcctacctcc aaggcaccaa ccagataaca ggaagatatg
4740

aagatggaac cctctccctc acctccacct ctgatctgca aagtggcatc attaaaaata
4800

ctgcttccct aaagtatgag aactacgagc tgactttaaa atctgacacc aatgggaagt
4860

ataagaactt tgccacttct aacaagatgg atatgacctt ctctaagcaa aatgcactgc
4920

tgcgttctga atatcaggct gattacgagt cattgaggtt cttcagcctg ctttctggat
4980

cactaaattc ccatggtctt gagttaaatg ctgacatctt aggcactgac aaaattaata
5040

gtggtgctca caaggcgaca ctaaggattg gccaagatgg aatatctacc agtgcaacga
5100

ccaacttgaa gtgtagtctc ctggtgctgg agaatgagct gaatgcagag cttggcctct
5160

ctggggcatc tatgaaatta acaacaaatg gccgcttcag ggaacacaat gcaaaattca
5220

gtctggatgg gaaagccgcc ctcacagagc tatcactggg aagtgcttat caggccatga
5280

ttctgggtgt cgacagcaaa aacattttca acttcaaggt cagtcaagaa ggacttaagc
5340

tctcaaatga catgatgggc tcatatgctg aaatgaaatt tgaccacaca aacagtctga
5400

acattgcagg cttatcactg gacttctctt caaaacttga caacatttac agctctgaca
5460

agttttataa gcaaactgtt aatttacagc tacagcccta ttctctggta actactttaa
5520

acagtgacct gaaatacaat gctctggatc tcaccaacaa tgggaaacta cggctagaac
5580

ccctgaagct gcatgtggct ggtaacctaa aaggagccta ccaaaataat gaaataaaac
5640

acatctatgc catctcttct gctgccttat cagcaagcta taaagcagac actgttgcta
5700

aggttcaggg tgtggagttt agccatcggc tcaacacaga catcgctggg ctggcttcag
5760

ccattgacat gagcacaaac tataattcag actcactgca tttcagcaat gtcttccgtt
5820

ctgtaatggc cccgtttacc atgaccatcg atgcacatac aaatggcaat gggaaactcg
5880

ctctctgggg agaacatact gggcagctgt atagcaaatt cctgttgaaa gcagaacctc
5940

tggcatttac tttctctcat gattacaaag gctccacaag tcatcatctc gtgtctagga
6000

aaagcatcag tgcagctctt gaacacaaag tcagtgccct gcttactcca gctgagcaga
6060

caggcacctg gaaactcaag acccaattta acaacaatga atacagccag gacttggatg
6120

cttacaacac taaagataaa attggcgtgg agcttactgg acgaactctg gctgacctaa
6180

ctctactaga ctccccaatt aaagtgccac ttttactcag tgagcccatc aatatcattg
6240

atgctttaga gatgagagat gccgttgaga agccccaaga atttacaatt gttgcttttg
6300

taaagtatga taaaaaccaa gatgttcact ccattaacct cccatttttt gagaccttgc
6360

aagaatattt tgagaggaat cgacaaacca ttatagttgt agtggaaaac gtacagagaa
6420

acctgaagca catcaatatt gatcaatttg taagaaaata cagagcagcc ctgggaaaac
6480

tcccacagca agctaatgat tatctgaatt cattcaattg ggagagacaa gtttcacatg
6540

ccaaggagaa actgactgct ctcacaaaaa agtatagaat tacagaaaat gatatacaaa
6600

ttgcattaga tgatgccaaa atcaacttta atgaaaaact atctcaactg cagacatata
6660

tgatacaatt tgatcagtat attaaagata gttatgattt acatgatttg aaaatagcta
6720

ttgctaatat tattgatgaa atcattgaaa aattaaaaag tcttgatgag cactatcata
6780

tccgtgtaaa tttagtaaaa acaatccatg atctacattt gtttattgaa aatattgatt
6840

ttaacaaaag tggaagtagt actgcatcct ggattcaaaa tgtggatact aagtaccaaa
6900

tcagaatcca gatacaagaa aaactgcagc agcttaagag acacatacag aatatagaca
6960

tccagcacct agctggaaag ttaaaacaac acattgaggc tattgatgtt agagtgcttt
7020

tagatcaatt gggaactaca atttcatttg aaagaataaa tgatgttctt gagcatgtca
7080

aacactttgt tataaatctt attggggatt ttgaagtagc tgagaaaatc aatgccttca
7140

gagccaaagt ccatgagtta atcgagaggt atgaagtaga ccaacaaatc caggttttaa
7200

tggataaatt agtagagttg acccaccaat acaagttgaa ggagactatt cagaagctaa
7260

gcaatgtcct acaacaagtt aagataaaag attactttga gaaattggtt ggatttattg
7320

atgatgctgt gaagaagctt aatgaattat cttttaaaac attcattgaa gatgttaaca
7380

aattccttga catgttgata aagaaattaa agtcatttga ttaccaccag tttgtagatg
7440

aaaccaatga caaaatccgt gaggtgactc agagactcaa tggtgaaatt caggctctgg
7500

aactaccaca aaaagctgaa gcattaaaac tgtttttaga ggaaaccaag gccacagttg
7560

cagtgtatct ggaaagccta caggacacca aaataacctt aatcatcaat tggttacagg
7620

aggctttaag ttcagcatct ttggctcaca tgaaggccaa attccgagag actctagaag
7680

atacacgaga ccgaatgtat caaatggaca ttcagcagga acttcaacga tacctgtctc
7740

tggtaggcca ggtttatagc acacttgtca cctacatttc tgattggtgg actcttgctg
7800

ctaagaacct tactgacttt gcagagcaat attctatcca agattgggct aaacgtatga
7860

aagcattggt agagcaaggg ttcactgttc ctgaaatcaa gaccatcctt gggaccatgc
7920

ctgcctttga agtcagtctt caggctcttc agaaagctac cttccagaca cctgatttta
7980

tagtccccct aacagatttg aggattccat cagttcagat aaacttcaaa gacttaaaaa
8040

atataaaaat cccatccagg ttttccacac cagaatttac catccttaac accttccaca
8100

ttccttcctt tacaattgac tttgtcgaaa tgaaagtaaa gatcatcaga accattgacc
8160

agatgcagaa cagtgagctg cagtggcccg ttccagatat atatctcagg gatctgaagg
8220

tggaggacat tcctctagcg agaatcaccc tgccagactt ccgtttacca gaaatcgcaa
8280

ttccagaatt cataatccca actctcaacc ttaatgattt tcaagttcct gaccttcaca
8340

taccagaatt ccagcttccc cacatctcac acacaattga agtacctact tttggcaagc
8400

tatacagtat tctgaaaatc caatctcctc ttttcacatt agatgcaaat gctgacatag
8460

ggaatggaac cacctcagca aacgaagcag gtatcgcagc ttccatcact gccaaaggag
8520

agtccaaatt agaagttctc aattttgatt ttcaagcaaa tgcacaactc tcaaacccta
8580

agattaatcc gctggctctg aaggagtcag tgaagttctc cagcaagtac ctgagaacgg
8640

agcatgggag tgaaatgctg ttttttggaa atgctattga gggaaaatca aacacagtgg
8700

caagtttaca cacagaaaaa aatacactgg agcttagtaa tggagtgatt gtcaagataa
8760

acaatcagct taccctggat agcaacacta aatacttcca caaattgaac atccccaaac
8820

tggacttctc tagtcaggct gacctgcgca acgagatcaa gacactgttg aaagctggcc
8880

acatagcatg gacttcttct ggaaaagggt catggaaatg ggcctgcccc agattctcag
8940

atgagggaac acatgaatca caaattagtt tcaccataga aggacccctc acttcctttg
9000

gactgtccaa taagatcaat agcaaacacc taagagtaaa ccaaaacttg gtttatgaat
9060

ctggctccct caacttttct aaacttgaaa ttcaatcaca agtcgattcc cagcatgtgg
9120

gccacagtgt tctaactgct aaaggcatgg cactgtttgg agaagggaag gcagagttta
9180

ctgggaggca tgatgctcat ttaaatggaa aggttattgg aactttgaaa aattctcttt
9240

tcttttcagc ccagccattt gagatcacgg catccacaaa caatgaaggg aatttgaaag
9300

ttcgttttcc attaaggtta acagggaaga tagacttcct gaataactat gcactgtttc
9360

tgagtcccag tgcccagcaa gcaagttggc aagtaagtgc taggttcaat cagtataagt
9420

acaaccaaaa tttctctgct ggaaacaacg agaacattat ggaggcccat gtaggaataa
9480

atggagaagc aaatctggat ttcttaaaca ttcctttaac aattcctgaa atgcgtctac
9540

cttacacaat aatcacaact cctccactga aagatttctc tctatgggaa aaaacaggct
9600

tgaaggaatt cttgaaaacg acaaagcaat catttgattt aagtgtaaaa gctcagtata
9660

agaaaaacaa acacaggcat tccatcacaa atcctttggc tgtgctttgt gagtttatca
9720

gtcagagcat caaatccttt gacaggcatt ttgaaaaaaa cagaaacaat gcattagatt
9780

ttgtcaccaa atcctataat gaaacaaaaa ttaagtttga taagtacaaa gctgaaaaat
9840

ctcacgacga gctccccagg acctttcaaa ttcctggata cactgttcca gttgtcaatg
9900

ttgaagtgtc tccattcacc atagagatgt cggcattcgg ctatgtgttc ccaaaagcag
9960

tcagcatgcc tagtttctcc atcctaggtt ctgacgtccg tgtgccttca tacacattaa
10020

tcctgccatc attagagctg ccagtccttc atgtccctag aaatctcaag ctttctcttc
10080

cacatttcaa ggaattgtgt accataagcc atatttttat tcctgccatg ggcaatatta
10140

cctatgattt ctcctttaaa tcaagtgtca tcacactgaa taccaatgct gaacttttta
10200

accagtcaga tattgttgct catctccttt cttcatcttc atctgtcatt gatgcactgc
10260

agtacaaatt agagggcacc acaagattga caagaaaaag gggattgaag ttagccacag
10320

ctctgtctct gagcaacaaa tttgtggagg gtagtcataa cagtactgtg agcttaacca
10380

cgaaaaatat ggaagtgtca gtggcaaaaa ccacaaaagc cgaaattcca attttgagaa
10440

tgaatttcaa gcaagaactt aatggaaata ccaagtcaaa acctactgtc tcttcctcca
10500

tggaatttaa gtatgatttc aattcttcaa tgctgtactc taccgctaaa ggagcagttg
10560

accacaagct tagcttggaa agcctcacct cttacttttc cattgagtca tctaccaaag
10620

gagatgtcaa gggttcggtt ctttctcggg aatattcagg aactattgct agtgaggcca
10680

acacttactt gaattccaag agcacacggt cttcagtgaa gctgcagggc acttccaaaa
10740

ttgatgatat ctggaacctt gaagtaaaag aaaattttgc tggagaagcc acactccaac
10800

gcatatattc cctctgggag cacagtacga aaaaccactt acagctagag ggcctctttt
10860

tcaccaacgg agaacataca agcaaagcca ccctggaact ctctccatgg caaatgtcag
10920

ctcttgttca ggtccatgca agtcagccca gttccttcca tgatttccct gaccttggcc
10980

aggaagtggc cctgaatgct aacactaaga accagaagat cagatggaaa aatgaagtcc
11040

ggattcattc tgggtctttc cagagccagg tcgagctttc caatgaccaa gaaaaggcac
11100

accttgacat tgcaggatcc ttagaaggac acctaaggtt cctcaaaaat atcatcctac
11160

cagtctatga caagagctta tgggatttcc taaagctgga tgtaaccacc agcattggta
11220

ggagacagca tcttcgtgtt tcaactgcct ttgtgtacac caaaaacccc aatggctatt
11280

cattctccat ccctgtaaaa gttttggctg ataaattcat tactcctggg ctgaaactaa
11340

atgatctaaa ttcagttctt gtcatgccta cgttccatgt cccatttaca gatcttcagg
11400

ttccatcgtg caaacttgac ttcagagaaa tacaaatcta taagaagctg agaacttcat
11460

catttgccct caacctacca acactccccg aggtaaaatt ccctgaagtt gatgtgttaa
11520

caaaatattc tcaaccagaa gactccttga ttcccttttt tgagataacc gtgcctgaat
11580

ctcagttaac tgtgtcccag ttcacgcttc caaaaagtgt ttcagatggc attgctgctt
11640

tggatctaaa tgcagtagcc aacaagatcg cagactttga gttgcccacc atcatcgtgc
11700

ctgagcagac cattgagatt ccctccatta agttctctgt acctgctgga attgtcattc
11760

cttcctttca agcactgact gcacgctttg aggtagactc tcccgtgtat aatgccactt
11820

ggagtgccag tttgaaaaac aaagcagatt atgttgaaac agtcctggat tccacatgca
11880

gctcaaccgt acagttccta gaatatgaac taaatgtttt gggaacacac aaaatcgaag
11940

atggtacgtt agcctctaag actaaaggaa cacttgcaca ccgtgacttc agtgcagaat
12000

atgaagaaga tggcaaattt gaaggacttc aggaatggga aggaaaagcg cacctcaata
12060

tcaaaagccc agcgttcacc gatctccatc tgcgctacca gaaagacaag aaaggcatct
12120

ccacctcagc agcctcccca gccgtaggca ccgtgggcat ggatatggat gaagatgacg
12180

acttttctaa atggaacttc tactacagcc ctcagtcctc tccagataaa aaactcacca
12240

tattcaaaac tgagttgagg gtccgggaat ctgatgagga aactcagatc aaagttaatt
12300

gggaagaaga ggcagcttct ggcttgctaa cctctctgaa agacaacgtg cccaaggcca
12360

caggggtcct ttatgattat gtcaacaagt accactggga acacacaggg ctcaccctga
12420

gagaagtgtc ttcaaagctg agaagaaatc tgcagaacaa tgctgagtgg gtttatcaag
12480

gggccattag gcaaattgat gatatcgacg tgaggttcca gaaagcagcc agtggcacca
12540

ctgggaccta ccaagagtgg aaggacaagg cccagaatct gtaccaggaa ctgttgactc
12600

aggaaggcca agccagtttc cagggactca aggataacgt gtttgatggc ttggtacgag
12660

ttactcaaaa attccatatg aaagtcaagc atctgattga ctcactcatt gattttctga
12720

acttccccag attccagttt ccggggaaac ctgggatata cactagggag gaactttgca
12780

ctatgttcat aagggaggta gggacggtac tgtcccaggt atattcgaaa gtccataatg
12840

gttcagaaat actgttttcc tatttccaag acctagtgat tacacttcct ttcgagttaa
12900

ggaaacataa actaatagat gtaatctcga tgtataggga actgttgaaa gatttatcaa
12960

aagaagccca agaggtattt aaagccattc agtctctcaa gaccacagag gtgctacgta
13020

atcttcagga ccttttacaa ttcattttcc aactaataga agataacatt aaacagctga
13080

aagagatgaa atttacttat cttattaatt atatccaaga tgagatcaac acaatcttca
13140

atgattatat cccatatgtt tttaaattgt tgaaagaaaa cctatgcctt aatcttcata
13200

agttcaatga atttattcaa aacgagcttc aggaagcttc tcaagagtta cagcagatcc
13260

atcaatacat tatggccctt cgtgaagaat attttgatcc aagtatagtt ggctggacag
13320

tgaaatatta tgaacttgaa gaaaagatag tcagtctgat caagaacctg ttagttgctc
13380

ttaaggactt ccattctgaa tatattgtca gtgcctctaa ctttacttcc caactctcaa
13440

gtcaagttga gcaatttctg cacagaaata ttcaggaata tcttagcatc cttaccgatc
13500

cagatggaaa agggaaagag aagattgcag agctttctgc cactgctcag gaaataatta
13560

aaagccaggc cattgcgacg aagaaaataa tttctgatta ccaccagcag tttagatata
13620

aactgcaaga tttttcagac caactctctg attactatga aaaatttatt gctgaatcca
13680

aaagattgat tgacctgtcc attcaaaact accacacatt tctgatatac atcacggagt
13740

tactgaaaaa gctgcaatca accacagtca tgaaccccta catgaagctt gctccaggag
13800

aacttactat catcctctaa ttttttaaaa gaaatcttca tttattcttc ttttccaatt
13860

gaactttcac atagcacaga aaaaattcaa actgcctata ttgataaaac catacagtga
13920

gccagccttg cagtaggcag tagactataa gcagaagcac atatgaactg gacctgcacc
13980

aaagctggca ccagggctcg gaaggtctct gaactcagaa ggatggcatt ttttgcaagt
14040

taaagaaaat caggatctga gttattttgc taaacttggg ggaggaggaa caaataaatg
14100

gagtctttat tgtgtatcat a
14121

<210> 85
<211> 424
<212> DNA
<213> Homo sapiens

<400> 85
accagcccag cctttcagtg caggctccag ccctccaccc ccacccgagt tgcaggatgt
60

cgatgacaga cttgctgaac gctgaggaca tcaagaaggc ggtgggagcc tttagcgcta
120

ccgactcctt cgaccacaaa aagttcttcc aaatggtcgg cctgaagaaa aagagtgcgg
180

atgatgtgaa gaaggtgttt cacatgctgg acaaggacaa aagtggcttc atcgaggagg
240

atgagctggg attcatccta aaaggcttct ccccagatgc cagagacctg tctgctaaag
300

aaaccaagat gctgatggct gctggagaca aagatgggga cggcaaaatt ggggttgacg
360

aattctccac tctggtggct gaaagctaag aagcactgac tgcccctggt cttccacctc
420

tctg
424

<210> 86
<211> 1520
<212> DNA
<213> Homo sapiens

<400> 86
gaattccggg gggggtcccc ttccgatcct ccctcctgac gcccccccca gcagcccccct
60

cccccaccat tgaaagccat gaattttgaa tttgagaggg agattgggtt tataaacagc
120

cagccgtcgc tcgccgagtg tctgacttcc ttccccgctg tcttggagac atttcaaact
180

tcatcaatca aggagtcgac attaattcct cctcctcctc ctttcgagca aaccttcccc
240

agcctccagc ccggcgcctc cacccttcag agacccagga gccaaaagcg agccgaagat
300

gggcctgctc tgccgccgcc accgccgccg ccactccccg ctgccccccc ggcccccgag
360

ttcccttgga tgaaagagaa gaaatccgcc aagaaaccca gccaatccgc cacgtctcct
420

tctccggccg cctccgccgt tccggcctcc ggggtcggat cgcctgcaga tggcctggga
480

ctgccggagg ctggtggcgg cggggcgcgc aggctgcgca cggcttacac caacacgcag
540

ctgctggaac tggagaagga attccacttt aataagtacc tgtgccggcc acgccgcgtc
600

gagatcgcgg ccttgctgga cctcaccgaa aggcaggtca aagtctggtt tcagaaccgg
660

cgcatgaagc acaagcggca gacgcagcac cgagagccgc cggatgggga gcctgcctgc
720

ccgggagccc tggaggacat ctgcgaccct gccgaggaac ccgcggccag cccgggcggc
780

ccctccgcct cgcgggcggc gtgggaagcc tgctgtcacc cgccggaggt ggtgccgggg
840

gccttaagcg cggacccccg gcctttagcc gttcgcttag agggcgcagg cgcgtcgagt

900

cccggctgcg cgctgcgcgg ggccggcggg ctggagcccg ggccattgcc agaagacgtc
960

ttctcggggc gccaggattc acctttcctt cccgacctca acttcttcgc ggccgactcc
1020

tgtctccagc tatccggagg cctctcccct agcctacagg gttctctcga cagcccggtc
1080

cctttttccg aggaagagct ggattttttc accagtacgc tctgtgccat cgacctgcag
1140

tttccctaac ctgtttcctc ctcccggtcc tttcgacccc cgcgctcctt ggccgtctac
1200

tggaaaaatc gagcctctcc caccctcagt cgcatagact tatgtgtttt gctaaaattc
1260

aggtattact gaattagcgt ttaatccact tcctttcttc ttcttctaaa atattgggca
1320

ctcggttatc ttttaaaatt cacacagaaa aattccgttt ggtagactcc ttccaatgaa
1380

atctcaggaa taattaaact ctagggggac tttcttaaaa ataactagag ggacctattt
1440

tcctcttttt tatgttttag actgtagatt atttattaaa attctttaat aataggaaaa
1500

ggggaaagta tttattgtac
1520


<210>  87
<211>  753
<212>  DNA
<213>  Homo sapiens


<220>
<221>  misc_feature
<222>  (633)..(634)

<220>
<221>  misc_feature
<222>  (633)..(633)
<223>  n is a, c, g, or t

<400>  87
ttttttcctg ggggaaagac tgcaaccaca gacacaaaca ttcagaaaca ctggcggatt
60

tgggacaagc agaagggagt tgggagcatg gaaggaaaat aataataata attattatta
120

acaataataa caagataacc agtccaggag agagggagcc acaggaagac ctaagaccaa
180

acgaaatatc gtaacacaag gcgaggcagg cttgtgggaa ccggtcccca gcgggcggcg
240

gcagagcggg gaggattgga ggggccaggg ctgggggtgg cacgggctct tgggccgctg
300

ggctcctctg ggcgggctca gggctggaag gcgctgccag ctgtagccag gctcatactt
360

ttcaatttgt tgtccttctt ccacttcatg cgccggttct ggaaccagat cttgatctgg
420

cgctcggaca ggcagagtgc gtgggcgatc tcgatgcgcc gtcgccgggt caggtagcgg
480

ttgaagtgga actccttttc cagctccagg gtctggtagc gggtatacgc ggtccgggcc
540

cttttcccgt ccggcccggt catatcatgg ctgatgtgaa gcttcctcat ccaggggaat
600

atctgcggag tctgcccctc gggcgcggct gtngaggtgg ccatgggctc tggctgcgcc
660

cgagctaggc ttgggctgct tagctggctt gccgcttcct caggctccga ggacccgctg
720

ggctcgtcta atttggtgaa aattgcgctt gga
753

<210> 88
<211> 2037
<212> DNA
<213> Homo sapiens

<400> 88
cattgaatcg gccctaacga ttctcggatc gtcattaggt gtaaccatag agcatgaatt
60

acctcttgag gtcatcagcg agaatttacg actgtcaaca aaagcacgtg attccctaac
120

gccccccacc cccccaaccc cccccccata tttggccgca tacatagcaa aacgaagtac
180

agtgcatcgc tataattcat taatacatca taaatcgtga agcacagggt tataacgacc
240

acgatccaca aatcaagccc tccaaaatca cccaaatgag ctcgtacttt gtaaactcct
300

tctcggggcg ttatccaaat ggcccggact atcagttgct aaattatggc agtggcagct
360

ctctgagcgg ctcttacagg gatcccgctg ccatgcacac cggctcttac ggctacaatt
420

acaatgggat ggacctcagc gtcaaccgct cctcggcctc ctccagccac tttggggcgg
480

tgggcgagag ctcgcgcgcc ttccccgcgc ccgcccagga gccccgcttc aggcaagcgg
540

cttcgagctg ctccctgtcc tcgcccgagt ccctgccctg caccaacggc gacagccacg
600

gcgccaagcc ctctgcttcg tccccctccg accaggcgac ctcagccagc tccagcgcca
660

atttcaccga aatagacgag gccagcgcgt cctcggagcc tgaggaagcg gcaagccagc

720

taagcagccc cagcctagct cgggcgcagc cagagcccat ggccacctcc acagccgccc
780

ccgaggggca gactccgcag atattcccct ggatgaggaa gcttcacatc agccatgata
840

tgaccgggcc ggacgggaaa agggcccgga ccgcgtatac ccgctaccag accctggagc
900

tggaaaagga gttccacttc aaccgctacc tgacccggcg acggcgcatc gagatcgccc
960

acgcactctg cctgtccgag cgccagatca agatctggtt ccagaaccgg cgcatgaagt
1020

ggaagaagga caacaaattg aaaagtatga gcctggctac agctggcagc gccttccagc
1080

cttgagcccg cccagaggag cccagcggcc caagagcccg tgccaccccc agccctggcc
1140

cctccaatcc tccccgctct gccgccgccc gctggggacc ggttcccaca agcctgcctc
1200

gccttgtgtt acgatatttc gtttggtctt aggtcttcct gtggctccct ctctcctgga
1260

ctggttatct tgttattatt gttaataata ataattatta ttattttcct tccatgctcc
1320

caactccctt ctgcttgtcc caaatccgcc agtgtttctg aatgtttgtg tctgtggttg
1380

cagtctttcc cccaggaaaa aaaaaaaaaa agaaattcgc atgtttaatg tgaactctcc
1440

cctccccatc tgtgttctaa cttatttata aaagatgatc gctgtatttt gagtttcagc
1500

tggaaacttc tgtaaggggc agcagttgag gtggggtagt gccgcagtgg ggtcaagctg
1560

agctggcttc ggagatggag tcccttttca ttctcctcct cctccctcct cactccctag
1620

gcccaagtct cctaggggct tggtcctagg gtgggaaggg gctagggagg accaaaggga
1680

tggtattgag aagagagaaa gaagatagtg agatttaagt tcctgctgcc tgggtaggcc
1740

ccacaaggcc tggtctggga gtatacggaa acaaaaatga tcctcagtgc aaaatgtctt
1800

gtgtatttct ctgtgaatcc atgggtctgg ctagagggcc caaagcttgt aaatatgggg
1860

atagtctggg tcagacccat ctctccctta cccatcttgc ttccaagacc atttgtagtg
1920

agcgagtgga tgctgtgcta cgtgtgaaat ctgtctttgc ggggcctgtc tcagtgattc
1980

gcttttggta tttgtttgta gctttcctgg aagtcaaata aatgtttccc ccactcc

2037

<210> 89
<211> 2149
<212> DNA
<213> Homo sapiens

<400> 89
cagctgactc aggcaggctc catgctgaac ggtcacacag agaggaaaca ataaatctca
60

gctactatgc aataaatatc tcaagtttta acgaagaaaa acatcattgc agtgaaataa
120

aaaattttaa aattttagaa caaagctaac aaatggctag ttttctatga ttcttcttca
180

aacgctttct ttgaggggga aagagtcaaa caaacaagca gttttacctg aaataaagaa
240

ctagttttag aggtcagaag aaaggagcaa gttttgcgag aggcacggaa ggagtgtgct
300

ggcagtacaa tgacagtttt cctttccttt gctttcctcg ctgccattct gactcacata
360

gggtgcagca atcagcgccg aagtccagaa aacagtggga gaagatataa ccggattcaa
420

catgggcaat gtgcctacac tttcattctt ccagaacacg atggcaactg tcgtgagagt
480

acgacagacc agtacaacac aaacgctctg cagagagatg ctccacacgt ggaaccggat
540

ttctcttccc agaaacttca acatctggaa catgtgatgg aaaattatac tcagtggctg
600

caaaaacttg agaattacat tgtggaaaac atgaagtcgg agatggccca gatacagcag
660

aatgcagttc agaaccacac ggctaccatg ctggagatag gaaccagcct cctctctcag
720

actgcagagc agaccagaaa gctgacagat gttgagaccc aggtactaaa tcaaacttct
780

cgacttgaga tacagctgct ggagaattca ttatccacct acaagctaga gaagcaactt
840

cttcaacaga caaatgaaat cttgaagatc catgaaaaaa acagtttatt agaacataaa
900

atcttagaaa tggaaggaaa acacaaggaa gagttggaca ccttaaagga agagaaagag
960

aaccttcaag gcttggttac tcgtcaaaca tatataatcc aggagctgga aaagcaatta
1020

aacagagcta ccaccaacaa cagtgtcctt cagaagcagc aactggagct gatggacaca
1080

gtccacaacc ttgtcaatct ttgcactaaa gaaggtgttt tactaaaggg aggaaaaaga
1140

gaggaagaga aaccatttag agactgtgca gatgtatatc aagctggttt taataaaagt
1200

ggaatctaca ctatttatat taataatatg ccagaaccca aaaaggtgtt ttgcaatatg
1260

gatgtcaatg ggggaggttg gactgtaata caacatcgtg aagatggaag tctagatttc
1320

caaagaggct ggaaggaata taaaatgggt tttggaaatc cctccggtga atattggctg
1380

gggaatgagt ttatttttgc cattaccagt cagaggcagt acatgctaag aattgagtta
1440

atggactggg aagggaaccg agcctattca cagtatgaca gattccacat aggaaatgaa
1500

aagcaaaact ataggttgta tttaaaaggt cacactggga cagcaggaaa acagagcagc
1560

ctgatcttac acggtgctga tttcagcact aaagatgctg ataatgacaa ctgtatgtgc
1620

aaatgtgccc tcatgttaac aggaggatgg tggtttgatg cttgtggccc ctccaatcta
1680

aatggaatgt tctatactgc gggacaaaac catggaaaac tgaatgggat aaagtggcac
1740

tacttcaaag ggcccagtta ctccttacgt tccacaacta tgatgattcg acctttagat
1800

ttttgaaagc gcaatgtcag aagcgattat gaaagcaaca aagaaatccg gagaagctgc
1860

caggtgagaa actgtttgaa aacttcagaa gcaaacaata ttgtctccct tccagcaata
1920

agtggtagtt atgtgaagtc accaaggttc ttgaccgtga atctggagcc gtttgagttc
1980

acaagagtct ctacttgggg tgacagtgct cacgtggctc gactatagaa aactccactg
2040

actgtcgggc tttaaaaagg gaagaaactg ctgagcttgc tgtgcttcaa actactactg
2100

gaccttattt tggaactatg gtagccagat gataaatatg gttaatttc
2149

<210> 90

<400> 90
000

<210> 91
<211> 1600
<212> DNA
<213> Homo sapiens

<220>
<221> misc_feature
<223> unknown bases

<220>
<221>  misc_feature
<222>  (1147)..(1148)
<223>  unknown bases

<400>  91
atgaccatga gctcgttttt gataaactcc aactacatcg agcccaagtt ccctcccttc
60

gaggagtacg cacagcacag cggctcgggc ggcgcagacg gcggcccggg cggggtcccc
120

ggctaccagc agcccccagc gcccccgacc cagcacctgc cgctgcagca gccccagctc
180

cctcacgcgg gcggcggccg agagccccct gcctcctact acgcgccgcg gaccgcccgc
240

gagcccgcct accctgctgc cgcgctgtac cccgcgcatg gggccgcgga caccgcctac
300

ccctatggct accgcggcgg gcccagcccc gggcggccgc cccagcccga gcagcccccg
360

gcgcaagcca agggcccagc gcacggcctg catgcgagcc acgtcctgca gccccagccg
420

ccgccgcccc tgcagcctcg cgccgtgccc ccagcggccc cgcggcgctg cgaggcggcc
480

cccgccaccc caggcgtccc ggcagggggc agcgcccccg cgtgcccgct gctcttggcc
540

gacaagagcc cgctgggcct gaagggcaag gagcccgtgg tgtacccctg gatgaagaag
600

atccatgtca gcgccgttaa ccccagttat aacggagggg agcctaagcg ctctcgaacc
660

gcctacaccc ggcagcaggt cttggagctg gagaaggagt tccacttcaa tcgctacctg
720

acccggcggc gccgcatcga gatcgcccac acgctctgtt tgtctgagcg ccaggtcaag
780

atctggtttc agaaccggag aatgaagtgg aagaaagacc acaaactgcc caacaccaag
840

atgcgatcct ccaattcggc ctcggcctct gccggcccac cagggaaagc acaaactcag
900

agcccacacc tccatcccca cccccacccg agcacctcca cacccgttcc ctcctccata
960

taatcttcta gagatcttaa ccagtttcta tcccttacct gcttttctct tctcttctcc
1020

tgctccgttc ctcatccacc cctccccatc tggaccataa tagggacacc aaaacaaacc
1080

caaattggta aaaagaataa tcaaaaagaa gacattatcc ggttaagagt ctgtgctggt
1140

tgccacnnaa gagagaacag ttgtccagga tgctggctgg tggaacaacc tgctggcccg
1200

aaacaaggct gccaggtgtg gaacagccca tgctgactgg ggacatacac ttgcatcttt
1260

gttgaaagca gaagaagaca gaccctttcc ccaccttcct tacctcctct tcccccatta
1320

aggcagctca tccaagcttg tatttaactg aataaatgag tagacattgt ggacctcaca
1380

agattattta attcttaaga tgtgtagacc ttgatggtag gtgtgacatg ttagtttttc
1440

ttacttgcat ttatttaaga cacactgtta cagagatact gttgtcacct tctggggcac
1500

ggtctttggg gagaggggag tgcatttaga cttatgtgga acctgtacaa attgtgatgt
1560

ggctacatag aaagccatgt gctaagaata aactccattt
1600

<210> 92
<211> 1871
<212> DNA
<213> Homo sapiens

<400> 92
   atggggcagc cgagagagtt ctggcatctc aggtcccagg ttccagttcc aacctgccac
   60
   ctgctcaccg tgcagccttg gtgcctgctt cctcacccca ccccagctgg agggcgtgag
   120
   cgcaggcaca gtgcttcctc cctgcttgcg gctctgtgcc tgaggaaggc ggttctgcgg
   180
   ccccatcctg ctgtcctgcc tggagttgga gggtgtgtgg tcctttcctc tgggcggatg
   240
   aaggctccct tgaggggcgg cctggctctg gggacagggc cagctcccac gaagctgcag
   300
   cttctccatg actggcagag cgagcctggg ccgagtggct ggcagcaagg cccttctccg
   360
   cctctcttcc cggcaggcac ttccctcgtc cagcacctga tgaccgtcct catccgcaaa
   420
   cacagccagc tcttcacggc accggtcccg gaagggccca cctccccgcg cgggggcctg
   480
   caatgcgcag tggggtgggg ctccgaggag gtcaccaggg acagccaagg agagcccggc
   540
   ggccccggcc tgcccgcgca caggacctct tccctggacg gggcggccgt ggcggtgctc
   600
   tccagaacag cccccacggg gccggggagc cggtgcagcc ctgggaagaa ggtgcagacc
   660
   ctgcccagtt ggaagtcctc cttccggcag ccgaggtccc tatcgggaag cccgaagggg
   720
   ggcggctcat ccctggaggt gcccatcatc tcctccggcg ggaactggct tatgaacggg
   780
   ctgtcctccc tgcgcggaca ccgccgggcc tcgtcgggag accggctcaa ggactcgggc
   840
   tccgtgcaga gactctccac ctacgacaat gtgcccgcgc cgggcctggt ccccggcata
   900
   cccagcgtgg ccagtatggc gtggtccggg gcctcgtcca gcgagtcgtc ggtggggggc
   960
   tcactcagca gctgcacggc ctgccgcgcc agcgactcgt ctgcccgcag ttccctgcac
   1020
   accgactggg ccctggagcc ctccccgctc cccagcagca gcgaggaccc caagtccctg
   1080
   gacctggacc acagcatgga cgaggcgggc gcgggtgcca gcaacagcga gcccagcgag
   1140
   ccggacagcc ccacccggga acacgcgcgc cgctccgagg ccttacaggg gctggtcact
   1200
   gagctcaggg ccgagctgtg ccgccagcgg actgagtacg agaggagtgt gaaaagaatc
   1260
   gaagaaggga gtgctgacct gagaaaacga atgtcccggt tagaagaaga actggaccag
   1320
   gaaaagaaaa aatacatcat gctggaaata aagctgcgga actctgaacg ggcgcgggag
   1380
   gatgcggaga ggaggaacca gctgttgcag agggaaatgg aggagttttt ttcgacccta
   1440
   ggaagcttga ctgttggggc aaaaggtgcc agggccccaa agtaaaagga atggcagagc
   1500
   tcacttctgt accacgtctg ctggtctcca gccttgtatg gagttagaag cgtctgtatc
   1560
   tctggagcag ccaggcgctc tggagccagc tggagagaga gagatcctga tacctctgtg
   1620
   gggactgtgg ggacttttgg gaccccacac actccaggtg ggatcagatg ctgctccaac
   1680
   catgcagttc ctggtgaggg tcagaagggg acggtaccaa gagcagcgct tagcccttac
   1740
   ccaggaaata tccttcatgg ccacagaaat ggagggcgcc caggatccag gcagccaccg
   1800
   ggaacagtca gctttcttta ttaaatgtgc tcacaaagca aaaaaaaaaa aaaaaaaaaa
   1860
   aaaaaaaaaa a
   1871

<210> 93
<211> 3475
<212> DNA
<213> Homo sapiens

<400> 93

cgaggcggca tccgagggct gggccggcgc cctgggggac cccgggctcc ggaggccatg
60

ccggcgttgg cgcgcgacgc gggcaccgtg ccgctgctcg ttgttttttc tgcaatgata
120

tttgggacta ttacaaatca agatctgcct gtgatcaagt gtgttttaat caatcataag
180

aacaatgatt catcagtggg gaagtcatca tcatatccca tggtatcaga atccccggaa
240

gacctcgggt gtgcgttgag accccagagc tcagggacag tgtacgaagc tgccgctgtg
300

gaagtggatg tatctgcttc catcacactg caagtgctgg tcgatgcccc agggaacatt
360

tcctgtctct gggtctttaa gcacagctcc ctgaattgcc agccacattt tgatttacaa
420

aacagaggag ttgtttccat ggtcattttg aaaatgacag aaacccaagc tggagaatac
480

ctacttttta ttcagagtga agctaccaat tacacaatat tgtttacagt gagtataaga
540

aataccctgc tttacacatt aagaagacct tactttagaa aaatggaaaa ccaggacgcc
600

ctggtctgca tatctgagag cgttccagag ccgatcgtgg aatgggtgct ttgcgattca
660

cagggggaaa gctgtaaaga agaaagtcca gctgttgtta aaaaggagga aaaagtgctt
720

catgaattat ttgggacgga cataaggtgc tgtgccagaa atgaactggg cagggaatgc
780

accaggctgt tcacaataga tctaaatcaa actcctcaga ccacattgcc acaattattt
840

cttaaagtag gggaaccctt atggataagg tgcaaagctg ttcatgtgaa ccatggattc
900

gggctcacct gggaattaga aaacaaagca ctcgaggagg gcaactactt tgagatgagt
960

acctattcaa caaacagaac tatgatacgg attctgtttg cttttgtatc atcagtggca
1020

agaaacgaca ccggatacta cacttgttcc tcttcaaagc atcccagtca atcagctttg
1080

gttaccatcg taggaaaggg atttataaat gctaccaatt caagtgaaga ttatgaaatt
1140

gaccaatatg aagagttttg tttttctgtc aggtttaaag cctacccaca aatcagatgt
1200

acgtggacct tctctcgaaa atcatttcct tgtgagcaaa agggtcttga taacggatac
1260

agcatatcca agttttgcaa tcataagcac cagccaggag aatatatatt ccatgcagaa
1320

aatgatgatg cccaatttac caaaatgttc acgctgaata taagaaggaa acctcaagtg
1380

ctcgcagaag catcggcaag tcaggcgtcc tgtttctcgg atggataccc attaccatct
1440

tggacctgga agaagtgttc agacaagtct cccaactgca cagaagagat cacagaagga
1500

gtctggaata gaaaggctaa cagaaaagtg tttggacagt gggtgtcgag cagtactcta
1560

aacatgagtg aagccataaa agggttcctg gtcaagtgct gtgcatacaa ttcccttggc
1620

acatcttgtg agacgatcct tttaaactct ccaggcccct tccctttcat ccaagacaac
1680

atctcattct atgcaacaat tggtgtttgt ctcctcttca ttgtcgtttt aaccctgcta
1740

atttgtcaca agtacaaaaa gcaatttagg tatgaaagcc agctacagat ggtacaggtg
1800

accggctcct cagataatga gtacttctac gttgatttca gagaatatga atatgatctc
1860

aaatgggagt ttccaagaga aaatttagag tttgggaagg tactaggatc aggtgctttt
1920

ggaaaagtga tgaacgcaac agcttatgga attagcaaaa caggagtctc aatccaggtt
1980

gccgtcaaaa tgctgaaaga aaaagcagac agctctgaaa gagaggcact catgtcagaa
2040

ctcaagatga tgacccagct gggaagccac gagaatattg tgaacctgct gggggcgtgc
2100

acactgtcag gaccaattta cttgattttt gaatactgtt gctatggtga tcttctcaac
2160

tatctaagaa gtaaaagaga aaaatttcac aggacttgga cagagatttt caaggaacac
2220

aatttcagtt tttaccccac tttccaatca catccaaatt ccagcatgcc tggttcaaga
2280

gaagttcaga tacacccgga ctcggatcaa atctcagggc ttcatgggaa ttcatttcac
2340

tctgaagatg aaattgaata tgaaaaccaa aaaaggctgg aagaagagga ggacttgaat
2400

gtgcttacat ttgaagatct tctttgcttt gcatatcaag ttgccaaagg aatggaattt
2460

ctggaattta agtcgtgtgt tcacagagac ctggccgcca ggaacgtgct tgtcacccac
2520

gggaaagtgg tgaagatatg tgactttgga ttggctcgag atatcatgag tgattccaac
2580

tatgttgtca ggggcaatgc ccgtctgcct gtaaaatgga tggcccccga aagcctgttt
2640

gaaggcatct acaccattaa gagtgatgtc tggtcatatg gaatattact gtgggaaatc
2700

ttctcacttg gtgtgaatcc ttaccctggc attccggttg atgctaactt ctacaaactg
2760

attcaaaatg gatttaaaat ggatcagcca ttttatgcta cagaagaaat atacattata
2820

atgcaatcct gctgggcttt tgactcaagg aaacggccat ccttccctaa tttgacttcg
2880

tttttaggat gtcagctggc agatgcagaa gaagcgatgt atcagaatgt ggatggccgt
2940

gtttcggaat gtcctcacac ctaccaaaac aggcgacctt tcagcagaga gatggatttg
3000

gggctactct ctccgcaggc tcaggtcgaa gattcgtaga ggaacaattt agttttaagg
3060

acttcatccc tccacctatc cctaacaggc tgtagattac caaaacaaga ttaatttcat
3120

cactaaaaga aaatctatta tcaactgctg cttcaccaga cttttctcta gaagccgtct
3180

gcgtttactc ttgttttcaa agggactttt gtaaaatcaa atcatcctgt cacaaggcag
3240

gaggagctga taatgaactt tattggagca ttgatctgca tccaaggcct tctcaggccg
3300

gcttgagtga attgtgtacc tgaagtacag tatattcttg taaatacata aaacaaaagc
3360

attttgctaa ggagaagcta atatgatttt ttaagtctat gttttaaaat aatatgtaaa
3420

tttttcagct atttagtgat atattttatg ggtgggaata aaatttctac tacag
3475


<210>  94
<211>  3149
<212>  DNA
<213>  Homo sapiens

<400>  94
   ctaaagtttt tttctttgaa tgacagaact acagcataat gcgtggcttc aacctgctcc
   60
   tcttctgggg atgttgtgtt atgcacagct gggaagggca cataagaccc acacggaaac
   120
   caaacacaaa gggtaataac tgtagagaca gtaccttgtg cccagcttat gccacctgca
   180
   ccaatacggt ggacagttac tattgcactt gcaaacaagg cttcctgtcc agcaatgggc
   240
   aaaatcactt caaggatcca ggagtgcgat gcaaagatat tgatgaatgt tctcaaagcc
   300
   cccagccctg tggtcctaac tcatcctgca aaaacctgtc agggaggtac aagtgcagct
   360
   gtttagatgg tttctcttct cccactggaa atgactgggt cccaggaaag ccgggcaatt
   420
   tctcctgtac tgatatcaat gagtgcctca ccagcagggt ctgccctgag cattctgact
   480
   gtgtcaactc catgggaagc tacagttgca gctgtcaagt tggattcatc tctagaaact
   540
   ccacctgtga agacgtgaat gaatgtgcag atccaagagc ttgcccagag catgcaactt
   600
   gtaataacac tgttggaaac tactcttgtt tctgcaaccc aggatttgaa tccagcagtg
   660
   gccacttgag ttgccagggt ctcaaagcat cgtgtgaaga tattgatgaa tgcactgaaa
   720
   tgtgccccat caattcaaca tgcaccaaca ctcctgggag ctacttttgc acctgccacc
   780
   ctggctttgc accaagcagt ggacagttga atttcacaga ccaaggagtg gaatgtagag
   840
   atattgatga gtgccgccaa gatccatcaa cctgtggtcc taattctatc tgcaccaatg
   900
   ccctgggctc ctacagctgt ggctgcattg taggctttca tcccaatcca gaaggctccc
   960
   agaaagatgg caacttcagc tgccaaaggg ttctcttcaa atgtaaggaa gatgtgatac
   1020
   ccgataataa gcagatccag caatgccaag agggaaccgc agtgaaacct gcatatgtct
   1080
   ccttttgtgc acaaataaat aacatcttca gcgttctgga caaagtgtgt gaaaataaaa
   1140
   cgaccgtagt ttctctgaag aatacaactg agagctttgt ccctgtgctt aaacaaatat
   1200
   ccatgtggac taaattcacc aaggaagaga cgtcctccct ggccacagtc ttcctggaga
   1260
   gtgtggaaag catgacactg gcatcttttt ggaaaccctc agcaaatgtc actccggctg
   1320
   ttcgggcgga atacttagac attgagagca aagttatcaa caaagaatgc agtgaagaga
   1380
   atgtgacgtt ggacttggta gccaaggggg ataagatgaa gatcgggtgt tccacaattg
   1440
   aggaatctga atccacagag accactggtg tggcttttgt ctcctttgtg ggcatggaat
   1500
   cggttttaaa tgagcgcttc ttccaagacc accaggctcc cttgaccacc tctgagatca
   1560
   agctgaagat gaattctcga gtcgttgggg gcataatgac tggagagaag aaagacggct
   1620
   tctcagatcc aatcatctac actctggaga acgttcagcc aaagcagaag tttgagaggc
   1680
   ccatctgtgt ttcctggagc actgatgtga agggtggaag atggacatcc tttggctgtg
   1740
   tgatcctgga agcttctgag acatatacca tctgcagctg taatcagatg gcaaatcttg
   1800
   ccgttatcat ggcgtctggg gagctcacga tggacttttc cttgtacatc attagccatg
   1860
   taggcattat catctccttg gtgtgcctcg tcttggccat cgccaccttt ctgctgtgtc
   1920
   gctccatccg aaatcacaac acctacctcc acctgcacct ctgcgtgtgt ctcctcttgg
   1980
   cgaagactct cttcctcgcc ggtatacaca agactgacaa caagacgggc tgcgccatca
   2040
   tcgcgggctt cctgcactac cttttccttg cctgcttctt ctggatgctg gtggaggctg
   2100
   tgatactgtt cttgatggtc agaaacctga aggtggtgaa ttacttcagc tctcgcaaca
   2160
   tcaagatgct gcacatctgt gcctttggtt atgggctgcc gatgctggtg gtggtgatct
   2220
   ctgccagtgt gcagccacag ggctatggaa tgcataatcg ctgctggctg aatacagaga
   2280
   cagggttcat ctggagtttc ttggggccag tttgcacagt tatagtgatc aactcccttc
   2340
   tcctgacctg gaccttgtgg atcctgaggc agaggctttc cagtgttaat gccgaagtct
   2400
   caacgctaaa agacaccagg ttactgacct tcaaggcctt tgcccagctc ttcatcctgg
   2460
   gctgctcctg ggtgctgggc atttttcaga ttggacctgt ggcaggtgtc atggcttacc
   2520
   tgttcaccat catcaacagc ctgcaggggg ccttcatctt cctcatccac tgtctgctca
   2580
   acggccaggt acgagaagaa tacaagaggt ggatcactgg gaagacgaag cccagctccc
   2640
   agtcccagac ctcaaggatc ttgctgtcct ccatgccatc cgcttccaag acgggttaaa
   2700
   gcctttcttg ctttcaaata tgctatggag ccacagttga ggacagtagt ttcctgcagg
   2760
   agcctaccct gaaatctctt ctcagcttaa catggaaatg aggatcccac cagccccaga
   2820
   accctctggg gaagaatgtt gggggccgtc ttcctgtggt tgtatgcact gatgagaaat
   2880
   cagacgtttc tgctccaaac gaccatttta tcttcgtgct ctgcaacttc ttcaattcca
   2940
   gagtttctga gaacagaccc aaattcaatg gcatgaccaa gaacacctgg ctaccatttt
   3000
   gttttctcct gcccttgttg gtgcatggtt ctaagcgtgc ccctccagcg cctatcatac
   3060
   gcctgacaca gagaacctct caataaatga tttgtcgcct gtctgactga tttaccctaa
   3120
   aaaaaaaaaa aaaaaaaaaa aaaaaaaaa
   3149

<210> 95
<211> 982
<212> DNA
<213> Homo sapiens

<400> 95
agacaatttt cagcaggaag aagtagaaag gataaaatgg atcctgcacc acgggaacct
60

cacagcacct cacttctttt ggttttcttt ctctttgggg cacctctgga ctcactcccc
120

agcatgaagg cgctgagccc ggtgcgcggc tgctacgagg cggtgtgctg cctgtcggaa
180

cgcagtctgg ccatcgcccg gggccgaggg aagggcccgg cagctgagga gccgctgagc
240

ttgctggacg acatgaacca ctgctactcc cgcctgcggg aactggtacc cggagtcccg
300

agaggcactc agcttagcca ggtggaaatc ctacagcgcg tcatcgacta cattctcgac
360

ctgcaggtag tcctggccga gccagcccct ggaccccctg atggccccca ccttcccatc
420

cagacagccg agctcgctcc ggaacttgtc atctccaacg acaaaaggag cttttgccac
480

tgactcggcc gtgtcctgac acctccagaa cgcaggtgct ggcgcccgtt ctgcctggga
540

ccccgggaac ctctcctgcc ggaagccgga cggcagggat gggccccaac ttcgccctgc
600

ccacttgact tcaccaaatc ccttcctgga gactgaacct ggtgctcagg agcgaaggac
660

tgtgaacttg tggcctgaag agccagagct agctctggcc accagctggg cgacgtcacc
720

ctgctcccac cccaccccaa gttctaaggt cttttcagag cgtggaggtg tggaaggagt
780

ggctgctctc caaactatgc caaggcggcg gcagagctgg tcttctggtc tccttggaga
840

aaggttctgt tgccctgatt tatgaactct ataatagagt atataggttt tgtacctttt
900

ttacaggaag gtgactttct gtaacaatgc gatgtatatt aaacttttta taaaagttaa
960

cattttgcat aataaacgat tt

982

<210>  96
<211>  1677
<212>  DNA
<213>  Homo sapiens

<400>  96
ggccaagcaa gcttctatct gcacctgctc tcaatcctgc tctcaccatg agcctccgcc
60

tgcagagctc ctctgccagc tatggaggtg gtttcggggg tggctcttgc cagctgggag
120

gaggccgtgg tgtctctacc tgttcaactc ggtttgtgtc tgggggatca gctgggggct
180

atggaggcgg cgtgagctgt ggttttggtg gaggggctgg tagtggcttt ggaggtggct
240

atggaggtgg ccttggaggt ggctatggag gtggccttgg aggtggcttt ggtgggggtt
300

ttgctggtgg ctttgttgac tttggtgctt gtgatggcgg cctcctcact ggcaatgaga
360

agatcaccat gcagaacctc aacgaccgcc tggcttccta cctggagaag gtgcgcgccc
420

tggaggaggc caacgctgac ctggaggtga agatccgtga ctggcacctg aagcagagcc
480

cagctagccc tgagcgggac tacagcccct actacaagac cattgaagag ctccgggaca
540

agatcctgac cgccaccatt gaaaacaacc gggtcatcct ggagattgac aatgccaggc
600

tggctgtgga cgacttcagg ctcaagtatg agaatgagct ggccctgcgc cagagcgtgg
660

aggccgacat caacggcctg cgccgggtgc tggatgagct cactctgtct aagactgacc
720

tggagatgca gatcgagagc ctgaatgaag agctagccta catgaagaag aaccatgaag
780

aggagatgaa ggaatttagc aaccaggtgg tcggccaggt caacgtggag atggatgcca
840

ccccaggcat tgacctgacc cgcgtgctgg cagagatgag ggagcagtac gaggccatgg
900

cagagaggaa ccgccgggat gctgaggaat ggttccacgc caagagtgca gagctgaaca
960

aggaggtgtc taccaacact gccatgattc agaccagcaa gacagagatc acggagctca
1020

ggcgcacgct ccaaggcctg gagattgagc tgcagtccca gctgagcatg aaagcggggc
1080

tggagaacac ggtggcagag acggagtgcc gctatgccct gcagctgcag cagatccagg
1140

gactcatcag cagcatcgag gcccagctga gcgagctccg cagtgagatg gagtgccaga
1200

accaagagta caagatgctg ctggacatca agacacgtct ggagcaggag atcgccacct
1260

accgcagcct gctcgagggc caggacgcca agaagcgtca gcccccgtag cacctctgtt
1320

accacgactt ctagtgcctc tgttaccacc acctctaatg cctctggtcg ccgcacttct
1380

gatgtccgta ggccttaaat ctgcctggcg tcccctccct ctgtcttcag cacccagagg
1440

aggagagagc cggcagttcc ctgcaggaga gaggaggggc tgctggaccc aaggctcagt
1500

ccctctgctc tcaggacccc ctgtcctgac tctctcctga tggtgggccc tctgtgctct
1560

tctcttccgg tcggatctct ctcctctctg acctggatac gctttggttt ctcaacttct
1620

ctaccccaaa gaaaagatta ttcaataaag tttcctgcct ttctgcaaac ataaaaa
1677


<210>  97
<211>  1783
<212>  DNA
<213>  Homo sapiens

<400>  97
aaggaataaa gttgctgatt cattccttta cactgaaacc ctttgttgtg ccctcctctt
60

ccttcctctt tatagggaga cactctgaga aagagcacat tgtgggggcc cactccatgt
120

gatgtttgct tggttgcctg ttcccttttc tacctgcaga gcacggttcc cataagggcg
180

gcgagatcag cctcctgtct catctggaag accaccactc tggggtctca gaggaatgat
240

ggaagccttg gggtttctaa aattggaagt gaatggcccc atggtgacgg tggccctgtc
300

agtggctctc ttggccctcc tgaaatggta ctccacatca gcattctcaa gactggagaa
360

gttaggcctc agacatccca agccttctcc tttcattgga aacttgacat ttttccgcca
420

gggtttttgg gaaagccaaa tggagctcag aaagctgtat ggacctctgt gtgggtacta
480

tcttggtcgt cggatgttta ttgttatttc tgagccagac atgatcaagc aggtgttggt
540

tgagaacttc agtaacttta ccaacagaat ggcgtcgggt ttggagttca agtcggtagc
600

cgacagcgtt ctgtttttac gtgacaaaag atgggaagag gtcagaggtg ccctgatgtc
660

tgctttcagt cctgaaaagc tgaacgagat ggttcccctc atcagccaag cctgcgacct
720

tctcctggct catttaaaac gctatgcgga atctggggac gcatttgaca tccagaggtg
780

ctactgcaat tacaccacag atgtggttgc cagcgtcccg tttggcaccc cggtggactc
840

ctggcaggcc cctgaggatc cctttgtgaa acactgcaag cgtttcttcg aattctgcat
900

ccccagacct atcctggttt tactcttatc atttccatcc ataatggtcc cactggcccg
960

gattttgccc aataagaacc gagacgaact gaatggcttt tttaacaaac tcattaggaa
1020

tgtgattgcc ttgcgggacc agcaagctgc cgaagagagg cggagagact tcctccaaat
1080

ggtcctggat gcccgacatt ctgcaagtcc catgggcgtg caagactttg acatcgtcag
1140

agacgttttc tcctctactg ggtgcaagcc gaacccttcc cggcaacacc agcccagccc
1200

tatggccagg cctttgactg tggatgagat tgtgggccag gccttcatct tcctcatcgc
1260

tggctatgaa atcatcacca acacactttc ttttgccacc tacctactgg ccaccaaccc
1320

tgactgccaa gagaagcttc tgagagaggt agacgttttt aaggagaaac acatggcccc
1380

tgagttctgc agcctcgagg aaggcctgcc ctatctggac atggtgattg cagagacgct
1440

gaggatgtac ccgccagctt tcagattcac acgggaggca gctcaggact gcgaggtgct
1500

ggggcagcgc atccccgcag gcgctgtgct agagatggcc gtgggtgccc tgcaccatga
1560

ccctgagcac tggccaagcc cggagacctt caaccctgaa aggtaccgct gcagctagaa
1620

tccaaatctg ccctaggtcc aaaaaatggt gtctatatca agatcgtatc ccgctgacac
1680

agaaggctgc cgggtggggg gagggcaccc ccaaattcaa agaaaaccct aagtgtggat
1740

gttcagaatt ttggaaaaat gtcactgaag tgattgaaag ccg
1783

<210> 98
<211> 1935
<212> DNA
<213> Homo sapiens

<400> 98
cggggaaaaa atttggaacc ataaagttga aaactttttt ctctcagttt ggaagaagcc

60

cttcgtcatg aatgggatct gcagagttcg ggcgagagga ggcgagaggc gcaaaggagg
120

ggagatttgt cgcctgccgc tcgctctggg gctcgatgtg aatatatatt atgtctgcct
180

gttctcccct cgtcggtggc taaggtcagc cgcttggaac agaccccgga ggaggggggc
240

agagagggga ggtggggggg ggggggtccg gcgtgtcacg tgacccccag ggttgccaat
300

gtccggtcct gagggtatca ggcctttcca agttgccacc cactgcccag gcctcaccca
360

gcgatgcaga aagccaccta ctacgacaac gccgcggctg ctctcttcgg aggctattcc
420

tcgtaccctg gcagcaatgg cttcggcttc gatgtccccc cccaaccccc atttcaggcc
480

gccacgcacc tggagggcga ctaccagcgc tcagcttgct cgctgcagtc cctgggcaac
540

gctgccccac atgccaagag caaggagctc aacggcagct gcatgaggcc gggtctggcc
600

cccgagaccc tgtcggcccc gcctggctca cccccgccca gtgccgcacc taccagtgcc
660

actagcaaca gcagtaatgg gggcgggccc agcaaaagtg gtcccccaaa gtgcggtccc
720

ggcaccaact ccaccctcac caaacagata ttcccctgga tgaaagagtc gaggcaaacg
780

tccaagctga aaaacaactc ccccggcaca gcagagggct gtggtggcgg cggcggtggc
840

ggcggcggcg gaggcagtgg tggcagcggg ggcggtggcg gcggcggcgg gggaggggac
900

aagagccccc cggggtcggc ggcgtccaag cgggcgcgga cggcgtacac gagcgcgcag
960

ctggtggagc tggagaagga gttccatttt aaccgctacc tgtgccggcc tcgccgtgta
1020

gagatggcca acctgctgaa cctcagcgag cggcagatca agatctggtt ccagaaccgg
1080

cgcatgaagt acaagaagga ccagaaggcc aagggattgg cctcgtcgtc ggggggccca
1140

tctccagccg gcagcccccc gcagcccatg cagtccacgg ccggcttcat gaacgcctta
1200

cactccatga cccccagcta cgagagcccg tccccacccg ccttcggtaa agcccaccag
1260

aatgcctacg cgctgccctc caactaccag ccccctctca aaggctgcgg cgccccgcag
1320

aagtaccctc cgaccccggc gcccgagtat gagccgcacg tcctccaagc caacgggggc

1380

gcctacggga cgcccaccat gcagggcagt ccggtgtacg tgggcggggg cggctacgcg
1440

gatccgctgc cgccccctgc cggcccctcc ctctatggcc tcaaccacct ttcccatcac
1500

ccttccggga acctggacta caacggggcg ccccctatgg cgcccagcca gcaccacgga
1560

ccctgcgaac cccaccccac ctacacagac ctctcctctc accacgcgcc tcctcctcag
1620

ggtagaatcc aagaagcgcc caaattaaca cacctgtgat gggaaagggc gaacgaggat
1680

taggggatgg ggaggaagag agagactgtg gagctctggg gggcaacctg gaggtctgaa
1740

aagaggagcc agagaaggtg gtacccaggc ttcctggtca gaaccggcct ggagctcctt
1800

cccttccccc tggcctgaga ggttgctttt aagtcttcca ccccttgttc catctgcctg
1860

ccaacccatc ggaaaggaat ccacatcata ttggagatga ccccatcaac cccagggctc
1920

cagcactacc aagtt
1935


<210>  99
<211>  3064
<212>  DNA
<213>  Homo sapiens


<220>
<221>  misc_feature
<222>  (2700)..(2701)

<220>
<221>  misc_feature
<222>  (2701)..(2701)
<223>  n is a, c, g, or t

<400>  99
aggcagggcg ggcgggcgct ctaagggttc tgctctgact ccaggttggg acagcgtctt
60

cgctgctgct ggatagtcgt gttttcgggg atcgaggata ctcaccagaa accgaaaatg
120

ccgaaaccaa tcaatgtccg agttaccacc atggatgcag agctggagtt tgcaatccag
180

ccaaatacaa ctggaaaaca gctttttgat caggtggtaa agactatcgg cctccgggaa
240

gtgtggtact ttggcctcca ctatgtggat aataaaggat ttcctacctg gctgaagctg
300

gataagaagg tgtctgccca ggaggtcagg aaggagaatc ccctccagtt caagttccgg
360

gccaagttct accctgaaga tgtggctgag gagctcatcc aggacatcac ccagaaactt
420

ttcttcctcc aagtgaagga aggaatcctt agcgatgaga tctactgccc ccctgagact
480

gccgtgctct tggggtccta cgctgtgcag gccaagtttg gggactacaa caaagaagtg
540

cacaagtctg ggtacctcag ctctgagcgg ctgatccctc aaagagtgat ggaccagcac
600

aaacttacca gggaccagtg ggaggaccgg atccaggtgt ggcatgcgga acaccgtggg
660

atgctcaaag ataatgctat gttggaatac ctgaagattg ctcaggacct ggaaatgtat
720

ggaatcaact atttcgagat aaaaaacaag aaaggaacag acctttggct tggagttgat
780

gcccttggac tgaatattta tgagaaagat gataagttaa ccccaaagat tggctttcct
840

tggagtgaaa tcaggaacat ctctttcaat gacaaaaagt ttgtcattaa acccatcgac
900

aagaaggcac ctgactttgt gttttatgcc ccacgtctga gaatcaacaa gcggatcctg
960

cagctctgca tgggcaacca tgagttgtat atgcgccgca ggaagcctga caccatcgag
1020

gtgcagcaga tgaaggccca ggcccgggag gagaagcatc agaagcagct ggagcggcaa
1080

cagctggaaa cagagaagaa aaggagagaa accgtggaga gagagaaaga gcagatgatg
1140

cgcgagaagg aggagttgat gctgcggctg caggactatg aggagaagac aaagaaggca
1200

gagagagagc tctcggagca gattcagagg gccctgcagc tggaggagga gaggaagcgg
1260

gcacaggagg aggccgagcg cctagaggct gaccgtatgg ctgcactgcg ggctaaggag
1320

gagctggaga gacaggcggt ggatcagata aagagccagg agcagctggc tgcggagctt
1380

gcagaataca cagccaagat tgccctcctg gaagaggcgc ggaggcgcaa ggaggatgaa
1440

gttgaagagt ggcagcacag ggccaaagaa gcccaggatg acctggtgaa gaccaaggag
1500

gagctgcacc tggtgatgac agcacccccg cccccaccac cccccgtgta cgagccggtg
1560

agctaccatg tccaggagag cttgcaggat gagggcgcag agcccacggg ctacagcgcg
1620

gagctgtcta gtgagggcat ccgggatgac cgcaatgagg agaagcgcat cactgaggca
1680

gagaagaacg agcgtgtgca gcggcagctc gtgacgctga gcagcgagct gtcccaggcc
1740

cgagatgaga ataagaggac ccacaatgac atcatccaca acgagaacat gaggcaaggc
1800

cgggacaagt acaagacgct gcggcagatc cggcagggca acaccaagca gcgcatcgac
1860

gagttcgagg ccctgtaaca gccaggccag gaccaagggc agaggggtgc tcatagcggg
1920

cgctgccagc cccgccacgc ttgtctttag tgctccaagt ctaggaactc cctcagatcc
1980

cagttccttt agaaagcagt tacccaacag aaacattctg ggctgggaac cagggaggcg
2040

ccctggtttg ttttccccag ttgtaatagt gccaagcagg cctgattctc gcgattattc
2100

tcgaatcacc tcctgtgttg tgctgggagc aggactgatt gaattacgga aaatgcctgt
2160

aaagtctgag taagaaactt catgctggcc tgtgtgatac aagagtcagc atcattaaag
2220

gaaacgtggc aggacttcca tctgtgccat acttgttctg tattcgaaat gagctcaaat
2280

tgattttttt aatttctatg aaggatccat ctttgtatat ttacatgctt agaggggtga
2340

aaattatttt ggaaattgag tctgaagcac tctcgcacac acagtgattc cctcctcccg
2400

tcactccacg cagctggcag agagcacagt gatcaccagc gtgagtggtg gaggaggaca
2460

cttggatatt tttttagttc tttttttttt ggcttaacag ttttagaata cattgtactt
2520

atacacctta ttaatgatca gctatatact atttatatac aagtgataat acagatttgt
2580

aacattagtt ttaaaaaggg aaagttttgt tctgtatatt ttgttacctt ttacagaata
2640

aaagaattac atatgaaaaa ccctctaaac catggcactt gatgtgatgt ggcaggaggg
2700

nagtggtgga gctggacctg cctgctgcag ctgcagtcac gtgtaaacag gattattatt
2760

agtgttttat gcatgtaatg gactatgcac acttttaatt ttgtcagatt cacacatgcc
2820

actatgagct ttcagactcc agctgtgaag agactctgtc tgcttgtgtt tgtttgcagt
2880

ctctctctgc catggccttg gcaggctgct ggaaggcagc ttgtggaggc cgttggttcc
2940

gcccactcat tccttctcgt gcactgcttt ctccttcaca gctaagatgc catgtgcagg
3000

tggattccat gccgcagaca tgaaataaaa gctttgcaaa ggcaaagaaa aaaaaaaaaa
3060

aaaa
3064


<210> 100
<211> 3064
<212> DNA
<213> Homo sapiens


<220>
<221> misc_feature
<222> (2700)..(2701)

<220>
<221> misc_feature
<222> (2701)..(2701)
<223> n is a, c, g, or t

<400> 100
aggcagggcg ggcgggcgct ctaagggttc tgctctgact ccaggttggg acagcgtctt
60

cgctgctgct ggatagtcgt gttttcgggg atcgaggata ctcaccagaa accgaaaatg
120

ccgaaaccaa tcaatgtccg agttaccacc atggatgcag agctggagtt tgcaatccag
180

ccaaatacaa ctggaaaaca gctttttgat caggtggtaa agactatcgg cctccgggaa
240

gtgtggtact ttggcctcca ctatgtggat aataaaggat ttcctacctg gctgaagctg
300

gataagaagg tgtctgccca ggaggtcagg aaggagaatc ccctccagtt caagttccgg
360

gccaagttct accctgaaga tgtggctgag gagctcatcc aggacatcac ccagaaactt
420

ttcttcctcc aagtgaagga aggaatcctt agcgatgaga tctactgccc ccctgagact
480

gccgtgctct tggggtccta cgctgtgcag gccaagtttg gggactacaa caaagaagtg
540

cacaagtctg ggtacctcag ctctgagcgg ctgatccctc aaagagtgat ggaccagcac
600

aaacttacca gggaccagtg ggaggaccgg atccaggtgt ggcatgcgga acaccgtggg
660

atgctcaaag ataatgctat gttggaatac ctgaagattg ctcaggacct ggaaatgtat
720

ggaatcaact atttcgagat aaaaaacaag aaaggaacag acctttggct tggagttgat
780

gcccttggac tgaatattta tgagaaagat gataagttaa ccccaaagat tggctttcct
840

tggagtgaaa tcaggaacat ctctttcaat gacaaaaagt ttgtcattaa acccatcgac
900

aagaaggcac ctgactttgt gttttatgcc ccacgtctga gaatcaacaa gcggatcctg
960

cagctctgca tgggcaacca tgagttgtat atgcgccgca ggaagcctga caccatcgag
1020

gtgcagcaga tgaaggccca ggcccgggag gagaagcatc agaagcagct ggagcggcaa
1080

cagctggaaa cagagaagaa aaggagagaa accgtggaga gagagaaaga gcagatgatg
1140

cgcgagaagg aggagttgat gctgcggctg caggactatg aggagaagac aaagaaggca
1200

gagagagagc tctcggagca gattcagagg gccctgcagc tggaggagga gaggaagcgg
1260

gcacaggagg aggccgagcg cctagaggct gaccgtatgg ctgcactgcg ggctaaggag
1320

gagctggaga gacaggcggt ggatcagata aagagccagg agcagctggc tgcggagctt
1380

gcagaataca cagccaagat tgccctcctg gaagaggcgc ggaggcgcaa ggaggatgaa
1440

gttgaagagt ggcagcacag ggccaaagaa gcccaggatg acctggtgaa gaccaaggag
1500

gagctgcacc tggtgatgac agcacccccg cccccaccac cccccgtgta cgagccggtg
1560

agctaccatg tccaggagag cttgcaggat gagggcgcag agcccacggg ctacagcgcg
1620

gagctgtcta gtgagggcat ccgggatgac cgcaatgagg agaagcgcat cactgaggca
1680

gagaagaacg agcgtgtgca gcggcagctc gtgacgctga gcagcgagct gtcccaggcc
1740

cgagatgaga ataagaggac ccacaatgac atcatccaca acgagaacat gaggcaaggc
1800

cgggacaagt acaagacgct gcggcagatc cggcagggca acaccaagca gcgcatcgac
1860

gagttcgagg ccctgtaaca gccaggccag gaccaagggc agaggggtgc tcatagcggg
1920

cgctgccagc cccgccacgc ttgtctttag tgctccaagt ctaggaactc cctcagatcc
1980

cagttccttt agaaagcagt tacccaacag aaacattctg ggctgggaac cagggaggcg
2040

ccctggtttg ttttccccag ttgtaatagt gccaagcagg cctgattctc gcgattattc
2100

tcgaatcacc tcctgtgttg tgctgggagc aggactgatt gaattacgga aaatgcctgt
2160

aaagtctgag taagaaactt catgctggcc tgtgtgatac aagagtcagc atcattaaag
2220

gaaacgtggc aggacttcca tctgtgccat acttgttctg tattcgaaat gagctcaaat
2280

tgattttttt aatttctatg aaggatccat ctttgtatat ttacatgctt agaggggtga
2340

aaattatttt ggaaattgag tctgaagcac tctcgcacac acagtgattc cctcctcccg
2400

tcactccacg cagctggcag agagcacagt gatcaccagc gtgagtggtg gaggaggaca
2460

cttggatatt tttttagttc tttttttttt ggcttaacag ttttagaata cattgtactt
2520

atacacctta ttaatgatca gctatatact atttatatac aagtgataat acagatttgt
2580

aacattagtt tttaaaaggg aaagttttgt tctgtatatt ttgttacctt ttacagaata
2640

aaagaattac atatgaaaaa ccctctaaac catggcactt gatgtgatgt ggcaggaggg
2700

nagtggtgga gctggacctg cctgctgcag ctgcagtcac gtgtaaacag gattattatt
2760

agtgttttat gcatgtaatg gactatgcac acttttaatt ttgtcagatt cacacatgcc
2820

actatgagct ttcagactcc agctgtgaag agactctgtc tgcttgtgtt tgtttgcagt
2880

ctctctctgc catggccttg gcaggctgct ggaaggcagc ttgtggaggc cgttggttcc
2940

gcccactcat tccttctcgt gcactgcttt ctccttcaca gctaagatgc catgtgcagg
3000

tggattccat gccgcagaca tgaaataaaa gctttgcaaa ggcaaagaaa aaaaaaaaaa
3060

aaaa
3064


<210>  101
<211>  2930
<212>  DNA
<213>  Homo sapiens

<400>  101
ggggttggga cagcgtcttc gctgctgctg gatagtcgtg ttttcgggga tcgaggatac
60

tcaccagaaa ccgaaaatgc cgaaaccaat caatgtccga gttaccacca tggatgcaga
120

gctggagttt gcaatccagc caaatacaac tggaaaacag ctttttgatc aggtggtaaa
180

gactatcggc ctccgggaag tgtggtactt tggcctccac tatgtggata ataaaggatt
240

tcctacctgg ctgaagctgg ataagaaggt gtctgcccag gaggtcagga aggagaatcc
300

cctccagttc aagttccggg ccaagttcta ccctgaagat gtggctgagg agctcatcca
360

ggacatcacc cagaaacttt tcttcctcca agtgaaggaa ggaatcctta gcgatgagat
420

ctactgcccc cctgagactg ccgtgctctt ggggtcctac gctgtgcagg ccaagtttgg
480

ggactacaac aaagaagtgc acaagtctgg gtacctcagc tctgagcggc tgatccctca
540

aagagtgatg gaccagcaca aacttaccag ggaccagtgg gaggaccgga tccaggtgtg
600

gcatgcggaa caccgtggga tgctcaaaga taatgctatg ttggaatacc tgaagattgc
660

tcaggacctg gaaatgtatg gaatcaacta tttcgagata aaaaacaaga aaggaacaga
720

cctttggctt ggagttgatg cccttggact gaatatttat gagaaagatg ataagttaac
780

cccaaagatt ggctttcctt ggagtgaaat caggaacatc tctttcaatg acaaaaagtt
840

tgtcattaaa cccatcgaca agaaggcacc tgactttgtg ttttatgccc cacgtctgag
900

aatcaacaag cggatcctgc agctctgcat gggcaaccat gagttgtata tgcgccgcag
960

gaagcctgac accatcgagg tgcagcagat gaaggcccag gcccgggagg agaagcatca
1020

gaagcagctg gagcggcaac agctggaaac agagaagaaa aggagagaaa ccgtggagag
1080

agagaaagag cagatgatgc gcgagaagga ggagttgatg ctgcggctgc aggactatga
1140

ggagaagaca aagaaggcag agagagagct ctcggagcag attcagaggg ccctgcagct
1200

ggaggaggag aggaagcggg cacaggagga ggccgagcgc ctagaggctg accgtatggc
1260

tgcactgcgg gctaaggagg agctggagag acaggcggtg gatcagataa agagccagga
1320

gcagctggct gcggagcttg cagaatacac tgccaagatt gccctcctgg aagaggcgcg
1380

gaggcgcaag gaggatgaag ttgaagagtg gcagcacagg gccaaagaag cccaggatga
1440

cctggtgaag accaaggagg agctgcacct ggtgatgaca gcacccccgc ccccaccacc
1500

ccccgtgtac gagccggtga gctaccatgt ccaggagagc ttgcaggatg agggcgcaga
1560

gcccacgggc tacagcgcgg agctgtctag tgagggcatc cgggatgacc gcaatgagga
1620

gaagcgcatc actgaggcag agaagaacga gcgtgtgcag cggcagctcg tgacgctgag
1680

cagcgagctg tcccaggccc gagatgagaa taagaggacc cacaatgaca tcatccacaa
1740

cgagaacatg aggcaaggcc gggacaagta caagacgctg cggcagatcc ggcagggcaa
1800

caccaagcag cgcatcgacg agttcgaggc cctgtaacag ccaggccagg accaagggca
1860

gaggggtgct catagcgggc gctgccagcc ccgccacgct tgtctttagt gctccaagtc
1920

taggaactcc ctcagatccc agttccctta gaaagcagtt acccaacaga aacattctgg
1980

gctgggaacc agggaggcgc cctggtttgt tttccccagt tgtaatagtg ccaagcaggc
2040

ctgattctcg cgattattct cgaatcacct cctgtgttgt gctgggagca ggactgattg
2100

aattacggaa aatgcctgta aagtctgagt aagaaacttc atgctggcct gtgtgataca
2160

agagtcagca tcattaaagg aaacgtggca ggacttccat ctgtgccata cttgttctgt
2220

attcgaaatg agctcaaatt gattttttaa tttctatgaa ggatccatct ttgtatattt
2280

acatgcttag aggggtgaaa attattttgg aaattgagtc tgaagcactc tcgcacacac
2340

agtgattccc tcctcccgtc actccacgca gctggcagag agcacagtga tcaccagcgt
2400

gagtggtgga ggaggacact tggatttttt tttttgtttt tttttttttg cttaacagtt
2460

ttagaataca ttgtacttat acaccttatt aatgatcagc tatatactat ttatatacaa
2520

gtgataatac agatttgtaa cattagtttt aaaaagggaa agttttgttc tgtatatttt
2580

gttacctttt acagaataaa agaattacat atgaaaaacc ctctaaacca tggcacttga
2640

tgtgatgtgg caggagggca gtggtggagc tggacctgcc tgctgcagtc acgtgtaaac
2700

aggattatta ttagtgtttt atgcatgtaa tggactatgc acacttttaa ttttgtcaga
2760

ttcacacatg ccactatgag ctttcagact ccagctgtga agagactctg tttgcttgtg
2820

tttgtttgtt tgcagtctct ctctgccatg gccttggcag gctgctggaa ggcagcttgt
2880

ggaggccgtt ggttccgccc actcattcct tctcgtgcac tgctttctcc
2930

<210> 102
<211> 1577
<212> DNA
<213> Homo sapiens

<400> 102
ggcacgaggc tgctgtttgt ctacttcctc ctgcttcccc gccgccgccg ccgccatcat
60

gagggaaatc gtgcacttgc aggccgggca gtgcggcaac caaatcggcg ccaagttttg
120

ggaggtgatc agcgatgagc acggcatcga ccccacgggc acctaccacg gggacagcga
180

cctgcagctg gaacgcatca acgtgtacta caatgaggcc accggcggca agtacgtgcc
240

ccgcgccgtg ctcgtggatc tggagcccgg caccatggac tccgtgcgct cggggccctt
300

cgggcagatc ttccggccgg acaacttcgt tttcggtcag agtggtgctg ggaacaactg
360

ggccaagggg cactacacag aaggcgcgga gctggtggac tcggtgctgg atgttgtgag
420

aaaggaggct gagagctgtg actgcctgca gggtttccag ctgacccact ccctgggtgg
480

ggggactggg tctgggatgg gtaccctcct catcagcaag atccgggagg agtacccaga
540

caggatcatg aacacgttta gtgtggtgcc ttcgcccaaa gtgtcagaca cagtggtgga
600

gccctacaac gccaccctct cagtccacca gctcgtagaa aacacagacg agacctactg
660

cattgataac gaagctctct acgacatttg cttcagaacc ctaaagctga ccacgcccac
720

ctatggtgac ctgaaccacc tggtgtctgc taccatgagt ggggtcacca cctgcctgcg
780

cttcccaggc cagctcaatg ctgacctgcg gaagctggct gtgaacatgg tcccgtttcc
840

ccggctgcac ttcttcatgc ccggctttgc cccactgacc agccggggca gccagcagta
900

ccgggcgctg accgtgcccg agctcaccca gcagatgttt gatgccaaga acatgatggc
960

tgcctgcgac ccccgccatg gccgctacct gacggttgcc gccgtgttca ggggccgcat
1020

gtccatgaag gaggtggatg agcaaatgct taatgtccaa aacaaaaaca gcagctattt
1080

tgttgagtgg atccccaaca atgtgaaaac ggctgtctgt gacatcccac ctcgggggct

1140

aaaaatgtcc gccaccttca ttggcaacag cacggccatc caggagctgt tcaagcgcat
1200

ctccgagcag ttcacggcca tgttccggcg caaggccttc ctgcactggt acacgggcga
1260

gggcatggac gagatggagt tcaccgaggc cgagagcaac atgaatgacc tggtgtccga
1320

gtaccagcag taccaggatg ccacagccga ggaggagggc gagttcgagg aggaggctga
1380

ggaggaggtg gcctagagcc ttcagtcact ggggaaagca gggaagcagt gtgaactctt
1440

tattcactcc cagcctgtcc tgtggcctgt cccactgtgt gcacttgctg ttttccctgt
1500

ccacatccat gctgtacaga caccaccatt aaagcatttt catagtgaaa aaaaaaaaaa
1560

aaaaaaaaaa aaaaaaa
1577


<210>  103
<211>  724
<212>  DNA
<213>  Homo sapiens


<220>
<221>  misc_feature
<222>  (500)..(700)

<220>
<221>  misc_feature
<222>  (533)..(533)
<223>  n is a, c, g, or t

<220>
<221>  misc_feature
<222>  (667)..(667)
<223>  n is a, c, g, or t

<220>
<221>  misc_feature
<222>  (695)..(695)
<223>  n is a, c, g, or t

<400>  103
acagtcgcct ctcctcctcc tctacctcct ccttcaccac cacctcctct tcctcctcct
60

cctcctcctc ctcctccgcc aactcctcgg ctgcacacca gctctaagag cgagagtgaa
120

cgagagaggg agggagagag tgagagcgag cgagatcttt ggagagactc tttttttgc
180

ctcctacttc tgtcttgaag ccagacaatc gacttcagct ctccctcccc tccctctttc
240

tccacgttct gctcccactc gctctcctgt ccccttcccc tcccctcccg gcggaaagcc

300

ccccgaaacc aacaaagctg agccgagaga aacaaacaaa acaaacacac cgggccagac
360

aagccatcga caaaactttg caaaaacaaa aaaaaaaaaa aaaaactcga gggggggccc
420

ggtacccaat tcgccctata gtgagtcgta ttacaattca ctggcccgcg ctttacaacg
480

tcgtgactgg gaaaaccctg gcgttaccca acttaatcgc ctggcagcac atnccccttt
540

cgccagctgg gcgtatagcg aagaggcccg caccgatcgc ccttcccaac agttgcgcag
600

ccttgatggg gaatggcaca ctgtaagcgg taatactttg ttaaacaatc gccgtaaacc
660

ttttgtnaaa tcagctcacc ttttaaccca taggnccgaa atcggcgaaa atcccctata
720

aatc
724

<210> 104
<211> 724
<212> DNA
<213> Homo sapiens

<220>
<221> misc_feature
<222> (500)..(700)

<220>
<221> misc_feature
<222> (533)..(533)
<223> n is a, c, g, or t

<220>
<221> misc_feature
<222> (667)..(667)
<223> n is a, c, g, or t

<220>
<221> misc_feature
<222> (695)..(695)
<223> n is a, c, g, or t

<400> 104
   acagtcgcct ctcctcctcc tctacctcct ccttcaccac cacctcctct tcctcctcct
   60
   cctcctcctc ctcctccgcc aactcctcgg ctgcacacca gctctaagag cgagagtgaa
   120
   cgagagaggg agggagagag tgagagcgag cgagatcttt ggagagactc ttttttttgc
   180
   ctcctacttc tgtcttgaag ccagacaatc gacttcagct ctccctcccc tccctctttc
   240
   tccacgttct gctcccactc gctctcctgt ccccttcccc tcccctcccg gcggaaagcc
   300
   ccccgaaacc aacaaagctg agccgagaga aacaaacaaa acaaacacac cgggccagac
   360
   aagccatcga caaaactttg caaaaacaaa aaaaaaaaaa aaaaactcga gggggggccc
   420
   ggtacccaat tcgccctata gtgagtcgta ttacaattca ctggcccgcg ctttacaacg
   480
   tcgtgactgg gaaaaccctg gcgttaccca acttaatcgc ctggcagcac atnccccttt
   540
   cgccagctgg gcgtatagcg aagaggcccg caccgatcgc ccttcccaac agttgcgcag
   600
   ccttgatggg gaatggcaca ctgtaagcgg taatactttg ttaaacaatc gccgtaaacc
   660
   ttttgtnaaa tcagctcacc ttttaaccca taggnccgaa atcggcgaaa atcccctata
   720
   aatc
   724


<210>  105
<211>  1916
<212>  DNA
<213>  Homo sapiens

<400>  105
ggcacgaggg ccggagtcgt cttcgggacg cgcctgctct tcgcctttcg ctgcagtccg
60

tcgatttctt tctccaggaa gaaaaatggc atccgttgca gttgatccac aaccgagtgt
120

ggtgactcgg gtggtcaacc tgcccttggt gagctccacg tatgacctca tgtcctcagc
180

ctatctcagt acaaaggacc agtatcccta cctgaagtct gtgtgtgaga tggcagagaa
240

cggtgtgaag accatcacct ccgtggccat gaccagtgct ctgcccatca tccagaagct
300

agagccgcaa attgcagttg ccaataccta tgcctgtaag gggctagaca ggattgagga
360

gagactgcct attctgaatc agccatcaac tcagattgtt gccaatgcca aaggcgctgt
420

gactggggca aaagatgctg tgacgactac tgtgactggg gccaaggatt ctgtggccag
480

cacgatcaca ggggtgatgg acaagaccaa aggggcagtg actggcagtg tggagaagac
540

caagtctgtg gtcagtggca gcattaacac agtcttgggg agtcggatga tgcagctcgt
600

gagcagtggc gtagaaaatg cactcaccaa atcagagctg ttggtagaac agtacctccc
660

tctcactgag gaagaactag aaaaagaagc aaaaaaagtt gaaggatttg atctggttca
720

gaagccaagt tattatgtta gactgggatc cctgtctacc aagcttcact cccgtgccta
780

ccagcaggct ctcagcaggg ttaaagaagc taagcaaaaa agccaacaga ccatttctca
840

gctccattct actgttcacc tgattgaatt tgccaggaag aatgtgtata gtgccaatca
900

gaaaattcag gatgctcagg ataagctcta cctctcatgg gtagagtgga aaaggagcat
960

tggatatgat gatactgatg agtcccactg tgctgagcac attgagtcac gtactcttgc
1020

aattgcccgc aacctgactc agcagctcca gaccacgtgc cacaccctcc tgtccaacat
1080

ccaaggtgta ccacagaaca tccaagatca agccaagcac atgggggtga tggcaggcga
1140

catctactca gtgttccgca atgctgcctc ctttaaagaa gtgtctgaca gcctcctcac
1200

ttctagcaag gggcagctgc agaaaatgaa ggaatcttta gatgacgtga tggattatct
1260

tgttaacaac acgcccctca actggctggt aggtcccttt tatcctcagc tgactgagtc
1320

tcagaatgct caggaccaag gtgcagagat ggacaagagc agccaggaga cccagcgatc
1380

tgagcataaa actcattaaa cctgccccta tcactagtgc atgctgtggc cagacagatg
1440

acaccttttg ttatgttgaa attaacttgc taggcaaccc taaattggga agcaagtagc
1500

tagtataaag gccctcaatt gtagttgttt ccagctgaat taagagcttt aaagtttctg
1560

gcattagcag atgatttctg ttcacctggt aagaaaagaa tgataggctt gtcagagcct
1620

atagccagaa ctcagaaaaa attcaaatgc acttatgttc tcattctatg gccattgtgt
1680

tgcctctgtt actgtttgta ttgaataaaa acatcttcat gtgggctggg gtagaaactg
1740

gtgtctgctc tggtgtgatc tgaaaaggcg tcttcactgc tttatctcat gatgcttgct
1800

tgtaaaactt gattttagtt tttcatttct caaataggaa tactaccttt gaattcaata
1860

aaattcactg caggataaat aaaaaaaaaa aaaaaaaaaa aaaaaaaaaa aaaaaa
1916

<210> 106
<211> 1843

<212>  DNA
<213>  Homo sapiens

<400>  106
gggcacgagg gcagagccag ttcctagcgc agagccgcgc ccgccatgag ggagatcgtg
60

cacatccagg cgggccagtg cgggaaccag atcggcacca agttttggga agtgatcagc
120

gatgagcacg gcatcgaccc ggccggaggc tacgtgggag actcggcgct gcagctggag
180

agaatcaacg tctactacaa tgagtcatcg tctcagaaat atgtgcccag ggccgccctg
240

gtggacttag agccaggcac catggacagc gtgcggtctg ggccttttgg gcagcttttc
300

cggcctgaca acttcatctt tggccagacg ggtgcaggga acaactgggc gaaagggcac
360

tacacggagg gcgcggagct ggtggacgca gtgctggacg tggtgcggaa ggagtgcgag
420

cactgcgact gcctgcaggg cttccagctc acgcactcgc tgggcggcgg cacgggctca
480

ggcatgggca cgctgctcat cagcaagatc cgtgaggagt tcccggaccg catcatgaac
540

accttcagcg tcatgccctc gcccaaggtg tcggacacgg tggtggagcc ctacaatgcc
600

acactgtcgg tgcaccagct ggtggagaat acagacgaga cctactgcat cgacaacgag
660

gcgctctatg acatctgctt ccgcactctg aagctgacaa cgcccaccta cggggacctc
720

aaccacctgg tgtccgccac catgagtggg gtcaccacct cgctgcgctt cccgggccag
780

ctcaatgctg acctgcgcaa gctggcggtg aacatggtgc ccttcccgcg cctgcacttc
840

ttcatgcctg gcttcgcgcc gctcaccagc cgcggcagcc agcagtaccg ggccctgacc
900

gtgcccgagc tcacccagca gatgttcgac gccaggaaca tgatggccgc ctgcgatccg
960

cgccatggcc gctacctgac cgtggccacc gtgttccgcg ggcccatgtc catgaaggag
1020

gtggacgagc agatgctggc catccagagt aagaacagca gctacttcgt ggagtggatt
1080

cccaacaacg tgaaggtggc cgtgtgcgac atcccgcccc gcggcctgaa gatggcctcc
1140

accttcatcg gcaacagcac ggccatccag gagctgttca agcgcatctc cgagcagttc
1200

tcagccatgt tccggcgcaa ggccttcctg cactggttca cgggtgaggg catggatgaa
1260

atggagttca ccgaggcgga gagcaacatg aacgacctgg tatccgagta ccagcagtac
1320

caggatgcca ccgccaatga cggggaggaa gcttttgagg atgaggaaga ggagatcgat
1380

ggatagtcgg aatagagccg ccccaactca gatcctacaa cacgcaagtt ccttcttgaa
1440

ccctggtgcc tcctacccta tggccctgaa tggtgcactg gtttaattgt gttggtgtcg
1500

gcccctcaca aatgcagcca agtcatgtaa ttagtcatct ggaacaaaga ctaaaaacag
1560

cagagaattg cgggttctac ccagtcagaa gatcacacca tggagacttt ctactagagg
1620

acttgaaaga gaactgaggg gccacaaaat aaacttcacc ttccattaag tgttcaagca
1680

tgtctgcaaa ttaggaggga gttagaaaca gtctttttca tcctttgtga tgaagcctga
1740

aattgtgccg tgttgcctta tatgaatatg cagtatggga ctttgaaata atgattcata
1800

ataaaatact aaacgtgtgt cttcaaaaaa aaaaaaaaaa aaa
1843


<210>  107
<211>  661
<212>  DNA
<213>  Homo sapiens


<220>
<221>  misc_feature
<222>  (600)..(650)

<220>
<221>  misc_feature
<222>  (625)..(625)
<223>  n is a, c, g, or t

<220>
<221>  misc_feature
<222>  (630)..(630)
<223>  n is a, c, g, or t

<400>  107
ttttttaaat ttcaagctta ctttattaag cagcatataa caaacagctt ttaaagttaa
60

atattgttat ggccggggag tttcattaaa cagtatttct attcacaccc aaccactggt
120

aagtttgtag aacatctctt catctaaact ctcgttttgg tcttttgcac gtgttgagag
180

aaatatgtag ccaccaatga attcatgaac cactttgcaa tctacttcag tacaaatgaa
240

ggacaatcgt acttcatctg caaactctac ggtgaccatt ttgatttccc agttgacatt

300

ccactgtttc atgttgctga aacgccatgt tttaattgca tctccagtgc tggcatccat
360

ccgaatcagt ctgttgtatg caattccaat aagttcttct tttttgcccc cttggaacct
420

tgcaatgaag tgagtgatgc caaattcagg tagtgactgc caagcttgaa taaatctcat
480

cttggcttca attagactca tctgagctac attctgatgg gcctccaaga ttctcgctgg
540

tatctgcttg ttcttatact tttttgatag cggggagaca ccaaacattc aggaggtata
600

tctgtctgat ctggtctggt attanctgan gatcgtggtt aatgctgctc tttcgaacga
660

a
661


<210>  108
<211>  3270
<212>  DNA
<213>  Homo sapiens


<220>
<221>  misc_feature
<222>  (2708)..(2709)

<220>
<221>  misc_feature
<222>  (2708)..(2810)

<220>
<221>  misc_feature
<222>  (2708)..(2708)
<223>  n is a, c, g, or t

<220>
<221>  misc_feature
<222>  (2746)..(2746)
<223>  n is a, c, g, or t

<220>
<221>  misc_feature
<222>  (2806)..(2806)
<223>  n is a, c, g, or t

<400>  108
caaaaagtgt gtggaaaggt ggattgaggg agcgggaccc ccgcgggacc cgagggggcg
60

gcaggcgggg aacggggagt cagcccgcgc tgtgtctcgg ggccggccgg caggaaggag
120

ccatggctct ggacgggata aggatgccag atggctgcta cgcggacggg acgtgggaac
180

tgagtgtcca tgtgacggac ctgaaccgcg atatcaccct gagagtgacc ggcgaggtgc
240

acattggagg cgtgatgctt aagctggtgg agaaactcga tgtaaaaaaa gattggtctg
300

accatgctct ctggtgggaa aagaagagaa cttggcttct gaagacacat tggaccttag
360

ataagtatgg tattcaggca gatgctaagc ttcagttcac ccctcagcac aaactgctcc
420

gcctgcagct tcccaacatg aagtatgtga aggtgaaagt gaatttctct gatagagtct
480

tcaaagctgt ttctgacatc tgtaagactt ttaatatcag acaccccgaa gaactttctc
540

tcttaaagaa acccagagat ccaacaaaga aaaaaaagaa gaagctagat gaccagtctg
600

aagatgaggc acttgaatta gaggggcctc ttatcactcc tggatcagga agtatatatt
660

caagcccagg actgtatagt aaaacaatga cccccactta tgatgctcat gatggaagcc
720

ccttgtcacc aacttctgct tggtttggtg acagtgcttt gtcagaaggc aatcctggta
780

tacttgctgt cagtcaacca atcacgtcac cagaaatctt ggcaaaaatg ttcaagcctc
840

aagctcttct tgataaagca aaaatcaacc aaggatggct tgattcctca agatctctca
900

tggaacaaga tgtgaaggaa aatgaggcct tgctgctccg attcaagtat tacagctttt
960

ttgatttgaa tccaaagtat gatgcaatca gaatcaatca gctttatgag caggccaaat
1020

gggccattct cctggaagag attgaatgca cagaagaaga aatgatgatg tttgcagccc
1080

tgcagtatca tatcaataag ctgtcaatca tgacatcaga gaatcatttg aacaacagtg
1140

acaaagaagt tgatgaagtt gatgctgccc tttcagacct ggagattact ctggaagggg
1200

gtaaaacgtc aacaattttg ggtgacatta cttccattcc tgaacttgct gactacatta
1260

aagttttcaa gccaaaaaag ctgactctga aaggttacaa acaatattgg tgcaccttca
1320

aagacacatc catttcttgt tataagagca aagaagaatc cagtggcaca ccagctcatc
1380

agatgaacct caggggatgt gaagttaccc cagatgtaaa catttcaggc caaaaattta
1440

acattaaact cctgattcca gttgcagaag gcatgaatga aatctggctt cgttgtgaca
1500

atgaaaaaca gtatgcacac tggatggcag cctgcagatt agcctccaaa ggcaagacca
1560

tggcggacag ttcttacaac ttagaagttc agaatattct ttcctttctg aagatgcagc
1620

atttaaaccc agatcctcag ttaataccag agcagatcac gactgatata actcctgaat
1680

gtttggtgtc tccccgctat ctaaaaaagt ataagaacaa gcagataaca gcgagaatct
1740

tggaggccca tcagaatgta gctcagatga gtctaattga agccaagatg agatttattc
1800

aagcttggca gtcactacct gaatttggca tcactcactt cattgcaagg ttccaagggg
1860

gcaaaaaaga agaacttatt ggaattgcat acaacagact gattcggatg gatgccagca
1920

ctggagatgc aattaaaaca tggcgtttca gcaacatgaa acagtggaat gtcaactggg
1980

aaatcaaaat ggtcaccgta gagtttgcag atgaagtacg attgtccttc atttgtactg
2040

aagtagattg caaagtggtt catgaattca ttggtggcta catatttctc tcaacacgtg
2100

caaaagacca aaacgagagt ttagatgaag agatgttcta caaacttacc agtggttggg
2160

tgtgaataga aatactgttt aatgaaactc cacggccata acaatattta actttaaaag
2220

ctgtttgtta tatgctgctt aataaagtaa gcttgaaatt tatcatttta tcatgaaaac
2280

ttctttgcct taccagacca gttaatatgt gcactaaaca agcacgacta ttaatctatc
2340

atgttatgat ataataaact tgaatttggc acacattcct tagggccatg aattgaaaac
2400

tgaaatagtg ggcaaatcag gaacaaacca tcactgattt actgatttaa gctagccaaa
2460

ctgtaagaaa caagccatct attttaaagc tatccagggc ttaacctata tgaactctat
2520

ttatcatgtc taatgcatgt gatttaatgt atgtttaatt tgatatcatg ttttaaaata
2580

tcctacttct ggtagccatt taattcctcc ccctaccccc aaataaatca ggcatgcagg
2640

aggcctgata tttagtaatg tcattgtgtt tgaccttgaa ggaaaatgct attagtccgt
2700

cgtgcttnat ttgtttttgt ccttgaataa gcatgttatg tatatngtct cgtgttttta
2760

tttttacacc atattgtatt acacttttag tattcaccag cataancact gtctgcctaa
2820

aatatgcaac tctttgcatt acaatatgaa gtaaagttct atgaagtatg catttgtgt
2880

aactaatgta aaaacacaaa ttttataaaa ttgtacagtt ttttaaaaac tactcacaac
2940

tagcagatgg cttaaatgta gcaatctctg cgttaattaa atgcctttaa gagatataat
3000

taacgtgcag ttttaatatc tactaaatta agaatgactt cattatgatc atgatttgcc
3060

acaatgtcct taactctaat gcctggactg gccatgttct agtctgttgc gctgttacaa
3120

tctgtattgg tgctagtcag aaaattccta gctcacatag cccaaaaggg tgcgagggag
3180

aggtggatta ccagtattgt tcaataatcc atggttcaaa gactgtataa atgcatttta
3240

ttttaaataa aagcaaaact tttatttaaa
3270

<210> 109
<211> 543
<212> DNA
<213> Homo sapiens

<220>
<221> misc_feature
<222> (100)..(543)

<220>
<221> misc_feature
<222> (102)..(102)
<223> n is a, c, g, or t

<220>
<221> misc_feature
<222> (334)..(334)
<223> n is a, c, g, or t

<220>
<221> misc_feature
<222> (392)..(392)
<223> n is a, c, g, or t

<220>
<221> misc_feature
<222> (426)..(426)
<223> n is a, c, g, or t

<220>
<221> misc_feature
<222> (442)..(442)
<223> n is a, c, g, or t

<220>
<221> misc_feature
<222> (477)..(477)
<223> n is a, c, g, or t

<220>
<221> misc_feature
<222> (502)..(502)
<223> n is a, c, g, or t

<220>
<221>  misc_feature
<222>  (511)..(511)
<223>  n is a, c, g, or t

<220>
<221>  misc_feature
<222>  (530)..(531)
<223>  n is a, c, g, or t

<220>
<221>  misc_feature
<222>  (543)..(543)
<223>  n is a, c, g, or t

<400>  109
gcctggttat aaactttatt tttggctttc agcttccatt agttctatga gtcagataaa
60

atttacattt cctgactaat ttacaaggtt attgtgagga anaaatacca taatgtatgt
120

gaaagtgctt tataaactaa aacaggatac aaacataata gtacattaca ctcctatgtt
180

tgttttcaag aaagcttcat tgaatcattc accaatccta ggataattaa ccttttacct
240

ggattcaaag attgaggatt tgatggaaag ctggttgcat aaaacacatg agcaaaatgg
300

atatagtggg ttaggtttgc tcgaaaatgg atantgtgtt aagtttgctc ttatttttaa
360

gtctggattc ccagaagaca tttaagtttg anaaataact cctagttgtg gtatgttgct
420

ttcacntctg atgcctttga ancacactgc tttaatgcac atttacttta gatttanatc
480

tccagaaaga accttatccc tngttaggct nccaattgtt ggttccatcn nccattcccg
540

tcn
543


<210>  110
<211>  1342
<212>  DNA
<213>  Homo sapiens

<400>  110
gaattcgggc ggccgcggcc cgggtccctc gcaaagccgc tgccatcccg gagggcccag
60

ccagcgggct cccggaggct ggccgggcag gcgtggtgcg cggtaggagc tgggcgcgca
120

cggctaccgc gcgtggagga gacactgccc tgccgcgatg ggggcccggg gcgctccttc
180

acgccgtagg caagcggggc ggcggctgcg gtacctgccc accgggagct ttcccttcct
240

tctcctgctg ctgctgctct gcatccagct cgggggagga cagaagaaaa aggagaatct
300

tttagctgaa aaagtagagc agctgatgga atggagttcc agacgctcaa tcttccgaat
360

gaatggtgat aaattccgaa aatttataaa ggcaccacct cgaaactatt ccatgattgt
420

tatgttcact gctcttcagc ctcagcggca gtgttctgtg tgcaggcaag ctaatgaaga
480

atatcaaata ctggcgaact cctggcgcta ttcatctgct ttttgtaaca agctcttctt
540

cagtatggtg gactatgatg aggggacaga cgtttttcag cagctcaaca tgaactctgc
600

tcctacattc atgcattttc ctccaaaagg cagacctaag agagctgata cttttgacct
660

ccaaagaatt ggatttgcag ctgagcaact agcaaagtgg attgctgaca gaacggatgt
720

tcatattcgg gttttcagac cacccaacta ctctggtacc attgctttgg ccctgttagt
780

gtcgcttgtt ggaggtttgc tttatttgag aaggaacaac ttggagttca tctataacaa
840

gactggttgg gccatggtgt ctctgtgtat agtctttgct atgacttctg gccagatgtg
900

gaaccatatc cgtggacctc catatgctca taagaaccca cacaatggac aagtgagcta
960

cattcatggg agcagccagg ctcagtttgt ggcagaatca cacattattc tggtactgaa
1020

tgccgctatc accatgggga tggttcttct aaatgaagca gcaacttcga aaggcgatgt
1080

tggaaaaaga cggataattt gcctagtggg attgggcctg gtggtcttct tcttcagttt
1140

tctactttca atatttcgtt ccaagtacca cggctatcct tatagtgatc tggactttga
1200

gtgagaagat gtgatttgga ccatggcact taaaaactct ataacctcag ccttttaatt
1260

aaatgaagcc aagtgggatt tgcataaagt gaatgtttac catgaagata aactgttcct
1320

gactttatac tattttgaat tc
1342

<210> 111
<211> 3205
<212> DNA
<213> Homo sapiens

<400> 111
ggaattccct ggcctggccc ggccccgccg cgctcccgct cgccccgacc cgcactcggg
60

cccgcccggg ctccggcctg ccgccgcctc ttccttctcc agccggcagg cccgcgccgc
120

ttaggaggga gagcccaccc gcgccaggag gccgaacgcg gactcgccac ccggcttcag
180

aatggcagaa gatgatccat atttgggaag gcctgaacaa atgtttcatt tggatccttc
240

tttgactcat acaatattta atccagaagt atttcaacca cagatggcac tgccaacagc
300

agatggccca taccttcaaa tattagagca acctaaacag agaggatttc gtttccgtta
360

tgtatgtgaa ggcccatccc atggtggact acctggtgcc tctagtgaaa agaacaagaa
420

gtcttaccct caggtcaaaa tctgcaacta tgtgggacca gcaaaggtta ttgttcagtt
480

ggtcacaaat ggaaaaaata tccacctgca tgcccacagc ctggtgggaa aacactgtga
540

ggatgggatc tgcactgtaa ctgctggacc caaggacatg gtggtcggct tcgcaaacct
600

gggtatactt catgtgacaa agaaaaaagt atttgaaaca ctggaagcac gaatgacaga
660

ggcgtgtata aggggctata atcctggact cttggtgcac cctgaccttg cctatttgca
720

agcagaaggt ggaggggacc ggcagctggg agatcgggaa aaagagctaa tccgccaagc
780

agctctgcag cagaccaagg agatggacct cagcgtggtg cggctcatgt ttacagcttt
840

tcttccggat agcactggca gcttcacaag gcgcctggaa cccgtggtat cagacgccat
900

ctatgacagt aaagccccca atgcatccaa cttgaaaatt gtaagaatgg acaggacagc
960

tggatgtgtg actggagggg aggaaattta tcttctttgt gacaaagttc agaaagatga
1020

catccagatt cgattttatg aagaggaaga aaatggtgga gtctgggaag gatttggaga
1080

tttttccccc acagatgttc atagacaatt tgccattgtc ttcaaaactc caaagtataa
1140

agatattaat attacaaaac cagcctctgt gtttgtccag cttcggagga aatctgactt
1200

ggaaactagt gaaccaaaac ctttcctcta ctatcctgaa atcaaagata aagaagaagt
1260

gcagaggaaa cgtcagaagc tcatgcccaa tttttcggat agtttcggcg gtggtagtgg
1320

tgccggagct ggaggcggag gcatgtttgg tagtggcggt ggaggagggg gcactggaag
1380

tacaggtcca gggtatagct tcccacacta tggatttcct acttatggtg ggattacttt
1440

ccatcctgga actactaaat ctaatgctgg gatgaagcat ggaaccatgg acactgaatc
1500

taaaaaggac cctgaaggtt gtgacaaaag tgatgacaaa aacactgtaa acctctttgg
1560

gaaagttatt gaaaccacag agcaagatca ggagcccagc gaggccaccg ttgggaatgg
1620

tgaggtcact ctaacgtatg caacaggaac aaaagaagag agtgctggag ttcaggataa
1680

cctctttcta gagaaggcta tgcagcttgc aaagaggcat gccaatgccc ttttcgacta
1740

cgcggtgaca ggagacgtga agatgctgct ggccgtccag cgccatctca ctgctgtgca
1800

ggatgagaat ggggacagtg tcttacactt agcaatcatc caccttcatt ctcaacttgt
1860

gagggatcta ctagaagtca catctggttt gatttctgat gacattatca acatgagaaa
1920

tgatctgtac cagacgccct tgcacttggc agtgatcact aagcaggaag atgtggtgga
1980

ggatttgctg agggctgggg ccgacctgag ccttctggac cgcttgggta actctgtttt
2040

gcacctagct gccaaagaag gacatgataa agttctcagt atcttactca agcacaaaaa
2100

ggcagcacta cttcttgacc accccaacgg ggacggtctg aatgccattc atctagccat
2160

gatgagcaat agcctgccat gtttgctgct gctggtggcc gctggggctg acgtcaatgc
2220

tcaggagcag aagtccgggc gcacagcact gcacctggct gtggagcacg acaacatctc
2280

attggcaggc tgcctgctcc tggagggtga tgcccatgtg gacagtacta cctacgatgg
2340

aaccacaccc ctgcatatag cagctgggag agggtccacc aggctggcag ctcttctcaa
2400

agcagcagga gcagatcccc tggtggagaa ctttgagcct ctctatgacc tggatgactc
2460

ttgggaaaat gcaggagagg atgaaggagt tgtgcctgga accacgcctc tagatatggc
2520

caccagctgg caggtatttg acatattaaa tgggaaacca tatgagccag agtttacatc
2580

tgatgattta ctagcacaag gagacatgaa acagctggct gaagatgtga agctgcagct
2640

gtataagtta ctagaaattc ctgatccaga caaaaactgg gctactctgg cgcagaaatt
2700

aggtctgggg atacttaata atgccttccg gctgagtcct gctccttcca aaacacttat
2760

ggacaactat gaggtctctg ggggtacagt cagagagctg gtggaggccc tgagacaaat
2820

gggctacacc gaagcaattg aagtgatcca ggcagcctcc agcccagtga agaccacctc
2880

tcaggcccac tcgctgcctc tctcgcctgc ctccacaagg cagcaaatag acgagctccg
2940

agacagtgac agtgtctgcg acacgggcgt ggagacatcc ttccgcaaac tcagctttac
3000

cgagtctctg accagtggtg cctcactgct aactctcaac aaaatgcccc atgattatgg
3060

gcaggaagga cctctagaag gcaaaattta gcctgctgac aatttcccac accgtgtaaa
3120

ccaaagccct aaaattcact gcgttgtcca caagacagaa gctgaagtgc atcccaaagg
3180

tgctcagaga gccggccgga attcc
3205

<210> 112
<211> 3930
<212> DNA
<213> Homo sapiens

<400> 112
gggcgggagg aggactgtat ctctggatgc ctggggcctg gtttcagggc ctgatttatt
60

cctcttcctg ggagctcact caggaaaggt cctttctggg gatcacccca ttggctgaag
120

atgagaccat tcttcctctt gtgttttgcc ctgcctggcc tcctgcatgc ccaacaagcc
180

tgctcccgtg gggcctgcta tccacctgtt ggggacctgc ttgttgggag gacccggttt
240

ctccgagctt catctacctg tggactgacc aagcctgaga cctactgcac ccagtatggc
300

gagtggcaga tgaaatgctg caagtgtgac tccaggcagc ctcacaacta ctacagtcac
360

cgagtagaga atgtggcttc atcctccggc cccatgcgct ggtggcagtc ccagaatgat
420

gtgaaccctg tctctctgca gctggacctg gacaggagat tccagcttca agaagtcatg
480

atggagttcc gagggcccat gcctgccggc atgctgattg agcgctcctc agacttcggt
540

aagacctggc gagtgtacca gtacctggct gccgactgca cctccacctt ccctcgggtc
600

cgccagggtc ggcctcagag ctggcaggat gttcggtgcc agtccctgcc tcagaggcct

660

aatgcacgcc taaatggggg gaaggtccaa cttaacctta tggatttagt gtctgggatt
720

ccagcaactc aaagtcaaaa aattcaagag gtgggggaga tcacaaactt gagagtcaat
780

ttcaccaggc tggcccctgt gccccaaagg ggctaccacc ctcccagcgc ctactatgct
840

gtgtcccagc tccgtctgca ggggagctgc ttctgtcacg gccatgctga tcgctgcgca
900

cccaagcctg gggcctctgc aggctccacc gctgtgcagg tccacgatgt ctgcgtctgc
960

cagcacaaca ctgccggccc aaattgtgag cgctgtgcac ccttctacaa caaccggccc
1020

tggagaccgg cggagggcca ggacgcccat gaatgccaaa ggtgcgactg caatgggcac
1080

tcagagacat gtcactttga ccccgctgtg tttgccgcca gccagggggc atatggaggt
1140

gtgtgtgaca attgccggga ccacaccgaa ggcaagaact gtgagcggtg tcagctgcac
1200

tatttccgga accggcgccc gggagcttcc attcaggaga cctgcatctc ctgcgagtgt
1260

gatccggatg gggcagtcgc aggggctccc tgtgacccag tgaccgggca gtgtgtgtgc
1320

aaggagcatg tgcagggaga gcgctgtgac ctatgcaagc cgggcttcac tggactcacc
1380

tacgccaacc cgcgacggtg ccaccgctgt gactgcaaca tcctggggtc ccgggagatg
1440

ccgtgtgacg aggagagtgg gcgctgcctt tgtctgccca acgtggtggg tcccaaatgt
1500

gaccagtgtg ctccctacca ctggaagctg gccagtggcc agggctgtga accgtgtgcc
1560

tgcgacccgc acaactccct cagcccacag tgcaaccagt tcacagggca gtgcccctgt
1620

cgggaaggct ttggtggcct gatgtgcagc gctgcagcca tccgccagtg tccagaccgg
1680

acctatggag acgtggccac aggatgccga gcctgtgact gtgatttccg ggaacagag
1740

ggcccgggct gcgacaaggc atcaggccgc tgcctctgcc gccctggctt gaccgggccc
1800

cgctgtgacc agtgccagcg aggctactgc aatcgctacc cggtgtgcgt ggcctgccac
1860

ccttgcttcc agacctatga tgcggacctc cgggagcagg ccctgcgctt tggtagactc
1920

ccgaatgcca ccgccagcct gtggtcaggg cctgggctgg aggaccgtgg cctggcctcc

1980

cggatcctag atgcaaagag taagattgag cagatccgag cagttctcag cagccccgca
2040

gtcacagagc aggaggtggc tcaggtggcc agtgccatcc tctccctcag gcgaactctc
2100

cagggcctgc agctggatct gcccctggag gaggagacgt tgtcccttcc gagagacctg
2160

gagagtcttg acagaagctt caatggtctc cttactatgt atcagaggaa gagggagcag
2220

tttgaaaaaa taagcagtgc tgatccttca ggagccttcc ggatgctgag cacagcctac
2280

gagcagtcag cccaggctgc tcagcaggtc tccgacagct cgcgcctttt ggaccagctc
2340

agggacagcc ggagagaggc agagaggctg gtgcggcagg cgggaggagg aggaggcacc
2400

ggcagcccca agcttgtggc cctgaggttg gagatgtctt cgttgcctga cctgacaccc
2460

accttcaaca agctctgtgg caactccagg cagatggctt gcaccccaat atcatgccct
2520

ggtgagctat gtccccaaga caatggcaca gcctgtgcgt cccgctgcag gggtgtcctt
2580

cccagggccg gtggggcctt cttgatggcg gggcaggtgg ctgagcagct gcggggcttc
2640

aatgcccagc tccagcggac caggcagatg attagggcag ccgaggaatc tgcctcacag
2700

attcaatcca gtgcccagcg cttggagacc caggtgagcg ccagccgctc ccagatggag
2760

gaagatgtca gacgcacacg gctcctaatc cagcaggtcc gggacttcct aacagacccc
2820

gacactgatg cagccactat ccaggaggtc agcgaggccg tgctggccct gtggctgccc
2880

acagactcag ctactgttct gcagaagatg aatgagatcc aggccattgc agccaggctc
2940

cccaacgtgg acttggtgct gtcccagacc aagcaggaca ttgcgcgtgc ccgccggttg
3000

caggctgagg ctgaggaagc caggagccga gcccatgcag tggagggcca ggtggaggat
3060

gtggttggga acctgcggca ggggacagtg gcactgcagg aagctcagga caccatgcaa
3120

ggcaccagcc ggtcccttcg gcttatccag gacagggttg ctgaggttca gcaggtactg
3180

cggccagcag aaaagctggt gacaagcatg accaagcagc tgggtgactt ctggacacgg
3240

atggaggagc tccgccacca agcccggcag cagggggcag aggcagtcca ggcccagcag

3300

cttgcggaag gtgccagcga gcaggcattg agtgcccaag agggatttga gagaataaaa
3360

caaaagtatg ctgagttgaa ggaccggttg ggtcagagtt ccatgctggg tgagcagggt
3420

gcccggatcc agagtgtgaa gacagaggca gaggagctgt ttggggagac catggagatg
3480

atggacagga tgaaagacat ggagttggag ctgctgcggg gcagccaggc catcatgctg
3540

cgctcagcgg acctgacagg actggagaag cgtgtggagc agatccgtga ccacatcaat
3600

gggcgcgtgc tctactatgc cacctgcaag tgatgctaca cgttccagcc cgttgcccca
3660

ctcatctgcg cgctttgctt ttggttgggg ggcagattgg gttggaatgc tttccatctc
3720

caggagactt tcatgtagcc caaagtacag cctggaccac ccctggtgtg tgtagctagt
3780

aagattaccc tgagctgcag ctgagcctga gccaatggga cagttacact tgacagacaa
3840

agatggtgga gattggcatg ccattgaaac taagagctct caagtcaagg aagctgggct
3900

gggcagtatc ccccgccttt agttctccac
3930


<210>  113
<211>  624
<212>  DNA
<213>  Homo sapiens

<400>  113
tttgtttttt gttttttaga tttcaaggca aggcacgtaa tgtggacatt atatcttcgt
60

gcaaattagg attactggaa agagtatttt taattaaaat tttaagagac atagaggcaa
120

aatgtgtctg cccatgcaca ctatggatct gtcaatacaa gaaatttgtt gaacaaggct
180

aatgtctgaa agcaccatgc aagttttcag caccctgatt acatttgttt tctcaagagt
240

gcgtttttat atcctacacc ctggcgttcc cagtttgtaa actgtaagct ttacccttgt
300

gacatggatt tgcctgcctc tttgtctcta taatgcagat tttatagaac cttttgtaca
360

ccctatgggt tcttgatgca accagtaatt ttaaataaat aaattctacc tccaaggagg
420

ctgcagctaa accaacataa gtgctgtgtt ttcattaatt ttatttttct caagcacatg
480

atttgtcttg atttaatgcc tttttaatgc cctcaaaaac tgaggggaaa ataaattcgt
540

tcaaaattat tttaaattct ttttaatccc ctcaatttag agtccaaggg ctgaaggact
600

tataatcatg attccccttt agat
624

<210> 114
<211> 1166
<212> DNA
<213> Homo sapiens

<400> 114
gcaagaatgg tgcctgtcct gctgtctctg ctgctgcttc tgggtcctgc tgtcccccag
60

gagaaccaag atggtcgtta ctctctgacc tatatctaca ctgggctgtc caagcatgtt
120

gaagacgtcc ccgcgtttca ggcccttggc tcactcaatg acctccagtt ctttagatac
180

aacagtaaag acaggaagtc tcagcccatg ggactctgga gacaggtgga aggaatggag
240

gattggaagc aggacagcca acttcagaag gccagggagg acatctttat ggagaccctg
300

aaagacattg tggagtatta caacgacagt aacgggtctc acgtattgca gggaaggttt
360

ggttgtgaga tcgagaataa cagaagcagc ggagcattct ggaaatatta ctatgatgga
420

aaggactaca ttgaattcaa caaagaaatc ccagcctggg tccccttcga cccagcagcc
480

cagataacca agcagaagtg ggaggcagaa ccagtctacg tgcagcgggc caaggcttac
540

ctggaggagg agtgccctgc gactctgcgg aaatacctga aatacagcaa aaatatcctg
600

gaccggcaag atcctccctc tgtggtggtc accagccacc aggccccagg agaaaagaag
660

aaactgaagt gcctggccta cgacttctac ccagggaaaa ttgatgtgca ctggactcgg
720

gccggcgagg tgcaggagcc tgagttacgg ggagatgttc ttcacaatgg aaatggcact
780

taccagtcct gggtggtggt ggcagtgccc ccgcaggaca cagcccccta ctcctgccac
840

gtgcagcaca gcagcctggc ccagcccctc gtggtgccct gggaggccag ctaggaagca
900

agggttggag gcaatgtggg atctcagacc cagtagctgc ccttcctgcc tgatgtggga
960

gctgaaccac agaaatcaca gtcaatggat ccacaaggcc tgaggagcag tgtgggggga
1020

cagacaggag gtggatttgg agaccgaaga ctgggatgcc tgtcttgagt agacttggac
1080

ccaaaaaatc atctcacctt gagcccaccc ccaccccatt gtctaatctg tagaagctaa
1140

taaataatca tccctccttg cctagc
1166

<210> 115
<211> 772
<212> DNA
<213> Homo sapiens

<400> 115
gccaaagagt tcaagacacg tctggggatc tttcttcaca aatcagagct gggctgcgat
60

actgggagta ctggcaagtt cgagtggggc agtaaacaca gcaaagagaa tagaaacttc
120

tcagaagatg tgctggggtg gagagagtcg ttcgacctgc tgctgagcag taaaaatgga
180

gtggctgcct tccacgcttt cctgaagaca gagttcagtg aggagaacct ggagttctgg
240

ctggcctgtg aggagttcaa gaagatccga tcagctacca agctggcctc cagggcacac
300

cagatctttg aggagttcat ttgcagtgag gcccctaaag aggtcaacat tgaccatgag
360

acccgcgagc tgacgaggat gaacctgcag actgccacag ccacatgctt tgatgcggct
420

caggggaaga cacgtaccct gatggagaag gactcctacc cacgcttcct gaagtcgcct
480

gcttaccggg acctggctgc ccaagcctca gccgctctgc cactctgtcc agctgcagcc
540

tggacgagcc ctcacacacc tgagtctcca cggcagtgag gaagccagcg ggaagagagg
600

ttgagtcacc catccccgag gtggctgccc tgtgtgggag gcaggttctg caaagcaagt
660

gcaagaggac aaaaaaaaaa aaaaaaaaac gggggcacta gcgggggggg aaaaaggggt
720

tcgaaaggcc aggtgccaag ggtccggaac ccattttctt gtccaggggc ac
772

<210> 116
<211> 2132
<212> DNA
<213> Homo sapiens

<400> 116
gccatgtgcc gcaccctggc cgccttcccc accacctgcc tggagagagc caaagagttc
60

aagacacgtc tggggatctt tcttcacaaa tcagagctgg gctgcgatac tgggagtact
120

ggcaagttcg agtggggcag taaacacagc aaagagaata gaaacttctc agaagatgtg
180

ctggggtgga gagagtcgtt cgacctgctg ctgagcagta aaaatggagt ggctgccttc
240

cacgctttcc tgaagacaga gttcagtgag gagaacctgg agttctggct ggcctgtgag
300

gagttcaaga agatccgatc agctaccaag ctggcctcca gggcacacca gatctttgag
360

gagttcattt gcagtgaggc ccctaaagag gtcaacattg accatgagac ccgcgagctg
420

acgaggatga acctgcagac tgccacagcc acatgctttg atgcggctca ggggaagaca
480

cgtaccctga tggagaagga ctcctaccca cgcttcctga agtcgcctgc ttaccgggac
540

ctggctgccc aagcctcagc cgcctctgcc actctgtcca gctgcagcct ggacgagccc
600

tcacacacct gagtctccac ggcagtgagg aagccagccg ggaagagagg ttgagtcacc
660

catccccgag gtggctgccc ctgtgtggga ggcaggttct gcaaagcaag tgcaagagga
720

caaaaaaaaa aaaaaaaaaa aaaatgcgct ccagcagcct gtttgggaag cagcagtctc
780

tccttcaaat actgtgggac tcatgctgga gaggagccgc ccacttccag gacctgtgaa
840

taagggctaa tgatgagggt tggtggggct ctctgtgggg caaaaaggtg gtatgggggt
900

tagcactggc tctcgttctc accggagaag gaagtgttct agtgtggttt aggaaacatg
960

tggataaagg gaaccatgaa aatgagagga ggaaagacat ccagatcagc tgttttgcct
1020

gttgctcagt tgactctgat tgcatcctgt tttcctaatt cccagactgt tctgggcacg
1080

gaagggaccc tggatgtgga gtcttcccct ttggccctcc tcactggcct ctgggctagc
1140

ccagagtccc ttagcttgta cctcgtaaca ctcctgtgtg tctgtccagc cttgcagtca
1200

tgtcaaggcc agcaagctga tgtgactctt gccccatgcg agatatttat acctcaaaca
1260

ctggcctgtg agccctttcc aagtcagtgg agagccctga aaggaggctc acttgaatcc
1320

agctcagtgc tctgggtggc cccctgcagg tggcccctga ccctgcgttg cagcagggtc
1380

cacctgtgag caggcccgcc ctggggcctc ttcctggatg tgccctctct gagttctgtg
1440

ctgtctcttg gaggcagggc ccaggagaac aaagtgtgga ggcctcgggg agtggctttt
1500

ccagctctca tgccccgcag tgtggaacaa ggcagaaaag gatcctagga aataagtctc
1560

ttggcggtcc ctgagagtcc tgctgaaatc cagccagtgt tttttgtggt atgagaacag
1620

gcaaaaagag atgccccgag atagaagggg agccttgtgt ttctttcctg cagacgtgag
1680

atgaacactg gagtgggcag aggtggccca ggaccatggc acccttagag tgcagaagct
1740

ggggggagag gctgcttcga agggcaggac tggggataat cagaacctgc ctgtcacctc
1800

agggcatcac tgaacaaaca tttcctgatg ggaactcctg cggcagagcc caggctgggg
1860

aagtgaacta cccagggcag cccctttgtg gcccaggata atcaacactg ttctctctgt
1920

accatgagct cctccaggag attatttaag tgtattgtat cattggtttt ctgtgattgt
1980

cataacattg tttttgttat tgttggtgct gttgttattt attattgtaa tttcagtttg
2040

cctctactgg agaatctcag caggggtttc agcctgactg tctccctttc tctaccagac
2100

tctacctctg aatgtgctgg gaacctcttg ga
2132

<210> 117
<211> 988
<212> DNA
<213> Homo sapiens

<400> 117
ggtgacttgc agctggagag aatcaacgtg tactacaatg aggctgctgg taacaaatat
60

gtacctcggg ccatcctggt ggatctggag cctggcacca tggactctgt caggtctgga
120

cccttcggcc agatcttcag accagacaac ttcgtgttcg gccagagtgg agccgggaat
180

aactgggcca agggccacta cacagaggga gccgagctgg tcgactcggt cctggatgtg
240

gtgaggaagg agtcagagag ctgtgactgt ctccagggct tccagctgac ccactctctg
300

gggggcggca cggggtccgg gatgggcacc ctgctcatca gcaagatccg ggaagagtac
360

ccagaccgca tcatgaacac cttcagcgtc atgccctcac ccaaggtgtc agacacggtg
420

gtggagccct acaacgccag cctctctgtc caccagctgg tggaaaacac agatgaaacc
480

tactccattg ataacgaggc ctgtatgaca tctgcttccg caccctgaag ctgaccaacc
540

ccaacctacg ggggacctca accacctggt gtcggcacca tgagcggggt caccacctgc
600

ctgcgcttcc cgggccagct gaacgcagaa cctgcgcaag ctggcggtga acatggtgcc
660

cttccctcgc tgcacttctt catgccggct tcgcgcccct gaccagccgg ggcagcagca
720

gtaccgggcg ctcacggtgc ccgaactcac ccagcagatt gtgcgactcc aggaacatga
780

tggccggctt ggaaccgggc cacggccggt actgaacgga tggtgccaac ttaccggggc
840

cgaatgtcca ggaaggaggg gcgagcgagg ccaacggtgc gaagaggaac gagagaacgg
900

gaaggagccc cacacgaaaa gacggtggca accgcccggg agagaaacag gcccacatcg
960

ggaacgagca ccagagagga agcgatca
988

<210> 118
<211> 2106
<212> DNA
<213> Homo sapiens

<400> 118
aattccggca gagctccgtt tcctcataga acaaagaaca tccaccatgc agctcttcct
60

cctcttgtgc ctggtgcttc tcagccctca gggggcctcc cttcaccgcc accacccccg
120

ggagatgaag aagagagtcg aggacctcca tgtaggtgcc acggtggccc ccagcagcag
180

aagggacttt acctttgacc tctacagggc cttggcttcc gctgccccca gccagaacat
240

cttcttctcc cctgtgagca tctccatgag cctggccatg ctctccctgg gggctgggtc
300

cagcacaaag atgcagatcc tggagggcct gggcctcaac ctccagaaaa gctcagagaa
360

ggagctgcac agaggctttc agcagctcct tcaggaactc aaccagccca gagatggctt
420

ccagctgagc ctcggcaatg cccttttcac cgacctggtg gtagacctgc aggacacctt
480

cgtaagtgcc atgaagacgc tgtacctggc agacactttc cccaccaact ttagggactc
540

tgcaggggcc atgaagcaga tcaatgatta tgtggcaaag caaacgaagg gcaagattgt

600

ggacttgctt aagaacctcg atagcaatgc ggtcgtgatc atggtgaatt acatcttctt
660

taaagctaag tgggagacaa gcttcaacca caaaggcacc caagagcaag acttctacgt
720

gacctcggag actgtggtgc gggtacccat gatgagccgc gaggatcagt atcactacct
780

cctggaccgg aacctctcct gcagggtggt gggggtcccc taccaaggca atgccacggc
840

tttgttcatt ctccccagtg agggaaagat gcagcaggtg gagaatggac tgagtgagaa
900

aacgctgagg aagtggctta agatgttcaa aaagaggcag ctcgagcttt accttcccaa
960

attctccatt gagggctcct atcagctgga gaaagtcctc cccagtctgg ggatcagtaa
1020

cgtcttcacc tcccatgctg atctgtcccg catcagcaac cactcaaata tccaggtgtc
1080

tgagatggtg cacaaagctg tggtggaggt ggacgagtcg ggaaccagag cagcggcagc
1140

cacggggaca atcttcactt tcaggtcggc ccgcctgaac tctcagaggc tagtgttcaa
1200

caggcccttt ctgatgttca ttgtggataa caacatcctc ttccttggca aagtgaaccg
1260

cccctgaggt ggggcttctc ctgaaatcta caggcctcag ggtgggagat gaagggggct
1320

atgctatggc ccatctgtat gctggtagct agtgatttac gacaggttta gttgactaga
1380

tgaggcatta caaataatat tactctatgg atgattgctt ccacccacac gactgcaaca
1440

tacaggtgcc ttggggaaat gtggagaaca ttcaatctgc cgtcactatt catcaatgaa
1500

gattagcact gagatccaga gaggctggat ggacttgctc aagttcacca gcatggtagt
1560

ggcaaagaga ggtccagagt cctggccctt gatgccgagc tcatgccaca aagctcagta
1620

ggagggatgt tccagtggat gagggccagc caggaagcac aggtccaagg ctggtcccac
1680

acttatcagc agcaacaact gtcagttcat cctgcatggg aaaaatgttg gaatgggagt
1740

ctgaaatggg gctactgttt cagtcctaac gtgctgtgtg acattgggac aacactttcc
1800

ctctctggac ctcagtttcc ctctgtatac aaggatcaga ttcttgctgt gacccaagaa
1860

ctcctgaaat catatagaaa ggctggggtg ggccctgtca ttcgtggttg atttcaatac

1920

actcaagtgc cattcatcct ttaagaaaaa catctggata tcaaggtgga aatggcccat
1980

ttaatgattg attatatcat tttgtggata tagttataat ctgatgggcc tggctgggag
2040

tggaagaagg gaagcctttt gcaaatagta gagtgtcagt tgcaggtgcc aatgactaac
2100

tttttg
2106


<210>  119
<211>  1553
<212>  DNA
<213>  Homo sapiens

<400>  119
tctaaaggag gtggagagcg caccgcagcc cggtgcagcc cggtgcagcc ctggctttcc
60

cctcgctgcg gcccgtgccc cctttcgcgt ccgcaaccag aagcccagtg cggcgccagg
120

agccggaccc gcgcccgcac cgctcccggg accgcgaccc cggccgccca gagatgaccg
180

cgaccgaagc cctcctgcgc gtcctcttgc tcctgctggc tttcggccac agcacctatg
240

gggctgaatg cttcccggcc tgcaaccccc aaaatggatt ctgcgaggat gacaatgttt
300

gcaggtgcca gcctggctgg cagggtcccc tttgtgacca gtgcgtgacc tctcccggct
360

gccttcacgg actctgtgga gaacccgggc agtgcatttg caccgacggc tgggacgggg
420

agctctgtga tagagatgtt cgggcctgct cctcggcccc ctgtgccaac aacgggacct
480

gcgtgagcct ggacggtggc ctctatgaat gctcctgtgc ccccgggtac tcgggaaagg
540

actgccagaa aaaggacggg ccctgtgtga tcaacggctc cccctgccag cacggaggca
600

cctgcgtgga tgatgagggc cgggcctccc atgcctcctg cctgtgcccc cctggcttct
660

caggcaattt ctgcgagatc gtggccaaca gctgcacccc caacccatgc gagaacgacg
720

gcgtctgcac tgacattggg ggcgacttcc gctgccggtg cccagccggc ttcatcgaca
780

agacctgcag ccgcccggtg accaactgcg ccagcagccc gtgccagaac gggggcacct
840

gcctgcagca cacccaggtg agctacgagt gtctgtgcaa gcccgagttc acaggtctca
900

cctgtgtcaa gaagcgcgcg ctgagccccc agcaggtcac ccgtctgccc agcggctatg
960

ggctggccta ccgcctgacc cctggggtgc acgagctgcc ggtgcagcag ccggagcacc
1020

gcatcctgaa ggtgtccatg aaagagctca acaagaaaac ccctctcctc accgagggcc
1080

aggccatctg cttcaccatc ctgggcgtgc tcaccagcct ggtggtgctg ggcactgtgg
1140

gtatcgtctt cctcaacaag tgcgagacct gggtgtccaa cctgcgctac aaccacatgc
1200

tgcggaagaa gaacctgctg cttcagtaca acagcgggga ggacctggcc gtcaacatca
1260

tcttccccga gaagatcgac atgaccacct tcagcaagga ggccggcgac gaggagatct
1320

aagcagcgtt cccacagccc cctctagatt cttggagttc cgcagagctt actatacgcg
1380

gtctgtccta atctttgtgg tgttcgctat ctcttgtgtc aaatctggtg aacgctacgc
1440

ttacatatat tgtctttgtg ctgctgtgtg acaaacgcaa tgcaaaaaca atcctctttc
1500

tctctcttaa tgcatgatac agaataataa taagaatttc atctttaaat gag
1553

<210> 120
<211> 772
<212> DNA
<213> Homo sapiens

<400> 120
ccgttgtctt cctcaccctg ctctcggtcg tgatcttgat cttcttttac ctgtacaaga
60

acaaaggcag ctacgtcacc tatgaaccta cagaaggtga gcccagtgcc atcgtccaga
120

tggagagtga cttggccaag ggcagcgaga aagaggaata tttcatctaa tgactcccag
180

gccccaagga gcttattcct ggctccatcg ctaacacgtt gactgcttat tatgggaaag
240

ttttctctga agccagggag aagcattgat tgatgtgggc aaatccaagc tccagccagg
300

tcgcagtccc aaatgccgac atcactgact ccagggacca gggacatgga gaaagctgtt
360

tatgatatct ttaaccaggc cctcttacta gagctggtgt ttgtgactgg ccaacaagat
420

gtggctatgc caggggacat ctgagtatgt gcccagtcat cttttttcac aggttgaagg
480

gagagaaaag attttgagtt aaggtcattg gctgctctac tctgtcccct acctggtcac
540

ctagtgatag ccccagtgga gatactgtcc atacaaggtc ttcccagagg ctggatacca
600

cagtaaaagg ccaggccagg aggggtagga gactatggag atcttacctc ctgataaatg
660

tgctacaccc cctaatctga gcccttcctt tccgtgttcc ccaacaacct catgcttacg
720

tgatttttat tcaaattaaa aattttcatt gctacagaaa aaaaaaaaaa aa
772


<210> 121
<211> 3552
<212> DNA
<213> Homo sapiens

<400> 121
ctgactggtg ctccctctct ttcatcttgg gctgtctgca tgtgtctcat tcccccactc
60

tctcctgtgc ctcccctcta ctctaataat caggtccagg tttctctgta ctgggagaag
120

acctgtggct ggagcaggca gggatgcacc ctatctgttc cccattcctc caggtgggag
180

ggagaaggag taacccactt tattggccac agatgcaggg gagaaaggag aaagcatgct
240

gggagctgga aagagcccta agatcacctg gtttctagag agtggagcct gcttcctgcc
300

taggcccctc ccacaatgct tgtcgccggt cttcttctct gggcttccct actgactggg
360

gcctggccat ccttccctac ccaggaccac ctcccggcca cgccccgggt acggctctca
420

ttcaaagagc tgaaggccac aggcaccgcc cacttcttca acttcctgct caacacaacc
480

gactaccgaa tcttgctcaa ggacgaggac cacgaccgca tgtacgtggg cagcaaggac
540

tacgtgctgt ccctggacct gcacgacatc aaccgcgagc ccctcattat acactgggca
600

gcctccccac agcgcatcga ggaatgcgtg ctctcaggca aggatgtcaa cggcgagtgt
660

gggaacttcg tcaggctcat ccagccctgg aaccgaacac acctgtatgt gtgcgggaca
720

ggtgcctaca accccatgtg cacctatgtg aaccgcggac gccgcgccca ggattacatc
780

ttctacctgg agcctgagcg actcgagtca gggaagggca agtgtccgta cgatcccaag
840

ctggacacag catcggccct catcaatgag gagctctatg ctggtgtgta catcgatttt
900

atgggcactg atgcagccat cttccgcaca cttggaaagc agacagccat gcgcacggat

960

cagtacaact cccggtggct gaacgacccg tcgttcatcc atgctgagct cattcctgac
1020

agtgcggaga atgatgataa gctttacttc ttcttccgtg agcggtcggc agaggcgccg
1080

cagagccccg cggtgtacgc ccgcatcggg cgcatttgcc tgaacgatga cggtggtcac
1140

tgttgcctgg tcaacaagtg gagcacattc ctgaaggcgc ggctcgtctg ctctgtcccg
1200

ggcgaggatg gcattgagac tcactttgat gagctccagg acgtgtttgt ccagcagacc
1260

caggacgtga ggaaccctgt catttacgct gtctttacct cctctggctc cgtgttccga
1320

ggctctgccg tgtgtgtcta ctccatggct gatattcgca tggtcttcaa cgggcccttt
1380

gcccacaaag aggggcccaa ctaccagtgg atgcccttct cagggaagat gccctaccca
1440

cggccgggca cgtgccctgg tggaaccttc acgccatcta tgaagtccac caaggattat
1500

cctgatgagg tgatcaactt catgcgcagc cacccactca tgtaccaggc cgtgtaccct
1560

ctgcagcggc ggcccctggt agtccgcaca ggtgctccct accgccttac cactattgcc
1620

gtggaccagg tggattcagc cgacgggcgc tatgaggtgc ttttcctggg cacagaccgc
1680

gggacagtgc agaaggtcat tgtgctgccc aaggatgacc aggagatgga ggagctcatg
1740

ctggaggagg tggaggtctt caaggatcca gcacccgtca agaccatgac catctcttct
1800

aagaggcaac aactctacgt ggcgtcagcc gtgggtgtca cacacctgag cctgcaccgc
1860

tgccaggcgt atggggctgc ctgtgctgac tgctgccttg cccgggaccc ttactgtgcc
1920

tgggatggcc aggcctgctc ccgctataca gcatcctcca agaggcggag ccgccggcag
1980

gacgtccggc acggaaaccc catcaggcag tgccgtgggt tcaactccaa tgccaacaag
2040

aatgccgtgg agtctgtgca gtatggcgtg gccggcagcg cagccttcct tgagtgccag
2100

ccccgctcgc cccaagccac tgttaagtgg ctgttccagc gagatcctgg tgaccggcgc
2160

cgagagattc gtgcagagga ccgcttcctg cgcacagagc agggcttgtt gctccgtgca
2220

ctgcagctca gcgatcgtgg cctctactcc tgcacagcca ctgagaacaa ctttaagcac

2280

gtcgtcacac gagtgcagct gcatgtactg ggccgggacg ccgtccatgc tgccctcttc
2340

ccaccactgt ccatgagcgc cccgccaccc ccaggcgcag gccccccaac gcctccttac
2400

caggagttag cccagctgct ggcccagcca gaagtgggcc tcatccacca gtactgccag
2460

ggttactggc gccatgtgcc ccccagcccc agggaggctc caggggcacc ccggtctcct
2520

gagccccagg accagaaaaa gccccggaac cgccggcacc accctccgga cacatgaggc
2580

cagctgcctg ttctgccatg ggcagcctag cccttgtccc ttttaatata aaagacatat
2640

atatatatat atatatatat atataaaaat atctatattc tatacacacc ctgcccctgc
2700

aaagacagta tttattggtg ggttgaatat agcctgcctc agtggcagca tcctccaaaa
2760

cttagaccca tgctggtcag agacggcaga aaacagagcc tgcctaacca ggccagccag
2820

ttagtggggc caggccagga ccacacagtc cccagactca gctggagtct acctgctgga
2880

caggccttgc gccaagatct acaggacaaa gggagggagc aagccctact cggatggggc
2940

acggaccgtc cacctttctg atgtgtgttg tcagcctgtg ctgtggcata gacatggatg
3000

cgaggaccac tttggagact ggggtggcct caagagcaca cagagaaggg aagaaggggc
3060

catcacagga tgccacccct gccttgggtt gggggcactc tgccacgacc agccccttcc
3120

tgggtattta ttctctattt attggggata ggagaagagg catcctgcct gggtgggaca
3180

gccccttggg ccccttctcc cctccccgcc tggccaggga gggccacccc actctacctc
3240

cttagctttc cctgtgccac tttgactcag aggctgggag catagcagag gccaggccca
3300

ggcagagctg acgggaggcc ccagctctga ggggaggggg tccgtggtag agcctggggc
3360

aggtagaggc tccccagggc tcccttatgt ccaccacttc aggggatggg tgtggatgta
3420

attagctctg gggggcagtt gggtagatgg gtgggggctc ctggtggcct tctgctgccc
3480

aggccacagc cgcctttggg ttccatcttg ctaataaaca ctggctttgg aactaaaaaa
3540

aaaaaaaaaa aa

3552

<210> 122
<211> 2026
<212> DNA
<213> Homo sapiens

<400> 122
gaattccggt gccatggctg agtcacacct gctgcagtgg ctgctgctgc tgctgcccac
60

gctctgtggc ccaggcactg ctgcctggac cacctcatcc ttggcctgtg cccagggccc
120

tgagttctgg tgccaaagcc tggagcaagc attgcagtgc agagccctag ggcattgcct
180

acaggaagtc tggggacatg tgggagccga tgacctatgc caagagtgtg aggacatcgt
240

ccacatcctt aacaagatgg ccaaggaggc cattttccag gacacgatga ggaagttcct
300

ggagcaggag tgcaacgtcc tccccttgaa gctgctcatg ccccagtgca accaagtgct
360

tgacgactac ttccccctgg tcatcgacta cttccagaac cagactgact caaacggcat
420

ctgtatgcac ctgggcctgt gcaaatcccg gcagccagag ccagagcagg agccagggat
480

gtcagacccc ctgcccaaac ctctgcggga ccctctgcca gaccctctgc tggacaagct
540

cgtcctccct gtgctgcccg gggccctcca ggcgaggcct gggcctcaca cacaggatct
600

ctccgagcag caattcccca ttcctctccc ctattgctgg ctctgcaggg ctctgatcaa
660

gcggatccaa gccatgattc ccaagggtgc gctagctgtg gcagtggccc aggtgtgccg
720

cgtggtacct ctggtggcgg gcggcatctg ccagtgcctg gctgagcgct actccgtcat
780

cctgctcgac acgctgctgg gccgcatgct gccccagctg gtctgccgcc tcgtcctccg
840

gtgctccatg gatgacagcg ctggcccaag gtcgccgaca ggagaatggc tgccgcgaga
900

ctctgagtgc cacctctgca tgtccgtgac cacccaggcc gggaacagca gcgagcaggc
960

cataccacag gcaatgctcc aggcctgtgt tggctcctgg ctggacaggg aaaagtgcaa
1020

gcaatttgtg gagcagcaca cgccccagct gctgaccctg gtgcccaggg gctgggatgc
1080

ccacaccacc tgccaggccc tcggggtgtg tgggaccatg tccagccctc tccagtgtat
1140

ccacagcccc gacctttgat gagaactcag ctgtccagct gcaaaggaaa agccaagtga
1200

gacgggctct gggaccatgg tgaccaggct cttcccctgc tccctggccc tcgccagctg
1260

ccaggctgaa aagaagcctc agctcccaca ccgccctcct cacctccctt cctcggcagt
1320

cacttccact ggtggaccac gggcccccag ccctgtgtcg gccttgtctg tctcagctca
1380

accacagtct gacaccagag cccacttcca tcctctctgg tgtgaggcac agcgagggca
1440

gcatctggag gagctctgca gcctccacac ctaccacgac ctcccagggc tgggctcagg
1500

aaaaaccagc cactgcttta caggacaggg ggttgaagct gagccccgcc tcacacccac
1560

ccccatgcac tcaaagattg gattttacag ctacttgcaa ttcaaaattc agaagaataa
1620

aaaatgggaa catacagaac tctaaaagat agacatcaga aattgttaag ttaagctttt
1680

tcaaaaaatc agcaattccc cagcgtagtc aagggtggac actgcacgct ctggcatgat
1740

gggatggcga ccgggcaagc tttcttcctc gagatgctct gctgcttgag agctattgct
1800

ttgttaagat ataaaaaggg gtttcttttt gtctttctgt aaggtggact tccagatttt
1860

gattgaaagt cctagggtga ttctatttct gctgtgattt atctgctgaa agctcagctg
1920

gggttgtgca agctagggac ccattcctgt gtaatacaat gtctgcacca atgctaataa
1980

agtcctattc tcttttaaaa aaaaaaaaaa aaaaaaaacg gaattc
2026


<210>  123
<211>  1716
<212>  DNA
<213>  Homo sapiens

<400>  123
cggcgccagc agcggagcca acgcacccga gtttgtgttt gaggccaccc tgaggatcgg
60

gacagctgtt cctttgggct gcagaaactc cgctgagcag aacttgccgc cagaatgctc
120

ctcctgttgc tgagtatcat cgtcctccac gtcgcggtgc tggtgctgct gttcgtctcc
180

acgatcgtca gccaatggat cgtgggcaat ggacacgcaa ctgatctctg gcagaactgt
240

agcacctctt cctcaggaaa tgtccaccac tgtttctcat catcaccaaa cgaatggctg
300

cagtctgtcc aggccaccat gatcctgtcg atcatcttca gcattctgtc tctgttcctg
360

ttcttctgcc aactcttcac cctcaccaag gggggcaggt tttacatcac tggaatcttc
420

caaattcttg ctggtctgtg cgtgatgagt gctgcggcca tctacacggt gaggcacccg
480

gagtggcatc tcaactcgga ttactcctac ggtttcgcct acatcctggc ctgggtggcc
540

ttccccctgg cccttctcag cggtgtcatc tatgtgatct tgcggaaacg cgaatgaggc
600

gcccagacgg tctgtctgag gctctgagcg tacataggga agggaggaag ggaaaccaga
660

aagcagacaa agaaaaaaga gctagcccaa aatcccaaac tcaaaccaaa cagaaagcag
720

tggaggtggg ggttgctgtt gattgaagat gtatataata tctccggttt ataaaaccta
780

tttataacac tttttacata tatgtacata gtattgtttg ctttttatgt tgaccatcag
840

cctcgtgttg agccttaaag aagtagctaa ggaactttac atcctaacag tataatccag
900

ctcagtattt ttgttttgtt ttttgtttgt ttgttttgtt ttacccagaa ataagataac
960

tccatctcgc cccttccctt tcatctgaaa gaagatacct ccctcccagt ccacctcatt
1020

tagaaaacca aagtgtgggt agaaacccca aatgtccaaa agcccttttc tggtgggtga
1080

cccagtgcat ccaacagaaa cagccgctgc ccgaacctct gtgtgaagct ttacgcgcac
1140

acggacaaaa tgcccaaact ggagcccttg caaaaacacg gcttgtggca ttggcatact
1200

tgcccttaca ggtggagtat cttcgtcaca catctaaatg agaaatcagt gacaacaagt
1260

ctttgaaatg gtgctatgga tttaccattc cttattatca ctaatcatct aaacaactca
1320

ctggaaatcc aattaacaat tttacaacat aagatagaat ggagacctga ataattctgt
1380

gtaatataaa tggtttataa ctgcttttgt acctagctag gctgctatta ttactataat
1440

gagtaaatca taaagccttc atcactccca catttttctt acggtcggag catcagaaca
1500

agcgtctaga ctccttggga ccgtgagttc ctagagcttg gctgggtcta ggctgttctg
1560

tgcctccaag gactgtctgg caatgacttg tattggccac caactgtaga tgtatatatg
1620

gtgcccttct gatgctaaga ctccagacct tttgtttttg ctttgcattt tctgatttta
1680

taccaactgt gtggactaag atgcattaaa ataaac
1716

<210> 124
<211> 3062
<212> DNA
<213> Homo sapiens

<220>
<221> misc_feature
<222> (2252)..(2253)

<220>
<221> misc_feature
<222> (2252)..(2252)
<223> n is a, c, g, or t

<400> 124
tgacgttcaa gttcgcaggg acgtcacgtc cgcacttgaa cttgcagctc aggggggctt
60

ttgccatttt tttcatctct ctctctctct ctccctctat ctctcttctc tctctctctc
120

tccctctttt tttttttttt tttttttttt tttttttgct taaaaaaaag ccatgacggc
180

tctcccacaa ttcatcttcc ctgcgccatc tttgtattat ttctaattta ttttggatgt
240

caaaaggcac tgatgaagat attttctctg gagtctcctt ctttctaacc cggctctccc
300

gatgtgaacc gagccgtcgt ccgcccgccc cgcagcccac catgtctcgc cgcaagcaag
360

gcaaacccca gcacttaagc aaacgggaat tctcgcccga gcctcttgaa gccattctta
420

cagatgatga accagaccac ggcccgttgg gagctccaga aggggatcat gacctcctca
480

cctgtgggca gtgccagatg aacttcccat tgggggacat tcttattttt atcgagcaca
540

aacggaaaca atgcaatggc agcctctgct tagaaaaagc tgtggataag ccaccttccc
600

cttcaccaat cgagatgaaa aaagcatcca atcccgtgga ggttggcatc caggtcacgc
660

cagaggatga cgattgttta tcaacgtcat ctagaggaat ttgccccaaa caggaacaca
720

tagcagataa acttctgcac tggaggggcc tctcctcccc tcgttctgca catggggctc
780

taatccccac gcccgggatg agtgcagaat atgccccgca gggtatttgt aaagatgagc
840

ccagcagcta cacatgtaca acttgcaaac agccattcac cagtgcatgg tttctcttgc
900

aacacgcaca gaacactcat ggattaagaa tctacttaga aagcgaacac ggaagtcccc
960

tgaccccgcg ggttggtatc ccttcaggac taggtgcaga atgtccttcc cagccacctc
1020

tccatgggat tcatattgca gacaataacc cctttaacct gctaagaata ccaggatcag
1080

tatcgagaga ggcttccggc ctggcagaag ggcgctttcc acccactccc cccctgttta
1140

gtccaccacc gagacatcac ttggaccccc accgcataga gcgcctgggg gcggaagaga
1200

tggccctggc cacccatcac ccgagtgcct ttgacagggt gctgcggttg aatccaatgg
1260

ctatggagcc tcccgccatg gatttcttta ggagacttag agagctggca gggaacacgt
1320

ctagcccacc gctgtcccca ggccggccca gccctatgca aaggttactg caaccattcc
1380

agccaggtag caagccgccc ttcctggcga cgccccccct ccctcctctg caatccgccc
1440

ctcctccgtc ccagcccccg gtcaagtcca agtcatgcga gttctgcggc aagacgttga
1500

aatttcagag caacctggtg gtgcaccggc gcagccacac gggcgagaag ccctacaagt
1560

gcaacctgtg cgaccacgcg tgcacccagg ccagcaagct gaagcgccac atgaagacgc
1620

acatgcacaa atcgtccccc atgacggtca agtccgacga cggtctctcc accgccagct
1680

ccccggaacc cggcaccagc gacttggtgg gcagcgccag cagcgcgctc aagtccgtgg
1740

tggccaagtt caagagcgag aacgacccca acctgatccc ggagaacggg gacgaggagg
1800

aagaggagga cgacgaggaa gaggaagaag aggaggaaga ggaggaggag gagctgacgg
1860

agagcgagag ggtggactac ggcttcgggc tgagcctgga ggcggttgtg ggcgtgggcg
1920

acgagagccg cgccctgccc gacgtcatgc agggcatggt gctcagctcc atgcagcact
1980

tcagcgaggc cttccaccag gtcctgggcg agaagcataa gcgcggccac ctggccgagg
2040

ccgagggcca cagggacact tgcgacgaag actcggtggc cggcgagtcg gaccgcatag
2100

acgatggcac tgttaatggc cgcggctgct ccccgggcga gtcggcctcg gggggcctgt
2160

ccaaaaagct gctgctgggc agccccagct cgctgagccc cttctctaag cgcatcaagc
2220

tcgagaagga gttcgacctg cccccggccg cnatgcccaa cacggataac gtgtactcgc
2280

agtggctcgc cggctacgcg gcctccaggc agctcaaaga tcccttcctt agcttcggag
2340

actccagaca atcgcctttt gcctcctcgt cggagcactc ctcggagaac gggagtttgc
2400

gcttctccac accgcccggg gagctggacg gagggatctc ggggcgcagc ggcacgggaa
2460

gtggagggag cacgccccat attagtggtc cgggcccggg caggcccagc tcaaaagagg
2520

gcagacgcag cgacacttgt tcttcacaca cccccattcg gcgtagtacc cagagagctc
2580

aagatgtgtg gcagttttcg gatggaagct cgagagccct taagttctga gaaaatttga
2640

agcccccagg ggtggggtgg acgcgtgccg cccagtcgac gtcagcgtgg tctgtcatcc
2700

tgctagtttg tgatgttttc tgacagtagc ctccaagaag ccgttgtgcg aagacagagt
2760

cctgcagagt ccttccagcc taggcctgca gcgccatttt atttatattt tttaataaaa
2820

agtaaaaaca aaaaaacaga cccacattgg aacagtgaat cagtcccata gagagggccc
2880

gtggaccatc gctgtcatga gtgatgccct ggcccttctg aaaccagcca acctaattac
2940

ctgtattgtg gaaatgcgca tgagtcccca accccttgtt tctatacatt ctatgttgtc
3000

ttttaaaaag tgtgcttaac attgacacaa taaatgttgg agctttagaa aaaaaaaaaa
3060

aa
3062


<210>  125
<211>  1972
<212>  DNA
<213>  Homo sapiens

<400>  125
atggagaggg ggctgccgct cctctgcgcc gtgctcgccc tcgtcctcgc cccggccggc
60

gcttttcgca acgataaatg tggcgatact ataaaaattg aaagccccgg gtaccttaca
120

tctcctggtt atcctcattc ttatcaccca agtgaaaaat gcgaatggct gattcaggct
180

ccggacccat accagagaat tatgatcaac ttcaaccctc acttcgattt ggaggacaga
240

gactgcaagt atgactacgt ggaagtgttc gatggagaaa atgaaaatgg acattttagg
300

ggaaagttct gtggaaagat agcccctcct cctgttgtgt cttcagggcc atttcttttt
360

atcaaatttg tctctgacta cgaaacacat ggtgcaggat tttccatacg ttatgaaatt
420

ttcaagagag gtcctgaatg ttcccagaac tacacaacac ctagtggagt gataaagtcc
480

cccggattcc ctgaaaaata tcccaacagc cttgaatgca cttatattgt ctttgcgcca
540

aagatgtcag agattatcct ggaatttgaa agctttgacc tggagcctga ctcaaatcct
600

ccagggggga tgttctgtcg ctacgaccgg ctagaaatct gggatggatt ccctgatgtt
660

ggccctcaca ttgggcgtta ctgtggacag aaaacaccag gtcgaatccg atcctcatcg
720

ggcattctct ccatggtttt ttacaccgac agcgcgatag caaaagaagg tttctcagca
780

aactacagtg tcttgcagag cagtgtctca gaagatttca aatgtatgga agctctgggc
840

atggaatcag gagaaattca ttctgaccag atcacagctt cttcccagta tagcaccaac
900

tggtctgcag agcgctcccg cctgaactac cctgagaatg ggtggactcc cggagaggat
960

tcctaccgag agtggataca ggtagacttg ggccttctgc gctttgtcac ggctgtcggg
1020

acacagggcg ccatttcaaa agaaaccaag aagaaatatt atgtcaagac ttacaagatc
1080

gacgttagct ccaacgggga agactggatc accataaaag aaggaaacaa acctgttctc
1140

tttcagggaa acaccaaccc cacagatgtt gtggttgcag tattccccaa accactgata
1200

actcgatttg tccgaatcaa gcctgcaact tgggaaactg gcatatctat gagatttgaa
1260

gtatacggtt gcaagataac agattatcct tgctctggaa tgttgggtat ggtgtctgga
1320

cttatttctg actcccagat cacatcatcc aaccaagggg acagaaactg gatgcctgaa
1380

aacatccgcc tggtaaccag tcgctctggc tgggcacttc cacccgcacc tcattcctac
1440

atcaatgagt ggctccaaat agacctgggg gaggagaaga tcgtgagggg catcatcatt
1500

cagggtggga agcaccgaga gaacaaggtg ttcatgagga agttcaagat cgggtacagc
1560

aacaacggct cggactggaa gatgatcatg gatgacagca aacgcaaggc gaagtctttt
1620

gagggcaaca acaactatga tacacctgag ctgcggactt ttccagctct ctccacgcga
1680

ttcatcagga tctaccccga gagagccact catggcggac tggggctcag aatggagctg
1740

ctgggctgtg aagtggaagc ccctacagct ggaccgacca ctcccaacgg gaacttggtg
1800

gatgaatgtg atgacgacca ggccaactgc cacagtggaa caggtgatga cttccagctc
1860

acaggtggca ccactgtgct ggccacagaa aagcccacgg tcatagacag caccatacaa
1920

tcaggtatca aataaaatac gaaatgtgac agaaaaaaaa aaaaaaaaaa aa
1972

<210>  126
<211>  3750
<212>  DNA
<213>  Homo sapiens

<400>  126
atggagaggg ggctgccgct cctctgcgcc gtgctcgccc tcgtcctcgc cccggccggc
60

gcttttcgca acgataaatg tggcgatact ataaaaattg aaagccccgg gtaccttaca
120

tctcctggtt atcctcattc ttatcaccca agtgaaaaat gcgaatggct gattcaggct
180

ccggacccat accagagaat tatgatcaac ttcaaccctc acttcgattt ggaggacaga
240

gactgcaagt atgactacgt ggaagtgttc gatggagaaa atgaaaatgg acattttagg
300

ggaaagttct gtggaaagat agcccctcct cctgttgtgt cttcagggcc atttcttttt
360

atcaaatttg tctctgacta cgaaacacat ggtgcaggat tttccatacg ttatgaaatt
420

ttcaagagag gtcctgaatg ttcccagaac tacacaacac ctagtggagt gataaagtcc
480

cccggattcc ctgaaaaata tcccaacagc cttgaatgca cttatattgt ctttgcgcca
540

aagatgtcag agattatcct ggaatttgaa agctttgacc tggagcctga ctcaaatcct
600

ccagggggga tgttctgtcg ctacgaccgg ctagaaatct gggatggatt ccctgatgtt
660

ggccctcaca ttgggcgtta ctgtggacag aaaacaccag gtcgaatccg atcctcatcg
720

ggcattctct ccatggtttt ttacaccgac agcgcgatag caaaagaagg tttctcagca

780

aactacagtg tcttgcagag cagtgtctca gaagatttca aatgtatgga agctctgggc
840

atggaatcag gagaaattca ttctgaccag atcacagctt cttcccagta tagcaccaac
900

tggtctgcag agcgctcccg cctgaactac cctgagaatg ggtggactcc cggagaggat
960

tcctaccgag agtggataca ggtagacttg ggccttctgc gctttgtcac ggctgtcggg
1020

acacagggcg ccatttcaaa agaaaccaag aagaaatatt atgtcaagac ttacaagatc
1080

gacgttagct ccaacgggga agactggatc accataaaag aaggaaacaa acctgttctc
1140

tttcagggaa acaccaaccc cacagatgtt gtggttgcag tattccccaa accactgata
1200

actcgatttg tccgaatcaa gcctgcaact tgggaaactg gcatatctat gagatttgaa
1260

gtatacggtt gcaagataac agattatcct tgctctggaa tgttgggtat ggtgtctgga
1320

cttatttctg actcccagat cacatcatcc aaccaagggg acagaaactg gatgcctgaa
1380

aacatccgcc tggtaaccag tcgctctggc tgggcacttc cacccgcacc tcattcctac
1440

atcaatgagt ggctccaaat agacctgggg gaggagaaga tcgtgagggg catcatcatt
1500

cagggtggga agcaccgaga gaacaaggtg ttcatgagga agttcaagat cgggtacagc
1560

aacaacggct cggactggaa gatgatcatg gatgacagca aacgcaaggc gaagtctttt
1620

gagggcaaca acaactatga tacacctgag ctgcggactt ttccagctct ctccacgcga
1680

ttcatcagga tctaccccga gagagccact catggcggac tggggctcag aatggagctg
1740

ctgggctgtg aagtggaagc ccctacagct ggaccgacca ctcccaacgg gaacttggtg
1800

gatgaatgtg atgacgacca ggccaactgc cacagtggaa caggtgatga cttccagctc
1860

acaggtgcag aaaccatctt catcccattg ctgtaccatt tcagttcatg tcttagttgg
1920

gaccagctaa cgcctgtgtg tgtgcttgta actccgcatg gcagagagct gcccaggaat
1980

agaagctgcc ttgccagaac acgtgcatct tcatttcctc atgtcatctg gatagatgag
2040

ttgttcttaa tagccacaac catttgtaac aataatttga gccatttcga gagccaaagg

2100

ctaggattat cttaattgaa taagaatttc cccatggcta aatgtactaa accgatcctt
2160

attttcatta ttttcagacc cccccccaat aacacgtgca ccacccctac gtaaccacac
2220

acttcagaat gtttattttc attttctaat gaggacaagt ttttttgagt ggatccttta
2280

tccaacaaat ggataaagtt aggcttatat ttgaagtgcg gcttttaagt tgggatttgg
2340

taaatcattg tacattaaca tttacaaagg cagaattagc tgctatttca catcaccacc
2400

gaccagcaaa tgcatcagag atgttgtgtg ccaaatgtct cactaattaa tctagagtat
2460

tactctctaa tctcctaaaa ccctaaaagc taggaaatga ccttctcttc cacttggaat
2520

ggcagaggaa atattaggca agtggagaaa tgggattata ccatttggaa attggccaag
2580

gcactagggt tttcaccctg tggttgcaaa aggtatcgtg ggacttttac tgctccctgg
2640

gtcaaaacgc agtttcaata gcatctgact ctcatttgag attaaacatg agcttttgaa
2700

tgcaggtctg catttttacc cagaagggac tcggagagcc cacataatta atttccatgg
2760

gcaaaactga aggcgttacc atgtaacttc ataataaagc catgttattg gctaatgcat
2820

tcgtacaaaa tctggaagga agtgtcctag agtacatttc caattatttc aaaagctctc
2880

ctctgaagga gtgagagggc tctgcactct aagatggaat aaagagtaaa agaaagaaaa
2940

aaaaattgaa aatctgtccc agaaatttag tactgtgaac tgagtttaca tttaaatccc
3000

acctcctaaa gctgtctctc tccaaatttc caggataatc atttacattc atatggccaa
3060

taaatgtcac attgctatga gcttttgaat gcaaatcttg tatacaagca gccacaagcc
3120

acaaaactta gattaattaa tgcagagaca ttttctgtgt aacatgtcaa gttcaagttg
3180

aaaactcaac cactgatata aaaaacagag tactattcca tgaaccaagt cctctggaag
3240

cttcccgggg ttgcctgtaa acactctgcc acaaacctca ggctttttac agaccccgtc
3300

acacatccat agtgaaattt ctcataaaaa aggaatgatt gcaggatccc agtgtgccct
3360

ttcaattgcc ctcttgtgga aggggttctt ttcttcataa accctaaagt ggtcagttaa

3420

tattgattag aaacaaagcc tcagatttac tcctcatatc agaacaacca gccttctcat
3480

ttttgcatta gttcatgact gaatcaattg aaactgaagg ctttgaatga aatagggcga
3540

gtatgttagc ctggagcaaa ttgtgtatgg cagcgagtta caaaccacag aaaatgggtc
3600

atttatagtg gaaacactga caacctttca aaactataag gttttgcttt acctacagtg
3660

tttcaattat ttctttggta gatagaagaa agttcattct gattttttct ttttacatat
3720

tagcatggtg aaaaaaaaaa aaaaaaaaaa
3750

<210>  127
<211>  2425
<212>  DNA
<213>  Homo sapiens

<400>  127
   tcagctggaa ttccggctga aagcaagcaa tgctccccac cctgcaaatc cagggcccag
   60
   cagcctttgc tcctggggat agaggccctg gcaggcactg tcccttccct gtgcccatca
   120
   cccccactgg tgccctcctg ccagtctctg actcttgtga cagtctggtg gacctggtct
   180
   ggccatctgt tacctatctt gccttgggga cccagagcag agtctggcca catcccttgg
   240
   gggctcctgg tcaggctggg gagtcacctg aacaaagaag acagtgtcta gagctgtggg
   300
   acatggccag ctccctgggg gacaaggtcc ccagagcagc atgtgggaag agggggcaga
   360
   cagtgtggca gctgcatctc gcctgcctct gcctggccca gttccactct ccacctgctc
   420
   aacccccacc tctctccaga agaggagggg gacccgaccc ggatccaata tcccgctccc
   480
   tgcctgggcc tcccacacct gcactgccca cacactcata cagctctcac tccccacgtg
   540
   ctccacgcct cctgtcccca ctgaggagag ctcccagagg ctcgcctgct ccccaccgac
   600
   acgcgtccct gcagacaaac gaggcgccca gggagcttcc ccactgcact tggccagggc
   660
   tgccggggcg cagccttgcc cctagcttcc tctggcggga gccatggctc ggaggacaat
   720
   ggggacctct gaacatacct gcccgcaagg gggaccggag gcgctgggag tgggggtgtg
   780
   agggaggtgg tgccacagcc tccgctgagc agcctggccc ccagatcgtg tacttcactg
   840
   ctacattccc ctacgtggtc ctggtcgtgc tgctggtgcg tggagtgctg ctgcctggcg
   900
   ccctggatgg catcatttac tatctcaagc ctgactggtc aaagctgggg tcccctcagg
   960
   tgtggataga tgcggggacc cagattttct tttcttacgc cattggcctg ggggccctca
   1020
   cagccctggg cagctacaac cgcttcaaca acaactgcta caaggacgcc atcatcctgg
   1080
   ctctcatcaa cagtgggacc agcttctttg ctggcttcgt ggtcttctcc atcctgggct
   1140
   tcatggctgc agagcagggc gtgcacatct ccaaggtggc agagtcaggg ccgggcctgg
   1200
   ccttcatcgc ctacccgcgg gctgtcacgc tgatgccagt ggccccactc tgggctgccc
   1260
   tgttcttctt catgctgttg ctgcttggtc tcgacagcca ggtttgcatg gggctctggg
   1320
   acagggagcc aggagggggg cggagggagg gctgcaggca aggaaagggg tggaggaggt
   1380
   gcggggatcg gcctgagctg ccctggccac agtttgtagg tgtggagggc ttcatcaccg
   1440
   gcctcctcga cctcctcccg gcctcctact acttccgttt ccaaagggag atctctgtgg
   1500
   ccctctgttg tgccctctgc tttgtcatcg atctctccat ggtgactgat gtgagtgggg
   1560
   tggggggtct gcctgtgacc tctggtggcc gtctgccatc ctccctgact gggctctgtc
   1620
   cccagggcgg gatgtacgtc ttccagctgt ttgactacta ctcggccagc ggcaccaccc
   1680
   tgctctggca ggccttttgg gagtgcgtgg tggtggcctg ggtgtacggt aggtcatggc
   1740
   tgagggctgg gctgggggat ggtggcgggg aaggcaggtc tccagcttgg ccctcccgcc
   1800
   tcacctcgcc gcagggagct gaccgcttca tggacgacat tgcctgtatg atcgggtacc
   1860
   gaccttgccc ctggatgaaa tggtgctggt ccttcttcac cccgctggtc tgcatgggca
   1920
   tcttcatctt caacgttgtg tactacgagc cgctggtcta caacaacacc tacgtgtacc
   1980
   cgtggtgggg tgaggccatg ggctgggcct tcgccctgtc ctccatgctg tgcgtgccgc
   2040
   tgcacctcct gggctgcctc ctcagggcaa agggcaccat ggctgagcgc tggcagcacc
   2100
   tgacccagcc catctggggc ctccaccact tggagtaccg agctcaggac gcagatgtca
   2160
   ggggcctgac caccctgacc ccagtgtccg agagcagcaa ggtcgtcgtg gtggagagtg
   2220
   tcatgtgaca actcagctca catcaccagc tcacctctgg tagccatagc agcccctgct
   2280
   tcagccccac cgcacccctc cagggggcct gcctttccct gacacttttg gggtctgcct
   2340
   gggggaggag gggagaaagc accatgagtg ctcactaaaa caactttttc catttttaat
   2400
   aaaacgcccg gaattccgcc gatac
   2425

<210> 128
<211> 1878
<212> DNA
<213> Homo sapiens

<400> 128
ggaaaaagca tgacatttag aagtagaaga cttagcttca aatccctact ccttcactta
60

ctaattttgt gatttggaaa tatccgcgca agatgttgac gttgcagact tgggtagtgc
120

aagccttgtt tattttcctc accactgaat ctacaggtga acttctagat ccatgtggtt
180

atatcagtcc tgaatctcca gttgtacaac ttcattctaa tttcactgca gtttgtgtgc
240

taaaggaaaa atgtatggat tattttcatg taaatgctaa ttacattgtc tggaaaacaa
300

accattttac tattcctaag gagcaatata ctatcataaa cagaacagca tccagtgtca
360

cctttacaga tatagcttca ttaaatattc agctcacttg caacattctt acattcggac
420

agcttgaaca gaatgtttat ggaatcacaa taatttcagg cttgcctcca gaaaaaccta
480

aaaatttgag ttgcattgtg aacgagggga agaaaatgag gtgtgagtgg gatggtggaa
540

gggaaacaca cttggagaca aacttcactt taaaatctga atgggcaaca cacaagtttg
600

ctgattgcaa agcaaaacgt gacaccccca cctcatgcac tgttgattat tctactgtgt
660

attttgtcaa cattgaagtc tgggtagaag cagagaatgc ccttgggaag gttacatcag
720

atcatatcaa ttttgatcct gtatataaag tgaagcccaa tccgccacat aatttatcag
780

tgatcaactc agaggaactg tctagtatct taaaattgac atggaccaac ccaagtatta
840

agagtgttat aatactaaaa tataacattc aatataggac caaagatgcc tcaacttgga
900

gccagattcc tcctgaagac acagcatcca cccgatcttc attcactgtc caagacctta
960

aaccttttac agaatatgtg tttaggattc gctgtatgaa ggaagatggt aagggatact
1020

ggagtgactg gagtgaagaa gcaagtggga tcacctatga agataacatt gcctcctttt
1080

gaagccaatg gaaaaatctt ggattatgaa gtgactctca caagatggaa atcacattta
1140

caaaattaca cagttaatgc cacaaaactg acagtaaatc tcacaaatga tcgctatcta
1200

gcaaccctaa cagtaagaaa tcttgttggc aaatcagatg cagctgtttt aactatccct
1260

gcctgtgact ttcaagctac tcaccctgta atggatctta aagcattccc caaagataac
1320

atgctttggg tggaatggac tactccaagg gaatctgtaa agaaatatat acttgagtgg
1380

tgtgtgttat cagataaagc accctgtatc acagactggc aacaagaaga tggtaccgtg
1440

catcgcacct atttaagagg gaacttagca gagagcaaat gctatttgat aacagttact
1500

ccagtatatg ctgatggacc aggaagccct gaatccataa aggcatacct taaacaagct
1560

ccaccttcca aaggacctac tgttcggaca aaaaaagtag ggaaaaacga agctgtctta
1620

gagtgggacc aacttcctgt tgatgttcag aatggattta tcagaaatta tactatattt
1680

tatagaacca tcattggaaa tgaaactgct gtgaatgtgg attcttccca cacagaatat
1740

acattgtcct ctttgactag tgacacattg tacatggtac gaatggcagc atacacagat
1800

gaaggtggga aggatggtcc agaattcact tttactaccc caaagtttgc tcaaggagaa
1860

attgaagcca tagtcgtg
1878

<210> 129
<211> 3901
<212> DNA
<213> Homo sapiens

<400> 129
gccacaggcg cggcgtcctc ggcggcgggc ggcagctagc gggagccggg acgccggtgc
60

agccgcagcg cgcggaggaa cccgggtgtg ccgggagctg ggcggccacg tccggtcggg

120

accgagaccc ctcgtagcgc attgcggcga cctcgccttc cccggccgcg agcgcgccgc
180

tgcttgaaaa gccgcggaac ccaaggactt ttctccggtc cgagctcggg gcgccccgca
240

ggcgcacggt acccgtgctg cagctgggca cgccgcggcg ccggggcctc cgcaggcgcc
300

ggcctgcgtt ctggaggagg ggggcacaag gtctggagac cccgggtggc ggacgggagc
360

cctccccccg ccccgcctcc gcgaccagct ccgctccatt gttcccgccc ggctggaggc
420

gccgagcacc gagcgcgccg ggagtcgagc gccggccgcg agctcttgcg accccgccag
480

acccgaacag agcccggggg ccggcgcgga gccgggacgc gggcacacgg cctcgcacaa
540

gccacgggca ctctcccgag gcggaacctc cacgccgagc gagggtcagt ttgaaaagga
600

ggatcgagct cactgtggag tatccatgga gatgtggagc cttgtcacca acctctaact
660

gcagaactgg gatgtggagc tggaagtgcc tcctcttctg ggctgtgctg gtcacagcca
720

cactctgcac cgctaggccg tccccgacct tgcctgaaca agcccagccc tggggagccc
780

ctgtggaagt ggagtccttc ctggtccacc ccggtgacct gctgcagctt cgctgtcggc
840

tgcgggacga tgtgcagagc atcaactggc tgcgggacgg ggtgcagctg gcggaaagca
900

accgcacccg catcacaggg gaggaggtgg aggtgcagga ctccgtgccc gcagactccg
960

gcctctatgc ttgcgtaacc agcagcccct ccggaagtga caccacctac ttctccgtca
1020

atgtttcaga tgctctcccc tcctcggagg atgatgatga tgatgatgac tcctcttcag
1080

aggagaaaga aacagataac accaaaccaa accccgtagc tccatattgg acatccccag
1140

aaaagatgga aaagaaattg catgcagtgc cggctgccaa gacagtgaag ttcaaatgcc
1200

cttccagtgg gaccccaaac cccacactgc gctggttgaa aaatggcaaa gaattcaaac
1260

ctgaccacag aattggaggc tacaaggtcc gttatgccac ctggagcatc ataatggact
1320

ctgtggtgcc ctctgacaag ggcaactaca cctgcattgt ggagaatgag tacggcagca
1380

tcaaccacac ataccagctg gatgtcgtgg agcggtcccc tcaccgcccc atcctgcaag

1440

cagggttgcc cgccaacaaa acagtggccc tgggtagcaa cgtggagttc atgtgtaagg
1500

tgtacagtga cccgcagccg cacatccagt ggctaaagca catcgaggtg aatgggagca
1560

agattggccc agacaacctg ccttatgtcc agatcttgaa gactgctgga gttaatacca
1620

ccgacaaaga gatggaggtg cttcacttaa gaaatgtctc ctttgaggac gcaggggagt
1680

atacgtgctt ggcgggtaac tctatcggac tctcccatca ctctgcatgg ttgaccgttc
1740

tggaagccct ggaagagagg ccggcagtga tgacctcgcc cctgtacctg gagatcatca
1800

tctattgcac aggggccttc ctcatctcct gcatggtggg gtcggtcatc gtctacaaga
1860

tgaagagtgg taccaagaag agtgacttcc acagccagat ggctgtgcac aagctggcca
1920

agagcatccc tctgcgcaga caggtaacag tgtctgctga ctccagtgca tccatgaact
1980

ctggggttct tctggttcgg ccatcacggc tctcctccag tgggactccc atgctagcag
2040

ggtctctgga gtatgagctt cccgaagacc ctcgctggga gctgcctcgg gacagactgg
2100

tcttaggcaa acccctggga gagggctgct ttgggcaggt ggtgttggca gaggctatcg
2160

ggctggacaa ggacaaaccc aaccgtgtga ccaaagtggc tgtgaagatg ttgaagtcgg
2220

acgcaacaga gaaagacttg tcagacctga tctcagaaat ggagatgatg aagatgatcg
2280

ggaagcataa gaatatcatc aacctgctgg gggcctgcac gcaggatggt cccttgtatg
2340

tcatcgtgga gtatgcctcc aagggcaacc tgcgggagta cctgcaggcc cggaggcccc
2400

cagggctgga atactgctac aaccccagcc acaacccaga ggagcagctc tcctccaagg
2460

acctggtgtc ctgcgcctac caggtggccc gaggcatgga gtatctggcc tccaagaagt
2520

gcatacaccg agacctggca gccaggaatg tcctggtgac agaggacaat gtgatgaaga
2580

tagcagactt tggcctcgca cgggacattc accacatcga ctactataaa aagacaacca
2640

acggccgact gcctgtgaag tggatggcac ccgaggcatt atttgaccgg atctacaccc
2700

accagagtga tgtgtggtct ttcggggtgc tcctgtggga gatcttcact ctgggcggct

2760

ccccataccc cggtgtgcct gtggaggaac ttttcaagct gctgaaggag ggtcaccgca
2820

tggacaagcc cagtaactgc accaacgagc tgtacatgat gatgcgggac tgctggcatg
2880

cagtgccctc acagagaccc accttcaagc agctggtgga agacctggac cgcatcgtgg
2940

ccttgacctc caaccaggag tacctggacc tgtccatgcc cctggaccag tactccccca
3000

gctttcccga cacccggagc tctacgtgct cctcagggga ggattccgtc ttctctcatg
3060

agccgctgcc cgaggagccc tgcctgcccc gacacccagc ccagcttgcc aatggcggac
3120

tcaaacgccg ctgactgcca cccacacgcc ctccccagac tccaccgtca gctgtaaccc
3180

tcacccacag cccctgcctg ggcccaccac ctgtccgtcc ctgtcccctt tcctgctggc
3240

aggagccggc tgcctacagg ggccttcctg tgtggcctgc cttcacccca ctcagctcac
3300

ctctccctcc acctcctctc cacctgctgg tgagaggtgc aaagaggcag atctttgctg
3360

ccagccactt catcccctcc cagatgttgg accaacaccc ctccctgcca ccaggcactg
3420

cctgagggca gggagtggga gccaatgaac aggcatgcaa gtgagagctt cctgagcttt
3480

ctcctgtcgg tttggtctgt tttgccttca cccataagcc cctcgcactc tggtggcagg
3540

tgcttgtcct cagggctaca gcagtaggga ggtcagtgct tcgagccacg attgaaggtg
3600

acctctgccc cagataggtg gtgccagtgg cttattaatt ccgatactag tttgctttgc
3660

tgaccaaatg cctggtacca gaggatggtg aggcgaaggc aggttggggg cagtgttgtg
3720

gcctggggcc agccaacact ggggctctgt atatagctat gaagaaaaca caaagttgat
3780

aaatctgagt atatatttac atgtcttttt aaaagggtcg ttaccagaga tttacccatc
3840

ggtaagatgc tcctggtggc tgggaggcat cagttgctat atattaaaaa caaaaaaaaa
3900

a 3
901

<210> 130
<211> 873
<212> DNA

<213>  Homo sapiens

<400>  130
aaagaaggac ctgggctttg ggaagatcta aagacccagg aaggtctctg ggtgggataa
60

agccaagatg aaactccccc tacttctggc tcttctattt ggggcagttt ctgctcttca
120

tctaaggtct gagacttcca cctttgagac ccctttgggt gctaagacgc tgcctgagga
180

tgaggagaca ccagagcagg agatggagga gaccccttgc agggagctgg aggaagagga
240

ggagtggggc tctggaagtg aagatgcctc caagaaagat ggggctgttg agtctatctc
300

agtgccagat atggtggaca aaaaccttac gtgtcctgag gaagaggaca cagtaaaagt
360

ggtgggcatc cctgggtgcc agacctgccg ctacctcctg gtgagaagtc ttcagacgtt
420

tagtcaagct tggtttactt gccggaggtg ctacaggggc aacctggttt ccatccacaa
480

cttcaatatt aattatcgaa tccagtgttc tgtcagcgcg ctcaaccagg gtcaagtctg
540

gattggaggc aggatcacag gctcgggtcg ctgcagacgc tttcagtggg ttgacggcag
600

ccgctggaac tttgcgtact gggctgctca ccagccctgg tcccgcggtg gtcactgcgt
660

ggccctgtgt acccgaggag gctactggcg tcgagcccac tgcctcagaa gacttccttt
720

catctgttcc tactgagctg gtcccagcca gcagttcaga gctgccctct cctgggcagc
780

tgcctcccct cctctgcttg ccatccctcc ctccacctcc ctgcaataaa atgggtttta
840

ccgaaaaaaa aaaaaaaaaa aaaaaaaaaa aaa
873


<210>  131
<211>  1305
<212>  DNA
<213>  Homo sapiens

<400>  131
atgagggaaa tcgtgctcac gcagaccggg cagtgcggga accagatcgg ggccaagttc
60

tgggaggtga tctctgatga acatgccatc gactccgctg gcacctacca cggggacagc
120

cacctgcagc tggagcgcat caacgtgcac caccacgagg ccagcggtgg caggtacgtg
180

ccccgcgctg tgctcgtgga tctggagccg ggcaccatgg actctgtgcg ctcggggccc
240

ttcgcggagg tcttcaggcc agacaacttc atttcccgtc agtgtggggc cggaaacaac
300

tgggccaagg gacgctacac cgaaggcgcg gagctgatgg agtcagtgat ggacgttgtc
360

agaaaggagg ctgagagctg tgactgcctg cagggtttcc agctgaccca ctccctgggt
420

ggggggactg ggtctgggat gggtaccctt ctgctcagta agatccggga ggagtaccca
480

gacaggatca taaacacatt cagcatcctg ccctcgccca aggtgtcgga caccgtggtg
540

gagccctaca acgccaccct ctcagtccac cagctcatag aaaacgcaga tgagaccttt
600

tgcatagata acgaagcgct atatgacata tgttccagga ccctaaaact gcccacaccc
660

acctatggtg acctgaacca cctggtgtct gctaccgtga gtggggtcac cacgtgcctg
720

cgcttcccag gccagctgaa tgctgacctg cggaagctgg ccgtgaacat ggtcccgttt
780

ccccggctgc atttcttcat gcccggcttt gccccactga ccagccaggg cagccagcag
840

taccgggcct tgactgtggc tgagctcacc cagcagatgt ttgatgctaa gaacatgatg
900

gctgcccgtg acccccgtca cggctgctac ctaacggcgg ctgccatttt caggggtcgc
960

atgcccatga gggaggtgga tgaacagatg ttcaacattc aagataagaa cagcagctac
1020

tttgctgact ggctccccaa caacataaaa acagccgtct gtgacatccc accctggggg
1080

ctaaaaatgt cagtcacctt cactgggaac aacacagccg tccaggaact caagcgggtc
1140

tcagagcagt ttacagcaac gttcaggcgc aaggccttcc tccactggta cacgggcgag
1200

ggcatggatg agatggaatt cactgaggcc gagagcaaca tgaacgactt ggtgtctgaa
1260

tatcagcaat atcaggatgc cacggccgag ggagaaggag tatga
1305


<210>  132
<211>  6847
<212>  DNA
<213>  Homo sapiens


<220>
<221>  misc_feature
<222>  (5824)..(6631)

<220>
<221> misc_feature
<222> (5824)..(5825)
<223> n is a, c, g, or t

<220>
<221> misc_feature
<222> (6248)..(6248)
<223> n is a, c, g, or t

<220>
<221> misc_feature
<222> (6252)..(6252)
<223> n is a, c, g, or t

<220>
<221> misc_feature
<222> (6510)..(6510)
<223> n is a, c, g, or t

<220>
<221> misc_feature
<222> (6557)..(6557)
<223> n is a, c, g, or t

<220>
<221> misc_feature
<222> (6564)..(6564)
<223> n is a, c, g, or t

<220>
<221> misc_feature
<222> (6613)..(6613)
<223> n is a, c, g, or t

<220>
<221> misc_feature
<222> (6615)..(6615)
<223> n is a, c, g, or t

<220>
<221> misc_feature
<222> (6626)..(6626)
<223> n is a, c, g, or t

<220>
<221> misc_feature
<222> (6631)..(6631)
<223> n is a, c, g, or t

<400> 132
agccggccgt ggtggctccg tgcgtccgag cgtccgtccg cgccgtcggc catggccaag
60

cgctccaggg gccccgggcg ccgctgcctg ttggcgctcg tgctgttctg cgcctggggg
120

acgctggccg tggtggccca gaagccgggc gcagggtgtc cgagccgctg cctgtgcttc
180

cgcaccaccg tgcgctgcat gcatctgctg ctggaggccg tgcccgccgt ggcgccgcag
240

acctccatcc tagatcttcg ctttaacaga atcagagaga tccaacctgg ggcattcagg
300

cggctgagga acttgaacac attgcttctc aataataatc agatcaagag gatacctagt
360

ggagcatttg aagacttgga aaatttaaaa tatctctatc tgtacaagaa tgagatccag
420

tcaattgaca ggcaagcatt taagggactt gcctctctag agcaactata cctgcacttt
480

aatcagatag aaactttgga cccagattcg ttccagcatc tcccgaagct cgagaggcta
540

tttttgcata acaaccggat tacacattta gttccaggga catttaatca cttggaatct
600

atgaagagat tgcgactgga ctcaaacaca cttcactgcg actgtgaaat cctgtggttg
660

gcggatttgc tgaaaaccta cgcggagtcg gggaacgcgc aggcagcggc catctgtgaa
720

tatcccagac gcatccaggg acgctcagtg gcaaccatca ccccggaaga gctgaactgt
780

gaaaggcccc ggatcacctc cgagccccag gacgcagatg tgacctcggg gaacaccgtg
840

tacttcacct gcagagccga aggcaacccc aagcctgaga tcatctggct gcgaaacaat
900

aatgagctga gcatgaagac agattcccgc ctaaacttgc tggacgatgg gaccctgatg
960

atccagaaca cacaggagac agaccagggt atctaccagt gcatggcaaa gaacgtggcc
1020

ggagaggtga agacgcaaga ggtgaccctc aggtacttcg ggtctccagc tcgacccact
1080

tttgtaatcc agccacagaa tacagaggtg ctggttgggg agagcgtcac gctggagtgc
1140

agcgccacag gccacccccc gccgcggatc tcctggacga gaggtgaccg cacacccttg
1200

ccagttgacc cgcgggtgaa catcacgcct tctggcgggc tttacataca gaacgtcgta
1260

cagggggaca gcggagagta tgcgtgctct gcgaccaaca acattgacag cgtccatgcc
1320

accgctttca tcatcgtcca ggctcttcct cagttcactg tgacgcctca ggacagagtc
1380

gttattgagg gccagaccgt ggatttccag tgtgaagcca agggcaaccc gccgcccgtc
1440

atcgcctgga ccaagggagg gagccagctc tccgtggacc ggcggcacct ggtcctgtca
1500

tcgggaacac ttagaatctc tggtgttgcc ctccacgacc agggccagta cgaatgccag
1560

gctgtcaaca tcatcggctc ccagaaggtc gtggcccacc tgactgtgca gcccagagtc
1620

accccagtgt ttgccagcat tcccagcgac acaacagtgg aggtgggcgc caatgtgcag
1680

ctcccgtgca gctcccaggg cgagcccgag ccagccatca cctggaacaa ggatggggtt
1740

caggtgacag aaagtggaaa atttcacatc agccctgaag gattcttgac catcaatgac
1800

gttggccctg cagacgcagg tcgctatgag tgtgtggccc ggaacaccat tgggtcggcc
1860

tcggtgagca tggtgctcag tgtgaacgtt cctgacgtca gtcgaaatgg agatccgttt
1920

gtagctacct ccatcgtgga agcgattgcg actgttgaca gagctataaa ctcaacccga
1980

acacatttgt ttgacagccg tcctcgttct ccaaatgatt tgctggcctt gttccggtat
2040

ccgagggatc cttacacagt tgaacaggca cgggcgggag aaatctttga acggacattg
2100

cagctcattc aggagcatgt acagcatggc ttgatggtcg acctcaacgg aacaagttac
2160

cactacaacg acctggtgtc tccacagtac ctgaacctca tcgcaaacct gtcgggctgt
2220

accgcccacc ggcgcgtgaa caactgctcg gacatgtgct tccaccagaa gtaccggacg
2280

cacgacggca cctgtaacaa cctgcagcac cccatgtggg gcgcctcgct gaccgccttc
2340

gagcgcctgc tgaaatccgt gtacgagaat ggcttcaaca cccctcgggg catcaacccc
2400

caccgactgt acaacgggca cgcccttccc atgccgcgcc tggtgtccac caccctgatc
2460

gggacggaga ccgtcacacc cgacgagcag ttcacccaca tgctgatgca gtggggccag
2520

ttcctggacc acgacctcga ctccacggtg gtggccctga gccaggcacg cttctccgac
2580

ggacagcact gcagcaacgt gtgcagcaac gaccccccct gcttctctgt catgatcccc
2640

cccaatgact cccgggccag gagcggggcc cgctgcatgt tcttcgtgcg ctccagccct
2700

gtgtgcggca gcggcatgac ttcgctgctc atgaactccg tgtacccgcg ggagcagatc
2760

aaccagctca cctcctacat cgacgcatcc aacgtgtacg ggagcacgga gcatgaggcc
2820

cgcagcatcc gcgacctggc cagccaccgc ggcctgctgc ggcagggcat cgtgcagcgg
2880

tccgggaagc cgctgctccc cttcgccacc gggccgccca cggagtgcat gcgggacgag
2940

aacgagagcc ccatcccctg cttcctggcc ggggaccacc gcgccaacga gcagctgggc
3000

ctgaccagca tgcacacgct gtggttccgc gagcacaacc gcattgccac ggagctgctc
3060

aagctgaacc cgcactggga cggcgacacc atctactatg agaccaggaa gatcgtgggt
3120

gcggagatcc agcacatcac ctaccagcac tggctcccga agatcctggg ggaggtgggc
3180

atgaggacgc tgggagagta ccacggctac gaccccggca tcaatgctgg catcttcaac
3240

gccttcgcca ccgcggcctt caggtttggc cacacgcttg tcaacccact gctttaccgg
3300

ctggacgaga acttccagcc cattgcacaa gatcacctcc cccttcacaa agctttcttc
3360

tctcccttcc ggattgtgaa tgagggcggc atcgatccgc ttctcagggg gctgttcggg
3420

gtggcgggga aaatgcgtgt gccctcgcag ctgctgaaca cggagctcac ggagcggctg
3480

ttctccatgg cacacacggt ggctctggac ctggcggcca tcaacatcca gcggggccgg
3540

gaccacggga tcccacccta ccacgactac agggtctact gcaatctatc ggcggcacac
3600

acgttcgagg acctgaaaaa tgagattaaa aaccctgaga tccgggagaa actgaaaagg
3660

ttgtatggct cgacactcaa catcgacctg tttccggcgc tcgtggtgga ggacctggtg
3720

cctggcagcc ggctgggccc caccctgatg tgtcttctca gcacacagtt caagcgcctg
3780

cgagatgggg acaggttgtg gtatgagaac cctggggtgt tctccccggc ccagctgact
3840

cagatcaagc agacgtcgct ggccaggatc ctatgcgaca acgcggacaa catcacccgg
3900

gtgcagagcg acgtgttcag ggtggcggag ttccctcacg gctacggcag ctgtgacgag
3960

atccccaggg tggacctccg ggtgtggcag gactgctgtg aagactgtag gaccaggggg
4020

cagttcaatg cctttcccta tcatttccga ggcagacggt ctcttgagtt cagctaccag
4080

gaggacaagc cgaccaagaa aacaagacca cggaaaatac ccagtgttgg gagacagggg
4140

gaacatctca gcaacagcac ctcagccttc agcacacgct cagatgcatc tgggacaaat
4200

gacttcagag agtttgttct ggaaatgcag aagaccatca cagacctcag aacacagata
4260

aagaaacttg aatcacggct cagtaccaca gagtgcgtgg atgccggggg cgaatctcac
4320

gccaacaaca ccaagtggaa aaaagatgca tgcaccattt gtgaatgcaa agacgggcag
4380

gtcacctgct tcgtggaagc ttgcccccct gccacctgtg ctgtccccgt gaacatccca
4440

ggggcctgct gtccagtctg cttacagaag agggcggagg aaaagcccta ggctcctggg
4500

aggctcctca gagtttgtct gctgtgccat cgtgagatcg ggtggccgat ggcagggagc
4560

tgcggactgc agaccaggaa acacccagaa ctcgtgacat ttcatgacaa cgtccagctg
4620

gtgctgttac agaaggcagt gcaggaggct tccaaccaga gcatctgcgg agaaggaggc
4680

acagcaggtg cctgaaggga agcaggcagg agtcctagct tcacgttaga cttctcaggt
4740

ttttatttaa ttcttttaaa atgaaaaatt ggtgctacta ttaaattgca cagttgaatc
4800

atttaggcgc ctaaattggt tttgcctccc aacaccattt ctttttaaat aaagcaggat
4860

acctctatat gtcagccttg ccttgttcag atgccaggag ccggcagacc tgtcacccgc
4920

aggtggggtg agtctcggag ctgccagagg ggctcaccga aatcggggtt ccatcacaag
4980

ctatgtttaa aaagaaaatt ggtgtttggc aaacggaaca gaacctttga tgagagcgtt
5040

cacagggaca ctgtctgggg gtgcagtgca agcccccggc ctcttccctg ggaacctctg
5100

aactcctcct tcctctgggc tctctgtaac atttcaccac acgtcagcat ctaatcccaa
5160

gacaaacatt cccgctgctc gaagcagctg tatagcctgt gactctccgt gtgtcagctc
5220

cttccacacc tgattagaac attcataagc cacatttaga aacagatttg ctttcagctg
5280

tcacttgcac acatactgcc tagttgtgaa ccaaatgtga aaaaacctcc ttcatcccat
5340

tgtgtatctg atacctgccg agggccaagg gtgtgtgttg acaacgccgc tcccagccgg
5400

ccctggttgc gtccacgtcc tgaacaagag ccgcttccgg atggctcttc ccaaggaggg
5460

aggagctcaa gtgtcgggaa ctgtctaact tcaggttgtg tgagtgcgtt aaaaaaaaaa
5520

aaaaaaaaaa gaatccctat acctcatttg tatttttaaa atgcgtgatg ttttatgaaa
5580

ttgtgtccat tttttaggta ttagatatgg cagaaaaacc atttccacta tgcaaagttc
5640

ttttagacgt cagtgaaaat caactctcat acctcatggg tctctcttta attgaccaaa
5700

accttccatt tttctcttaa atacaaagcg atctgtgttc tgagcaacct ttccccgaac
5760

acacagcttc agtgcagcac gctgacctga gtatccacca ggtgccaggc acagttgctg
5820

ggcnnacgga ggcaccaagg tccgggccac ctgcccgcag gcaaggccca gctgaggtgg
5880

tgggagggga gcccctgagg tcaggggccg tttcggttca gggtggcagg tgtccagcac
5940

tggggtatgg cgtcgaggct tccatggggt gggggaggcc agcttccttc tgacaggatg
6000

ggcgcataca gtgcctggtg tgatttgtgc acaacccgtg ttccaggtgc acatcctccc
6060

aaggagacac ccagaccctt ccagcacggg ccggccaagt tgctgcggcg gaggcagcat
6120

ttcagctgtg aggaaggtca ttggattcat gtgttttatc tgtaaaaatg gttgtcttaa
6180

cttcttaact catattggta agtgattgat aaaaattggt tggtgttttc atgacatgtg
6240

gacttctntt gnatagaagt caaatgtagt gacaatttgt ggaagagatt cttgtcaaag
6300

tgaaatagga aatgtgtaag ttcgtctaaa agctgatggt tatgtaagtt gctcaggcac
6360

tcagatgaca gcagattctg ggttctggga gtgttctgtg cctcttacat gccctggagg
6420

cctcatggtc tcagtgctga ggcggcacac ctgtagcaca cctgcgtaat gtgcggtctg
6480

ggccagtcac aaggaattgt gttgtctaan ccaaaggggg aagctgactg tgtattacca
6540

aaaaaaattc tgtaatncaa accnaaatgt ctgcggaatc accagtttga tactctctgt
6600

aatcagagca gtngnctgag ggcggncagt ncctgggtga acgtgtctag cagccactgt
6660

gggggatcgc tgtaacagga gtggaatgta catatttatt tacttttcta actgctccaa
6720

cagccaaatg ccttttttat gaccattgta ttcagttcat taccaaagaa atgtttgcac
6780

tttgtaatga tgcctttcag ttcaaataaa tgggtcacat tttcaaatgg aaaaaaaaaa
6840

aaaaaaa
6847

<210>  133
<211>  6847
<212>  DNA
<213>  Homo sapiens


<220>
<221>  misc_feature
<222>  (5824)..(6631)

<220>
<221>  misc_feature
<222>  (5824)..(5825)
<223>  n is a, c, g, or t

<220>
<221>  misc_feature
<222>  (6248)..(6248)
<223>  n is a, c, g, or t

<220>
<221>  misc_feature
<222>  (6252)..(6252)
<223>  n is a, c, g, or t

<220>
<221>  misc_feature
<222>  (6510)..(6510)
<223>  n is a, c, g, or t

<220>
<221>  misc_feature
<222>  (6557)..(6557)
<223>  n is a, c, g, or t

<220>
<221>  misc_feature
<222>  (6564)..(6564)
<223>  n is a, c, g, or t

<220>
<221>  misc_feature
<222>  (6613)..(6613)
<223>  n is a, c, g, or t

<220>
<221>  misc_feature
<222>  (6615)..(6615)
<223>  n is a, c, g, or t

<220>
<221>  misc_feature
<222>  (6626)..(6626)
<223>  n is a, c, g, or t

<220>
<221>  misc_feature
<222>  (6631)..(6631)
<223>  n is a, c, g, or t

<400>  133
   agccggccgt ggtggctccg tgcgtccgag cgtccgtccg cgccgtcggc catggccaag
   60
   cgctccaggg gccccgggcg ccgctgcctg ttggcgctcg tgctgttctg cgcctggggg
   120
   acgctggccg tggtggccca gaagccgggc gcagggtgtc cgagccgctg cctgtgcttc
   180
   cgcaccaccg tgcgctgcat gcatctgctg ctggaggccg tgcccgccgt ggcgccgcag
   240
   acctccatcc tagatcttcg ctttaacaga atcagagaga tccaacctgg ggcattcagg
   300
   cggctgagga acttgaacac attgcttctc aataataatc agatcaagag gatacctagt
   360
   ggagcatttg aagacttgga aaatttaaaa tatctctatc tgtacaagaa tgagatccag
   420
   tcaattgaca ggcaagcatt taagggactt gcctctctag agcaactata cctgcacttt
   480
   aatcagatag aaactttgga cccagattcg ttccagcatc tcccgaagct cgagaggcta
   540
   tttttgcata acaaccggat tacacattta gttccaggga catttaatca cttggaatct
   600
   atgaagagat tgcgactgga ctcaaacaca cttcactgcg actgtgaaat cctgtggttg
   660
   gcggatttgc tgaaaaccta cgcggagtcg gggaacgcgc aggcagcggc catctgtgaa
   720
   tatcccagac gcatccaggg acgctcagtg gcaaccatca ccccggaaga gctgaactgt
   780
   gaaaggcccc ggatcacctc cgagccccag gacgcagatg tgacctcggg gaacaccgtg
   840
   tacttcacct gcagagccga aggcaacccc aagcctgaga tcatctggct gcgaaacaat
   900
   aatgagctga gcatgaagac agattcccgc ctaaacttgc tggacgatgg gaccctgatg
   960
   atccagaaca cacaggagac agaccagggt atctaccagt gcatggcaaa gaacgtggcc
   1020
   ggagaggtga agacgcaaga ggtgaccctc aggtacttcg ggtctccagc tcgacccact
   1080
   tttgtaatcc agccacagaa tacagaggtg ctggttgggg agagcgtcac gctggagtgc
   1140
   agcgccacag gccacccccc gccgcggatc tcctggacga gaggtgaccg cacacccttg
   1200
   ccagttgacc cgcgggtgaa catcacgcct tctggcgggc tttacataca gaacgtcgta
   1260
   cagggggaca gcggagagta tgcgtgctct gcgaccaaca acattgacag cgtccatgcc
   1320
   accgctttca tcatcgtcca ggctcttcct cagttcactg tgacgcctca ggacagagtc
   1380
   gttattgagg gccagaccgt ggatttccag tgtgaagcca agggcaaccc gccgcccgtc
   1440
   atcgcctgga ccaagggagg gagccagctc tccgtggacc ggcggcacct ggtcctgtca
   1500
   tcgggaacac ttagaatctc tggtgttgcc ctccacgacc agggccagta cgaatgccag
   1560
   gctgtcaaca tcatcggctc ccagaaggtc gtggcccacc tgactgtgca gcccagagtc
   1620
   accccagtgt ttgccagcat tcccagcgac acaacagtgg aggtgggcgc caatgtgcag
   1680
   ctcccgtgca gctcccaggg cgagcccgag ccagccatca cctggaacaa ggatggggtt
   1740
   caggtgacag aaagtggaaa atttcacatc agccctgaag gattcttgac catcaatgac
   1800
   gttggccctg cagacgcagg tcgctatgag tgtgtggccc ggaacaccat tgggtcggcc
   1860
   tcggtgagca tggtgctcag tgtgaacgtt cctgacgtca gtcgaaatgg agatccgttt
   1920
   gtagctacct ccatcgtgga agcgattgcg actgttgaca gagctataaa ctcaacccga
   1980
   acacatttgt ttgacagccg tcctcgttct ccaaatgatt tgctggcctt gttccggtat
   2040
   ccgagggatc cttacacagt tgaacaggca cgggcgggag aaatctttga acggacattg
   2100
   cagctcattc aggagcatgt acagcatggc ttgatggtcg acctcaacgg aacaagttac
   2160
   cactacaacg acctggtgtc tccacagtac ctgaacctca tcgcaaacct gtcgggctgt
   2220
   accgcccacc ggcgcgtgaa caactgctcg gacatgtgct tccaccagaa gtaccggacg
   2280
   cacgacggca cctgtaacaa cctgcagcac cccatgtggg gcgcctcgct gaccgccttc
   2340
   gagcgcctgc tgaaatccgt gtacgagaat ggcttcaaca cccctcgggg catcaacccc
   2400
   caccgactgt acaacgggca cgcccttccc atgccgcgcc tggtgtccac caccctgatc
   2460
   gggacggaga ccgtcacacc cgacgagcag ttcacccaca tgctgatgca gtggggccag
   2520
   ttcctggacc acgacctcga ctccacggtg gtggccctga gccaggcacg cttctccgac
   2580
   ggacagcact gcagcaacgt gtgcagcaac gaccccccct gcttctctgt catgatcccc
   2640
   cccaatgact cccgggccag gagcggggcc cgctgcatgt tcttcgtgcg ctccagccct
   2700
   gtgtgcggca gcggcatgac ttcgctgctc atgaactccg tgtacccgcg ggagcagatc
   2760
   aaccagctca cctcctacat cgacgcatcc aacgtgtacg ggagcacgga gcatgaggcc
   2820
   cgcagcatcc gcgacctggc cagccaccgc ggcctgctgc ggcagggcat cgtgcagcgg
   2880
   tccgggaagc cgctgctccc cttcgccacc gggccgccca cggagtgcat gcgggacgag
   2940
   aacgagagcc ccatcccctg cttcctggcc ggggaccacc gcgccaacga gcagctgggc
   3000
   ctgaccagca tgcacacgct gtggttccgc gagcacaacc gcattgccac ggagctgctc
   3060
   aagctgaacc cgcactggga cggcgacacc atctactatg agaccaggaa gatcgtgggt
   3120
   gcggagatcc agcacatcac ctaccagcac tggctcccga agatcctggg ggaggtgggc
   3180
   atgaggacgc tgggagagta ccacggctac gaccccggca tcaatgctgg catcttcaac
   3240
   gccttcgcca ccgcggcctt caggtttggc cacacgcttg tcaacccact gctttaccgg
   3300
   ctggacgaga acttccagcc cattgcacaa gatcacctcc cccttcacaa agctttcttc
   3360
   tctcccttcc ggattgtgaa tgagggcggc atcgatccgc ttctcagggg gctgttcggg
   3420
   gtggcgggga aaatgcgtgt gccctcgcag ctgctgaaca cggagctcac ggagcggctg
   3480
   ttctccatgg cacacacggt ggctctggac ctggcggcca tcaacatcca gcggggccgg
   3540
   gaccacggga tcccacccta ccacgactac agggtctact gcaatctatc ggcggcacac
   3600
   acgttcgagg acctgaaaaa tgagattaaa aaccctgaga tccgggagaa actgaaaagg
   3660
   ttgtatggct cgacactcaa catcgacctg tttccggcgc tcgtggtgga ggacctggtg
   3720
   cctggcagcc ggctgggccc caccctgatg tgtcttctca gcacacagtt caagcgcctg
   3780
   cgagatgggg acaggttgtg gtatgagaac cctggggtgt tctccccggc ccagctgact
   3840
   cagatcaagc agacgtcgct ggccaggatc ctatgcgaca acgcggacaa catcacccgg
   3900
   gtgcagagcg acgtgttcag ggtggcggag ttccctcacg gctacggcag ctgtgacgag
   3960
   atccccaggg tggacctccg ggtgtggcag gactgctgtg aagactgtag gaccaggggg
   4020
   cagttcaatg ccttttccta tcatttccga ggcagacggt ctcttgagtt cagctaccag
   4080
   gaggacaagc cgaccaagaa aacaagacca cggaaaatac ccagtgttgg gagacagggg
   4140
   gaacatctca gcaacagcac ctcagccttc agcacacgct cagatgcatc tgggacaaat
   4200
   gacttcagag agtttgttct ggaaatgcag aagaccatca cagacctcag aacacagata
   4260
   aagaaacttg aatcacggct cagtaccaca gagtgcgtgg atgccggggg cgaatctcac
   4320
   gccaacaaca ccaagtggaa aaaagatgca tgcaccattt gtgaatgcaa agacgggcag
   4380
   gtcacctgct tcgtggaagc ttgcccccct gccacctgtg ctgtccccgt gaacatccca
   4440
   ggggcctgct gtccagtctg cttacagaag agggcggagg aaaagcccta ggctcctggg
   4500
   aggctcctca gagtttgtct gctgtgccat cgtgagatcg ggtggccgat ggcagggagc
   4560
   tgcggactgc agaccaggaa acacccagaa ctcgtgacat ttcatgacaa cgtccagctg
   4620
   gtgctgttac agaaggcagt gcaggaggct tccaaccaga gcatctgcgg agaaggaggc
   4680
   acagcaggtg cctgaaggga agcaggcagg agtcctagct tcacgttaga cttctcaggt
   4740
   ttttatttaa ttcttttaaa atgaaaaatt ggtgctacta ttaaattgca cagttgaatc
   4800
   atttaggcgc ctaaattggt tttgcctccc aacaccattt ctttttaaat aaagcaggat
   4860
   acctctatat gtcagccttg ccttgttcag atgccaggag ccggcagacc tgtcacccgc
   4920
   aggtggggtg agtctcggag ctgccagagg ggctcaccga aatcggggtt ccatcacaag
   4980
   ctatgtttaa aaagaaaatt ggtgtttggc aaacggaaca gaacctttga tgagagcgtt
   5040
   cacagggaca ctgtctgggg gtgcagtgca agcccccggc ctcttccctg ggaacctctg
   5100
   aactcctcct tcctctgggc tctctgtaac atttcaccac acgtcagcat ctaatcccaa
   5160
   gacaaacatt cccgctgctc gaagcagctg tatagcctgt gactctccgt gtgtcagctc
   5220
   cttccacacc tgattagaac attcataagc cacatttaga aacagatttg ctttcagctg
   5280
   tcacttgcac acatactgcc tagttgtgaa ccaaatgtga aaaaacctcc ttcatcccat
   5340
   tgtgtatctg atacctgccg agggccaagg gtgtgtgttg acaacgccgc tcccagccgg
   5400
   ccctggttgc gtccacgtcc tgaacaagag ccgcttccgg atggctcttc ccaagggagg
   5460
   aggagctcaa gtgtcgggaa ctgtctaact tcaggttgtg tgagtgcgtt aaaaaaaaaa
   5520
   aaaaaaaaaa gaatccctat acctcatttg tatttttaaa atgcgtgatg ttttatgaaa
   5580
   ttgtgtccat tttttaggta ttagatatgg cagaaaaacc atttccacta tgcaaagttc
   5640
   ttttagacgt cagtgaaaat caactctcat acctcatggg tctctcttta attgaccaaa
   5700
   accttccatt tttctcttaa atacaaagcg atctgtgttc tgagcaacct ttccccgaac
   5760
   acacagcttc agtgcagcac gctgacctga gtatccacca ggtgccaggc acagttgctg
   5820
   ggcnnacgga ggcaccaagg tccgggccac ctgcccgcag gcaaggccca gctgaggtgg
   5880
   tgggagggga gcccctgagg tcaggggccg tttcggttca gggtggcagg tgtccagcac
   5940
   tggggtatgg cgtcgaggct tccatggggt gggggaggcc agcttccttc tgacaggatg
   6000
   ggcgcataca gtgcctggtg tgatttgtgc acaacccgtg ttccaggtgc acatcctccc
   6060
   aaggagacac ccagaccctt ccagcacggg ccggccaagt tgctgcggcg gaggcagcat
   6120
   ttcagctgtg aggaaggtca ttggattcat gtgttttatc tgtaaaaatg gttgtcttaa
   6180
   cttcttaact catattggta agtgattgat aaaaattggt tggtgttttc atgacatgtg
   6240
   gacttctntt gnatagaagt caaatgtagt gacaatttgt ggaagagatt cttgtcaaag
   6300
   tgaaatagga aatgtgtaag ttcgtctaaa agctgatggt tatgtaagtt gctcaggcac
   6360
   tcagatgaca gcagattctg ggttctggga gtgttctgtg cctcttacat gccctggagg
   6420
   cctcatggtc tcagtgctga ggcggcacac ctgtagcaca cctgcgtaat gtgcggtctg
   6480
   ggccagtcac aaggaattgt gttgtctaan ccaaaggggg aagctgactg tgtattacca
   6540
   aaaaaaattc tgtaatncaa accnaaatgt ctgcggaatc accagtttga tactctctgt
   6600
   aatcagagca gtngnctgag ggcggncagt ncctgggtga acgtgtctag cagccactgt
   6660
   gggggatcgc tgtaacagga gtggaatgta catatttatt tacttttcta actgctccaa
   6720
   cagccaaatg ccttttttat gaccattgta ttcagttcat taccaaagaa atgtttgcac
   6780
   tttgtaatga tgcctttcag ttcaaataaa tgggtcacat tttcaaatgg aaaaaaaaaa
   6840
   aaaaaaa
   6847

<210> 134
<211> 7032
<212> DNA
<213> Homo sapiens

<400> 134
gccgcgtgcg tcctgcccgg agccggcggg acatgcccgg agcgcgcgct ggagcggcgc
60

gaggaggagg cgaacgtggt gctcaccggg acggtggagg agatcctcaa cgtggacccg
120

gtgcagcaca cgtactcctg caaggttcgg gtctggcggt acttgaaggg caaagacctg
180

gtggcccggg agagcctgct ggacggcggc aacaaggtgg tgatcagcgg ctttggagac
240

cccctcatct gtgacaacca ggtgtccact ggggacacca ggatcttctt tgtgaaccct
300

gcacccccat acctgtggcc agcccacaag aacgagctga tgctcaactc cagcctcatg
360

cggatcaccc tgcggaacct ggaggaggtg gagttctgtg tggaagataa acccgggacc
420

cacttcactc cagtgcctcc gacgcctcct gatgcgtgcc ggggaatgct gtgcggcttc
480

ggcgccgtgt gcgagcccaa cgcggagggg ccgggccggg cgtcctgcgt ctgcaagaag
540

agcccgtgcc ccagcgtggt ggcgcctgtg tgtgggtcgg acgcctccac ctacagcaac
600

gaatgcgagc tgcagcgggc gcagtgcagc cagcagcgcc gcatccgcct gctcagccgc
660

gggccgtgcg gctcgcggga cccctgctcc aacgtgacct gcagcttcgg cagcacctgt
720

gcgcgctcgg ccgacgggct gacggcctcg tgcctgtgcc ccgcgacctg ccgtggcgcc
780

cccgagggga ccgtctgcgg cagcgacggc gccgactacc ccggcgagtg ccagctcctg
840

cgccgcgcct gcgcccgcca ggagaatgtc ttcaagaagt tcgacggccc ttgtgacccc
900

tgtcagggcg ccctccctga cccgagccgc agctgccgtg tgaacccgcg cacgcggcgc
960

cctgagatgc gcctacggcc cgagagctgc cctgcccggc aggcgccagt gtgtggggac
1020

gacggagtca cctacgaaaa cgactgtgtc atgggccgat cgggggccgc ccggggtctc
1080

ctcctgcaga aagtgcgctc cggccagtgc cagggtcgag accagtgccc ggagccctgc
1140

cggttcaatg ccgtgtgcct gtcccgccgt ggccgtcccc gctgctcctg cgaccgcgtc
1200

acctgtgacg gggcctacag gcccgtgtgt gcccaggacg ggcgcacgta tgacagtgat
1260

tgctggcggc agcaggctga gtgccggcag cagcgtgcca tccccagcaa gcaccagggc
1320

ccgtgtgacc aggccccgtc cccatgcctc ggggtgcagt gtgcatttgg ggcgacgtgt
1380

gctgtgaaga acgggcaggc agcgtgtgaa tgcctgcagg cgtgctcgag cctctacgat
1440

cctgtgtgcg gcagcgacgg cgtcacatac ggcagcgcgt gcgagctgga ggccacggcc
1500

tgtaccctcg ggcgggagat ccaggtggcg cgcaaaggac cctgtgaccg ctgcgggcag
1560

tgccgctttg gagccctgtg cgaggccgag accgggcgct gcgtgtgccc ctctgaatgc
1620

gtggctttgg cccagcccgt gtgtggctcc gacgggcaca cgtaccccag cgagtgcatg
1680

ctgcacgtgc acgcctgcac acaccagatc agcctgcacg tggcctcagc tggaccctgc
1740

gagacctgtg gagatgccgt gtgtgctttt ggggctgtgt gctccgcagg gcagtgtgtg
1800

tgtccccggt gtgagcaccc cccgcccggc cccgtgtgtg gcagcgacgg tgtcacctac
1860

ggcagtgcct gcgagctacg ggaagccgcc tgcctccagc agacacagat cgaggaggcc
1920

cgggcagggc cgtgcgagca ggccgagtgc ggttccggag gctctggctc tggggaggac
1980

ggtgactgtg agcaggagct gtgccggcag cgcggtggca tctgggacga ggactcggag
2040

gacgggccgt gtgtctgtga cttcagctgc cagagtgtcc caggcagccc ggtgtgcggc
2100

tcagatgggg tcacctacag caccgagtgt gagctgaaga aggccaggtg tgagtcacag
2160

cgagggctct acgtagcggc ccagggagcc tgccgaggcc ccaccttcgc cccgctgccg
2220

cctgtggccc ccttacactg tgcccagacg ccctacggct gctgccagga caatatcacc
2280

gcagcccggg gcgtgggcct ggctggctgc cccagtgcct gccagtgcaa cccccatggc
2340

tcttacggcg gcacctgtga cccagccaca ggccagtgct cctgccgccc aggtgtgggg
2400

ggcctcaggt gtgaccgctg tgagcctggc ttctggaact ttcgaggcat cgtcaccgat
2460

ggccggagtg gctgtacacc ctgcagctgt gatccccaag gcgccgtgcg ggatgactgt
2520

gagcagatga cggggctgtg ctcgtgtaag cccggggtgg ctggacccaa gtgtgggcag
2580

tgtccagacg gccgtgccct gggccccgcg ggctgtgaag ctgacgcttc tgcgcctgcg
2640

acctgtgcgg agatgcgctg tgagttcggt gcgcggtgcg tggaggagtc tggctcagcc
2700

cactgtgtct gcccgatgct cacctgtcca gaggccaacg ctaccaaggt ctgtgggtca
2760

gatggagtca catacggcaa cgagtgtcag ctgaagacca tcgcctgccg ccagggcctg
2820

caaatctcta tccagagcct gggcccgtgc caggaggctg ttgctcccag cactcacccg
2880

acatctgcct ccgtgactgt gaccacccca gggctcctcc tgagccaggc actgccggcc
2940

ccccccggcg ccctccccct ggctcccagc agtaccgcac acagccagac cacccctccg
3000

ccctcatcgc gacctcggac cactgccagc gtccccagga ccaccgtgtg gcccgtgctg
3060

acggtgcccc ccacggcacc ctcccctgca cccagcctgg tggcgtccgc ctttggtgaa
3120

tctggcagca ctgatggaag cagcgatgag gaactgagcg gggaccagga ggccagtggg
3180

ggtggctctg gggggctcga gcccttggag ggcagcagcg tggccacccc tgggccacct
3240

gtcgagaggg cttcctgcta caactccgcg ttgggctgct gctctgatgg gaagacgccc
3300

tcgctggacg cagagggctc caactgcccc gccaccaagg tgttccaggg cgtcctggag
3360

ctggagggcg tcgagggcca ggagctgttc tacacgcccg agatggctga ccccaagtca
3420

gaactgttcg gggagacagc caggagcatt gagagcaccc tggacgacct cttccggaat
3480

tcagacgtca agaaggattt ccggagtgtc cgcttgcggg acctggggcc cggcaaatcc
3540

gtccgcgcca ttgtggatgt gcactttgac cccaccacag ccttcagggc acccgacgtg
3600

gcccgggccc tgctccggca gatccaggtg tccaggcgcc ggtccttggg ggtgaggcgg
3660

ccgctgcagg agcacgtgcg atttatggac tttgactggt ttcctgcgtt tatcacgggg
3720

gccacgtcag gagccattgc tgcgggagcc acggccagag ccaccactgc atcgcgcctg
3780

ccgtcctctg ctgtgacccc tcgggccccg caccccagtc acacaagcca gcccgttgcc
3840

aagaccacgg cagcccccac cacacgtcgg ccccccacca ctgcccccag ccgtgtgccc
3900

ggacgtcggc ccccggcccc ccagcagcct ccaaagccct gtgactcaca gccctgcttc
3960

cacgggggga cctgccagga ctgggcattg ggcgggggct tcacctgcag ctgcccggca
4020

ggcaggggag gcgccgtctg tgagaaggtg cttggcgccc ctgtgccggc cttcgagggc
4080

cgctccttcc tggccttccc caccctccgc gcctaccaca cgctgcgcct ggcactggaa
4140

ttccgggcgc tggagcctca ggggctgctg ctgtacaatg gcaacgcccg gggcaaggac
4200

ttcctggcat tggcgctgct agatggccgc gtgcagctca ggtttgacac aggttcgggg
4260

ccggcggtgc tgaccagtgc cgtgccggta gagccgggcc agtggcaccg cctggagctg
4320

tcccggcact ggcgccgggg caccctctcg gtggatggtg agacccctgt tctgggcgag
4380

agtcccagtg gcaccgacgg cctcaacctg gacacagacc tctttgtggg cggcgtaccc
4440

gaggaccagg ctgccgtggc gctggagcgg accttcgtgg gcgccggcct gagggggtgc
4500

atccgtttgc tggacgtcaa caaccagcgc ctggagcttg gcattgggcc gggggctgcc
4560

acccgaggct ctggcgtggg cgagtgcggg gaccacccct gcctgcccaa cccctgccat
4620

ggcggggccc catgccagaa cctggaggct ggaaggttcc attgccagtg cccgcccggc
4680

cgcgtcggac caacctgtgc cgatgagaag agcccctgcc agcccaaccc ctgccatggg
4740

gcggcgccct gccgtgtgct gcccgagggt ggtgctcagt gcgagtgccc cctggggcgt
4800

gagggcacct tctgccagac agcctcgggg caggacggct ctgggccctt cctggctgac
4860

ttcaacggct tctcccacct ggagctgaga ggcctgcaca cctttgcacg ggacctgggg
4920

gagaagatgg cgctggaggt cgtgttcctg gcacgaggcc ccagcggcct cctgctctac
4980

aacgggcaga agacggacgg caagggggac ttcgtgtcgc tggcactgcg ggaccgccgc
5040

ctggagttcc gctacgacct gggcaagggg gcagcggtca tcaggagcag ggagccagtc
5100

accctgggag cctggaccag ggtctcactg gagcgaaacg gccgcaaggg tgccctgcgt
5160

gtgggcgacg gcccccgtgt gttgggggag tccccggttc cgcacaccgt cctcaacctg
5220

aaggagccgc tctacgtagg gggcgctccc gacttcagca agctggcccg tgctgctgcc
5280

gtgtcctctg gcttcgacgg tgccatccag ctggtctccc tcggaggccg ccagctgctg
5340

accccggagc acgtgctgcg gcaggtggac gtcacgtcct ttgcaggtca cccctgcacc
5400

cgggcctcag gccacccctg cctcaatggg gcctcctgcg tcccgaggga ggctgcctat
5460

gtgtgcctgt gtcccggggg attctcagga ccgcactgcg agaaggggct ggtggagaag
5520

tcagcggggg acgtggatac cttggccttt gacgggcgga cctttgtcga gtacctcaac
5580

gctgtgaccg agagcgagaa ggcactgcag agcaaccact ttgaactgag cctgcgcact
5640

gaggccacgc aggggctggt gctctggagt ggcaaggcca cggagcgggc agactatgtg
5700

gcactggcca ttgtggacgg gcacctgcaa ctgagctaca acctgggctc ccagcccgtg
5760

gtgctgcgtt ccaccgtgcc cgtcaacacc aaccgctggt tgcgggtcgt ggcacatagg
5820

gagcagaggg aaggttccct gcaggtgggc aatgaggccc ctgtgaccgg ctcctccccg
5880

ctgggcgcca cgcagctgga cactgatgga gccctgtggc ttgggggcct gccggagctg
5940

cccgtgggcc cagcactgcc caaggcctac ggcacaggct ttgtgggctg cttgcgggat
6000

gtggtggtgg gccggcaccc gctgcacctg ctggaggacg ccgtcaccaa gccagagctg
6060

cggccctgcc ccaccccatg agctggcacc agagccccgc gcccgctgta attattttct
6120

atttttgtaa acttgttgct ttttgatatg attttcttgc ctgagtgttg gccggaggga
6180

ctgctggccc ggcctccctt ccgtccaggc agccgtgctg cagacagacc tagtgctgag
6240

ggatggacag gcgaggtggc agcgtggagg gctcggcgtg gatggcagcc tcaggacaca
6300

cacccctgcc tcaaggtgct gagcccccgc cttgcactgc gcctgcccca cggtgtcccc
6360

gccgggaagc agccccggct cctgaatcac cctcgctccg tcaggcggga ctcgtgtccc
6420

aaaaaggaag gggctgctga ggtctgatgg ggcccttcct ccgggtgacc ccacagggcc
6480

tttccaagcc cctatttgag ctgctccttc ctgtgtgtgc tctggaccct gcctcggcct
6540

cctgcgccaa tactgtgact tccaaacaat gttactgctg ggcacagctc tgcgttgctc
6600

ccgtgctgcc tgcgccagcc ccaggctgct gaggagcaga ggccagacca gggccgatct
6660

gggtgtcctg accctcagct ggccctgccc agccaccctg gacatgaccg tatccctctg
6720

ccacacccca ggccctgcga ggggctatcg agaggagctc actgtgggat ggggttgacc
6780

tctgccgcct gcctgggtat ctgggcctgg ccatggctgt gttcttcatg tgttgatttt
6840

atttgacccc tggagtggtg ggtctcatct ttcccatctc gcctgagagc ggctgagggc
6900

tgcctcactg caaatcctcc ccacagcgtc agtgaaagtc gtccttgtct cagaatgacc
6960

aggggccagc cagtgtctga ccaaggtcaa ggggcaggtg cagaggtggc agggatggct
7020

ccgaagccag aa
7032


<210> 135
<211> 7032
<212> DNA
<213> Homo sapiens

<400> 135
gccgcgtgcg tcctgcccgg agccggcggg acatgcccgg agcgcgcgct ggagcggcgc
60

gaggaggagg cgaacgtggt gctcaccggg acggtggagg agatcctcaa cgtggacccg
120

gtgcagcaca cgtactcctg caaggttcgg gtctggcggt acttgaaggg caaagacctg
180

gtggcccggg agagcctgct ggacggcggc aacaaggtgg tgatcagcgg ctttggagac
240

cccctcatct gtgacaacca ggtgtccact ggggacacca ggatcttctt tgtgaaccct
300

gcacccccat acctgtggcc agcccacaag aacgagctga tgctcaactc cagcctcatg
360

cggatcaccc tgcggaacct ggaggaggtg gagttctgtg tggaagataa acccgggacc
420

cacttcactc cagtgcctcc gacgcctcct gatgcgtgcc ggggaatgct gtgcggcttc
480

ggcgccgtgt gcgagcccaa cgcggagggg ccgggccggg cgtcctgcgt ctgcaagaag
540

agcccgtgcc ccagcgtggt ggcgcctgtg tgtgggtcgg acgcctccac ctacagcaac
600

gaatgcgagc tgcagcgggc gcagtgcagc cagcagcgcc gcatccgcct gctcagccgc
660

gggccgtgcg gctcgcggga cccctgctcc aacgtgacct gcagcttcgg cagcacctgt
720

gcgcgctcgg ccgacgggct gacggcctcg tgcctgtgcc ccgcgacctg ccgtggcgcc
780

cccgagggga ccgtctgcgg cagcgacggc gccgactacc ccggcgagtg ccagctcctg
840

cgccgcgcct gcgcccgcca ggagaatgtc ttcaagaagt tcgacggccc ttgtgacccc
900

tgtcagggcg ccctccctga cccgagccgc agctgccgtg tgaacccgcg cacgcggcgc
960

cctgagatgc gcctacggcc cgagagctgc cctgcccggc aggcgccagt gtgtggggac
1020

gacggagtca cctacgaaaa cgactgtgtc atgggccgat cgggggccgc ccggggtctc
1080

ctcctgcaga aagtgcgctc cggccagtgc cagggtcgag accagtgccc ggagccctgc
1140

cggttcaatg ccgtgtgcct gtcccgccgt ggccgtcccc gctgctcctg cgaccgcgtc
1200

acctgtgacg gggcctacag gcccgtgtgt gcccaggacg ggcgcacgta tgacagtgat
1260

tgctggcggc agcaggctga gtgccggcag cagcgtgcca tccccagcaa gcaccagggc
1320

ccgtgtgacc aggccccgtc cccatgcctc ggggtgcagt gtgcatttgg ggcgacgtgt
1380

gctgtgaaga acgggcaggc agcgtgtgaa tgcctgcagg cgtgctcgag cctctacgat
1440

cctgtgtgcg gcagcgacgg cgtcacatac ggcagcgcgt gcgagctgga ggccacggcc
1500

tgtaccctcg ggcgggagat ccaggtggcg cgcaaaggac cctgtgaccg ctgcgggcag
1560

tgccgctttg gagccctgtg cgaggccgag accgggcgct gcgtgtgccc ctctgaatgc
1620

gtggctttgg cccagcccgt gtgtggctcc gacgggcaca cgtaccccag cgagtgcatg
1680

ctgcacgtgc acgcctgcac acaccagatc agcctgcacg tggcctcagc tggaccctgc
1740

gagacctgtg gagatgccgt gtgtgctttt ggggctgtgt gctccgcagg gcagtgtgtg
1800

tgtccccggt gtgagcaccc cccgcccggc cccgtgtgtg gcagcgacgg tgtcacctac
1860

ggcagtgcct gcgagctacg ggaagccgcc tgcctccagc agacacagat cgaggaggcc
1920

cgggcagggc cgtgcgagca ggccgagtgc ggttccggag gctctggctc tggggaggac
1980

ggtgactgtg agcaggagct gtgccggcag cgcggtggca tctgggacga ggactcggag
2040

gacgggccgt gtgtctgtga cttcagctgc cagagtgtcc caggcagccc ggtgtgcggc
2100

tcagatgggg tcacctacag caccgagtgt gagctgaaga aggccaggtg tgagtcacag
2160

cgagggctct acgtagcggc ccagggagcc tgccgaggcc ccaccttcgc cccgctgccg
2220

cctgtggccc ccttacactg tgcccagacg ccctacggct gctgccagga caatatcacc
2280

gcagcccggg gcgtgggcct ggctggctgc cccagtgcct gccagtgcaa cccccatggc
2340

tcttacggcg gcacctgtga cccagccaca ggccagtgct cctgccgccc aggtgtgggg
2400

ggcctcaggt gtgaccgctg tgagcctggc ttctggaact ttcgaggcat cgtcaccgat
2460

ggccggagtg gctgtacacc ctgcagctgt gatccccaag gcgccgtgcg ggatgactgt
2520

gagcagatga cggggctgtg ctcgtgtaag cccggggtgg ctggacccaa gtgtgggcag
2580

tgtccagacg gccgtgccct gggccccgcg ggctgtgaag ctgacgcttc tgcgcctgcg
2640

acctgtgcgg agatgcgctg tgagttcggt gcgcggtgcg tggaggagtc tggctcagcc
2700

cactgtgtct gcccgatgct cacctgtcca gaggccaacg ctaccaaggt ctgtgggtca
2760

gatggagtca catacggcaa cgagtgtcag ctgaagacca tcgcctgccg ccagggcctg
2820

caaatctcta tccagagcct gggcccgtgc caggaggctg ttgctcccag cactcacccg
2880

acatctgcct ccgtgactgt gaccacccca gggctcctcc tgagccaggc actgccggcc
2940

ccccccggcg ccctccccct ggctcccagc agtaccgcac acagccagac cacccctccg
3000

ccctcatcgc gacctcggac cactgccagc gtccccagga ccaccgtgtg gcccgtgctg
3060

acggtgcccc ccacggcacc ctcccctgca cccagcctgg tggcgtccgc ctttggtgaa
3120

tctggcagca ctgatggaag cagcgatgag gaactgagcg gggaccagga ggccagtggg
3180

ggtggctctg gggggctcga gcccttggag ggcagcagcg tggccacccc tgggccacct
3240

gtcgagaggg cttcctgcta caactccgcg ttgggctgct gctctgatgg gaagacgccc
3300

tcgctggacg cagagggctc caactgcccc gccaccaagg tgttccaggg cgtcctggag
3360

ctggagggcg tcgagggcca ggagctgttc tacacgcccg agatggctga ccccaagtca
3420

gaactgttcg gggagacagc caggagcatt gagagcaccc tggacgacct cttccggaat
3480

tcagacgtca agaaggattt ccggagtgtc cgcttgcggg acctggggcc cggcaaatcc
3540

gtccgcgcca ttgtggatgt gcactttgac cccaccacag ccttcagggc acccgacgtg
3600

gcccgggccc tgctccggca gatccaggtg tccaggcgcc ggtccttggg ggtgaggcgg
3660

ccgctgcagg agcacgtgcg atttatggac tttgactggt ttcctgcgtt tatcacgggg
3720

gccacgtcag gagccattgc tgcgggagcc acggccagag ccaccactgc atcgcgcctg
3780

ccgtcctctg ctgtgacccc tcgggccccg caccccagtc acacaagcca gcccgttgcc
3840

aagaccacgg cagcccccac cacacgtcgg ccccccacca ctgcccccag ccgtgtgccc
3900

ggacgtcggc cccccggccc ccagcagcct ccaaagccct gtgactcaca gccctgcttc
3960

cacgggggga cctgccagga ctgggcattg ggcgggggct tcacctgcag ctgcccggca
4020

ggcaggggag gcgccgtctg tgagaaggtg cttggcgccc ctgtgccggc cttcgagggc
4080

cgctccttcc tggccttccc caccctccgc gcctaccaca cgctgcgcct ggcactggaa
4140

ttccgggcgc tggagcctca ggggctgctg ctgtacaatg gcaacgcccg gggcaaggac
4200

ttcctggcat tggcgctgct agatggccgc gtgcagctca ggtttgacac aggttcgggg
4260

ccggcggtgc tgaccagtgc cgtgccggta gagccgggcc agtggcaccg cctggagctg
4320

tcccggcact ggcgccgggg caccctctcg gtggatggtg agacccctgt tctgggcgag
4380

agtcccagtg gcaccgacgg cctcaacctg gacacagacc tctttgtggg cggcgtaccc
4440

gaggaccagg ctgccgtggc gctggagcgg accttcgtgg gcgccggcct gagggggtgc
4500

atccgtttgc tggacgtcaa caaccagcgc ctggagcttg gcattgggcc gggggctgcc
4560

acccgaggct ctggcgtggg cgagtgcggg gaccacccct gcctgcccaa cccctgccat
4620

ggcggggccc catgccagaa cctggaggct ggaaggttcc attgccagtg cccgcccggc
4680

cgcgtcggac caacctgtgc cgatgagaag agcccctgcc agcccaaccc ctgccatggg
4740

gcggcgccct gccgtgtgct gcccgagggt ggtgctcagt gcgagtgccc cctggggcgt
4800

gagggcacct tctgccagac agcctcgggg caggacggct ctgggccctt cctggctgac
4860

ttcaacggct tctcccacct ggagctgaga ggcctgcaca cctttgcacg ggacctgggg
4920

gagaagatgg cgctggaggt cgtgttcctg gcacgaggcc ccagcggcct cctgctctac
4980

aacgggcaga agacggacgg caagggggac ttcgtgtcgc tggcactgcg ggaccgccgc
5040

ctggagttcc gctacgacct gggcaagggg gcagcggtca tcaggagcag ggagccagtc
5100

accctgggag cctggaccag ggtctcactg gagcgaaacg gccgcaaggg tgccctgcgt
5160

gtgggcgacg gcccccgtgt gttgggggag tccccggttc cgcacaccgt cctcaacctg
5220

aaggagccgc tctacgtagg gggcgctccc gacttcagca agctggcccg tgctgctgcc
5280

gtgtcctctg gcttcgacgg tgccatccag ctggtctccc tcggaggccg ccagctgctg
5340

accccggagc acgtgctgcg gcaggtggac gtcacgtcct ttgcaggtca cccctgcacc
5400

cgggcctcag gccacccctg cctcaatggg gcctcctgcg tcccgaggga ggctgcctat
5460

gtgtgcctgt gtcccggggg attctcagga ccgcactgcg agaaggggct ggtggagaag
5520

tcagcggggg acgtggatac cttggccttt gacgggcgga cctttgtcga gtacctcaac
5580

gctgtgaccg agagcgagaa ggcactgcag agcaaccact ttgaactgag cctgcgcact
5640

gaggccacgc aggggctggt gctctggagt ggcaaggcca cggagcgggc agactatgtg
5700

gcactggcca ttgtggacgg gcacctgcaa ctgagctaca acctgggctc ccagcccgtg
5760

gtgctgcgtt ccaccgtgcc cgtcaacacc aaccgctggt tgcgggtcgt ggcacatagg
5820

gagcagaggg aaggttccct gcaggtgggc aatgaggccc ctgtgaccgg ctcctccccg
5880

ctgggcgcca cgcagctgga cactgatgga gccctgtggc ttgggggcct gccggagctg
5940

cccgtgggcc cagcactgcc caaggcctac ggcacaggct ttgtgggctg cttgcgggat
6000

gtggtggtgg gccggcaccc gctgcacctg ctggaggacg ccgtcaccaa gccagagctg
6060

cggccctgcc ccaccccatg agctggcacc agagccccgc gcccgctgta attattttct
6120

atttttgtaa acttgttgct ttttgatatg attttcttgc ctgagtgttg gccggaggga
6180

ctgctggccc ggcctccctt ccgtccaggc agccgtgctg cagacagacc tagtgctgag
6240

ggatggacag gcgaggtggc agcgtggagg gctcggcgtg gatggcagcc tcaggacaca
6300

cacccctgcc tcaaggtgct gagcccccgc cttgcactgc gcctgcccca cggtgtcccc
6360

gccgggaagc agccccggct cctgaatcac cctcgctccg tcaggcggga ctcgtgtccc
6420

aaaaaggaag gggctgctga ggtctgatgg ggcccttcct ccgggtgacc ccacagggcc
6480

tttccaagcc cctatttgag ctgctccttc ctgtgtgtgc tctggaccct gcctcggcct
6540

cctgcgccaa tactgtgact tccaaacaat gttactgctg ggcacagctc tgcgttgctc
6600

ccgtgctgcc tgcgccagcc ccaggctgct gaggagcaga ggccagacca gggccgatct
6660

gggtgtcctg accctcagct ggccctgccc agccaccctg gacatgaccg tatccctctg
6720

ccacacccca ggccctgcga ggggctatcg agaggagctc actgtgggat ggggttgacc
6780

tctgccgcct gcctgggtat ctgggcctgg ccatggctgt gttcttcatg tgttgatttt
6840

atttgacccc tggagtggtg ggtctcatct ttcccatctc gcctgagagc ggctgagggc
6900

tgcctcactg caaatcctcc ccacagcgtc agtgaaagtc gtccttgtct cagaatgacc
6960

aggggccagc cagtgtctga ccaaggtcaa ggggcaggtg cagaggtggc agggatggct
7020

ccgaagccag aa
7032

<210>  136
<211>  859
<212>  DNA
<213>  Homo sapiens

<400>  136
agcaaccttg aaaaaataga cttaaatgaa tgcttctaga aacttccagc ggctcacaaa
60

gaataagcct gccttagggc tggcaacatc taagcctcta acagcacagg gaagcaaata
120

tcttaccagg cagcctatga attaacccaa agaagctttg gttggtttgg tggattttta
180

tcatgccatg ttggacatga gattttttag atcttccttc ccacattgct agacgtctca
240

ctcaaagaca tttgttggga gtcacatttg catcatagac gagacagtcc attcatctta
300

gttaaattgg attgagaatg ccttttgttt ccaggaaaat attgatcacc atgaaagaag
360

aatagttttt tgtccccaga gacattcatt tagttgatat aatcctacca gaaggaaagc
420

actaagaaac actcgttgtg gtttttaaag gcaacagact taaagttgtc ctcagccaag
480

gaaaaatgat actgcaactt taaaatttaa agtatcttgc actgataaat atatttaaaa
540

attatatgtt tataaagtta ttaatttgta aaaggcagtg ttacaaaaaa tgttcagttt
600

atattgtgtc tcagatgttt gtaattttta aaggtgtcaa ataacatatt tttttcttta
660

tggaaatcta taaaactttc tgtagtaaaa tgttcttcat tttactgcta aacaaacaca
720

cacaacacaa agggggcggg gtaaaaattc ccggggggcc agttaccctc caggttctgg
780

acaagggccc atgggagcga ataaacagcg gggggttaaa ggcccggaaa gcctgggtcg
840

ggggcctctg ggggcccgg
859

<210>  137
<211>  2581
<212>  DNA
<213>  Homo sapiens

<400>  137

tcaaatgaga aaattgcaag tagtgtgaca gagctgattg attttgttgc tttcttgatt
60

ttttttttca aaatgggttt actaaaatgt agatgactta actgcctcct ccttcgtctg
120

aaaaatgcca atattcaatc atcatgcagc attataacaa gccttataag tcctaaagca
180

ttaagttgca cttttttgag gaggggtagt gcagtatttc tctggccagt atgaatgaag
240

tttatactta ccatatttga tagaaacata gatcaagcta tggcacagcg actcatcaga
300

tagctagctt tgacgtctgg gcacaattga accaacttcc atcgtgaatc tttataatga
360

ttgactttgg tgtatagtgc agtaaacaaa tagtgctcct agttaagtat ttgtcagcat
420

ccttttgtct ctaacttgtt tctattttta cagccacaca attcttggca tgtattaaga
480

aaaaaaaaaa tccctgttca agtagttttt ccacctatca gcactgagta aatgccataa
540

atccattgaa atggtctaaa tgttccatct gttctcctgt tttgccagtt atatagtaat
600

gaaatacatt tgtaaatttt atgcaacaaa tggcaaacgt atcattattt tgaaattgtg
660

tatgtaaaag ttatattttt acatgtagac tcttgttatt atgtgtttta atacattgta
720

tcagtttttg ttttttttta aaaactgtgg tttaaaaaga agtctcattt aaatgaaata
780

gctacaagaa tcagaatttt atgttcattt ctgaaaatgt aagaacaaat aagatagtta
840

ccacgtggtc atcttttaca aacccataaa cattttgatt agctgtgtgt gtgttgaaaa
900

actgtaaata tgttcagtag cgataaaact aaaataactt tgatttgttg ataagttcct
960

aaaatgtgga ggtggattaa aaccttagga gaatagcaga aatcaaactt catgaaaagt
1020

tattttgggg ctttcctgtg aaatgtatga acaaagaggc tcagagaagg acatggaaga
1080

caataatgta tactctctcc tcctccctga ataatgaaaa ccatgtgtat ttgttccctc
1140

cgtatgttaa agatttcctt ttagtggtac attctgcact cattttgtat agtctaccaa
1200

ggcgggtatc cctaggaaca atattatata ggaagcaggt atactctgat cacattcagg
1260

ataagtgtac agaagaaaat acggtgttta ctctttaggg aactggaaac actccctgca
1320

ttgatgtaca ttttaagaat ggcacttttg atacatgtta tcataaaggt gcttaataga
1380

gctgaattaa agtttttcaa atctgtaaac aaagcaaaaa agtaaattgt agtcatttga
1440

ttatttttta aattggtgct ttatattttg ttctcactca gagtaaaagc tgcaatttat
1500

tgttcaccag ctttgatgta ttcattactc agtaatgcaa tacctctatt gttgaattcc
1560

ctttggaaat aagtgaaaat tctaacggcc actgaaagct gctcgctagg ttttgcttgg
1620

tggagaaaca taatctgcac ctatccatat taattgggtt gtatccccat taaaaaagaa
1680

aaaaagggaa tgtggccttt ttagtgtgtt ttttattgtt gttgttttgt aattatcaaa
1740

cccaggtaag atattggtat cctgcactgg atcttcaaat gaagttcagc agaagacagt
1800

taagattaaa gtactataca aaaatttcaa aagggtcctt actacgctat ctgtatgacg
1860

acacttaggc tggggatctc tttcagaaac tcggacttta aaagcaactt ggagcagttg
1920

atccacctcc acattcaagt aatttatgaa tatgcagaat agggatctgt tcatctagaa
1980

atttttacca tttgtcttct gtgtagctgc aaggaacact aatgtttata caactgtcag
2040

tccacccagt ggtgcaactg gttctgattc agtcttccga ttccttttta tttttcactt
2100

tttcctattt ctgaattttt tttttatttt gtgatcttga ttttgatgag gggttgggga
2160

gtggggaggg agtcgaacca agacttggag ttaagaggat tttcatcttt tgcatccaac
2220

aggcagaata tgatctgtgt ccaaaagtga acttgagtca ggattgaatc aatttcagca
2280

taaacaagca caaaaattta gtctgctggc tgactggaag caaaaaagtc aagatggaat
2340

atgatgaatt ccaacacaat ggggcaccaa ggcctttagg cctctctttt tattttgctt
2400

tggttttgtt tgtttttctt tagagacatg ctctttctca tgggacttga agtggactca
2460

tctttgtgca gtgctggttt tgccatactc atttcaagta ttatagacat atgtaatggt
2520

gaaaatatat gaactgtggc ctttttcatt cttgttactt gtgatgcaat taagtgaaga
2580

t 2
581

<210>  138
<211>  2581
<212>  DNA
<213>  Homo sapiens

<400>  138
tcaaatgaga aaattgcaag tagtgtgaca gagctgattg attttgttgc tttcttgatt
60

ttttttttca aaatgggttt actaaaatgt agatgactta actgcctcct ccttcgtctg
120

aaaaatgcca atattcaatc atcatgcagc attataacaa gccttataag tcctaaagca
180

ttaagttgca cttttttgag gaggggtagt gcagtatttc tctggccagt atgaatgaag
240

tttatactta ccatatttga tagaaacata gatcaagcta tggcacagcg actcatcaga
300

tagctagctt tgacgtctgg gcacaattga accaacttcc atcgtgaatc tttataatga
360

ttgactttgg tgtatagtgc agtaaacaaa tagtgctcct agttaagtat ttgtcagcat
420

ccttttgtct ctaacttgtt tctattttta cagccacaca attcttggca tgtattaaga
480

aaaaaaaaaa tccctgttca agtagttttt ccacctatca gcactgagta aatgccataa
540

atccattgaa atggtctaaa tgttccatct gttctcctgt tttgccagtt atatagtaat
600

gaaatacatt tgtaaatttt atgcaacaaa tggcaaacgt atcattattt tgaaattgtg
660

tatgtaaaag ttatattttt acatgtagac tcttgttatt atgtgtttta atacattgta
720

tcagtttttg ttttttttta aaaactgtgg tttaaaaaga agtctcattt aaatgaaata
780

gctacaagaa tcagaatttt atgttcattt ctgaaaatgt aagaacaaat aagatagtta
840

ccacgtggtc atcttttaca aacccataaa cattttgatt agctgtgtgt gtgttgaaaa
900

actgtaaata tgttcagtag cgataaaact aaaataactt tgatttgttg ataagttcct
960

aaaatgtgga ggtggattaa aaccttagga gaatagcaga aatcaaactt catgaaaagt
1020

tattttgggg ctttcctgtg aaatgtatga acaaagaggc tcagagaagg acatggaaga
1080

caataatgta tactctctcc tcctccctga ataatgaaaa ccatgtgtat ttgttccctc
1140

cgtatgttaa agatttcctt ttagtggtac attctgcact cattttgtat agtctaccaa
1200

ggcgggtatc cctaggaaca atattatata ggaagcaggt atactctgat cacattcagg
1260

ataagtgtac agaagaaaat acggtgttta ctctttaggg aactggaaac actccctgca
1320

ttgatgtaca ttttaagaat ggcacttttg atacatgtta tcataaaggt gcttaataga
1380

gctgaattaa agtttttcaa atctgtaaac aaagcaaaaa agtaaattgt agtcatttga
1440

ttatttttta aattggtgct ttatattttg ttctcactca gagtaaaagc tgcaatttat
1500

tgttcaccag ctttgatgta ttcattactc agtaatgcaa tacctctatt gttgaattcc
1560

ctttggaaat aagtgaaaat tctaacggcc actgaaagct gctcgctagg ttttgcttgg
1620

tggagaaaca taatctgcac ctatccatat taattgggtt gtatccccat taaaaaagaa
1680

aaaaagggaa tgtggccttt ttagtgtgtt ttttattgtt gttgttttgt aattatcaaa
1740

cccaggtaag atattggtat cctgcactgg atcttcaaat gaagttcagc agaagacagt
1800

taagattaaa gtactataca aaaatttcaa aagggtcctt actacgctat ctgtatgacg
1860

acacttaggc tggggatctc tttcagaaac tcggacttta aaagcaactt ggagcagttg
1920

atccacctcc acattcaagt aatttatgaa tatgcagaat agggatctgt tcatctagaa
1980

atttttacca tttgtcttct gtgtagctgc aaggaacact aatgtttata caactgtcag
2040

tccacccagt ggtgcaactg gttctgattc agtcttccga ttccttttta tttttcactt
2100

tttcctattt ctgaattttt ttttttattt gtgatcttga ttttgatgag gggttgggga
2160

gtggggaggg agtcgaacca agacttggag ttaagaggat tttcatcttt tgcatccaac
2220

aggcagaata tgatctgtgt ccaaaagtga acttgagtca ggattgaatc aatttcagca
2280

taaacaagca caaaaattta gtctgctggc tgactggaag caaaaaagtc aagatggaat
2340

atgatgaatt ccaacacaat ggggcaccaa ggcctttagg cctctctttt tattttgctt
2400

tggttttgtt tgtttttctt tagagacatg ctctttctca tgggacttga agtggactca
2460

tctttgtgca gtgctggttt tgccatactc atttcaagta ttatagacat atgtaatggt
2520

gaaaatatat gaactgtggc ctttttcatt cttgttactt gtgatgcaat taagtgaaga
2580

t                                                                 2
581


<210>  139
<211>  2581
<212>  DNA
<213>  Homo sapiens

<400>  139
tcaaatgaga aaattgcaag tagtgtgaca gagctgattg attttgttgc tttcttgatt
60

ttttttttca aaatgggttt actaaaatgt agatgactta actgcctcct ccttcgtctg
120

aaaaatgcca atattcaatc atcatgcagc attataacaa gccttataag tcctaaagca
180

ttaagttgca cttttttgag gaggggtagt gcagtatttc tctggccagt atgaatgaag
240

tttatactta ccatatttga tagaaacata gatcaagcta tggcacagcg actcatcaga
300

tagctagctt tgacgtctgg gcacaattga accaacttcc atcgtgaatc tttataatga
360

ttgactttgg tgtatagtgc agtaaacaaa tagtgctcct agttaagtat ttgtcagcat
420

ccttttgtct ctaacttgtt tctattttta cagccacaca attcttggca tgtattaaga
480

aaaaaaaaaa tccctgttca agtagttttt ccacctatca gcactgagta aatgccataa
540

atccattgaa atggtctaaa tgttccatct gttctcctgt tttgccagtt atatagtaat
600

gaaatacatt tgtaaatttt atgcaacaaa tggcaaacgt atcattattt tgaaattgtg
660

tatgtaaaag ttatattttt acatgtagac tcttgttatt atgtgtttta atacattgta
720

tcagtttttg ttttttttta aaaactgtgg tttaaaaaga agtctcattt aaatgaaata
780

gctacaagaa tcagaatttt atgttcattt ctgaaaatgt aagaacaaat aagatagtta
840

ccacgtggtc atcttttaca aacccataaa cattttgatt agctgtgtgt gtgttgaaaa
900

actgtaaata tgttcagtag cgataaaact aaaataactt tgatttgttg ataagttcct
960

aaaatgtgga ggtggattaa aaccttagga gaatagcaga aatcaaactt catgaaaagt
1020

tattttgggg ctttcctgtg aaatgtatga acaaagaggc tcagagaagg acatggaaga

1080

caataatgta tactctctcc tcctccctga ataatgaaaa ccatgtgtat ttgttccctc
1140

cgtatgttaa agatttcctt ttagtggtac attctgcact cattttgtat agtctaccaa
1200

ggcgggtatc cctaggaaca atattatata ggaagcaggt atactctgat cacattcagg
1260

ataagtgtac agaagaaaat acggtgttta ctctttaggg aactggaaac actccctgca
1320

ttgatgtaca ttttaagaat ggcacttttg atacatgtta tcataaaggt gcttaataga
1380

gctgaattaa agtttttcaa atctgtaaac aaagcaaaaa agtaaattgt agtcatttga
1440

ttatttttta aattggtgct ttatattttg ttctcactca gagtaaaagc tgcaatttat
1500

tgttcaccag ctttgatgta ttcattactc agtaatgcaa tacctctatt gttgaattcc
1560

ctttggaaat aagtgaaaat tctaacggcc actgaaagct gctcgctagg ttttgcttgg
1620

tggagaaaca taatctgcac ctatccatat taattgggtt gtatccccat taaaaaagaa
1680

aaaaagggaa tgtggccttt ttagtgtgtt ttttattgtt gttgttttgt aattatcaaa
1740

cccaggtaag atattggtat cctgcactgg atcttcaaat gaagttcagc agaagacagt
1800

taagattaaa gtactataca aaaatttcaa aagggtcctt actacgctat ctgtatgacg
1860

acacttaggc tggggatctc tttcagaaac tcggacttta aaagcaactt ggagcagttg
1920

atccacctcc acattcaagt aatttatgaa tatgcagaat agggatctgt tcatctagaa
1980

atttttacca tttgtcttct gtgtagctgc aaggaacact aatgtttata caactgtcag
2040

tccacccagt ggtgcaactg gttctgattc agtcttccga ttccttttta tttttcactt
2100

tttcctattt ctgaattttt tttttatttt gtgatcttga ttttgatgag gggttgggga
2160

gtggggaggg agtcgaacca agacttggag ttaagaggat tttcatcttt tgcatccaac
2220

aggcagaata tgatctgtgt ccaaaagtga acttgagtca ggattgaatc aatttcagca
2280

taaacaagca caaaaattta gtctgctggc tgactggaag caaaaaagtc aagatggaat
2340

atgatgaatt ccaacacaat ggggcaccaa ggcctttagg cctctctttt tattttgctt

2400

tggttttgtt tgtttttctt tagagacatg ctctttctca tgggacttga agtggactca
2460

tctttgtgca gtgctggttt tgccatactc atttcaagta ttatagacat atgtaatggt
2520

gaaaatatat gaactgtggc ctttttcatt cttgttactt gtgatgcaat taagtgaaga
2580

t                                                                 2
581

<210>  140
<211>  2581
<212>  DNA
<213>  Homo sapiens

<400>  140
   tcaaatgaga aaattgcaag tagtgtgaca gagctgattg attttgttgc tttcttgatt
   60
   ttttttttca aaatgggttt actaaaatgt agatgactta actgcctcct ccttcgtctg
   120
   aaaaatgcca atattcaatc atcatgcagc attataacaa gccttataag tcctaaagca
   180
   ttaagttgca cttttttgag gaggggtagt gcagtatttc tctggccagt atgaatgaag
   240
   tttatactta ccatatttga tagaaacata gatcaagcta tggcacagcg actcatcaga
   300
   tagctagctt tgacgtctgg gcacaattga accaacttcc atcgtgaatc tttataatga
   360
   ttgactttgg tgtatagtgc agtaaacaaa tagtgctcct agttaagtat ttgtcagcat
   420
   ccttttgtct ctaacttgtt tctattttta cagccacaca attcttggca tgtattaaga
   480
   aaaaaaaaaa tccctgttca agtagttttt ccacctatca gcactgagta aatgccataa
   540
   atccattgaa atggtctaaa tgttccatct gttctcctgt tttgccagtt atatagtaat
   600
   gaaatacatt tgtaaatttt atgcaacaaa tggcaaacgt atcattattt tgaaattgtg
   660
   tatgtaaaag ttatattttt acatgtagac tcttgttatt atgtgtttta atacattgta
   720
   tcagtttttg ttttttttta aaaactgtgg tttaaaaaga agtctcattt aaatgaaata
   780
   gctacaagaa tcagaatttt atgttcattt ctgaaaatgt aagaacaaat aagatagtta
   840
   ccacgtggtc atcttttaca aacccataaa cattttgatt agctgtgtgt gtgttgaaaa
   900
   actgtaaata tgttcagtag cgataaaact aaaataactt tgatttgttg ataagttcct
   960
   aaaatgtgga ggtggattaa aaccttagga gaatagcaga aatcaaactt catgaaaagt
   1020
   tattttgggg ctttcctgtg aaatgtatga acaaagaggc tcagagaagg acatggaaga
   1080
   caataatgta tactctctcc tcctccctga ataatgaaaa ccatgtgtat ttgttccctc
   1140
   cgtatgttaa agatttcctt ttagtggtac attctgcact cattttgtat agtctaccaa
   1200
   ggcgggtatc cctaggaaca atattatata ggaagcaggt atactctgat cacattcagg
   1260
   ataagtgtac agaagaaaat acggtgttta ctctttaggg aactggaaac actccctgca
   1320
   ttgatgtaca ttttaagaat ggcacttttg atacatgtta tcataaaggt gcttaataga
   1380
   gctgaattaa agtttttcaa atctgtaaac aaagcaaaaa agtaaattgt agtcatttga
   1440
   ttatttttta aattggtgct ttatattttg ttctcactca gagtaaaagc tgcaatttat
   1500
   tgttcaccag ctttgatgta ttcattactc agtaatgcaa tacctctatt gttgaattcc
   1560
   ctttggaaat aagtgaaaat tctaacggcc actgaaagct gctcgctagg ttttgcttgg
   1620
   tggagaaaca taatctgcac ctatccatat taattgggtt gtatccccat taaaaaagaa
   1680
   aaaaagggaa tgtggccttt ttagtgtgtt ttttattgtt gttgttttgt aattatcaaa
   1740
   cccaggtaag atattggtat cctgcactgg atcttcaaat gaagttcagc agaagacagt
   1800
   taagattaaa gtactataca aaaatttcaa aagggtcctt actacgctat ctgtatgacg
   1860
   acacttaggc tggggatctc tttcagaaac tcggacttta aaagcaactt ggagcagttg
   1920
   atccacctcc acattcaagt aatttatgaa tatgcagaat agggatctgt tcatctagaa
   1980
   atttttacca tttgtcttct gtgtagctgc aaggaacact aatgtttata caactgtcag
   2040
   tccacccagt ggtgcaactg gttctgattc agtcttccga ttccttttta tttttcactt
   2100
   tttcctattt ctgaattttt tttttatttt gtgatcttga ttttgatgag gggttgggga
   2160
   gtggggaggg agtcgaacca agacttggag ttaagaggat tttcatcttt tgcatccaac
   2220
   aggcagaata tgatctgtgt ccaaaagtga acttgagtca ggattgaatc aatttcagca
   2280
   taaacaagca caaaaattta gtctgctggc tgactggaag caaaaaagtc aagatggaat
   2340
   atgatgaatt ccaacacaat ggggcaccaa ggcctttagg cctctctttt tattttgctt
   2400
   tggttttgtt tgtttttctt tagagacatg ctctttctca tgggacttga agtggactca
   2460
   tctttgtgca gtgctggttt tgccatactc atttcaagta ttatagacat atgtaatggt
   2520
   gaaaatatat gaactgtggc ctttttcatt cttgttactt gtgatgcaat taagtgaaga
   2580
   t                                                                 2581

<210>  141
<211>  597
<212>  DNA
<213>  Homo sapiens

<400>  141
atgcccctag gtctcctgtg gctgggccta gccctgttgg gggctctgca tgcccaggcc
60

caggactcca cctcagacct gatcccagcc ccacctctga gcaaggtccc tctgcagcag
120

aacttccagg acaaccaatt ccaggggaag tggtatgtgg taggcctggc agggaatgca
180

attctcagag aagacaaaga cccgcaaaag atgtatgcca ccatctatga gctgaaagaa
240

gacaagagct acaatgtcac ctccgtcctg tttaggaaaa agaagtgtga ctactggatc
300

aggacttttg ttccaggttg ccagcccggc gagttcacgc tgggcaacat taagagttac
360

cctggattaa cgagttacct cgtccgagtg gtgagcacca actacaacca gcatgctatg
420

gtgttcttca agaaagtttc tcaaaacagg gagtacttca agatcaccct ctacgggaga
480

accaaggagc tgacttcgga actaaaggag aacttcatcc gcttctccaa atatctgggc
540

ctccctgaaa accacatcgt cttccctgtc ccaatcgacc agtgtatcga cggctga
597

<210>  142
<211>  903
<212>  DNA
<213>  Homo sapiens

<220>

<221>  misc_feature
<222>  (43)..(44)

<220>
<221>  misc_feature
<222>  (43)..(43)
<223>  n is a, c, g, or t

<400>  142
tttttttttt wttttkthtt tgccttaaat cacaaagagc aantgggaag aacaagctcc
60

ctttctcctt ttctcagaca cctttggtct aaagttgttc tttttctagt tcttccctcc
120

cctgtaattt ttaattatca gaaataaata ccagtgcatt tcaggtggtc gtctgtgtta
180

gagtgcataa ctgaagttat tagtggcttg ggagtttgca catataactg gaggtaaaca
240

gcagattact acacctaatc tcaccctgag catttctcct ctgtcaagtc taaattctgc
300

catcttcttt tgcaaaaaca agcagacatc cccaccaaag gccaaaatat aatgataact
360

gacagtgaac agaaacccat tgtaaagaca aactattcca gctttcaaat tcactgtgac
420

tggatactat tagttttttg agtttataac tccccagaat tgtcaaaagt aaccttgtag
480

ccaatccgct tgcagacact aaaaaccacc tgamatasgc cacccagggt accaaaagca
540

gtgtcaaasa scagtgagac mstsccacca aggcccagtg caatgycctt gcmaacmatt
600

sgmatascgc ccatsgtgta cacasggaaa gtggccacat ccacaaggmc acggacaggg
660

atgctcasaa ctcsgcaaat ggccttcagc agacaacaaa sgatccggac gcagagccct
720

gatgacttts gccacgacct tgaccmcctt ccagggaatg ctcaccaact cttccccaat
780

ggccatgaag tatgagatgg tggctgagcc caggcggtaa aggcaacttt ctatgccatt
840

aatcacctgc ttggtaacac accacaggaa atagacaatg ttcttcagga ttgctaccac
900

aag
903


<210>  143
<211>  903
<212>  DNA
<213>  Homo sapiens


<220>
<221>  misc_feature

<222>  (43)..(44)

<220>
<221>  misc_feature
<222>  (43)..(43)
<223>  n is a, c, g, or t

<400>  143
tttttttttt wttttkthtt tgccttaaat cacaaagagc aantgggaag aacaagctcc
60

ctttctcctt ttctcagaca cctttggtct aaagttgttc tttttctagt tcttccctcc
120

cctgtaattt ttaattatca gaaataaata ccagtgcatt tcaggtggtc gtctgtgtta
180

gagtgcataa ctgaagttat tagtggcttg ggagtttgca catataactg gaggtaaaca
240

gcagattact acacctaatc tcaccctgag catttctcct ctgtcaagtc taaattctgc
300

catcttcttt tgcaaaaaca agcagacatc cccaccaaag gccaaaatat aatgataact
360

gacagtgaac agaaacccat tgtaaagaca aactattcca gctttcaaat tcactgtgac
420

tggatactat tagttttttg agtttataac tccccagaat tgtcaaaagt aaccttgtag
480

ccaatccgct tgcagacact aaaaaccacc tgamatasgc cacccagggt accaaaagca
540

gtgtcaaasa scagtgagac mstsccacca aggcccagtg caatgycctt gcmaacmatt
600

sgmatascgc ccatsgtgta cacasggaaa gtggccacat ccacaaggmc acggacaggg
660

atgctcasaa ctcsgcaaat ggccttcagc agacaacaaa sgatccggac gcagagccct
720

gatgacttts gccacgacct tgaccmcctt ccagggaatg ctcaccaact cttccccaat
780

ggccatgaag tatgagatgg tggctgagcc caggcggtaa aggcaacttt ctatgccatt
840

aatcacctgc ttggtaacac accacaggaa atagacaatg ttcttcagga ttgctaccac
900

aag
903


<210>  144
<211>  1140
<212>  DNA
<213>  Homo sapiens


<220>
<221>  misc_feature
<222>  (10)..(11)

<220>
<221>  misc_feature
<222>  (10)..(10)
<223>  n is a, c, g, or t

<400>  144
gtcggggtgn cctctcttcc tcacaggcac caggccttgt ttctctccca gataagctaa
60

ggtcggcagg ggctgagggg agtgaggcag ggaggatgca tggggcctgt gggaatcaga
120

gagaagctgg ggtcagaggt aggagctgac gctgtggggg gtggggggtg aaaggtgagg
180

caaggtcaaa ggtgaactgc agctcggagg gaggggtcca atataaacca ggacttgcag
240

gtgtagaagt aaaatgggac tcatagaggg tgaaagagaa gttggagtca gaggggattc
300

agagatcaaa gagaagttgg ggtcagaggt aggagctggg gctcaggtgg ggggttaaag
360

gtgaggcaag gtcagcggtg aagtgcagct cagagggagg ggtcaaacat aaaccagaac
420

ttataggtct agaggtaaaa tgggattcat ggggggcaga ggtcaaaggt gaagcagaag
480

tcaggggtga aggaaggtct gcaaagttaa aggtgcggtt tccagagtga gaaggggtga
540

agcggggctc ctcctgggaa tgtcaaggtt ggaagagctc aagggggtta aagataaatt
600

agggctcaaa ggggagccag ggtcggagat gaagtagcca gaggtaaagc gagctccaaa
660

ggtrkkgggg gggtsagggg ggagtcagcc ttcgagggaa agcggggctc tagggttcag
720

agatgggggg cctagcggat caaaggtcag aagcctcgag ggggsrggkg ggaagcgatg
780

ggagcagcct aggccacctc ctcctccgcc tcctcctcga actcgccctc ctcggccgtg
840

gcgtcctggt actgctgggt actcagatac caggtcattc atgttgctct cggcctcggt
900

gaactccatc tcgtccatgc ctcgcccgtg tamcagtgca raaggscttg cgcgggamat
960

ggcggtramt gstsgrgatg cgcttaacas cyctggatgg cgtgykttgc satgaaggts
1020

gcggcatatt mggcgggggg ggggwgtsgm cagggcgtkt tamgtttttg ggatcmctca
1080

caawasctgc tktyttyttk cactcaaaat ctbtgtccct cttmttgaat cggcccggaa
1140

<210>  145

<211>  757
<212>  DNA
<213>  Homo sapiens


<220>
<221>  misc_feature
<222>  (99)..(681)

<220>
<221>  misc_feature
<222>  (99)..(99)
<223>  n is a, c, g, or t

<220>
<221>  misc_feature
<222>  (681)..(681)
<223>  n is a, c, g, or t

<400>  145
ttacagttta actgctttga atagcatttt taatagcaaa aatgtgttta tactataata
60

gcttacaacc atcttttcaa ccatcttttt cgatacaana ataaacatat tcattttcat
120

attttacatt tctcacctct ataacatata caataaaaat cccagacatt gttggctggc
180

tgattcttgc aaagagatgc ataagtaaac tagtcaagta catcttaacc attttgggtg
240

tcatttagtc agtcagtcat gaaacagtaa tatggagaca taattttgaa aattaagcaa
300

atgaatctgt tctctagaaa tgccttttct cgagttatag tattatattt ataactatta
360

aaatttatac aaatcagtag gttttatttc cacaccttca caatcccatc tctagaagca
420

gttacgatga agccctgtgt ggtctggaat gtggcgacat cagtgatgat gtcatgatgt
480

cccacgggca gggactctgg gccccttcga ggggtgtcat cacttggtcc tactttctgc
540

ttattctgaa tttcctggac aacttcagtg ccttcaatta ttttcctgta gtaggacaca
600

gaatgggaac tagtactttc tgcaacaaca taggaccttt ctgggtaagc caagtcccaa
660

aaccttattt tcatatctga nccagccggt agtagggata gattccatct gcaggactac
720

agtagaaacc ttggacgcta tgatgagaag gctggat
757


<210>  146
<211>  562
<212>  DNA
<213>  Homo sapiens

<220>
<221>  misc_feature
<222>  (438)..(555)

<220>
<221>  misc_feature
<222>  (438)..(438)
<223>  n is a, c, g, or t

<220>
<221>  misc_feature
<222>  (470)..(470)
<223>  n is a, c, g, or t

<220>
<221>  misc_feature
<222>  (489)..(489)
<223>  n is a, c, g, or t

<220>
<221>  misc_feature
<222>  (500)..(500)
<223>  n is a, c, g, or t

<220>
<221>  misc_feature
<222>  (522)..(522)
<223>  n is a, c, g, or t

<220>
<221>  misc_feature
<222>  (528)..(528)
<223>  n is a, c, g, or t

<220>
<221>  misc_feature
<222>  (551)..(551)
<223>  n is a, c, g, or t

<220>
<221>  misc_feature
<222>  (555)..(555)
<223>  n is a, c, g, or t

<400>  146
aaattttttt taatagtcac attcagctcg cttgctcaaa ccagactccc acattgggtg
60

agcaagatga gcccatagga ttccagagtt aatacgtaac cgtatataca aacagccaaa
120

aaaccataat ggtgccacag ggatggagca gggaagggca tctctaacgt gtcctctagt
180

ctatcttcgc taaacagaac ccacgttaca catgataact agagagcaca ctgtgttgaa
240

acgaggatgc tgaccccaaa tggcacttgg cagcatgcag tttaaagcaa aagagacatc
300

ctttaataac tgtataaaat ccaggcagtt ccattaaagg ggttaagaaa accaacaaca
360

acaaaaagcg agggactgtc tgttgcactg tcaaaaaggc cttggagtta atgggaccca
420

ggattggagg actcttanct gatacagatt tcagtccatt tcattaaaan gcttggatgt
480

taagaagang acacttaacn ggttctgaag gaaacccttg anatggancg ttaaaaacgg
540

acagatgaac ncaanggatc aa
562

<210> 147
<211> 1491
<212> DNA
<213> Homo sapiens

<400> 147
atgccaggcc ccccaccagc cacgttgggc agcccccaca gctccccgtt cgggccaagg
60

tgtcggggtg cgtctcctgg cccatcaata cagattacat atttatatca atcgcgggct
120

ctgagggcgc cctcggagag cgggccccgc gcctacgaaa ccaaactggg agtggtcgcg
180

cggaaactct ggctcgggat tggctcgggg cgccggccgg tgcgggggga ttgctatcgt
240

attcagcatg ttttgcacaa gaaatgtcag ccagaagggc tatctgctcc cttcgccaaa
300

ttatcccaca acaatgtcat cgtcggagag ccccgccgcg aactcttttt tggtcgactc
360

gctcatcagc tcgggcagag gcgaggcagg cggcggtggt ggtggcgcgg ggggcggcgg
420

cggtggcggt tacacgccca cgcggggtct acctgccgcc cgccgccgac ctgccctacc
480

tggcagagct gcgggctctt ccccacgctg gggcgcaagc gcaatgaggc agcgtcgccg
540

ggcagcggtg gcggtggcgg gggtctaggt cccggggcgc acggctacgg gccctcgccc
600

atagacctgt ggctagacgc gccccggtct tgccggatgg agccgcctga cgggccgccg
660

ccgccgcccc agcagcagcc gccgcccccg ccgcaaccac cccagccagc gccgcaccgg
720

acctcgtgct ctttcgcgca gaacatcaaa gaagagagct cctactgcct ctacgactcg
780

gcggacaaat gccccaaagt ctcggccacc gcgccgaact ggctcccttc cgcgggcccg
840

ccgcccgacg gcgctgcgcc ctgggcacct ccagcggggt gcagtgcctg gctacttcgc
900

cttctcaggc ctacgcgacc gccaagggct atggcagctg gcggctgctg gcgcgcacga
960

actcggggct ggccgtcccc gcgcagcccc cggggcggcg gtttcgatct cccgcccgcg
1020

ctagcctccg gctcggctcg gatgcggccc ggaaggagcg agccctcgat tcgccgccgc
1080

cccccacgct ggcttgcggc agcggcgggg gctcgcaggg cgacgaggag ggccacgcgt
1140

cgttcctcgg ccgcggagga gctctccccg gccccttccg agagcagcaa agcctcgccg
1200

gagaaggatt ccctgggcaa ttccaaaggt gaaaacgcag ccaactggct cacggcaaag
1260

agtggtcgga agaagcgctg cccctacacg aagcaccaga cactggagct ggagaaggag
1320

tttctgttca atatgtacct tactcgagag cggcgcctag agattagccg cagcgtccac
1380

ctcacggaca gacaagtgaa aatctggttt cagaaccgca ggatgaaact gaagaaaatg
1440

aatcgagaaa accggatccg ggagctcaca gccaacttta atttttcctg a
1491

<210> 148
<211> 1491
<212> DNA
<213> Homo sapiens

<400> 148
atgccaggcc ccccaccagc cacgttgggc agcccccaca gctccccgtt cgggccaagg
60

tgtcggggtg cgtctcctgg cccatcaata cagattacat atttatatca atcgcgggct
120

ctgagggcgc cctcggagag cgggccccgc gcctacgaaa ccaaactggg agtggtcgcg
180

cggaaactct ggctcgggat tggctcgggg cgccggccgg tgcgggggga ttgctatcgt
240

attcagcatg ttttgcacaa gaaatgtcag ccagaagggc tatctgctcc cttcgccaaa
300

ttatcccaca acaatgtcat cgtcggagag ccccgccgcg aactcttttt tggtcgactc
360

gctcatcagc tcgggcagag gcgaggcagg cggcggtggt ggtggcgcgg ggggcggcgg
420

cggtggcggt tacacgccca cgcggggtct acctgccgcc cgccgccgac ctgccctacc
480

tggcagagct gcgggctctt ccccacgctg gggcgcaagc gcaatgaggc agcgtcgccg
540

ggcagcggtg gcggtggcgg gggtctaggt cccggggcgc acggctacgg gccctcgccc
600

atagacctgt ggctagacgc gccccggtct tgccggatgg agccgcctga cgggccgccg
660

ccgccgcccc agcagcagcc gccgcccccg ccgcaaccac cccagccagc gccgcaccgg

720

acctcgtgct ctttcgcgca gaacatcaaa gaagagagct cctactgcct ctacgactcg
780

gcggacaaat gccccaaagt ctcggccacc gcgccgaact ggctcccttc cgcgggcccg
840

ccgcccgacg gcgctgcgcc ctgggcacct ccagcggggt gcagtgcctg gctacttcgc
900

cttctcaggc ctacgcgacc gccaagggct atggcagctg gcggctgctg gcgcgcacga
960

actcggggct ggccgtcccc gcgcagcccc cggggcggcg gtttcgatct cccgcccgcg
1020

ctagcctccg gctcggctcg gatgcggccc ggaaggagcg agccctcgat tcgccgccgc
1080

cccccacgct ggcttgcggc agcggcgggg gctcgcaggg cgacgaggag ggccacgcgt
1140

cgttcctcgg ccgcggagga gctctccccg gccccttccg agagcagcaa agcctcgccg
1200

gagaaggatt ccctgggcaa ttccaaaggt gaaaacgcag ccaactggct cacggcaaag
1260

agtggtcgga agaagcgctg cccctacacg aagcaccaga cactggagct ggagaaggag
1320

tttctgttca atatgtacct tactcgagag cggcgcctag agattagccg cagcgtccac
1380

ctcacggaca gacaagtgaa aatctggttt cagaaccgca ggatgaaact gaagaaaatg
1440

aatcgagaaa accggatccg ggagctcaca gccaacttta atttttcctg a
1491


<210> 149
<211> 2894
<212> DNA
<213> Homo sapiens

<400> 149
ggagaccgcg tctgcttcaa cttgggccgt gagctctatt tctacccagg ctgctgtcgt
60

cgtgggagcc aacggtccat tgacctcaac aagccaattg acaagcggat ctacaagggc
120

acccagccca cctgccacga tttcaaccag ttcactgctg ccaccgagac catctcgctg
180

ctggtgggct tctcagcggg tcaagtgcag tacctggatc tcatcaaaaa ggacaccagc
240

aagctgttca atgaggagcg gttgatcgac aagaccaagg tgacatatct gaagtggctg
300

cctgagtcgg agagcctgtt cctggcatca cacgccagtg gccacctgta cctgtacaac
360

gtcagccacc cctgcgcctc ggccccgccc cagtacagcc tgctgaagca gggcgagggc
420

ttctctgtct atgctgccaa gagcaaggca ccccgcaacc cgctggccaa gtgggcggtg
480

ggtgagggtc ccctcaacga gttcgccttc tcgcccgatg gccggcacct ggcctgtgtg
540

agccaggatg cgtgcctgcg cgtcttccac ttcgactcca tgctcctgcg tgggctcatg
600

aagagctact ttgggggcct gctgtgtgtg tgctggagcc ctgacggccg ctacgtggtg
660

acgggtggcg aagatgacct ggtcaccgtg tggtccttca ccgagggccg cgtggtggct
720

cgaggccatg gccacaagtc ctgggtcaac gctgtggcct ttgactctct ctacaccaca
780

agggcagagg aggcggcgac agcagccggt gctgatgggg agcggagcgg cgaagaggag
840

gaggaggagc ccgaggctgc gggcacaggc tcggccgggg gcgccccact ctctccactg
900

cccaaggctg gctccattac ttaccgcttt ggctcggcgg gccaggacac gcagttctgc
960

ctgtgggacc tcactgaaga cgtgctctac ccgcacccgc ccctggcccg cacccgcacc
1020

ctccctggca cacctggcac cacgccaccg gccgccagca gctcgagggg tggcgagcct
1080

ggcccaggcc ccctgcctcg ctcgctgtcc cgctccaaca gtctcccgca cccagctggc
1140

gggggcaagg cgggcggccc gggtgtggcg gcagagcctg gcacaccatt cagcattggc
1200

cgcttcgcca cgctcacact gcaggagcgg cgggaccggg gggcagagaa ggagcacaag
1260

cgctaccaca gcctgggcaa catcagccgg ggtggcagtg gcggcagtgg cagtggtggg
1320

gagaagccca gcggccctgt tccccgcagc cgcctggacc ccgccaaggt gctgggcact
1380

gcgctgtgcc cgcgcatcca cgaggtgccc ctgctggagc cccttgtgtg caagaagatc
1440

gcccaggagc ggctcacagt cctcctgttc ctggaggact gcatcatcac tgcctgccag
1500

gagggcctca tctgcacctg gccgcggccg ggcaaggcgt tcacagacga ggagaccgag
1560

gcccagacag gggaaggaag ttggcccagg tcacccagca agtcagtggt agagggcatc
1620

tcctcccaac caggcaactc cccgagtggc acagtggtgt gaagccatgg atatcgggcc
1680

cccccaaccc catgccccca gcctcctagc cataaccctc cctgctgacc tcacagatca
1740

acgtattaac aagactaacc atgatggatg gactgctcca gtccccccac ctgcacaaaa
1800

tttgggggcc ccccagactg gcccggacac gggcgatgta atagcccttg tggcctcagc
1860

cttgtccccc acccactgcc aagtacaatg acctcttcct ctgaaacatc agtgttaccc
1920

tcatccctgt ccccagcatg tgactggtca ctcctgggga gagactcccc gcccctgcca
1980

caagagcccc aggtctgcag tgtgcccctc agttgagtgg gcagggcggg ggtggtccag
2040

ccctcgcccg gcccccaccc cagctgccct tgctattgtc tgtgcttttg aagagtgtta
2100

aattatggaa gcccctcagg ttcctccctg tcccgcagga cctcttattt atactaaagt
2160

tccctgtttt ctcagcgggt ctgtcccctt cggaggagat gatgtagagg acctgtgtgt
2220

gtactctgtg gttctaggca gtccgctttc cccagaggag gagtgcaggc ctgctcccag
2280

cccagcgcct cccacccctt ttcatagcag gaaaagccgg agcccaggga gggaacggac
2340

ctgcgagtca cacaactggt gacccacacc agcggctgga gcaggaccct cttggggaga
2400

agagcatcct gcccgcagcc agggcccctc atcaaagtcc tcggtgtttt ttaaattatc
2460

agaactgccc aggaccacgt ttcccaggcc ctgcccagct gggactcctc ggtccttgcc
2520

tcctagtttc tcaggcctgg ccctctcaag gcccaggcac cccaggccgg ttggaggccc
2580

cgacttccac tctggagaac cgtccaccct ggaaagaaga gctcagattc ctcttggctc
2640

tcggagccgc agggagtgtg tcttcccgcg ccaccctcca ccccccgaaa tgtttctgtt
2700

tctaatccca gcctgggcag gaatgtggct ccccgccagg ggccaaggag ctattttggg
2760

gtctcgtttg cccagggagg gcttggctcc accactttcc tcccccagcc tttgggcagc
2820

aggtcacccc tgttcaggct ctgagggtgc cccctcctgg tcctgtcctc accacccctt
2880

ccccacctcc tggg
2894

<210> 150
<211> 543

<212>  DNA
<213>  Homo sapiens

<400>  150
gaggaaaatg tctgtattaa aagccaaaaa accacataag tacaagaaga aacgcccacc
60

aggccacgtc actggataga tcaacttgga aaaggttcat aaagttcatt ttcagcggta
120

acaatatatc ggtgttaaat ccttcccatg ccctcccccc aattttaaat aaattaaaaa
180

accacaaatt tcaaatacaa ctatgttcag atacaaagag cttctcttac tacagcaata
240

gcaatttaat ctttttgttt tgtatttttt ttactaaaac tttcatttaa aacagttatt
300

aaatatataa aacaaataca caggatttgg tcattttctg ccatgaatct agggcacgtg
360

ggtgcgggga aggagtcggg cagggggatg caatagaggg gaaagggccc catttccctc
420

ctctccgtct tcggagctgc gatcccaccc tcagtccaag ggcttaaaca tctgcttttc
480

ggaactggaa gcgcagcaca aaccttgctt ttcaaaggcg ctggggtctc tcggcggccc
540

cat
543


<210>  151
<211>  658
<212>  DNA
<213>  Homo sapiens

<400>  151
ttcataattt cattgtcatt tatttcaatc ctgtggcaca cactgaatat aacaggcttg
60

aaaaacactt tgatatttcc tttgtagatt actaaatgct accattgatt tctgtaaatt
120

aaatttacta ggaaaaccca cagtttaaaa aaagtacaca agaataagct agctgatcac
180

aatgaaatga atgaattcaa aatagtataa agtcaggaac agtttatctt catggtaaac
240

attcacttta tgcaaatccc acttggcttc atttaattaa aaagctataa ggatagccgt
300

ggtacttgga acgaaatatt gaaagtagaa aactgaagaa gaagaccacc aggcccaatc
360

ccactaggca aattatccgt ctttttccaa catcgccttt cgaagttgct gcttcattta
420

gaagaaccat ccccatggtg atagcggcat tcagtaccag aataatgtgt gattctgcca
480

caaactgagc ctggctgctc ccatgaatgt agctcacttg tccattgtgt gggttcttat

540

gagcatatgg aggtccacgg atatggttcc acatctggcc agaagtcata gcaaagacta
600

tacacagaga caccatggcc caaccaagtc tgttatagat gaaactccag tggttctt
658


<210>  152
<211>  516
<212>  DNA
<213>  Homo sapiens

<400>  152
atggaacata gcacgtttct ctctggcctg gtactggcta cccttctctc gcaagtgagc
60

cccttcaaga tacctataga ggaacttgag gacagagtgt ttgtgaattg caataccagc
120

atcacatggg tagagggaac ggtgggaaca ctgctctcag acattacaag actggacctg
180

ggaaaacgca tcctggaccc acgaggaata tataggtgta atgggacaga tatatacaag
240

gacaaagaat ctaccgtgca agttcattat cgaatgtgcc agagctgtgt ggagctggat
300

ccagccaccg tggctggcat cattgtcact gatgtcattg ccactctgct ccttgctttg
360

ggagtcttct gctttgctgg acatgagact ggaaggctgt ctggggctgc cgacacacaa
420

gctctgttga ggaatgacca ggtctatcag cccctccgag atcgagatga tgctcagtac
480

agccaccttg gaggaaactg ggctcggaac aagtga
516


<210>  153
<211>  459
<212>  DNA
<213>  Homo sapiens


<220>
<221>  misc_feature
<222>  (40)..(450)

<220>
<221>  misc_feature
<222>  (46)..(46)
<223>  n is a, c, g, or t

<220>
<221>  misc_feature
<222>  (377)..(377)
<223>  n is a, c, g, or t

<220>
<221>  misc_feature
<222>  (442)..(442)

<223>  n is a, c, g, or t

<400>  153
tttttttttt tctgtaggat ttttattggt ggcacctggg gcaatnggag ggagtcctca
60

gcacaggcgc tggggtgtgg gaaatttcag aggcccctcc tgggatgtca cccttcaggt
120

cctcatgagt caatcttgag tttctccttc actttctgaa atgtctctga aaaccactcc
180

cgcatcttgg cagaaagttc actctgtttg atgcggctga tgagttcccg agccttgtcc
240

tccagtgtgt ttccaaactc cttcagctta tccaaggcac tggagacgtc tggggtcccc
300

tgggctgggg ctgggcttcc aagacgatcg acagaaccac caccaggacc gggagcgaca
360

ggaagagcct catggcnagg ccggaggggc actctctcaa tcttcctgac tctgtggggt
420

cctcagacca ccttaagtcc cnttccccac tcagaatgt
459


<210>  154
<211>  595
<212>  DNA
<213>  Homo sapiens


<220>
<221>  misc_feature
<222>  (538)..(540)

<220>
<221>  misc_feature
<222>  (538)..(538)
<223>  n is a, c, g, or t

<400>  154
tttggcgttt tattaaaaat ggaaaaagtt gttttagtga gcactcatgg tgctttctcc
60

cctcctcccc caggcagacc ccaaaagtgt cagggaaagg caggccccct ggaggggtgc
120

ggtggggctg aagcaggggc tgctatggct accagaggtg agctggtgat gtgagctgag
180

ttgtcacatg acactctcca ccacgacgac cttgctgctc tcggacactg gggtcagggt
240

ggtcaggccc ctgacatctg cgtcctgagc tcggtactcc aagtggtgga ggccccagat
300

gggctgggtc aggtgctgcc agcgctcagc catggtgccc ttggccctga ggaggcagcc
360

ctagaggtgc agcggcacgc acagcatgga ggacagggcg aaggcccagc ccatggcctc
420

accccaccac gggtacacgt aggtgttgtt gtagaccagc ggctcgtagt acacaacgtt

480

gaagatgaag atgcccatgc agaccagcgg ggtgaagaag gaccagcacc atttatcnca
540

gggcaggtct gtacccgatc atacaggcaa tgtcgtccat gaagcgggca gctcc
595

<210> 155
<211> 511
<212> DNA
<213> Homo sapiens

<220>
<221> misc_feature
<222> (178)..(180)

<220>
<221> misc_feature
<222> (178)..(178)
<223> n is a, c, g, or t

<400> 155
gcggccgctc gcaccgcgct cctgcagctc actctgagag atgtgggccc caggcagaga
60

acaccaggcg ggtgggcgtg tcgggcacac cagcagtcag gcgagagtct gggccccagg
120

aggcgactgc tggcctccca gccaggatta tagagtctcg tgggcccctg gaaggggnat
180

atcctttcat ctgagcccga ctccgggacc ccagggccca ggacagcgga agctgactcc
240

tcccgtgttc ccagatggga gggaggccgt gggaggagac ggaggtgagg gtggagtagt
300

ccctcggcag tgtggtcgag tgtgagtgtt ctgagcgggt cagtgagttg tagtctcgtg
360

tgaggatgga ggatgtgctt agcacgcggt ggggcacgtg tgggctcagg tgggtgccat
420

agcagcagca ctggtcgtgg tcatcctatg acagagattg gtgctgccgg aatagcaaag
480

ttcagccgtc cattcgccag gtgctcaccg g
511

<210> 156
<211> 1429
<212> DNA
<213> Homo sapiens

<400> 156
cagcatggct acgaaatgtg ggaattgtgg acccggctac tccacccctc tggaggccat
60

gaaaggaccc agggaagaga tcgtctacct gccctgcatt taccgaaaca caggcactga
120

ggccccagat tatctggcca ctgtggatgt tgaccccaag tctccccagt attgccaggt

180

catccaccgg ctgcccatgc ccaacctgaa ggacgagctg catcactcag gatggaacac
240

ctacagcagc tgcttcggtg atagcaccaa gtcgcgcaac aagctggtct tgcccagtct
300

catctcctct cgcatctatg tggtggacgt gggctctgag cccgggcccc aaaagctgca
360

caaggtcatt gagcccaagg acatccatgc caagtgcgaa ctggcctgtc tccacaccag
420

ccactgcctg gccagcgggg aagtgatgat cagctccctg ggggacgtca agggcaatgg
480

caaagggggt tttgtgctgc tggatgggga gacgttcgag gtgaagggga catgggagag
540

acctgggggt gctgcaccgt tgggctatga cttctggtac cagcctcgac acaatgtcat
600

gatcagcact gagtgggcag ctcccaatgt cttacgagat ggctttaacc ccgctgatgt
660

ggaggctgga ctgtacggga gccacttata tgtatgggac tggcagcgcc atgagattgt
720

gcagaccctg tctctaaaag atgggctgat acccttggag atccgcttcc tgcacaaccc
780

aagtgccacc cagggttttg taggctgtgc ctcagctcca aacatccagc gcttctacaa
840

aacgagggaa ggtacatggt cagtggagaa ggtgatccag gtgcccccca agaaagtgaa
900

gggctggctg ctgccagggg tgccaggcct gatcaccgac atcctgctct ccctggacga
960

ccgcttcctc tacttcagca actggctgca tggggacctg aggcagtatg acatctctga
1020

cccacagaga ccccgcctca caggacagct cttcctcgga ggcagcattg ttaagggagg
1080

ccctgtgcaa gtgctggagg acgaggaact aaagtcccag ccagagcccc tagtggtcaa
1140

gggaaaacgg gtggctggag gccctcagat gatccagctc agcctggatg gcaagcgcct
1200

ctacatcacc acgtcgctgt acagtgcctg ggaaaagcag ttttaccctg atctcatcag
1260

ggaaggctct gtaatgctgc aggttgatgt agacacagta aaaggagggc tgaagttgaa
1320

ccccaactgc ctggtggact tcgggaagga gccccttggc ccagccctgg ctcacgagct
1380

tcgctaccct gggggcgatt gtagctctga catctggatt tgaaggctc
1429

<210>  157
<211>  2241
<212>  DNA
<213>  Homo sapiens

<400>  157
agtagaaggc tcaaacaacc caaggttagt tggtctttgt gtgacagtgg gaagaagtgg
60

agagaacact ctgtctcccc gacttccttc ttgaccctcc ttccgtatct ttcattcttc
120

cccactggcc tttccagatg aggtccaaaa gtaagctcac tcttcaggga aaagtgatac
180

taaacccagg cgtgaggagt catccttctg ctcttacttt tccctgcatt tccttaggct
240

ggcccagact ctagattcct gggcacttac ctgtatctga gacccttaga cctcctgtct
300

cagtttctcc acctgtgaga tgggcagtca cacagatgag tgtgcatgag ggaatcagaa
360

gcccatctgg gttgactcag aacccgggct ctccccctgc tgtgggtgga aacaaaacac
420

cttttcacag aagctgttgt ccctccccca tccctgatac catcacccag aagctgggtg
480

gggaatggga agctggagga gggggttaga gttagaccaa tgcgaggagc tagtaagagc
540

tcgcctctcc gtgacttccc cccatgcaag ggtggcccaa ggcttcccct gatatccaaa
600

gcaggacagc agccccttgg tgggattcta actgctgcta ctctgtttct ttcctcactt
660

tgctttccaa ggtggtatgt gatccccagc tcaggcctgt gcagacagga aattctcccc
720

tgcagcaagt aggggaggtg ggttgtggga tgtgacctcc ttccagatat caggcagtga
780

gtgtaaacct gccacctcca gccctgatcc attctcacct agcggctaca ggaagctgtg
840

tctgttcgat ttggtgggag gagatgtgca gggagctgta tcttgtcctc cgcttgtgaa
900

aaactcaagg atgtggagaa gagtagaccg tggaaccctg ctcttctgca gccaagctga
960

ggggcaggat gcgtgtggga cagtggtaga gaagcagggg ataaactcat aggctgcaac
1020

aaaggtgact ctgtccctgg acactgcctc cgtactttct ccttgcttca ctggccacag
1080

catctccctc cagccctcgc tatgtgcctc tgccatcttc acccatcatg gagcagaggt
1140

gaggagaggc agcctgggaa tatggagacc agtgaaggac caggcctgga gagcacaggg
1200

tcctacctgg gcatccagca gaggagcccc taaaggccag gagcacccca agaggaggga
1260

gggcagccag cctccattga cggcgagcct ccagccctct cctactttga tcaccatttc
1320

tctccaggct ttctgcctcc gagatgtggc accatagtgc ggtgccctgt ggcttcaccg
1380

ccctacttcc acctccgccc agcctgtaat gtttatataa gcagcctcaa ggaccaagaa
1440

ccatctgcga aaggacacac acaggaaatt cataaaagaa atctgaatgg ataaaaccat
1500

gaaaaaaagt atgcttcatt agtaattaaa gaaaggcaaa tagagctgga agcatttttc
1560

ccttagcaaa ccataacaga aaaaaataag acccaatatt ggcaaagaga ctactgaaaa
1620

aacattccca tacattgcgt gtgggagtat acatcggtgc aggcttcctg gatgacagtt
1680

gggtgatatg tgtcatgtgg cctaaaagcc tccatgtcat ttgacctacg aattctatct
1740

ttgggaattt atcctaagaa aatacttaag gatttagtta gtgataagat gttcatccca
1800

gcattgcaat ggagaaaaat gggaagcaat ggtttggttg ggaatttatt ccttttctgc
1860

tgtaacgaaa gtttgcaata ggggattgct taagtaaatt attgtatctc catccagatg
1920

gtggagtacc gcgcagacat taaaagtcat gtaaaagaac atctgactga aagaaaaatg
1980

ctccttgaat attaaaaggt tgtaaaaata gtgcatgtta tgtgatttca attttgtttt
2040

ttaaaatatg ggtgtatgct tgtatacgta gagcagataa aaaagacgga aggcatacta
2100

aaaaatgttg agtggttatc tttgtatggt ggaacaaagt cactgtaatt ttcatctttg
2160

gtttttctgt aatttccaaa ttttccacat tttgtatttc atataataaa tataatttaa
2220

gaaaaaaaaa aaaaaaaaaa a
2241

<210> 158
<211> 1720
<212> DNA
<213> Homo sapiens

<400> 158
agaccatgga ccctggacct tgtagatgag ggacactggc ctggccctcg ggtcttcgga
60

ggacgtaggg ggctggcatg ggtgccgact ggctgcctga cttcatcatg ctccctgcac
120

ttaggctgcg tgggacaagg gctgtgttgt cacagcagga ataggttttc ctctgttggc
180

ctccctttcc tccaccctgg cctcaaatgg atgccagatg ccaaccccag ttctggccac
240

gtacagccag cgggtcagcc cagaggcagc ctcagctcca gggctaagga ctctcggctc
300

ccattttctc tgctggcgtt tctgctgtgc ccagcagtgg ctgctgggga agcagctgca
360

gcaggaggga gacggtcttg cctctcagcc cctccctgcc ccaccccagc tcctgccctg
420

gaaatctgga gccccttgga gctgagctgg acggggggcc agctgcgagc atgtgcacta
480

aacgcagccc tttccagggg aagagaacag gatggagaat ggaaggaaag ccccccaggc
540

ttcgtgaatt gcaagaaggg actcttccag gatgacacta ggaacagggc tagggcactc
600

gctcagtccc taggggcttg tttgttcttt attattgtgt ttaaatcctt atagagcaat
660

atcaggatgg tgttaatagg tctgcctcag aatgagaatc aatcctttta gaaaaccttt
720

atactaagcc tcctcttcaa aattcacagt ggcgattagc ggactggagt ctggtggcga
780

ttagcggact ggagtctggg gacatccgtg gcaaagacac cagctcaact ttagtgcttc
840

ccaactttat ttagaatgac atggggtggg tgtctggtgt gtgtgttttc cctacgcacc
900

tcccatagct attaacaact gaggaaggcc agtgcagaat atttttggag aacgattttt
960

tttttaaata atatatcatt cctatggggg gaaagccttt tttttctttt tggctgagtt
1020

attccctccc tcccctcaat accctcagta ctgactactt ccctttcttt tctcaggcct
1080

ccccccaccg acttttgagg ccagggttgg ccagatttag caaaaccaaa acagagtgct
1140

gagttaaacg caaatttcag gtaaacaaaa gataattttc tagcattaat atgccccacg
1200

caatatttgg aacacttatg tgaaaaatga tttgtttttc tgaaattcac gtttctctct
1260

gagtcctgta actgtccccg aggggattga gcagaagctc gggtatgagc cctgaggttg
1320

actgccggtt atttttctgt cctgggaaca gcctgaccca cctccctgtc tccatgtagc
1380

cagtgagggg agggggagac acagaaccaa ccacagccag gggcgtcccc atggcgactg
1440

tggcccggcc cctcctctct tgcctgactc tcctctcttg cctgactcta gacactaact
1500

tagttccagg ttcggtgccc tgttggtgct cctgtttcca atagcttagg tcccatggtg
1560

ggggaggaac ctcaggggct atgcagcccc cgccagctgc cctcgaatcc cgtccaggcc
1620

aattccagat tctaaactga tttttttcat gatattgtca aaacagtgag gaaacattaa
1680

aaaaaaagcc ctaaagcaaa aaaaaaaaaa aaaaaaaaaa
1720

<210> 159
<211> 1100
<212> DNA
<213> Homo sapiens

<400> 159
tttttcgtcc gtttggtagg taaaaccccc atcgaaaccc tcatccggga tatgttactg
60

tccggcagca gttttaactg gccgtatatg gcaattcaat aaataaataa aataagaagg
120

gggagtgaaa cagagaaaga aaaggcaaaa gactggtttg tttgcttaat ttccttctgt
180

taagaaagga tataaaagga tgttacaagt ttgctaaaag aagagagggg aagaatttaa
240

tggactgtga atttcaaaaa aaaaaaaaag actgttaaat ktkcttttac agaatgcatt
300

raaaaaaaaa aaactcctkt kttgtgtyag wtywwcttky twyttwtcat ttttgtwtaa
360

aaaggaaatt agtctttttc tttttttggt aaatttttga aaaatattgc taaaagtgca
420

tttaaggrga ttgggagaca attagcagaa tggagaaagt aagtcttttt tttttccaaa
480

ttattaattg tcctgtgtct atgtccctct agctgttctt ttttgtactt ttctggttcc
540

aaaccagttt attctgtggt tctataataa gttttgatat aatcttggct tcttaaaaac
600

tgtgtatcat taaaatatat gttctgcaag aattaaaact gagtccatga aaataccata
660

ggaagacata aaactttaaa aggcaactca aagatgatgg aaacgcactt acaagtggtg
720

accaaaattt ttaggtgaag tcgagcactc taattagaga actggaggaa ccacatataa
780

cacttaactt cccctaccct gcccctcccc aaaagaaacc atkaaaaacc tagcttttaa
840

aaaatatttt aagaaagaga atgaactgtg gaaattattg gcagcaagga atgtgtccaa

900

gamacatgct gaggktttga ataaaagtga actttttgtt awtttraatt gggyccgctt
960

akttcttgaa ttktawgaaa tccyatatyk kttkkttwtt tkcaaaccyt tktwttawaa
1020

ttkttgraat tttccytttt aaaaawamma ttraawaagm tgaaaaaaaa aatkttggmc
1080

taagggamgg gattattagt
1100

<210> 160
<211> 2052
<212> DNA
<213> Homo sapiens

<400> 160
gtattagaag gcaaagcaaa actcccaatg gataaatcag tgcctggtgt tctgaagtgg
60

gaaaaaatag actaacttca gtaggattta tgtattttga aaaagagaac agaaaacaac
120

aaaagaattt ttgtttggac tgttttcaat aacaaagcac ataactggat tttgaacgct
180

taagtcatca ttacttggga aatttttaat gtttattatt tacatcactt tgtgaattaa
240

cacagtgttt caattctgta attacatatt tgactctttc aaagaaatcc aaatttctca
300

tgttcctttt gaaattgtag tgcaaaatgg tcagtattat ctaaatgaat gagccaaaat
360

gactttgaac tgaaactttt ctaaagtgct ggaactttag tgaaacataa taataatggg
420

tttatatatg tcatagcata gatgaattta gaaacaatgc tcctactgtt taaatacata
480

tggacacatc tggtgctgag aaagaaacaa acacattacc attggtgtca agaaatatta
540

ctatatagca gagaaatggc aatacatgta ctcagatagt tacatcccta tataaaaagt
600

atgtttacat ttaaaaaatt agtagataac ttccttcttt caagtgcaca atttcatttt
660

gacttgagtc aacttttgtt ttggaacaaa ttaagtaagg gagctgccca atctgtctga
720

tatttcttga ggctgccctc tatatttatc tttcccatgg gcagagatgt tgtaagtggg
780

attcttaata tcaccattct tgggactggt atacataagg cagccgtgaa actggaaagt
840

cattttgatg actgatgtga tacatccaga ggtaaaatgc atttaaacat attaaagtat
900

ttgccaaaga tacaattttc ttgctgacat aaaaatcaca caaacaagtc ccccccaaac
960

cacaactgtc tctcaaatag cttaaagaaa ttgaaaaaca ttttaggatt tttcaagttt
1020

tctagatttt aaaaagatgt tcagctatag aggaatgtta aaaattttat attatctaga
1080

acacaggaac atcatcctgg gttattcagg aatcagtcac acatgtgtgt gtgtctgaga
1140

tatagctaaa ttagcaaagc acatagtatt acatacttga ggggttggtg aacaaaggaa
1200

aaatatactt tctgcaaaac caaggactgt tgctgcgtaa tgagacagct gtgatttcat
1260

ttgaaactgt gaaaccatgt gccataatag aattttgaga attttgcttt tacctaaatt
1320

caagaaaatg aaattacact tttaagttag tggtgcttag cataattttc ctatataaca
1380

gtataaaatc tcaagtaaaa ttttccagtg ccagaacatg ttaggtggaa ttttaaaagt
1440

gcctcggcat cctgtattac atgtcataga attgtaaagt caacatcaat tactagtaat
1500

cattctgcac tcactgggtg catagcatgg ttagaggggc tagagatgga cagtcatcaa
1560

ctggcggata tagcggtaca tatatcctta gccaccaggg cacaagctta cagtagacaa
1620

tacagacaga gcttttgttg agctgtaact gagctatgga atagcttctt tgatgtacct
1680

ctttgcctta aattgctttt tagttctaag attgtagaat gatcctttca aattgtaatc
1740

ttttctaaca gagatatttt aatatagttg ctttcttaaa aaacaaaaaa actactgtca
1800

gtattaatac tgagccagac tggcatctac agatttcaga tctatcattt tattgattct
1860

taagcttgta ttaaaaacta ggcaatatca tcatggatac ataggagaag acacatttac
1920

aatcattcat tgggcctttt atctgtctat ccatccatca tcatttgaag gcctaatata
1980

ttgccaagta ctcacatggt atgcattgag acataaaaaa gactgtctat aacctcaata
2040

agtattaaaa at
2052

<210> 161
<211> 3835
<212> DNA
<213> Homo sapiens

<400> 161
catgcgtgac tgcccccaca ctcacacagc tctcactccc cacatgctcc atgcctcctg
60

tccccactga ggagagctcc tagaggctcg cccgctcccc actgacatgc atccctgcag
120

acaaacgagg cgcccagaga gcttccccac tgcacttgcc agggctgcgg gcccagcctt
180

gcccctagct tcctctggcg ggagctatgg ctcggaggag aatggggact tctgaacata
240

cctgcccgca agggggaccg gaggtgctcg gagtgggctt gtgagggagg tggtgccgca
300

gtccccgctg agcagcctgg ccccccagat cgtgtacttc actgctacat tcccctacgt
360

ggtcgtggtc gtgctgcttg tgcttggagt gctgctgcct ggcgccctgg acagcatcat
420

ttactatctc aagcctgact ggtcaaagct ggggtcccct caggtgaggt ggaggtgggg
480

aggctgcagc agggtgttgt gggggagccc tgcaggcccc tcatgcctgc actctccagc
540

cctttctctg taggtatgga tagatgtggg gacccagatt ttcttttctt atgccattgg
600

cctgggggcc ctcacagccc tgggcagcta caaccgcttc aacaacaact gctacaagta
660

agcactgctg ccctgccacc cgtgccctgt cccgccctgc cctgcccagc agcctaaccc
720

atccactctg gcccctccac ccctccagga cgccatcatc ctggctgtca tcaacagtgg
780

gaccagcttc tttgctggct tcgtggtctt ctccatcctg ggcttcatgg ctgcagagca
840

gggcatgcac atctccaagg tggcagagtc aggtagggcc ctacccccag ccccgcctcc
900

agagcagcaa ctgccaccca gatgcatgat gtacaagaac acgcaataga aatgctgaaa
960

agtgatgagg attcaaacag aacttctcag attgtgggcc tgtgggggca ggtcctggga
1020

tttttcaatg ttgacagaga caggacctcc cagcccctgc tgcatgaccc agggttgaca
1080

gcacctcaga ggcaggcgtg ggcatgggcg tgagtgttgc aggcagggct cagggtgcgc
1140

gcagggcacg acatcggctg caaggtctag agcctgcacc tttcccacag ggccgggcct
1200

ggccttcatc gcctacccac aggctgtcac actgatgcca gtggccccac tctgggctgc
1260

cctgttcttc ttcatgctgt tgctgcttgg tctcgacaac cagtttgcat gggctctggg
1320

acagggagcc aggagagggg cggagtgagg gctgcgggca aggaaagggg tggagggtgg
1380

tgcggggctc ggcctgagct agcctggcca cagtttgtag gtgtggaggg cttcatcacc
1440

ggcctcctca acctcctccc ggcctcctac tacttctgtt tccaaaggga gatctctgtg
1500

gccctctgtt gtgccctccg ctttgtcatt gatctctcca tggtgactga tgtgagtggg
1560

gtggggggtc tgcctgtgac ctctggtggc cgtctgccat cctccctgac tgggctctgt
1620

cccccagggt gggatgtatg tcttccagct gtttgactac tactcggcca gcggcaccac
1680

cctgctctgg caggcctttt gggagtgcgt ggtggtggtc tgggtgtatg gtaggtcatg
1740

gctgagggct gggctggggc atggtgacgg ggaaggcagg tctccagctt ggccctcccg
1800

cctcgccttg ccacaggagc tgaccgcttc acggacgaca ttgcctgtat gatcgggtac
1860

cgaccttgcc cctggatgaa atggtgctgg tccttcttca ccccgctggt ttgcatggta
1920

agggctgggg gaggtggggc ggggtggggg gggcggggcg gggtgggggc cccattaagg
1980

acgggcattc tggtctgtag ggcatcttca tcttcaacgt tgtgtactac aagccgctgg
2040

tctacaacaa cacctacgtg tacccgtggt ggggtgaggc catgggctgg gccttcgtgc
2100

tgtcctccat gctgtgcatg ccactgcacc tcctgggctg cctcctcagg gccaagggca
2160

ccatggctga ggtaaggctc cctcccggcc tgccctcccc tcccctgcta tgaacattca
2220

acccagcctg cttcctagcc aaggagtggc cctgactagg gtggcaggca gcaggagctg
2280

gagagagagg cagaggaagt caccgtgggg atgagcaggt gactctgggg gcttcaacat
2340

gtcctctcct gcagtgctgg aagcacctga cccagcccat ctggggcctc caccacttgg
2400

agtaccgagc tcaggatgca gatgtcaggg gcctgaccac cctgacccca gtgtccgaga
2460

gcagcaaggt cgtcgtggtg gagagtgtca tgggacagct cagctcacat caccagctca
2520

cctctggtag ccatagcagc ccctgcttca tccccacccc acccctccag ggggcctgcc
2580

tttccctgac acttttgggg tctgcctggg agaggagggg agaaagcacc atgagtgctc
2640

actaaaacaa ctttttccat ttttaataaa acgccaaaaa tatcacaacc caccaaaaat
2700

agatgcctct ccccctccag tcctagccca gctggtccta ggccccgcct agtgccccac
2760

ccccacccac agtgctgcac tcctcctgcc cctgccacgc ccaccccctg cccacctctc
2820

caggttctgc tctgtagcac acccttgggt gacccctcac cccagaagca gcagtggcag
2880

cttgggaaat gtgaggaagg gaaggaggga gagacgggag ggaggagaga gaggagaagg
2940

gaggcagggg aggggcagca gaaccaagac aaatatttca gctgggctat acccctctcc
3000

ccatccctgt tatagaagct tagagagcca gccagcagtg gaaccttctg gttcctgcgc
3060

caatcaccac caatatcaat tgtgtgagct tgggtgcgag tgcacgcgtg cgtgagcacg
3120

tagagtatat atagatctct atctcttagc aaaggtgaat accagatgta aatggtgcct
3180

ctgggcaaag gaggcttgta ttttgcacat tttataacaa cttgagagaa tgagatttct
3240

gcttgtatat ttctaaaaag aggaaggagc cccaaaccca tcctctcctt taccactccc
3300

catttcctgt gagccctacc ttacccctct gcccctagcc taggagtgtg aatttataga
3360

tctaactttc agaggcaaaa caaaagcttc gagctgttga tgtgcagtct gttgtgtgga
3420

tgtgtgtgtg tggtccccca gacccagaat ggattggaaa agtgcatggt ggggcctcgg
3480

ggctgtcccc acgctgtccc tttgcccaca ggtctgtggg gcaacaggct gcaatattcc
3540

atcctgggtg tctgggctgc taacctggcc tgctcaggct tcccaccctg tgccctgggc
3600

tgggcacacc cccgggaagg gaccccggac acggctccca catccaggct caaggcggat
3660

gcacttcctg cacctccagt cttctgtgta gcggctttaa cccacgtatg tctgtcacgt
3720

ccagtcccga gacggctgag tgaccccaag aaaggcttcc ctgacacccg gacagaggct
3780

ggagggctgg ggctgggtga gggtggtggg cctgcgggga cattctactg tgcta
3835

<210> 162
<211> 8134
<212> DNA
<213> Homo sapiens

<220>
<221>  misc_feature
<222>  (5453)..(5869)

<220>
<221>  misc_feature
<222>  (5453)..(5453)
<223>  n is a, c, g, or t

<220>
<221>  misc_feature
<222>  (5767)..(5767)
<223>  n is a, c, g, or t

<220>
<221>  misc_feature
<222>  (5776)..(5776)
<223>  n is a, c, g, or t

<220>
<221>  misc_feature
<222>  (5835)..(5835)
<223>  n is a, c, g, or t

<220>
<221>  misc_feature
<222>  (5869)..(5869)
<223>  n is a, c, g, or t

<400>  162
   gggattccgg gccgggccgg cctgggcgca atcaatgcgg ctttgtctgg gacgcccaca
   60
   tcccagaggc cattcccggg tcggcaaatc ggagcgcggc ggggcgcgcg ggggtgagat
   120
   aagcggccat gtgatcccac ctgggctgga aggggagggg cgccaggtga ggcggcggcc
   180
   ggtggggcgc gggcggccac gcggggctcc tgcagcatgg ctgtcagcag gaaggactgg
   240
   tccgcgctgt ccaggtgagc gccggaggga gccgggagag atggaggttg ccatgttgct
   300
   caagctggtc tcaaactcct gagctcaggc aatccgcgtg tctcagcctc ctaaagtgct
   360
   aggatcatag gcatgagcga ccgcgcctgg ccgatgtaca tagttcttaa tgagaccttt
   420
   cactggtctt caagatccca gtgtgtattt cacacagcat gccttggttc acaccagcca
   480
   cgtttcaagc acttctgagc tgctagtagc cagtgcaggc ctagagggca tttggtctct
   540
   tctcccagcc ctagaggagc ctgcctcggg agggtcctgg gtttccccag ctgtgctgac
   600
   cctctcctcc cctcccccag ccttgcccgg cagaggactc tggaggatga ggaggaacag
   660
   gagcgcgagc gcaggcggcg gcaccgcaac ctgagctcca ccacggacga tgaggctccc
   720
   aggctcagcc agaatggaga ccggcaggcc tctgcttctg agaggtaggt gctcaggttt
   780
   ctatcctgat ggatggggat ggactggttg cctcagacta agtgcagcat attcagtctg
   840
   ctcaggggag ttggggccca ggaaatgagg ctgaagggtg tgatgatgaa tttccagttc
   900
   cccaccttcc ccagactcag tcctgccagc ttccatcctc ttcataacaa tgcttgctac
   960
   tatttgtaga ccatccatga gccaatactg tgctggacat tttatggcca tagctcacgt
   1020
   aatccacaca atgtccctgg agagagaggt attgttaacc cattttacag gtggagtaac
   1080
   agggttgaaa agtacttagg tgacttgctg agtcacgtac ctcaagatgg cagggccaga
   1140
   atgtccatcc agatctgtca agctcccgcc tccttccatt atccaggctg ccttcttcta
   1200
   accctgaacc tctataattt acttcgtttt tttttttttt ttttgagacg aagtctcgct
   1260
   gtgtcgctag gctggagtgc agtggcgcga tctcagctca ctgcaagctc cgcctcctag
   1320
   gttcaagcaa ttcttctgcc tcagcctcct gagtagctgg gactacaggc acccaccacc
   1380
   acagccagct aatttttgta tttttagtag aaatggggtt ttaccatgtt ggccaggatg
   1440
   gtctcgatct gttgacctca tgatccacct gccttggcct cccaaagtgc tgggaataca
   1500
   ggcatgagcc accgtgccca gctgcctttt tttttttgga gatggagtct tgctctgtca
   1560
   ccccagctgg agtgcagtgg cgcaatcttg gctcatgcaa cctccgcctc ccaggttcaa
   1620
   gtattttgcc tcagcctccc gagtagctga gattaaaggc acctgccacc atgcccggat
   1680
   aatttttgta tttttaggag agatggggtt tcaccatgtt gggcaggctg atcttgaact
   1740
   cctgacctca ggtgatccac cagcctcaac ttcccagtgc tgggattaca ggtgtgagcc
   1800
   gctgcaccgg cctataattt acttctgagg agaagaacct tggggctagt tttgccatct
   1860
   agattttggg attatgaggt gggggaagag cctggggttt actgaatgcc acctacatgc
   1920
   caggactgtc ctgggcaccc tcatgagtct cattttgctt cctaacaagc ccagtttgta
   1980
   gatgtgaaaa ctgaggctcc gacagtaagt taacttgcct gaaagcacat ggagagcagg
   2040
   gtgatgagga aggaaaataa tctccttttg ctggagccag gtgctgtggg ttatggacaa
   2100
   tcctctcatg ttacttttgt ttctttgggt cccccagaag ggagaacagg ctgtgatgac
   2160
   cctctcctct cctgtcctcc cctcccccag ccttgcccgg cagcagactc tggaggatgt
   2220
   ccagagctgg gctgcaccag tacctgtggc tgtcacctag tcttggctgc acccacaaga
   2280
   ctgacaggag tgtggaatgg tgcatcggaa gacttaggtt ctgctcccag ctcccccacg
   2340
   aatcagctgt gtgaccttga caaaatccct ggacatctct agtcttgatc taagatctaa
   2400
   ggggcagaga ctgtgcttat tccttttgat gccccaatgc cccaagcagg ctctggcaca
   2460
   tattaaaaca gttattcaga gtctgttgac tacgtctgaa catggagtcg ttggacttca
   2520
   taggaattta taagcttttt aaaaagcata gtgtgctttg ctcagatgat accttggaag
   2580
   tgcaatatat cgagtggata aaggtggtgg tgagccgggg atccagggcc tgacatcctc
   2640
   acctcctcct tggtggccct ggcagcatct ccattgggtt aggtatactt agggtatctt
   2700
   ccacctctat cctctgattc tgtctcccat cttctgattc agactaccga gcgtggaaga
   2760
   agcagaggtg cccaagccac tgcccccagc ctccaaagat gaggacgagg acatccagag
   2820
   catcctcaga acacggcagg agcggaggca gaggcggcag gtggtggagg ctgcacaggc
   2880
   ccccatccag gagaggctgg aggcagagga ggggaggaac agcttgagcc ctgtgcaggc
   2940
   cacacagaaa cccctagtct ccaagaagga actggaaatc ccacctcgcc ggagactgag
   3000
   tcgggaacag cggggcccct ggcccctgga ggaggagagc ttggtgggca gggagccaga
   3060
   agagaggaag aaaggggttc cagaaaagtc cccagtcttg gagaagtcct ccatgccaaa
   3120
   gaagacggca cctgaaaaga gcctggtctc cgataaaacc tccatctctg agaaggtgct
   3180
   ggcctcagag aagacatctc tatcagagaa gatagcagtg tcagagaaaa gaaacagctc
   3240
   agagaagaag tctgttctag aaaaaaccag tgtctctgag aagtcgctgg ccccagggat
   3300
   ggcactgggc tcaggaagga ggctggtgtc tgagaaagct tccatctttg agaaggcact
   3360
   ggcctcagag aagagcccaa ctgcagatgc taagccggcc ccaaagaggg ccacagcctc
   3420
   agagcagccc ctggcgcagg agccgccagc ctctggggga agcccagcca ccaccaagga
   3480
   gcagagagga agggccctcc ctgggaagaa cctgccctct ttggcaaagc agggggcttc
   3540
   agaccctccg actgtggcct cccgcctccc acccgtcaca ctccaggttg gcagtgtccc
   3600
   gtccctccca cccttctact ctgccacctg gctggaactg ccggccagtc ccacccaggt
   3660
   agtccctggt tccctgggtg cctccatcct cctacctgca ggctggcctt ggaagtctct
   3720
   tctttccctg cctgcttcta gccatggtca gagcagggag gctgagatgg ggcaggctgc
   3780
   tggagcttaa aggggcccca gctgtggggc agggagcatc tggtgccccc caccatatcc
   3840
   tcatccaggg cctgggctcc aggaccccgg cagactagtg gggagcctca gatccttcta
   3900
   gaagctcccc tggaaagctc tgcctggtgc cctggcccca agggactttg tctgtgagcc
   3960
   actcccacag cagatgaagg aggcaggaga gaaggcacag ggctgtctca ttctcccctc
   4020
   ttcctgcagc tcctggggtg agccatgggg tttatggctg ccccggcttg atgggcccta
   4080
   gacctcttat gtgacactcc ccctgctcca tccaggtgaa aatccccagc aaggaggaag
   4140
   aggcagatat gtcctcaccc acacagcgaa cctacagcag ctccctcaaa cgctccagcc
   4200
   ccaggaccat ctcctttcgg gtgagagccc tgggcaccct ggtctgcgag ccactggagg
   4260
   gcaagtggag gaagcccgca tccattacaa cataggagcc gtaggttagg taggaaggtc
   4320
   ccctggatag agaggagctt ttgattccca tgggtggccc tgaacaaaca aggttgtatt
   4380
   cagcagagga gaacaggtta gtaacctggg gggtaggtgc cagtaagcat gagcagcccc
   4440
   acctagtggg caaggcaggg tgtatgaaga ccggacaggc agggctggcc ctggtttgca
   4500
   gggaggtcca cacctgcttc atcagttcct ggctcaccta ggagattgtc taggagggga
   4560
   ggggaggaac agagagcagc aaggctctcc tactgctccc tgccctgcca ctctcccaat
   4620
   agtacaaact atgtggattg gggaaatctc gtcttgcttt gtcctggtgt ttattcttgg
   4680
   ttcttctgtt cactgggtca ttcactcaat cattgggtat catgcctcta atgtgtgcca
   4740
   ggccctggag aggtggccca atagcgactg gtgcccccac agagctcatg ggtagtggga
   4800
   ggcagatagg aaacaggtgg ttacaggtgc cctgcgaagg aagtctgaag aaagtcttct
   4860
   gacccatatt agtgggtggg aacctgtgtc ttccagatac aaacaaggat ctttaacgaa
   4920
   ggcttgtcca catatttctt tggtggggtt gacccctggt agaaggggtg ggaggtctga
   4980
   agaggaggag gacatgtctt gagagctggg ctcctcactc gagggggcca ccgatttccc
   5040
   ggggactccc acacaacaat gcctttcctt ctcatttcag atgaaaccca agaaagaaaa
   5100
   ctcggaaaca accctaactc gcaggtgggg ggcctggagg ctctggttct ttctatttgg
   5160
   ccatgtccag gctattttct gagtacctgc acccgccctc ctgtgccagg ctgtggggta
   5220
   gggcacatga ggggaaggtg cagtccttgc ccagggctcc agatctggca gggggatggg
   5280
   attaacagga tgaactcagg cgggataaga tggaacaagg atggggaagg atcagggaga
   5340
   gctgctcaga ggaggcaggg cttgggtggg gctccaaagg gggatgaggg tttagccttg
   5400
   aggttaggac agggcaaagg gggctttctg ggagggagac cacgaacagt gtnagggagg
   5460
   ggaagcccct gctccagcag gcctggttgg ggggctatgc cagactgtgg gggagggaag
   5520
   gggaggccca gatcactaga gccttgtatg ctgtgccaag gcctatgggg tctgagctgg
   5580
   aaggacagga tcagatttga ggtaggagca gccatgtgga gagctcacgg tggcaggctg
   5640
   ggagagcagg taagtggcga ttgctgtgat ggggaagacg gaaaggaagg acctgctttg
   5700
   ggaggtgttt tgcagtggaa atgacaaggt atgggggaga agggatgtcc tattgaatgg
   5760
   gacgttnctt ggaagnggag ggaaatccct ggcgtctgta agttcctcat gttggccaag
   5820
   tcaacacagc ctgcngtgtg atggagggcc acattctctt ggccacaang agaacgtgtg
   5880
   atggggctgc tcctcaggcc ctgggggcag gcctggtctg ccctgggtgt ctcacttggc
   5940
   tcgcctgttc catcttccat cctgcagtgc cagcatgaag ctcccagaca acacagtgaa
   6000
   gttgggagag aagctggaga gataccacac ggccatacgg gtgggtgggg gcttctcctg
   6060
   aggggcggag ggtgcaggat gagtagtggg catggccctt tcccctcccc agcatgggtc
   6120
   agctgttggt gttcttggtt cccagagatc agaatctgtc aagtctcggg gtctgccttg
   6180
   cactgagtta ttcgtggctc ctgtgggtgt agccagcaag cgccacctct ttgagaagga
   6240
   actggcgggc cagagccgag cagaaccagc ctccagccgg aaggtgaggg gctcaggccc
   6300
   cacagggtcc ttagggtgat tagccctgac gtctccaaaa cccaaatctt ggctgtggca
   6360
   atgctagaaa actgtcctca tgtgtcaatt aatgttggca gaactccatt tgagttccca
   6420
   ttcacgttcc tgagcccatc tccttagctg ggcacacagc tccctggggg aaggggaggg
   6480
   ggctgtccca ggtatatgtg gcatgtgctg gatgcatagg ggagtgaggg agagtcctat
   6540
   cccattccag ggcctctgaa catgcctcgc tgctgcaagg gcctaaaccc tgagatggaa
   6600
   agttctcttt ttaggagaac ttgaggctct caggggttgt gacatcaagg ctcaacctgt
   6660
   ggatcagcag gacccaggaa tctggagatc aggaccccca ggtatcctca ccctccctga
   6720
   tgtctcttac tgtggagcct ttgtggtggg atgactgggg acagaggagg gacagtcggt
   6780
   agaggaggag tcatttcggg agaggaggag tcatttcggg agaggaggag tcatttcagg
   6840
   agtggaggca gtcgggaata gatgggtgtg tgggtcagac tctggcagag aggaggggtg
   6900
   gtctggttcc ttttctcttt cgggaagaga aaggacatcc tgggctggga gctgggcctg
   6960
   gggtatcccg agccctttgg agcctggtct tagactctgg cctccaccct gataccctca
   7020
   atcaggaggc acagaaagca tcatctgcaa ccgagaggac tcagtgggga cagaaatctg
   7080
   actcctcgct ggacgctgag gtgaatggtg gggaggagcc tggccctacg gggaggcccc
   7140
   cggtgtactg tgggagaccc catcgtcgcc gtgtgcacac actaattggc ctgcaatcgc
   7200
   gcagtccacc cagaacgccc ccatgccctg ctcatgcaga ttggtctgag ccaaccgtga
   7260
   gaacaaggtt gagccctcac ctggttgtgc tgcctgcagc tctgcagagc ccctccctct
   7320
   tcagctggca gattacaggg ctctgactcc ttgtgggtgt ggggcttggg caaagggatg
   7380
   ccttcctatc cagtggcccc cttccccttc catgccctga caccccattg acttgtgcta
   7440
   acagctcttt tccccacagg tgtgacaagc cccgccaaga cagacctgca agtcttcgtc
   7500
   tcaagggacc tccctcatgc caggcccctg cctctcacag cagcaccctt tcctctcatt
   7560
   gtccctgttc ccttgttggc tgtggatctg tttggccagg gtccctgggg tcaggaatat
   7620
   ttgcaagact cagccagctc cttcccagcc cagcctcttg gggctgggac tttctcaccc
   7680
   tgcggcaggc acaacagatg ctgggaccca gtctctgccc aggtcacagc acaagtgcac
   7740
   atcagcacta tggggcctat gtcctgccca gagacctctg ctccttcctg ctcacatcca
   7800
   cagtcagggc acggcgcccc tcaagaactc cagagtcacc tgtctcatcg gctcccaaca
   7860
   agtgcctctt tgtctatgat gtcccccttc tctgaggcct ggacccaccc atctttgtcc
   7920
   ctgggggctg ctcccagcca ctgaggcccg ctctggccag gggagaagga gctgccgtgc
   7980
   gtcttccctg tgccccgtct ccctgcttgg ttctcccctc ccttccctgg ccggctgcca
   8040
   tggccaggag ctaagtgcct ttttgtgtgc aaccacttac cctttctctg aaaaacctgt
   8100
   tctcaggaag gatctgataa actcatttac tctc
   8134

<210>  163
<211>  133457
<212>  DNA
<213>  Homo sapiens

<400>  163
gatccaaagt agctcttgag gggtgtcatg ctagctgggg ctacagcaat gtttcctcaa
60

gaagcctggg acttccgcag gagaacatgc cttgggttgt gaggctctgg ggtggtgctg
120

agaggccttc tagaggccgg gtgcagaact cctctcgccc ccatgccagt tcatacatct
180

gcacgtggac atgtgtgaca tacatgccac agctccagag ctgggaaaag tgttacaagt
240

gggagacagt ttcaggtctt acaggaaagg ccgtgcagct atgaaaattc accttgcagc
300

caggaagaat ggaacacact catctctaga gagaagcttt gtggagccag gagaaaatga
360

tgacctacag ggctcagccc attggcccag gccctgtggg aaggctttgc cattacaggg
420

cgggtgcggg tgggtctgat gctggtttct ccttttcagc cttatcaatg ggaccccagg
480

actcctctag ggatgcttga tcctttaatg ctgctttgct ggaacaaggg agaggtatag
540

tagggagggg acagaggagg aaaggaaggc tggtgcgtgc ctgtgatccc ctcttggaaa
600

aacagccctg ggtcctgtgg ccagtgcctc ccctgccccc aagcagctgt gatttagtgg
660

aggggccttg aagcaacaag tcacaggctc tgtaaaagca tcagaagtgg gagaatctag
720

cacagaattg acgactgtct ggtccccacc ccactgtctg gtcttggtcc ttaggttcac
780

agtgagggtt caatgactgc agctccataa acagaaaccc agagaatctg ggtctgggct
840

tggctgtcgg cttgagcctg accctgggct gtcagctgca gttagaggaa accatctgag
900

aagaactggt taacaaaaaa atcagggaca attacaaatc aggggattct tctagttttt
960

aaatgggaag agcaaagtgc ttggctctaa gaagactctg tggacatttt acccacccgt
1020

gagctctggg cttggtcgct gtgggattcc accctcctgg gggtccggct tcatctccag
1080

tcctgacctc cttgagatct gggagacaat ggagctaacc tttatgcagc cctcacccct
1140

gcccccagtg gggttgttga ctcctctgag ggtcactttc cacctttgta agatggggat
1200

cattatggga tgtttgcatg aattaaatga gaccatgcgc ctgtcccagt gccttctggg
1260

agcacaggaa agggctcagt cgtcatccgc cctttaggcg tctgcgtgtg tcatcctcag
1320

acaccgatga ggcgaggact gttattcttc ccctcagaaa atgagaacat ttatggcttt
1380

agagaggtca gataactgaa gtcggttagt ggaagctcca agaccaaatt ccaggtctgt
1440

ctgagcgttg gaagcaccat ggagtgtctg cactggccca agggagaagg aaggtggaat
1500

agaaggaaag gaagaagtgt ctcaagggat cagctcatgc atcagaaaac acacaaacga
1560

aaagcagaat ctcgctctcc acagcggtgg aggccgcagt aaggagatgg tgtgggcacg
1620

gccagtttaa gaagctgcct tcatgggcat cccaggatgg gcccggtctc caatgccaga
1680

gctgagctgg cagctggcac gacccctccg cttctaatga aggcaggccc tgaggccgga
1740

gagagaggag cagaaaggct gcatgcaggc ttccatgctg ggctctccac cccaggccg
1800

gggagggagg gaggcacttg ctcctccaga caccgcacta aagccactgc cagcagccag
1860

ggtggcggcc actgccgaag gggctgggct gcagcacggt ggctgggaga gtgatggagt
1920

ggcctttaat tgtttgtcct ttgaaacccg ggaagtgttg gcaacaaaat gagactagag
1980

aggctgctgg cagggacatg acgggctgga gtagaagctt ctacctccta aatggttaaa
2040

tggctggtct gccaaggcca ccaagcaaag tggtacttca ccacggggca gattaaaata
2100

tgcattttct cctaattgag catagtgaaa tcagccggcg gtcctgtcac aagtgcagga
2160

atgaattgtc acagcacgac acacacgact cgacagagca tctattatat ggggaaaaga
2220

gccacataaa cttccttccc gctggaattt caactaattt gttgtccagc gggctgggat
2280

gtggaagctt ggagaaggct acgggaaatg gtcacccatc cgtccctatt tccccaacag
2340

catcagatga tccagttgtg gcatcttgtg cttataacgt accatggcaa ggtctggttg
2400

ccaaattggc aagagagcta aaggctcttg tatatgatta cccttttggg ttgtgtcaat
2460

ctgtgttttg tatccagtgt ggttgtaggg catatgcatt tgaggcaaat tctatttttc
2520

ccactgcctt ccattataaa attagaagtg tgttcttaga agaaaactga aaatctcatg
2580

acagtctacc ctattcaaga ctctgagagc cagtgagggc agagggcaag gtgaaagcct
2640

acgtgtctca cgcagatttc tgggggatgc tgacatgctc ggccatgaaa gctgccacca
2700

caagaagagg agaagctcag tcctgctcag gtgccctgtg gctggagcca tctgttatga
2760

ggcttctgtt atccactgga gcatctgttg taaggtgggc ttcctgctaa attgacatca
2820

agcctctgca acagacagaa cctgtgttct cctttttttt tttttttttt tttgagacgg
2880

agtctctctc tgtcgccagg ctggggtgca gcggtgcgat cttgactcac tacaacctcc
2940

acctcccagt tcaagcaatt ctcctgcctt agcctcctga gtagctggca ctacaggtgt
3000

gcgccaccac acccagctaa tttttgtatt tttagtagag acagggtttc accatgttgg
3060

ccaggatggt cacaatctct tgacaacatg atctgcctgc ctcagcctcc caaagtgctg
3120

ggattacagg cgtgagccac cacgcccggc ctgtgttctc catttttaat gtcacagtgc
3180

ttgacatttt tgaacacatc tataatattt ttctgttttc aatatttatt ttaaactgtt
3240

tacagacact tcaccatctt aattcccctc aatttctctc tttgaccaag gcatccattt
3300

gaatcaatat tcccatacaa cacagggtca ttgtggccca atgctgatct caaatgagta
3360

gcagatgctc acatggttgg caccctttga ggtcatagcc aaccaaaacc cacaactctt
3420

tttcacatga accactggga ttgtgtttgt gtagtttact ctatgaacct aaatgcagga
3480

ctttgcattt acttttatta gtttcctcat catggtttca gctgtgtgtc agcctattga
3540

gaacattttg aatcctgatt gtgtcatctg taaaggagct gtttctccta gataacagat
3600

gtactatgtc tacaaacttg ataaacatgt cttctctgta ctcagctaaa tcaacaataa
3660

aaatactcaa cagagccaag cacgagcccc acatagcctc tagaagcttc ttccgtttct
3720

cagaactctc ctaagttacc gagtcagcca tctgcctgaa tttttgtggg agggaccatg
3780

acaagcatat ggtgggcacc taacaaatat tagtggaata aattcaaagg aggaaatttg
3840

agttcgctta ttttttaatt aaaagatata accacagttt aggtaagact taggtgtgat
3900

tccaggtaac cacatagaaa tatgtctagg atcctgatga atgcatggca tgtaagatgt
3960

ggcccacata tggaaattca aatgcacttt tcactgaaga tgtagttgac gataatgacc
4020

tctttggttt caagaaatga ttatagatga aaatagaaga tgaattagcc ttaatgagca
4080

tgcttacttg ttattcttcc ctgctggtga cattagctgt gttctacctt tggtagcaat
4140

gcacacattg ttacatttgg ggtcctggag agtagattgc tgaaagagcc caccatgcca
4200

ctctgggtcc tcattccaga aggccactgt cagctttctg gattctgctg ccttcctctg
4260

tgaagagctg ggcacccggg ctgcctgaag ccagtgggcc aggggaagcc agcagccggg
4320

gccgcaggcc agggagtctc tccttgagca ttgcctcccc aacaggactc agtgtgaggt
4380

gctgccatgc cagtcatctc acccctgtcc actgggacat gaatcctaat gttacttgat
4440

ttctagatat attttctttg ccccccttct aatcggcagt aaatgccact ttcatactgg
4500

gagatcgatc ataataacag taataatgac agccgccatg cactggctgt ttggagtgtg
4560

tgtgtgcgtt atgtcatggc atccttgcag aaatcctgta atggaggccg tactgttatt
4620

ccccttatga acaggggaag gaagctgagg gagggggtca cttgcctaaa ccatgaagct
4680

gagaagtgcc tgctgacttc acagtgcctg ctggttgatt ttcctgccgt ctcattttac
4740

actggacatg gtttggttaa atttaattgc acaaataact tctggattct catcacatat
4800

caggcactgt gttaaatgtc acagatgttt caagtcaaaa ggcaaaactc ctacctgcat
4860

ggatcttaac aacccacgga actaggtttt gctgtggcaa caaccgcaaa ccctcaatga
4920

cttaaaatag caaaaatgtg tctctcatcc atgctacatg tgcgttatat cagctgggga
4980

ctgtgcacga ttgtcacaca aggaccaggc cgacaaaaca gccaccagtg caaacactga
5040

aggcctctgt ggagagggga agagagagat atagattgct cacttcagga gttgaacgtt
5100

catcccagaa aggacacgca ccgctcccac tcccgcacac tgtctaggac aagtccggtc
5160

ccccgacctg gaggggctta gaagtgaatc cttctatgat gaccaccata tggattagtc
5220

ccaaggctaa gacaagcaca ctagtcactg tgacataagg cagaatgcag tgcctcacaa
5280

gatatgagcc gcaggtcgaa aaggtttgca ggaatagaaa ataatttccc ttggtgagat
5340

caggagaggc tttggggaga aaggacagat tttgggagat aagtgaaatt gcagtaggca
5400

aagatgaggg tgtgaggtgg gatgagaacc tagggtcaga gactagtaag aggagcggcc
5460

caaagacagg agcggggagg tgatctggag cggagaccag gtgggcacag aaaaagaagg
5520

aggatggctg ggctgagcct gcacggagaa aactgctccc aatttcatgg tctttgggag
5580

gtgctggagg tggcagctgt gacatgatca gaagtgcact tagggagttt cttcagacag
5640

tggctcggac aggagttgga ggttgtggtt gcaggggcag gtgggaggct gtcacagtat
5700

ctcgggtggg agaggagggc ctaggttagg ggtgctgatg aggatggaaa gtaggggaag
5760

ggtctgagac atgacgtgga aagaaaaccc agagaacccc agaatcagag atgctagaat
5820

gttagataac ttaatgttat tggggcatgt gggtgaagga aagaaaatgc cccgattcca
5880

agcctacacg aagtacttgg atgacatcca tgattctctc cgagaagcca cgagtgacat
5940

ccccagcagg gtcaagccaa gtctcacttt ggtagatgac aacaggcccc tggtcccaga
6000

gatgtcctat tcgggcaaga gctttctgcc aaacctgtca aatattttcc tctccaagga
6060

gcatttgagt taattcacta tttatttact aaatgtgctg tagaatatgt gtggtaacac
6120

tgggacattg acactgacca tgaaaaattc ttttacaacc tatactttct tcaggcccct
6180

aagcataggg gcctgaagct gataaaaata tgcttcctgt atgccttgga cttagaaaca
6240

tacttatttt taaaaatgta ttcttagaga ctttaaggag ctacaacatg ttggcaagtt
6300

tattctacat gcacaagcaa gaattcaatt caggaaactt gcaataggta atagagtgtt
6360

ctgcagcgtc tgtaccatgt tatacactgg tcaccatcat gaagtccaag gtctaattaa
6420

cccttcccac atgtaactaa aatcaaaatc caccagagct aaggttattt taatttttat
6480

atggcatgca cacatcagag aagctcagca aatacctgca tctaatctgc ctgcatagca
6540

ggtgtatcta taaatgttta ggatctgctt ttttaaaaat atatatatat ttttattata
6600

ctttaagttc tagggtacat gtgcacaacg tgcaggtttg ttacaaatgt atacatgtgc
6660

catgttggtg tgctgcaccc attaactcat catttacatt aggtatatct cctaatgcta
6720

tcccttcccc ctcctcccac cccacaacag gccccagtgt gtggtgttcc ccttcccgtg
6780

tccaagtgtt ctcattgttc aattcccacc tatgagtgag aacatgcggt gtttggtttt
6840

ttgtccttgc gatagtttgc tgagaatgat ggtttccagc ttcatccatg tccctaaaaa
6900

ggacatgaac tcatcctttt ttatggctgc atagcattcc atggtgtata tgtgccacat
6960

tttcttaatc cagtctatca ttgatggaca tttgggtcgg ttccaagtct ttgctattgt
7020

gaatagtgcc gcaataaaca tacgtgtgca tgtgtcttta tagcagcatg atttatagtc
7080

ctttgggtat atatccagta atgggatggc tgggtcaaat ggtatttcta gttctagatc
7140

cctgaggaat cgccacactg tcttccacaa tggttgaact agtttacagt cccaccaaca
7200

gtgtaaaagt gttcctattt ctccacatag gatctgcttt tcattgaata taaaggttct
7260

gttagttata tgcatcagta aacatatttt aatgctttta tgttgtttat cttattgtta
7320

gatcaagaat ttcagagagt ttagtaaaca ggagaatgct agagttatgc atcagctttc
7380

tcttgggatt ttttttcttt tttacatatg gcaaacaagc atattgattc tctacctaag
7440

gaagtggttc ttggaactca agccacaggc gccatataag gtcttgctgc ttagggtgtg
7500

gtccatgaac caacagcatc agcatgcctc agagctcctt aggaatgcag aattttgggc
7560

cccacctcag acctactgcg gcagtgggtc atgacacagt ggactttggg gactctggag
7620

gaaaggctgg gaagggggtg acggataaaa gactacaaat tgggttcagt gtatactgct
7680

gggtgatggg tgcaccaaaa tctcacaatc gccactaaag aacttacgca tgtaaccaca
7740

caccacctgt tgcccaaaaa cctatagaaa taaagaaaaa agaactaatg tgttcatgag
7800

gattactaat cccaatgtca aatggaacac gttaattgca tgtgactaaa ctaatagagg
7860

acttaaaaat atagaacgaa accaagacaa gagagagttt aaagtgagag aacatttctc
7920

cagttcacaa ccaaattcag tctttgttaa gaaatcgatt tttcatcttg aagaatgaaa
7980

caattccttt agtctcccta gaactgtatc attattaatt attctttttt ggaacaagtt
8040

acagctgtct gctatccccc atggccagac agactctccc acgtctgagt gtccatgttt
8100

gtgcatcgtc ttctctccct tgtactactg tctcatacta ttccttcccg aaggcaagga
8160

ccatgccttc ttgacttctg tgtcaccaac agagctcagg ctccggtgat gggcagttca
8220

ttcattttta gaaaaaaatc acagccgaga ggagagtgac tgataaataa aggcggaaga
8280

aaatgggaca tttcatactc atatttgaaa aaacaaaaaa caagatctgc ctgcctgcag
8340

gtaatttgtg tgtacgctga agtctgggag acattgacaa aagggaccca tacagaaagt
8400

gcactaaaga aagttacgtg acaatatgac ctgcaacaag ccagaagctc agctgaactt
8460

atgggtctct tgaaattcaa gagctttgca ctgcctcata tcattattgc tttgctctct
8520

ttccacctgg catttctttg acaaagttgt cactgtctga tatctttttt cctgcctatc
8580

aggaagatgc gattcttctc ggaagattga cataattttt tctatgcatg cccattttgt
8640

ctctttagtt tccttggaag atcttggaaa gcaaggaccc ttcagtcttt ctagactagt
8700

tttctaaact agtagaccat tctagtgccc catttagaat cttctacttt taaatggtta
8760

gagatgatcc agtttgcata gcattcctca gacttttgaa attcatgact tattttgaaa
8820

aaaaaaaaaa gatctcatat cctattgttc agtgaaaaaa agcaaaatcc aaaacagtat
8880

ttatagcatg ctactcttca tgtaataaag aaggggaaat aagaaaatac ccagctatct
8940

gctcccttgt ataaagaaat ataagtggct gggcgcaggg ttcatgcctg aaatctcaac
9000

agttttggag gccgaggtgg gcggatcact tgagtccagg agttcaggac cagcctgggc
9060

aacatagtga gacccagtct attaaaaaaa aaaatttagc tgggcatggg catggtggtg
9120

catgcctgta gtcccagcta ctcgggaggc tgaggtagga ggatcacttg agcccaggag
9180

gttgaggctg cagcaagcca agatcccatc attgcattcc agcctggtga cagagtgaga
9240

ctccacctca aaaaacaaaa aaaaaaaaaa aaaaaaaaaa gagaaaagaa aggaaaaaca
9300

aatacaggaa cataaaccag aaactaaaga tgttggctac ctacaggggg tgagtgggaa
9360

aggggagggt tgaggaggga aagatccttc tctgtataca gctttttgta cagatctgac
9420

tctttgacca tagtaattat ttacataaca cccaaactaa acaaataatt aaaagcaact
9480

gggatgtgga gggaacccaa aatggaatat aataaacact aataaaggag tctaactcta
9540

ctgcaaataa ataaggtaac cacattcaag gaacagggaa gaaaataact aatctaaacc
9600

actgtggaaa acagtattcc gctggatgct gaaaagccaa gggcatggaa ctgtgcataa
9660

acgctgtgat cagttagtaa aagcatttct catggcagta tgagctagcc gttcaaaact
9720

actttatata gatacagttg acccttgaac aatgcaggtt tgaactgcga gggtccactt
9780

atacatgggc ttttttcaac caggcgtggt ttacggtatt cgagggattt gaaacctgca
9840

gaggcggagg gcagactttt cctctactca tgttgcccag gtctgacttc ggaacttgag
9900

tatgcacgaa ctttggcatg ggtgaggggg tcctggaacc aaacccctgt gtataccaag
9960

ggacaacggt agtggaattg aacaaataag taaatatatt gtatatgatg agagccaggg
10020

ttttcactgt tggagaaaga tgtcacaaat aagagaagaa acaagtataa aatgatttcc
10080

tgtggtatag cttagaatca tgggtatcac tatgaactca agatctttaa agagggcagg
10140

agagaagaag acagaggtag atgggtatgt ttgtgcagct tagcatgcac acatatatac
10200

ctagccctct gttttgaggg cttaaaagca ataccattcc aataacaatg gacccatcta
10260

caacatagat cttggtctct ccacaccatt ctcctgtaaa aggaaccagg aatcgtgaag
10320

aagtggctga ttcccaagcc aggacatgga aaacacaagg tcagtctgaa acatcttgtg
10380

gtgccagaaa ataagaaaat gctcaaaata aagataagag tctgtcaaaa gagctcggga
10440

accaatgtga aagagcgaag agctgagacg atctgagcag caagataaag atagcgttac
10500

attttaagct agactaaaaa catccacaaa tacaataatt aaacatccac actgatatga
10560

ataaacaaac aaaaaccaca taaataatga aggagaaaag acagcgcttc tgcacagtgg
10620

aacgccaatg aatacatgta gaagggaaga ggacaggact ccacagagtg agcactgcag
10680

acaagatctg ctgagggatg ctagcactgg tgagtgaaag ctggaggaca aacaggccat
10740

gcctagtctc aaagcgtcag cctagatact tatccactgc aaagggaaag ataataactt
10800

gacagtgaga aacccagcag acaccacctt aatcaagtga ccaagatcaa tgtcaccagt
10860

tataagatgt ggctctcatg aaccccttga atgatgcact gaggaagatg tgccatgatt
10920

tttgtgtatt ctcaccaaaa aggcataacc ttaccccact catgagaatg cacaaacaaa
10980

cccaaactga gggatattct cctgtggaac cgatccattt tcttccaaag tgtcagggtc
11040

atgaaagtaa aggagagacc atggaaccgt tacccagtgg aggagaagaa ggagaaatac
11100

agctaaatgc aatttaggat cttagattag atcctgagca gaaaaaaagg acattagtgg
11160

aaaaaaattg tgagtttcaa ataggactgt agttcagtta gttacattgt gccgatgtta
11220

atttccagtt cttaatagtt gtactatggt tatataagat ggtgatatca ggggaaatga
11280

tagaagagat ataaggaaag tatatattac ttttgccact tttctgtaag tctaaaatta
11340

gttcaaaaca aaaatttctt taaaaaaatc ctatgtcctt tcttttaaca ttagatgaga
11400

tatgttgatt gaactaaata aaaaaggacc aagcatggct ttgttttcga gttatttatg
11460

tatgcagatt tcagccttct gggtgagctt tcctgccgct gagtatcaca gattgttcca
11520

acactcactt tcatcatttc cttcacgagg aaagaggccc acgaaggtta aatgacttat
11580

atgaggtcac aaagtcactt ggagacaaaa tcagcagtag gatctcggtt tccagcccag
11640

tgcaatttct gctctatccc gctgacctca ttgttgaggt aggtggtaat gtaaagggaa
11700

gagctacccc ttatgggatg tttctttcta tcagccttta gagggtcagg tttatttgct
11760

ggtggtagac tgacagccca actacttgat agaaacccta cgttgatgtc agaatgctgg
11820

acaccgcaaa gctctgggaa gcatcttata acaacggctc aaccatttcc tcttcaaatg
11880

tactgcttaa tcacataagt tcctgatacg gtttggctgt gtccgcactc aaatctcatg
11940

ctgaattgca gttcccatta tccctatgtg tcctgggaag gacccggagg gaggtagttg
12000

aatcaagggg gcggttaccc tcatgcggtt ctcgtgatag caagtgagtt ctcacgagat
12060

ctgatggttt tatagaggct ttcccctcct tccctctgca cttctcgctg ctgccacgtc
12120

gcaaaggaag tgttttcttc tccttccgcc atgattataa atttcctgag gcctccccag
12180

ccctgcggaa ctgtgagtca attaaatctt tttctttata aattacccaa tcgtaggtat
12240

gtccttatag cagtgtgaga accgactaat acagttcctt aggaagcccc tgctgggtat
12300

ggatggggag tgaagcaagg tgggcccaga agctgccgct ggctgagaga ccatgaagaa
12360

agtgtataga atctccacca tcaccagagg aagagagctt ggcaggaaag ttacttccat
12420

gaatggatga gttgaagtga acagcgtctt gggcaaaact gaagggtagt tagtcgggtt
12480

tgtctctggt gggctttcag ctccactccc tgcatcccat tcccacatcg tgatggtccc
12540

ctcataggcc cagggctacc accttggagc ataggtgggg atcctccagc cagggtcctt
12600

ccttcacagg gagtcagagc cctctgctcc ggaattgccc tgccccaggt cggccatcta
12660

tcaaaagccc cactatcccc taaagaccca ccctcaagca gcagatgtgc agcaagggag
12720

ccgcaaatgt cattcctgtg accccgaagc agtctagtgt gttacctctc tcagggctgc
12780

tatgggctga attgcttttc cccaaaattt ctatgctgct tccctaaccc ccagcgcctc
12840

agaatctaac tctggatagg acctgtgaat aagtgattaa gttaaaatgg ggtcctgtgg
12900

atgggcccta acccaatctg actggtgtcc ttttaagaag aggaagagac accagagatg
12960

tgcacactca gagacaagac acatgaggac acagagacaa ggcggctacc tgcaagccag
13020

ggagaggggc ctcggggaag ccaaccctgc ctacgccttg atcttggact tccagcctcc
13080

agaactgtaa gacaataaat gcctgttgtt taaggccctg gtccgtggta ttttgttatg
13140

gcagccctag caaatggata caggggtatt tgaaatgtaa ttgagagagt tcctaggaaa
13200

tggaatcact gccttgattt agagaaatta atggaggagt tggcagtgtc atgacacatg
13260

gtgggaaagg tgtttgaggg ccactgctcc tcaggtcaca ctgtggaggt ggtctgctct
13320

gcctgtgcga gccagggctg gctgacacaa acacacatgt gcacatacat cccccccac
13380

acacaccaca aacacagaca cacacagaca taagcacaca cccacacaca cccaccacaa
13440

acagacacat gcgcacatca tacacaaaca cagacacaca cagaccacac acgtcataca
13500

cagacacacc acatgcacat atcatacaca aacacacaca cctaccacac acgcttcata
13560

cacaagcaca cacacacctg ccacacatac gtacacacat acacaccaca catacacata
13620

cacacatata cacacactac acacatacac atacacacat atacacacac cacacacata
13680

tacacacacc acaaatacac accacacaca agtatacacc acacacatac atccacacac
13740

atacatatca cacacacaac acatacatat cacacatagg cacccaccac acacatcgca
13800

caaaacatac acacacatag aagcatattc acaccccaca cacaccacac acatacacac
13860

cacattgcat gcatatacac acaccacact catacaccac acacacatac atattcacat
13920

acacacacac acacgtgtgt gttcgcgctt ggagtgagtc cttggcgtcg tgaggcacgc
13980

ttcccaagac cttcattaat tccccaaatc gcaatagtaa cagtggaaac atggcttttg
14040

agggccgtct acagacccat ccacagcagg cacacgggct gcccagtggc ctgccggcac
14100

cacaggtcac atgcacatag tgctctgcac gccatagctt gtttcagcct cccaagagtg
14160

ttaggaaggc attgttttcc actttaaaaa aggacccatg ctgtgacatt tgaggtcacc
14220

ccaaatagtc aaggccacca cgaatgctac atctacaagt tccatgtatg caatgtacat
14280

tagatatatt ttctgttccc ggtcctgtca tctccccact ctcctgggct ggttaagata
14340

gctgcctcct gcccctctct caccgctccg cactgtccca tccagtaccc agttgttccc
14400

taagtactgt tgattctcat ttgtgttgtt tccacagcca gaatctgagt gaaatgggta
14460

taaatcatga gctaggatac agttcaatta ttcctatgat gaaaaggtaa gttattttct
14520

ggacagtttt tcatgtagat actattaagg agaagaaata tctcaaacat gttttttcat
14580

gcaattttag gaaattcatg aacccaggtg agttcattct aggtgcacaa actccagatt
14640

aggaacccct ggtatcctgt gctttataaa aattattttt aattgtggta aaatacacat
14700

aacatcaagt ttaccatctt aaccactgtt aagtacagtc agcagcatta ggtacattca
14760

tattgttgtg caaccatcac caccatccat ccccagaaca cttctaacat gaaaaactga
14820

aactccgtcc ccattaaaca actccccatt cctccctccc acagcccctg ccaatcacta
14880

ttctactttc tgtcttcatg aatttaacta ctgcaggatg tcgtgtacat ggagtcatgc
14940

agtatttgtc cttttgtgag tggcttattt cacttagtat aatgtcttca aggtcatcca
15000

tggtgtagca tgggttcaaa ttccctcctt ctgaaggctg aatcatattc cattgtaggt
15060

acagtcagcc tctggatatc tgtgggttct gtatccatgg attcaaccaa atgcagatca
15120

agaatattaa aaaggccggg cacagtggct cacgcctgta agcccagcac tttggaaggc
15180

tgaggcgagt ggatcacttg aggtcaggag actagcctgg tcaacatggt gaaaccccat
15240

ctctactaaa aatacaaaaa ttagccagtc gtggtggtgc acacctgtac ttccacctac
15300

tcgggaggct gaggcaggag aattgcttga atccggcagg cagaggttgc agtgagctga
15360

gattgtgcca ctgcactcca gcctgggtga cacagcaaga ctctgtctca aaaaaatata
15420

tataaaatat atatataaaa tatatatata taaaatatat atataaaata tatatatata
15480

aaatatatat ataatatata tatataaaat atatatatat aatatatata tataaaatat
15540

atatatttac atatatatac acacatataa attgcatctg tgctgaacac gtgcagactt
15600

ttttcttatt attccctaaa caataccata taacaattgt ttacatagca tttacattgt
15660

atgaggcatt ataagtaata tagaaatgat ttaaagtatt tgggaggatg tacataggtt
15720

acatgcaaat ggtacatttc atatccacag attttggtgt ttgtgggaat tcttggaacc
15780

aattccctgt gggtaccgag gagcacctgt acatgtcaca tgttgcgtat tcattcatcg
15840

gtgggtggac actgcagcag tctccatccc ttgcctactg tgaataacgc tggtatggac
15900

atgtgctatc ctgcttttat atttacaaac tcttcactaa attatcagat tctcacaatg
15960

actttgtgaa gtacgtatca tgattacttt ctcatttttc agatgaggac actgtgactt
16020

tatatggcca tgtttccatt gttaatattg caatttgggg aattaatgaa gggtttgggg
16080

agcatgcctc acaatgccag ggattcactc caagtgtggg cacacatatg tgtgtatacg
16140

tatgtgtatg tgtgtgtgcg gtatatgtgt gtatgtgagt gcaatgtgtg atgcgtatgt
16200

gagtgtatgt ctgtgtgtgt gtgtgtgtgt gtgtgttgtg tgtgtggtgg atgtgtgcgg
16260

tggatgatga gtgtgtggtg tgtgtgatgc atatgtgtgt gtggtgtgag tgtatgtgtg
16320

tggtgtgtgt gtggtgtgtg agtgtatgtg tgtggtgtgt gtgtggtgta tatgaatgtg
16380

catgatgtgt atgtgtgtgt gcatgtgatg tgtgagtatg tgttttgtgt gtggtgtgtg
16440

cagtgtgtgt gtgatgtgtg agtgtatgtg tgtgctgtgt gtgtgtgtgg tgtgtgtgtg
16500

atgtgtatgt gagtgtgtga tgtgtgtgaa tgtgggttgt atgcatgtgt gtgatgtgta
16560

tgagtgtatg tgtatatata ctgtgtgtat atatagctag gtatagacat aggtatagaa
16620

atattaatag aatacatagt tttgtccatg gttcctggcc cataactccc atagcccttt
16680

tgttaccatg ttgggtgctt taggcctcag aaaacagaac ccttctctct gaccttctcc
16740

tgccctcctt tttctcccca aggcaggaat attcccccac ctttctttct tggagctgac
16800

cctaaagaaa atttctgacc taccttgcct gaccgtaggt cataagaccc ccatttcaga
16860

aggggtcccg ccccatccct ggggaaggaa agccacacag acaggccaag aaggatctgg
16920

acagacaggc cctgctgggt tccccactcc gtctgttcct gctggatggt accctttgtg
16980

tccaatcacc tttccacatg gttgtccgtg cctccatcat gcctgtccag tgatgtctcc
17040

gtaaaggcca aacaggatgg gagaggatga gcttcgggat ggctgagcat gtggagatgt
17100

ggagggtggc gcgccaggga ggactgggag ctctgcgccc ctcacgtgcc ttctcctatg
17160

cgtcttcacc tgtatctttc ctactgttct atatgataaa ccggtcaata taagtgtctc
17220

cctgagttcc atgagccact ctagcaaatt aatcaaaccc aaggagggga tcatgggatc
17280

cccaacttac ggccggtcag tcagaagcac aggtgagaac ctggggcctg gggatcagca
17340

ccgggagtga ggtgagtctt gtgggactga gcccttggtc tcagtggcgg cggggggggg
17400

gggggggtct tctgtggtct ccaggtgtgt cgcgccagaa ctgatttgga ggcccctcag
17460

ctggtgtctg ctgcagaact gactgcttgt tggtggggag aaatccccac gtttggtcga
17520

gaagtcttct gtgtggatcg ttgtggtgtg agggaagagg aaaaatcatt gtgtttttc
17580

ctactcagac acagaagctc gaagttcaca gttactgtat gatcaagcag ggtttgaatg
17640

caggttttcc caaatcaaga gtacttgaat ttcagctccg ttggttaaac atggggacca
17700

cacatatcct tcagttgttt ttgatgggga aaggaatact gtttagaaaa gtatcctccc
17760

caggacttca ctgaataatg gaaggtgggg atgacaccct tggtttccag aataatctga
17820

aatattctca ggggtcctga gtgaatacaa cgctaatccc catgaaatct ttccattctg
17880

ggcgctccta aggcctgggg taagaaggta aggaggggat gcatccccca agctcacaat
17940

gatggggagg cacagaatcc catgtctgtc cccttagaca gcctccttcc cctagagaaa
18000

agggggcttg atgcctacag aacccaggct ggatgggagg tgggggtgtg aagaggcagc
18060

ctgggcacta attcctggga gaactggtac ccacaactgc tctactaagc ctcgcctgca
18120

agaagagctg tccctgagcc ctgttgaaag gctccaaaac attaactgcc tgtaattttg
18180

acatgtcaga tatttaataa ggaacaaatt agcacttggg tctttttttt tctgagacaa
18240

tccaatttct atgccaggca ccaacttcta tatttcaaaa tgcatttttg aaggttaatt
18300

agagaagcag cagagatagt gttttccaag agggattttt ctagttcctt ccaggtctat
18360

ggatggcaga aaaaaataac tttcagacac tgtaaattga gtgcgtgaag ctgtgagaat
18420

aaacccatct cattagtcat tgtgtcaatt atagtatcta aggaacaatt ttgtagcaat
18480

agcatattta tttgatgttt cgaaagctac ctggacccac tgttaggagg tattggctct
18540

taatgctccg agtatctctt taacaaaatg gcactccata aaaatttagt gtgttcctac
18600

tgtaattaaa tgggcctagt catttttgca tttatgatat aaactataat ggctgtaaaa
18660

tcccgcaatt aactttcctt gtgccaacat ttcatacttg agaaattctc attacaaatt
18720

tcattactga ttcacttatc tgtaatttgt cctatgtaac ctaaagaata ttaacaaatt
18780

tagagtcact tttgctctat taaaaaagaa atataaaagt ctttcaccta gtgagtcaat
18840

catgatactt caaaagaccc ccagaatggg tcaagtgtga tggtgaggac gggaaggaaa
18900

agagagagca catttgattg gaaagagcaa agtggatgtg gaggaaagta agcaggtgtc
18960

attgagcagg cacagacaca cttgagaccc aggggaaagt gaactgaaca ctggagaaac
19020

gactggggaa gtaaatgaag ggccagggtt tcctatccag gggcccggag cgcttaccac
19080

ttctgctttg gttctcctct ctggtgcttc tttctctctg gggtcagggc tggctggtgt
19140

gaagcctgcc gcagtgtaac ccacccttct gccaggttgc tttggcagga attctgtcca
19200

agaaactttg ctgctaccaa tggcagtggc aagacggaag aggaattcat ctgagaaggt
19260

ccccacctaa gggatacgtg tatttcgatt ttccatctcc ctaggcacgg ttgtcttgag
19320

aggaaatttg gagaaacttt cctcctcctt taagcctcct cttcccgcac ttttaaggca
19380

gaaacgaaaa tacatccaca tttaagtgaa gtgaaagttg actgacctag tgtcaagagc
19440

cctgaattct catccttgtg ctgtaatgat cttgctggtg actgtggcaa gttttgtgac
19500

ctgtctgggc ccaatggcca gactaggaaa gggaggagtt tcactaggat gacagctgag
19560

accctatgtt cttccaagaa cagttcattt tctggtagag aaagccaaga gcttctcttt
19620

acccaacaaa tgggtggtgc ggggtgccct gacagccacc aggtacagca caatgatctc
19680

cacgagcagc agcctgcgta aatattttcc ccaataacat ctatttcagc acttctttgc
19740

cttccataac tgcatttatt ttattctaat ttgtaattaa cttttacaaa attaagttgg
19800

agatggaaaa ttactgtccc gacataaata accctgccgc tggatgtgac agtgttaacg
19860

caatcaacag gatgattcat aaagaaagaa atgttgtcat acttattact tctgtgcgct
19920

ccatctttta tagaatatta cagtgtgaat tgaaatttta tatgatttaa taagcctcaa
19980

aaagatatta tggccaagta accacataac tgtaacaaat cgtcctcctc cctcattaca
20040

gagctttctc cgcatgtggg actctcggcc tcattatgag tggcatcttt gacaatccag
20100

ggcccattaa aaacaccttg aagaaagcat tcaccatgct gtggagcttc acagtctata
20160

ccaggctgga atccttgagg gtggcattat caaatgagga aatggttcct ttcatttttc
20220

atctgaagac tcagacagca aacatgttat ctggccctat aagcaccccg atgcttctgt
20280

gaaacccaag caagttctgc atcaccacga cagccgcgga tgtcaaatca tcaaaacagc
20340

agcagaggaa ggggtttatt cctcctccaa atcttcagct ggttttatag ggttaccagt
20400

ttaaagtttc tttgttttca gagcatcaga cctcccattt gtagctgata gccatttttc
20460

aaaggtttct ggctcaccca ggaattcatt agggtggacg agaaaagaac agtggataga
20520

atagaccagc agggcaatct aagccccatt ccctgtctct ttacattggt tcttggtccc
20580

cgtgactcat ccaaactccc ttatattcct atctggaatg cgaaagctgg aaatacaagt
20640

gctctgtaat cacaggcagt ggttttcttg gagtctggtg tgatttctgt agcttgcagc
20700

aatatgtggg acacacttgc actgaagaat ttaatgactt gttttactgc tgtctgccat
20760

caatatttat ctatattaac tgaagaaagg cattactcga aatggcattt cacacctctg
20820

agcattatac agtaattcta aagtcatacg ttgtaccaaa ttctctgggc aaacacagag
20880

atacagtatg aatggaaaga atccaagttg aagtttttaa aacttgaatt gtctgcagta
20940

atctggaaaa ccaaagtcaa aatgactcga attgctatgg ctgggaatga ctggattaaa
21000

agcaatggaa acaatcgttt ctcggcttgg tttttcttga attagcagga gcacatttca
21060

agtatattta atagaaaaga aaaactgggc caggcgccgt ggctcatgcc tgtaatccca
21120

gcactttaag aagccgaggt ggatggatca cctgaagtca ggagttcaag accagcctgg
21180

ccaacatggt gaaaccccat ctctactaaa aaaaccaaaa attagtcggg tgtggtagca
21240

ggtgcctgta atgccagtta ctcaggaggc tgaggcagga aaatcgcttg aacccgatag
21300

gccgaggttg cagtgagccg agatcgtgcc actgcaactc cagcctgggt gacaagagca
21360

aaactctgtc tcaaaaaaaa aaaaaaagaa agaaagaaag aaaaactgac caggtgttaa
21420

gacaatgcag atgcatgtca tgcttctttc tgcactcagg caaggaaggc tgaattaccc
21480

tttccatctc ccctcacctg cctctgctgg ttagtttttt ctctctcagt ttcccattgt
21540

cagagagccc ttctctgacc atatttgatc acatttcttg gcttgcattt cttcagtgga
21600

cttatctgaa ggaatctttt ctgttacatg ctggtgtgtg cacttgtcat ttgcctcccc
21660

caccctcagt gaggaagctc cctgggggca gaaacaagcc tgggttcctc atgtcctgca
21720

tggagcacag gcctggtatg tggtgggggc cacttaacat ctgaagaatg aattattatc
21780

cacactggga gaacacgagc ttgaagcata gcaggccttt ctagtgtcta tacagaccac
21840

agaccacatc gttttgagca aacagcttta aaaggatcat ctgaatatct tatgcacgag
21900

ctttactcaa agaccagtgt ttttaggggt gaaagaacct gggagatcag ttgcgccaac
21960

ccttaacctt acagaggagg aaacttagtc tcacggggat caagaggctg gcccaaggtc
22020

gttctgcgct gtgcagtagc agaagtaccc ccggaactag ggctactggc agtctgatgg
22080

ctcagggcac cttctcatac cctggattct gtctccaaag acatgctctg cagacaagct
22140

cttctagcat ggcttgactt tcacattctg gtctgaagtg tttttcctat aattttccca
22200

tctttggaaa ttatgtttat gggacatctg ggcaaaagaa ttggttctca attcataaca
22260

gataaggttt acatttttta ttaatggaat tagaacttca gagaaacttt ataactgcac
22320

tactaaccaa ccatacttag attttatatt aaccctagat tactcaccta gtaactttat
22380

acacttggaa gaaacgcaac acttggaatt agaaacagtg aacagaagga cacatgttca
22440

gtctgaggcc cagtttttct gaagttgtaa aatcctgctg ttatttagtg gtttctgtga
22500

aacagaactg atgtattgca tattttcttt caaaaaataa agtggaatta atctctttca
22560

ttttaaggca tgcaagcact ccttgaatta cgtagagatg actcagagta tgaactgcca
22620

ggctaagagt cactgaatta agaagctaaa aactagtagg taagaccttg tatgtatcag
22680

cccacattca gccatgactt ttagcataac ttatagagtg atttgtatct cttaaataaa
22740

tagataggga tagattttaa aggggtcaag aaaaaaagcc tattctgaaa atattgatgc
22800

atcctgctgt ttgcagaaaa caagggaaag ggtctgtcta gtgtcctagg cattagcatt
22860

catagcaaat aaaattgtct actaaaataa agtatctttg ctctaaatgt catctgcata
22920

tcaacagttc cgaataaatt taataggaaa agtaaaaagc atggcttatt aatatggggc
22980

agaatctttg gaaaagagga aatatgtaaa gctttcaaca gccattcaac atcaaatcaa
23040

tatcccattt tcttagtgat taattcaaaa tgagcatcag ctcttaatgt tttcactgat
23100

atattagctc aatcttttat ttactctcta tacttccaat acaggataat tttagtgtca
23160

gttgatcatc ttactgatat ttcctgtact atggtcagtg gaacacacac agatggaaaa
23220

aaatggtttt aggtatctca aaatcagaat acagaattat tttgttcatt ctttcaggct
23280

aataagcgat gacaggtatt caaagaatca aactgtcagc actgtgactc ccactgttgt
23340

tggcatcata atctgtttct tagtaacact tggtctgagt agccgtaata gaattaatta
23400

tacttcattt tgctgccttt aacagtattg accaaattta agaacaaatg agaagtgctt
23460

atcaagcttg aagaaaataa tccatcagtg gcacaattta aattctctgc cgttctctta
23520

ttaatcaaag taacacttac tatcctatct ccacttccca ttaacagtca ttaagccctg
23580

ctgctgattg aagcaatttc tttagggatc tcaacctcat ttttaagaga accttcttca
23640

gtgcaaaaca atatataaat gtggtaactt aaaagagagg aaaaaggttc ttcaatcatg
23700

ttctgacctt gatatttttc aatggtgtaa gttcaagaaa gtatacaact ggattagaaa
23760

cttcaggcag tggacgggcc gcccttgcct catcacccaa aggaggcacc aatccaacag
23820

gcatggcata gggagacttg ggttaaacac cttaaccagc tcctccactc accaccgttt
23880

tagtctctca cagtattaga tgcatctcag aagcagaaca cattgggaag ggtttactaa
23940

gaatactttg tcttggctat ggctctgggt aggcacatgg tggagaggta ggagcaagag
24000

gtctgtggcc agaagaattt gaggtggcac tcaacactgc taacccatgg gagtctgaac
24060

aattgcctag tttttctctc aaatggaaat aatatcatct gctctgcttc ccttgccaaa
24120

ttattgtgga aagcacatga gaaaatgtgg agagaagtcc tttgaaattg gtaaacatta
24180

tacgagtgtt aactatttta gacctagaag tcatgggaag tgtttcattc aactctatcc
24240

ccaagtcctt gcccaatgct gatgcatatt attgttgaat taatgctaga gtagccctac
24300

ttctaaaaat gagaataaga taactcacat tattacaatg aagtaatggt cttttacaaa
24360

acacttgtac tatatgtgga gccatgaact tacaaaacaa gcatctaggg cactgggtaa
24420

aaacaacgta ttactagaat cagggaaatc tggagggcaa tgcctttggg ctaaaaagaa
24480

ttacgtgatc aacacgattt tgaaaatggt aagcttaaga ggaaaaagcc cagagaatgc
24540

tggtgattgg gcaagaaagg taaagcaatc cctgcctgcc agtttggtca tgccaggaaa
24600

gatggtacag ttgggacagt ttccatccca aagggcccac tgccttctgc cccatggaat
24660

ttaatgtcaa caccactgac agcagcttct gcagccaagc tgcgaataga caagtcacca
24720

agttgaatgc aatgtaatca tgtatgagga tgcggagtgg gcatgggaat taggctgtga
24780

gactatatct tatctgcact acaaatgtgt tgtgttaaag cagtctctcc tcatctgctt
24840

aaacccttgg aaaccagtat ttccttctgg tcattcatta taattccccg taatcccaat
24900

acttataaaa gccaaacaca acatggcact ggttataaag ttttaatcag gtaaaacact
24960

cagtattcat tctgctcaat tttatcagtg agaaaacagg ctcagagagg tccagtggcc
25020

tccctgaggt cacccagctg ggcactattg ggcatcttta gttcatttag ccttctgaga
25080

tgtaaagctc ttctctttcc ccaagaggag attgtgtata cctatggagg agacctgaag
25140

ggtgccctca ggaaagacct cagcactcct gtccccttct tatgtctgtg ctttaaatgc
25200

agaacccccc agctgggcgc ggtggctcac acctgtaatc ccagcacttt gggaggccga
25260

ggtgggcgga tcacctgaga tcgggagttc gagaccagcc tgactaacat ggagaaaccc
25320

tgtctctact aaaaatacaa aattagctgg gcgtggtggt gcatgcctgt aatcccagct
25380

actcaggagg ctgaggcagg agaatcgctt gaacctggga ggcggaggtt gcggtgagcc
25440

aagattgtgc cattgcactc cagcctgggt aacaagagaa aaactccatc tcaaaaaaat
25500

aaaataaaat aaataaatgc atagcccctt tgcaagatca gggcttcaga gactggacag
25560

tcgggataga tccattattt tcttttaatg gccaaagtcc ttggaaagat aaatcaagac
25620

ctggatctga atttgcttgg ccagcataga catgaagaca cctttcaata ggctactggg
25680

cctgatgcat aaagacaact acacaatcaa tgaagagagg gaacagcctc agattctgat
25740

gaccactgat gtttccaact gcaaaccatg ccgcactctc gagatgttcc cagaggccag
25800

atgagaacag agttcggtgg ggagcacatt ttctttcctt gaagagatga agggtaccat
25860

ccaccactta atttctgata agacattaaa tgtatgattc tcatctctaa gaaatgaaac
25920

gctgagctgg atttttggat caatggaata cctactcctg ttaaagcact taaaaaggaa
25980

aacatctaca aaatgtccca ttaggctgac aaatttatag cagtatgaaa agtgtgccag
26040

cagtttcaaa gccgaataga gcgttgtgtt taaagagtat gaatggatct gagttcagca
26100

atatttcatc tacatgaaca agtttagcta tcacatctct tcccaatatt tcccacattt
26160

acttttaact aggcatacgt atgccatgac agaatatgga catcatcttt aaaaataagt
26220

tgtaaaatga ataatattaa atggactttc acagcgtgaa gacgtggaaa tatattctca
26280

tcttgatgtg tggggctgta cagacataaa tgccattagc attaatggta attatgtgcg
26340

taaatcccca tgctctcaat gagaatatgc cccttagttt gcaagtgcaa tgactggctc
26400

atttctgagg ctagaaacag ctgatacttt accccctcta actgggagaa agatacgggg
26460

tggctcagag cataaaatct gatcacccac tttcccaaaa catgcaattc aagcacttta
26520

aaactccttc acctcagaaa cactgaagat gtaggaaaaa gcctcaaact gcggtctgag
26580

tattctggca atttatgcta tatcattcat aggttcctgt cacttgctag ggtggttaca
26640

ctacaggagc tatgtgttct cataacaaac tcattttatt atgttctgat aacgatcgag
26700

gataatattt ttcagccaac ccagaagagg ctgtttacaa tacaattttt cagaaagtaa
26760

gcaaatttag catcctgttc accagagcca atatattcta atcttattaa aacaggcagg
26820

cagatgacag accccaggaa aaggttcgtg tcttctcttt tcaattcata aattaataag
26880

gcagtgtgac ccaagtgttt ggttaggctt caccttacac ggtaggaaaa caaaaaagtc
26940

ttcttggcca aaaagtcaga aagcattaac agaaaaggtc ggctggttat aactttacaa
27000

acacttcatc agggtaagat ggagcaaagt gacatatgta attaaagtat actaaaaaaa
27060

caagtcattg tggttttcaa ttacaatggt actaagaagc aaccatatta tttctacaaa
27120

ccattttcac ttagcacagt tcagatggaa ttattgccat gaccttttaa aaatgtgaaa
27180

gagtgaatca ataccctttc agaggcaaga ctgcaggagc tgaattggcc atgcagtaat
27240

ggtgtgtatt tctgcatcag aaggcagttg aaacctaatt atcgtcaatc acatacaagg
27300

tagctccacg tactgatgag cctgtgcagg cagcagcgtg catgtgagga tatgggtgtg
27360

ggggtcttca cgatagacaa gcacgtgtgc caaagaggtc agggatcttg gatgctttgg
27420

gccagtggag tggggcttac aggtgagtta tagggactgt cactcattta caataactaa
27480

cactggcctg tcagaaacag ccacctgtaa atgatgtatg agtggctggc agcctgccat
27540

cccccaacag accttgcagc caagaatcac attttattgt cgaggtcatt ggaatgctta
27600

cttggataca tgccatatat tacttaaaaa taacaaacag ccaatgagcc acatttctta
27660

aggctcaagg gcaaaatagc caacctgctt ttagaaaata ggaaacaaac atccaagatc
27720

cattttaaca agtgattttt aaaaacttgc ttctctccag atacaaagaa gcaacactat
27780

ggagaggcct gagagtggtg aaatgatgtc agatggggag aacacatatg aatattcact
27840

tttcctaagc tgtttcagac acgatgccag caaaaaccaa caacaaaagc aaatgtcttg
27900

taatcaacaa cagcggtagc cccaactgcc agactatgaa agccatggaa accagtttgc
27960

acaggaagga aggatctctg gtttgatttt tccattcaat tgcataattc tcaaatctgc
28020

ctgagaatgg taaaatgatc ctatgtacca tggcaaatgt ttttcttgca caatcaatct
28080

ttttatatgc tatcagaagg tcatattgtt tttaatttta ttcagtagcc taaatatggg
28140

tagaagtctc aacattatgc tattacattt caacacaaga aatacagcat tatcaaatgt
28200

tatggatatt aagccacaaa gaagtgtttg gataacctct tgcaactttt cttgaaaatt
28260

aaattttaaa aaacctactc ttctataacc gtacaactgt aaatgttgtc ccctctagtc
28320

tggtgttgaa gatctctgca acaatcagcc ttcacagccc tgctccactg gcccccactt
28380

gttcccacag aaatgaaaga cttggcatat ttgttaccat tatacagtaa tcggacaatc
28440

ttttgttcaa tgctcttaat gttcttagaa taaaaacatt aaaaatgcac ttaaaaaaat
28500

aggaatactc attacacaca agcaatagtt tttaattgag aaaaacagtc taattgaaat
28560

tacatttgtt atgcagacac taccaataat tatcttggct ttaaatatgc aaacacaaag
28620

attccttttg aattgataac tcttttcaga agtggcgact actagttgaa ttcagtgatc
28680

cagggacccg ttggtgatat caggtggtcc ccaggtgtat cagatggcct cttagagctg
28740

ctgtggtaaa gcatgtggca gtcagttaca agtgatgtaa ctaaaggttg aaatcctgtg
28800

cctactattg ctcaggggtt ttgaggagat caccactgct aaaagcagtg acttacaggt
28860

aagaaggaac tcttacagga ttccataact cacttgctgg gctcaaaaac attgtgcaaa
28920

agcctctctc ttaaatagct catggccaga gacaacttta acacctcttc tgtatgataa
28980

atcatgttat tgcaatttat gtgacactag tatctactgt acagtaacca ctccaagtcc
29040

tcttaagcgg aggcttctca atatactgat ttctaatgcc ccacaagtgc cataaagtga
29100

ttgctgttga ccattgtctt gttttaatgt cccctaaata ataataaact tgaatgctaa
29160

aaaaaattat tgtcattcag gaatatattt cttgtaaacc attgtatgat gaaaaaaata
29220

aatttccctc ttggcaactt aatttgtaga taactgaggt tgtttttaga taggagtcct
29280

acactgaatt tggaatctag gtgactgttt aatgtcatta agggaatatt gtattggtta
29340

caatggctca gtaaagtaac tggtttctat gtggttatat caatctattt ctgtaacacc
29400

gtacactagt tttcccccta gagtactcct tgtaatgctg agaaagtgtc agaaataatg
29460

actgtctacc tatatctgca ataaaacgta gagcatggat attatagttg taagtatcaa
29520

agatttattt catgactgca ggtaatgatg aatcttttag cacaactaat gtctggagga
29580

aacctctctt cttctctgag ctgtcacatg gagactattt atagggccct tagtgctgtg
29640

gggttttagc ataaagaggc aatgtgtcta aggtgccact ggatggaaat gttagcaagg
29700

gcttcctctc attgaagatc ttttagtaga tggcattaga ttccctcttt gtgtgtttac
29760

gtccccccaa agacagggga cagatgttta acatcagcct attaatccct tagcccaggt
29820

tcctgtgaca gcacaaaccc aacagaatga gccctttgcc tttgctcagt gtaaatgccg
29880

caatttactg ctgatttaca gaacagggtc aggccattag aaacttggca atcctcagta
29940

cagctggaaa acaatatgca tgataagcta aaattcaata ccttcttgca ctaattaaat
30000

ggcttcccac caggacaaaa acacaaatta caatacagga cttttctgca tccctatgca
30060

tttttctaca gctgtgacca agctttattt gtatttttct tttggatatt aggttttaat
30120

tcacttccac tctttaaaga ttgtgttcat atcatagtcg ttcactgtgt cattccactg
30180

ttatactgtt tccaaacaag ttgataataa cttggtttcc tctggtatga gcagacaagt
30240

gtacaaacaa aaacacagat atgttctgaa agcctttctt gaatctccgt tatacagatg
30300

tgcagatcag ttacagctct tctcttaaaa taaaccaaga aactatcaaa aatattatac
30360

aaaccccaaa gagcttattc tggaatcaag acaatgaatc ttattgcaga aactctttca
30420

taacaacaca ggacagtaaa gagcaggtaa ctgggtttga gaatgagcac tgctttttga
30480

aagagtggaa cttacaaaga tatttcatac aaagagttaa tatgggtgaa gtacctctta
30540

ctttctatgg ttcatctacc aggatgtgtc tccggcccca gtgcaaaaaa aaaagacctt
30600

ctacaaaagc aagcacctat actcccagat tatgagtcta tgaaccccac ggctgcccga
30660

gattttcctg aagagtgaat aatatttaga tcatatgtgg gttgcctcca gatcacccac
30720

tgagaagcag aagcaagatt ttgaacagct ggaggtttaa gtaaaaaaca agaatagcta
30780

caggctttat cttggatcca aacggtctga ataataataa taatcataaa gaaatacctg
30840

gtaactataa atctcatcaa ccaatgtaaa agaacattct aaatctatta atattttcaa
30900

agacattatc tttctgccac cccactgctt tgtgacttgt tgtatcagct atattatttg
30960

ccttaaagca ccatctaagt gtttttcttc ccctgtgagg ggaatcttcc acagccttcg
31020

caagctacag cccactgctc ccctgcagga ggactggggt atcattaaca accaaagaca
31080

cttaaggcac aagttggaac tccagtgttt ccaaaccttc ctgcttccca tcaaaattca
31140

tatcctgtca ctgttctagg agcagaaagg cttctcatta tagcatcatt cccctcttgc
31200

aaaaaacact gattgctgca ctggtggctt tggaaggcaa agatgtaatt tagcaaaata
31260

atgtaccatg ctgattaatt taatgtagcc catgactaat tatggtaatt cattacatct
31320

acaattaggc taagtaattt attgcactgc agtctcataa agcagaggat ttatatcgag
31380

ttttgaatgt cacccaaagg acatttctgt accttgtctt tactgaagcg gaataaggct
31440

gccagctgag atgagccaaa gaaaaaaaaa atccccgccc cccccgcccc aacttacgca
31500

ctgacacaca ctttgccatt tttgatgttt gtattttgca attgaattca agtgcaagga
31560

gagtttttcc tgatctcctc tttctttctc ctcctcagac tgtgcctttg tcatgatttg
31620

gtgttgtttt tgtttgtatg ctgcatacca atttcctctc tgtaagctct ggagacctga
31680

cacaggaaag tgaacaatat ataagaagct attgcggtct tcccaaatat tatttgtagg
31740

catttggcgg tgatcagaga aaacgggtaa ttgtgctact gaggtctgtt gcttattctg
31800

cagatagtac tgcagtcgac ttcaacaaag acctcatctt ctctttgccg catgaaaagg
31860

ggacatatcc cctcacttag gaaagacgca aacatagcat tattttgaaa tacagcctcg
31920

cttgtaccct gcagcggggt ttgctctagc gatggggtac tttggagacc aatttgttta
31980

gctgatcact gtctctttga cgtggctgca aatgttacct ttctttttca atcaaaaaca
32040

cagcaggggg tgccatctgt ttggcacagg ccttagctca cttccccaat gcctttttgt
32100

gcagatcatt ttacgtggca atttgctaaa atctttcatt ccttggtttt actaaatata
32160

agtttccaaa atgggctgtt ttctcagaat gtcagcaaga ctcacaaatg aatgcagttt
32220

ttcctgaaca gccagtatca gatcaaatgg agcatttttc agattttttt tttaatgaac
32280

aaatacctca ctgtcatctg attgtttact taaaactgac atgtttactg aggatggtga
32340

aaacacattt atcctactga atttcacttc gttttctttg tttggcttct acagaggtgc
32400

actgtcacca acacaggcat cacaattttt aagatccttg aagagaatag gtaatgcaaa
32460

tgcatagagc aaccatatta aatagtcatt aggaaacaac agctagcaag aaagtggtta
32520

actattttcc ccagctcaga tgctgttcaa agcaatgaat acccaccccc cacccccca
32580

ccaactccaa gtaggaacag atgatcagca ccacgtgctg aagccacagg gatgcccgtg
32640

gatttccaag tgctttgtag aatacaaatg aattatcctc attcttctcc tagcagagca
32700

tataggccag acctcattag aattcaatat cactctgctt ttctaatgtt aatgtaccag
32760

ttatataaga aatacgtgtg ttggtggggc gggggcttat tccacaatta tgttcgacgt
32820

gatatcataa ttctgaaaac tggacataat ctttattaaa ggaaattatt gcattttgcc
32880

gttttatctt ttgcttctaa tgtaacttaa attcccattc taatagtgga ttataagggc
32940

cttaattctt tgtgtctctc ttaaagctgc agtgactaca atactactgg ctttatggct
33000

ttttaaaaaa tgccgtgact aaagatcaga ttgtgagggc tccatgtcca tctcactgtg
33060

aaaggaacac agcacattac attaataaac aactgtagga gttttcttca tccaggaata
33120

actcagggca ttaaggcttg aggtcttttg tctagagtgc agcaagaggt gtaatgaaaa
33180

caaaaacacc aacacataaa aatacaattt atgccctttt atcaagtgaa gaaaaagttg
33240

gttaacgttt gaaaagggag cagtaatgta aatttattga caattttatt gtaataatga
33300

gatgtttaca cagtctggac attgtggcta aatcacttct agcatgttta tttgcaggga
33360

gctgttttct gggcaatgcg gcaggttcat taagaagaca tacagtatta ggattcaaag
33420

aaacaaccag ctcccattct ccccttccca gttatatgtg tgtgtgtata gttttaaata
33480

tatatatact tataaataat gaagtaaaat acagaattcc cttcataaga aaaacaactt
33540

cccctgtggc cccaaaatag cttgaaaata aatgtcgtat tggtatctta atacatagca
33600

ttagaaagac aacctttaat cactataact agcttttaat acctaatgtg taaatggtaa
33660

tctgtttttg actaagaata aattatggca gagtttaggg cggctcttgc tttacccttt
33720

tcttcaaaaa acaaaaaaac ccatggtaat tgttttagat gagatgcttc tcttctcaac
33780

atttttttaa gagaaaaaac tcattcagag agaaatattt ctcttaagca gcctcaaacc
33840

agattaatgg attaaaatgg cacacttgaa taatgtgact aaatttatct ctatgtggct
33900

ctttcatttt ttgtgtgttt ctatactaat ctttaatatg ctgctctgat atggttttca
33960

tgtttccaaa ctgagctaaa atgaaaatga cagattttca gttatgcaaa aatagcaatg
34020

aagtgtcagt agaaatacat tgtgaacaac ataattttta ggaaagatgt gaaatgagaa
34080

taatcttcta gcatgaaacg ggtccaataa caaaatattc aggcaattat atcaattttt
34140

aaaatttcaa aactgtttaa aaattaaggc actaaatgct aagctacaat atgtttccag
34200

cctcagtata catccattaa aattaagtag tatcaataca gagtatttat tgctatagat
34260

catagcttta ggagaagcaa aggatggtat acttatagct tgaaattctc tactataatc
34320

acttaggaaa agtttagaaa acagacttat ctttaatcac agttgccaca atttagtata
34380

gtcccattgt tttcctttat caaagagtat gtaatgataa taatataaac acattataaa
34440

agtaatagtt tcccagctta aactggtaag gttaagatgg taagagaaac tttgatttta
34500

gaaaccagtg gtaacaggta cacaatgtgt ctatttttta agctgataga attgattaat
34560

ggttcaaaat aatcaaggaa tcacatcaca aagcaccgtt ttaaagagaa gtgctaagaa
34620

ttaactctat actaccatga catcatccga tgttaccaga attatgtact acatcacgct
34680

atagattaca attgctagac tagaaaatgg aatcttcagc gactctgcat aacaaatata
34740

cttaataatc ttaaataaac tcaaacttac tttgatatct tctaagtaat tcacatgaaa
34800

aatgaatttt taaaaaatca gttcatgaaa gccattatta tttgaaggaa tatgctcaat
34860

tttttagata atttaaatta tttaagagtc ctttgcatct tttattagct ttgcctaaag
34920

ttataacatt aaagcattaa ggttacatgt aacgaggttc tggtttgccc tcatttctag
34980

ccccatattt tcacaaacta caaaccaaga ttagtgctat attacaaggc taattagaga
35040

aaataaaatt gtgtcactcc tagatatgca tctgatgttt tttaactaga aagtgtgtat
35100

tcacacacaa acattcatag atagacatct ggaagaaaca gttcataaaa ggattagaca
35160

atgggcaaag gaagtcagca caaaaatttt ccttagaatg tcagtttaaa aaaatattac
35220

aacatatacc taaagactat tattagcaga ttggtggttt taaatatcat gagaaaagtt
35280

tatttagaaa ctttatagac agaagtaaca tctctgtgat taaaatcacc attaacagaa
35340

attgaccata gcttggaagt caatgacttg atcaaaaaat gcattgaaaa ttaaaggaga
35400

atcaaatctc aaactcctct aggattattt gcatcaattt ctgttataaa taataatacc
35460

aagggctcac taaaattatt tcaagtagta ttacagccaa tgctgagtat caacattttt
35520

ttcccttgct agatttcggt gtgaaggaga aattatttga tcattgtttc atttcagttc
35580

tcatatcttt aaatgcagga aagccatgtt tgacgctatt gttgattttt caagtcactt
35640

ctaaacataa tctcacttga gcctaaccac aatcctgaca gggcagagag aacaggcacc
35700

actggtattc cgcagggaag gaaacttatt ctagggcaca aggcactaga gagtgaaact
35760

agaattttgg ttgactccaa ttccagtgtt cttactacta ctcactttca gctgaatact
35820

gctggtacaa ctctgtctcc aactgaagga aaagagtcct tactcctaaa ggggtagggt
35880

cactctcagc ttccagagtt atgttaccat aactggtagc aactctgtaa aagacgttca
35940

cattattcat atacacagct ctttgcttcc cctcacactt aattcaggag ctacaagcat
36000

tttggaattt ttttaaaaat attttcatct gaaacaatac tgaaaccctt gcgtttccct
36060

ccccgtttat ttttaaaaat acatacataa ggaaagattc ccagtttgtt tcccagataa
36120

tccctaaggt tcagatatca caaaatcaga gaacctttta tgtcttagga cactgctagt
36180

ggcttaacct gctaagaact gtaccaaggt aaggttatgt ggggctgtcc ttatccttcc
36240

ctgtgtttat gccctatctt attcagacta tgaatctact tgggaaagac tatgtcaaat
36300

tctatgtttg aaatacccct tgcacaggat ggcacttaaa tgtaatttat tcaaaatgga
36360

atcatttaat tagataggaa gaaaaatagc aagccctgag acgataataa agaataaagc
36420

aaatatgctg accaactgaa gcaaagcaac agagagataa taacacacat tcacaagatt
36480

gctacatttc aggtttttca attcaggatg cttataacag gataaacaca cagttacaca
36540

acttgttttt ctgctgggtt cccagaaagc agagggcaca cttgacgttg tgaactatgt
36600

agagcgataa gaattgtaac atctcatgcc tcatatgact ccagctcctt ggaatagcct
36660

gaggaggagg aggaaagtgg cgcaaggaga tgaaagcagg gagcaacaga gactctacgg
36720

accacaagcg cttgacctgg gaattaggaa ttagtagggt atcctagatg ggacaggtta
36780

ttttacacca ctgagatcta cttgggcagc tggagatcag ggtaatgcag tcattcactc
36840

cttgtccttc agaagcatga tgttctgaat agtaggcatg gaaacatttc aaagaactct
36900

acagaatcaa ataccctttg aaaatagaac tttggtacac ccctttctct tgggctataa
36960

gacagtcagt actatagggt gtattttaga ctatgagagg gctcaatttt accacctctc
37020

tggttaaatt ccagagccat aatagtgaat gttagtcctt acttatctct taatcttttc
37080

actctcccca accctcagcc tccaatcact gaagtacaaa attatttaaa aaggacttgt
37140

tcaaaaaaca caacagaagt ctttatgagg tccagtacaa ggcactgctt gggtgtccac
37200

ggaataagac cttgtacgta cttatgtatc agatattcca ataaaaattc ttcttaaaca
37260

aattcatatc tcaacaagca aaagtcaggt cagacattgc catcactcag ttacaaaatt
37320

atacatgttt tacaatgaag tttactgatg gtacagtttg ttaacaatcc tttatgtgtt
37380

taaaccaatc aattaaaacc catctcctaa aaaacaacta tttgagacag aagcagctgg
37440

atttgcagtc ctcttcaagg ctcccctggc tggtcccttt actgggcttc acattggagg
37500

aggtgggaag gatgggaaaa ggtccttaga aaagggtcag tttggcttgg tgtcctctct
37560

ggactctgct gctttgattg acttcctatt ttcatctgga ctggaacgtc aagtgttcct
37620

gcataaaagt ttcgtctcca ttgcggatca gctgctttgg atctgatatg ataaccatgg
37680

tatgctttcc tgactagtga gccaaattca gctatccaat tgtcaattat tggatgattc
37740

ttttcacatg cttcagatta ttttctagaa atggtgacct catcagtggt caacaagcat
37800

ttgttgtgtg cctattatat gcaaagaatc caaataagca tttggtttta attcatcaca
37860

gtactgcgca gcagaacagt gttcaattca gggcaaggag gctggtgtga ggcttggtta
37920

ggtaactcac taccctaagg ctttggggac aaattattca gcctgtttcc taatcgggaa
37980

aatggtaatg aataacagaa cttacttcat agggttgtta taagaattga atgaaaagtg
38040

cacagcatga caaatagtaa acactcagta aatgttagct attactatta ctagtctgac
38100

ttaaactgtt atcatcacat ttgatgtgat aaagaacaca aggttttcta aatagactcc
38160

catgggagct gggaggggag ggtagtagat gagaatctgc ttatttgttg gaattttctc
38220

ctccacgaaa gcagctgtct ttaaccagca tacactagtg tcattaattt tctcctttga
38280

gagtacctgt gtgacacttt cctcaaacaa gaagtaaatt gcagttcaag ctacaaaatg
38340

gcacctgctt ttactctagc aggagtgata tttttatatt agaaagttga ggccaataaa
38400

cagaaaacag acaagggctg tgctatattg cctctccgag taatggataa caaaaaagaa
38460

aactgaaaag aaaaaaaaaa aaaaaaaaga ggagagaaac ctagggaaac accttccaaa
38520

gttccagcat taagaaaggt acttaagaga tgggctttgg gagcagtttc tatattcacg
38580

cctttgcaca tagtcaagtg ttttggcttg ctcaacttcc agcccctctc tcaacactga
38640

agacctaaac tgagcagtgg tatgactaag gcgactttct gcatttacag ttcctttcat
38700

tttggggact caaagcactc aagttaacat aatcatacaa ctttaaataa acagggagta
38760

aaagttaaca aaaaggacaa aagctaaatt atgattagat ttggcagcaa ctgtgaaact
38820

tcccacttac ttgcacaaat ttttgatctg cccaaggcga cagctagaat gctttcaaga
38880

tcagatgcag gaaaggcttc tctgatatta cgcaagtgga atgcagggca gcagtgattt
38940

gagatgtttc actggaaagt gaaagtagat cacttgcccc aagctgcccg aggaggcacc
39000

tgtgcctctt atttgaggtc acaacactga acacaaggga agaaaggaag gaaacatcta
39060

aggacaatgt accacaactc aactttctta ttattcacaa tctcactcaa ttctgttata
39120

acaataaaat ccaatctact tgttgatagt attatgggat atactaactg tataaaggca
39180

ttttctctac caggtttact agtcaagctc ttttcttctt aaatctcatg tcttgagata
39240

ttgtggggtg ccaaatcagt taattaacaa atatttactg gatccacacc atataatgtg
39300

taccatgctg gcaataaata atatgtttgt gtataagtaa tggtagaaca gattctacta
39360

gcagaaacaa gaaaaagccc ctcaaacata gcattaatga aatgtcattc catgctactg
39420

tgatcatatg atttttaaag tatttatctc atcataaaat agtatgctat cagaggttct
39480

aacagatatt ctggaagaac tcaactataa agcagtaggc gtataataaa ctggctaaag
39540

ctgagatgtc tagaaatctc ttgttcccaa tccagagtat aaactgttgt gcatttgagt
39600

atatattaaa cttgtataat aatattttaa caatgtagta cttattgttt ctctgtattt
39660

ttgaatacta agtatattac tataaataag atcatattca tcaaatgttt ttattttata
39720

tatatataca gtgtacacac acacacacac acacaaagta ttctttcaac ctttgagtga
39780

agctgtgttt tccatggctt gatcatgttt tgatctccac tcttcaccac ctctttcttt
39840

accatgtgga ttgctttcaa tagtcatgaa cctactgaac tcaagagaag caaaagtgat
39900

ctctatatgg tgtgtgtcta taactccaac aaatgaaata caaatgactg atttatctga
39960

aatttagaac tttaaaaatt ctcacaatgc ttcactcagt ctcactgatg aagttcaagc
40020

tcttaaacaa catgtatatt agggttaatt ttcttctaat ggctgaactg caaatctgaa
40080

ttcataatat ctaactcagt tctattttaa attatgaccc tggatagtac cagtctatga
40140

ataagtagca agtagtagag tgcagaaatt acatgaaatt atataaaaag tagaaatcat
40200

aagaactaag agtgagcctt tgtacacatt taagaaacag ttgaatggat gccctactct
40260

ttgtgcaagg ctatattcaa agtggttgtg taatgatagc aaaaagtgaa acaaaacaaa
40320

acatgaaaca aaaacgaaac cccatatata tggatttcac ctacacatgg ttgaaaagtg
40380

atttcttttt cccatggtcc tataatactt tatgtaatta tctgtaatta aattagcaca
40440

cagtaaaaat aatcagcttc caataaaata aagtattcaa aacaattgct ggactaaatg
40500

acattctttt tctatgaaag aaagtgtcat atgcagcaaa gtggcaaaaa tagaacggag
40560

tgagctgtgt taagtgtaaa tcctaaagca atgaaagtca tttgtattat ccttacttct
40620

ttgaataaat tcagatgaca gccctatttc atttcttcat tcagtttggc ggccagatgg
40680

cattagaatc atttacttca ccttgttgtg cttaattact acaatcaaca cactattttc
40740

caaattaatc ataacaaagt aatttcatta gtaacacagc aggctgttga attattagca
40800

tcataacagt ttgcaaacaa ttaaacttta tttgaacctt aaaaatttgc agagtggaac
40860

attattgtaa gatctaatgg caatagagta caaattcagt acagagcaaa taatcaagca
40920

aaatgctata taaaatatga cttacaatca cctttgaaaa aaattagctc tctaactgtt
40980

cacagaaaaa caaataaaag ctaagcaaac ataatgagac catagcaatt agcatatttt
41040

ctaacaagcc atgttgttta aatttatatt aatgatatta ttatgatctt gaagagtatg
41100

tgatatattg tcaagggtgt aaagaaaact ctccctggtt tgtatacaaa tgatttcatc
41160

aaatgaatat aaagcagtca tgaattgaaa gtaatgatta tttgtcctgt cactccagtt
41220

ttaagtcttt tctttccttt cttatgtctc ccacctatat tcaagtttta aaagaacaaa
41280

ggccggtgga gatctgaaca gaaataacac cttatttctg tattgtcact gttcacgaga
41340

ctatctggct tggaactgtt ggtcatcagt tggcaaagga ctccctctcc tggattaact
41400

gtaaaaggag gcagaaatct agatgctttc catcatgggc gagtttgtgc catgggggaa
41460

gggtttcaag aggttttgtc tgcaatgaaa agctgctgcc atctactggc ctaggactaa
41520

aaacttcttt tcaacccaaa gcacaaaccc aaatccacaa aggctacatt gtcatgggaa
41580

acaacggctt ttgcttttca gctgctcaag ttttgaaaac cagtccacta aagtcgtctt
41640

tggttgtcta acttttgcca agataatttc agtctatttc aatggcacta tatgaggaaa
41700

aggtaagaac tgcacaaatg taacttctga aaactttaac ttcacaatgt ctctctatat
41760

aaacttgttt actcacaatc cctaaaatca aatttctttt tttttttcac atttgagatt
41820

ggatgatcat gatattgagg gggtaatggt aaagcagacc ctctcaaaca ctgcttataa
41880

acagttttac acaaataatg gagattttaa aatattacca gctcagatac atgtttagaa
41940

tctttataaa gcaaagatat tgaaaagttc cataaagata atacctgata ggtaatgatt
42000

caatattagg agtttatgtg tagtccaaat aattatctaa ccagcaatgc attttgacat
42060

atgttaaggc tacattggta tctcataggt attttaaaca cctctgattt caaatataca
42120

tgaaaaggta attatatgat tctacaaatc ttgctcaggc agcacagagt ataactcaaa
42180

tgtcaggcta acactacctt tctactcacc tgttggtaca tgctaccact gggatttcta
42240

cactctcctc tgcaaaccct tcctttcttt tcagtgctct gagaagtata tagaaaagcc
42300

tcaaatctgc tgttctgcag tgggatgtaa atagtatagc aataaggcaa gataagagag
42360

tgcttggtta tgccttttta ctttgagaat acactgagat tctgtttttt tttttttttt
42420

tttctgagat ggagtctcac tctgtcgccc aggctggagt gcagtggcgc gatcttggct
42480

cactgcaacc tctgcctacc ttgttcaagc aattctcgtg ccttggcctc ccaagtagct
42540

gggactgcag gtgaacatta ccacacttgg ctaattttta gtagagacag ggtttcacca
42600

tgttggcctg gctgggagat tctttatacc attttgttag tggtagggtg gtatttctta
42660

ggaatttttt gtttcattaa atttaaacaa aagtacagta gaaaatgttg ccaggcccat
42720

catcgcacca ggatatttgt acggtaaaat gctttgcatg agtcatactt ctggtacgca
42780

cttctgttgc aggtgacagc acactgccat ttgcaacttc atcatttttt ctttcttgcg
42840

gctgtactta ataggaatta aatgtgtgaa aataaataat aacaataaag ttatctataa
42900

agtttgaaca ttctttagca ttctttgttt agcaaccaca ttcttcagca tctaccaaaa
42960

aaaactcgca tgatacacta aatttaaaat gctatgaaat tatacattac tctctaaaaa
43020

taattgatgt ccaatgatta agaaacatta caaatcacac cagaagtagc aatagtagca
43080

tactatgcta tagtagacta tatgttacgt ataaaatagt gatgaattct tcacacaaat
43140

caaatgggta aataaaaact ataattattt ctttgcaaaa tagcaacagg tataacttaa
43200

ctggttgcat ctaatttaca ttaatatcct tcatttaatt tcacatcaga ggaatatttt
43260

aatgagcgac tgcataaaat aacagtcaaa gataagtaat gtttaataaa acaaggagag
43320

gttaggagct gtcactcgtg ctttcatgac gcttctaaat ccaaagcatt tgcagacaag
43380

cagaagaatt ttccttttct gcaggagaaa gggaaagctg cattttaatt tttgattcag
43440

ctgaaacttg atttttcctg cttaaaaaaa ttacactttt agccaaggaa aaatgtattt
43500

ttactcaaca ttttgtcaat tcaactcttc agaaaggcca taaaaagtgg tattttactg
43560

aaaaggagga agtttgggaa aacagctggt tactatcaaa agaactttct ctaaataatc
43620

tattcatttc caaagtaaat tccataaagt ttttatttta aaaattctta ttttaaaaat
43680

taaaccagat caaacaaaac aaaaaagatt tcggggcaca atctttgtgt tatcagaaaa
43740

ctctaaagac ttttaaaaag gcaaagactc ataacgaaga gaccaagaga acatatgagc
43800

tattacagaa tattggatta aaatgtactt acccaggact ctcaaatatc atacatctcc
43860

attatgtagg ctttaggcac caagccaatg gctcccttca tttctcctac ccaccagcca
43920

tatctattgt attcctaatt gaaaacaata tgcaatatta acctttagac tggtgtaagt
43980

acaatgtcgt ttgtatgcaa cttcttagaa atgttatttc tatttataaa gttaaaagag
44040

cttgagctgc aaatgtaaac attttcatct ttttgtttct ggacacttta aaatgctgtt
44100

aggcaacgat ttaaattaag gtataacaca aagcctgttt attattgggc acagaacaca
44160

gaaacaatca ctcatgatga aaacctagaa aatgaaacta cctcaggaag ccttctctcg
44220

gggacactct ctctctccac agagcctggg gatacctttc atgtgtaaga ggctatagaa
44280

ggttaaagcc aggatcctta agctattata tttgccttca agaagcttat catctagcaa
44340

ggataaaata atttttttta caataaacta cttatttctt aaacttatat gacctaaaat
44400

gatttagaat gttgatagat gtgtgcccct tcttaagaaa atctaatata cacactttaa
44460

cagataaatt agagaatacg gaaacactaa taataccaaa aaactgctta aaaaataatt
44520

cacaaatagg ttcctttaaa atagtttccc taaggcattt attattcctt tattattgac
44580

cataaactac cttagataaa gaataggtag aaggaattta aaggtgctgt tagagtcacc
44640

cttatagtgg aagatcaata ggaaataaaa gtatctcaag gtataaaggc aaaatattaa
44700

tctacctcta aaacagagtg gtcgatgagt cacttttgca tttactttgc aacttgagat
44760

aaacaaacct atacctcctt ttctcccact ttttcctcct ttgacagata caccactgct
44820

ttaggcattg tgaatactcc ataaccggtc tcatatgtct gccagattga ctaatgtatt
44880

tttatctcaa atttatttat gtgtagtgag atacaaacaa agtaaaatga atttcaagtt
44940

tcaagaataa tatcttccaa aagcaatact cttcacaagc attgtagtgc tcaaatttaa
45000

tttttcatat aagataaatt ttcaaaatca atcccaaaag taatttaata ggttatgatt
45060

ttccagcatc tgagtcatag ataaatgtgc atatgtatat tgagttatat gtggggaggg
45120

aatgttaata ctagtacaga cttaatattt gtattgatta gtatgctatt gaccatcaga
45180

aaggaaaatt atgcaattct ttaatacaaa tgtagcatta cattagcgtg tatgtattga
45240

tatgtagtct atcaatagag agattatagg ataaaatcca taggcacttt ttcaaacagt
45300

gggcagtatc aggattaggt ttattggaag aacaaagtgg agggggagag gggagaaaag
45360

accactcctg ctattgttat tctattgagg gcacagctct ggcttgtgag gcagtaaact
45420

caagatttta agttttaaac ttttaaagtt cattgatagt gtgaacttag tggacgaagc
45480

tgtataagga acgaggatac tctgatgccc agaacccaat gatggctata atctatgtag
45540

gacatattta tcagaagaaa atactaactt actgaaagtc atgattagca acgattatat
45600

cttaataggt acctaataaa actacccttt tctattttta aggcaatgaa tacaattaaa
45660

gcctaatgct taaaaaaaca cacgtactta acgttccaca gactactgta agatttcaaa
45720

gtggataatt aacagggtag gtctgagtgt taaagctctt attcaatgcc aatataagat
45780

ttttgaaact gattatttgg aatgagaaaa ggaactagcc ccctgctccc cagtgacaga
45840

catagaaaac attcagtgag aagacatatt attggcataa acttgcttcc agagaatggc
45900

cctcaatctg ggggtcaatg cttagattca aattacaaga cagaaagtta ttactttata
45960

atattctact ttggtcatag tctatgctat caactaaagg catgttatca ttttcaaaaa
46020

gttactcaca cccaaattta agaaattagt tcccaagaaa tcacatcaac aagggcagac
46080

ttcacagttg agagaattct gtgctatggt agaacaggaa gatttattaa ctagaaagca
46140

tagtggtaaa cagcagggag gagcttaatc aactataaac aaatccctta aaagttatac
46200

tctagggaga aaaaaggttt agactaaaat aatcttaaat gcttcactta atttaaaata
46260

attgaaaaac ataggaaaca tacacttgat aaaaatactc taaaatatca gatttaaatc
46320

agaaaaggta aaactaaaaa ccagaatatt accttatatt tgtaaagaat tgcaaattat
46380

attcctgttt gaaattaatg cattatgatc aaatatagtg aggtaaaaat acaggaggca
46440

aatttatgga tttacactgc agccattaga gtgttgacaa gactgcaaat acaggagctt
46500

tttactttta tcattactat tctctgtatg agcctcctga gcaacatgaa tcccaattaa
46560

accatttgca gcctataaat tggaatgaaa atgcttcttg gagtacagta ttctaaactt
46620

tggcctgaac attcaatatt gtacaaggtt tccttctgaa aaagaaaatc tgcctgtcta
46680

tgcctttcct cttttccacc cccaaacaaa tgtgattatg aatgatatat tacttacagt
46740

ggaaaataat gcaactgata caatgttgtt ttgcagtcca caaactaatt atagtattgt
46800

tctataaagt cacaagtgga tcatttaaaa aacaaaaagg aaaactctca aactttatct
46860

tcttagcaaa caaaaaatag acaatccata ccaggctgtt gtctaaatct ttgaaatgtt
46920

aggaagcatc atgtaatctt tatggaacac aaggtttcag gtacaaggag gtctgccatc
46980

tggtatcctt cacctattca gctcttaaca tcagctgaaa tgctaacata ggcaagtgac
47040

tgtacttcag gaggactgat aaaataatga agagtccttt aatctacagg aaaccattct
47100

agtcaattta taagaaatat tgcatccctt tgtaggacgt gtaattattt tctattaatc
47160

atgtgctata aagctacatt atgcggttat tctgttatca ctagaaagag gcctgcagtc
47220

agcttctgac aatgttaggt aattactaac acaaagctgc tttctcctcc tgcagtaatg
47280

ctatcaagat ccagaggtgt agtgatgctt attgtataaa aatagtactc aaaagaaatt
47340

caatatctgc aaaatggatg gcttcaaggc aaaatgtagt gtggcaatca aagaagcaaa
47400

aaaaagccaa acttgcccag taatccgaga aaataatagt aaggcggtag tgaatgcttg
47460

tgatctaact cccatgtttt caattttcat accattatag tcattttatt tgttttattt
47520

tgtagttgaa attttagtcc ccaattaata tttattgagc agtaagagtg actgggtaag
47580

aaggcaggaa aaaggactag ttatgataaa gtgaaacaga gactttatcc aacataatag
47640

taatatttac tcattaacta aatcttcttt ctctgctgga atattatttt acacttaaat
47700

aaggccccaa catttactta aatctttttc agcaccatga ctgtcattta tcgagtatgt
47760

taatccgctt tctgcgaatg atatggtaaa agaatgctga cttaacagaa acctgaaaca
47820

ttaaaagatt taataatgta ctaacccttt cagaaattag ttaccgatgt gtttgaaagc
47880

aacaggtgtt tgcaatgtta aaaaaaattt gtgtcctttt ccaaacatat atctgtgctt
47940

caaaacacac cagccttttg tgaaaatgaa agtaatttta ttcacaaaac ataaaataca
48000

gcaagtttaa gaaccaatgg acttgactgt tgttttccaa gtattcttca ttgtgatatc
48060

tttattcata aacactgtaa tactatgaaa actagatacc tatttaaagt gtataaatac
48120

tttcatcaag gctacctctt tagtctcttt tttattaact ccgattagaa actttgtaag
48180

aatcctctca tttctcatac attcattgtt tcaaaaggtt atctttataa atgcacaaca
48240

ggaactgtac aacctcggcg ttttactatt actatctata cattaactac ttgaatctta
48300

gattcctaac ctgttaaatg aggaaactga cacaatctct aaggacctcc atgatgtttg
48360

agcaccacca ttcaggaacc tgattgaaga cactaagttc tctagtggtg tacggagatc
48420

aatctcatta ctttgcaatt tactttgcag gtgtgacttt gagttcctct aattcaaaga
48480

attactgttt ttcctccttg cattgtatac tccattttta aagcaagaga gacaaaaaca
48540

aggaagctga cagtaagaat tctcataaga aattttagtt tatatccctg agtcatgcta
48600

tagttaattc caatttctac aaagttgaca gttacaatac catagagtaa aacctgacat
48660

tcagactggt gattcgcaga taatgttagg caaagaagat catgtcatgc tcagtagaga
48720

gtgactttgg tattcagtgt atggtatttt acctacatga ctctattaac tgttctcagg
48780

ttcacccatt ccactttatt ctcactggca ccactaattc aggccttcat caccttgccc
48840

tggattactg cattatcaat ggtgcctctc tgttcttccc gggaactctt ttcatcagtt
48900

taccctgctg ctcaaaaact tacactaact ctccactgca ttaactgtag actttagctt
48960

gttcccaccc tgctggcaac cttttcctgt cactgttcta gctggcacaa tacaacctgc
49020

cgcactacac tcattctacc tctgtatgta gtgcttcctt cccagagaca tctcctctcc
49080

ctcagccaaa tcctgtccaa tgactaagac ctgactcagc tgtcatgatt ttttctctga
49140

attagctaat ccatatttat atctctcatt tcaagctaag catttattcc tcagtagatt
49200

ttacgtacat tagttttatt ccttcaacta aaataaagta caaactcatt aagatcagag
49260

gaaggactta cacttaatct gtatgcctcc cactgcagga gatataaaag atgttccata
49320

agtaggtact agatgattaa atagactaag tcttcatttt ggatgatgca ccagttgact
49380

atcctaaata ttttcttaag aaatgtaatt tccaatagta atgtattttc ttttgtgtat
49440

attaggcata gttaaagaat tgtagtagtg acgaaacaag catttgttct gaagcattca
49500

catgtagctg agtgggcgat ctgaaggagc atgctgttcc cacctgtggc aggctgttgc
49560

caccatgttt tcaaatgtac tacatgggat gtgccttgtc taaaaagctt aaatcccatg
49620

tgcagaaaga aggtatttgt ccacattgga acaggaccca actaattcca gttcttggaa
49680

aaaatgaatt cagtttaact tacttgtaag cactgaaaga agttaagaca gaattttgca
49740

aaggcaaaaa atagtatgac aggtagaatg gaaaatctcc tggttttttc agctccttat
49800

cttttttgtt tatatttata ttcttcattg agactctaca atagtgcact taggtactta
49860

atatatatca ataagtctaa ttattataca tttggaccct cacagaattc cctttataaa
49920

atcttttatt atatttacta tgaaaagttt ttgttgaggc caatacctgc cacttatgca
49980

cttatttatg taaagttgtt gaggccagat attcagggca gagtctagaa attcatgtaa
50040

gaatatttaa gtagccagtg ctgggaaaag ccaagatcct tcctttatga ttaaaagaaa
50100

ttactagctc ccatctctcc cttttttggt ttggctgcca agaagttcag ggccaaggtg
50160

gaagtacaac ctcagaaaca ccattgtctt tggaggtctc tgtaggagtg gtccattttt
50220

cctttgggct tttatttctt tttcccttat tatgttgagc ctcgatttct ttacctataa
50280

agtgaagggc tagcactaag gtccccctca ctctatgatc ctatcattct aagttctttt
50340

aagaagtcag gattatttcc tgttgttaac tgtattttgg taaaagcata gtaaacatct
50400

atctgccata ctgttaacat attgggatac aaaaatattt aagacacagt gtctacctca
50460

agaagttcaa agtttagaca gaaagccaga gaaacaggta atcattatat tgaatgttag
50520

gtaagagagg gaaaaagttt gtaaaaagag aagcaactaa tcctacagca ggatctgaga
50580

gactttacat agaaagtgac agctgggctg agactccaga aggaataatg aaaggagcaa
50640

tacatggcag gcgtgaaggc ctgcagagat aaaagaatgt gctgtgttca gtgaagagta
50700

agtagttgag agtgtggtta acacacaagg tacaggcgaa gataagggaa aagtggtgta
50760

agctgctgta gaaaaactag cttggaaaca tcaggttgat ttggtttctc acagggtttc
50820

tctggctgcc taccattctc caaggtaagg aaagagaagt gatggctcat gcctgtaatc
50880

ccagcacttt gggaggccga ggcgggagga tcacctgaag tcaggagttc aagaccagcc
50940

tggccaacat ggtgaaaccc tatgtctact aaaaatacaa aaaattagcc gggcatggtg
51000

gcaggagcct gtaatcccag ctacttggga ggctgaggca ggagaaatgc ttgaacccgg
51060

gaggcagagg ttgcagtgag ctgaaattgc atcactgcac cccagcctgg gtgacagagc
51120

aagactctgt ctcaataaaa aaaaaaaaaa aaaaaaagaa gtgatcctga atgactgatg
51180

tctattggct ctatgctccc ctttcatcaa tatagccatc cacccctttc catagcacag
51240

ccgttcttct aggttttata tgtaatagac acagaaaata agcattcata tttgttaaat
51300

gaataaactc catttataca taaagttcca gttcaagttc catttcctat gaatctttca
51360

ttacttactt actccagtat ttactgactt ccctttcttc tgggattctc caggactaca
51420

acactgatac cctacattgt gttttattgc tattatctat tttatgcatt ggtcttctct
51480

tcgacttaat tgcagtctcc ttgaataaca gtttgtggct tttctggatc ctgctcaaaa
51540

tagtactttg catgagtgtt caccaaatat ttgttgatta gaaaatgaaa taattttgac
51600

catctataca agatttaatt atgtaaaaga tactgtcaat aacatggttc gctgttgttt
51660

agaactggga tgtgataatt acaattaacc tctgatatag aagaaaagtt aaagtcctaa
51720

tcaagtccca cttaaccgta gcaaagtaga aagagaaaag gctttagggt cagacctgta
51780

ttgatacctg gagaaggcac ttaatagctg cgtatcctca gataatttcc ataataacct
51840

tactgcacat cagtttccta atctgtaaaa ttataacatc atctacttca cagggttatt
51900

atattaatta tatgtgtgaa agcatattaa gtacctagaa caggacattg taaatgctca
51960

ataaacactt tttcattccc tcccccactc cctgcccact aattggtata gcaaagtatt
52020

atggcaacaa caaaaaaaca gagtactttg ggacgcacta taaggcaatc tgattattat
52080

gagcaggtct ccaccctgct cttccaaaga gaatcttatc ttctcccaaa gagaacaagt
52140

atttgaggaa tataacattt tattacaatt ttaactggtt gttctttttt tttttttttt
52200

tgagatggag tcatgctctg tcaccaggct ggagtgcagt ggtacaatct cagctcactg
52260

caacctctac ttcccgggtt caagtgattc tcctgcctca gcttcccaag tagctgggac
52320

tacaggtgcg caccaccacg cccaggtaat ttttctattt ttagtagaga cggggtttca
52380

ccatgttggc caggttggtc tcgaactcct gacttcaagt gatcctcctg cctcggcctc
52440

ccaaagtgct gggattacag gagtgagcca caatacctgg catgctggtt gttccttgat
52500

agctgaatac tcaatgaaca tctgtcactt atctataatt tactaaatat cgactctaaa
52560

acttttctta atcagtagtc tatctctagt ctctatgcct aatgatttcc ctggaatatc
52620

tagctccatg ttctaaaggg atgaggaaga agacatctca aaagagtgaa agtatgctat
52680

acacactgag ggagttcctc attttcaagg ctgaaatcat gaattgttca actgagaatt
52740

aaaaaaaata tcaacgtagt gcttttcttt aaggttttgt tttgctgctg ccaaactctt
52800

agttattcat gctgaagtaa atcacatagg ccttacaggt gggctccttt ccaaagcagc
52860

cccctccact gccacctctg ctcaccttgc ctctctgaca ctgccttctg gcttttcctc
52920

tacctgtcag gccattgcaa gggctgcagc tgccagcctg cctcctataa gcacctttg
52980

ctttcatctg ccaacatttt tctggaatgt ttcctccaat ttatgtaaga gaaagtaggg
53040

aagtacttaa gcatattgtt cacaaacaga taacaagagg agtcctatga gtagaactag
53100

aacattttct gacatggtgt tgtgtccata aactgctttt cactcctagg aggagatggg
53160

tactaaatag gaggttggcc tggttaatgc ttccagtgta ctctagacca tacatccata
53220

ctgacagcat acaaactaaa tgtcatcgga gcaaacaaat gcaagcacaa atacaaacac
53280

ttcttcttat caaaaggcta taaaatataa cattttggag ctagcttagt gtcaacaatg
53340

catgtcccac attcagatat cctagttact ctgcagcagt tggccacgct atttgtggca
53400

tactgggtca tacggttaca aatgaaaaac taagacagct aaaaagtaac ttatttccct
53460

ctgccaataa agctcaacaa gactttcatt tcatttatta acataggttt gaagccaaaa
53520

aaagccatag taacttaacc tttacattct tacaatgaga agcaaatggt caatgacagt
53580

cacaattcac caatcactgg aaaaagtcaa ccaaacagat ataaagggag gtactgcttg
53640

aaactccaac caatatacac caaaaagtac ccttttattt tatattggtg accacagtct
53700

taacctacat ttcctgtgtt tgacttaata aaaagtttct caaaggctgc aaagctagtc
53760

gcatatgccc tagactagat aaaatattcc agaattctta actctcattt ttcttttaat
53820

atcaaagtac tgtgcctctt ttctgaagta tagcttgtct gttattaaat ctctaaaaat
53880

agtttattgc tttaaaagta agtatagcaa tgtggtttgc aaattaatga tgagtacaga
53940

ttgaaagata aactaataga ggatagaaat ccagaagagc aagatagaat aacataacca
54000

aaaaggcatt tgggacctca gaatatacct attgctatat aatctggatc ctgctattaa
54060

aatgataaaa tttcaaaaac atggttctag gaaaatactg ttagctataa tattatgttc
54120

ctgttttatt tagtggtaat aaaaatgaaa tgaaaactaa caaaaatatt tagcattatt
54180

ttaaacaacc caatttctta aggaaataca ggttttctat ctcaaatatg attttccccc
54240

atcaaatgaa tctgaaacca attgttaatt cactaacaat acaactgtca ttaaaatcac
54300

aaattctgct ttcatcagtc attataaatt tgatccattt tttaagagaa aaaagagatt
54360

tccaaatctc cactgaacta ctgaagcttt taattttaac acataaaaaa acagcagcaa
54420

ccctatgtga atagaagtat aaagcaaaaa gtactggctt gccttttaag gcaggcggtg
54480

tttagattta ttattagtga ggttcaaagg tgaaacctaa ggaaatgctg gacactttga
54540

aggaggggct atctcagcca ggggtcacat gatagctgct aattggaaaa tgttaaaaag
54600

ggaaggagga agggcacagt atctgcctac tcttctttct ttattgcaac atttgatttg
54660

caccagaaga aaaaaaaaaa taaaggctta gatttgtata actttcaaaa gaggcctaaa
54720

ataaataaat cacccccagg ttgactgagc acagctaaca ttccatcaag gctttcttgc
54780

aaaacagaaa ataatatatt gaatttttaa ataaaactat atttctgtag tgatttttaa
54840

atgaaaacaa cctaatcccc aagctagtta aatgtttttc tgccattaca tttacaaaat
54900

taaaacactt gccgggtgca gtggctcatg cctctaatcc cagcactttg ggaggccgag
54960

gcgggcagat cacctgaggt caggagttcg agaccagcct gaccagagaa cagtgaaacc
55020

ccatctctac taaaaatgca aaaattagct gggtgtggtg gcacactctc agctacttgg
55080

gaggctgagg caggagaatc gcttaaacct gggaggcagc ggttgcggtg agccgagatc
55140

gtgccattgc actccagcct gggcaacaga gtgagactcc atctcaaaac aaacaaacaa
55200

acaaaaaaca aaattaaaac actcttcatt ataatgcatg ggattttatt tcttgttttg
55260

cctaaatgtt tatttatatt gaaagtatgc atcttttctt aaactatttt atgttgcctt
55320

tttaaagaat attttcagaa ctgacacact tttagaaggt aaatcaaaca gtgataattt
55380

ggtatttcaa attataaatg agtccattac taaaagaaag ctgcatatct ttaaaatctt
55440

ttaaatggtc ttatttatta ttaacagaca agtcatggca ttaatttctt tacaaagtac
55500

acatttcctg tatctctaca aatactattt tagaatcctt aaaatgtatt cttccttata
55560

tatacaattt ctatatttaa ttcagaatga tgaacaaagt ttttcatgat taatgctaat
55620

attttattga catggaatag gaaagtaata acccatagct ggatttaaag ttgaatttat
55680

gacaccattt tgattgtgta caacagctgc aattacctgc accactcatt ctgagccact
55740

agatggcgta aaaaaaaaca gaaaggtaaa atttcatccc atcaccacaa aatcttaata
55800

taactctgag aaactcaatt aaacacactg cgaggcacct aaaaatgcta gtggtttcta
55860

tactaattgt acttaatttg taataatatc aagcattcaa aattgcagtt attcacatat
55920

taaatgaatt tatggtatta acttacagtc tgagtttgcc aaagtttcaa taaaactgga
55980

aggccaagtc aataaagact actagaaagt ctcaagagat tcctagctga atttgtctga
56040

aggaaggtaa ggaagtaagt cttcaaagac aaacatatac tcaacccaaa gcaacctgtc
56100

tactcgcagc caagacaccc atcaatagcc cagaatatgt ccaaatctca tcaaggaaag
56160

ctaaatgtct tctccttttt atcctcacca agatttacca ggttgattcc actaggtcca
56220

cagctgagta acgatgaaca ctgaacctta ttcagccaaa ggtaataaca tgctggcgaa
56280

cagggtcttc tttcttggct cttttttcaa actccaaaat gaaccccagg taaattacac
56340

atctctagaa ggcccttaat aaatatgatc atgatggtga aacttcaagc cttgcaaaac
56400

actggcagag gaagtacttt cgatcagctc tctcccaaat aatagcattt aaaaatgtta
56460

gatggaagta tgtgcttccc atgggctgga acacctttaa cctttgttct ctaatcaggc
56520

ttcacaaata aaattagtct ccacaaaaca ggaactgcct ccttcggaat cctaaaagta
56580

tgatacacac ctatgattat acttttaatt tcatgttaac tgaaaattat ttcctgaggc
56640

aacagtagaa acaagagatg cctatggtgg cttgtttcac agccagttct ggaagccggt
56700

gctctaccca ggctttcagc aacaaagaca acctgattca taaagagggt tttacaaata
56760

gatccacatg ggctttataa atttcatatt tacaaaagga gtataatagc tatatgccac
56820

tcactcctat aatctgaact tgcaagtgaa atctaatcaa cgttttagtt ttatacacaa
56880

ttgcagctaa attggtttta aaaagactgt gagctatata aatttaggaa tgattccaag
56940

aaagtttctt gacaggagct gaaactaatc agtttgattt ctttattaag tagttaacca
57000

aaaagctgtt atcttaactc aggacaattt aatgcttaat aacaggctta atcaagagtt
57060

gtgatgtgac tatctatagc gtccaagaac ttaaaacatc tttctgggaa caacagtaac
57120

aaaaacccat gctaatgagc agaagactga gagaaataag tgaccagcct tagctttggg
57180

caccctaact agcaattatc acagttggct ttatttattt tatcacagtt gtcagagtgg
57240

gtcctggcac actgcaggtg ctttatgatg gatggatgga aggaaggaag gaaggaagga
57300

aggaaggaaa gaaggagcac aggaaagaaa acaaagggaa aagcactggt ctgggagtca
57360

agacacagct caaatcatga ctctggcatt ttcttgaccc aagttccttt atctataaaa
57420

ccaagaatta gattatcatc attgagactc atcactctaa tgttcgatga ttctctgtgt
57480

tatgatttta tgcaccagcc atctactgtt aaaattcagt atgtggcctt ctttccaaat
57540

tgtcttctgc ctctaacaag tcatactatc tctctgaaaa gtccaaaata tttctctaat
57600

caagaaagaa accacatcaa cctcctagac ttatgattta acatttttct acttatattt
57660

caactgtttt cctacatcat ttgtttctaa agtagtcttg taagtagaca gagggaaatt
57720

tttaaatccc catattacga taggggaaat taaaatatga aaattaaggg agctcacata
57780

cattaccaaa agtataatca caataagaaa cttgactttt agatctaagg acaaaaagat
57840

tctgaataat ccagtatttt aagaagcaga cacaagaatt tatgtatata cacacacaca
57900

tatacatata tatatatgaa gatgggttta tagaattata ctttgccatt ttgtttacaa
57960

atatagtatg ctaaacaact taaaaaatta cctaagcctg tctgaataga aaagcattgc
58020

ttttaaattg ccggaacacc caacatattt acataaagat gaaaaacttg ttctataaac
58080

atagtgtttc cagtagtgaa tggcagacaa aatcagaata agcacacaga agaataaaaa
58140

ggaagaggaa agaaaagcat aataaataat ctaatacctt acaatgttaa ctttcacata
58200

agtatattgt ttctggagaa ggcagtaggt ggatgtcagc caaggctgaa agtgataggt
58260

aaacacactg ttaagttgaa tcctgttatc tgattgtgtt taaaatttga ttacatttca
58320

cttgacaaaa taattaatta ttgtgaactc ccaattgtta acaaggacct agaagaattc
58380

caattggcat tcttccctag ctctgtaaat ccaaatgcat taaaaatcga gctttgtatt
58440

ccctctttac aaacgtcatt ttggggtatc ttcttacctt gctaagaatg taaatcacat
58500

caccacgctt aaatgacaac tcatcagaaa aagctccagt acaatcccac aatccctggt
58560

aaaaattagc ataatcagtg ctcttatccc ctaggaagaa gaaagagaaa gcatgaatta
58620

gggccttcat gtaataaccc ctcatttcaa aattaaccag tagaaattaa atgatgccct
58680

agatcactaa aatagtctaa aaaaattatg tcctgagtaa acgaatactt tattacattc
58740

[…]
59160

cagcaatttt ctttagggac atttgtaccc aaagccaaag ttatctttga aaatttttag
59220

caactttgaa attgccagtg ttgaagctgc atttttcaac cgatttttgt cagccccggt
59280

ttaggacatt aggctctaaa taaacatgca acatacactg gagtatcagc tcatccttct
59340

aacagcataa gcacaatgga tgtgatgtgc tcaaactgcc atacagacca ttaactgatt
59400

gattatttag cactttccac tctgtaagac cacaataaat gggtaataaa tgctttgtca
59460

cttatacagt gcagatctcc tgaaacacgg aacacagcta aacacactag acgcttttgt
59520

tacactaaga actgtttcct gttttcaatt tcatgataat tgagttttga tgagttaaca
59580

caaaaagaat gcaagtaata tcaataagat gtccgtgtgt tcactagcca ggcagaatat
59640

atacgtgcta acaacttcca tcactggaat gtcatctttg aattctgagg ggataactgt
59700

ggagacacag gctggaccag ggttgtcatc tcagtattgt tacatggctc tcaaacagca
59760

ggctttttgg tgattccaac acaactagtc agattagcat gtcattaggg tgttcatctt
59820

attacacatt ttttgtgatt tatacaaagt gttaggttta ctagaaaatt ttaaaattct
59880

ctttaatata agaaatattt taaagccaaa aaaaagagaa tctgaataat atggagtctg
59940

aatatttagc aaatagaatg ctactttttg cttataagaa aataaagatg gactagaaaa
60000

tatacaacct agtaaaatcc ctctattaaa ttttgtctct tgttttctaa gaattaaaaa
60060

aacaagggac aatgggagtg gagagagaag gaggaagaga aaggaggagg cagtgggaga
60120

gacagatacc ttattgactt ctccacacta gcatctgagc actcatcacg tacctgcagg
60180

gatgtacaca aggttgggaa tccaaccaag aactaacaat gcttgtggcc actcttcatt
60240

acattttttt ccccaaagca tatattaagt aaattataaa aatggacact agagggcact
60300

gcagctggcc agatcacttc aaacattgtc aattttaaaa gaaaaaaatc acttaataat
60360

aaagtgactg atttccttta aaaaccactt atttagacaa atagagattt agaagttgaa
60420

acattctagt cagattaaat caattggtaa atgggtcaaa tgttgtgccc cttgcagtct
60480

gtcagtgtca gtttaaaagt cagggaatta gagcaaagag aggatgacat catttcacat
60540

gcaaggcctt cccccacagt aacatgcgag gccctccccc acagtaactg gccaggaggt
60600

aaaaggatct aaatggctgg tttctttagt ctgcgttccc catgctgctc agccttaggc
60660

ttgctccaaa aaagtggaag aaacaaaact tggactaaaa actagagtag aggttttggc
60720

ttagctatgt tgtcccattg cgttccccag ctgtccaggc tgctcttcct ttttttcaga
60780

ttctcttaca atagttttta ttcaatagat tctcaaaggg gaggggagag aaagaaggaa
60840

aagaaaagta tatggtgatt tctaacacat aatccttatc actttggggt ctctcttccc
60900

ctcgatacgc ttgctgaaac tcccattaac actaatggga gttacaggag cacatgaggg
60960

gaagagagac ccccaccgtg tgcaagatgc atctctccct agatgtgcat catgagcatt
61020

tcattaaatc tcactgctgt aaagaggcaa cagagctagt gatttggcaa tatgggaaaa
61080

agatgcattt tcaactgtgc tttctataat cttgatgatg atgttaccca aagaccaacc
61140

tcatttttgt gtcccgtatg agagctctgt taaagaataa tttgtttatg tcacacagct
61200

acatacatgt atgatttact agtctatcta tttcagcatg caatactagc tataaagcag
61260

aaaaaatcgt aagtaggggg aaaaaaaacc ctggcagtat atgtttatta gccttagtct
61320

cagggaagta atgtttgaat tcttgatatg aaatctggac acaaaaatca ggaagagtct
61380

tgttccttaa agggcagcaa tctcctctag cttacttttt aaaatttaaa gtgtagctaa
61440

acatacacaa aaaattaagc accgacagcc tcaaaagcgc aggtttgcaa aagttaacac
61500

tgttcacccc taaacactga aaaataatcc agttgagata tgtattttta aagatgggat
61560

gaggcaaata tcataaacat acattttaaa gcacaaatta agtatgttta gttggcttta
61620

ctacatggat taattttcct attctatcca catcatgatc tatattttaa aaattacaaa
61680

aaaaaactac attatgtttc ttgtcctgag aagcatgaat gattaaaaaa aggtaagaaa
61740

gaaaaaaatt aattagccag cctgtgaaag attaaaacaa tgtttttact cctgctttta
61800

aaaacataca agaagagatc attgggcaaa gctaacagaa ttgttatgaa acatgtagac
61860

agaggagata ctttgcaaag atgaaaaaca tgatgaccac gggtgaaaca tatgagaata
61920

agatagtgga gaatctctcc tttggtgtta aaaggtttaa aaactatttt agaaaggtaa
61980

atatattcta taatgttcaa aaaaaatcaa atggaatctt tattttatat atatactcat
62040

ccacatatct gcaagatata taagtttgaa catgaaggaa cttgtaagaa acacaagatt
62100

aattttgttt ttgcttacat actttacatt agaaaagtct gtggtggggg gtttagtaaa
62160

atcagtcaat gtgaaatata tataacattc ttgagtaagg ttagaagttc atgtcatgtg
62220

atgctcagca agatagtaat acccttgtca actcactgcc ctgcatggca aacaaacttc
62280

tagtgttaca tctttgacag cctagggaag agcaaaggtc acttctatgc aaaagcatta
62340

aaactgttgc ttgaaaagaa tttcatgtca ctaaagataa gaattattgt agtgttttat
62400

agccattgca tgaaaggcta gatctatttg atgcattcct ttgaggaaaa ctgatttttt
62460

ttcctaccct caaaggcaaa aatttgggag atgtttactt taaaagggct tttgaaaaat
62520

acaattgctt taagccaaaa tatttgtatc tgtggaagaa ccatctgtat gtatccactt
62580

tctatacctg cactgaaatt ttcttaaaaa gaaaactaca tatttttggc agaagtttca
62640

tgatacataa accgaaaaca tatatactta aactactatt gaacatagtc ctcaaggttt
62700

cactaagcct cagtgtttta tagcttcatt ttataggact agagtgctaa catgtaaaga
62760

ttttgttctc gcttcctttc tgaaaaaaca ctaaccaaaa ccttttgtag caacctactt
62820

ctgctttcct gtccaggtct agaatcaggc ctctgtttcc tttccacatg tcacagtagg
62880

aaggactctg gaaagctact tcctgaacac acacttcttg tccacccggc cttgacagct
62940

tgaaagagtg atgtatggac atatcagaga ggggaaggtg accctaactc tagctttttg
63000

caagacacag agatgatatg gcagaccttg ccccagctga catgtggcta tgaagaggtg
63060

taaagtcctg ataagaaggc agtctttctc ttctttccgt gtaaaataat tgctggttta
63120

tcaaaactaa cctcatgctt gcactacaga tatggtttat ctcctgctat ttggaaataa
63180

caacaagcaa gaaaagttgt ccgtatcatg tgattttgta cagagtgtgt gcaattattt
63240

ttccagtctc attcataatt agtacttggg gatatgtgcc aagtggaaat ggataagggg
63300

aacatccttc ctagcctttt aggccactag tgtccttttc caatttttaa gctgctgtca
63360

ctggactctg gcatttctta cccaattcat ttgctttctg tgttaatcat atgcaatgaa
63420

gatatgcatg gccaacattt aaacaggctg cagatcaatt agcagtctta agtttatctg
63480

atttttatca aagaagacag agctgtgggt cgactgtcag agcctgtgtc aagttgacct
63540

tttcagagat gtctacattg ccagtcccct gggcaacttg ttgtcccctg ttagtctctg
63600

aatggcagaa gtctgttctg ctcaagcaca aagtatacac agatgtctat attacattct
63660

tataggtttg acagcaactg catgttgtat ctataaactg tcctttagca gtgacacttc
63720

ctcaatgatg ataagctaat ttatgcaact aaatctcctt tgtgcagaaa tgaaagctaa
63780

ttgagtcatt acaaagtaat tcaggaagga ataccttgta taaattggct tactttataa
63840

atccttctca agtggttttc atgcatccta aatgggacta agcagcttat cagccataag
63900

atatccaagc caaaaatgcc aactttgggg agctgtgtaa acatctttag aacagtaaga
63960

acatggataa aatgaaagtg aacaaaagca aaataaggtt tgccaagaag ctacacaagt
64020

cattacctga ggtgtgatgg acactatcct gactcatctt cctttgttct tccactttca
64080

ctggagcact gtcctcttct tcttctgtaa ataaacatta tcaatggcac attgaagggt
64140

tttctcaggg ataacactac agtactcact caattttaaa agcaaaacta aaagtttata
64200

acacataaac attatttttc aaagaataaa ataactcaag attcacagtc ctttttcgac
64260

aatctaacac tgaataaata aaatactagc tactgccaaa tagcaacctt tccaactttc
64320

cacatagcaa actttccaac tttccaataa aatactagct acttccaaat agcaaccttt
64380

ccaactggct tggcaaaatt taagatatgt actaatgcac tgcatgtttt gttttaaata
64440

cttttagaaa ctcatctgga acaatcttaa tttcaggaaa acccttaaat ccagtaaagt
64500

aacacccagc gttatactga tacaccacat tgtactgtgt ggcaggagta tcatatataa
64560

aatcatggtc cctaaaacat catatagcaa ggagaaatgt gtgattaaaa atgcagttaa
64620

cctataaatc atttttttga gtatcacata atttcatacg ttttctcaat cttcacagga
64680

agatactata atgatcttca tcctttgagg taattccccc aaaattatac caaggaccta
64740

ttatgtgtca tatacaatgc tggatgttaa aatctcatag acaaataatt tttaatcctc
64800

attctacaga caagtttatc tttagagact caaaaaggtg aagtaacttc caaagtgtgt
64860

cattcgaact taagtttgtg attataaagt caatgttctt tatcctacat ataatcagtg
64920

ctccatttac atttgttcca cactcaccta taccattcct tccccaaatt atgcaaagaa
64980

ataagataaa attaaatgag aagagcttta aatagactta ataaactaat tcatttacac
65040

caagaaaaat atttattaca tgatttttcc tcacctaagg atttgcatac aaacctagaa
65100

atacttaagg caggacatat aaacaaatgg catgccaaag aaaaactgga ttcaaagaga
65160

atcataattt caagaaaact cctaaaactt taaagaaaaa tatgcaaaat atataaatgt
65220

agtcattgta actattcaat caatcagtca atcaatcttc attggatatt aactaactgt
65280

gagacagaag agatctacca aggaggtaaa agtaacagga agcctctgct cacaggaagc
65340

tgacagcaca gatgggacag aagacaagct ctaaaggaaa acaggctgaa ggggcctaaa
65400

cctgacaagt gatgcacaaa gcactgaaga agtgcagcag agaaaccccc caactaggga
65460

gtaccacgga ggctccaagg gatggagaga gtttaaataa ggagagagga taagaaggat
65520

atttccggag ggataggtat gatctttaaa agaaaaaggc atgttcatat tcattttgac
65580

taaagaatct ggtacacata ggggaagagc tgggtaggag ttacgctcat agtttggacc
65640

agctgttgag gggccatgaa catcttgtta tggactgcag gcttttattt ggtgggcaat
65700

aagagccttc gaaggttttt tgagcagaga aagggcagga ctgaagctat gcttcaggaa
65760

tggacatctg atcccattgt ctcagacagc ctaaaaaagg ggcgggagag agactactgt
65820

ggataaaggg gaaggaagtc ctgaactgaa ggggcaaagg aaatggaaag gagatggtta
65880

taagaggtaa aggaactaga atggataagc tttgtgcctg aatgtggagg aatgaggggg
65940

gggaaaagag caacaaagga tggctaaggt tttgagctta gttaactgag aaatggacat
66000

attattacta aaaggaggat agccaaaata atctatctta ctggggaaca caaagagttg
66060

tgttttcaac aggaggaatt taatgtattg gtgaggcaac ccagagaaca tgtctgggca
66120

actgcaaagt atggtagagc agtcaggact acaggtggaa atttaggagt ctcctgaaaa
66180

tgttaaatgg ggatgaatca aaatcagcat ttcaaccaag cttgagaggg tagctgtact
66240

tacacgacaa atagaactgt tctgaatttc tgtaactcaa ttcaaacaaa ataatgacaa
66300

ctccaatata agggacacct gaaaatatct ggcattttat tcatattgtt aaacataaca
66360

ctgacaattt aatatggttc agtcaaatat atagaagacc taataaaagt ataccactct
66420

tttaaaaagt atcctgtctc ttttcttttt aacaatagtt atctcactta tgccaaacct
66480

taaaggctag ctttaataaa tgggttaata gggtgtttcc atataaattt ttaaaggtag
66540

aaggaacgtt tacagtcatt attccaacct actcatttta ttgacagata atttggtaaa
66600

aggtgaagga gaaagctttc cttacttttc acactttctt tccatagcac ccactcccct
66660

tttaatttag tcacaaattc aatagtaaga aataaattaa aaaatggata aaggcactcg
66720

tcctcagtca aaagctataa ccgcatcaat acaataagta ttattagtgg cttttaagaa
66780

gagactgtgg gggctgggca tggtcggtca cgcctgtaat cccagcactt tgagaggccc
66840

aggcgggtgg atcacctgag atcaggagtt cgagacaagc ctggccaaca tggcaaaacc
66900

acgtctctac taaaaataca aaaattagca tggcatggtg gcaggcgctt gtaatctcag
66960

ctactcagga gactgaggaa ggagaatcgc ttgaacctgg gaggcggagg ttgcaatgag
67020

ctgagatcgc gccactgcac tccagtctgg gcgacaagag tgaagctcca tctcaaaaaa
67080

aaaaaaaaaa aaaaagagac tgtgggattt agatttcacc acccaaatca gcatgaacat
67140

ataaaaattc attacaagac ttaatgaaaa aactctgctc tttgacaaag ttaacaatga
67200

atttagaagt atttgttact atgaattttt tattttatta aaacttaagt gccagtaaca
67260

tgtgctctgg attgtatatt ggtgtcagag agctttgttc ttagaaatat gcactcttaa
67320

attatcagaa taatgatatt aaaagtattt acttaagaat taatgtttac tataaaatat
67380

ccaaattgaa atccatgtaa gatgccattt aataagcaga ttttagagga tgagatgctc
67440

attaaggaca cttccttttt ccattttaaa cctggtagcg atatttttaa accaaagctt
67500

tatgcattta atatatcaat ttttcttaac aatatttaaa caactcattt tatcttggct
67560

aacaatacaa atagcttcca tataaaagat aacaaaatca aatatggtgt atgtttttga
67620

atcttaggca gggaaaaaaa cctcagactg aaccttgaat agaacttggc acaaaggaga
67680

tgttcgataa atacagaata aacaaatgga ttaaaaaaat catacaaata aatcttaaca
67740

tattcattta ttaatagaat aaagctctta aaaatatgta agaacaagtg accaaaattc
67800

taggcaataa tcaccttcac aatatattag aaatatatac attcacatat tttttattt
67860

aggtttatta ttaaatatgt attattttgg tgttgaggca caatgtcatt gtacaatact
67920

aattaatacc tcaagtagtc aatgtttgtg agttaatata ttccattcaa cattttaata
67980

acagtaaaca aaaacagctc agctatgcag taaaatccat caaaccacac agatggttat
68040

aaggttttca ttatgtggtt tccatagcaa tcatattgca aacctggaag cttcttgcaa
68100

ataagcagct tgtatttaca tgcctctcag ctgcagcaca aaactcttca acatgtgaag
68160

ttattattcc aatcacacat tgttagaaga atcatcagga gatttttttt tttttttttg
68220

aacagacagg atgaaagtaa cagtaaagtg agctaagtgc agaagaaaat tcaaacatag
68280

tcacacattt actaaactga tcagtgagaa cagcagaaat gttctggtag tcatcaaaag
68340

gttttaacaa taaactctgt caaaaatatg aaacttccag gagcaatcag accctgaaga
68400

attaaaaaaa aaaacaactg cacccacccc tctttttaat agatatttgg ccttctctct
68460

gagttaagca aagagaggaa gaatggcaaa tagtttgagt cttcccaaat ctttttagca
68520

tttgagaaaa ggcttactgt gggaaaagtc atggcccttt ttcccccttt ttaatctatt
68580

taatttacaa aattgatcca gttccataac agatggatta ttaccctaat agggattgca
68640

aatctgtcta gctatgcagt ttttacaaaa tgggcattta cggcttacta atgttcttat
68700

ctttaggata aataaccaaa aagaagagtg ataagcaaat ggcaccattt agctgacact
68760

gcttgaaaaa aggttgaaga aaatttgtta acaccaactc aaggtatgcg atgttcacat
68820

caaggccgag gactgatttt atcatctctt ccagggagaa gaggaagatg atataaaaaa
68880

tctttcactt acataatgat cacctttctc actaaatacc ctcagaaaac agctttacta
68940

aattataatg tactttaaaa cataaaacac agaatcaaaa gttaagctct gaaataaagt
69000

ttggatttag atctccacct gtccaggcat tatttaatat tatatatttt tctcaggaaa
69060

tgtgaatatt tattgcttaa atgttcacat tatatgcatt tatatccatt atgtaactaa
69120

atcctccaac aattctatga ggtaattata agtactttta tcatcaccat tttatgaata
69180

tgaaaaatga atttatggag cttgaataac ttgtcaaggt tcatacatcc agttagtaca
69240

taagctggga ttttaacttt aggcatatgc cttaactcta agccacagtg tctcctcctt
69300

tattttcatt aaaatcttca atttacaggt ggattgcttt ctaaaatttt acttaaaata
69360

ctgtctgttt gtggactttg aaaacatttt cacatagaat agtaggttct acactatcat
69420

agcatcgtat ctgaagaaca tcttaagcaa actttttagt gctgatttag aaacaattgc
69480

aaccagggct gcccaaaata tatggtcttc tttctcctct cccagttccc cttcctcacc
69540

tctagctttt aacacactcc tctgttccta aagttacaca ctatttgggc attgcacaca
69600

agatgcccga gtgggagatg gaagaggtga catgggggaa cagaggacac caccggctag
69660

tcctagtggt aaaatgtaaa agatagaagt actgggagga tacatacaaa tggaacccca
69720

cccgctcttc tctcccatga cctgtggcaa gtcacaaagg ctggaagaag ggttggaaaa
69780

gttagagtcg aatctaaggg gtatgggcaa aagattcaca attatgacag caagggaaag
69840

tgagctcccc aggggcctat aaatctagaa ctactggtaa tatgaaggat ttttatatta
69900

ctatatttta ttatacttct aaaggactgt tctgtctatt caatagacta ttattttaca
69960

gcaataaaat acatattttt aaaaagatat aagcatgtgc cttttagcat tatacaaact
70020

tcagaatgaa tggaaggata aagatgactg aaatagcata gttagagtca tgtttactct
70080

tatggaaatt tggcaaagtg gggcctattg ttacagctct gttactcaca taaactggac
70140

actgcttcta attcttagtg tcatctctgt atttcaaagt aggaagagta agtctatccc
70200

atagcacttt gaagaaggat ttggggaaga caatgagata accagatttc tcaggggaaa
70260

aaatgaaaaa ttagtatgct gtatcaagtt tattacctta ggaactggga gttttgcaac
70320

aatttattaa ttaagcaacc agaaaggtta atgtgatata aaggcaagaa aataagcaat
70380

agttgttcaa ccacaaaatc taattttcac ctttgacatt ctattattct aaggtttaat
70440

atgaaaaggt tatcactacc cttcaaaagc aaaaggaaat accattggat tgtagtaaat
70500

ttctgtacta tgacattaaa attaataatt taaacacaaa tctgctttgg gaggccaagg
70560

caggaggatc gcttaacagt ttgagtttat agtgagcaaa gatcatgcca ctgcatcctg
70620

gcctgggtga cagagcaaga cgctgtctct aacaaaaaca aaaacaaaaa aatacccata
70680

cacacacaaa tcagaaaatg tgaaaataat ttttcacata gacatatttg ttaatatcat
70740

gattgggatg ttgaggaaaa aacttgaact caagcaaaga tctcatttga tagagtcagg
70800

aaaaaaaatc ttagaaggga aagaaacctt agatagaatc ttgttcagtt tcctcatttc
70860

acagaagatg aggtaaactg tgggtgctga atgactggac cgagggcaca cagcttgcac
70920

actgtaccct tctccctgaa actgccttct gtgcaccgca gacatttctg aaaagttatc
70980

tgctgctcga gaagcactcc tttgtttcag gagcagtctt gtatacactc gatcacctac
71040

aggcaattag gcaattatgg caaaagggaa tgtatatagg ttaaaaacaa agttttcata
71100

ggtcagattc ggttggtcag aagcgactgt gaattagaaa atgatcctac tcctaaagag
71160

tactaggtat taggaagtta tgaaatggag ctccttcttg tcccattgaa ttattcactg
71220

tatgataaaa attctccttt acaacccaaa ttcttggctt tttattaaag tttataacaa
71280

attgtgtaat tctaaaattc ttttagtcca ggaaagtctg aattcattta tttttcactt
71340

acagtaacct ctatctggct ctggatttct gatacaatta cagctcattt tttttttcat
71400

ttaaagtaac ctttttctta ttattcttgt tacaaaagca gtatctactc attgctgaaa
71460

gtcagaaaat agagataagc aaaataaata aataaatcat ccatgatcca agcataacca
71520

ctattaacac cttcttctag gctaccttta ttcagctact ggtttttctt aattcttatt
71580

ttaaataaaa taccccaaat gcatatgtat tcattgtccc catacatttc ttatcccaaa
71640

agtatatatg catttcattg taaactgttt tgaatccttt tttagaaagt aaaatacatc
71700

aactcaattt agattttttt ggcttgcttt aaattttatt agattaaaaa atactatcta
71760

gtgacaaata ggctgatgtt atgattattt gattgtaatg tggctgtctt atcagggtct
71820

acaactgcct actataacct ctggaaaaac agctgatata tggatgcaga gctcatttgt
71880

tataggcttt tgctctgttt cctctgcttg cctatgcaaa tatacccttt ctcatcatat
71940

gcagtgcatc ttaacctcat tttcaagagc ccaagaaaga aaaagcacag tttcatagaa
72000

agaaccatca ccattagact tactggctcc acatccctta taccagcagt gctttccaag
72060

atctgtttgc agggtggagg gtctgtgggg tcaaagctat tttcataata atactaagac
72120

attatgtgtc tttttcacta tgttaacatt tgcactgatg ttgcagagca atgatgggta
72180

aaactgctga caccttagca taaatcaact tagactgccc tgcactggca taaaagtaaa
72240

aaaatgaaag aagtcagttg cactaaagaa tgtccttgat gaagcagtaa taattatttt
72300

cacctaatat caaccttgag tacgtaactt taatattcta tgtgacaaaa tcagaagaat
72360

gtataaagca tttctgctac accaccagat gcgtaagaat ctgagtgttg agctgaatta
72420

gccatttttt tattcatgga acaccatttt tacttgaatg actgacaaac tggttattta
72480

gacttgggaa tctggcagac attttctcaa aagtaaatga aataagcttg tcatttcaag
72540

ggaaacaact gacggtattt attgccaatg ataaaatttg agctttcaag caaaaattag
72600

gattttagaa acttgtatct gccactgtta gcttgacagc tccccactat ttaaagactt
72660

ttccaatgag atggatggtg atataaacaa aatgtgggtt tttcttaggt tgtgtcatag
72720

ttgtgtcaat atttgtaggt tctgcataat gtaatgaact agtattttcc aactgaccaa
72780

agcatgttac aaaatcaagc atgggtaaaa gatccattca acaaagtgca agacaggcct
72840

atgaatttta atgtaaaaga gtatgaaaag ttcattggta tggtttcaga ttccacattg
72900

taccagcttt taactactac ttgtcgcgtc tgggtacaat atcaaaaaag aatacctaca
72960

attacctgca agtaaagtac ctcccttttc cttcctacat atctgtatga ggtggatttt
73020

ctttataagt taactgtact taaacaatat atcacaataa actgaatgta gaagcagata
73080

tgaaactcta ttgtcttcta ccaagacaga cattcaagaa agctgtaaaa atgtaaacaa
73140

tgccatattc ttctaatttt agggggaaat atagttattt ttcctaaaaa taggttatgt
73200

acagatgtaa caggtttatc atcatgactt ttaaattcat taataaatca acattcttta
73260

aagatctgtt ttaatttcca atagggtaaa tattgagatt taactattta ccaacctatt
73320

tatcattaga tagtattttt taatctaata aaatttaaaa caaaccaaaa aaatctacat
73380

tgagatgaaa tactttactt tctagaaaaa aattcaaaac ataaacaaaa gcttttaagg
73440

gtccttgata atttttaaga ttgtaaaggg atcctgagat caataagttt aagaatggct
73500

accctgtact ttctagggta gttttagtta aatatctaaa cattgttcat ccaccacatg
73560

aaaaaaaatg atatcctgct aacctagaac actatttgat aattatttta tgttcccaaa
73620

gcacacaatc tcaggattga atcacattaa actgttgact catattgagc tattgaccaa
73680

agtccgtatg tgtttttccc attttctgtg aagccaagtc tttcctttct tgtacttgtg
73740

caactagatt tttggagcaa gtggaatatt ctatcatatt tcatcttgtt ggatttgacc
73800

catgattttg ttctattgag agcaagcctg tatccaaaac aatgatgaat aggttggata
73860

tgataatatt gaaaataaag ccctaaggta ttcataaaat caattaatta gcactctttg
73920

gacacagctg tagagtcagt tatctggtgg tctctatatc tataaagatt tcatgagaat
73980

cctgaccaca tgtcttactg aagtccagtt atatgtccac caattcctgg aactcatact
74040

gggttccagt aattagtgtt tcatcttcta gttaacataa aagactctct ttgatactct
74100

atgttagcag tttctggagt cctctttata gcccattaac tatctagaat tttcagaatt
74160

catctgaaaa gggataccag atacgctttt cagaaccaac acagccatcc ccaactgctg
74220

ggactcaagc tgcagttggt tcataactgc gtctctcact agaaattgcc ctcaactgca
74280

gggaattgct tcactcaagg ttatagggcc tcccagagaa tggcttggag ccaatgaata
74340

gctaatgcag gaatacaaag acaagcccct atgcctcaat ttggcacaat cctgaagagt
74400

ccgcctagcc tagtctggcc ttcttcactt tcatccggcg tatgtcctaa gatcactcat
74460

ccataaacct tctacataaa actgtcttag agtctgtttc agggaaccca acttgagaca
74520

ctttctactg tttttgaagt ttataataca attgctagac tccagacctc taaagtcttt
74580

ctcatccttc atgtttccct caaaatgagt gacagcagct caatgacttc atttacaatt
74640

tctcttgtaa tgtatgacag ttttacctgg gccagaagac aaacccaagt cctacttatc
74700

tttaaagcaa agctcaatcc ccactctggg aagcagctgg cctctctccc ctttgtgttc
74760

tcacagactt ggtccaggcc tctattattc cactcataag gtactgcaaa cattcctcta
74820

ctgtccactt ttactccagg tgtggactcc ccagtgactt atttatatta cctaaacaag
74880

cctggaacaa agaaggagtt tgttttggct agctaggtat tttatttgaa tctttgtcaa
74940

tcctgggctt ctgttccctc ttaccaatct tattacttgg aggaagattc ttaacagaaa
75000

aggagcaaat agaaactgag ctgttttctc tgaaccactg tctctaataa gttttgaaaa
75060

caaaaacaca caactgtcat acatttttgg ttgtgcttat cataatttat aaatgtaagc
75120

tattttcata ttttctttgg ttattcagct atttcaattg ttgatggata ccccctttaa
75180

aattcgcatt caggctgggc gcagtggctc acacctgtaa tcccagcact ttgggaggcc
75240

aaggcaggta gatcacctgc agtcaggagt ttgaaaccag cctgaccaat atggtgaaac
75300

cccatctcta ctaaaaatac aaaaattagc caggtgtggt ggtgggcacc tgtaaaccca
75360

ggaggctgag gcaggaggac tgcttgaacc cgggaggtgg aggttgcagt gagctgagat
75420

tgccccactg cactctggcc tgggcgacag aacaagactt cgtctcaaaa ataaataaat
75480

aaataaataa ataaataaac aaataaataa ataaaacttg catcattgaa tagattctag
75540

cacaaggata ttattttaac ttctcttttt ttccctcatt aagatcattt acaattaaaa
75600

ttcaaatggc atttaaaaat acttgtttgt cattaatacc acaaaatacc acaatatttt
75660

tgcttattct ttccctgaat tttctgaaat ctgccttcct acagtcttag caaatgtgtg
75720

tgggtaatgg gcagcctttt cttcggtggc tatcatggat tctaagatga tatctgcttt
75780

tgtccaattt tttccacaat taaaagtgga caccgtatca gtttccttat ttatttccta
75840

catccttctg tctcactcat tttaaaatat gccacttcca tgtttgaact atattttttg
75900

tttttgtttt ttttgaggca gggtctcaca ctgtcaccca agttggagtg cagtggcatg
75960

atctcagcac actgaaacct ctacctccca ggctcatgtg atcctcccaa gtagctgaga
76020

ctgcaggcgc acactactac atccggctga tttttgtatt ttttgtagag acagggtttt
76080

gccatgttgc ccaggctggt cttgaactcc tgggctcaag caatccacct gcctcggctt
76140

cccaaagtgc ttggggttac aggcatgagc cactgcgccc agccatgttt gaaccgtatc
76200

tcaagaaaga aaaagatatg atgataatga ttgtttattg ctctctctcc ccttctctat
76260

attcttagtg acagtttgtc agttcttctt gtttgaagat tcctagttgc tgcatactag
76320

aaaaactaac catcctgatc cctggttctg tgtgtcccag tcagtaagaa actgagggtg
76380

gggagagagt cacactcagt cttactccct tgtgcttagt ttgctgtgcc ctacagtaat
76440

agcttctacc tgcttagaag gcctcaatcc aaggaatgaa agggatcaag cacatagtgt
76500

tgaatattta atatttatag ggcaattatc aaataatagc ctcacttgta tttttctact
76560

atcaccacat ttcccctctg caatatgagg taacttctaa tttgcgaata gagattatga
76620

gacacatcaa catctcggca taccaccacg atgtggtatt tatcttgagc agtggttatc
76680

aaacttcagc agcatcagaa tcacctggtg ggctttttaa aacccagatt gctgtgcccc
76740

atgcacagag tttctgattc agtatatttt ggctgagctg gagaaggtgc atttctaaca
76800

agtttccagc ccatgctgct ggtcccagga cagtactttt gagaaccatg gctcagagcc
76860

gtatcttcga aggcacaggt gtccaacctt ttggcttctc tgggccaccc cagaagaaga
76920

agaattgtct tgggccacac ataagataaa ctaacactaa caatacctga tgagctttaa
76980

aaaaaaaaat tccatgcata attttcatga tatccaccac cacaaataag caaaaaactc
77040

cctgcattca aaggattgga catggttgtc ctaaggcttt cttttgcata aggtctctaa
77100

ggtttcagtg ttctatcatt atttgaaggt aagtacattt taagcttcaa aatacagata
77160

tatgtcacta aataatacca aagtaaaatt aattatataa tggaattagc aaaacaggag
77220

ttaatcccat atatgcattt gaaagataca acataactat ataaatgcaa tataatttgt
77280

agtccagaag tttatattac cctctttcac tagctataca aacttagaca agttaattaa
77340

actctctgaa cctcagtttt ttcatctgga caacagaact ataaaatctg ctttagataa
77400

ttattatgag gatgaaataa ggtaaagtgg tcaacacaaa tctgcatagc agatttgtaa
77460

cagtgattat aataatctgt ttactgtgta ctaaacacca ctaggtacta tatacataaa
77520

tctcattcaa aacccatcat taacatttta cagataatga aacgaaggca tagaaacact
77580

taagtaactt gttcagcata acacagctat aataataatt agtgtcagga tttacaccca
77640

ggcagtctgc ctccagagcc caggttctga agcactacag tttttcttcc ctttgggtgt
77700

aaaaccttgc atatattact tgctcctgtt tgttaattaa ttaaacagca ttttaaccta
77760

ttgtcacaac atgcaactta tgagaaagtt aatatcatga tgtcatattg acatatattc
77820

ttatttttaa cactcttcaa tgataaaata atttggctaa ctgacaatcg tttaaaaaaa
77880

tgctcctttg aaaaatagtg tacactgctg aaaaaacagg agacaagtca tgtttgtaat
77940

ggccacagaa agtgaaaaac ttggcatttg ccccttccat ttttcttcca gttgaaaggc
78000

agcttgcaaa tggaaaaatc atctcccatc acctccctcc cagagactaa atgtgatgaa
78060

aaaacagagt taggctaagc cctgaagcaa gcagggctga gagcagcttc aatcctagcc
78120

aggagcctaa gaagattgat taatcctgaa taagatagct atagtttctt accagatatg
78180

actgcagtaa gacatgaaaa atacagcaaa ttaaagcact tttcgtaata cgaaagaatg
78240

gcactctcaa aatatgaatt tccacgtacc tatgagtggc tgtgcacaga ctttcagaat
78300

tgtttttaag aagttttcaa ttctaatgta agagatatct tcttgacata cagactaatt
78360

tatgataaaa taggttataa tggctgaaga gaaaagatgt gctagaacta gtatatttct
78420

gtagaaaggg gctgccaaaa gaagcaaggt agaacctgca gggggaagaa ggggaggtta
78480

ggcatgtaaa actcatctga tggaggtctt gatgctcatt cttgcaaaat agcatttgct
78540

gtccttcaaa gtacgtaaga ataaaaattt gagctcaaaa accaacacac ttgctcagtt
78600

ttggccccct aaagggatag gttgaactct catttacaaa gtttctctta cttggattcc
78660

agaaataaag ccaacatcaa caaaatgtct gctcttatgt aaaagagcca gaatattttt
78720

aatgtacatg gcaggcaaac cagatcttac ttttttaagg tctcacccac agtgtcccag
78780

agtagcagat gaagtaaatt tatccactct gggaggacaa atccctgcca ccttaggggc
78840

ctagggtcta ggcagtgcca gagctccaaa actagcctct agcctatttt ccagtatgac
78900

gaaactttct tgttttctca tccccaaata acaactgatt ttttttaaag ttaaatatga
78960

gattttataa acttttccta gcacttttag ttaaaataaa tgttatgcca taattatgta
79020

tattaaaatg caatttggga ttttatttcc atctaattaa ataatgcaat caaacatgta
79080

aatttaatgg caagcccaac catagtactt aattaaggga tcattcaatg tttattgtcc
79140

ttgcttagag taaatcattg atttaaagag aatacacaaa atgacatatg cactttcctg
79200

aacagtctac ttaagagcat aatgttaact atctgaatgt agtttaactg catgacttat
79260

ttacacctat tctagctaaa taaaagtagt tatcaagcag tgattaaaga attcaggcgg
79320

ttttattata aacaacttta tcatatgacc ctttacttag ctttggaaaa taatttctgg
79380

attaaaaaaa ctcaactaga aaaattatct cagtatagtt caatttagtg aggaaataca
79440

atacactact tggcatgtgt ccacaggaaa agtctggctc ccagatgctt cttgccaacc
79500

cacagtggat tcaacaatgt ttaaacaact gacaacgtac actcatagct ctgatgacaa
79560

agtggccaaa aatttagcag gagcctgtct catgggtatt ttcatatatt gaaaagttaa
79620

aatcaaaaca aaacaaatca tagggacata catacacact tcacaaaaga ttcaacacaa
79680

ttcagttagg ctttttttaa agtagatgtt gccaaaatta ttttaaaatt aacaaataag
79740

acattctgct gctagaaact caaaatatat caatgttttt tattgtataa gtgttattaa
79800

aagtgagaaa tataacccta tttggttacc aaagtccaac aataactctt tctaacaaat
79860

agattaactg tctccataac tcattcatta taatcatact atttatatta atatccatga
79920

ctctctaaca catgtaccca tagctaacaa gtgtttttat tagtttgaat aaaatgaaat
79980

tgccaatatt ttcctaattt tgacctccca aaacaaaatt ttataagagt caagggtaaa
80040

gtaaaacttc aaacttttgg caccaaattt tgacatcaaa gtggtagctt tattttctta
80100

gttatataat atctatgttt tttttataac tagccctcac gtgtctcatt taactgaaca
80160

gtcttctttc caaccaggaa tattctgagt aaaatctata tcctactgca gcagattcaa
80220

gtataaacag aatattgtta atatttaata taaaatcagc tgtcacaaat gtagcgaaca
80280

gtgaaaaagc catgatacaa ataaaattac ttcatgtgat ttttcctgtt tccagatgac
80340

aagtttcttt gagatgatat tctctctctt aataacaagc aacttctgtc acataatgtt
80400

tatgtcatgt aagttctaca aatgaaaagt gcaaaaactg gtttgacttt ctaggggaga
80460

aaatgattga tcagcacaca gaataaaata ggttatttaa attaggactt cagctgattg
80520

aaatgccagc cccattttaa agatatattc taaagtaacc acattaacct aatttaacat
80580

gacttcaaat gcaaatatgt attagtctta gcttataaaa ccaaagtgta catttgggtc
80640

tggatggatc tctatttgat ttaatttttg agtaattctt tgtatataca aatgtaattt
80700

ttatcctaaa tagcattaag cagctaattg ggataatttg cttcataaat tcagcggcca
80760

tttaaaatct cttccttcat aaatttaaca aaaatttctt cttgtcaaga agtaattaaa
80820

gccaggcaca gtgggtcaca cctgtaatct tagcactttg agaggccaag gcaggtggat
80880

tgcttgagcc caggagtttg agaccagcct gggcaacata gtgaaatctt gtctctacta
80940

aaaatacaaa aattagctgg gcatggtggt gcacacctgt agtcccagct attcggtagg
81000

cttaggtggg aggactgctt gagcccagga agtcgacgct gcagtgagcc aagatcacac
81060

cactgcactc cagcctgggt gacagagtga gataccattt cagaaaaaaa aaaagaagta
81120

attaatatta taattataaa gccccttagt acataggaga caggatatta gacacctagc
81180

acagaacctt gcctatagca catacacaaa attttgtttg acttgaaata aagtttctaa
81240

atcaagcact tataattaag tcttcactgc agtgttaatt cataacttat ggcttaggaa
81300

ttcaacctct agcaatatgg tcaaccaagc tgaaacagac ctgtccttcc ccacttgctc
81360

aaagcaacat actgatcatt tgcatggtgg ggttgaggct ggagttttaa catcttcatg
81420

gacacaggag acaaggcttt gggcctgtga gaagcacaga gatacaaatg agacccccaa
81480

cagcccaggg aagaaacaag gaagctttct gtttttccag ggctggaggt catggggctt
81540

cctgtgagaa atcaaacccc caagcctgtc caatgtgaga tccaagttta taccatcctc
81600

aagcatggta ttcccaagcc aagaaattta acataaaaat ttcaaaactc ctggggttct
81660

agcagaagca aacataaaac catccttagg agaagcactt aagaaaaaca cttccagaac
81720

ccagggcata cagcactctc atagaggtaa ggagaaaacc tgataagctc acaatacatg
81780

gacctagcag aagagacttc caagtaaaca ggcaaattcc tgtgtggcca gctctaacag
81840

aagtcaaccc tgaaagctga aggagcacag agaagagcta agacattaag gaaaagtcca
81900

aggcaatgtg aattgttatt ttcatagcat ttctaagcta gagattcaaa acacattatt
81960

acactggtcc catctctttg ctttcactgg gtgaaggatt gatcccacgt gaatgtttgc
82020

tggggctttg gaccatctgg tttttaatgt cttgagcaat ggggtttctt ccaccacttc
82080

ctttggaaag tcttttttta ttattgtagg ggcaaaaaaa atgggtttaa atgttgaagg
82140

gatttggggg gtgtttttca aagtcttttt cttattaact ctttcttaaa tctcttcatg
82200

aatatgaact cccgtttccc tataacattg aatgtgctat aatctggtct cttccgtata
82260

taccaatagt taccccacct aaacattttt caaaaggatc tccatttagt aaactcccca
82320

accttaatcc ttttactttc atcttcagta tgttctatag gtggtgttca gaaccacata
82380

tttcttactg ggtatgagta gcactgaaca gacaagccct tttaggtatc tcatttactg
82440

ccatagactt ggcttggatc tttgttcttc atcagtctct cttcggcctc ttttcataac
82500

catttcttat catttgttat atttatttct aattaataaa ttctgagtca tattggtcaa
82560

cactgtatct ccagtgtcta gcacagtgcc tgacatgtaa tagctgccta ataaatattt
82620

gttgcatgaa taattgaatt gcttacataa aaattgggtg ttttcttttt gcttttctca
82680

tttcatgttt cattttcttt agtattcttt ttcagttgat ttattagaat attagatgat
82740

ctctgcagaa tacttttaaa ttaatgctga gctcttagaa agtataaaat aaaaacctag
82800

gctcaaattc acaaagtgtc tagacatgta tatctacatg tgttgggtta ctgtctttta
82860

tacttataag tataaaacat agggagatgg gatattttat tatgggtaca gatgaccttc
82920

accttatcag gctaggtttc caggaagaac tgagaagtaa ggtagtttct gaaagaaaac
82980

acaggctgct aacagaagtg aaatgaggct atctggtata tgagagagtg taataggcaa
83040

tgtgaaattg ggagcaggac tcataagcag tggtctgaga aaaggaacta gcttaataga
83100

agcaaagggt gtggagtgga aaagggaaac aaagggctta aagtatgtca gagctagtac
83160

taggagtgag cagcctgaat cttaacctca gccttcctct ttttgaggcc ctccaatgat
83220

tcctctctgc ccactgaatc aaagtaattg cctgaaagat aatgtttaaa cattttcccc
83280

aaggacttat ctaatcattg attcttaaac cttgtttcac catcctttaa gttgcttgtg
83340

cagatttcca tatctgagtt tacttttaaa tataaaatgt accatttgct tactagtcac
83400

attaagtcct tagagaaggc aggttttaaa aatcctaact atgatcctgt gctctttaga
83460

tctactttac ctgtgttaat gaatgagtgg aagcaaagga gaatgtcatt tcctgggaaa
83520

tgtcttgctc gagatgacta gcctgccata gggaactggt aactcaagtt ggttctgttg
83580

ctcatagtag atgatttatc tttaatggcc caaaggattg atgatataag agcttttcat
83640

cactcaatgt attacatttc ctaagtttat ttgagactct gcccattgga atagtaatgt
83700

tagattttaa tgaagttatg ggggatcagc catgcttcaa tataccactg atagtatact
83760

gtttggtaat cattctttta ggattctgca gtaattcatg tcaggaaaat aatcatcata
83820

aagagaaagt ttctcttaat ttacctaata gaccccttta actaaagact ttgcactatt
83880

caggtattat ttattgaatt cttatgagga aggcacttgg tcaagtactc tgggggtgcc
83940

aggaaatata aggtttgatc cttgcactag aggagtttgc aaatataata aagctataag
84000

gaaccatcca tttgcttgtt tgccaaataa tcacatgaaa agtgttaaga tttacagaat
84060

agcacatatc aacatttaaa atgtcacgaa gttgtaatta atattcaaat gaatggtgtt
84120

agaaaagttt agagaagctc agagaagcag aggtcacttc atgctgggat ggctcttttg
84180

gggagatggg atttgatctg ggcctagaga gcagggagga tatggttaga aactgaacaa
84240

acgggacagc attctataaa gacagaagtg taagaacaca aatgtgaggt tggaaatgta
84300

tgggatatgc gtggggaaaa taaagagcaa gtcagcttgt taaaattgga gaagtgttgc
84360

attatagcag tgattaggct agactgtgag atttcagcaa gactgtggaa gacctttgaa
84420

tgccaaggta acaaatacgc attttatcct atggtctgtg gtgagccatt caacactttg
84480

gagcagcaga aagataggac ttaagaaact tctgaagtct ttttttcttg aaaacatttt
84540

ccccactaaa tgaaaaatta ttaaccggtt tataaaggag gttttgaatt cttttatata
84600

cgttgtctca gcatcagatg agatgttctg aatatgtgtt aatttccaga atgctggagg
84660

ataatgatat tataatacta tatttactat tataccatga tactatactg tgtttattaa
84720

ctcaccctat acttataaaa atactgtatt atgcccaata aatatagaga atttcaaggg
84780

tattttgttt ctagtggaat gaccacgaag gttccacctg agcaggttta acgaaaatac
84840

tatatttata ccaatgctat ataatggtgt aataaatata tagaatcaac aagggtattt
84900

tcattgtttc tgaagcagtg actataaagg ttccacaaag gtgtggatag aggaaataat
84960

ggccaagata tatagtaata cataccaggc tttggaattg gtgagagcat atgtctttta
85020

attgtttttt ttctatagtt agatttcata tatctgttcc tcttattaaa ttatggtata
85080

tttgagaaca ggggctattt attgatcatc tgagtaccca ccctatacct tgcagatagt
85140

gagatatgtt agaaaatatg gtagagttca tcagcgactt tgtggacaaa aaaatgaaaa
85200

gccacatcaa ctaaagtttg atctgtgtgc tatttggctg gatttttgtc actgaattaa
85260

ttaactcaat atattgattt tgtttaaggc atctagagct acacagttga tgaaggcagt
85320

ccagggaagg gctaagggtt agtgaagagt tcagtctgtc tggagcataa gtctgagcag
85380

cagaaatggg attggaaagg tggatttgtg tgggaccatg gctcagggcc tggccagtta
85440

gtttgaactt ggtttactag gggttgggaa ctcataaagg ttaatgagca agggattaat
85500

ttgaccatgt tatattttag gaaatcaatc tgttggcacc acgtagaata ggctatatca
85560

ggctggttag agaaagagat acacacacag gaagtgggga gaccacttag gagcttttgt
85620

aatgatccag gtgagaaatt attaaagcct gagtgagcag agagccaggg atggatgatg
85680

ttttagtggc agaatcagga gtaactggca actgaccaaa ttaggagagg tggggagacc
85740

aagaaagaag tcaatgatgg gtttgagggt tcttttttta aacttgagta acaattgata
85800

ccgttagtag aaatagaaaa gaaaggaggt agagcagtat aggttgaata ttcctcatct
85860

gaaatgcttg ggactagaag cgtttcaaat ttcagatttt tttgaatttt ggaacacttg
85920

cattatactg gttgagcatc cctaatctga aaatctgaaa tctgaaatac tccaatgagc
85980

atttcctttg agtgtcaaaa agtttcagat tttggaacat ttcagatttc aaattaggaa
86040

tattcaatct gtaatttgat cttaaataca agtttgatat gatggccaaa aattatctga
86100

aataaagcca gagctcagag gagagaacat ttatgaagat gagatttggt tggtacgttc
86160

tctcttctgg tgtctttttt atatgtgtgc agtaaatgct tatctgataa ctaaggacaa
86220

ggcaattctt aacatatttt tgttgagaga cagtacagtt attggctaaa agcttaacct
86280

ccagagtcag aatgcctagg ttcaaatccc aactctgcta tctcctagct tgtgcctcta
86340

aacaagttac tgaatctttc tgtgccttag tttccttgtc tataaaacag agatagtaat
86400

aatacctata ccatcaacca cttatgctta ttaaataata cttaggaaag tacctggcat
86460

atgctaagtg ttaaccatta ttagtataat tatcatcata tcttctacat tcttttgatg
86520

agctagctac ccagtgaaga tgttttactt tgctctgacc atatgcacct ctgtgtagtg
86580

atgtggaagg tataaagttg tccagggaga aggagtaaag agaaaaaggt ctcttccaat
86640

ccaaattgtt catctcataa acagcatctg atttgtgtgg gcaatcttat ataattatac
86700

atgtcagcct cactaaagta attcaggaca catttttcaa gtgtcattta tgcacatggg
86760

gctgtgattc tgttctagga aggccctgct taacctggct gttgcccttc tctccaatct
86820

cattttaacc tctctgcctg gctacatatg gccctccttt ctggttcttg aacaccccag
86880

gattgtccat gctcagagcc tttgaatttg gtgttcactg tgcctggaaa gtgcttcgtt
86940

tggctttttg catttatcca aaaggcttca gtttaaatgt catctcttct ttaaccatag
87000

aatacacatc atttactccc tctcacatta tcctcttatc ttgtgtcacc tactacaatc
87060

tgtaactatc ttaataggga ctaggtctgc ctttttcatg cctaaactcc cagcacctag
87120

aacagaatct gacatgcaat gggctttcaa taaatatttg ctggatgagt taattaagtg
87180

aataataata agatgacatg aggagaaggt aagacaagat aaaaacaaaa agcttcagta
87240

ttaggaaagg atagactgga aaataatttg aaacaatcag taaaaagcaa caggaaatgt
87300

ggaaatgtaa tacagtgcta agatgcttat ttgttctttt ttgttttttt ttttgttttt
87360

agatggagtc tcgctctgtc gcccaggctg gagtgcagtg gcgcgatctc ggctcactgc
87420

aagctccgcc tcctgggttc acaccattct cctgcctcag cctcccgagt agctgggact
87480

acaggtgccc gccaccacgc ccagctcatt ttttgtattt ttagtagaga cggggtttca
87540

ctgtgttagc caggatggtc tctatctcct gacctcgtga tccacctgcc tcggcctccc
87600

aaagtgctgg gattacaggc atgagccatc gcgcccgacc taagatgctt atttgtaaat
87660

ataatatgta aacaagaaaa caaactggag aagggcaaag gtggagacag tttcaagaca
87720

aaagcggtgg taagtaaaat caaatttcta gagatgaatt tgaggaccaa gactgatgat
87780

taggagagta ctggtgactc tgaagaagct ggtttagtta aggaatgagg atagaaaacc
87840

aaactgcctg aggataaaga gtaagcaggt gaagagacaa caggaacaaa gggagctctt
87900

ccaagaattt gtgtgcttaa gagaagcacg attaggttgt agttccttga accaggatgt
87960

gaaaagaggc ccatgcagga ggcagatttg ggatctagtc caaaaaactc agaccttgca
88020

actgaatgtc cttattatat atattaaaaa aactgcccca tcttaacttc tttaataagg
88080

aacaaagggg ctaatggata agtggctata agcagttgtt atttcttcgt aaaaattcta
88140

atgaagttct aataaagagg aaaacatcta taaacgaatg ttttatttga aacaaaatta
88200

aggtaaaatt tcacttaagc ttgattaagt taaattggat tttttcccat tttgttgaaa
88260

gttttacatt actcagctgt agcacttgcc gaggccagat atgaagagtg cttatattta
88320

tcactgacat tttgttgaac ttgttcattc ttattaatca ctgctttaaa gtaagcagct
88380

gagtactctt aggaagttga tattcccagc tgacctatct gcaaaaatag tgaagcttga
88440

tactctcttg ctgcagtctg tctctataga tgtatgtgtt cgtgtggcta tgtgtacata
88500

tgtgtgtatt tttttttctg ggattaaaga agcaaataca ttttcgtgaa gctactacaa
88560

actttatcat attctatctt ctgaaagaat ggatgaatta aaaacaaaag caggctgtga
88620

tctgtttata ttctatatgg gagacaaatc atatctgaag aatgaagact ccagtttaaa
88680

agtctgatat gaagactgtt agaagctatg tctttacttg ttgagatttc ttggtggcaa
88740

aggataaaac acttgattat gttcagatac aaatcctatc gtgtgaatat ctgaccttct
88800

ggtacagtgt taggattttt catccaggat tcacagaaaa aacataaaag cctagcccca
88860

ttaacataat ttttcttttt tctttacaaa tattagcatt ttaatttgat taaagtggtt
88920

gacatttgct tatcttctgg ttcagaattt taatatgtat tctattggtt ccctcagcaa
88980

tgtaaacata tgtatgtgac tctaaaagtt ttgaattcaa tggctaagtg atccaaacca
89040

tcaataattt ctagtgattc tatttggttc tttaaaaaaa tctgatcttt tttttacagg
89100

atctttttct cttgttaaag tttcttttcc ttattttatc cttgaatcat tgtaaataat
89160

gttgatttta tagcctttat agttctgtca tctccagttc tttggggatt aattctcccc
89220

aaagaattaa taacatggcc aagcacagtg gctcaagcct gtaatcgcag cactttggaa
89280

agccgaggcg ggtggatcac ctgacgtcag gagttcaaga ccagcctggc taacatggtg
89340

aaaccccgtc tctactaaaa atactaaaat attggccagg catggtggtg cacacctata
89400

atcccagcta ttcggaaggc taaggcagga aaatcacttg aacccaggag gtggagagtg
89460

cagtgagccg agatcatgcc attgcactcc agcctgggtg acaagaacaa aactctgttc
89520

cctcccccaa aaaaagagtt aacaacaatg ctacaatgct acaagttgtt atttgtacta
89580

atttcagtgt atttcatatg gaaaactaca tattttgtca tttccattgt aatttcctct
89640

tttaactcat gaactattat tttttcagct tccaaacaca tgggtttggg tattttcaac
89700

taccttttca gagttggctc ttaattaaat ttaattgtag ttaaagacca tagtctgtat
89760

aacacagatt cagtgtaatt tgttgagagt ttaactgtga acacatatgt agtcaatttc
89820

tatgattatt ctatgtgtac tgaatgaaca aatattctct actatgcaat gttactttat
89880

atctttttga tagattatta attttgtggt taaatcttta tatccttact tattttttgc
89940

ctccttggtc tatcagtttt tgaaaggggt atcttaaaat ttcccactat gattaagaac
90000

ttgtcaaatt agtcttatat gttttcgtgt tatagatatt agggttatat agttagatga
90060

atttgagctt tatcttcttt catttttgtt atgtagtgtc atctgtagtc ctattattgc
90120

tttatgcctt aaattccata ttatctgatg ttaacatagc tatagtagct ttctttggat
90180

agaaatgcat ggtgtattta tttccatttc cttactttga aactttctat gtggttttgt
90240

ttcaggtgta tctccaagaa gaatagtgct agacttttaa aaatgtctaa tgcaaacatt
90300

tattttaata gactaagtta tatttatcat tcttactttt atattatggc ttaattttac
90360

catattttct ctttgatttc ttgtttgttg ccttcttttg tgtttacatt gttttacgtg
90420

gtcacctttt atttaccctc tgcttctttg agcaacgcaa gattgcattc tatttcctta
90480

gtacttaact ctaaaatttt aacatacatt tcctaacaaa aattaaaatt agtattcctc
90540

tcctccgtcc ttctgaacag gacaacatct ggcatgcttt cactaccccc catctccatc
90600

ttgccccagc taacttttaa attattgcct aaatttagtt ttgcctcttt tttagattaa
90660

aacaaagctt attttaaaat aatcaataaa gttactgaca cattttgccc atttccatgc
90720

tccatctttc ctgtgtattc tccatctttc ctgtgttcac tccttttctg gctgaagcac
90780

atataacggg cagagctggg attcaaagtg atgtgtgttt ccttccagag ccccttctca
90840

caaccacaac aatctactag tcacagatac agaggaaggt actctgggtg tctgcagcaa
90900

ggcgtagagc cagacaagag acagatggag agaaacaggg gaagtggagg atggcttcta
90960

agcaggcatt ggcctggcac agtggctcat gtctgtaatc ccaacagttt gggaggctga
91020

agtgggcaga tcgcttgaac tcagaagttg gagaccatct tgacgacatg gcaaaacacc
91080

gtctctacaa aaaaattaaa aattagccag gcgtggtggc atgcacctgt agttccagct
91140

acttaggagg ctgaggtgag aggacagctt gaacccagga ggtggaggtt gcagtgagcc
91200

aagatcacat cattgcattc tagcctgggt gacagagcaa gaccccatct caaaaaaaaa
91260

aaaaaaaaaa aaaaaaaaaa aaaaggccca gatgctgtgg ctcataccta taatcccagc
91320

actttgagag gccaaggtgg gcgaatcacc tgaggtcaaa agttcaagac cagcctggcc
91380

aacatggcaa aaccctgtct ctactaaaaa aaaaaataca aaaaattagc tgggcgtggt
91440

ggcgcatgcc tgtaatccag gtacttggga ggctgaggca ggaaaatcac ttgaacctcg
91500

ggggtagagg ttgcagggag ttgagatcat gccgctgcac tccagaatgg gtgacagagc
91560

aagaccctgt ctcaaaaaaa ataaaaaaat aaaaataatt aaatttttaa aaaattaaaa
91620

aatatttttt taaaagtgtc aagatataaa gacacaaagc tacttttgac actatctgct
91680

ggaagactct gcccattgga gacagaattt ctatatcaga aaagcaaaaa cagaaaactc
91740

atgcgatgag gatattatat atatatacat acatacatac atacatacat aagacataaa
91800

taaacatgga ggtatggtag taagtctagg cttaggccaa aaagggcact cctttgaggc
91860

ctgtgtaagg atgctggggg attcccaaca cttaggcaca gtgcctggca cataacaggt
91920

actcaagaat acttatgaaa tgaaagaaat gaaagcaagg aagaagagaa aggatggaaa
91980

tgaaaatagg ggagaggaat gagaaaataa aagcatgaga aaggaggaag gtaaaaatga
92040

aataggaaga aaatgaaagg aagaaaattt tacaaaaggc cagaagaaag taaataaaaa
92100

ggatctcttt tttgttttaa aacttttata tgtatatgta tatttaaaaa taggcaaaaa
92160

aggaacaaaa agattcacat caaactgtta acaaagttta cgtagggtag ggagtgactt
92220

ccactttttt attttatttt aatttatttt attttatttt attttatttt taagatgaag
92280

tctcgctctg tcaccaggct ggagtgcagt ggtgagatct cgatctcacc tccacctccc
92340

agattcaagc aattctcctg cctcagcacc ccgagtagcc gggactacag gcacgtgcca
92400

ccacgcccag ctaatttttg tatttttagt agagacgggg tttcaccacg ttggccagga
92460

tgatgtcaat ctcttgacct tgtgatctgc ccacctcggc ctcccaaagt gctgggatta
92520

caggcatgcg ccactgcacc cagccccact tttttatttt atgcacatca ggaatattta
92580

agcttattcc agcaagcatg tattgtttgt aaataaggat ttaaaaatta agacttagta
92640

gctagtgaaa tggctcatgc ctgtaatccc agcactttgg gaggatgagg cagaaggatc
92700

acttgaagct gggagttcaa aaccagcctg gtcagcatag taagactcca tttctacaaa
92760

attaaaaact aaaaaattag gcatggcagc gcatgcctgt agtcccagct acttaagagg
92820

ctaaggcagg agaaacactt gaggagtttg aagctgcagt gagccatgaa tgcaccactg
92880

cactccagcc tgggcaacag agcaagaccc tatctttaaa aagaaaaaaa agaaatctaa
92940

gatttagcaa taatgaaagg gaagtgaaca aataatataa aataatcaca cagtatggct
93000

tgttgtgaag tacattaaag tgagctcctc ttcacggatc cccttggagg gctgcctcca
93060

agacttcccg tctaactcca ggggcagaga tcatctcaat cctactagcc tcttcaaagt
93120

ccaggtccaa aaagatagag aagtcacata aaagggtttc cacttctcct aaagccactc
93180

gtcaaattcc agacagccct ggaattgctc agccttgcat tcccccagga tgcctgactc
93240

cctttacaga cagtccaatc ccacacccac cggtcttttc cttcagcctc aaaagaacaa
93300

ccctatcaga ataggataaa tcaagtattc acacatctac aaggtatttt tagaaacctc
93360

ttccttccaa gatagtagct catgaaaata gagaaaatgc ttccttcatt ttttatcttc
93420

ctggcttgct catttctaca agatggatag aatttcaata ctactccttg aaggaaacgt
93480

gataggcaag tccagctgat ctcttaaaac ctagaaacat tctatgtaat cctagtttca
93540

tgtatgtaag aaaacctgtg tccataaaaa tgaattaact catatggagc ttaggttatg
93600

aatctttatt aattcaagtg ataagaataa aaaggtgata aaccaaaagt tgtctattac
93660

atccctgtgg ccagggtttt tcaacctcag cactattgac gtttgggcca gacaattctt
93720

tgttgtgggg gttgccctgt gcattgtagg tcgtttaaca gcatccttgg cccttaccca
93780

ccacatatct gtaacacaca cacacacaca cacacacaca cacacacaca cacacatccc
93840

agttatgata accaaaagtg tctccaggca ttgccaaaca tactctgggg tcaatatctt
93900

ccctggctgg gaaccactga tgtagctcca atttgcaaaa atgagtcatt ttatctcacc
93960

accagtggaa atatatgttt tgtgtgcaca cagatatttt cggtttagta tgtgttaagc
94020

catcccttgt aatgaaagga atttgcaatg ccaatggcaa catagagaaa agctacaaga
94080

caggcagaac tgtattccaa agcttcatac tatattatga aacaacataa agaatactat
94140

gaacaaatat tcagcaaaga cacatttata ttcagcaaag ataaaaataa taaatattat
94200

atgtgtgtag tgaccctatt ttatgaatga tttcattttt ttttataact tcggataacc
94260

ctctaagcag attttagagc tgaaaccatc tgtattagtc tgttctgact ctgctaataa
94320

agacataccc aagactggca aatttataaa ggaaagaggt ttaattgact cacagtttag
94380

catggttagg gaggcctgag gaaacttaca atcatggcgg aaggggaagc aaacatgtcc
94440

ttcttcacat ggcagcatcg agaagaatga gtgcccagca aaggggtgag ccccttataa
94500

aaccatcaga tctcatgaga actcactcac tatcacgaga actggatagg ggacactgcc
94560

cccatcattc aattatctcc acctggtccc tcccaggaca catggggatt ttatgggtac
94620

tacaattcaa gatgaaattt gggtggggac acagccaaac catatcacca tccaaaaatt
94680

caaccctaat ctagttatct acttgggaaa aactattccc acaatatcac cacattgata
94740

acttcaccat tcagtaccta gtttaagaaa aaataatgtt agcaggctgc ctagactaac
94800

aatattaaaa tactgaatat atgtggttct atgaaaatta attgacatct ttccattgca
94860

ttccaaagaa gaaaggagaa aaattgtttg gtgaagtcag gatctactat cacttaaaac
94920

aatgttaata ccacagactg cagggtcccc tgtaaccctg atattaactt agtaatatga
94980

gtgataatta ttcaggagga aaataaagcc aaaatgtatt ttgggggcct ataaccactt
95040

attgactaaa ttagcatttg tgtccatttg ctgtaaacta cttcaaatta aagtacccaa
95100

acactgttct tttttatgta tatataatag tctcatggaa aaaaattata gctgcatttt
95160

actttatgtt ctattttaga tgcagatagt tggactgaat acatttaaaa tgtaattgaa
95220

tggccaaagg ataaaaatat ccttttctga ctcagaatag ttcaatctgt cattgtaatt
95280

taaaaaaaaa tgaattcaca cttaaaaaaa ataggttaat ggaatagaaa tttgtcttcc
95340

tgttttatgt tgccctctct cctccagtcc acttgccaga agacatttca agtgcaatcc
95400

ctctgaaagc tacagattta ataagttaat ggatggttac cattctcatt actaggctga
95460

ttctattcag tttagatcta gaaaaatctt tttgttgttg ttgtttctgt acaatttcct
95520

gccattaaag aaaaaaaatc aagtgacata gtgaatagaa gagaagagca aacagggttg
95580

gtttctgcag caatttcacg agtattattt tccccttttg agattcaaaa tgagacaaag
95640

acactcctct gtatttctaa tgaaaaagtg agcaataaat ttacaaacct gtgctgagcc
95700

aaatgaactc attaccatgt taaacagttt aacacatttt gatcaatata ttagctgcac
95760

aaacacaaat atgctatagt attattacaa tgtaaacaaa cctgactggc ctatgctaat
95820

aagcaataag acataaaaga tgtaaaagga ttaggatatt tgaagaaaag ccaaatacct
95880

ttaatactcc ccagaaatgg cattctcgca actcttttgt acatatgtat aaatccctta
95940

aacacaaaga agtcttacaa atttattcct attcctttac gaagttagtt aaaaataaat
96000

gaacaagaaa ttaaacagaa ggtactgtaa tgaagttaga atattctaac tgaaaaacaa
96060

ttacaatgaa acaatttaga ttcctgtaga attataaaca tttttcaagg gatattatgc
96120

aatgcaattt tttttttttt tttttttttt tgagacacag tctcactctg ttgcccaggc
96180

tggagtgtag tgcctcaatc ttggctcact gcagcctccg cctcccggat tcaagtgatt
96240

ctcctgcctc agcctcctga atagcttgga ttacaggcac ctgccaccat gcccggctaa
96300

ttttttattt atttttattt ttttattttt agtagagacg gggtttcacc atgttggcca
96360

ggctcgcttc gaactcctga cctcaagtga tccacctaac tcggcctccc aaagtgctag
96420

gattacaggc gtgagccact gcgcccagcc cattgcaatt tttaaaacca cgttttcttc
96480

ttactcttga aactcaaaac actgcaggtt tatttcatag tttttaaaaa atacacttat
96540

tagagtaaga gctttcgttt ctcagaaaaa gagctttgtg atttaaagta gcataataaa
96600

tgcttagatt tctcttcaaa tacatttgta tacctcatct ttcaactaag aggattagtt
96660

ttcaacaatg cataattgac agaaaattgt gttgaaattg tagtattatt taattctggg
96720

aatttagcca gtcagatatc agagatacat agttctttgc acagaactgg aaaaatatca
96780

acagtatgta aatgtgctta ctgctcttcc attgttctaa ctcctctatc aaaattatat
96840

tcaaatttca cagaagtgaa agcaataagc ttaattcttg tcctgaaaga gagacaattg
96900

ttttaattaa aaaagttacc ttcctagttt cagacattta atcaaacaaa acctctaact
96960

ctgagacacc tttagggcct ttactagaaa aatcccctct gtagccgttg gcacaaactt
97020

gacaaacttg ctgaaccgtg cagatgttga agcagctctc agttacattg gggaagaagg
97080

gaactagaca ttccacagct gcctacaacg gcttccttcc cggggcacag gtgcaggctc
97140

catcaagctc gatcactttg cagagcaact ggggatccca agcaggggaa aagaaattcc
97200

agggcagctt aaatttctcc cagtaccaca gcaagaagta acactactta aaaagtaacc
97260

agttacttcc aacaattatg tgtgagggtg ggtgaggagg ggggcaagga agaatgaaga
97320

ataatatctc cagtaagaaa aaataaaaga tgtttcatat ttttaaaatt tcaagctact
97380

cagaccttcc ttaggtcttt gttatgaaaa taactatgaa tatctggtct ttattcctga
97440

tcaatttttt ttttctttag gggtggggag agaacagcca agttgaaaaa atacagaact
97500

taacagttat ttcaacaatt gtaatgtaac acattcattg cagggtttta aaaggatgta
97560

atggcagtaa tatcgtgttt aaatgtattc cactcttcca ttttatgagt atacattctc
97620

acaagttctt ttaaacattt ttaaatacta aaataattgt attaatgata tggtgtgacc
97680

aaaaacaagc agccagggaa tattcttccc tacttgagga taactgatgc tacagcagat
97740

gccatttcct tagggtaaac cagaaaggaa ggggttaata aacatgtagc tccactaaac
97800

aactgaaata cacagatcac tattccccat aaaattatac ttttatacta atcttctggg
97860

gtagtaaaat acaatatgta gatgcataca tagtttttat accttaaatt tataatattg
97920

ttaaaattat tactttttac ttggttagaa aaccattact gtaccaccac ataagcactt
97980

gcaagaattc accagataca ataataaaac aaaaatgatg gtgatgttac aattaattgt
98040

gggatcaagc actatgctga aagccttcca taaatcactt cacctacccc tcccaatgac
98100

attatttcca ttttataaat gaagaaatta acttataaag tcactaagct agtcaatggt
98160

acagccagaa tttaaatttt tttcttctaa agtccatact cttggtatta tgctgtattg
98220

tcttgaaaat gacatttgta cattaatttg gaacatttta agtatttttg aaatcaattt
98280

taaaggttta acattacatc attaaattgt tatattcaga gcaaaatggc ctctctacat
98340

gagatgtact agttaaatct tgcttatatt gcttatgata cctttttctt ctctaactac
98400

tgatttttta atagccaaaa aagctcattt cagcctagag tattagtaat acaatttcct
98460

aaatgttttc atatgtttcc taaatttcag atgtcttttt attttagatt gatcctcatg
98520

gccgcaagtc aagtagctaa tagataatta attttatgtt acataacaga caggcatgta
98580

aacactaaac taggtctgag catttggaat aagaaaactt ttttattcca tattgatgct
98640

aaaatatcaa caggaagact tgaagcctca aaaggccttt gtttatagtt caacaacatt
98700

cctctgagac agtgcaatat actacaagca aggagaaata cgaggcagaa gttggtaaca
98760

tctggggtgt gcatagaact tctggttaat ggaagttcca agatgtataa ataaaaaacg
98820

atgtgtgagt tggtggaaaa aactgtaagg caagtcaatt ttttaattaa aaaattatgt
98880

agcataaaca atgctaatga gccacaagtc ccaagagatt agtattgtat ctgcttcatt
98940

ttaatgtttc attcactttt cgtaagttac atctactcac ttgaaatatc tcaagcagaa
99000

ccctttttgt aaaatagagt tttgaagata ttctttgtcc ttgttctcaa taggtcacca
99060

ttttctactc aagctgtcga ggacaaatca cacacacaaa cacacacaca cactcacaca
99120

cacacacaca gccctaaaat ctttaaatga atcaccagaa agtaaatacc tggaagttct
99180

tcataaattt catcatctat tggttgactg cttgttagtg gattgcttat tggtagagga
99240

tgatcaacat catcatataa ttctcctctc tcatcataat cctcaggaat aatatcagat
99300

tccatatcta taaaacacat ttcatgaagt aaattttaaa agaatgcaga agatattttt
99360

aaaactatac tttataaata aatgcagccc tacaaagcaa caacaatatt gttatatgtt
99420

atgtccttaa ctaaaaacat gtttaaaaat tgttttgtaa atatgtgaat tttaaagtaa
99480

gccaattggt acttatagaa aagatgctgt ttaaaatctg aagtgagtgg tctgtattaa
99540

taacagtcac attcaagttg gtcattctca cagaagtcaa taagcttttg tatatgaaaa
99600

cccaaactac tctgttattt agtatttaaa gactgtgata acttgttcga ttgatttatc
99660

ttaccttgca atacaaattt cagctgctgt acccattctt cagcatcttt gggagaagct
99720

gctgtaaact aatataaaac aaacagtaag aacgaaaatc accatagtag gaatgaaagg
99780

tatgttgtat tcttaccatt tctttaagac tattagtaat tcttttcatg tctagtggaa
99840

cttttcttgc aaggatgctt cttataatgt acagttaatt ttgacaaatt tatgcaattg
99900

aagcagagga tgatgaattt caaacgctga tcagcagctg gcagctgtgc aggaaaaaaa
99960

gctgcttctc tttcatcaat gtcaaagaca gaactgatga acaaaatttg cccttgacaa
100020

ttagaagaca cccatgaatt tctttagact gctgcacaca gttttcttaa aaatgttctt
100080

gtcagattcc aacacactac ctgacaactt aattaactgg ctttcaactg caatctcaga
100140

tatttaaaac ttattaaaca tacattccat attgatgcct agctgatagc tattgaattg
100200

cagagctgat gtgtactttt accttcattt tctcccacac tcctaagcaa tacaatatgt
100260

attatcactc tgatattttt gttttaataa ttttactctt tctgctatgt aattcttttg
100320

gaagatcacc tagtgctatg caacatgtga tcacctatgt tctatgcaac atgtgtgcaa
100380

catgtgaagt aattgataaa ttatatattc tctttatctt tttcaagaag atagtgtgtg
100440

aattaatctg aaatttttaa aaatttcctc ttatatgaat gttcaatgca taatatattt
100500

aaacatcagt atttttatta ttgttaacat gtcaaggaaa gtattaatca aatgttaatg
100560

tctctataaa tgaaatcaaa acattttatc aacttaattt caaggcaaca taaaaactac
100620

aacctgatat atacgtttat caggagcaga gatttcaaaa cagcaatctt tctttccatc
100680

ctttcttaga gtgttattca ttctgacact gtagccatct attgcaaatt cacctttctg
100740

ttgtttgtct gttgaagata aaaccagtta gaatttcatt tactaagtat taatgctcaa
100800

ttatctttta tgtaaaattt cttcatcaat tcttggaaat aacatttttt ggtcatattg
100860

ctttagtaat ataatgctta ggtaatgttt ttagaaatca tttttaattc tgaaacatgg
100920

aagtattagg gatgattgga cttacaagga ggtctgcaga tcatggtctt atttggaagg
100980

aagtatggaa acatgacact ctagggtagg gaaaattcag gaactagaac atagaacttg
101040

ctagacaaac ccaccacaaa cctttggctc tttcagattt agtaggccag tgggaagatc
101100

tgtccagctg aaaacttgtc gggctttatc tactgttatc tcctttcacc tatccctccc
101160

cagcaccacc accaaaaaga agcttctaaa acaaaagttt tctttaaaaa aggtctccta
101220

acttggtaca aatttatctc taaccttaca aagcaaacca aaactaaact aaactactca
101280

tgacgaaaaa gatgtgaagg aaaaaaaaac tatgtggata aagaagagtg gtaaagcaag
101340

tacagaagca ttagttacca aaaatagagg gaaagtttct tatccagtca gtgaacagtt
101400

aaactagtaa agcacatttg aatctcacat gataaagagg caaaagatga ttacagcttt
101460

ggtcagatat tattttaaag acaagtaaag tgcgaatata acaaaggact taatattctg
101520

aaactgaaaa taaaattagt caaccttcct acttcaccca gttacaccat tttgatagaa
101580

aaactataca gctataggtt tatttcctag attaacaaac aatattaaaa ccatgagtag
101640

ggacaaactc ttttactgag ctcggggcaa atacagaaat gctttcgtgt tgtggggtgt
101700

gctttctcag ttcagtgtac caataaaaga tacatctcct tttcttaaat acacaagtta
101760

aatgcctaaa gtaactaaca tatgttaggt catagagatt atatcttgtt caaaatatca
101820

aatgagaaaa aaaatctcag ttcctgggaa actctaatca catttagtca taatttacat
101880

ctctctgaat gtctaaagaa acaatcaaag tttatttcat gaaaaaattc tacaataaac
101940

tctatgtttt tcacaaaaac gatacagtac tttaaagatt agagaagcat ttcccaaaga
102000

tattaatagg tttctgtgat caaatcaatt tgggaaacgt tttgccgaat taaacaagag
102060

ttaagcagtt ttgttagttc aggccttctt ggagccctta gtacatttac atagtaatta
102120

aacttctata agaaaaattt agtatgcagc atttccaaaa attatttgtc cgcggaatca
102180

tttttcacag aatattttat ggtactccca ggatatattt cgtaagacac ctaactagga
102240

aactattggg aaaaaagtat taaaatgata aatggttaag catggttggt tcttgtaaat
102300

tctcaaaaaa agactaaatc attcactcac tcatttgaca aattgatacc aagcacttaa
102360

aaccagcacg cagcattata ggtttgccga gataaaagct agaacacctt atcagatctt
102420

tcgcgccacc ttcccaatga tctactaaat tgcaaaagga acttaatgtt cactacgaaa
102480

agtggaagta atgtgaggca aaatatcaaa gccatattgg acaattttga gagagttcct
102540

taaactctcc agaagaaaga gaaatttcag ggaccagatt tagaggaaag ctgctccctt
102600

gctgcgcaag ggttcaatcg ctccactcac caggtccatc tgacctaaaa cctatgcctt
102660

ctacctccaa tttcggtcta aaattctcat caatgtaact tgaatgcagg tggaaatgca
102720

tgaggagtgg gagtctacac ctggagatat aaaatcagca ctataaagat ttagtttgga
102780

agagctgagg ggaacagatc tttctgggcc agattatcta ataatctaga aataagacaa
102840

ttaggcagca aaattaggca accccaactc agactagcag attcatttta gccaaacccc
102900

ctagaaatat gctatggggc tatgtatcag aacttctgat acagtccaag aggaggacag
102960

tatcagcctc tactgagatg aaccagagag aagccacaaa tccaatttat aggtaagcat
103020

ctcagatttc atagacatgt acttgttcga ggataagaaa agaggaacag caaatacaga
103080

tgccataaaa atattatgta aaacagaaac ataacatagc acttgagaag tagaaagcac
103140

aagcgaagta ggagaaatga aggtaaactt tagccaactg ctttgcatat tttataaaag
103200

taaacagatt ctgaaagaga tgaaagatga aacaatgtaa tataaaataa aaagaggaaa
103260

ggctgagaca agtagaaatt aaaatgaaac tggcaaaata aggaacaaat atgctgtata
103320

aggaagtgga aaatgcagca gcaaaacaaa tgatgtatta gaagtgataa aaagtagaac
103380

taacattgtg gaaaatcaga tcagatatac cttgaaaagc tcactcagca gatagaaaat
103440

aagaataagg ggatggggaa gggacaaaag ggagtatgat aaatatggag ggctgacaaa
103500

aatacaacat aagaaaaatg ggttttctgg aagaagagta ataaataaat gccaataacc
103560

aaagacataa tatagcaaaa gaaaacttca ttccatcaaa gaaagaccta actttgcaga
103620

acaaaatgtg ctgttgttta accaagtaaa aataataaaa agaaactaat atctagacct
103680

atccaactga ttgtttgtat ttgaggataa acaattatat aaacatccaa gtcgggtagg
103740

gatatgggcc cagaaaggtt gaacagagga aactcacaac tctttctaat agcttttgca
103800

gaaccataac catgaagtag ccatatctcc aattagccaa tacaggacag gaagttcaag
103860

acgtgaaatg ggcagacatg gctataagac aacactgttc tcctcctttc acattagttt
103920

ctcttctgga gagagaaact tcaccattat gtggtaagtc ataagaattt acatatagat
103980

tagttccaca attgcttttt gggatagttt caaggatacc tgttaacaaa attatcaaaa
104040

atagtaaaat gccaatcaaa taggaaataa agctataaag tccatttacc cagcactgtg
104100

aaagactaat attttatcta ggtgaacttc acaaatagca aaagcttatt cctttaagtg
104160

ccaaaacttt taaaaatgtt aatcattgtg atggaaagct ttctaaaata tatacttgcc
104220

taacttctca aacaaatctt ttaaacataa catttttttc ttaacatagt atcattttga
104280

gtattagttt ttataacaca ttgatgcctt cagaattttt gaggtgtttg ttcctctatt
104340

acatttctct cagcttctgt ttcacacttt aacctaacct ctatcatgct gtttgaatgt
104400

aatgtctaca gaactgaagg ctgccaggaa aaactagata atgaaatcta ttatatcagt
104460

aacttaattt tacacaatcc taattcagca ggttcaaggt aaaaagcagt tggctctgat
104520

ttcctacaaa acaaaatgca taaggctaat aagagcaatt tccttctact tttcactgg
104580

cttttaggtt tacagctgtg ttctgggggt tttttaagct gtcaaaaaga caactcacat
104640

ttactgagct tctacatgtc aagaacttaa tagaacatat ctcatataca cctggtaata
104700

attctaagca atagaggaga catattatat tcccagcact gatgggaaaa caaactttaa
104760

ccaatttgct cacggtcaca caaatagtaa acagtaaaag ccaggaccct aggaaagtct
104820

ggttctaaag ccttgctttt tcagccattt cacactgttt tctaaaaagg tgtttctcaa
104880

agtatatttt aagaatcacc tatgttagaa tcaacttgga cgcctgttaa cacagaattg
104940

gctaccactc tgcacttgct atataggaat ctctgtgggt gagtctagga aaccaaattt
105000

ttagcaagca ccccaggtga ttttgataca cactacaacc actgtcctca atacaatgtg
105060

aaatatacaa aatcacggta gagaagataa atgttatttt gcatttcgtc ctacaactta
105120

ggtcaaactt tctcagcttt ctttatgggt tcctctacct ctgcctatct cttaaaaagc
105180

tatgatgcgt tgttctaagt cttgctctct tcttacatta cactctccct gggcaatctc
105240

atctaattcc aagaattaaa ttcctatcaa tatacttgtg actcatgatt ctctactttt
105300

agttcatttt caaaactgtt tatccaaatc ttcatctgct cactaaatat gtttcatttc
105360

tcaaatgtcc tataggctca ttgaatagaa agtgcccaca atggagatca tcttatcccc
105420

actcccaaca cttgtgatgc caacaactgt gatatttctt gttgtatccc ccatctcaat
105480

aaatggcacc tccattaatt caagctccca aaccagaaaa ctgggcacca ttttttattt
105540

tgcccttacc ttcaccaact gccccgacgt taccccctac actccaatgc cacctaccaa
105600

aatctaaaca gaccttctat ttttctggca aatatacttg ttttttctct tcttcattgg
105660

ctctcagaaa aatatgctta aaacaccaac cactctcata ccattttaaa tacagcataa
105720

atattgcaac caaataattc caaatccttt agcatcccta caagttatgg acatttatta
105780

tttggggctc cccagcatcc atgcatcttt cctattttaa ttccaagatt gtcaggata
105840

gggcttagtt ttccatcaca gaagctggag agagatggat tttccctgtt ccaacttaat
105900

caaagtggat gctccagact ggaacctaaa tctccagtaa gtaacaaaag gatcaagaga
105960

gggttcagaa tccattctgg tggcaatggc actcactgat tgatggcagt gcacagtggc
106020

aacatcacat gtcctggctt ggcaaggccc aagccagcag tgtgtcccaa taagattttt
106080

catggattat gctcaagatc cagccccctt acctacctta gttcttgctt gcttgctgag
106140

cctgtgtgta gcattcttag caattctgag agctgatatt ttcctaataa tttccctttc
106200

tgcataaaca aagttggttt ccgatgctta taccaagaac actaacttgg cacaaatggc
106260

tagcaaacaa aaatatgaaa tgtgttcagc aatatctact acttatattt aagaaggcct
106320

tttcccttca gtgaataact tacgttattt ccacaagtat tagtactcat tagtagggaa
106380

aagaaaaata tgcccatcaa tactagacct tgccactaaa ttttatcaaa ctgactagca
106440

agatgttcca gctcctcagt taaagatata tgaacattct cctttggttt atacagcttg
106500

cagatgccaa tgctaaatca gccacttgaa atatatctct ggggttttgt gtagaagtga
106560

gactagatat caaaccaaca acattcgtgg tcatcagacc cttagtaaaa cttggtttgt
106620

acctgactat ttctgcatgt tgatgaaatc ctactaaaca tcagatgtac attttaactt
106680

ctatatactg ttagagagtc tcttagccat aaaggcagaa aataaaggca tagctttaat
106740

ttaatgtttg taatcccagc tactcaggag gctgaggcag aagaatcgct tgaacccggg
106800

aggcggaggt tgcagtgtgc tgagatcatg ccactgcact ctggcctggg caacagagcg
106860

agaccccgtc tcaaaaaaaa aaaaaagttt attgtgataa gttttaaagt taatactttc
106920

atatcaaaga cccaaaagtc tataaaattg taataatttc tgataaaagt agaattaatg
106980

gttcctattt cttaaggaat aaagatatcc ttcacatacc agatggaaac agaattgcaa
107040

tttaaatttt ttaaatatca gaattcactt acacatttaa tatatatcat cttgatataa
107100

ttataaaatt atataattac aattataaaa tgacagaatt tgtttctgtc aaatgaatca
107160

gaagaaaaaa ggtaagcttt tatggctttc tattaagttg ttgttacaaa acaacagaac
107220

ttttaattat acaaatataa gtactgtcac tttaacatat tcaaaacaat attaaaagag
107280

aaatatgatg ctttttcaac atcaaaatat attttataat tttcaaactt caaagagaac
107340

atatatgtcc tctttttttc ctatcaacgg tgaagttgtg gagaagggga gagcagggag
107400

gtaaatttca atgaaaaatt ttatttctct gccaagttct gaaattttgt tatcttaaat
107460

tctccatatt aagcccaatt ttctacggac ttttattata tattgaaaag atattacaat
107520

gaaaaaatta aaaccatatg atgtaagaaa taacctttga atgactttgt agatcagggc
107580

tacttgtagc tcccatttac tagacggtgc tagtgtattc tttaatcagg tttcagcaaa
107640

agccctgaga ggaggtacta agaactgaca atcccctcct taaaaatggg gagagactag
107700

gttcatatca taatgttgat tcaagatcat atagaagata aacagaaaaa agggaaaaaa
107760

cacacaatag tttagaacag gttcctaaat ggtccaccac ttatgggcac cttggagggg
107820

ttggtaataa tcatcaagtt gtagaaaaag cataactttg gaggcagaga gatctgcatt
107880

aaatcctcgc tctgcctctc aacagccctg tgttgacctc tcttgtttag actacttcga
107940

agtcctgcta aatggtcctg ttgacccaga ctctatccat cccaatccat tctccatcat
108000

cctattacaa ttacttccta aaaaccaaat ctggtcatac aatcctctgc ttaaaggtca
108060

tattcaaaat aaaatcttta gcaaggccct tattctcaga aggttcttac caacctggtc
108120

taatccactt atttagcctt acgtgctccc tcccctcact cattctaagc tccagccttc
108180

attccatttt tgatcccatc cacagtcttt cacaactcag aatctctgct attcccttac
108240

ctggaaagcc cattttaccc ttcttccctg tcttagtctg tttatgctgc tataataccc
108300

aaaactggga aatttataaa caatagaaat ttatttctca cagttctggg agctgggaag
108360

tccaagatca aggcaccagc agggtcagtg cctagtgagg gctgctctct gcttcaagat
108420

gccaccttgt tgctgcatcc cccagaagag ttgaatgctg taccgtcaca tggcggaaag
108480

gcaagagagg ggttccacag ctctctctag ccttttttat agagacatat cccattcttg
108540

agggtaaagc cctcatgcct taatcacctc ccaaaagccc cagctcttaa tacaatatcc
108600

ttgggagtta atttccaaca tatgaatttt ggaggtacac atacattcac accatagcat
108660

tcaaccaaca aactcctact cattctttca ggcccaatga agtgttctct ccaccaggac
108720

atcttcccta acttccctag gcaattagtt tccctccttt gtgatccaaa gataccttat
108780

ttaaattact tactgtactg cattgaaatg gtcagtttac ctgcccagct ccctcctcca
108840

accccacagc atccttccct ctctcactga tatccacaaa acccaaactg tgagcacctc
108900

taaggatctt tggatcctca gtgcctacta gctaacaagt gcctgaaaaa atatttatca
108960

aatttagaaa aagaatgagt aagtgaatga atgaatgaat gacttgaagg acatctgaac
109020

atctaggacc taagaatttt gatgaataag actcacgaca atcaaaggaa aaggaaggat
109080

atgaaaggat gaataagatt tcataaggtg ttaaaataca tactatatgt atttgttact
109140

taagggtccc aaatggctat aaataataaa tacagacaat atttattggt acaatggtaa
109200

gtgcattatt tacccaacag gttggaacta ctctttctaa gccactccta tcttgatagg
109260

tcaatatgtt ttttggcaca cgcaatatgg tagaaaatgt gaactatgta tctgtcaaac
109320

agtaacaaag cacagtttaa cctagtaagt ggtgtcaact ccttctgtgc ctctataatc
109380

catttatcta aatcctactt agttggagga ttatagttct catatagcta gatatgagtt
109440

cttcccatta ggtaaatggt tcacacatac agtaaacaag gctataaaaa atcatatcta
109500

atttcacaaa agaaaaaaat tatttgacat attttcctgg taagaaaaag atataaagga
109560

aggaaatcgg gatcatgcca aacaagtaat taaaaactgt ctaaagacag atcttttttt
109620

ggagtccctt aaaagagagg aactggctaa aaatttcgaa tgaaaccgcc aatagaaaaa
109680

agaaccacaa gaaacaaatc taaaatttca ctggaaacag gaggctgcaa agtaaaaggg
109740

aaaaatttaa ggaaacacaa gccacatctt cagacatcaa catgaaaaag gaatgctaaa
109800

atggtaattt ttttaaagtg gaagtgtcag aggcttttaa accagagcaa ctccgtcttg
109860

aataggggct gagtaaaata aggctttgac ctatgggggc tgcattccca ggaggttagg
109920

cattcttagt cacatgatga gataggaggt tggcacaagg tacagatcac aaagaccttg
109980

ctgataaaag gatgcaggaa agcaactggc caaaacccac caaaatcaag atggtgatga
110040

aaatgacctt tggttttcct cactgctcat tatatgctaa ttataacgca ttagcatgct
110100

aaaagacact cctaccagtg ccatgatagt ttacaaatgc catggcaaca tcaggaagtt
110160

accctatatg gcttaaaaag ggggagaaac cctcagttct gagaattgcc aaccccagtt
110220

caggaaaact catgaataat ccactgcctg tttagcatat aatcaagaag taacaataag
110280

tgtaagcagc tgagcagcct atgctctgct ttgcctatgg agtagtgatt cttttgtttc
110340

tttactttct taataaactt gctttcactt ccctctacgg attggcctca aattctttct
110400

tgcatgagat ccaagaaccc tctcctgggg tctggatcaa gacccccttc cagtaacaga
110460

aatgacagaa agggctaaat ggatcacaaa gtagttaaaa tgcaaattac aaacaatgtt
110520

actaccatta ggtctcatca aataagaata aaaatgaact caggaattca agattgcagt
110580

aagctatgat ggcaccactg cactccagct tgggtgacag agtgaggccc ccgcatctct
110640

aaaaagaaaa aaaaaaaaag gaatgtaact aatatctaaa taactctaac taaaatgttt
110700

tcattgaata tattagtgtt tgaagtaaat attaacacgc tttgggtttt ggggaaactc
110760

ttgctaatct ttgacctaac cattgttata tgccagtcag gactagaaat ggaggaaaca
110820

ctgacattag tattcattga ttcttaaaga aattagaaat gagaagccta attgaatcaa
110880

agtcacaagg atctgaaacc tccctcccct ggtctggtga aaaagaggat attttgtgga
110940

aggtagtatc tcgatctaaa ttcacaatat catacaaaac tatatttcta tgatctgtga
111000

taatcgtaac tgaacttgga ctctaaattt tctgacctac ttcactactg acccttagca
111060

gaagaaagat gcttgtttat agctgtttac agtttacact tcactgatgc cattactaca
111120

tctgaacata ctgtaaatta gtgatggaat gcaaactagc tactaaatta attaatttgt
111180

taatctcatt agccaaaagg taagaacaac ttccagcatt atgtaagtat ttcagactta
111240

tctgaaatat gacaatctta cctgtatgtt tttagaatgg gggaaaaaac tctaaaaact
111300

ataaataatg tcaaataaaa cataaatttc caaacaacac tgataacaat tccataggaa
111360

agcttacatt cagtgatact tttgcctttt tcttcttaaa gtgtcctttc tggaaatgaa
111420

cattatctat tttgatacaa cctttacttt ataatattca actcctagcc tgttttcccc
111480

agaataaaac aaaactcaga aagcttacaa tctttaaatt tcattatttg gtggctttat
111540

ttgttcaaat aacaatctta aaattatgtg aactacaact gagagattta ttaattaaca
111600

atgaagttat aaaatgtatc ctattatttc taaattaatg tagtgacaat agtggttgag
111660

aacatggact ctaaccatat tctctaggtt aaaatcccaa atttgagctg ggtacagcgg
111720

tgtgcacctg taatcccagc tacctgggag actgagacag gaagatcact tgagcccagg
111780

agtttgaggc tgcagggtgt gatgactaca cctgtgaaca gtcacagctc tccagcctag
111840

gcaccgagac aagaccctgt ttctaaaaac aaaggaaaaa caaattccta gatctgccat
111900

tttccaactc tgtgaccttg aacaagtcac ttaacctctc tgttctcagt tttcctaaaa
111960

gtcaaattaa tggtgctaat aatagtactt acagagttgt tgtgagctaa acaagttaac
112020

atagctaacg tacttagaca aagtgcttag aacggtgccc agtatatagt aaattctatg
112080

tacacgtttg ctgttattcc ttaaatctgt aaagggagct cagaaaggtg ctgttcgtat
112140

ttaattttat ctaattttat tttaaaatat ttaagaaaaa taatctattt tctgccattg
112200

aaactctcac aaccctatgt tcttaacttt tccagaaatt ctacaacagt aggctggata
112260

ataaatattc cctaaggtga aaggccacac agattaaagt tattttactc tcttttctaa
112320

ttcaatacaa cacaattgtt ttagctctga gggtattctg gtatttggag catttgtcaa
112380

ataatgaaat gaatcataaa ttagtcagat tataattgtt ataattctca gttattatta
112440

gtttgtcttc agtttactct gtactcaaaa gagggggaag ggatggccaa aaattctttt
112500

gcaatagtag ttgatataat tgaacaccaa cattaccttt atcacttcca taataataga
112560

ataccgtttt actgagagca caccaccgtt tctgccattc aaatcccaga aagctgtgat
112620

ctgcaataaa gaggggaaaa tgtaaggcca tttccttact gtaaaagaaa aagttaatta
112680

atatgatttc taagattcct ctgagctcta acattgtgtg actaatttgt atcttctttt
112740

ccaagagaca aaaaaccaaa caacaacaaa ccaacacagc taaagggaat aaagtaaagt
112800

agctatgggg ctgccatact tatgggatca acagcatggc taacaaagtt ttctgacagg
112860

gacagtcaaa aaggtatctg actatacagt cttgagataa ttgatccatc tgggtctcat
112920

tttctcatct gttataaaaa gaaggggagg agctaaccta gattatttct gttatctctt
112980

tcagttttaa aattcagcta agaaagctaa gaaaagcaaa atgaaatata aatagaaagg
113040

cctaaaggga ctacaagaga gataagcaga atggtaatga aaagactgtc tgcttttcat
113100

ttctcatatt ataaaaccac ctccatattt catttccatt aaatattggt gttgaaagag
113160

tgagaccata tggtcaactc ccaattatca tagccaatgg agaggaacac tgaaatgaat
113220

aatagacaat ggcagacaaa actccaggct tgatctgaac catggtgcat gccagtttgg
113280

gcataggaga acctgctgct cagcccattg agatactggt gagaaatccc acctttgaga
113340

atacagtcat ggcctgcagc tttcctttga gaggcaggaa acagctcctg aatcatttac
113400

aaataaccaa ggttgacaac taaaaaccta acctaatgtt ttcttattaa aaattattta
113460

aagctaagag ctatattttg tggtttggat aatgtcatcc agattttctt acaatcttta
113520

aactctttaa tcacctaaaa cgaatactgc ctccacatgt atactacaaa catgttctat
113580

ctaaggatga aattattttt acatttttca ttagaacctt cagtttacct tttctgcgtt
113640

tttcaaggta gccagccttt agaacaaaag gaaggtcttg tgctgcaatt ggaggaaact
113700

gggctcctat gagaagttgg ggagagaaaa aaaaaagcag tgggtaatcc atttcagaat
113760

aaacaagtgc cagatctcat tagattcagt gtgcttttaa ttactgaaat tgtttaaatc
113820

taagtctatt agctttgttt cagatccatt ccctagatta atcccctcta ttcctcctaa
113880

aagtctcaaa agtaaaaaaa aaaaaaaaaa aaaaaaagtt ctgtactcct atatgttatt
113940

atagcctccc aaaatacagg actgagaaac tattataaat aaatgcaata aatccctgaa
114000

agagaggtta agaatttcaa ataaatcata gcttcgttat atttatattg aattaactct
114060

ttaaaaattt caatacatat tgactctttc ctgcataatc cctattccta ccaagtaaaa
114120

cataaaatgc ttttccgaat caaatgctga aacttgtttc tctgagccca cagcaaatat
114180

tctctgtgtt tgtattcaga gaaatgttat ggacattcca ctgagtgagt atgctttagg
114240

tttttatagg gcatttaaga gtctaaaact agacagatct gataaaacta ttataaacaa
114300

atactggatc cctaaaaaaa gtttttaaat caagcaaaag ttaagttgat tagaaattgt
114360

atattcacgg tcatggatta taggttactc aattttacaa gtgttcaata tttttataaa
114420

cttcattaga aatataatag ttatctgccg ggcgcagtgg ctcacgcttg taatcccagc
114480

acttttggag gccgaggcgg gtggatcact tgaggacagg agttcgagac cagcctggcc
114540

aacacagtga aacctggtct ctactaaaac tacaaaagaa attagccagg catggtggcg
114600

ggtgcctgta atcccagcta cttgggagct gacgcaggag aatcactcga acccaggagg
114660

cagaggttgc agtgagccaa gatcacgcca ttgcaatcca gcctgggaaa aaaaagagaa
114720

aaaaacgaaa acagaaaaga aaagaaatat aatagttatc ctcagcattt tactagaact
114780

ctagcaagtg aaaacaacca atcaaaagaa agtttgtatg gaatacatcc agtggaaata
114840

gtttcagtac aacttaggat agaaaatgtg tttgagtaca aacatgtaat tagaatgaat
114900

aagatctagt atttgatagc acaacagagt gactatagtc aacaataatt tattgtacat
114960

ttaaaaataa ctaagtggcc gggtgcaatg gctcatgcct acaatcccag cactttggga
115020

ggctaaggaa ggagggtcgc ttgagcccag gagtttgaca ccagcttggg taacacagtg
115080

agacccccat ctctacgaaa aatttaaaaa taactgggtg tagtggtata cgcgcctgca
115140

gtcccagcca gtggaggagg agggtagagg tggggggaag ggggtctgag gtgggaggat
115200

cccccaagag gtcaaggctg cagtgagcca tgattgtgcc actgcgctcc agcctgggta
115260

atagagtgag accctgtctc taaataaata aataaataaa taaataaata aataaataaa
115320

ttaataaaat gaaataacta aacaaatata attggattct ttgtaacaga gaaagaataa
115380

atggttgagg tgatggatac ctcatttatc ctgatgtgat tatcatgcat tgtatgcctg
115440

tatcaaaaca tacaatatgc ccataaattt gcctattgtg tacccacaaa aattaaaaat
115500

taaaaaaaag aatgggtttg ataggaagtt tattatttac aaactcatca cctaatttta
115560

ccagtttttt tttttaatac catggggatc acagtagtga taaagtcatg gatatttttt
115620

cttaagaaat aaagcttaaa attagagcac taatttcagc tccttggtgg atcaacttca
115680

cccagtaaac accacacagt atcattaaaa catatttaaa atagagccct gattttgaaa
115740

tgtatatgaa attttaggga aagtcagtgt acatatttta atagctgttc aataaatgta
115800

tccagttttt ttcaaaataa acttggtaca tataaaggta taaattattt attataggat
115860

tttgtacata aaacaaaaat tacaaacaga aatcttatct ctgtaaacaa gtcctggtga
115920

accactaatc taaagtggat aattgtggaa tgatctggtc tcattaaggg gtagtcccac
115980

aaccctctca cttccattgg gcataataaa tgctctttac ataaggaatg gatgctcaca
116040

aaatcttaag aaaagttaaa ttattctgaa aagaaatggg tacctttagc atagctttca
116100

actctaaaat gttactggtc aaaaaggaga cagagagaga tgggaagaag ggagagggga
116160

gaggaagtgg gggaaaagga aaaggagaag ggaaattaga atttactagc gttcaatata
116220

cacatagcct gagtcttttg ctttaagaaa agtgttgact aaatttccac ctgatacatc
116280

acaataaaat ttgatgtcat aagcaatact tgttctgaat tctaaattca tgtttctaat
116340

ataaaatctt tgatttcttc cctggggagt gggggagtac atttttttta actgaaattt
116400

catttttcta aacctttatt gctgtttgtc caatatccag gatttgctta atagccatat
116460

gaattcttta aaacacaaaa gtacactaat agcaatccct gtaaacactt tagattttta
116520

aattttcaga agaaccattt ggtttgtata tcatcatgag catatggaca atactttctg
116580

atggcaggga tggaaaatct gttataatat ataaaaaagt agtaatttcc ttttaaaaaa
116640

atatgaattc tcatatcatt tagacctcat atcactatac ctacaacgat taacgctatt
116700

gacatttagg aagatttaga ttggtacaaa gaaacaagac tataaaaata catgcataat
116760

gatacatgaa gaaaaggaaa ttaaggctaa tgtaagccta tctgccaggc aacaacaaag
116820

agctgccaca tgtttgccat acaccgcaat tacgttatct cactatggaa cagtcaatta
116880

tacaacattc tggtgaattc acaatcacat tctcagcttc actattaaaa ttcaaacctt
116940

gtcctaaaaa aaagttttgc actgggtctt agtcctttgc acatagtagg cattcagtaa
117000

acgttagctt tcattaccta ttacagttaa atattgttat atgttactat aactgtaact
117060

actagttcaa gtgaatggat gctaatgtaa acgtaagact atgttgctca gagaaagtta
117120

atcagaaatg aaatacagga aaaagcatga ttcagcaact atacaagtta agcattgtag
117180

ctacttctaa agcagttaaa agatttccct ttacttaaat gaaagtggtt aatgaaaaaa
117240

gtatatccta aggggagaac aaaaattaac ccatatgtta gggactagac tatgctacag
117300

gaatttatta tatgggcttg cttaagtggc cacaaacttt cagtgcccga aaaagtaagg
117360

tatatactga gaaagactag ttttcttttt ataaaatgag tacgtatttc ttatatgata
117420

aacctacttt tctaaagcag ctcaaaatat tagacattaa aatcgacacc ctacaaggac
117480

aactaagcaa ggaatgggtc ttcttccagc atgcaatttg agaatctgaa gcattcaaaa
117540

tggtaacaag gtcacaatga attcaaataa acaagtatca agtgcctata ttcccagcaa
117600

ttcaggaaaa aaggcaatag tgaaatccaa atttccctat agttaggttg atattttatt
117660

cataaggtaa ctgattctaa aagataactc aagaattcta gaatgtgcct taaagctttt
117720

gctttaaaaa caaatggtgt atattgtaga catagtacag caagcttcaa gtatggatac
117780

tagaatttca aaagcagaga agagatacaa agtatacaaa ttgataacaa attaccttaa
117840

aactaaggta ttacccactc ctggtgattt tattaaacta ttactttatc tttttttttc
117900

ttgaatctag gatgaaaaac actggaatgt ttaaatgcct gtaatatact taagaaataa
117960

gttttatctt ttaaaaagtg ttaacaatta aaaataatta tttcaaaatg aaaaatagta
118020

gttacttcaa tttcagagaa aaatatgaag tctgtggtag gaagtatcac ttagtgacat
118080

ttcaggtgtt aggttggtca ggtctaaacc cagtcttggc attaacaggg tagacaattg
118140

cattactgta tttaggacaa agaaaagtag aaaaatgtgt ctagatcaca gaagcttcac
118200

tcagaagaca aggcctagaa cagcacgcaa gtcccttgag taggcaaggg aatgggggtg
118260

agagagagtt agagttgggg gagagagaga aagagctaga gttgaggaga atgggagatc
118320

taatggtcct ttctttttta tttagatttc tcaaatctaa atgaacaaat gttgattcac
118380

aattttgtcc agtattgggt gcctcaatac actatattcc agggtcttcc ctaacattga
118440

tgtgtctcct aatagactcc tccctgatac atgttacaca tacacacaca cacacaaaca
118500

cacacacaca ctatatccca gtctgatacc tctttaaatg cattaatttt tctaagacaa
118560

tataagacat cattaccaaa gtgcaatgac tcagagacat tttcaaaata gacatgtttt
118620

tattatcatt tttatttttt gatatgcaac tttggtgatc aatttaaatg ctagacttct
118680

aaggaatcca aagccttatt tttactattt tatcatcaat aattcatcat attattggct
118740

tcattacact aatatgttca cacttttttg gttgtttaat ctagacttta tgcaaaatta
118800

cgactaggtt tctgagcttc accttttgcc cccttcaatt ttcccttttc tagttggcat
118860

ttatatagtt ttcaacatga acaccaagct ttcacatagg gtaatttttc ctaccaccta
118920

cccgactaaa gtttcctttt cccttatatt ggaaaagaaa ggaaagatac tttttaaaag
118980

actgttgtta ttcctaagaa ttttcactct gagtcaagat gttggtccat ttagtcaaat
119040

attctgtgtc accacatgat gtgagttttc cctgaactcc aaaaagtaaa gaataaactt
119100

ctcaaccata caaccatatc ttttttcctg acaattcaaa taaataaagc atctacaagt
119160

ttatctaaac cttttaaaat tattcatttt cacctaatag ttgggatact gagttccata
119220

aattggccac ctcctggggg gagatagagc ttcgttttat ctcttctaaa ctcatgtagt
119280

aaactaccaa aaatgcagcc caggctggag tgcagtggca tgattttgtt actgcagcct
119340

ctgccttctg ggctcaagcg atcctcccac ctcagcctcc tgagtagctg ggattatagg
119400

tacacaacac catgcctggc taatttttgt atgttctgta gaggcaaggt ttcgccatgt
119460

tgcccaggct gatctcaaat tactgggatc aagtgatctg cctgccttgg cctcccaaag
119520

tgctggtatt ataggcatga gccaccatgc ctggataata gacccatttt ttaaaaaaag
119580

tatttaccca cttaatgatt gctttatatt tgaaacatat attaggcttt gattctccaa
119640

aaatattatc tcatttcatg aaggtgatac tctcacctat ccactagtgg ttattttatt
119700

atctagatta gcttctccag ttctagttca tttttcttaa gaagtgataa acagaatgaa
119760

catacatttt caggtgccag tatgtcataa taccgatata atcccaatgc actgtaattt
119820

tgtataaagg tggagaaatg ctttctgttt tgcttccact atccttccag ttgaggtcca
119880

ctatcttagt gacatttttg gttgaagggt acatttagta cttagcttta ggaaatagtc
119940

cattaaaaag cctctgttcc cagagctgtg actgactcag aactcatcat tccacaagtg
120000

cagtttaatt acactttact ttataattgc acacataaac ttagaagcta ttttccctgt
120060

ttctagaaat tatgtaatag atacaaagat gcagggatgc agacactttg tttcttattc
120120

taataggttt taagtataaa aattcactag catatctgca aagtaaacaa agaaccaaaa
120180

caaaataaga ctatgcacca acaaaggaga gaaacctaca cttaacttgg atataaatat
120240

taaataaaat attttagtgg tgtggcacat atccagaagc ttagctgtac attgatactg
120300

acttcgaaat ccatggcaca ggttaaagaa gtctgtaatg tgatctcagt tccatacctt
120360

tttttttttt tttttttacc ttaaacatgt cactgagcct ctctggactc aatttcttct
120420

tttaaaacct gaaaaaaata ggttcaatca tctccaaagc cctcctaatt ccaggacttc
120480

atgaaatata tccacactac tatttttctt gtactttcag tagcaaatgg ccaaggtcca
120540

agattagatt gaagtttcaa ttatatcaaa tgtggaaatt gctcacaaca tattttttgt
120600

agaacaacta gtaacttctt ttacatctag catagaacta ttttatgtca tttaaaaggt
120660

ttaatattca gctagaacct tggcctggaa tatgtatgag agaaataaat acataaatta
120720

aatatttttc atatttgaat aattcttccc tccatgtggg aaatttggaa tttcaaatct
120780

gtttatcagt atttttagta gcaaaaggaa gatattatat attcaacagg cacctcaaac
120840

aataatcact gccgtaactc taaaagagga ctaattgtgt tactttagca tagtcttttc
120900

aaaaagcttg ttttctcatc gtagcactat tactttcttc agaagagatt caccaatgaa
120960

tatactagta ctggcgatct ctaagcctat tatgatattt cttttaagaa aaacaaaaac
121020

aaaaaagctc acaccatgct ttgacataaa gatggtcaac tttcaattag tagaaccata
121080

gaattctcag gagggttatg ctttggggaa aaggctttaa aaactgatat agcagcctat
121140

gtgactacac tggggaaaaa gcagaggtta caactcaagg gtttaacatc tgctaatctc
121200

ctccctcctc tgtaaagtgg ccattttgaa ggctaccaag caaaaacgga ataaccatga
121260

ggataattca aatttgcctt tattaactct aaaggagtta gggtggaaat tttcactatg
121320

ccttcaatag gtctatttag aacaaaagaa gacagtaact attaatatca tagctacatg
121380

catgtaagat atgctcatga atataatcca aaagattaag catttgttca gagtccaatc
121440

aaaagcttct attttacatc tcttccttcc tcctctcctc tcctgctcct cccttctctt
121500

cttctcacct tccttttccc cctcttccct cccctccgct cccctttctc ctccctctcc
121560

tctcttctcc tctcctttcc ctttttttct tccttccttt ctttcttgct gctactaatt
121620

tccatagaaa tctaccaaac tatgaaaaat tctaatggca agattccaga ataatgaaat
121680

ctgaaatcaa ttgtgttcac caacatccat taaaataatg ctcaaaaagt cacagatgaa
121740

tagtatatgt gaattatatg acacggagtc caattatcct tctccactca tttaaaactg
121800

cttcttttac tttaacatac atttaagtat acgaccagaa actgaactat cttattacat
121860

atctgaagac tgcttcttta gataaatctt taatatagaa ctgctgtccc tttctcataa
121920

aagtagatgt gaaaaaaatc tctttaagat ttctgattcg ttggtctaac aataaaccac
121980

tagttatcac attgttaaaa tatattaaac ttgttcctta tagaatttta aaatatggtt
122040

tttatctaaa aattctagag ttattaattt gtggaaaacc ttataagaat ctctcaatta
122100

tttccgggga agatagcata tcaatatact gaatcaatca tttaaaagta aggttttact
122160

ttagagacaa gactactagt ttatatattt ataatatagg gcaccctctg tgtttttcta
122220

ttttagtaaa gggatcttta gattccacat aaaaaattta aatcaaattt tgcctgccac
122280

ataaagtaac tcctcagcaa ctctttatct gcctatatct tagtcatctg ctctgtagca
122340

agtaaaacat taaattctga aaaataatag cctttttaaa aagtctataa tacatacttt
122400

gagaaaatga gatgcaagct taaaatggaa tacattctcc ttaagttaaa aatataattc
122460

taaatatatc aaaatgttca gactctaaat attataaaat taaatatata caattatgaa
122520

ctgggtttta atttaatgca tctctttcca caacaaaagt gcccccaaac tgatactgca
122580

ttttagaatc acgttcaaaa ccctggattt acattcagat tgcaggatag ctaccagctc
122640

tgtgacttag caagtcactt gatttcatta agctttagtt ttcaaatctt tcaatgtaga
122700

taataggagg gcatactatg gagagctatg gtggggatgt agtaggatag cacttaagat
122760

aactctttaa gaaatgttgt ctcttattac tactagtatt tttaaaaggc aactcaaatt
122820

tgccacaagt ccattgttcc atacacatac aaaaaaagga ggctgaaaat ataaaccact
122880

agaataaaaa catgaatgtt aaacaccttt taaccatacg actttctttc tggttacaga
122940

tatttggcca aaaacaaatc tgatgcataa acacatattt gacaagatta tacataataa
123000

agattagcat aaatgaaaag gaacgttctt aactcatata ttaaacaata ttttactagt
123060

ctatattatg cgccaggctc ttttctaaat attacccaaa atagaaaact aggaaacata
123120

aactcaactt cactgaaaat ttgttgaagg catcttaaaa gtcaaacaat tatgtataaa
123180

atttaaacaa taacaataat aggtcctcgg ctgggggtag tgattcacac ctgtaattct
123240

agcactttgg gaggctgagg cagaaggatc acttgaggcc aggtgttcga gacagcctag
123300

gtaacacagc aacaccccat ctctataaaa aatgtattaa ttacccaggc atggtaactc
123360

acacctgtag tcctagctac tcagaaggca ggaggatagt ttgagtccag gagttcaagg
123420

ctgcatatga tagtgccact gcactccagc ctgggtgaca gagtgagacc ctgtctcaaa
123480

taataacaat aataataata ataataataa taataataat aataataggt cctacttctt
123540

tcttccttac ctatgctatc tctgtatcaa acacaactgt ttctattatc tccatactat
123600

aagcttaaat atgtttttta agcactatca gtggcatgat tacaaataac agcactgcaa
123660

cagttaacat ttattgagcc tttaccatga gttaggtaaa gatgatgaca ccagtcatca
123720

tctcatgtca tcatcacata accctaagag gttaagtatt actaactcta tattaccaat
123780

cagaaatcaa gataacacac aataagtgga gcaaggacct taagtctact tgcccagaag
123840

cccattttct gaaatgctat gttccactgc tgatggaata aatgaatgcc atctatagaa
123900

accgtgtcag acaacatcac tgaattactt ttctatggca ttaaagcaat taccattatt
123960

taaggttagt tccctaagtg taacatgata tattttaata taccatatat accatatacc
124020

taatatacct aatagatata cctaatatac catacttttt tttttttttt tgagatggag
124080

tcttgctctt tcacccagga tggagtgcag tggcgtgatc ttggctcgct gcaacctcca
124140

accaccaggt tcaacgattt ccctgtctca gtctcccaag tagctgggat tacaggcatg
124200

cgctaccatg cccaactaat tttgtatctt gtagtagaga tggggtttca ccatgttggc
124260

caggctggtc tcaaactcct gacctcagct gatccaccca ccttggcctc ccaaagtgct
124320

aggattatag gtgtgagcca ctgcacacgg ccaatatacc atacatttaa attgtggggt
124380

gttgtttcat ctgtaagatt ttgggtcaat tttgcttctt aagaatgtat ttgaaatact
124440

tccattttgg tttaaaaaca catatacaca aacatggaac aaattagtaa tggggaaaat
124500

gatgtttatg gaaggaaaag gaggttcctt ttgtatgcag attcctctag caaactgcat
124560

agaaataaga aatgaatata atttagattg aattctttag cttctttcca ctcaactatg
124620

ctttatcttt acataggcca taggacaatt ctttcattga tttacttact tttgcaacag
124680

ctttatgttg tggattttga ggtataaatt tttaaaatta ttttcagatt ataagtaaca
124740

tttttataat ttgtgatatt ctttaaactc atttttatta atttattttt cctattctgt
124800

atacaaaaag tttagagtca ttaataacat aagattatat cttaagtatc tgaaatagga
124860

atatgcagag ttataatgtc ttctacattg tacagtataa taaaacacgt ttttgttttt
124920

gtcttaagaa aaataacagg tacataaaca gttgtcattc tattcccatc tcccaaaaca
124980

accttattat tgaggggtgg ttaatatgaa taaataaaca agaaaaataa gtgacaaatg
125040

tgtagtttga aaactatcag acatgaaatt attttatgac taacttatta acagaaggta
125100

aattgctgaa ttaaggctct cactctattt cacagtccta ctctgattat ttgattacta
125160

aattgagctt gattactaac gcaaatacgt tcccaaacat tcttaatgct ttagtcttca
125220

taggtaaata gtatacctgt ttctatttaa gaaggaaact atctaatttt ttatttaaat
125280

caagaagtct tggcattttc cctcttctgt cattcaaagt acctactctg gtttaaagta
125340

ttataagcat cttcctaaaa acacatgtgt atgcaatctt tctcccctct ctcttacaga
125400

cacacacacc atttgttttc cttccaccta aaacaagagg tttttccata actctttttt
125460

caatatagat atgaaattca tacattctta tctattatta ttgttaattt agtaatatac
125520

tgcctccttc tccccccaaa aagcatttcc caggcatttg tacagataca tttggagagg
125580

caaattaaaa gcaaacagga gctatcagtg tgtatacaca cagggtggga gggaggggga
125640

ggatggaaga tggagaagaa aggaagggta aggaaaggga ggaggcagag tgacaaagca
125700

aatacctgta tagaaaaatg ttaatgttag gagaattggg gtgaaaggta tatgggaact
125760

ctttgtgtta ttctcgcaac ttttctgtgt cactatttca atatttttta aaatcaaggc
125820

aaaattagaa gtcagggtgg aacagaagac aacttggaac tacaaagtca tttacacttg
125880

tgaggagtgg gccaaaaatt cacttataag ctttataaca accaacatga agcaagaaat
125940

acaataaatt atatgagcca ttgcccataa gataaataca tatcattgtt cagggaagaa
126000

aacacaacta tttctggctc ctggtactga aatgggaaag aaatttctcc catggttctc
126060

aatgtcctca ataacatttt tacagtatat aaaataaaag tttcataggg ctgttttgta
126120

taacatcatt catattgagc caaaagctta atgccaaagt gaaattcagt aaaagcaatt
126180

ctacagaata caaaacgagg aggcccaagt atacagctaa ctcccaaaag ctttgttaaa
126240

ctcaaggata agatcataga gtatctatag gaacagacac cctgcacaat tttcagacaa
126300

tcattccaaa atcctgtttg ccttgatcag acttctggta aatattaaat ctcaagaaaa
126360

actccattct ctgcaagaag gtaacacaac tctttttatg ttgctgctta agtcaagagc
126420

catatccttt aacaagcagc caggctgggg gtaaaattaa tacacttatt tatcaattta
126480

caaagtttaa tgactttgca gggacataaa cctggatgaa gatcatctta acacaaaaac
126540

aaatcagcaa ctggcctata aagctctaac tcagtgccag gacaattcca gcctgtgaaa
126600

ttttaagcat atacctactt tgtatccacc aaatatccac agaacaacat gttactgaga
126660

atgataaaat gcctgagtct actcttcagt atgaactggt aatcatatgt acacctgggt
126720

aactgaaagg attataaagc attgttatcc atataactga gtaaattact agagaattca
126780

tactacaatt tcaatattag aacagttaat gtgtagcagt ttttcttctt actatggaaa
126840

taaaagcata ccacatgctt aatccttttt ctgataccta gtttcaaagg ggaataactg
126900

taaacagcca tttgtgagac tggccaggga gacagacata gacattcatg tggtatttac
126960

agtatcaggc taaaatagtg tgtcacaaga gtgtcttttt cactggaatt tgagcaaggg
127020

gatcataagg cagtgttctg aaatttgagt gcacatggaa atcaccaagg gtacttcttt
127080

caaatgtaga agtataattt taaacaaaat catctatatg gctcagaatg gtatatttaa
127140

aaaccttgtt ttgtgtacag gatttgtaaa atatggtcta agctaacgtt cactgcaaaa
127200

taaaaaaatc aatatacaga taaaacacag ttctgcaatc agtacacact gaaaacagaa
127260

ggaaaatccc caaaatataa atgatagtgc aactaatgat ttttatttat aactacctaa
127320

ttttggggtc attaaaacaa ttttgtaaac atttaataca cctttcacag tagtcttgag
127380

agagaaagag aggctgtcat aataaaaagc agtactgagc agccagttgc ctgcatgtgt
127440

tggttcattc atcaggcagt taccgagcat ccactatgtg ccaggcactg tgatggagca
127500

agaggtacag ctacagtcct tgcctttgag aagctcatga ttaggtgcaa ttccccacta
127560

ataaagagat tcaagaaacc aatgaggaga cagaagaatg agaagcaaga tagccgtaag
127620

acatgaagaa caaagaggtc atccttatat ttatatccca ccttaatatc tactttcaca
127680

attactaaca ctcccattca gttctaaact ttataaccac caacatgaag aaacataata
127740

aaaaggaaga aaattgggac agaacgtata atataaaaag tgcctattta gctatgtgat
127800

tttagcatat ttttaaaaaa gtaagccggg cacggtggct catgtctgtc attccagcac
127860

tttgggaggc tgaggcgagt ggatcacttg aggtcaagag tttgagacca acccagccaa
127920

gatggtgaaa ccccttctcc actgaaaata caaaaattag cagggcgtgg tggcgtacac
127980

ctgcaatccc agccactctg gaggctgagg tgggagaatt gcttgaatac aggaggcaga
128040

cgttgcagca agccaagatc acaccacaga actccagtct gggcaacaca gtgagactcc
128100

gtcacacaca cacacacaca cacaaaaagt attcaagatg tgaaaaaaaa aaaaaaaccc
128160

aactgacaaa tatagaaact gatatttact ataagcaaga catcaaaagg caattcatta
128220

atatgttcac tgtgtgggct atctcactgc aggatctgcc agagctgaca tagcccaatc
128280

gtacatggtc tctttttctc ccaactaaga gttgtcatta ccagaacaat ttcaccaaga
128340

aattttaatg atttagttca aattagaaac gtcaaaatta aactttgaaa aagaaactga
128400

tggaatattg agcagaacaa aacgattgct aacaacacac aacaactgat gtaacttcat
128460

gtaaacaacc agtgctgttc agccacactt aaacatcaca tcaagagcag ttcagtgctc
128520

cctcatttgc cctgtaaggc actggaagtt acctcagagg aggctagggc aataaaatgg
128580

tctgaactct gaatctgtag tttgctcata aatatgtgaa atatacaatg acctcttgaa
128640

aatatgaaaa agacttaaaa tccaaaagat acacatcccg ctaaaattcc atatgaataa
128700

atggcataac acaagttatt cctagtcaat ttagatatgg taagatctta taataagata
128760

tcctattaca tgtttctcta agaattcagc tgtaattaaa tcatgtctga atagaccatg
128820

atgtacattc tcactgaaaa acaaaattta caaatatgtt ttatctacct taatattgat
128880

aacaagttat caattaaatt aattcaatta agtccccttc acctggatag cttcaagtct
128940

cacacaagac tcatggttct aatgtacttt tacaaataca aaaataattt ttaagaattc
129000

ctcagaggat gctgaaatat ggaaatttaa gacttagtcc aattctttct caccaaccaa
129060

agaagtcagg tatactgtta taattccata catggataag aactggtaca gtcattaact
129120

tgtcaaatga tgacttattc aaggtgtatt tataggagaa aacatttgca tggatgtcac
129180

aatagataat gaactacaaa ttaataagaa aaaagtgaac aggtcaatag aaaaacaggt
129240

aaaggatatg aacccagtaa tttatggaat aagtacaaag gttaataaac atgaaaagag
129300

atgagtgttt attgatttgt agtgtttcct gagctctccc cacctccatt agaatataag
129360

ttccaacagc ccagagacca taactgtctt ctcttcactg ctgtatccct agggcctaga
129420

ttagtgcctg ggacacagga agtgtttgtt aaatttgctg aatgaacaaa caaatgaata
129480

aacctcacta aaaactactt tttaacttat aggactggca aaaactgcaa agattaagaa
129540

tatcaaacag ggaggagggc aagaagagat actcgcatct ccactcaccc cgttttgggg
129600

agtatagctt aatacagctt tgtggatgaa aatttgacaa taccctgata attcaactcc
129660

taagaatcta ccctacaaat taaaatgctg ttacatgatg gtgtataagc cagaatattc
129720

actgatatac catttgtgac aacagaaaga tggaaaatgc ccagcaagag gaacattatg
129780

aagccattct aaataatgct atagatctgt gtgtattaat atggaaagat atatactaat
129840

gtagaaagat gtccacgata cttggttgaa aaacacaagt ggtatactaa tatatagtga
129900

tatacttttt taatactttt tgttaatttt ttttattata ctttaagttc gggtacaggt
129960

gcacaacgtg caggtttgtt acatatgtaa acatctgcca tgctggtgtg ctgcacccat
130020

taactcatca tttacattag gtatatctcc taatgctatc cctcccacct cctcccgccc
130080

cacaacaggt cccggtgtgt gatgttcccc ttcctgtgtc caagtgttct cattgttcaa
130140

ttctcaccta tgagtgagaa catgtggtgt ttggtttttt gtccttgtga tagtttgctg
130200

agaatgatgg tttccagctt catccatgtc cctacaaagg acatgaactc atcctttttt
130260

atggctgcat agtattccat ggtatatata tgtgccacat tttcttaatc cagtctatca
130320

tcgatggaca tttgcattgg ttccaagtct ttgctattgt gagtagtgcc gcaataaaca
130380

tacacgtgca tgtgtcttta tagcagcatg atttataatc ctttgggtat atacccagta
130440

atgggatggc tgagtcaaat ggtatttcta gttctagatc cttgaggaat caccacactg
130500

tcttccacaa tggttgaact agtttacagt cccaccaaca gtgtaaaagt gttcctattt
130560

ctccatatcc tctccagcac ctgtagtttc ctgacttttt aatgatcacc attctaactg
130620

gtgtgagatg gtatctcatt gtggtttcga tttgcatttc tctgatggcc agtgatgatg
130680

agcatttttt cacgtgtgtg ctggctgcat aaatgtcttc ttttgagaag tgtctgttca
130740

tacccttcac ccactttttg atggggttgt ttttttcttg tgaatttgag ttctttgtaa
130800

attctggata ttagcccttt gtcggatgag cagattgcaa aaattttctc ccattctgta
130860

ggttgcctgt tcactctgat ggtagtttct tttgctgtgc agaagctctt gagtttaatt
130920

agatcccatt tgtcaatttt gtcttttgtt gccattgctt ttggtgtttt agacatgaag
130980

tccttgccca tgcctatgtc ctgaatggta ttgcctaggt tttcttctag ggtttttatg
131040

gttttaggtc taacatttaa gtctttactc catcttgaat taatttttgt acaaggtgta
131100

aggaagggat ccagtttcag ctttctacat atggctggcc agttttccca gcaccattta
131160

ttaaataggg aatcctttcc ccatttcttg tttttgtcag gtttgtcaaa gatcagacgg
131220

ttgcagatgt gtggtattat ttctgagcgc tctgttctgt tccattggtc tatatctctg
131280

tttttggtac cagtaccatg ctgttttggt tactgtagcc ttgtagtata gtttgaagtc
131340

aggtagcgtg atgcctccag ctttgttctt ttggcttagg attgacttgg caatgcgggc
131400

tcttttttgg ttccatatga actttaaagt agttttttcc aattctgtga agaaagtcat
131460

tggtagcttg atggggatgg cattgaatct atttttaaaa atgccagtat ggccattttt
131520

tgagatggag tcttgctctg tcacccaggc tggagtgcag tggtgcaatc ttggctcact
131580

gcaacctctg cctcccaggt tcaagtgatt ctcctgcttc agcttcccaa gtagctggga
131640

ctacaggcac gtgccaccac gcctggctaa ttttttgtat ttttaataga gacagggttt
131700

caccgtgtta gccaggatgg tctcgatctc ctgacctcat gatccaccca cctcggcctc
131760

ccaaagtgct gggattacgg gcgtaagcca ctgcatccag catgatacac tattttttaa
131820

aatttaagaa atatctggtt ccatgttttt tctagcacat atatgtattc ttaaaactca
131880

ggaaaaaagt ctggaagggc acatagaact gttagcagtt cttacttcta gggagtgtag
131940

ggaatttttt ttttcctttt gcagagaggg aacaacttcg ttataagcat gtactataca
132000

tttaaaattt tatataatac aatttttaaa actgtaaaac aaaaaatagg cttttaagta
132060

atttaagtcg atttatatta ttaaaatcat agccagaaaa atgccaccca ttcatctata
132120

aaatttaatt taatcaatac aagtatagtt ttatagtaca atgatacaat ttgaaaacaa
132180

tattcccaat atatatgaag gtactacttt gagaatgttc acctacctgg ctattcttca
132240

aatagtccaa gtctcatgta cccacacaag ttttcactct gcttgaaatg tgctattccc
132300

tcacctttct ccacttaccc actcctcacc actatcattg cctagaaagc tttcagctta
132360

ccctttaaga tacaactcaa ctcactgaaa agctttcctg ttgtgacata aagaaaacat
132420

ttgcaagaca gatgatttct taaaaaaatt aaaggaacat attttctctg atatagataa
132480

ttctaattct tgctactaaa tttttagagt aataaaaagt tacctaagtg aaaataaaat
132540

agccattaac ttttaattac ccagatattt ctaccttttc caaaaatctc catgtgctta
132600

cagaaacata gccaaaggct tcaaaacaat tcctgatctg ggaaaaatac aaacggattt
132660

ttggacgtcc acatgaacta gacatgttct tgtttagaaa gctggcattt atgacaaatt
132720

ttccgaaatg aaatgaatct aaatttgttg atattacaaa aataacacaa taatgtattt
132780

ttgctctatg tcaacttaaa tgtcttcaaa ctgatgtaaa aatatgcata aaaataactg
132840

tgccaggtaa taactagtat tccaaattcc acctggggaa atgaatttta tggctatact
132900

ataaacttct aaaatcctga agttttaata gatatactga cacaacctta accataacag
132960

ctctagcaac tgctgctata atcaatctgt cagtttctat aatagctatg acaaatccat
133020

taatatgaat gatgtacact tcacatagcg cactacacag caaccgcaca agttatgtca
133080

tctccattct gagtagcaaa tcagtaatca atatttgtaa aagcagcagc taatcctttg
133140

taaaatcact ggttcagtga ttgaggagtt tgctcaatcc acacagttaa tccctcttca
133200

tctgcttgag ccctctcttt atcccagcct cttacaatgc tagctatggc agattaatac
133260

atctccacat gctaaacatg acctgacaat tgacacagga tattgctgac aggataagct
133320

ctacaaaccc tggcctttta aaagggaagc ttaaaattaa cattcagaaa cccaaagttt
133380

acaaacagac tcaccactat cccttctcaa ctccagttat ctgatttatt aagctcaccc
133440

tccttgggaa atagatc
133457

<210>  164
<211>  100097
<212>  DNA
<213>  Homo sapiens

<400> 164
gatctcggct cactgcaagc tccgcctccc aggttgacgc cattctcctg cctcagcctc
60

ctgagtagct gggactacag gcgcccgcca ccacgcccgg ctaatttttt gtatttttag
120

tgtagacggg gtttcaccat gttagccagg atagtctcga tctcctgacc tcgtgatcca
180

cccgcctcgg cctcccaaag tgctgggatt acaggcgtga gccaccacgc ctggcctacg
240

cccgggtaac ttttgtattt ttttagtaga cacggggttt caccatgttg gccagactgc
300

tctcaaactc ctgacctcag gtgatccacc tgccctggcc tcccaaagtg atgggattac
360

aggcatgagc cactgcaccc ggcctgactt cacaatatta attggacata aaataggaga
420

aaaacaaggg gaaaaactgg gacaacagga agatctgttg tcctagtttt ggaacaacaa
480

aaatttggaa aatgagagcg caggcaaaga gtggagagta aggggaaatg acaacagagg
540

gagcatgggg agaggatgga agaaacagac acaaggggaa tgagcagagg gaagacaaca
600

aatcacagca gaagggaagg gatgagaagc aggagccttc agaggtcaca ggtctactac
660

tggatcacct gataattaat tggaaggttt atgtatacat ttaaaacatc atgttccatt
720

tctaacatta ttcataagtt caaaaaaaga acaggtcttt catagaaaat gattagtggt
780

attagcagaa agaaaggaat agtaaatatt tctccaattc tgtgagtttt ctatcaacaa
840

gctgatgatt aacaaacaag aaactttgtt ttgtttttgt ttttgagaca gggtcttgtt
900

ctgtcagcca ggctggagta cagtggcatg agcacagctc actgcagcct cgatctcctg
960

ggctcaagtg atcctcctgc ctcagcctcc caagtagctg ggactatagg cctgcaccac
1020

cccgcctggc taatttttaa atttttttgt agagacagtg gtcttgctat attgcccatg
1080

ctggtcctga actcctggct tcaagcgatc ctcttgccac agcctcctcg agtgttggga
1140

ttacaggtgt gagccattgt gcctggtcaa ggaacttgtt ttgaacctgg caggctacag
1200

gaatcttcag acacaggaaa tagtttctat ccaacaagga gaggaacaca gagctgacag
1260

tgctcaagta acagaggagt aggtggaaaa aagtggttac gacattagta aaaataatta
1320

aacccttcac tgactgacac agaataactc agaacaaaaa agaaacagaa aacaatgctg
1380

gagaccaggt gcagtggctc actcctgtta aatcccagaa ctccaggaag tggagacagg
1440

tggatcactt tagcccaagg gttttgagac cagcctaggc aacatggcga aacctcttct
1500

ctacagaaca tgtaaaaatt agctgggcat ggtgatgcat gcctgtagtc ccagctactt
1560

gggaggctga ggtgggagga ttgcttgagc ctgagaggtc gaggctgcag tgagctgtga
1620

tcgcagcact gcatgccagc ctgggaaaca gcaagaccct gtctcaaaaa aaaaaaaaaa
1680

aaaaaaaaat catctagtac aaagagcaag taccaagaga aattataagc cccatttatg
1740

cctgatcaaa aatagaactt gatgtttcat tagtaacatc cttcatgttc acgctttatt
1800

cacactccct ttgccctctc caggtagaag cttgctgtgg acctccctat tgctgtactc
1860

acgtgagggc ttctttgcag cactgtcctg aggggttaag atccagcaga gacaaatgga
1920

aatgtctgcc aacagtgtca ccttgtctta tctggctgat gcctcttgaa cttcctcgtt
1980

cttactacac atccaggacc cagagctctt catagaaaat caccattaag tttggaagct
2040

gggaatcctc atcacaagga cagtccaggt gcaagttcgt ggaattacac aaagcagtgt
2100

attggaatca cactaatcac acaggatgga agaagacaga aagccaacag aaggatgctt
2160

ggtagtttcg ggaatgtggt accgggacaa tgggttggga ttagacggat gatcagttcg
2220

cctggtcatc tctgctagga aagatattcc cccgacagag gcacctaaat tgtaatccat
2280

gggaacggag cagaactgcc tcccaggcaa ctgagtggct gaagaggtga ggacttggaa
2340

ctcaattatt tctgatttta aagagcaaaa actacttagc aataaaaaac ccacctggaa
2400

tcagggtcac agtaccatgc ttctggagtt aaatgaacac aaacattctc actttccatc
2460

tggtctacaa aaggggatag gtagtctcta actcctcaac actcaattga acacatcaga
2520

gatcccacag ctaacacaca gagagacaaa accatcttat cagacagtaa gcctgcatcc
2580

gtcttaccct ggggaaatgc gctcccataa ttctcctgcc tgttgtccct actgagattc
2640

ctccgagcca gattctgaca tccccattcc tccagaatga gggacacagc catgtcctcg
2700

atcttcacca ttgcctaaaa taacacgaga caaacagtca tttcccccca aactcaggga
2760

caatcccatt gctcaaacct ggagtcagga agagaggtgg ctgcaaagct acaggacgct
2820

gggaagaaaa gggtggccag aacctggggg tggaggggag tttgtggtag aaagatgcta
2880

aaaggagagg acaggttggg ggcttctttg agagcagggg catccagagt ggcctgtgga
2940

gataatgaag agagctcagg aacagaggca gacactgagg actcaggaga gcagaggggc
3000

cacagctcac ctgggaatcc gctgtgaata gtgcagatgc catcgcctgg tctctgggac
3060

taccctcatg agggggtgca ggaatgtggg cagcaggaag agctggggga ggagaaaggg
3120

tctgtgagta aatcagctcc gctctgagcc ttgctcaaag aagggctcgg ggcatgttcc
3180

ttcctgcacc agcatgttca ctgttcagtg tttaggtccc aaatacagaa ttccagacaa
3240

cacaaacgag agaacacagg gcctcagttt tcccaagacc tctactcttg agcaggagtc
3300

ataaaagggt cctaatcacc tggtccaacc cttttatttt aaagaagggg aaacttgagt
3360

ccctgagact gcaaatgact tccctaatgt cacattccca gtggcagagc caggatgggg
3420

acccaggttc ctattctcag ctataaaagt gggtaccttc agcccatagc tcaagtctgg
3480

ctcctaattt cctccagtcc aaggaacgat accaggatat gagaatccca tttatgccta
3540

cacttgtgga aaacacaaca cagctttaaa atctttagtg gcaggtgagg cgggaggatt
3600

gcttgagctc aaggggtcga ggctgcagtg agctatgatc acgcactcca gcctggatga
3660

tagagtgaga ccccgtctca atgaaatcca gaaaaacaac aacaaaaaaa acactttagt
3720

agcaatatcc agaaaggcag ggaagtaagg aggtctcctt agctctgggt aagttttttt
3780

tttttttttt ttttttttga gacagagttt tgctcttgtt gcccaggctg gagtgcaatg
3840

gtgcgatctt agctcatcgc aactttcggc tcccgggttc aagtgattct cctgcctcag
3900

cctccagagt agctgggatt acaggcatga gccaccacgc ccagctaatt ttgtattttt
3960

agtagaggcg gggtttctcc atgttggtca ggctggtctc gaactcctga cctcaggtga
4020

tctgcccgcc tcggcctccc aaagctctgg gattacaagt gttagccact gtgtccggcc
4080

acctctggct aagtttttgc cagtttttgt ccctctactt ttctccctca cttggccaca
4140

ataaatactg tttttttttt tttttttgga taagatgtat gcatggttca aatgctgaga
4200

caaaagttgg ggctgacaca caacaaaaga catgcataac attatcacgt gatcacaata
4260

actgtgatgc ataacaatta tgaaaatttt aaattgtgca atttaacccc ctccccatct
4320

cttgccaagt actattagat ttggaaatgt tactggtaga cagatgatgt tagtgagata
4380

caaggatatt tgagaaatga atttgaaact gaatgaccaa accttgtacc tatttcaaac
4440

ctttatacaa caataagata ggctctctgt cacccaggtt ggaatgcagt gctcaaacat
4500

gtctcactgc agccttgacc tcccaggctc aagtgatcct cccaccttgg tctccccaaa
4560

tgctaggatt acaggtgtga gccactgtac ccaagtactt tttttttttt tggagatgga
4620

gtctcgctct atcacccagg ctggtgtgca tggcgcgatc tcggctcact gcaacctccc
4680

ctcctgggtt caagtgattc tgctgcctca gcctcccaaa tagctgggac tacaggtgcc
4740

tgccaccacg ctcagctagt tttgtatttt ttagtagaga cggggtttca ccatgttggc
4800

caggatgatc tcgatctctt gaccttatga tctggccacc tcagcctccc aaagtgctgg
4860

gattacaggc gtgagtcact gcgcccagcc ccaagtactt aattttttaa acttctgaga
4920

gtaaggaaac actctggaaa acaaataaga aagaggaaat tgagaataaa tgtacatgtc
4980

tcaatttatt taagaactat ttactaaaga gcaactgtgt gtgccaggat ctttgaaact
5040

gttactctgg caacctaaac tctataatct agtgcttaag accatgaata tcctctccag
5100

tttcctaagg tcgccctgtg acttctgctt gtagagtgtg gagaaacctt acaagaacat
5160

caacagtagt ggagttaaac atcacggtgc accggtgttt tttttttttt tttttgagac
5220

agcatctccc tctgttgccc aggctggagt gcagtggcgc aatctcggct cactgcaagc
5280

tccgcctccc gggttcacgc cattctcctg cctcagcctc ctgagtagct gggactacag
5340

gcacccgcca ccatgcctgg ctaatttttt gtatttttag gagagacggg gtttcaccgt
5400

tagccaggat ggtctcaatc tcctgacctt gtgatcctcc tgcctcggct tcccaaagtg
5460

ctggaattac aggcgtgagc caccacgcct ggccacggtg caccagttta cacagctttc
5520

tctagcacat acctgctggc tgtcctccag gatgcacgga agagaggatg catccttaag
5580

ctccatgtct aatattcctt aaatgtcatc accaataaac acactggtta aggaatgtgc
5640

ctcgaccaat tctttttttt ttttttttga gatgagtgtt gctctgtcgc ccaggctgga
5700

gtgcagagga gccatcttgg tttgctgcaa cctccacctc ccgggttcaa gcgttcttgt
5760

gtctcagcct cctgagtagc tgcgattaca ggtgtacacc accacaacca actaattttt
5820

gtatttttag tacagacggc gtttcgccat gttggccagg ctggtcttga actcctgacc
5880

tcaagtgatt tgcccacctt cgcctcccaa agtgccagga ttagaggcgt gggccactgc
5940

gcctggcctt tttttttctt ttttgagatg gagttttgct ctgttgccta gcttggaggg
6000

tgtatgaatt tttaacaaaa aaagcttaaa aagtataaaa taaaaatttt tttaactaga
6060

aaaaccccta tagaataggg ggataaaaaa tgtttttgta tagctgtgca acatgtttgt
6120

cttttgagtt atgttattac aacagtcaaa aagttaaaaa aatcaaaaag ttcataaagt
6180

aaaaaagtta cagtaagcta atttattatt tgtatttatt tatttatttt ttttttctga
6240

gacagagtct cactctgtca cccaggctgg agtgcagtgg tgcaatcttg gctcaccaca
6300

acctctgcct acaggttcaa gcaattcttg tgcctcagcc tcctgagtaa ctggaattac
6360

aggcatgcac caccacgcct ggctaatttt tttattttgt agagacagtg tttcactatg
6420

ttggccaaac cagtctgaga ctcctggcct caagtaatcc gcccaccttg gactcccgaa
6480

gtgctgggat taagccatga gccaccatgc ctggctgatt tattattgaa gaaacaaaaa
6540

ttttaaagat aaatttagtg tagcctaagt gtacagtgtt tctacagtct atggtagagt
6600

agtgtcctag gctttcacat tcactcacca ctcactcact gactcatcca gagcaacttc
6660

cggtcctgca agctccagtc atggttaagt gttccataca ggtgtacttt tttttttttt
6720

tttttttttt ttgagatgag tttcactctt gttgctcagg ctggagtgcg atggcatgac
6780

aatggtgcga ttttggctca ccgcaacctc tgctgcctga gttcaagcaa ttctcctgcc
6840

tcagcctcct gagtagctgg gattataggc acctgccacc aggcctggct aattttgtat
6900

ttttagtaaa gacggggttt ctccacgttg gtcaggctga tctcgaactc ctgacctcag
6960

gtgatctgcc tgccttggcc tcctaaagtg ctgggattat aggtgtgagc cactgcgctc
7020

agccaatttt tgtattttca gtagagaccg ggttttaaca tgttggccag gctggtcaca
7080

aactcctaac ctcaagtgat ccacccacct tggcctccca aagtgctggg attacaggcg
7140

tgagccactg tgcctgggct aaaaatcttt tatactctgt ttttactttt tcgatgttta
7200

gatacacaaa tacttaccac tgtgttacaa ttccctacag tgtttagtac agtaacatac
7260

tgtacagctt tgtagcgtag gtgtggagaa ggctagacca tctaggtttg tataagtata
7320

ctttatgatg ttcgcacaac aaaatcactt aacattgcac ttaccataac ttatccctgc
7380

cattaagtga taaatgtact tttagcattc cttgtcttta cattctaagc acatactttg
7440

attttaaaac tggataaatg tattacatat ttccgtcaaa agagaaaaaa caagtaacat
7500

gactttttat ttatatgact gtgaactgat cttttttcgt gttcctcaac tcccctcaaa
7560

agttcttcca ttcttttccc attgtttgaa atttctgaaa ataacctaga aaagactgat
7620

gctatagttt tttttttttt ttttggaaac agactctctg tcacccaggc tgcagtgcag
7680

tgattatttt tatatatttt aaataaaatg gacctttaaa aaagatttat gatccttttg
7740

aagataattc atgacatact aaaatgtttt aaaacctagg ctgaaaataa tgacatctgt
7800

tcccaatact ttcctattaa aaatgtgctg gacatctgga atgctgataa ttcaataaat
7860

aatgcctacc aggttcatta cgcatagttc atttgccttc gtagtaacat ttaactcctg
7920

agattggtct gtaagttgta ttttccccaa tatttggtaa taatgacaac aatgtagaat
7980

aactaaaaac aaaaaacaag catgcagctt tttttcattt ttttttaaat ttttaattaa
8040

ttcttttttt tttttttttt tttttaagac agtctcgctc tgtcaccaag gctagagcac
8100

agtggtacga tctcggctta ctgcaacctc cacctcctgg gttcaagtga ttctcctgcc
8160

tcagcctccc gagtagctgg gattacagga atgtgccacc atgcctggct aattttcata
8220

tttttagtag agacaaggtt tcaccatgtt ggccaggctg gtctcaaact cctgacctca
8280

agtgatctgc ccgcctcagc ctcccaaagt gctgggatta caggtgtgag ctaccgtgcc
8340

gagcctaatt cttttctgta agtagctttt gagacagggt ccttgctgtg ttgcccaggt
8400

tggagtagtg gcatgattac agctcactgc agcctcaacc tcctgggctc aagcaatcct
8460

cccacctcag cctcctgcgt agctggaatc acaggtgtgc gccactatac ctggctaatt
8520

tttctatttt ttgtagagat ggggtctcgc tacgttgccc aggactaaca tgcttcttca
8580

tcacaggcac tcagcagcac aaagactctc gtcctgaatc atttcccttc ccctaaatga
8640

aaccttgctt cttacctcgt gactgtaaga ggcggggttt ccgagacgaa tgtttgaagt
8700

gggactgggt ggcctcgtga tgaaggtcaa agctcgagga ctcctgaact ggatccagag
8760

gcaccatccc ccttgcgagc atctcaggtc catgaacttg acctgggacc tgaagacaga
8820

caggttgggc ttgtgggtaa atcactttgt aaatgggaat taaaatattt aaaaacagac
8880

catgagtatg ccagtggatg caggaagaaa gccagaacat ttttaagaca gagtaaccac
8940

ctaataacct aaagcctgcc aatacttctc ctggctccag tttcaacgtc tgtgccctgc
9000

tcacacataa taggtttcac ctctttttac ctgttgtcct gataaatcaa gctccaagtc
9060

ttctagaagg gtcacggcct cctctccact atcggggcgg tattcctgca gccagacctg
9120

gagctccttg ggcaggatgg aaagaaactg ctctagcacc agaagctcca ggatctgttc
9180

cttggtgttt atttctggcc gcagccactg atgacaaagt tccttcagcc gactgagagc
9240

ctctcggggc ccaaaagtgt tctggtaaca gaagcgcctg aagcgttggc ggaatatctc
9300

tgggtctgga ggaggcgtgt cctgtagggt ggaatcctgc ccccacatgt ggtcttcctc
9360

atcttcctct tccaccttca ctattacgat accatccttc tcctgtgcag cctgtgggga
9420

cagacccgtg gcttcccgtg attcagcagt catcattcag gctccaggaa ctgacttgat
9480

ccaaacaggg tctgtgctca cctttatgtc ctgggaggtt ttatgatgtg tttctttact
9540

attcctgaag tataaaaaaa aagtcattag tacgtacctt tacaaaaagt gatctgtgct
9600

gctaacactt tataggaggc agcacatcga ggcactatca gaacgctcct gaggccgggg
9660

gtggtggctc acgcctgtaa tctcagcact ttgggaggcc gaggcaggcc aatcgcctgc
9720

ggtcaggagt tcaagaccag cctggccaac atggtgaaac cccgtctcta ctaaaaatac
9780

aaaagtcagc agggcatggt ggcaggcgcc tgtaatccca gctactcgga aagctgaggc
9840

aggagaaacg cttgaaccca ggaggtggag gttgtagtga gccgagatta cgccactgca
9900

cttcctacct gggagacaag aagttaaact ccgtcaaaaa aaaaaaaatc ctgacaaatc
9960

gaggaaaaaa ctgtacaatc tccactgtca cttcatatat caagatttgc aaacagaaac
10020

ctcaaacagg ccgactccac cctcagggtg tttgcttgga gtcagagtga tttacctgca
10080

ggttgacagc aggttacgaa atggagcatt aacagtgccc agcccagaga gctgaatgtt
10140

agcagagtgc caggtgccag gcttaaagca gtacacgcac catcttcttt tgtccctaca
10200

acagccctag caggtcggcc aaactgttaa gtgctgtttt acaaatgaca taactgaggg
10260

ttaggtttgc tcagtagtag gtggctgcct actggagtca atattcaacc aaatttgaag
10320

ccctgggacc tacttttgta ccatccagct acttttgcta tttttatgag tgacttgggc
10380

agttaaattg tggtttccaa agtacaagat gatccgcaaa ggatacagaa agaaaataat
10440

tggaatgtct gtatcttaaa agagagagaa attaagctat aaattgacac taatacactt
10500

acttagtctg tgctgacctg tcacaggtgt tgttcatttt agacattgtg atgtagactg
10560

gtttatctga gaccagttat gtgaaataat ggatttctaa atctagcttt ggctgggcgc
10620

agtggctcat gcctataatc ccaaaacttt gggaggccaa ggcgtgcgga tcacgaggtc
10680

aggagatcgg gaccatcctg gtcaacatgg tgaaaccctg tctctactaa aaatagaaaa
10740

attagctggg cgcggtggcg ggcacctgta gtcccagcta cttgggaggc tgaggaagga
10800

gaatcacttg aacccaggag gcggaggttg cagtgagccg agatcgcgcc actgcactcc
10860

atcctggcca cagagcgaga ctctgtcata aaagagacaa gcagctttaa agattcttgt
10920

ggccagatat ggtagctcat gcctataatt ccagcccttt gggaggatga ggcaggagga
10980

tgcttgaagc cagaagtttg agaccagcct gggcaacata gcaagaccct gtccctatga
11040

aaaagaaaaa aattagctgg gcatggggac tgtgtctgca gtcacagcta ctcaggaggc
11100

taaggtggga ggattgcatg aaaccagaag tttgaggctg cagtcagcta cgatcacgcc
11160

actgcattcc agcctgggtg acagagcaaa gccctatcag gaaaaggaaa aaaaaaaaaa
11220

aaaagattct tatagagaaa ctgcaattac aggacacact aataatataa acagatgtga
11280

acaagaagaa aatggcacag atgaagcttc tatctagagt aaggttttag ccacattagg
11340

aggtaaaaac caatgtcagt ctaaatcaaa gaaaaagtca ccaatgagta atcagcttct
11400

ttaaagaaag tgtgaagaaa gggcaattac aggaacagct gtatgaaatg gttccattgt
11460

tatgtaattt aactgccaaa aatgatgtca agtatccagg aaaaacccat ctatgttcac
11520

attttagaaa cttggggctg ggtgaggtgg ctcatgcctg taataccagc actttgggag
11580

gctgaggcag ggagcagggg gccacctgcg gtcaggagtt caagaccagc ttggccatca
11640

tcgtgaaacc ccgtctctac taaaaataca aaaaattagc tgggcatggt agcaggcgcc
11700

tgtaatccca gctatttggg agactgaggc aggagaatcg cttgaaccca ggaggctgag
11760

gttgcagtga actgagatca cgccattgca ctccagcctg ggtaacaaag tgagactcca
11820

tctcaaaaaa aaaaaaaaaa aaaaaaggaa aaaaaaaaga aacttggaaa tattttctaa
11880

cctgtttaaa aatctttcaa atgaagaatt tcaggtgggt cttaagccta tttgttaaaa
11940

atataaaatg ccaccacctg attagtttgt aagactatac cctgcatttc aacaacaaaa
12000

attcagcatg gttgataggt tggatcaatc acaagttact aagcagaact gacactgtga
12060

ttcttccatc tgaagctaca aatctttctg aatttattct tttcacctct acagccatca
12120

aaactaagca tcaaattgaa gttttagaaa cagaccatta aactgcttta tcatgatggg
12180

ttaaaccgag ttttaaacta gcaaaagcat ttttagtttt atggctctca ctaaacatat
12240

tcttaatttt ggtgaaaaag ggatcatgta catataacag cacaaaaatt atatatacat
12300

atatattgaa acggagtctt gctctatcgc ccaggctgga gtgcaatggc atgatctcag
12360

ctcactgcaa catccgcctc tcgggttcaa gtaattctcc tgcctcagcc tcctgagtag
12420

ctgggattac aggccatact tatttttttt aatctagttc catgaagtcc agcttccttt
12480

ctttggttcc ctgccttcat tttctccctt ctctcccaca atccaccttt cagattattc
12540

tttctaaaac tctacagcct tgcagttaat actttccagt gcctggtccc aattttccct
12600

caagcttctt ttcccatcaa tccccccata aaccttccct atgcaacaaa agccctctca
12660

tcagtatttc ctatatatgc cttgtatttt gactgccatc ctttcctcac atcacactcc
12720

atgcctggga cttccccccg cagtcctctc tgccttttca aatcctactt aacctttaaa
12780

gctagctaaa acgcgacttg cctcttatca ctgttcaaga accctctcat aactataaca
12840

gtaagagcca atgtttactg agtgctttct aggtaccggg tatttaagca ttttcacttg
12900

accacataac aatcttagca ggtagctaca cttatcatct cactgtataa agaactgaga
12960

ggctgagcgc agcggctcat gcctgtcatc ccagcacttt gggaggccaa ggcagataga
13020

taacctgagg tcaggagttc gagaccagcc tggataacat ggcaaaaccc catctctact
13080

aaaaatacaa aaattagcca ggtgtggtgg tgcaatgcct gtagtcccag ctactcggga
13140

ggctgaggca ggagagttgc ttcaacctgg gaggcggagg ctgcagtgag ctgagattac
13200

accactgcac tccagtctgg gcaacagagt gagactccat ctcaaaaaaa aacaaaagaa
13260

gaactgagat atactgccat taaataactt actcaaggtc acacagcaag tatatggtag
13320

aggcagaatt tgaaataaaa gtgattcccc aatgtaaact ctttaaccat tatacaaaat
13380

ttatcttgta cctgacatgt ggggtataca gatcctaaaa tgaaggccta tgaacgtgac
13440

aatgaagaat caatcagggc caggcacggt agctcacgcc tgtaatccca gcactttggg
13500

atgccaagga gggtcgatca cctgagctga ggagctcggg accagcctgg ccaacatgac
13560

gaaaccccgt ctctactaaa aatacaaaaa ttagctggat gtgtggcaca tacctgtaat
13620

ctcagctact cgggaggctg aggcaggaga atcacttgaa cctgggaggt ggagggtgca
13680

gtgagctgag aacgcgccac tgcactccag cctgggtgac agagagagac tccttctcaa
13740

agagaaaaaa aaatcactca ggaacgggcc ttatctacca cccagttttc actggagggt
13800

cactatgtga ctgaagccct tcctgggaaa gggaggagac acagcagaag attcaagtgg
13860

ggactgtcta caacataacc aggtgggatc ttcaaacatg gtaactgagc agaactagtg
13920

tgatagcgag cccagccacc agaaatccct acttttgtga aagttttatc ctgccttagc
13980

tattgagcag gacacttctt gggtcagtgc gaagatgcga ctctaaggag gaaatgagtc
14040

cagggggaaa tagacacaga catgggcaca ttccctctgc ccacaactgg gacagatcct
14100

cctcctcacc ttcagtatct ctctgctcct actctggggt gcagggaaaa gccatcacag
14160

cagtggaaag gctgggtttt atttgaggtt tcccagatga tctgagcaca tactcttctg
14220

tctctaaagg ccctaatact cttcaccaaa catttgctag gtaatttctc atgatgcgcc
14280

gggcgcagtg gctcatgcct gtaatcctag cactttggga ggctgaggtg ggcggatcac
14340

gaggtgagga gatcgagacc atcctggtaa cacgatgaaa ccccgtctct actaaaaata
14400

taaaaaatta gctgggtgtg gtggcgggcg cctgtagtcc cagctacttg ggaggctgag
14460

gcaggagaat ggcgtgaacc cgggaggctg cgattgcgcc actgcactcc agcctgggtg
14520

acagagcgag actctgtctc aaaaaaaaaa aaaaaatttc tcatgatgtt tctttctctg
14580

taatttttct tgccttcttc ctcaactaga ttattaagag cagtcctctt tgtactcctt
14640

agaccactta gcatgattcc ttgcaagcct cagtttcaaa aatacacttg actgaattga
14700

caacgttact attttttaaa agcccgatca ttctacattc aatgttgcac tcaaatttgt
14760

tccatggact agttggatga gtttcaggtt agcattaaag ctgctccacc ccttactagt
14820

tgacggcctt aggcaagtca acaagttaac ctctctatgc gtcagtttcc tcatctgtat
14880

aatgggaata aaaatcagca tttgtcccat agagttgccg tgaggattaa atgaggtaat
14940

agatataaag cacttaaaat agtgcctggc atttagtagg cactacagaa atgtcttaat
15000

ttttttcaat aagcttaaaa gatgtaactg tacattccaa ttttcagcct gcttagaaga
15060

ttaagcaatg ggacaactcc tgtaatagtg aaagctgcac aggcaataac tgaattctag
15120

tctcccacaa gtaagtggca catccctgag agaggtattt aacttctctg gcattttgta
15180

tctttatcag tcaaatgggg gtagggtggg ggttccagat agtaggaaac tacctctggg
15240

tgaatatggg ctgaaaaaca tactgacata ccatccactt taaataaaat tacctccagc
15300

agctaaggat acctcagttc tcacctagga taaacagaaa tgattttctt aaacaacggc
15360

ttgaagttac aagctcaaag gctgtgttcc aaacagccca gtatttttcc agggcatagt
15420

cttgaaaatg taaacaaagg ctgggcacac agtggctcac gcccgtaatc ccagcacttt
15480

gggaggctga ggttggcgga ctgcctgaga tcaggcgttc gagaccagcc tggccaacat
15540

ggcgaaactc tgtctctact aaaaatacaa aaatcagcca agccttggga cacatgcctg
15600

tagtcccagc tattcgggag gcggaggcag gaggatcgct tgaactcggg aggcagaggt
15660

tgcagtaagc cgagatcgca ccactgcact ccagcctggg cgacagagca agactctgtc
15720

tcaaaaaaaa aaaaattttt ttttaaataa attgcccttt gccctttaca ctcatttttt
15780

taaagagcaa tcaaatgttt taatatgaaa aagctacctt aaaagacagc tagctattca
15840

aagaatattg atactcccac agaatgaaaa tgttagattt aatatgctat tcatctgtat
15900

tttaaatgct tttaaatcga gaatgagcaa aagactaagt agctagcttc ctcgtttgta
15960

ttttaagagt ggtgaaaact gcctgaaata ataaacaaga atcttatatg ttaattcaga
16020

gaagatacaa acctggatac acattctggg gcaaatcacc atggtttcct atgtaaaaca
16080

gcgatttctc cacatttgtt cagcatatat actaacagtg ctttgctgat gtggttacaa
16140

tgaattctcc acaattcaat tcaacaagta tttattgacc gcctccaatc cgcctagcac
16200

tggcccgtac aataaatgct ttctttggtc ccaggccatg gaacagccac tctctataat
16260

gaaagtatct tactgtgtac agggggcact gcaggaaagg gaggggaggg atggaggagt
16320

gtcaagctaa actgtagtta attcttcaat tacattgacc aaatcatttc gaagtcggaa
16380

gggccttttg agaaaatcca gttagttttt attgatgacg aaattaagac ttggtgagtg
16440

tctcgtctta tccaaggtca ctcagctaat cagtctccta gtgacacagt gcaatttcca
16500

caaattccct tcacttctca ccccactcca tgtcaaacag agcaactatc cagaattgaa
16560

aaatccacct aacgtcccgg acagctcttt tccaaagcct tcaaagcatt ccatcaagct
16620

gaaagagtgg ggtgtatata atctctacta gatcccaatc acctatttat ccactatgac
16680

taggcttcaa atactcttta atcaccgctc agtgatgagg gtgggagaag gaaaggcagg
16740

tctggaaacg tatgcggcac ctcaaaatgt gtccatctct catccttccg ctcatttcac
16800

ctcgaggtcc cgaaggaacc ggggggggaa aaagacacct caccggaatc aaagcaaatg
16860

gcgactaagg gagagcagaa aaatcaagac cggggtcggt tcgctcgcga ccccgtttgc
16920

agaaacgtca aagggcgatg acgacactgc gggagagggg cccacagacg gcttctcccc
16980

gggtctccgg gcaccctcag ccactgaaac cctcgccccc tccccgcctg atcctggggg
17040

tggggcgggg acgtctccca ggaagaacgg cgctttctac ggccgggccc ggccaggagg
17100

caggggcatg gactctcttc gcacaacagc ggggccgcct ggagggggtc ccagggccgc
17160

agctgcggag cccaccctcg cgcacacacc cttgcggccc cgccctccca ccccagaggc
17220

cggccccaag tctcaccgtg agccccggga gcggcctggg ggcgctgggc gagaaagggg
17280

agctgactct ggggctcagg ccggccgaag ggcaccgcac acggacttag gccctcccga
17340

cgcctcgaca cagacacaat gagtcacaaa ggccggaagc gtgtcaccac ctcgtcttcc
17400

gggaccgcgc cgcgcttccg ggtcctctct aggagcgcgg gaggactttg catctcggtt
17460

tccgaatcgc tggaccaggc gcctgccctt gagttttcaa gcctccagct tggaggatca
17520

ggtcccagtc cgcccctagt taatgcaggg actaacggga gcaggtggtg gggggtttca
17580

ggctttcccc tactcaaact caaacagact ctccagctca gacaccagtc catgtgaaag
17640

ttcgatttca cctgtctttt tcatctcagg cagtccttgt agtaattata gttattatca
17700

agcaatatag tacttggtaa ttgtatgtct gtggcaaagg aactgaaaca aggatgtaga
17760

ctgaggaaga tcaaagtgaa gccaagggtg gctggtttcc caaagctgcc acggtagctt
17820

ctctcagggc cctggagcta tatggagtgg tattttcaaa tacttggccc gtggacaagg
17880

gatgtctttc cacaagcaag catagttata aggttctgtg ctttgctcat gtggcatatg
17940

ctattatagc cgtttaaaag gagttttcag agtaacaaca gacactttta ccttcaaagt
18000

gctttcacaa cctaccacag ttacagcctt catctttgcc ttgcagggta aggcacgcat
18060

aagagggggc cacgttgtgc aagggtgggg gggactacag gctttaatgg ttgtgacttt
18120

ccactatgtt ccgagacaag ttcatgggag aaactcagta gcttggttgc atgactctta
18180

ctgcttggag agatttactt ttttttttta gatggagtct cgctctgtca cccagcctgc
18240

agtgcagtgg caccatcttg gctcactgca acctccacct cccaggttca agcgattctc
18300

ctgccttagc ctcctgagta cctgggacta caggcgcaca ccaccatgcc tggttaattt
18360

ttgtattttt ggtagagaac agggtttcac catattggcc aggatggtct cgatctactg
18420

accttgtgat ccacccgcct cagcctccca aagtgctgga attacaggcg tgagccaccg
18480

tgcccggcca gatttacttt ttaaaaaatg tttgaaaact aaaaagcaat gacagggcca
18540

gacacgatga ctcaagcctg taatcttagc acttcgggag gctgggacgg gaggattctt
18600

gagcccaaga gttggagacc agcctaagca acgtaatgag accctctctc tacaaaaaat
18660

actaaaatta gctgggtgca gggtagtgtg tgccagtggt cccagctgct tgggaggctg
18720

aggtagaagg atagattgag cccaggaggt tgaggctgca gtgagctatg attgtgccat
18780

tgcaccccag tctgggccac agagcaagat cccatctcaa aaaagaaaaa aagggccggg
18840

cacggtggct catgcctgta atcccagcag tttgggaggc caaggtgggt ggatcacgaa
18900

gtcaagagat tgagatcatc ctggcccata tggtgaaacc ccgtctctac taaaaataca
18960

aaaattagct gggcgtggtg gcacacgcct atagtcccag ctacttggga ggctgaggca
19020

ggagaatcgc ttgaactcca gaggcggagg ctgcagtgag ccgagatcgt gccactgctc
19080

tccagcctgg ggacagagct agactctgta tccaaaaaag aagcaacaac agtatcatta
19140

aaggaaattt gcaaaatgga gaaaaataat taccggtaat ctctccaccc taacacaatc
19200

cccatggctt tagtttacct ttttcctgtt ttcatataca aataaatatt tattgatatt
19260

attttaaaca ctgttcaaaa ccatagttgc agttgtattg aagaggaggc tggtatcctg
19320

gaagtatctg ctacattcat atcatgtgaa cactgttttt taaggaagag aaaatatatt
19380

tactattcag taagtggaaa tagatcatca tcaaggtctt catactcatc gtcttcacac
19440

tgagtaagct gaggaggaag aggaagggtt gctcttgctg tctcaggggt ggcagaggca
19500

ggaagtgatc caccagcctc ggcctcccaa agtgctagga ttacaggtgt gagtcactgc
19560

acctggcctc ttttctttct ttttttttga gagagtttcc ctcagttgcc atgttggagt
19620

gcaatggcgt gatctcagct cactgcaacc tccgcctcct gggctcaagt gattctcatg
19680

cctcagcctc ccaagtagct aggattacag gtgtgcactc accatgcctg gctaatttgt
19740

gtatttttag tagagatggg gttttaccat gttggccatg ctggtctcga actcctggcc
19800

tcaagtgatc cacccacctc agcctctcaa agtgctggga ttacaagtgt gagccaccgc
19860

gcctggcctc gacatctttg attacttcct aaggatggat tccaagaaat gagattactg
19920

gtcccacaga tagtcagagg gaatataggc aataacgttt gtaaccccag ggttctagac
19980

ctgtcagcaa acaagctcag ctgacaagat caggtggtat ccacccaaga gttttgaaag
20040

gactctaaga tgaaagtgtt gaaagaatca tgaaactaat gatttatgat tacaaagaga
20100

cattgtgctt aaaaattggc tggctgccct gaacaaataa aagttcctga agggcaccct
20160

agaaactgtg gttgcctaag tttgacttct gtgttgtgaa agctggtgga gtcaatgctg
20220

aaggggagcc cgggtttgcg gctgtgactg ggtggctgca gctactcctg ggagggaggg
20280

tctcctgtcc ctccaacttg gaaggagtgg gggcttccat gtgttcccag ctccctccag
20340

ctctgtggag tgctgcgatc ccagctatgc ctcactcact gcagccagca tcatggcagt
20400

ggccactcca gatggggtgc tgctgccaag gcgggcagat cacctgaggt caggagctcg
20460

ggaccagcct gaccaacacg gagaaacccc gcctctacta aaaatacaaa attagctggg
20520

catggtggtg catgcctgta atcccagcta ctcagtaggc tgaggtagga gaatcgcttg
20580

aacccaggag gcagaggttg cggtgagccg agattgaacc attgcactcc agcctgggca
20640

ataagagcga aactctgtct taaaaaaaac aaaacaaaac aaaaaaaaca aacccaagtc
20700

ttatatctga ttccaaaggt cccattttac tgagtcttct tctttcttct tgacggagtt
20760

tttgcttttg tcaccgaggc tgggatcact gcaacctccg cctcccaggt tcaagcgatt
20820

ctcctgcctc agcctcccac gtagctggga ttaaaggcgt gtgccacctg cctgactagt
20880

ttttgtattt gtagtagaga tgggattttg ccatgttggc caggctggtg tcgaactcct
20940

gacctccagc gatccgccca cctcggcctc ccaaagtgtt gggattatag gcgtgagcca
21000

ccgtgcccag cctgagtttt ctttactgtc ggctagggct gtggctgggg aacctgctgt
21060

gggaagggta ctttcatcta tgcttttcct cctccccttc cctctccctg ggtggcaggg
21120

agtgagtgat ctgactgttg gagacacctt ccctctctat tttcctattg gaagctgtgg
21180

cttctctgtt gtgttgagca aagtggacgg atagggagag ggtcaaaaat ctcttactta
21240

actgactatt ctctcctgtt ggttgttgct cttccctcgg taacacagtg ttccagctac
21300

ttaagcagca aaacaaatta ttcaacaatt tcgtgggtga aagcaatgac aacattgttt
21360

attttgctcc taaatcttcc atttggtgga gggatccaca tggatggctc atctatgctc
21420

cacctggctc ctctgggggg cctcaaaggt tggaccatct gaaggctcgc cagctcctaa
21480

gattggtgat tggtctggct gtcagcggag atcttagctg gggcagtgga tctgaacatg
21540

tgcaggtggt ctcttcatgt gctctgggct tcctcacagg atggtggctg gggtccaagg
21600

acaagtatcc agagagagag agaggaggaa ggaaggaagg aaggaaggaa agggaaggga
21660

agggaaggaa ggaaggaagg aaggaaggaa gagcaggtga gcgggcaggc aggtagggag
21720

ggagggagga aaggaaggaa ggaaggcagg gagggaggga gggaaagaag gaaaggggag
21780

ggagggaaag aaggaaagaa agggagggag ggagggagtg aaggaaggaa ggaaaggaaa
21840

gggaagggaa tggaagggag ggaaggaagg aggtacggga aggaaggaag tggagtgggt
21900

gacagagaga cagaggggac aggtgaatga aggccacatg tcctttataa cctaatctta
21960

gaagcactca gcatcccttt cactgcattc aatgccttga ggcagtcata aagtcacacc
22020

tagtttcagg ggaagggaaa atatgttccc ccctcttttc ttcttttttt tatgacacgg
22080

ggtctcactc ttgtcaccca ggctggagta caatcatgac tcgctgcagc ctcgacctcc
22140

tgggctcaag tgatcctcca ccctcatctt cccgtgtagc tgggactaca ggcatgagca
22200

actatgcccg gctaatgttt aaattttttt tgtagagatg ggggtttcac tatgttgccc
22260

aggctggtct caaactcctg ggttcaagca atccttccac ctcagcctct tgagtagctg
22320

ggagtacagg tgcacgccac cacacccagc taattttatt ttattttttg ttagagatgg
22380

ggtctcacta tgttgcctaa gctggtctta aactcctggg ctaaagggat actcctgcct
22440

tggcctccca aagtgctggt tttacaggca ccatgcctgg cgatgccacc tttttttttt
22500

tttttgagac taagtctcac ttttgtcgcc caggctagag cgcagtaacg cgatcttggc
22560

tcactgcaac ctccacctcc cgggttcaag caatcctcct gcctcagcct cctgagtagc
22620

tgggattaca ggtgcctgcc actgtgcccg gctaattttt gtatttttag tagagacggc
22680

catgttggcc aggctggtct tgaactcctg acctcaggtg atctgcccac cttggcctcc
22740

taaagggctg ggattacagg cgtgagccat ggcacccagc cagatgccac cttttgatgg
22800

gaagtgatgt ggttctggaa ttgcatgtgg gaccaaaaac gctgccaagg tcacttttgg
22860

aaaacacagt ctaccgctac tgtgcgtcag ccacacaaac gttggcttgt ggtgttcagg
22920

gatgaaatcc tcagggggcc ttgggggcct gcttactact cagtcccctg tcccagtcga
22980

tctgccttct gcccctagca gggcactgca caacctcctt ttgaggagat tcttgggtgg
23040

gggcaccagt tggacaactc aagtccagct acctgctcct gggtccattt gatctgtgac
23100

aaactcaggc acccttggcc actcaaatgg ctggaacgtg gacctcctgc ggtgctcctg
23160

cccctccctg gccaccttcc cctcttagtg aatggaacgt ggacctcctg tggtgctcct
23220

gcccctccct ggccaccttc ccctcttagt ggctttgttg aatcacccgc cacctgccta
23280

ggcacgagag tctcctattt aaaataatgc tcaactagag gttgagggtt tttttgtttt
23340

tgtttttgtt tttgagtctt gccctgtcgc caggctggag tgcagtggcg tgatctcggc
23400

tcactgcaat ttccgcctcc tgggttcaag tgattctcct gcctcagcct cctgagtagc
23460

tgggactaca ggcctgtgct tccacaccta gctaattttt gtatttttag tagagatggg
23520

gtttcaccat gttggccagg atggtcttga tctcctgacc ttgtgatctg cctgcctcgg
23580

cctcccaaag tgctgggatt acaggtgtga gccactgtgc ctggccgagg atgaggtttt
23640

ttgtagatat ggagtctccc tggtgactgg gattcctgcc cgccctgccc cctgctcttt
23700

tttttttttt ttttgagatg gagtctccct ctgttgtgca ggctggagtg cagagtgcag
23760

tggtgcaatc tcagctcact ccaacctctg cttcccggtt caagtgattc tcctgcctca
23820

gcctccccag tagctggggt tacaggtgca tgccactacg cccagctaat tttttgtatt
23880

ttcagtagat atgagacttc tccatgttgg ccaggctggt cttgaactcc tggcctcaag
23940

tgatctgccc acctcagcct cccaaagtgc tgggatcaca ggtgagagac accgcaccca
24000

gcctctgtcc ccaggttttg aagacattct cacttgtctt catggtgagg gggcaaggga
24060

accagtcatg gaggaggaga gggaagcccc caccaatagg cagaaggcaa agtcctttct
24120

ctttggcttc taccaagtcc tttctctctt tcttgctgct ttcgtccaga cttgaggagc
24180

agaaaaactg gctgagcaag caaagctcgc gagcgtttct gcctgtcacg taggtggcat
24240

ctgggatctt tgatgtgccg tgctgtgggc tgggagtgtc ctcctgaagt tttccttct
24300

ttgttctcag ctatggactc cagccacgga caacagagtc ctagcccacg tccagtcaca
24360

tgactgcaaa agcacctgcg tctggatacg gatgtttatg agaacattgg tgcctaatca
24420

aatcaaatca aaacaaacca aacagcccct agccttgtgg cctcattccc aagtcagccc
24480

aagccatatc cttcctagct atggtgtata gtagaggggc gcaggactca gatggtcttg
24540

gttctcatct tgtctgtacc atgtactctt ggtctagtta cttgaacttt tggagcctca
24600

gtttctctaa gtgtaaaatg aggattcaaa cagcacctac ctcattggga tgtcatgagg
24660

cttgcatgat ggagttgatt catgtaaatc agcagttgca gggtctggca ttggacacag
24720

atgaatttag ggctgtgggc tgttgactga cccttgggct tgagtgacat gtgtggctcc
24780

atcctgaagc tcttttctag cagtgctgag gtgggaggag ggacttgact ctgcaggtgg
24840

ggcttggaca gtggaccaaa ttgaggatta gctaaaatgg ggccagggca gaagcagctt
24900

tccataagac atgcccacca gagtgccatg tcagtttacc gttgccatgg caacacccgg
24960

gagttaccac ccctttccat ggcaatgagc tgacaaccta gaagttacta cctcttccct
25020

agtaatttct gcactaactg ccccttaatc tacacgtaat taaaagtagg taggccaggt
25080

gtggtggctc acactcacac ctgtaattcc agcactttgg gaggccgaga taggtggatc
25140

acttgaggtc aggagttcga gaccagcctg gccaacatgg tgaaacccca tttctactaa
25200

aaatacaaaa atcagccggg tgtggtggtg cacgcctgca ttcccagcca ctccggaggc
25260

tgaggaatga gaatcggtta agcccaggag gcagacgttg cagtgagcca atatcgcgcc
25320

actgtacact gtactccggc ctgggcaaca gagggagact ctgtctcaaa caaaacaaaa
25380

caaaacaaaa ccccaaaaaa caaaaaataa tctatggtgg tgctgaatgt agacagagaa
25440

gagccagtgt ggttgttcac gtctgtaatc ccagctactc gggaggctga ggtgggagga
25500

gggcttgagc ccaggagttt aagaccagct tgggcaacac agtgagactc catctcaaaa
25560

aaaaaacaaa caaacgaaac aaaaacaaaa acaaaaaacc agtgactgta gagagagaat
25620

aaagtggttg gggaaaaaga agtcagggct tcaaagtaga aagagtactg aaaacttata
25680

aatagcactt tatcaggaag gtaatgaaag acctgaatgt aggttggtta aaagcatgat
25740

agcaggaaga atccaggcac attgaagatg aagacagaaa aaaacaaaca agccacattt
25800

agcaatttca gcaggaaatg aacacagatc ctacggggag gcgaagttaa cttgtaaatg
25860

agactctgta tgaaggtacc tctatataca tttgcgtaaa actagtttaa aagctgcaaa
25920

acacagtcac acagttgtta cagaaacgtg atcactaaag aataaattgg ggttggggca
25980

gggcatggtg gctaacgcct gtgatcccag cattttgaga ggctgaggca ggtggatcgc
26040

ttgaggtcag gagttcgaga ccagcctggc caacatggcg aaaccctgtc tctactaaaa
26100

tacaaaataa ttagccacgc gtggtggcac atgcctgtaa tttcagctac ttgggaggct
26160

gaggaacaag aatcacttga accccggagg aggagagatt gtgccactgc gctttagcct
26220

gggcaacaga gtgagactct gtctcaaaat aaataaaata caggctgggc gcagtggccc
26280

atgcctgtaa ttgcagcact ttgggaggcc aaggtgggtg gatcacttga ggtcaggagt
26340

tcgagaccag gttggttaac atggtgaaac cctctctcta ctaaaaatat tttaaaaatt
26400

agccaggtgt ggtggtgcgc acctgtaatc ccagttactc aggaggctga ggcagaagga
26460

tcacttgaac ctgggaggtg gaggttgcaa tgagccgaga ttgcgccact gcactccagc
26520

ctggggaaca agagcaaaaa ctctgtctaa aaaaaaaaaa aaaaaaaaag ggccaggcat
26580

ggtggctcat gcctgtaatc tcagcacttc gagaggctga ggtgggcggg cggatcatga
26640

ggtcaagcga ttgagatcat cctggccaac atgatgaaac ccagtctcta ctaaaaatac
26700

aaaaattagc tgggtgtggt ggcgggcgcc tgtagtccca gctacttggg aggctgaggc
26760

aggagaatcg cttgaatctg gggggtagag gttgcagtga gccaagatca tgccactgca
26820

ctccagcctg gcgacagagc gagactccat ctcaaaaaaa aatatatata tgtgtgtgtg
26880

tgtgtgtgtg tgtgtgtgtg tgtgtgtgtg tgtgtaaaca ataaatacat aaattgagga
26940

gaaggcaggg gaaccaggat ttgacagtga caagaaagaa aagcccagga ggacctggtg
27000

gctgagccag atgctggggc tggactaagg gtgatgggag cagagctggg acctttgggt
27060

ggcagagaag ttctaatcag tgaaactggg caaagctatt tggatggagc agtagagttg
27120

gggttaagag acagagacag gccgggcctg gtggctcaca cctgtaatcc caacagtttg
27180

ggaggccaat gtgggaggat cattgagacc aggagtttga gaccagcctg ggtaacatac
27240

tgaggccttg tctctacaaa aaatttaaaa aattagccag gtgtggtggt gcgtgcctgt
27300

ggtcctagct actcaggagg ctgaggtggg aggatcgctc gagctcagta gactgaggct
27360

gcagtgagac tagatcgtgc cactgtactc cagcctgggc cacagagtga gaccctgtct
27420

caaaacaaaa caaaacaaac aaacaaacaa caaaaaaaag aggcagagag acaaaggcgg
27480

cgaagaagat ggaatgatga tgcctttcgg gtaggtttgg tgttttgggt tgtaaaaggg
27540

aggataaaga agtaggagaa agtagaaaga acactaattg ggactcaggt aatggcttca
27600

ggtagattgt acaggtttga atgcggggag acaaagccca cgaggagaca agaggaaaca
27660

aacttggagg aaagtttctg tgattctaaa cacgttccct ctcttatctc catgatttca
27720

gctagtggtg ggatgctctg tgtatgtttc atgcagtttc acatagttgg gggctcagtc
27780

tctccacata aggcatttta tttcttttct cttttttttc tttttttgag acggagtttc
27840

attcttgttg cccaggctgg tgcgcaatgg ggcaatctca gctcactgca acctccgcct
27900

cccaggttca agtgattctc ctgctcagcc tcccaagtag ctgggattac aggtgcctgc
27960

caccacaccc agctaatttt tatattttta atagagacag ggtttcgcca tgttggccag
28020

gctggtctcg aactcctgac ctcggtgatc cacccgcctc agcctcccta aagtgctggg
28080

attacaggtc tgcgccactg cgcctggcct ctccacgtaa ggctttgagt ggcgtactgg
28140

gaacgccccg ctggtggtgt ccttgggtgg actgcagggc cctctaagtg ccccttgagg
28200

ctgaccactt cggcttccag catgtctgag atgagatgtg acactctctc ctgggagcca
28260

cctgcaaggt gattatgtat tggaacaagc tctcttcttg ccttatttta cagcccaatg
28320

gagaggaagt aggggtcaga gtagtagggg tgtaaaattt ggagtcactc ttgttgggcg
28380

tgtggtttgt gcaagtgacc cgacctctct aagtcttgac tccctcatct gtaaaatagg
28440

gatggttttg gcaaatagct acccacttag ctactctgag gacggtatga cattgtggag
28500

gtggcaatgc tttgtaaact gcagaggact tttaggtgcc agccatcgcc atgttttaaa
28560

tgcggagaac tgcatttctc accacctcgc aaaataaaac ttcacagttt atttgggttg
28620

ggtataatta ccgggcgaaa aatgtaaaaa taacaatgtt ttagaaattt ccccaaagaa
28680

gaaacagtat tggctggggc tgccttccca ggggccctgg aatcctgagg gtgcctgacc
28740

tgcaaatgcc cagcgcttag caggagaaca gccagatggc cctgctagtt ccctctctgt
28800

gcaaacatct ctcacccagc cgagtcaaca accctccctg ggtcaagagg ctcctccatg
28860

ctgcttctgt gtggtgtagc ctagaaactg ggcggctggg gcacagggct ctggactttg
28920

gttatgtgtt taatctaggt ccctgagcta cagggtcatc tgttttggct ccttctctgc
28980

ttatgtaaac agggctggtg ggcccatcag tacagccagg ccccgctctc accccctcct
29040

cctgtaaaca cattacaaag aatctttgta actgcccagg aagagcagct ctgctcaggg
29100

gaagagagcc tgcatccgga gggggtgaga ctcggggtgg agatggatgg gggaagagta
29160

ggcaggaggg gcaaaggtgg ggcaggagtg gtgcttgcca atacctcctg tactatgtgg
29220

tacagaaaag ccagctttgc tcaaagtctg gccgtgatag gagggggcct gttgtctgca
29280

gcagaaacca aggtggtagg aagggtggtg ttagaaaagg ggagaactgg ggtggggcat
29340

ggtggctcac acctgtaatc ccagcaattt gtgaggctga ggcgggagga ttgcttgagg
29400

ccaggagttt gagatcagcc tgagcaacat gggaagattc tgtgtctaca aaaaatacaa
29460

aaattagctg agcgtggtgg catgtgccta tagtcctagc tacttgggag accgaggcag
29520

gaggatcgct tgaggccaag agttggaggc tataatgagg tacgatcaca ccactgcact
29580

ccatcctggg tgtcagagcg agatcctgtc tctaaaaaaa gaaagaaaga aaaggggaga
29640

atctttattt cctaacatcc tgcagccctg acacttttct taccttagca attcaaacat
29700

ggttaaaaaa aaaaaaaagg agggggtgcc aagtgcggta cttcacacct gcaatcccag
29760

cactttggga tgctgaggcg ggtggatcac ctgaggtcag gagtttgaga caagcctgac
29820

ctacatggtg aaaccctgtc tctactaaaa aaatacaaaa aattaaccgg gcctggtggt
29880

gggcacctgt aatcccagct actctggagg ctgaggcggg agaatcgctt gagctgggag
29940

gcggaggttg cagtgagtcg agatggtgcc attgcactcc agtctgggca ataagagaga
30000

aactctttct caaaaaaaaa aaaaaaaaaa agaactaggc caggtgtggt ggctcacgcc
30060

tgtaatccca gcactttggg aggctgaggt aggtggatca cccgaggtca ggcgagacca
30120

gcctggccaa catggtgaga ccttgtctct gctaaaaata caaaaattag ccaggcatga
30180

tggtatgcgc ctgtaatccc agctacttgg gaggctgagg caggagaatt gcttgaaccc
30240

tggaggtgga ggttgcagtg aggtgacagg gcgccattgc actctagcct gtgcgacaag
30300

agcaaaactc catctcaaaa taaataaata aatacataca tacatacata catacataaa
30360

taaatacaat aaaaagaact aaagcctcct tttcaagtag tacaggttag aagcaagaat
30420

attagctgga gagtcaagaa aacaaattgt attcctgctc ctacccctaa tgagctgtgt
30480

gccctttgga tgatgcctta acatcttgga gtttccttgt ctagataaat cagaatgata
30540

atatttgcca ttttttgaac acccttgata tgacagggat ttttacctgc tattttatta
30600

atctacacaa atacccaatg aggtagggtg ggttatcccc attttagaga taaagacatt
30660

aagacttagc taggagaact gacttgctta aaaatatgta tgtgtaagta gcaggaacag
30720

ctcttttgta ttccaaaccc ccattctgcc cctgcaacta agaaaggtgg gggcggggtg
30780

gtggctcaaa cctgtaatcc cagcattttg ggaggccgag gcaggaggat cacttgagcc
30840

aggagttcaa gaccagcctg ggcaacgaag caagaccctg gttttttttt tttttttttt
30900

tttgagacaa agttttgctt ttgttgctca ggctatagtg caatcgcata atcttggctc
30960

actgcagcct ctacctcctg ggttcaagtg attctcttgc ctcagcctcc taagtagctg
31020

ggattgcagg catgtaccac cacgcctggc taatttttgt atttttagta gagatggggt
31080

ttcaccatgt tggtcaggct ggtcttgaac tcctgacctc aggtgatcca cccgcctcag
31140

ccacccaaag tgctgggatt acaggcatga gccactgcgc ccagccagca agatcctgtt
31200

tctacaaaat ataaaaaatt agccaggtgt ggtggcatgc acctgtagtc ccagctactt
31260

gggagactga ggcaggagga tcacttgaac ccaaaggcaa aaaaaaaaaa aaaaaaaaaa
31320

aagaagaaaa agaaaaaaaa aactattttt attgcttagg gacaaagttt ctgacttcca
31380

taaactcaag atcagctggc acggtggctc atgcctgtaa tcccagcact ttggaaggcc
31440

gaggcgggcg gatcacttgg ggtcaagagt ttgataccag cctaaccaac atagtaaaac
31500

cctgtctcta ctaaaaatac aaaaattagc caggggtgat ggtggtcgcc tgtaatccca
31560

gctacttgtg aggctgaggc aggagaatcg cttgaaccgg ggaggcagag gttgtagtga
31620

gccaagattg caacactgta ctccagcctg ggcaacagag tgagactctg tctcaaaaaa
31680

aaaaaaaaaa aaaaaaaaaa gctcaaggtc tggagtcaaa gcctctacct ctcccagata
31740

tctcctccca ctgtatttat caccttggtt aaaatccagg gaagggaaca atcagcaact
31800

tgttccactc ggggtagaac ttggcaggaa ctctcgaagt tgctgttact acctttccat
31860

tgcaaggcaa ttcataaatg tctaaatttc catagtacat cttggaggct gcatgaggga
31920

gaaagagcca gcaaccctta tgcctacaga caaaaattag tttttttttt tttttgcta
31980

tggggtctca ctctgtcacc caggttgagg ggcagtggca cgatcttggc ttactgtaac
32040

ctctgcttcc tgggttcaag cgatcctccc acctcctaca ggtgtgagcc actgtgccct
32100

gcctatctgg ccctttatag aaaaatgttt gccatctcct gttctagata aaagtagcca
32160

tctgagcagg gatgagggag ggtctcacca ttcgggaggt aaatatctat ttaatcctct
32220

tgttttctct gtagcgtcca caccctcaac tgtgcccggt attcctcagg ccagcggggt
32280

ctgattgctg gcctgagggg tgtgggatct cagacccacc atggatgggc agccctaaac
32340

ttccttctcc caccttcaat ggttaagtct ttacttctgt tggtatagag tggtggctgg
32400

gcttggactt ttcaggaagg agactggaag gtgttcctct ggagaaactg acccagggct
32460

aagagctaca gatactggcc gggcatggtg gctcacgcct gacatcccag gactttggga
32520

ggccgaggca ggtagatcac ctgaggtcag gagttcgagg tcagcctggc catcatggtg
32580

aaacgccgtt tctaataaaa atagaaaact tagccgagtg tggcggcagg cgcctgtaat
32640

cccagctcct cgggaggctg agacaggaga atcgcttgaa tctgggaggc aaaggttgca
32700

gtgagccgag atcgtgccac tgcactccag ccgggtaaca gagtgagact ctgtctcaaa
32760

aaaaaaaaaa aaggtcactt tataccacac tttgctcttg ttaatcagac tctgcaaata
32820

gcaagtgact gaaccctgcg attcagttac aacaaagaca tttgaatgga cagagaagat
32880

gtttgaggaa agctcctacc tttttttttt cttttttttt gactgagtct tcctccatca
32940

cccagactgg agtgcagtga tgtgatcacg ctcactgcag cctcaaactc ctgggctcag
33000

gcaatcctcc catctcagcc tcctgagtag ctgggactac aggcgtgtgc cactataccc
33060

agggaatttt tgtatatttt cttggtaagg acgaggtttt gccatgttgc ccagggtggt
33120

ctggaactcc cgggctcaag ccatcctcct gcactggtct accaaagtgc taagactaca
33180

ggcgtgagcc accacacctg gccagtgcta acctctttaa tgaggattgt tattgctttt
33240

tcttgaagtt ttgattgcaa atatatatag ttttcagtag tttgcccaac tctattaatt
33300

ttggtcttgt tttgcgtgcc agacaggggc tttcaaacag aacaatacag ttcataagga
33360

acacaggttt aataattgct cagaggaaaa aagacctacc tggtaccctc tccacaaaga
33420

caaatccaaa gacacaaaag aaagggaggg ccgggcatgg tggctcatac ctgtaatccc
33480

agcactttgg gaggccaagg cttgagccca ggaggtcgag gctgctgtca gccgagacca
33540

tcacgccact gcactccatt ctgggcaaaa cagccagacc ctgtctcaaa aaaaaaaaaa
33600

agtgatttac aaagtatatt tattttttaa aagatacatt atcatgcttg ctttcactca
33660

gataaaaatg ctacaagata ttttatgggt atagtaagag acagtatgat gccctattca
33720

aattttgtga aaattgaata ggttgctatc atttctacat acatctttct taacactttc
33780

tggaatttac tcctcttcct ctttccacat tgcttactgt acagagaagg aggctccata
33840

atgggaaggg atactatcac atttttaatg attattaaca gtaaaaacaa tgataaacct
33900

ccgacgtatt aatactacct gccacacaca ctgtggtgga tgctttctac aaattaacct
33960

aatccattcc tccctattac aagatattat tgctatgccc ctagaaacaa gggatttgaa
34020

agtcagaaag gttaaataaa ttggccaata agtggcagtc agttctcctg ctatactatt
34080

tctagaaaac aaacctttat tttttaaggg gcttggctat tttgagcaga agtgatctgt
34140

ggtcacacag atcttaatca ccactccccg acagagaata aacatttatc aggatggggc
34200

cggggaaggt ggctcacgcc tgtaatccca gcaatttggg aggccgaagt gggaggatca
34260

ctgaggtcag gagttcgaga ccagcctggc caacatagcg aaaccctgtc tctattaaaa
34320

atacaaaaat tagctgggct tggtggcagg cgcctgtaat tctagctact ggggaggctg
34380

aggcaggaga atcgcttgaa cccgggagac ggaggttgca gtgagccgag atcgtgccac
34440

tgcactccag cctgggcaac agagcaagac cctgtccccc atcccaaaaa accaaaaaca
34500

accatttatc tggatggtgt cgaaatattt accacttccc ctgcacttct gacttttcga
34560

actttgtgct tttgcctgag ttccatctgg aatgttcttc ttggatggtg gagcatgtcg
34620

tccacctaac caattcctac ctctcctttg aatacacacc acttagtgga agtcttccct
34680

gatccctgcc gacaagcttt tcaactgcat ctctctctta agctaaccca agtcagtacc
34740

ggcacccttg ctggaatgtc ccattttggg aaaggcagta tttcttgtat gaatcttcat
34800

ttgctctcta ctgggggtaa gtgcacgtta gagaataatg aaaacttcaa gtcaagggat
34860

gcagagtgcg tttaccagtt caacaacctg gactttctac caggccattt tcaaactgag
34920

ctgccacgga cagcagcatg gactttggtg ctagcggggt cggcctgaaa tctgcagcct
34980

gcatgctcca aggatgcctg aaaagaaggg tcgaaccatg ggccgctccc aggacccacc
35040

cttttagtct tcccagctgg ctcaggaaac aaaggggggc cggagagagg cggcgcccag
35100

ggcgcaacag ccggagactg gcgctgcgtg cctggggaca gaggcctcgt gctaggagcc
35160

ccaagcaact acacacctag ccgctggcaa gaacaaacag cagtgacagc aacaaacaga
35220

accgacagcc gcagaccgcc gtcgctgcgg cccacaaagg ccggaagtgt ggcgggtgcc
35280

ggcggccgag gcgccctctc taggacttcg gagggcccgc atctcggtgg ttccctatac
35340

tggaagaccc ccgattccca gggcgtgtac cctacgacgg gggcctcaaa atgagtcaaa
35400

agggagaaaa gcctgggcgc agtgactcgt acctgtaatc ccagtccttt gggaggccga
35460

ggtgggagga tcgcttgagg ccaggagttc gagactggcc tgggcaacag agagagacct
35520

ggtctctacc aagaattaat aaaatgagcc gggtgtggtg gggcacacct gtagccccag
35580

ctattcggga ggcttaagtg gaaggatcac ttgagccctg gagtcagagg ctgcagtgag
35640

ctttgcttgc accattgcac tccagcctgg gaaacacagt gagaccccat ctcaaaaagg
35700

aaaaaaaagg aaagaagaga gggaaggaga gagggaggaa ggacagaggg agggaggagg
35760

gagagaggga aggaaggaag agaggaagga agaaaggaag gaaggaaaga aggaaggaag
35820

gaaggaagaa ggaatcgaat tcagcggctc acctgtggaa gaggaaatcc atttacaatg
35880

catccaggcc tgagtttcag ctgaggggtt ggagcatggc tgattatcat cctggcttcc
35940

cccagagacc aaggaaactg aggcctggag caagttcttg cttgctcagg gtctcacagt
36000

gatccgggtc agaagccagg acttgtggga ccacgatttg tgccctttgc actgcctgga
36060

ctgcctctca tcataaatga atacggataa aattcgttat gctttcaaag tggtaggaaa
36120

cacatgacag atcaaagcag acaaaatagt gtgcaagact tttgagaaga cgcctcacac
36180

tcgtcgggat ggctacagtt taaatatccc ttatctgaaa tgcttgggac cggaaatgtt
36240

tcagatttca gaattttttt ttgactttgg aatatttgct ttactggttg agcagcccta
36300

atcccaaaat cctaagtctg aaatgctcta atgagcattt tctctaagtg ccatgatggc
36360

cctcaagttc tggttttgta acttttggag ctcagatatt tggattaggg atactctccc
36420

tgtactaaaa acaaacaaac aaacaaacac aaaaaaccac caccaccacc aacaaaacaa
36480

aacaaaatag caggtgtttg gtgaggatgt ggacaaattg gaatctttgt gcattgctgg
36540

tgggaacata acatggtgca gcccccgtga gaaatagtac agcgcttctt caataaaatt
36600

aaaaatagaa ttggctggtg tggtggctcc cgcctgtaat cccagcactt tgggaggtga
36660

aggcaggcgg atcatttgag atcaggagtt ccagaccagc ctgaccaaca tggcaaaacc
36720

ctgtctctac taaaaataca aaaaaaaatc agctgggcgt ggtggctcac gcctgtaatc
36780

ccagctactc gggaggctga ggcaggacag ttgcttgaac ctgtgagggg gaggttgcag
36840

tgagcaaaga tggtgccact gcactccagc ctgggcgaca gagctagatt ctgtcaaaat
36900

gaaatgaaat gaaatgaaat aaaataaaat aaaataatct ctgaaggcta aagagagaaa
36960

ttgggtcagc tgaagagttt acagatttgc agagaataga ggcaaggaag ctttgtgaac
37020

tacttttagg ttgtgaaact tggtcttgat tcagccccca catggtcttc atatacaaag
37080

cttctttgca gaggaagctc tttggagaaa gataactggt gagatagaaa gcacagccaa
37140

gagataacaa gggagacttc tctggagaat tttgtggatg aagaggaaaa acaaatccat
37200

aaggtcatgg ggataatgag ttatttgaaa tcagaaagag tgattaacat ggaggcgggg
37260

cgcagtggct catgcctgta atcccagcgc tttgggaggc tgaggcaggc ggattgcctg
37320

aggtcagaag ttcgagacca gtctggccaa catggtgaaa ccccatctct actaaaaata
37380

caaaaaaatt agccgggcgt ggtggtgtgc acctgtaatc tcagctactc gggaggctga
37440

gacaggggaa ttgcttgaac cagggaggtg gaggttgcag tgagccgaga tttcgccact
37500

acactccagc ctgggcaaca gggcgagact ccatctcaaa aaaaaaaaaa aaaaaaaaaa
37560

aaaagcaaac caaaccaaag cacacaaaca aataaacaaa caaaaaacat aaggcaattc
37620

tctacaggtg ttttactgct agaagatcat gctcctcatt tcctaaagac tcacagagaa
37680

cagaagactg gttgcatgag aagtgaataa caagaggtta tagtgcttat ttgggttggg
37740

ggcaaacatg acatttcaat attaacttgt ttatttattt atttttcggt tatacatcat
37800

tgctggtata tgtactttat tttatttttt agagatgggg ggtctcgcta tgttgaccag
37860

gctggtctcg aactcctggc atcaagccat ccacccatct tggcctccca aagtgttggt
37920

attacaggtg tgagccgctg tgcccaggct aactcgtttt ttttctttgg tccagcaagg
37980

cttgtgaatg tgtctgggaa cgacgtaaaa tctggcttga aaaaaaaact ctcaactcaa
38040

agctccaaga cagagctgag atgcttttga aggagacagc ctcattggtc tccttgtcgg
38100

agaggcccca acttatcagt aaatattggt taacatctat catctggtgg gactaagcat
38160

ggaaccaatt gcccttgcac agaaggccac gtattgtagt attcaacata gatggaatga
38220

ccaggaaagg caaaaccaca gatataaaaa gtagattaag gccaggcgcg gtggctcacc
38280

cctgtaatcc tagcacgttg ggaggccaag atgggcggat cacccaccag cctggtctat
38340

atggtgaaac cccgtctcta ctaaaaatac aaaaattagc cgggcatgtt ggtgcgcgcc
38400

tgtaatccca gctactcggc agactgaggc aggagaatcg cttgaaccca ggaggcggga
38460

ggcggaggtt acagtgagcc gagatggcac cactgcactc cagcctggcg acggagcaag
38520

aaaaaaaaaa taaataaaaa attgctaaga ttgttattaa atccgctggg agagtctttc
38580

atcatgttag caggatcatt gtgtcctctg gtggcaataa cacgcgactg caactgactt
38640

gcctgtgcag aaaaatcttt cctgaatcct gaaagctgca ttctgttcta ttagcttaag
38700

aaggaaatct gacactgatc cgcacggtgt gttttatgat cctgcacata aagattaggt
38760

gctcaaggat gcagaggagc ccactgctcc cagagcaggt gcctgagcct aggaggcatc
38820

tgaccagagt ctctgtcgcc caagctggag tgcagtggtg cgatctcggc tcactgcaac
38880

ctctgcctcc agggttcaag cgattctcct gtctcagcct cccaagtagt tgggattata
38940

ggcacgcacc accatgcatg cctgtaattt tttgtatttt tagtagagac ggggtttcac
39000

catgttggcc agactggtct cagacacttg acctcaggtg atctgcccgc ctcgaccccc
39060

agagggctga tattacagcg tgagccaccg tgcctggccg aacgacagga tttcaagcct
39120

gaactcgaga ggtagcccag actggggatc tgcactggga agtcatccgc aaggacgtgg
39180

tggctggagc tatagacaaa gggaaagtta tccagggaga gtgagggtgt ggaatgggga
39240

gagagagggc ccgcaacaca gtcctaagga acaccagcac tttttttttt tttttttttt
39300

tttgagacgg agtcacccag gctggagtgc agtggcatga tctcggctct ttgcaacctc
39360

cacctcccag gttcaagcaa ttctcctgcc tcagcctctc aagtagctgg gattacagac
39420

gcccaccacc atgcccagaa tttttgtagt tttagtagac acgggtttca ccatgttggc
39480

ctggctggtc ttgaactcct gacctcaagt gatccacctg cctaggcctc ccaaaatgct
39540

gggattacag gcatgagcca ccgtgtctgg cccagttatc gctttctgag agaggagttt
39600

ctccaaagtt ttcagtttga cataatcaat ataaccatcc tgcgtattag gggatggctg
39660

gttcttcact ccttcagcat ctttggttaa ccggtacctc gacttggcca tatccacagg
39720

tgggggaggt caggagatgt cacccctagt cactggggct gtggggaaca tggtctctga
39780

aatggaacaa ggctgaccag gccgatggat ggctcacagt gtctgctgga ggttcctttt
39840

gctgctcagg gccagggcgt ggctggagga agaggcccca cccaggaagt tccgcagggg
39900

cttacacagt cttcccagac ccagaagcac aatctccaaa agagtgaaca agagacggaa
39960

cccactgacc ccaaacatgg cttttagaaa gatgatcttc tccgaggggt gggaaaaggt
40020

gcaagttaat ctatacgggc aaggctcttg gccacatgca aaggagctgg gcatctggaa
40080

cccatacagg tggtactgca accccggcgc tgtcccctcc aggaccagct gagctcccag
40140

ctgagccaca taagctcaga gcagcctgag gcttccagcc cctggggtat ctctgctgct
40200

ctcccctccc tggatcaggg tgtcctcttc ctccgtcccc tttcatgatt cttcccagtg
40260

ccagatcacg tgatacagag tgaaacccat gtagaggacg ctaggtacag ccaccaagat
40320

gacctggaag acccagaaac gcagcgggga gagcgggtgg aaggcatcga agcaggcagc
40380

cttgcagccc ggctgctggg tgtgacacac gaattcactc tgctcatcgc catagactcc
40440

aggcccactg gcagccagca gcacaaggcg gaatcccagg agcacgggaa acaggaggcg
40500

ccccacgggg gtggagtgcc agctctcctc cgccagcagc caccacctca ggaacctgcc
40560

acacatcctg ttagggagtt ttaaaaaaag attattacac gttattgatt ttttgttttt
40620

aaagacctct gttgcccagg ctggagtaca atggtgtgat catagctcac tgcagcttcc
40680

aactgttggg ctcaagcaat cgtcctgcct cggcttcctg agtagctggg actacaggtg
40740

catgacacca cgcccagcta agttttaatg tttttataga tatggggtct tgctatgttt
40800

cccagtctgg tctcaaactc ctgacttcaa gcaatcctcc tgcctcggcc tcccaaagtg
40860

ctgggattac aggtgtgaac cattgcacca ggttatgagt gatttatttt ttattttttg
40920

agacagagtc ttgctctgtc gcctagggtg tagtgtagtg tcgcgatctc tgctcactgc
40980

aacctctgcc tcccggcttc aagagatttt cctgcctcag ccttctgagt agctgggatt
41040

ataggcgtgc accaccacag ctggctaatt ttgtgtattt ttagtagaga tggggtttca
41100

ccatgttggc caggctggtc ttgaactcct gacctcaggt gatccacctg ccttggcctc
41160

ccaaagtgtt ggaattacag gcgtgagcca ctgtgccggg ccatgtattg atttatgact
41220

ttgcctgtaa cttctgcttc tctgcctcta aaaaccctta cctacaagcc attggggaat
41280

ttaggtctta cacttgagct ggctgattct ccttgcttgg caccctgcaa taaatgcctc
41340

agtttctctc actggaatcc acatgtcagt gtttgcagca cccggtggtt ggacccaagt
41400

tcgatgaggt aacagacgtc cactgggtgt gctgtaatcc cgcggaggtt ttcctccata
41460

ataaaaggca ggaattggct gggcgccgtg gttcatgcct gtaatcccag cactttggga
41520

ggtcgaggcg agtggatcat ctgaggtcag gagttggaga ccagcctggc caacatgatg
41580

aaaccccgtc tctactaaaa atacaaaaaa ttagctgggt gtggtggcgg gcgcctgtaa
41640

tctcagctac atgggaggct gaagcaggag aatcgcttga acccaggagt tggaagttgc
41700

agtgagccga gatcgcgcca ctgcactcca gcctgggcaa caagagtgaa attccctctt
41760

aaaaaaaaaa aagacggcga gaattgtgtg agttgtgcga ggaagaccgg atgaccgtta
41820

tctcatggcc tttgcatcca ggctttgagc caaattctca aggactggat cctaacactt
41880

ggctgcaact atttccttga ccactagggg gcaggcaaaa gccatagcga atccggccta
41940

gcgctgattg gaacctgctt gagaaaaaga cctctttgtt taagccggcg gtccccaatc
42000

tctttggcac tagggaccgg ctccgtagaa gacagttttt ccacggactg gggaggggaa
42060

tggttttggg atgattccag tgcatcccgt ttattgtgca ctttatttct attattatta
42120

catcgtaata tataatgaaa taattctaca actcgccacg gtgtagaatc agtgggagcc
42180

ctgagcttgt tttcttgcaa ctagatggtc ccatctgggg ttgatgggag acagtgacag
42240

atcatcaggc gttagatctc ataaggagcg ggcagcctac atacctttct tgcacagttc
42300

acaatagggt tcgtgcttct gtgagagtct aatgctgctg ccgatctgac aggaggtgag
42360

ctcaggcggt aattggagca atggtcgggg ggtgggggag cagctgtaaa tacagatgaa
42420

gcttcgaccc tgcagctcac cttctgctgc gtggttcggc tcctaactgg ctatgaacgg
42480

gtaccgggag ttggggaccc ctgccttaga ctatcttgtc tctctctggt ggactctcct
42540

ggggatagtt cccaggtagg ctggaggaat gatgcacact gaatcaggca aaagacatca
42600

atcagatata gcaaggtata catatgttaa ggtcctcttc aatttctctg agctttgctt
42660

tttcaaactt ctttcccagt agaattcact gtcccagcca aaaggttctt cgtggtcgtc
42720

agaaatattt agtgctttct attgcctttg cttacgtatt tattgattca tccagaaatt
42780

atgtattaat tacctattgt attaatatgt gtatttattt atttattttt gagacacggt
42840

cttgctctgt tgctcaggct ggagtgcaat agtacaatca tagctcacag cagccttgac
42900

ctcccatgct caagtgatct tcctgcctca gcctcccaag tagctgggac tacaagtgtg
42960

tgccaccatg cccagataat tttttatttt tttaaatgta gagacagggt cttgctatgt
43020

tgcccagact gatctggaac tcctggctca agcaatcctc ccactttggc atcccaaaga
43080

gctgagatta caggcatgag ccactgtgcc cggacctatt gtatatttga ttctctggta
43140

ggcactggag actcaaaaga acagggaatg taaaatccca ttctgcatgg aaatcttttg
43200

acctcgactt ggcaagctgt cttaggcctc cacatctctc tgaaatccga attgctccag
43260

gaagttttcc ctgagtatct cagtagccct gtgtccctct cctcctgccc cacacctgcc
43320

caggacttga atcctgtggc ttctctctcc tcctttatca tcatccatgt ctcctgttat
43380

agatgaggtc acatctccta ttccaatttt ccttagccct ggtctgatca tgtggatttt
43440

ggtttcttcg ttgatcttaa atgtttaact ttcacctttt atttgctcca agtgtcctca
43500

tattcacatt tcacatgcta tattttttcc tcttgattat ttgcattttc tttttttgag
43560

acagagtctt gcactgtcgc ccaggctgga gtgcagtggc gagatctcag ctcactgcaa
43620

cccctgcctc ctgggttcac gtgatcctcc tgcctcagcc tcccccgtag ctgggattac
43680

aggcacacat caccaaaccc agctattctc ctgcctcagc ctcccaagta gctgaaatta
43740

taggcacaca ccaccacacc cggctaattt tttgtgtttt cagtgaagac ggggtttcac
43800

tatgttggcc aggctggtct tgatccttga cctcgtgatc cgcccgcctg ggcctcccaa
43860

agtgctgaga ttataggcgt gagccaccgc gcctggcgac tatttgcatt ttctttcctg
43920

aacttggcta ctgttcatat aggatgtttt tgccctggaa gggagaaagg tggatttcat
43980

gtagacttta aaaaaaaaag taatttctca gctttgattt tcaaattgga gtcgaagagt
44040

taaaacaaaa aattgtagcc ttcaaattta ggctttcatg aaagataaga tgattaactc
44100

agaattccaa agactgggaa tgattcttct atggaaccca gcttggagga tggattttgg
44160

aataatccag agtgggaggc tgagtatagg atgtaaagaa agttcttgtc gctagacaca
44220

gtggctcacg cctgtaatgc cagcattttg ggaggccgag gcaggcagat cacctgaggt
44280

caggagttcg agacgagcct tgccaacatg gtgaaaccct gtctctacta aaaatagaaa
44340

aattagccgg gcgtagtggc tgatgcctgt aatcccagct acttgggagg ctgaggcagg
44400

agaatcgttg gaacccggga ggcggagttt gcagtaagcc aagattgcaa cactgcaatc
44460

caggctgggc aacagagcaa gactccatct caaaaaaaaa aaaaaaaaaa aaaaaaagaa
44520

aaaggaagaa aaaaaggaaa gttcttttcc cacaggaagg aaaagcacag acacaggcag
44580

gtgaggcttc atgagaaggt caaggtgaga gctctgctgt gtgtttgggg ttgatacctg
44640

tgcatggcag ttggcgactc atttgaggaa actcactctc agggactcag agggagtctg
44700

agaacctcag ggccaattcc cgcttcattg agctgtaccc tggagagctg agagctctca
44760

aataagcttt ggctggtggg ccaaggcgaa catggtgtgg agatgtggag cagctggagt
44820

ggagggctgg gtctcccagg aagccaagaa gtgtggggaa actgaggcac agaagaggct
44880

tggctgtgtg aaagagcttg cagggttcta gcacctcaca gccagagtgg ccatggccac
44940

gcctgtggat gtcagcaggg gacacctgaa caatccctaa gggacagcgg gtccgggcca
45000

tgcttgggat tgcccaagtc agggtgccca ggaaccatgg cctccaaggg aaaccaagag
45060

tttcagatga attcagtgct tgggagcagg accaatgaga aaatcacatc acatttctat
45120

agcattttac agttaatact gttctctgtc gtgcttgaca aagagagatg attatttatt
45180

agtttccaca gattagacaa tggcgggggg tggttcaagg tgagatggtt tttgggtccg
45240

agtcagctca ggacaggcat cccagtcttc ggtctccaaa tccacctcct gtctgtcccc
45300

ccacactgct ccttgggcct tgaggatcca ttgaccgtga tttcggtggt tcagctccca
45360

catcaggcag gaagggcagc tactggggct gagatcccac attgcctcca gcccttgctt
45420

cctacctggc ctcccccggc accacgaggg gctgggccag gctgctgtgc tgcacgtggc
45480

aggagtaggg ggctgtgtcc tgcgggggca catgcaccaa caaccaggtc aggtaagtgc
45540

cgtttccacc gtgaagaaca tctccccgta actcaggctc ctgcacctcg ccggcccgag
45600

tccagtgcac atcaattttc cctgggtaga agtcgtaggc caggcacttc agtttcttct
45660

tttctcctgg ggcctggtgg ctggtgacca ccacagaggg aggatctgtg gagggataga
45720

gtgtgacact gcaggtttga ggtcgacttc tcccggccca ggtctcttct cacccctacc
45780

cctggatgtc agcaatcagc agagctctaa gctctgtatc tgcccgcccc actgccccct
45840

gccctgggac actttccaga agatcaggga aggctgaggc tttccctgat ccttctctgt
45900

tggatcatgg aatgtcctgg ctcccctctc gcccattctc caccccaccc atgtattgac
45960

tctgataagt tgtgaatgct ctgtgcactt tcccatgttt tcccacatct gagtgggctt
46020

tggagttctg tcccccaatc tcagtccagc tgcttggttc agcccatgcc tctatccagc
46080

aaccgcttcc ctgcagcact gcacaatcct gagtctcact atgtctgcaa tccaagtctc
46140

agcttgcagc catcgattcc attcacactg gctcgctgtt gtctttctgg ggctgacatt
46200

ttacacacct cccttctctc caccacccag atgggaggca tcattgtaga gagaaacaac
46260

gatggcattt ggaatctgaa atctggaatt caagtcctag ctcttccttt tttttttct
46320

tttttttttt tttgagactg agtctcacac tattgcccag gctaaagtgc agtggcgcga
46380

tcttggctca ttgcaatcta gtcctcctgg gttcaagcaa ttttcatgcc tcagcctccc
46440

gagtagctgg gattacaggc gcctgccacc acgccaggct aatttctgta tttttggtag
46500

agacgaggtt tcatcatgtt ggccaggctg gtctcgaact cctaacctca agtgatccac
46560

ccaccttgga ctcactccca aagtgttggg attacaggcg tgagccagca tgcctggctg
46620

cagctctgcc tcttacacac agtgttaccc tcacctctgt cacctccagc tcctcatctc
46680

tcaatgggtg atatttaggg aagcctacca gtgaatcagt ggctttcctg gcccattctg
46740

cctgaaattt tgatgtcctc ctaggaaagg aatgaatcct ggaagttgaa aactggggtt
46800

ctacgtgact tacatggata gaggagattg gcctaagatg ctctctgagc tcccagcctg
46860

agatcacctt ggtttttccc agcttagcac tggatagcag gaagcagtga gtaccttgcc
46920

ggtccaggat attttcgctg tatttcaggt atttccgcag agttgcaggg cactcctcct
46980

ccaggtaagc cttggcccgc tgcacgtaga ctggttctgc ctcctacttc tgcttggtgt
47040

tctgggcttc cgggaccaag gggactcagg ctgggatttc tttgttgaat tcaatgtagt
47100

cctttccatc ataggcattc ttccagaatg ctccagtgct tctgttattc tggatctcac
47160

aaccaaacct ttcctgcaag gcatgagacc ctgaaacctc ccctgacccc acaggaagcc
47220

agtcagccta gtgccaactg aataactctt agtcctgcac tcttcacaac tcaataggag
47280

gaaaggcttt tgcaatctaa gcatgcagtt ccgtaatcat ggcatatgat tttatctgga
47340

cgtacagttg acacagagtg gcaaaggcta tatggattgg catttcgggc caatttgaga
47400

tgaaatatgc attcctccca gccagggaag ttaagagagc tacaggccgt gggcagattt
47460

ctgtctcttc tgtagcatct ctttttattt gtttgaactt acggggagta tttaggaacc
47520

tgtcacaacc acaccaaagt acatttccga atttgataga aattatttgt gattccatat
47580

gaggaaagga aggctctcct tttctctcag ttctaggtgg agttcatatc atcacctaaa
47640

tcaacctaat ctgtctgact atttctttgt ttcttttttt tgagacagag tcttactctg
47700

ctgcccaggc tggagtgcag tgtcatgatc ttggctcact gcaacctcca tctcccgggt
47760

tcaagcaatt ctcctgcctc agtctgctga gtagctggaa ttccaggtgc gcaacaccac
47820

acgaggctaa tttttgtata tttagtatag atggggtttc gccatgttgg ccaggctggt
47880

cttgaactcc tgacctcagg tgatctgact gccttggtgt cccaaagtgc tgggattaca
47940

gatgcgagcc actgcgcctg gcctgatcac ttctttcttc ctttctggtg tttgatatct
48000

ttatcccaaa tctctaaagt cccaaccttc ttgcccatta gcaacaactc tcagaaatgg
48060

acctcatagg gcagaggaca cagcctcttc attctgatta taaaaacagg tgtttaccta
48120

gcacaggttg gtgctgttgg catagactct cacagctctc aagctgtctg ggctcaagtc
48180

ccaggtctct tcccgcccca acctggtgtg ttgtgtgaac tggagccagt cacctccttg
48240

tgcctttatt tcctcacccc tgaaacagca gtgccaaaag cacggtttca gggggttatt
48300

ctgagcatta tatatgctca taaatgggcc tggcacaaag taagtgctca gtaaatgtca
48360

gctactctta ttttctaata ccctgtgttc atctcttcat cctccttcct cgtaattatc
48420

cactttgcca tttctattcc tgtccatcaa tgcttccccc agtgtctttt ctgatcacct
48480

gtgccccctc ctctcctatg gctaacacac agcctgggtc attttcctta ccaagacctc
48540

ccatccctca tccttcaaac gtctgcttat ctcttgcctc ctttgggaaa ccttcagatc
48600

ccatgtagca ccttgggcat ttttgtactt agaagcccat cttttgatac ccactccatg
48660

ataatttaac accagcagtt caccaggcct ggggttggct gccttggggc tggcagtgtg
48720

gtttgggaga aagaacgctg ggccgggggc agggggaccg tctctgcacg ggctctgcca
48780

ccagctcctc cgaccacagt gcaggtggcc cttcctgggt ctgcatctta tggtgaggag
48840

gaactttagg gctgcaggga tctctaacag cctgtggttt tgaatgcagc acttcctgag
48900

cccccagcat gggctacggt gtgtgctggg tgcttttctc tgttgattcc acaaatttca
48960

attgagccaa tattggctgt accctgtgct aggtgatgga gctgcaaggg agatcaggac
49020

agactgtgac atccttcata gaacagacat agttaaatat tacagacttg tttggagata
49080

ggtctcagtg ctccaatgtt tgcagatttc gaccagatat tttgggaaca tttcactgtc
49140

aacaggtagg actggcagag ccaccaggtg agcccaggtc tgagtgactt tgagagtggc
49200

tcattctcca ggcaacctcc ctggacaggg gtgagtctga tgccttctca tggggagatc
49260

agcctctgat gggtggggag tggaggtgag aggtcagagg tgcctagata ggaagtgact
49320

ctgccatcac ttcctgtgtg acctgagacc tgggatgagg atggggcaat cgatggggaa
49380

ggggacaaca ctgtgctgtg gggggggtgg gatgcggaca tgtctgtgtt tctgctgtgt
49440

ctcctgttcc tcccctggga ttgggactat ttccatcctg ctgacccctt gccactcaca
49500

cctggggggc tgcctcttgg gttagacctt ccacccccgc ggtctgttgt tcactgaccg
49560

ttaccgtcgt tgtaatactc catgatgttg ttcagggtct ccataaagat gtcctccctg
49620

gccttctgaa cttggctctg cttctcccag tcctctactc cttccgcatg tctccatggt
49680

cccaggggct cagccttcct gtcttcactg ttgtagtgga agaaggcatg gccattgagg
49740

aagacagtgc cctgcagcct gtgggtgcct ttgccagacc tggacagccc agtgtagaga
49800

taggtcagag agtaatgacc tgcaaaagaa aagactctga gggctggggt ccatgcaagg
49860

gtgtcccatt gtggggctgc agagggccag gaggggaggc ctggccactg gcctcttcct
49920

ccccagctct ctcctctgcc atcagcttca caccattgga gcctcactca aggaaacagg
49980

ctctactttc atgctgggga tgatatttca gagaccattc tgtttcttca ctacagagtt
50040

taatctgctt ggcacagaag aagttaacat ttctcagcct ccttatagat agattggaac
50100

catgtgactg agttttgtca aacaagaatg tgagtggaag tgactgatgt ctttcttctg
50160

gtccaaggct ctaccatctg gcttgcccct acggtctctt atccctttcg ttgactgtgg
50220

aggccacagg atgaaacaga agagtcccaa gatgcaaaca gcctggatcc ctgagtcacc
50280

ctgtggagga gaggaacctt gcccaatctg cattgaactt gacatgagaa ataaacattt
50340

ctagtctaac cggacaaata catatagtta gaaagtgtag gaaggtgaat ttactagaaa
50400

actcaagaag tgacccagaa tggatcagca tagggtaagg ctaggatacc tctcagaatt
50460

cctggcattg tatcttgcag tttggtacaa gatggaagct gagagagtgg gacatttcca
50520

caattagttc catagttaat ttagaatata atttacatga gtgttagtaa tgttggaagt
50580

catatggcag atttctaatt taagtactga agtcattctt gctcatgagt ttctgaatgt
50640

gattagcctt taaatcagac cttgagtaaa acagattacc ctctacagtg tggatgggcc
50700

tcatccaatc agtggaaggc atcaagagaa aaggctgagg tcccccagag aagaaagaat
50760

tctgcctcta gactaccctt ggactcagga ccgcaacacc aactctttgc ttggtctcta
50820

gcctgctggc ttgctttgct ggtttaggac ttttagccct cagaattgtg tgagccacaa
50880

gaaatattaa ataaaactcc tccctttctc tgttttgtat atacatacac gtatatatgt
50940

atattataca tatacacata tatttagtgt acatatgaat atacaatgta tattcatata
51000

tacatacgtg agtgtttata caatgtatat tagttttgtt tctctggaga accccgaata
51060

attcatattt gggtgcagaa tgggattaat attataaatg tgattattat aaaactaacg
51120

tgaaacacct attttgacaa tttgtaaatg tcatgaattt tattttattt tatttattta
51180

tttatttttt tgagacagag tctcgctctg ttgcccaggc tggagtgcag tggcgcgatc
51240

tctgctcact gcaagctcca tctctcgggt tcacaccatt ctcctgcctc agcatcccga
51300

gtagctgaga ctacaggccc ccgccattac gcccggctaa ttttttgtat atttagtaga
51360

gacggggttt caccgtgtta gccaggatgg tctcgatctc ctgacctcgt gatccaccca
51420

cctcggcctc ccaaagtgct gggattacag gcgtgagcca ccgcgcccgg catgaatttt
51480

aaactcacac aactgagaag ccattcactt ccatgtcttt atcctgcaca ttctcatgcc
51540

acaaacagcc tccttcccct ctcctataaa tgaatcttga tctccccaag ctcccactgt
51600

cttctcttcc ggccactcgc tttccagacc cactgcagtg gtgtctgagt gtggggtgag
51660

ggcacgggtt tccttctagc accatccctc cagccaacct gccttcccac tttccccttg
51720

ggggctgttc tctcttttcc tcagcgtcag gcaggtgtac ccagccatcg gaaggaggga
51780

aggaacagga gcagaagcag ctccaggcct gtggttcctg ctccgtcctg gctctccacc
51840

ccaagcaggt cgcttggtct ttggagtctt tgttttcact gtaaactaag ggttttaga
51900

ttcagattcc tgaaggtctt attttgggtg agggcagagg ctccctgaat gctcctggaa
51960

gtgtgtgagc tgagggagtt gacagccttg gacacctgtt cctgccatat gccagggaat
52020

cccagctgca tccagccctt ctcccagcca catgtcctgt tcctcacctc cttccagtcc
52080

tctccagcac ccatcccttg ctttccccac tcaccatctc gggtctcctg gaggacagca
52140

ggacccagaa gcagcagcag agacagcagg acagacacca ttcttaccat tgtgtctgct
52200

tggaaggttc tgggcaggag gcacaggtat tatcctgggt cctggggcca tgcccagggg
52260

aggtgaatct acaggccaat gggagtacca ggccgggctc ctcccctgac agtaagaaag
52320

aagtatgcac aagccaggct gggcagaggt tgtgatcagt gagtctacac acaggatatg
52380

caaatttaga gctgagacag ttgatctctg gactgaggat ttgtgtgtgc ctggggcgga
52440

aggatgccag tgtgtgggag gtgtgtgtga tgggtggcag gtgcctggga tgtgtcgccc
52500

ttccagtcta ttatgacacc ctctggggtc acttgggtgt tgtcagacca cacttcatct
52560

ctgccttctg gaaaaactct caacatgtcc aagacacgga catgggtaga cccccgctgg
52620

ggtgggaccc tcactctccc acatccgatc ctgccagagc tgtctggagg acattcccat
52680

tcccatctct ccctctttcc tttaggtgcc tgtggaatcc cagccttcca tgatggaagc
52740

agaaatagcc gcatcctcct tttgcagcct ccctggcaat agaacgagca ggtgtgcaca
52800

gggtttatgc tcagcaagtc gaagctactg ccttggatat caggtcaagt gttagggaaa
52860

caaagaggag ggcacagtgg acaatgtttt ctggtggtct tggcaggggt tgcaactgcc
52920

agctcccggg gcagtggggc tgcagtagct gcggggggcg tcttgagctt cagtcccccg
52980

ggatgttgta gatcccgtgc tgtggcctct gctgccccct gctggcagct tctgtatatt
53040

tactgggccg gttgcaggtg tgattttgac tgttgggctc ctctggaatt ctgagcctgg
53100

gttctccatc ctttctgtgg attctgagag ctaaccaata aatacgttta ttttctgctt
53160

aaggtcagaa ctgcattacg tttccaataa caaatactcc tggcggatac agagattggt
53220

acaaggattg tttccaggca gtatttactc agggaaatgg agggctggga agctgaggtt
53280

ggttactggg ccagattggg gctgcgcagt gaaaacacaa gagtcttggg ggattctggc
53340

aggcctggtg tgcagtgata acttcaacag ttggctgagg tatctgttat ggtagattgg
53400

tgtagggacc agccccacag ggtcggaggg tttttctccc cgtgtgcaga gacgagagag
53460

tgtagaaata aagacacaag acaaagagat aaaagaaaag acagctgggc cccggggacc
53520

actaccacca agacgtggag accggtagtg gccccgaatg tctggctgcg ctgttattta
53580

ttggatacaa agcaaaaggg gcagggtaaa gagtgtgagt catctccaat gataggtaag
53640

gtcacgtggg tcatgtgtcc actggacagg gggcccttcc ctgcctggca gccaaggcag
53700

agagaaagag gaagagagag agacagctta tgccattatt tctgcttatc agagacttag
53760

tactttcact aatttgctat tgttatctaa aaggcagagc caggtgtaca ggatggaaca
53820

tgaaggcgga ctaggagcgt gaccactgaa gcacagcatc acagggagac ggttaggcct
53880

ctggataact gcgggcgggc ctgactgatg tcaggccctc cacaagaggt ggaggagtag
53940

agtcttctct aaactccccc aaggaaaggg agactccctt tcccggtcag ctaagtagcg
54000

ggtgttttcc ttgacactga ggctaccgct agaccaccgt ccgctcggca aggggcgtct
54060

tcccagacac tggcgttact gctagaccaa ggagcccttc tggtggccct gtctgggcat
54120

aacagaaggc tcgcactctt gtcttctggt cacttctcac tatgtcccct cagctcctat
54180

ctctgtatgg cctggttttt cttaggttat gattatgcag cgaagattat tataatattg
54240

gaataaatta taatattgga ggattattat aatattataa tattggaata ataaatttgg
54300

aaaaaactaa tgattaatga tattgatata taatcatatc tatgatctag atctagtata
54360

actcttgttg ttttatatat tttattatac tggaacaact catgccctcg gtctcttggc
54420

ttggcacctg gatggcttgc cgcccacatc tccccccttt ttattaacta ggatcgccat
54480

cgccatcatt gcttgtcgtt gacttcggat ttgttttcgg actccttgga ggcatctgca
54540

ggctaaaagg agacaacgta agcataccaa tattaataat gccagtgaca acaatgatcc
54600

tccaaggggt ttgatctatt taaagggatt aagatcagat aattgtttag ttatgccttc
54660

aaaaatgtct gagccaggaa cagtggataa atgagcttgt gaatcctcga aaatttgctc
54720

tttaagtttt gaaatatcca aggttaagtt atcatcccag gcttttaaat gtcttgagac
54780

attttcccag ctatgttgat atttattata agcataaggc attatgcaat aatcagaagt
54840

attccaatca ctctgtaatt gcatacggtg ttccaaattc atatctccca gccagattac
54900

actttggcgg agatcattaa tttgattagc taatttttga tcaatttgag cctgagaatt
54960

ccaaagtctg gaggagtttt tctgccatgc ttcaattcaa catattgaac ggtttgaaca
55020

gaattgtgga tggcaactcc agcagttgct gctgttgcag taaccgcaat tagtcctgca
55080

atgatggcaa taagagtaaa aataaatctc tttgttcttt tgcggatacc tttaagaact
55140

tcattgacta tgtgaataga gggggaagac tcccatggac gatgtaaaga aactggtatc
55200

cataccccct ccctagccct taccaagaga gtacttgtag tgggattaaa agtagcatca
55260

atgcacgtga acagcttaca gttatcacat tctatagttt gtgtattggg aatgataatt
55320

atatttccaa ccaacagcat gtaagggggt ttgacatagc tcctgatggg tatcacccgt
55380

tcagatatga gggtgatgtt gaatgtgggt gttttggtat tagtgtgaag gagttgatag
55440

gtagtgttcc atatccttat tcctgtcata gctgcagcta attttcatag ttgaggatgt
55500

tctggggtaa caaagggatg aatcattttt ggtctaggag gagtaatgcc tgcatccatc
55560

catttaaata ggtaagggga cacccattgt ctcagcctgt atgactgcca tccatcctct
55620

acataatcta acaaataatt aaactctgag catgaggtat ttttgctgga gcaatcttgc
55680

caataatgcc cttttggagc ccaatcaata acaatacctg cggctagact ttggagcaca
55740

acggccttag gagcattaca attattccat aaaattgaag tcactataaa aggtcccttt
55800

gtaggtttct ttaaccaaaa gagaggctgg gcacagttgc tcacactagt aaacccagca
55860

ctttgggaga ccgaggcggg cagatcacga ggtcaggagt tcaaaaccag cttggccaac
55920

atggtgaaac ccctgcctca gcttcctgag tgctgggatt ataggcatgt gccaccacgc
55980

gcagctaatt cttgtatttt ttcagtagag acgaggtttc atcatgttgc ccaggctggt
56040

ctcgagtacc tgacctcaac ctgaggtgat ccaaccacct cagcctccca aagtgctggg
56100

attacaagca tgagtcaccg cacccgggcc ccagtcactt tagaatagca tgttgctttg
56160

tattcggagg gtctctctgc aaatagccca tcaacactga gcgtgcctgg aaagacctgg
56220

ttttcaaata actggcttcg tctgtgtaaa acgagtcttg ttgtatgcat taaaaattat
56280

cttggctggg cgtggtggct cacgccttta atcccagcat tttgggaggc tcgttctgtt
56340

gccagggagg ctgcaaaagg aggatgcggc tatttctgct tccatcatgg aaggctggga
56400

ttccacaggc acagaaagga aggagggaga gatgggaatg tgactgtcct ccagacacag
56460

cctctggcag gatcggatgt gggagagtga gggtcccacc ccagctgggg tctacccagg
56520

tccatgtctt ggacatgttg agagtttttc tggaaggcag ggatacagtg tggtccaaaa
56580

acacacaaat gcccctactg gcccaggggt tgtcacaata gactggaagg gtgacacatc
56640

ccaggcgctt gccacccatc acacgcacct cctacccact ggcatccttc caccccaggc
56700

acacacaaag cctcagtcca gagatcaact ctggactcag ctctgaattt gcatatcctg
56760

tgtgtagatt cattcttcat aacctctgcc cagcctagct tgtgtatcat tttttttct
56820

ctattagggg aggagcccgt cctggcactc ccattggcct gtagattcac ctcccctggg
56880

cagggcccca ggacccagga taatatctgt gcctcctgcc cagaaccctc caagcagaca
56940

caatggtaag aatggtgcct gtcctgctgt ctctgctgct gcttctgggt cctgctgtcc
57000

cccaggagaa ccaagatggt gagtggggaa agcaagggat gggtgctgga gaggactgga
57060

aggaggtgag gaacaggaca tgtggctggg agacaggctg gatgcagctg ggataccctg
57120

gcatacggca ggaatgggtg cccaaggctg tcaactccct cagctcacac acttccagga
57180

gcattcaggg agcctctgcg ctggcccgaa ataagacctt caggaatctg aatctaaaac
57240

ccctagttta cagtgaaaac aaagactcca aagaccaagc gacctgcttg ggtagacag
57300

tcaggacgga gtaggaacca tatgcctgga gctgcttctg ctcctgttcc ttccctcctt
57360

ccgatggctg ggtacacctg cctgacgctg agggaaagag agagcagccc caaggggaaa
57420

gtgggaaggc aggttggctg gagggatggt gctagaagga aacccgtgcc caaatcccac
57480

actcagacac cactgcagtg ggtctggaag gcgagtggct ggaagagaag agagtgggag
57540

ctccgggaga tcaagagtca ctcctaggat aagggaagga ggctgtttgt ggcatgagaa
57600

tgtgcaggat aaagacatgg aagcgaatgg cttctcagtt gtgtgagttt aaaattcatg
57660

acatttacaa attgtcagaa aaggtgttat atgtttgtta tataacaatc actttggaat
57720

gttaatctga ttctgtgcca aaatctgaat tactcagggt tctccagaga aacagaacta
57780

ataggtggta cacatataca tatatatgta cgtacacata catacataca ctgtatacac
57840

atggatacac acacacatag gaagagattt acatatatgt atacaaaaga gagagagagt
57900

agagatttat tttaagaaat tgactcacac tattgggagg agtaacaagt cctaaatctt
57960

cagagccggc cagcaggctg gagacccagg gaagagttga tgtcttagtc ttgattccaa
58020

gggcagactg taggcagaat tctttcctct ttaggggaca tctgaggctt tttctcttaa
58080

ggccttcaac tgattggatg aagcccacca ctatggagag taatccactt tactcaaggt
58140

ctactgattt ttttgtaaat taaaaaaaaa actgtgggtg catagtatgt gtatatattt
58200

atggggtaca tgagaggttt tgattcaggc atgcaatgtg aaataatcac atcatcaaaa
58260

atgaggtatc catcccttca agcttttatc gtttgtgtta cagacaatcc aattatactt
58320

ttttggttat tttagttttt aaaagtattt gattatttat ttatttattt atttttgaga
58380

cagagtctca ctctgtcacc caggcaggag tgcagtggca tgatctcggc tcactgcaac
58440

ctccgcctcc caggttcaag caattttcct gcctcagtct cctgagtagc taggactaca
58500

ggcacctgcc accacacctg gctaattttt ttgtattttt agtagagacg ggtttcatca
58560

tgttggccag gctagtcttg atatcctgac ctcgtgatct gcccgccttg gtctcccaaa
58620

gtgccgggat tacaggtgtc agcaactgcg cctggcctct cttttggtta tttaaaagtg
58680

tacaattaaa ttatgattat tattattatt tttgagatgg attcttgttc tgtcacccag
58740

gctggagtgc agtggcgtga tcttggctta ctgcaaacct ccgcctgttg ggttcaagca
58800

attatcttgc ctcgggtgta cactgccaca cacggctaac ttatgtattt ttaatagaga
58860

tagggtttca ccatgttggc tagactggtc ttgacctctt gacctcaagt gatccactca
58920

cttcagcctc ccagagtgct ggaattacag gcacgagcca ccacacctgg ccccagttaa
58980

attattattg actatagtca ccctgttgtg ctatcaaata gtaggtctta ttcattcttc
59040

tttttttttt tttttttgtg acagagttgc ccaggctgga atgcagtggt gcaatcttgg
59100

ctcactgcaa cctctgcctc ccgggcttaa gcgattctcc tgcctcagcc ttctgagtcg
59160

ctgggactac aggtgtgtgc caccacgccc ggctaattta tgtattttta gtagagatgg
59220

ggtttcacca tgttggccag gctggtttcg aactcctgac ctcaagtgac ccacctgcct
59280

cagcttccca aagtgttgga attacaggca tgagccacca cacctggccc cagttaaatt
59340

attattcact ggagtcactt tgttgtgcta tcaaatagtt ttctaactat tttttttgta
59400

cccattaacc accctcccaa tttcccccca accctgccac tacccttccc agcctttggt
59460

aaccatcctt ctactctcta tgtccatgaa ttcaattgta gggtctactg atttaaaggc
59520

taatcacatt tagacactca ggagcaagaa taattttagt aattgaacta ggattctgcc
59580

atatgacctc caacatcatt agcacctgtg taaattgtat cataaaataa ttatggaact
59640

attatggaaa tgtccctctc tcccagatcc caccttgtac caaaatgcaa ggtacaaccc
59700

cgggaattct gagctccatc ctagtcttac cctgtgctaa ttcagtctgg gtcatttctt
59760

gaattttctg gtaaattctc ctttctaccc tttctaacta tatgtatttg tcaggttaag
59820

ctagaagtgt taattttttt ttttttgaga tggagccttg ctttgtcacc taggctgaag
59880

tgcagtggca tgatctcagc tcactgcaag ctccgcctcc cgggttcatg ccattctcct
59940

gcctcagcct cctgagtagc tgggactaca ggcacccgcc accatgcttg gctaattttt
60000

tgaattctta gtagagacgg ggtttcacca tgttagccag gatggtctcg atctcctgac
60060

ctcgtgatcc acccgcctcg gccccctaaa gtgctgggat tacaggcgtg agccactgag
60120

cccggacgaa atgttaattt gttttttttg agacggagtc tcactctgtc atccaagctg
60180

gagtgcagtg gcatgatctt ggcttgttgc aacctctgcc tctctggttc aagtgatttt
60240

cctgcctcag cctccagcat gactgggatt acaggcccgc accaccatgc ccagctaatt
60300

tttgtatttt ttaatagaga tggggtttca ccatgttggc caggctggtc ttcaactcct
60360

gatctcaagt aatctgcctg ccttggcctc ccaaagtcct gggattacag gcatgagcca
60420

cggagcccag cctagaaatg ttaatttcta acgcatgtca gattccatgc acactgggca
60480

aggttccatt cctccatggg gtgactcagg gatccaggcc aattgcatat tgagactctt
60540

tcatattatc ctgtggcctt caaagtcgtc acctctaggg atgagaaaca aaagggacaa
60600

gccagctggt agggtcttgg acaagaagaa agacatcact tctgctcaca ttctcttttg
60660

acaaaactca gtcacatggt cccaatatat cttcgaggtg gctgagtaat gttatcttcc
60720

tatgtgtcaa gcagaggaaa taatgtagtg aagacacagg atggtctctg aaatatcatc
60780

tcaggcatga aagtagagca tattcacttg agtgagcctc cagtggtgtg aagttgatgg
60840

caggagaaag agctggggaa gaaaaggcca gtggcaggtc tcccctccta gccctatgca
60900

gccccacagt gggacccttg catggacctc aaccatcaga atcttttctt ttgcaggtcg
60960

ttactctctg acctatatct acactgggct gtccaagcat gttgaagacg tccccgcgtt
61020

tcaggccctt ggctcactca atgacctcca gttctttaga tacaacagta aagacaggaa
61080

gtctcagccc atgggactct ggagacaggt ggaaggaatg gaggattgga agcaggacag
61140

ccaacttcag aaggccaggg aggacatctt tatggagacc ctgaaagaca tcgtggagta
61200

ttacaacgac agtaacggtc agtgaataac agaccacagg ggtggaaggt ctaacccaag
61260

aggcagcccc cccagtgtga gtggcaaggg atcagcagga tggaaatagt cccaatccca
61320

ggggaagaac aggagacaca gcagaaacac agacatgtcc acatcccacc caccccacag
61380

cacaggtgct ccccgcttcc ccatcaattg ccccatcctc atcccaggcc tcaggtcaca
61440

caggaagtga tggcagagtc acttcctatc caggcaccta tgacctctca cctccacacc
61500

ccacccatcg gaggctgata cccccgtgag aaggcatcag actcacccct gtccagggag
61560

gttgcctgga gagtgagcca ctctcaaagt cactcagacc tgggctcacc tggtggttct
61620

gccagtccta gctgttgaca gtgaaacgtt cccaaaatat ctggttgaaa tctgcaaaca
61680

ttggagcact gagacctacc tccaaacaag tctgtaatat ttaactatgt ctgttctatg
61740

aaggatgtca cagtctgtcc tgatctccct tgcagctcca tcacctagca cagggtacag
61800

ccaatattgg ctcaattgaa atttgtggaa tcaacagaga aaagcaccca gcacacaccg
61860

tagcccatgc tgggggctca ggaagtgctg gattcaaaac tgtgggctgt tagagttcct
61920

tggagcccta aagttcctcc ttaccatacg atgcagaccc aggaagggcc acctgcgcta
61980

tggtcagagg agctggtggc agagcccgtg cagagatggt ccctgtgccc ccggcccagt
62040

gctctttctc ctaaaccaca ctgccagccc caaggcagcc aacctcaggt ctggtgaact
62100

gctggtgtta aattatcata gagtgggtgt caaaagatgg gctactaagt acaaaaatgc
62160

ccaaggtgct acatgggatc tgaagatttt caaaaggagg caagaaagag ataggcagat
62220

gtttcaagga tgtggggtgg gggaggtctt ggtaaggaaa atggcccagg ctgtgtgtca
62280

gcaataggag aggagggggc acaggtgatc agaaaagaca ctgggggaag cattgatgga
62340

caggaataga aatggcaaag tggataatta agaggaagga ggatgaggag atgaacacag
62400

ggtattagaa aataatagaa ggcagggctt ggtggctcac tcttgtaatc ccagcacttt
62460

gggaggctga ggcaggcaga tcacctaagg tcaggagttc gagaccagcc cggccaacat
62520

ggtgaaaccc tgtctctact aataatacaa aaatagcctg gcatggtggc acacgtctgt
62580

ggtcccagct actcaggagg ctgaggcagg agaattgctt gaacccagga ggcagaggtt
62640

acagtgagcc aaaatcctac cattgcacta cagcctgggt gacaagagtg aaacgttgtc
62700

taaaaacaaa aaacaaaaaa caaaaaaagg aaataatagt agctgacatt tactgagcac
62760

ttactttgtg ccaggcccat ctatgagcat atataatgct cagaatagcc ccctaaaaca
62820

gtgctcttgg cattgccatt tcagaggtga ggaaatagag gcacagggag ttgagtggct
62880

ccagttcagg caacacacca ggtgggggtg gggggctggg gagagacctg ggacgtgagc
62940

ccagacagct tgagagcttt cagagtctat gccaacagca ccaaccagtg ctgggtaaac
63000

acctgctttt atcatcagaa caaagaggct gtgtcccctg ccctatgagg tccatttctg
63060

agagttgtgg ctaatgggca agaaggttgg ggctttagag atttgggata aagatatcaa
63120

acaccagaaa ggtagaaaga agtgatcaga ttagggttac ttaggtgatg atatgaactc
63180

ttcctagaac tgagagaaaa agagagcctt cctttactca tatgaaatca caaataattt
63240

ctatccaatt tggaagtaca ctttggtgta gttgtgacag cttcctcagg actcagcata
63300

aattcaaaca aataattgtc cttagaagag atgctataga agagatagaa atatattcat
63360

attctgtagc tttttttttt ttgagatgga gttttgctct tgtcacccaa gctggagtgc
63420

agtgatgcaa tctcagctca ctgcaaactt tgcctcctgg gttcaaggga ttctcctgcc
63480

tcagcctccc gataactggg actacaggct acaggcatgt gtcactactc ctggttaatt
63540

tttttttttt ttttaagact gagtcttgct ctgtctttca ggctgatgta caatggctcc
63600

atctcggctc actacaactt ctgtccccca ggttcaagcg attctcctgc ctcagcctca
63660

tgagtagctg ggattacagg catgtgccag cacacccagc aaatttttgt atttttagta
63720

gagatgaggt cttaccatgt tggccaggct ggtctcaaac tcctgacctc aggtgatcct
63780

ttggcctcag cctccctaac tgctgggatt acaggcatga gccactgcgt ccagcctaat
63840

tttatatttt tggtagagat ggggtttcac catattggcc aggctggtct cgaactcatg
63900

acctaaggtg atccatcctc ctcagcctct caaagtgctg ggattacaag tgtgagccac
63960

tgggcctggt gctttttttt tttttttttt tttttttttt gagatagggt ctcactctgt
64020

cacccaggct gaaatgcagt agtgtgattt tggctcattg cagccttgac ttcccaggct
64080

gaagtgatcc tcccacctca gcctcctgag tagctggggc tacaggcatg caccaccatg
64140

ctgcgctaat tttatatttt tttgtagtgg tgggatttcg ccatatcacc ctggctggtc
64200

tggaacccct gggctcaagc gatccactcg cttcagcttc tcaaagtgct gggattacag
64260

gcatgagcca cagcgcccag gctgtagctc tcttaaggag gaacatatct catctgagac
64320

aaacctgaaa tgccaaacca aactgagtta gcccctctct gtctgttgta tatattggag
64380

taataaccta tttgtcttga taaagggatt gcatgcttga attgcaaaaa cctttatttc
64440

ttttgggttg cccaatgtgc aagactaaga gttattttga taaatttctc accaggctga
64500

ctgtctctct gtggggtcgg gggagttttc agggtctcac gtattgcagg gaaggtttgg
64560

ttgtgagatc gagaataaca gaagcagcgg agcattctgg aaatattact atgatggaaa
64620

ggactacatt gaattcaaca aagaaatccc agcctgggtc cccttcgacc cagcagccca
64680

gataaccaag cagaagtggg aggcagaacc agtctacgtg cagcgggcca aggcttacct
64740

ggaggaggag tgccctgcga ctctgcggaa atacctgaaa tacagcaaaa atatcctgga
64800

ccggcaaggt actcactgct tcctgctccc cagtactgag cccagaataa aagacgatct
64860

caggctagga gctcaggcaa catcttagtc cggtctcatc tgttcctgga tgtccctcag
64920

acccccagct ttcatctttt aggatttatt ccttccctgg gataatataa tttgtggtcc
64980

aaaaagaaca tcatcaaaat ttcaggcaga atgggccagg aaggccattc tttcttgatg
65040

agtgtcccca aatcatctcc aattaacaga caaggagctt gaggttaggg aggtgagggt
65100

aacactgtct gtaagaggca gagctgggac tcaaattcca gatttcagat tccaaatccc
65160

atcgtttttt atctctacaa tgatgcctcc catctgggtg gtggagagaa gggaggcgtg
65220

taaaatgtca gccccagaag gacaagagca agccagtgtg agcggaattg atggctgcaa
65280

gctgagactt ggattggaga cgtagtgaga ctcaggattg tgcagtgctg cagggaagtg
65340

gttgctggat agaggcatgg gctgaaccaa gcagctggac tgagactggg ggacagaact
65400

ccaaagccca ctgagatgtg ggaaaacatg gagaagcaca cggagcattc acaacttatt
65460

gccgtcagag tcaatacatg ggtgaggtgg ggattgggca agagggaaag cgtcagcctt
65520

ccctgatatt ctggaaagtc tcccggggct gggggtgggc aggtacagag cttcgagctc
65580

tgctgatcgc tgacatccag gggtgggggt aggaagagac ctgggccggg agaagtccac
65640

ctcaagcctg cagtgtcaca ctctatccct ccacagatcc tccctctgtg gtggtcacca
65700

gccaccaggc cccaggagaa aagaagaaac tgaagtgcct ggcctacgac ttctacccag
65760

ggaaaattga tgtgcactgg actcgggccg gcgaggtgca ggagcctgag ttacggggag
65820

atgttcttca caatggaaat ggcacttacc agtcctgggt ggtggtggca gtgcccccgc
65880

aggacacagc cccctactcc tgccacgtgc agcacagcag cctggcccag cccctcgtgg
65940

tgccctggga ggccagctag gaagcaaggg ttggaggcaa tgtgggatct cagacccagt
66000

agctgccctt cctgcctgat gtgggagctg aaccacagaa atcacagtca atggatccac
66060

aaggcctgag gagcagtgtg gggggacaga caggaggtgg atttggagac cgaagactgg
66120

gatgcctgtc ttgagtagac ttggacccaa aaaatcatct caccttgagc ccacccccac
66180

cccattgtct aatctgtaga agctaataaa taatcatccc tccttgccta gcataacaga
66240

gaatcctttt tttaacggtg atgcgctgta gaaatgtgac tagattttct cattggttct
66300

gccctcaagc actgaattca tctgaaactc ttggtttccc ctggaggcca tggttcctgg
66360

gcaccttgac ctgggcaatc ccaagtgtgg cctgaacccc ctttcccttg gggattgttc
66420

aggtgtccct agacgccttg tggtattgta cctaataccc atgaagggag aggatgatat
66480

tacttgccag tgtacacccc cctgtgatat tgttcataat gtccagagtg aagaaagatg
66540

atattactcc caatatcaca gaaggtgtac accccccctt gatattgttc ctaataccca
66600

gttggggagg ggagaatatc tctcccaata tacaaggggt gtttaaactc tctgtgatat
66660

tgttcctaat attcaggggg gacaaggatg atattaccca aatattgcag gggttgtaca
66720

cccccccttt gatattgttc ctaatatcca ggggtggaga ggatattact cccaatattg
66780

caggggtcta catcctcccc ccgtgacatt gttcttaata accaaaaggt gagaagctga
66840

cattactccc aataccacag ggggtgtaca ccccctatga gatattgttc ttaatatcca
66900

ggaggggaga aaatgatatt actctcaata gcgcagggaa tttacatccc ccgtcgtaat
66960

cttgttctta atattcagga agggagagga tgatacgact cccagtatcg cagggggtgt
67020

gcaccccccc gtgatttttt tgctaatatc cagggtggga gtggataata cgcaggaagt
67080

gtacaggtct ctgtgatatt tttcctaata tccagggggg agaggaagat attactttta
67140

atagtgtacg gggggtgtac acccctctgt gatagtgttc ctaacatctt gggagggaga
67200

ggatgatatt actcccaata tcgcaggggg tgaaaacttt tttttgatat ttttcctaat
67260

atccaggggt ggagacgatg aaattactcc caatatcaca gaaggtgtac actccttttg
67320

tcatattgtt cctgatatcc agggtaggag aggatgatat tacttctaat atcgcaggaa
67380

gtgcatacgc ccttgtgata ttgttcctaa catccaaagg aagagagaat agtattactt
67440

ccaatatcgc agggggtgta cagcccccct tgtgatattg ttcctaatat ccaagatgga
67500

gaaatttcta ttattctcaa tatcacaaag gatgtacacc accaccgtaa tattgtttgt
67560

aatttccagg gaggggagag gataatatta ctcccaatat cccaagggtt gtacacccct
67620

ctgtgatatt tttcataata tttaggggtg aagaggatga tataaatgca aatatcacag
67680

ggggtggtca ctgtcttgtg atattgttcg aaatatccaa gaggggagag gatgaaatta
67740

ctcccaatat ctcagcgggg gtacacccct ctgtgacatt ccttgtagta ttcaggggaa
67800

gagaggataa tattactccc aatgtcgctt tgggtattca ttcccctgtg atattgtttg
67860

taatatccag gaaaggagac gatgacatta cttttaaaat ctaaggggtg tacacacctc
67920

tgtgatatgg ttcataatat ccaggggaag agaggataat attactccca atatcacagg
67980

gggtgtacac ccgcctgtaa tattgttcgt aatattcagg gtggaagagg atgaaattac
68040

tcccaatatc gcagtgggtg tgtaccccac tgtgatattg tttgtagtat ccagtcaagg
68100

ggaggatgat attactccca ataccgcagg agatgtacac cactctgtga tattgttcgc
68160

agtatccaga gggggagtgg gtaatattat tcccaatatc gcagtaggtg tacacttccc
68220

tgtgatattg tctgtaatat ccagggaaaa aagaatgata ttactcccaa aatttcaggg
68280

aatgtacacc ctcctgtgat attgtgcata atatgcaagg gggaagagga tgatattaca
68340

gcaaatatca cagggggtgt acaagcccct gtgttatagt ttgtaatatc cagaagggga
68400

gaggataata ttacttccaa atcacagggg gtgtacaccc ccctgtgata ttgtccgtaa
68460

tatccagggg ggagaggatg atattattcc gaatatcgca ggggtgtaca ctctgctgtg
68520

gtattgttcg caatatccgg ggaggaggat gatatcaatc ccaagatggt aaacaccctg
68580

tgtgtacacc accctgtgat attgtccata atatccgggg aaggggggag aaaataatat
68640

tactcccagt atcgcaggga atgtacactc ccctgtgata ttgtttgtaa tatgcggggg
68700

ggaggggaga atatgatact acttccaata tcgcaggggg tgtaaacccc cctgtaatat
68760

tgttcgtaat attctgggag gggagaggat gatattactc tcaatattgc agggggtata
68820

caccaccctc tgtgatattg ttcttaatat ccagaggagg agaggatgat attattccca
68880

atatcacagg gagtgtactc tccctttggg atattgttcc taatatccat gggaagagag
68940

gataatatta ctcccaatat cgcagggaat gtatatcccc ccctttgata tagtttctaa
69000

tatccaattt gggagaggat attaatccta atatcgcatg gggtgtacac gctccctgtg
69060

atattgttcc taatgttcag gttgggagag gatgaggttg tacaccgcct gagatattgg
69120

gagtagtgtc atcctctctt cccgttgata ttaggaagaa tttcacagga ggggtgtaca
69180

tcttctgtgc ttttgggagt aatatcatct tctccttccc tgggtattag gagcaatatc
69240

tcgggggggt gtacatactc tgcaatattg ggagtcatat cgtgttctct caccctggac
69300

agtaggaaca atatcacagg aggggtgtac accccctgcg atattaagag tagtatcatt
69360

ttcaggccgg gtgcggtggc tcacgtctgt aatccctgca cttttggagg cagaggcggg
69420

aggatcacga ggtcaggaga ttgagaccat tctggtaaac acggtgaaac cccggctcta
69480

ctaaaaatat aaaaaattag ctgggcgagg gggtgggcgc ctgtagtccc agctactcgg
69540

gaggctgagg caggaaaatg gtgtgaacca ggggggcgga gcctgcagtg agccgagatc
69600

gcaccactgc actccagctg gggcgacagc gagactccgt ctcaaaaaaa aaaaaaaaag
69660

tagtatcatt ttcccttcct ggatattaga aacaatatca catttggggt gtaaaacccc
69720

tgctatattg gaagtaatat catcctctct tttcatggtt attaggaaca atatcacagg
69780

ggtgtataca ccttctgcga tattgggagt aatatcatcc tttccccacc tggatgttag
69840

gaagaatatt gcaggcggag ggtacacccc ctgcgatatt gccagtaata tcatcttatt
69900

ccttcctgaa tattaaaaat aatatctctg ggggggtgta cacccccttc aatattgggg
69960

gtaatatcat tctctttccc ccgggatatt ggaaagaata tcacggggtg gtggtgtaca
70020

ccccctgcga tattggtagt agtatcaacc tctcacccct aaatattagg aacaacatca
70080

caaagggggt gtacaccccc tgagttattg ggagtaatgt cattgtctat cccctggata
70140

ttaggaaaat taacacagga ggtgtgtaca cctcctgcaa tattgggagt aatatcaacc
70200

tttccccacc aggatattag gaacaatatc atgtgggggt gtacacctct ttccatattg
70260

aaagtaatat catcctcttt tttcctggat attagaaaca atatcacagg aggttgtaca
70320

cctcctgcga tattgagagt aatatcatcc tctctccttc tggatattaa aaacaatatc
70380

acaggcaggg tgtacacccc ctgcaatatt gggagtaata tcatcctctc tccacttgga
70440

tattaagaac aatgtcacag ggggtatgta ccccccctgc gatattgaaa gtaatatcat
70500

cttctcttta cctaaatatt aggaacaatg tcacagggag ggtgtatacc ccctgcgata
70560

ttgaccataa tatcatgctc tttcttcttg gatattagga ccaatatcac aaggggggtg
70620

tacaccctct gcaatattgg gagtactatt atcctctccc cacctggata ttaggaacaa
70680

tatcacagag aaggtgtaca ccctcttcga tattgggtgt aatatcatcc tctccctact
70740

tggaacaata tcacggcatt ggggggaggc tgtacacttt ctgcggtatt gacagtcaca
70800

tcatcctctc accccctgga tattaggaac aatgtcacag aaggtgtgta cacgccctac
70860

gatattggta gtaatatcct ctctccacct ggatattaaa aacaatgtca cagggggtat
70920

gtacaccccc tgcgatattg ggggtaaagc aacctttcct tctttggata ttacataaaa
70980

tatcacagaa agggtgtaca cctcccgcga tattgggagt aatatcatcc tctccccact
71040

tggatattag aaacaacatg atgggggttg tacacttcct ccgatattgg gaataatata
71100

atcctctttt gccctcgata ttaggaaaaa tattacatta agggtgtaca cccccttcga
71160

tattgggagt aatattatcc tctacccccc cgatattagg aacaatatta ccagagaagt
71220

gtacaccccc tgagacattg ggataatatc ctctccccgc ctggatttta ggaacaatat
71280

cacagtgggg gtgtataccc cctgcgatat taagagtaat attatcacag ggaggtgtac
71340

actttttgag atattgggag taacatcatc ctctcgcccc cgaatattag aaacaatatt
71400

acggagtggg gatacactcc ctgcgacatt gggagaaata tcatcctctt ccctctgaat
71460

attaggaaca aaatcacaga gggttgtata ttccttgcga tattggcagt aatatcctct
71520

ctgcccctgg atattaggaa caatatcaca gaaggggtgt atcccccttc gacattggga
71580

gtaatatcat cctctctgcc gctggatatt aaaaacaata ttaaaggggg gtgtaaaacc
71640

cctgcgatat taaggtaata tcatcctctc cccccccgga tattaggaac aatatcacag
71700

ggggagtgta caccccctgc tatattgaga gaaatatcat actctcctca aatggatatt
71760

aggagtaata tcacagggat ggggtgtaca cgttctgcta tattgggagt aatatcatcc
71820

tctccctacc tggatattaa atacaatatc acagggggtg ttaccccccc tgcaatattg
71880

ggagtaatat catcctctcc cattctgaat attaggaaca atatcacagt gggggttgta
71940

caccccctgt gatattggca gtaatgtcat tctctcccgc tttgaatatt aggaacagca
72000

tcacggaggt tgcacacaac ctgcaatatt gggagtaatg tcattttctc ttccacctgg
72060

acataagaac aaatattaca gggaggaata caccttctgc gatattggag caatatcatc
72120

ctcatttccc ctggatatta ggaaagatat tacagtgggg ggtggacacc cctgcgacat
72180

tgggagtaat attatcctct gccccgttgg atattagaaa aaacatcaca aaggaagtgt
72240

acaccccctg ctatattggg agtaatatca tcctctcccc cctggatatt agaaacaata
72300

tcaaggggga gagtacacca ccttcgatat tgggagtaat atcatccttt gcccttctcg
72360

atattaggaa caatatcgca gtgtggtgta cactgcctgc gatattggaa gtagtatcat
72420

actcttcctc cctcgatatt atgaacaaca tcacagggtt gtgtaccccc ctgcaatatt
72480

gggtttaata ttgtcctctc tttccctaga tattactaac aatattacca gggagtgtac
72540

acacagggtg tttaaaatat taaacttgat gtcatcctct cccccccgga tatggcaagc
72600

aatatcacat ggaggtgtac accccctgtg atattcgaag taatataatc ctctccctcc
72660

ctggatatta caaaaaatat cacagcggaa tgtacacccc ctgcgatatg gggagtaata
72720

tcttccattt cctccataaa tattatggac aatatcacag gggcatgtga accacccgtg
72780

atgtgggaaa taatgtcatc ctctcccctt ctgaatatta caaacaatat tacagggggg
72840

tgtacacacc ttgtgatata ggaaataata ttattttctc cccctctgga tattacaaac
72900

aatatcacgg agaggtaata tcatcctctc ccccactgga tattatgaac aatagcacaa
72960

ggggatgtac accccctgtg atatggggag taatatcatg ccttcccccc tgcatattag
73020

gaacaatata ataggggggt gtacatcttc tgcgatatgg ggagtaatat cttcctctcc
73080

ctccctaagt attacaaaca atatcacagg gggatgtcca ctccctgtga tatgggaagt
73140

aatgtcatcc tctccccttc taaatattac aaacaatatt acaggggggt gtacacacct
73200

tgcgatatat gaaataatat tattttctcc ccctctggat actatgaaca atatgaaagt
73260

gggatgtata ccccctgcga tatggggagt aatatcattc tctcccctcc tggatattac
73320

gaacaatatt acaatcgggt gtacaccctc tacgatatga agagcagtaa tctcctctcc
73380

cccttggata ttaggaacaa tattacaggg gatgtacacc ccctgcgata ttgggagtaa
73440

tatcatcccc tcccctcctg gatattacga acaatatcac agacggtgta cacacagggt
73500

gtttaccata ttgggagtaa tatcatctcc cttccaggag attacgaaca aaatcacaac
73560

ggggtgttta ccccctgtga tattgggagt aatattatcc tcttccctcc tggatattat
73620

gaacaatatt acagaagggt gtacaccctc tgcgatattg ggagtaacat cactctctcc
73680

ctttctgaat ataatgaaca atatcacagg gaagtaacat catcctcttc ccccctggat
73740

gttacgaaca atatcacagg aaggtgtaca ctccttgcaa tatggggagt aatatcatcc
73800

tctttcccct gggtattacc aacaatatca caggagggtt tacaccatca gcgatataag
73860

aagtaatgtc accctctccc tccagggata ttatgaacaa tatcacgggg gggtgtgccc
73920

cctctgccat ataaggagta atgttatcct ctcattccct ggatattaca aacaatatca
73980

cagggggatg tacataccct gcgatatggg gagtaatatt atcctctccc cactgaatat
74040

tatgaacatt atcacggggc agtgtacacc ctctgccata tgggaagtta catcatcctg
74100

accctcattg gatattttgg agaatatgac ttgtgggtgt gaccatcctg caatatgggc
74160

agtaatatca tcctctcccc ttctggatat tacaaacaat atcacaggag ggtgtatact
74220

gcctgcgata tcgggagtaa tattatcctc cctcctagaa tatcacagag gagtgtacac
74280

tccctgcgat attgggagta atatcaccct ctcccctcct ggatattacg aacaatatca
74340

cagatggtgt acacacaggg tgtttaccat attgggagta atatcatctc ccttcctgga
74400

tattatgaag aatattacag gggggtgtac acctccctgt ttatgggagt aatatcattc
74460

tcttcccctt ggatattaga aacaatatca caggtgggtg tacaacccct gcgatatggg
74520

gagtaatatt atattctttc tccctggata ttatgaacaa tatcacagaa gggtgcacac
74580

ccccggtgaa atggggagta atagcatcct ctcccctcct ggatattacg aataatatta
74640

taggagggtc tatatcctct gccatatggg gagcaatatc atttcttccc ctctggatat
74700

tacaaacaat attacagggg gttgtacaca ccctgcgata taaggagtaa tatcattctc
74760

tccccacctg gatattacga acaatatcac agaggggtgt acaccttcta tgatataagg
74820

agtaaaatca tctttttttc cctgtggata ttaggaacaa tgtcacagac agtgtacaca
74880

cacagtgctt acgatatcga aaatagtatc atctcccccc tgaatattac aaacaatgtc
74940

acgggggggt ccatcccctg tgatattggg agtaatatca tcttttcctc ccctaaatat
75000

taaaaacaat atcacagggg aatgtatacc tcctgcgata ttgaaagtag tatcctcctc
75060

tcccccactt ggtattagga acaatattac aggagaagta tacaccccct gcgatattgg
75120

gagtaatatc gtcctcttgc ccctggatat tagggacaat atcacagggg ggtatacatc
75180

tcctgcaata ttgggagcaa tatcatcctc ttcctccctg gatattacaa acaatatcac
75240

agggggtgta cacaccctgc gatatgtgga ataatgtcat cctctctatt ctggatgcta
75300

cgggcaatat cacaggggat tgtacctccc ctcttctaat atcatccgct cttctgttga
75360

atattatgaa caatataaca tctggtgtac aaagtgacat gctattttaa ggtggctgag
75420

aaattattaa aggagatcat ttttctctct gcccctgaaa attataaatg atatcacagg
75480

ggagtgtatg ttccctgaga tattgggagt aatattatca tctctgcccc ttaatattac
75540

gaacaatatc agcgggcgta ttacgaacaa ttttatgaac aatatcacac cccctgtgat
75600

attgctcata atattcacag gaagggagga tgatattact cccagtattg cagggggtgt
75660

acattcccct atgatattgc ttttactatt taggggagga gaagatgata ttactcccaa
75720

tatcgcaggg gatgaacctc cccccgtgat attgtttgta atattcaggc gggagagaat
75780

aatattcttt ttttccctta atattatgaa cagtatcaca agggcgtgta caccccctgc
75840

aatattggga gtaatatcat tgtttctcct catgtatatt tcaaaaaata tcatgggggg
75900

tgtacactcc cgtgatattt ggagtaatat caccctcttt ccccttggat attatgaaaa
75960

atatcacagg gggggctgta cacccccctg ggatattgga agtagtatca tcctcttccc
76020

ctctggagat taggaacaat atcatagggg agtgtacacc tcctgtgaca ttgggagtaa
76080

tatcatcctc tcttctgtta aatgttatga acagtataac atggggtgta caaagtgaca
76140

tgctattcta aggtgactga gaaattatta aaagagatca tttttggcaa tgagactctt
76200

tgggttgggg cctcttcaat ttgcacttgg atttggtcct acctgtggga gaatctaaca
76260

gtggtcctcc tttgggggag caagcagtga ctgtcgaagg aaccacattg ggttctctgg
76320

actcctttat catccctgat gcctagtcta tgctcatggc ccagccctac ccccaggtca
76380

cctcccttgt caggctcctg tgtcccccac acccacttgc tgtttcatgc tgacctggtt
76440

agaaacactc tttttttttt ttttttgaga tggagtcttg ctctctcacc caggctggag
76500

tgcagtggcg tgatcttggc ttactggaac ctccatcttc tgggttcaag tgattctcct
76560

gcctcagctt tgtgagtagc tgggattaca ggtgtctgcc accatgccaa gctaattttt
76620

gtatttttag tagagatggg gtttcaccat gttggccagg ctggtctcaa actcctgacc
76680

tcaggtgatc caccagcctc agcctacaaa agtgctggga ttacagtagt gaccgacctc
76740

ccctggcaga aacattcttt tgaccgcctc ttggcaagct cttaggtctt cacatctctg
76800

atacatcatt ccctacgggg gtttttctct gggtgactca gtggctctgt gcccctctcc
76860

ccaaccctga cacctgtcca ggacttcagt tttctgaggt ttcctccccc ttgtccaccc
76920

cactcctctt tcctgttaca gatgaggcca gatcccattt ttaatgttct ttagccctga
76980

cctgctcaca cgagcttttg gtttccttat tgacctaagc tgttgattct ttatcctttt
77040

atttgttgag ggtgtcatcc tttttgaatt gaaaaataca gttgctgtat tttccctctc
77100

tgattgagct ttcttccctg gggctctata tgttcagata gaactttttg ccccagaaga
77160

ggaaatgcgg atttcattag tctgaagaag taatttctca gctttgattt ccaagttgaa
77220

cccaaagcat taagaaaaaa aaaaaaaagc gggtggtggg gaaggataac ttcaaattta
77280

ggctttccaa aaagatgaga tgtttgactt agaattctga aggctgggaa tgatttcctt
77340

aaggagccca gcttggagga tggactttgg aataatccag agtgggaggc tgaggacagg
77400

atgtaaggaa agttctgtgc cacgggaagg aaaagcacag acacaggcag gtgaggtttc
77460

atgagaaggt caaggtgaga gctctgctgt gtcttttgcg gttgaaacct gtgcatggcg
77520

gttggtgact catttgagga aactcactcc ctgggacttg gaggggatct gacaacccca
77580

gggccaattc ctgtatcctt gagctgtgcc ctggagagct gagggttctc aaacaagctc
77640

tggatgtgtg agccaagacc aacatggtgt ggagatgcgg agcggctgga gtgaagggcc
77700

gggtctccca ggaagccaag gagtgtgggg aaactgaggc acagaagacg cttgggtttg
77760

tgtaaaagag ttttcaggcc gggcgctgtg gctcacgcct gtaatcccag cactttggga
77820

ggctgaggtg ggtggatcac ctgaggtcag gcgttcaaga ccagcctggc caacatggtg
77880

aactaaacac actaaacaca caaaacacac taaaacacac aaaaatttct actaaacaca
77940

caaaaatcag ccgggtgtgg tggcgggcgc ctgtaatctc agctactcag gaggctgagg
78000

caggagaatt gcttgaacct gggaggcgga ggttgcagtg agccgagatt gcaccaccgc
78060

actccagcct gggtgacaca gcaagactcc atctcaaaaa taaataaata aataaataaa
78120

taaataaata aataaataaa taaatagagt ttgcagaatt ctgagtagct ctgtgcccca
78180

ttgctccccc tccacatctg gccaagactt ggatcttctg aggtttcctc cccttcttct
78240

acccctaccc atctctcctg tcatgacgcc agagctcaca ttcaaatctt cctttgccct
78300

ggcctgctca tgttgccctt tgcttttttt aaaaaaattg aaatgttcct cctgtcatgt
78360

gctattcttt atttgctcaa agtattcgac attactgtga aatgtcactg gctgggcaat
78420

ttcctcctga gcgctttggc ttatcttctc tgactaaatg gaataaacct gttcaaatca
78480

gtatttttac atattgctca tccaatattg tacaggaaaa atactaatat ggggccaggt
78540

gtggtggctc acgtctgtaa tcctagcact ttgggaggcc aaggcaggca gattacgagg
78600

tcaggagatc aagaccatcc tggctaacat ggtgaaaccc tgactctact aaaagtacaa
78660

aaaaaaaaaa aagggaaaat actaatatgg ggctaggcat ggttgttcat acctgtaatg
78720

ccagtgcttt ggaaggctga agtgggagga tcacttgagt ccaggagttc gagaccagct
78780

gggcaacata ttgagacccc tgtctctacg aaaaccaaaa aaaaaaaaaa aaaaaaaatg
78840

agccaggcat agtggtgcat gcctgtagtc cttgctaccg agtaggctaa ggaggaagga
78900

ttgcttgagc tcaggagttc gaggctgcag tgagctatga tttcgccact gcactccagc
78960

ctgcaatggc ctgaatttcc tgttcagatc ttttatgaac caaatactac acaactgtag
79020

tcatgatctt cttggctgga gagaggctgg ctgtcaccca gggccacagg gtgattaggt
79080

agaatgaaaa gagagtcacc aactcaagac agtagcacag tgtccttgtg gttttggctg
79140

gtgtagtcta aaatctgaat tatgcaaagt gtatttgatc ttgcatcaca tcaggaaaaa
79200

gcccagtaat ctatttcatc atggggtgtg gagaagttgc tggaaccatt tctagacaca
79260

gattttggtt ctccagaggc cagcaagtta gcttcactaa gcttcggggc ccacagtggg
79320

aagagatgtc ctgctgaggc tcagcgttga cttcactctt gtggagcaaa gctgtaggaa
79380

ctaagggctt tatgctaatt aaaagatgag gccaggtgca gtggctcata cctgtaatgg
79440

ggtcttgcta tattgcccag gctggtctcg aactcctggg ttcaagccat cctcccacct
79500

cagcctcctg aatcactggg attaaaagtg tgagccactg tgcccagcct cccttttaat
79560

agaactggtg cttaatccac agctctcctg tcagcccaca gttcttgaat tttggcctct
79620

tttatgtcca catgaatgta aatatacttc agaatttcca cccagaatct ttggaatcta
79680

atttcttcaa tcattttact ttagagtcat ttttagggta agtgaatctg cctggttttc
79740

aacttgacac cctctctaaa cgtgaatgag ttcaaatcat attcgttcct gaggaatcac
79800

actcaagcat agtacaaatc tgtgggatat gccattacaa ctctaggaat catgttctca
79860

aggtaaaagc ctttaagaaa agctgtcttg acatatcatg taagcatgac aatggaatac
79920

taaaccaaca tagctgagat tttccttatg tcctatgctt ttaaatgatc aaccctacag
79980

tcctggaacc aacctttatg gttatcaata cctccattat tttaaactat ctcaggtatc
80040

atattccttc tttttttttt ttttttgaga cggagcctct cactgtcacc caggctggag
80100

tgcagtggcg caatctcggc tcattgcaag ctccacctcc aatggaatac taaatcaaca
80160

tagctgagat tttccttatg tcctatgctt ttaaatgatc aaccctacag tcctggaacc
80220

aacctttatg gttatcaata cctccattat tttaaactat ctcaggtatc atattccttc
80280

tttttttttt tttttttgag acggagcctc tcactgtcac ccaggctgga gtgcaggggc
80340

gcaatctcgg ctcattgcaa gctccacctc caatggaata ctaaatcaac atagctgaga
80400

ttttccttat gtcctatgct tttaaatgat caaccctaca gtcctggaac caacctttat
80460

ggttatcaat acctccatta ttttaaacta tctcaggtat catattcctt cttttttttt
80520

tttttttgag acggagcctc tcactgtcac ccaggctgga gtgcagtggc gcaatctcgg
80580

ctcattgcaa gctccacctc ctgggttcac gccattctcc tgcctcagcc tcccgagtag
80640

ctgggactac aggcacccgc cacgaccccc ggctaatttt ttgtattttt agtagtgccg
80700

gggtttcacc atgttaacca ggatggtctc gatctcctga cctcgtgatc cacccgcctc
80760

ggcctcccaa agtgctaggt ttacaggtgt gagccaccat gcctagcttt ccttcttata
80820

tgtatcaaaa ctcatactga aaaatgagtt ctgggttaca aaagagggtt tatttttgc
80880

acctgccttt gaatcagtcc ttaagcttaa cctttaactt ccaaggaact actttttttt
80940

tttttttttt ttagatagag tctcgctctg tcacccaggc tggagtgcaa tgatgcaatc
81000

tcggctcact gcaaccttcg tctcttgggt tcaagtgatt ctcctgtctc agcctcctga
81060

gtagctggga ttgcaggcat gtgccaccat gccctgctaa cttttgtatt ttttgtagag
81120

acagggtttc tccatgctgg tcaggctggt cacgaactcc tgacctcagg tgatccacct
81180

gcctcagcct cccaaagtgc tgggattaca ggcatgagcc accgtgccag gccccatccc
81240

tggttattct tattcagtaa aatttgcaac ctcctaggaa gaaaggattt tgaaaacaaa
81300

actctttttt aaaactttta ttttaggttt gggggtacgt gtgaagattt gttacatagg
81360

taaacatgtg tcatcgtggt ttgttgtata tattatttca tcacccaggt attaagccca
81420

gtacccaaca gttgtctttt ctgctcctct ccctcctccc accctcaagc agaccccagt
81480

gtctgttgtt tccttctttg tgttcataag ttctcatcat ttagctccca cttataagtg
81540

agaacgtgtg gtatttggtg ttctgttgct gcattagttt gctaaggatt atagactcca
81600

gcttcatcca tattccctca aaagacatga tcttgttctt ttttatggct gcataatatt
81660

tcatggtgta tatgtaccac agtttcttta gccaatctgt cactgatggg catttaagtt
81720

ggttccatgt cttcactgtt gggagcaagc cccccaaaat ctggccataa tctggcccca
81780

agactggtca taaacaaaat ctctgcagca ctgtaacatg ttcataatgg ccctaacgcc
81840

caagctggaa ggttgtgggt ttacaggaat gaaggaatga gggcaaggaa cacttggcct
81900

gcccagggcg gaaaaccgct taaacgcatt ctcaagctgc aaacaatagc atgagcgatc
81960

tgtgtcttaa ggacgtgttc ctgctgcagt taactagccc aacctattcc tttaatttgg
82020

cccatccctt tgtttctcat aatggatact tttagttaat ttaatatcta cagaaacaat
82080

gataataact ggtttgctgt tactaaatat gtgggtaaat ctctgtttgg ggctctcagt
82140

tctgaaggct gtgagacccc tgatttccca cttcacacct ctatatttct gtgtgtgcat
82200

ctttaactcc tctagcgccg ctgggttagg gtctccccaa ccgagctggt ctcggcactt
82260

cagtattgta aatagtgctg caatgaacac tcatgtggat gtgtctttgt tttttttttt
82320

tttggagacg gagtctagct ctgtcacccg ggctggagta cagtggtgca atcttggctc
82380

actacaacct ctgccacctg ggtttaaatg attctcctgc ctgggcctcc tgagtagctg
82440

ggattacagg tgtgagccac tgtgcccagc taacttttgt atttttagta gagcccaggg
82500

tttcatcatg ttggccaggc tggtcttgaa ctcctgacct caggtgatct gcctgccttg
82560

gcatcccaaa gtgctgggat tacaggcatg agctaccacg cctggcccat gtgtctttat
82620

ggtagaatgc tttatgttcc tctgggtata tactcagtaa tgggattgct gggtcgaatg
82680

gtagttctgc ttttagctct ttgaggaatc gccatattgc ttcccacaat gattgaacta
82740

atttacattc ccagcaacaa tgtgaaaatg ttcccttttc tctgcaacct tgccagcatc
82800

tgtcattttt tgacttttta ataatagcca ttctgactgg tatgagatgg tatctcattg
82860

tggttttgat ttgcatttcc ctaatgacca gtgatgttga gttttttcat atgtctgttg
82920

gccgcctgta tgtcttcttt tgggaactgt ctgttcctct cctttgccca ctttttcatg
82980

gggttgtttg tttttctctc ataaatttgt ttaagttcct tatagatgct agctattaga
83040

cctttgtcag atgcacagtt tgcaaataaa acaaaactct taatgtcttc acttgccaga
83100

tatttgtgct taggttggtg tttgagtctt aggagtaata tcatgttttg ccacaagaag
83160

gagcctatga gtgggctgga agagaaaaat cagatttgca gccgctgaag tcagtaaaca
83220

gtttctttgg atatattgta aatagtaaga gaaccagaca tgacattcat aagtattcca
83280

aaaccaagta aaattatttt aaagctctaa cgttttgcaa cttttcttcc cgtcttaaga
83340

gttcatctgc ccagtctaat gattgtagaa ttcttacttc tagtcttttt tttttttttt
83400

tgagatggag tttcactctt gctgcccagg ctggagtgca atggtgctat cttggctcac
83460

tgcaaccttc gcctcctggg ttcaagccat tctcctgcct cagcttcccg agcagccaca
83520

atttcaggga tgcgccacca cacccggcta attttgtatt tttagtagag ttggggtttc
83580

tccatgttgg tcaggctggt ctcgaacttc tgacctcagg tgatctgccc accttggcct
83640

cctaaagcgc tgggattaca ggagtgagcc actgtgcctg gccatcattt atcttacttt
83700

tagcatcctc atcccaaatt cctggtgtcc ccatccctac catccatttg taataattct
83760

ccattgtttt cctctccttg aatcccactt tctgagatca gtgcaacctg ggcttagagc
83820

ttcaacctcc tccatctgat tttttaaaag agtaactcct aataaggatt ttacccaata
83880

tagtgagcat tatgattaat gaccctgact ctacaaaaaa taatttaaaa aaattagtca
83940

ggtgtggtgg cgcatgcctg tagtcccaga tactcagggt gaggtgggag gattgcttga
84000

gtccaggagt ttgagatcac ggtgagctgt actgcatcat cacattccag cctgggcaac
84060

agagtgagac cctgtttcta aaacaattaa ggtaaaataa aagaaataac atgcacctgg
84120

tacacagtac ttaataaatc ccgtctactg taaataccta gtgcccatca tccctgtaac
84180

tcataacttc ttctttccca gttccccgtg ctttaatcac tcctgcttag gagacagtcc
84240

aggctacctt cttatctctc ttcctggagc cagattctca gcattttgtt ctctaattcc
84300

accctttacc ccttattgca tgccttggta tcagcacttc acctggtctg aggaaccagc
84360

atctagtctt gccctgtgtc tggtagtcca tatggcctgt atgtatcact tagtctgagt
84420

cagtttccac gtgtgtcagc tggattagta gctccttagg gttccgtgta gttctcacag
84480

tctgtggcaa ggaaaacaac aatttgtgag tagctagcat ggactgcaaa ctatgggctc
84540

tatttaccaa gcaagttcta aatgctgaac attgtctttg gcaaaggggt ttcagggaag
84600

atcaagaggc acatcttccc tgccctcagg aaactcatat aggctggttt tgctacactg
84660

aattctcaca actcattgat gcaatgcagc ctagaattaa gttacttgct cattgtcaag
84720

caggtagtga ctagcagagc caccatcctg attctttctt agtttctttt tttttttttt
84780

gagttagggt ctcgctctgt cactcaggct ggagtgcagt ggtgccatca tagctcactg
84840

cagtctcact cctgggctca agcgatcttc ctgcctcagc ctcctaagca gcagggactg
84900

caggtgcaca ccaccatgcc cagctaattt ttaaattttt gtagaggtgg gaatcttgct
84960

cttttgccca gtccggtctg gaactcctgg cctcaagcat cctcctgcct tggcttctca
85020

aagcactgga attacaagca tgagccacca tgtccagccc taagtctgat accttctaag
85080

agggatcatc ctcagatgag ttgggtgtgg aggtgagagg tcaggggtgc ctggataaga
85140

agtgactctg ctatcacttc ctgtgtgagc tgaggcctgg agagaagatg agagaatgat
85200

ggggaaggag gaagcagatg tgttgtgggg ttgggaaatg gggaaatgtc tgtgcatttc
85260

tacttttacc tttgatctct ccttcagggc tttgatattt tcttcctgct aatcctcctt
85320

ttcacacctg cttggggctg ccccatgagc ttgtgaaaac agcatgtttc taccttccaa
85380

ccacagtcct cagtgcattg gccctctctc tccttggcag agtctacgat gtttttcaag
85440

gttaccatga agatttcttt ctttttattt taattttaat tttatttttt tttgagatgg
85500

agtttcgctc ttgttgctca ggctggagtg caatggcacg atctcagctc actgcaacct
85560

ctgcctcccg ggttcaagcg attctcctgc ttcagcctcc caagtagctg ggattacagg
85620

catgtgccac catacctggc taattttgtg tttttagtac agacaaggtt tttccatgtt
85680

ggtcaggctg gtcttgaact cctgacctca ggtaagccac ctgccttggc ctcccaaagt
85740

gctgggatta caggcaggag ccactgtgct tggctttatt ttatttttta agttcctggg
85800

tacaagtgta ggatgtacag gtttgttaca taggtaaacg tgtgccatgg tggtttgctg
85860

cacgtatcaa cccatcacct aggtgttaaa ccctgcatgc attagctctt tttcctaatg
85920

ctctccccta acctacatcc tcccctgaca ggacccagtg tgtgttgttc ccctccctgt
85980

gtccatgtgt tctcattgtt cagctcccag ttataagtga gaacatgcag tgtttggttt
86040

tctattcctg cgttagtttg ctggggataa tggttttcca gcttcatcca tgtccctgca
86100

aaggacatga tctcattatt ttttatgact gcatagtatt ccatggtgta tatgtagcat
86160

attttcttta ttcagtctat catcgatgag cacttgggtt gattccatgt ctttgctatt
86220

gtgaatagtg ctgcaatgaa catatatgtg catgtatctt tataatagaa ttatttatat
86280

tcctttgagg tatataccca gtaatggaat tgctgggtca aatggaattt ctggttctaa
86340

atctttgagg aatcgccaca ctgtcttcca caatggttga attaattcac attcccacaa
86400

gatttctttc ctgactttct gaacttggat cttctcctat cctccatccc caccacctgg
86460

ctccaggatc ccagagactc agccttcctg cattcaaaac catagtggaa gaaggcctga
86520

ttgctggggt gggcagtggc ctgaaacctg gggaggactt cactgggcct aaacattctg
86580

gtgtagagaa agtcactaag taaggacttg tgaaaatgta gaggactttg cggtgggggt
86640

gaactgcaga gtcctactgt ggggcagcaa gaagtcaaga aggaggacct ggcaactggg
86700

gtcaagagct gcccacatgc tcttcatgtc cagccttttt tctcctgtca gctttctagt
86760

gttgcatgct catatgggca gaaagacttg ccattatatg ctagaaagca catggtggag
86820

gctgggcgcg gtggctcact cctgtaatcc cagcacttca ggaggctgag gtgggtggat
86880

cacctgaggt caggagtttg agaccagcct ggccaacatg gtgaaacccc atctctacta
86940

aaatacaaaa aattagctgg gcgtggtggc aggcacctgt aatcccagct actcgggagg
87000

ctgaggcggg agaatcactt gaacctggga ggcagaggtt gcagtgagct gagatggcac
87060

cactgcactc cagcctgggt gacagagcta gactctgtct ccaaaataaa aaaaaaaaaa
87120

agaaagaaag aaagtacagg aaggagaatt tattagaaaa ttcaggcagc ctgggcaaca
87180

tagtgagacc acggcgctac aaaagaatac aaaaaaaaaa aaaaaaaaaa aggctgggcg
87240

cagtggctca agcctgtaat cccagcactg tgggaggctg aggagggtgg atcatgaggt
87300

caggagatcg agaccatcct ggctaacaca gtgaaacccc atctctacta aaaatacaaa
87360

aaaaaaatta gctaggcatg gtggtgggcg cctgtagtcc tagctactcg ggaggctgag
87420

gcaggagaat gacgtcaacc tgggagcgga gcttgcagtg agctgagatc acgccactgg
87480

actccagcct gggtgacaga gctagactcc gtctcaaaac aaacaaacaa acaaaaaaaa
87540

accacacaaa aaaacaaaaa aaattagcca agcatggtgg tgtacacctg ttgtcccagc
87600

tactcaggag gctgaggtgg gaggatcact tgagcctgga ggttgaggtt gcagtgacct
87660

aagatcgtgc tactgcactc cagcctggat gataaagtga gaccctgttt cacaaagaaa
87720

aagaaaaaga gttaaaaaaa tccaggcatg aacaggaagg gatgagcatg gaataaaacc
87780

aggatgctgc ctccgtacca ccagttcttt gatttcaact tgttgctcag ttgaatcatt
87840

gccatactgg tgagaaagaa atgtatttgc tgactgttgg tcaatattac agatttaaaa
87900

cctgtgcact cttaagacct caccagttta tagttttgtt tcttccagat ttctgcggaa
87960

agatgggccc aaggctggct ttgctccctt ttctctaggt cacacagtat cctaggccct
88020

gtgctgttat atttgactct gtggaagatg ctttgttttc agttctgagc cttttcttct
88080

gctgattcct tctcaacctt tgttccttgt gtcgctcacc ctcccccaaa ccaggtagat
88140

gcacccaggt gtggaaaaga gggagggaga agaaagatcc cttctgtcat tgatacagga
88200

gctagaaaaa aattatttag gcagatagtg agggtaagag agtccgtggt aagatttccc
88260

tttaatgaaa agcagccccc aaataatttc ttttctaaca ataagcagcc tgaaaaatta
88320

agctgcaagc atagataagc aagctaaaag cttgcatagg taaatgccaa cagctgtgcc
88380

aatagaaaag ggacacctgg aagccaggta tattcaacat ggaggtgccc tcttcccttt
88440

cctttgtcaa cacgcttgca gtaaaaaagc aggcaacgtg gtgatggcca agtagagacc
88500

ccatctgcat aataaaaggt taaggtgaga tggccaactt gttggtgcgc tatgcaaatg
88560

gcacacctgg tctgactaat ctctcgggcc ctatgtaaat cagacactgc ctcctcaagc
88620

ttgtctataa aacccccccc ccgtgcattt caccacaaaa ccggaagacc cactcggaag
88680

tccctctctc tctgcaggag agagagcttt tcttttttct ctttcttttg cctattaaac
88740

ctccaatctt aaactcctcc ttgtgcgtct ccaccttctt gatttccttg gcgtgaggca
88800

atgaatcttg ggtattactc cagacaaatg acgccacttc atcatcgttc ttggggcaca
88860

gaggtgctga gcttccacat ttctcttcgc tccctactct tgggtgagcc cttcaccctg
88920

ggtaccctgt tgccccaggc cttggcacgt gttgtcaacg tatggctgtg ggaggaggct
88980

ggtcttgtct ggtgggaagg agagagacaa agagggcatt gttgatagaa gaagtggaat
89040

gagagctccc tttacccttg ggggcttctt ttctggcatt aatcctcaga aaataattgt
89100

gaaagagaag tttgtctttg tgctttaggg tgctgtcaga agcttagaaa taattaggca
89160

gaatccccat ccccccaatc cccatcacca actttagtct cctcccccga tacccacact
89220

gacccagggt tagggacatt tataaatgga gccattgagg actcaggaga aactcatgca
89280

actgaaaaat gtcccctatt tcctgggtcc taggcagctc cccactttcc actcccactt
89340

cagacattta gacaaggact ttggtcactc tggctagacc atctgtgtaa cccagaatgg
89400

tggggtcaca actcttggtt tccaaggata taatcacatg ttgtgagcaa aaatgtgctg
89460

aacacacctg actctgtggc ttcttcttga gaagtgattt tgcagagcgg ggaagggtcg
89520

actctggaaa cgtttcattt gtctattggc catttggatt tcttctgtga agtgcctttt
89580

catgactatc atctatattc tcttaagttt gtccttgtta ttgatttgta gttctttata
89640

tatccctgat aacaaggctt gctttttcat gctctttata tatattttta aaagcagctt
89700

tagcaaagtg taacatacaa aaatctgcac ataaaatgta aaatgtgatg ttttgacata
89760

tatatatatg cactgaaacc atccctatta attccattgt gatcaaagac atatattgta
89820

tcacttaaat ccttttacat gtatccaggc ttattttatg gtgtagaaca tgatctatct
89880

tggtagatgt tctgtgtgct cttgacaaga atgtattgtt gcgtagaatg ttctattgtc
89940

aattttgttg attaggtcaa tttggcttat agtattgttc aagccttcta tttgtttact
90000

gattttctgt ctacttgtcc tatcaatttt tgagagatgg gtgttggctc cttaactatt
90060

ttgtgaattt gtctatttct ccatgtattt ctattaattt ttgctgtacg tattttgaag
90120

ctctgttatt acgtgtgtaa acatttagga tttgataaat ggactcattt gtaacaatca
90180

aatatcttct ttatctctgg taatattttt tgctctgaaa tctactttga tattaatata
90240

gtcactgtag gttacttttg attagtgtca gcatggtata ttttccatta ttttactttt
90300

aaccttgtac ctttataatt aaagtgagtt atctttgggc agcatactgt taggtcttgc
90360

tttgtaagtt aatatgttaa tctctgccta taaattggaa tggttagatc atttatattt
90420

aatgtgatga tttgtatggt taggtttaaa cctctcatgt tgctatttgt tttctatttg
90480

tgccatttgt ttgatctgtt ccccctttcc tctttttcta ccttctgttg gattgagcat
90540

atatttatat ataattttat ttttacctcc atgtttgtat tattaactat aacccttttt
90600

gggttatttt aggcttatgg tttatatctt catgttatta gtctacttca attgatatta
90660

tagcactctt cacacattgt aagaacctta cagtatactc ccatttcttc cctcctggct
90720

tttatataat tgttaccata tatcttacct caatatatgc tataaacccc ataatacatt
90780

gttatctttt cgctttaagt gaaagattat ctttgtcttt atctttagag ataaagtggg
90840

gaagggttga ctctggaaac gtttcatttg tccattggcc atttggattt cttctgtgaa
90900

gtgccttttc acgactatca tctatattct cttaagtttg tccttgttaa ttatctttat
90960

caattatctt taaaacgatt taaataataa caaaaataat ctttatattt accctcatag
91020

ttaccacttc tggtgttctt tattctctaa cgtagatcca ggtttctatc tggtttcaaa
91080

ttcttctagc tcaaaggact tcctttacca tttatcatgg tgcatatatg tttttcatt
91140

cactctggag atgaattctt ccaactgtta tatatcttaa aaagttttaa tttttgcctc
91200

agcttttgaa aaaaaatatt tttgagatag ggtctccctc tgtcacccag gctggagtgc
91260

agtgatgtga tcatagctta ctgcaacctc cacctcccag gctcaagctg tcctcccacc
91320

tcagcctccc aagtagctgg actacaggcg tgtgccacca cacctggata atttttgtat
91380

ttttttgttt gtagagacgg ggtttcacca tgttgcccag gctggtcttg aattcccaag
91440

cttaagcaat ctgcccactt cagcctccca aagtgctaag attacaggtg taagccaccg
91500

tacctggcct gaaaaatatt tttgctgggt atataattct acattggcaa tttattcctt
91560

tgattacttt aaacatgttt ttccactgtc ttctagctta tattgtttcc aacaagaaag
91620

ctactgtcat tcttatctac tgttggtgcc ctgtctagat gtccttttct gggctggcat
91680

gcccactcac aggctgttgt taatggtggc tattattggc tcatgaactc tctccttttc
91740

taaatagatg gcttggccgg gcatggtggc tcacgcctgt aatcgcagca ctttgggagg
91800

ccaaggcagg cggatcacga ggtcaggaga tcgagaccat cctgactaac acggtgaaac
91860

tctgtctcta ctaaaaagac aaacaactag ccgggcatgg tggcaggcgc ctgtagtccc
91920

agctactcgg gaggctgagg caggagaatg gcgtgaaccc gggaggcgga gcttgcagtg
91980

agccgagatc atgccactgc actccagcct aggtgacaga gtgagactcc atctcaaaca
92040

aaaaaaataa aaataaaaaa aagagatggc ttcacccggg ataatatgat ttcttcctct
92100

ccccgatggc atcccaatgc caatgactga atgttattgg agtacaaaag aacagccctt
92160

ttacctcaag tggataaaat ctgtggtatg atttatgctt cagggtcccc tgtggacaag
92220

atcaagttca aaccttacct gagatcacat ttggcacagt tttttttttt tttacattac
92280

cttttctgct tcctctattt ccttagagat ttttactggt gagtccaacc taaaaaatca
92340

catgtattca agtctctttt aagttctgct tctaaggaat caaacctaag acagtgggta
92400

ccaggagcgg tcctaggaag cacaacataa gaatgggatt ctagggctgg gttacttgtc
92460

agccctctgg caagggggac agccatgttg atggcaggca tagtttctga cttgctttag
92520

cagtgcaagt gttaagactt ttactcggaa taactaggct aggacacagg tggaattaga
92580

ttcactggct gatgtaatgt gtctggcatt taagagatat aagacaaatt gcaagtataa
92640

gggctatgga gccaggtgtg gtggctcacg cctgtaatct tagcactttg ggaggctgag
92700

gcaggtggat catctgaggt caggagttca agaccagcct ggccaacatg gtgaaacccc
92760

atctctacta aaaagtcaaa aattagccag gcatggtggt gggcacctgg aatcccagct
92820

actcgggagg ctggggcagg agaatcactt ggcggaggtt gcagtgaact gagatcacgc
92880

cattgcactc cagcctgggt gacaagagtg agactctgtc tcaaaaataa aataaaacaa
92940

aacaaaataa aataaaataa aggactatgg aattaggtgg ctattactga gcttcagtga
93000

tgccttaaag agaaaaaaat gaaaggctca ggacaataag taatcaattt aaagcaaaat
93060

ataagagtta caatgccttc ttggcaacat ttaaagagac ccttatctct cgttgcctga
93120

aggcagagag accaggctga atatttaact ttaagagtgg cataactaca gagaagttca
93180

aatttttcag cctagggaag tttctagtac caaaaccagg acctcaatag gaaagaataa
93240

gaccctgaga catgggatgg tgatattaga tagatgccct ttgaggtcct tggatcatca
93300

gattattctg gaccatttgg acctgcagaa gtggctcaca ctttttattg gaagatagag
93360

gcccactctt gcttgaaaac cttgcaaaag tcttacacta gacaaattca ttgtaaggaa
93420

atgctggctc ccctcaggat gtgttgcacc tcccccttttg gccactagac ccataacaaa
93480

aatcaaatct tagcatggcc caactgggga agtacttgac ttactaagga gagggatgga
93540

ttagatacta aaggagcttc aagacttgga caacatgtgt catcaggagc aaggaaaata
93600

aatcagagag aagattctga agatcctgga tcaaagggag gggactaaaa atagaaagct
93660

ggatgtagga gtttgtcaat atagggcatt cttatgggac acaggattta acaccctagc
93720

aagtttattc ttaggaggtt gatatgaaat gatactgggg tggcttttgg aaagctggaa
93780

aacaacaaga gcttctatta ataaagttga gatgtcagaa ctcatatgga aaattgtgag
93840

taaagggttc acagttctca gagaaatgga tgtgggagaa tagatctata acaaaagatt
93900

ggaaaatcca ccaccagact ctattccttg ggaagtcctg gataatactc attttcacca
93960

aagaaataat gaatgtgctg gtgatgagga gtttagaagc tgtaagaatc tcagtagtgt
94020

tcatcctctg tagacggagg ctgacagcag atgcccttat atactggata tactggttcc
94080

ctaatagcaa taggaatgat aggattctga atcagcagaa gctatttggc agcacttaat
94140

aatcagaagc aaagtagtta ttattattat aataatcagg aaggttgaaa ttatggccaa
94200

agggccttga ccttcaggga tctgtggaga tggctaatag aaaatagtgt ttccatggag
94260

aaaatatacg gttgcaaatg catcacattt gctatcaaca aactccaagt aggataaact
94320

caaagacatc catacttagg tacatgatga tcaaactgtc aaaagtcaaa gaccagataa
94380

aacaacaaca acttaagaga gaatcttgaa agtagcaaga gaaaagcaac tcattaagga
94440

caaaggatcc tcaataagtt taacagctga tatctcctca aaaccatgga ggccagaaga
94500

cactggaacg atatactcaa aatgcagaaa gaaaagggct gtcagtcaag gattctacaa
94560

ccagcaaaag tatccttcaa aaatgaagga ggaattgaga caattctaga taataaaaac
94620

tgagagaatt tattattagc ataactgccc tacagaaaat actaagagaa gtcctacaga
94680

ctgaaatgaa agaatgctag taagagatca tatgaagaaa taaagaacag ttgcaaaggt
94740

aatgacatag gtaaacataa aagacatata caaaactgaa agctttcctc ctagaagaag
94800

gaacaagaga aggatgtgtg atcttatcac acctattcaa caacacctct agctagtact
94860

ggcggttcta tttagggcaa ttaggcaaga aaaaaagaca tctagattag aaaggaagaa
94920

ataaaacttt gtttgaaaat gacataatct tgtgtagaga aaatcctaag gaacccacaa
94980

cagcttacta taattaataa attagttcag ccacatcaca gtatacaaga tgagtatatt
95040

aaaatcaatt ctatttctat acactagcaa taaacaatac aaaaaggaga atgagaaaac
95100

aattcaattt acaatagcat aaagaagaac aaaatactta gaaataaatg taatcaagga
95160

agtgccaggc ttctacacag aaaacttcaa gacattgttg aaagaaatta aagaaaacat
95220

aaataatata tattccatgc ttatggatta gaagatttaa tgttgttaac atggtggtgc
95280

tctcaaattg ataaaaaaat tcattgcaat ccttataaaa attccagttt cctttttga
95340

agaaatagac aaacagatcc taaaattcaa acagaattac aaaagaccca aagtagataa
95400

aaaatattga aaaagaagaa caatatacat aaacataaat tcaatcagtg gaatagaatt
95460

gaaagctggg aagcaaattc ttaaatttat ggccaattta tttttgataa gagtgtcaag
95520

acaattcagt cgggaaagaa cagtcatttc aacaaattgt gctgggacaa ctggatatgc
95580

atatgcaaaa gaatgagatt acctcacaac atacacagaa attaactcaa ggctgggcat
95640

ggtggctcac acttgtaatc ccagcacttt gggaggtcaa ggtgggcaga tcgcttgagg
95700

ccaggagttc tagacaagcc tgggcaatgt ggagaaaccc tgtctctaca aaaactacaa
95760

aacttagctg ggcatgatgg tgcacacctg tagtctcagc tgtttggggg gctgaggtgg
95820

gaggatcact tgagcctggg aaattgaagc tgcagtgagc cgtgttcata ccactgctct
95880

acagctgggt gacaaagtga gaccctgtct caaaaaaaaa aaaaatccat catttattaa
95940

gcactgaata atgttagtca aacaggatgt taaactggaa attttgattg ggatttcgag
96000

atgcaatgtt tgggaaatac aacacatatc atattccaaa gtgatttgct ggtctgagat
96060

gcagcaattc aaaggaaaag caaataaata aataaataaa taaataaata aataaaggtt
96120

aagctactct taaaatctac acccacatca aagttgtata aaatggcaga gttgttggaa
96180

attacaggga gagagaatta actttggaga gaagatatct ttgttttttt tttttaaatt
96240

tattttcttt ttttaggcta gttgagtgaa gcagtgggag tagagaagga acaaagacat
96300

ttgtaactgg ttgtgataaa ttgtttgaaa acagcactgc actttgacca gccaagagag
96360

aagatctttt tggagagagg tgtgctgtga gttcttctcc ccaagagcaa gggctaaggg
96420

tgctgccctt ggaatcttaa gaccatcagc agatgaatgg aagacgttgg aattggcctg
96480

catgcccagg ttataaagtg tgatatggcc gggcgcagtg gctcagcctg taatcccagc
96540

aatttgggac gctgaggcgg gtaggtcact tgaggccaag agtttgagac cagcctggcc
96600

aacatggtga aaccccatct ctactaaaaa tataagaatt agccgggcat ggtggcatgt
96660

gcctatagtc ccagctactc gggaggctga ggcaggagaa tcacttgaac ctgggaggtg
96720

gaggttgcag tgagctgaga ttgcgccact gcactccagc ttgggtgaca gagtgagatt
96780

ccatctcaaa aaaaaaaaaa atagtgtgat atgatcactt tacacaaaac tagtctgtga
96840

caagtggata aaaatagtat ttcatcacta ttttactatt tctttgattc ctacctggac
96900

taaacattta caggctttga ataacctttt ctatgaatgg tcttttcatg tctttgctaa
96960

gtttacgtac cggtgcactt agaattatct tataatttgt ataagctctc aatataattt
97020

tagtaagatg cttttttgat atgctatttt gtgctatttt tttcctttgc tttagagaga
97080

ggtgccaaga aaatttctct ttggacctta gtttctctta ggatgctctg caggctgctc
97140

cattctggga atcataggaa ggttgcaagg cttcccagag gcacttagga tagtcccctt
97200

tcagtcaaga agcttcactg gctggagaat gttctcaaga ccccctcctt gaatccatgg
97260

agagcaccag agaattagga catcttagct gctgcaggga ccacagtcct gcagagccaa
97320

ttgctggcag cagatatttt ggacaagaag gtactagagt cctggagaga gtctgaggaa
97380

aggaaggcca tctgggaata ggattttggg tggtggacgt ctgtttcagt cctgggttat
97440

gaggatgttt tgcacccagg aagtggttgt tctcatgaat agaaaggact attgtgactg
97500

agcctcatgc cttagtgagg ggcagtatct ggaaccgagg tgacatggtt tggctgtgtc
97560

cccacccgaa tctcatcatg aatttccaca tgttgtggga gggacctggt aggaagtaat
97620

tgaatcatgg gggcaggtct ttcctgtgct gttctcgtga tagtgaataa gtctcaggag
97680

atctgatgtt ttcataggtt tttttttctg cacaagctct ctcttggcct gctgccatcc
97740

atgtgagata tgacttgctc ctccttgcct tcaccatgat tgtgaggctt cctcagccac
97800

gtggaactgt aagttcactg aacatctttc ttttgtaaat tgcccagttt caggtatgtc
97860

ttcattagca gcgtgaaaat ggactaatac atgaggttgc ctctggcatc tcctctgtga
97920

agagtccagc attgaccctg agcactgtca cttcgaacac aggagaccag tccctctctg
97980

cacactccct cagccctgtg agttactctc ctcactttgc tgcttcccat tcttggcggt
98040

atctctggag agactcctgg ctctagcagt ggggctagga atggccatgg ggacaggcgt
98100

cacagttctc cctgggcctg acggagaggg gtctgggttc tctggattcc tgaagggacg
98160

tggagcagat tcccatcatg ctgtggcggg gagtctgtgc tattctccca gccaagagga
98220

gaggacatcc cgaaatcacc acaaattctg ggatgaaggc tgtctgagga aatgaagcaa
98280

acacacacaa acacacaaag acatacaaat acacactcac acttatacat acgtgcacac
98340

acacattctt ctgtgggtaa gcttgtgctt cagctacttt gggcaggaga gcagggggt
98400

tttattctac ctgtcattgc cttcatgccc cagtgctgga aacacactga agccattgct
98460

cccccttcag atgatctgaa aatccctgtt tcagttcact ttgaaaacac ccctcactct
98520

caacaacaga gggttggtta ttctgcagaa ctcattcttt aacagcaggc ctgctgctgc
98580

tccagctgca attcttttcc ccttcatgaa ggctgccagg aatgaaaagg ggagggaaaa
98640

gaagtaggtg gtggcaggag gctgggccag ggaagtgtgg gaccccacag gctccagcct
98700

gaggactcct ctctcggcct ctctcccctg ttgtgaatgc ttctgctgaa agccactggg
98760

tgagtgcagg gacagagagc cactgagctg gctcagggcc tcgtggtggt ggtggtggtg
98820

gtaggggggc tgctctgggt tctcgagagg cttctccaac tctgacatga tcatacctga
98880

ggatattatc gaaattcaga tatgacttag gaggcctggg atacggcccg agattctgca
98940

tgcctggtga tgccagtgtt gctgctctgt tgccacactg gataaaagca tctagaaggt
99000

attccaggcc agatggggtg ggcattccct ccctcatacc ttaactcttt gacatggatt
99060

ctgccaccaa cagaatcctg gacgttttct cccaagagat gataagaaaa attcacacca
99120

aaatcatgat ggaatttcaa aatctgtttg gatattagaa aatttctcca aaccctggcc
99180

tttccttcct ctcccaactc ttgagctcac aggtgttgct tcactgacta aaatcctgac
99240

agagctccaa ttctactacc tcccagacaa ctttatttgc aagctgtttc tctactcaga
99300

aacaggggtg tcccttcagc cgagcttttc caacaatgct gagctggggg ctttggtaca
99360

tctccccctc cttctcagcc tcagctctct aggtcctgaa gtttgacctg gggccctgt
99420

ccaccgtggg gcaaagctgc agacttgttg atgagaggag gatgtgggtc ttagggaaag
99480

agaaaggtga gctggggaca gctggaccag attggagaga ccacagggtc cctgaaaagc
99540

agggggtaag ggggtaggag gctatcatat agggaggtcc ctttggtatg cagactttgg
99600

gacagaaagc acagttttgt ctgaaccctc cagaaggccc tgaaagcatg ggacataagc
99660

attgtgttgg cgataacagc tgcaggtgaa ggcaggtgaa tgacaaggaa gggtgcttag
99720

ggttagatat gagagctcca aaccaagggg ccctgggcag gtccttttgt tctactgttg
99780

cccctctctc ctttccaaaa ccccaaagct ctgtggtgag ctctggagac ctcagctttg
99840

ttctgagagc ctctgagagg atttaggtgt ctcttcatgg gcctggggtg ggaatgggag
99900

agacagaaag aaaggggaca ctaggtacgg cctctggaaa tgatttgaat ccctgaccct
99960

tgaaatatgt cagacccacc ctgtgtgaaa ttccacgtct gctggaaatg tgacggaaat
100020

gtgtttagag ggtgctggac ctgcacttcg aaataaaaca aggctgacag caagacttga
100080

gaatttacct aatgatc
100097


<210> 165
<211> 100097
<212> DNA
<213> Homo sapiens

<400> 165
gatctcggct cactgcaagc tccgcctccc aggttgacgc cattctcctg cctcagcctc

60

ctgagtagct gggactacag gcgcccgcca ccacgcccgg ctaatttttt gtatttttag
120

tgtagacggg gtttcaccat gttagccagg atagtctcga tctcctgacc tcgtgatcca
180

cccgcctcgg cctcccaaag tgctgggatt acaggcgtga gccaccacgc ctggcctacg
240

cccgggtaac ttttgtattt ttttagtaga cacggggttt caccatgttg gccagactgc
300

tctcaaactc ctgacctcag gtgatccacc tgccctggcc tcccaaagtg atgggattac
360

aggcatgagc cactgcaccc ggcctgactt cacaatatta attggacata aaataggaga
420

aaaacaaggg gaaaaactgg gacaacagga agatctgttg tcctagtttt ggaacaacaa
480

aaatttggaa aatgagagcg caggcaaaga gtggagagta aggggaaatg acaacagagg
540

gagcatgggg agaggatgga agaaacagac acaaggggaa tgagcagagg gaagacaaca
600

aatcacagca gaagggaagg gatgagaagc aggagccttc agaggtcaca ggtctactac
660

tggatcacct gataattaat tggaaggttt atgtatacat ttaaaacatc atgttccatt
720

tctaacatta ttcataagtt caaaaaaaga acaggtcttt catagaaaat gattagtggt
780

attagcagaa agaaaggaat agtaaatatt tctccaattc tgtgagtttt ctatcaacaa
840

gctgatgatt aacaaacaag aaactttgtt ttgtttttgt ttttgagaca gggtcttgtt
900

ctgtcagcca ggctggagta cagtggcatg agcacagctc actgcagcct cgatctcctg
960

ggctcaagtg atcctcctgc ctcagcctcc caagtagctg ggactatagg cctgcaccac
1020

cccgcctggc taatttttaa atttttttgt agagacagtg gtcttgctat attgcccatg
1080

ctggtcctga actcctggct tcaagcgatc ctcttgccac agcctcctcg agtgttggga
1140

ttacaggtgt gagccattgt gcctggtcaa ggaacttgtt ttgaacctgg caggctacag
1200

gaatcttcag acacaggaaa tagtttctat ccaacaagga gaggaacaca gagctgacag
1260

tgctcaagta acagaggagt aggtggaaaa aagtggttac gacattagta aaaataatta
1320

aacccttcac tgactgacac agaataactc agaacaaaaa agaaacagaa aacaatgctg

1380

gagaccaggt gcagtggctc actcctgtta aatcccagaa ctccaggaag tggagacagg
1440

tggatcactt tagcccaagg gttttgagac cagcctaggc aacatggcga aacctcttct
1500

ctacagaaca tgtaaaaatt agctgggcat ggtgatgcat gcctgtagtc ccagctactt
1560

gggaggctga ggtgggagga ttgcttgagc ctgagaggtc gaggctgcag tgagctgtga
1620

tcgcagcact gcatgccagc ctgggaaaca gcaagaccct gtctcaaaaa aaaaaaaaaa
1680

aaaaaaaaat catctagtac aaagagcaag taccaagaga aattataagc cccatttatg
1740

cctgatcaaa aatagaactt gatgtttcat tagtaacatc cttcatgttc acgctttatt
1800

cacactccct ttgccctctc caggtagaag cttgctgtgg acctccctat tgctgtactc
1860

acgtgagggc ttctttgcag cactgtcctg aggggttaag atccagcaga gacaaatgga
1920

aatgtctgcc aacagtgtca ccttgtctta tctggctgat gcctcttgaa cttcctcgtt
1980

cttactacac atccaggacc cagagctctt catagaaaat caccattaag tttggaagct
2040

gggaatcctc atcacaagga cagtccaggt gcaagttcgt ggaattacac aaagcagtgt
2100

attggaatca cactaatcac acaggatgga agaagacaga aagccaacag aaggatgctt
2160

ggtagtttcg ggaatgtggt accgggacaa tgggttggga ttagacggat gatcagttcg
2220

cctggtcatc tctgctagga aagatattcc cccgacagag gcacctaaat tgtaatccat
2280

gggaacggag cagaactgcc tcccaggcaa ctgagtggct gaagaggtga ggacttggaa
2340

ctcaattatt tctgatttta aagagcaaaa actacttagc aataaaaaac ccacctggaa
2400

tcagggtcac agtaccatgc ttctggagtt aaatgaacac aaacattctc actttccatc
2460

tggtctacaa aaggggatag gtagtctcta actcctcaac actcaattga acacatcaga
2520

gatcccacag ctaacacaca gagagacaaa accatcttat cagacagtaa gcctgcatcc
2580

gtcttaccct ggggaaatgc gctcccataa ttctcctgcc tgttgtccct actgagattc
2640

ctccgagcca gattctgaca tccccattcc tccagaatga gggacacagc catgtcctcg

2700

atcttcacca ttgcctaaaa taacacgaga caaacagtca tttcccccca aactcaggga
2760

caatcccatt gctcaaacct ggagtcagga agagaggtgg ctgcaaagct acaggacgct
2820

gggaagaaaa gggtggccag aacctggggg tggaggggag tttgtggtag aaagatgcta
2880

aaaggagagg acagggttgg ggcttctttg agagcagggg catccagagt ggcctgtgga
2940

gataatgaag agagctcagg aacagaggca gacactgagg actcaggaga gcagaggggc
3000

cacagctcac ctgggaatcc gctgtgaata gtgcagatgc catcgcctgg tctctgggac
3060

taccctcatg agggggtgca ggaatgtggg cagcaggaag agctggggga ggagaaaggg
3120

tctgtgagta aatcagctcc gctctgagcc ttgctcaaag aagggctcgg ggcatgttcc
3180

ttcctgcacc agcatgttca ctgttcagtg tttaggtccc aaatacagaa ttccagacaa
3240

cacaaacgag agaacacagg gcctcagttt tcccaagacc tctactcttg agcaggagtc
3300

ataaaagggt cctaatcacc tggtccaacc cttttatttt aaagaagggg aaacttgagt
3360

ccctgagact gcaaatgact tccctaatgt cacattccca gtggcagagc caggatgggg
3420

acccaggttc ctattctcag ctataaaagt gggtaccttc agcccatagc tcaagtctgg
3480

ctcctaattt cctccagtcc aaggaacgat accaggatat gagaatccca tttatgccta
3540

cacttgtgga aaacacaaca cagctttaaa atctttagtg gcaggtgagg cgggaggatt
3600

gcttgagctc aaggggtcga ggctgcagtg agctatgatc acgcactcca gcctggatga
3660

tagagtgaga ccccgtctca atgaaatcca gaaaaacaac aacaaaaaaa acactttagt
3720

agcaatatcc agaaaggcag ggaagtaagg aggtctcctt agctctgggt aagttttttt
3780

tttttttttt ttttttttga gacagagttt tgctcttgtt gcccaggctg gagtgcaatg
3840

gtgcgatctt agctcatcgc aactttcggc tcccgggttc aagtgattct cctgcctcag
3900

cctccagagt agctgggatt acaggcatga gccaccacgc ccagctaatt ttgtattttt
3960

agtagaggcg gggtttctcc atgttggtca ggctggtctc gaactcctga cctcaggtga

4020

tctgcccgcc tcggcctccc aaagctctgg gattacaagt gttagccact gtgtccggcc
4080

acctctggct aagtttttgc cagtttttgt ccctctactt ttctccctca cttggccaca
4140

ataaatactg tttttttttt ttttttttga taagatgtat gcatggttca aatgctgaga
4200

caaaagttgg ggctgacaca caacaaaaga catgcataac attatcacgt gatcacaata
4260

actgtgatgc ataacaatta tgaaaatttt aaattgtgca atttaacccc ctccccatct
4320

cttgccaagt actattagat ttggaaatgt tactggtaga cagatgatgt tagtgagata
4380

caaggatatt tgagaaatga atttgaaact gaatgaccaa accttgtacc tatttcaaac
4440

ctttatacaa caataagata ggctctctgt cacccaggtt ggaatgcagt gctcaaacat
4500

gtctcactgc agccttgacc tcccaggctc aagtgatcct cccaccttgg tctccccaaa
4560

tgctaggatt acaggtgtga gccactgtac ccaagtactt tttttttttt tggagatgga
4620

gtctcgctct atcacccagg ctggtgtgca tggcgcgatc tcggctcact gcaacctccc
4680

ctcctgggtt caagtgattc tgctgcctca gcctcccaaa tagctgggac tacaggtgcc
4740

tgccaccacg ctcagctagt ttttgtattt ttagtagaga cggggtttca ccatgttggc
4800

caggatgatc tcgatctctt gaccttatga tctggccacc tcagcctccc aaagtgctgg
4860

gattacaggc gtgagtcact gcgcccagcc ccaagtactt aattttttaa acttctgaga
4920

gtaaggaaac actctggaaa acaaataaga aagaggaaat tgagaataaa tgtacatgtc
4980

tcaatttatt taagaactat ttactaaaga gcaactgtgt gtgccaggat ctttgaaact
5040

gttactctgg caacctaaac tctataatct agtgcttaag accatgaata tcctctccag
5100

tttcctaagg tcgccctgtg acttctgctt gtagagtgtg gagaaacctt acaagaacat
5160

caacagtagt ggagttaaac atcacggtgc accggtgttt tttttttttt tttttgagac
5220

agcatctccc tctgttgccc aggctggagt gcagtggcgc aatctcggct cactgcaagc
5280

tccgcctccc gggttcacgc cattctcctg cctcagcctc ctgagtagct gggactacag

5340

gcacccgcca ccatgcctgg ctaatttttt gtatttttag gagagacggg gtttcaccgt
5400

tagccaggat ggtctcaatc tcctgacctt gtgatcctcc tgcctcggct tcccaaagtg
5460

ctggaattac aggcgtgagc caccacgcct ggccacggtg caccagttta cacagctttc
5520

tctagcacat acctgctggc tgtcctccag gatgcacgga agagaggatg catccttaag
5580

ctccatgtct aatattcctt aaatgtcatc accaataaac acactggtta aggaatgtgc
5640

ctcgaccaat tctttttttt ttttttttga gatgagtgtt gctctgtcgc ccaggctgga
5700

gtgcagagga gccatcttgg tttgctgcaa cctccacctc ccgggttcaa gcgttcttgt
5760

gtctcagcct cctgagtagc tgcgattaca ggtgtacacc accacaacca actaattttt
5820

gtatttttag tacagacggc gtttcgccat gttggccagg ctggtcttga actcctgacc
5880

tcaagtgatt tgcccacctt cgcctcccaa agtgccagga ttagaggcgt gggccactgc
5940

gcctggcctt tttttttctt ttttgagatg gagttttgct ctgttgccta gcttggaggg
6000

tgtatgaatt tttaacaaaa aaagcttaaa aagtataaaa taaaaatttt tttaactaga
6060

aaaaccccta tagaataggg ggataaaaaa tgtttttgta tagctgtgca acatgtttgt
6120

cttttgagtt atgttattac aacagtcaaa aagttaaaaa aatcaaaaag ttcataaagt
6180

aaaaaagtta cagtaagcta atttattatt tgtatttatt tatttatttt tttttctga
6240

gacagagtct cactctgtca cccaggctgg agtgcagtgg tgcaatcttg gctcaccaca
6300

acctctgcct acaggttcaa gcaattcttg tgcctcagcc tcctgagtaa ctggaattac
6360

aggcatgcac caccacgcct ggctaatttt tttattttgt agagacagtg tttcactatg
6420

ttggccaaac cagtctgaga ctcctggcct caagtaatcc gcccaccttg gactcccgaa
6480

gtgctgggat taagccatga gccaccatgc ctggctgatt tattattgaa gaaacaaaaa
6540

ttttaaagat aaatttagtg tagcctaagt gtacagtgtt tctacagtct atggtagagt
6600

agtgtcctag gctttcacat tcactcacca ctcactcact gactcatcca gagcaacttc

6660

cggtcctgca agctccagtc atggttaagt gttccataca ggtgtacttt tttttttttt
6720

tttttttttt ttgagatgag tttcactctt gttgctcagg ctggagtgcg atggcatgac
6780

aatggtgcga ttttggctca ccgcaacctc tgctgcctga gttcaagcaa ttctcctgcc
6840

tcagcctcct gagtagctgg gattataggc acctgccacc aggcctggct aattttgtat
6900

ttttagtaaa gacggggttt ctccacgttg gtcaggctga tctcgaactc ctgacctcag
6960

gtgatctgcc tgccttggcc tcctaaagtg ctgggattat aggtgtgagc cactgcgctc
7020

agccaatttt tgtattttca gtagagaccg ggttttaaca tgttggccag gctggtcaca
7080

aactcctaac ctcaagtgat ccacccacct tggcctccca aagtgctggg attacaggcg
7140

tgagccactg tgcctgggct aaaaatcttt tatactctgt ttttactttt tcgatgttta
7200

gatacacaaa tacttaccac tgtgttacaa ttccctacag tgtttagtac agtaacatac
7260

tgtacagctt tgtagcgtag gtgtggagaa ggctagacca tctaggtttg tataagtata
7320

ctttatgatg ttcgcacaac aaaatcactt aacattgcac ttaccataac ttatccctgc
7380

cattaagtga taaatgtact tttagcattc cttgtcttta cattctaagc acatactttg
7440

attttaaaac tggataaatg tattacatat ttccgtcaaa agagaaaaaa caagtaacat
7500

gactttttat ttatatgact gtgaactgat cttttttcgt gttcctcaac tcccctcaaa
7560

agttcttcca ttcttttccc attgtttgaa atttctgaaa ataacctaga aaagactgat
7620

gctatagttt tttttttttt ttttggaaac agactctctg tcacccaggc tgcagtgcag
7680

tgattatttt tatatatttt aaataaaatg gacctttaaa aaagatttat gatccttttg
7740

aagataattc atgacatact aaaatgtttt aaaacctagg ctgaaaataa tgacatctgt
7800

tcccaatact ttcctattaa aaatgtgctg gacatctgga atgctgataa ttcaataaat
7860

aatgcctacc aggttcatta cgcatagttc atttgccttc gtagtaacat ttaactcctg
7920

agattggtct gtaagttgta ttttccccaa tatttggtaa taatgacaac aatgtagaat

7980

aactaaaaac aaaaaacaag catgcagctt tttttcattt ttttttaaat ttttaattaa
8040

ttcttttttt tttttttttt tttttaagac agtctcgctc tgtcaccaag gctagagcac
8100

agtggtacga tctcggctta ctgcaacctc cacctcctgg gttcaagtga ttctcctgcc
8160

tcagcctccc gagtagctgg gattacagga atgtgccacc atgcctggct aattttcata
8220

tttttagtag agacaaggtt tcaccatgtt ggccaggctg gtctcaaact cctgacctca
8280

agtgatctgc ccgcctcagc ctcccaaagt gctgggatta caggtgtgag ctaccgtgcc
8340

gagcctaatt cttttctgta agtagctttt gagacagggt ccttgctgtg ttgcccaggt
8400

tggagtagtg gcatgattac agctcactgc agcctcaacc tcctgggctc aagcaatcct
8460

cccacctcag cctcctgcgt agctggaatc acaggtgtgc gccactatac ctggctaatt
8520

tttctatttt ttgtagagat ggggtctcgc tacgttgccc aggactaaca tgcttcttca
8580

tcacaggcac tcagcagcac aaagactctc gtcctgaatc atttcccttc ccctaaatga
8640

aaccttgctt cttacctcgt gactgtaaga ggcggggttt ccgagacgaa tgtttgaagt
8700

gggactgggt ggcctcgtga tgaaggtcaa agctcgagga ctcctgaact ggatccagag
8760

gcaccatccc ccttgcgagc atctcaggtc catgaacttg acctgggacc tgaagacaga
8820

caggttgggc ttgtgggtaa atcactttgt aaatgggaat taaaatattt aaaaacagac
8880

catgagtatg ccagtggatg caggaagaaa gccagaacat ttttaagaca gagtaaccac
8940

ctaataacct aaagcctgcc aatacttctc ctggctccag tttcaacgtc tgtgccctgc
9000

tcacacataa taggtttcac ctctttttac ctgttgtcct gataaatcaa gctccaagtc
9060

ttctagaagg gtcacggcct cctctccact atcggggcgg tattcctgca gccagacctg
9120

gagctccttg ggcaggatgg aaagaaactg ctctagcacc agaagctcca ggatctgttc
9180

cttggtgttt atttctggcc gcagccactg atgacaaagt tccttcagcc gactgagagc
9240

ctctcggggc ccaaaagtgt tctggtaaca gaagcgcctg aagcgttggc ggaatatctc

9300

tgggtctgga ggaggcgtgt cctgtagggt ggaatcctgc ccccacatgt ggtcttcctc
9360

atcttcctct tccaccttca ctattacgat accatccttc tcctgtgcag cctgtgggga
9420

cagacccgtg gcttcccgtg attcagcagt catcattcag gctccaggaa ctgacttgat
9480

ccaaacaggg tctgtgctca cctttatgtc ctgggaggtt ttatgatgtg tttctttact
9540

attcctgaag tataaaaaaa aagtcattag tacgtacctt tacaaaaagt gatctgtgct
9600

gctaacactt tataggaggc agcacatcga ggcactatca gaacgctcct gaggccgggg
9660

gtggtggctc acgcctgtaa tctcagcact ttgggaggcc gaggcaggcc aatcgcctgc
9720

ggtcaggagt tcaagaccag cctggccaac atggtgaaac cccgtctcta ctaaaaatac
9780

aaaagtcagc agggcatggt ggcaggcgcc tgtaatccca gctactcgga aagctgaggc
9840

aggagaaacg cttgaaccca ggaggtggag gttgtagtga gccgagatta cgccactgca
9900

cttcctacct gggagacaag aagttaaact ccgtcaaaaa aaaaaaaatc ctgacaaatc
9960

gaggaaaaaa ctgtacaatc tccactgtca cttcatatat caagatttgc aaacagaaac
10020

ctcaaacagg ccgactccac cctcagggtg tttgcttgga gtcagagtga tttacctgca
10080

ggttgacagc aggttacgaa atggagcatt aacagtgccc agcccagaga gctgaatgtt
10140

agcagagtgc caggtgccag gcttaaagca gtacacgcac catcttcttt tgtccctaca
10200

acagccctag caggtcggcc aaactgttaa gtgctgtttt acaaatgaca taactgaggg
10260

ttaggtttgc tcagtagtag gtggctgcct actggagtca atattcaacc aaatttgaag
10320

ccctgggacc tacttttgta ccatccagct acttttgcta tttttatgag tgacttgggc
10380

agttaaattg tggtttccaa agtacaagat gatccgcaaa ggatacagaa agaaaataat
10440

tggaatgtct gtatcttaaa agagagagaa attaagctat aaattgacac taatacactt
10500

acttagtctg tgctgacctg tcacaggtgt tgttcatttt agacattgtg atgtagactg
10560

gtttatctga gaccagttat gtgaaataat ggatttctaa atctagcttt ggctgggcgc

10620

agtggctcat gcctataatc ccaaaacttt gggaggccaa ggcgtgcgga tcacgaggtc
10680

aggagatcgg gaccatcctg gtcaacatgg tgaaaccctg tctctactaa aaatagaaaa
10740

attagctggg cgcggtggcg ggcacctgta gtcccagcta cttgggaggc tgaggaagga
10800

gaatcacttg aacccaggag gcggaggttg cagtgagccg agatcgcgcc actgcactcc
10860

atcctggcca cagagcgaga ctctgtcata aaagagacaa gcagctttaa agattcttgt
10920

ggccagatat ggtagctcat gcctataatt ccagcccttt gggaggatga ggcaggagga
10980

tgcttgaagc cagaagtttg agaccagcct gggcaacata gcaagaccct gtccctatga
11040

aaaagaaaaa aattagctgg gcatggggac tgtgtctgca gtcacagcta ctcaggaggc
11100

taaggtggga ggattgcatg aaaccagaag tttgaggctg cagtcagcta cgatcacgcc
11160

actgcattcc agcctgggtg acagagcaaa gccctatcag gaaaaggaaa aaaaaaaaaa
11220

aaaagattct tatagagaaa ctgcaattac aggacacact aataatataa acagatgtga
11280

acaagaagaa aatggcacag atgaagcttc tatctagagt aaggttttag ccacattagg
11340

aggtaaaaac caatgtcagt ctaaatcaaa gaaaaagtca ccaatgagta atcagcttct
11400

ttaaagaaag tgtgaagaaa gggcaattac aggaacagct gtatgaaatg gttccattgt
11460

tatgtaattt aactgccaaa aatgatgtca agtatccagg aaaaacccat ctatgttcac
11520

attttagaaa cttggggctg ggtgaggtgg ctcatgcctg taataccagc actttgggag
11580

gctgaggcag ggagcagggg gccacctgcg gtcaggagtt caagaccagc ttggccatca
11640

tcgtgaaacc ccgtctctac taaaaataca aaaaattagc tgggcatggt agcaggcgcc
11700

tgtaatccca gctatttggg agactgaggc aggagaatcg cttgaaccca ggaggctgag
11760

gttgcagtga actgagatca cgccattgca ctccagcctg ggtaacaaag tgagactcca
11820

tctcaaaaaa aaaaaaaaaa aaaaaaggaa aaaaaaaaga aacttggaaa tattttctaa
11880

cctgtttaaa aatctttcaa atgaagaatt tcaggtgggt cttaagccta tttgttaaaa

11940

atataaaatg ccaccacctg attagtttgt aagactatac cctgcatttc aacaacaaaa
12000

attcagcatg gttgataggt tggatcaatc acaagttact aagcagaact gacactgtga
12060

ttcttccatc tgaagctaca aatctttctg aatttattct tttcacctct acagccatca
12120

aaactaagca tcaaattgaa gttttagaaa cagaccatta aactgcttta tcatgatggg
12180

ttaaaccgag ttttaaacta gcaaaagcat ttttagtttt atggctctca ctaaacatat
12240

tcttaatttt ggtgaaaaag ggatcatgta catataacag cacaaaaatt atatatacat
12300

atatattgaa acggagtctt gctctatcgc ccaggctgga gtgcaatggc atgatctcag
12360

ctcactgcaa catccgcctc tcgggttcaa gtaattctcc tgcctcagcc tcctgagtag
12420

ctgggattac aggccatact tatttttttt aatctagttc catgaagtcc agcttccttt
12480

ctttggttcc ctgccttcat tttctccctt ctctcccaca atccaccttt cagattattc
12540

tttctaaaac tctacagcct tgcagttaat actttccagt gcctggtccc aattttccct
12600

caagcttctt ttcccatcaa tccccccata aaccttccct atgcaacaaa agccctctca
12660

tcagtatttc ctatatatgc cttgtatttt gactgccatc ctttcctcac atcacactcc
12720

atgcctggga cttccccccg cagtcctctc tgccttttca aatcctactt aacctttaaa
12780

gctagctaaa acgcgacttg cctcttatca ctgttcaaga accctctcat aactataaca
12840

gtaagagcca atgtttactg agtgctttct aggtaccggg tatttaagca ttttcacttg
12900

accacataac aatcttagca ggtagctaca cttatcatct cactgtataa agaactgaga
12960

ggctgagcgc agcggctcat gcctgtcatc ccagcacttt gggaggccaa ggcagataga
13020

taacctgagg tcaggagttc gagaccagcc tggataacat ggcaaaaccc catctctact
13080

aaaaatacaa aaattagcca ggtgtggtgg tgcaatgcct gtagtcccag ctactcggga
13140

ggctgaggca ggagagttgc ttcaacctgg gaggcggagg ctgcagtgag ctgagattac
13200

accactgcac tccagtctgg gcaacagagt gagactccat ctcaaaaaaa aacaaaagaa

13260

gaactgagat atactgccat taaataactt actcaaggtc acacagcaag tatatggtag
13320

aggcagaatt tgaaataaaa gtgattcccc aatgtaaact ctttaaccat tatacaaaat
13380

ttatcttgta cctgacatgt ggggtataca gatcctaaaa tgaaggccta tgaacgtgac
13440

aatgaagaat caatcagggc caggcacggt agctcacgcc tgtaatccca gcactttggg
13500

atgccaagga gggtcgatca cctgagctga ggagctcggg accagcctgg ccaacatgac
13560

gaaaccccgt ctctactaaa aatacaaaaa ttagctggat gtgtggcaca tacctgtaat
13620

ctcagctact cgggaggctg aggcaggaga atcacttgaa cctgggaggt ggagggtgca
13680

gtgagctgag aacgcgccac tgcactccag cctgggtgac agagagagac tccttctcaa
13740

agagaaaaaa aaatcactca ggaacgggcc ttatctacca cccagttttc actggagggt
13800

cactatgtga ctgaagccct tcctgggaaa gggaggagac acagcagaag attcaagtgg
13860

ggactgtcta caacataacc aggtgggatc ttcaaacatg gtaactgagc agaactagtg
13920

tgatagcgag cccagccacc agaaatccct acttttgtga aagttttatc ctgccttagc
13980

tattgagcag gacacttctt gggtcagtgc gaagatgcga ctctaaggag gaaatgagtc
14040

cagggggaaa tagacacaga catgggcaca ttccctctgc ccacaactgg gacagatcct
14100

cctcctcacc ttcagtatct ctctgctcct actctggggt gcagggaaaa gccatcacag
14160

cagtggaaag gctgggtttt atttgaggtt tcccagatga tctgagcaca tactcttctg
14220

tctctaaagg ccctaatact cttcaccaaa catttgctag gtaatttctc atgatgcgcc
14280

gggcgcagtg gctcatgcct gtaatcctag cactttggga ggctgaggtg ggcggatcac
14340

gaggtgagga gatcgagacc atcctggtaa cacgatgaaa ccccgtctct actaaaaata
14400

taaaaaatta gctgggtgtg gtggcgggcg cctgtagtcc cagctacttg ggaggctgag
14460

gcaggagaat ggcgtgaacc cgggaggctg cgattgcgcc actgcactcc agcctgggtg
14520

acagagcgag actctgtctc aaaaaaaaaa aaaaaatttc tcatgatgtt tctttctctg

14580

taatttttct tgccttcttc ctcaactaga ttattaagag cagtcctctt tgtactcctt
14640

agaccactta gcatgattcc ttgcaagcct cagtttcaaa aatacacttg actgaattga
14700

caacgttact attttttaaa agcccgatca ttctacattc aatgttgcac tcaaatttgt
14760

tccatggact agttggatga gtttcaggtt agcattaaag ctgctccacc ccttactagt
14820

tgacggcctt aggcaagtca acaagttaac ctctctatgc gtcagtttcc tcatctgtat
14880

aatgggaata aaaatcagca tttgtcccat agagttgccg tgaggattaa atgaggtaat
14940

agatataaag cacttaaaat agtgcctggc atttagtagg cactacagaa atgtcttaat
15000

ttttttcaat aagcttaaaa gatgtaactg tacattccaa ttttcagcct gcttagaaga
15060

ttaagcaatg ggacaactcc tgtaatagtg aaagctgcac aggcaataac tgaattctag
15120

tctcccacaa gtaagtggca catccctgag agaggtattt aacttctctg gcattttgta
15180

tctttatcag tcaaatgggg gtagggtggg ggttccagat agtaggaaac tacctctggg
15240

tgaatatggg ctgaaaaaca tactgacata ccatccactt taaataaaat tacctccagc
15300

agctaaggat acctcagttc tcacctagga taaacagaaa tgattttctt aaacaacggc
15360

ttgaagttac aagctcaaag gctgtgttcc aaacagccca gtatttttcc agggcatagt
15420

cttgaaaatg taaacaaagg ctgggcacac agtggctcac gcccgtaatc ccagcacttt
15480

gggaggctga ggttggcgga ctgcctgaga tcaggcgttc gagaccagcc tggccaacat
15540

ggcgaaactc tgtctctact aaaaatacaa aaatcagcca agccttggga cacatgcctg
15600

tagtcccagc tattcgggag gcggaggcag gaggatcgct tgaactcggg aggcagaggt
15660

tgcagtaagc cgagatcgca ccactgcact ccagcctggg cgacagagca agactctgtc
15720

tcaaaaaaaa aaaaattttt ttttaaataa attgcccttt gccctttaca ctcatttttt
15780

taaagagcaa tcaaatgttt taatatgaaa aagctacctt aaaagacagc tagctattca
15840

aagaatattg atactcccac agaatgaaaa tgttagattt aatatgctat tcatctgtat

15900

tttaaatgct tttaaatcga gaatgagcaa aagactaagt agctagcttc ctcgtttgta
15960

ttttaagagt ggtgaaaact gcctgaaata ataaacaaga atcttatatg ttaattcaga
16020

gaagatacaa acctggatac acattctggg gcaaatcacc atggtttcct atgtaaaaca
16080

gcgatttctc cacatttgtt cagcatatat actaacagtg ctttgctgat gtggttacaa
16140

tgaattctcc acaattcaat tcaacaagta tttattgacc gcctccaatc cgcctagcac
16200

tggcccgtac aataaatgct ttctttggtc ccaggccatg gaacagccac tctctataat
16260

gaaagtatct tactgtgtac agggggcact gcaggaaagg gaggggaggg atggaggagt
16320

gtcaagctaa actgtagtta attcttcaat tacattgacc aaatcatttc gaagtcggaa
16380

gggccttttg agaaaatcca gttagttttt attgatgacg aaattaagac ttggtgagtg
16440

tctcgtctta tccaaggtca ctcagctaat cagtctccta gtgacacagt gcaatttcca
16500

caaattccct tcacttctca ccccactcca tgtcaaacag agcaactatc cagaattgaa
16560

aaatccacct aacgtcccgg acagctcttt tccaaagcct tcaaagcatt ccatcaagct
16620

gaaagagtgg ggtgtatata atctctacta gatcccaatc acctatttat ccactatgac
16680

taggcttcaa atactcttta atcaccgctc agtgatgagg gtgggagaag gaaaggcagg
16740

tctggaaacg tatgcggcac ctcaaaatgt gtccatctct catccttccg ctcatttcac
16800

ctcgaggtcc cgaaggaacc ggggggggaa aaagacacct caccggaatc aaagcaaatg
16860

gcgactaagg gagagcagaa aaatcaagac cggggtcggt tcgctcgcga ccccgtttgc
16920

agaaacgtca aagggcgatg acgacactgc gggagagggg cccacagacg gcttctcccc
16980

gggtctccgg gcaccctcag ccactgaaac cctcgccccc tccccgcctg atcctggggg
17040

tggggcgggg acgtctccca ggaagaacgg cgctttctac ggccgggccc ggccaggagg
17100

caggggcatg gactctcttc gcacaacagc ggggccgcct ggagggggtc ccaggccgc
17160

agctgcggag cccaccctcg cgcacacacc cttgcggccc cgccctccca ccccagaggc

17220
cggccccaag tctcaccgtg agccccggga gcggcctggg ggcgctgggc gagaaagggg
17280
agctgactct ggggctcagg ccggccgaag ggcaccgcac acggacttag gccctcccga
17340
cgcctcgaca cagacacaat gagtcacaaa ggccggaagc gtgtcaccac ctcgtcttcc
17400
gggaccgcgc cgcgcttccg ggtcctctct aggagcgcgg gaggactttg catctcggtt
17460
tccgaatcgc tggaccaggc gcctgccctt gagttttcaa gcctccagct tggaggatca
17520
ggtcccagtc cgcccctagt taatgcaggg actaacggga gcaggtggtg gggggtttca
17580
ggctttcccc tactcaaact caaacagact ctccagctca gacaccagtc catgtgaaag
17640
ttcgatttca cctgtctttt tcatctcagg cagtccttgt agtaattata gttattatca
17700
agcaatatag tacttggtaa ttgtatgtct gtggcaaagg aactgaaaca aggatgtaga
17760
ctgaggaaga tcaaagtgaa gccaagggtg gctggtttcc caaagctgcc acggtagctt
17820
ctctcagggc cctggagcta tatggagtgg tattttcaaa tacttggccc gtggacaagg
17880
gatgtctttc cacaagcaag catagttata aggttctgtg ctttgctcat gtggcatatg
17940
ctattatagc cgtttaaaag gagttttcag agtaacaaca gacactttta ccttcaaagt
18000
gctttcacaa cctaccacag ttacagcctt catctttgcc ttgcagggta aggcacgcat
18060
aagagggggc cacgttgtgc aagggtgggg gggactacag gctttaatgg ttgtgacttt
18120
ccactatgtt ccgagacaag ttcatgggag aaactcagta gcttggttgc atgactctta
18180
ctgcttggag agatttactt ttttttttta gatggagtct cgctctgtca cccagcctgc
18240
agtgcagtgg caccatcttg gctcactgca acctccacct cccaggttca agcgattctc
18300
ctgccttagc ctcctgagta cctgggacta caggcgcaca ccaccatgcc tggttaattt
18360
ttgtattttt ggtagagaac agggtttcac catattggcc aggatggtct cgatctactg
18420
accttgtgat ccacccgcct cagcctccca aagtgctgga attacaggcg tgagccaccg
18480
tgcccggcca gatttacttt ttaaaaaatg tttgaaaact aaaaagcaat gacagggcca

18540

gacacgatga ctcaagcctg taatcttagc acttcgggag gctgggacgg gaggattctt
18600

gagcccaaga gttggagacc agcctaagca acgtaatgag accctctctc tacaaaaaat
18660

actaaaatta gctgggtgca gggtagtgtg tgccagtggt cccagctgct tgggaggctg
18720

aggtagaagg atagattgag cccaggaggt tgaggctgca gtgagctatg attgtgccat
18780

tgcaccccag tctgggccac agagcaagat cccatctcaa aaaagaaaaa aagggccggg
18840

cacggtggct catgcctgta atcccagcag tttgggaggc caaggtgggt ggatcacgaa
18900

gtcaagagat tgagatcatc ctggcccata tggtgaaacc ccgtctctac taaaaataca
18960

aaaattagct gggcgtggtg gcacacgcct atagtcccag ctacttggga ggctgaggca
19020

ggagaatcgc ttgaactcca gaggcggagg ctgcagtgag ccgagatcgt gccactgctc
19080

tccagcctgg ggacagagct agactctgta tccaaaaaag aagcaacaac agtatcatta
19140

aaggaaattt gcaaaatgga gaaaaataat taccggtaat ctctccaccc taacacaatc
19200

cccatggctt tagtttacct tttcctgttt ttcatataca aataaatatt tattgatatt
19260

attttaaaca ctgttcaaaa ccatagttgc agttgtattg aagaggaggc tggtatcctg
19320

gaagtatctg ctacattcat atcatgtgaa cactgttttt taaggaagag aaaatatatt
19380

tactattcag taagtggaaa tagatcatca tcaaggtctt catactcatc gtcttcacac
19440

tgagtaagct gaggaggaag aggaagggtt gctcttgctg tctcaggggt ggcagaggca
19500

ggaagtgatc caccagcctc ggcctcccaa agtgctagga ttacaggtgt gagtcactgc
19560

acctggcctc ttttctttct ttttttttga gagagtttcc ctcagttgcc atgttggagt
19620

gcaatggcgt gatctcagct cactgcaacc tccgcctcct gggctcaagt gattctcatg
19680

cctcagcctc ccaagtagct aggattacag gtgtgcactc accatgcctg gctaatttgt
19740

gtatttttag tagagatggg gttttaccat gttggccatg ctggtctcga actcctggcc
19800

tcaagtgatc cacccacctc agcctctcaa agtgctggga ttacaagtgt gagccaccgc

19860

gcctggcctc gacatctttg attacttcct aaggatggat tccaagaaat gagattactg
19920

gtcccacaga tagtcagagg gaatataggc aataacgttt gtaaccccag ggttctagac
19980

ctgtcagcaa acaagctcag ctgacaagat caggtggtat ccacccaaga gttttgaaag
20040

gactctaaga tgaaagtgtt gaaagaatca tgaaactaat gatttatgat tacaaagaga
20100

cattgtgctt aaaaattggc tggctgccct gaacaaataa aagttcctga agggcaccct
20160

agaaactgtg gttgcctaag tttgacttct gtgttgtgaa agctggtgga gtcaatgctg
20220

aaggggagcc cgggtttgcg gctgtgactg ggtggctgca gctactcctg ggagggaggg
20280

tctcctgtcc ctccaacttg gaaggagtgg gggcttccat gtgttcccag ctccctccag
20340

ctctgtggag tgctgcgatc ccagctatgc ctcactcact gcagccagca tcatggcagt
20400

ggccactcca gatggggtgc tgctgccaag gcgggcagat cacctgaggt caggagctcg
20460

ggaccagcct gaccaacacg gagaaacccc gcctctacta aaaatacaaa attagctggg
20520

catggtggtg catgcctgta atcccagcta ctcagtaggc tgaggtagga gaatcgcttg
20580

aacccaggag gcagaggttg cggtgagccg agattgaacc attgcactcc agcctgggca
20640

ataagagcga aactctgtct taaaaaaaac aaaacaaaac aaaaaaaaca aacccaagtc
20700

ttatatctga ttccaaaggt cccattttac tgagtcttct tctttcttct tgacggagtt
20760

tttgcttttg tcaccgaggc tgggatcact gcaacctccg cctcccaggt tcaagcgatt
20820

ctcctgcctc agcctcccac gtagctggga ttaaaggcgt gtgccacctg cctgactagt
20880

ttttgtattt gtagtagaga tgggattttg ccatgttggc caggctggtg tcgaactcct
20940

gacctccagc gatccgccca cctcggcctc ccaaagtgtt gggattatag gcgtgagcca
21000

ccgtgcccag cctgagtttt ctttactgtc ggctagggct gtggctgggg aacctgctgt
21060

gggaagggta ctttcatcta tgcttttcct cctccccttc cctctccctg gggtggcagg
21120

agtgagtgat ctgactgttg gagacacctt ccctctctat tttcctattg gaagctgtgg

21180

cttctctgtt gtgttgagca aagtggacgg atagggagag ggtcaaaaat ctcttactta
21240

actgactatt ctctcctgtt ggttgttgct cttccctcgg taacacagtg ttccagctac
21300

ttaagcagca aaacaaatta ttcaacaatt tcgtgggtga aagcaatgac aacattgttt
21360

attttgctcc taaatcttcc atttggtgga gggatccaca tggatggctc atctatgctc
21420

cacctggctc ctctgggggg cctcaaaggt tggaccatct gaaggctcgc cagctcctaa
21480

gattggtgat tggtctggct gtcagcggag atcttagctg gggcagtgga tctgaacatg
21540

tgcaggtggt ctcttcatgt gctctgggct tcctcacagg atggtggctg gggtccaagg
21600

acaagtatcc agagagagag agaggaggaa ggaaggaagg aaggaaggaa agggaaggga
21660

agggaaggaa ggaaggaagg aaggaaggaa gagcaggtga gcgggcaggc aggtagggag
21720

ggagggagga aaggaaggaa ggaaggcagg gagggaggga gggaaagaag gaaaggggag
21780

ggagggaaag aaggaaagaa agggagggag ggagggagtg aaggaaggaa ggaaaggaaa
21840

gggaagggaa tggaagggag ggaaggaagg aggtacggga aggaaggaag tggagtgggt
21900

gacagagaga cagaggggac aggtgaatga aggccacatg tcctttataa cctaatctta
21960

gaagcactca gcatcccttt cactgcattc aatgccttga ggcagtcata aagtcacacc
22020

tagtttcagg ggaaggaaaa atatgttccc ccctcttttc ttcttttttt tatgacacgg
22080

ggtctcactc ttgtcaccca ggctggagta caatcatgac tcgctgcagc ctcgacctcc
22140

tgggctcaag tgatcctcca ccctcatctt cccgtgtagc tgggactaca ggcatgagca
22200

actatgcccg gctaatgttt aaattttttt tgtagagatg ggggtttcac tatgttgccc
22260

aggctggtct caaactcctg ggttcaagca atccttccac ctcagcctct tgagtagctg
22320

ggagtacagg tgcacgccac cacacccagc taattttatt ttattttttg ttagagatgg
22380

ggtctcacta tgttgcctaa gctggtctta aactcctggg ctaaagggat actcctgcct
22440

tggcctccca aagtgctggt tttacaggca ccatgcctgg cgatgccacc tttttttttt

22500

tttttgagac taagtctcac ttttgtcgcc caggctagag cgcagtaacg cgatcttggc
22560

tcactgcaac ctccacctcc cgggttcaag caatcctcct gcctcagcct cctgagtagc
22620

tgggattaca ggtgcctgcc actgtgcccg gctaattttt gtatttttag tagagacggc
22680

catgttggcc aggctggtct tgaactcctg acctcaggtg atctgcccac cttggcctcc
22740

taaagggctg ggattacagg cgtgagccat ggcacccagc cagatgccac cttttgatgg
22800

gaagtgatgt ggttctggaa ttgcatgtgg gaccaaaaac gctgccaagg tcacttttgg
22860

aaaacacagt ctaccgctac tgtgcgtcag ccacacaaac gttggcttgt ggtgttcagg
22920

gatgaaatcc tcagggggcc ttgggggcct gcttactact cagtcccctg tcccagtcga
22980

tctgccttct gcccctagca gggcactgca caacctcctt ttgaggagat tcttgggtgg
23040

gggcaccagt tggacaactc aagtccagct acctgctcct gggtccattt gatctgtgac
23100

aaactcaggc acccttggcc actcaaatgg ctggaacgtg gacctcctgc ggtgctcctg
23160

cccctccctg gccaccttcc cctcttagtg aatggaacgt ggacctcctg tggtgctcct
23220

gcccctccct ggccaccttc ccctcttagt ggctttgttg aatcacccgc cacctgccta
23280

ggcacgagag tctcctattt aaaataatgc tcaactagag gttgagggtt tttttgtttt
23340

tgtttttgtt tttgagtctt gccctgtcgc caggctggag tgcagtggcg tgatctcggc
23400

tcactgcaat ttccgcctcc tgggttcaag tgattctcct gcctcagcct cctgagtagc
23460

tgggactaca ggcctgtgct tccacaccta gctaattttt gtatttttag tagagatggg
23520

gtttcaccat gttggccagg atggtcttga tctcctgacc ttgtgatctg cctgcctcgg
23580

cctcccaaag tgctgggatt acaggtgtga gccactgtgc ctggccgagg atgaggtttt
23640

ttgtagatat ggagtctccc tggtgactgg gattcctgcc cgccctgccc cctgctcttt
23700

tttttttttt ttttgagatg gagtctccct ctgttgtgca ggctggagtg cagagtgcag
23760

tggtgcaatc tcagctcact ccaacctctg cttcccggtt caagtgattc tcctgcctca

23820

gcctccccag tagctggggt tacaggtgca tgccactacg cccagctaat tttttgtatt
23880

ttcagtagat atgagacttc tccatgttgg ccaggctggt cttgaactcc tggcctcaag
23940

tgatctgccc acctcagcct cccaaagtgc tgggatcaca ggtgagagac accgcaccca
24000

gcctctgtcc ccaggttttg aagacattct cacttgtctt catggtgagg gggcaaggga
24060

accagtcatg gaggaggaga gggaagcccc caccaatagg cagaaggcaa agtcctttct
24120

ctttggcttc taccaagtcc tttctctctt tcttgctgct ttcgtccaga cttgaggagc
24180

agaaaaactg gctgagcaag caaagctcgc gagcgtttct gcctgtcacg taggtggcat
24240

ctgggatctt tgatgtgccg tgctgtgggc tgggagtgtc ctcctgaagt tttccttct
24300

ttgttctcag ctatggactc cagccacgga caacagagtc ctagcccacg tccagtcaca
24360

tgactgcaaa agcacctgcg tctggatacg gatgtttatg agaacattgg tgcctaatca
24420

aatcaaatca aaacaaacca aacagcccct agccttgtgg cctcattccc aagtcagccc
24480

aagccatatc cttcctagct atggtgtata gtagaggggc gcaggactca gatggtcttg
24540

gttctcatct tgtctgtacc atgtactctt ggtctagtta cttgaacttt tggagcctca
24600

gtttctctaa gtgtaaaatg aggattcaaa cagcacctac ctcattggga tgtcatgagg
24660

cttgcatgat ggagttgatt catgtaaatc agcagttgca gggtctggca ttggacacag
24720

atgaatttag ggctgtgggc tgttgactga cccttgggct tgagtgacat gtgtggctcc
24780

atcctgaagc tcttttctag cagtgctgag gtgggaggag ggacttgact ctgcaggtgg
24840

ggcttggaca gtggaccaaa ttgaggatta gctaaaatgg ggccagggca gaagcagctt
24900

tccataagac atgcccacca gagtgccatg tcagtttacc gttgccatgg caacacccgg
24960

gagttaccac ccctttccat ggcaatgagc tgacaaccta gaagttacta cctcttccct
25020

agtaatttct gcactaactg ccccttaatc tacacgtaat taaaagtagg taggccaggt
25080

gtggtggctc acactcacac ctgtaattcc agcactttgg gaggccgaga taggtggatc

25140

acttgaggtc aggagttcga gaccagcctg gccaacatgg tgaaacccca tttctactaa
25200

aaatacaaaa atcagccggg tgtggtggtg cacgcctgca ttcccagcca ctccggaggc
25260

tgaggaatga gaatcggtta agcccaggag gcagacgttg cagtgagcca atatcgcgcc
25320

actgtacact gtactccggc ctgggcaaca gagggagact ctgtctcaaa caaaacaaaa
25380

caaaacaaaa ccccaaaaaa caaaaaataa tctatggtgg tgctgaatgt agacagagaa
25440

gagccagtgt ggttgttcac gtctgtaatc ccagctactc gggaggctga ggtgggagga
25500

gggcttgagc ccaggagttt aagaccagct tgggcaacac agtgagactc catctcaaaa
25560

aaaaaacaaa caaacgaaac aaaaacaaaa acaaaaaacc agtgactgta gagagagaat
25620

aaagtggttg ggggaaaaga agtcagggct tcaaagtaga aagagtactg aaaacttata
25680

aatagcactt tatcaggaag gtaatgaaag acctgaatgt aggttggtta aaagcatgat
25740

agcaggaaga atccaggcac attgaagatg aagacagaaa aaaacaaaca agccacattt
25800

agcaatttca gcaggaaatg aacacagatc ctacggggag gcgaagttaa cttgtaaatg
25860

agactctgta tgaaggtacc tctatataca tttgcgtaaa actagtttaa aagctgcaaa
25920

acacagtcac acagttgtta cagaaacgtg atcactaaag aataaattgg ggttggggca
25980

gggcatggtg gctaacgcct gtgatcccag cattttgaga ggctgaggca ggtggatcgc
26040

ttgaggtcag gagttcgaga ccagcctggc caacatggcg aaaccctgtc tctactaaaa
26100

tacaaaataa ttagccacgc gtggtggcac atgcctgtaa tttcagctac ttgggaggct
26160

gaggaacaag aatcacttga accccggagg aggagagatt gtgccactgc gctttagcct
26220

gggcaacaga gtgagactct gtctcaaaat aaataaaata caggctgggc gcagtggccc
26280

atgcctgtaa ttgcagcact ttgggaggcc aaggtgggtg gatcacttga ggtcaggagt
26340

tcgagaccag gttggttaac atggtgaaac cctctctcta ctaaaaatat tttaaaaatt
26400

agccaggtgt ggtggtgcgc acctgtaatc ccagttactc aggaggctga ggcagaagga

26460

tcacttgaac ctgggaggtg gaggttgcaa tgagccgaga ttgcgccact gcactccagc
26520

ctggggaaca agagcaaaaa ctctgtctaa aaaaaaaaaa aaaaaaaaag ggccaggcat
26580

ggtggctcat gcctgtaatc tcagcacttc gagaggctga ggtgggcggg cggatcatga
26640

ggtcaagcga ttgagatcat cctggccaac atgatgaaac ccagtctcta ctaaaaatac
26700

aaaaattagc tgggtgtggt ggcgggcgcc tgtagtccca gctacttggg aggctgaggc
26760

aggagaatcg cttgaatctg gggggtagag gttgcagtga gccaagatca tgccactgca
26820

ctccagcctg gcgacagagc gagactccat ctcaaaaaaa aatatatata tgtgtgtgtg
26880

tgtgtgtgtg tgtgtgtgtg tgtgtgtgtg tgtgtaaaca ataaatacat aaattgagga
26940

gaaggcaggg gaaccaggat ttgacagtga caagaaagaa aagcccagga ggacctggtg
27000

gctgagccag atgctggggc tggactaagg gtgatgggag cagagctggg acctttgggt
27060

ggcagagaag ttctaatcag tgaaactggg caaagctatt tggatggagc agtagagttg
27120

gggttaagag acagagacag gccgggcctg gtggctcaca cctgtaatcc caacagtttg
27180

ggaggccaat gtgggaggat cattgagacc aggagtttga gaccagcctg ggtaacatac
27240

tgaggccttg tctctacaaa aaatttaaaa aattagccag gtgtggtggt gcgtgcctgt
27300

ggtcctagct actcaggagg ctgaggtggg aggatcgctc gagctcagta gactgaggct
27360

gcagtgagac tagatcgtgc cactgtactc cagcctgggc cacagagtga gaccctgtct
27420

caaaacaaaa caaaacaaac aaacaaacaa caaaaaaaag aggcagagag acaaaggcgg
27480

cgaagaagat ggaatgatga tgcctttcgg gtaggtttgg tgttttgggt tgtaaaaggg
27540

aggataaaga agtaggagaa agtagaaaga acactaattg ggactcaggt aatggcttca
27600

ggtagattgt acaggtttga atgcggggag acaaagccca cgaggagaca agaggaaaca
27660

aacttggagg aaagtttctg tgattctaaa cacgttccct ctcttatctc catgatttca
27720

gctagtggtg ggatgctctg tgtatgtttc atgcagtttc acatagttgg gggctcagtc

27780

tctccacata aggcatttta tttcttttct cttttttttc tttttttgag acggagtttc
27840

attcttgttg cccaggctgg tgcgcaatgg ggcaatctca gctcactgca acctccgcct
27900

cccaggttca agtgattctc ctgctcagcc tcccaagtag ctgggattac aggtgcctgc
27960

caccacaccc agctaatttt tatattttta atagagacag ggtttcgcca tgttggccag
28020

gctggtctcg aactcctgac ctcggtgatc cacccgcctc agcctcccta aagtgctggg
28080

attacaggtc tgcgccactg cgcctggcct ctccacgtaa ggctttgagt ggcgtactgg
28140

gaacgccccg ctggtggtgt ccttgggtgg actgcagggc cctctaagtg ccccttgagg
28200

ctgaccactt cggcttccag catgtctgag atgagatgtg acactctctc ctgggagcca
28260

cctgcaaggt gattatgtat tggaacaagc tctcttcttg ccttatttta cagcccaatg
28320

gagaggaagt aggggtcaga gtagtagggg tgtaaaattt ggagtcactc ttgttgggcg
28380

tgtggtttgt gcaagtgacc cgacctctct aagtcttgac tccctcatct gtaaaatagg
28440

gatggttttg gcaaatagct acccacttag ctactctgag gacggtatga cattgtggag
28500

gtggcaatgc tttgtaaact gcagaggact tttaggtgcc agccatcgcc atgttttaaa
28560

tgcggagaac tgcatttctc accacctcgc aaaataaaac ttcacagttt atttgggttg
28620

ggtataatta ccgggcgaaa aatgtaaaaa taacaatgtt ttagaaattt ccccaaagaa
28680

gaaacagtat tggctggggc tgccttccca ggggccctgg aatcctgagg gtgcctgacc
28740

tgcaaatgcc cagcgcttag caggagaaca gccagatggc cctgctagtt ccctctctgt
28800

gcaaacatct ctcacccagc cgagtcaaca accctccctg ggtcaagagg ctcctccatg
28860

ctgcttctgt gtggtgtagc ctagaaactg ggcggctggg gcacagggct ctggactttg
28920

gttatgtgtt taatctaggt ccctgagcta cagggtcatc tgttttggct ccttctctgc
28980

ttatgtaaac agggctggtg ggcccatcag tacagccagg ccccgctctc accccctcct
29040

cctgtaaaca cattacaaag aatctttgta actgcccagg aagagcagct ctgctcaggg

29100

gaagagagcc tgcatccgga gggggtgaga ctcggggtgg agatggatgg gggaagagta
29160

ggcaggaggg gcaaaggtgg ggcaggagtg gtgcttgcca atacctcctg tactatgtgg
29220

tacagaaaag ccagctttgc tcaaagtctg gccgtgatag gagggggcct gttgtctgca
29280

gcagaaacca aggtggtagg aagggtggtg ttagaaaagg ggagaactgg ggtggggcat
29340

ggtggctcac acctgtaatc ccagcaattt gtgaggctga ggcgggagga ttgcttgagg
29400

ccaggagttt gagatcagcc tgagcaacat gggaagattc tgtgtctaca aaaaatacaa
29460

aaattagctg agcgtggtgg catgtgccta tagtcctagc tacttgggag accgaggcag
29520

gaggatcgct tgaggccaag agttggaggc tataatgagg tacgatcaca ccactgcact
29580

ccatcctggg tgtcagagcg agatcctgtc tctaaaaaaa gaaagaaaga aaaggggaga
29640

atctttattt cctaacatcc tgcagccctg acacttttct taccttagca attcaaacat
29700

ggttaaaaaa aaaaaaaagg agggggtgcc aagtgcggta cttcacacct gcaatcccag
29760

cactttggga tgctgaggcg ggtggatcac ctgaggtcag gagtttgaga caagcctgac
29820

ctacatggtg aaaccctgtc tctactaaaa aaatacaaaa aattaaccgg gcctggtggt
29880

gggcacctgt aatcccagct actctggagg ctgaggcggg agaatcgctt gagctgggag
29940

gcggaggttg cagtgagtcg agatggtgcc attgcactcc agtctgggca ataagagaga
30000

aactctttct caaaaaaaaa aaaaaaaaaa agaactaggc caggtgtggt ggctcacgcc
30060

tgtaatccca gcactttggg aggctgaggt aggtggatca cccgaggtca ggcgagacca
30120

gcctggccaa catggtgaga ccttgtctct gctaaaaata caaaaattag ccaggcatga
30180

tggtatgcgc ctgtaatccc agctacttgg gaggctgagg caggagaatt gcttgaaccc
30240

tggaggtgga ggttgcagtg aggtgacagg gcgccattgc actctagcct gtgcgacaag
30300

agcaaaactc catctcaaaa taaataaata aatacataca tacatacata catacataaa
30360

taaatacaat aaaaagaact aaagcctcct tttcaagtag tacaggttag aagcaagaat

30420

attagctgga gagtcaagaa aacaaattgt attcctgctc ctacccctaa tgagctgtgt
30480

gccctttgga tgatgcctta acatcttgga gtttccttgt ctagataaat cagaatgata
30540

atatttgcca ttttttgaac acccttgata tgacagggat ttttacctgc tattttatta
30600

atctacacaa atacccaatg aggtagggtg ggttatcccc attttagaga taaagacatt
30660

aagacttagc taggagaact gacttgctta aaaatatgta tgtgtaagta gcaggaacag
30720

ctcttttgta ttccaaaccc ccattctgcc cctgcaacta agaaaggtgg gggcggggtg
30780

gtggctcaaa cctgtaatcc cagcattttg ggaggccgag gcaggaggat cacttgagcc
30840

aggagttcaa gaccagcctg ggcaacgaag caagaccctg gttttttttt tttttttttt
30900

tttgagacaa agttttgctt ttgttgctca ggctatagtg caatcgcata atcttggctc
30960

actgcagcct ctacctcctg ggttcaagtg attctcttgc ctcagcctcc taagtagctg
31020

ggattgcagg catgtaccac cacgcctggc taatttttgt atttttagta gagatggggt
31080

ttcaccatgt tggtcaggct ggtcttgaac tcctgacctc aggtgatcca cccgcctcag
31140

ccacccaaag tgctgggatt acaggcatga gccactgcgc ccagccagca agatcctgtt
31200

tctacaaaat ataaaaaatt agccaggtgt ggtggcatgc acctgtagtc ccagctactt
31260

gggagactga ggcaggagga tcacttgaac ccaaaggcaa aaaaaaaaaa aaaaaaaaaa
31320

aagaagaaaa agaaaaaaaa aactattttt attgcttagg gacaaagttt ctgacttcca
31380

taaactcaag atcagctggc acggtggctc atgcctgtaa tcccagcact ttggaaggcc
31440

gaggcgggcg gatcacttgg ggtcaagagt ttgataccag cctaaccaac atagtaaaac
31500

cctgtctcta ctaaaaatac aaaaattagc caggggtgat ggtggtcgcc tgtaatccca
31560

gctacttgtg aggctgaggc aggagaatcg cttgaaccgg ggaggcagag gttgtagtga
31620

gccaagattg caacactgta ctccagcctg ggcaacagag tgagactctg tctcaaaaaa
31680

aaaaaaaaaa aaaaaaaaaa gctcaaggtc tggagtcaaa gcctctacct ctcccagata

31740

tctcctccca ctgtatttat caccttggtt aaaatccagg gaagggaaca atcagcaact
31800

tgttccactc ggggtagaac ttggcaggaa ctctcgaagt tgctgttact acctttccat
31860

tgcaaggcaa ttcataaatg tctaaatttc catagtacat cttggaggct gcatgaggga
31920

gaaagagcca gcaaccctta tgcctacaga caaaaattag tttttttttt tttttgcta
31980

tggggtctca ctctgtcacc cagggttgag ggcagtggca cgatcttggc ttactgtaac
32040

ctctgcttcc tgggttcaag cgatcctccc acctcctaca ggtgtgagcc actgtgccct
32100

gcctatctgg ccctttatag aaaaatgttt gccatctcct gttctagata aaagtagcca
32160

tctgagcagg gatgagggag ggtctcacca ttcgggaggt aaatatctat ttaatcctct
32220

tgttttctct gtagcgtcca caccctcaac tgtgcccggt attcctcagg ccagcggggt
32280

ctgattgctg gcctgagggg tgtgggatct cagacccacc atggatgggc agccctaaac
32340

ttccttctcc caccttcaat ggttaagtct ttacttctgt tggtatagag tggtggctgg
32400

gcttggactt ttcaggaagg agactggaag gtgttcctct ggagaaactg acccagggct
32460

aagagctaca gatactggcc gggcatggtg gctcacgcct gacatcccag gactttggga
32520

ggccgaggca ggtagatcac ctgaggtcag gagttcgagg tcagcctggc catcatggtg
32580

aaacgccgtt tctaataaaa atagaaaact tagccgagtg tggcggcagg cgcctgtaat
32640

cccagctcct cgggaggctg agacaggaga atcgcttgaa tctgggaggc aaaggttgca
32700

gtgagccgag atcgtgccac tgcactccag ccgggtaaca gagtgagact ctgtctcaaa
32760

aaaaaaaaaa aaggtcactt tataccacac tttgctcttg ttaatcagac tctgcaaata
32820

gcaagtgact gaaccctgcg attcagttac aacaaagaca tttgaatgga cagagaagat
32880

gtttgaggaa agctcctacc tttttttttt cttttttttt gactgagtct tcctccatca
32940

cccagactgg agtgcagtga tgtgatcacg ctcactgcag cctcaaactc ctgggctcag
33000

gcaatcctcc catctcagcc tcctgagtag ctgggactac aggcgtgtgc cactataccc

33060

agggaatttt tgtatatttt cttggtaagg acgaggtttt gccatgttgc ccagggtggt
33120

ctggaactcc cgggctcaag ccatcctcct gcactggtct accaaagtgc taagactaca
33180

ggcgtgagcc accacacctg gccagtgcta acctctttaa tgaggattgt tattgctttt
33240

tcttgaagtt ttgattgcaa atatatatag ttttcagtag tttgcccaac tctattaatt
33300

ttggtcttgt tttgcgtgcc agacaggggc tttcaaacag aacaatacag ttcataagga
33360

acacaggttt aataattgct cagaggaaaa aagacctacc tggtaccctc tccacaaaga
33420

caaatccaaa gacacaaaag aaagggaggg ccgggcatgg tggctcatac ctgtaatccc
33480

agcactttgg gaggccaagg cttgagccca ggaggtcgag gctgctgtca gccgagacca
33540

tcacgccact gcactccatt ctgggcaaaa cagccagacc ctgtctcaaa aaaaaaaaaa
33600

agtgatttac aaagtatatt tattttttaa aagatacatt atcatgcttg ctttcactca
33660

gataaaaatg ctacaagata ttttatgggt atagtaagag acagtatgat gccctattca
33720

aattttgtga aaattgaata ggttgctatc atttctacat acatctttct taacactttc
33780

tggaatttac tcctcttcct ctttccacat tgcttactgt acagagaagg aggctccata
33840

atgggaaggg atactatcac atttttaatg attattaaca gtaaaaacaa tgataaacct
33900

ccgacgtatt aatactacct gccacacaca ctgtggtgga tgctttctac aaattaacct
33960

aatccattcc tccctattac aagatattat tgctatgccc ctagaaacaa gggatttgaa
34020

agtcagaaag gttaaataaa ttggccaata agtggcagtc agttctcctg ctatactatt
34080

tctagaaaac aaacctttat tttttaaggg gcttggctat tttgagcaga agtgatctgt
34140

ggtcacacag atcttaatca ccactccccg acagagaata aacatttatc aggatggggc
34200

cggggaaggt ggctcacgcc tgtaatccca gcaatttggg aggccgaagt gggaggatca
34260

ctgaggtcag gagttcgaga ccagcctggc caacatagcg aaaccctgtc tctattaaaa
34320

atacaaaaat tagctgggct tggtggcagg cgcctgtaat tctagctact ggggaggctg

34380

aggcaggaga atcgcttgaa cccgggagac ggaggttgca gtgagccgag atcgtgccac
34440

tgcactccag cctgggcaac agagcaagac cctgtccccc atcccaaaaa accaaaaaca
34500

accatttatc tggatggtgt cgaaatattt accacttccc ctgcacttct gacttttcga
34560

actttgtgct tttgcctgag ttccatctgg aatgttcttc ttggatggtg gagcatgtcg
34620

tccacctaac caattcctac ctctcctttg aatacacacc acttagtgga agtcttccct
34680

gatccctgcc gacaagcttt tcaactgcat ctctctctta agctaaccca agtcagtacc
34740

ggcacccttg ctggaatgtc ccattttggg aaaggcagta tttcttgtat gaatcttcat
34800

ttgctctcta ctgggggtaa gtgcacgtta gagaataatg aaaacttcaa gtcaagggat
34860

gcagagtgcg tttaccagtt caacaacctg gactttctac caggccattt tcaaactgag
34920

ctgccacgga cagcagcatg gactttggtg ctagcggggt cggcctgaaa tctgcagcct
34980

gcatgctcca aggatgcctg aaaagaaggg tcgaaccatg ggccgctccc aggacccacc
35040

cttttagtct tcccagctgg ctcaggaaac aaaggggggc cggagagagg cggcgcccag
35100

ggcgcaacag ccggagactg gcgctgcgtg cctggggaca gaggcctcgt gctaggagcc
35160

ccaagcaact acacacctag ccgctggcaa gaacaaacag cagtgacagc aacaaacaga
35220

accgacagcc gcagaccgcc gtcgctgcgg cccacaaagg ccggaagtgt ggcgggtgcc
35280

ggcggccgag gcgccctctc taggacttcg gagggcccgc atctcggtgg ttccctatac
35340

tggaagaccc ccgattccca gggcgtgtac cctacgacgg gggcctcaaa atgagtcaaa
35400

agggagaaaa gcctgggcgc agtgactcgt acctgtaatc ccagtccttt gggaggccga
35460

ggtgggagga tcgcttgagg ccaggagttc gagactggcc tgggcaacag agagagacct
35520

ggtctctacc aagaattaat aaaatgagcc gggtgtggtg gggcacacct gtagccccag
35580

ctattcggga ggcttaagtg gaaggatcac ttgagccctg gagtcagagg ctgcagtgag
35640

ctttgcttgc accattgcac tccagcctgg gaaacacagt gagaccccat ctcaaaaagg

35700

aaaaaaaagg aaagaagaga gggaaggaga gagggaggaa ggacagaggg agggaggagg
35760

gagagaggga aggaaggaag agaggaagga agaaaggaag gaaggaaaga aggaaggaag
35820

gaaggaagaa ggaatcgaat tcagcggctc acctgtggaa gaggaaatcc atttacaatg
35880

catccaggcc tgagtttcag ctgaggggtt ggagcatggc tgattatcat cctggcttcc
35940

cccagagacc aaggaaactg aggcctggag caagttcttg cttgctcagg gtctcacagt
36000

gatccgggtc agaagccagg acttgtggga ccacgatttg tgccctttgc actgcctgga
36060

ctgcctctca tcataaatga atacggataa aattcgttat gctttcaaag tggtaggaaa
36120

cacatgacag atcaaagcag acaaaatagt gtgcaagact tttgagaaga cgcctcacac
36180

tcgtcgggat ggctacagtt taaatatccc ttatctgaaa tgcttgggac cggaaatgtt
36240

tcagatttca gaattttttt ttgactttgg aatatttgct ttactggttg agcagcccta
36300

atcccaaaat cctaagtctg aaatgctcta atgagcattt tctctaagtg ccatgatggc
36360

cctcaagttc tggttttgta acttttggag ctcagatatt tggattaggg atactctccc
36420

tgtactaaaa acaaacaaac aaacaaacac aaaaaaccac caccaccacc aacaaaacaa
36480

aacaaaatag caggtgtttg gtgaggatgt ggacaaattg gaatctttgt gcattgctgg
36540

tgggaacata acatggtgca gcccccgtga gaaatagtac agcgcttctt caataaaatt
36600

aaaaatagaa ttggctggtg tggtggctcc cgcctgtaat cccagcactt tgggaggtga
36660

aggcaggcgg atcatttgag atcaggagtt ccagaccagc ctgaccaaca tggcaaaacc
36720

ctgtctctac taaaaataca aaaaaaaatc agctgggcgt ggtggctcac gcctgtaatc
36780

ccagctactc gggaggctga ggcaggacag ttgcttgaac ctgtgagggg gaggttgcag
36840

tgagcaaaga tggtgccact gcactccagc ctgggcgaca gagctagatt ctgtcaaaat
36900

gaaatgaaat gaaatgaaat aaaataaaat aaaataatct ctgaaggcta aagagagaaa
36960

ttgggtcagc tgaagagttt acagatttgc agagaataga ggcaaggaag ctttgtgaac

37020

tacttttagg ttgtgaaact tggtcttgat tcagccccca catggtcttc atatacaaag
37080

cttctttgca gaggaagctc tttggagaaa gataactggt gagatagaaa gcacagccaa
37140

gagataacaa gggagacttc tctggagaat tttgtggatg aagaggaaaa acaaatccat
37200

aaggtcatgg ggataatgag ttatttgaaa tcagaaagag tgattaacat ggaggcgggg
37260

cgcagtggct catgcctgta atcccagcgc tttgggaggc tgaggcaggc ggattgcctg
37320

aggtcagaag ttcgagacca gtctggccaa catggtgaaa ccccatctct actaaaaata
37380

caaaaaaatt agccgggcgt ggtggtgtgc acctgtaatc tcagctactc gggaggctga
37440

gacaggggaa ttgcttgaac cagggaggtg gaggttgcag tgagccgaga tttcgccact
37500

acactccagc ctgggcaaca gggcgagact ccatctcaaa aaaaaaaaaa aaaaaaaaaa
37560

aaaagcaaac caaaccaaag cacacaaaca aataaacaaa caaaaaacat aaggcaattc
37620

tctacaggtg ttttactgct agaagatcat gctcctcatt tcctaaagac tcacagagaa
37680

cagaagactg gttgcatgag aagtgaataa caagaggtta tagtgcttat ttgggttggg
37740

ggcaaacatg acatttcaat attaacttgt ttatttattt atttttcggt tatacatcat
37800

tgctggtata tgtactttat tttatttttt agagatgggg ggtctcgcta tgttgaccag
37860

gctggtctcg aactcctggc atcaagccat ccacccatct tggcctccca aagtgttggt
37920

attacaggtg tgagccgctg tgcccaggct aactcgtttt ttttctttgg tccagcaagg
37980

cttgtgaatg tgtctgggaa cgacgtaaaa tctggcttga aaaaaaaact ctcaactcaa
38040

agctccaaga cagagctgag atgcttttga aggagacagc ctcattggtc tccttgtcgg
38100

agaggcccca acttatcagt aaatattggt taacatctat catctggtgg gactaagcat
38160

ggaaccaatt gcccttgcac agaaggccac gtattgtagt attcaacata gatggaatga
38220

ccaggaaagg caaaaccaca gatataaaaa gtagattaag gccaggcgcg gtggctcacc
38280

cctgtaatcc tagcacgttg ggaggccaag atgggcggat cacccaccag cctggtctat

38340

atggtgaaac cccgtctcta ctaaaaatac aaaaattagc cgggcatgtt ggtgcgcgcc
38400

tgtaatccca gctactcggc agactgaggc aggagaatcg cttgaaccca ggaggcggga
38460

ggcggaggtt acagtgagcc gagatggcac cactgcactc cagcctggcg acggagcaag
38520

aaaaaaaaaa taaataaaaa attgctaaga ttgttattaa atccgctggg agagtctttc
38580

atcatgttag caggatcatt gtgtcctctg gtggcaataa cacgcgactg caactgactt
38640

gcctgtgcag aaaaatcttt cctgaatcct gaaagctgca ttctgttcta ttagcttaag
38700

aaggaaatct gacactgatc cgcacggtgt gttttatgat cctgcacata aagattaggt
38760

gctcaaggat gcagaggagc ccactgctcc cagagcaggt gcctgagcct aggaggcatc
38820

tgaccagagt ctctgtcgcc caagctggag tgcagtggtg cgatctcggc tcactgcaac
38880

ctctgcctcc agggttcaag cgattctcct gtctcagcct cccaagtagt tgggattata
38940

ggcacgcacc accatgcatg cctgtaattt tttgtatttt tagtagagac ggggtttcac
39000

catgttggcc agactggtct cagacacttg acctcaggtg atctgcccgc ctcgaccccc
39060

agagggctga tattacagcg tgagccaccg tgcctggccg aacgacagga tttcaagcct
39120

gaactcgaga ggtagcccag actggggatc tgcactggga agtcatccgc aaggacgtgg
39180

tggctggagc tatagacaaa gggaaagtta tccagggaga gtgagggtgt ggaatgggga
39240

gagagagggc ccgcaacaca gtcctaagga acaccagcac tttttttttt tttttttttt
39300

tttgagacgg agtcacccag gctggagtgc agtggcatga tctcggctct ttgcaacctc
39360

cacctcccag gttcaagcaa ttctcctgcc tcagcctctc aagtagctgg gattacagac
39420

gcccaccacc atgcccagaa tttttgtagt tttagtagac acgggtttca ccatgttggc
39480

ctggctggtc ttgaactcct gacctcaagt gatccacctg cctaggcctc ccaaaatgct
39540

gggattacag gcatgagcca ccgtgtctgg cccagttatc gctttctgag agaggagttt
39600

ctccaaagtt ttcagtttga cataatcaat ataaccatcc tgcgtattag ggatggctg

39660

gttcttcact ccttcagcat ctttggttaa ccggtacctc gacttggcca tatccacagg
39720

tgggggaggt caggagatgt cacccctagt cactggggct gtggggaaca tggtctctga
39780

aatggaacaa ggctgaccag gccgatggat ggctcacagt gtctgctgga ggttcctttt
39840

gctgctcagg gccagggcgt ggctggagga agaggcccca cccaggaagt tccgcagggg
39900

cttacacagt cttcccagac ccagaagcac aatctccaaa agagtgaaca agagacggaa
39960

cccactgacc ccaaacatgg cttttagaaa gatgatcttc tccgaggggt gggaaaaggt
40020

gcaagttaat ctatacgggc aaggctcttg gccacatgca aaggagctgg gcatctggaa
40080

cccatacagg tggtactgca accccggcgc tgtcccctcc aggaccagct gagctcccag
40140

ctgagccaca taagctcaga gcagcctgag gcttccagcc cctggggtat ctctgctgct
40200

ctcccctccc tggatcaggg tgtcctcttc ctccgtcccc tttcatgatt cttcccagtg
40260

ccagatcacg tgatacagag tgaaacccat gtagaggacg ctaggtacag ccaccaagat
40320

gacctggaag acccagaaac gcagcgggga gagcgggtgg aaggcatcga agcaggcagc
40380

cttgcagccc ggctgctggg tgtgacacac gaattcactc tgctcatcgc catagactcc
40440

aggcccactg gcagccagca gcacaaggcg gaatcccagg agcacgggaa acaggaggcg
40500

ccccacgggg gtggagtgcc agctctcctc cgccagcagc caccacctca ggaacctgcc
40560

acacatcctg ttagggagtt ttaaaaaaag attattacac gttattgatt ttttgttttt
40620

aaagacctct gttgcccagg ctggagtaca atggtgtgat catagctcac tgcagcttcc
40680

aactgttggg ctcaagcaat cgtcctgcct cggcttcctg agtagctggg actacaggtg
40740

catgacacca cgcccagcta agttttaatg tttttataga tatggggtct tgctatgttt
40800

cccagtctgg tctcaaactc ctgacttcaa gcaatcctcc tgcctcggcc tcccaaagtg
40860

ctgggattac aggtgtgaac cattgcacca ggttatgagt gatttatttt ttattttttg
40920

agacagagtc ttgctctgtc gcctagggtg tagtgtagtg tcgcgatctc tgctcactgc

40980

aacctctgcc tcccggcttc aagagatttt cctgcctcag ccttctgagt agctgggatt
41040

ataggcgtgc accaccacag ctggctaatt ttgtgtattt ttagtagaga tggggtttca
41100

ccatgttggc caggctggtc ttgaactcct gacctcaggt gatccacctg ccttggcctc
41160

ccaaagtgtt ggaattacag gcgtgagcca ctgtgccggg ccatgtattg atttatgact
41220

ttgcctgtaa cttctgcttc tctgcctcta aaaaccctta cctacaagcc attggggaat
41280

ttaggtctta cacttgagct ggctgattct ccttgcttgg caccctgcaa taaatgcctc
41340

agtttctctc actggaatcc acatgtcagt gtttgcagca cccggtggtt ggacccaagt
41400

tcgatgaggt aacagacgtc cactgggtgt gctgtaatcc cgcggaggtt ttcctccata
41460

ataaaaggca ggaattggct gggcgccgtg gttcatgcct gtaatcccag cactttggga
41520

ggtcgaggcg agtggatcat ctgaggtcag gagttggaga ccagcctggc caacatgatg
41580

aaaccccgtc tctactaaaa atacaaaaaa ttagctgggt gtggtggcgg gcgcctgtaa
41640

tctcagctac atgggaggct gaagcaggag aatcgcttga acccaggagt tggaagttgc
41700

agtgagccga gatcgcgcca ctgcactcca gcctgggcaa caagagtgaa attccctctt
41760

aaaaaaaaaa aagacggcga gaattgtgtg agttgtgcga ggaagaccgg atgaccgtta
41820

tctcatggcc tttgcatcca ggctttgagc caaattctca aggactggat cctaacactt
41880

ggctgcaact atttccttga ccactagggg gcaggcaaaa gccatagcga atccggccta
41940

gcgctgattg gaacctgctt gagaaaaaga cctctttgtt taagccggcg gtccccaatc
42000

tctttggcac tagggaccgg ctccgtagaa gacagttttt ccacggactg ggaggggaa
42060

tggttttggg atgattccag tgcatcccgt ttattgtgca ctttatttct attattatta
42120

catcgtaata tataatgaaa taattctaca actcgccacg gtgtagaatc agtgggagcc
42180

ctgagcttgt tttcttgcaa ctagatggtc ccatctgggg ttgatgggag acagtgacag
42240

atcatcaggc gttagatctc ataaggagcg ggcagcctac atacctttct tgcacagttc

42300

acaatagggt tcgtgcttct gtgagagtct aatgctgctg ccgatctgac aggaggtgag
42360

ctcaggcggt aattggagca atggtcgggg ggtgggggag cagctgtaaa tacagatgaa
42420

gcttcgaccc tgcagctcac cttctgctgc gtggttcggc tcctaactgg ctatgaacgg
42480

gtaccgggag ttggggaccc ctgccttaga ctatcttgtc tctctctggt ggactctcct
42540

ggggatagtt cccaggtagg ctggaggaat gatgcacact gaatcaggca aaagacatca
42600

atcagatata gcaaggtata catatgttaa ggtcctcttc aatttctctg agctttgctt
42660

tttcaaactt ctttcccagt agaattcact gtcccagcca aaaggttctt cgtggtcgtc
42720

agaaatattt agtgctttct attgcctttg cttacgtatt tattgattca tccagaaatt
42780

atgtattaat tacctattgt attaatatgt gtatttattt atttattttt gagacacggt
42840

cttgctctgt tgctcaggct ggagtgcaat agtacaatca tagctcacag cagccttgac
42900

ctcccatgct caagtgatct tcctgcctca gcctcccaag tagctgggac tacaagtgtg
42960

tgccaccatg cccagataat tttttatttt tttaaatgta gagacagggt cttgctatgt
43020

tgcccagact gatctggaac tcctggctca agcaatcctc ccactttggc atcccaaaga
43080

gctgagatta caggcatgag ccactgtgcc cggacctatt gtatatttga ttctctggta
43140

ggcactggag actcaaaaga acagggaatg taaaatccca ttctgcatgg aaatcttttg
43200

acctcgactt ggcaagctgt cttaggcctc cacatctctc tgaaatccga attgctccag
43260

gaagttttcc ctgagtatct cagtagccct gtgtccctct cctcctgccc cacacctgcc
43320

caggacttga atcctgtggc ttctctctcc tcctttatca tcatccatgt ctcctgttat
43380

agatgaggtc acatctccta ttccaatttt ccttagccct ggtctgatca tgtggatttt
43440

ggtttcttcg ttgatcttaa atgtttaact ttcacctttt atttgctcca agtgtcctca
43500

tattcacatt tcacatgcta tattttttcc tcttgattat ttgcattttc tttttttgag
43560

acagagtctt gcactgtcgc ccaggctgga gtgcagtggc gagatctcag ctcactgcaa

43620

cccctgcctc ctgggttcac gtgatcctcc tgcctcagcc tcccccgtag ctgggattac
43680

aggcacacat caccaaaccc agctattctc ctgcctcagc ctcccaagta gctgaaatta
43740

taggcacaca ccaccacacc cggctaattt tttgtgtttt cagtgaagac ggggtttcac
43800

tatgttggcc aggctggtct tgatccttga cctcgtgatc cgcccgcctg ggcctcccaa
43860

agtgctgaga ttataggcgt gagccaccgc gcctggcgac tatttgcatt ttctttcctg
43920

aacttggcta ctgttcatat aggatgtttt tgccctggaa gggagaaagg tggatttcat
43980

gtagacttta aaaaaaaaag taatttctca gctttgattt tcaaattgga gtcgaagagt
44040

taaaacaaaa aattgtagcc ttcaaattta ggctttcatg aaagataaga tgattaactc
44100

agaattccaa agactgggaa tgattcttct atggaaccca gcttggagga tggattttgg
44160

aataatccag agtgggaggc tgagtatagg atgtaaagaa agttcttgtc gctagacaca
44220

gtggctcacg cctgtaatgc cagcattttg ggaggccgag gcaggcagat cacctgaggt
44280

caggagttcg agacgagcct tgccaacatg gtgaaaccct gtctctacta aaaatagaaa
44340

aattagccgg gcgtagtggc tgatgcctgt aatcccagct acttgggagg ctgaggcagg
44400

agaatcgttg gaacccggga ggcggagttt gcagtaagcc aagattgcaa cactgcaatc
44460

caggctgggc aacagagcaa gactccatct caaaaaaaaa aaaaaaaaaa aaaaaaagaa
44520

aaaggaagaa aaaaaggaaa gttcttttcc cacaggaagg aaaagcacag acacaggcag
44580

gtgaggcttc atgagaaggt caaggtgaga gctctgctgt gtgtttgggg ttgatacctg
44640

tgcatggcag ttggcgactc atttgaggaa actcactctc agggactcag agggagtctg
44700

agaacctcag ggccaattcc cgcttcattg agctgtaccc tggagagctg agagctctca
44760

aataagcttt ggctggtggg ccaaggcgaa catggtgtgg agatgtggag cagctggagt
44820

ggagggctgg gtctcccagg aagccaagaa gtgtggggaa actgaggcac agaagaggct
44880

tggctgtgtg aaagagcttg caggttctta gcacctcaca gccagagtgg ccatggccac

44940

gcctgtggat gtcagcaggg gacacctgaa caatccctaa gggacagcgg gtccgggcca
45000

tgcttgggat tgcccaagtc agggtgccca ggaaccatgg cctccaaggg aaaccaagag
45060

tttcagatga attcagtgct tgggagcagg accaatgaga aaatcacatc acatttctat
45120

agcattttac agttaatact gttctctgtc gtgcttgaca aagagagatg attatttatt
45180

agtttccaca gattagacaa tggcgggggg tggttcaagg tgagatggtt tttgggtccg
45240

agtcagctca ggacaggcat cccagtcttc ggtctccaaa tccacctcct gtctgtcccc
45300

ccacactgct ccttgggcct tgaggatcca ttgaccgtga tttcggtggt tcagctccca
45360

catcaggcag gaagggcagc tactggggct gagatcccac attgcctcca gcccttgctt
45420

cctacctggc ctcccccggc accacgaggg gctgggccag gctgctgtgc tgcacgtggc
45480

aggagtaggg ggctgtgtcc tgcgggggca catgcaccaa caaccaggtc aggtaagtgc
45540

cgtttccacc gtgaagaaca tctccccgta actcaggctc ctgcacctcg ccggcccgag
45600

tccagtgcac atcaattttc cctgggtaga agtcgtaggc caggcacttc agtttcttct
45660

tttctcctgg ggcctggtgg ctggtgacca ccacagaggg aggatctgtg gagggataga
45720

gtgtgacact gcaggtttga ggtcgacttc tcccggccca ggtctcttct cacccctacc
45780

cctggatgtc agcaatcagc agagctctaa gctctgtatc tgcccgcccc actgccccct
45840

gccctgggac actttccaga agatcaggga aggctgaggc tttccctgat ccttctctgt
45900

tggatcatgg aatgtcctgg ctcccctctc gcccattctc caccccaccc atgtattgac
45960

tctgataagt tgtgaatgct ctgtgcactt tcccatgttt tcccacatct gagtgggctt
46020

tggagttctg tcccccaatc tcagtccagc tgcttggttc agcccatgcc tctatccagc
46080

aaccgcttcc ctgcagcact gcacaatcct gagtctcact atgtctgcaa tccaagtctc
46140

agcttgcagc catcgattcc attcacactg gctcgctgtt gtctttctgg ggctgacatt
46200

ttacacacct cccttctctc caccacccag atgggaggca tcattgtaga gagaaacaac

46260

gatggcattt ggaatctgaa atctggaatt caagtcctag ctcttccttt tttttttct
46320

tttttttttt tttgagactg agtctcacac tattgcccag gctaaagtgc agtggcgcga
46380

tcttggctca ttgcaatcta gtcctcctgg gttcaagcaa ttttcatgcc tcagcctccc
46440

gagtagctgg gattacaggc gcctgccacc acgccaggct aatttctgta tttttggtag
46500

agacgaggtt tcatcatgtt ggccaggctg gtctcgaact cctaacctca agtgatccac
46560

ccaccttgga ctcactccca aagtgttggg attacaggcg tgagccagca tgcctggctg
46620

cagctctgcc tcttacacac agtgttaccc tcacctctgt cacctccagc tcctcatctc
46680

tcaatgggtg atatttaggg aagcctacca gtgaatcagt ggctttcctg gcccattctg
46740

cctgaaattt tgatgtcctc ctaggaaagg aatgaatcct ggaagttgaa aactggggtt
46800

ctacgtgact tacatggata gaggagattg gcctaagatg ctctctgagc tcccagcctg
46860

agatcacctt ggtttttccc agcttagcac tggatagcag gaagcagtga gtaccttgcc
46920

ggtccaggat attttcgctg tatttcaggt atttccgcag agttgcaggg cactcctcct
46980

ccaggtaagc cttggcccgc tgcacgtaga ctggttctgc ctcctacttc tgcttggtgt
47040

tctgggcttc cgggaccaag gggactcagg ctgggatttc tttgttgaat tcaatgtagt
47100

cctttccatc ataggcattc ttccagaatg ctccagtgct tctgttattc tggatctcac
47160

aaccaaacct ttcctgcaag gcatgagacc ctgaaacctc ccctgacccc acaggaagcc
47220

agtcagccta gtgccaactg aataactctt agtcctgcac tcttcacaac tcaataggag
47280

gaaaggcttt tgcaatctaa gcatgcagtt ccgtaatcat ggcatatgat tttatctgga
47340

cgtacagttg acacagagtg gcaaaggcta tatggattgg catttcgggc caatttgaga
47400

tgaaatatgc attcctccca gccagggaag ttaagagagc tacaggccgt gggcagattt
47460

ctgtctcttc tgtagcatct ctttttattt gtttgaactt acggggagta tttaggaacc
47520

tgtcacaacc acaccaaagt acatttccga atttgataga aattatttgt gattccatat

47580

gaggaaagga aggctctcct tttctctcag ttctaggtgg agttcatatc atcacctaaa
47640

tcaacctaat ctgtctgact atttctttgt ttcttttttt tgagacagag tcttactctg
47700

ctgcccaggc tggagtgcag tgtcatgatc ttggctcact gcaacctcca tctcccgggt
47760

tcaagcaatt ctcctgcctc agtctgctga gtagctggaa ttccaggtgc gcaacaccac
47820

acgaggctaa tttttgtata tttagtatag atggggtttc gccatgttgg ccaggctggt
47880

cttgaactcc tgacctcagg tgatctgact gccttggtgt cccaaagtgc tgggattaca
47940

gatgcgagcc actgcgcctg gcctgatcac ttctttcttc ctttctggtg tttgatatct
48000

ttatcccaaa tctctaaagt cccaaccttc ttgcccatta gcaacaactc tcagaaatgg
48060

acctcatagg gcagaggaca cagcctcttc attctgatta taaaaacagg tgtttaccta
48120

gcacaggttg gtgctgttgg catagactct cacagctctc aagctgtctg ggctcaagtc
48180

ccaggtctct tcccgcccca acctggtgtg ttgtgtgaac tggagccagt cacctccttg
48240

tgcctttatt tcctcacccc tgaaacagca gtgccaaaag cacggtttca gggggttatt
48300

ctgagcatta tatatgctca taaatgggcc tggcacaaag taagtgctca gtaaatgtca
48360

gctactctta ttttctaata ccctgtgttc atctcttcat cctccttcct cgtaattatc
48420

cactttgcca tttctattcc tgtccatcaa tgcttccccc agtgtctttt ctgatcacct
48480

gtgccccctc ctctcctatg gctaacacac agcctgggtc attttcctta ccaagacctc
48540

ccatccctca tccttcaaac gtctgcttat ctcttgcctc ctttgggaaa ccttcagatc
48600

ccatgtagca ccttgggcat ttttgtactt agaagcccat cttttgatac ccactccatg
48660

ataatttaac accagcagtt caccaggcct ggggttggct gccttggggc tggcagtgtg
48720

gtttgggaga aagaacgctg ggccgggggc agggggaccg tctctgcacg ggctctgcca
48780

ccagctcctc cgaccacagt gcaggtggcc cttcctgggt ctgcatctta tggtgaggag
48840

gaactttagg gctgcaggga tctctaacag cctgtggttt tgaatgcagc acttcctgag

48900

cccccagcat gggctacggt gtgtgctggg tgcttttctc tgttgattcc acaaatttca
48960

attgagccaa tattggctgt accctgtgct aggtgatgga gctgcaaggg agatcaggac
49020

agactgtgac atccttcata gaacagacat agttaaatat tacagacttg tttggagata
49080

ggtctcagtg ctccaatgtt tgcagatttc gaccagatat tttgggaaca tttcactgtc
49140

aacaggtagg actggcagag ccaccaggtg agcccaggtc tgagtgactt tgagagtggc
49200

tcattctcca ggcaacctcc ctggacaggg gtgagtctga tgccttctca tggggagatc
49260

agcctctgat gggtggggag tggaggtgag aggtcagagg tgcctagata ggaagtgact
49320

ctgccatcac ttcctgtgtg acctgagacc tgggatgagg atggggcaat cgatggggaa
49380

ggggacaaca ctgtgctgtg gggggggtgg gatgcggaca tgtctgtgtt tctgctgtgt
49440

ctcctgttcc tcccctggga ttgggactat ttccatcctg ctgacccctt gccactcaca
49500

cctggggggc tgcctcttgg gttagacctt ccacccccgc ggtctgttgt tcactgaccg
49560

ttaccgtcgt tgtaatactc catgatgttg ttcagggtct ccataaagat gtcctccctg
49620

gccttctgaa cttggctctg cttctcccag tcctctactc cttccgcatg tctccatggt
49680

cccaggggct cagccttcct gtcttcactg ttgtagtgga agaaggcatg gccattgagg
49740

aagacagtgc cctgcagcct gtgggtgcct ttgccagacc tggacagccc agtgtagaga
49800

taggtcagag agtaatgacc tgcaaaagaa aagactctga gggctggggt ccatgcaagg
49860

gtgtcccatt gtggggctgc agagggccag gaggggaggc ctggccactg gcctcttcct
49920

ccccagctct ctcctctgcc atcagcttca caccattgga gcctcactca aggaaacagg
49980

ctctactttc atgctgggga tgatatttca gagaccattc tgtttcttca ctacagagtt
50040

taatctgctt ggcacagaag aagttaacat ttctcagcct ccttatagat agattggaac
50100

catgtgactg agttttgtca aacaagaatg tgagtggaag tgactgatgt ctttcttctg
50160

gtccaaggct ctaccatctg gcttgcccct acggtctctt atccctttcg ttgactgtgg

50220

aggccacagg atgaaacaga agagtcccaa gatgcaaaca gcctggatcc ctgagtcacc
50280

ctgtggagga gaggaacctt gcccaatctg cattgaactt gacatgagaa ataaacattt
50340

ctagtctaac cggacaaata catatagtta gaaagtgtag gaaggtgaat ttactagaaa
50400

actcaagaag tgacccagaa tggatcagca tagggtaagg ctaggatacc tctcagaatt
50460

cctggcattg tatcttgcag tttggtacaa gatggaagct gagagagtgg gacatttcca
50520

caattagttc catagttaat ttagaatata atttacatga gtgttagtaa tgttggaagt
50580

catatggcag atttctaatt taagtactga agtcattctt gctcatgagt ttctgaatgt
50640

gattagcctt taaatcagac cttgagtaaa acagattacc ctctacagtg tggatgggcc
50700

tcatccaatc agtggaaggc atcaagagaa aaggctgagg tcccccagag aagaaagaat
50760

tctgcctcta gactaccctt ggactcagga ccgcaacacc aactctttgc ttggtctcta
50820

gcctgctggc ttgctttgct ggtttaggac ttttagccct cagaattgtg tgagccacaa
50880

gaaatattaa ataaaactcc tccctttctc tgttttgtat atacatacac gtatatatgt
50940

atattataca tatacacata tatttagtgt acatatgaat atacaatgta tattcatata
51000

tacatacgtg agtgtttata caatgtatat tagttttgtt tctctggaga accccgaata
51060

attcatattt gggtgcagaa tgggattaat attataaatg tgattattat aaaactaacg
51120

tgaaacacct attttgacaa tttgtaaatg tcatgaattt tattttattt tatttattta
51180

tttatttttt tgagacagag tctcgctctg ttgcccaggc tggagtgcag tggcgcgatc
51240

tctgctcact gcaagctcca tctctcgggt tcacaccatt ctcctgcctc agcatcccga
51300

gtagctgaga ctacaggccc ccgccattac gcccggctaa ttttttgtat atttagtaga
51360

gacggggttt caccgtgtta gccaggatgg tctcgatctc ctgacctcgt gatccaccca
51420

cctcggcctc ccaaagtgct gggattacag gcgtgagcca ccgcgcccgg catgaatttt
51480

aaactcacac aactgagaag ccattcactt ccatgtcttt atcctgcaca ttctcatgcc

51540

acaaacagcc tccttcccct ctcctataaa tgaatcttga tctccccaag ctcccactgt
51600

cttctcttcc ggccactcgc tttccagacc cactgcagtg gtgtctgagt gtggggtgag
51660

ggcacgggtt tccttctagc accatccctc cagccaacct gccttcccac tttccccttg
51720

ggggctgttc tctcttttcc tcagcgtcag gcaggtgtac ccagccatcg gaaggaggga
51780

aggaacagga gcagaagcag ctccaggcct gtggttcctg ctccgtcctg gctctccacc
51840

ccaagcaggt cgcttggtct ttggagtctt tgttttcact gtaaactaag ggttttaga
51900

ttcagattcc tgaaggtctt attttgggtg agggcagagg ctccctgaat gctcctggaa
51960

gtgtgtgagc tgagggagtt gacagccttg gacacctgtt cctgccatat gccagggaat
52020

cccagctgca tccagccctt ctcccagcca catgtcctgt tcctcacctc cttccagtcc
52080

tctccagcac ccatcccttg ctttccccac tcaccatctc gggtctcctg gaggacagca
52140

ggacccagaa gcagcagcag agacagcagg acagacacca ttcttaccat tgtgtctgct
52200

tggaaggttc tgggcaggag gcacaggtat tatcctgggt cctggggcca tgcccagggg
52260

aggtgaatct acaggccaat gggagtacca ggccgggctc ctcccctgac agtaagaaag
52320

aagtatgcac aagccaggct gggcagaggt tgtgatcagt gagtctacac acaggatatg
52380

caaatttaga gctgagacag ttgatctctg gactgaggat ttgtgtgtgc ctggggcgga
52440

aggatgccag tgtgtgggag gtgtgtgtga tgggtggcag gtgcctggga tgtgtcgccc
52500

ttccagtcta ttatgacacc ctctggggtc acttgggtgt tgtcagacca cacttcatct
52560

ctgccttctg gaaaaactct caacatgtcc aagacacgga catgggtaga cccccgctgg
52620

ggtgggaccc tcactctccc acatccgatc ctgccagagc tgtctggagg acattcccat
52680

tcccatctct ccctctttcc tttaggtgcc tgtggaatcc cagccttcca tgatggaagc
52740

agaaatagcc gcatcctcct tttgcagcct ccctggcaat agaacgagca ggtgtgcaca
52800

gggtttatgc tcagcaagtc gaagctactg ccttggatat caggtcaagt gttagggaaa

52860

caaagaggag ggcacagtgg acaatgtttt ctggtggtct tggcaggggt tgcaactgcc
52920

agctcccggg gcagtggggc tgcagtagct gcggggggcg tcttgagctt cagtcccccg
52980

ggatgttgta gatcccgtgc tgtggcctct gctgccccct gctggcagct tctgtatatt
53040

tactgggccg gttgcaggtg tgattttgac tgttgggctc ctctggaatt ctgagcctgg
53100

gttctccatc ctttctgtgg attctgagag ctaaccaata aatacgttta ttttctgctt
53160

aaggtcagaa ctgcattacg tttccaataa caaatactcc tggcggatac agagattggt
53220

acaaggattg tttccaggca gtatttactc agggaaatgg agggctggga agctgaggtt
53280

ggttactggg ccagattggg gctgcgcagt gaaaacacaa gagtcttggg ggattctggc
53340

aggcctggtg tgcagtgata acttcaacag ttggctgagg tatctgttat ggtagattgg
53400

tgtagggacc agccccacag ggtcggaggg tttttctccc cgtgtgcaga gacgagagag
53460

tgtagaaata aagacacaag acaaagagat aaaagaaaag acagctgggc cccggggacc
53520

actaccacca agacgtggag accggtagtg gccccgaatg tctggctgcg ctgttattta
53580

ttggatacaa agcaaaaggg gcagggtaaa gagtgtgagt catctccaat gataggtaag
53640

gtcacgtggg tcatgtgtcc actggacagg gggcccttcc ctgcctggca gccaaggcag
53700

agagaaagag gaagagagag agacagctta tgccattatt tctgcttatc agagacttag
53760

tactttcact aatttgctat tgttatctaa aaggcagagc caggtgtaca ggatggaaca
53820

tgaaggcgga ctaggagcgt gaccactgaa gcacagcatc acagggagac ggttaggcct
53880

ctggataact gcgggcgggc ctgactgatg tcaggccctc cacaagaggt ggaggagtag
53940

agtcttctct aaactccccc aaggaaaggg agactccctt tcccggtcag ctaagtagcg
54000

ggtgttttcc ttgacactga ggctaccgct agaccaccgt ccgctcggca aggggcgtct
54060

tcccagacac tggcgttact gctagaccaa ggagcccttc tggtggccct gtctgggcat
54120

aacagaaggc tcgcactctt gtcttctggt cacttctcac tatgtcccct cagctcctat

54180

ctctgtatgg cctggttttt cttaggttat gattatgcag cgaagattat tataatattg
54240

gaataaatta taatattgga ggattattat aatattataa tattggaata ataaatttgg
54300

aaaaaactaa tgattaatga tattgatata taatcatatc tatgatctag atctagtata
54360

actcttgttg ttttatatat tttattatac tggaacaact catgccctcg gtctcttggc
54420

ttggcacctg gatggcttgc cgcccacatc tccccccttt ttattaacta ggatcgccat
54480

cgccatcatt gcttgtcgtt gacttcggat ttgttttcgg actccttgga ggcatctgca
54540

ggctaaaagg agacaacgta agcataccaa tattaataat gccagtgaca acaatgatcc
54600

tccaaggggt ttgatctatt taaagggatt aagatcagat aattgtttag ttatgccttc
54660

aaaaatgtct gagccaggaa cagtggataa atgagcttgt gaatcctcga aaatttgctc
54720

tttaagtttt gaaatatcca aggttaagtt atcatcccag gcttttaaat gtcttgagac
54780

attttcccag ctatgttgat atttattata agcataaggc attatgcaat aatcagaagt
54840

attccaatca ctctgtaatt gcatacggtg ttccaaattc atatctccca gccagattac
54900

actttggcgg agatcattaa tttgattagc taatttttga tcaatttgag cctgagaatt
54960

ccaaagtctg gaggagtttt tctgccatgc ttcaattcaa catattgaac ggtttgaaca
55020

gaattgtgga tggcaactcc agcagttgct gctgttgcag taaccgcaat tagtcctgca
55080

atgatggcaa taagagtaaa aataaatctc tttgttcttt tgcggatacc tttaagaact
55140

tcattgacta tgtgaataga gggggaagac tcccatggac gatgtaaaga aactggtatc
55200

cataccccct ccctagccct taccaagaga gtacttgtag tgggattaaa agtagcatca
55260

atgcacgtga acagcttaca gttatcacat tctatagttt gtgtattggg aatgataatt
55320

atatttccaa ccaacagcat gtaagggggt ttgacatagc tcctgatggg tatcacccgt
55380

tcagatatga gggtgatgtt gaatgtgggt gttttggtat tagtgtgaag gagttgatag
55440

gtagtgttcc atatccttat tcctgtcata gctgcagcta attttcatag ttgaggatgt

55500

tctggggtaa caaagggatg aatcattttt ggtctaggag gagtaatgcc tgcatccatc
55560

catttaaata ggtaagggga cacccattgt ctcagcctgt atgactgcca tccatcctct
55620

acataatcta acaaataatt aaactctgag catgaggtat ttttgctgga gcaatcttgc
55680

caataatgcc cttttggagc ccaatcaata acaatacctg cggctagact ttggagcaca
55740

acggccttag gagcattaca attattccat aaaattgaag tcactataaa aggtcccttt
55800

gtaggtttct ttaaccaaaa gagaggctgg gcacagttgc tcacactagt aaacccagca
55860

ctttgggaga ccgaggcggg cagatcacga ggtcaggagt tcaaaaccag cttggccaac
55920

atggtgaaac ccctgcctca gcttcctgag tgctgggatt ataggcatgt gccaccacgc
55980

gcagctaatt cttgtatttt ttcagtagag acgaggtttc atcatgttgc ccaggctggt
56040

ctcgagtacc tgacctcaac ctgaggtgat ccaaccacct cagcctccca aagtgctggg
56100

attacaagca tgagtcaccg cacccgggcc ccagtcactt tagaatagca tgttgctttg
56160

tattcggagg gtctctctgc aaatagccca tcaacactga gcgtgcctgg aaagacctgg
56220

ttttcaaata actggcttcg tctgtgtaaa acgagtcttg ttgtatgcat taaaaattat
56280

cttggctggg cgtggtggct cacgccttta atcccagcat tttgggaggc tcgttctgtt
56340

gccagggagg ctgcaaaagg aggatgcggc tatttctgct tccatcatgg aaggctggga
56400

ttccacaggc acagaaagga aggagggaga gatgggaatg tgactgtcct ccagacacag
56460

cctctggcag gatcggatgt gggagagtga gggtcccacc ccagctgggg tctacccagg
56520

tccatgtctt ggacatgttg agagtttttc tggaaggcag ggatacagtg tggtccaaaa
56580

acacacaaat gcccctactg gcccaggggt tgtcacaata gactggaagg gtgacacatc
56640

ccaggcgctt gccacccatc acacgcacct cctacccact ggcatccttc caccccaggc
56700

acacacaaag cctcagtcca gagatcaact ctggactcag ctctgaattt gcatatcctg
56760

tgtgtagatt cattcttcat aacctctgcc cagcctagct tgtgtatcat tttttttct

56820

ctattagggg aggagcccgt cctggcactc ccattggcct gtagattcac ctcccctggg
56880

cagggcccca ggacccagga taatatctgt gcctcctgcc cagaaccctc caagcagaca
56940

caatggtaag aatggtgcct gtcctgctgt ctctgctgct gcttctgggt cctgctgtcc
57000

cccaggagaa ccaagatggt gagtggggaa agcaagggat gggtgctgga gaggactgga
57060

aggaggtgag gaacaggaca tgtggctggg agacaggctg gatgcagctg ggataccctg
57120

gcatacggca ggaatgggtg cccaaggctg tcaactccct cagctcacac acttccagga
57180

gcattcaggg agcctctgcg ctggcccgaa ataagacctt caggaatctg aatctaaaac
57240

ccctagttta cagtgaaaac aaagactcca aagaccaagc gacctgcttg gggtagacag
57300

tcaggacgga gtaggaacca tatgcctgga gctgcttctg ctcctgttcc ttccctcctt
57360

ccgatggctg ggtacacctg cctgacgctg agggaaagag agagcagccc caaggggaaa
57420

gtgggaaggc aggttggctg gagggatggt gctagaagga aacccgtgcc caaatcccac
57480

actcagacac cactgcagtg ggtctggaag gcgagtggct ggaagagaag agagtgggag
57540

ctccgggaga tcaagagtca ctcctaggat aagggaagga ggctgtttgt ggcatgagaa
57600

tgtgcaggat aaagacatgg aagcgaatgg cttctcagtt gtgtgagttt aaaattcatg
57660

acatttacaa attgtcagaa aaggtgttat atgtttgtta tataacaatc actttggaat
57720

gttaatctga ttctgtgcca aaatctgaat tactcagggt tctccagaga aacagaacta
57780

ataggtggta cacatataca tatatatgta cgtacacata catacataca ctgtatacac
57840

atggatacac acacacatag gaagagattt acatatatgt atacaaaaga gagagagagt
57900

agagatttat tttaagaaat tgactcacac tattgggagg agtaacaagt cctaaatctt
57960

cagagccggc cagcaggctg gagacccagg gaagagttga tgtcttagtc ttgattccaa
58020

gggcagactg taggcagaat tctttcctct ttaggggaca tctgaggctt tttctcttaa
58080

ggccttcaac tgattggatg aagcccacca ctatggagag taatccactt tactcaaggt

58140

ctactgattt ttttgtaaat taaaaaaaaa actgtgggtg catagtatgt gtatatattt
58200

atggggtaca tgagaggttt tgattcaggc atgcaatgtg aaataatcac atcatcaaaa
58260

atgaggtatc catcccttca agcttttatc gtttgtgtta cagacaatcc aattatactt
58320

ttttggttat tttagttttt aaaagtattt gattatttat ttatttattt atttttgaga
58380

cagagtctca ctctgtcacc caggcaggag tgcagtggca tgatctcggc tcactgcaac
58440

ctccgcctcc caggttcaag caattttcct gcctcagtct cctgagtagc taggactaca
58500

ggcacctgcc accacacctg gctaattttt ttgtattttt agtagagacg ggtttcatca
58560

tgttggccag gctagtcttg atatcctgac ctcgtgatct gcccgccttg gtctcccaaa
58620

gtgccgggat tacaggtgtc agcaactgcg cctggcctct cttttggtta tttaaaagtg
58680

tacaattaaa ttatgattat tattattatt tttgagatgg attcttgttc tgtcacccag
58740

gctggagtgc agtggcgtga tcttggctta ctgcaaacct ccgcctgttg ggttcaagca
58800

attatcttgc ctcgggtgta cactgccaca cacggctaac ttatgtattt ttaatagaga
58860

tagggtttca ccatgttggc tagactggtc ttgacctctt gacctcaagt gatccactca
58920

cttcagcctc ccagagtgct ggaattacag gcacgagcca ccacacctgg ccccagttaa
58980

attattattg actatagtca ccctgttgtg ctatcaaata gtaggtctta ttcattcttc
59040

tttttttttt tttttttgtg acagagttgc ccaggctgga atgcagtggt gcaatcttgg
59100

ctcactgcaa cctctgcctc ccgggcttaa gcgattctcc tgcctcagcc ttctgagtcg
59160

ctgggactac aggtgtgtgc caccacgccc ggctaattta tgtattttta gtagagatgg
59220

ggtttcacca tgttggccag gctggtttcg aactcctgac ctcaagtgac ccacctgcct
59280

cagcttccca aagtgttgga attacaggca tgagccacca cacctggccc cagttaaatt
59340

attattcact ggagtcactt tgttgtgcta tcaaatagtt ttctaactat tttttttgta
59400

cccattaacc accctcccaa tttcccccca accctgccac tacccttccc agcctttggt

59460

aaccatcctt ctactctcta tgtccatgaa ttcaattgta gggtctactg atttaaaggc
59520

taatcacatt tagacactca ggagcaagaa taattttagt aattgaacta ggattctgcc
59580

atatgacctc caacatcatt agcacctgtg taaattgtat cataaaataa ttatggaact
59640

attatggaaa tgtccctctc tcccagatcc caccttgtac caaaatgcaa ggtacaaccc
59700

cgggaattct gagctccatc ctagtcttac cctgtgctaa ttcagtctgg gtcatttctt
59760

gaattttctg gtaaattctc ctttctaccc tttctaacta tatgtatttg tcaggttaag
59820

ctagaagtgt taattttttt ttttttgaga tggagccttg ctttgtcacc taggctgaag
59880

tgcagtggca tgatctcagc tcactgcaag ctccgcctcc cgggttcatg ccattctcct
59940

gcctcagcct cctgagtagc tgggactaca ggcacccgcc accatgcttg gctaattttt
60000

tgaattctta gtagagacgg ggtttcacca tgttagccag gatggtctcg atctcctgac
60060

ctcgtgatcc acccgcctcg gccccctaaa gtgctgggat tacaggcgtg agccactgag
60120

cccggacgaa atgttaattt gttttttttg agacggagtc tcactctgtc atccaagctg
60180

gagtgcagtg gcatgatctt ggcttgttgc aacctctgcc tctctggttc aagtgatttt
60240

cctgcctcag cctccagcat gactgggatt acaggcccgc accaccatgc ccagctaatt
60300

tttgtatttt ttaatagaga tggggtttca ccatgttggc caggctggtc ttcaactcct
60360

gatctcaagt aatctgcctg ccttggcctc ccaaagtcct gggattacag gcatgagcca
60420

cggagcccag cctagaaatg ttaatttcta acgcatgtca gattccatgc acactgggca
60480

aggttccatt cctccatggg gtgactcagg gatccaggcc aattgcatat tgagactctt
60540

tcatattatc ctgtggcctt caaagtcgtc acctctaggg atgagaaaca aaaggacaa
60600

gccagctggt agggtcttgg acaagaagaa agacatcact tctgctcaca ttctcttttg
60660

acaaaactca gtcacatggt cccaatatat cttcgaggtg gctgagtaat gttatcttcc
60720

tatgtgtcaa gcagaggaaa taatgtagtg aagacacagg atggtctctg aaatatcatc

60780

tcaggcatga aagtagagca tattcacttg agtgagcctc cagtggtgtg aagttgatgg
60840

caggagaaag agctggggaa gaaaaggcca gtggcaggtc tcccctccta gccctatgca
60900

gccccacagt gggacccttg catggacctc aaccatcaga atcttttctt ttgcaggtcg
60960

ttactctctg acctatatct acactgggct gtccaagcat gttgaagacg tccccgcgtt
61020

tcaggccctt ggctcactca atgacctcca gttctttaga tacaacagta aagacaggaa
61080

gtctcagccc atgggactct ggagacaggt ggaaggaatg gaggattgga agcaggacag
61140

ccaacttcag aaggccaggg aggacatctt tatggagacc ctgaaagaca tcgtggagta
61200

ttacaacgac agtaacggtc agtgaataac agaccacagg ggtggaaggt ctaacccaag
61260

aggcagcccc cccagtgtga gtggcaaggg atcagcagga tggaaatagt cccaatccca
61320

ggggaagaac aggagacaca gcagaaacac agacatgtcc acatcccacc caccccacag
61380

cacaggtgct ccccgcttcc ccatcaattg ccccatcctc atcccaggcc tcaggtcaca
61440

caggaagtga tggcagagtc acttcctatc caggcaccta tgacctctca cctccacacc
61500

ccacccatcg gaggctgata cccccgtgag aaggcatcag actcacccct gtccagggag
61560

gttgcctgga gagtgagcca ctctcaaagt cactcagacc tgggctcacc tggtggttct
61620

gccagtccta gctgttgaca gtgaaacgtt cccaaaatat ctggttgaaa tctgcaaaca
61680

ttggagcact gagacctacc tccaaacaag tctgtaatat ttaactatgt ctgttctatg
61740

aaggatgtca cagtctgtcc tgatctccct tgcagctcca tcacctagca cagggtacag
61800

ccaatattgg ctcaattgaa atttgtggaa tcaacagaga aaagcaccca gcacacaccg
61860

tagcccatgc tgggggctca ggaagtgctg gattcaaaac tgtgggctgt tagagttcct
61920

tggagcccta aagttcctcc ttaccatacg atgcagaccc aggaagggcc acctgcgcta
61980

tggtcagagg agctggtggc agagcccgtg cagagatggt ccctgtgccc ccggcccagt
62040

gctctttctc ctaaaccaca ctgccagccc caaggcagcc aacctcaggt ctggtgaact

62100

gctggtgtta aattatcata gagtgggtgt caaaagatgg gctactaagt acaaaaatgc
62160

ccaaggtgct acatgggatc tgaagatttt caaaaggagg caagaaagag ataggcagat
62220

gtttcaagga tgtggggtgg gggaggtctt ggtaaggaaa atggcccagg ctgtgtgtca
62280

gcaataggag aggagggggc acaggtgatc agaaaagaca ctgggggaag cattgatgga
62340

caggaataga aatggcaaag tggataatta agaggaagga ggatgaggag atgaacacag
62400

ggtattagaa aataatagaa ggcagggctt ggtggctcac tcttgtaatc ccagcacttt
62460

gggaggctga ggcaggcaga tcacctaagg tcaggagttc gagaccagcc cggccaacat
62520

ggtgaaaccc tgtctctact aataatacaa aaatagcctg gcatggtggc acacgtctgt
62580

ggtcccagct actcaggagg ctgaggcagg agaattgctt gaacccagga ggcagaggtt
62640

acagtgagcc aaaatcctac cattgcacta cagcctgggt gacaagagtg aaacgttgtc
62700

taaaaacaaa aaacaaaaaa caaaaaaagg aaataatagt agctgacatt tactgagcac
62760

ttactttgtg ccaggcccat ctatgagcat atataatgct cagaatagcc ccctaaaaca
62820

gtgctcttgg cattgccatt tcagaggtga ggaaatagag gcacagggag ttgagtggct
62880

ccagttcagg caacacacca ggtgggggtg gggggctggg gagagacctg ggacgtgagc
62940

ccagacagct tgagagcttt cagagtctat gccaacagca ccaaccagtg ctgggtaaac
63000

acctgctttt atcatcagaa caaagaggct gtgtcccctg ccctatgagg tccatttctg
63060

agagttgtgg ctaatgggca agaaggttgg ggctttagag atttgggata aagatatcaa
63120

acaccagaaa ggtagaaaga agtgatcaga ttagggttac ttaggtgatg atatgaactc
63180

ttcctagaac tgagagaaaa agagagcctt cctttactca tatgaaatca caaataattt
63240

ctatccaatt tggaagtaca ctttggtgta gttgtgacag cttcctcagg actcagcata
63300

aattcaaaca aataattgtc cttagaagag atgctataga agagatagaa atatattcat
63360

attctgtagc tttttttttt ttgagatgga gttttgctct tgtcacccaa gctggagtgc

63420

agtgatgcaa tctcagctca ctgcaaactt tgcctcctgg gttcaaggga ttctcctgcc
63480

tcagcctccc gataactggg actacaggct acaggcatgt gtcactactc ctggttaatt
63540

tttttttttt ttttaagact gagtcttgct ctgtctttca ggctgatgta caatggctcc
63600

atctcggctc actacaactt ctgtccccca ggttcaagcg attctcctgc ctcagcctca
63660

tgagtagctg ggattacagg catgtgccag cacacccagc aaatttttgt atttttagta
63720

gagatgaggt cttaccatgt tggccaggct ggtctcaaac tcctgacctc aggtgatcct
63780

ttggcctcag cctccctaac tgctgggatt acaggcatga gccactgcgt ccagcctaat
63840

tttatatttt tggtagagat ggggtttcac catattggcc aggctggtct cgaactcatg
63900

acctaaggtg atccatcctc ctcagcctct caaagtgctg ggattacaag tgtgagccac
63960

tgggcctggt gctttttttt tttttttttt tttttttttt gagatagggt ctcactctgt
64020

cacccaggct gaaatgcagt agtgtgattt tggctcattg cagccttgac ttcccaggct
64080

gaagtgatcc tcccacctca gcctcctgag tagctggggc tacaggcatg caccaccatg
64140

ctgcgctaat tttatatttt tttgtagtgg tgggatttcg ccatatcacc ctggctggtc
64200

tggaacccct gggctcaagc gatccactcg cttcagcttc tcaaagtgct gggattacag
64260

gcatgagcca cagcgcccag gctgtagctc tcttaaggag gaacatatct catctgagac
64320

aaacctgaaa tgccaaacca aactgagtta gcccctctct gtctgttgta tatattggag
64380

taataaccta tttgtcttga taaagggatt gcatgcttga attgcaaaaa cctttatttc
64440

ttttgggttg cccaatgtgc aagactaaga gttattttga taaatttctc accaggctga
64500

ctgtctctct gtggggtcgg gggagttttc agggtctcac gtattgcagg gaaggtttgg
64560

ttgtgagatc gagaataaca gaagcagcgg agcattctgg aaatattact atgatggaaa
64620

ggactacatt gaattcaaca aagaaatccc agcctgggtc cccttcgacc cagcagccca
64680

gataaccaag cagaagtggg aggcagaacc agtctacgtg cagcgggcca aggcttacct

64740

ggaggaggag tgccctgcga ctctgcggaa atacctgaaa tacagcaaaa atatcctgga
64800

ccggcaaggt actcactgct tcctgctccc cagtactgag cccagaataa aagacgatct
64860

caggctagga gctcaggcaa catcttagtc cggtctcatc tgttcctgga tgtccctcag
64920

acccccagct ttcatctttt aggatttatt ccttccctgg gataatataa tttgtggtcc
64980

aaaaagaaca tcatcaaaat ttcaggcaga atgggccagg aaggccattc tttcttgatg
65040

agtgtcccca aatcatctcc aattaacaga caaggagctt gaggttaggg aggtgagggt
65100

aacactgtct gtaagaggca gagctgggac tcaaattcca gatttcagat tccaaatccc
65160

atcgtttttt atctctacaa tgatgcctcc catctgggtg gtggagagaa gggaggcgtg
65220

taaaatgtca gccccagaag gacaagagca agccagtgtg agcggaattg atggctgcaa
65280

gctgagactt ggattggaga cgtagtgaga ctcaggattg tgcagtgctg cagggaagtg
65340

gttgctggat agaggcatgg gctgaaccaa gcagctggac tgagactggg ggacagaact
65400

ccaaagccca ctgagatgtg ggaaaacatg gagaagcaca cggagcattc acaacttatt
65460

gccgtcagag tcaatacatg ggtgaggtgg ggattgggca agagggaaag cgtcagcctt
65520

ccctgatatt ctggaaagtc tcccggggct gggggtgggc aggtacagag cttcgagctc
65580

tgctgatcgc tgacatccag gggtgggggt aggaagagac ctgggccggg agaagtccac
65640

ctcaagcctg cagtgtcaca ctctatccct ccacagatcc tccctctgtg gtggtcacca
65700

gccaccaggc cccaggagaa aagaagaaac tgaagtgcct ggcctacgac ttctacccag
65760

ggaaaattga tgtgcactgg actcgggccg gcgaggtgca ggagcctgag ttacggggag
65820

atgttcttca caatggaaat ggcacttacc agtcctgggt ggtggtggca gtgcccccgc
65880

aggacacagc cccctactcc tgccacgtgc agcacagcag cctggcccag cccctcgtgg
65940

tgccctggga ggccagctag gaagcaaggg ttggaggcaa tgtgggatct cagacccagt
66000

agctgccctt cctgcctgat gtgggagctg aaccacagaa atcacagtca atggatccac

66060

aaggcctgag gagcagtgtg gggggacaga caggaggtgg atttggagac cgaagactgg
66120

gatgcctgtc ttgagtagac ttggacccaa aaaatcatct caccttgagc ccaccccac
66180

cccattgtct aatctgtaga agctaataaa taatcatccc tccttgccta gcataacaga
66240

gaatcctttt tttaacggtg atgcgctgta gaaatgtgac tagattttct cattggttct
66300

gccctcaagc actgaattca tctgaaactc ttggtttccc ctggaggcca tggttcctgg
66360

gcaccttgac ctgggcaatc ccaagtgtgg cctgaacccc ctttcccttg gggattgttc
66420

aggtgtccct agacgccttg tggtattgta cctaataccc atgaagggag aggatgatat
66480

tacttgccag tgtacacccc cctgtgatat tgttcataat gtccagagtg aagaaagatg
66540

atattactcc caatatcaca gaaggtgtac accccccctt gatattgttc ctaataccca
66600

gttggggagg ggagaatatc tctcccaata tacaaggggt gtttaaactc tctgtgatat
66660

tgttcctaat attcaggggg gacaaggatg atattaccca aatattgcag gggttgtaca
66720

cccccccttt gatattgttc ctaatatcca ggggtggaga ggatattact cccaatattg
66780

caggggtcta catcctcccc ccgtgacatt gttcttaata accaaaaggt gagaagctga
66840

cattactccc aataccacag ggggtgtaca ccccctatga gatattgttc ttaatatcca
66900

ggaggggaga aaatgatatt actctcaata gcgcagggaa tttacatccc ccgtcgtaat
66960

cttgttctta atattcagga agggagagga tgatacgact cccagtatcg cagggggtgt
67020

gcaccccccc gtgatttttt tgctaatatc cagggtggga gtggataata cgcaggaagt
67080

gtacaggtct ctgtgatatt tttcctaata tccagggggg agaggaagat attactttta
67140

atagtgtacg gggggtgtac acccctctgt gatagtgttc ctaacatctt gggagggaga
67200

ggatgatatt actcccaata tcgcaggggg tgaaaacttt tttttgatat ttttcctaat
67260

atccaggggt ggagacgatg aaattactcc caatatcaca gaaggtgtac actccttttg
67320

tcatattgtt cctgatatcc agggtaggag aggatgatat tacttctaat atcgcaggaa

67380

gtgcatacgc ccttgtgata ttgttcctaa catccaaagg aagagagaat agtattactt
67440

ccaatatcgc agggggtgta cagcccccct tgtgatattg ttcctaatat ccaagatgga
67500

gaaatttcta ttattctcaa tatcacaaag gatgtacacc accaccgtaa tattgtttgt
67560

aatttccagg gaggggagag gataatatta ctcccaatat cccaagggtt gtacacccct
67620

ctgtgatatt tttcataata tttaggggtg aagaggatga tataaatgca aatatcacag
67680

ggggtggtca ctgtcttgtg atattgttcg aaatatccaa gaggggagag gatgaaatta
67740

ctcccaatat ctcagcgggg gtacacccct ctgtgacatt ccttgtagta ttcaggggaa
67800

gagaggataa tattactccc aatgtcgctt tgggtattca ttcccctgtg atattgtttg
67860

taatatccag gaaaggagac gatgacatta cttttaaaat ctaaggggtg tacacacctc
67920

tgtgatatgg ttcataatat ccaggggaag agaggataat attactccca atatcacagg
67980

gggtgtacac ccgcctgtaa tattgttcgt aatattcagg gtggaagagg atgaaattac
68040

tcccaatatc gcagtgggtg tgtaccccac tgtgatattg tttgtagtat ccagtcaagg
68100

ggaggatgat attactccca ataccgcagg agatgtacac cactctgtga tattgttcgc
68160

agtatccaga gggggagtgg gtaatattat tcccaatatc gcagtaggtg tacacttccc
68220

tgtgatattg tctgtaatat ccagggaaaa aagaatgata ttactcccaa aatttcaggg
68280

aatgtacacc ctcctgtgat attgtgcata atatgcaagg gggaagagga tgatattaca
68340

gcaaatatca cagggggtgt acaagcccct gtgttatagt ttgtaatatc cagaagggga
68400

gaggataata ttacttccaa atcacagggg gtgtacaccc ccctgtgata ttgtccgtaa
68460

tatccagggg ggagaggatg atattattcc gaatatcgca ggggtgtaca ctctgctgtg
68520

gtattgttcg caatatccgg ggaggaggat gatatcaatc ccaagatggt aaacaccctg
68580

tgtgtacacc accctgtgat attgtccata atatccgggg aaggggggag aaaataatat
68640

tactcccagt atcgcaggga atgtacactc ccctgtgata ttgtttgtaa tatgcgggg

68700

ggaggggaga atatgatact acttccaata tcgcaggggg tgtaaacccc cctgtaatat
68760

tgttcgtaat attctgggag gggagaggat gatattactc tcaatattgc aggggtata
68820

caccaccctc tgtgatattg ttcttaatat ccagaggagg agaggatgat attattccca
68880

atatcacagg gagtgtactc tccctttggg atattgttcc taatatccat gggaagagag
68940

gataatatta ctcccaatat cgcagggaat gtatatcccc ccctttgata tagtttctaa
69000

tatccaattt gggagaggat attaatccta atatcgcatg gggtgtacac gctccctgtg
69060

atattgttcc taatgttcag gttgggagag gatgaggttg tacaccgcct gagatattgg
69120

gagtagtgtc atcctctctt cccgttgata ttaggaagaa tttcacagga ggggtgtaca
69180

tcttctgtgc ttttgggagt aatatcatct tctccttccc tgggtattag gagcaatatc
69240

tcgggggggt gtacatactc tgcaatattg ggagtcatat cgtgttctct caccctggac
69300

agtaggaaca atatcacagg aggggtgtac accccctgcg atattaagag tagtatcatt
69360

ttcaggccgg gtgcggtggc tcacgtctgt aatccctgca cttttggagg cagaggcggg
69420

aggatcacga ggtcaggaga ttgagaccat tctggtaaac acggtgaaac cccggctcta
69480

ctaaaaatat aaaaaattag ctgggcgagg gggtgggcgc ctgtagtccc agctactcgg
69540

gaggctgagg caggaaaatg gtgtgaacca gggggcggga gcctgcagtg agccgagatc
69600

gcaccactgc actccagctg gggcgacagc gagactccgt ctcaaaaaaa aaaaaaaaag
69660

tagtatcatt ttcccttcct ggatattaga aacaatatca catttggggt gtaaaacccc
69720

tgctatattg gaagtaatat catcctctct tttcatggtt attaggaaca atatcacagg
69780

ggtgtataca ccttctgcga tattgggagt aatatcatcc tttccccacc tggatgttag
69840

gaagaatatt gcaggcggag ggtacacccc ctgcgatatt gccagtaata tcatcttatt
69900

ccttcctgaa tattaaaaat aatatctctg ggggggtgta cacccccttc aatattgggg
69960

gtaatatcat tctctttccc ccgggatatt ggaaagaata tcacggggtg gtggtgtaca

70020

ccccctgcga tattggtagt agtatcaacc tctcacccct aaatattagg aacaacatca
70080

caaagggggt gtacaccccc tgagttattg ggagtaatgt cattgtctat cccctggata
70140

ttaggaaaat taacacagga ggtgtgtaca cctcctgcaa tattgggagt aatatcaacc
70200

tttccccacc aggatattag gaacaatatc atgtgggggt gtacacctct ttccatattg
70260

aaagtaatat catcctcttt tttcctggat attagaaaca atatcacagg aggttgtaca
70320

cctcctgcga tattgagagt aatatcatcc tctctccttc tggatattaa aaacaatatc
70380

acaggcaggg tgtacacccc ctgcaatatt gggagtaata tcatcctctc tccacttgga
70440

tattaagaac aatgtcacag ggggtatgta ccccccctgc gatattgaaa gtaatatcat
70500

cttctcttta cctaaatatt aggaacaatg tcacagggag ggtgtatacc ccctgcgata
70560

ttgaccataa tatcatgctc tttcttcttg gatattagga ccaatatcac aagggggtg
70620

tacaccctct gcaatattgg gagtactatt atcctctccc cacctggata ttaggaacaa
70680

tatcacagag aaggtgtaca ccctcttcga tattgggtgt aatatcatcc tctccctact
70740

tggaacaata tcacggcatt ggggggaggc tgtacacttt ctgcggtatt gacagtcaca
70800

tcatcctctc accccctgga tattaggaac aatgtcacag aaggtgtgta cacgccctac
70860

gatattggta gtaatatcct ctctccacct ggatattaaa aacaatgtca caggggtat
70920

gtacaccccc tgcgatattg ggggtaaagc aacctttcct tctttggata ttacataaaa
70980

tatcacagaa agggtgtaca cctcccgcga tattgggagt aatatcatcc tctccccact
71040

tggatattag aaacaacatg atgggggttg tacacttcct ccgatattgg gaataatata
71100

atcctctttt gccctcgata ttaggaaaaa tattacatta agggtgtaca cccccttcga
71160

tattgggagt aatattatcc tctacccccc cgatattagg aacaatatta ccagagaagt
71220

gtacaccccc tgagacattg ggataatatc ctctccccgc ctggatttta ggaacaatat
71280

cacagtgggg gtgtataccc cctgcgatat taagagtaat attatcacag ggaggtgtac

71340

actttttgag atattgggag taacatcatc ctctcgcccc cgaatattag aaacaatatt
71400

acggagtggg gatacactcc ctgcgacatt gggagaaata tcatcctctt ccctctgaat
71460

attaggaaca aaatcacaga gggttgtata ttccttgcga tattggcagt aatatcctct
71520

ctgcccctgg atattaggaa caatatcaca gaaggggtgt atcccccttc gacattggga
71580

gtaatatcat cctctctgcc gctggatatt aaaaacaata ttaaaggggg gtgtaaaacc
71640

cctgcgatat taaggtaata tcatcctctc cccccccgga tattaggaac aatatcacag
71700

ggggagtgta caccccctgc tatattgaga gaaatatcat actctcctca aatggatatt
71760

aggagtaata tcacagggat ggggtgtaca cgttctgcta tattgggagt aatatcatcc
71820

tctccctacc tggatattaa atacaatatc acagggggtg ttaccccccc tgcaatattg
71880

ggagtaatat catcctctcc cattctgaat attaggaaca atatcacagt gggggttgta
71940

caccccctgt gatattggca gtaatgtcat tctctcccgc tttgaatatt aggaacagca
72000

tcacggaggt tgcacacaac ctgcaatatt gggagtaatg tcattttctc ttccacctgg
72060

acataagaac aaatattaca gggaggaata caccttctgc gatattggag caatatcatc
72120

ctcatttccc ctggatatta ggaaagatat tacagtgggg ggtggacacc cctgcgacat
72180

tgggagtaat attatcctct gccccgttgg atattagaaa aaacatcaca aaggaagtgt
72240

acaccccctg ctatattggg agtaatatca tcctctcccc cctggatatt agaaacaata
72300

tcaaggggga gagtacacca ccttcgatat tgggagtaat atcatccttt gcccttctcg
72360

atattaggaa caatatcgca gtgtggtgta cactgcctgc gatattggaa gtagtatcat
72420

actcttcctc cctcgatatt atgaacaaca tcacagggtt gtgtaccccc ctgcaatatt
72480

gggtttaata ttgtcctctc tttccctaga tattactaac aatattacca gggagtgtac
72540

acacagggtg tttaaaatat taaacttgat gtcatcctct cccccccgga tatggcaagc
72600

aatatcacat ggaggtgtac accccctgtg atattcgaag taatataatc ctctccctcc

72660

ctggatatta caaaaaatat cacagcggaa tgtacacccc ctgcgatatg gggagtaata
72720

tcttccattt cctccataaa tattatggac aatatcacag gggcatgtga accacccgtg
72780

atgtgggaaa taatgtcatc ctctcccctt ctgaatatta caaacaatat tacagggggg
72840

tgtacacacc ttgtgatata ggaaataata ttattttctc cccctctgga tattacaaac
72900

aatatcacgg agaggtaata tcatcctctc ccccactgga tattatgaac aatagcacaa
72960

ggggatgtac accccctgtg atatggggag taatatcatg ccttcccccc tgcatattag
73020

gaacaatata ataggggggt gtacatcttc tgcgatatgg ggagtaatat cttcctctcc
73080

ctccctaagt attacaaaca atatcacagg gggatgtcca ctccctgtga tatgggaagt
73140

aatgtcatcc tctccccttc taaatattac aaacaatatt acaggggggt gtacacacct
73200

tgcgatatat gaaataatat tattttctcc ccctctggat actatgaaca atatgaaagt
73260

gggatgtata ccccctgcga tatggggagt aatatcattc tctcccctcc tggatattac
73320

gaacaatatt acaatcgggt gtacaccctc tacgatatga agagcagtaa tctcctctcc
73380

cccttggata ttaggaacaa tattacaggg gatgtacacc ccctgcgata ttgggagtaa
73440

tatcatcccc tcccctcctg gatattacga acaatatcac agacggtgta cacacagggt
73500

gtttaccata ttgggagtaa tatcatctcc cttccaggag attacgaaca aaatcacaac
73560

ggggtgttta ccccctgtga tattgggagt aatattatcc tcttccctcc tggatattat
73620

gaacaatatt acagaagggt gtacaccctc tgcgatattg ggagtaacat cactctctcc
73680

ctttctgaat ataatgaaca atatcacagg gaagtaacat catcctcttc ccccctggat
73740

gttacgaaca atatcacagg aaggtgtaca ctccttgcaa tatggggagt aatatcatcc
73800

tctttcccct gggtattacc aacaatatca caggagggtt tacaccatca gcgatataag
73860

aagtaatgtc accctctccc tccagggata ttatgaacaa tatcacgggg gggtgtgccc
73920

cctctgccat ataaggagta atgttatcct ctcattccct ggatattaca aacaatatca

73980

cagggggatg tacataccct gcgatatggg gagtaatatt atcctctccc cactgaatat
74040

tatgaacatt atcacggggc agtgtacacc ctctgccata tgggaagtta catcatcctg
74100

accctcattg gatattttgg agaatatgac ttgtgggtgt gaccatcctg caatatgggc
74160

agtaatatca tcctctcccc ttctggatat tacaaacaat atcacaggag ggtgtatact
74220

gcctgcgata tcgggagtaa tattatcctc cctcctagaa tatcacagag gagtgtacac
74280

tccctgcgat attgggagta atatcaccct ctcccctcct ggatattacg aacaatatca
74340

cagatggtgt acacacaggg tgtttaccat attgggagta atatcatctc ccttcctgga
74400

tattatgaag aatattacag gggggtgtac acctccctgt ttatgggagt aatatcattc
74460

tcttcccctt ggatattaga aacaatatca caggtgggtg tacaacccct gcgatatggg
74520

gagtaatatt atattctttc tccctggata ttatgaacaa tatcacagaa gggtgcacac
74580

ccccggtgaa atggggagta atagcatcct ctcccctcct ggatattacg aataatatta
74640

taggagggtc tatatcctct gccatatggg gagcaatatc atttcttccc ctctggatat
74700

tacaaacaat attacagggg gttgtacaca ccctgcgata taaggagtaa tatcattctc
74760

tccccacctg gatattacga acaatatcac agaggggtgt acaccttcta tgatataagg
74820

agtaaaatca tctttttttc cctgtggata ttaggaacaa tgtcacagac agtgtacaca
74880

cacagtgctt acgatatcga aaatagtatc atctcccccc tgaatattac aaacaatgtc
74940

acgggggggt ccatcccctg tgatattggg agtaatatca tcttttcctc ccctaaatat
75000

taaaaacaat atcacagggg aatgtatacc tcctgcgata ttgaaagtag tatcctcctc
75060

tcccccactt ggtattagga acaatattac aggagaagta tacaccccct gcgatattgg
75120

gagtaatatc gtcctcttgc ccctggatat tagggacaat atcacagggg ggtatacatc
75180

tcctgcaata ttgggagcaa tatcatcctc ttcctccctg gatattacaa acaatatcac
75240

agggggtgta cacaccctgc gatatgtgga ataatgtcat cctctctatt ctggatgcta

75300

cgggcaatat cacaggggat tgtacctccc ctcttctaat atcatccgct cttctgttga
75360

atattatgaa caatataaca tctggtgtac aaagtgacat gctattttaa ggtggctgag
75420

aaattattaa aggagatcat ttttctctct gcccctgaaa attataaatg atatcacagg
75480

ggagtgtatg ttccctgaga tattgggagt aatattatca tctctgcccc ttaatattac
75540

gaacaatatc agcgggcgta ttacgaacaa ttttatgaac aatatcacac cccctgtgat
75600

attgctcata atattcacag gaagggagga tgatattact cccagtattg cagggggtgt
75660

acattcccct atgatattgc ttttactatt taggggagga gaagatgata ttactcccaa
75720

tatcgcaggg gatgaacctc cccccgtgat attgtttgta atattcaggc gggagagaat
75780

aatattcttt ttttccctta atattatgaa cagtatcaca agggcgtgta caccccctgc
75840

aatattggga gtaatatcat tgtttctcct catgtatatt tcaaaaaata tcatgggggg
75900

tgtacactcc cgtgatattt ggagtaatat caccctcttt ccccttggat attatgaaaa
75960

atatcacagg gggggctgta cacccccctg ggatattgga agtagtatca tcctcttccc
76020

ctctggagat taggaacaat atcatagggg agtgtacacc tcctgtgaca ttgggagtaa
76080

tatcatcctc tcttctgtta aatgttatga acagtataac atggggtgta caaagtgaca
76140

tgctattcta aggtgactga gaaattatta aaagagatca tttttggcaa tgagactctt
76200

tgggttgggg cctcttcaat ttgcacttgg atttggtcct acctgtggga gaatctaaca
76260

gtggtcctcc tttgggggag caagcagtga ctgtcgaagg aaccacattg ggttctctgg
76320

actcctttat catccctgat gcctagtcta tgctcatggc ccagccctac ccccaggtca
76380

cctcccttgt caggctcctg tgtcccccac acccacttgc tgtttcatgc tgacctggtt
76440

agaaacactc tttttttttt ttttttgaga tggagtcttg ctctctcacc caggctggag
76500

tgcagtggcg tgatcttggc ttactggaac ctccatcttc tgggttcaag tgattctcct
76560

gcctcagctt tgtgagtagc tgggattaca ggtgtctgcc accatgccaa gctaattttt

76620

gtatttttag tagagatggg gtttcaccat gttggccagg ctggtctcaa actcctgacc
76680

tcaggtgatc caccagcctc agcctacaaa agtgctggga ttacagtagt gaccgacctc
76740

ccctggcaga aacattcttt tgaccgcctc ttggcaagct cttaggtctt cacatctctg
76800

atacatcatt ccctacgggg gtttttctct gggtgactca gtggctctgt gcccctctcc
76860

ccaaccctga cacctgtcca ggacttcagt tttctgaggt ttcctccccc ttgtccaccc
76920

cactcctctt tcctgttaca gatgaggcca gatcccattt ttaatgttct ttagccctga
76980

cctgctcaca cgagcttttg gtttccttat tgacctaagc tgttgattct ttatcctttt
77040

atttgttgag ggtgtcatcc tttttgaatt gaaaaataca gttgctgtat ttttccctct
77100

tgattgagct ttcttccctg gggctctata tgttcagata gaactttttg ccccagaaga
77160

ggaaatgcgg atttcattag tctgaagaag taatttctca gctttgattt ccaagttgaa
77220

cccaaagcat taagaaaaaa aaaaaaaagc gggtggtggg gaaggataac ttcaaattta
77280

ggctttccaa aaagatgaga tgtttgactt agaattctga aggctgggaa tgatttcctt
77340

aaggagccca gcttggagga tggactttgg aataatccag agtgggaggc tgaggacagg
77400

atgtaaggaa agttctgtgc cacgggaagg aaaagcacag acacaggcag gtgaggtttc
77460

atgagaaggt caaggtgaga gctctgctgt gtcttttgcg gttgaaacct gtgcatggcg
77520

gttggtgact catttgagga aactcactcc ctgggacttg gaggggatct gacaacccca
77580

gggccaattc ctgtatcctt gagctgtgcc ctggagagct gagggttctc aaacaagctc
77640

tggatgtgtg agccaagacc aacatggtgt ggagatgcgg agcggctgga gtgaagggcc
77700

gggtctccca ggaagccaag gagtgtgggg aaactgaggc acagaagacg cttgggtttg
77760

tgtaaaagag ttttcaggcc gggcgctgtg gctcacgcct gtaatcccag cactttggga
77820

ggctgaggtg ggtggatcac ctgaggtcag gcgttcaaga ccagcctggc caacatggtg
77880

aactaaacac actaaacaca caaaacacac taaaacacac aaaaatttct actaaacaca

77940

caaaaatcag ccgggtgtgg tggcgggcgc ctgtaatctc agctactcag gaggctgagg
78000

caggagaatt gcttgaacct gggaggcgga ggttgcagtg agccgagatt gcaccaccgc
78060

actccagcct gggtgacaca gcaagactcc atctcaaaaa taaataaata aataaataaa
78120

taaataaata aataaataaa taaatagagt ttgcagaatt ctgagtagct ctgtgcccca
78180

ttgctccccc tccacatctg gccaagactt ggatcttctg aggtttcctc cccttcttct
78240

acccctaccc atctctcctg tcatgacgcc agagctcaca ttcaaatctt cctttgccct
78300

ggcctgctca tgttgccctt tgcttttttt aaaaaaattg aaatgttcct cctgtcatgt
78360

gctattcttt atttgctcaa agtattcgac attactgtga aatgtcactg gctgggcaat
78420

ttcctcctga gcgctttggc ttatcttctc tgactaaatg gaataaacct gttcaaatca
78480

gtatttttac atattgctca tccaatattg tacaggaaaa atactaatat ggggccaggt
78540

gtggtggctc acgtctgtaa tcctagcact ttgggaggcc aaggcaggca gattacgagg
78600

tcaggagatc aagaccatcc tggctaacat ggtgaaaccc tgactctact aaaagtacaa
78660

aaaaaaaaaa aagggaaaat actaatatgg ggctaggcat ggttgttcat acctgtaatg
78720

ccagtgcttt ggaaggctga agtgggagga tcacttgagt ccaggagttc gagaccagct
78780

gggcaacata ttgagacccc tgtctctacg aaaaccaaaa aaaaaaaaaa aaaaaaaatg
78840

agccaggcat agtggtgcat gcctgtagtc cttgctaccg agtaggctaa ggaggaagga
78900

ttgcttgagc tcaggagttc gaggctgcag tgagctatga tttcgccact gcactccagc
78960

ctgcaatggc ctgaatttcc tgttcagatc ttttatgaac caaatactac acaactgtag
79020

tcatgatctt cttggctgga gagaggctgg ctgtcaccca gggccacagg gtgattaggt
79080

agaatgaaaa gagagtcacc aactcaagac agtagcacag tgtccttgtg gttttggctg
79140

gtgtagtcta aaatctgaat tatgcaaagt gtatttgatc ttgcatcaca tcaggaaaaa
79200

gcccagtaat ctatttcatc atggggtgtg gagaagttgc tggaaccatt tctagacaca

79260

gattttggtt ctccagaggc cagcaagtta gcttcactaa gcttcggggc ccacagtggg
79320

aagagatgtc ctgctgaggc tcagcgttga cttcactctt gtggagcaaa gctgtaggaa
79380

ctaagggctt tatgctaatt aaaagatgag gccaggtgca gtggctcata cctgtaatgg
79440

ggtcttgcta tattgcccag gctggtctcg aactcctggg ttcaagccat cctcccacct
79500

cagcctcctg aatcactggg attaaaagtg tgagccactg tgcccagcct cccttttaat
79560

agaactggtg cttaatccac agctctcctg tcagcccaca gttcttgaat tttggcctct
79620

tttatgtcca catgaatgta aatatacttc agaatttcca cccagaatct ttggaatcta
79680

atttcttcaa tcattttact ttagagtcat ttttagggta agtgaatctg cctggttttc
79740

aacttgacac cctctctaaa cgtgaatgag ttcaaatcat attcgttcct gaggaatcac
79800

actcaagcat agtacaaatc tgtgggatat gccattacaa ctctaggaat catgttctca
79860

aggtaaaagc ctttaagaaa agctgtcttg acatatcatg taagcatgac aatggaatac
79920

taaaccaaca tagctgagat tttccttatg tcctatgctt ttaaatgatc aaccctacag
79980

tcctggaacc aacctttatg gttatcaata cctccattat tttaaactat ctcaggtatc
80040

atattccttc tttttttttt ttttttgaga cggagcctct cactgtcacc caggctggag
80100

tgcagtggcg caatctcggc tcattgcaag ctccacctcc aatggaatac taaatcaaca
80160

tagctgagat tttccttatg tcctatgctt ttaaatgatc aaccctacag tcctggaacc
80220

aacctttatg gttatcaata cctccattat tttaaactat ctcaggtatc atattccttc
80280

tttttttttt tttttttgag acggagcctc tcactgtcac ccaggctgga gtgcaggggc
80340

gcaatctcgg ctcattgcaa gctccacctc caatggaata ctaaatcaac atagctgaga
80400

ttttccttat gtcctatgct tttaaatgat caaccctaca gtcctggaac caacctttat
80460

ggttatcaat acctccatta ttttaaacta tctcaggtat catattcctt cttttttttt
80520

tttttttgag acggagcctc tcactgtcac ccaggctgga gtgcagtggc gcaatctcgg

80580

ctcattgcaa gctccacctc ctgggttcac gccattctcc tgcctcagcc tcccgagtag
80640

ctgggactac aggcacccgc cacgaccccc ggctaatttt ttgtattttt agtagtgccg
80700

gggtttcacc atgttaacca ggatggtctc gatctcctga cctcgtgatc cacccgcctc
80760

ggcctcccaa agtgctaggt ttacaggtgt gagccaccat gcctagcttt ccttcttata
80820

tgtatcaaaa ctcatactga aaaatgagtt ctgggttaca aaagagggtt tatttttgc
80880

acctgccttt gaatcagtcc ttaagcttaa cctttaactt ccaaggaact actttttttt
80940

tttttttttt ttagatagag tctcgctctg tcacccaggc tggagtgcaa tgatgcaatc
81000

tcggctcact gcaaccttcg tctcttgggt tcaagtgatt ctcctgtctc agcctcctga
81060

gtagctggga ttgcaggcat gtgccaccat gccctgctaa cttttgtatt ttttgtagag
81120

acagggtttc tccatgctgg tcaggctggt cacgaactcc tgacctcagg tgatccacct
81180

gcctcagcct cccaaagtgc tgggattaca ggcatgagcc accgtgccag gccccatccc
81240

tggttattct tattcagtaa aatttgcaac ctcctaggaa gaaaggattt tgaaaacaaa
81300

actctttttt aaaactttta ttttaggttt gggggtacgt gtgaagattt gttacatagg
81360

taaacatgtg tcatcgtggt ttgttgtata tattatttca tcacccaggt attaagccca
81420

gtacccaaca gttgtctttt ctgctcctct ccctcctccc accctcaagc agaccccagt
81480

gtctgttgtt tccttctttg tgttcataag ttctcatcat ttagctccca cttataagtg
81540

agaacgtgtg gtatttggtg ttctgttgct gcattagttt gctaaggatt atagactcca
81600

gcttcatcca tattccctca aaagacatga tcttgttctt ttttatggct gcataatatt
81660

tcatggtgta tatgtaccac agtttcttta gccaatctgt cactgatggg catttaagtt
81720

ggttccatgt cttcactgtt gggagcaagc cccccaaaat ctggccataa tctggcccca
81780

agactggtca taaacaaaat ctctgcagca ctgtaacatg ttcataatgg ccctaacgcc
81840

caagctggaa ggttgtgggt ttacaggaat gaaggaatga gggcaaggaa cacttggcct

81900

gcccagggcg gaaaaccgct taaacgcatt ctcaagctgc aaacaatagc atgagcgatc
81960

tgtgtcttaa ggacgtgttc ctgctgcagt taactagccc aacctattcc tttaatttgg
82020

cccatccctt tgtttctcat aatggatact tttagttaat ttaatatcta cagaaacaat
82080

gataataact ggtttgctgt tactaaatat gtgggtaaat ctctgtttgg ggctctcagt
82140

tctgaaggct gtgagacccc tgatttccca cttcacacct ctatatttct gtgtgtgcat
82200

ctttaactcc tctagcgccg ctgggttagg gtctccccaa ccgagctggt ctcggcactt
82260

cagtattgta aatagtgctg caatgaacac tcatgtggat gtgtctttgt tttttttttt
82320

tttggagacg gagtctagct ctgtcacccg ggctggagta cagtggtgca atcttggctc
82380

actacaacct ctgccacctg ggtttaaatg attctcctgc ctgggcctcc tgagtagctg
82440

ggattacagg tgtgagccac tgtgcccagc taacttttgt atttttagta gagcccaggg
82500

tttcatcatg ttggccaggc tggtcttgaa ctcctgacct caggtgatct gcctgccttg
82560

gcatcccaaa gtgctgggat tacaggcatg agctaccacg cctggcccat gtgtctttat
82620

ggtagaatgc tttatgttcc tctgggtata tactcagtaa tgggattgct gggtcgaatg
82680

gtagttctgc ttttagctct ttgaggaatc gccatattgc ttcccacaat gattgaacta
82740

atttacattc ccagcaacaa tgtgaaaatg ttcccttttc tctgcaacct tgccagcatc
82800

tgtcattttt tgacttttta ataatagcca ttctgactgg tatgagatgg tatctcattg
82860

tggttttgat ttgcatttcc ctaatgacca gtgatgttga gttttttcat atgtctgttg
82920

gccgcctgta tgtcttcttt tgggaactgt ctgttcctct cctttgccca ctttttcatg
82980

gggttgtttg tttttctctc ataaatttgt ttaagttcct tatagatgct agctattaga
83040

cctttgtcag atgcacagtt tgcaaataaa acaaaactct taatgtcttc acttgccaga
83100

tatttgtgct taggttggtg tttgagtctt aggagtaata tcatgttttg ccacaagaag
83160

gagcctatga gtgggctgga agagaaaaat cagatttgca gccgctgaag tcagtaaaca

83220

gtttctttgg atatattgta aatagtaaga gaaccagaca tgacattcat aagtattcca
83280

aaaccaagta aaattatttt aaagctctaa cgttttgcaa cttttcttcc cgtcttaaga
83340

gttcatctgc ccagtctaat gattgtagaa ttcttacttc tagtcttttt tttttttttt
83400

tgagatggag tttcactctt gctgcccagg ctggagtgca atggtgctat cttggctcac
83460

tgcaaccttc gcctcctggg ttcaagccat tctcctgcct cagcttcccg agcagccaca
83520

atttcaggga tgcgccacca cacccggcta attttgtatt tttagtagag ttggggtttc
83580

tccatgttgg tcaggctggt ctcgaacttc tgacctcagg tgatctgccc accttggcct
83640

cctaaagcgc tgggattaca ggagtgagcc actgtgcctg gccatcattt atcttacttt
83700

tagcatcctc atcccaaatt cctggtgtcc ccatccctac catccatttg taataattct
83760

ccattgtttt cctctccttg aatcccactt tctgagatca gtgcaacctg ggcttagagc
83820

ttcaacctcc tccatctgat tttttaaaag agtaactcct aataaggatt ttacccaata
83880

tagtgagcat tatgattaat gaccctgact ctacaaaaaa taatttaaaa aaattagtca
83940

ggtgtggtgg cgcatgcctg tagtcccaga tactcagggt gaggtgggag gattgcttga
84000

gtccaggagt ttgagatcac ggtgagctgt actgcatcat cacattccag cctgggcaac
84060

agagtgagac cctgtttcta aaacaattaa ggtaaaataa aagaaataac atgcacctgg
84120

tacacagtac ttaataaatc ccgtctactg taaataccta gtgcccatca tccctgtaac
84180

tcataacttc ttctttccca gttccccgtg ctttaatcac tcctgcttag gagacagtcc
84240

aggctacctt cttatctctc ttcctggagc cagattctca gcattttgtt ctctaattcc
84300

accctttacc ccttattgca tgccttggta tcagcacttc acctggtctg aggaaccagc
84360

atctagtctt gccctgtgtc tggtagtcca tatggcctgt atgtatcact tagtctgagt
84420

cagtttccac gtgtgtcagc tggattagta gctccttagg gttccgtgta gttctcacag
84480

tctgtggcaa ggaaaacaac aatttgtgag tagctagcat ggactgcaaa ctatgggctc

84540

tatttaccaa gcaagttcta aatgctgaac attgtctttg gcaaaggggt ttcagggaag
84600

atcaagaggc acatcttccc tgccctcagg aaactcatat aggctggttt tgctacactg
84660

aattctcaca actcattgat gcaatgcagc ctagaattaa gttacttgct cattgtcaag
84720

caggtagtga ctagcagagc caccatcctg attctttctt agtttctttt tttttttttt
84780

gagttagggt ctcgctctgt cactcaggct ggagtgcagt ggtgccatca tagctcactg
84840

cagtctcact cctgggctca agcgatcttc ctgcctcagc ctcctaagca gcagggactg
84900

caggtgcaca ccaccatgcc cagctaattt ttaaattttt gtagaggtgg gaatcttgct
84960

cttttgccca gtccggtctg gaactcctgg cctcaagcat cctcctgcct tggcttctca
85020

aagcactgga attacaagca tgagccacca tgtccagccc taagtctgat accttctaag
85080

agggatcatc ctcagatgag ttgggtgtgg aggtgagagg tcaggggtgc ctggataaga
85140

agtgactctg ctatcacttc ctgtgtgagc tgaggcctgg agagaagatg agagaatgat
85200

ggggaaggag gaagcagatg tgttgtgggg ttgggaaatg gggaaatgtc tgtgcatttc
85260

tacttttacc tttgatctct ccttcagggc tttgatattt tcttcctgct aatcctcctt
85320

ttcacacctg cttggggctg ccccatgagc ttgtgaaaac agcatgtttc taccttccaa
85380

ccacagtcct cagtgcattg gccctctctc tccttggcag agtctacgat gtttttcaag
85440

gttaccatga agatttcttt ctttttattt taattttaat tttatttttt tttgagatgg
85500

agtttcgctc ttgttgctca ggctggagtg caatggcacg atctcagctc actgcaacct
85560

ctgcctcccg ggttcaagcg attctcctgc ttcagcctcc caagtagctg ggattacagg
85620

catgtgccac catacctggc taattttgtg tttttagtac agacaaggtt tttccatgtt
85680

ggtcaggctg gtcttgaact cctgacctca ggtaagccac ctgccttggc ctcccaaagt
85740

gctgggatta caggcaggag ccactgtgct tggctttatt ttatttttta agttcctggg
85800

tacaagtgta ggatgtacag gtttgttaca taggtaaacg tgtgccatgg tggtttgctg

85860

cacgtatcaa cccatcacct aggtgttaaa ccctgcatgc attagctctt tttcctaatg
85920

ctctccccta acctacatcc tcccctgaca ggacccagtg tgtgttgttc ccctccctgt
85980

gtccatgtgt tctcattgtt cagctcccag ttataagtga gaacatgcag tgtttggttt
86040

tctattcctg cgttagtttg ctggggataa tggttttcca gcttcatcca tgtccctgca
86100

aaggacatga tctcattatt ttttatgact gcatagtatt ccatggtgta tatgtagcat
86160

attttcttta ttcagtctat catcgatgag cacttgggtt gattccatgt ctttgctatt
86220

gtgaatagtg ctgcaatgaa catatatgtg catgtatctt tataatagaa ttatttatat
86280

tcctttgagg tatataccca gtaatggaat tgctgggtca aatggaattt ctggttctaa
86340

atctttgagg aatcgccaca ctgtcttcca caatggttga attaattcac attcccacaa
86400

gatttctttc ctgactttct gaacttggat cttctcctat cctccatccc caccacctgg
86460

ctccaggatc ccagagactc agccttcctg cattcaaaac catagtggaa gaaggcctga
86520

ttgctggggt gggcagtggc ctgaaacctg gggaggactt cactgggcct aaacattctg
86580

gtgtagagaa agtcactaag taaggacttg tgaaaatgta gaggactttg cggtgggggt
86640

gaactgcaga gtcctactgt ggggcagcaa gaagtcaaga aggaggacct ggcaactggg
86700

gtcaagagct gcccacatgc tcttcatgtc cagccttttt tctcctgtca gctttctagt
86760

gttgcatgct catatgggca gaaagacttg ccattatatg ctagaaagca catggtggag
86820

gctgggcgcg gtggctcact cctgtaatcc cagcacttca ggaggctgag gtgggtggat
86880

cacctgaggt caggagtttg agaccagcct ggccaacatg gtgaaacccc atctctacta
86940

aaatacaaaa aattagctgg gcgtggtggc aggcacctgt aatcccagct actcgggagg
87000

ctgaggcggg agaatcactt gaacctggga ggcagaggtt gcagtgagct gagatggcac
87060

cactgcactc cagcctgggt gacagagcta gactctgtct ccaaaataaa aaaaaaaaaa
87120

agaaagaaag aaagtacagg aaggagaatt tattagaaaa ttcaggcagc ctgggcaaca

87180

tagtgagacc acggcgctac aaaagaatac aaaaaaaaaa aaaaaaaaaa aggctgggcg
87240

cagtggctca agcctgtaat cccagcactg tgggaggctg aggagggtgg atcatgaggt
87300

caggagatcg agaccatcct ggctaacaca gtgaaacccc atctctacta aaaatacaaa
87360

aaaaaaatta gctaggcatg gtggtgggcg cctgtagtcc tagctactcg ggaggctgag
87420

gcaggagaat gacgtcaacc tgggagcgga gcttgcagtg agctgagatc acgccactgg
87480

actccagcct gggtgacaga gctagactcc gtctcaaaac aaacaaacaa acaaaaaaaa
87540

accacacaaa aaaacaaaaa aaattagcca agcatggtgg tgtacacctg ttgtcccagc
87600

tactcaggag gctgaggtgg gaggatcact tgagcctgga ggttgaggtt gcagtgacct
87660

aagatcgtgc tactgcactc cagcctggat gataaagtga gaccctgttt cacaaagaaa
87720

aagaaaaaga gttaaaaaaa tccaggcatg aacaggaagg gatgagcatg gaataaaacc
87780

aggatgctgc ctccgtacca ccagttcttt gatttcaact tgttgctcag ttgaatcatt
87840

gccatactgg tgagaaagaa atgtatttgc tgactgttgg tcaatattac agatttaaaa
87900

cctgtgcact cttaagacct caccagttta tagttttgtt tcttccagat ttctgcggaa
87960

agatgggccc aaggctggct ttgctccctt ttctctaggt cacacagtat cctaggccct
88020

gtgctgttat atttgactct gtggaagatg ctttgttttc agttctgagc cttttcttct
88080

gctgattcct tctcaacctt tgttccttgt gtcgctcacc ctcccccaaa ccaggtagat
88140

gcacccaggt gtggaaaaga gggagggaga agaaagatcc cttctgtcat tgatacagga
88200

gctagaaaaa aattatttag gcagatagtg agggtaagag agtccgtggt aagatttccc
88260

tttaatgaaa agcagccccc aaataatttc ttttctaaca ataagcagcc tgaaaaatta
88320

agctgcaagc atagataagc aagctaaaag cttgcatagg taaatgccaa cagctgtgcc
88380

aatagaaaag ggacacctgg aagccaggta tattcaacat ggaggtgccc tcttcccttt
88440

cctttgtcaa cacgcttgca gtaaaaaagc aggcaacgtg gtgatggcca agtagagacc

88500

ccatctgcat aataaaaggt taaggtgaga tggccaactt gttggtgcgc tatgcaaatg
88560

gcacacctgg tctgactaat ctctcgggcc ctatgtaaat cagacactgc ctcctcaagc
88620

ttgtctataa aacccccccc ccgtgcattt caccacaaaa ccggaagacc cactcggaag
88680

tccctctctc tctgcaggag agagagcttt tcttttttct ctttcttttg cctattaaac
88740

ctccaatctt aaactcctcc ttgtgcgtct ccaccttctt gatttccttg gcgtgaggca
88800

atgaatcttg ggtattactc cagacaaatg acgccacttc atcatcgttc ttggggcaca
88860

gaggtgctga gcttccacat ttctcttcgc tccctactct tgggtgagcc cttcaccctg
88920

ggtaccctgt tgccccaggc cttggcacgt gttgtcaacg tatggctgtg ggaggaggct
88980

ggtcttgtct ggtgggaagg agagagacaa agagggcatt gttgatagaa gaagtggaat
89040

gagagctccc tttacccttg ggggcttctt ttctggcatt aatcctcaga aaataattgt
89100

gaaagagaag tttgtctttg tgctttaggg tgctgtcaga agcttagaaa taattaggca
89160

gaatccccat ccccccaatc cccatcacca actttagtct cctcccccga tacccacact
89220

gacccagggt tagggacatt tataaatgga gccattgagg actcaggaga aactcatgca
89280

actgaaaaat gtcccctatt tcctgggtcc taggcagctc cccactttcc actcccactt
89340

cagacattta gacaaggact ttggtcactc tggctagacc atctgtgtaa cccagaatgg
89400

tggggtcaca actcttggtt tccaaggata taatcacatg ttgtgagcaa aaatgtgctg
89460

aacacacctg actctgtggc ttcttcttga gaagtgattt tgcagagcgg ggaagggtcg
89520

actctggaaa cgtttcattt gtctattggc catttggatt tcttctgtga agtgcctttt
89580

catgactatc atctatattc tcttaagttt gtccttgtta ttgatttgta gttctttata
89640

tatccctgat aacaaggctt gctttttcat gctctttata tatattttta aaagcagctt
89700

tagcaaagtg taacatacaa aaatctgcac ataaaatgta aaatgtgatg ttttgacata
89760

tatatatatg cactgaaacc atccctatta attccattgt gatcaaagac atatattgta

89820

tcacttaaat ccttttacat gtatccaggc ttattttatg gtgtagaaca tgatctatct
89880

tggtagatgt tctgtgtgct cttgacaaga atgtattgtt gcgtagaatg ttctattgtc
89940

aattttgttg attaggtcaa tttggcttat agtattgttc aagccttcta tttgtttact
90000

gattttctgt ctacttgtcc tatcaatttt tgagagatgg gtgttggctc cttaactatt
90060

ttgtgaattt gtctatttct ccatgtattt ctattaattt ttgctgtacg tattttgaag
90120

ctctgttatt acgtgtgtaa acatttagga tttgataaat ggactcattt gtaacaatca
90180

aatatcttct ttatctctgg taatattttt tgctctgaaa tctactttga tattaatata
90240

gtcactgtag gttacttttg attagtgtca gcatggtata ttttccatta ttttactttt
90300

aaccttgtac ctttataatt aaagtgagtt atctttgggc agcatactgt taggtcttgc
90360

tttgtaagtt aatatgttaa tctctgccta taaattggaa tggttagatc atttatattt
90420

aatgtgatga tttgtatggt taggtttaaa cctctcatgt tgctatttgt tttctatttg
90480

tgccatttgt ttgatctgtt ccccctttcc tctttttcta ccttctgttg gattgagcat
90540

atatttatat ataattttat ttttacctcc atgtttgtat tattaactat aacccttttt
90600

gggttatttt aggcttatgg tttatatctt catgttatta gtctacttca attgatatta
90660

tagcactctt cacacattgt aagaacctta cagtatactc ccatttcttc cctcctggct
90720

tttatataat tgttaccata tatcttacct caatatatgc tataaacccc ataatacatt
90780

gttatctttt cgctttaagt gaaagattat ctttgtcttt atctttagag ataaagtggg
90840

gaagggttga ctctggaaac gtttcatttg tccattggcc atttggattt cttctgtgaa
90900

gtgccttttc acgactatca tctatattct cttaagtttg tccttgttaa ttatctttat
90960

caattatctt taaaacgatt taaataataa caaaaataat ctttatattt accctcatag
91020

ttaccacttc tggtgttctt tattctctaa cgtagatcca ggtttctatc tggtttcaaa
91080

ttcttctagc tcaaaggact tcctttacca tttatcatgg tgcatatatg ttttttcatt

91140

cactctggag atgaattctt ccaactgtta tatatcttaa aaagttttaa tttttgcctc
91200

agcttttgaa aaaaaatatt tttgagatag ggtctccctc tgtcacccag gctggagtgc
91260

agtgatgtga tcatagctta ctgcaacctc cacctcccag gctcaagctg tcctcccacc
91320

tcagcctccc aagtagctgg actacaggcg tgtgccacca cacctggata atttttgtat
91380

tttttgtttt gtagagacgg ggtttcacca tgttgcccag gctggtcttg aattcccaag
91440

cttaagcaat ctgcccactt cagcctccca aagtgctaag attacaggtg taagccaccg
91500

tacctggcct gaaaaatatt tttgctgggt atataattct acattggcaa tttattcctt
91560

tgattacttt aaacatgttt ttccactgtc ttctagctta tattgtttcc aacaagaaag
91620

ctactgtcat tcttatctac tgttggtgcc ctgtctagat gtccttttct gggctggcat
91680

gcccactcac aggctgttgt taatggtggc tattattggc tcatgaactc tctccttttc
91740

taaatagatg gcttggccgg gcatggtggc tcacgcctgt aatcgcagca ctttgggagg
91800

ccaaggcagg cggatcacga ggtcaggaga tcgagaccat cctgactaac acggtgaaac
91860

tctgtctcta ctaaaaagac aaacaactag ccgggcatgg tggcaggcgc ctgtagtccc
91920

agctactcgg gaggctgagg caggagaatg gcgtgaaccc gggaggcgga gcttgcagtg
91980

agccgagatc atgccactgc actccagcct aggtgacaga gtgagactcc atctcaaaca
92040

aaaaaaataa aaataaaaaa aagagatggc ttcacccggg ataatatgat ttcttcctct
92100

ccccgatggc atcccaatgc caatgactga atgttattgg agtacaaaag aacagccctt
92160

ttacctcaag tggataaaat ctgtggtatg atttatgctt cagggtcccc tgtggacaag
92220

atcaagttca aaccttacct gagatcacat ttggcacagt tttttttttt tttacattac
92280

cttttctgct tcctctattt ccttagagat ttttactggt gagtccaacc taaaaaatca
92340

catgtattca agtctctttt aagttctgct tctaaggaat caaacctaag acagtgggta
92400

ccaggagcgg tcctaggaag cacaacataa gaatgggatt ctagggctgg gttacttgtc

92460

agccctctgg caagggggac agccatgttg atggcaggca tagtttctga cttgctttag
92520

cagtgcaagt gttaagactt ttactcggaa taactaggct aggacacagg tggaattaga
92580

ttcactggct gatgtaatgt gtctggcatt taagagatat aagacaaatt gcaagtataa
92640

gggctatgga gccaggtgtg gtggctcacg cctgtaatct tagcactttg ggaggctgag
92700

gcaggtggat catctgaggt caggagttca agaccagcct ggccaacatg gtgaaacccc
92760

atctctacta aaaagtcaaa aattagccag gcatggtggt gggcacctgg aatcccagct
92820

actcgggagg ctggggcagg agaatcactt ggcggaggtt gcagtgaact gagatcacgc
92880

cattgcactc cagcctgggt gacaagagtg agactctgtc tcaaaaataa aataaaacaa
92940

aacaaaataa aataaaataa aggactatgg aattaggtgg ctattactga gcttcagtga
93000

tgccttaaag agaaaaaaat gaaaggctca ggacaataag taatcaattt aaagcaaaat
93060

ataagagtta caatgccttc ttggcaacat ttaaagagac ccttatctct cgttgcctga
93120

aggcagagag accaggctga atatttaact ttaagagtgg cataactaca gagaagttca
93180

aatttttcag cctagggaag tttctagtac caaaaccagg acctcaatag gaaagaataa
93240

gaccctgaga catgggatgg tgatattaga tagatgccct ttgaggtcct tggatcatca
93300

gattattctg gaccatttgg acctgcagaa gtggctcaca ctttttattg gaagatagag
93360

gcccactctt gcttgaaaac cttgcaaaag tcttacacta gacaaattca ttgtaaggaa
93420

atgctggctc ccctcaggat gtgttgcacc tcccccttttg gccactagac ccataacaaa
93480

aatcaaatct tagcatggcc caactgggga agtacttgac ttactaagga gagggatgga
93540

ttagatacta aaggagcttc aagacttgga caacatgtgt catcaggagc aaggaaaata
93600

aatcagagag aagattctga agatcctgga tcaaaggagg gggactaaaa atagaaagct
93660

ggatgtagga gtttgtcaat atagggcatt cttatgggac acaggattta acaccctagc
93720

aagtttattc ttaggaggtt gatatgaaat gatactgggg tggcttttgg aaagctggaa

93780

aacaacaaga gcttctatta ataaagttga gatgtcagaa ctcatatgga aaattgtgag
93840

taaagggttc acagttctca gagaaatgga tgtgggagaa tagatctata acaaaagatt
93900

ggaaaatcca ccaccagact ctattccttg ggaagtcctg gataatactc attttcacca
93960

aagaaataat gaatgtgctg gtgatgagga gtttagaagc tgtaagaatc tcagtagtgt
94020

tcatcctctg tagacggagg ctgacagcag atgcccttat atactggata tactggttcc
94080

ctaatagcaa taggaatgat aggattctga atcagcagaa gctatttggc agcacttaat
94140

aatcagaagc aaagtagtta ttattattat aataatcagg aaggttgaaa ttatggccaa
94200

agggccttga ccttcaggga tctgtggaga tggctaatag aaaatagtgt ttccatggag
94260

aaaatatacg gttgcaaatg catcacattt gctatcaaca aactccaagt aggataaact
94320

caaagacatc catacttagg tacatgatga tcaaactgtc aaaagtcaaa gaccagataa
94380

aacaacaaca acttaagaga gaatcttgaa agtagcaaga gaaaagcaac tcattaagga
94440

caaaggatcc tcaataagtt taacagctga tatctcctca aaaccatgga ggccagaaga
94500

cactggaacg atatactcaa aatgcagaaa gaaaagggct gtcagtcaag gattctacaa
94560

ccagcaaaag tatccttcaa aaatgaagga ggaattgaga caattctaga taataaaaac
94620

tgagagaatt tattattagc ataactgccc tacagaaaat actaagagaa gtcctacaga
94680

ctgaaatgaa agaatgctag taagagatca tatgaagaaa taaagaacag ttgcaaaggt
94740

aatgacatag gtaaacataa aagacatata caaaactgaa agctttcctc ctagaagaag
94800

gaacaagaga aggatgtgtg atcttatcac acctattcaa caacacctct agctagtact
94860

ggcggttcta tttagggcaa ttaggcaaga aaaaaagaca tctagattag aaaggaagaa
94920

ataaaacttt gtttgaaaat gacataatct tgtgtagaga aaatcctaag gaacccacaa
94980

cagcttacta taattaataa attagttcag ccacatcaca gtatacaaga tgagtatatt
95040

aaaatcaatt ctatttctat acactagcaa taaacaatac aaaaaggaga atgagaaaac

95100

aattcaattt acaatagcat aaagaagaac aaaatactta gaaataaatg taatcaagga
95160

agtgccaggc ttctacacag aaaacttcaa gacattgttg aaagaaatta aagaaaacat
95220

aaataatata tattccatgc ttatggatta gaagatttaa tgttgttaac atggtggtgc
95280

tctcaaattg ataaaaaaat tcattgcaat ccttataaaa attccagttt cctttttga
95340

agaaatagac aaacagatcc taaaattcaa acagaattac aaaagaccca aagtagataa
95400

aaaatattga aaaagaagaa caatatacat aaacataaat tcaatcagtg gaatagaatt
95460

gaaagctggg aagcaaattc ttaaatttat ggccaattta tttttgataa gagtgtcaag
95520

acaattcagt cgggaaagaa cagtcatttc aacaaattgt gctgggacaa ctggatatgc
95580

atatgcaaaa gaatgagatt acctcacaac atacacagaa attaactcaa ggctgggcat
95640

ggtggctcac acttgtaatc ccagcacttt gggaggtcaa ggtgggcaga tcgcttgagg
95700

ccaggagttc tagacaagcc tgggcaatgt ggagaaaccc tgtctctaca aaaactacaa
95760

aacttagctg ggcatgatgg tgcacacctg tagtctcagc tgtttggggg gctgaggtgg
95820

gaggatcact tgagcctggg aaattgaagc tgcagtgagc cgtgttcata ccactgctct
95880

acagctgggt gacaaagtga gaccctgtct caaaaaaaaa aaaaatccat catttattaa
95940

gcactgaata atgttagtca aacaggatgt taaactggaa attttgattg ggatttcgag
96000

atgcaatgtt tgggaaatac aacacatatc atattccaaa gtgatttgct ggtctgagat
96060

gcagcaattc aaaggaaaag caaataaata aataaataaa taaataaata aataaaggtt
96120

aagctactct taaaatctac acccacatca aagttgtata aaatggcaga gttgttggaa
96180

attacaggga gagagaatta actttggaga gaagatatct ttgttttttt tttttaaatt
96240

tattttcttt ttttaggcta gttgagtgaa gcagtgggag tagagaagga acaaagacat
96300

ttgtaactgg ttgtgataaa ttgtttgaaa acagcactgc actttgacca gccaagagag
96360

aagatctttt tggagagagg tgtgctgtga gttcttctcc ccaagagcaa gggctaaggg

96420

tgctgccctt ggaatcttaa gaccatcagc agatgaatgg aagacgttgg aattggcctg
96480

catgcccagg ttataaagtg tgatatggcc gggcgcagtg gctcagcctg taatcccagc
96540

aatttgggac gctgaggcgg gtaggtcact tgaggccaag agtttgagac cagcctggcc
96600

aacatggtga aaccccatct ctactaaaaa tataagaatt agccgggcat ggtggcatgt
96660

gcctatagtc ccagctactc gggaggctga ggcaggagaa tcacttgaac ctgggaggtg
96720

gaggttgcag tgagctgaga ttgcgccact gcactccagc ttgggtgaca gagtgagatt
96780

ccatctcaaa aaaaaaaaaa atagtgtgat atgatcactt tacacaaaac tagtctgtga
96840

caagtggata aaaatagtat ttcatcacta ttttactatt tctttgattc ctacctggac
96900

taaacattta caggctttga ataacctttt ctatgaatgg tcttttcatg tctttgctaa
96960

gtttacgtac cggtgcactt agaattatct tataatttgt ataagctctc aatataattt
97020

tagtaagatg cttttttgat atgctatttt gtgctatttt tttcctttgc tttagagaga
97080

ggtgccaaga aaatttctct ttggacctta gtttctctta ggatgctctg caggctgctc
97140

cattctggga atcataggaa ggttgcaagg cttcccagag gcacttagga tagtcccctt
97200

tcagtcaaga agcttcactg gctggagaat gttctcaaga ccccctcctt gaatccatgg
97260

agagcaccag agaattagga catcttagct gctgcaggga ccacagtcct gcagagccaa
97320

ttgctggcag cagatatttt ggacaagaag gtactagagt cctggagaga gtctgaggaa
97380

aggaaggcca tctgggaata ggattttggg tggtggacgt ctgtttcagt cctgggttat
97440

gaggatgttt tgcacccagg aagtggttgt tctcatgaat agaaaggact attgtgactg
97500

agcctcatgc cttagtgagg ggcagtatct ggaaccgagg tgacatggtt tggctgtgtc
97560

cccacccgaa tctcatcatg aatttccaca tgttgtggga gggacctggt aggaagtaat
97620

tgaatcatgg gggcaggtct ttcctgtgct gttctcgtga tagtgaataa gtctcaggag
97680

atctgatgtt ttcataggtt tttttttctg cacaagctct ctcttggcct gctgccatcc

97740

atgtgagata tgacttgctc ctccttgcct tcaccatgat tgtgaggctt cctcagccac
97800

gtggaactgt aagttcactg aacatctttc ttttgtaaat tgcccagttt caggtatgtc
97860

ttcattagca gcgtgaaaat ggactaatac atgaggttgc ctctggcatc tcctctgtga
97920

agagtccagc attgaccctg agcactgtca cttcgaacac aggagaccag tccctctctg
97980

cacactccct cagccctgtg agttactctc ctcactttgc tgcttcccat tcttggcggt
98040

atctctggag agactcctgg ctctagcagt ggggctagga atggccatgg ggacaggcgt
98100

cacagttctc cctgggcctg acggagaggg gtctgggttc tctggattcc tgaagggacg
98160

tggagcagat tcccatcatg ctgtggcggg gagtctgtgc tattctccca gccaagagga
98220

gaggacatcc cgaaatcacc acaaattctg ggatgaaggc tgtctgagga aatgaagcaa
98280

acacacacaa acacacaaag acatacaaat acacactcac acttatacat acgtgcacac
98340

acacattctt ctgtgggtaa gcttgtgctt cagctacttt gggcaggaga gcaggggggt
98400

tttattctac ctgtcattgc cttcatgccc cagtgctgga aacacactga agccattgct
98460

cccccttcag atgatctgaa aatccctgtt tcagttcact ttgaaaacac ccctcactct
98520

caacaacaga gggttggtta ttctgcagaa ctcattcttt aacagcaggc ctgctgctgc
98580

tccagctgca attcttttcc ccttcatgaa ggctgccagg aatgaaaagg ggagggaaaa
98640

gaagtaggtg gtggcaggag gctgggccag ggaagtgtgg gaccccacag gctccagcct
98700

gaggactcct ctctcggcct ctctcccctg ttgtgaatgc ttctgctgaa agccactggg
98760

tgagtgcagg gacagagagc cactgagctg gctcagggcc tcgtggtggt ggtggtggtg
98820

gtaggggggc tgctctgggt tctcgagagg cttctccaac tctgacatga tcatacctga
98880

ggatattatc gaaattcaga tatgacttag gaggcctggg atacggcccg agattctgca
98940

tgcctggtga tgccagtgtt gctgctctgt tgccacactg gataaaagca tctagaaggt
99000

attccaggcc agatggggtg ggcattccct ccctcatacc ttaactcttt gacatggatt

99060
ctgccaccaa cagaatcctg gacgttttct cccaagagat gataagaaaa attcacacca
99120
aaatcatgat ggaatttcaa aatctgtttg gatattagaa aatttctcca aaccctggcc
99180
tttccttcct ctcccaactc ttgagctcac aggtgttgct tcactgacta aaatcctgac
99240
agagctccaa ttctactacc tcccagacaa ctttatttgc aagctgtttc tctactcaga
99300
aacaggggtg tcccttcagc cgagcttttc caacaatgct gagctggggg ctttggtaca
99360
tctccccctc cttctcagcc tcagctctct aggtcctgaa gtttgacctg ggggccctgt
99420
ccaccgtggg gcaaagctgc agacttgttg atgagaggag gatgtgggtc ttagggaaag
99480
agaaaggtga gctggggaca gctggaccag attggagaga ccacagggtc cctgaaaagc
99540
agggggtaag gggtaggagg gctatcatat agggaggtcc ctttggtatg cagactttgg
99600
gacagaaagc acagttttgt ctgaaccctc cagaaggccc tgaaagcatg ggacataagc
99660
attgtgttgg cgataacagc tgcaggtgaa ggcaggtgaa tgacaaggaa gggtgcttag
99720
ggttagatat gagagctcca aaccaagggg ccctgggcag gtccttttgt tctactgttg
99780
cccctctctc ctttccaaaa ccccaaagct ctgtggtgag ctctggagac ctcagctttg
99840
ttctgagagc ctctgagagg atttaggtgt ctcttcatgg gcctggggtg ggaatgggag
99900
agacagaaag aaaggggaca ctaggtacgg cctctggaaa tgatttgaat ccctgaccct
99960
tgaaatatgt cagacccacc ctgtgtgaaa ttccacgtct gctggaaatg tgacggaaat
100020
gtgtttagag ggtgctggac ctgcacttcg aaataaaaca aggctgacag caagacttga
100080
gaatttacct aatgatc
100097

<210> 166
<211> 2222
<212> DNA
<213> Homo sapiens

<400> 166
cgaggcacaa tttgttagta ggtttgcata gatataacac cttttcactg gtataataca
60

tcttttccaa aggattatgg caggcttgtt tctattcttc atgtatattc tctttttcac
120

tctttttttc tgttttacat gaaattaacc atatttatca tctggctacc atagttccaa
180

aataaggtcc agtagtagtt tgaagcacag caagctcagc agtttcttcc ctttttaaag
240

cccgacagtc agtggagttt tctatagtcg agccacgtga gcactgtcac cccaagtaga
300

gactcttgtg aactcaaacg gctccagatt cacggtcaag aaccttggtg acttcacata
360

actaccactt attgctggaa gggagacaat attgtttgct tctgaagttt tcaaacagtt
420

tctcacctgg cagcttctcc ggatttcttt gttgctttca taatcgcttc tgacattgcg
480

ctttcaaaaa tctaaaggtc gaatcatcat agttgtggaa cgtaaggagt aactgggccc
540

tttgaagtag tgccacttta tcccattcag ttttccatgg ttttgtcccg cagtatagaa
600

cattccattt agattggagg ggccacaagc atcaaaccac catcctcctg ttaacatgag
660

ggcacatttg cacatacagt tgtcattatc agcatcttta gtgctgaaat cagcaccgtg
720

taagatcagg ctgctctgtt ttcctgctgt cccagtgtga ccttttaaat acaacctata
780

gttttgcttt tcatttccta tgtggaatct gtcatactgt gaataggctc ggttcccttc
840

ccagtccatt aactctcgtg ccgaattcgg caccagctga tttgtttcta tcttcctttc
900

agcatgtcct gttttctaat acaataaaat gcttcgggtt tatcttgtac ttccctttcc
960

aattctggaa taaccattta tccaaggaac tccgattcct ttggggagtg ctatttggaa
1020

accaaggtct ggcactctag gtatgctcgt agcgatggga gcgtcattgc ttccaggctc
1080

tggctgtgtc tcttcagggt ccactatagt catcctgttt ctgtctgctg tagattatat
1140

taaagcttca ttcttcatcc cttccatttt cctccctata agttagtgtc tggaagcatt
1200

cttagaggtc agacgaaatc attatattca ggtatggaat gcaatttttg tatagataac
1260

aaatctatat tcatgaaatc tgatgctgcc catcatccat ctttttgcaa ggtcttttcc
1320

tgtgaaaggt atgttttttc ttttcttttc tttttttttt ttttttgaga cagagtctct
1380

ctctgtcacc taggctgaag tgcagtggca cagtctcggc tcactgcaac ctctgccttc
1440

cgggttcaag cgattctcct gcctcagcct cctgagtagc tgggattaca ggcatgcgcc
1500

accatgcctg gctgattttg tatttttagt agagttgggg tttctccatg ttggtcaggc
1560

tggtctcgaa ctcccaacct caggtgatcc acccacctca gcctcccaaa gtggtaggat
1620

tacaggtgtg agccaccgtg cccggccagt cctttacttt tataggtgga gaaactgaga
1680

cctaagaact gaagtgactt acgtccatgg caaattgatt gcagagccaa aagtggagtc
1740

cagatctcct aacttccata ctagaacatt tttgctgtgg ggagtggaga gagattgggt
1800

tgatggtacc tgcctgaagt ttccatggtt gctgggtgga aacagcactc ttccttctgt
1860

cttggcactc aagcatcccg atggacattg aatttttcca atgattggtc atttgatgac
1920

caggcagggg agcagaaaag gagatagtac agaccagaag ctgtttgagg atgtgttgtt
1980

gcctgaccag acaccttgag gccagaattc taagtagttt caaaactcct ccctttcata
2040

ccccatctac tgactttctg ctttcttcaa aatgctctgt tggccaggtg cagtggttca
2100

cgcctgcaat cccaacactt aggctgaggc gggaggatta catgggccca ggagttcaag
2160

accagttgag gcaacataag gggctgggcc cttttctcta cgaaaaaaaa aaaaaaaaaa
2220

aa
2222

<210> 167
<211> 1996
<212> DNA
<213> Homo sapiens

<400> 167
atccccagca agcaccaggg cccgtgtgac caggccccgt ccccatgcct cggggtgcag
60

tgtgcatttg gggcgacgtg tgctgtgaag aacgggcagg cagcgtgtga atgcctgcag
120

gcgtgctcga gcctctacga tcctgtgtgc ggcagcgacg gcgtcacata cggcagcgcg
180

tgcgagctgg aggccacggc ctgtaccctc gggcgggaga tccaggtggc gcgcaaagga
240

ccctgtgacc gctgcgggca gtgccgcttt ggagccctgt gcgaggccga gaccgggcgc
300

tgcgtgtgcc cctctgaatg cgtggctttg gcccagcccg tgtgtggctc cgacgggcac
360

acgtacccca gcgagtgcat gctgcacgtg cacgcctgca cacaccagat cagcctgcac
420

gtggcctcag ctggaccctg tgagacctgt ggagatgctg tgtgtgcttt tggggctgtg
480

tgctccgcag ggcagtgtgt gtgtccccgg tgtgagcacc ccccgcccgg ccccgtgtgt
540

ggcagcgacg gtgtcaccta cggcagtgcc tgcgagctac gggaagccgc ctgcctccag
600

cagacacaga tcgaggaggc ccgggcaggg ccgtgcgagc aggccgagtg cggttccgga
660

ggctctggct ctggggagga cggtgactgt gagcaggagc tgtgccggca gcgcggtggc
720

atctgggacg aggactcgga ggacgggccg tgtgtctgtg acttcagctg ccagagtgtc
780

ccaggcagcc cggtgtgcgg ctcagatggg gtcacctaca gcaccgagtg tgagctgaag
840

aaggccaggt gtgagtcaca gcgagggctc tacgtagcgg cccagggagc ctgccgaggc
900

cccaccttcg ccccgctgcc gcctgtggcc cccttacact gtgcccagac gccctacggc
960

tgctgccagg acaatatcac cgcagcccgg ggcgtgggcc tggctggctg ccccagtgcc
1020

tgccagtgca acccccatgg ctcttacggc ggcacctgtg acccagccac aggccagtgc
1080

tcctgccgcc caggtgtggg gggcctcagg tgtgaccgct gtgagcctgg cttctggaac
1140

tttcgaggca tcgtcaccga tggccggagt ggctgtacac cctgcagctg tgatccccaa
1200

ggcgccgtgc gggatgactg tgagcagatg acggggctgt gctcgtgtaa gcccggggtg
1260

gctggaccca agtgtgggca gtgtccagac ggccgtgccc tgggccccgc gggctgtgaa
1320

gctgacgctt ctgcgcctgc gacctgtgcg gagatgcgct gtgagttcgg tgcgcggtgc
1380

gtggaggagt ctggctcagc ccactgtgtc tgcccgatgc tcacctgtcc agaggccaac
1440

gctaccaagg tctgtgggtc agatggagtc acatacggca acgagtgtca gctgaagacc
1500

atcgcctgcc gccagggcct gcgaggggct atcgagagga gctcactgtg ggatggggtt
1560

gacctctgcc gcctgcctgg gtatctgggc ctggccatgg ctgtgttctt catgtgttga
1620

ttttatttga cccctggagt ggtgggtctc atctttccca tctcgcctga gagcggctga
1680

gggctgcctc actgcaaatc ctccccacgg cgtcagtgaa agtcgtcctt gtctcaggat
1740

gaccaggggc cagccagtgt ctgaccaagg tcaaggggca ggtgcagagg tggcagggat
1800

ggctccgaag ccagaaatgc cttaaactgc aacgtcccgt cccttcccca cccccatccc
1860

atccccaccc ccagccccag cccagtcctc ctaggagcag gacccgatga agcgggcggc
1920

ggtggggctg ggtgccgtgt tactaactct agtatgtttc tgtgtcaatc gctgtgaaat
1980

aaagtctgaa aacttt
1996


<210> 168
<211> 1996
<212> DNA
<213> Homo sapiens

<400> 168
atccccagca agcaccaggg cccgtgtgac caggccccgt ccccatgcct cggggtgcag
60

tgtgcatttg gggcgacgtg tgctgtgaag aacgggcagg cagcgtgtga atgcctgcag
120

gcgtgctcga gcctctacga tcctgtgtgc ggcagcgacg gcgtcacata cggcagcgcg
180

tgcgagctgg aggccacggc ctgtaccctc gggcgggaga tccaggtggc gcgcaaagga
240

ccctgtgacc gctgcgggca gtgccgcttt ggagccctgt gcgaggccga gaccgggcgc
300

tgcgtgtgcc cctctgaatg cgtggctttg gcccagcccg tgtgtggctc cgacgggcac
360

acgtacccca gcgagtgcat gctgcacgtg cacgcctgca cacaccagat cagcctgcac
420

gtggcctcag ctggaccctg tgagacctgt ggagatgctg tgtgtgcttt tggggctgtg
480

tgctccgcag ggcagtgtgt gtgtccccgg tgtgagcacc ccccgcccgg ccccgtgtgt
540

ggcagcgacg gtgtcaccta cggcagtgcc tgcgagctac gggaagccgc ctgcctccag
600

cagacacaga tcgaggaggc ccgggcaggg ccgtgcgagc aggccgagtg cggttccgga
660

ggctctggct ctggggagga cggtgactgt gagcaggagc tgtgccggca gcgcggtggc
720

atctgggacg aggactcgga ggacgggccg tgtgtctgtg acttcagctg ccagagtgtc

780

ccaggcagcc cggtgtgcgg ctcagatggg gtcacctaca gcaccgagtg tgagctgaag
840

aaggccaggt gtgagtcaca gcgagggctc tacgtagcgg cccagggagc ctgccgaggc
900

cccaccttcg ccccgctgcc gcctgtggcc cccttacact gtgcccagac gccctacggc
960

tgctgccagg acaatatcac cgcagcccgg ggcgtgggcc tggctggctg ccccagtgcc
1020

tgccagtgca acccccatgg ctcttacggc ggcacctgtg acccagccac aggccagtgc
1080

tcctgccgcc caggtgtggg gggcctcagg tgtgaccgct gtgagcctgg cttctggaac
1140

tttcgaggca tcgtcaccga tggccggagt ggctgtacac cctgcagctg tgatccccaa
1200

ggcgccgtgc gggatgactg tgagcagatg acggggctgt gctcgtgtaa gcccggggtg
1260

gctggaccca agtgtgggca gtgtccagac ggccgtgccc tgggccccgc gggctgtgaa
1320

gctgacgctt ctgcgcctgc gacctgtgcg gagatgcgct gtgagttcgg tgcgcggtgc
1380

gtggaggagt ctggctcagc ccactgtgtc tgcccgatgc tcacctgtcc agaggccaac
1440

gctaccaagg tctgtgggtc agatggagtc acatacggca acgagtgtca gctgaagacc
1500

atcgcctgcc gccagggcct gcgaggggct atcgagagga gctcactgtg ggatggggtt
1560

gacctctgcc gcctgcctgg gtatctgggc ctggccatgg ctgtgttctt catgtgttga
1620

ttttatttga cccctggagt ggtgggtctc atctttccca tctcgcctga gagcggctga
1680

gggctgcctc actgcaaatc ctccccacgg cgtcagtgaa agtcgtcctt gtctcaggat
1740

gaccaggggc cagccagtgt ctgaccaagg tcaaggggca ggtgcagagg tggcagggat
1800

ggctccgaag ccagaaatgc cttaaactgc aacgtcccgt cccttcccca cccccatccc
1860

atccccaccc ccagccccag cccagtcctc ctaggagcag gacccgatga agcgggcggc
1920

ggtggggctg ggtgccgtgt tactaactct agtatgtttc tgtgtcaatc gctgtgaaat
1980

aaagtctgaa aacttt
1996

<210>  169
<211>  1141
<212>  DNA
<213>  Homo sapiens

<400>  169
tccttgggtt cgggtgaaag cgcctggggg ttcgtggcca tgatccccga gctgctggag
60

aactgaaggc ggacagtctc ctgcgaaacc aggcaatggc ggagctggag tttgttcaga
120

tcatcatcat cgtggtggtg atgatggtga tggtggtggt gatcacgtgc ctgctgagcc
180

actacaagct gtctgcacgg tccttcatca gccggcacag ccaggggcgg aggagagaag
240

atgccctgtc ctcagaagga tgcctgtggc cctcggagag cacagtgtca ggcaacggaa
300

tcccagagcc gcaggtctac gccccgcctc ggcccaccga ccgcctggcc gtgccgccct
360

tcgcccagcg ggagcgcttc caccgcttcc agcccaccta tccgtacctg cagcacgaga
420

tcgacctgcc acccaccatc tcgctgtcag acggggagga gcccccaccc taccagggcc
480

cctgcaccct ccagcttcgg gaccccgagc agcagctgga actgaaccgg gagtcggtgc
540

gcgcaccccc aaacagaacc atcttcgaca gtgacctgat ggatagtgcc aggctgggcg
600

gcccctgccc ccccagcagt aactcgggca tcagcgccac gtgctacggc agcggcgggc
660

gcatggaggg gccgccgccc acctacagcg aggtcatcgg ccactacccg gggtcctcct
720

tccagcacca gcagagcagt gggccgccct ccttgctgga ggggacccgg ctccaccaca
780

cacacatcgc gcccctagag agcgcagcca tctggagcaa agagaaggat aaacagaaag
840

gacaccctct ctagggtccc caggggggcc gggctggggc tgcgtaggtg aaaaggcaga
900

acactccgcg cttcttagaa gaggagtgag aggaaggcgg ggggcgcagc aacgcatcgt
960

gtggccctcc cctcccacct ccctgtgtat aaatatttac atgtgatgtc tggtctgaat
1020

gcacaagcta agagagcttg caaaaaaaaa aagaaaaaag aaaaaaaaaa accacgtttc
1080

tttgttgagc tgtgtcttga aggcaaaaga aaaaaaattt ctacagtaaa aaaaaaaaaa
1140

a                                                                 1
141

<210> 170
<211> 2732
<212> DNA
<213> Homo sapiens

<400> 170
aggagccatc ctgcccgcgg ggatcccgtc agccgcctta tgatctcctc attgcgtccc
60

cctctgccca ctggacttgg acttcagatc tgaccccaga cctgccggct acctcgggag
120

ggcccacctc cccgcccatc cagcaagatg ccaatcctca agcaactggt gtccagctcg
180

gtgcactcca agcgccgttc ccgagcggac ctcacggccg agatgatcag cgcccccctg
240

ggcgacttcc gccacaccat gcacgttggc cgggccggag acgcctttgg ggacacctcc
300

ttcctcaata gcaaggctgg cgagcccgac ggcgagtcct tggacgaaca gccctcttct
360

tcatcttcca aacgcagtct cctgtccagg aagttccggg gcagcaagcg gtcacagtcg
420

gtgaccaggg gggagcggga gcagcgtgac atgctgggct ccctgcggga ctcggccctg
480

tttgtcaaga atgccatgtc cctgccccag ctcaatgaga aggaggccgc ggagaagggc
540

accagtaagc tgcccaagag cctgtcatcc agccccgtga agaaggccaa tgacggggag
600

ggcggcgatg aggaggcggg cacggaggag gcagtgcccc gtcggaatgg ggccgcgggt
660

ccacattccc ctgaccccct cctcgatgag caggcctttg gggatctgac agatctgcct
720

gtcgtgccca aggccacgta cgggctgaag catgcggagt ccatcatgtc cttccacatc
780

gacctggggc cctccatgct gggtgacgtc ctcagcatca tggacaagga ggagtgggac
840

cccgaggagg gggagggtgg ttaccatggc gatgagggcg ccgctggcac catcacccag
900

gctcccccgt acgccgtggc ggcccctccc ctggcaaggc aggaaggcaa ggctggccca
960

gacttgccct ccctcccctc ccatgctctg gaggatgagg ggtgggcagc agcggccccc
1020

agccccggct cagcccgcag catgggcagc cacaccacac gggacagcag ctccctctcc
1080

agctgcacct caggcatcct ggaggagcgc agccctgcct tccgggggcc ggacagggcc
1140

cgggctgctg tctcaagaca gccagacaag gagttctcct tcatggatga ggaggaggag
1200

gatgaaatcc gtgtgtgagg cggacagtgg gtggccaccg ggagctcttg gctgcatctc
1260

ctccctgccc ccaccccact atgacctttg accctacggc gcaggggcag ccaggaccct
1320

tgattcagac catggaccct ggaccttgta gatgagggac actggcctgg ccctcgggtc
1380

ttcggaggac gtagggggct ggcatgggtg ccgactggct gcctgacttc atcacgctcc
1440

ctgcacttag gctgcgtggg acaagggctg tgttgtcaca gcaggaatag gttttcctct
1500

gttggcctcc cttttcctcc accctggcct caaatggatg ccagatgcca accccagttc
1560

tggccacgta cagccagcgg gtcagcccag aggcagcctc agctccaggg ctaaggactc
1620

tcggctccca ttttctctgc tggcgtttct gctgtgccca gcagtggctg ctggggaagc
1680

agctgcagca ggagggagac ggtcttgcct ctcagcccct ccctgcccca ccccagctcc
1740

tgccctggaa atctggagcc ccttggagct gagctggacg gggggccagc tgcgagcatg
1800

tgcactaaac gcagcccttt ccaggggaag agagcaggat ggagaatgga aggaaagccc
1860

cccaggcttc gtgaattgca agaagggacc ctggtgtgtg tgttttccct acgcacctcc
1920

catagctatt aacaactgag gaaggccagt gcagaatatt tttggagaac gatttttttt
1980

ttaaataata tatcattcct atggggggaa agcctttttt ttctttttgg ctgagttatt
2040

ccctccctcc cctcaatacc ctcagtactg actacttccc tttcttttct caggcctccc
2100

cccaccgact tttgaggcca gggttggcca gatttagcaa aaccaaaaca gagtgctgag
2160

ttaaacgcaa atttcaggta gacaaaagat aattttctag cattaatatg ccccacgcaa
2220

tatttggaac acttatgtga aaaatgattt gtttttctga aatttacgtt tctctctgag
2280

tcctgtaact gtccccgagg ggattgagca gaagctcggg tatgagcccc tgaggttgac
2340

tgccggttat ttttctgtcc tgggaacagc ctgacccacc tccctgtctc catgtagcca
2400

gtggggggag ggggagacac agaacctacc acagccaggg gcgtccccat ggcgactgtg
2460

gcccggcccc tcctctcttg cctgactctc ctctcttgcc tgactctaga cactaactta
2520

gttccaggtt cggtgccctg ttggtgctcc tgtttccaat agcttaggtc ccatggtggg
2580

ggaggaacct caggggctat gcagcccccg ccagctgccc tcaaatcccg tccaggccag
2640

ttccagattc taaactgatt tttttcatga tattgtcaaa acagtgagga aacattaaaa
2700

aaaaagccct aaagcaaaaa aaaaaaaaaa aa
2732

<210> 171
<211> 998
<212> DNA
<213> Homo sapiens

<400> 171
gcggcgggcg cagacagcgg cgggcgcagg acgtgcacta tggctcgggg ctcgctgcgc
60

cggttgctgc ggctcctcgt gctggggctc tggctggcgt tgctgcgctc cgtggccggg
120

gagcaagcgc caggcaccgc cccctgctcc cgcggcagct cctggagcgc ggacctggac
180

aagtgcatgg actgcgcgtc ttgcagggcg cgaccgcaca gcgacttctg cctgggctgc
240

gctgcagcac ctcctgcccc cttccggctg ctttggccca tccttggggg cgctctgagc
300

ctgaccttcg tgctggggct gctttctggc tttttggtct ggagacgatg ccgcaggaga
360

gagaagttca ccacccccat agaggagacc ggcggagagg gctgcccagc tgtggcgctg
420

atccagtgac aatgtgcccc ctgccagccg gggctcgccc actcatcatt cattcatcca
480

ttctagagcc agtctctgcc tcccagacgc ggcgggagcc aagctcctcc aaccacaagg
540

ggggtggggg gcggtgaatc acctctgagg cctgggccca gggttcaggg gaaccttcca
600

aggtgtctgg ttgccctgcc tctggctcca gaacagaaag ggagcctcac gctggctcac
660

acaaaacagc tgacactgac taaggaactg cagcatttgc acaggggagg ggggtgccct
720

ccttccttag gacctggggg ccaggctgac ttggggggca gacttgacac taggccccac
780

tcactcagat gtcctgaaat tccaccacgg gggtcaccct ggggggttag ggacctattt
840

ttaacactag gggctggccc actaggaggg ctggccctaa gatacagacc cccccaactc
900

cccaaagcgg ggaggagata tttattttgg ggagagtttg gaggggaggg agaatttatt

960

aataaaagaa tctttaactt taaaaaaaaa aaaaaaaa
998

<210> 172
<211> 4848
<212> DNA
<213> Homo sapiens

<400> 172
gccggccgaa gcgtggcggc cacagactgt gggtaccggg tccgagggac tcgcgctttt
60

ctctccgtgc catggcgcca gcgaaagcca cgaacgtggt gcggctgcta ctaggctcca
120

cagcgctgtg gctttcgcag ctcggctccg ggacggtcgc cgcgtccaag tcggtgactg
180

cccacttggc cgcgaagtgg cccgagaccc cgctgctgct ggaggcaagt gaatttatgg
240

cagaagaaag taatgaaaaa ttttggcagt ttttggaaac tgtgcaagaa ttagcaattt
300

ataagcaaac agaatcagat tattcttatt acaacttaat cctgaagaaa gctggacagt
360

ttctagacaa tttacacatc aaccttttaa agtttgcttt ctctataagg gcatactccc
420

cagctattca gatgtttcag cagattgcag ctgatgagcc accaccagat ggttgtaatg
480

catttgtggt tattcataag aagcacacct gtaaaattaa tgagattaaa aagctgctga
540

agaaagctgc ttcaaggact agaccttatc tatttaaagg agatcacaaa tttcctacaa
600

acaaagagaa cttaccagtg gtgattctct atgccgaaat gggtactaga acatttagtg
660

catttcacaa agtattgtct gaaaaagctc aaaatgagga aattctgtat gttcttcgcc
720

attatattca gaaaccaagc tcacggaaaa tgtacttatc tgggtatggt gtggagctag
780

caattaagag tacagaatac aaagcactgg atgataccca agttaaaact gtgactaata
840

ctactgtaga ggatgagact gaaacaaatg aagttcaagg atttctcttt gggaaactaa
900

aagaaatata ttcagatctt agagataatc tgacagcatt ccaaaaatac ctgattgaga
960

gtaacaaaca aatgatgcct ttgaaagtct gggaactaca agatcttagt tttcaagcag
1020

cttctcaaat aatgtccact ccagtttatg atgccattaa attaatgaaa gacatttcac
1080

agaacttccc cataaaagcc agatctctaa ccagaattgc tgtaaatcaa catatgagag
1140

aagaaataaa ggaaaatcaa aaggatcttc aagttagatt taaaattcag ccaggcgatg
1200

ctcgtctatt tataaatggc cttcgtgttg atatggatgt ttatgacgct tttagtattt
1260

tggatatgct gaaattagaa ggaaaaatga tgaatggcct tcgcaatctt gggatcaatg
1320

gggaagatat gagcaaattt ttaaaattaa attcacacat ttgggaatat acttatgtat
1380

tagatattcg acattcttct ataatgtgga ttaatgactt agaaaatgat gatttgtata
1440

ttacatggcc tacaagttgc cagaaacttc tgaagccagt atttcctgga agtgtacctt
1500

ccataaggcg caattttcat aatttggttc tgtttattga tccggcccaa gaatatacct
1560

tggattttat aaaacttgct gatgttttct attctcacga agttcctctt agaattggtt
1620

ttgtgttcat tcttaataca gatgatgaag ttgatggagc aaatgatgct ggagttgctc
1680

tctggcgagc tttcaactat attgcagaag aatttgatat atcagaagca tttatttcta
1740

tagtacacat gtaccaaaaa gtgaagaagg atcaaaatat actcactgtg gacaatgtga
1800

agagtgttct ccaaaataca tttcctcatg ctaatatttg ggatattttg ggaattcatt
1860

ctaaatatga tgaagaaaga aaggctggag caagctttta taagatgact ggcctgggtc
1920

ctttgcctca agctctttat aatggtgaac cctttaaaca tgaagagatg aatattaaag
1980

aactaaaaat ggctgttctt caaagaatga tggatgcatc tgtatattta caaagagaag
2040

tttttttggg cacattaaat gatcgcacga atgcaattga ttttctaatg gataggaata
2100

atgttgtacc ccgtataaat actttgattt tgcgtactaa ccagcagtac ctcaatttaa
2160

tatctacatc agtaactgct gatgttgaag atttctctac tttctttttc ttggattcac
2220

aagataagag tgctgtaatt gcaaagaaca tgtattattt aacccaagac gatgagagta
2280

taatttctgc agtcactctc tggattattg cagattttga taagccttct gggagaaaac
2340

ttctttttaa tgcattaaag cacatgaaaa caagtgttca tagtcggttg ggattattt
2400

ataatcctac atcaaaaata aatgaagaga acacagctat ttctagaggg attttggcag
2460

cttttcttac acagaagaac atgtttttga gaagctttct tgggcaactg gcaaaggaag
2520

aaattgctac aactatttac tctggagata aaattaaaac attccttatt gaggggatgg
2580

ataagaatgc ttttgagaaa aaatataata ctgttggagt gaatattttt cgaactcacc
2640

agttgttctg tcaagatgta cttaaattac gtcctggaga aatgggtatt gtcagcaatg
2700

ggagattctt aggaccttta gatgaagatt tttatgcaga agatttttac ttgttggaaa
2760

agataacatt tagtaattta ggagagaaaa ttaaaggcat tgttgaaaat atgggaatca
2820

acgcaaataa catgagtgac tttattatga aagttgatgc ccttatgtcc tctgtgccta
2880

agcgtgcatc tcgatatgat gtcacatttc ttagggagaa tcacagtgtt ataaagacga
2940

atcctcaaga gaatgatatg ttcttcaatg tcattgctat tgttgatcca ttaacaagag
3000

aagcacagaa aatggcacag ttgttggttg tacttggcaa gattatcaac ctgaagataa
3060

agttgttcat gaactgtagg ggcaggcttt cagaagcccc tttagaaagc ttttaccgtt
3120

ttgttctgga accagaactg atgtcagggg ctaatgacgt ttcttctctt ggaccagtgg
3180

caaaattttt ggatattcct gaatcacccc tcctaatcct caacatgatt actccagaag
3240

gctggttggt tgaaacagtg cacagcaact gtgaccttga taatattcac ttaaaggata
3300

ctgagaaaac tgttacagca gaatatgaac tagaatactt actactggaa ggacaatgct
3360

ttgataaagt gacagaacag cctcctcggg gtctgcagtt cacactaggc acaaaaaata
3420

aacctgctgt ggttgataca atagtgatgg cacatcatgg gtattttcaa ttaaaagcaa
3480

acccaggtgc ttggatactg aggttacacc aaggaaaatc tgaagatatt tatcaaatag
3540

ttgggcatga aggaactgac tctcaagcag acctagaaga tatcattgtt gtattaaaca
3600

gcttcaaaag caagatactc aaagtaaaag tgaaaaaaga aacagacaaa attaaggaag
3660

atatccttac cgatgaagat gaaaaaacaa aaggactgtg ggattccatt aaaagtttca
3720

cagtaagctt gcataaagaa aacaaaaagg aaaaagatgt cctaaacatt ttttcagttg
3780

cttctggtca tttatatgaa cgttttttaa gaattatgat gctttctgtt ttgcgtaaca
3840

ccaaaacacc agtgaaattc tggttgctaa aaaattatct ctcaccgaca tttaaagaag
3900

taattcctca catggctaaa gagtatggat tccgatatga actagttcaa tataggtggc
3960

cccgttggct tcgtcaacag actgaaagac agaggattat ttggggttac aaaattcttt
4020

tccttgatgt tcttttccca ctagcagtgg acaaaatcat ttttgttgat gctgaccaga
4080

ttgtgagaca tgatctaaaa gaacttcgag atttcgatct ggatggagct ccttatgggt
4140

atactccatt ttgtgatagc cgcagggaaa tggatggata tcgtttctgg aaaacaggat
4200

actgggcatc acatctttta agacggaaat accatatcag tgctttatat gtagtggatc
4260

tcaagaagtt caggagaatt ggagcaggtg acaggctcag gagccagtat caagctctca
4320

gtcaagatcc aaacagtctt tcaaacctag atcaggatct ccccaataat atgatttacc
4380

aagtcgccat taagtctctt cctcaagact ggctgtggtg tgaaacctgg tgtgatgatg
4440

aatccaaaca aagagccaaa acaattgatc tgtgcaataa tcccaaaaca aaagaatcca
4500

aactaaaagc tgctgccaga attgtcccag aatgggtgga gtatgatgct gagataagac
4560

aactattaga tcatcttgaa aacaagaagc aagatacaat tttgacacat gatgaactct
4620

agcactggtg tatatgagaa ggaaggcgaa agcatgacag gaaacctgcc gcctgctggg
4680

gaagtctgga gcccctgctg agacgatttg gaagtctcgt taagatcagt gacatattct
4740

ttaattttaa aaaattgtaa ttatttaaaa cagttattta atgtattgaa tgagtttaag
4800

ttatataata aatgaccatt gagtatttaa aaaaaaaaaa aaaaaaaa
4848

<210> 173
<211> 2877
<212> DNA
<213> Homo sapiens

<400> 173
aagggagagc gggcagcctt ccaggtggac agaaatggac tcagaactca aggaagaaat
60

tcctgtgcat gaggaattca ttttgtgtgg tggagccgaa acccaggttc taaaatgtgg
120

gccctggaca gacctctttc atgatcaaag tgtcaaaagg cctaagctgc ttattttcat
180

tattcctggt aacccaggtt tttctgcctt ttatgtgcca tttgcaaagg ctttatactc
240

tttgacaaac agacgctttc cagtttggac tatcagtcat gctgggcatg cgttggctcc
300

caaagacaag aagattctta caacatcaga ggattcaaac gctcaagaaa ttaaggacat
360

ttatggacta aatggacaaa tagagcacaa actagctttc ctgagaactc atgtgccaaa
420

ggacatgaaa cttgtgctca ttggccattc aataggcagc tatttcacac ttcagatgct
480

gaagcgagtc cctgagctcc cggtaattcg tgcctttctg ctctttccaa caattgaacg
540

aatgtctgag tcacccaatg gcagaattgc cactccactt ttgtgctggt ttcgatatgt
600

tctctatgtt actggctact tattattgaa accgtgtcct gagacaatca agtccttgct
660

aatcagaagg ggccttcaag taatgaacct agagaatgaa ttttcaccat tgaatatatt
720

agaaccattc tgccttgcta atgctgccta ccttgggggc caagaaatga tggaggtggt
780

gaagagagat gacgaaacca taaaggagca tttatgtaag cttacatttt attatggtac
840

tatagatcct tggtgtccaa aagagtacta tgaagacatt aagaaggatt ttccagaagg
900

agacattcga ctctgtgaga aaaacatacc tcatgctttc atcacccatt ttaaccagga
960

aatggcagac atgattgctg actccctaaa ggatgacttg tccaaaatgt aaattggcct
1020

gaggaacgag cccccactgc cagtacatgg aggcagtcag tgtactagac ttagtaggta
1080

aatgtttaat tttgaagact gatattagaa atgaagaaag tgagaacctt tgtcttacaa
1140

accaactctc cgctcgccat gttataggct gaagtaaaca cagttgatga atcattccat
1200

aggtttaacc atacattttc caagactcag ggaacacagt gatctacaca gagtcttgtg
1260

tttgcacaag atgcccagtg gcaccatatg gtttattttg gtaggcagga tctttgcaga
1320

tgaaaaaaaa atctacatgt acttgatttt aattgagtta cattgtagaa taggctcctc
1380

tggaggaaat tatgaaatac ctactagaaa atgtaaaata aatcagtgaa tgttaagagt
1440

atagttagat atgtgaagtg tatgagatta tgacaaggat acactcatgt tccaggagca
1500

ggaagtgaac ctgggtctcc tgtaagacag aagatgaaga tgagcccagg ctaacttagc
1560

acagatcttg gctgagatca tcaatgtgac gtctaatgta cctgcactag acagagaata
1620

aagttcacca gacattactc tggtcagcta accagataaa gaattgttga aggaccccaa
1680

ctgtgcctcc tgccacagga caaccagcaa gttctatgct gagccttagc ctcccaggtt
1740

ataagctccc tgcaggctcc tcctctccag agccaggatg gagaggcact gggctgtccc
1800

aaagcaggct tggatgtgcc aacgtacagt tgctccttct gtaattcttg cactaaaact
1860

ccattaaaga ccatcaatga gccaaatagt gtgctagcca ttgaggatgg agttcttagc
1920

tacttaatga ttcttttctt ctggaaatct tctagtgaat aatttttaaa agcttctttt
1980

aaactgctca gtaaaacatt gccatacatt aactctccat ttctgcatta acttcatttg
2040

ctggaaagaa attattttgt taatgaacca aacccagcca ttaagttatg tgaattcacc
2100

ttctatagac acattgaggt atggcagaga agacacatat tccctggcct agagtgggct
2160

gacaatattg ctgcctcccc taaattgact caatcttgag aaccacatac tgcagtgaca
2220

gttgatcatg gatcagaaat attccttttt ctcctagagg aaatccctca tggcattatc
2280

attccctaag gacaatgaga tgtccctccg ctcaaatcct aaagccccct gatgaaagat
2340

ttggctctga tttgctgcca acttattggt aagtgctgaa tgagttactg tgctttggca
2400

tagcttctac atcacagcaa gtgacttctc agaggggaaa agccaagctc cagacaagga
2460

gtgctgttag cccaagggga agagctaata gatacatagg ccgtaagcac agggctgtac
2520

acagatcctt tattggcatc tgcatgagag gcctagaaat cggatgttag aagctaggaa
2580

tcatggctct gagcgccttg catacaggag atactcactg atactaaatc aaggcatgta
2640

aacaatactg ggtaaatcat acctaccaga ataaagagta acattcagag cttttctttt
2700

atctgttatt ttaaatgtta ctccatattt caaaagtctg cacgtatgaa attgtgtctt
2760

atttctggtt tttagagcct aagaaagata ctcattctta attcctattt atcatctgaa
2820

aaccaaatga acttttgaaa taaatgtttt accagcaaaa aaaaaaaaaa aaaaaaa
2877

<210> 174
<211> 2022
<212> DNA
<213> Homo sapiens

<400> 174
ttttggtttc ctttggaaat gcactgttct cagcccagct gggttccaac gggggcacct
60

gggacgacag aggcatctcg gggcaggggg cagaggccac gggagggtca ggtgggaccc
120

ttgacggcac cctctgatct cttgggggga cgaccgtgta agatgaaagt cggtcaagtt
180

tatttgcttt cagtgcatct cctagaaaag aagtgttgga gcagggggtg gtgatgaggg
240

gcccgtgggg aaaggattca agaggcaaag cccagaccag ggagtgtgac aacagccgtg
300

agcatctcac ctgtgttcaa aacaagacaa agacgaacaa atattttaaa gtattgataa
360

gaaaaagcaa tgtttggatt gtatctgctg aatcatattc caacctatat ctgatttctg
420

tttccggggc cagttggtct gaggccaagg agtctggcct ccacccagag cagggagggg
480

ctggcccctc gcccccctca ccctcgccgc cctggcacac tcgggaagca gggcccagct
540

ctgagcccct cctcacccct ggggtcctaa ctttcctgaa agtagtaggt gccgtgagaa
600

ggggcagttt ggccaggttc ctgaactggg cagggctcgg ggcctatgag ggcaggttca
660

cagtcccacc agattctctc ctcaccccca agcagaatgc atgcaaaaga cacccctttt
720

cccacccacc ttattggtgc ccccaaaccc ctggcctgct gcgtagatgg tggtgaggcc
780

aggccagcag tgctgtggcc aggggagaag aaaataaaac gcaggccctg cccttgggga
840

aggccccttt ctgggccccc ttttccacca gccaaatctg cctggccctc ggacccctct
900

gcctgcccca acccctttgg aggtttctcg gccttttctg tgccacttgg tggggcagat
960

ggcctgatgg gctagtgctt ggggcataga atgagggtcc ccctgaccac ctgagcccaa

1020

atcctggccc caggtgcaag cagcacccct cgagggctct gccccagtat ccccagggaa
1080

ttcacacccc tccccttctc ccacagccaa ggacagacag gctgcctgga cctgagccca
1140

acagccttca gcctcagaaa cgcatggggg gccacacact ccttatatcc tcccacacta
1200

aggttcccct ggccccacgg gagcttcagg aaagcccccc aagttagcca ctgctctagg
1260

acgagctctg tgtcccccac accacaggcc tcgaagcagg gtgctggtgg gtgccctgca
1320

ccccaatccc aggtcccctt ggccccctat ttttctcggg cccattgggg cctgtttctc
1380

acctgctggc tggaccccct gaagggccgt tcccagaggc tccccaggag gctcaaggct
1440

gggggcttat gttgtggtcg ggggtccccg cctccagccc cccgctccca cccccgagcc
1500

taccctttgt ctcgcatgag tgcaatattt cattcccggt ggttgtctgg ggaggtggtt
1560

ggacgaggtc ctgcttgagg ctcggggatc agaacgatgt caagtgaaga aaaatgcacg
1620

ggggcatgct gagccactgg agttccagag agggccgagg agggtgcagc cacggaggtt
1680

ggatcctctg ctccgccgcc tcgtcctcgt cccgcttctg ctgggttccg ctttgtccct
1740

ggttttttcc tttttttttc tgtgtgcgtg cgaatggtgc ggcccctccc ctctcaccgt
1800

ggttaacgtg acgaaggcac gattcctgta aatgtgtaaa ctaaggggat ggttggattt
1860

tttttcaatg taaacactaa aaacaaaatg gacaaaaaaa cacacaaagg ttttatgaac
1920

agcagactct atgtaaaggc attttagtat caaatttttt attgataact tgcttaagaa
1980

taaatatatg gactattgta aaaaaaaaaa aaaaaaaaaa aa
2022

<210> 175
<211> 1700
<212> DNA
<213> Homo sapiens

<400> 175
cggaattggt gggttcttgg tctcactgag ttctagaatg aagctgcaga ccctcgcagt
60

gagtgttaca gctcttaagg ctctctgact gccacccctg cctgcctgcc cggccctgca
120

caacatgcag ccctccggcc tcgagggtcc cggcacgttt ggtcggtggc ctctgctgag
180

tctgctgctc ctgctgctgc tgctccagcc tgtaacctgt gcctacacca cgccaggccc
240

ccccagagcc ctcaccacgc tgggcgcccc cagagcccac accatgccgg gcacctacgc
300

tccctcgacc acactcagta gtcccagcac ccagggcctg caagagcagg cacgggccct
360

gatgcgggac ttcccgctcg tggacggcca caacgacctg cccctggtcc taaggcaggt
420

ttaccagaaa gggctacagg atgttaacct gcgcaatttc agctacggcc agaccagcct
480

ggacaggctt agagatggcc tcgtgggcgc ccagttctgg tcagcctatg tgccatgcca
540

gacccaggac cgggatgccc tgcgcctcac cctggagcag attgacctca tacgccgcat
600

gtgtgcctcc tattctgagc tggagcttgt gacctcggct aaagctctga acgacactca
660

gaaattggcc tgcctcatcg gtgtagaggg tggccactcg ctggacaata gcctctccat
720

cttacgtacc ttctacatgc tgggagtgcg ctacctgacg ctcacccaca cctgcaacac
780

accctgggca gagagctccg ctaagggcgt ccactccttc tacaacaaca tcagcgggct
840

gactgacttt ggtgagaagg tggtggcaga aatgaaccgc ctgggcatga tggtagactt
900

atcccatgtc tcagatgctg tggcacggcg ggccctggaa gtgtcacagg cacctgtgat
960

cttctcccac tcggctgccc ggggtgtgtg caacagtgct cggaatgttc ctgatgacat
1020

cctgcagctt ctgaagaaga acggtggcgt cgtgatggtg tctttgtcca tgggagtaat
1080

acagtgcaac ccatcagcca atgtgtccac tgtggcagat cacttcgacc acatcaaggc
1140

tgtcattgga tccaagttca tcgggattgg tggagattat gatggggccg gcaaattccc
1200

tcaggggctg gaagacgtgt ccacataccc ggtcctgata gaggagttgc tgagtcgtgg
1260

ctggagtgag gaagagcttc agggtgtcct tcgtggaaac ctgctgcggg tcttcagaca
1320

agtggaaaag gtacaggaag aaaacaaatg gcaaagcccc ttggaggaca agttcccgga
1380

tgagcagctg agcagttcct gccactccga cctctcacgt ctgcgtcaga gacagagtct
1440

gacttcaggc caggaactca ctgagattcc catacactgg acagccaagt taccagccaa
1500

gtggtcagtc tcagagtcct ccccccacat ggccccagtc cttgcagttg tggccacctt
1560

cccagtcctt attctgtggc tctgatgacc cagttagtcc tgccagatgt cactgtagca
1620

agccacagac accccacaaa gttcccctgt tgtgcaggca caaatatttc ttgaaataaa
1680

tgttttggac atagaaaaaa
1700

<210> 176
<211> 1663
<212> DNA
<213> Homo sapiens

<400> 176
atcgagtcgg ccttgttggg tgctaggatt tcaagtattt ggtgtctggc cagaaggatc
60

tgatgggaca gatatcaatg cactggtgcg atcccacaat agaaaggtga tagctgttgc
120

cgatgacttt tgtaaagtcc atctgtttca gtatccctgc tccaaagcaa aggctcccag
180

tcacaagtac agtgcccaca gcagccatgt caccaatgtc agttttactc acaatgacag
240

tcacctgata tcaactggtg gaaaagacat gagcatcatt cagtggaaac ttgtggaaaa
300

gttatctttg cctcagaatg agactgtagc ggatactact ctaaccaaag cccccgtctc
360

ttccactgaa agtgtcatcc aatctaatac tcccacaccg cctccttctc agcccttaaa
420

tgagacagct gaagaggaaa gtagaataag cagttctccc acacttctgg agaacagcct
480

ggaacaaact gtggagccaa gtgaagacca cagcgaggag gagagtgaag agggcagcgg
540

agaccttggt gagcctcttt atgaagagcc atgcaacgag ataagcaagg agcaggccaa
600

agccaccctt ctggaggacc agcaagaccc ttcgccctcg tcctaacacc ctggcttcag
660

tgcaactctt ttccttcagc tgcatgtgat tttgtgataa agttcaggta acaggatggg
720

cagtgatgga gaatcactgt tgattgagat tttggtttcc atgtgatttg ttttcttcaa
780

tagtcttatt ttcagtctct caaatacagc caacttaaag ttttagtttg gtgtttattg
840

aaaattaacc aaacttaata ctaggagaag actgaatcat taatgatgtc tcacaaatta
900

ctgtgtacct aagtggtgtg atgtaaatac tggaaacaaa aacagcagtt gcattgattt
960

tgaaaacaaa cccccttgtt atctgaacat gttttcttca ggaacaacca gaggtatcac
1020

aaacactgtt actcatctac tggctcagac tgtactactt tttttttttt ttttcctgaa
1080

aaagaaacca gaaaaaaatg tactcttact gagataccct ctcaccccaa atgtgtaatg
1140

gaaaattttt aattaagaaa aacttcagtt ttgccaagtg caatggtgtt gccttcttta
1200

aaaaatgccg ttttcttaca ctaccagtgg atgtccagac atgctcttag tctactagag
1260

aggtgctgcc ttttctaagt cataatgagg aacagtccct taatttcttg tgtgcaactc
1320

gacggtttcg ccggcagtct cgatgatagt atttctgctg caagtacttc tgatgttcaa
1380

gatcgcctgt cagctcttga gtcacgagtt cagcaacaag aagatgaaat cactgtgcta
1440

aaggcggctt tggctgatgt tttgaggcgt cttgcaatct ctgaagatca tgtggcctca
1500

gtgaaaaaat cagtctcaag taaaggccaa ccaagccctc gagcagttat tcccatgtcc
1560

tgtataacca atggaagtgg tgcaaacaga aaaccaagtc ataccagtgc tgtctcaatt
1620

gcaggaaaag aaactctttc atctgctgct aaaagtggta cag
1663


<210>  177
<211>  2071
<212>  DNA
<213>  Homo sapiens

<400>  177
catcaccctt tttaacattg tttacacatc actgcctgtt ttagccatgg ggatttttga
60

ccaggatgtg agtgaccaga acagcgtgga ctgtccccag ctctacaaac caggacagct
120

gaatctgctt tttaacaagc gtaaattttc catttgcgtg ttgcatggaa tctacacctc
180

attagtcctt ttcttcatcc cctatggggc cttttacaac gtggctggag aagatgggca
240

acatattgct gactaccagt cctctgcagt tacatggcca catctttggt cattgtggtc
300

agtgtgcaga tagccttgga taccagttac tggactttca ttaatcacgt cttcatctgg
360

gggagcattg ccatttattt ctccatttta tttacaatgc acagtaatgg catctttggc

420

atcttcccaa accagtttcc atttgttggt aatgcacgac attccctgac ccagaagtgc
480

atctggcttg taattctctt aacaacagtg gcttcagtta tgccagtggt ggcattcaga
540

tttttgaagg tggatttata cccaaccctg agtgatcaga tccgccggtg gcagaaggct
600

caaaagaagg caaggcctcc aagtagccga aggcctcgga cccgcaggtc aagctcaaga
660

aggtctggat atgcttttgc tcaccaagaa ggctatggag agcttatcac atctggaaaa
720

aatatgcgag ctaaaaatcc acccccaaca tcagggctgg aaaagacaca ttataatagc
780

actagctgga ttgaaaattt atgtaagaaa accacagaca ccgtgagcag ctttagccag
840

gataaaacag tgaaactgtg agtcaatatg aatttaaacc acgtagttat cttttcactt
900

caggtggagc tgaaattctg ctggctccag agtttgagat ttgaggcaag aggtggggca
960

ggcagattgc ctcacttaac ttaaatctgc ggcagacaac tgccagtgcc catcaaacag
1020

gagtgtgcgc tatggaaaac caggccagag ggtcactgtc tggtttgtga tttggtggac
1080

aaaacactcg ctgttacaag tacagatttt tttttttttt aaatcaacct agataccaat
1140

tgacctgaac tttagaatct tatttatgga gaaaaacttg taaagctgca tattcactga
1200

atggatcctc aggcggataa aagggtgcat tttaaaggta tatatccaag ctgaaaagca
1260

tgcctattga cagataaaca tgtatctgta agatcagcct ttcccaaggt atacttttaa
1320

aatttaaagc gtgtactgtg ttgctttcag actgagttgc atgtcactct ttagtcttga
1380

tatctacctg tctgttcagc caggacaaca aatggcttcc aagcctgaag aatacaaaag
1440

tgtgcttgtg tttctcattt ttataccagt ctagggacaa aggagactga acatctttgc
1500

agcaggatag gctggtaatt tgatcaaatt tattcaaaaa gctctcagtc tgtgtcatgt
1560

aaggacatgc ttatgaaatg tgagagaggc tcgccactaa gtattctaaa tacttttcaa
1620

tggcttttct aacaacctca gtagtaattt gctgagcatc atccagacca ttaatagaat
1680

cagcaaagca ctggaatttc acactttaat gataatattc cacatagtct atgggcaaat

1740

attttcaaca tttccaattt ttaaagcttc agaattgaag ccaaacaaat taataaataa
1800

ttgttttaat tactatttaa aaactcaggt ttagattgtt taaaattagt tgcttttgat
1860

actcagctgt catgtttata attcaaacat gtagtaaaca tatgtaggta aggttgtttt
1920

tttggagatg ttgcagctca aatttcagtc cacatatgaa tcatcagtgt attttccata
1980

aagtgattcg ggcatatttg tgtgaaaacc tcagttctgt cacttcttac ctctataaac
2040

ttggacgata atgaaaaaaa aaaaaaaaaa a
2071

<210> 178
<211> 1241
<212> DNA
<213> Homo sapiens

<400> 178
ctgagcctgt ctctcacggg accgggaagc tggagagagc cccaaccctg cccgctgggg
60

ccgagctccc tgctcctgca gcagtcccgt gccccacact ctgagtctgc cctatccaca
120

gctgctgggc ctctctgtgg ccaccatggt gactcttacc tacttcgggg cccactttgc
180

tgtcatccgc cgagcgtccc tggagaagaa cccgtaccag gctgtgcacc aatgggcctt
240

ctctgcgggg ttgagcctgg tgggcctcct gactctggga gccgtgctga gcgctgcagc
300

caccgtgagg gaggcccagg gcctcatggc agggggcttc ctgtgcttct ccctggcgtt
360

ctgygcacag gtgcaggtgg tgttctggag actccacagc cccacccagg tggaggacgc
420

catgctggac acctacgacc tggtatatga gcaggcgatg aaaggtacgt cccacgtccg
480

gcggcaggag ctggcggcca tccaggacgt gtttctgtgc tgtgggaaga agtctccttt
540

cagccgtctg gggagcacag aggctgacct gtgtcaggga gaggaggcgg cgagagagga
600

ctgccttcag ggcatccgga gcttcctgag gacacaccag caggtcgcct ccagcctgac
660

cagcatcggc ctggccctca cggtgtccgc cttgctcttc agctccttcc tgtggtttgc
720

catccgctgt ggctgcagct tggaccgcaa gggcaaatac accctgaccc cacgagcatg
780

tggccgccag ccccaggagc ccagcctctt gagatgctcc cagggtggac ccacacattg
840

tctccactcc gaagcagttg ctattggtcc aagaggatgc tcgggtagtc ttcggtggct
900

gcaggagagc gatgctgcgc ctctgcccct ctcctgccac ctggctgccc acagagctct
960

ccagggcaga agtcgcggtg ggctcagtgg gtgccctgag cggggtctct cagactgacg
1020

tcaggccttg gtgggctgca ctctcacctg gaggctccgg ggaagcatct gcctccagga
1080

ccattcaggc tgttgacaag tcaactcctc atggctgtag gactgaggtt cccaagtcct
1140

tgtccctggt cctgtggtcc ctccaccttc aaaccagcaa tggtgcattg agcaaattgt
1200

ggtcaaatat acatcacatc aaatttacca tcttaaaaaa a
1241

<210> 179
<211> 1860
<212> DNA
<213> Homo sapiens

<400> 179
gcggatcctc acacgactgt gatccgattc tttccagcgg cttctgcaac caagcgggtc
60

ttacccccgg tcctccgcgt ctccagtcct cgcacctgga accccaacgt ccccgagagt
120

ccccgaatcc ccgctcccag gctacctaag aggatgagcg gtgctccgac ggccggggca
180

gccctgatgc tctgcgccgc caccgccgtg ctactgagcg ctcagggcgg acccgtgcag
240

tccaagtcgc cgcgctttgc gtcctgggac gagatgaatg tcctggcgca cggactcctg
300

cagctcggcc aggggtgcgc gaacaccgga gcgcacccgc agtcagctga gcgcgctgga
360

gcgcgcctga gcgcgtgcgg gtccgcctgt cagggaaccg aggggtccac cgacctcccg
420

ttagcccctg agagccgggt ggaccctgag gtccttcaca gcctgcagac acaactcaag
480

gctcagaaca gcaggatcca gcaactcttc cacaaggtgg cccagcagca gcggcacctg
540

gagaagcagc acctgcgaat tcagcatctg caaagccagt ttggcctcct ggaccacaag
600

cacctagacc atgaggtggc caagcctgcc cgaagaaaga ggctgcccga gatggcccag
660

ccagttgacc cggctcacaa tgtcagccgc ctgcaccggc tgcccaggga ttgccaggag
720

ctgttccagg ttggggagag gcagagtgga ctatttgaaa tccagcctca ggggtctccg
780

ccatttttgg tgaactgcaa gatgacctca gatggaggct ggacagtaat tcagaggcgc
840

cacgatggct cagtggactt caaccggccc tgggaagcct acaaggcggg gtttggggat
900

ccccacggcg agttctggct gggtctggag aaggtgcata gcatcacggg ggaccgcaac
960

agccgcctgg ccgtgcagct gcgggactgg gatggcaacg ccgagttgct gcagttctcc
1020

gtgcacctgg gtggcgagga cacggcctat agcctgcagc tcactgcacc cgtggccggc
1080

cagctgggcg ccaccaccgt cccacccagc ggcctctccg tacccttctc cacttgggac
1140

caggatcacg acctccgcag ggacaagaac tgcgccaaga gcctctctgg aggctggtgg
1200

tttggcacct gcagccattc caacctcaac ggccagtact tccgctccat cccacagcag
1260

cggcagaagc ttaagaaggg aatcttctgg aagacctggc ggggccgcta ctacccgctg
1320

caggccacca ccatgttgat ccagcccatg gcagcagagg cagcctccta gcgtcctggc
1380

tgggcctggt cccaggccca cgaaagacgg tgactcttgg ctctgcccga ggatgtggcc
1440

aagaccacga ctggagaagc cccctttctg agtgcagggg ggctgcatgc gttgcctcct
1500

gagatcgagg ctgcaggata tgctcagact ctagaggcgt ggaccaaggg gcatggagct
1560

tcactccttg ctggccaggg agttggggac tcagagggac cacttggggc cagccagact
1620

ggcctcaatg gcggactcag tcacattgac tgacggggac cagggcttgt gtgggtcgag
1680

agcgccctca tggtgctggt gctgttgtgt gtaggtcccc tggggacaca agcaggcgcc
1740

aatggtatct gggcggagct cacagagttc ttggaataaa agcaacctca gaacaaaaaa
1800

aaaaaaaaaa aagcggagct cacagagttc ttggaataaa agcaacctca gaacaaaaaa
1860

<210> 180
<211> 803
<212> DNA
<213> Homo sapiens

<220>

<221>  misc_feature
<222>  (487)..(500)

<220>
<221>  misc_feature
<222>  (487)..(747)

<220>
<221>  misc_feature
<222>  (487)..(487)
<223>  n is a, c, g, or t

<220>
<221>  misc_feature
<222>  (602)..(602)
<223>  n is a, c, g, or t

<220>
<221>  misc_feature
<222>  (701)..(701)
<223>  n is a, c, g, or t

<220>
<221>  misc_feature
<222>  (747)..(747)
<223>  n is a, c, g, or t

<400>  180
tttgaagagc tgaagtactg aagtgagtac atacattttg tcatgttaaa caggagaaag
60

attgtgactt ggctcctgaa attttaatac tgcttgttag ccatctacaa tccctaaagt
120

gctttcagag gctctacaca tcttcaatga gtcaaacccc aaagacttat aaataggtat
180

ataggttgct aatctattcc tgatgctttg cctaaaaaat tggcattgcc atcttccaca
240

tctttctttt ggaagcatgg ttttgagttt gttccttgtt ggtaagcatc cagaaaatgg
300

cagattccgg ggatttcact agggaagttt ggtggaagtt cctcccttga tcgaggggta
360

aacacgaaac caggacagtc tttgagtaat ctgtaagttg ttgggctgac attgattttt
420

cgtggtacac tgcaggactc aaatttgtta gccagagtga cattgtttcc aaaaagacag
480

taacggngca ttttaactcc aacgacgcca gcaaaaactg atccagagtg cagtccaatt
540

cgcatcttga taggttctcc atggggagac ataacttcat cagagagctc catcatcttc
600

anggccatca gcgctatctg aacagcatga gtatcacttc tttttgtgta tcccccagct
660

acacaatagg catcgccaat ggtctccacc ttgtagacat ncagctcttc acacttgctg
720

ggtcgaagca gtgtacatgc atttgancat gtgatgacct gcagcgtgag cacttggagc
780

gagatgagtg aaccccacga tgt
803

<210> 181
<211> 140167
<212> DNA
<213> Homo sapiens

<400> 181
aagctttggc gccaaatcgg gaacacttca ggatccattg agatgatctt tgcagagctc
60

acactggtgc agccagaggg aggatcctga tttagtagtt tctacttcag gtgaggacac
120

gagacacagg tcaagcatcc agtgtggcaa cgcagttagt ttatgtgtga agtgagtgct
180

ggcaggtgct caggcatgaa gcccagctac tgagggaggg attccactgc tatgcagccc
240

agactgcccc catggccaag ggcagccagc aggttataat tttgaggctt tttacaatgg
300

cagatttttg aatcacttat ttttttctct cttaaaacaa tgcatcaagt tgaggggctt
360

tttgctcatt gccacagcct tgagaaactc ctgttgccga ttagttttat tttggggctt
420

ctctttgctc agagttatct agccctgcag gtggatttga tgagctttac tccagggttg
480

aaaggagact agtatttgtg ccttatcatt tgttaaagcc cacctaatgt gggagccgct
540

cagtagccaa tcaatatcct ggggaaaaaa agccttgtaa ttggagtctg aagacctaga
600

tctaagccct aagctctacg aagtattagc tatgtcatta aggcaagtga ttaaacctct
660

ctgaaactca gtttcctcat atgtcgaaat ggtccttggt tatatgaaca gagagattgc
720

cccaaactag cagcagtttt aagagagata cccatgagtg gagggaggag ggatagcatt
780

aggagatata cctaatgtaa atgatgagtt aatgggcgca gcacaccaac atggcacatg
840

tatacatatg taacaaacct gcacattgtg catacgtacc ctggaatgta aagtataata
900

aaaatgaata aattaataaa aattttaaaa agagagattt tcgctatctg gctaataata
960

aatgggacat gagcagcagc tttaagccct cattgtcatt tttggaagag aagttgtcca
1020

aactttaata gcttttccaa caggtataca acagcttatc tcccttaatt atttaagaaa
1080

atattttgca actaactttc ctggaggggt tggtaaaaat tacgctatat gacatgataa

1140

atctcatagg agtgtccttg atttatttgg tcaccgtgcc agacattaag ctagatgctg
1200

aggacactgg cactgtcttc aaagacttta cagccttgaa agggagagag gcaactacat
1260

ataaaactgc aatgaagtat aataaaactg tgatagactc ttccataggg tgccaagaga
1320

ggcaaacaga taatacatac ctcctgttga atgttcacga tcccacctcc gaagtagctt
1380

tgcaaaaaat tgaacttgaa tttcacaagc cagaagaagt ggctcatgcc tataatccta
1440

gctactcagg aggctgaggt gagagggtcg cttgaggcca gaagttcaag accaccctgg
1500

gcaacatagc aagacttcct ctccctggcc cctctctaaa agaattttaa aagtcagcta
1560

ggcatgaatc cactccagcc tgggcaacag agtgaaatac tgcctgtaaa gaaaaaataa
1620

agcaagaaca agtaactggg gcctcacaaa tttggatgaa gaggaaaaca gctgtgagat
1680

ttaaatgtaa aaataagcca caatcacaca gctatatagt tgaagaagga gtattttaat
1740

aatctttgga caactgttga gtatccttct tcgacattac aacaaaattt aagtgatttt
1800

cttaaaaatt agttgtaata tgggatatga aaccatacga atgaatattt tatacacttt
1860

gaactgcgtt ggctatcttt actttttaaa atttgtgtac acttatggat actttttaaa
1920

atttgtatac atttatgagt acaagtataa ttttgttata tattgcatag tgatgaagtc
1980

aaggcatttg gtgtacacat cactgaagta atgtacatgg tacccattaa gtaatttctc
2040

atcatccacc tccttcccat gcccctcacc ctttagatcc tccactaaca ttccactctc
2100

tacttccaag tgcacacatt attcagctcc ctcttataag ttagaatatg cagtacttgt
2160

ctttctgtgt ctgcgttgct tcacttaaga aaatggcctc tggttgcatc cacattgctg
2220

caaaagacat gatttcattc tcttttatgt ttatatacca cattttcttt atccaatagt
2280

ctactggtgg acacttaggt tgattctata tctttgctat tgtgaatagt gctgcaataa
2340

atatacaagt gcaggtgcct ttttaatata atgatttatt ttcctttagg taggtaccca
2400

gtagtaggat cgttagatag tatggttgtt ctatttttag ttccttgaga aatcttcata

2460

ccattttcca ttgaggttgt actaatttac atttcatcaa cagtgtataa atgttcccat
2520

ttctctgcat ccttgcattg ggctatcttt aaaataaaaa catgaaagta ctaaaaaaac
2580

aaataaatac tttgataaat ttagggaaga acattccaaa catgacaatt aaaatggaac
2640

ccttttaaat atgaggaaac actttaaaca agagacaaat aaaatatcag aaaaaaaatt
2700

gcagcatatg tggcaaaaca gttgacattc tctgtacaat aaaccattac aaattaatgc
2760

agaagaaaat acacattcaa atagaaacac agataaagga tatgactggg caactgacca
2820

aagaaagaat taaaatggca aataaacatt gaaaattttt caaattgaat aatattctat
2880

gaaatacaag ttgaaactat gagctaccac tttttactca gaataaacaa caggtgaaaa
2940

ggaatgacaa tagtgtgacg gtgtggatgt ggaaaagtaa gaactgtcat atactctttt
3000

ggggactata ttagtttcct attgccactg tgacaattta cccacaactt catgacttaa
3060

agcaacacaa atttattatc ttacagttcc gaagtcagaa gtctaaaatg gataagccag
3120

gctgtttctt ctggaggctc cagaggagaa tccatttcct taccttctcc agcatttaga
3180

gtcggctctg attcttggct cctgacccct cctttatcac tccaacctgt ttccctctga
3240

ctctgaccct cctgcctccc ttttttaaga acccttgtga ctacactggg cccacccaga
3300

tagtatggga tagtctcccc atctcaaaat ccctaacgta atcatatttt caaagttcct
3360

tttataacat aaggtaacat attcacaggt tctggggatt cagatgtgga catctttgga
3420

gaagggggtt tattattcag cctgtcatgg gagtaaactg gtataaactt tctggaggaa
3480

aatttgacaa tatgtatcaa catccttata aggtacatat ctttgatcca cttattcaac
3540

ttcagggaat ttattctatc agtcaaacaa gtgcacaaag aataggactt gtctgcagca
3600

ttgttttaat agaaaaaact tggaaatgtt ctaaataaaa agaaaaaaaa tttttttttg
3660

agccaaggtc ttgctctgtc acccatgctg gagtgcagtt acatgatcat ggcttgctgc
3720

agctttgatc tcctgagttc aagcaattct ccttgcctca gcctccagag tagctgaaac

3780

tgcaggtgag caccatcatg tccagttgtt ttaatttttt gtagagacgg ggtctcacta
3840

tgttgctagt gactaggctg gtctcgaact cctaggctca agtaatctgc ccaccttggc
3900

ctcccaaagt gctgggatta cagatgtgag ccactgtact cagcccaaag caaaattttg
3960

gttaaatata ttatacttca ttaaaataat agaatatcat atagccatta ctaatgattt
4020

tccatatttg ttgatatgga aactctccat tgttaaatga aagttccagg tagcaaaaga
4080

gtttacgtag tatgattcca ttctgataaa tatcaaaatt taaatctaaa tatatattaa
4140

tttaaatcta attaattaat ttaaatctaa atatcagaat ttaaatctaa atgtagattt
4200

gtgtagattt gtgcatatat atatatatat tgcataataa ataagcaagg ctatgcacaa
4260

aacttccagt gcaaaatatt gctgagtgaa atattgctga gtgacttttc ctttctcttg
4320

tggaaattgt gagaattgac taggaatatg tgagaagatg aaggtttagc cgagttcaca
4380

ggccatgaat ctgcagtgaa gagcaagcga aagcaagatc ctggaagata ctggcaagtg
4440

gccggatcac agaatccagg ttagaaaggg gggaaaaaaa ggtcaagaga ggtctgacaa
4500

atcaggagaa aatgaatgga ataacgaaat ggaagaatct ttctaaagaa cataatctca
4560

aattccctgc tgctcacttt ttgtaaaaat taagaaatat tcaccaaaaa aaaaaaagtc
4620

aggagaatat gaagccactc attttacttc tagaagtttt tcttacagaa aatcaatcaa
4680

ggagagagaa ttgtgtatta atttaattgt ttggccttta gaatcatgtt ccttagaggg
4740

catcggtgcg acggcttcac cattctgcac atccacctga gttttaaaac acggtttcta
4800

ctaaacatct ggtatcttgt atacagggga tgaatgtggt cttgtttcct tctttagctg
4860

ctggaaacct tagagcaaaa aataaggcca cttttgtaac tgtgtctgac tcagcagcat
4920

aacttttccg ttgtcttcca tgtgctatcc tgtttttatt tttcctttcc cttgattgct
4980

ctctatacat acatttttga gaagttgttt ttctttattt ttcaaactaa ttcattcaat
5040

actctcctcc aggtagagtt tataattgtt tgtagttgag aaaattggac tagaatgaag

5100

ccttgtggaa tccaagaaga attcgttatt ctttagctgc tttcatctcc aactgtgtta
5160

ggcatcctcc taagatggtc cccaagatgg cagacccctg gaatcatctg tacaatttcc
5220

tctcttttca caggtaaggg attttataaa cataataaga tttctttcct gtaattgggt
5280

tacattgaat aacaaaggtg aagggattac acagatgtaa tgaaggtccc tactcacttg
5340

gctctgagtt aatcaaaagg gagattatcc taagtgggcc tgacctaatc aggtgagctc
5400

ttgataagag ggtgctggcc ttccctgaga gattcaaagc agcacaaatg ctctcctgtt
5460

ggatttgtag aaacataatg ctatgctgtg aaaagggcca cttagggctg cagtcctcca
5520

gctataagaa ctgaattctg ctgttggggc tcagaaaaca atatcccaga atgaaacctc
5580

aaacgcagcc tggaaagaaa aagttcttct ctgacctcct acccttctgt ctctcagtcc
5640

cattctcccc tgaggctatc catagaaacc agaactcctt ttccccaaag gcagccataa
5700

acctaaaaat gttactctaa ctttccctct gccctgtcta cgtaaaaact ggccataaag
5760

aaattatctg acttaccttg tttaactgtg gatgataaga cccccattcc agagagggtt
5820

ctgccctata cccagaagga aggaatactg cacagagaag ccaagaagaa tctagacaga
5880

caggccttac tgagtttccc attcagttta ttagcatttg attgtacctt ttttgtccaa
5940

tcccatttcg acacggctgt ccataatttg ctgaacctag gcataaaaat ggaaaattgc
6000

ccccattctg aaggctcccg tgtttactta catgttaaat aaatttgtat gccttttctt
6060

ctgtaaatag gcttttatga gttgattttt cagcgaacct tcaaggggct aaggggaaca
6120

ttacccttgg ccactatact gccaacaacc tatgagcctg gaagaggatc ccaagtctca
6180

gatgaaacca ggacctcagc caacaccttg atttcagcct catgagatcc taagcagagg
6240

gtacagtaaa gccacatatg gtgcctaacc aaaaaaacta tgagataata aagatgtgtt
6300

ttggaagctg ctaaactggt gttgatttgt tacatagcaa ttaaaaacta atatacaaac
6360

actacggtat ttgttttctt cattaattaa acataacgta tcctcacttc tgtatccatc

6420

agtaaggcaa ggttctgtga taaagacctg cctgcattgt acgtctgtat ggttcctttc
6480

cctatcccaa atcttagatg catattttga ttgatgatga ttgtccttga tctaagagat
6540

gatgtgatat cctgctaccc cattcttcaa ccccctcaga cactgggatc attgtagaat
6600

gattcataac attccctggg accccaacac atggtgttct aacgtttctc agtctacaat
6660

acagtagtcc ctaaatcaca tgacaacttt tcctattgta gaaaaagtgc acgcagaggc
6720

gatgtggtgg ctgatctgcg gcagcagatg aggtcctgta ttctgctcct gccccacact
6780

gaccagatgc ccatttagac aaggtttccc ctgcttccac tcatagacac agttatctct
6840

cagactcaag gtggcctcca agtccttagc atccacttct tgtagaagca gtgccttccc
6900

ctttgcaggc cagtgacttt ttttagattc tcaaacacaa agtgactgat cattcacctt
6960

ttccttggcc tgattgacta taggaatacc agaatggtgg tggagagggg taaggagttg
7020

gtgtaatgtt tcacacctca aaggtattca ccaacactag gtcacaagct ctatctctct
7080

ctctttctca cacacacaca cacacacaca cacacacatt ctctggaagg tcatctcatc
7140

acagatttac agccctgtaa tcacccttga atgaacctct tcgggaggta cttgggaaag
7200

caaggagaga agacagcttg ctcattctgt cacatgcatt attggatttg ggcaattatt
7260

ttaatcccca tagtagaaac ttgctcatat ttccatattg gtacaaactc ttcagtctac
7320

ttaagtggat atccaaggaa ctatttaatt tccaatttaa tagtattatg gggaaaaaag
7380

acaataacaa aacaaatttg agtcaaataa atggtctcat atgaatggca tctgcaatac
7440

cttcaacaac taggcagaag atatatgatg ctatcatagt tttcttttaa aaatcacca
7500

aagcagtttc atctttaaca ccctacctaa gatttctaaa tgatagccta tcaaattata
7560

tccccattag gtatataata gctcctttct taccttagat aaatatattc ttgtttcaca
7620

agttttttta aaaggctttg cttgaaatta aaatctgacc aacaaactag agcagagata
7680

attcctcagt gtcaataggc ttccttggaa acttacccac atataaccaa tgcagcttca

7740

ctcgtgtata gaatatactg acagagaaaa agaaaataca gatgacatta caatcataaa
7800

caccctattt cctgttttta aaaaaacatg tcttcagaag tgcttttttt acaaaaattt
7860

attttaagtt ctgggataca cgtgcagaat gtgcagattt gttccatagg tatacatgtg
7920

ccatggtggt ttgctgcacc catcaacctg tcatctaggt tttaagccac gcatagatta
7980

ggtgtttgtc ctaatgctct ccctcccctt tcccccaacc cccgacaggc cccagtgtgt
8040

gatgttctcc tccctgtgtt catatgttct cattgttcaa ctcccacttc tgagtgagaa
8100

catgtggtgt ttggttttct gtttctgtgt tagtttgctg agaatgatgg cttccagctt
8160

catccatgtc ccttcaaagg acatgaactc atcctttttt atggctgcat agtattccat
8220

ggtgtgcatg tgccacattt tcgttatcca atctgtcatt gacgggcatt tgggttggtt
8280

ccaggtcttt gctattgtaa atagtgctgc aataaaaata catgcacatg tgtctttata
8340

gtagagtaat ttatattcct ttgggtatat accaagtaat gggattgctg ggtcaaatgg
8400

tatttctggt tctatatcct tggggaatcg ccacactgtc ttccacaatg gttgaactaa
8460

tttacactcc caccaacagt gtaaaagtgt tcctatttct ccacagcctc gctagcagct
8520

gttgtttcct gactttttaa taatcaccat tctaactggc atgagatggt atctcattgt
8580

ggttttgatt tgcatttctc taatgaccag tgatgatgag cttttttcat ttgtttgttg
8640

gccacataaa tgtcttcttt tgagaagtgc ctgttcatat ccttcaccca atttttgatg
8700

ggatttctca tttgttcttt ttttcctgta aatttgttta agttccttgt agattctgga
8760

tattagacct ttatcagttg ggtagactgc aaaaattttc tcccattctg taggttgcct
8820

gttcacttta atgatagttt cttttgctgt gcagaagctc tttagtttat ttagatccca
8880

tttgttaatt ttggcttttg tcacaattgc tttttgtgtt ttagtcatga agtctttccc
8940

catgcctatg tcctaaatgg tattgcctag gttttcttct agggttttta tggttttggg
9000

ttttacaatt aattctttaa tccatcctga gttaattttt gtataaggtg taaggaaggg

9060
gtccaggttc agttttctgc atatgactag ccagttttcc aaacaccatt tattaaataa
9120
ggaatacttt ccccattgct tgtttttgtc aggttcgccg aagattggat ggttgtagat
9180
gtgtggtgtt atttctgagg tctctgttct gttccattgt tctatatatc tgtgttggta
9240
ccagtaccat gctgttttgg ctactgtagc cttgtagtat agcatgaggt caggtagcat
9300
gatgcctcca gctttgttat ttttgcttag gattgtcttg gctaaactga ctctttttcg
9360
gctccatatg aaatttaaag tagctttttt ctaattctgc aaagaaagtc gatggtagct
9420
tgatgggaat agcattgaat ctataaatta ctttgagcag tatggccatt ttcatgatat
9480
tgattctttc tatccatgaa tatggaattt ttttccattt gtttgtgtgc tctcttattt
9540
ccttgagcag tgatttgtag ttctccttga aaaggtcact catgtccctt gtaagttgta
9600
ttcctacata ttatattctc tttgtagcaa ttttgacgtg agttcactca aaatttggct
9660
ctctgcttgt ctactattgg tgtataggaa tgcttgtcat ttttgcacat tgattttta
9720
tcctgagact ttgctaaagt tgcttatcag cttaaggagt tttggggctg agacaatagg
9780
gttttctaaa tatactatca tgtcatctgc aaagagagac aatttgactt cctgtcttcc
9840
tgtttgaata cgctttattt ctttctcttg cctgattgcc ctggccagaa cttccaatat
9900
tatgttgaat aagagtggtg agagagggca tccttgtctt ctgctggttt tcaaagggaa
9960
tgcttccagc ttttgcccat tcagtatgat ataggctgtg ggtttgtcat aaatagctct
10020
tattattttg agatatgttc catcgatact tagtttattg agagttttta gcatgaaggg
10080
atgttgaatt tcatcaaagg ccttttctgc atctatgaga taatcttgtg gtttttgtca
10140
ttggttctgt ttatgtgatg cattatgttt actgattagc atatgttgaa ccagccttgc
10200
atctcaggga tgaagccgac ttgatcgtgg tggataagct ttttgatgtg ctgctcgatt
10260
cagtttgcca gtattttact gaggattttc acatcaatgt tcatcaggga tattggcctg
10320
aaattttctt tttttgttgc atctctgcca ggttttggta tcaggatgac gctggcctca

10380

taaaatgagt tagggaggaa tctctctttt tctattgttt ggaatagttt cagaaggaat
10440

ggtacaagct cctcttagta cctctggtag aatttggcta tgaatccgtc tggtactggg
10500

cttttttgtt gttgttggtg gtggtggtgg aggtggtagg ctattaatta ctgcctcaat
10560

ttcagaactt gttactgatc tattcaaaga ttcaacttct tcctgattta gtctttggag
10620

gttgtgtgtg tccaggaatt tatctatttc ctctagattt tctagtttat ttgcacatag
10680

gtgtttatag tattctctga tggtagtttg tatttctgtg gcatcagtgg tgatatcccc
10740

tttatcattt tttattgtgt atatttgatt cttctctctt ttcttcttta ttagtctagc
10800

tagcagtcta actattttgt taatcttttc aaaaccccag ctcctggatt cattgatttt
10860

ttagaagggt ttttgtgtct ctatctcctt cagttctgct ctgatcttag ttatttcttg
10920

tactctgcta gcttttgaat ttgtttgctc ttgcttctct agttctttta attgtgatgt
10980

tacggtgtca atttcagttc tttccagctt tctgatgtgg gcatttagtg ctatatattt
11040

cccccttaac actggtttag ctgtgtccca gagattctgg tacattgtct ctttgttctc
11100

attagtttca gaaaacttct ttatttctgt cttaattttg ttatttaccc agtagtcatt
11160

caggagcaag ttgtttcaat ttccatgtag ttgtgcagtt ttgactgagt ttcttaatcc
11220

tgagttctaa tttgattgca ctgtggtctg agagactgtt atgatttcca ttcttttgca
11280

tttgctaagg agtgttttac ttccaattat gtggtcaatt ttataaagtg ctatgtggca
11340

ctaagaaaaa tgtatattgt gttgatttgg ggtggagagt tctgtagatg tctattaggt
11400

ccacttggtc cagagctgag tttgagtcct gaatatcctt gttaattttt cttcattaat
11460

taattgatct gttcattgat ctaatattga cagtggggtg ttaaagtccc ccattacatt
11520

acaactgtgt gggagtataa gtctctttgt aggcctctaa gaacttgttt tatgaaacta
11580

ggtgctcctg tattgggtgc atatatattt aggatagtta gctcttcttg ttgcattgat
11640

ccctttacca ttatgtaatg ccctgaaagt gctttttaaa aaaaaaaaaa aaaaaaaaaa

11700

aaaacaaaca aaaaccaggt aaataaggac gtatacacat ataaacacta aagcattagc
11760

gcaagacttc ttaatgtgtg acctttaatt tatgtctttc tatctagcgg aatgagctcc
11820

aaccttgata gctgaggact aaagcaagaa ccaggacaga gggaaaaaaa agatttgtg
11880

tagcacatag aaggttgtga gagtttctcc caatagaaaa gagttcctgc tggaattggg
11940

atctatcctt tcagagtgat cagtattttc tgagtctagg ataagagctg gaattttcct
12000

ggaggtagat cttccttttg ttctagaagg aaccactctg tgagccagtt tagtcataga
12060

tctataaggc tgtttgttgt ctgggggaaa gtctcatgga aagccccaaa ccaataaaat
12120

gatattaatg cagatgtttc agagtgacat gttgaacaca ttttttagtg aagttccttg
12180

gcatgcaatt tttggcagga ctaagccttt gggaaaccag aaccaccatg aagctgctta
12240

cacattgggc cctcctccag gacacctggc taagggcaga gaaatcagct agtgctctgc
12300

ttaccaatcc atgagttgct agccttccca gagaaagggc atctttttat aatttttaca
12360

taagctaagg tttctgagag acattataca taccgctagg ctatacaaga agaatctggg
12420

atctgatctg atctttactg ctcactagac aaagactatg gaaagagaat aaactgggat
12480

tggtttagaa gtacctgaac tgtttgattt tgttcagtag agagttaacc ctgaataacc
12540

cagacttaag gaaatatata gtcaactgtt gagagtgtct tatctaagca gtgggtgaga
12600

gagaaggagt gggcagacca tatgctatag tggagagtgg gagttcccct aaatgaatgg
12660

atgttcctac tgcagcggat catcctggaa taggaaagga aacatatcta ggactgcgaa
12720

aggaggctct aggtttgtga aaaactgaac ccagatttca ggtaaggaga caccagcaaa
12780

gatccataca agatggcagc aatttgaaga tcaaggagag agaaggtcac catgggatga
12840

acctgggaat ggcacaaata gtggacttaa aaatagtcat agatgctggg cgcggtggct
12900

cacgcttgta atcccagcac tttgggaggc cgaggcgggc ggatcacgag gtcaggagat
12960

cgagaccatc ctggctaaca gggtgaaacc ccgtctctac taaaaaaaaa atacaaaaaa

13020

attagccggg cgtgatggtg ggcgcctgta gtcctagcta ctcgggaggc tgaggcagga
13080

gaatggcgtg aacccgggag gcggagcttg cagtgagccg agattgcgcc actgcactcc
13140

cgcctgggcc acagagcgag actccatctc aaaaaaaaaa aaaaaaaaaa aaaaatagtc
13200

atagatattt tttgcctaca ttacaatcca ttcagtaatc tccaagcctt caatgaagtt
13260

ttcttaaaaa ccccgacttt catagctatg ttttggagaa aaaaaaaaga tgtcggaaaa
13320

catatgtgca gtgccattgt aagggagaag ccactgcaac ctgaagaagc cagcatctga
13380

gagtggagaa gcagtttgga cccaggtgac tggctaaagt ggcaaatgca gggaaacatt
13440

cgcggaatag aagaattaat tgaggactct cagaaatact cggtgagggc ccactccagt
13500

ggctcacacc tgtaatccca gcactttagg aggccaaggt gggtgaattg cttgagctca
13560

gggcaacaca gcaaaatcct atctctacaa aaaatacaaa aattaggcac gatagctcac
13620

atctgtagtc tcagctactt gggaagctga ggtgggagaa tcactcaagc ctgggagatg
13680

gaggttgcag tgagccaaga ctgtgctact atactccagg ttgggtgaca gagcaagatc
13740

ctgtgagaaa gaaaagggaa gggaagggaa gggaaaggaa agggaaaggg aaagggaaag
13800

ggaaagggaa gggaaaggga agggaaggga agggaaagga aaggaaagga agaaagaagg
13860

gaagggaagg gaagagggag ggagggagga aggaaagagg gagggaagga aggaatcagg
13920

aaggaagaaa gaagggaggg agagagggag ggaggaggaa gggagggagg aaggaaggaa
13980

ggaaagaagg aaggaaggaa caagggaggg aaggaaggaa ggaacaagga agggagggag
14040

gaaggaagga aggagaaaag gagataattg atatgtttaa atataaaaaa atacttcgca
14100

agaggaacca gataacctgg actaaaaaca attgttcaag taggaacttg aattaaatta
14160

attggtatta aaggaaatat tgatcctgga aggagggcta agtcatgggg acatgtgaga
14220

tactgaggaa gaatgaatgg ctagaaggga aaggaatgtg gtattgctga ctctgaagta
14280

ccacagaaaa ttttctctta gcccttaaaa ggaaaagctg aaagttccag tcttataaag

14340

ggaaaaacac attgctagtt gtaatctcat tttcatttcc tctctgttat gactgaaata
14400

ttgattaatt ataaatactg atgcagaaga atcaatattc agattcaata attaactatt
14460

taaaagttaa agtatattct tctaaaacac atgcaaagac caaaaaaatt caggttttgt
14520

acccttacct tttccttttt ctttcttact ttattttttt attttttatt tttttgaggt
14580

agggtcttgc tctgtcaccc agactggagt gcagtggtga gatcacaccc tacttcagtc
14640

ttgacctcct gagctgaaag tgatcctccc acctcagcct cccaagtagt tgggaccaca
14700

ggtgtacacc accatgccca gctaatttat ttttactgtt ggagagataa agtcttgcta
14760

tgttgctcag gctggtctca aactcctgga ctcaagcaat cttacagcct cggcttccca
14820

aagtgctggg agtacaggca tgagccacca cacctggcct gtactctttt ctttatcact
14880

cttttggtat tgactaggta tctacccatt ttcctgtctt ccggtcatct caactcagat
14940

gcctacaaaa agccaaaact gcctacagtt ttaaaaataa aattaaaatt ctacttcaga
15000

aaatgctgca ctattttatt ttaaataaaa ttgcatgtta cttcatgatg attagttcta
15060

acccaatcag caaaagtctt cttaaaataa taaaatctga agaaaacaag atgtaaacgc
15120

aagaatactt attagaatta ttaatacata attttcctgg aatgctaata atctaaagta
15180

atctaaggaa atgtttattt catcaacttg tgacttaatc taattacgtt gattgaaact
15240

gttaaaactg tgccaataaa agaaatattt gtaatgaaat catgtattta ctatttccat
15300

tgtaactgaa caatgcacag aaaatttccc tccaaaacac cactgccaag cactatggga
15360

accacttgtg tctatttacc tacaacatcc acgatacaac atccatctac aacatccatt
15420

taacataaat catggatttc agaaaagcta atgtttttaa taaatgagaa ttgattgtta
15480

aataagacaa cagtaaagcc accgaaaggt atatgcaaat tagtccatta agagtattta
15540

ttgttatttt atgtgagcac acagaactag agctaaagtt aaagatgtga gtagggcata
15600

tatcatttct tcttcacaaa gctgacactc tatcctgaca ttaaacaagt aaatgtaaca

15660

agttacacaa aagtgtatga gaagcccaga tgtgtatagt atggaaagag gcatcagtga
15720

agtgtgggac aacctcaagt gccctgatat atgtgtgacc gaagtccttg aatgagaagg
15780

gaagaaatat ttaaggatat aatgtcagat tttttccaaa tttgctgaaa ttataaaaac
15840

gcatattcaa gacattcaac aaatcataag cacaagaaac atgaagaaaa ctacaccaag
15900

gtatactata accaaatttt cagagaaaca atgataaaac aataaatgga aaaactttaa
15960

aacaaccagg aggaatgggg tgaggcatta tatacagaga taaagatgat agcaagttgc
16020

tagccagaaa tgactcacgt gagcagacag tggagcaata tctttaaagt tctggagggt
16080

aaaagaaaag ccctgtcaac taggtataat tttatatcca gtgaagctat cttccaaaaa
16140

tgaaggcaaa tcacattttc atatatacaa aaggtggaat aatttatcac caagatatct
16200

gcactataag aaacgtcaaa agtttttcag gtgaaggaaa aattatacca gaagtctgga
16260

cctatatgaa agaatgaaga gtcctagaaa taataaatat ttgtataata acaatatttt
16320

ttcttattaa ttaaatttat ttgaaagata agtgattgta tgaagaaaat aataacaata
16380

tattgttggg gtttataaaa tacagagaaa taaaaagtgc aaaaacaaca gcagaaaaaa
16440

aagcaagggg agaaatcgaa gtacacagtt gtattgttct tatactttac atgacgtggt
16500

ataacatcac tttcaggtag actgtgataa gttagagttg tgtatcataa agcctaaggc
16560

aactactaaa aacacaataa ggatatgtag gtaatgagct aacaaattag ataaaatgca
16620

accattaatt tgaaaattaa ttcaaaataa gacagaaaaa aagagagaaa agagaaaaaa
16680

gagcacatgg tacaaataat aaaagcaaga tggcagattt acacacaacc acatcaacta
16740

ccacatcaaa tttaaacggt ataaacatcc caattaaaag gcagggatac tttttcctat
16800

acggcaaatt agacgctttt agcctgcctc aggcataaag attaactctg tgacctttaa
16860

ttcaagaagg aaaatgagaa tccaccagaa ttgtaaagga aactccagat cctgggaagg
16920

caaatgtggg caaacagccc ctgtgatggc atctggctaa taagtgagtg aagccccacc

16980

acacaaaaga ggcagacagc ctccctctgt gactcacctt tccaatgggg atttgagtaa
17040

ccaaggccaa aggagagcac tttgtttctc ccaagccctg gagctaactt gggatgaaga
17100

tggagatgtt gtgagggaaa gacactggga aaacctgaag ccattttatc agacctggga
17160

acaagagcag gacgccatgt tttatcctgg tgcatacaat gtcagccatt atttggcaat
17220

ccggcaggat ggcctcacag gtattttggt ctcaggccag agattggagt gcctgctctg
17280

aagtggggta ggggcctcca cagccagaac tgtgaaaatc acctcagcag aaggcattag
17340

aactgtactc tccactgtgg caggcctggg gcaggagagc tgctacagct gcagtttctc
17400

ctggacaata agacttgcag ctacagccag cttggtaaca tggaaccagt ctccatgtgc
17460

tattgttggg tgccccagcc tgctcttctg agattacagt acagtggagt cctctctgat
17520

ccacacccag gcaaatctcc aggtattcag accaccctct cacctggatc agccacctga
17580

acccctccag ccttcctgtg catagattgt gctgcagtga agccctctcc actccacgtg
17640

taggcagatc tccagatatg tggagcaact gcttgcatgg actagcagtc tcagtctccc
17700

acccttcctg tgcagggatc ctgtgtgggt tatgggggaa tgggaggagg gggagttcta
17760

cttcacaccc aggcagatct cctggcattt ggagcactca ctcacctgga acagcagctt
17820

cactccctgc attcttcctg tgcatagacc atagtgcaac tgggccatct cgactctaca
17880

cccaggcaga ccttcaggca tccaaagcac ctgcttgcct agactggcag gctgagccac
17940

tccacccttc ctgtgcaatt atctgggtgc ataggggccc tctttgtttc aagcccaggc
18000

agatctctag gcatcaggag cacccaatct tctggattag gactttagcc caccccatcc
18060

ccatgcagag aacttggggc caaggagatt tctcagcttc ctgcctaaac acacttctgg
18120

gcacttggtg gctgcccact ggattcccca ttggccctgg tacttgtccc tgccatcaag
18180

ggacttgtaa atggacctgc caggtccagc cttacacatc ttgcccccca ccccacagcg
18240

ttaagggaac ttggaccact gttcactcta agaatcagcc tactgcctaa ggcaacagag

18300

agcttctctg taaacaaaga tcaggtatat aaccagatca ttggccatag gcagcgctta
18360

caaaaagcac catctactgg cttgtgggtt gaactgcaca gcccaatata aaatctgcca
18420

acagaagtgc ctagggatat aaaagcaaag tcaaaagacc ctacccagca ctctctgcag
18480

tcacacccca gggagtaagg gaaagggaaa gaagaaagaa acagtaatat cacaggagaa
18540

gaaggaaaga gaaaaaatcc tacctggagg aaaatagtta caaaaattat aactacagca
18600

tctccagatg agaaggaacc agtgcaagaa ttctagcaca acgaaaaatc tgaatgtaat
18660

aacaccacta aaggatcaca ctaacggctc cagcaattgt ccgtaatgaa tatggaaact
18720

aagaaatgac agaggaagaa ttcaaagcat gaattgcaaa gaagctgaag gagatctgag
18780

ctaaggttga aaatcaacac aaagtaactc ctaaagcaat tcgggaaatg caggaagaga
18840

taaacatctt caaaagaaat caatcataac ttctggaatt taaaaactca cttaaggaat
18900

ttcaaaatac aattgaacga tttctcaatg gactgaacca agcagaagaa tttcagagct
18960

taaaggctag tcttttgaac taactcagtc agacaaaaat aaagaaaaat aatttttgaa
19020

aatgaagagt ctttgaaaaa tatgagataa tttaaagtaa ctaaacctat aaattatcac
19080

cattcctgaa aaaaacggag gaaaagaaaa caacctggaa aatatatttg agggaataat
19140

tcaatattgt tttcctagtc ttgctagaga agcaaatatc cagatataag aaatctacag
19200

aacacttgtg agatactatg caaaatgaac atcaccaagg tgtattgtaa ccagaatgtc
19260

caaggtcaac accaaagaaa aaatcttaaa ggcagctaga gaaagaggtc agatcacata
19320

caaagggact aacagcggat gtctcaacag aaacctcata agccaggggt gattcgctgc
19380

ctatttttat cattcttaaa gaaaagaaat ttcggccggg cgcggtggct cacgcttgta
19440

atcccagcac tttgggaggc cgaggcgggc ggatcacgag gtcaggagat acagaccacg
19500

gtgaaaccct gtctctactg aaaatacaaa aaattagccg ggcgtggtgg cgggcgcctg
19560

tagtcccagc tactcggaga ggctgaggca ggagaatggc gtgaacccgg gaggcggagc

19620

ttgcagtgag ccgagatcgt gccactgcac tccagcctgg gcgacagaga ctctgtctca
19680

aaaaaaaaaa aaaaagaaaa gaaaagaaat ttcaaccaaa aataaatttc atatactgcc
19740

aaatcaagct tcataagtga acaagaaata aaatattttc caaagaagca agagctaagg
19800

aaatttattg acactagatc acctttacaa gagatcctta aaagagttct aaacatggaa
19860

acaaaagagc aatatctgct accacagaag cacacttaag tatatagcct acaaaccctt
19920

aaaaaaccca tgcaataaaa actaccaagg aaccagctaa caacttcatg ataggatcaa
19980

aactgcacat ctcaatatta accttgactg taaatggtct aaatgccctc acttaaaagg
20040

cacagagtgg caatttgaaa aaaaaaatac atctactgtc ttcaagagat ccatctcaca
20100

tttaataaca ttcataggct caaagtaaag tgttggagaa agagctactg tgcaaataga
20160

acacaaaaaa cagtaagacc aatcctagat aaaacagact ttaaatcaac aacagtaaaa
20220

aagaacaaag aagggcatta cataatgata agggcttcaa ttcaacaaga tggctcatgt
20280

atcctaaaca catatacacc caacactgga gcactcaaat tcataaaaca agtactttta
20340

gatctatgaa aagacagaca gccataaaac aatagtagag gacttcaaca caccattgcc
20400

agcattagac agatcattaa ggcagaaaac taacagaaat tctgcagttc aattgaccaa
20460

ctggacctaa gagacactga cagaatattt cacccatcaa ccacagaata tccactcttt
20520

ttgtctgcac acagaacata ctgcaggatc atccacatcc tcagtcataa agcaagtgtc
20580

aataaatttt tttaaaattg aaatcatacc catcatattc atgaaccaca gtggaatgaa
20640

aatcgaaatc aataccatga agctcccttg aagccataca attacatgga aattaaacaa
20700

cttgctcctg aaaaaatttt aggtaaacaa aaaaattaag gcagaaataa aaaacattct
20760

ttgaaatgaa ttaaaacaga gacaaaacat accataatct ctgggataca ccaaaagcag
20820

tgttaagagg aaagtttata gcactaaatg cctacctcaa aaagttagaa caatctgaaa
20880

ttaacagtct aacatctcac ttagaggaac tagaaaaaca agaacaaact aactccaaag

20940

caagtagaag aaaataaata actaaaaata gatcagaact gcataaaact gagacacaaa
21000

agcccccaaa aaatcaacaa aaccaaaaga tgtttctttg aaagaataaa caaaatcaac
21060

ctaccgctat ttagattaac aaagaaaaca aaaggtgatc caagcacaat tagaaatgac
21120

aaaggtgaca ttacaactga tcgcacagaa ataaaaagat ttccagagac tattatgaac
21180

acatctttgc acacatttta ctgtagaaaa tttatgtttc tatagtattg gaaattctgg
21240

ccagggaaat caggcaagat aaacatataa agggtattca aataggaaga gaggaagtga
21300

aattgtcact gtttgcagat gacatgattg tatatttaga aaacgccatt gtctcagccc
21360

aaaaactcct taagctgatg agcaacatca gcaaagtctc aggatacaaa atcaatgtgc
21420

aaaaatcaca agcacttcta tacacgaata ataaacagag agccaaatca tgagtgaact
21480

cccattcaca attgatacaa agagaataaa atacctatgg gacagaacag aggcctcaga
21540

aataacgcca cacatctaca accatctgat ctttgacaaa cctgacaaaa acaagcaatg
21600

cggaaaggat tccccattta ataaatggag ctaggaaagc tggctaccat atgcagaaaa
21660

ctgaaactgg accccttcct tacaccttat acaaaaatta gctcaagata gatgaaagac
21720

ttaaacataa gaccaaaaac cataacccta gaagaaaacc cagacaataa cattcaggac
21780

ataggcatga gcaaagactt catgaaaaaa tgccaaaagc aattgcaaca aaagccacaa
21840

ttgacaaaag ggatctaatt aaactaaaga gcttctgcac agcaaaagaa actatcatta
21900

gagtgaacag gcaacctaca gaatgggaga aaatttttgc aatctatcca tctggcaaaa
21960

gtttaacatc cagattctac aaggaactta aacaaattta caagaaaaaa acaaacgacc
22020

ccatcaaaaa gtgggcaaag gatatgaaca gacatttctc aaaaaaagac atttatgcgt
22080

ccaacaaaca aatgaaaaaa gttcatcatc actggtcatt agagaaatgc aaatcaaaac
22140

cacaatgaga taccatctta tgccagttag aaaggcaatc attaaaaagt caggaatcaa
22200

aagatgctag tgaggctgtg gagaaatagg aaggatttta cactgctggt gggagtgtga

22260

atcagttcaa ccattgtgga agacagtgtg gcaattccct aaggatctag aaccagaaat
22320

accatttgac ccagcaatcc cattattgtt tatataccca aaggattaca aatcattcta
22380

ctataaagac acatgcacac atacgtttat tgtagcacta ttcacaatag caaagacttg
22440

gaaccaaccc aaatgcccat caatgataga caggaaaaag aaaatgtggc acatacacac
22500

catggaatac tatgcagcca taaaaaagga agagttcatg tcctttgcag ggacatggat
22560

gaagctggaa accatcattc tcagcaaact aacacagaaa cagaaaacca aacaccacat
22620

gttctcactc aaaagtggga gttgaacaat gagaacacat ggacagaggg aggggaacat
22680

cacacaccag ggcctgtcat ggggtagggg ggacaaggga agggataaca ttaggacaaa
22740

tacctaatgc atgcatggct taaaacctag atgacgggtt aatgggtgca gcaaaccacc
22800

atgacacatg tatacctatg gaacaaacct gtgcattctg cacatgtatc ccagaactta
22860

aagtataata aaaataaata aataatccat tttataactt ctagaaataa atcataactc
22920

atatagcatg tgagaataca gtatcaaaat atcacatgta tcccataaat atgtacaatt
22980

attatgtatc agtaaaaaag aaccccttct agtttagaag atatccctgt gtcttatcat
23040

ttagggtcgc atatactttc ttttttataa cattcattca tcttaatttt ttggttgtta
23100

tatatttctt aaagagtgtt ccattagccc agaaactatt gtttattcac tattgtattt
23160

ttcatgccaa ataagtcaaa ataaagattc aataagcatg aaaaaaaaga aaatttatgt
23220

ttctaggata aattcctaga aacacataat ctcccaagat tgatttagaa agaaattgaa
23280

accctgaaca aaccaatatt aagtcctaaa cttgaatcag ttatttttaa aaaaaaaaaa
23340

cctaccaacc aataaaaacc tccagaccag atggattcac agctgaattt taccagacat
23400

acaaaaaaga actggtacca atactactat aactgttcaa ataaatcaag gagaagggac
23460

tcctccctaa ctcactcaat gaagccagca tcaccctgat accaaaacct ggtaaagaaa
23520

caatgagaaa agaaaactac aagccaatat tcctgataaa cataaacaca aaaatcctca

23580

acaaaatact agcaaaccaa attcaacagc acatcaaaaa gttaatcaca atgatcaagt
23640

aggcttcatt cccgcgaggc taggttggtt tgatatgagc aaatcaatgt gattccccac
23700

ataaccagaa ttttttgaaa aaggtataat catccaatag acatggaaaa agcttttgat
23760

acaatccaac atcccttcat attcaaaaac cctcaagact aggcattgaa ggaacatgcc
23820

ccaaaataat aagagccatc tatgacaaac ccacagccta catactgagt gggcaaaagc
23880

tggaagcatt cccttgagaa ttggaacaag gcaaggatgt ctactcttac cactcctgtt
23940

taacatagta gtagaaggcc ttgccagagc aatcaggaaa gataaaaaaa caaaaaaggg
24000

cattcaaata ggaaaataca tcaacgtatc tctttttgct gatgatatga ttctatacct
24060

agaaaatcct aaagactcca cccaaaggct cctggaacta ataagggact tcagtaaagt
24120

ttcaggatac aaaatcatgt ataaaaatca gtagcatttc tgtacaccaa taatgtttca
24180

gctgagagcc aaatgaagaa tgcaatcaca tttacaatag catcaaaaaa ctaaactaca
24240

aaagaaaaat aaaatatttg tgtcatttta ttacctagaa atacaccaaa ccatcaaacc
24300

aagaaggtaa aaatctctac aaagagaatg accaagcact accaaaagag atcatagatg
24360

acacaaacaa atggaaaagc atcccatgtt catggatagg aagaatcaat actgttaaaa
24420

tggtcatacc ccccaaagca atctacagat tcaacattat tcctatcaag ctatcaatgt
24480

catttttcac agaactagaa aagattcttc taaaattcat atgaaaccaa aaaaaaaagc
24540

ccaaagccta aactaaaaga acaaagacag aggcatcaca ttacccagct tcaaactata
24600

caaggccaca gtaaccacaa tattctggca ctggcacaag aacagacaaa tagaccactg
24660

gaatagaata tagaactcgg aaataaagct gcacacctat agccatctga tcttcaacga
24720

agttgacaaa aacaagcaat gaggaaagga ctccctagtt gataaatggt gctgggatag
24780

ctggtgagcc atatgctgaa gaatgaaact gaacccctat ctttcaccat atacagaaat
24840

taattcaaga tggattaaag atttaaatgt aagactccaa tctttaagaa tcctagggga

24900

aaatctagga aacactatta tggacattgg ccttgggaaa gaatttatga ccaagtcctc
24960

aaaagcaatt gcaacaaaaa caaaaattta caagtggggc ctaattaaac taaaaagctt
25020

ctgcacagca aaggaaacta tcaatagagg taacagacag cctacagaat gggagaaagt
25080

actcacaaac tatgcatccc acaaaagtct aacatctaga acctacatgg aattaaaaca
25140

attaagcaag ctagaaacaa ataattccac taaaaaaaag gcaaaaaaaa aagacacgaa
25200

cagatacttc tgaaatgaag acatgcaagt aaccaaaaac atatgaaaac aatgctccac
25260

atcactaatc atcagagaaa tgcaaaccat gagataccat ctcacaccag tcagaatggt
25320

tattatttag aaaatcaaaa ggcaacagat gctggtaagg ctggggagaa aagagagggc
25380

ttatatattg ttgggggact gtaaactagt tcagccactg tggaaagtag tttggagatt
25440

tctcaaagaa cttaaagtag aattaccctg acccagcaat cctattactg gatatatacc
25500

caaaagaaaa caaattattc taccacaaag acacatgcat tttatgttca tcacagcact
25560

atttgcaata gcaaagacat gaaatcaacc taggtgctca tcgacagtgg cttgcatata
25620

gaaaatatgg tacatataca tcatggaata ctatgcagcc acaaaaaata atgaaatcat
25680

gtcctttgca acaacataga tgcagctgga ggccattatc ttaagcaaat taatgcaata
25740

acagaaaacc aaatacccat gttctcagat ataagtgaga gttgaacatt tgatactctt
25800

ggacataaaa atggcaacaa tagacactgc aaactaatga aagggggagg gtggcaggtt
25860

tgaaaaacta tcaggtagta tgctttgtac tgagtgatgg gattaataat accccaaacc
25920

tcagcatcac ggaatacacc catgtaataa aactacacat gtactcccaa atctaaaata
25980

aaatttgaaa aataaaataa aaaattaaaa atgagaggtg gagccaagat ggccgaatag
26040

gaacagctcc agtctacagc tcccagcgtg agcgacacag aagatgggtg atttctgcat
26100

ttccatctga ggtaccaggt tcatctcact agggagtgcc agacagtggg tgcaggacag
26160

tgggtgcagc gcaccgtgca tgagctgaaa cagagtgagg caatgcctca ctcgggaagt

26220

gcaaggggtc agggagttcc ctttcctagt caaagaaagg ggtgacaaga cggcacctgg
26280

aaaatcaggt cactcccacc ctaatactgt gcttttccaa taggcttaac aaatggcaca
26340

ccaggagatt atatcccgca catggctcag agggtcctac gcccacagag tctccctgat
26400

tgctagcaca gcagtctgag atcaaactgc aaggcagcag cgaggctggg ggaggggcac
26460

ctgccattgc ccaggcgtgc ttaggtaaac aaagcggggg gaagctcaaa ctgggtggag
26520

cccagcacag ctcaaggagg cctgcctgcc tctgtaggct ccacctctgg gggcagggca
26580

cagacaaaca aaaagacagc agtaacctct acagacttaa atgtctctgt ctgacagctc
26640

tgaggagagt agtggttctc ccagcacgaa gctggagatc tgagaacggg cagactgcct
26700

cctgaagtgg gtccctgacc cccgagctgc ctaactggga ggcacccccc agtaggggca
26760

gactgacacc tcacacggcc aggtacccct ctgagacaaa acttcctgat agcagcattt
26820

gcggttcatg aaaatctgct gttctacagc catcgctgtt ttgcagccac tgctgctgat
26880

acccaggcaa atagggtctg gagtggacct ctagcaaact ccaacagacc tgcagctgag
26940

ggtcctgact gttagaagga aaactaacaa acagaaagga catctacacc aaaaacccat
27000

ctgtacatca ccatcatcag agacaaaaac tagataaaac cacaaagatg ggaaaaaaac
27060

agagcagaaa aactggaaac tccaaaaatc agagcacctc tcctactcca aaggaatgca
27120

gttcctcacc agcaatggaa caaagctgga tggagagtga ctttgattag ttgagagaag
27180

aaggcttcag acgatcaaac tactccaagc tacaggagga aattcaaacc aatggcaaaa
27240

aagttaaaaa ctttgaaaaa aaattagaca aatgaataac tagaataacc aatgcagaga
27300

agtgcttaaa ggagctgatg gagctgaaag ccaaggctcg agaactacgt gaagaatgca
27360

gaggcctcag gagccgatgc gatcaactgg aagaaagggt atcagtgatg gaagatgaaa
27420

tgaatgaaat gaagcgagaa gggaagttta gagaaaaaag aataaaaaga aatgaacaaa
27480

gtctccaaga aatatggggc tatgtgaaaa gaccaaatct acgtctgatt ggtgtacctg

27540

aaagtgatgg ggagaatgga accaaggtgg aaaacactct gcaggatatt atccaggaga
27600

acttccccaa tctagcaagg caggccaaca ttcagattca ggaaatacag agaacgccac
27660

aaagatactc ctcaagaaga gcaactccaa gacacataat tgtcagattc accaaagttg
27720

aaatgaagga aaaaatgtta agggcagcca gagagaaagg ttgtgttacc cacaaatgga
27780

agcccatcag actaacagct gatctctcgg cagaaactct acaagccaga agagagtgga
27840

ggccaatatt caacattctt aaagaaaaga attttcaatc cagaatttca tatccaccca
27900

aactaagctt cataaatgaa ggagaaataa aatactttac agacaagcaa atgctgagag
27960

attttgtcaa caccaggcct gccctaaaag agctcctgaa ggaagcacta aacatggaaa
28020

ggaacaacca gtaccagcca ctgcaaaaac atgtcaaatt gtaaaggcca tcaaggctag
28080

gaagaaactg catcaactaa cgagcaaaat agccagctaa catcataatg acaggatcaa
28140

attcacacat aacaatatta actttaaatg taaatgggct aaatgctcca actaaaagac
28200

atagactggg gaattggata aagagtcaag acccatcagt gtgttgtatt caggaaaccc
28260

atctcacatg cagagacaca catgggctca aaatacaggg atggaggaag atctaccaag
28320

caaatggaaa acaaaaaaag gcaggggttg caatcctagt ttctgataaa acagacttta
28380

aactaacaaa gatcaaaaga gacaaagagg ccattacata atggtaaagg gatcaattca
28440

acaagaaggg ctaactatcc taaatatata tgcacccaaa acaggagcac ccagattcat
28500

aaagcaagtc ctgagtgacc tacaaagaga cttagactcc cacacattaa taatggtaga
28560

ctttaacacc ccactgtcaa cattagacag atcaacgaga cagaaagtta acaaggatac
28620

ccaggaattg aactcagctc tgcaccaagc agacctaata gacatctaca gaactctcca
28680

tcccaaatca atagaatata catttttttc agcaccacac cacacctatt ccaaaattga
28740

ccacatagtt ggaattaaag cactcttcag caaatgtaaa agaacagaaa ttataacaaa
28800

ctgtctctca gaccacagtg caatcaaact agaactcagg attaagaaac tcactcaaaa

28860

ccacgcaact acatggaaac tgaacaacct gctcctgaat gactactggg tacataatga
28920

aatgaaggca gaaataaaga tgttctttga aaccaacgag aacaaagaca caacatacca
28980

gaatatctgg gacacgttca aagcagtgtg tagagggaaa tttatagcac taaatgccca
29040

caagagacag caggaaatat ccaaaattga caccctaaca tcacaattaa aagaactaga
29100

aaagcaagag caaacacatt caaaagctag cagaaggcaa gaaataacta aaatcagagc
29160

agaactgaag gaaatagaga cacaaaaaac cgttcaaaaa attaatgaat ccaggagctg
29220

gtttcttgaa aagatcaaca aaattgatag accgctagca agactaataa agaagaaaag
29280

agagaagaat caaatagaca caataaaaaa tgataaagca gatatcacac caatcccaca
29340

gaaatacaaa ctaccgtcag agaatactac aaacacctct atgcaaataa actagaaaat
29400

ctagaagaaa tggataaatt cctcgacaca tacaccctcc caagactaaa ccaggaagaa
29460

gttgaatctc tgaatagacc aataacaggc tctgaaattg aagcaataat caatagttta
29520

ccaaccaaaa agagtccagg accagacgga ttcacagccg aattctacca gaggtacaag
29580

gaggagctgg taccattcct tctgaaacta ttccaatcaa tagaaaaaga gggaatcctc
29640

cctaactcat tctatgaggc cagcatcatc ctcatagcaa agcctggcag agacacaaca
29700

aaaaaacaga attttagacc aatatccttg atgaaccttg atgcaaaaat cctcaataaa
29760

atactggcaa accaaatcca gcagcacatc aaaaagctta ttcaccatga tcaagtgggc
29820

ttcatccctg ggatgcaagg ctggttcaac atacgcaaat caataaatgt aatccagcat
29880

ataacagaac caaagacaaa aaccacatga ttatctcaat agatgcagaa aaggcctttg
29940

acaaaattca acaactcttc atgctaaaaa ctctcaataa attaggtatt gatgggacat
30000

atctcaaaat aataagagct atttatgaca aacccacagc caatatcata ctgaatgggc
30060

aaaaactgga agcattccct ttgaaaactg gcacaagaca aggatgccct ctctcaccac
30120

tcctattcaa catagtgttg gaagttctgg ccagggcaat caggcaggag aaggaaataa

30180

agggtattca attaggaaaa gagggagtca aattgtccct gtttgcagat gacatgattg
30240

tatatctaga aaaccccatc gtctcaggcc aaaatctcct caagctgata agcaacttca
30300

gcaaattctc aggatacaaa atcaatgtac aaaaatcaca agcattctta tacaccaata
30360

acagacaaac agagagccaa atcatgagtg aactcccatt cacaattgct tcaaagagaa
30420

taaaatacct aggaatccaa cttacaaggg atgtgaagga cctcttcaag gagaactaca
30480

aaccactgct caaggaaata aaagaggata caaacaaatg gaaaaacatc ccatgctcat
30540

gggttggaag aatcaatatc gtgaaaatgg tcatactgtc caaggtaatt tatagattca
30600

atgacactcc catcaagcta ccaatgactt tcttcacaga attggaaaaa actactttaa
30660

agttcatatg gaaccaaaaa agagcccaca tcaccaggtc aatcctaagc caaaagaaaa
30720

aagccagagg tatcatgcta cctgacttca aactatacta caaggctaca gtaaccaaaa
30780

cagcatggta ctggtaccaa aacagagata tagatcaatg gaacagaaca gagcccacag
30840

aaataatgcc gcatatctac aactacctga tctttgacaa acctgagaaa aacaagcaac
30900

ggggaaagga ttccctattt aataaatggt gctgggaaaa ctggctagcc atatgtagaa
30960

agctgaaact ggatcccttc cttatacctt atacaaaaat taattcaaga tggattaaag
31020

actgaaatgt tagacctaaa accataaaaa ccctagaaga aaacctaggc aataccattc
31080

aggacacagg catgggcaag gacttcatgt ctaaaacacc aaaagcaatg gcaacaaaag
31140

ccaaaattga caaatgggat ctaattcaac taaagagctt ctgcacagca aaagaaacta
31200

ccatcagagt gaacaggcaa cctacagaat gggagaaaat tttcacaact tactaatctg
31260

acaaagggct aatatccaga agctacaatg aactcaaaca aatttacaag aaaaaaacaa
31320

acaaccctgt caaaaagtgg gcaaaggata tgaacagaca cttctcaaaa gaagacattt
31380

atgcagccaa aaaacacatg aaaaaatgct cgtcatcact ggccatcaga gaaatgcaaa
31440

tcaaaaccac agtgagatac catctcacac cagttagaat ggcaatcatt aaaaagtcag

31500

gaaacaacag gtgctggaga ggatgtgtag aaataggaac acttttacac tgttggtggg
31560

actgtaatat agttcaacca ttgtggaagt cagtgtggcg attcctcagg gatctagaac
31620

tagaaatacc atttgaccca gccatcccat tactgcatat atactcaaag gactataaat
31680

catgctgcta taaagacaca tgcacactta tgtttattga ggcactattc acaatagcaa
31740

agacttggaa ccaacccaaa tgtccaacaa cgatagactg gattaagaaa atgtggcaca
31800

tatacaccat ggaatactat gcagccataa aaaatgatga gttcatgtcc tttgtaggga
31860

catgcatgaa atttaaaatc atcattctca gcaaactatt gcaaggacaa aaaaccaaac
31920

accgcatgtt ctcactcata ggtgggaatt gaacaatgag gacacatgga cacaggaagg
31980

ggaacatcac actccaggga aagttgtggg gtggggggag gggggaggga tggcattagg
32040

agatatacct aatgctaaat gacgagttaa tgggtgtagc acatcaacat ggcacatgta
32100

tacatatgta acaaacctgc acattgtgca catgtaccct aaaacttaaa gtatcataat
32160

aataataata attaaaaatg aaaggcagag atttcagatt ggattaaaac agaaaaatgc
32220

aagtatatgc tgcctaaaag aaacacgctt taaatataga gacacaaata gcttaacagt
32280

aaaaagatgg gaaaagatac accatgttaa catagtcaaa agatagctgg agaggctata
32340

ttaatatcaa agtagatttc agagcacaga ttattacctg agataaagag ggtcatttca
32400

tattagttaa gtggttagtt taagagaata caatcctaaa taattacaca ctaataatag
32460

agttccaaaa tacttgaaaa aaaatcaagg atagagaaat ccacaattac agctaaagat
32520

tcgaacaaca ctacaaacca attttatcta attgacattt gtataatact gcacctatca
32580

tcaacagaat agacgttttt ttcaagtgca tacagaacat ttaccaagat atattctggg
32640

ccatataaca agtctcaata gattttaaat tactcaaatt gtatgatgtg tgttctctgt
32700

ttacaacaga attaaatttc taaaataagc aacagaaaca tatctggaaa atcttcaaat
32760

atctgaaagt taaataacac acttctaaat accccatgtt ataaacaaga aatcaaaaag

32820

aaaattagaa ggtattttga actgaatgag aatgaaagga cagcatacaa aatttgttgg
32880

aggcagctga ggtagtattt agcttatgta gcttgaggta ttatttagct cagtttatga
32940

agaaataatt taaattattt ttacagacat gactgtcctt ggagaaaatt ttatagaata
33000

aacaaaaatg ccatgacagt tgtttttctt ttcttttttt ttttttttta aatcttcagg
33060

ggggtaaatt ttaagacctc cagtggatgt ctgaaacctc agatggtacc gaactatata
33120

tactacatgt acgctacctt tttccagtgt aaacatacct aagataaaat ttaatttata
33180

aattagtcac agtaagagat taacaacagc taatgttaaa atagaacaat taagtaaaat
33240

aagggttact tgaactgtga tactactgaa ataccacaac agttgatctt gtaaatgaga
33300

tggttactaa gtgactaata agtaaggagc gaatacattt ggacaaagga atgattcaac
33360

ttctgagcag aatggagtgg gatggcaaga gattttatca cactactcag aacagcgcac
33420

acttcaaaat tcataaactc tttatttctg taattttcca tttttgaacc acagttgacc
33480

ttaggtaact gaaaccatac aaagtgaaga ataagggggg actacggtat agctactatg
33540

tgagtttagc aaggttgcaa gatacaagac caatgtacca aagtcaattg catttctata
33600

cactagtgat gaacactatt aaagtgaaat tcttaaaaaa taatgccgtt ccccttagca
33660

tgaagagaat atttagagat aactatgaca agtaaaagat ctgtactctg aaaactataa
33720

aatagtactg atcaaaaaat aaagatgtaa ataaatggag agatattccc tgttcatgaa
33780

taggaagatt gaatattgtt aaggtttcag ttcttcccaa attgaactat aaaattaatg
33840

caatgccaag caaagtctta gcgagcttgt tttgtagaaa tcaacaagct aattctaaaa
33900

ttcatatgaa aatacaaagg actttgaata gccaaaacaa ctttgaaaaa gaatagcaaa
33960

gtcaaaaact ttaactattt gatttccaga tttattataa gtctatagta atcaaggcaa
34020

tgtggtatta gcatcaaaat agatttaaaa aatcaatgaa acagaataaa aagtccagaa
34080

atagacccac acatgtaatc aattgatttt tgacaaagtt gcaaaggcaa tccagtggag

34140

aaatgataat ctattcagca aggagtgctg gcacaactga ctaaccacat gcaaaaaaag
34200

tcttcaaatg aacttttaat tattccttgc accatataca aaaattactt caaaatggat
34260

gataaactgt aaaacctaaa agtatataac tttttaaaaa ctggagaaaa tttttgtgat
34320

ttgggctata caaagatttc tagggtataa cagcaaaaac acagtgcata aaataagaaa
34380

ttgattagtt agttgatggt cactaactat ccctgccagt attcatagat taatcgctag
34440

aagtagatga atattgacaa attatcctcc agaaatgtgt catcactaac agagtatgag
34500

tgtctatttc actgtcatat tttctatatt ggataatata attcaacaga tatttgccaa
34560

tataaaaggc cctttatcag gtaccttatt gtttctttag tttgtcttta ttattataaa
34620

agtaataaag tgattttgta aaaataataa aaagtgattc tatgtgtatg tttattaccc
34680

actggtgttt cttcttttgt caaatgtcat ttcatgccaa agattaaaat gacaatggcg
34740

agctccaaca caacggttga ccttccagag gtaggagatg aaatagcaga gagtttcatg
34800

aactagacct ccaggctttc cctgtctata tttactccat gcagtaagct aaggacactt
34860

cattaatttc tgcaataaaa accttttagg ctattttggg caaatactct gattttctgc
34920

cctgtctctg ctgacactcc aaggtgtcat ccaggtatac agcaggtaga aggccttatt
34980

gatctcagga agagggaatg gtggaaattg ggtatttact cagccccttg aactgccctc
35040

tgtccacttt ggtatgcaca atgatgtccc tcccttggct gcttgcttct cccagtgcct
35100

gtagctgatg caccattggc tttgggcttg atttgggatg gtggtcctct ctgtgacttt
35160

gtccccatta tagctacagg cccctgcatt gtggttttga ttcctatttg gttgctggct
35220

gaggcagtcc accctgcagt ctggacttgc tgtcacttca ttgtactgcc atagcccacc
35280

ctggtctact tctgagttat gctttcaacc aacttccatg tcccaattcc atattccatg
35340

tctcacagga accaagacag atcagagtta ggaatctttt ctctcactaa ctgtgcttat
35400

agacaccatt actctgatgc agcttgagaa gcattcactg ttaggggtcg cagatgttac

35460

ctggaacaaa gtctgctctc accatatccc tgaaaatacc taacatattc tcaagttgtg
35520

gaaagtcagt ggcagggaga actcataacc cagctattta gccacacatc ttccctacct
35580

acatccccct tcctcttccc acaatctccg agaccaggaa aaaagcacac tttcaaactt
35640

ctcaagatta aagctctatg atccattctg atatgtcaaa ggaggagctc tatcacatag
35700

aaatttctct ttctctcaac aaagcccagt ttttaaaatc attatctaaa gatgcgagaa
35760

aagtcatctg gtttgagact caatgctgat tggtgatttt cttatttatt aattaataca
35820

ttcaagaaat atttcttgtg aaaattactt ctcaggtgtg gaaatgcaat ggcatacaag
35880

gaaaaaaaag gtcttgacct catagagttt aaattctagt acagaagaca tacaatgtaa
35940

taatcaaatc cagtttgtaa gttttatgaa aataaagcag ggtaaagtga cagagaaaaa
36000

tagaataaga gaggatatag gtgttatttt caaaaagtaa tccaacaaat ttctctgtga
36060

aataaaatct aacctgccac ctgtatgagg tgaaggaagg atccaagaac aagactagag
36120

agtttccagg cacagggacc aaaggctttg gggtgggaac ctgcttgaag agtccaagga
36180

ctggcaggag gttagaacag agctagaaca gagagtgaga aagtagagat ggttacagat
36240

aaagtccaag agacaggtag gggccagatc atgttgaact ttgtaggtta gctgtttcaa
36300

cgtgtcgtct ggggaaccag agggatccct gatatcctca ggggttccat aaggtcctcc
36360

ttttcccaaa tacttttttc taccaggttg aattttcttc atatagttca atcaaaacaa
36420

tatattgcaa cagattgaat gcagaagaaa atatgaaaat ccagctgtct tttattaaac
36480

caaatattca agagatttgc aaaaatgtaa aacaatgtca ttctcctcac tgtattattt
36540

ttgtttttga aaatatatat tttttcacgc caggtgcagt ggctcacacc tgtaatcacg
36600

gcattttggg aggccaagac aggcagatca cttgagccca ggagtttgag agcagcctgg
36660

tcaacatggc aaaacccacc tccacaaaaa aaattgttta attaactggg tgtggtggtg
36720

cacacctata gtcccagcta ctcaggaggc tgaggtggga ggatcacctg agcccaggag

36780

gcagaggttg cagtgagcca tgatcatgcc actgcagtct agattgggca acagcaccca
36840

tatatatatg ggtgctcaaa tatatatata tttgagacag ggtctcactc tattgcccaa
36900

tatataaaaa atatgtcatt tacattaaca tataataggt ttttattgtt gttttaaaaa
36960

tatttaacaa ataaatatgt ttacatattc ttagttataa ttttaaataa attacatatt
37020

tacagatata gcccacgtaa acaaaagctc tttggagccc ttcctcaata atttttcaga
37080

gtgaaaggga ttctgagatt aaaatgtttg agaatcattt ttataaaaca taagatttta
37140

ttcaaaggat aatacgatcc atctggaagt cttgaacaga gtgctgatag actctattgc
37200

ttttagaata tccatctggc cgggcgcggt ggctcacgcc tgtaatccca gcactttggg
37260

aggccgaggc gggcggatca cgaggtcagg agatcgagac catcccggct aaaacggtga
37320

aaccccgtct ctactaaaaa tacaaaaaat tagccgggcg tagtggcggg cgcctgtagt
37380

cccagctact tgggaggctg aggcaggaga atggcgtgaa cccgggaggc ggagcttgca
37440

gtgagccgag atcccgccag tgcactccag cctgggcgac agagcgagac tccgtctcaa
37500

aaaaaaaaaa aaaaaaaaaa aaagaaagaa aaaaaaaaga atatccatct gactgctgag
37560

tgggaaattc actgcagaga aggaagagat gatgcaagaa aatggtagag gttgttgcat
37620

tagtccaggc aagagatgac tgtggcttgg cctcagatga tataagtgga gatggttaga
37680

agtcacatag aataaatatt ttaaaggtag agccaataga atgtgttgat taaatgaatg
37740

tggggtatga ggagaaaagg ggccaaggtt gactccaggg attctggtct aagcccctgg
37800

ttagatagtg gcacaatcta ctgagaagga ggaaagaggt ttcttttttt tttttttttt
37860

tttaaacaca gaatctcgct ctgtcaccca ggctggagtg cagtggcgcg atctcagctc
37920

actgcatgct ccaccttccc aggttcacgc cattctccct cctcagcctc ccgagtagct
37980

gggactacag gcgcccgcca ccacgcccgg ttaatttttt tgtattttta gtagagacga
38040

ggtttcaccg tgttagccag gatggtctcc atctcctgac ctcgggatcc gcctgcctcg

38100

gcctcccaaa gttctgggat tacaggcgtg agccaccgca cccggccaga aagtggtttc
38160

taaggtacta ccagctctct ctctccctgg agcaagaagc cttatgaatc tatcttgcac
38220

tttaaaagaa tgcaaggaaa aaagagataa attaaaaggt attaatttaa tatttccaaa
38280

atgttacccc tgttacattt catatagttt catcttaact atctaaaaca agtgcataga
38340

aaaatactgg aaggaaatat caaaatgtta acagtggtct tcactaatta aaggtttgcc
38400

agaggatttt attttctctt gcataggttg cttctatatc tcacaaactc ttacagtgag
38460

tgtttattac tgtaattttt ctcagaagta aaattatttt aggaaaaacc attcaaagct
38520

cagatgcact atgtggtgtc tatgaacttg aagatacctc catttgaaga gatgttctcc
38580

cttgagaaga tgagtcggtg gaaagtgccc tatattatgc atcaagcata atttccatct
38640

tggttcatct gggatatttg tacctttaca aataattaat ttttaattaa tttttatacc
38700

tttaaaaata atcacagata attattttct gccacaatag tttcttcaat tttttaatta
38760

aaagtcaatc caccttagac tcgactcttt ggggtgataa aaaagagttc ttgaataagg
38820

attctgaaag tccctaatac agccctcaaa gaagtaaact aagtttaaag ttattttctt
38880

gtttcttatg tctccatgct tgcagagtaa atttaatgcc aggttttgga gaaatctatc
38940

ttagaataaa aacacagtta tcagtgaagc tacacatgtg acacttaaat aaacagggaa
39000

cagatagaag gaagttctca ctgatgctgg tgaactggat gtcctagtga gcgtgccacg
39060

tcaccagagt tgataagcaa ctgactgaaa accttggtct gggaagctgc tgtggtcaga
39120

gatcaaagtg agaagggaaa gggaaatgcc ttttccacac ctaataagat tcatttttct
39180

tgttttcctt caccaactcc acaagactgt caaggtaatt ttttggtttg ctactaagct
39240

aaaaaaggac tcaaccattt taaagttagt aagacatggg aaataattac ctttcaagac
39300

tccataagag tcttcagagt cagggaacat tgaccacctg cttgattata aaaaatagtc
39360

tttgttcacc aaggtagccc tatagcttcc tagtgtctac agcatttaca catctacttt

39420

ctccttcaca gcctctacct ctggaataga gattcttctg gaatattcca gaggttgtaa
39480

ggggtgtgac tgttgcccca tggggaaaat aggggctgct tggtaagttt ctaggcagtt
39540

tttttttttt ttcataagtg gacccattct gaggtctgtc ctttcccctc tctgaaagga
39600

ccagaacctg ttgttctggg tttaattaag aaaagaagac atgcactcat ggcgttcttg
39660

atctaaggca tccactcacc cacaggaaga gtctttggca agacggtctc ccgaggtgtc
39720

ttctagagct atagttccat gttcaataag ggagaaatta cttaaactca tctcacaact
39780

tgattacatt ctcacaataa tctagataat gaatacatgg gagtagtaaa tgaaagttca
39840

ctttctacta aaatttcaga aagaaaataa gtaagtctgt tctctagaaa ggaaaaagtt
39900

ttctcattta tgtaaaatga atgagattag tcatcatgga ggtaaatcta gagattgtaa
39960

atctttacct ggagaacttt gacaacagtc aaaactctaa tcaaaagaat ggtgtgattt
40020

ctaggaggca ggattacaac gtaagggaaa atctagccaa ttttacttac cctgtctgaa
40080

ccaaattggg tggccaatgt gaagcataga aaagagaaac ccaagattca taaaatggta
40140

atatttgtca aactacagcc tatgaggaat ttggatgaat gaagatagat tattaagatg
40200

tgtgtgttca gttaggatag gagtaactca cagagcctca cctcctaata aatatccttc
40260

caatttttcc ataggctttt tcattcatca tagtagtcac aaagtgactc tgtaattact
40320

aattcagttt ctcagctact tcagtattaa tagaaggtat ttcagataca aacacaatct
40380

ggtcattaaa aataaaaatc tcttacacct ctaaacctag catgttggga agccaaggca
40440

ggacaattgc ttgaagccag gagtttgaga caaacctggg caacgtagtg agatcatgtc
40500

tttgcaaaaa caaatataaa acttagctga gcatggtagc atgcacctat agtcctaggt
40560

actccaaagg tttcagtagg aagatcactt gagtccagga gtttgatggt gcagtaagtt
40620

atgatttcac caatgtactc cagtctgagt gacacaacca gacttatctc taaaaataaa
40680

taaataaata aataaatatc ttactttcaa taaatataaa tcctctactg gtattatgtg

40740

gataacaaca catactttca ataggttgtg atgaagatca aaatagagaa catatttaat
40800

acatctgaca tatgcctgac ccataataaa tatttggtaa ttctcctacc tttttgcctc
40860

tctttgcttt tattcaaatt acaacaaata aaagatacag agtaatatta tccagctgat
40920

atggagaacc tgctctctaa cactgactac ctctgtcctt gggcaagtca ttgatctctg
40980

gccatcggat tcctaagata aagcaaggat ttggactaga tgatatctga tttcttttct
41040

atctctaata ttaaaagaga ctgagtattt aagattggtc cttggggtgt cattttgatt
41100

ccattaagcc tgagattcaa ttattctgct caattgatct ttcttaataa taaacaaaat
41160

caaacaactt agacaacatt caggttaaca aggcagcaaa ttagcaacac ataacagacc
41220

ttctcttaac ttcccattaa aacacctacg aggatataca aaaagataaa aactggcacc
41280

agtattgtaa actgaaaata agagaagaag atatgtcaaa gttcagaggg actttccact
41340

aatcgcatca ttaatggata gggattgaga aatataatct tggatggcac aaacactacc
41400

ctttgaacca gaacagcaac aaccccaaag caagaggaag accttggctc tcccccactg
41460

gccgaggtca acttcaggac aaggggttgc cctgaccgga aaaataggtg ccatccccca
41520

ctgaaataga gttatcatct gagcaagcca acatcataac ctctcttttt tccccatcag
41580

cagttctatt tctgtatcca tttagagtct acaattccca gtgaggtact aatcttgaag
41640

tagaataaca gttggttaac agtgtagtga gtactaagta ggggttggga caaggtgaat
41700

actgaaaact gagtgtggtg agcgttagga agcagggagg aagagtgtta tctttcatgt
41760

actgatattt catacgaatt gcattttaat cattgtcaaa aattgcaact ctttcagcag
41820

atgtatgctg tgcccatact gtatcatata caggaccttt aaccctcctt ggctagagct
41880

aggaaggaaa aattgtcagt ccctgagtgg gcaatgagag agtcaaagac cacatcactc
41940

accctgtcta ttggtaaccc aatactggat ctttgaaatt attctgcaac atttagggaa
42000

tgagatattt atactgaagg ctcaagggag agcttatttt ttaaaattat cactatgaag

42060

acaaatagag caatgcttta tttatgaaac aatttcttca aagtttttac tagctaaata
42120

ttctcaggct cttttagtgt ttggtggcca tttcccggtt gattccactc acatgggatg
42180

ttttgatcat agcttttcac aaaaatttgc caagataccg tattaactag tgtaactata
42240

tcacccttaa tggttctgtc aactggcata tagcagatat aattcgtcaa ctttgcagca
42300

gaatgcattc ttaatttgaa agtaaaaacc aagtggaaat ccttgttgtg ttcaataatg
42360

cattagacac agtatagact gttaccagaa ggatttggtt ctgctttctc ttcaataagt
42420

catgttaaaa gaatgactgc aatattccaa actgttatat tattgcaatc aaactgatgc
42480

tggagaggtt tcgaatctgg atatttcctt ataaaataac aacctgaata aatgaaaagc
42540

aattcataac tttcccattt ttcactttac cattaggata gcagatagcc atattttagg
42600

tcctttattt ttcctcctaa actgatgtat ataatttaac ataaataata ttttagaaat
42660

atgttaataa ttaagaaatt tctccctttt catattttta gtggtttccc tacaggaatt
42720

tagaggcggc agagggggaa ggagtggtgg tggtcatagc taacacttgt agtgacactt
42780

accatgtgtc acatattatg ctcagcattt tacttacatt ctcttgcaca atcttgatga
42840

tcattctagg aaatagatat gtaattacct ttattctaca aatagggggt gaaaatatca
42900

ggcatctgga aagcgttgtt tcagaaagaa aatctgcaag agtggaaagg tattggctca
42960

tagaatgttg taagaaatta aattttatgt ataaccaaga gagtttctac cttaattaaa
43020

tttaacaagt atttattgta tgcccacaag gcaattttct aggcaattag ggacaaaaca
43080

atgaacaaaa catataacac catcatgtga cttactttct tcagtctata aatgaacaaa
43140

tgaacaggtg atggtttcca tgaaaaaaaa acatgataaa agatttgaca gtgatggtaa
43200

atgatcaagg atacagaggg tgtgaaaaca atgcctaagg atgatctctc tgaggagaga
43260

tctgctttta ggtagagaac tcagtgaaga acagaagtga gatgtgaaaa actggggaaa
43320

agagttccag acagaataaa cagcagttgc aaagatcctt ggatgaaaac aaagttggta

43380

agttcaagaa agagaaagag ggcaatgagg ctggattaaa atcagcaagg ggaagagtga
43440

agaaagtgta gagatgtagt catctttgta agccatcgct ttgtaagggc tttgagttgt
43500

ggtaaatgct ttgggtttca ttaaaaaata aagaaggtgg gaagcttgag aggtgttcca
43560

aatctttgtt cagctctctt cctggacacc tagccaggcc acacttcaca gcatacgttg
43620

cagctcagtg tggtcatatg actgagagcc agcctgtgga gtgtgagtgg aagtgaggtg
43680

cactacttcc aggattgaac aataaaaatt atccacccac gcatctctgt gtcattcctc
43740

ctttcttgcc ttcagggata gaacggtgga tgacagcctt caaatccata tgttgaagat
43800

ggcagagcca ccatcacctg gaaacctaat gaccacattg agaaaagcct ggccaacaac
43860

cagttcactc accaaaaact cttacgtgag agagaattaa cttccatttt gtcgaggcat
43920

aacacacgtg ggtctatgta ttatcacaac ctagtctacc ataacttccc cagtatccat
43980

tctgcccttt tgctattgca atagaaccct taattttcag cttagcactt gagtacacgg
44040

aataaagatt gtatttctaa gcgtcccttg cagttaagtg tggtagcaca actaattttg
44100

ggctaaaatg gtatgaagtg gaagtgatat acatatatat atattcccaa aggggcatag
44160

cctcctcttt tccacttccc actggcctga aagcagatgt ggtgatgtcg agtcaccttg
44220

aaccatatgg acaagtttta tactctatgc ataacagaaa aataagatga agtagcttgg
44280

gtccctgaac tgtggaggca tcattaacct cacgatctta ttaagcctgc agctatttgg
44340

agtacaaaaa ttaattcaag atggattaaa gactgaaatg ttagacgtaa aaccataaaa
44400

accctagaag aaaacctagg caataccatt caggacatag gcatgggcaa ggacttcgtg
44460

actaaaacac caaaagcaat ggcaacaaaa gccaaaatag acaaatagga tctaattaaa
44520

ctaaagagct tctgcacagc aaaagaaact accatcaaag tgaataggca acctacagaa
44580

tgggagaaaa tttttacaat ctacccatct gacaaagggc taatatccag aatctacaaa
44640

gaacttaaac aaatttacaa gaaaaaatca aacaacccca tcaaaaagtg ggcgaaggat

44700

atgaacagac acttctcaaa agaagacatt tatgcagcca aaaggcacat gaaaaaaatg
44760

ctcatcatca ctgggcatca gagaaatgca aatcaaaacc acaatgagat accatctcac
44820

accagttaga atggcgatta ttaaaaagtc aggaaacaat aggtgctgga gaggatgtgg
44880

agaaatagga atgcttttac gctgttggtg ggactgtaaa ctcgttcaac cactatggaa
44940

gacagtgtgg tgattcctca aggatctagt actggaaata ccatttaacc cagccatcct
45000

attactgggc atatacccaa aggattataa atcatgctgc tataaaaaca gatgcatacg
45060

tatgtttata gtggcactat tcacaatagc aaaggcttgc aaccaaccca aatgtccatc
45120

aatggtagac tggattaaga aaatgtggca catatacacc atggaatagt atgcagccat
45180

aaaaaaggat gagttcatgt cctttgtagg gatatgaatg aagctagaaa ccatcattct
45240

gagcaaacta tcacaaggac agaaaaccaa acaccacatg ttctcactca taggtgggag
45300

ttgaacgatg agaacacttg ggcacagagt ggggaacatc acataccggg ccctgtcgtg
45360

gggtgggggg aagggggaga gacagtatta ggagatatac ctaatgtaaa tgatgagtta
45420

acgggtgcag cacaccaaca tggcacatgt atacatatgt aacaaacctg catgttgtgc
45480

acacataccc tagaacttaa agtataataa taataataat ttttaaaaaa acagaaaaaa
45540

tatagaaaag aaaaaaaaag aaaaagaaaa tggagtagct tgggtctctg acctgtggag
45600

gcatcattaa cctcgctatc ttgttaaacc tacagctatt tggagtttca ttttcaatag
45660

ttacctatat gctgaataat ctagaagtca tcaggaggct ttaagtaggt gaaacataat
45720

tcaacacaga ctggtttata tgttacaact ctggctgctg ggtggaaaat aagctgtggg
45780

ggagggcaga gtaaaatcag ggaaagcagt taaaatgcta ttgcagtaat ctaggtgaca
45840

gatgatggag caagctaagg aggaaagaag tagtaggatt cagagttttt ggaaggtggt
45900

gccaatggta tctaatatag gcttgaggta aggtgattgt cagaaagaga agagaaaatg
45960

actctggttt ctgacttaag caattgggca aacaggccac taactgagat ggagaacatt

46020

gggaaagaag caagtttgag gaagaaaatc aaactttttt taaaattttt agctagttat
46080

tcaaatggag atagcaagtg acattctatg gaatctagaa acttccagct tggagtttag
46140

gaaagatgtt aggctagaaa caaaactttg ggaaaatcat taataaattt ataatacaat
46200

gtactagcat aatatcaaag ggctccattt atggcaagaa tccagataca atagaggcaa
46260

tatcatgttg gattgaccca acataacatt gcatattttt ctaattggca acaaaatatg
46320

acttaatact tacatgatat tcttgtttat ttataattgg catgtagaac aataattggt
46380

atccagttct gggtccttca ggatgacatg atattaagag acaggacaga tattattggc
46440

ataatacaca agaagacctt ggtgaattat cttctacttg tgtttttaat cacgttaact
46500

atagaagcat tcctgtctat gttgtactat ttgtctttca agggactggg aaaggcttca
46560

tcactgggca ctatgcttgc tgaagaatag aaggaaatgg catttgcact tccaaagtca
46620

caggataaaa gagatgcctc ccactgtcat cttctaccct ctttgtgggt acttattctc
46680

tgggtaatta ttgtctaatt ttcctgtttg atattagcct cacccttagc acctaggaag
46740

gaattgaatg aatactttcc tgaggctcta agactcacat aaagcccaca tgcataatgg
46800

gattaactat ctagactgaa tgacttttcc cagttaatgt aagtactatt tacaagagtc
46860

agcttaatat ttttgaaatg ggaagccttc cttgcctctt gcacctgctg gtggctccag
46920

gcgttctttg actgtggctg cataactcca atctctgcct ctgcctttac ccctctattc
46980

ttttctgtgt ctcttttaag gatacttgtc attgaatata agacccacct agacaatcca
47040

ggatggtctc atcttgagat ccttaattaa ttacatctaa aaagactatt tttccaaata
47100

agggcatgtt cagtttccag agattagaat gtggacatat attttgggga gtcaccatta
47160

aacccactgc agtctcaagt cttgtcaatc attagaacca cctagataac ttgataaagt
47220

acagattctc aggtcccatc cttagccatt cgaattccat agaccgggtt tgtatgcatg
47280

tttgtgtttt attgtagata agaactaaat ctaggtttaa aaaagaaaat cactaggtga

47340

ttctgatggt tattgtcccc tattctttca agatcttaat tttggaaaat gtcattgtta
47400

tctgttacat catcaatgca gtgaaagaca atagcatatc aagaatggca agatggtatg
47460

ggggaaattg tgggcttgga aatcaaccaa ggctgaacct gaaatttggc gatatcttgt
47520

gttattactt aaattctgtg agcatcagtt ttctcacctg tagaatggaa gtctttacat
47580

tatagtgtta ttgtgaagat taatgagata acgtatgtaa aaccccagca cgtgctcagt
47640

gatcaattaa atggaaacta ttattagcaa aaggtggatc tggcagccta tgttaatcca
47700

tgtagctata atacaatcat ccaaactgac tcattaatca tatgtcatga tttgcttaat
47760

tttgatatat aaaaagaata gtatttataa attttggaat tatgaacagg ttctataaaa
47820

caatgagaga gtgtaatgga aaatattacc tctttaatta ttatagtgat gatttttcaa
47880

cttccatata gtttccaaaa ccctaggtaa atgaaaaatc tctaatgttc aattgattaa
47940

gtggcttcac ccaaacttct cctatggact atattctgtc tccccaaaat tcatatattg
48000

aacgtcacaa gggtgggatg ccaatctaat aagacagtgg ctttataaga aaacaaagag
48060

agagagagat cttaagcaat ctttctctgc catgtgagga cacagtgaga aggcagccat
48120

ctgcaaacca aaaagagacc cctcaatgtt gaacttccag cttccagaac tatgagaaat
48180

atacatcatt taagccactc tgtttgtggt attttgttat ggcagcccta gcagatgaat
48240

acaccccccc atctagccta tatccctttt tagcatgatt cttagaaaat atgtcctcat
48300

tgttgtcatg aacatttccc aggcctgcta cttctcctta attctaatag atggggtaaa
48360

ggaaagttat gtgttacatg ggagacaaca gagagtgttt cctattaatt tgtaagaaac
48420

caagctcatc aagcacaaac tatcatcagt atagacttct ttaatgggat tatcagaccc
48480

atagtttctc ttaattcagg atggatattt ttccttgagt aatttttttt cctctttctg
48540

tgagtaaatt ctctggccat ctaggtaaat ttattgctca tacttctgtt caaaaagctt
48600

tgatattcac cgttgctttc agaatggccg gaggacccat caattcataa ctgcaaagac

48660

ctccaattcc ttaactccta ctcctctttg tgtaaacttt tgatccaaac tgtatgctga
48720

tgtcctactg accagcattg ttctttcact ttctcacatt tttccatctc attccttcta
48780

cctcacatgc cctgctagca cagtcaacta tatccaatct aaacaacagg tcctacttca
48840

cctcctacca gctccaagaa ggctttgctg accactccag gaaatgactc agtccatgct
48900

agctttatcc ttgttgtgtc acattactct caggactttg cacaggataa atgttcataa
48960

atattaatac ttcttggtct aaatatattt ctctatgtaa agagcccttg aacttctaag
49020

gcacttcttg tctgtatcag caatttgaaa cgaatcacga accttctgag gacagcccat
49080

atgtatgttt gtgttattta cattttctat tgtgatttca ctttctgctt ctagtgtgag
49140

ctacattggg agcttcttga ggactacatc ccatgaatga gatttggaac taggaaagac
49200

tacaggttcc aacctagact tccctgctgc taaaattaca ctgtttagta agtcacttca
49260

caaccaattt tatgagtaaa tggaaatgat aatccctgtc cttcagggca tggtggctca
49320

cacctgtagt gccaactact tgagacctgg gtaggaagat ctcttgagct caggagtttg
49380

agtccagcct ggacaacata gtgagatgcc atctctaaaa caaaacaaaa ccataataag
49440

tacctcatag gatttatatg aggatagaat gatgtattgt aaagtatcta gcacagcatc
49500

tgcctcatgg taaattgtca atatgtggga gctttattct tatgtcccag tactcccaac
49560

acttttgtag gttaaatatt tttaattgtt aatttcaata tatatttcta tataaagata
49620

aaatggggga agggatgcca tatttaaatg tttttgtaag gcctcttcta actcaagatg
49680

tcctgatgct ataaacttta aagtgtatca gaacgcatag cacagaaggc agcacagtgt
49740

tgaacaataa tgctctttaa agtagctgct gcatttccca catttctttc attgttcaga
49800

aaatggctca ataagcccac tagatgattt ggaattcatt caggtcttgt gagcccattg
49860

tttgcaattc tctaactgtc attttacagt cacattggac ccaaaggata gagtctgaaa
49920

atttattgaa tgatggaatt ttccagttac agagcagaat cctaataatt cagagacatc

49980

tcactcctgt agaggtccca gaaaaaggtt aacgtgaaca caattgcctg tcaaaatctt
50040

ccaattataa actagttact agttaccttt tcctgcagtt gtgtacacaa attaatattt
50100

aatttggatg atcttcaccc aaaaatgaag gttataggaa tctgtgctgt tgatttaaat
50160

tagaatatta tactcctttt gtttttcctt tccctatatt gattccatgc ctgagcagtt
50220

aaaacctttg tggaacagag acatgacatg gtgtcactgt tggcagcaca gaaagacaca
50280

aaggtagaga gatttcagga acagcaaaat ccctgtttct tccaaatata gcctttcctg
50340

gtttttactt agcctgaagt ttctacttct ggaattatat ttgtggaaat aatagttttt
50400

atgcactagt tttttagaat agcatgtaca cacatacaaa ggcacacagc aaaaaaatgt
50460

aaaaaacatg tggaggtata gaaggtctac aaagcagtcc tgaaaagggg aaggaagtaa
50520

aagttttaaa ttctgtttga ctctgttgct gagaatataa attagtacaa ccactacaga
50580

gaacagtttg gtttggaaac agttttagtt tcctaaaaaa actaaaaata gagctaccat
50640

acactccagc aattccactg ctgggtatat atgcaaaaga aaggaaacca gtatatcaaa
50700

gaggtatctt cattatcatg tttgttatag cactgttcag aatagccaag atttggaagt
50760

aacttaagtg tccactgaca gacaaatgga taaagaaaat gtggtacata tacacaatgg
50820

agtactgttt agccataaaa aagaaggaga ttcaggtatc tgcaacatca tggatggaac
50880

tggaggtcat catgttaagt gaaataagcc aggcacagca agacaaacat tgtatagagt
50940

cacttattta tgggatctag aaattgaaac aatttaactc atggacatag agagtagaaa
51000

gatggttacc agaggctggg aagtgtagtg agaggttgag gagacatggg gatggttaat
51060

gggtacagaa aattagaatg aatgaataag gactagtatt ttatagcaca aggtgactac
51120

agtcaataat aatttaattg tacatttaaa aataactaag agtatagttg gatttgtttg
51180

taacacaaag gataaatgct tgaggggatg tatacctgat ttccatgatg tgattatcat
51240

gcacagcatg cgtatattaa aatatctcat gtacctcaca agtatgtaca tctgctacta

51300

catatccaca aaaatgaaaa ttagaaatta ttatttttaa ttctgcttga aaattttgtc
51360

ctgtgagaaa acattttaaa tggttcattt tcaaggcatg atcaaagtac tggcagccag
51420

cctgtggatg taacaaacca catggctcat gcacctagaa ggtcacaata aactgacaga
51480

atgtagagga ggagtcagcc cataaaaggg aagaaagttt cgttattggg aaatcaaaat
51540

ttaagcggga aaggggaccg gggtatgggc ttataagggg ataatgaaac taaggttatg
51600

tctgggaaga ttgtaacccc atagtactca actgatgagg aactggaaga gggatttgcg
51660

tgctgggaga taaattacct gctataactg ccctgggtgt gcctgcctac ctgacaccct
51720

atctggaaag actgccatta aaagtctcac ttccgctatt ctttgtgtct ctgagtccat
51780

tctttgggtt tggatggatg aatgtgtgtt tctcacagtc tggtgcagcc ctgagtaaaa
51840

accaagatct tttatgcctt aaatgttttt aaaaaaatga aaaagcataa atctatgttt
51900

tggtgtgttc atagtgtgca gtcattctta ggagcaactg tgaatttaca aaacctctcc
51960

attctctccc ctttcactcc tacacctact atctgttcag ccctgcgtct cttagaattt
52020

tttaaactat attctctctc atctcctagc ttctactttc tttcattttc cttcagggct
52080

aaactgtgat aaaagatgga tcctctgctc tgcttagcaa ccccgaaagc tctctgatgc
52140

tgcaggagac agtctcagct cctttgagtg gccttctggc cccttgtaca aagtcctcac
52200

aaactcatca ccttctcttc tccccaagca cactgcattc caaccacacc ctttgtcttc
52260

ccattcccta aacaggtctc aaacattgaa tcatgctaca aacatcttca agccttttca
52320

catgccctct ggatgtcctc ctccttttct gcctagtaaa agcatccaaa tccttcctgg
52380

cctcacccag taccacacag attctacagg cagaatcagg ttatatccta gactctcatg
52440

atttcagagg cttaagctct tcctcccaca aagttatccc atcatgggat tatgcaacac
52500

aaattatgac tgaagcaaga ggcaaacaga agcccaagtc ccacctggct caggtttctc
52560

tgcaccccac ccagtccagc tgagcatgac cacctctgtt cacaaggtgc tcctacacac

52620

caccatcatc ggtgttggtc ttctaagttt ctcaaaagaa cccaaggcta gaaggccaga
52680

ctcaaattta gaattctcag acttcttgaa gatccaccca ggaattttaa gaacctacca
52740

tcacctgact tttcctccat accggctctg cccagtactg tgagcataga tacaaggata
52800

tcttagccgg catgtccaaa ctcaagccac accaccacca ccattacgtc ctccaagcac
52860

catctcttgg aaaatgagac tatctggaca aaaaattccc tggccatgga tacttagaat
52920

atggtagaaa ggaggccaca ggatgtaggt ggccacatct ctgtggtccc aagactcctt
52980

gcattatgga gagaagtgta gccacctgca cccgggtctt ctgacaaaag gagggggcag
53040

aaatgggagt cggggaagtt aggatagcat ctacggacag gggcttctct tgtcctgatt
53100

taagacgatc aggcagtggt gtgtcttcaa ataaaaacaa agacaagtct cactctgtga
53160

actgtttgtc tctctagaac actggatctc aaccaggggt gatttgttct ctatgggata
53220

cttggcaacg cctggagata ttttgagttg tcgtgatttg agaagattgg gagttgctac
53280

tgtcatccag ttggtagaga ccaaggatgc tgctaaaact cctacagcac ataagacagt
53340

tccatcacaa caataatata atattagttt ggtacattac ttctaatggc gaaaaccaca
53400

attatctttg caccaaccta ataataataa tggaaaacat tttctaaaag ttaatttgta
53460

aattggttat atataatatt gttgtcaaac agatcttaaa gcaatggttc atgtcactta
53520

actgcacaaa agatttaatc acacacacac gcacacatgt gcatgcatgc atgcacacac
53580

ccagcttatg aactaaagtt caactttcta agcttgacat tcaatatgac tcttgatctg
53640

gagcctaccc tgtttatcaa tttaatcttt ctctatgatc tccttatgta acccaaagcc
53700

tgtgtctatg ctactatcag acttaaatat tttagactgc catatggcta tctcccatat
53760

tttccctatc aatttttggc atactcaacc ccattcctga tagtgaatta agtcttcagc
53820

tggaccttct aatgtaaaat gttcaaattc agaaactaac tacacttaaa acagaattac
53880

ccactagaat acatcaaggc tgtaccacta agataataat aactagctct tgggattgga

53940

aggataagac aacgatacca tgtataaatc actgtgaaga tgttaccatt atgatgtccc
54000

tacctcataa tagcactcaa tgaaatgtct gtgtctctcc tataattaaa aaggcattcc
54060

aagtaggaaa atagcttttg caaataaatt aaagtaaggc tatttacagt aaaataataa
54120

taataataat aataataata atgtagcaca atagaagctt atttctcact cacataaaat
54180

ccaaaacaga tgttcctgct tggtggcagg agtggaagtt aggctgtgtg gatgaaggaa
54240

gactgcttca tagcttcacg cagagccgta ggctggaagt ctccatcacc ttccacatgt
54300

ggcttccatg atcatcctgt gtgtcaacat ccagtgggca gatgggaaaa gagaaagcac
54360

agggtatgtg ccaccacttc ttaaccactt ggccaagaca tggtacacat catctcagct
54420

catattccac cggcaagaac tagtcccacc tggatgcaag ggagggtagg actggaaaat
54480

gagggccctg gctgggcaga gcttctcagc aacaacgcta cactatggaa aggacattag
54540

ctatctttac cacagcaaag cacaagggta taggagatga agcaaagaca gagagcagtg
54600

ggcaggcttg cctggctaga atatgaattg gagcttcgtg gcagatgaga acggttaggt
54660

aaggaaagac agccagataa caggagactc atgactctat cattatgaat gttttctatt
54720

tgagagacag ctgtgaatcc gaccttgaag aagagttatt tgataaataa tgatgtttaa
54780

aacagacact gtagccacaa agcaattttt gtttcattac gaaatgaatc tggtttttat
54840

tcaggcatat cttccttatg tggaaattca taactttaaa aacgaatcat tgcatcacta
54900

agaactcttt tcacagcatt ctcttacccc ttccaagtac ccttttcaat caatgtgctt
54960

tctccttcct tcaattgctt ctatgggctt gcagaatctg ctgctgtgtt tgttcctttg
55020

ctgttctcct ttaattgctg tctctgctgt gtctggttct cctcctttct tttggcccag
55080

actatttatt tctaaaaccc atcagcattc tgtgagggtt aggattcatt tcagagaaac
55140

tcaaggaaat tacctctggg tgatttttca gcccaggtat ttggtgcagt taaaatgtca
55200

tttatttctc tagacttgca tgatgttgtg caaaacatga aattaatctt aactacacaa

55260

cattcaactc ttgaaatttc aagaccttta ttaaaatgtt gaaatagaat tttcctttaa
55320

atatgttaat agaagagact tgttgctttc cctatgtgat aactttcttc aactcccaca
55380

tcatctttca ttacccaaag aaacacacct ttgattttat aacggaagtc aatggaaaaa
55440

aaacttgcta tatagaaata cacatttaca gtaaagtttt ttatgctgat attcactcag
55500

taatagaatg tatacccata taatctattg ttctagcttt ccatagatgt caaaaatgtt
55560

gtcagcctga tgctagaata tcactgctat tagtgtgcca tctagtggct gatgttgtga
55620

tattttccat tctttgttga ggtagaagag gctttaagtg acatatggaa agtccagtga
55680

gattccttct ttgatacaat gcagggatga cacagttgta taaagccaat ggccaaatgt
55740

tgagtcatct gatgatgtgt agaggatcgt ttgtaagttc ttacttagcc aatataatta
55800

agaaatgaag ctattacaag aacaattaaa tatatttgtt agctcgctgt atcatgatgc
55860

acaaaccata tgtgcattaa ccatatacca agaaccagaa tacaataaaa tgcatttgac
55920

cctaaattta aattagattt aattttactc ttgttattca gatggtactt taggatctta
55980

taggtctagg ctttgaatac taattctcat tgtgtagtat acaaaaatgt atgtttgagc
56040

tttctggttg cttggattta aaactaaaag tggttttgta tttataaagg atcaaaccta
56100

tcatgcatag tgtgagtggc agcatatcca tcattttagt tgattctttg tagtattata
56160

accataatcg catgatctta tatattgctt tattccaata ttaaagattg agagataatg
56220

ataatgattt tatcttttag ttatagaatt gccttcaaat gccttgtttt ataatcatct
56280

caaagtcaaa caagagattc ctttaatcgt tacatcattt ttcataggca agtaatgagg
56340

gactgaatgg ttttgctgtt gaagacagaa ggacaaaaaa gtaaaatcat attctccaaa
56400

catatacaga tagtattgaa aagaagtgta agaattagtt attgtatgtt tgcccttgga
56460

aataacagtt attattctgt tattattttt aaacatctat tttatttatt attattaaag
56520

gagcagttat cactaatgca atcatttcag aaaaattttc tttaaagcaa acacaggaaa

56580

acagaaacaa aaaccacctc catttaattg agttcttagt atgtgccacg tgatttaaat
56640

actttacata cttttcttac ttagtcctca cacaacctga taaagtttca ttttataaag
56700

gaagaaacca ggctgagtca ttgaataact tattttaagc cacattgaca gtaaagggca
56760

gcttctggat aaaacctaag tgtgtccaat tttgaagccg tgcttataaa tgtgacaaaa
56820

aatactttat aaccatgtca ggaaaacatg cattgtacct actactctac aaagtctagc
56880

caagagcaga actgacatgc attgtcttat ttgccatagg caaatcaaca ggaaagcgaa
56940

gtataaacag ttaactcaga ctagtatatt acccagggtt ttatactggt taatctgcca
57000

ttattccaga ggcaagacta caaatcactt ggataacatg atcctgcaat gtcaaggtag
57060

gtaaataact tgtgagagtt tttaaatgat gccatcacca ctgcagaaga gtatatttac
57120

tccttctgct ttccttaaaa tggctagcat taggctcttt attcagaaat ttttatatta
57180

taacatacct tatcttatat cattatcttc tcccaacaat ctattaggcc aacgtgctag
57240

atcagtgatg ttactttaag gccctctcaa tctttttgtt gcaaacagac cactgtgcca
57300

cgtaaagatt aaagtgtaaa gactgagact tcaagaataa attttaatgc tgtgatcata
57360

taatatataa attttgccaa actacttttt gagctgcatg ctaattaaat acaatgctta
57420

acaaccactg atatacacac acacacacac acacacacac acacacacac atagaactac
57480

ttttacatag ctaaaccaag aaatgggaaa gcttgttagc ttgtttctag attcctggat
57540

tcataattca agggcattgt gatagatctc ctttgttgtt cttcctatat ccacccctcc
57600

ctccccaagc caatggaacc ttaagttcgt tcaggtggct gctgacatct ttccctctgt
57660

cgagtattta tggatgggtt taaaccaatc gtgattcatc ccagggccag ggataaatta
57720

tgattggtct aaaccagcct tagtaatctt agtaatcttg taactttgtc tgggaatgaa
57780

gtgacaaatt ttggtcaatg agatgaaagc agaagttttt gaatgagcct ccagggcttg
57840

tctttctgat taacacagat aacatagaaa agaacttttg tctttctgat taacattgat

57900
aaacttacag aagacagcct ttgcctttct cttctcttct gcctgcaatg aagacatagt
57960
ccctggaggc acagcagaca tttgtgacca taagatgaca agcctgagga caaaagtcaa
58020
cttttatttt ttgagacgga gtctcatcct gtggcccagg ctggaataca gtggcatgat
58080
ctcggctcac tgcaaccaca tccccggttc aagggattct catgtcttag cttccctagt
58140
agctgggaat acaggcatgc accaccatgc ccagctaatt tttgtaattt taatagagac
58200
ggggtatcac catgttggcc aggctgatct cgaactcctg acctcaggcg atctgcccac
58260
ctgcacttcc caaagtgcca ggattacagg cgtgagccat ctcacctgga caaaagtcaa
58320
ttttctaagg atgatgcaac aggatgatag gtctctgaca gcatcgctgc gttcattgaa
58380
tagctgtatc aggacgtttc ttacctttgg attccttgca atgtgacaaa aagttttatt
58440
tatataagct attgtttgta ggggtttctg ttacttgcag cctcatgtat taggttgaac
58500
catatgatat gttgtaaaca aaaatagttg aagcttagca gatttctatg attcaaccaa
58560
ttttatgaat tgatgctgaa ttcaagaccc tttcacttct ccagcaatca aaacctctcc
58620
ttttcctctc tcctgccacc tgataccaca caaaatagat ctttatatat atatatttct
58680
gacatttaat caatgatgtg ctggtaaact agtttgttag gaggaaaagc cctgatttgt
58740
actgtattta tggtataact actcccacta tgtctcattt taagcttcca gcttgaggtc
58800
actgaaaaag gagttggaaa gaaatgctcg caatcagctc ttctgagctg ctactacagc
58860
cagctccagc atgccactgc ccacgcattg gccaaagctc tgttacagtg tatgatggtc
58920
agagctaatg ttagaaagta atttattatc cttactatta ttctctttgt agcattgaga
58980
caaagataca agaaaaatat aatatatcca ataattatat gatatatatg ctaagtacat
59040
attatgtaca cataaaataa ttataaaata caataattat atcatataat taggatatgg
59100
ggaaattgac aagataaatg tagatctttg ccctctgatt catttagatc tagtaaaaaa
59160
aactaaagaa aatgtgtcag agtagccacc aaggagacat aaaatagtat gaatttttac

59220

caacaaaata acttataatt ttgatataat tataattcta cattatggct ttattataat
59280

ttataagaat atataatttt accaacaaat taattaatat gaatttttac caactagctc
59340

ttctattgcc taacttttat cagttatctc taatgggtaa gaaagaaaac caagatatcc
59400

ttcactggtt attcattttt tgaaaaagct gtattctgtt gcagttaaag aagatgaagc
59460

atttgacttc catttgggac agtgaccttt ataaaatacc gccctccctc ccccaaaccc
59520

tcagccccca aactcatttt ctctagctgt tgaatgccaa aaatccattt agttttcaaa
59580

cattgccttt tttctattag ttttaggtag ctggtccaag aaatgattga gattcttccc
59640

agtttaaact taagatgctt gaatgtactg ccagtaccaa atgaaggtct ctgcattctt
59700

catcaccctg tgcccagtct ccgtctttta tcatcttctt ttaacaatct tagtaatccc
59760

aaataaagtg aaatggaaca tattaatagt tactcttggg gaattttttt tggactggag
59820

ttgatatata actgtggctg gattatcaac atggactatc ctgtctggag agacaactca
59880

atattaataa tgcagattcg agcattcaat gatcctatac tttgtcaagc cagaaaactg
59940

tgaaccaacc tagatctgtc ctctcactca acctccaagt ctaatcagtc aatagaaaaa
60000

ataacaaata tatactgagc acttactgta tggcaagcag agagttaaat gctcttcacc
60060

tattatttta tataatctcc acaatcatcc actgtgttgt gttactacac ccattttata
60120

gatgacaaaa ctgagataca aataagctaa gcaactcatc caaagtcatg gcactgcggt
60180

agagcctgca aaatttgaac cccagcagcc tatcttcagg gcataccatg tttactatgt
60240

tagcagaatt ataatagatt ctcctgctta attacttctc aaataagttt gtcccttcct
60300

caatatacct actgacttaa cagtagttag aatcatcgtg agaccttgcc tggatttact
60360

gtgatagtct cctaactgaa ctccttgtct ctggtctttt ccccactcta atgcatcctc
60420

tacctagatg acagagtgct tttgccaaac tatagacctg attaagctcc tcactgtagc
60480

cttcaggata aagctgaaac gtcatctctt aataaccctg actctgtgcg tagtggaccc

60540

tgccttcctt tgtccccatt tccctgcaca tctcgacagg gcagccctac taaacagcct
60600

gaattttccc aaacatatca tcttctcaca gcaacagctt ccttcggaca taccttgctt
60660

tctgcccaaa atgtgctgac tccgtttttt tcttctatgt ggctaatttc tagcagtcct
60720

gcaaatccct agtgaaggag catctctcag ccctctacta ttgatcacag tatgctggcc
60780

atggtcctca tcatagctgt cctcaaagag ctctcagttc aggaggcaga caagtaaacc
60840

aataatccaa acaagtgggc aacagctgtt atcaagccac agatcttaat attctataaa
60900

gcgtaatctt atagcattca caaagaaaaa catcattatt taaattgtta tgttttagac
60960

atgaaaaaca ctttgactag tcaaaaagtt ttaggcaggc caggcgcagt ggctcatgcc
61020

tgtaaaccca gcactttggg aggcagaggc aggtggatca cctgaggtca ggagtttgag
61080

accagcctag ccaacatggt gaaaccccgc ctctactaaa actacagaaa ttagccgggc
61140

atggtgtcac acacctgtag ttccagctat ttgggaggct gaggcaggag aatcacttga
61200

acccaggagg cagaggcaaa gattacagtg aactgacacc atgccgctgc actccagcgt
61260

gggcaacaga gtgaaactct gtcaaacaac agcaacaaca acaaagtctt aggccttggc
61320

tgctctgtct atggagtagc catccttttg tttctttact tctctaataa acttgctctt
61380

actttaccta tggactcgcc ccaaattctt tcttgcatga gctccaagaa gcctctcttg
61440

gggtctggat caggaccgct ttccagtaac atcttcctgg caaaccacag agggactata
61500

cggaagagac ccaaacccaa aggaaaatta tctgctcagc accacttgcc tgactttgcg
61560

taagtggtag agtacatttt atcctgggta aaggacggaa caaggctaga gacccaactt
61620

aggacattta gagttcttcc taagatttag ggggttaggg acctcccttg gtaaagtccc
61680

tgttggttaa aaatggattt ggcactacag gatattaact gctattccct ttggaaaaag
61740

ttttagctat gatgcgtgtt tctagatgta tctgtgctat tctcttcatt tggagtatgg
61800

taaaatattc ttacagcaga cataatttta aggaaaatgt atttccctca gcacttgtaa

61860

ctttttgttt atttatttat tggatgttct tgctttaatt tctttaaaaa gcaaaaggta
61920

cgaaaatagc cataaaaacg atatcaaagg gcactttatt ttttgaaatg aactaagtgc
61980

tacatgttac tctacttcat gagttattga gatttaatcc tggacgtgaa gtagggtgtg
62040

gagagtgatt gactgtagta gaagaagcaa tacacttcat gggttggcag tgctggaata
62100

agattatcta caattaaatg agaagtaaga tagtccaaac tggaaacagt gtgtgaggtt
62160

aatctgcatt cctaggttag actctgacag attatgaggc tcccccactt aatgcccctc
62220

acacttctct ctctcaatac tcattttgat atttaatctt ttgtgcagcc aatcaattga
62280

ttagtaattt gcttgtgtac attaaaggcc aggagaaaga agttgcacta taccaaaacc
62340

atgtcccact ttaaaaggta cctagatata gattgtccaa aacattttta cttaaagtat
62400

gctcaggaag tgttgagtaa gagacaatat aattgaccaa cgtatgggca ttctttggaa
62460

gccaaggaga gaagaaagca gacggtaaca aacggataag aaccataagc tttcccctac
62520

tatagaaagt cagaaataca gggaggctga ggcaggagaa ttgcttgaac cctagaagcg
62580

gagtttgcag tgagccgaga tcttgccact ggactccatc ctgggtgaca gagtgagact
62640

ccatctcaaa aaaaaaaaaa agaaagaaag aaagtcagaa atgcaaatat ttatgaagta
62700

atgtagaagt tgcagttttc agaaaatgtt tagtcttaaa accttttgca agactgcaac
62760

cttgtacatg ttttggctgg atattagcta ctggtacttg aaatctagtc tcatcttcct
62820

ttgtatgccc tctgtatttt aaagactaga catctaaaaa ccacattttt taaagtcact
62880

tggagcaaag gttttaaatg tgatttgggt tctgctaata atacgcagtt ggcccttcat
62940

atccatgggt tctgaatctg tggattcagc cgattgcaga cctaaaatag gaaaaataaa
63000

atttctccaa gtccaaaagg caaaacttga aattgccaca tgccatgtac tagactaaat
63060

ccatgtcaag aaagtaatgt ttagatattg cattaggtat tataagtaat ctagatatga
63120

cttgaagtat atgagaggat gtgtgttggt tatatgtaaa tattatgaca ttttatatga

63180

agggcttgag cattcatgga tactggcatc catgaggagt cctggaacca attgccatga
63240

atagcaaggg acaattgtgt gttcgtatat atctagaaag tagaattgtg aggaagtcca
63300

gctttgtagc tatgaacgct gacaagcaag tttgccaaaa cataagtgcc tgtggtagct
63360

ggactccagg ttctcatgtc tagttttcag cttcatgagt gtgagcagct gtgatggcaa
63420

tagcaagagc ggcaatggtg gttaaacctg aaacccatag ttcgatggta gtcccataac
63480

ctttcaatga ttttgtaagt gtttaattgt ctacattaaa ttccctccta attgaaaaat
63540

ctaaatagat ttatatttca tgccctgaat gctaatatga catccaaaca gcaacttgaa
63600

gaagcccaat ttattgaaaa aaaaaacaac ctataattca acatctgtga ctaatcaaga
63660

tatgactaac tctgccatac ctccagtatt caaggtcagg agacagagtc caaaactggc
63720

attaagggtt actgcaggtc tatttatgct tttgatactt ccatatacat cctgtggttt
63780

gaagtgtgtg aggcagaaac tgcattgaat agggtatggg agaaatctat gtttatggaa
63840

agaatgacct ctgaattaat gaaggaccat tataggaaac tgtgtttttt acggtcattg
63900

gtattctcct cttctccctt catcatctta tctgctagat cttccttttc cagacctcta
63960

aatgttgaag tattccaggg tccatacctt ggatatctct ctgcacctat actcactctt
64020

tcagtaattg aatctggtta tatggctgta aatgacatcc ttatgatgac gactataagt
64080

cctcaaagtc cgacacattc tacttcacca catggttagt ttcttcatgg atgaagttgg
64140

gaagaggatt gtaggaaaaa tcatattaac ttcagaaatg gggcagatag ctgggcatgg
64200

tggttcacac ctgtaatccc aatactttgg gagtctgagg caggtggatt tcttgagctc
64260

aggagttcaa gaccagtctg ggcaacatgg tgaaacctgt ctctacaaaa catatgagag
64320

ttagctgggt gtggtggcac atgcctgtag tgtcagctgc tcaggaagct gaggtaggag
64380

gatcacctga gcctgggagg tcaaggctgc ggtaagcatg attgtaccac tgctgcactc
64440

cagcctgggt gacagagtga gaccctgtct ctaacaacaa aaaagaaaat aaagaaaaaa

64500

aaatgaagct gatagaatag aatgtagtca tcagattaga ggtcagatga gagagagtcc
64560

tgctagtttt accattatgt acttgcataa tacatattca gttaggttaa aacactgaac
64620

tgaatcaacg agaggacgaa gtaatagttt ggatctatgt ctctgcccaa atctcatgtc
64680

aaattttaat ccccaattgg aggtggggcc ttgtaggagg taactggatc atggggacag
64740

tttctcatga atggtttagc accctccccg ttggtgccat tcttgtgata gagttctcat
64800

gagatctggt tgtttaaaag tgtgtggcac ctccccactc tctctattcc tcctgttcca
64860

gccatgtaag acgtgcctgc ttccccttca ccttctgcca tgattgtaag tttcccaagg
64920

cctcaccaga agcagaagcc tgtatagcct gcagaaccat gagccaatta aacctctttt
64980

ctttataaat tactcagtct caagtatttc tttatagcag tgagagaacg gactaataca
65040

caaaagttat gcatgtgcaa aactgaagtc agtattttgc tttctgctta ctgtctcttt
65100

cataacattc tttagtatgt acaaaatact gggttcagga gtccattgta ttctggctgg
65160

cggccccata acgttgaatt atggcatctt acctatctgc caattttcta tgcttgattt
65220

tctctacctt tgaccatcat tatacttatc aaaattgtgt ctattccttc agagaactgt
65280

ttttgtttaa atatcctgaa ggaaagcaat cctaaaacat agtcactcta aagaatgaaa
65340

cattcttaat agtttgatgt atcatttctc tcagaattta tattttttac agggaacact
65400

gctttgtgct ttgattttgt gtaggcatca taattgtgga aggcctctaa gaaccaaaac
65460

tgtctacctg atagtctgaa cagtgagaac tgaaaacaca tctacatcta tattagcaag
65520

acttcgcaat tcctctgact tcaccaacat aactttattt tttcaggagt cttctgccca
65580

ggcatatggc ttgaatgttc actacaggaa cttcccaaaa gactcatcct ctcttcagtg
65640

gaaaacgtca accaacagct tgcacgtaag attctttgaa tccctcgcct tagccatcag
65700

tcttggacta ttgtatcctg atccttaccc aatcctgaac cccattttta aagacccacc
65760

tgaaatcaca ctttcatttt ccaataagtt ggggtttctt ccccccactc ggaaacaatc

65820

ccaaagtttg tgaggtggtg cccaccctta ccaaggtaag taataaacta tggtttgtct
65880

tgtcagtggg ttatgttggg gtcagtggtt aagctctttg gggaaccagc ttttgacagc
65940

tccaaaatct tgcattcaga agtctgtaac tattgaaagg agaagtctgt acctgtgaac
66000

caaaaacatg tgagacaggt ctcaatcaat ttagaaagtt tattttgcca aggctaagga
66060

cacacttgtg acacaacctt gggaggtcca ggtgacaggt gcccaaggtg gttggggtac
66120

agcttggttt tatagatttt agggagacat gagacatcaa tcaatatgtg taagatgtac
66180

attggctcag tctggaaagg cgggataact tgtatagggg gcttccagat catacgtaga
66240

taggagacaa atggttgcat tcttttgagt ctttgatcag cctttcactg aatacacaat
66300

ctgcatgtga gactcgggta gaggaatagt tacttaagcc ttagtctggc tcaatgaatc
66360

tgtattttat aggtgaagga ggtatatagc ttttaaaaat ctttgtagtt atcttactta
66420

gaaataaaat gggaggcagg tttgcctgat gaagtttcca gcttgacttt tctttttggc
66480

ttagtgattg gggtcctgag atttattttc ctttcacaac cttaaaatca tcagacactg
66540

cataaatgtg aaggcctagg ctgatttttt tttgaactgg atattatgaa agatacaaag
66600

gttgaggcta taaacttcaa agctgccagg atagactaaa acttcataac caatccacaa
66660

gatgccagca ttccatcagt gctcaaaaat ccaagaacgc acatgggtca ttacagtgtt
66720

catgttattt gaaaggaaat gcaaatatgg taacatttca tttggaaaaa gaaaaagaat
66780

atatatgaac taccacaagt tttccaatat ctgtctttaa actcctaaga gctaatatgt
66840

aacaaattta aagagagaat atttggtgag agaaggtggg ggttttccta ttcagcttaa
66900

tggtaatttt tagaatttcc tattcaagaa gccactagat ggccctaaag cccaccttta
66960

tcaaaacaca cacacagaga gctggcaaga cgaagaagaa aaaaatttag ccatttgctc
67020

ataagttaaa actgaaaaaa gataattcat acaaagacat aaggtctatc ctgtgtttac
67080

ttaaggtttt aagaaaatca agttttgcta caaaacttgg agcatttttc tttctgggaa

67140

catataagta catatcataa taaccaactt ctctacaaca tgcatgattt ttgatggtgc
67200

taatatacta gtggttagat tttccaagat tgatcgaatt attagagctt ctaagcagaa
67260

attacatagt aaagaaatcc aatacataag aaatagtgaa cttgagtgag attacaactc
67320

agctgagttg tttgatcagc ataagaaaca gtactacaca gcctctttca caaccatgtg
67380

ctgacatagt gcacagagtg ttcctggctc ccgtggcctc ccgtatccta ccctggcact
67440

tccattcctc actggttcct gcgtttctgt ctgccgctct gcagggacaa ggactgtggc
67500

tctcttgtgc aaggttacct ctccatcacc tattgtaact ggtcattgga gcatttctgg
67560

catgccagtg ccaactatag tgtgcttaaa acatatccac caaagtttta caccgtactt
67620

agaagtgttc catgttattt gtatttagag ctataaatca gttgatttat tcaaatcaga
67680

aatacaaatg agataaaggg acaaagttcc acaattctgc tgattcaaag taaaaataag
67740

gtattagtcc aggtagagtg gaaagcacaa attcatcagc agtaacaagt ggaagccagg
67800

cgtgtccatg agaacagtgg aagtctcctg ggaattagga gacatcttgg agaagacagt
67860

gggtttttcc acagggcata aaatgggtat tcaagccaat ctgattaaaa tcttcagagg
67920

caaaagaccc tagcagaccc aaagttatta tataagcatt ttaaaataga aactgcatag
67980

agaagtggca gctgaaatac aaatgacaaa aagcctttgg gtaaattggc tcaggagctt
68040

tgtgatgcta aagaaaatgg aatcagccac aggcaacaga gaaggcagat ggggaaagac
68100

acagtcctga ggaataacct ttgcaagcac atctgcgacg tgacacagcc actgaacatt
68160

ccctgaatac agccgataag atcaagagcg agcatttctt aatcagtcat tgctctagag
68220

tcttcaacac aaatgcctag atttttctgg taaaagaaaa gctcattcca tgagtgagca
68280

gcaagttaat aaggagagaa aaattatcct gcccttttct ctgttttgtt tttcctatca
68340

ctccttctcc tattaaggtt aattttatgt ttgagaagag catgtgtttt cattatcagg
68400

aaaaaaatgt ctgtttttaa aatgtattag catattttat ttgtttttgt cataatgcac

68460

ccagaaataa aagaaaccac aaaagtccat gtgatgactc atgagttttt atttctcttg
68520

ggtatacctg gtttccttac aggagcgtca cccttctcct gactgaacca gacacctggg
68580

gaaatctttc catagcactg gtttctgatt tgcaagttca ctcatcacaa tttatcaaat
68640

gaagtgggtt cactcatcta acttctactg aatgcctagt atatgctgta gatatagaaa
68700

taaataagcc caaagtccta cctacaaaaa agtcatgact actaaaaaag aaataaaact
68760

gtgattataa gatactgtgg taaatgttgc aattaaggtt cacatcgtgt aaagctttgg
68820

gctgcatgct atatttattt ctcaggaatc cattccaagg caagaaaacc caaccaatac
68880

tcagctaaac aaaaagacat ttatttgtct cagacaacac aaaagccagg gaggtaggca
68940

gtccagggct gggaaaatag ctccctaatg tatcagggac ccaggctcgt ctgtctttac
69000

cactccttca ccaccatcct tggcttggag gcatttgtct tcatatttgc tgccttgtgg
69060

ttgtaaggtg gctgctacat tttgaaacat cacatctgtt catgttccag gcagggagaa
69120

gggggaagaa caaagggcaa aatgagtcat gttagcccat tttttgcttt tttatcagga
69180

aaacaaaaaa tttcccagag accccagcac atagacattg ttttatctta tccgtcagag
69240

ctgtgtcaca aagccaccct ggccagaagg tggctagcga aaagcgggca tgggatagga
69300

attaggctgg cggacccaca gcgtgagaca ctaatgtacc ccacatgctc ctgttgctat
69360

cttctaccca tttgaacaac tactgtcctc tctattctgc atgctggcta ggagagccta
69420

ggcaacaaac agtccacagg tatgcctatg gttactgcta tttcaacaga gttcactgga
69480

gtaagatgat gagggaggaa gttcatcttt ttgaggattt accccctgtt tacattcagt
69540

ctttccatgt tgaactagaa attcaaagag gaggcatcat gtctccttaa atatatatat
69600

ctttcccctc tttggagatc aggatctgtt ttagactgtg gggaattcag tagccccaac
69660

tctgttttca ggtgaggtaa ctgaaacctc cctgcttcag cagtgcgatt cccactacct
69720

gtttacaaga gaactgaata tcccctcccc tagggatcct ctgctggaac tccgattacc

69780

ccataagttg ctgttaaatc agtgaatggc attcattagg cacatatcat gtgacaagca
69840

gtgtatcata ttcctctaat tcttcaaaaa caactttatg agagaggtac taacgtcatc
69900

tatttaacca tgaggaagtc aaagcacaca gaagttaagt catttgtcca aggttacatg
69960

gctaatatgc agtgaggcag ggattcaaac ccaggcagtt ggcttcttat ggagaactgg
70020

gcaagggatc ttagtactaa atcagctaac ctcatctagt ggaaatgtca ttctcagagt
70080

tcttcaggct gcagacacca ggcttctgta actagctcta ggtcagtttg gaaaggtgca
70140

tggtaaacac caataattta ggaatataag gcagcttaag attttaattc attcattctt
70200

caataaatat ttattgaacc cctgaaacgt gctaggtttc actccatggc ctggggattc
70260

aactatgggc aagagagcat ggtcccttac ctgaaagttt actgtttcta gggaagacaa
70320

ggaattaagt aaaatttgat gagtaatata ctgagaaaaa ttcaaggtac tatacaagtg
70380

tattatagaa gacattcgta ttttgggggt ggaggtgggg agtttgtcgg ggagtgcttt
70440

tttttttttt tttttttttt tttaggcaga gtctcactct gttacccagg ctggagtgca
70500

gtggtgcaat cttggctcac tgcagcctcc atctactggg ttcaagcgat tctcttgcct
70560

cagcctctca agtagctggg attacaagtg cgcaccacca tgcctagtta atttttgtat
70620

ttttagtaga gatggggttt caccatgttg gccaggctgg tctcgaactc ctgaccccaa
70680

gtcatctgcc cgcttcagcc tcccaaactg ctgggattac aggcataagc caccacgcca
70740

ggccatcagg gagtacttct aagaggaagt aacatttgag ttctctgata tgttgcagtt
70800

agccagacaa agatcctgac tggaaagatt ctgactggaa agaagaggta tgggtgtttg
70860

tgtagttcta gagcggggaa aacctagttt gttgaagaac tggaatgaat tgcaaaaccc
70920

cctgaagtaa aagatacctg aagaggtggg cagaagtgga gtcatgaaga gctctgtgac
70980

cttctaagcc aacttaaagt caagtatttt gtttatggat ttaatttttt ttacagttat
71040

aatcacaatg tccccagtaa agaaaccata atatagctca tgttgttttt gttcttaatt

71100

agcttgttaa cttcatggta cattgaccac ttaaaaatga gatttattct tctcattaac
71160

tttaaaatgt ttggaaatga attgcttaat atcagaaaac atttcttatg ctttttgaga
71220

gaggaaaata ggtgagcgtc tatttattat ctggtgatgt ctatgtgctg gagtcttggt
71280

tctccctatt tcatgattcc tgataaaaga cttatgcctc cgttcttgta gcccattcag
71340

agaccaggtt taggcgtgag tgccaccaaa caaattagag tttttgccag agtatggaat
71400

tccaagtctt attttcaggc ccctaggcat ctgtgccaat tgtgtgagtt gcactaacga
71460

ctagtaagtg agtggtcatt gctctgctaa atcttggata tacaaattta gggggtctca
71520

acacatgaac aggatgggga acaggcagag aggcaaagct gtgtttgtga ggaaccaaga
71580

gaggcaaggc ttgattcctg agttgtttgg atatatgctg tccagaaagt ggggtgaata
71640

tagtacccca tcctgaggag attggagctc tgtgcagcca agagaaatgg ccccataaca
71700

ggtagccaga agctcagggc tctctattca cagccagaat agaggctgtc acaatcctgg
71760

ggaaatttgt tgatttcttt attcattcat ccaataaata tgccatagag acagggcagt
71820

aaaacacaca atgacaacaa caaaacagac agaaaactct gccttcatga aacttatact
71880

ctagtgatgc agatagaatc cagtaatatg tagtgaaaaa aaaaattcat gaccacactg
71940

ggtttactcc aagaatgcaa ttttgtattt tggtcttatg tgaaaaaatc agtcaaagaa
72000

tgaaagagaa taattacatg tcacttcaat agttgcacaa aaaaaattag taaaattcaa
72060

tacccaagca tgatttgaaa aaacaaacac tcttagcaaa ctcgaactat aagggaattt
72120

tcttaaccta attaaggata tttacaaaag aacctttgat gaatatcatg cttaatagta
72180

aaatgttgaa agctttcttt ttaggattta taacaagaca agcattgtct tttattacta
72240

cttttattca atgtgcaaaa ttgaaggaca ccgaaggccc tggccagtgc agtatgaaag
72300

aaaaataaat aaatgacaaa gttattggaa ataaagaaat aaaacatcat tattcacaaa
72360

tatgattata tctgtggaaa actgtgtgaa aataaataaa atttaaaagc tcttggaacc

72420

ctgaaaatat tttaggcttt gagagatatg tgactgtgat ctgaatcaca tgtagttaca
72480

acctctattc tcagattata gattaacctg ctttcttgtt tttcttgttc tgtgcaatga
72540

ctagagagaa ttaaacaatg ccaaagacaa aacctcctgc cttcttcatt aatgaccctt
72600

gttatagatt aacttcccca tttttgtctt gatttgctag ataagatgac agaaaactca
72660

tgactattac tccctctgga aaaaaaaaaa agaaagaaag aaaaaaaaaa gttaaatgta
72720

cccttcccag aataaacaca gcctataacc aatcaaattg ctgtaactat gcaccaacct
72780

tatatggaaa aatgttgtaa tcctcctaaa aactccctgt ctctgcctac ataaatgaaa
72840

ccttaagttc cttacttcgg catacttact ctagtcctct gcagttggtg tttccaggta
72900

ggctatcttc aaatgttgct cttgaatcaa ctatccttaa attagattct taacatttta
72960

ttattttagg ctgacagttg aaaagaacct acagccaaat caaaagaatt cataagtgag
73020

tttagcaaag ttactagaaa caatatttaa aaatttattt gaatttttct atacaagcaa
73080

taaaaaaaac aaaaagatgt tatttataac agcttcaaaa atatcaaata actaaaaata
73140

agtctagcaa aagatatgca aggccaccat gccaaaaact ataagacact attaagataa
73200

aaaaaataag acctaaatac tctaactgat agaaaagact aattcatgga ttgcagaaat
73260

caaattgtaa aggataattc ttcccagatg gatctagata tccaatgtaa acacctttga
73320

ttattttttt ttaaatgaaa cttgacaagt gagtctacat ttaactagaa attccaaggg
73380

tcaatagcta agaaatgttt tacacgagta acagtggatt ctgattatca agactataat
73440

gcttcggtag taatacattg gcacaagtta agagattgga tgaggattat aaggatagac
73500

aagttaacca atgaaataga aagagagcca aaaacagact taaccacaca tgggtgcttg
73560

atttatgaga aaggtggtat catagaccag tggggagaaa gggatcttgt tgataaataa
73620

tattgggaca attgaatatc catattaaga aaaaatttga cccctgcctg acacaataaa
73680

tagatattaa ttttaaattt ttaggcataa atatgaaaga aaaaagcaaa aaatattaag

73740

atgatacagt aagagaatag tttcatgatt ttagagtaag aaaagaatta aaataggaca
73800

taagaaacgc taactataaa ggaaccaagc cacatacgta cattgaaatt attaacttct
73860

gtttgccaaa agacaccttt aaaaacgaaa agggaagagc agagtggaag aaaatattta
73920

caacacaaac aaaagttaaa agtctcttac ctcaaatata taaataactt ctacaaatta
73980

ataagaaaag attaacaatt cagtagaaca atgagtcaga gcaaatcaca agaggctatt
74040

caaatggcca ataaatccta tgatgttaaa attcctcaga aacaatcaaa aagtgaaaaa
74100

caaactggac ccagaagagc agaagcctgt ggttgaaagg aaaacagagc agggagaagg
74160

aagatcagga cagtattgag agggtatacc agcactgggt taaaacaatc tgggtgacgt
74220

tcagtaaagc tccattattt ttaactggga tttttggggc cagccactga agtgggaagg
74280

tttatccctt tcctgtgggt ctttggattt tcattaaatc tttccctatt cccttaaaca
74340

aattttgccc ttctgtcatc tagtccactt cctctctgtt ggtggctaaa tgggatcatc
74400

ctggaggaaa cttaaggcta tttcatttcc cattttaagt gccttttggg gctgaacaga
74460

gatctgcagt tgatttgtta gctggcagaa gtgtggacag tgaaaattgc aaattgattt
74520

tttcttgaag ggtcagctat tggctcacat tttcaggtaa tcagttatga tgctatcaaa
74580

taaagccact tgcaatagtg gccatctaat aataacaaat gtagattacc ccacacatta
74640

tttaactctc taaaacacta gcttgtggct gcaggtacaa aactccagtg agaaaatctt
74700

gcaaaaacat ttggtgcaaa atcaatgata aaatctttca cagcatttta agagtaactg
74760

taagaagaaa gcaattgtag ttattcattt aatcgttttt ctaaatagag tctacacatg
74820

agctgaactc ttcatttgga atgaaataat ccttttgaaa attttactct gcattagatt
74880

ttatcccaag tggcccaaga agcactatct aatatgacta acaaccacac atctataact
74940

agtacaactc caactataac tttgtaggac ctattgaagt ggttgactta tgcttattaa
75000

aatattaaat aagaatgaaa ccagtcattc tttggaagat tccaaataat gttgtagaaa

75060

cttctattca tttatatatt ccaagatatc ttccataaag tggataagat ttttaaaaag
75120

atgtaaaagg accaattttg agtgtggccc aaccagactc aattctggaa atcaattctt
75180

acacattatc aattattcat attaatatat cattatatat atataacttt ttacactgat
75240

ttatttattt tatttgacac tataaaataa agcaacctaa aataaagtca gtattgcctt
75300

ggagaaatat tctgttaatc tttccattct gaaaatgaat gccataggag aatgacgaac
75360

aacagaaaat ttattagcgt aatgaaagaa aatttaaagt actcccctaa atgcatagaa
75420

gtagactatg ccctgaaact gtttttgagt agaaactttc tgaatattga gttacaatgg
75480

tgcatttgat ctaacaaaaa agatgaaaat ataaagaatt aattgtctca attccagaga
75540

atattgtata tagttgtgta tatagttgtg aaaaaactgg acagcacttt tttcatatta
75600

gttcacattc acaaagttca taacaataca atatgactca tagttgttca ttactaggac
75660

cttgaactac aaagaaaccc gcagaatatt ccttggcagc tctggcttca tggatatatg
75720

actttgcatt catacacgaa tcttaagctt ggaaaatcag ctgagcgcag tggctcacac
75780

ctgtaattct agtactttga gaggcagagg tgggcaaatc acctgaggtc cggagttcaa
75840

gaccagcctg gccaacatgg tgaaacccca tctctacaaa aaatacaaaa attagccagg
75900

catgatggtg tgcgcctata atcatagcta ttcaggaggc tgaggtggga gaatcacttg
75960

aacctgggag gtggaagttg cagtgagcca agattgtgcc actgcactcc agcctgagca
76020

acagagcaag accctgtcaa gaaagaaaga gagagagaga gagagagaaa gagaaagaaa
76080

gaaagaaaga gaaagaaaga aagaaagaaa gaaagaaaga aagaaagaaa gaaagaaaag
76140

aaagaaagaa agaaagaaag aaagaaaaaa agaaagaaaa gagaccctgc agtacgaaaa
76200

ctgaactgtg atttatccat atgttatgcc cctctatttc ataaatatta agaaaaatca
76260

ttttgactgt gattaaatgt atatatactg agccaaatat ttttctttca actattttcc
76320

cttccttccc ccttcttcct tggatttttt ttctccttaa cattgactat ggtctatata

76380

tatgtgtgtg taggtctagt actgagctct ctatgatgtt ccatttacct ttccttgtgc
76440

catatacaat gtcttaatta ctatagcttt ataataaagt ttgatatttg atcatacaag
76500

ttcccatctt gttctttaga actgtcttgg ttatttttgg tcctttgcta gtctatataa
76560

attttaaaat caacttgtga tgttgggatt ctaggagtgt taatcaattc atgaaaatta
76620

atgtcaatac aatattaaat ctgactatcc atgaatatgg tatatctctt catttgttta
76680

ggttttattt aatgtcgttt aataaagctt tataattttt accagatatg ggcttacaca
76740

tctcccacta gatttttttt ctcttggata aaatatttat ttttattgta atttaaaata
76800

gtataaatat ttttaaagtt ttttactttt taaatatttg tttacagttc atttttgagg
76860

taaatataca tacagtaaaa tagacaaata caagtgcaca atttgttgaa ttttgacaaa
76920

tgcaaacaaa cgtagaggta agaatgtttt acatagtata atctttttaa taacattttt
76980

agctgatcat tgctggtgta taaaaatgca atttcctttt tattttgtag acaattatac
77040

tacccataaa taatgaccag tttgttttgt ttcattctaa ctcctaattc cttttatttt
77100

acttttttat tcacattact ataccatcta ggccatttag tagagtgtta aacaaatgta
77160

gtaataataa acatctttac ctattttata ttaaacttaa aatttttcac cattaagtat
77220

aatattgctg tagtttttg ataatattta cacgtttaag aaatttttct tccattatta
77280

gtttgcttaa gattttctaa accataaacg gatattaaat tttatctaat acaatgtgtc
77340

tacttagctc attatatagc tttcattctt taatatgtta atggtgcagc ggggtgcggt
77400

ggctcacgcc tgtaacccca gcactttggg aggctgaggt gagtggatca caaggtcagg
77460

aattcaagac cagcctggcc aacatggtga aactcaatct ctactaaaaa tacaaaaatt
77520

agctgggcgc agtggcaggt gcctgtaatc ccagctactt gggaggctga gacaggtgaa
77580

tcacttgaac ccaggaggca gagtttgctg tgagcccaga tcgtgccact gcactctagt
77640

ctgggagaca gagtgggact ccatctgaaa aaaaaaaaaa aaaaaagaaa tatgttaatg

77700

gggcatgagt tggttagcca actgcacagt ttgaggtggc atagtccacg caagactgtc
77760

ctcacttttg acagcaattg caagtttagg ggcttcctaa aaccaccttc aggttaccta
77820

atccacaagg actcctggaa ctcactaaaa gttgttataa tccaggttag agtgtattac
77880

aggaaggata cagattaaag tcagtcaagg aggggcacct agagcagagt ccaggaaaag
77940

tatcaaacat gtagcttctg tttttctttc tccatggagt cctggacaca ttacttttca
78000

aatatcaaag tataacaatg tgcacaaaat attgctaatg aggaaagctc agcagagcca
78060

ggtgttcaga ctttttattg aggctccatg atgtagacat gggcgattgc caatgtggct
78120

gatttcaatc ttcagcctcc accctctcca gacatgaccc aaatccccca cgctaaatca
78180

catttctggt ctttctggtg tagccattct ccatcttaaa tcatattttt agacaatctg
78240

gtgacccaag gctcacagcc aaacaaagac actcctatca accatgatat tccatcaatt
78300

tacccatcac ctctcagaag ccaaggacaa agaccagact tcttttcgga caaagttgaa
78360

ttctttacta tgtgtggggt gaataacata atttaacttt ctaatattaa atcaactttg
78420

tattcctgaa atgaattcaa cttgataata aaatatttac ttttttatac attatataat
78480

ttggctttat aagattttgt ttaggatttt tatatctatg ttcatgaata agactggtct
78540

gtaagttttc tactttatgc tgtccttgcc tagttttgct atcaagatta tgctaacctt
78600

ataaaataag tttggggatg tttcctcctc ttctataacc taaaagagtt tttgtaaaat
78660

tgaaattatt atttattaaa tatttggttg aacttgtcct ttagtaattg gtagtacata
78720

ttaaggaaat acgcacatca atggacatgt tcaggaaaaa tgagtgttga attttatctc
78780

tttttttttt tacatatttt aagattacct tataattttt ttctttacaa tgttaatgtc
78840

atatattcat aggtcttaaa tgttgaacca tttactatct ttttgttttg tgtgttgctg
78900

gacttgattt gcaaatattt ttatgaaata atatttataa gtataattga cctactattt
78960

ttatcttgca ttactcttat tttattatca aaattatact aaatgtataa ataaactaaa

79020

taattttttt ctttttttct atagtagttc gtataaaata aagattgttt cttaaagatt
79080

tagtactgcc ataaccaatt actacaaact aggtggctta aaatgctagt aatttattct
79140

ctcacacatc tggaggccag acttccaaaa tcaaggtgtc agcagggtta ggttagttcc
79200

ttctggaagc tttaagggaa aaacattcca tgcttctcta ctagtttcta gtgttgctgg
79260

catgtcttgg agttttttgg cttgtggtag cgtagttcca atatctgcct ccagtttccc
79320

atcaccttct ttcctgtgtg tccctctatg tcatctcttc ttacaaggac aacagttatt
79380

ggatttaaaa ttcaacctaa atccaggatg atttcatctt aagatcctta acaaattata
79440

tctacaaaga ccttactggc aaaaacatga cattttaaag tttgggtgca cgttactttg
79500

tatgggatac tattcaatcc cttccaattg cctgaaatac aaaaatatgc caggtataat
79560

tccaaaatat aaaagaaaaa cagtcttgct aagtgagact tttgaattac ttgatacttt
79620

gtcttggatc tgaggaaatg aagtatattt gaggtataat tgctttaaaa agtactccag
79680

tggataaaag taggtgagta gagaagaaag agtgttaaca gccattagtg gtggcttctg
79740

gaaaggacaa ttactgatca gaccatctgt taattctacc agacagttta atgtgcaaat
79800

gtaaataaaa ctaataacat ctccatggaa aattaaaaaa tgcttaacca acattgattt
79860

gatattgaga gtcttcgtct gttttgtgtt gctgtaacag aatatgcaag actaggtaat
79920

ttgtacagga cagaaattta tttctcacag ttctggaggc tggaaagtct aagattaagg
79980

taccaatagg tttggtgtct gaggagggtc cagtctccac tgccaagatg atgccttgaa
80040

tgctgcatcc tccagagggg agaaactctc ttctttacat gacaggagag cagaagagag
80100

agaacccact cttgaaagtt attttggtag ttgcatcaat cattcataaa ggtagagtcc
80160

tcacagccta aacatctctc attaagcccc acctctcaac acgattgcat tggggattaa
80220

gattccagta cattaatttg gaggggccaa aacattcaaa ctaaaatatt gaatttctgc
80280

tagaactaat aactgtgtta gatgaataat agaaagtaga tcagtaccaa taagcatttc

80340

actcaagtca gccccaaccc ctttcagctg caaataattt agagtgattt attggcatga
80400

tttaacttta atcagaaatt gagaagtgag tagctatatt gtaatattta ataatatatt
80460

taaagatata attataccaa cctcctagat tattgtctct tgttactatg tttctctgga
80520

agcatttttc ttggatttcg taaaaggttc tctagaagag gaagtagtct agagaacaaa
80580

ctgaaaacaa tttgcaattg tttaaaagca acatttcaca aattctagtt ataagaactt
80640

ccaacaaaaa tcactgtcag aaaaaaatat atgggcatcc tctggggcct ctatgggatg
80700

gagattgttg gaatcctttt catctctaag attctgtgaa gtctctgagt tatgaaagag
80760

tgagctgaga atctcagcat tcccaggtga agggaactaa ggcaggtgat ttagcaatgt
80820

ggagggtcat gcttcaagat cttgttggaa acccatatcc caaggaaaaa ggtggcccag
80880

gtaacaataa cacagaacaa aataatagaa agataccagt tttgcaggca ggtagatcag
80940

tctttgaatc ctagctcctc catctctgtg tctctgttct gtcatatacc ttaattaaaa
81000

ttcaaatgag ataacatatg caaagtacct gaacattagg tgcacaaaaa atggtaggtg
81060

gtattaactg tgactaggaa agagaaacaa aattgctgca taaatggcaa aaattttggt
81120

catcaattaa aactacttac agagaatgta tatactttta agatttatga taatttttct
81180

catcataaag aggactgtat cagcctaaaa ttatcataat atggtagtta ttttgcacat
81240

gtcaacatgg caacacccct agttgtgtgc tgttcatgaa aatacatgct gttcatgctg
81300

cttctgagaa atagagttat tataggtctt tccctctcca tttcacttct caaacaaaag
81360

aatatttcat acatttgtat atgtgttttt ttgtttttgt ttgtttgttt gtttgttttg
81420

agacagggtc tccttctgtc gccccagctg gagtgcagtg gtgtaatgac agctcactgc
81480

agcttcgacc tcccaggctc aagcgatcct cccacttcag cctcccaatt agctgggacc
81540

ataggcacat gccaacatgt tcagtttttt actttatgta ggaacgaggt ctcactatgt
81600

tgcccaagct ggtctcaaac ttttgggctc aagtgatcct cctgccttgg cttcccaaaa

81660

tgctgggatt aaaatcataa gccactgcac ctggccacat ttttatgtta aagaaaatat
81720

tctttctttc tttgaaagaa atgcctagga aaagtgtagt gtcattactt ttaaaaatta
81780

taatgttttc attcatattg agttatttag ctttcttgct gcaagacttt attgaaacaa
81840

tatgatattg gtgcctgatt tttcaaatag ttctgttgct ttacatactt taaaaatata
81900

aaaatagcat aacatttggt gagttgaact ttaagggtag ttttcttaaa tttcataaat
81960

tcatactatt gggagacaat tttccatggg tctctcaaca tttcagcaca cctggcagaa
82020

gcactgattg ccttttgttt acagcgtatc ctttcaagga tgtttgtata gagaacaagc
82080

ttgacagata gatagtgttt ccctctggag caaaagacaa ccatgtatgc tctctagtat
82140

aataagtatt tttctcattg gagcaaaaaa caggtatgct tactgcccat tataaaatag
82200

gtcagagttc ttctccttta acataaccca ctgcttgtgc agatatcatc tagccttttt
82260

tctgatgccc tccagaagtt gagacttgag gaactgatac aaaatgctaa taatctggct
82320

accactgttg ctgtgaataa taaactatcc tttgtctctg acccaggaat cttctgcttt
82380

ctaccagcat ccatgagact gtggcagaat aactagcttg cagatagggt acaaatatca
82440

gatgcgttga agttcttgac aaatatctga aacatttatt tgatgaccgt attagtctgt
82500

actcatgctg ctgataaagg cataaccaag actgggtaat ttataaagga aagaggttta
82560

atggtctcac atttctacat ggcaggggag gtcacacaat catggcagaa ggcaaaggag
82620

gagcaaggca catcttacgt ggtggcagcc aagagagcgt gtggagggga actcccattt
82680

gtaaaaccat cagatctcat gagacttatt cactaccatg agaacagtat gggggaaact
82740

gcctccatga ttcacttatc tccaccttgc cctgcccctt gacatatggg gattattaca
82800

attcaaggtg agatttgggt ggggacatgg ccaaaccata tcaatcacta acaaaaggaa
82860

ctaatcacta actaatcact aacaaaggaa ctaatcacta acaaaaggaa tcccatgact
82920

aattcacatt ctagcatatc tagagcaaag cagcaaaggg caagtgtgac catttgtaaa

82980

gttgtgagta tgtgtatgtg tgtatgtaca gccagttgtc tacttttaga agccaggaga
83040

ctatttaaca cttcctagaa cctgggattt ttctgaaaca aagaatcaga ttgatatttt
83100

atatcaactt ttaaaaattc aaagaaatca aaaccatagt acacagtata tacactaatg
83160

ataagattat gcctaaaaac acgtaagaga caaaaataat tataagcaat aaaaaatttt
83220

gagtcactct aatatggaag attggctata aaatcagctc ataaagtaaa aattgcatat
83280

agtaaaatac atatgttgaa aatgccttta atcacccata gagtatactg accatacaga
83340

ttttttaaaa caaataatgc atttaattta tttgtcaatg gctatgaaaa ctttaaaacc
83400

attcacatgt tctttccata ggttttggaa cttctgcagt tggttttctg acttaattgt
83460

agcccctgtg caagtcatag cattggtggt gcagagaagt gtctactcaa gctgtattgt
83520

taatttaaca aactttaaat gtatgcatga catgacttca ttttaccctt aaatattttc
83580

atcattaaac aggagaagat aaaaaataag ccaacatatt gaattttaaa aacaaccatt
83640

aaacaaactt aacaaggaac aaaaaaacaa acaaagttaa agacagatat taatgaatca
83700

gaaaaaaaat tggttgaatt aacgaattca aaagcaggtt tcagaagatg ataaaataaa
83760

atggaaaggt tttgaaagct caccaaacaa ctacaatata agccctgtga aggcacagta
83820

cctggcctat aaatacacaa taaatattat ttaaatgaat gaatacattt taaaaatcag
83880

gaataggaag agaaagaata agagtgagat gtttagtggc caagactaaa ggtggtgcac
83940

ataatttctg cccatatctc aggggccaca gctcagtcac ttggcttccc tggacaaagc
84000

ctgtttctgg gaagaaaatg aaatgatttg gttctctgca atgcctggat attatttcct
84060

ctcttgtatt agtttgttct cactctgcta taaagaagtg tgagactggg taatttataa
84120

agaaatgtat acccagtgaa actgagtaat ttataaataa aagaaattta actggctcat
84180

agttccacag gctatacagg aagcatgaca ccttctggga aggcctcaga aaactttcaa
84240

tcatggcaga aggtgaaggg gaagcaggca tgtcttccat ggctggagca agaggaagag

84300
agaagtcggg agatgtcaca cacttttata caacccaatc tcacaagcac tcactcacta
84360
gcatgaaagc agcactgagg gggaagcctg tgtccatgat caaatcacct cccaccaggc
84420
ctcacctcca acattgagga gtacaattga aaatgagatt tgggtgtgga cacagatcca
84480
aaccatatca ttccacctct ggaccctcca aatctcatgt ccttctcatg ttgcaaaata
84540
caatcatccc ttcccaacag tcccccaaaa tcttaactca ttccaacatt aactcaaaag
84600
ttcaaagtct catctgagac aagccttgtc tcttccacct atgagcctgt aaaatcaaaa
84660
gccaattaat tactaccaag atacaatggg tgggtgggtg ggggtggggg ataggcattg
84720
ggtaaatagt cccattccag aagggagaaa ttggacaaaa gaaagaggct acagtcccta
84780
tgcaagtcca gaacccagca gggcagtcat taattcttaa agctccaaaa tgatctcctt
84840
tgactccatt tttcacatcc agggcacact ggtgcaaggg gtgggctccg agagccttag
84900
gaagctctta ctctgtgggt ctgcaactgt acttgtggct tttccaggtt cacaatgaaa
84960
gctgttggtg gctctaccat tctgggctct gaaggacagt ggccctcttc ttacagcctc
85020
actaggtggt accccagtgg tgaccctgtg tggtggctaa aacccacatt ttccctccac
85080
actgccctag taaaggttct tcatgagggc tccacccctg tagcaggcct ctgcctggac
85140
agcaaggttt tttcatacat cctctgaaac ctaggtggag gctcccaagt ctcaactctt
85200
gcactctgtt cacctgcagg cttaacacca tatggaatcc accaaggctt acagcttgca
85260
ccttctgaag cagtggcctg agtacctagg tccctttgag ccatagctgg agctggagca
85320
gctaagacac agggagcagt gtctcgaggt tgcacagggc agtggggtcc tgggcctggc
85380
cctggcccac aaaaccattc ttcactccta ggcctctggg cctgtgatga gagaggatgc
85440
catgaaggtc tctgaaatcc cttggaagcc ttttcccatt gtcttggcta tcagcatttg
85500
gctccttttt acttatgcaa atttctgcag cctgcttgaa ttcctcccct aaaaatggtc
85560
tcttcttttc tatcacatga ccaggctgca aattttctac acgtttatgc tctgcttccc

85620

ttttaaatat aagttccggt tttgggtcat ttatttgtgc atgcatatga acatatgctg
85680

ttagaagcag ccaggtcaca cttgaatgct ttgctgctta gaaatttctt ccacaaggtt
85740

gcggtgagcc aagattacac cattgcactc cagcctgggc aacaagagtg aaactccatc
85800

tcaaaaaaaa aaaaaatttc tgctaccaga taccccaaat catcactctc aagttcaaag
85860

ttccacagat ttctagagca gggacacaat gcttcaggtt ctttgctaat gcataacaaa
85920

agtgaccttt gttccagttc ccaataagtt cctcatctcc atctgagacc tcattagctt
85980

ggactttctt gtccatatca ctatcagcat ttcagtcaca agtcaagcag tccctaagga
86040

gttccaaact ttccctcatt ttctgtcttc ttctgagccc tctacactgt tccaacctct
86100

gcccattacc cagttccaaa gtcgttttca gattttcagg tatttatagt aatgccccac
86160

tcctcagtac caattttctg tactagtcca ttctcgcact gctataaaga aatacctgag
86220

actgggtaac ttataaagaa aagaagttta attggctcac agttccacag gctgtacagg
86280

aaacattata gcttcttggg atctaaacat tctaaacctg tatcagtttc ccctcaggaa
86340

actttcaatc atggtagaag gtgaaggaga agcagacaca tcttacatag tcagagaagg
86400

aggaagagag agaaagcaga gatgccacac acttttaaac agcctgatct caggagcatt
86460

cactcactat cacgaaatca gcaccaagtg ggaaatccac ccccatgatc cagtcacctc
86520

ccaccaatcc ccactgggga ctacaattga catgaagatt ggtgtgagga cacaaattca
86580

aaccatatca gttccctttc acctctaatc gtgttccttg tctgtagctc tgttaggtct
86640

ttaccacttg agattgtgtg catcttttca cagtttatct ttcccaacca gatggtaagg
86700

tccatgagaa gagaaatcat gtcataacct aaatttcttg ttgtaaacaa cacccagcat
86760

ggggctctgt gtgctcaaaa actcctcact cctacagagt gcatttcaat ttacagagtg
86820

tcttcacaga tttgtctcat tgttgatttc tgaagtggaa gaccaatatt tctgacttta
86880

tgatatacga atgcttggat gagctgcaaa aggttgagca aagcaaatgt ttccattcca

86940

tctagttcag ccacatagac ataacctttt tcgttctctc taaaataaac tcaactgagt
87000

aatactacat ttttctttta ttactggtgg cattttattt aaatgtttgc atccatattc
87060

ctaagtgaga tgatatttcc tttttttgtg ctatattttc tatgttttgt atcagagtta
87120

tcttagttag aaagtaataa ctccaagatg gcagagtcat tcacaggagc cccaatctct
87180

gagtcttttc ttgaaggaca actgccagga gagctgcctg gctcaaattg gattgtgaca
87240

tgagtgagaa atggagtttt attatgataa gtcatattat ggtaagtttg tcttgttata
87300

gtagctagca ttgcttttcc tgactcatag attcttgtca tctcaggtcc tgccatcatc
87360

ctaggtgact tctgtgtcca catcaatgac ctttccaaaa ccttagccat tcctcttaac
87420

ctcattaaca tttgtacctt catttcctct gctgccattt agtcacagcc ctagagcttg
87480

tcgtccccta gagcttccca atcacatcta ttcttcaccc tcatgtggtt ctcctatcta
87540

cttttctgtt agacctttcc tgccctcaat tccttcccat ccatcttatt ctcaatggcc
87600

atgagctcac ttattcttgg gctaatctat cacttcccct taactctcat taaacgacct
87660

tctctatttc acaaagaaga tgtgatcttc aggcaaagag ttctctcact ccctacacag
87720

tcctccaaat cttgtttgtg tccacaccaa tcctcttctc cattcctctc ctctgaaaaa
87780

aaaagagctg ttcctcctcc cttcccatgt actcactgcc ttccttcctg ctgtgtcacg
87840

cctttgctcc actctttgtg ccctgtctca gctagatctt cattctgtcc tctactggtt
87900

tctccaccct ggcctttaga tatgcccaaa cttgaacttc acagctctgt ctttattctg
87960

catccttctg catagtcaag gctctatttt ttgtttacca tcttcccatc caaacttctt
88020

gaaagggaag cctgcagttt cttatttttc atgcattcct cagctcccca ctgccatttg
88080

tctccctcct ccacttaacg atttatgcca aggtaatctg ttcctagatc tagtggacat
88140

ttctaggctt tctctctctt gatctctgaa gtatctggcc ctactggaca ctgtctctcc
88200

ttctcttcat ttggctttag tgatactatc ttctcctctg gatttgctct catctctttg

88260

gtccctcctt ttctggctcc tgctcttcca actgcttaaa cattgctgtc tccactgttc
88320

catcctcttc tgttctcttg ccacatattc tctgatttat ttcatacact cccatgtctt
88380

gaattcctaa ctaaatgctt atggccagaa tctaaatctc tggctcagct gtatcttctg
88440

agctccaggc atcttcatcc atctttgtac taatctttct tctttgatgc cctacaggct
88500

gcttaaactc aacatgacca aaacaaaaca aaacaaactt tcttctcctt caaacatctt
88560

agagaattgc atccttttct gcaccaccta agccataagt tataaagtca gcctccatgc
88620

tctcttctac cctcctccat ctaatctact caccgatctg atcaatccta gggtctaaat
88680

aactctcaaa tatatccccc tctccatctc catcactact tctcaattag gcctgcataa
88740

tttcttccca attagtctta taatgcccta actcgtctgc ctcttcctct ttactgcagt
88800

cagaaagctc ttattaaatt acaaatctac tcataccact ccagctgcta aaatctgttc
88860

aaagaaaaat tagacaaatt taatttaata gagtctaatt gagcaaagaa taattcacaa
88920

atcaaatagc ctcctgaaac ggaataggct gaaagagtct ccagcactgc cttgtgatag
88980

aaaaagaaaa aggaaagtgc tgtacagaaa cagaagtgag gtacggaaat ggctggattg
89040

gttacagctt ggcatttccc ttatttgaat ataatttgaa gagttggcca catttgatta
89100

gccaaaactt ggagattggc acaattgtag gttacagcct gtttacacat tcggttaggt
89160

tacagctcac taggtaggag aaacctttag gctgaagtta aaatacgcaa ggagggagct
89220

ttagatgaag cttaatttaa caaacccttc acaggccaac cacatagctt tcagaacaag
89280

gtggaaatga tgggcatggc acatttcata ctgagatctg cctgctttcc aggcttcccc
89340

tctgcacagc ccctgcctct acccatcttc aactgtggtc tggcaaaagc aaactatttt
89400

caatttcctg aatgcacttc attctctcca gtttcttagc ctttaaccat actattctct
89460

cagactggac tgatatcccc atcttcccat gccacttcac tttacaaact gtttcttcag
89520

ggcttaccct gggatactat atccttagga agtcttccct agacattcag caccaccacc

89580

cttctttgtt ctcctacagc accttgtgca tactttatta gtgcacttat gtgattctga
89640

aatgatttgt ctcatttcaa tcaattatga agtcctaaaa gctgtgcctc ttcaatatct
89700

ccaaaatcct gccttctctt gaacctggtt ttcctgcttt catttctgtc ccccgcccat
89760

ttcaaaatta gcaacagagg ggtctgtttc aaatgcaaat cgaatgtccc ttccttgctt
89820

caaacggatc ctttaaggag ggttgaaatc agaaccagta gatatttgga aaatgttact
89880

ttaacatgtg aatacgtgaa tgaggtcaaa aatggcttca tggaagtcaa atttctgtca
89940

ggtcttttag gattaataaa ctatcagtca atggaaaatg ctagggagta cattctttaa
90000

ggatttggaa caggaattca aagaaattaa agagtgtgta agcagaaact cagttgtatg
90060

taagaaaacc cagttccccc tgagaaagag aaaaagatga agtcctttaa aaactatact
90120

gcctattttt ctgtggctag tgagccttat ctctcctccc ttcccaggca ttgtgaagac
90180

cctgtttctc tagctgtgca gctgcgaggt cactagacag ataaactcaa gttgcaaaac
90240

atgtttttcc ttgaaaagta agaaatgatg taatgactgt ctcaattgaa taactgccat
90300

tgtttctcac ttctgtagta tgcttccccc tgcacagatc tccacccccc cacacacacc
90360

cccgccccac aaaatgctta aaaagtaaac taactctttg ttcagggctt agtcctttgg
90420

atgttaatcc gactgggcca gtgcacctaa ataattaata catatcctcc tgaaccccat
90480

caatctctct gattccttaa caatcctgct acattttctt taaggaatct gagtaggcta
90540

gattagaatg ggttgcttag atggaagaat tatgggacat aacactggaa atgttgcccg
90600

aagctgcaag gagaattttg aatgcaatac tgaagagttg caccactaca agtgctttat
90660

gcaagacaaa atttagttct gcatctaatt tccctactta aaactaacac agcttaagaa
90720

cgttaaatag gaaatgaatg tttattaggt ggcaggtcat attcagggtc tcaaaagaca
90780

ctctatgtca gagcaatgtc agaagaatac aggagagctg ttgggaggga cctgaggggc
90840

cagagagata taaatttaag agctacaatt tgaatgtctt gatgtgaaaa taactaagag

90900

ggggaggagc caagatggcc gaataggaac agctccggtc tacagctcgc agcgtgagcg
90960

acacagaaga ccagtgattt ctgcatttcc aactgaggta ccaggttcat ctcactggga
91020

agtgccacac agtaggtgca ggagagtggg tgcagcacac tgtgcatgag ccaaagcagg
91080

gcgaggcatc acctcaccca ggaagcgcaa ggggtcaggg aattcccttt cctagtcaaa
91140

gaaagggctg acagacagca cctggaaaat tgggtcactc ccaccctaat aatacgcttt
91200

tccaacgggc ttaaaaaacg gcacaccagg agattatatc ccgcacctgg ctcagagggt
91260

cccacgccca cggagtctct ctcattgcta gcacagcagt ccaagatcaa actgcaaggt
91320

ggcagcaagg ctggaggagg ggcgcccgcc attgccgagt tagttgtttg attaggtaaa
91380

caaagcggcc gggaagctcg aactgggtgg agcccaccac agctcaagga ggcctgcctg
91440

cctctgtagg ctccacctct gggggcaggg cacagacaaa tgaaaagact gcagtaaact
91500

ctgcagactt aaatgtccct ctctgacagc tttgaagaga gtattggttc tcccagcacg
91560

cagcttcaga tctgagaaca ggcagactgc ctcctcaagt gggtccctga ccccagagta
91620

gcctaacagg gaggcacccc ccagtagggg cggactgaca cctcacacag ccaggtactc
91680

ctctgagaca aaacttccag aggaacgatc aggcagcagc atttgcggtt caccaatatc
91740

cgttgttctg cagccaccgc tgctgatacc caggcaaaca gggtctggag tggacctcta
91800

gcaaactcca acagacctgc agctgagggt cctgtctgtt agaaggaaaa ctaacaaaca
91860

gaaaggtcat ccacaccaaa aacccatctg tacttcacca tcatcaaaga ccaaaggtag
91920

ataaaaccac aaagatggga aaaaaacaga gcagaaaaac tggaaactct aaaaatcaga
91980

gcacctctcc tcctccaaag caacgcagct cctcaccagc aacggaataa agctggacgg
92040

agaatgactt tgacgaattg agagaagaag gcttcagacg atcaaactac actgaactac
92100

aggaggaaat tggaaccaat ggcaaagaag ttaaaagctt tgaaaaaaaa ttagatgaat
92160

ggataactag aataaccaat gcagagaagt ccttaaagga cctgatggac ctgaaaacca

92220

aggtacgaga gctacgtgat gaaagcagga gcctcagtag ccgatgcaat caactggaag
92280

aaagggtatc agtgatggaa gatgaaatga atgaaattaa atgagaagtt tagagaagaa
92340

agaataaaaa gaaatgaaca aagcctccaa gaaatatggg actatgtgaa aagaccaaat
92400

ctatgtctga ttggtgtacc tgaaaatgac ggggagaatg caaccaagtt ggaaaacact
92460

ctgcaggata ttatccagga gaacttcccc aatctagcaa ggcaggccaa cattcacatt
92520

caggaaatac agagaacgcc acaaaggtac tccttgagaa gagcaactcc aagacacata
92580

attgtcagat tcaccaaagt tgaaatgaag gaaaaaatgt taagggcagc cagagagaaa
92640

ggtcaggtta cccacaaagg gaagcccatc agactaacag ctgatctctc ggcagaaact
92700

ctacaaacca gaagagagtg ggggccaata ttcaacatgc ttaaagaaaa gaattttcaa
92760

tccagaattt catatccagc caaactaagc ttcataagtg aaggagaaat aaaatacttt
92820

acagacaagc aaatgctgag agattttgtc accaccaggc ctgccctaaa agagctcctg
92880

aaggaagcac taaacatgga aaggaacaac cggtaccagt cactgcaaaa acatgccaaa
92940

ttgtaaagat gatcaaggct aggaagaaac tgcatcaact aatgagcaaa atcaccagct
93000

aacatcataa tgacaggatc aaattcacac ataacaatat taactttaaa tgtaaatggg
93060

ctaaatgctc caattaaaag acacagactg gcaaattcga taaagagtca agacccatca
93120

gtgtgctgta ttcaggaaac ccatctcaca tgtagagaca cacataggct caaaataaag
93180

ggatggagga agatctacca agcaagtgga aaacaaaaaa aggcaggggt tgcaatccta
93240

gtctcagata aaacagactt taaatcaaca aagatcaaaa gagacaaaga aggccattac
93300

ataatggtaa agggatcaat tcaacaggaa gagctaacta tcctaaatat atatgcaccc
93360

aatacaggag cacccagatc ttaaagcagg tccttagtga cccacaaaga gacttagact
93420

cccacacaat aataatggga gactttagca ccccactgtc aacattagat agatcaatga
93480

gacagaaagt taaaaaggat acccaggaat tgaactcagc tctgcaccaa gcggacctaa

93540

tagacatcta cagaaatctc caccccaaat caacagaata tacatttttt tcagcaccac
93600

accacaccta atccaaaact gaccacatag ttggaagtaa agcactcctc agcaaatgta
93660

aaagaacaga aattgtaaca aactgtctct cagaccacag tgcaatcaaa ctagaactca
93720

ggattaagaa actcactcaa aaccgctcca ctacatggaa actgaacaac ctgttcctga
93780

atgactactg ggtacataat gaaatgaagg cagaaataaa gatgttcttt gaaatcaaca
93840

agaacaaaga cacaaagaat ctctgggaca cattcaaagc agtgtgtaga gggaaattta
93900

tagcactaaa tgcccacaag agacagcagg aaagatctaa aattgacacc ctaacatcac
93960

aattagaaga cctagaaaag caagagcaaa cacattcaaa agctagcaga agacgagaaa
94020

taactaagat cagagcagaa ctgaaggaaa tagagacaca aaaaaccctt caaaaattaa
94080

tgaatccagg agctggtttt ttgaaaacat caacaaaatt gatagaccgc taccaagact
94140

aataaagaag aaaagagaga agaatcaaat agatgcaata aaaaatgata aaggagatat
94200

caccactgat cccacagaaa tacaaactac catcagagaa tactgtaaac acctctacgc
94260

aaataaacta gaaaatctag aagaaatgca taaattcatc gacacataca ccctcccaag
94320

actaaaccag gaagaagttg actctctgaa tagaccaata acaggctctg aaattgaggc
94380

aataatcaat agctcaccat ccaaaaaaag tccagtacca gatggattca cagccgaatt
94440

ctaccagagg tacaaagagg agctgttacc gttccttctg aaactattcc aatcaattga
94500

aaaagaggga atcctcccta actcattcta tgaggccagc atcatcctga taccaaagcc
94560

tggaagagac acaaacaaaa aagagaattt tagaccaata tccttgatga acatcgatgc
94620

aaaaatcctc aataaaatac tggcaaacag aatccagcag cacatcaaaa agcttatcca
94680

ccatgatcaa gtgggcttca tccctgggat gcaaggctgg ttcaacatac gcaaatcaat
94740

aaatgtaatc cagcatagaa agagaaccaa agacaaaaac cacatgatta tctcaataga
94800

tgcagaaaag gcctttgaca aaattcaaca actcttcatg ctaaaaactt tcaataaatt

94860

aggtattgat gggatgtatc tcaaaataat aagagctatc tatgacaaac ccacagacaa
94920

tatcatactg aatgggcaaa aactggaagc attccctttg aaaactggca caagacaggg
94980

atgccctctc tcaccactcc tattcaacac agtgttggaa gttctggcca gggaaatcag
95040

gcaggagaaa taaataaagg gtattcaatt aggaaaagag gaagtcaaat tgtccgtttg
95100

cagatgacat gattgtatat ctagaaaacc ccatcatctc aggccaaaat ctcctcaagc
95160

tgataaacaa cttcagcaaa ttctcaggat acaaaatcaa tgtgcaaaaa tcacaagcat
95220

tctcatacac caataacaga caaacagaga gccaaatcat gagtgaactc ccattcacaa
95280

ttgcttcaaa gagaataaaa tacctaggaa tccaacttac aagggatgtg aaggacctct
95340

tcaaggagaa ctacaaacca ctgctcaatg aaataaaaga ggatacaaac aaatggaaaa
95400

acatcccatg ctcatgggtt ggaagaatca atatcgtgaa aatggccata ctgcccaagg
95460

tcatttatag attcaatgcc atccccatca agctaccaat gactttcttc acagaattgg
95520

aaaaaactac tgtaaagttc atatggaact aaaaaagagc cctcattgcc aagtcaatcc
95580

taagccaaaa gaacaaagct ggaggcatca tgctacctga tttcaaacta tactacaaga
95640

ctacagtaac caaaacagca tggtactggt accaaaacag agatacagac caatggaaca
95700

gaacagagcc ctcagaaata atgccccata tctacaacca tctgatcttt gacaaacctg
95760

acaaaaacaa gcaatgggga aaggattccc tatttaataa atggtgctgg gaaaactggc
95820

tagccatatg tagaaagctg aaactggatc ccttccttac atcttataca aaaattaatt
95880

caagatggat taaagactga aatgttagac ataaaaccat aaaaacccta gaagaaaacc
95940

taggcaatac cattcaggac ataggcatgg gcaaggactt catgtctaaa acaccaaaag
96000

caatggcaac aaaagccaaa attgacaaat gggatctaat tcaactaaag agcttctgca
96060

cagcaaaaga aaccaccatc agagtgaaca cgcaacttac agaatgggag aaaatttctg
96120

caacctactc atctgataaa gggctaatat ccagaatcta caatgaactc aaacaagttt

96180

acaagaaaaa atgaacaacc ccatcaaaaa gtgggcaaag gatatgaaca gacacttctc
96240

aaaagaagac atttatgcag ccaaaaaaca catgaaaaaa tgctcatcat cactagccat
96300

cagagaaatg caaatcaaaa ccacaatgag ataccatctc acaccagtta gaatggcgat
96360

cattaaaaag tcaggaaaca acaggttctg gagaggatgt ggagaaatag gaacactttt
96420

acactgttgg tgggactgta aactagttca accattgtgg aagttggtgt ggcgattcct
96480

cagggatcta ggactagaaa tactatttga cccagccatc ccattactgg gtatataccc
96540

aaaggattat aaatcatact gctataaaga cacatgcaca cgtatgttta ttgcggcact
96600

attcacaata gcaaagactt ggaaccaacc gaaatgtcca tcagtgatag actggattaa
96660

gaaaatgtgg cacatataca ccacggaata ctatgcagcc ataaaaaagg atgagttaat
96720

gtcctttgta gggacatgga tgaaactgga aaccatcatt ctcagcaaac tatctcaagg
96780

acaaaaaacc aaacactgca tgttctcact cacagatggg aattgaacaa tgagaacaca
96840

tggacacagg aaggggaaca tcacacactg gggactgttg tggggtgggg ggaggggga
96900

gggatagcat taggagatat acctaatgct aaatgatgag ttaatgggtg tagcacacca
96960

acatggcaca tgtatacata tgtaacaaac ctgcatgttg tacacatgca ccctaaaact
97020

taaagtataa taataataaa atttaaaaaa aaaagaaaat aactaagaca tatactgacc
97080

tagaaaaaaa aatttaaaga aaatcattac ttactgtagt atttgaccaa agccagtatt
97140

tgtttaaata ctaatactta aatatatatt aatcctattt tattttttga aaaactttag
97200

ttaaaacaaa tttaatagag tttatgtgag caaaggacaa ttcccaaatc aaagcagccc
97260

tcaagaatca gaagggttca tacaactcca cttggcaacc tggcagtatt tcagacagaa
97320

aaaggctgaa gaaaacaaag aaggaacaaa aagcagatag gttgtttcaa agttactttc
97380

cttgtaaagg ttaaaccaga ggggacttcc ttattatgct gctaaaactg gactgtttgg
97440

gaattgggct attattgctc tctcttggaa ggtcagataa acaatttagt tttgcttgg

97500

tggtatggaa ctttagcatt agtgactcca ttttggtttg gtctgttggg gcctagtcca
97560

gaaactcagt ccaaagcaat ggcttcccat tattttattt aacacattgt aggggatgaa
97620

aagtatcttt tcctcatcca tccctaagtt cacagcagaa ggccctataa caaaacacag
97680

attaacaaga gaagagtaaa tacattattt aatatgtttt ttgtgacaca ggggccttca
97740

gaaattaaga caaaaagaaa taggtaaact tgtgtatttt tacacttagg ttaatgaaga
97800

ggtagatagt cgtagagagg tatgattaga caaagagagt gtgatctaac ggtaataaac
97860

aggggaattt ggcaaggtct gtttgttcac attcttccct gtgtccctct cttcagagag
97920

gcgggcggat cacgaggtca ggagttcgag accatcctgg ctaacatggt gaaaccccgt
97980

ctctactaaa aatacaaaaa aaaaaaaatt agccaggcgt ggtggcgggc acctgtaatc
98040

ccagctactg ggaggctgag gcaggagaat ggcatgaacc cgggaggcgg agcttgcag
98100

tgagccaaga tcgtgccact gcactccagc ctgggcaaca gagctagact atgtctcaaa
98160

aaaaaaaaaa aaaaaaaaag aatgttcctt ccctccaggt ataggaagga tgcctcttga
98220

atgaagatat tttctcaaag gagaagggtt ggggaaggtt gagcatggca tctacttcct
98280

ctgttttctc aaattccaag cctccatatt ttgagatggc atgttctgaa gcccatggct
98340

attccaaaat aatccattct catgacaaca atctaattgg tgagaatagg caacaactac
98400

aataaaatta gaaaaaaggg tgccataatt ttaaaagaaa aaatgctaca aaaattattt
98460

gtccccatcg agaaagcaat aggttgttta agactgtgct ctggtgtgta gaagcttggt
98520

tatcaaattc aagtgctcag aggaagatgt tttattctct gtttataaaa aataaatagt
98580

tctgcattag gaaaagtttc tgcttaatga tatgaatcat ctttaactag ctaagagtga
98640

aaccgttgat ttttgacttg ttaatctgct tgaagactgg gaaagggtga attactgtag
98700

tttcagttat tattataact attatttttg ttttctgatg aagtttcgct cttgtccccc
98760

aggctggaat gcaatggcgc aatctcggct cactgcaacc tctgcctccc agattcaaac

98820

aattttcctg cctcaccctc ccaagtagct gggattacca gcatgcgcca ccacgcccag
98880

ctaatttttg tatttttagt agagacaggg tttcaccatg ttggctaggc tggacttgaa
98940

ctcctgactt caagcgatcc actcaccttg gcctcccaaa gtgctaggat tacaggtgtg
99000

agccaccgca cctagccttc tgttactatt tttaaggctt taaaaaaaaa tagattgata
99060

atagaaacac ctggattaaa ttccttgacc tgcaatgatt gtagagcttt atgttgcccc
99120

attttatcag tcaacaaatt tgtcttaaca ttaaaaaaaa aatcttgaca ttaatgagat
99180

acatattttg caaacttaaa gttacgtaag tatttctcag aaacagttat tatcatgatt
99240

cagaatgtca gttttgataa tattatagga tattttatga catcttaaag taacatttaa
99300

aagtaggtga aacggactca tggtggattg aatatggtta tctccctccc tccattaacc
99360

tggcatttac tgaaatggca acacttttag gagtaacaca gaagggtaat ctctcctggt
99420

tacatgccat ttaagaaata ctggtgatag caatgattta aacttaatgt tagaaattga
99480

gctgaatgaa aaaacataat gtgtttttag actgcaaatg gcatgtatta gtagactcca
99540

aatagtgaga ttctaaaaag tatactagtc tgggagagga ctagactttt ctagactaga
99600

actaaattat tttgaaacta gtattttaat taaaaaaaaa aagaaagaaa agggagagag
99660

gaaaaagaga gagagaaata gagagaggga gagaaagaga caaaggaagg aaggaaggaa
99720

gaaagagagg gagggaggga agaaggaagg aaggagagat atggaaggag aagagagaaa
99780

attaaataga acaaacaata cttggtgcca aattcagtgg acatttttcc atgagaataa
99840

aaacaatcat aaaactataa acatgaataa tacataggaa cataaatttt gtggctgctg
99900

tcaaaatgat aatgctgctc ctatttttag aaatattttt actgtataca ttcagttaat
99960

tttatccagc tgtcagtatg cattctggaa gttgccagaa agtgaatgaa tgtgaacatt
100020

taaaagaatg aataaaataa tcttaacatg tctgtgagtg taaagaagag ctcgtgcacc
100080

aaatggcttc actgtctact ttttggcaca ggtatcaagg tggtacatgt aggatatgct

100140

cttggaattg tttttccaac agaaatttta gatttgtaat gaatattttc aatcttaaag
100200

ggtctttctc atggccaata gtagcatcct tttctgagat ggctggtggg gaccacagaa
100260

ccagtctgct gtctgataaa ctaatccaga caagctcaga taaggatttg gctcttgagc
100320

tcacactcaa aagttcccca atacaatgac aagcattctt aattcctcca ttacaatttt
100380

ttttaatggt gtggttaggt tgtctgggta cccatcagca tatggccttt gcactgacca
100440

ctggtaaaca gaggactgtg tggccatgca atgatcacag gctttgggat cgagggatgt
100500

ggggtcaggt cccatcctgg ctacccacta gctatgttat ataacttctg gcaattttcc
100560

catctgaaaa tgggtctaat aatgtctact tcacagggat agcttacctc ttcacaatta
100620

tgtaagcaaa gaatttagca ctgtgccaaa cacagaatac agtcaccagg gccgacagca
100680

gtcacttttg gcagctctaa catgttgctg ctcagatttt catgtactgg ttgttttttt
100740

tttaaatgaa gccaaaaatt ataggaactt tacctaacac ccaatgatat ctgaatgcct
100800

tcttcccaca aaagttatgg ttattccaaa attagaatgt taataataaa tcaaaaacag
100860

tgggtcttaa ccaagaaaga cagccaagtg ttttgaagcc agacaaatcg gtttctaatc
100920

ttaacttcct acgtactagg cacatggcct tgggtgagta attcatgtct ttcaattttc
100980

tcatatgtaa aatgggcaac agtacttact tcatggtgtt gtcatctaga tgaaatgaaa
101040

caacgtgtat gaattctggc tgtgccattt accagctgtg tgatcttggg caagtttcta
101100

aatctctggg tgctgcagtt tgctcatagg tcaggtggat ttcacagtca tcctaacgca
101160

gaggttgtta ggaggattac atgggttagc tcagagaatg cacctaaaac aatgactggc
101220

acatggtaag tactcaataa atgttataac gctactcatt tttcattgtc acttcttctt
101280

cattggtttt attgttaata gccagaaatt aggttgatga gataacaatg gcgtagagtt
101340

gaaaaaattg ctttaacact ttagagcata acacgccaag aggattaacc tactggaagt
101400

ggtatgctgc ccactgctct cttggattta atattacatg gactctcaat ggaaagcaaa

101460

gaggagttga ggtggggcag aggagaagta gttgctctgg aaacactgat ggaagtgaga
101520

gggccactca gctgtggtga gccacctctg agccactgaa actggcccag tgtcatcttt
101580

catagttgtc ttcaccttgc atttcacagt tcacaaaaca cttcatatcc accatcacac
101640

acttactctc cccatgctac cccttgcagt aggtggatat gctgagtggc caatccccat
101700

actgcatacc ccactccact caggtttctt ttttgcttgg ttttctctct agtgatggag
101760

gtctcccctc atatcaggaa ttaattaaaa aaaaaagttg ccatggaagt tgctgaaagt
101820

ttccaagtat cgacaggaga atagttaatt ggcctgtcag actacacaac ctcctcagag
101880

gtcatctctg gctgagtaaa cactgcatct ctgccattta ctcactaaag cagtcaaaat
101940

tcctcttgac agaactggat tccaaaacaa caacaacaaa aaaaacctgc tccaaaggta
102000

agagacagct tattaaattt gtgtcaagcc tactgcctga taccttttaa gcccagtctg
102060

atttctttca ggcccttgac acgtgctaat tggcattact ctgttagcag atgctgagag
102120

gagacaggca tccaggcttc ctgcttctct gtactcagca tgacatcctc tcataaatac
102180

caaatgggat gccttaaacg actgggaaat acatgctaaa tgggtactta gatgtattta
102240

gacagcttat ttatcactga atatggcaaa actctttgtg tgctcttttg gggaggtaaa
102300

gaatgtcaca agcgatggag caaagtagaa aatgtgccac atggaaatca accatattgt
102360

aaacagtaca tgcctattat ggcctttcca caccttacag tttattacta tgggagctct
102420

tacagctcct tcctgggctt tcattctttt gacaaatatt tatcattgct gggctaggtt
102480

ctgttctggg cactgggaat acaccaccaa acaaaacaga taaaaaatgc ctgtcctcgt
102540

ggaacttata tagtaataaa agatgtagga tgtcgcatgg agatgaatgt gatggagatg
102600

ggatggggtg gggtggggtg gggtggggtg ggatggggtg ggcttgggac agagttgggg
102660

aatcgacttt aattaacctg gtcagaggcc aggctgagca aagacctgaa ggaggccaag
102720

ccagtcaggc cggaatagga ggagtgtttt agaggagggg cagaagcctg aggggcagcc

102780

agcttggtgc acagagactg gcagatgaga gcacagggag tgtgggacag cgtggtggga
102840

aatgcggcta gagaactggc tggaggtgga tacaagccga cctttgtaag ggctcaacca
102900

ccactgcact ttagaatccc ctgtggagct tttaaaccaa gtgaatcaga atctcctgcg
102960

gtgaagccca ggcctgggga tgtttttgaa atctccacat gtgcctccag tgagtagtca
103020

gaggaaggac atgagctgcc ttctgctcag agggatcctg agctttaaga acagacccca
103080

ggaggggacg ggtggatgca gggaggccag tcaggtgctg caatcatcca gggagggagc
103140

tggtgactgg gatgtgggat caagtggtgg tggtgagaag agctcagact ccaggtatat
103200

tttgaaggca gagccaagag aatgttcctg cagatcaggc atagggagta aaagcagaag
103260

gtagtcaaag atgacaccaa ggttttgggg ctgagcaatt ggaagaatgg aattgccatt
103320

tgactgaaat gagaaaagtc atgggaagaa cctagaaggc atttttacat catatacgaa
103380

cactttgcat ggaaatgccc atagatagta aaatgctcag tacctggcac gtaaaggatt
103440

cactaatgct tttaaaagac agtatgtgtg gtgaggctga agggcaagta tctacagctg
103500

agtatcttta aggtggtttg gtagcaaata ataaacaagt gttccaagca tttttccaga
103560

gatacgtaga ggagatgaag atgtagccag gaaaactccc tataaatgtc tctgcctcca
103620

tctaaatctt tagataatgt gggaagaggc ttcattgcct ggcagaaatt ctggcaactg
103680

cccctacatt caaggaaaag tatgagatat acttaatact ggtagatgac actcccatct
103740

cccccagtgt gtggtactta aagctgaata ccctgagtga caaatagcag gggagttagc
103800

cagtcacctt gccactatat gtagaaaata cctacagagc agtgccttct ctagttcttt
103860

aggaaaacct cagccctttt acggggctgt atataatttc attttcaaca accctgccaa
103920

attcctaaca tgcttaatat tgaccaatta aacaagaaaa aatgtaataa tacatttgtc
103980

attcaaatca ctatgtacta tagctaatta aggtccttat tacaaattat atacaatagt
104040

caattatatg ctttttcaat tgaatcctta catgagtaag atttattaac acaaatagct

104100

cctgacaaac ttggcccagg aacaagcact gtatttcctc tgggttttgg aaaaaagtca
104160

aagttattat aaacggttgc ttaggggaga tatttgctat tttctccact gatttgaagt
104220

aggtcagatt gagagctgaa tgcttcccta aggttataat taagtatgaa tattaaaaag
104280

ctgagaacaa ataaaaaaaa tacaactgct ttaagctttc acatcacaga ccttgcattg
104340

tgttttcacc tcaggcaaga tactttctct gggaaaagga atacggttga aaaaataggc
104400

tttaaaaaaa ctgcaatagc tctacaagcc tagactttga gaaagatttg tgcaaacaaa
104460

agtgctttga agaagtattt aaccaggtag agttggggca aaaatgcaca caaatgctga
104520

aaaagcagcc tgaactattg tgatttaatt ctgcaggaca gtctgatgtt tatccccccg
104580

cccccttgaa aatcacatct ttctgcctct tcccatccaa atcattcact cttataaaac
104640

tagcttagtc agatcaatat ctgagctagc aatgtaaatt taatcatggt gaggcctggg
104700

taaacattag ctctaagaca tttcttggtc tatagagtga taactgaatt aagtaggatt
104760

atttcatcat tacagtatta agcccacaat gttacacaag agaagctaat atttctaact
104820

cttttcagct tagcaaaata tgctataagc atttacatga gccaatttag tatgtttcct
104880

ccttgaagag ctatgaaata ggaatacatg gcagctaaat tttcagtgta gagagaggaa
104940

tgtctagtaa tcaaactatc gaagggcctc attataagac cgttcctggg atgctggcac
105000

tctggcctcc tgacagccac tttagttgaa ttcccatgga cctgggaatt cacagtcttg
105060

ccttccatct cctgcatttt cagctgtgca gattctctaa acatagtctt ccttgaagcc
105120

cccacttggg cattatttct tccaggaatc tttccctgac ttccctgtct ttgggcattt
105180

ctgtatcatg gcactggcct catgataaaa ctatatttct gcctctcttt gaaacctggg
105240

agcagcttga tcatcattga tgtctgggtc aagcacaagg actggcatat attaggtatt
105300

caatactatt tatggaatgc atgaatgaat gaacgaatga atagaattaa aaagctaaga
105360

catggaaaca tatcccatgt gctaggtact ggataaagag atagaaggca tatcctctag

105420

aagttgaaga tctgggtcag gaagggggga atataaagag aggcagaaga atataagccg
105480

gcatttctta aggaccaaag cagtgagctg ctaggactaa ggcagccgca gcatggaaag
105540

acagagtaga atcctggaaa cagccagcac actgaaggaa aggtagcctc aagccagaca
105600

aaaggaggca ggagggcaaa ggggacatga tgagcaacgc cctcttatgg agctgagaag
105660

aattacagtt taatttatta gatgacagac attgaaagac tgggttctga atccagacaa
105720

ctgggtttaa atcctgactc ctttactaca tgctctctgg gcaaatcacg taatattcct
105780

aagcctcagt atctccatct gtcaaatgga cacgaaaata atatccccct cacagatatg
105840

agataaaata tataaagcca ttagcaagga gcttacatat aagaaatgtt attattgttt
105900

ttattcacat tctgtatggc atggctagga cacacacatt taaaaggctt cagcctaact
105960

gtatggaggt gagggctgtg catatagata gaggaccagt gcaaaatttt aaggtccctg
106020

actcctctag atttgattaa gaatgataat gtataaccct aaaacatcag ctagctgcag
106080

gtcaagatcc tagctaaggt aatatttgcg gcatttgtgg gatggttatg tagtatttaa
106140

acctgtacca cattggccta gttggttaaa acaagatctc tgagtatatt cccttctcac
106200

ctcctactgg ccattatagt tacaaataga ttacctcatt tcttttgaat gggtttattt
106260

gtttgctctc tcagttgtac tgtaatctcc tttaagagga gaaagtggtt attatacatc
106320

atttatggct gtgtctagca atgcccaata atcttctttg ggttactact agccattcat
106380

caccaaagtg atcaccctca atcctagatt cttgactcca tgactctaag atcctgtgga
106440

cactacaaaa attttctaca aatacatttg acactctgct aacctcatag ggaagtttaa
106500

tcaagtaagc tagtgagact accctgttaa ataattttaa aattatcttg agaaatctat
106560

ttttaaaatt agttggaaga atctcccatg gggtctgact tgtctctgcc actttcctag
106620

aaatttaaaa caaattctct gatattccct caaatagaat gtttacccat acatttgggt
106680

cattttttaa tttaaaaata ttgaatgtaa tcacttattc atttcaatat tttgatatat

106740

agtttataac gataaagcag atatttattt tgcatattag gcaatttcat gtgtgaagtg
106800

caactggact aaaaagagcc aatagactgg agccaatagt ttataaacct cttatcttct
106860

taggtagatt tcaaattttg ctacttagta acacattgta ttttataggg aaaaaaacat
106920

gttcaccttt attatgattt ataatcccct gaccttccat ttctgttctc cccagacttg
106980

ctgcgagcat tagttcattc aactcttaca tttatagaga gcttgcttct cactcatgct
107040

ttacaaagtc attctattgg ttaaacgtct atgcgagtac tgtggagaga tattttttaa
107100

aataattatc atgataaaaa cagcttatta actcggtcac ctgggaaaat gatttatcct
107160

ttcccatctt ttgcttttta ccatctgtaa cagagtaata tttcctgtca tttaaggctg
107220

ctataacaag gaatgataat aaataaaaac tatctcacac agtgtccctg gtatttagta
107280

ggtgcttaat aagttaaaac cagtataaat gccatatgca cattttacaa ttgacaaagt
107340

atctattttt atatactctg gtccttattg taaaagagct tccttccctg gcatctatcc
107400

acaagcaaca atggccgctt aagcagtcct ctaaattatt caagagatag ttataagact
107460

ttttagttct actttttata tattaccatc caatattact tgcaagggca aattaatcat
107520

catctttcca cactaacttt catattataa taaatcatga atcagggggc tggctcttgg
107580

gcagggatga atgagagaac catgaaaaaa atcttgtgct ttctttaaaa actattcttc
107640

taatatattg gagaagcaaa gagtttgctt ctccgtagac gaaatctgca ctcaaacatc
107700

cttggaatat gtcctcactt tattataatc taatcaaatt tgaaagctcc aaagctcatt
107760

gctaggaacc caatctgttg tagctgcttc aaactagaaa tagttttaac aaatgaaaga
107820

atcagggata tagtcatctc aaagtaatta ttccatgttc cccagtttag aaaaatataa
107880

gtcataagtt acagccaatt tactctatgt ttcatttagt gtccgtgaat caaactcttc
107940

tgtttttcta atatatgaga atatttttag agggctaaaa ttcttctttt tattaataaa
108000

tgtattagca agtaaaagga atgtattttg acatagcctc aaatacacaa tttgctatgg

108060

tcccatttca ggatgtgttt catcaaaata ggtagaatca gaataggtag aatcagaatg
108120

gcacagtaaa tttaaaaaaa aaaaaaagtt gggcaggcct taccccaaac atcttgaata
108180

ggaatctctg gttgagaagt gtgcaggggg tagctgctgg aaacccttgc ttgtttagct
108240

ttcccctacg agtctattga gaaacctagt tttgaaccta ctgctagatc atttggtaaa
108300

ctagatgaca ctggaaggtg acaccaagct agcagcagtt atggcaaagg aaaagcaatg
108360

atgctgtagg atgcttaggc tgtatcctgt tttaatagtg cttcgccaga ggatcccaaa
108420

tcaaatatct tttcagaatt tgaactggta atgtctcttc ctgttcctct gatgtttcca
108480

cccccctaag tttaatctac gtcccttgtg attgttagga aaatgtttat tttaaaatat
108540

aaaaaacatt tttccgtgca aataattcaa gcaattaaag ctaaaaaatg aatatatttt
108600

agtgtctcta gactgaacag tgaggactaa tgcagaagtt aattataaat aagaaaatct
108660

tttaggtcct tctttcagat tctagatctt gaaagtgtct ttaaaatttt aactcacaac
108720

tatagaatat attatcatat gtatataggg aatagtggac ccagtatctt tattataata
108780

aaaaatactc taagccttgg aaaatttcac ttttatgaac tttgattgat ttctccagtg
108840

agtttccact tctcgcaact atataataaa aaatactaag tggcttaaaa aaaatcagtt
108900

acatagatta ttacttggaa agaaaattat ataaaaggtc tcagtgagat aaattttaaa
108960

agggtaagtt acttttgttc ttatatttaa aatatatgtg atcaggctcc tataaaaaaa
109020

ttggtacatc caagaactta aaattctaca tcataacatt cagtttcata cttttttgat
109080

caacagccct tcctactttt gctcaaaaca cagctttgct tttcagtatt tgggattttc
109140

tttctgcttt tgtttgtttg tttgttttgg ttataagtat cttagtttct atctcagctc
109200

atcctatcct tttgccagaa agccattcct tactcctagc actgtccata tttccctggc
109260

aaatgcaaca cttactttca taggatcaag gatttgagga atggaagaac cttagcggtc
109320

ccttccgaaa aagatttaat ttaggaatcc tctctccaac ttccttgtca gaggatggtc

109380

ctagctctat taaacattcc cggaatatca cattgctgga gagctttcat ggatcaagag
109440

gtttcccttg tgtccagact agctgtatga tcctaaacaa atcacagcat ctcttgagtc
109500

tgcgtttcta taaagcaaga agaggtgatg atttccaagg tcatacaagt cacagaattc
109560

ttcctaatta ttttaatggc agtaagcaga ataaaatgct tgcttgtaac atctttggct
109620

ctgaaggata ttgtccaaag ggtgaggttg gtgtttaagg ggcagggaca gtaattttta
109680

acaccatcca atagggactt ttcaatgcta ccgagcagcg gatctacata ctgttcccta
109740

aaaggacacc tcctggctac ttgttcctta acttcccgct gcccagtctg ccactgaaag
109800

ccccacatgg cacttaggga gctctcacca tgcatccctg attatcgtta ttactattac
109860

tattctgatt tgttcacaga aaaccattac taccagtcag tggttgatct tctatcctta
109920

tctacttatc tgcaatattt ttcactgttg cccactccca ttctttaaaa attatcttct
109980

ttcttagccc caggacaatc aatctttgat tctcctttgc aatgttttga tcactttttt
110040

atgcccatcc cttaaaaagg agtcatttct ttggacctcc aatcttcacc agggagaatg
110100

ctaaacccca gctctccttc tttccctgcc actgtttgac cttacacagg tcgtattctc
110160

tctgggcctc atttgtccct gagaaaagag aaggagctgg agtgatgatg tggaacatct
110220

cttcaagctg tacactgcta tgctagtcat cttaatccca ttcctgtatt ccttcagtat
110280

gtggtagagt ttcagaaagc aagtataggt ctgtgatgat gctaatacca ccaagaaatc
110340

attcattcca gtatggggag aaaccttaaa caccatctgg tctgaaacct ttgttttaca
110400

gatgagggaa cctaggttca gagagggtac ttgattgcct caaagcagag acaggataat
110460

aaggaggtct cctgggctct cacttgttgc aaaataaatt tgagtgtttt ctggatctgg
110520

ctaaagtgca caatgatatc agagtaaaac gtcatacgga aggtataaat tgatctagaa
110580

cacattaaag ccttcccctc cccctttaaa aaaatcaact ttaagaattt tggagagaga
110640

tttgctatca gtggtgcttt atacaaaata gcataatctg ataacataag acattttaga

110700

tttatgtttt taaatagcca aataaaacaa aatatcaagt cacagattac atatatttac
110760

attaattcaa atgtccaaag cacagtacag tagggtctat ttaatagttc acataattta
110820

agatttacat atacacaagc acatgaacca atattagttt gctagaacag ggatttaaga
110880

agttactcag acattttggt attgacactt acatatttat ggcaacaaat tatgatgact
110940

ttaaattttc aatgagatct tttgtacaag aatacagaat gggaagaatg tacaaaatga
111000

aaagacaggc aaacaaatgt actttccttg cactatttct ataacaccat ataggatgtg
111060

gcattatcca gtacatttta tcagtgtagt ctattcattc tggtctaaaa cggtaatttt
111120

ggctgaataa cccccaaatc taactttgct ttaggcttta taattaattg atgcttgatt
111180

cttctttcta catcttttaa aaatgaaatt ggacactagt gtttttcttt aaaataactc
111240

tggtactagt aagttagcaa agtctatttt taaaggacac taatacataa agtttatttt
111300

ttctttcaat atcctaaata aagggacaat gggagtcata tcacctttgg gcctactctg
111360

aaattaccaa tagtgctaaa agctaagata ttgtgatcta gaacttaaac tttggaaaac
111420

aaccccatct ttctatggca cattgaggaa ctgaagtact ttacctcatt tcctaccaat
111480

cattttaaga gaatttggtt gtatttcaaa gaacaaaaca acacaatttc tgtcctgctg
111540

tttattttgc agaccacaca caaagttaaa tatgaacaga ttaaattatg acatcataca
111600

aatataggca caaattaagt ggacgccaca aaaggtgtgt cagaaaacac tgaattgact
111660

gaatctgagt cagatttccc cctcgtggac tttaggatgc cctcagctat cactgcctcg
111720

ctgggaaaga gcctgacttt cccgttctgc cctgctgcag tatccttcag gggttccaaa
111780

aacaccactc ttttacctgc acctgccttc cgttcatcag cggaggcatc actagcgggg
111840

ccaggactga gaatcgatga atgggcattg ctttggtgta gcatattttt ctgtctcttg
111900

gttttacact tgcaggggca tggagtcaga tagaggtaca aaagtaccaa aacgatactg
111960

gccacgcaag cagcaagagt ggtaaaagct gtgttaaatg cctcatgagc atgggatctg

112020

cttacagtga aattgctcac atttattgtg acgtccacag tttcatttaa caggcgttgc
112080

ttattcattg cgatacaaga atacactcca gcatcctcaa aacgagggct ttctataacc
112140

agacttccat tgtgaaacac gtaaaagttt tccatctctt tatccggctc tagcagtctg
112200

ttatctggac ccacccagat gaaatccgta tttgcattac ctgtcttgct gtcacagtgg
112260

accatcagtc tttccccgac ctgagcctca tgaataaagc caagcgcacg aaaggaacca
112320

ttgatgatgc tgtcagagca attcataaag ctatcctgga gcagaagtac ctgacgcgag
112380

tgcctggagt cagaccacag gcgacaggtg taatcgttct taaaatccat cactgagcta
112440

aagtgcctac gataccaaaa gaccagcaag gagtacaggg aacagtcaca gacaaatggg
112500

tttccatgaa ggtagatgcc tctcagctgt tttcctggca ctaaatttat gtggtgcatt
112560

ggcatggaag gaattcggtt ataagaaaca tctaaaaaca tcagttctgc cagcttgaac
112620

cttccaacat acaaatccat cggaaactgt gtgagaaaat ttccacttaa gtagagtttc
112680

tgcaactggg agagccctcc aaacgctgaa ggatcgagat aggatatgtg attgttgtaa
112740

agcagaagca cttccagaac cttcaactct tggaatacag catttttcac cgtcttcagc
112800

ttattggacg ataagtcaag acacttcaaa tttggagttg tggaaaaact gcccgtggaa
112860

atgctggtga tgttgttatg acgaagaatt agggtgttca gctttgcaaa cgatactgga
112920

atccactcag aatccagaag cccaattctg ttataactca ggtccagtct cttaatcagt
112980

ctgaaaaggt tcccaggcac cttggacagg tttttgttgg tgcagctgac gatgtcagtg
113040

gcacagatgc aagcggtggg gcacacccca gaggcaccag ggcccacagt cactgtgatc
113100

atcagcaaac acagcagctc cctgcagccc ggtctgacga cggctccaag cagggtgggc
113160

agagtgtgta cacgtaacga cattatggtc gcctctgagt ctcttcccgg tgtcttttcc
113220

accggctcag cctcccacca gtgaacctgg caaacaatta ataattcaga gggagtaaaa
113280

agcagaaact gactaccact agttaaccca taagagccat accttacttt ctttccaata

113340

acttttgaaa tgggttttat ttcacaatag ggagctctgt gttgcctctt cactgttaaa
113400

tgtatttttt cctctttcct gggttagtcc tgcttataaa aatgtacagt cacattctct
113460

ccatacaacc caccgtctca cccaccatca gaaactcaat tttttcctaa ggtgcatcct
113520

gcgttgttgt tgttgttgtt gttgttttaa actccctcca acagacacaa ctatacgcaa
113580

acgtacgcta caaacaatta aacggaaata cttagcaacc acccatccag gaaaaaactc
113640

acggtgctgg ggagcctcgt gggctccggg gaggctgcct acgcagtgcc ttccgaaggt
113700

ctgcgcgtcc gtctgtccgt gtctgtcact cttgcacctt ccgacttcct ttccctccgg
113760

ctcccggcgg gcggcaccct ctagccggct ctcaactttg aggagtttca agcagccgcg
113820

gcggcagcag cagccctgga cgcagcagcc aagctcttcg ctggctcccg gggctctcc
113880

cgggttcctc tcattgcagg ctgaaggtac cgcctcggcg ctctggccgc cgtgtgcccg
113940

cgcgcggggc gccccgctcc cagagccggg gccgcgggag ggggcgcagg cagccgggcg
114000

gcagcgggcg gccccgcctc tccgcactcg ggaggctgca ggacccgggg ttcccgcgcg
114060

cccggggccg ggagacgggc tggggcgccg gtcccacccc tgcgcgtcct gcctcctgcg
114120

ggcagcagct cggagcctgc gggagggagc aggctgggcg cgtggtgggg ggcgcgcgat
114180

gcggaggggg cggcggcgca gccaatccga gaggcggccg gcgccccctc ataccgcccc
114240

gcggcctcgc cgcctcctcc cgctttcctc ctccagctcc cacccggacc tctagaacgg
114300

ccgcgcagag cggggagggg gcaggggtcc acaccagagg cccagactct ggtccttgag
114360

tcaagatgcc caacccactc gcctaccaat tacgtccctt cgtccagctc caagccgggc
114420

ggtaatgggg tccaggaagg ctaaagggga cacgcccagc caccgaggga ggggtctcac
114480

ccttggagac ccacttcgcg gcggccgcac aggtcacgtc tctctcccca cccaggccaa
114540

ggaatccccg gacctcttag agctttgctt tggggcaagg gccaaggagg ctttgcctgc
114600

gccaagtgca gttgactgtc tgggtttaca aaataaaccc agaagcagcc tgacgtctcc

114660

gctcgtttct agggaggagg tcgcggggag ggaagtcggg aacgtttcct ctccacaggg
114720

atatctgctg ggaggaagta accgggaaat cgccacgggg acacccctca cctccctggg
114780

aaagttctat ccctttcctg actcaggagg cggcaacagg gatggtgtcc aattcaagaa
114840

ggaggagggc aggaaagaag gcttggagcc gaccttccgg ctggaatgca aagataagag
114900

tattaaccca aataataata ataatcccag agcagacctt tttttctttt ttctctcttc
114960

tccttcccct cttctctctc cctctctctc tccctctctc tcctttctct ttcgttctct
115020

ctctctcatt tcctctgtct ctgtccccgc cacctccccg tcttttgcaa gcgtgctcgt
115080

ggcgcgtgcg ggacagggaa gtaaggggcg tgtgcgcgga ggctcttcat cccttccgga
115140

gatgccgcgg ccgaccagcg cgctttggtt cagcaagcac agaaggtgca agccgaacaa
115200

ggccattcag aaatgaggtg gagactatga ggctgatgag ctctacgatt aaaaatccat
115260

ttttgtttaa ttccaagata agttttaacg atgggatttg agtgagtcag atcgcccatt
115320

tggccattat atccggatta aaagaaataa attactgcct tgacaaaaga tgccaaccac
115380

agtggaatga aaagcaacca aattgaggaa acagttgggg caggatagat aacttgtaat
115440

attacttaaa aataaaaatg atgaggacaa ccaactcttc ctcccctccc ccgcgcacct
115500

tttcacttga gcaatcttga atttagtgaa agttgtttcc tacatggtcc gtgagtcatt
115560

gctgtggtca ggcagatttg ttagaccaac acctaattta aatagaattg tttgtggaat
115620

aaagccgggc tagcactaat aaatctaggc attttcagat gaggacaact tgattcagat
115680

taatttattt catgaaagtt gaattttatc ataagaacac agcctaaaat gcttcatgca
115740

agcatttctt actgtgtact taaaggggag aggcagaagt tgagattttc tagtattagg
115800

ccacacttcc tgggattctc aaaagcttgg tctccaggac ttcaatttta tagagttaga
115860

atagttaata cagaggaata gaattaaaat cgctcagagg aatatttttt aaatgactaa
115920

taaatgacca ggtttttgca ggattttact aattaacatc gtattttaag gaaaccatga

115980

aggcagaaga aagacctgta tttaatgcaa aaatagtcta aaaagaataa gtgattcaaa
116040

aaaatggaaa caggaaggtt atattattat aactacaggc atattgtaga aacattatgc
116100

agagattaca cagcaaaagt aaactaaatg cactcaaaat ccatagacaa ggctatttat
116160

ccatttgaag acctgatttg ggggatttca catgtttgtg aaagggaaga acgttgtaaa
116220

aggattgagt ttactagctg ggaggaaaga aagataagag ttgatcttaa ggtataagac
116280

gtgaccttat tttgtggagg ataacatttt tatctttcta gcatcccttc tccttctaac
116340

agattccctc tttccactag aaggtaaatt ccaagaggat agcagtttta tctgttttgt
116400

gtattgctgt attctcagct ctcagaaccc tgcttaacac attcaataaa tttttgataa
116460

atgaactgat gaacagggaa cctattcaaa gtggttaaga taacggttta ccccaactga
116520

atctgctggt tgctgtcctg ccttttatgc tttgaaagct tgtcttaaaa aaaaggagaa
116580

gaaagagaaa gaggagtcct gtaaatgtta tttgagtcct ggatctggta gtacctgaag
116640

gccactgcat acttttactt cctttttaat tatattttcc accccacccc cttttttagc
116700

ttaaattatt ttgagttgca tgtcacttgt gacggacaga gtactaacac atgtaatgat
116760

ggtgataata aatatgtact gagcctttat tatatgccag acactgagct atgcgtttta
116820

catgcattat ttcatttatt gctcaccaca gccctgtgac attatataat cctataaccc
116880

agatggtaaa caactgtgtt ccaaattatt tgaaaatgaa atgggatgca attatctcaa
116940

ggttctattt gaaggattta gattaaacag aagagagacg tttctgccag ggtggttcca
117000

acctggaatg ggttgccaag acagatggtt gagttgcctt ctcaggagtt taaaatagga
117060

tgcgatagat attcctctgg cctcttccac atgacataat acattggaat tttgaccat
117120

tcagtaatga ctccaaggga tagtagaatg tggcttgaca cttgacataa ttctgaatct
117180

agtatgctga gaagggggtg tggtcatgtg agtgtctagc tgggtttgga aatactatct
117240

gcctgaggca gtaatttgag gtgatgaaat cacaggagtt ctagccctgt aatttccaca

117300
atttctcagg ttgtaatcaa agatttaagt aattagagat atgagacaag aaattaatag
117360
cacatgtttg atgattctaa gtcattttta ctaataatta acacttcaat agcactttat
117420
gtcctaagaa gggctttcac attctttatc tcctttgact ctaccacgcg tcctgtagtc
117480
aggtggaatt tatacactgg cagatatctt gttgcctctt ttttcggcgt gaggcacact
117540
gggtgttctt tggagatgga gctcactggg gtcactgatt gctgcatgca ggagcttcct
117600
gtggagtttc atcactctgg cgggcagcac tcttaatagg atttttacag tgtaacttag
117660
cttgaaccca caagaaaaac atacctttgc ccaacaacta tggaagtgca ctcagctcct
117720
acagcatcct gttcttctct tggcctgctc cagttggttt ttgtaaattg cctaagattt
117780
tttatagaac cagatctctc accccatcct gtgtctttca ttatatggag atacattttc
117840
tgggaaaact cagactccag ctcctgtgct ttctgaactc caggaggcat gggtcagctt
117900
atggcatgtt tctctggacg ctccctttac ttggctggaa atggggggat agaatgggag
117960
tattcagtat tctgcaggag gtactgcatg tcctccccag gaagagctca caatggggct
118020
actctttaag cagtctccac tcactcatct ggttggtctg tctcttatct tcattgacat
118080
aggccggaca gttttttgtt ttttaattca cttgagataa aatccacatg acataaaatt
118140
caccattgta acaattttaa agtgcgcagt tcagtgattg tttaatatat tcacaatact
118200
gtgcaaccat caccactatc ttaattacag aatacagatg ctcctcaatt tacaatggga
118260
ttacattcca aaaaaaactc aataaataag ttaaaagtgc atttattaca ccaaatctac
118320
tgaacatcat agtttagcct agcctgcctt aaatgtgcac agaacactta cactagccta
118380
cagttgagca aaatcgtcta acacaaagtc tatattatat ttaaaaaagt gttgaattgc
118440
tcatggaatt cattgaatac tatactgaaa gtgaaaaaca gaatgattgt atgggtactt
118500
gaagtatggt ttctactgaa tgtgtatcac ttttgcacca ttgtaaagtt gaaacatcct
118560
aaggcaaact gaaccatcat aagtcagaga ctgtctgtat tttcatcacc ccaaaaagag

118620

gtcctatgcc cattaagcag ttactcctat ttccctttct ctcagcccct ggcaaccact
118680

aatgtacttt ctgtttctat ggatttgcct attctggaca ttttatataa atggaatcat
118740

tcaatatgtg tttttttgtg tctggcttct ttcacttagc atgttttcta ggtttggact
118800

gggattttta gctaagggtt gcacagaatc tgttaggatc acgctgttga aagacctgca
118860

taggttagct gcactttaac ttagaatgtg gttgtgggga ccaaagttcc acttacccag
118920

agtcttgaat tataaaagta ttaaaactct gtgtgattgc agaaacctac caaaattaca
118980

aggtgaggag atttaagagg tgatataaaa tttagcttca gcagaaaaca tttaggctgg
119040

gatcggtggc tcatgcctgt aatcccagca ctttgggagg ccgaggctag tgaatcactt
119100

gaggtcagga gttgggagac cagcctggcc aacatctctt ctggcaaatc cccgtctcta
119160

ctaaagttac aaaaattagc caggcatggc agcacacacc tataatccca gttactcagg
119220

aggctaaggc acgagaatcg cttgaacctg ggagacagag gttgcagtga gcagagatca
119280

cattattgca ctccagcctg ggtgacagag caagactccg tctcaaaaca cgaaacaaaa
119340

aacccattta acctctccag gaacctcaag agttaattga ggatgtaaca attgactatt
119400

tctaaagttc ctttcagctt caaaaaattc acttgatttt ctgataacaa tgtgtcctct
119460

gttgcccaag tcccgggaac cctgggtttt gtttcttcac ctgccagtta gcactgccct
119520

gggcagacac agactcctct tctctctcca gtgaatgacg tcagtcccat tgctacttca
119580

ggaaaagtaa cttaaggcaa gttctttata gttgagaggt cactttaatc tattaagtat
119640

tctattgtga tgggtatcac atgttcattg cagtattttc ttaatgtgca acagggatgt
119700

gggcaaataa attcgggtac atccatctgg tatgatatac attcttactg aaataatgtg
119760

gcaggggact atctggtgac aaagcaaaaa aattacaaaa tggtatggtt ccagtttttg
119820

taaaagaaaa aatatatatg tatgttaatt atatggtatg catagataat caaatgaaaa
119880

attatgtacc tgtgccataa cagtgtttct tttaacatat ttcttctttt tccttatctg

119940

tacatcctat tattctagag tcctttgtga gaagaaaaag tacaatgaaa acttaatagt
120000

ttttaaagtc atgtgcatca atgtttctgc ttaatttggt tcctacttca gaatcaacta
120060

ctgtactttc agagagaaaa accctcttct ggcttctcag gcaggaagtg actcagacct
120120

cccctgtttg aggcatgata attgctgtcc tgctttagct ctgcattgtt tattcggcgg
120180

atatttactg agcgcccact gtgtgctagg cgctattctt tagtctgaga attatagcca
120240

tgcataaatg ctgcaatagc catacaagtc tgactaaaat taggcacata atagacattt
120300

aaatatttct tattgcccaa ttgtccatat tctcagcgtt cagggttcaa caaacttggg
120360

tgtgatcaac tcacaatagc tatgagtcct ccccgatggc acattcttag tccagccttg
120420

gtgaaggtgg catggtcccc tgcccacact gctttttgtt tttcaatgag aaataaaaat
120480

aaaaataaca actaaaaaga tttttcctaa ttcttaaagt actgtactat gtgcttatgg
120540

taaaaaaaaa aaaaaaaaaa aaaaggaagt tacaggaaaa agaatcaaag aatgaatgcc
120600

atttacaatc tcactggaag gttacaattt ctgctcaaag aataaatcca ttgaagtgag
120660

gaacctgaga agtttgcaaa aggatgatct tttttctttg tcataattat ataatgagag
120720

aaacaagcaa acctcttttt aaatacttta acattctcag ctttaggaat aaaataagca
120780

gatgaaaatt agctttaaaa aactcagtgt gtcaaaacta tgcatctaga attatcgtta
120840

tctcttgaga ttttggtcat aaatggcagg tgctctagtt tttattttgc tcattgttag
120900

cctgaagagg tcatctgatt gaattcttaa acgctattat atccctcaaa actggggtta
120960

attttgcctc tctgataaat atggataata tttaacaaac ccagtttaga gaactatatg
121020

aagcattcag agtcctattt tatgcccttt aaaaaattgg atttcctgta gcagtaacta
121080

atttatcata gctttataaa gggaatctaa ttattcaaag taatatctaa tttgaagcag
121140

ccattattca ccaaccaacg ttaacactaa acaaaataaa attgagagag aaaagaatca
121200

gctctctcca ttatacagtc agcactattg caaatactta ttcaattact ttcagcaaac

121260

acaaggccct ctgtggtggc tgctccatgg gtaatttgct ctggaactct gacctttcag
121320

tttgtgacag tgaaaagaaa catcacatta atataggaga agatagaagg acctctcgaa
121380

agacccaaga aagtggtctt gataataata cctaccactt attatattct caccatatac
121440

caggcttctc atttaattct tataaagggc ttcagagatg ggcattagca tccccatttt
121500

ctaatgaggg ccatggggct ttgttggttt atataacctg cccaatatta gtggtagaag
121560

cagatttctc acagaaatct gccttgctct ggattgatgc ttaacaatta gttctaatgc
121620

ttcccattta gggagtagca aggtcagcca taatttaaat tccattcagc aaatattgtt
121680

agtcctggct acactcccag agccgtgata tggttgctct cctctaggaa tttgtagtat
121740

tattagaaat aattatagga tataaggtgg tatagaattc aattccaaat tggataaaac
121800

catgagccaa agtatttgca acagaaaata tatacttggg gcattcaaaa attccaaata
121860

ttccaattca gaattttaga tcttactcat tttgttggaa aagtatctga ttcaacaaag
121920

taaattgtat gcaaataaaa atataatctg ggaaaaacca ttaaacggtc agagatgaaa
121980

attttctctt agaattaaga gtactaacat gaatccctat tggtcatctt atctgtggca
122040

ctcgcaataa agaattataa gcaaggaaag tagtgtctct ctcctgcaca taccaaggaa
122100

ctgaaacacc ttctaattaa atgccaaata gagtattgaa taaagaatca aatccctgga
122160

ttttgttcca gaacatccat ttattaacta tataactcta ggtaaatgac tcaacctttt
122220

ggattctcag cttccccttt gtaagatgga aacgcagtac taatatgtgc actgtttaat
122280

ttcacaaagt tgactgagat gaaacatgta aaaagtgctt tggtgaacta taaagctgta
122340

aaagagtact tgccttattg cccttgtgcc ttggatgtgg tctgacgcag cacagatgtt
122400

taacaaatgt ttgctgaacc taatggctag gtttatgtgc aaaataaaca gagctggaga
122460

ggtgtctcaa cacaatgatg tttgtactaa ttatgtggct cccccgagcc tggctgtacc
122520

agagtccagc aggtcctcat ttcactcagc tctgccttac tctccatggt gctctctctc

122580

agctgctgca gactggaatg agtccactgg tctgcatgcc taggcttctc aaaacccctg
122640

gggactcgaa ggtggcatgt tgtggggagc aacctcagtg acctttagta gagagtgagg
122700

gtcagcaatc tcaaaacaca ctggagagta cccaaagggg ttctgaactg catggaaagc
122760

aaaataaata aaaaataata ataatcactt tctccagaga gcagttttga catgctgcta
122820

cttgcctctt gcattgtgat aaaaatacgt ggtaggaaat caatgtaggt ttccttggca
122880

gctgttgtat agaagaggtc aggtgcaaat ctgaacaagt tgctgaagga tgagtctgct
122940

cacaggcgtt tcctacttca tggaaacagc cagatgatag aaaacatgct taacatgacg
123000

attgttcata tttatctcaa agtaaaattt tccattcact caatatttat tagaactact
123060

ggatgcctaa attccttttg tctcaatttc ctttcctggg aaggctctaa gaaccttggc
123120

tccttggctg tatttttaga aacagaatat gcattgccac agtgaacatt aatatgtttg
123180

tttgtagggg tacctgggtg caagataggc aatagggtct tcagacaggg ccgtctttat
123240

aacattgact tagcccagct tgacaggtcc agaaacttga tatttttggt gtcccagggc
123300

taattttaca catggagagg gacttaaaat ataaaccaaa aggccaggcg tggtggctca
123360

cgcctgtaat cccagcactt tgggaggcca aggcaggcgg atcatgaggt caggagatcg
123420

agaccatcct ggctaacacg gtgaaaccga tctctactaa aaatatgaaa acaaaaaatt
123480

aacagggcgt ggtgacgggc acctgtagtc ccagctactt gggaggctga ggcagaagaa
123540

tggcatgaac ccgggtggcg gggcttgcag tgagctgaga tcatgccact gaactccagc
123600

ctgggtgaca gagcaagact ccatctcaaa aaaaaaaaat acatatatat atatatatat
123660

atatatatat atatgtatat accaaaaaac atacctacct atctggcatt tattctctgg
123720

ctgcaagaga gctttgggaa gccaagttta taatcataaa tatgtttggt gagtgtgaaa
123780

taataaagaa gagtaattca tttgatgact tgaatgttta gtggtatcaa gctattgtgc
123840

atgacaaatc cattgtcaca agagagatac gtttgccaat aagaccaaat atttagatgg

123900

gctggataag acactgtcat ccccaggcag aaggcatgaa gcagttaatc agtgaagtct
123960

ctctcttttt ttgagacaga gccttgctgt gtcacccagg ctggagtgca gtggcatgat
124020

cttggctcac tgcaacctct gcctcccggg ttcaagtgat tctcctgcct caacctcccg
124080

agtagctggg attacaggca cacacaccat gcccagctga tttttgtatt ttcagcagag
124140

acagggtttc accatgttgg ccaggctggt ctcaaacccc tgacctcaag tgatccaccc
124200

acctcggctt cccaaagtgc tgggattaca ggcgtgagcc acagcagtgg gcctgaagtc
124260

ttaatataca ggaaatacca tggcctcatg attctgctca aatcagaaaa ttgccctttc
124320

tattaaagat gatctctccc tattccttta tcagcccaat aacggagaat gcaaagacaa
124380

tacatcataa atatggtaga atgatgatca cacacaagag aggcaaatgg acaacgaaca
124440

gagaactcat atcagaaaga cattatcaga cagctgagga gccacgtgga gaaaaaagcc
124500

agtgcccaga agaatgtcat gagatgcagg cattttgcag agcctcaata aaatcaatat
124560

aaatatgtat atcgaatgtg aactaaacaa ttacataagc agaagttaag ttctgtccaa
124620

tccttctgat ggaatgcaaa attccaacaa agacaatcat gaggcaagaa tcatgtacca
124680

tgcatagaac cggagtacca gagtaaaggc aaccacaccc ttgtaaccat cacccaaatc
124740

aagaaacagg acattaccag ctctccaaaa attccctctg ttcccttcta gtcactaatt
124800

tcccagtggt aaccactctg ctgacatcta acaacataga ttagctcacc tgtttttata
124860

ctttgtataa atggaatata tgcttttttg tgtctggttt ctactgctca tctttatgtt
124920

ggagagattc atccatactt ttgtatgtag ttgtaaatca ttcttactgc tactgatata
124980

gcccaaattc tgtggctata tcacaattgg tttaactatt ttgttgttca tgggccggtt
125040

tgttggaaca acattggaat atttggaaca ttgttccaaa aattggaaca accatttgtt
125100

ccaaccatcc aacaaatggt tgttccaatt tttgtctact aacaagtact gttgccatga
125160

acattctgtt cccccaaatt ccccccgatt ctaattcttt tggtgaacat gtgcatacat

125220

ttctagtagg tgtatgcctt gaaatctgtt tgatgggtta caggttatgc atatgttcaa
125280

ctctagtagt aactcccact tctccaaagt ggttgtacca atttattatc ccacaagtgg
125340

tatatgataa ttcttattcc ttcacgtcct tgtcaacact tattattttc catctttttc
125400

atcttagcca ttttcgtgga tagcaagtgt tattttatta tggttttatg ttattttatt
125460

atgcatttcc ctgagaatta ataaacaatt ttctgtatgt ttatttacac atctctttta
125520

tgagactttt gcccattatg ctgttgagtt gttcaacttt ttcttattga cttgcaggtg
125580

ttctttatac attctagatt tgtgtttttt aatcatatat atgtgtgttt gcattggata
125640

taaattaata tatgttaata tttttctctc accttgtggg ttgccttttc actcaatttg
125700

ggggaacaga agtttttagt ttcaatgtag ccaagtttat cacatttttt ccttttatga
125760

ttaatgcttt ttcatttgct tattttctat ttaacaaagg attgctgact ccaaggtcat
125820

gaagatgttc tactctagaa gttttattgt ttcatctttc aattatttct gcaatctgtc
125880

tggtattgac tcttgtgtgt aatgtgaaat aaacattaga ataccttttt ttccacttga
125940

atgttgaatt gacacagcac aatttatctg aaagattatc cttttcccta ctgcattgca
126000

ggatcacctt tatcataaat catgccacca tatatgtgca ggtctacttc tggattccct
126060

gttgtgtttc attggacagt tatctgttct tgcactagta ccacacagtt ttgattgtca
126120

taactttata ataatagttt ctgatatctc acagtgtaag tcctgtatat ttattcttca
126180

agaataactt gatgatttcc aacttttaca tttgatatga attttaggaa aacttgtcaa
126240

tttctggaaa aagaagaacc tgtagcttag attttgacca aaattgcata gatgaatgta
126300

ttaatttgga gagaactaac atctttacaa tattgagtct tccaattata atgatgcata
126360

tacttccatt tatttagctc tcctttaatt tttctcaata atattttagt tttcagttag
126420

agatcttgca catctttaat tagatttatt tctaggtgtt tgagtttttt gatgctattg
126480

taaatatgtt ttattttcta tttgtttatt gccaatatat acaaatgcaa ctagttttta

126540

tatgttgact gtatatccgg ttcccttcct atattctctt attaattcta agaatttgtc
126600

cttagattct ctagaaattt tgaatgtgca tgatcttatt gtctggaaat gacaatttta
126660

tttcttgctt tccaatctcc atgacattta tttctttctc tgaccttttg cattggctag
126720

acctccttct acaatgttag actgaaatga tgacagtggt cattcttgac ccagttccag
126780

tcttaggggg aagttttcaa catactacca ttgagaatga tgtttgctac ggatattttg
126840

tagatattct ttatgagatt aagaaagttc tgttcttaat tgattacgtg ttttcatcac
126900

aaatggatgt tgaatttatc aaatgctttt tctgcatttg agatgattgt atgattttac
126960

tcctttttct gttaatgtgg tgaattatgt ttatgatttt tgaatggtaa gccacacttg
127020

tacacttgga ctgaacttgc actccattta ctgtatcaat ggatttggct tcctaatatt
127080

ttgttaagaa tttttaagta atgtttatga gaaagatggg cttgcagttt tctttctcac
127140

aatcttgtca gggtttaaaa atcaagttta tgctattaca atttttttta agtctggaaa
127200

gctcaccttc tctttctgtg ttctggaaga attatacaag acagctgtta atttttcctt
127260

aagtgtttgg aagaattcac tgataaacta tctgagccca ggcttttctt ttttaaaata
127320

atgggcttaa ttttaaaaaa aaatacatcc taaattattg atattttcat ctcttctcct
127380

gtcagtcttg gtaaattgta ttttcaaaga gtttgtttat ttcatccaaa ttgtcaaatt
127440

tatcagcata aatttgtttt aagtttcctt ttattgtcct tttgatgctt gtatgatcta
127500

tagtgatatc tcctttttca ttgctgttgt tgcgaatttg ttttgtctat ttttttttct
127560

tttgatcagt cttctttaga ttttgatgtt tttaaacaac tgactttggc tttattgtct
127620

ttcttatatg ttccttggtt tttgctttta tctttatcat ttctttttgt aagtttgatt
127680

cctgctctct ctctactttc ttgaaattaa cacttaaatc attgattttc aggctttttc
127740

ctttctaata tggaatataa gactatatat ttccttctaa gcaccatttt agtttttca
127800

catgattttt caaatgtgat ttttattatt taagtttaag aactaatttc tgtttttttt

127860

ctttatccca tggattattt agagtatatt gtttaatttt gagcacttgg gtttttcctg
127920

ttgttgttat tgatctttag cttaacttcc actgtggtca gagaagacat tctgaaagat
127980

gtcaatattt tgaaattgtt gagacttttt tcttgttatg atatattgat tgtaataaat
128040

gttcttatgt tctttgtata cttgaagtga atatgaagtg gtcattaggt accattttgc
128100

atacatgaca ttagattaca tatgttaact gtgctgttta tatcttctat atccttactg
128160

atttttttat ttttcacttt ttctgctatg tgtttgaaag agatgcacta atgcctccca
128220

ctgtgagtgc tgatttgtac atttttttct cattgttctg tccatttttg tttagtttat
128280

tttgaagcta tattattagg tgcacagaga tttaggattg tgctcatttc ctattggact
128340

tatttattta taattatgaa ctgtctcact ttatctctaa taatgtttct tgccttacag
128400

ttcacattgt ctgatattag tatagctaga cagctttagg agcaggggca ggtttgtgtt
128460

tgcgtggtat atttggcttt cctttcacat taaacatttc tttttattag atttgaagat
128520

ttgggtttgt tttttatcta atctgacagt attttacttc aaatttagct tatttacatt
128580

cagtgtaatc actgacatca ttgggcttat agctaccatc ttatttttca ttttctattg
128640

tgctcttttc ttttatattc cttttgttgt cattttatgt tctttttttc gccctctctt
128700

tcctctattt ttttaattat tcaaggattt cattctaaaa acttgcaagt tttacttctt
128760

taattctaat agtaactatc ctagagattg caatatatat ccttgattta ttacagaatt
128820

atacaaatta tctttgccac tcctctgata ctgttagacc ctttgacctt ttaaatgatt
128880

tatccctcct gattttgttc tttttgccac acatcttagt tctacatata ttttatacta
128940

caaacgacgt tataattact gtcttttata atcaatagtg acttacaaat atccatgttt
129000

accctcacca gtgtttttta ttccttcctt catttccatg tttttctgtc tggaattctc
129060

ttttctttct ctctctcttt cttttttttt tttttctttt ttgaggcagg tgtctcacgt
129120

tttgtcacac aagctggaat gcagtggcgt gatcatggct cactgcggcc tcaacctcct

129180

gggctcaagc aatcctccca cctcggcttc ctgagtagct gggactactg gtgtgtgcca
129240

ctgtgcccac aatttttttt tttttttttt ttgtagagac aaggtcttgc tttgttgcct
129300

aggctgagat tattttcctt ctgcctgaag aacatccttt agttttcctg aaaacatctt
129360

tattctactt tcatatttga aggatatgat tgcaagatat aattctagtc tggcaattat
129420

tttctttaag ctcttaaagt tgacaatatt gtcttctcgc atctataatt tatgttgata
129480

aatcaactgt gaatcttatt gttgcttctt tgaagctaat ttcttttttt tcttctggct
129540

gcttttatga tttttctctt tatctttggt tttcaacagt ttgactatga tgtggttagc
129600

tacagttttc tttatcttgc tccttgttct tacatcaata catagatatt cttatccatt
129660

atctctttga atattgcttc tcctctattc tctctctcct ttttggaact ccaattatat
129720

atatgtgaaa agtttcatgt gtatcttgtt cttgccattg tttccattct tttttctctt
129780

cctgcttcag gagtagatac tttctattta tctgggttcc agctcactga tccagcccat
129840

tattcactaa ttatgctgtg tgtcatctgc tgtaaactta tccatttgaa ttcctaattt
129900

caaatatttt ttcagtgttt agagttctaa aatatacatg tgattttttc acagattcca
129960

accttctagt gaaattctcc ttttattcat catttcacta tttatttttc atgttaatca
130020

taagtattat aaattccttt tctggtaact ccaacttttg gctcatttgt gggtcagaca
130080

gatctgagaa gcttggtttt tttttactct gtctcaggta ctttatattt agtgtttaca
130140

ttcctagcca gagaagcatt tatgtaaaca ctctctatat ttaatgactc agtttaagaa
130200

aatgcttaca ataattgagg tctccttgtt tctgaaccaa gttttatatc agaagaccac
130260

atcctcatct cggggagaag agcgaggaga gtcattgtct aagcattcct gagcactacg
130320

tctgtgcttc agtgttccag ccccatggaa aacccatttc tgtacagtat catatcccat
130380

agttaatcat acttttagat tttttaggaa tattttagtg tcattgctat ttagagatta
130440

tctgtatctt atattacaca ttagctaaac atgctctgat aatataaatg ttatttgagc

130500

taattagata aactttgatt tcaataattg ttcatcttta aaactgatgc agctttattt
130560

taggactaaa ggattattta tcttctttat atataataca cattatatat tatatattat
130620

atatggatag atataatagt aatgtaatgt gtacagctgt tcacataaat taattcacct
130680

atatttagct tcccctgtat cctgtcctct taagcacatt gagggcagag gcagttttct
130740

tgtatcttgt cactctacag cacctaacac aatacctttc ataaacataa tctgggctta
130800

gcaagtctgt ggattggatt cacttgcaaa atctatccat tgctttaaga gaaaccaata
130860

tttaaaagta gacttttcgt taaaaggata ttacagatat tacttgcttt gtttaataat
130920

tatctgcttt ggagatgtgt ttgcattttt aatgagtgct ttaatgagct tctattacat
130980

atattagaaa acaaacttgg aatagtgcca ctatgtaagt taactctgct ggtttaaaat
131040

atatttgaga tggaatttga ttaaataatt accaaaagta gatatgtaag actgtataag
131100

attgctttag tagcaagatc aggcagcaat attgattttt taaattaagt actaaatata
131160

cccagtcgaa ttcaaacact tactaaatga tattttgcaa ttttcaaaaa gccctaacat
131220

ttagcccctc agttgattcc cttaataact tgataaatta ggcaaaaaag caaagaagaa
131280

gaggatcatg gagagcaagg gatttaccca catttacatt tatattggca gagtcaggac
131340

ttgaactgaa gtctcctgag gcctaatttg atcttctatc tgtatcaacc agaaatacct
131400

aaaataactc atatgtctat aaagataaat gtacctgtag aaaatacatc cctcaaaaca
131460

cacatttaat tacaacttct taaacgagca ccctcttcct ttttcttaca gctctttatc
131520

cctagagttt aaagaatcaa aacacacata aacatctccc tagagcaaac atagggagtt
131580

tcctcatgac tggaagccca cattaaagcc acgcctaagg gaagaaggga aggcagggct
131640

gataatattt tgtttatttt agtccttctg cttagttttt gtttgcaggc actggactga
131700

acttagtttt cagagccaga aattatcatt gtttgtgtat ctaaatttgt taatactctg
131760

cttttagaaa tttttatgta caatggattc caagagtatt ttgggaactt ctcagtggtg

131820

aaatctaaac tgtggttcag tgaagagccg acaaacacaa actgcaaaga gcagttggca
131880

cagtacactt ttggaggcta tacgactctt ctgctcttct agcacaaggc acggagtagg
131940

tgcttgctac agttttcgga tgaatgaatg aaaagtacct tagtccccag cactgtctcc
132000

tcagttcacc caaagattcc ctgtgatggc cgtgccacca ttacagcagc agtttatttt
132060

ctgtgcaatt tgtagaagaa gaaccagaaa ccaaattgtt acccatgctc caaattgtta
132120

tccatgctcc aaagactatt cttggtttta tgccatagca aataaggtct taccatttcc
132180

aagtaaggct agccacatgc cagtctgtga aaaggaggca tttgcttttg ggtagtagtt
132240

gacatgcttt ggggcattta tgctgcctcc ccagctccca tctcagagaa ttcctgctat
132300

tcacagtcgc tgctggaaag gacaggctta agtggcccta tccccactcc atgtctgcct
132360

tgccccactc caatgagacc aggggtagac tcgtggacca caacaatcag actctctctt
132420

acgattctga gccgggttac agaaactacg gtcagtgact atggactgca gctgagagct
132480

cccaaccagc gagggctgag gttggtgcca gtgcaagcca aagtcaagga caactaaagc
132540

tacttaagag caagattata aatagacaaa gccaaagtct atgaacaaag gaaatcagac
132600

atgcttttaa aaagcaaaga gtatgcaaca ctcacaggga attaataaca atattattta
132660

ttaatttatt tattttattt atgttttgag atggagtctg tcgcccaggc tggagtgcag
132720

tggcacaatc tgggctcact gcaagctccg ccttccgggt tcaccccatt ctcctgcctc
132780

agcatcccga gtagctggga ctacaggcgc ccgccaccac gcccggctaa tttttctat
132840

ttttagtaga gatggggttt cattgtgtaa gccaggatgg tcttgatctc ctgacctcgt
132900

gatccgcccg cctccgcctc ccaaagtgct gggattacag gcgtgagcca ccgcgcctgg
132960

ccataatatt tttttaaaag caaagagact caaaagagag agtcaaaaaa caaaagagag
133020

aaagaaagag tgggagacta cagaccaatc aacttgagtt tcagtctctc tctttcccag
133080

ctttggtggc tatgaagtct agctggactt tgtttcctgc tggtggatgt gtgcaaaatt

133140

gttggcttaa tctttacagt taactcctgt ttttaatttg agttaatttg agtgagattc
133200

gattcccagc caccactcat ctctgaccaa tggacttccc cgtccccttc tcccctgcga
133260

accaggtgct tcagtttggc ccttctcgct cctatcaaga aagaatggga tctgaaaaac
133320

tttcacactg tcaaatactg agtttgctga cttttccagt aacattttag aagagcacag
133380

cagtgagaag gccacatgag acagtaaggg ataacaattc acataccatt ttgggcatca
133440

gttgtaatct atttcaggag gagcaccgga acaccggcta ttacctcagt agcagatcac
133500

ttactatccc ctttttgagg gctacttacc atcacacata aagcttttcc ttcctgccac
133560

actccgcaca ccacatacta cataagctct tctgtccatt gtaaatgcct tgaaagcggg
133620

gatcgtctcc actgtggttt tatactcact gctctcacac tagcagtgac aacatccatt
133680

tgaatgaata catgttctac atataatttt aatggatatt ccttctccct ctctcagaat
133740

gaatagtttg agaacttctg ggcctaatat tcagataaga ccccctcaaa aaccttcctt
133800

ttcccttttg ccactccctc tgcactacca tgcgcccttg tacttctgtg agatctccta
133860

aaactgaaga gcatgaggaa aacaggaaat tactattagc ttttccttag tccaggaaaa
133920

aaagactgag aggtggccca gtcatattca taaagtgttg cttaaagaag ccactattag
133980

caattgaaaa ggactttcta gttcagccct gcctcccatt ccatctgggc acctccagca
134040

tcagcccaca gtttctcagg ctccaatcca ttccatgcca gtcataaagc tgactttcca
134100

atgaagacac aaggcagaga gcacatttgc atcaatgcag gtacaggtgt ataaactcag
134160

tgactccaag cggctacact gagcaatgtt tacatacttg gaaaaaggac cattctcaca
134220

gattctattt aagaccaatg tggaaatatt aatagaaatt cttgaatcta gaactattca
134280

taaatactgt tttagaagaa tgtattttca ttcagaaagg aacacagttt ctgacagact
134340

gtgattaccg ttttatttcc tttttctata tcattaaacg caagggtagt attcagaaca
134400

ctcaaaagtc atatatggaa agaaaagaaa atgatgtttt tggcatgctt ggatctctat

134460

catattatgt tcttgtcttt tctggtacaa ttgtaggatt tattttctaa acattccaat
134520

tttacagcat gagtcattgc taatgtagaa accaactgaa tcatccatcc atccatcaat
134580

gaatattctt gtcttctttt tccttatatg tgcttatctt tatactaaaa aagatattag
134640

gaattcttgc atttaaagca cttcttctcc ctttcactta ttttgtttgt tttacaggta
134700

atacagtaaa gctatcatta actttgtttt ggttcagatt tccattcaaa atattgagtc
134760

agaaactcaa atcaagtgta ctcttaagta tctgaaatct gatgtcttcc atacaggagg
134820

taaatataaa gtctagaaaa aggagaatag aaaagaaata ttttcacact ttctggcctt
134880

aagaaagacc aaacagactg gattgtggta ataagtctat gtcttcttca cttagccttt
134940

aagagttgac taaaagttat ttgattggcc aggcatggtg gcttatggtt gtcatcccag
135000

cactttagga ggccgagggg ggcagatcat ctgagatcag gagttcgaga ccagcctggc
135060

cagtatggtg aaacactgtg tctattaaaa ataaaaaaaa ttagccgggc atggtgatgc
135120

atgccttata gtcccagcta ctcaggaggt cgaggcagga gaatcacttg aacccgggag
135180

gtggaggttg cagtgagccg agatcgtgcc tctgcactcc agcctgggcg acagagtgag
135240

gtccatctca aaaaaagaaa aaaaaaagtt atttgatcat cttgtttcaa atatgaagaa
135300

tctagtttat tccattccat cttaaatata tcacctagga tacttgatat tgtaatataa
135360

ttacttgtta ggcacatctt ttattattat tatctatacc agatggtttc aaggagaaga
135420

catatttact tggaaccaac ctatggtttg aggtggggct ggggagtggg caagagcact
135480

ggtccaggag tcaagagaac taaatgttag tcactgtgat accaccagtg agtgaagctg
135540

tcacttactg ggctttcttg ttcgttcgtt tctttttcag taaaatgaat ttccttcagt
135600

gcctgaaatc tctttctaag actcaatgac tgagcctttt cctattgttt atctcttgcc
135660

ccaaaacttg taacacaaag tctccccaat ttttatgcac ttaacaatcc actgttcagt
135720

agccattcta cattacatgc aattatcatc atgagccccc tttcccattc tgactatatg

135780

gagattatat ggggaaaact ataatatgga tcacagggaa aaaactgaat taaaaggaag
135840

atttggtccg ggcacggtgg ctcacacctg taatcccagc actttgggag gctgagacgg
135900

ccggatcagt tgagattggg agctcgaggc cagcctgatt aacagggaga aaccccttct
135960

caactaaaaa tacaaaatta gctgggcgtg gtggtgcatg cctgtaatcc cagctaccgg
136020

ggaagctgag gcaggagtgt catttgaacc caggaggcgg aggttgcagt gagccgagat
136080

ggcaccattg cactccagcc tgggcaacaa gagcaaaact ccatctcaaa aaaaaaaagg
136140

agggggaaga tttgaattta ttatgcatta tttctaagag agaactcaga gaaatcattt
136200

gtcctaaaca atagcttcta tctattgact cccaagtagt tcccaagaaa ctaaattgct
136260

tttaccaaat tatcctttta atacatccat gttatagact catcaactac ctgttcatta
136320

aaacacatgg cctaccaaaa ttatatctta acatcattag cttttaaata ctagaagtca
136380

tttcaaccct tagaaaagtc tggtgagcat gattacatgt ggaaactttc agatacaatg
136440

tatccaaaaa agtaagtgta ttaaaaatgt aaatatattt cagatatcca aaaatgtaga
136500

catattgtca ggctttgcta cttttttgca gtttcacatc atgtctttac ttgtaataat
136560

atcaataatt gctgcaaaga taataaacta cattcccatt cgcctatccc tctaccccag
136620

tactgtgtta tacagttttc cctgcaacat gatgtcagtc tgcaaagggt ctttgtgaca
136680

tcaggaagag agtctagatt tccttccagg gcaacacatt ttgctattag gttgagccat
136740

aaaactgcca tttttggtca ggaatgattt accactggta gtttcatatg gcacaagcta
136800

aagagccttc cttgggtttg agttacagtc ctgagagtcg cttgtgtgtc caatgggcca
136860

gttacttaat tgctgagtct tggtgtgtga aatggtaata acacccactt tataggattg
136920

attattaact caacattgag gcttagcaat gacgtatagg agcaccgtgc ctagaaacag
136980

ttattgttca agaaataggt aataatacaa tataattatc agtataagta acgtagaaga
137040

gtttcctcac ttttccttta gccatttttg catatatggt gaaggtaatg gttgctatgg

137100

tagagagaaa tggaaaatat ttgtgaagca agcacaattt ttaaaacccc aataacgtta
137160

aaggccttcc attcctgtct tgtatgctct cttatgtcca agtaagggca cagataaagt
137220

ttgacacata ctcggtggag tatctaaata gaaaatgtac tgaagcaaac caagaacaga
137280

gttttaaaaa aaaaatcaaa gcactaagca aacattctga acttttggca gaggaattgg
137340

actgtctgaa tctccatgac tctttggagt tattaatagt gatgcatttt gccatatttt
137400

gagcagatgc atatttatag gtcacgcaat atctgtgtgg ggagactttc aaggctttat
137460

ggccttttaa aaatgtctta tcttggaaga gaacagggca aggaggatgc cattttgtca
137520

tctgtgcagc accaatcacc ctgtgctcat taaatgttac gggaaagatg cgcactgcag
137580

cagggcctgg aatgtggctc ccatctgcac tgccagaagc catgatgaag tcacgagttg
137640

cacaacagta gtaattcgca gctatttatg accattttaa tagcaaaggt ctagattaga
137700

aggctgtgtt tacatgccat gtagcagaat agttcacgaa tcattcccac agtaacgttt
137760

ggtcaaacca atcaaccaag taatgaaaaa tgaaccggtt gttcttgagg ttgtgactaa
137820

tagatctcta agttagcctc tactttcctc atgctcttcc atacctgaga cagaattgac
137880

tcctgcccca tgcccacaga ctctcctcct ctgtcttccc ggcaagccac agccctgtta
137940

ccctttctcc acccaagctc aaagccgtaa gacatcaggt aagttaaagg agctttggaa
138000

actcctgtag agcacctgtg cacataatta aggaaacata atttttagca cgtttatttt
138060

ctcattgtca ttgctccatt atgtgacttt aattagtgac cccgatggcc cctgtgcatc
138120

cagatgagta aaatcctttc atttgtctgc ttaaactgac ttgaggtttt agtgattatc
138180

acatccatgc agcctgaagg tcagactatt tccttttgtt tcactagaca gttaatcccc
138240

tcattagggg tgatggaaaa tgaataatta aacatgccat tttcacattt caggtaatga
138300

gaggccctaa gatcccagag gatgccataa gaggaaaagt caggggagtg ttgaagactc
138360

tgcctcctac aaatatgtga gtttttctcc cctggcttca tggtagaaaa ggtttggcga

138420

aggctcttcc tctaaattga aacaaaacaa aatgaaaacc ttagttattg ggctgtactc
138480

tgacacttag tagctgtgtg acttggggca aagcagctaa ccttttgaca ctaaaagttt
138540

tctcaattat ataactggaa tgataatggt aaccattgta ttgaaatgtt atgagcatta
138600

aaaacatgaa tgagtgacaa tacttacaac agtgtctacc acatggtaag agctctgtaa
138660

tgtgttaatt atattatcat tttcattatc acccaggcaa gtcacctgaa tgctgtgggt
138720

atcaggccac agccaaacaa ccacttgcag gttatcacaa agttcccttt ggcctattgg
138780

aataaggatt aagagtaaat tatgttgtac cctggaggga aaaaacccaa gtagtcatac
138840

ctctagggcc ttagggaatt caactttcac caaacctctt ctaattactc taaaaagatt
138900

ttttggaggg tggaggtggt agaaggtaga gagccacaat agtcgggacc cagatgacca
138960

cacttggtga gtgaaggtga agccacccac agcagcctcc actggtgcaa tctgaattcc
139020

tgcctccaat cccaggcccc acagcttctg ccccttcctc atgttctcca actcccagga
139080

agccggctcg ccccgggctt ccccagccct gtaccatgga gactgggcag ggacctcctt
139140

ttgtactctg acttctgtct catctgacca tattttaggc agaccttacg ctcatgccga
139200

ctaaaaatgg actcactctg ctaaggaaat ccaagcccca ttgggaaata tctctgagtc
139260

taatttggtc atttcttttg ccgcttcagg ctgtggacct tacaaagcag agacagcacc
139320

cttcaagatg cagagttcag ggaaagctcc tatacagagg ggagtgggag tctccgctga
139380

gtttggaaag atgactgggg tttgattgac agagtgaacg cggtgggggg cggcggggga
139440

gatgtttcaa gtgaagaggg tctggaatgt gcctagaaca gggaatcttc tgacaggctg
139500

aaacaggctg tgtaagggag tgcaggtggc taaagctgct cagagagaca cagaccagtg
139560

cggaaagaaa gttctgaggg ttcatgaaga tattggactt tattctgcat aaatacagat
139620

ggtgaagggt ggaagcagtg tttcagaaaa gaaaaagtgt gtagtgctgt gcatgcagca
139680

ttggtagaaa tgggcttgat gggaggaaaa ttacttagcg gactagggcc tgaggtcatg

139740

gggacctatg aaaaggaaaa taaaatgcat gagggtggct atatgattat gtgcagaggg
139800

ggtggaaact gttgctcaaa actgaatctt aaaatttgtt ttgtcattgc tttacccaaa
139860

atagtcgacc ctagttggat ttttttggag aaaatatttt gtccctttta aaaaggttct
139920

agtaagaagc agctttaatg ctgtttgtgg aaggaaaaag ttacaaggag aaagagtgaa
139980

tacagtgcat gattaaagga gtgatggaaa gtgacaatgg ctttcaaaag agcacaggcg
140040

aattgacacg tattatgaca aacacgatct tacagtgact tcattttctt gtgagtgttg
140100

ggaaatactt tctagatatt ctaataatga tgccgttagc attgtacaat ttttaattaa
140160

aaagctt
140167

<210> 182
<211> 2116
<212> DNA
<213> Homo sapiens

<400> 182
tattagaacc attctgcctt gctaatgctg cctaccttgg gggccaagaa atgatggagg
60

tggtgaagag agatgacgaa accataaagg agcatttatg taagcttaca ttttattatg
120

gtactataga tccttggtgt ccaaaagagt actatgaaga cattaagaag gattttccag
180

aaggagacat tcgactctgt gagaaaaaca tacctcatgc tttcatcacc cattttaacc
240

aggaaatggc agacatgatt gctgactccc taaaggatga cttgtccaaa atgtaaattg
300

gcctgaggaa caagccccca ctgccagtac atggaggcag tcagtgtact agacttagta
360

ggtaaatgtt taattttgaa gactgatatt agaaatgaag aaagtgagaa cctttgtctt
420

acaaaccaac tctccgctcg ccatgttata ggctgaagta aacacagttg atgaatcatt
480

ccataggttt aaccatacat tttccaagac tcagggaaca cagtgatcta cacagagtct
540

tgtgtttgca caagatgccc agtggcacca tatggtttat tttggtaggc aggatctttg
600

cagatgaaaa aaaaatctac atgtacttga ttttaattga gttacattgt agaataggct
660

cctctggagg aaattatgaa atacctacta gaaaatgtaa aataaatcag tgaatgttaa
720

gagtatagtt agatatgtga agtgtatgag attatgacaa ggatacactc atgttccagg
780

agcaggaagt gaacctgggt ctcctgtaag acagaagatg aagatgagcc caggctaact
840

tagcacagat cttggctgag atcatcaatg tgacgtctaa tgtacctgca ctagacagag
900

aataaagttc accagacatt actctggtca gctaaccaga taaagaattg ttgaaggacc
960

ccaactgtgc ctcctgccac aggacaacca gcaagttcta tgctgagcct tagcctccca
1020

ggttataagc tccctgcagg ctcctcctct ccagagccag gatggagagg cactgggctg
1080

tcccaaagca ggcttggatg tgccaacgta cagttgctcc ttctgtaatt cttgcactaa
1140

aactccatta aagaccatca atgagccaaa tagtgtgcta gccattgagg atggagttct
1200

tagctactta atgattcttt tcttctggaa atcttctagt gaataatttt taaaagcttc
1260

ttttaaactg ctcagtaaaa cattgccata cattaactct ccatttctgc attaacttca
1320

tttgctggaa agaaattatt ttgttaatga accaaaccca gccattaagt tatgtgaatt
1380

caccttctct agacacattg aggtatggca gagaagacac atattccctg gcctagagtg
1440

ggctgacaat attgctgcct cccctaaatt gactcaatct tgagaaccac atactgcagt
1500

gacagttgat catggatcag aaatattcct ttttctccta gaggaaatcc ctcatggcat
1560

tatcattccc taaggacaat gagatgtccc tccgctcaaa tcctaaagcc ccctgatgaa
1620

agatttggct ctgatttgct gccaacttat tgggattaga ctaaaatgga caaacagtgg
1680

aagaaatcca ttttccagaa ggagctctgc ctgtatgtcc cagtcaacca catggatggt
1740

cgttgtgaaa gttttaactt cctgcagagt taccgagtgc tcagcgattg agctttgacc
1800

acacccgtgt ttgttcctag tctgttccca gccataagca tctccacaag ccctctgtgg
1860

cttgtgtggc ctgatgtcca tggccaccaa gggcctctca ccatacgctt ctccactcaa
1920

cctttggaga gacacggacc accaaaggct tgatttttct tcatcctaca ttatattcaa
1980

aacaaacagg ctgggtgccg tagctcacac ctgtgatccc agcactttgg gaggatgagc
2040

tgggaagatc gtttgaggct aggagtttga gaccagcccg tgcaaaaaac gagacctcgt
2100

gtctaaaata attttt
2116

<210> 183
<211> 2116
<212> DNA
<213> Homo sapiens

<400> 183
tattagaacc attctgcctt gctaatgctg cctaccttgg gggccaagaa atgatggagg
60

tggtgaagag agatgacgaa accataaagg agcatttatg taagcttaca ttttattatg
120

gtactataga tccttggtgt ccaaaagagt actatgaaga cattaagaag gattttccag
180

aaggagacat tcgactctgt gagaaaaaca tacctcatgc tttcatcacc cattttaacc
240

aggaaatggc agacatgatt gctgactccc taaaggatga cttgtccaaa atgtaaattg
300

gcctgaggaa caagccccca ctgccagtac atggaggcag tcagtgtact agacttagta
360

ggtaaatgtt taattttgaa gactgatatt agaaatgaag aaagtgagaa cctttgtctt
420

acaaaccaac tctccgctcg ccatgttata ggctgaagta aacacagttg atgaatcatt
480

ccataggttt aaccatacat tttccaagac tcagggaaca cagtgatcta cacagagtct
540

tgtgtttgca caagatgccc agtggcacca tatggtttat tttggtaggc aggatctttg
600

cagatgaaaa aaaaatctac atgtacttga ttttaattga gttacattgt agaataggct
660

cctctggagg aaattatgaa atacctacta gaaaatgtaa aataaatcag tgaatgttaa
720

gagtatagtt agatatgtga agtgtatgag attatgacaa ggatacactc atgttccagg
780

agcaggaagt gaacctgggt ctcctgtaag acagaagatg aagatgagcc caggctaact
840

tagcacagat cttggctgag atcatcaatg tgacgtctaa tgtacctgca ctagacagag
900

aataaagttc accagacatt actctggtca gctaaccaga taaagaattg ttgaaggacc
960

ccaactgtgc ctcctgccac aggacaacca gcaagttcta tgctgagcct tagcctccca
1020

ggttataagc tccctgcagg ctcctcctct ccagagccag gatggagagg cactgggctg
1080

tcccaaagca ggcttggatg tgccaacgta cagttgctcc ttctgtaatt cttgcactaa
1140

aactccatta aagaccatca atgagccaaa tagtgtgcta gccattgagg atggagttct
1200

tagctactta atgattcttt tcttctggaa atcttctagt gaataatttt taaaagcttc
1260

ttttaaactg ctcagtaaaa cattgccata cattaactct ccatttctgc attaacttca
1320

tttgctggaa agaaattatt ttgttaatga accaaaccca gccattaagt tatgtgaatt
1380

caccttctct agacacattg aggtatggca gagaagacac atattccctg gcctagagtg
1440

ggctgacaat attgctgcct cccctaaatt gactcaatct tgagaaccac atactgcagt
1500

gacagttgat catggatcag aaatattcct ttttctccta gaggaaatcc ctcatggcat
1560

tatcattccc taaggacaat gagatgtccc tccgctcaaa tcctaaagcc ccctgatgaa
1620

agatttggct ctgatttgct gccaacttat tgggattaga ctaaaatgga caaacagtgg
1680

aagaaatcca ttttccagaa ggagctctgc ctgtatgtcc cagtcaacca catggatggt
1740

cgttgtgaaa gttttaactt cctgcagagt taccgagtgc tcagcgattg agctttgacc
1800

acacccgtgt ttgttcctag tctgttccca gccataagca tctccacaag ccctctgtgg
1860

cttgtgtggc ctgatgtcca tggccaccaa gggcctctca ccatacgctt ctccactcaa
1920

cctttggaga gacacggacc accaaaggct tgatttttct tcatcctaca ttatattcaa
1980

aacaaacagg ctgggtgccg tagctcacac ctgtgatccc agcactttgg gaggatgagc
2040

tgggaagatc gtttgaggct aggagtttga gaccagcccg tgcaaaaaac gagacctcgt
2100

gtctaaaata attttt
2116


<210> 184
<211> 717
<212> DNA
<213> Homo sapiens

<400> 184
cagaggcagg cagcatctct gagggtcccc aaggaacatg gctgggagcc gtgaggtggt

60

ggccatggac tgcgagatgg tggggctggg gccccaccgg gagagtggcc tggctcgttg
120

cagcctcgtg aacgtccacg gtgctgtgct gtacgacaag ttcatccggc ctgagggaga
180

gatcaccgat tacagaaccc gggtcagcgg ggtcacccct cagcacatgg tgggggccac
240

accatttgcc gtggccaggc tggagatcct gcagctcctg aaaggcaagc tggtggtggg
300

tcatgacctg aagcacgact tccaggcact gaaagaggac atgagcggct acacaatcta
360

cgacacgtcc actgacaggc tgttgtggcg tgaggccaag ctggaccact gcaggcgtgt
420

ctccctgcgg gtgctgagtg agcgcctcct acacaagagc atccagaaca gcctgcttgg
480

acacagctcg gtggaagatg cgagggcaac gatggagctc tatcaaatct cccagagaat
540

ccgagcccgc cgagggctgc cccgcctggc tgtgtcagac tgaagcccca tccagcccgt
600

tccgcaggga ctagaggctt tcggcttttt gggacagcaa ctaccttgct tttggaaaat
660

acatttttaa tagtaaagtg gctctatatt ttctctacgc caaaaaaaaa aaaaaaa
717


<210> 185
<211> 2894
<212> DNA
<213> Homo sapiens

<400> 185
ggagaccgcg tctgcttcaa cttgggccgt gagctctatt tctacccagg ctgctgtcgt
60

cgtgggagcc aacggtccat tgacctcaac aagccaattg acaagcggat ctacaagggc
120

acccagccca cctgccacga tttcaaccag ttcactgctg ccaccgagac catctcgctg
180

ctggtgggct tctcagcggg tcaagtgcag tacctggatc tcatcaaaaa ggacaccagc
240

aagctgttca atgaggagcg gttgatcgac aagaccaagg tgacatatct gaagtggctg
300

cctgagtcgg agagcctgtt cctggcatca cacgccagtg gccacctgta cctgtacaac
360

gtcagccacc cctgcgcctc ggccccgccc cagtacagcc tgctgaagca gggcgagggc
420

ttctctgtct atgctgccaa gagcaaggca ccccgcaacc cgctggccaa gtgggcggtg
480

ggtgagggtc ccctcaacga gttcgccttc tcgcccgatg gccggcacct ggcctgtgtg
540

agccaggatg cgtgcctgcg cgtcttccac ttcgactcca tgctcctgcg tgggctcatg
600

aagagctact ttgggggcct gctgtgtgtg tgctggagcc ctgacggccg ctacgtggtg
660

acgggtggcg aagatgacct ggtcaccgtg tggtccttca ccgagggccg cgtggtggct
720

cgaggccatg gccacaagtc ctgggtcaac gctgtggcct ttgactctct ctacaccaca
780

agggcagagg aggcggcgac agcagccggt gctgatgggg agcggagcgg cgaagaggag
840

gaggaggagc ccgaggctgc gggcacaggc tcggccgggg gcgccccact ctctccactg
900

cccaaggctg gctccattac ttaccgcttt ggctcggcgg gccaggacac gcagttctgc
960

ctgtgggacc tcactgaaga cgtgctctac ccgcacccgc ccctggcccg cacccgcacc
1020

ctccctggca cacctggcac cacgccaccg gccgccagca gctcgagggg tggcgagcct
1080

ggcccaggcc ccctgcctcg ctcgctgtcc cgctccaaca gtctcccgca cccagctggc
1140

gggggcaagg cgggcggccc gggtgtggcg gcagagcctg gcacaccatt cagcattggc
1200

cgcttcgcca cgctcacact gcaggagcgg cgggaccggg gggcagagaa ggagcacaag
1260

cgctaccaca gcctgggcaa catcagccgg ggtggcagtg gcggcagtgg cagtggtggg
1320

gagaagccca gcggccctgt tccccgcagc cgcctggacc ccgccaaggt gctgggcact
1380

gcgctgtgcc cgcgcatcca cgaggtgccc ctgctggagc cccttgtgtg caagaagatc
1440

gcccaggagc ggctcacagt cctcctgttc ctggaggact gcatcatcac tgcctgccag
1500

gagggcctca tctgcacctg gccgcggccg ggcaaggcgt tcacagacga ggagaccgag
1560

gcccagacag gggaaggaag ttggcccagg tcacccagca agtcagtggt agagggcatc
1620

tcctcccaac caggcaactc cccgagtggc acagtggtgt gaagccatgg atatcgggcc
1680

cccccaaccc catgccccca gcctcctagc cataaccctc cctgctgacc tcacagatca
1740

acgtattaac aagactaacc atgatggatg gactgctcca gtccccccac ctgcacaaaa
1800

tttgggggcc ccccagactg gcccggacac gggcgatgta atagcccttg tggcctcagc
1860

cttgtccccc acccactgcc aagtacaatg acctcttcct ctgaaacatc agtgttaccc
1920

tcatccctgt ccccagcatg tgactggtca ctcctgggga gagactcccc gcccctgcca
1980

caagagcccc aggtctgcag tgtgcccctc agttgagtgg gcagggcggg ggtggtccag
2040

ccctcgcccg gcccccaccc cagctgccct tgctattgtc tgtgcttttg aagagtgtta
2100

aattatggaa gcccctcagg ttcctccctg tcccgcagga cctcttattt atactaaagt
2160

tccctgtttt ctcagcgggt ctgtcccctt cggaggagat gatgtagagg acctgtgtgt
2220

gtactctgtg gttctaggca gtccgctttc cccagaggag gagtgcaggc ctgctcccag
2280

cccagcgcct cccacccctt ttcatagcag gaaaagccgg agcccaggga gggaacggac
2340

ctgcgagtca cacaactggt gacccacacc agcggctgga gcaggaccct cttggggaga
2400

agagcatcct gcccgcagcc agggcccctc atcaaagtcc tcggtgtttt ttaaattatc
2460

agaactgccc aggaccacgt ttcccaggcc ctgcccagct gggactcctc ggtccttgcc
2520

tcctagtttc tcaggcctgg ccctctcaag gcccaggcac cccaggccgg ttggaggccc
2580

cgacttccac tctggagaac cgtccaccct ggaaagaaga gctcagattc ctcttggctc
2640

tcggagccgc agggagtgtg tcttcccgcg ccaccctcca ccccccgaaa tgtttctgtt
2700

tctaatccca gcctgggcag gaatgtggct ccccgccagg ggccaaggag ctattttggg
2760

gtctcgtttg cccagggagg gcttggctcc accactttcc tcccccagcc tttgggcagc
2820

aggtcacccc tgttcaggct ctgagggtgc cccctcctgg tcctgtcctc accacccctt
2880

ccccacctcc tggg
2894


<210> 186
<211> 3552
<212> DNA
<213> Homo sapiens

<400> 186
ctgactggtg ctccctctct ttcatcttgg gctgtctgca tgtgtctcat tcccccactc
60

tctcctgtgc ctcccctcta ctctaataat caggtccagg tttctctgta ctgggagaag
120

acctgtggct ggagcaggca gggatgcacc ctatctgttc cccattcctc caggtgggag
180

ggagaaggag taacccactt tattggccac agatgcaggg gagaaaggag aaagcatgct
240

gggagctgga aagagcccta agatcacctg gtttctagag agtggagcct gcttcctgcc
300

taggcccctc ccacaatgct tgtcgccggt cttcttctct gggcttccct actgactggg
360

gcctggccat ccttccctac ccaggaccac ctcccggcca cgccccgggt acggctctca
420

ttcaaagagc tgaaggccac aggcaccgcc cacttcttca acttcctgct caacacaacc
480

gactaccgaa tcttgctcaa ggacgaggac cacgaccgca tgtacgtggg cagcaaggac
540

tacgtgctgt ccctggacct gcacgacatc aaccgcgagc ccctcattat acactgggca
600

gcctccccac agcgcatcga ggaatgcgtg ctctcaggca aggatgtcaa cggcgagtgt
660

gggaacttcg tcaggctcat ccagccctgg aaccgaacac acctgtatgt gtgcgggaca
720

ggtgcctaca accccatgtg cacctatgtg aaccgcggac gccgcgccca ggattacatc
780

ttctacctgg agcctgagcg actcgagtca gggaagggca agtgtccgta cgatcccaag
840

ctggacacag catcggccct catcaatgag gagctctatg ctggtgtgta catcgatttt
900

atgggcactg atgcagccat cttccgcaca cttggaaagc agacagccat gcgcacggat
960

cagtacaact cccggtggct gaacgacccg tcgttcatcc atgctgagct cattcctgac
1020

agtgcggaga atgatgataa gctttacttc ttcttccgtg agcggtcggc agaggcgccg
1080

cagagccccg cggtgtacgc ccgcatcggg cgcatttgcc tgaacgatga cggtggtcac
1140

tgttgcctgg tcaacaagtg gagcacattc ctgaaggcgc ggctcgtctg ctctgtcccg
1200

ggcgaggatg gcattgagac tcactttgat gagctccagg acgtgtttgt ccagcagacc
1260

caggacgtga ggaaccctgt catttacgct gtctttacct cctctggctc cgtgttccga
1320

ggctctgccg tgtgtgtcta ctccatggct gatattcgca tggtcttcaa cgggcccttt
1380

gcccacaaag aggggcccaa ctaccagtgg atgcccttct cagggaagat gccctaccca
1440

cggccgggca cgtgccctgg tggaaccttc acgccatcta tgaagtccac caaggattat
1500

cctgatgagg tgatcaactt catgcgcagc cacccactca tgtaccaggc cgtgtaccct
1560

ctgcagcggc ggcccctggt agtccgcaca ggtgctccct accgccttac cactattgcc
1620

gtggaccagg tggattcagc cgacgggcgc tatgaggtgc ttttcctggg cacagaccgc
1680

gggacagtgc agaaggtcat tgtgctgccc aaggatgacc aggagatgga ggagctcatg
1740

ctggaggagg tggaggtctt caaggatcca gcacccgtca agaccatgac catctcttct
1800

aagaggcaac aactctacgt ggcgtcagcc gtgggtgtca cacacctgag cctgcaccgc
1860

tgccaggcgt atggggctgc ctgtgctgac tgctgccttg cccgggaccc ttactgtgcc
1920

tgggatggcc aggcctgctc ccgctataca gcatcctcca agaggcggag ccgccggcag
1980

gacgtccggc acggaaaccc catcaggcag tgccgtgggt tcaactccaa tgccaacaag
2040

aatgccgtgg agtctgtgca gtatggcgtg gccggcagcg cagccttcct tgagtgccag
2100

ccccgctcgc cccaagccac tgttaagtgg ctgttccagc gagatcctgg tgaccggcgc
2160

cgagagattc gtgcagagga ccgcttcctg cgcacagagc agggcttgtt gctccgtgca
2220

ctgcagctca gcgatcgtgg cctctactcc tgcacagcca ctgagaacaa ctttaagcac
2280

gtcgtcacac gagtgcagct gcatgtactg ggccgggacg ccgtccatgc tgccctcttc
2340

ccaccactgt ccatgagcgc cccgccaccc ccaggcgcag gccccccaac gcctccttac
2400

caggagttag cccagctgct ggcccagcca gaagtgggcc tcatccacca gtactgccag
2460

ggttactggc gccatgtgcc ccccagcccc agggaggctc caggggcacc ccggtctcct
2520

gagccccagg accagaaaaa gccccggaac cgccggcacc accctccgga cacatgaggc
2580

cagctgcctg ttctgccatg ggcagcctag cccttgtccc ttttaatata aaagacatat
2640

atatatatat atatatatat atataaaaat atctatattc tatacacacc ctgcccctgc
2700

aaagacagta tttattggtg ggttgaatat agcctgcctc agtggcagca tcctccaaaa
2760

cttagaccca tgctggtcag agacggcaga aaacagagcc tgcctaacca ggccagccag
2820

ttagtggggc caggccagga ccacacagtc cccagactca gctggagtct acctgctgga
2880

caggccttgc gccaagatct acaggacaaa gggagggagc aagccctact cggatggggc
2940

acggaccgtc cacctttctg atgtgtgttg tcagcctgtg ctgtggcata gacatggatg
3000

cgaggaccac tttggagact ggggtggcct caagagcaca cagagaaggg aagaaggggc
3060

catcacagga tgccacccct gccttgggtt gggggcactc tgccacgacc agccccttcc
3120

tgggtattta ttctctattt attggggata ggagaagagg catcctgcct gggtgggaca
3180

gccccttggg ccccttctcc cctccccgcc tggccaggga gggccacccc actctacctc
3240

cttagctttc cctgtgccac tttgactcag aggctgggag catagcagag gccaggccca
3300

ggcagagctg acgggaggcc ccagctctga ggggaggggg tccgtggtag agcctggggc
3360

aggtagaggc tccccagggc tcccttatgt ccaccacttc aggggatggg tgtggatgta
3420

attagctctg gggggcagtt gggtagatgg gtgggggctc ctggtggcct tctgctgccc
3480

aggccacagc cgcctttggg ttccatcttg ctaataaaca ctggctttgg aactaaaaaa
3540

aaaaaaaaaa aa
3552


<210> 187
<211> 2026
<212> DNA
<213> Homo sapiens

<400> 187
gaattccggt gccatggctg agtcacacct gctgcagtgg ctgctgctgc tgctgcccac
60

gctctgtggc ccaggcactg ctgcctggac cacctcatcc ttggcctgtg cccagggccc
120

tgagttctgg tgccaaagcc tggagcaagc attgcagtgc agagccctag ggcattgcct
180

acaggaagtc tggggacatg tgggagccga tgacctatgc caagagtgtg aggacatcgt
240

ccacatcctt aacaagatgg ccaaggaggc cattttccag gacacgatga ggaagttcct

300

ggagcaggag tgcaacgtcc tccccttgaa gctgctcatg ccccagtgca accaagtgct
360

tgacgactac ttccccctgg tcatcgacta cttccagaac cagactgact caaacggcat
420

ctgtatgcac ctgggcctgt gcaaatcccg gcagccagag ccagagcagg agccagggat
480

gtcagacccc ctgcccaaac ctctgcggga ccctctgcca gaccctctgc tggacaagct
540

cgtcctccct gtgctgcccg gggccctcca ggcgaggcct gggcctcaca cacaggatct
600

ctccgagcag caattcccca ttcctctccc ctattgctgg ctctgcaggg ctctgatcaa
660

gcggatccaa gccatgattc ccaagggtgc gctagctgtg gcagtggccc aggtgtgccg
720

cgtggtacct ctggtggcgg gcggcatctg ccagtgcctg gctgagcgct actccgtcat
780

cctgctcgac acgctgctgg gccgcatgct gccccagctg gtctgccgcc tcgtcctccg
840

gtgctccatg gatgacagcg ctggcccaag gtcgccgaca ggagaatggc tgccgcgaga
900

ctctgagtgc cacctctgca tgtccgtgac cacccaggcc gggaacagca gcgagcaggc
960

cataccacag gcaatgctcc aggcctgtgt tggctcctgg ctggacaggg aaaagtgcaa
1020

gcaatttgtg gagcagcaca cgccccagct gctgaccctg gtgcccaggg gctgggatgc
1080

ccacaccacc tgccaggccc tcggggtgtg tgggaccatg tccagccctc tccagtgtat
1140

ccacagcccc gacctttgat gagaactcag ctgtccagct gcaaaggaaa agccaagtga
1200

gacgggctct gggaccatgg tgaccaggct cttcccctgc tccctggccc tcgccagctg
1260

ccaggctgaa aagaagcctc agctcccaca ccgccctcct cacctccctt cctcggcagt
1320

cacttccact ggtggaccac gggcccccag ccctgtgtcg gccttgtctg tctcagctca
1380

accacagtct gacaccagag cccacttcca tcctctctgg tgtgaggcac agcgagggca
1440

gcatctggag gagctctgca gcctccacac ctaccacgac ctcccagggc tgggctcagg
1500

aaaaaccagc cactgcttta caggacaggg ggttgaagct gagccccgcc tcacacccac
1560

ccccatgcac tcaaagattg gattttacag ctacttgcaa ttcaaaattc agaagaataa

1620

aaaatgggaa catacagaac tctaaaagat agacatcaga aattgttaag ttaagctttt
1680

tcaaaaaatc agcaattccc cagcgtagtc aagggtggac actgcacgct ctggcatgat
1740

gggatggcga ccgggcaagc tttcttcctc gagatgctct gctgcttgag agctattgct
1800

ttgttaagat ataaaaaggg gtttcttttt gtctttctgt aaggtggact tccagatttt
1860

gattgaaagt cctagggtga ttctatttct gctgtgattt atctgctgaa agctcagctg
1920

gggttgtgca agctagggac ccattcctgt gtaatacaat gtctgcacca atgctaataa
1980

agtcctattc tcttttaaaa aaaaaaaaaa aaaaaaaacg gaattc
2026

<210> 188
<211> 1929
<212> DNA
<213> Homo sapiens

<400> 188
acccccgcac ccagctccgc agaccggcgg gcgcgcgcgg gctctggagg ccacgggcat
60

gatgcttcgg gtcctggtgg gggctgtcct ccctgccatg ctactggctg ccccaccacc
120

catcaacaag ctggcactgt tcccagataa gagtgcctgg tgcgaagcca agaacatcac
180

ccagatcgtg ggccacagcg gctgtgaggc caagtccatc cagaacaggg cgtgcctagg
240

acagtgcttc agctacagcg tccccaacac cttcccacag tccacagagt ccctggttca
300

ctgtgactcc tgcatgccag cccagtccat gtgggagatt gtgacgctgg agtgcccggg
360

ccacgaggag gtgcccaggg tggacaagct ggtggagaag atcctgcact gtagctgcca
420

ggcctgcggc aaggagccta gtcacgaggg gctgagcgtc tatgtgcagg gcgaggacgg
480

gccgggatcc cagcccggca cccaccctca cccccatccc cacccccatc ctggcgggca
540

gacccctgag cccgaggacc cccctggggc cccccacaca gaggaagagg gggctgagga
600

ctgaggcccc cccaactctt cctcccctct catccccctg tggaatgttg ggtctcactc
660

tctggggaag tcaggggaga agctgaagcc cccctttggc actggatgga cttggcttca
720

gactcggact tgaatgctgc ccggttgcca tggagatctg aaggggcggg gttagagcca
780

agctgcacaa tttaatatat tcaagagtgg ggggaggaag cagaggtctt cagggctctt
840

tttttggggg gggggtggtc tcttcctgtc tggcttctag agatgtgcct gtgggagggg
900

gaggaagttg gctgagccat tgagtgctgg gggaggccat ccaagatggc atgaatcggg
960

ctaaggtccc tgggggtgca gatggtactg ctgaggtccc gggcttagtg tgagcatctt
1020

gccagcctca ggcttgaggg agggctgggc tagaaagacc actggcagaa acaggaggct
1080

ccggccccac aggtttcccc aaggcctctc accccacttc ccatctccag ggaagcgtcg
1140

ccccagtggc actgaagtgg ccctccctca gcggaggggt ttgggagtca ggcctgggca
1200

ggaccctgct gactcgtggc gcgggagctg ggagccaggc tctccgggcc tttctctggc
1260

ttccttggct tgcctggtgg gggaagggga ggaggggaag aaggaaaggg aagagtcttc
1320

caaggccaga gggaggggga caacccccca agaccatccc tgaagacgag catccccctc
1380

ctctccctgt tagaaatgtt agtgccccgc actgtgcccc aagttctagg ccccccagaa
1440

agctgtcaga gccggccgcc ttctcccctc tcccagggat gctctttgta aatatcggat
1500

gggtgtggga gtgaggggtt acctccctcg ccccaaggtt ccagaggccc taggcgggat
1560

gggctcgctg aacctcgagg aactccagga cgaggaggac atgggacttg cgtggacagt
1620

cagggttcac ttgggctctc tctagctccc caattctgcc tgcctcctcc ctcccagctg
1680

cactttaacc ctagaaggtg gggacctggg gggagggaca gggcaggcgg gcccatgaag
1740

aaagcccctc gttgcccagc actgtctgcg tctgctcttc tgtgcccagg gtggctgcca
1800

gcccactgcc tcctgcctgg ggtggcctgg ccctcctggc tgttgcgacg cgggcttctg
1860

gagcttgtca ccattggaca gtctccctga tggaccctca gtcttctcat gaataaattc
1920

ttcggaatt
1929

<210> 189
<211> 13747

<212>  DNA
<213>  Homo sapiens

<400>  189
ggatccgcca aggactttga ttattgcgtg aaagtgctga ctgccaggac aggaagctag
60

ctaagatgca agttcccagc ctagagcagt ggcctctggg gggtctaggg cggacccaag
120

ggcaaggcca gggtggcagc agcttgggga ctctggctgg ctccctcccc tgacactggc
180

tgaagcccag gtggtctcta acccctccca tctctccctc tcatcttccc cagggcatct
240

cctcccaacc aggcaactcc ccgagtggca cagtggtgtg aagccatgga tatcgggccc
300

ccccaacccc atgcccccag cctcctagcc ataaccctcc ctgctgacct cacagatcaa
360

cgtattaaca agactaacca tgatggatgg actgctccag tccccccacc tgcacaaaat
420

ttgggggccc cccagactgg cccggacacg ggcgatgtaa tagcccttgt ggcctcagcc
480

ttgtccccca cccactgcca agtacaatga cctcttcctc tgaaacatca gtgttaccct
540

catccctgtc cccagcatgt gactggtcac tcctggggag acactccccg cccctgccac
600

aagagcccca ggtctgcagt gtgcccctca gttgagtggg cagggccggg ggtggtccag
660

ccctcgcccg gcccccaccc cagctgccct tgctattgtc tgtgcttttg aagagtgtta
720

aattatggaa gcccctcagg ttcctccctg tcccgcagga cctcttattt atactaaagt
780

tccctgtttt ctcagcgggt ctgtcccctt cggaggagat gatgtagagg acctgtgtgt
840

gtactctgtg gttctaggca gtccgctttc cccagaggag gagtgcaggc ctgctcccag
900

cccagcgcct cccacccctt ttcatagcag gaaaagccgg agcccaggga gggaacggac
960

ctgcgagtca cacaactggt gacccacacc agcggctgga gcaggaccct cttggggaga
1020

agagcatcct gcccgcagcc agggcccctc atcaaagtcc tcggtgtttt ttaaattatc
1080

agaactgccc aggaccacgt ttcccaggcc ctgcccagct gggactcctc ggtccttgcc
1140

tcctagtttc tcaggcctgg ccctctcaag gcccaggcac cccaggccgg ttggaggccc
1200

cgacttccac tctggagaac cgtccaccct ggaaagaaga gctcagattc ctcttggctc
1260

tcggagccgc agggagtgtg tcttcccgcg ccaccctcca ccccccgaaa tgtttctgtt
1320

tctaatccca gcctgggcag gaatgtggct ccccggccag gggccaagga gctattttgg
1380

ggtctcgttt gcccagggag ggcttggctc caccactttc ctcccccagc ctttgggcag
1440

caggtcaccc ctgttcaggc tctgagggtg ccccctcctg gtcctgtcct caccacccct
1500

tccccacctc ctgggaaaaa aaaaaaaaaa aaaaaaaaag ctggtttaaa gcagagagcc
1560

tgagggctaa atttaactgt ccgagtcgga atccatctct gagtcaccca agaagctgcc
1620

ctggcctccc gtccccttcc caggcctcaa cccctttctc ccacccagcc ccaaccccca
1680

gccctcaccc cctagccccc agttctggag cttgtcggga gcaagggggt ggttgctact
1740

gggtcactca gcctcaattg gccctgttca gcaatgggca ggttcttctt gaaattcatc
1800

acacctgtgg cttcctctgt gctctacctt tttattgggg tgacagtgtg acagctgaga
1860

ttctccatgc attcccccta ctctagcact gaagggttct gaagggccct ggaaggaggg
1920

agcttggggg gctggcttgt gaggggttaa ggctgggagg cgggaggggg gctggaccaa
1980

ggggtgggga gaaggggagg aggcctcggc cggccgcaga gagaagtggc cagagaggcc
2040

caggggacag ccagggacag gcagacatgc agccagggct ccagggcctg gacaggggct
2100

gccaggccct gtgacaggag gaccccgagc ccccggcccg gggaggggcc atggtgctgc
2160

ctgtccaaca tgtcagccga ggtgcggctg aggcggctcc agcagctggt gttggacccg
2220

ggcttcctgg ggctggagcc cctgctcgac cttctcctgg gcgtccacca ggagctgggc
2280

gcctccgaac tggcccagga caagtacgtg gccgacttct tgcagtgggg tgagtgccta
2340

ccctcggggc tcctgcagat ggggtggggg tggggcagca gacagctctg ggcacagagg
2400

cctggctgtt gggggggggc agcatggcag gatgggcatg gggagatcct cccatcctgg
2460

ggctcagagt gtggacctgg gccctggggc aacatttctc tgtcctatgc caccactctg
2520

gaggggcaga gtaaggtcag cagaggctag ggtggctgtg actcagagcc atggcttagg
2580

agtcacagca ggctaggctg ccaacagcct cccatggcct ctctgcaccc cgcctcaggg
2640

tcagggtcag ggtcatgctg ggagctccct ctcctaggac cctcccccca aaagtgggct
2700

ctatggccct ctcccctggt ttcctgtggc ctggggcaag ccaggagggc cagcatgggg
2760

cagctgccag gggcgcagcc gacaggcagg tgttcggcgc cagcctctcc agctgcccca
2820

acaggtgccc aggcgctggg agggcggtga ctcacgcggg ccctgtggga gaaccagctt
2880

tgcagacagg cgccaccagt gccccctcct ctgcgatcca ggagggacaa ctttgggttc
2940

ttctgggtgt gtctccttct ttagtaggtt ctgcacccac ccccaccccc agccccaaag
3000

tctcggttcc tatgagccgt gtgggtcaga caccattccc gccaccccgg gtccctgcgt
3060

cctttagttc tcctggccca gggcctccaa ccttccagct gtcccacaaa accccttctt
3120

gcaagggctt tccagggcct ggggccaggg ctggaaggag gatgcttccg cttctgccag
3180

ctgccttgtc tgcccaacct cctccccaag cccaggactc gggctcactg gtcactggtt
3240

tctttcattc ccagcaccct gctcctctgg ccctcatatg tctggccctc agtgactggt
3300

gtttggtttt tgggctgtgt gtaacaaact gtgtgtgaca cttgtttcct gtttctccgc
3360

cttcccctgc ttcctcttgt gtccatctct ttctgaccca ggcctggttc ctttccctcc
3420

tcctcccatt tcacagatgg gaaggtggcg gccaagaagg gccaggccat tcagcctctg
3480

gaaaaacctt ctcccaacct cccacagccc ctaatgactc tcctggcctc cctttagtag
3540

aggatgaagt tgggttggca gggtaaactg agaccgggtg gggtaggggt ctggcgctcc
3600

cgggaggagc actccttttg tggcccgagc tgcatctcgc ggcccctccc ctgccaggcc
3660

tggggcgggg gaggggccca gggttcctgc tgccttaaaa gggctcaatg tcttggctct
3720

ctcctccctc ccccgtcctc agccctggct ggttcgtccc tgctggccca ctctcccgga
3780

accccccgga acccctctct ttcctccaga acccactgtc tcctctcctt ccctcccctc
3840

ccatacccaa ccctctctcc atcctgtcct ccacttcttc cacccccggg agagccaggc
3900

ctcccctgtg ccccacagtg ccctgaggcc acaagcctcc accccagctg gtccccaccc
3960

aggctgccca gtttaacatt cctagtcata ggaccttgac ttctgagagg cctgattgtc
4020

atctgtaaat aaggggtagg actaaagcac tcctcctgga ggactgagag atgggctgga
4080

ccggagcact tgagtctggg atatgtgacc atgctacctt tgtctccctg tcctgttcct
4140

tcccccagcc ccaaatccag ggttttccaa agtgtggttc aagaaccacc tgcatctgaa
4200

tctagaggta ctggatacaa ccccacgtct gggccgttac ccaggacatt ctacatgaga
4260

acgtgggggt ggggccctgg ctgcacctga actgtcacct ggagtcaggg tggaaggtgg
4320

aagaactggg tcttatttcc ttctcccctt gttctttagg gtctgtcctt ctgcagactc
4380

cgttacccca ccctaaccat cctgcacacc cttggagccc tctgggccaa tgccctgtcc
4440

cgcaaagggc ttctcaggca tctcacctct atgggagggc atttttggcc cccagaacct
4500

tacacggtgt ttatgtgggg aagcccctgg gaagcagaca gtcctagggt gaagctgaga
4560

ggcagagaga aggggagaca gacagagggt ggggctttcc cccttgtctc cagtgccctt
4620

tctggtgacc ctcggttctt ttcccccacc acccccccag cggagcccat cgtggtgagg
4680

cttaaggagg tccgactgca gagggacgac ttcgagattc tgaaggtgat cggacgcggg
4740

gcgttcagcg aggtaagccg aaccgggcgg gagcctgact tgactcgtgg tgggcggggc
4800

ataggggttg gggcgggccc ttagaaattg atgaatgacc gagccttaga acctagggct
4860

gggctggagg cggggcttgg gaccaatggg cgtggtgtgg caggtggggc ggggccacgg
4920

ctgggtgcag aagcgggtgg agttgggtct gggcgagccc ttttgttttc ccgccgtctc
4980

cactctgtct cactatctcg acctcaggta gcggtagtga agatgaagca gacgggccag
5040

gtgtatgcca tgaagatcat gaacaagtgg gacatgctga agaggggcga ggtgaggggc
5100

tgggcggacg tgggggcttt gaggatccg cgccccgtct ccggctgcag ctcctccggg
5160

tgccctgcag gtgtcgtgct tccgtgagga gagggacgtg ttggtgaatg gggaccggcg
5220

gtggatcacg cagctgcact tcgccttcca ggatgagaac tacctggtga gctccgggcc
5280

ggggggacta ggaagaggga caagagcccg tgctgtcact ggacgaggag gtggggagag
5340

gaagctctag gattgggggt gctgcccgga aacgtctgtg ggaaagtctg tgtgcggtaa
5400

gagggtgtgt caggtggatg aggggccttc cctatctgag acggggatgg tgtccttcac
5460

tgcccgtttc tggggtgatc tgggggactc ttataaagat gtctctgttg cggggggtct
5520

cttacctgga atgggatagg tcttcaggaa ttctaacggg gccactgcct agggaaggag
5580

tgtctgggac ctattctctg ggtgttgggt ggcctctggg ttctctttcc cagaacatct
5640

cagggggagt gaatctgccc agtgacatcc caggaaagtt tttttgtttg tgtttttttt
5700

tgaggggcgg gggcgggggc cgcaggtggt ctctgatttg gcccggcaga tctctatggt
5760

tatctctggg ctggggctgc aggtctctgc ccaaggatgg ggtgtctctg ggaggggttg
5820

tcccagccat ccgtgatgga tcagggcctc aggggactac caaccaccca tgacgaaccc
5880

cttctcagta cctggtcatg gagtattacg tgggcgggga cctgctgaca ctgctgagca
5940

agtttgggga gcggattccg gccgagatgg cgcgcttcta cctggcggag attgtcatgg
6000

ccatagactc ggtgcaccgg cttggctacg tgcacaggtg ggtgcagcat ggccgagggg
6060

atagcaagct tgttccctgg ccgggttctt ggaaggtcag agcccagaga ggccagggcc
6120

tggagaggga ccttcttggt tggggcccac cggggggtgc ctgggagtag gggtcagaac
6180

tgtagaagcc ctacaggggc ggaacccgag gaagtggggt cccaggtggc actgcccgga
6240

ggggcggagc ctggtgggac cacagaaggg aggttcattt atcccaccct tctcttttcc
6300

tcccgtgcag ggacatcaaa cccgacaaca tcctgctgga ccgctgtggc cacatccgcc
6360

tggccgactt cggctcttgc ctcaagctgc gggcagatgg aacggtgagc cagtgccctg
6420

gccacagagc aactggggct gctgatgagg gatggaaggc acagagtgtg ggagcgggac
6480

tggatttgga ggggaaaaga ggtggtgtga cccaggctta agtgtgcatc tgtgtggcgg
6540

agtattagac caggcagagg gaggggctaa gcatttgggg agtggttgga aggagggccc
6600

agagctggtg ggcccagagg ggtgggccca agcctcgctc tgctcctttt ggtccaggtg
6660

cggtcgctgg tggctgtggg caccccagac tacctgtccc ccgagatcct gcaggctgtg
6720

ggcggtgggc ctgggacagg cagctacggg cccgagtgtg actggtgggc gctgggtgta
6780

ttcgcctatg aaatgttcta tgggcagacg cccttctacg cggattccac ggcggagacc
6840

tatggcaaga tcgtccacta caaggtgagc acggccgcag ggagacctgg cctctcccgg
6900

taggcgctcc caggctatcg cctcctctcc ctctgagcag gagcacctct ctctgccgct
6960

ggtggacgaa ggggtccctg aggaggctcg agacttcatt cagcggttgc tgtgtccccc
7020

ggagacacgg ctgggccggg gtggagcagg cgacttccgg acacatccct tcttctttgg
7080

cctcgactgg gatggtctcc gggacagcgt gccccccttt acaccggatt tcgaaggtgc
7140

caccgacaca tgcaacttcg acttggtgga ggacgggctc actgccatgg tgagcggggg
7200

cggggtaggt acctgtggcc cctgctcggc tgcgggaacc tccccatgct ccctccataa
7260

agttggagta aggacagtgc ctaccttctg gggtcctgaa tcactcattc cccagagcac
7320

ctgctctgtg cccatctact actgaggacc cagcagtgac ctagacttac agtccagtgg
7380

gggaacacag agcagtcttc agacagtaag gccccagagt gatcagggct gagacaatgg
7440

agtgcagggg gtgggggact cctgactcag caaggaaggt cctggagggc tttctggagt
7500

ggggagctat ctgagctgag acttggaggg atgagaagca ggagaggact cctcctccct
7560

taggccgtct ctcttcaccg tgtaacaagc tgtcatggca tgcttgctcg gctctgggtg
7620

cccttttgct gaacaatact ggggatccag cacggaccag atgagctctg gtccctgccc
7680

tcatccagtt gcagtctaga gaattagaga attatggaga gtgtggcagg tgccctgaag
7740

ggaagcaaca ggatacaaga aaaaatgatg ggcggcaggc aacgggtggg ctcacgcctg
7800

taacccccag caatttggca ggccgaagtg ggtggattgc ttgagcccag gagttcgaga
7860

ccagcctggg caatgtggtg agacccccgt ctctacaaaa atgttttaaa aattggttgg
7920

gcgtggtggc gcatgcctgt atactcagct actagggtgg ccgacgtggg cttgagccca
7980

ggaggtcaag gctgcagtga gctgtgattg tgccactgca ctccagcctg ggcaacggag
8040

agagactctg tctcaaaaat aagataaact gaaattaaaa aataggctgg gctggccggg
8100

cgtggtggct cacgcctgta atctcagcac tttgggaggc cgaggcgggt ggatcacgag
8160

gtcagaagat ggagaccagc ctggccagcg tggcgaaacc ccgtctctac ccaaaaatat
8220

aaaaaattag ccaggcgtgg tagagggcgc ctgtaatctc agctactcag gacgctgagg
8280

caggagaatc gcctgaacct gggaggcgga ggttgcagtg agctgagatt gcaccactgc
8340

actccagcct gggtaacaga gcgagactcc gtatcaaaga aaaagaaaaa agaaaaaatg
8400

ctggaggggc cactttagat aacccctgag ttggggctgg tttgggggga acatgtaagc
8460

caagatccaa aagcagtgag gggcccgccc tgacgactgc tgctcacatc tgtgtgtctt
8520

gcgcaggaga cactgtcgga cattcgggaa ggtgcgccgc taggggtcca cctgcctttt
8580

gtgggctact cctactcctg catggccctc aggtaagcac tgccctggac ggcctccagg
8640

ggacacgagg ctgcttgagc ttcctgggtc ctgctccttg gcagccaatg gagttgcagg
8700

atcagtcttg gaacctcact gtttggggcc cacagactcc taagaggcca gagttggagg
8760

accttaaatt tctcagatct atgtacttca aatgttagat tgaattttaa aacctcagag
8820

tcacagactg ggcttcccag aatcttgtaa ccattaactt ttacgtctgt agtacacaga
8880

gccacaggac ttcagaactt ggcaaatatg aagtttagac ttttacaatc agttgtaaaa
8940

gaatgcaaat tctttgaatc agccatataa caataaggcc atttaaaagt attaatttag
9000

gcgggccgcg gtggctcacg cctgtaatcc tagcactttg ggaggccaag gcaggtggat
9060

catgaggtca ggagatcgag accatcctgg ctaacacggt gaaaccccgt ctctactaaa
9120

aatacaaaaa aattagccgg gcatggtggc gggcgcttgc ggtcccagct acttgggagg
9180

cgaggcagga gaatggcatg aacccgggag gcggagcttg cagtgagccg agatcatgcc
9240

actgcactcc agcctgggcg acagagcaag actccgtctc aaaaaaaaaa aaaaaaaagt
9300

ttttatttag gccgggtgtg gcggctcacg cctgtaatcc agtgctttgg gaggatgagg
9360

tgggtggatc actgaggtca ggagttcgag accagcctga ccacgtggag aaacctcatc
9420

tctactaaaa aacaaaatta gccaggcgtg gtggcatata cctgtaatcc cagctactca
9480

ggaggctgag gcaggagaat cagaacccag gagggggagg ttgtggtgag ctgagatcgt
9540

gccattgcat tccagcctgg gcaacaagag tgaaacttca tctccaaaaa aaaaaaaaaa
9600

aagtactaaa tttacaggct gggcatggtg gctcacgctt ggaatcccag cactttggga
9660

ggctgaagtg gacggattgc ttcagcccag gagttcaaga ccagcctgag caacataatg
9720

agaccctgtc tctaccaaaa attgaaaaaa tcgtgccagg catggtggtc tgtgcctgca
9780

gtcctagcta ctcaggagtc tgaagtagga gaatcacttg agcctggagt ttgaggcttc
9840

agtgagccat gatagattcc agcctaggca acaaagtgag acctggtctc aacaaaagta
9900

ttaattacac aaataatgca ttgcttatca caagtaaatt agaaaataca gataaggaaa
9960

aggaagttga tatctcgtga gctcaccaga tgggcagtgg tccctggctc acacgtgtac
10020

tgacacatgt ttaaatagtg gagaacaggt gtttttttgg tttgtttttt tccccttcct
10080

catgctactt tgtctaagag aacagttggt tttctagtca gcttttatta ctgggcaaca
10140

ttacacatac tataccttat cattaatgaa ctccagcttg attctgaacc gctgcggggc
10200

ctgaacggtg ggtcaggatt gaacccatcc tctattagaa cccaggcgca tgtccaggat
10260

agctaggtcc tgagccgtgt tcccacagga gggactgctg ggttggaggg gacagccact
10320

tcatacccca gggaggagct gtccccttcc cacagctgag tggggtgtgc tgacctcaag
10380

ttgccatctt ggggtcccat gcccagtctt aggaccacat ctgtggaggt ggccagagcc
10440

aagcagtctc cccatcaggt cggcctccct gtcctgaggc cctgagaaga ggggtctgca
10500

gaaggtttag aaagagcagc tcccaggggc ccaaggccag gagaggggca gggcttttcc
10560

taagcagagg aggggctatt ggcctacctg ggactctgtt ctcttcgctc tgctgctccc
10620

cttcctcaaa tcaggaggtc ttggaagcag ctgcccctac ccacaggcca gaagttctgg
10680

ttctccacca gagaatcagc attctgtctc cctccccact ccctcctcct ctccccaggg
10740

acagtgaggt cccaggcccc acacccatgg aagtggaggc cgagcagctg cttgagccac
10800

acgtgcaagc gcccagcctg gagccctcgg tgtccccaca ggatgaaaca gtaagttggt
10860

ggaggggagg gggtccgtca gggacaattg ggagagaaaa ggtgagggct tcccgggtgg
10920

cgtgcactgt agagccctct agggacttcc tcgaacagaa gcagacagaa accacggaga
10980

gacgaggtta cttcagacat gggacggtct ctgtagttac agtggcgcat taagtaaggg
11040

tgtgtgtgtt gctggcgatc tgagaagtcg atctttgagc tgagcgctgg tgaaggagaa
11100

acaagccatg gaaggaaagg tgccaagtgg tcaggcgaga gcctccaggg caaaggcctt
11160

gggcaggtgg gaatcctgat ttgttcctga aaggtagttt gtctgagtca ctacctgaga
11220

aggctggaga ggccagcagg aaacacaacc cagcacggcc tgttgtcgtg tgggcactag
11280

ggagctggag ggattttgag caccagaggg acatagggtg tgttagtgtg tgagcaccag
11340

ccctctggtg ccctgtgtag atttagagga ccagactcag ggatgggtct gagggaggta
11400

gagaagggag ggggcttgga tcattgcagg agctatgggg attccagaaa tgttgagggg
11460

gcggaggagt aggggataaa caaggattcc tagcctggaa ccagtgtcca agtcctgagt
11520

cttccaggag ccacaggcag ccttaagcct ggtccccaca cacaggctga agtggcagtt
11580

ccagcggctg tccctgcggc agaggctgag gccgaggtga cgctgcggga gctccaggaa
11640

gccctggagg aggaggtgct cacccggcag agcctgagcc gggagatgga ggccatccgc
11700

acggacaacc agaacttcgc caggtcggga tcggggccgg ggccggggcc gggatgcggg
11760

ccggtggcaa cccttggcat cccctctcgt ccggcccgga cggactcacc gtccttacct
11820

ccccacagtc aactacgcga ggcagaggct cggaaccggg acctagaggc acacgtccgg
11880

cagttgcagg agcggatgga gttgctgcag gcagagggag ccacaggtga gtccctcatg
11940

tgtccccttc cccggaggac cgggaggagg tgggccgtct gctccgcggg gcgtgtatag
12000

acacctggag gagggaaggg acccacgctg gggcacgccg cgccaccgcc ctccttcgcc
12060

cctccacgcg ccctatgcct ctttcttctc cttccagctg tcacgggggt ccccagtccc
12120

cgggccacgg atccaccttc ccatgtaaga cccctctctt tcccctgcct cagacctgct
12180

gcccattctg cagatcccct ccctggctcc tggtctcccc gtccagatat agggctcacc
12240

ctacgtcttt gcgactttag agggcagaag ccctttattc agccccagat ctccctccgt
12300

tcaggcctca ccagattccc tccgggatct ccctagataa cctccccaac ctcgattccc
12360

ctcgctgtct ctcgccccac cgctgagggc tgggctgggc tccgatcggg tcacctgtcc
12420

cttctctctc cagctagatg gccccccggc cgtggctgtg ggccagtgcc cgctggtggg
12480

gccaggcccc atgcaccgcc gccacctgct gctccctgcc agggtacgtc cggctgccca
12540

cgcccccctc cgccgtcgcg ccccgcgctc cacccgcccc gtgccacccg cttagctgcg
12600

catttgcggg gctgggccca cggcaggagg gcggatcttc gggcagccaa tcaacacagg
12660

ccgctaggaa gcagccaatg acgagttcgg acgggattcg aggcgtgcga gtggactaac
12720

aacagctgta ggctgttggg gcgggggcgg ggcgcaggga agagtgcggg cccacctatg
12780

ggcgtaggcg gggcgagtcc caggagccaa tcagaggccc atgccgggtg ttgacctcgc
12840

cctctccccg caggtcccta ggcctggcct atcggaggcg ctttccctgc tcctgttcgc
12900

cgttgttctg tctcgtgccg ccgccctggg ctgcattggg ttggtggccc acgccggcca
12960

actcaccgca gtctggcgcc gcccaggagc cgcccgcgct ccctgaaccc tagaactgtc
13020

ttcgactccg gggccccgtt ggaagactga gtgcccgggg cacggcacag aagccgcgcc
13080

caccgcctgc cagttcacaa ccgctccgag cgtgggtctc cgcccagctc cagtcctgtg
13140

taccgggccc gccccctagc ggccggggag ggaggggccg ggtccgcggc cggcgaacgg
13200

ggctcgaagg gtccttgtag ccgggaatgc tgctgctgct gctgctgctg ctgctgctgc
13260

tggggggatc acagaccatt tctttctttc ggccaggctg aggccctgac gtggatgggc
13320

aaactgcagg cctgggaagg cagcaagccg ggccgtccgt gttccatcct ccacgcaccc
13380

ccacctatcg ttggttcgca aagtgcaaag ctttcttgtg catgacgccc tgctctgggg
13440

agcgtctggc gcgatctctg cctgcttact cgggaaattt gcttttgcca aacccgcttt
13500

ttcggggatc ccgcgccccc ctcctcactt gcgctgctct cggagcccca gccggctccg
13560

cccgcttcgg cggtttggat atttattgac ctcgtcctcc gactcgctga caggctacag
13620

gacccccaac aaccccaatc cacgttttgg atgcactgag accccgacat tcctcggtat
13680

ttattgtctg tccccaccta ggacccccac ccccgaccct cgcgaataaa aggccctcca
13740

tctgccc
13747